# EUROPEAN PATENT SPECIFICATION

(11) **EP 2 689 020 B1**
(45) Date of publication and mention of the grant of the patent: **16.05.2018**
(21) Application number: 12759932.2
(22) Date of filing: 22.03.2012
(51) Int. Cl.: C12P 1/00

(54) **MICROBIAL PRODUCTION OF CHEMICAL PRODUCTS AND RELATED COMPOSITIONS, METHODS AND SYSTEMS**
MIKROBIELLE HERSTELLUNG CHEMISCHER PRODUKTE UND ZUSAMMENSETZUNGEN DARAUS SOWIE VERFAHREN UND SYSTEME DAFÜR
PRODUCTION MICROBIENNE DE PRODUITS CHIMIQUES, ET COMPOSITIONS, PROCÉDÉS ET SYSTÈMES ASSOCIÉS

(30) Priority: 22.03.2011 US 201161466363 P; 22.03.2011 US 201161466433 P; 26.09.2011 US 201161539378 P; 26.09.2011 US 201161539162 P
(43) Date of publication of application: 29.01.2014
(73) Proprietor: OPX Biotechnologies Inc., Boulder, CO 80301 (US)
(72) Inventor: LYNCH, Michael, D., Boulder, CO 80301 (US); LIPSCOMB, Tanya, E.w., Boulder, CO 80301 (US); TRAHAN, Ashley, D., Boulder, CO 80301 (US); SINGH, Amar, Boulder, CO 80301 (US); WOLTER, Travis, Boulder, CO 80301 (US)
(74) Representative: Gowshall, Jonathan Vallance
(86) International application number: PCT/US2012/030209
(87) International publication number: WO 2012/129450

(56) References cited:
- WO-A2-2007/093848
- XUEFENG LU ET AL: "Overproduction of free fatty acids in E. coli: Implications for biodiesel production", METABOLIC ENGINEERING, vol. 10, no. 6, 1 November 2008 (2008-11-01), pages 333-339, XP055100754, ISSN: 1096-7176, DOI: 10.1016/j.ymben.2008.08.006
- XIN MENG ET AL: "Increasing fatty acid production inby simulating the lipid accumulation of oleaginous microorganisms", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 38, no. 8, 26 October 2010 (2010-10-26), pages 919-925, XP019928933, ISSN: 1476-5535, DOI: 10.1007/S10295-010-0861-Z
- ZHA ET AL.: 'Improving cellular malonyl-CoA level in Escherichia coli via metabolic engineering' METAB..ENG. vol. 11, no. 3, May 2009, pages 192 - 198, XP026034671
- FOWLER ET AL.: 'Increased Malonyl Coenzyme A Biosynthesis by Tuning the Escherichia coli Metabolic Network and Its Application to Flavanone Production' APPL. ENVIRON. MICROBIOL. vol. 75, no. 18, September 2009, pages 5831 - 5839, XP055003952

### SEQUENCE LISTING

<110> OPX BIOTECHNOLOGIES, INC.
<120> MICROBIAL PRODUCTION OF CHEMICAL PRODUCTS AND RELATED COMPOSITIONS, METHODS AND SYSTEMS
<130> 34246-760601
<140>
   <141>
<150> 61/539,378
   <151> 2011-09-26
<150> 61/539,162
   <151> 2011-09-26
<150> 61/466,433
   <151> 2011-03-22
<150> 61/466,363
   <151> 2011-03-22
<160> 215
<170> PatentIn version 3.5
<210> 1
   <211> 5385
   <212> DNA
   <213> Artificial Sequence
<220>
   <223> Description of Artificial Sequence: Synthetic pTrc-pTrc_THNS Ligation of inverted Flaviolin into pTrc vector polynucleotide supplied by Invitrogen
<400> 1
   gtttgacagc ttatcatcga ctgcacggtg caccaatgct tctggcgtca ggcagccatc      60
   ggaagctgtg gtatggctgt gcaggtcgta aatcactgca taattcgtgt cgctcaaggc     120
   gcactcccgt tctggataat gttttttgcg ccgacatcat aacggttctg gcaaatattc    180
   tgaaatgagc tgttgacaat taatcatccg gctcgtataa tgtgtggaat tgtgagcgga     240
   taacaatttc acacaggaaa cagcgccgct gagaaaaagc gaagcggcac tgctctttaa     300
   caatttatca gacaatctgt gtgggcactc gaccggaatt atcgattaac tttattatta     360
   aaaattaaag aggtatatat taatgtatcg attaaataag gaggaataaa ccatgaccac     420
   tctgtgccgc cctagcgtta gcgtcccgga acacgtgatt accatggagg aaacgctgga     480
   actggcgcgt cgtcgtcata cggatcatcc gcagctgccg ctggctttgc gtctgatcga     540
   gaataccggc gtgcgcactc gtcacattgt tcagccaatt gaggacacgc tggagcatcc     600
   gggtttcgag gaccgcaaca aagtctatga acgtgaagcc aagagccgcg ttccggctgt     660
   gatccagcgt gccctggacg atgcagaact gctggctacg gatattgatg ttatcatcta     720
   cgtgagctgc acgggtttta tgatgccgag cctgaccgcg tggttgatta acgagatggg 780
   tttcgactcc accacccgtc aaatcccgat tgcgcagctg ggttgtgcag cgggtggtgc 840
   ggcgatcaac cgtgcccatg atttttgcac cgcgtacccg gaagccaatg cactgatcgt 900
   cgcttgcgaa ttctgtagcc tgtgctatca gccgaccgac ttgggtgtgg gtagcttgct 960
   gtgcaacggt ctgttcggcg atggcattgc agcggccgtg gttcgcggtc gtggtggcac 1020
   cggtgttcgc ctggaacgta atggttctta cctgattccg aaaaccgaag attggattat 1080
   gtatgatgtg aaggcgacgg gtttccattt tctgctggac aagcgtgtcc cggcgaccat 1140
   ggaaccgttg gctccggcac tgaaagagct ggctggtgag cacggctggg acgccagcga 1200
   tctggacttc tacattgtgc acgcgggtgg tccgcgtatt ctggatgacc tgagcacctt 1260
   tctggaagtc gaccctcacg cattccgctt ttcccgtgca acgctgacgg agtacggcaa 1320
   tatcgcaagc gcggttgttt tggacgcact gcgtcgtctg ttcgacgagg gtggtgttga 1380
   ggaaggcgca cgcggcttgc tggcgggttt tggtccgggt attactgcag aaatgagcct 1440
   gggttgttgg cagaccgccg atgttcgtcg cggcatccgc caagatgtga cccgcacggc 1500
   ggcacgtggc gttagccgcc gtgtgcgtca agcttggctg ttttggcgga tgagagaaga 1560
   ttttcagcct gatacagatt aaatcagaac gcagaagcgg tctgataaaa cagaatttgc 1620
   ctggcggcag tagcgcggtg gtcccacctg accccatgcc gaactcagaa gtgaaacgcc 1680
   gtagcgccga tggtagtgtg gggtctcccc atgcgagagt agggaactgc caggcatcaa 1740
   ataaaacgaa aggctcagtc gaaagactgg gcctttcgtt ttatctgttg tttgtcggtg 1800
   aacgctctcc tgagtaggac aaatccgccg ggagcggatt tgaacgttgc gaagcaacgg 1860
   cccggagggt ggcgggcagg acgcccgcca taaactgcca ggcatcaaat taagcagaag 1920
   gccatcctga cggatggcct ttttgcgttt ctacaaactc tttttgttta tttttctaaa 1980
   tacattcaaa tatgtatccg ctcatgagac aataaccctg ataaatgctt caataatatt 2040
   gaaaaaggaa gagtatgagt attcaacatt tccgtgtcgc ccttattccc ttttttgcgg 2100
   cattttgcct tcctgttttt gctcacccag aaacgctggt gaaagtaaaa gatgctgaag 2160
   atcagttggg tgcacgagtg ggttacatcg aactggatct caacagcggt aagatccttg 2220
   agagttttcg ccccgaagaa cgttttccaa tgatgagcac ttttaaagtt ctgctatgtg 2280
   gcgcggtatt atcccgtgtt gacgccgggc aagagcaact cggtcgccgc atacactatt 2340
   ctcagaatga cttggttgag tactcaccag tcacagaaaa gcatcttacg gatggcatga 2400
   cagtaagaga attatgcagt gctgccataa ccatgagtga taacactgcg gccaacttac 2460
   ttctgacaac gatcggagga ccgaaggagc taaccgcttt tttgcacaac atgggggatc 2520
   atgtaactcg ccttgatcgt tgggaaccgg agctgaatga agccatacca aacgacgagc 2580
   gtgacaccac gatgcctgta gcaatggcaa caacgttgcg caaactatta actggcgaac 2640
   tacttactct agcttcccgg caacaattaa tagactggat ggaggcggat aaagttgcag 2700
   gaccacttct gcgctcggcc cttccggctg gctggtttat tgctgataaa tctggagccg 2760
   gtgagcgtgg gtctcgcggt atcattgcag cactggggcc agatggtaag ccctcccgta 2820
   tcgtagttat ctacacgacg gggagtcagg caactatgga tgaacgaaat agacagatcg 2880
   ctgagatagg tgcctcactg attaagcatt ggtaactgtc agaccaagtt tactcatata 2940
   tactttagat tgatttaaaa cttcattttt aatttaaaag gatctaggtg aagatccttt 3000
   ttgataatct catgaccaaa atcccttaac gtgagttttc gttccactga gcgtcagacc 3060
   ccgtagaaaa gatcaaagga tcttcttgag atcctttttt tctgcgcgta atctgctgct 3120
   tgcaaacaaa aaaaccaccg ctaccagcgg tggtttgttt gccggatcaa gagctaccaa 3180
   ctctttttcc gaaggtaact ggcttcagca gagcgcagat accaaatact gtccttctag 3240
   tgtagccgta gttaggccac cacttcaaga actctgtagc accgcctaca tacctcgctc 3300
   tgctaatcct gttaccagtg gctgctgcca gtggcgataa gtcgtgtctt accgggttgg 3360
   actcaagacg atagttaccg gataaggcgc agcggtcggg ctgaacgggg ggttcgtgca 3420
   cacagcccag cttggagcga acgacctaca ccgaactgag atacctacag cgtgagctat 3480
   gagaaagcgc cacgcttccc gaagggagaa aggcggacag gtatccggta agcggcaggg 3540
   tcggaacagg agagcgcacg agggagcttc cagggggaaa cgcctggtat ctttatagtc 3600
   ctgtcgggtt tcgccacctc tgacttgagc gtcgattttt gtgatgctcg tcaggggggc 3660
   ggagcctatg gaaaaacgcc agcaacgcgg cctttttacg gttcctggcc ttttgctggc 3720
   cttttgctca catgttcttt cctgcgttat cccctgattc tgtggataac cgtattaccg 3780
   cctttgagtg agctgatacc gctcgccgca gccgaacgac cgagcgcagc gagtcagtga 3840
   gcgaggaagc ggaagagcgc ctgatgcggt attttctcct tacgcatctg tgcggtattt 3900
   cacaccgcat atggtgcact ctcagtacaa tctgctctga tgccgcatag ttaagccagt 3960
   atacactccg ctatcgctac gtgactgggt catggctgcg ccccgacacc cgccaacacc 4020
   cgctgacgcg ccctgacggg cttgtctgct cccggcatcc gcttacagac aagctgtgac 4080
   cgtctccggg agctgcatgt gtcagaggtt ttcaccgtca tcaccgaaac gcgcgaggca 4140
   gcagatcaat tcgcgcgcga aggcgaagcg gcatgcattt acgttgacac catcgaatgg 4200
   tgcaaaacct ttcgcggtat ggcatgatag cgcccggaag agagtcaatt cagggtggtg 4260
   aatgtgaaac cagtaacgtt atacgatgtc gcagagtatg ccggtgtctc ttatcagacc 4320
   gtttcccgcg tggtgaacca ggccagccac gtttctgcga aaacgcggga aaaagtggaa 4380
   gcggcgatgg cggagctgaa ttacattccc aaccgcgtgg cacaacaact ggcgggcaaa 4440
   cagtcgttgc tgattggcgt tgccacctcc agtctggccc tgcacgcgcc gtcgcaaatt 4500
   gtcgcggcga ttaaatctcg cgccgatcaa ctgggtgcca gcgtggtggt gtcgatggta     4560
   gaacgaagcg gcgtcgaagc ctgtaaagcg gcggtgcaca atcttctcgc gcaacgcgtc     4620
   agtgggctga tcattaacta tccgctggat gaccaggatg ccattgctgt ggaagctgcc     4680
   tgcactaatg ttccggcgtt atttcttgat gtctctgacc agacacccat caacagtatt     4740
   attttctccc atgaagacgg tacgcgactg ggcgtggagc atctggtcgc attgggtcac     4800
   cagcaaatcg cgctgttagc gggcccatta agttctgtct cggcgcgtct gcgtctggct     4860
   ggctggcata aatatctcac tcgcaatcaa attcagccga tagcggaacg ggaaggcgac     4920
   tggagtgcca tgtccggttt tcaacaaacc atgcaaatgc tgaatgaggg catcgttccc     4980
   actgcgatgc tggttgccaa cgatcagatg gcgctgggcg caatgcgcgc cattaccgag     5040
   tccgggctgc gcgttggtgc ggatatctcg gtagtgggat acgacgatac cgaagacagc     5100
   tcatgttata tcccgccgtc aaccaccatc aaacaggatt ttcgcctgct ggggcaaacc     5160
   agcgtggacc gcttgctgca actctctcag ggccaggcgg tgaagggcaa tcagctgttg     5220
   cccgtctcac tggtgaaaag aaaaaccacc ctggcgccca atacgcaaac cgcctctccc     5280
   cgcgcgttgg ccgattcatt aatgcagctg gcacgacagg tttcccgact ggaaagcggg     5340
   cagtgagcgc aacgcaatta atgtgagtta gcgcgaattg atctg                    5385
<210> 2
   <211> 8145
   <212> DNA
   <213> Artificial Sequence
<220>
   <223> Description of Artificial Sequence: Synthetic pACYC-accABCD Pj251-cat-PtPiA-AccAD-PrpiA-AaccBC polynucleotide
<400> 2
   gcttttagaa aaactcatcg agcatcaaat gaaactgcaa tttattcata tcaggattat      60
   caataccata tttttgaaaa agccgtttct gtaatgaagg agaaaactca ccgaggcagt     120
   tccataggat ggcaagatcc tggtatcggt ctgcgattcc gactcgtcca acatcaatac     180
   aacctattat tgtaaaacga cggccagtga gcgcgcgtaa tacgactcac tatagggcga     240
   attggagctc ccgcggtgcg gccgctctag aactagtgga tcccccgggc tgcagcgacc     300
   aatgtggaat tcgcccttgg cacgtgcgaa gttcctattc tctaggaaag tataggaact     360
   tcgaggcttt tgacttgagg gggatccacg cgtttacgcc ccgccctgcc actcatcgca     420
   gtactgttgt aattcattaa gcattctgcc gacatggaag ccatcacaaa cggcatgatg     480
   aacctgaatc gccagcggca tcagcacctt gtcgccttgc gtataatatt tgcccatggt     540
   gaaaacgggg gcgaagaagt tgtccatatt ggccacgttt aaatcaaaac tggtgaaact     600
   cacccaggga ttggctgaga cgaaaaacat attctcaata aaccctttag ggaaataggc     660
   caggttttca ccgtaacacg ccacatcttg cgaatatatg tgtagaaact gccggaaatc 720
   gtcgtggtat tcactccaga gcgatgaaaa cgtttcagtt tgctcatgga aaacggtgta 780
   acaagggtga acactatccc atatcaccag ctcaccgtct ttcattgcca tacggaattc 840
   cggatgagca ttcatcaggc gggcaagaat gtgaataaag gccggataaa acttgtgctt 900
   atttttcttt acggtcttta aaaaggccgt aatatccagc tgaacggtct ggttataggt 960
   acattgagca actgactgaa atgcctcaaa atgttcttta cgatgccatt gggatatatc 1020
   aacggtggta tatccagtga tttttttctc cattttagct tccttagctc ctgaaaatct 1080
   cgataactca aaaaatacgc ccggtagtga tcttatttca ttatggtgaa agttggaacc 1140
   tcttacgaag ttcctatact ttctagagaa taggaacttc gcacgtgcca agggcgaatt 1200
   ccacagtgga tatcaagctt atcgataccg tcgacctcga gggggggccc ggtacccagc 1260
   ttttgttccc tttagtgagg gttaattgcg cgcttggcgt aatcatggtc atagctgttt 1320
   ccatcaggat attcttctaa tacctggaac gctgtttttc cggggatcgc agtggtgagt 1380
   aaccatgcat catcaggagt acggataaaa tgcttgatgg tcggaagtgg cataaattcc 1440
   gtcagccagt ttagtctgac catctcatct gtaacatcat tggcaacgct acctttgcca 1500
   tgtttcagaa acaactctgg cgcatcgggc ttcccataca agcgatagat tgtcgcacct 1560
   gattgcccga cattatcgcg agcccattta tacccatata aatcagcatc catgttggaa 1620
   tttaatcgcg gcctcgacgt ttcccgttga atatggctca tattcttcct ttttcaatat 1680
   tattgaagca tttatcaggg ttattgtctc atgagcggat acatatttga atgtatttag 1740
   aaaaataaac aaataggggt cagtgttaca accaattaac caattctgaa cattatcgcg 1800
   agcccattta tacctgaata tggctcataa caccccttgt ttgcctggcg gcagtagcgc 1860
   ggtggtccca cctgacccca tgccgaactc agaagtgaaa cgccgtagcg ccgatggtag 1920
   tgtggggact ccccatgcga gagtagggaa ctgccaggca tcaaataaaa cgaaaggctc 1980
   agtcgaaaga ctgggccttt cgcccgggct aattaggggg tgtcgccctt attcgactct 2040
   atagggaagt tcctattctc tagaaagtat aggaacttct gaagggggga cagctgtctc 2100
   aaatgtctcg gaatcgctga cgattcccag gtttctccgg caagcatagc gcatggcgtc 2160
   tccatgcgag aatgtcgcgc ttgccggata aaaggggagc cgctatcgga atggacgcaa 2220
   gccacggccg cagcaggtgc ggtcgagggc ttccagccag ttccagggca gatgtgccgg 2280
   cagaccctcc cgctttgggg gaggcgcaag ccgggtccat tcggatagca tctccccatg 2340
   caaagtgccg gccagggcaa tgcccggagc cggttcgagg cgccggtttg aataaatgac 2400
   aaaaagcaaa gcctttgtgc cgatgaatct ctatactgtt tcacagacct gctgccctgc 2460
   ggggcggcca tcttccttta ttcgcttata agcgcggggc aggaggaaaa acatgtccct 2520
   gaacttcctg gacttcgagc agccgattgc agaactggaa gcgaagattg acagcctgac 2580
   cgcggttagc cgtcaagatg agaaactgga cattaacatc gacgaagagg tccaccgttt 2640
   gcgtgagaag tctgttgaac tgactcgcaa aatctttgct gatttgggcg catggcagat 2700
   tgcccagttg gctcgccacc cacaacgccc atataccctg gactacgtgc gcctggcgtt 2760
   tgacgagttc gacgaactgg caggcgaccg cgcctatgcg gacgataaag caattgtcgg 2820
   cggtattgct cgtttggatg gccgtccggt gatgattatc ggccatcaaa aaggccgcga 2880
   gacgaaagaa aagattcgtc gtaactttgg tatgccggca ccggagggct accgcaaggc 2940
   cctgcgtctg atgcaaatgg ccgaacgctt taagatgccg attatcacgt tcattgatac 3000
   gccgggtgcg tacccaggcg ttggtgcgga agagcgtggt cagagcgagg ccatcgcacg 3060
   taacctgcgt gagatgtctc gtctgggtgt gccggtcgtt tgcaccgtga ttggcgaggg 3120
   cggtagcggt ggtgcgttgg cgatcggtgt cggtgataag gtcaacatgc tgcaatacag 3180
   cacgtacagc gtcattagcc cggaaggttg cgcttccatt ctgtggaaga gcgcggataa 3240
   agcaccattg gcagcggaag cgatgggtat catcgcaccg cgtctgaaag aactgaagtt 3300
   gattgattct atcatcccgg aaccgctggg cggtgctcac cgtaatccgg aggcgatggc 3360
   agccagcctg aaggcccagc tgctggcgga cctggcggat ctggacgtgc tgagcacgga 3420
   ggatctgaaa aaccgtcgct atcagcgctt gatgagctat ggctacgcgt agtaaaggag 3480
   gaaaatcatg agctggattg aacgcattaa gtccaatatc accccgaccc gcaaggcgag 3540
   catccctgaa ggcgtctgga ccaaatgcga tagctgcggt caggttttgt atcgtgcgga 3600
   gctggagcgt aacctggaag tgtgcccgaa atgcgaccat cacatgcgta tgaccgctcg 3660
   taatcgtctg catagcctgc tggatgaggg cagcctggtc gagctgggta gcgaactgga 3720
   accgaaagat gttctgaaat tccgtgattc caagaagtat aaggatcgtt tggcatctgc 3780
   acaaaaagaa accggtgaga aggacgcact ggttgttatg aaaggcaccc tgtatggtat 3840
   gccggttgtt gctgcggcgt tcgagtttgc gtttatgggt ggcagcatgg gttccgtggt 3900
   gggcgcacgc tttgtgcgtg ccgtggagca ggcgctggag gataactgtc ctctgatttg 3960
   tttcagcgcg agcggtggtg cgcgtatgca agaggccctg atgagcctga tgcagatggc 4020
   aaaaacctcg gcagccctgg cgaagatgca agaacgcggc ctgccgtaca tttccgtcct 4080
   gaccgaccct acgatgggcg gtgtcagcgc cagctttgcg atgctgggtg atttgaacat 4140
   cgcagagccg aaggctctga ttggttttgc tggtccgcgt gttattgaac agacggttcg 4200
   cgaaaagttg ccgcctggtt tccagcgcag cgagttcctg attgagaaag gtgccatcga 4260
   catgatcgtt cgccgtccag aaatgcgtct gaaactggcg agcattctgg cgaaattgat 4320
   gaatctgccg gctccgaatc ctgaagcacc gcgtgagggt gtcgtggttc cgccggtccc 4380
   ggaccaagag ccggaggcat aatagcacgt ggagttaacc acgcggcttg ccaacggggt 4440
   ctgaatcgct ttttttgtat ataatgcgtg tgaaatttca taccacaggc gaaggaggaa 4500
   aaaccatgga cattcgtaag atcaagaaac tgattgaact ggttgaagaa agcggcatca 4560
   gcgagctgga gatcagcgaa ggtgaagaga gcgtccgtat ttcccgtgcg gcaccggcag 4620
   cgagctttcc ggttatgcag caagcatacg ccgctccgat gatgcaacag ccggcacaga 4680
   gcaacgccgc tgcaccggcg accgttccaa gcatggaggc accggcagcg gccgagattt 4740
   cgggtcatat cgtgcgtagc ccgatggtgg gcaccttcta tcgcacgccg tcgccggacg 4800
   caaaagcctt catcgaagtc ggccagaagg tcaatgtcgg cgacacgctg tgtatcgttg 4860
   aggcaatgaa aatgatgaac cagattgaag cggataagag cggtactgtt aaagcgatcc 4920
   tggtggaatc cggccagcct gttgagttcg atgaaccgct ggttgtgatc gagtaacgag 4980
   gcgaacatgt tggacaagat cgtgattgca aaccgcggtg aaatcgcgct gcgtatcttg 5040
   cgcgcgtgta aagagctggg cattaagact gttgccgtgc attccagcgc agaccgcgac 5100
   ctgaagcatg ttctgctggc cgacgaaacg gtttgcatcg gtccggcacc gagcgtgaaa 5160
   agctatctga acatcccggc catcatctct gcggcagaga tcaccggtgc agtggcgatt 5220
   catccgggct acggtttcct gagcgagaac gctaactttg ctgaacaagt ggagcgtagc 5280
   ggtttcatct tcattggccc taaggcggag acgattcgcc tgatgggcga caaagtgagc 5340
   gccattgcag cgatgaaaaa ggccggtgtg ccgtgtgttc cgggcagcga tggtccgctg 5400
   ggtgacgata tggacaagaa ccgtgccatc gctaaacgta ttggctaccc ggtcattatc 5460
   aaagcctctg gtggtggcgg tggccgtggt atgcgtgtcg tccgtggtga tgcggaactg 5520
   gcgcaaagca tcagcatgac ccgtgcggaa gccaaagcgg cgttctctaa cgatatggtg 5580
   tatatggaga agtatctgga gaatccgcgc cacgttgaaa tccaagttct ggcggatggt 5640
   cagggcaatg cgatctactt ggcagaacgt gattgctcca tgcaacgccg tcatcagaag 5700
   gtggtggaag aggcaccggc tccgggtatt acgccggaac tgcgtcgcta catcggtgag 5760
   cgctgtgcga aagcgtgtgt ggacattggt taccgtggtg cgggtacgtt tgagttcctg 5820
   ttcgaaaatg gtgagtttta cttcattgaa atgaataccc gcatccaggt tgagcacccg 5880
   gtgaccgaga tgattactgg cgttgatctg atcaaagagc aactgcgcat tgcggctggt 5940
   cagccgctgt cgatcaagca agaagaggtg cacgttcgtg gtcacgcggt cgagtgccgt 6000
   atcaatgcgg aggacccgaa tacctttctg ccgagccctg gtaagatcac gcgttttcac 6060
   gcgccaggtg gttttggcgt tcgttgggag tctcacatct acgccggtta caccgtgccg 6120
   ccgtactatg acagcatgat tggtaaactg atctgctatg gcgaaaatcg tgatgtcgcg 6180
   atcgcccgca tgaaaaacgc gctgcaagag ctgatcattg atggcattaa gaccaatgtg 6240
   gatttgcaga tccgcattat gaacgacgag aatttccagc acggcggtac gaacattcac 6300
   tacctggaaa agaaactggg cctgcaagag aaataatagg gtaccctagc ataacccctt 6360
   ggggcctcta aacgggtctt gaggggtttt ttgaatcccc cgggatgtct atcttaaggt 6420
   agccaaagtt taaactttgg ctaccttaag atagacatac tgccggcctg gttcaaccag 6480
   tcggcagccg gcgctggcgc ccgcgtattg cggtgcagcc agcgcggcgc acaaggcggc 6540
   gggcgtttcg tttcgccgcc cgtttcgcgg gccgtcaagg cccgcgaatc gtttctgccc 6600
   gcgcggcatt cctcgctttt tgcgccaatt caccgggttt tccttaagcc ccgtcgcttt 6660
   tcttagtgcc ttgttgggca tagaatcagg gcagcggcgc agccagcacc atgttcgtgc 6720
   agcgcggccc tcgcgggggc gaggctgcag acagctgtca aaatgaaggg aagttcctat 6780
   actttctaga gaataggaac ttctataggg agtcgaataa gggcgacaca aaaggtattc 6840
   taaatgcata ataaatactg ataacatctt atagtttgta ttatattttg tattatcgtt 6900
   gacatgtata attttgatat caaaaactga ttttcccttt attattttcg agatttattt 6960
   tcttaattct ctttaacaaa ctagaaatat tgtatataca aaaaatcata aataatagat 7020
   gaatagttta attataggtg ttcatcaatc gaaaaagcaa cgtatcttat ttaaagtgcg 7080
   ttgctttttt ctcatttata aggttaaata attctcatat atcaagcaaa gtgacaggcg 7140
   cccttaaata ttctgacaaa tgctctttcc ctaaactccc cccataaaaa aacccgccga 7200
   agcgggtttt tacgttattt gcggattaac gattactcgt tatcagaacc gcccaggatg 7260
   cctggcagtt ccctactctc gccgctgcgc tcggtcgttc ggctgcggga cctcagcgct 7320
   agcggagtgt atactggctt actatgttgg cactgatgag ggtgtcagtg aagtgcttca 7380
   tgtggcagga gaaaaaaggc tgcaccggtg cgtcagcaga atatgtgata caggatatat 7440
   tccgcttcct cgctcactga ctcgctacgc tcggtcgttc gactgcggcg agcggaaatg 7500
   gcttacgaac ggggcggaga tttcctggaa gatgccagga agatacttaa cagggaagtg 7560
   agagggccgc ggcaaagccg tttttccata ggctccgccc ccctgacaag catcacgaaa 7620
   tctgacgctc aaatcagtgg tggcgaaacc cgacaggact ataaagatac caggcgtttc 7680
   cccctggcgg ctccctcgtg cgctctcctg ttcctgcctt tcggtttacc ggtgtcattc 7740
   cgctgttatg gccgcgtttg tctcattcca cgcctgacac tcagttccgg gtaggcagtt 7800
   cgctccaagc tggactgtat gcacgaaccc cccgttcagt ccgaccgctg cgccttatcc 7860
   ggtaactatc gtcttgagtc caacccggaa agacatgcaa aagcaccact ggcagcagcc 7920
   actggtaatt gatttagagg agttagtctt gaagtcatgc gccggttaag gctaaactga 7980
   aaggacaagt tttggtgact gcgctcctcc aagccagtta cctcggttca aagagttggt 8040
   agctcagaga accttcgaaa aaccgccctg caaggcggtt ttttcgtttt cagagcaaga 8100
   gattacgcgc agaccaaaac gatctcaaga agatcatctt attaa 8145
<210> 3
   <211> 8894
   <212> DNA
   <213> Artificial Sequence
<220>
   <223> Description of Artificial Sequence: Synthetic pTrc-ptrc-mcr-kan polynucleotide
<400> 3
   ccttcaactc agcaaaagtt cgatttattc aacaaagcca cgttgtgtct caaaatctct      60
   gatgttacat tgcacaagat aaaaatatat catcatgaac aataaaactg tctgcttaca     120
   taaacagtaa tacaaggggt gttatgagcc atattcaacg ggaaacgtct tgctcgaggc     180
   cgcgattaaa ttccaacatg gatgctgatt tatatgggta taaatgggct cgcgataatg     240
   tcgggcaatc aggtgcgaca atctatcgat tgtatgggaa gcccgatgcg ccagagttgt     300
   ttctgaaaca tggcaaaggt agcgttgcca atgatgttac agatgagatg gtcagactaa     360
   actggctgac ggaatttatg cctcttccga ccatcaagca ttttatccgt actcctgatg     420
   atgcatggtt actcaccact gcgatccccg ggaaaacagc attccaggta ttagaagaat     480
   atcctgattc aggtgaaaat attgttgatg cgctggcagt gttcctgcgc cggttgcatt     540
   cgattcctgt ttgtaattgt ccttttaaca gcgatcgcgt atttcgtctc gctcaggcgc     600
   aatcacgaat gaataacggt ttggttgatg cgagtgattt tgatgacgag cgtaatggct     660
   ggcctgttga acaagtctgg aaagaaatgc ataagctttt gccattctca ccggattcag     720
   tcgtcactca tggtgatttc tcacttgata accttatttt tgacgagggg aaattaatag     780
   gttgtattga tgttggacga gtcggaatcg cagaccgata ccaggatctt gccatcctat     840
   ggaactgcct cggtgagttt tctccttcat tacagaaacg gctttttcaa aaatatggta     900
   ttgataatcc tgatatgaat aaattgcagt ttcatttgat gctcgatgag tttttctaat     960
   cgagtcaggc aactatggat gaacgaaata gacagatcgc tgagataggt gcctcactga    1020
   ttaagcattg gtaactgtca gaccaagttt actcatatat actttagatt gatttaaaac    1080
   ttcattttta atttaaaagg atctaggtga agatcctttt tgataatctc atgaccaaaa    1140
   tcccttaacg tgagttttcg ttccactgag cgtcagaccc cgtagaaaag atcaaaggat    1200
   cttcttgaga tccttttttt ctgcgcgtaa tctgctgctt gcaaacaaaa aaaccaccgc    1260
   taccagcggt ggtttgtttg ccggatcaag agctaccaac tctttttccg aaggtaactg    1320
   gcttcagcag agcgcagata ccaaatactg tccttctagt gtagccgtag ttaggccacc    1380
   acttcaagaa ctctgtagca ccgcctacat acctcgctct gctaatcctg ttaccagtgg    1440
   ctgctgccag tggcgataag tcgtgtctta ccgggttgga ctcaagacga tagttaccgg    1500
   ataaggcgca gcggtcgggc tgaacggggg gttcgtgcac acagcccagc ttggagcgaa    1560
   cgacctacac cgaactgaga tacctacagc gtgagctatg agaaagcgcc acgcttcccg    1620
   aagggagaaa ggcggacagg tatccggtaa gcggcagggt cggaacagga gagcgcacga 1680
   gggagcttcc agggggaaac gcctggtatc tttatagtcc tgtcgggttt cgccacctct 1740
   gacttgagcg tcgatttttg tgatgctcgt caggggggcg gagcctatgg aaaaacgcca 1800
   gcaacgcggc ctttttacgg ttcctggcct tttgctggcc ttttgctcac atgttctttc 1860
   ctgcgttatc ccctgattct gtggataacc gtattaccgc ctttgagtga gctgataccg 1920
   ctcgccgcag ccgaacgacc gagcgcagcg agtcagtgag cgaggaagcg gaagagcgcc 1980
   tgatgcggta ttttctcctt acgcatctgt gcggtatttc acaccgcata tggtgcactc 2040
   tcagtacaat ctgctctgat gccgcatagt taagccagta tacactccgc tatcgctacg 2100
   tgactgggtc atggctgcgc cccgacaccc gccaacaccc gctgacgcgc cctgacgggc 2160
   ttgtctgctc ccggcatccg cttacagaca agctgtgacc gtctccggga gctgcatgtg 2220
   tcagaggttt tcaccgtcat caccgaaacg cgcgaggcag cagatcaatt cgcgcgcgaa 2280
   ggcgaagcgg catgcattta cgttgacacc atcgaatggt gcaaaacctt tcgcggtatg 2340
   gcatgatagc gcccggaaga gagtcaattc agggtggtga atgtgaaacc agtaacgtta 2400
   tacgatgtcg cagagtatgc cggtgtctct tatcagaccg tttcccgcgt ggtgaaccag 2460
   gccagccacg tttctgcgaa aacgcgggaa aaagtggaag cggcgatggc ggagctgaat 2520
   tacattccca accgcgtggc acaacaactg gcgggcaaac agtcgttgct gattggcgtt 2580
   gccacctcca gtctggccct gcacgcgccg tcgcaaattg tcgcggcgat taaatctcgc 2640
   gccgatcaac tgggtgccag cgtggtggtg tcgatggtag aacgaagcgg cgtcgaagcc 2700
   tgtaaagcgg cggtgcacaa tcttctcgcg caacgcgtca gtgggctgat cattaactat 2760
   ccgctggatg accaggatgc cattgctgtg gaagctgcct gcactaatgt tccggcgtta 2820
   tttcttgatg tctctgacca gacacccatc aacagtatta ttttctccca tgaagacggt 2880
   acgcgactgg gcgtggagca tctggtcgca ttgggtcacc agcaaatcgc gctgttagcg 2940
   ggcccattaa gttctgtctc ggcgcgtctg cgtctggctg gctggcataa atatctcact 3000
   cgcaatcaaa ttcagccgat agcggaacgg gaaggcgact ggagtgccat gtccggtttt 3060
   caacaaacca tgcaaatgct gaatgagggc atcgttccca ctgcgatgct ggttgccaac 3120
   gatcagatgg cgctgggcgc aatgcgcgcc attaccgagt ccgggctgcg cgttggtgcg 3180
   gatatctcgg tagtgggata cgacgatacc gaagacagct catgttatat cccgccgtca 3240
   accaccatca aacaggattt tcgcctgctg gggcaaacca gcgtggaccg cttgctgcaa 3300
   ctctctcagg gccaggcggt gaagggcaat cagctgttgc ccgtctcact ggtgaaaaga 3360
   aaaaccaccc tggcgcccaa tacgcaaacc gcctctcccc gcgcgttggc cgattcatta 3420
   atgcagctgg cacgacaggt ttcccgactg gaaagcgggc agtgagcgca acgcaattaa 3480
   tgtgagttag cgcgaattga tctggtttga cagcttatca tcgactgcac ggtgcaccaa 3540
   tgcttctggc gtcaggcagc catcggaagc tgtggtatgg ctgtgcaggt cgtaaatcac 3600
   tgcataattc gtgtcgctca aggcgcactc ccgttctgga taatgttttt tgcgccgaca 3660
   tcataacggt tctggcaaat attctgaaat gagctgttga caattaatca tccggctcgt 3720
   ataatgtgtg gaattgtgag cggataacaa tttcacacag gaaacagcgc cgctgagaaa 3780
   aagcgaagcg gcactgctct ttaacaattt atcagacaat ctgtgtgggc actcgaccgg 3840
   aattatcgat taactttatt attaaaaatt aaagaggtat atattaatgt atcgattaaa 3900
   taaggaggaa taaaccatgg ccggtacggg tcgtttggct ggtaaaattg cattgatcac 3960
   cggtggtgct ggtaacattg gttccgagct gacccgccgt tttctggccg agggtgcgac 4020
   ggttattatc agcggccgta accgtgcgaa gctgaccgcg ctggccgagc gcatgcaagc 4080
   cgaggccggc gtgccggcca agcgcattga tttggaggtg atggatggtt ccgaccctgt 4140
   ggctgtccgt gccggtatcg aggcaatcgt cgctcgccac ggtcagattg acattctggt 4200
   taacaacgcg ggctccgccg gtgcccaacg tcgcttggcg gaaattccgc tgacggaggc 4260
   agaattgggt ccgggtgcgg aggagacttt gcacgcttcg atcgcgaatc tgttgggcat 4320
   gggttggcac ctgatgcgta ttgcggctcc gcacatgcca gttggctccg cagttatcaa 4380
   cgtttcgact attttctcgc gcgcagagta ctatggtcgc attccgtacg ttaccccgaa 4440
   ggcagcgctg aacgctttgt cccagctggc tgcccgcgag ctgggcgctc gtggcatccg 4500
   cgttaacact attttcccag gtcctattga gtccgaccgc atccgtaccg tgtttcaacg 4560
   tatggatcaa ctgaagggtc gcccggaggg cgacaccgcc catcactttt tgaacaccat 4620
   gcgcctgtgc cgcgcaaacg accaaggcgc tttggaacgc cgctttccgt ccgttggcga 4680
   tgttgctgat gcggctgtgt ttctggcttc tgctgagagc gcggcactgt cgggtgagac 4740
   gattgaggtc acccacggta tggaactgcc ggcgtgtagc gaaacctcct tgttggcgcg 4800
   taccgatctg cgtaccatcg acgcgagcgg tcgcactacc ctgatttgcg ctggcgatca 4860
   aattgaagaa gttatggccc tgacgggcat gctgcgtacg tgcggtagcg aagtgattat 4920
   cggcttccgt tctgcggctg ccctggcgca atttgagcag gcagtgaatg aatctcgccg 4980
   tctggcaggt gcggatttca ccccgccgat cgctttgccg ttggacccac gtgacccggc 5040
   caccattgat gcggttttcg attggggcgc aggcgagaat acgggtggca tccatgcggc 5100
   ggtcattctg ccggcaacct cccacgaacc ggctccgtgc gtgattgaag tcgatgacga 5160
   acgcgtcctg aatttcctgg ccgatgaaat taccggcacc atcgttattg cgagccgttt 5220
   ggcgcgctat tggcaatccc aacgcctgac cccgggtgcc cgtgcccgcg gtccgcgtgt 5280
   tatctttctg agcaacggtg ccgatcaaaa tggtaatgtt tacggtcgta ttcaatctgc 5340
   ggcgatcggt caattgattc gcgtttggcg tcacgaggcg gagttggact atcaacgtgc 5400
   atccgccgca ggcgatcacg ttctgccgcc ggtttgggcg aaccagattg tccgtttcgc 5460
   taaccgctcc ctggaaggtc tggagttcgc gtgcgcgtgg accgcacagc tgctgcacag 5520
   ccaacgtcat attaacgaaa ttacgctgaa cattccagcc aatattagcg cgaccacggg 5580
   cgcacgttcc gccagcgtcg gctgggccga gtccttgatt ggtctgcacc tgggcaaggt 5640
   ggctctgatt accggtggtt cggcgggcat cggtggtcaa atcggtcgtc tgctggcctt 5700
   gtctggcgcg cgtgtgatgc tggccgctcg cgatcgccat aaattggaac agatgcaagc 5760
   catgattcaa agcgaattgg cggaggttgg ttataccgat gtggaggacc gtgtgcacat 5820
   cgctccgggt tgcgatgtga gcagcgaggc gcagctggca gatctggtgg aacgtacgct 5880
   gtccgcattc ggtaccgtgg attatttgat taataacgcc ggtattgcgg gcgtggagga 5940
   gatggtgatc gacatgccgg tggaaggctg gcgtcacacc ctgtttgcca acctgatttc 6000
   gaattattcg ctgatgcgca agttggcgcc gctgatgaag aagcaaggta gcggttacat 6060
   cctgaacgtt tcttcctatt ttggcggtga gaaggacgcg gcgattcctt atccgaaccg 6120
   cgccgactac gccgtctcca aggctggcca acgcgcgatg gcggaagtgt tcgctcgttt 6180
   cctgggtcca gagattcaga tcaatgctat tgccccaggt ccggttgaag gcgaccgcct 6240
   gcgtggtacc ggtgagcgtc cgggcctgtt tgctcgtcgc gcccgtctga tcttggagaa 6300
   taaacgcctg aacgaattgc acgcggcttt gattgctgcg gcccgcaccg atgagcgctc 6360
   gatgcacgag ttggttgaat tgttgctgcc gaacgacgtg gccgcgttgg agcagaaccc 6420
   agcggcccct accgcgctgc gtgagctggc acgccgcttc cgtagcgaag gtgatccggc 6480
   ggcaagctcc tcgtccgcct tgctgaatcg ctccatcgct gccaagctgt tggctcgctt 6540
   gcataacggt ggctatgtgc tgccggcgga tatttttgca aatctgccta atccgccgga 6600
   cccgttcttt acccgtgcgc aaattgaccg cgaagctcgc aaggtgcgtg atggtattat 6660
   gggtatgctg tatctgcagc gtatgccaac cgagtttgac gtcgctatgg caaccgtgta 6720
   ctatctggcc gatcgtaacg tgagcggcga aactttccat ccgtctggtg gtttgcgcta 6780
   cgagcgtacc ccgaccggtg gcgagctgtt cggcctgcca tcgccggaac gtctggcgga 6840
   gctggttggt agcacggtgt acctgatcgg tgaacacctg accgagcacc tgaacctgct 6900
   ggctcgtgcc tatttggagc gctacggtgc ccgtcaagtg gtgatgattg ttgagacgga 6960
   aaccggtgcg gaaaccatgc gtcgtctgtt gcatgatcac gtcgaggcag gtcgcctgat 7020
   gactattgtg gcaggtgatc agattgaggc agcgattgac caagcgatca cgcgctatgg 7080
   ccgtccgggt ccggtggtgt gcactccatt ccgtccactg ccaaccgttc cgctggtcgg 7140
   tcgtaaagac tccgattgga gcaccgtttt gagcgaggcg gaatttgcgg aactgtgtga 7200
   gcatcagctg acccaccatt tccgtgttgc tcgtaagatc gccttgtcgg atggcgcgtc 7260
   gctggcgttg gttaccccgg aaacgactgc gactagcacc acggagcaat ttgctctggc 7320
   gaacttcatc aagaccaccc tgcacgcgtt caccgcgacc atcggtgttg agtcggagcg 7380
   caccgcgcaa cgtattctga ttaaccaggt tgatctgacg cgccgcgccc gtgcggaaga 7440
   gccgcgtgac ccgcacgagc gtcagcagga attggaacgc ttcattgaag ccgttctgct 7500
   ggttaccgct ccgctgcctc ctgaggcaga cacgcgctac gcaggccgta ttcaccgcgg 7560
   tcgtgcgatt accgtctaat agaagcttgg ctgttttggc ggatgagaga agattttcag 7620
   cctgatacag attaaatcag aacgcagaag cggtctgata aaacagaatt tgcctggcgg 7680
   cagtagcgcg gtggtcccac ctgaccccat gccgaactca gaagtgaaac gccgtagcgc 7740
   cgatggtagt gtggggtctc cccatgcgag agtagggaac tgccaggcat caaataaaac 7800
   gaaaggctca gtcgaaagac tgggcctttc gttttatctg ttgtttgtcg gtgaacgctc 7860
   tcctgagtag gacaaatccg ccgggagcgg atttgaacgt tgcgaagcaa cggcccggag 7920
   ggtggcgggc aggacgcccg ccataaactg ccaggcatca aattaagcag aaggccatcc 7980
   tgacggatgg cctttttgcg tttctacaaa ctctttttgt ttatttttct aaatacattc 8040
   aaatatgtat ccgctcatga gacaataacc ctgataaatg cttcaataat attgaaaaag 8100
   gaagagtatg agtattcaac atttccgtgt cgcccttatt cccttttttg cggcattttg 8160
   ccttcctgtt tttgctcacc cagaaacgct ggtgaaagta aaagatgctg aagatcagtt 8220
   gggtgcacga gtgggttaca tcgaactgga tctcaacagc ggtaagatcc ttgagagttt 8280
   tcgccccgaa gaacgttttc caatgatgag cacttttaaa gttctgctat gtggcgcggt 8340
   attatcccgt gttgacgccg ggcaagagca actcggtcgc cgcatacact attctcagaa 8400
   tgacttggtt gagtactcac cagtcacaga aaagcatctt acggatggca tgacagtaag 8460
   agaattatgc agtgctgcca taaccatgag tgataacact gcggccaact tacttctgac 8520
   aacgatcgga ggaccgaagg agctaaccgc ttttttgcac aacatggggg atcatgtaac 8580
   tcgccttgat cgttgggaac cggagctgaa tgaagccata ccaaacgacg agcgtgacac 8640
   cacgatgcct gtagcaatgg caacaacgtt gcgcaaacta ttaactggcg aactacttac 8700
   tctagcttcc cggcaacaat taatagactg gatggaggcg gataaagttg caggaccact 8760
   tctgcgctcg gcccttccgg ctggctggtt tattgctgat aaatctggag ccggtgagcg 8820
   tgggtctcgc ggtatcattg cagcactggg gccagatggt aagccctccc gtatcgtagt 8880
   tatctacacg acgg 8894
<210> 4
   <211> 10578
   <212> DNA
   <213> Artificial Sequence
<220>
   <223> Description of Artificial Sequence: Synthetic pACYC184-cat-PtpiA-accAD-PrpiA-accBC-patalA-pntAB polynucleotide
<400> 4
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatgggtt 2040
   acagagcttt caggattgca tccacgctgg ctttggcgtc accaaacagc atgtgggtgt 2100
   tttccttgaa gaacagcggg ttttgcacac cagcatagcc agtgttcatc gaacgtttaa 2160
   agacaatcac gttctgcgct ttccacactt ccagcacagg cataccagca atcggactct 2220
   tcggatcatc ctgcgccgcc gggttaaccg tatcgttagc accaatcacc agtacggtat 2280
   cggtatcagc aaagtcatca ttgatctcgt ccatttccag cacgatgtca tacggtactt 2340
   ttgcttcagc cagcaatacg ttcatatgtc caggcaaacg ccccgcgacc gggtggatac 2400
   cgaaacgcac attaatacca cgagcgcgca atttctcagt aatttcagcg acaggatatt 2460
   gcgcctgcgc gactgccatg ccgtaccccg gagtaatgat cactgaatgg gagtttttca 2520
   gcagttccgc tgtctcttct gcggtgattt cgcggtgctc acccacttcc tgatcatcgc 2580
   cagtagaaga gccgtcggtg ccgaaaccac ccgcaataac gctgataaag gaacggttca 2640
   tcgcctcaca cataatgtaa gaaaggatag cccccgaaga accgaccagc gcaccggtca 2700
   caatcagcag gtcgttgctg agcataaagc ccgcagccgc agccgcccag ccggagtacg 2760
   agttcagcat cgacaccacc actggcatat ctgcaccacc gatggaggcg actaaatgcc 2820
   agccgaatac cagcgcaatt gcggtcatta tcagcaatgc cagcacttgc aggccgacgc 2880
   tgtccgtgcg aacaaataca atcagcagca ggaaggaaac gaccagagcc gccaggttca 2940
   ttttgtgacg gtttggcagc atcaatggtt tagacgaaat cttgccacac agtttgccga 3000
   acgccaccac cgaacccgtg aacgttaccg ccccgatgaa gataccgagg aacacttccg 3060
   tcaggtgaat attgaccaga atcggtgcca ttcccgcgtc atgatgcaga tagctgttaa 3120
   agccaaccag cactgccgcc agacccacga agctatgcag gatcgccacc agttctggca 3180
   tttcggtcat ttcaactttc ttcgccagac ggataccaat tgccccacca atgaccatcg 3240
   ccagcaagat ccagccaaca ttacccgtat ccggtccaaa aatggttgcg attaacgcaa 3300
   tcgccatccc ggcgataccg aagttgttac cctggcgaga cgtttcatgt ttcgaaagac 3360
   cggccagact gaagataaac aggatcgcgg caacaatgta tgcagctgta actaatcctc 3420
   cagacatatg ttacccctta atttttgcgg aacattttca gcatgcgctg agtcacggtg 3480
   aagccaccga aaatattaat gctggctata agcaccgcga taaaactaag gaagctaacc 3540
   cagccgccct ggccaatctg caacagtgct ccgacaacaa taatccctga aatcgcgttg 3600
   gtgaccgaca tcaacggtgt atgcagcgcg tgcgatacat tccacaccac gtaataaccg 3660
   acaacgcagg ccagcgcgaa aacggtgaag tgcccaagga attctttcgg cgcaacgctt 3720
   gccatccagc caaaaagaat gattgccagc gccatcaacg cgtatttacg ccacggtgag 3780
   caggtacatt tttcctcagt tttcacttcc ggtgccgctt tttgtgccgc ctgcggctga 3840
   gctgatacct gaatcggcgg tgccggccag gtaatttcgc ccgcacggat cacggtcacg 3900
   ccgcgaatca ccacatcatc aaaatcaaca gtgatattgc cgtctttctc tttgcacaac 3960
   agtttcagca gattaacgag gtttgtgccg taaagctgtg aggattgcgt cggcagacgg 4020
   cccggaagat cggtataacc aatcactttg acaccatttt ccgtagtgaa gatttcaccc 4080
   ggcacggtgt attcacagtt gccgccgttt tgggctgcca ggtcgacaat cacactgccc 4140
   gccttcatgg agtcaaccat ttcacgggta attagcttcg gcgctggttt gcctggaata 4200
   agcgcggtgg tgacaatgat atcgacctct tttgcctggg cggcaaagag ttccatttcc 4260
   gctttgatga acgcgtccga catcactttg gcatagccat cgccgctgcc agcttcctct 4320
   ttaaaatcca gctcgaggaa ttccgcgccc atactttgaa cttgttcttt cacttccggg 4380
   cgggtgtcga atgcacgcac aatcgcgccg agactgtttg ctgcgccaat ggcggccaga 4440
   cctgcaacac ccgcaccaat caccatcact tttgccggtg gcactttccc ggccgcagta 4500
   atttgcccgg taaagaagcg cccaaattca tgtgccgctt caacaatggc gcgataaccg 4560
   gcgatgttcg ccatcgagct tagtgcgtcc agcgattgtg cgcgtgagat acgcggcaca 4620
   gagtccatcg ccatcacggt cacgttacgt tccgcaagtt tttgcattaa ttccggattc 4680
   tgcgcaggcc agataaaact caccagcgtt gtcccaggat tcagtaacgc aatttcatca 4740
   tctaacggcg cattgacctt cagaatgatc tctgactgcc agacgctatt cccttctaca 4800
   atttcagcgc ccgcttgcac aaacgcttta tcgtcaaaac ttgccagttg acccgcgccg 4860
   ctctctaccg cgacggtaaa acccagtttc agcagctgtt ccactgtttt tggcgttgct 4920
   gcaacacggg tttcattggt taaccgttct cttggtatgc caattgccat ggtaaatact 4980
   ccttgaaaag taaagtgtta gatgagtgcg ttaattcaca cttctgagaa atttcgctaa 5040
   acgcatcaaa aaagcatagc agacaggcat ggtattgctg gattaagcag gtaacatcag 5100
   tgttatagga ttattaccaa aacattatat tcacaggtgt atcgctcaat actgaccatt 5160
   taaatcatac ctgacctcca tagcagaaag tcaaaagcct ccgaccggag gcttttgact 5220
   tgagggggat cagcttctat tatttctctt gcaggcccag tttcttttcc aggtagtgaa 5280
   tgttcgtacc gccgtgctgg aaattctcgt cgttcataat gcggatctgc aaatccacat 5340
   tggtcttaat gccatcaatg atcagctctt gcagcgcgtt tttcatgcgg gcgatcgcga 5400
   catcacgatt ttcgccatag cagatcagtt taccaatcat gctgtcatag tacggcggca 5460
   cggtgtaacc ggcgtagatg tgagactccc aacgaacgcc aaaaccacct ggcgcgtgaa 5520
   aacgcgtgat cttaccaggg ctcggcagaa aggtattcgg gtcctccgca ttgatacggc 5580
   actcgaccgc gtgaccacga acgtgcacct cttcttgctt gatcgacagc ggctgaccag 5640
   ccgcaatgcg cagttgctct ttgatcagat caacgccagt aatcatctcg gtcaccgggt 5700
   gctcaacctg gatgcgggta ttcatttcaa tgaagtaaaa ctcaccattt tcgaacagga 5760
   actcaaacgt acccgcacca cggtaaccaa tgtccacaca cgctttcgca cagcgctcac 5820
   cgatgtagcg acgcagttcc ggcgtaatac ccggagccgg tgcctcttcc accaccttct 5880
   gatgacggcg ttgcatggag caatcacgtt ctgccaagta gatcgcattg ccctgaccat 5940
   ccgccagaac ttggatttca acgtggcgcg gattctccag atacttctcc atatacacca 6000
   tatcgttaga gaacgccgct ttggcttccg cacgggtcat gctgatgctt tgcgccagtt 6060
   ccgcatcacc acggacgaca cgcataccac ggccaccgcc accaccagag gctttgataa 6120
   tgaccgggta gccaatacgt ttagcgatgg cacggttctt gtccatatcg tcacccagcg 6180
   gaccatcgct gcccggaaca cacggcacac cggccttttt catcgctgca atggcgctca 6240
   ctttgtcgcc catcaggcga atcgtctccg ccttagggcc aatgaagatg aaaccgctac 6300
   gctccacttg ttcagcaaag ttagcgttct cgctcaggaa accgtagccc ggatgaatcg 6360
   ccactgcacc ggtgatctct gccgcagaga tgatggccgg gatgttcaga tagcttttca 6420
   cgctcggtgc cggaccgatg caaaccgttt cgtcggccag cagaacatgc ttcaggtcgc 6480
   ggtctgcgct ggaatgcacg gcaacagtct taatgcccag ctctttacac gcgcgcaaga 6540
   tacgcagcgc gatttcaccg cggtttgcaa tcacgatctt gtccaacatg ttcgcctcgt 6600
   tactcgatca caaccagcgg ttcatcgaac tcaacaggct ggccggattc caccaggatc 6660
   gctttaacag taccgctctt atccgcttca atctggttca tcattttcat tgcctcaacg 6720
   atacacagcg tgtcgccgac attgaccttc tggccgactt cgatgaaggc ttttgcgtcc 6780
   ggcgacggcg tgcgatagaa ggtgcccacc atcgggctac gcacgatatg acccgaaatc 6840
   tcggccgctg ccggtgcctc catgcttgga acggtcgccg gtgcagcggc gttgctctgt 6900
   gccggctgtt gcatcatcgg agcggcgtat gcttgctgca taaccggaaa gctcgctgcc 6960
   ggtgccgcac gggaaatacg gacgctctct tcaccttcgc tgatctccag ctcgctgatg 7020
   ccgctttctt caaccagttc aatcagtttc ttgatcttac gaatgtccat ggtttttcct 7080
   ccttcgcctg tggtatgaaa tttcacacgc attatataca aaaaaagcga ttcagacccc 7140
   gttggcaagc cgcgtggtta actccacgtg ctattatgcc tccggctctt ggtccgggac 7200
   cggcggaacc acgacaccct cacgcggtgc ttcaggattc ggagccggca gattcatcaa 7260
   tttcgccaga atgctcgcca gtttcagacg catttctgga cggcgaacga tcatgtcgat 7320
   ggcacctttc tcaatcagga actcgctgcg ctggaaacca ggcggcaact tttcgcgaac 7380
   cgtctgttca ataacacgcg gaccagcaaa accaatcaga gccttcggct ctgcgatgtt 7440
   caaatcaccc agcatcgcaa agctggcgct gacaccgccc atcgtagggt cggtcaggac 7500
   ggaaatgtac ggcaggccgc gttcttgcat cttcgccagg gctgccgagg tttttgccat 7560
   ctgcatcagg ctcatcaggg cctcttgcat acgcgcacca ccgctcgcgc tgaaacaaat 7620
   cagaggacag ttatcctcca gcgcctgctc cacggcacgc acaaagcgtg cgcccaccac 7680
   ggaacccatg ctgccaccca taaacgcaaa ctcgaacgcc gcagcaacaa ccggcatacc 7740
   atacagggtg cctttcataa caaccagtgc gtccttctca ccggtttctt tttgtgcaga 7800
   tgccaaacga tccttatact tcttggaatc acggaatttc agaacatctt tcggttccag 7860
   ttcgctaccc agctcgacca ggctgccctc atccagcagg ctatgcagac gattacgagc 7920
   ggtcatacgc atgtgatggt cgcatttcgg gcacacttcc aggttacgct ccagctccgc 7980
   acgatacaaa acctgaccgc agctatcgca tttggtccag acgccttcag ggatgctcgc 8040
   cttgcgggtc ggggtgatat tggacttaat gcgttcaatc cagctcatga ttttcctcct 8100
   ttactacgcg tagccatagc tcatcaagcg ctgatagcga cggtttttca gatcctccgt 8160
   gctcagcacg tccagatccg ccaggtccgc cagcagctgg gccttcaggc tggctgccat 8220
   cgcctccgga ttacggtgag caccgcccag cggttccggg atgatagaat caatcaactt 8280
   cagttctttc agacgcggtg cgatgatacc catcgcttcc gctgccaatg gtgctttatc 8340
   cgcgctcttc cacagaatgg aagcgcaacc ttccgggcta atgacgctgt acgtgctgta 8400
   ttgcagcatg ttgaccttat caccgacacc gatcgccaac gcaccaccgc taccgccctc 8460
   gccaatcacg gtgcaaacga ccggcacacc cagacgagac atctcacgca ggttacgtgc 8520
   gatggcctcg ctctgaccac gctcttccgc accaacgcct gggtacgcac ccggcgtatc 8580
   aatgaacgtg ataatcggca tcttaaagcg ttcggccatt tgcatcagac gcagggcctt 8640
   gcggtagccc tccggtgccg gcataccaaa gttacgacga atcttttctt tcgtctcgcg 8700
   gcctttttga tggccgataa tcatcaccgg acggccatcc aaacgagcaa taccgccgac 8760
   aattgcttta tcgtccgcat aggcgcggtc gcctgccagt tcgtcgaact cgtcaaacgc 8820
   caggcgcacg tagtccaggg tatatgggcg ttgtgggtgg cgagccaact gggcaatctg 8880
   ccatgcgccc aaatcagcaa agattttgcg agtcagttca acagacttct cacgcaaacg 8940
   gtggacctct tcgtcgatgt taatgtccag tttctcatct tgacggctaa ccgcggtcag 9000
   gctgtcaatc ttcgcttcca gttctgcaat cggctgctcg aagtccagga agttcaggga 9060
   catgtttttc ctcctgcccc gcgcttataa gcgaataaag gaagatggcc gccccgcagg 9120
   gcagcaggtc tgtgaaacag tatagagatt catcggcaca aaggctttgc tttttgtcat 9180
   ttattcaaac cggcgcctcg aaccggctcc gggcattgcc ctggccggca ctttgcatgg 9240
   ggagatgcgg atctcgggca gcgttgggtc ctggccacgg gtgcgcatga tcgtgctcct 9300
   gtcgttgagg acccggctag gctggcgggg ttgccttact ggttagcaga atgaatcacc 9360
   gatacgcgag cgaacgtgaa gcgactgctg ctgcaaaacg tctgcgacct gagcaacaac 9420
   atgaatggtc ttcggtttcc gtgtttcgta aagtctggaa acgcggaagt cccctacgtg 9480
   ctgctgaagt tgcccgcaac agagagtgga accaaccggt gataccacga tactatgact 9540
   gagagtcaac gccatgagcg gcctcatttc ttattctgag ttacaacagt ccgcaccgct 9600
   gtccggtagc tccttccggt gggcgcgggg catgactatc gtcgccgcac ttatgactgt 9660
   cttctttatc atgcaactcg taggacaggt gccggcagcg cccaacagtc ccccggccac 9720
   ggggcctgcc accataccca cgccgaaaca agcgccctgc accattatgt tccggatctg 9780
   catcgcagga tgctgctggc taccctgtgg aacacctaca tctgtattaa cgaagcgcta 9840
   accgttttta tcaggctctg ggaggcagaa taaatgatca tatcgtcaat tattacctcc 9900
   acggggagag cctgagcaaa ctggcctcag gcatttgaga agcacacggt cacactgctt 9960
   ccggtagtca ataaaccggt aaaccagcaa tagacataag cggctattta acgaccctgc 10020
   cctgaaccga cgaccgggtc gaatttgctt tcgaatttct gccattcatc cgcttattat 10080
   cacttattca ggcgtagcac caggcgttta agggcaccaa taactgcctt aaaaaaatta 10140
   cgccccgccc tgccactcat cgcagtactg ttgtaattca ttaagcattc tgccgacatg 10200
   gaagccatca cagacggcat gatgaacctg aatcgccagc ggcatcagca ccttgtcgcc 10260
   ttgcgtataa tatttgccca tggtgaaaac gggggcgaag aagttgtcca tattggccac 10320
   gtttaaatca aaactggtga aactcaccca gggattggct gagacgaaaa acatattctc 10380
   aataaaccct ttagggaaat aggccaggtt ttcaccgtaa cacgccacat cttgcgaata 10440
   tatgtgtaga aactgccgga aatcgtcgtg gtattcactc cagagcgatg aaaacgtttc 10500
   agtttgctca tggaaaacgg tgtaacaagg gtgaacacta tcccatatca ccagctcacc 10560
   gtctttcatt gccatacg 10578
<210> 5
   <211> 9047
   <212> DNA
   <213> Artificial Sequence
<220>
   <223> Description of Artificial Sequence: Synthetic pACYC-CAT-accADBC-udhA polynucleotide
<400> 5
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatttaaa 2040
   acaggcggtt taaaccgttt aacgcagcta cccgataggc ttccgccatc gtcgggtagt 2100
   taaaggtggt gttgacgaag tactcaatag tgttgccgcc acctttctgt tccataatcg 2160
   cctgaccgat atgaataatt tcggcagcgc gctcgccaaa gcagtgaata cccagaatct 2220
   cttttgtttc ccgatggaac aaaattttca gcgtgcccac gttcatgccg acgatttgtg 2280
   cgcgtgccag atgtttaaac tgggcgcggc ccacttcata tggcactttc attgcggtca 2340
   gctgctgttc ggttttgccc acagagctga tttccgggat ggtgtaaata ccggtaggga 2400
   tatcttcaat cagatgtgcg gtggcttcgc cttttaccag cgcctgcgcg gcaatgcgcc 2460
   cctggtcata ggccgccgac gccaggctcg gataaccaat cacgtcgccc accgcgtaaa 2520
   cgtgtggctg tgcggtctga tacatgctgt tgaccttcag ctgtccgcgg ctgtcagttt 2580
   ctagcccaat gttctgtaac gccagcgaat cggtattacc ggtgcgaccg ttggcataga 2640
   gcaggcagtc agctttcagt tttttacccg acttcagatg catgatcaca ccatcgtcac 2700
   agccttcgat cttctcgtac tcttcgttgt gacgaatcac tacgccactg ttccagaagt 2760
   gataggagag agaatctgac atctcttgat cgagaaatgc cagcaggcga tcgcgggtgt 2820
   tgatcagatc cacttttaca tccataccgc ggaagatcga cgcatattca cagccgatca 2880
   ctccagcacc atagataagt acatggcgcg gttcgtggtg catgctgaga attgagtcgc 2940
   tgtcgtaaat gcgtggatgg gtgaaatcaa catctgttgg atgatatgga cgagagccgc 3000
   aggcaataac aaatttttca gcggttagtg tttcaacgct gccgtccggg caatccagcg 3060
   ccaacgtatg ctcgtcaaca aagcgagcgt ttccctgcaa tatttcacag tgattacgtt 3120
   cgtaaaatcc ctgacgcatg cgcgtttgtt gattaatcac gttatcggca tggttaagga 3180
   tatcggcaaa agaagagcgg agcagtcggg aatggtcgct gtaaagtggg ttttgattga 3240
   attctataat gcggctgacg gcgtgacgga gagctttcga cgggatggtg ccccagtggg 3300
   tgcaaccgcc gccaacattt tgataacgct cgataactgc gacgcgcgca ccttgcttaa 3360
   ccaggcccat tgcagcgcct tcgccgccgg ggccggaacc tattactatg gcatcgtaat 3420
   cgtaggaatg tggggccatg gtaaatactc cttgaaaagt aaagtgttag atgagtgcgt 3480
   taattcacac ttctgagaaa tttcgctaaa cgcatcaaaa aagcatagca gacaggcatg 3540
   gtattgctgg attaagcagg taacatcagt gttataggat tattaccaaa acattatatt 3600
   cacaggtgta tcgctcaata ctgaccattt aaatcatacc tgacctccat agcagaaagt 3660
   caaaagcctc cgaccggagg cttttgactt gagggggatc agcttctatt atttctcttg 3720
   caggcccagt ttcttttcca ggtagtgaat gttcgtaccg ccgtgctgga aattctcgtc 3780
   gttcataatg cggatctgca aatccacatt ggtcttaatg ccatcaatga tcagctcttg 3840
   cagcgcgttt ttcatgcggg cgatcgcgac atcacgattt tcgccatagc agatcagttt 3900
   accaatcatg ctgtcatagt acggcggcac ggtgtaaccg gcgtagatgt gagactccca 3960
   acgaacgcca aaaccacctg gcgcgtgaaa acgcgtgatc ttaccagggc tcggcagaaa 4020
   ggtattcggg tcctccgcat tgatacggca ctcgaccgcg tgaccacgaa cgtgcacctc 4080
   ttcttgcttg atcgacagcg gctgaccagc cgcaatgcgc agttgctctt tgatcagatc 4140
   aacgccagta atcatctcgg tcaccgggtg ctcaacctgg atgcgggtat tcatttcaat 4200
   gaagtaaaac tcaccatttt cgaacaggaa ctcaaacgta cccgcaccac ggtaaccaat 4260
   gtccacacac gctttcgcac agcgctcacc gatgtagcga cgcagttccg gcgtaatacc 4320
   cggagccggt gcctcttcca ccaccttctg atgacggcgt tgcatggagc aatcacgttc 4380
   tgccaagtag atcgcattgc cctgaccatc cgccagaact tggatttcaa cgtggcgcgg 4440
   attctccaga tacttctcca tatacaccat atcgttagag aacgccgctt tggcttccgc 4500
   acgggtcatg ctgatgcttt gcgccagttc cgcatcacca cggacgacac gcataccacg 4560
   gccaccgcca ccaccagagg ctttgataat gaccgggtag ccaatacgtt tagcgatggc 4620
   acggttcttg tccatatcgt cacccagcgg accatcgctg cccggaacac acggcacacc 4680
   ggcctttttc atcgctgcaa tggcgctcac tttgtcgccc atcaggcgaa tcgtctccgc 4740
   cttagggcca atgaagatga aaccgctacg ctccacttgt tcagcaaagt tagcgttctc 4800
   gctcaggaaa ccgtagcccg gatgaatcgc cactgcaccg gtgatctctg ccgcagagat 4860
   gatggccggg atgttcagat agcttttcac gctcggtgcc ggaccgatgc aaaccgtttc 4920
   gtcggccagc agaacatgct tcaggtcgcg gtctgcgctg gaatgcacgg caacagtctt 4980
   aatgcccagc tctttacacg cgcgcaagat acgcagcgcg atttcaccgc ggtttgcaat 5040
   cacgatcttg tccaacatgt tcgcctcgtt actcgatcac aaccagcggt tcatcgaact 5100
   caacaggctg gccggattcc accaggatcg ctttaacagt accgctctta tccgcttcaa 5160
   tctggttcat cattttcatt gcctcaacga tacacagcgt gtcgccgaca ttgaccttct 5220
   ggccgacttc gatgaaggct tttgcgtccg gcgacggcgt gcgatagaag gtgcccacca 5280
   tcgggctacg cacgatatga cccgaaatct cggccgctgc cggtgcctcc atgcttggaa 5340
   cggtcgccgg tgcagcggcg ttgctctgtg ccggctgttg catcatcgga gcggcgtatg 5400
   cttgctgcat aaccggaaag ctcgctgccg gtgccgcacg ggaaatacgg acgctctctt 5460
   caccttcgct gatctccagc tcgctgatgc cgctttcttc aaccagttca atcagtttct 5520
   tgatcttacg aatgtccatg gtttttcctc cttcgcctgt ggtatgaaat ttcacacgca 5580
   ttatatacaa aaaaagcgat tcagaccccg ttggcaagcc gcgtggttaa ctccacgtgc 5640
   tattatgcct ccggctcttg gtccgggacc ggcggaacca cgacaccctc acgcggtgct 5700
   tcaggattcg gagccggcag attcatcaat ttcgccagaa tgctcgccag tttcagacgc 5760
   atttctggac ggcgaacgat catgtcgatg gcacctttct caatcaggaa ctcgctgcgc 5820
   tggaaaccag gcggcaactt ttcgcgaacc gtctgttcaa taacacgcgg accagcaaaa 5880
   ccaatcagag ccttcggctc tgcgatgttc aaatcaccca gcatcgcaaa gctggcgctg 5940
   acaccgccca tcgtagggtc ggtcaggacg gaaatgtacg gcaggccgcg ttcttgcatc 6000
   ttcgccaggg ctgccgaggt ttttgccatc tgcatcaggc tcatcagggc ctcttgcata 6060
   cgcgcaccac cgctcgcgct gaaacaaatc agaggacagt tatcctccag cgcctgctcc 6120
   acggcacgca caaagcgtgc gcccaccacg gaacccatgc tgccacccat aaacgcaaac 6180
   tcgaacgccg cagcaacaac cggcatacca tacagggtgc ctttcataac aaccagtgcg 6240
   tccttctcac cggtttcttt ttgtgcagat gccaaacgat ccttatactt cttggaatca 6300
   cggaatttca gaacatcttt cggttccagt tcgctaccca gctcgaccag gctgccctca 6360
   tccagcaggc tatgcagacg attacgagcg gtcatacgca tgtgatggtc gcatttcggg 6420
   cacacttcca ggttacgctc cagctccgca cgatacaaaa cctgaccgca gctatcgcat 6480
   ttggtccaga cgccttcagg gatgctcgcc ttgcgggtcg gggtgatatt ggacttaatg 6540
   cgttcaatcc agctcatgat tttcctcctt tactacgcgt agccatagct catcaagcgc 6600
   tgatagcgac ggtttttcag atcctccgtg ctcagcacgt ccagatccgc caggtccgcc 6660
   agcagctggg ccttcaggct ggctgccatc gcctccggat tacggtgagc accgcccagc 6720
   ggttccggga tgatagaatc aatcaacttc agttctttca gacgcggtgc gatgataccc 6780
   atcgcttccg ctgccaatgg tgctttatcc gcgctcttcc acagaatgga agcgcaacct 6840
   tccgggctaa tgacgctgta cgtgctgtat tgcagcatgt tgaccttatc accgacaccg 6900
   atcgccaacg caccaccgct accgccctcg ccaatcacgg tgcaaacgac cggcacaccc 6960
   agacgagaca tctcacgcag gttacgtgcg atggcctcgc tctgaccacg ctcttccgca 7020
   ccaacgcctg ggtacgcacc cggcgtatca atgaacgtga taatcggcat cttaaagcgt 7080
   tcggccattt gcatcagacg cagggccttg cggtagccct ccggtgccgg cataccaaag 7140
   ttacgacgaa tcttttcttt cgtctcgcgg cctttttgat ggccgataat catcaccgga 7200
   cggccatcca aacgagcaat accgccgaca attgctttat cgtccgcata ggcgcggtcg 7260
   cctgccagtt cgtcgaactc gtcaaacgcc aggcgcacgt agtccagggt atatgggcgt 7320
   tgtgggtggc gagccaactg ggcaatctgc catgcgccca aatcagcaaa gattttgcga 7380
   gtcagttcaa cagacttctc acgcaaacgg tggacctctt cgtcgatgtt aatgtccagt 7440
   ttctcatctt gacggctaac cgcggtcagg ctgtcaatct tcgcttccag ttctgcaatc 7500
   ggctgctcga agtccaggaa gttcagggac atgtttttcc tcctgccccg cgcttataag 7560
   cgaataaagg aagatggccg ccccgcaggg cagcaggtct gtgaaacagt atagagattc 7620
   atcggcacaa aggctttgct ttttgtcatt tattcaaacc ggcgcctcga accggctccg 7680
   ggcattgccc tggccggcac tttgcatggg gagatgcgga tctcgggcag cgttgggtcc 7740
   tggccacggg tgcgcatgat cgtgctcctg tcgttgagga cccggctagg ctggcggggt 7800
   tgccttactg gttagcagaa tgaatcaccg atacgcgagc gaacgtgaag cgactgctgc 7860
   tgcaaaacgt ctgcgacctg agcaacaaca tgaatggtct tcggtttccg tgtttcgtaa 7920
   agtctggaaa cgcggaagtc ccctacgtgc tgctgaagtt gcccgcaaca gagagtggaa 7980
   ccaaccggtg ataccacgat actatgactg agagtcaacg ccatgagcgg cctcatttct 8040
   tattctgagt tacaacagtc cgcaccgctg tccggtagct ccttccggtg ggcgcggggc 8100
   atgactatcg tcgccgcact tatgactgtc ttctttatca tgcaactcgt aggacaggtg 8160
   ccggcagcgc ccaacagtcc cccggccacg gggcctgcca ccatacccac gccgaaacaa 8220
   gcgccctgca ccattatgtt ccggatctgc atcgcaggat gctgctggct accctgtgga 8280
   acacctacat ctgtattaac gaagcgctaa ccgtttttat caggctctgg gaggcagaat 8340
   aaatgatcat atcgtcaatt attacctcca cggggagagc ctgagcaaac tggcctcagg 8400
   catttgagaa gcacacggtc acactgcttc cggtagtcaa taaaccggta aaccagcaat 8460
   agacataagc ggctatttaa cgaccctgcc ctgaaccgac gaccgggtcg aatttgcttt 8520
   cgaatttctg ccattcatcc gcttattatc acttattcag gcgtagcacc aggcgtttaa 8580
   gggcaccaat aactgcctta aaaaaattac gccccgccct gccactcatc gcagtactgt 8640
   tgtaattcat taagcattct gccgacatgg aagccatcac agacggcatg atgaacctga 8700
   atcgccagcg gcatcagcac cttgtcgcct tgcgtataat atttgcccat ggtgaaaacg 8760
   ggggcgaaga agttgtccat attggccacg tttaaatcaa aactggtgaa actcacccag 8820
   ggattggctg agacgaaaaa catattctca ataaaccctt tagggaaata ggccaggttt 8880
   tcaccgtaac acgccacatc ttgcgaatat atgtgtagaa actgccggaa atcgtcgtgg 8940
   tattcactcc agagcgatga aaacgtttca gtttgctcat ggaaaacggt gtaacaaggg 9000
   tgaacactat cccatatcac cagctcaccg tctttcattg ccatacg 9047
<210> 6
   <211> 5592
   <212> DNA
   <213> Artificial Sequence
<220>
   <223> Description of Artificial Sequence: Synthetic pBT-3-PtiA:pntAB plasmid polynucleotide
<400> 6
   aacgaattca agcttgatat ccactgtgga attcgccctt ttacagagct ttcaggattg      60
   catccacgct ggctttggcg tcaccaaaca gcatgtgggt gttttccttg aagaacagcg     120
   ggttttgcac accagcatag ccagtgttca tcgaacgttt aaagacaatc acgttctgcg     180
   ctttccacac ttccagcaca ggcataccag caatcggact cttcggatca tcctgcgccg     240
   ccgggttaac cgtatcgtta gcaccaatca ccagtacggt atcggtatca gcaaagtcat     300
   cattgatctc gtccatttcc agcacgatgt catacggtac ttttgcttca gccagcaata 360
   cgttcatatg tccaggcaaa cgccccgcga ccgggtggat accgaaacgc acattaatac 420
   cacgagcgcg caatttctca gtaatttcag cgacaggata ttgcgcctgc gcgactgcca 480
   tgccgtaccc cggagtaatg atcactgaat gggagttttt cagcagttcc gctgtctctt 540
   ctgcggtgat ttcgcggtgc tcacccactt cctgatcatc gccagtagaa gagccgtcgg 600
   tgccgaaacc acccgcaata acgctgataa aggaacggtt catcgcctta cacataatgt 660
   aagaaaggat agcccccgaa gaaccgacca gcgcaccggt cacaatcagc aggtcgttgc 720
   tgagcataaa gcccgcagcc gcagccgccc agccggagta cgagttcagc atcgacacca 780
   ccactggcat atctgcacca ccgatggagg cgactaaatg ccagccgaat accagcgcaa 840
   ttgcggtcat tatcagcaat gccagcactt gcaggccgac gctgtccgtg cgaacaaata 900
   caatcagcag caggaaggaa acgaccagag ccgccaggtt cattttgtga cggtttggca 960
   gcatcaatgg tttagacgaa atcttgccac acagtttgcc gaacgccacc accgaacccg 1020
   tgaacgttac cgccccgatg aagataccga ggaacacttc cgtcaggtga atattgacca 1080
   gaatcggtgc cattcccgcg tcatgatgca gatagctgtt aaagccaacc agcactgccg 1140
   ccagacccac gaagctatgc aggatcgcca ccagttctgg catttcggtc atttcaactt 1200
   tcttcgccag acggatacca attgccccac caatgaccat cgccagcaag atccagccaa 1260
   cattacccgt atccggtcca aaaatggttg cgattaacgc aatcgccatc ccggcgatac 1320
   cgaagttgtt accctggcga gacgtttcat gtttcgaaag accggccaga ctgaagataa 1380
   acaggatcgc ggcaacaatg tatgcagctg taactaatcc tccagacata tgttacccct 1440
   taatttttgc ggaacatttt cagcatgcgc tgagtcacgg tgaagccacc gaaaatatta 1500
   atgctggcta taagcaccgc gataaaacta aggaagctaa cccagccgcc ctggccaatc 1560
   tgcaacagtg ctccgacaac aataatccct gaaatcgcgt tggtgaccga catcaacggt 1620
   gtatgcagcg cgtgcgatac attccacacc acgtaataac cgacaacgca ggccagcgcg 1680
   aaaacggtga agtgcccaag gaattctttc ggcgcaacgc ttgccatcca gccaaaaaga 1740
   atgattgcca gcgccatcaa cgcgtattta cgccacggtg agcaggtaca tttttcctca 1800
   gttttcactt ccggtgccgc tttttgtgcc gcctgcggct gagctgatac ctgaatcggc 1860
   ggtgccggcc aggtaatttc gcccgcacgg atcacggtca cgccgcgaat caccacatca 1920
   tcaaaatcaa cagtgatatt gccgtctttc tctttgcaca acagtttcag cagattaacg 1980
   aggtttgtgc cgtaaagctg tgaggattgc gtcggcagac ggcccggaag atcggtataa 2040
   ccaatcactt tgacaccatt ttccgtagtg aagatttcac ccggcacggt gtattcacag 2100
   ttgccgccgt tttgggctgc caggtcgaca atcacactgc ccgccttcat ggagtcaacc 2160
   atttcacggg taattagctt cggcgctggt ttgcctggaa taagcgcggt ggtgacaatg 2220
   atatcgacct cttttgcctg ggcggcaaag agttccattt ccgctttgat gaacgcgtcc 2280
   gacatcactt tggcatagcc atcgccgctg ccagcttcct ctttaaaatc cagctcgagg 2340
   aattccgcgc ccatactttg aacttgttct ttcacttccg ggcgggtgtc gaatgcacgc 2400
   acaatcgcgc cgagactgtt tgctgcgcca atggcggcca gacctgcaac acccgcacca 2460
   atcaccatca cttttgccgg tggcactttc ccggccgcag taatttgccc ggtaaagaag 2520
   cgcccaaatt catgtgccgc ttcaacaatg gcgcgataac cggcgatgtt cgccatcgag 2580
   cttagtgcgt ccagcgattg tgcgcgtgag atacgcggca cagagtccat cgccatcacg 2640
   gtcacgttac gttccgcaag tttttgcatt aattccggat tctgcgcagg ccagataaaa 2700
   ctcaccagcg ttgtcccagg attcagtaac gcaatttcat catctaacgg cgcattgacc 2760
   ttcagaatga tctctgactg ccagacgcta ttcccttcta caatttcagc gcccgcttgc 2820
   acaaacgctt tatcgtcaaa acttgccagt tgacccgcgc cgctctctac cgcgacggta 2880
   aaacccagtt tcagcagctg ttccactgtt tttggcgttg ctgcaacacg ggtttcattg 2940
   gttaaccgtt ctcttggtat gccaattcgc attttaattc tccacgctta taagcgaata 3000
   aaggaagatg gccgccccgc agggcagcag gtctgtgaaa cagtatagag attcatcggc 3060
   acaaaggctt tgctttttgt catttattca aaccaagggc gaattccaca ttggtcgctg 3120
   cagcccgggg gatccactag ttctagagcg gccgcaccgc gggagctaga gaattcgtca 3180
   acgaattcaa cgaattcaag cttgatatca ttcaggacga gcctcagact ccagcgtaac 3240
   tggactgaaa acaaactaaa gcgcccttgt ggcgctttag ttttgttccg cggccaccgg 3300
   ctggctcgct tcgctcggcc cgtggacaac cctgctggac aagctgatgg acaggctgcg 3360
   cctgcccacg agcttgacca cagggattgc ccaccggcta cccagccttc gaccacatac 3420
   ccaccggctc caactgcgcg gcctgcggcc ttgccccatc aattttttta attttctctg 3480
   gggaaaagcc tccggcctgc ggcctgcgcg cttcgcttgc cggttggaca ccaagtggaa 3540
   ggcgggtcaa ggctcgcgca gcgaccgcgc agcggcttgg ccttgacgcg cctggaacga 3600
   cccaagccta tgcgagtggg ggcagtcgaa ggcgaagccc gcccgcctgc cccccgagcc 3660
   tcacggcggc gagtgcgggg gttccaaggg ggcagcgcca ccttgggcaa ggccgaaggc 3720
   cgcgcagtcg atcaacaagc cccggagggg ccactttttg ccggaggggg agccgcgccg 3780
   aaggcgtggg ggaaccccgc aggggtgccc ttctttgggc accaaagaac tagatatagg 3840
   gcgaaatgcg aaagacttaa aaatcaacaa cttaaaaaag gggggtacgc aacagctcat 3900
   tgcggcaccc cccgcaatag ctcattgcgt aggttaaaga aaatctgtaa ttgactgcca 3960
   cttttacgca acgcataatt gttgtcgcgc tgccgaaaag ttgcagctga ttgcgcatgg 4020
   tgccgcaacc gtgcggcacc ctaccgcatg gagataagca tggccacgca gtccagagaa 4080
   atcggcattc aagccaagaa caagcccggt cactgggtgc aaacggaacg caaagcgcat 4140
   gaggcgtggg ccgggcttat tgcgaggaaa cccacggcgg caatgctgct gcatcacctc 4200
   gtggcgcaga tgggccacca gaacgccgtg gtggtcagcc agaagacact ttccaagctc 4260
   atcggacgtt ctttgcggac ggtccaatac gcagtcaagg acttggtggc cgagcgctgg 4320
   atctccgtcg tgaagctcaa cggccccggc accgtgtcgg cctacgtggt caatgaccgc 4380
   gtggcgtggg gccagccccg cgaccagttg cgcctgtcgg tgttcagtgc cgccgtggtg 4440
   gttgatcacg acgaccagga cgaatcgctg ttggggcatg gcgacctgcg ccgcatcccg 4500
   accctgtatc cgggcgagca gcaactaccg accggccccg gcgaggagcc gcccagccag 4560
   cccggcattc cgggcatgga accagacctg ccagccttga ccgaaacgga ggaatgggaa 4620
   cggcgcgggc agcagcgcct gccgatgccc gatgagccgt gttttctgga cgatggcgag 4680
   ccgttggagc cgccgacacg ggtcacgctg ccgcgccggt agtacgtaag aggttccaac 4740
   tttcaccata atgaaataag atcactaccg ggcgtatttt ttgagttatc gagattttca 4800
   ggagctaagg aagctaaaat ggagaaaaaa atcactggat ataccaccgt tgatatatcc 4860
   caatggcatc gtaaagaaca ttttgaggca tttcagtcag ttgctcaatg tacctataac 4920
   cagaccgttc agctggatat tacggccttt ttaaagaccg taaagaaaaa taagcacaag 4980
   ttttatccgg cctttattca cattcttgcc cgcctgatga atgctcatcc ggaattccgt 5040
   atggcaatga aagacggtga gctggtgata tgggatagtg ttcacccttg ttacaccgtt 5100
   ttccatgagc aaactgaaac gttttcatcg ctctggagtg aataccacga cgatttccgg 5160
   cagtttctac acatatattc gcaagatgtg gcgtgttacg gtgaaaacct ggcctatttc 5220
   cctaaagggt ttattgagaa tatgtttttc gtctcagcca atccctgggt gagtttcacc 5280
   agttttgatt taaacgtggc caatatggac aacttcttcg cccccgtttt caccatgggc 5340
   aaatattata cgcaaggcga caaggtgctg atgccgctgg cgattcaggt tcatcatgcc 5400
   gtttgtgatg gcttccatgt cggcagaatg cttaatgaat tacaacagta ctgcgatgag 5460
   tggcagggcg gggcgtaaac gcgtggatcc ccctcaagtc aaaagcctcc ggtcggaggc 5520
   ttttgacttt ctgctatgga ggtcaggtat gatttaaatg gtcagtattg agcgatatct 5580
   agagaattcg tc 5592
<210> 7
   <211> 309
   <212> PRT
   <213> Escherichia coli
<220>
   <223> malonyl-CoA-[acyl-carrier-protein] transacylase [Escherichia coli str. K-12 substr.] MG1655
<400> 7
<210> 8
   <211> 413
   <212> PRT
   <213> Escherichia coli
<220>
   <223> 3-oxoacyl-[acyl-carrier-protein] synthase II [Escherichia coli str. K-12 substr. MG1655]
<400> 8
   Met Ser Lys Arg Arg Val Val Val Thr Gly Leu Gly Met Leu Ser Pro
   1 5 10 15
   Val Gly Asn Thr Val Glu Ser Thr Trp Lys Ala Leu Leu Ala Gly Gln
   20 25 30
   Ser Gly Ile Ser Leu Ile Asp His Phe Asp Thr Ser Ala Tyr Ala Thr
   35 40 45
   Lys Phe Ala Gly Leu Val Lys Asp Phe Asn Cys Glu Asp Ile Ile Ser
   50 55 60
   Arg Lys Glu Gln Arg Lys Met Asp Ala Phe Ile Gln Tyr Gly Ile Val
   65 70 75 80
   Ala Gly Val Gln Ala Met Gln Asp Ser Gly Leu Glu Ile Thr Glu Glu
   85 90 95
   Asn Ala Thr Arg Ile Gly Ala Ala Ile Gly Ser Gly Ile Gly Gly Leu
   100 105 110
   Gly Leu Ile Glu Glu Asn His Thr Ser Leu Met Asn Gly Gly Pro Arg
   115 120 125
   Lys Ile Ser Pro Phe Phe Val Pro Ser Thr Ile Val Asn Met Val Ala
   130 135 140
   Gly His Leu Thr Ile Met Tyr Gly Leu Arg Gly Pro Ser Ile Ser Ile
   145                 150                 155                 160
   Ala Thr Ala Cys Thr Ser Gly Val His Asn Ile Gly His Ala Ala Arg
                   165                 170                 175
   Ile Ile Ala Tyr Gly Asp Ala Asp Val Met Val Ala Gly Gly Ala Glu
               180                 185                 190
   Lys Ala Ser Thr Pro Leu Gly Val Gly Gly Phe Gly Ala Ala Arg Ala
           195                 200                 205
   Leu Ser Thr Arg Asn Asp Asn Pro Gln Ala Ala Ser Arg Pro Trp Asp
       210                 215                 220
   Lys Glu Arg Asp Gly Phe Val Leu Gly Asp Gly Ala Gly Met Leu Val
   225                 230                 235                 240
   Leu Glu Glu Tyr Glu His Ala Lys Lys Arg Gly Ala Lys Ile Tyr Ala
                   245                 250                 255
   Glu Leu Val Gly Phe Gly Met Ser Ser Asp Ala Tyr His Met Thr Ser
               260                 265                 270
   Pro Pro Glu Asn Gly Ala Gly Ala Ala Leu Ala Met Ala Asn Ala Leu
           275                 280                 285
   Arg Asp Ala Gly Ile Glu Ala Ser Gln Ile Gly Tyr Val Asn Ala His
       290                 295                 300
   Gly Thr Ser Thr Pro Ala Gly Asp Lys Ala Glu Ala Gln Ala Val Lys
   305                 310                 315                 320
   Thr Ile Phe Gly Glu Ala Ala Ser Arg Val Leu Val Ser Ser Thr Lys
                   325                 330                 335
   Ser Met Thr Gly His Leu Leu Gly Ala Ala Gly Ala Val Glu Ser Ile
               340                 345                 350
   Tyr Ser Ile Leu Ala Leu Arg Asp Gln Ala Val Pro Pro Thr Ile Asn
           355                 360                 365
   Leu Asp Asn Pro Asp Glu Gly Cys Asp Leu Asp Phe Val Pro His Glu
       370                 375                 380
   Ala Arg Gln Val Ser Gly Met Glu Tyr Thr Leu Cys Asn Ser Phe Gly
   385                 390                 395                 400
   Phe Gly Gly Thr Asn Gly Ser Leu Ile Phe Lys Lys Ile
   405 410
<210> 9
   <211> 406
   <212> PRT
   <213> Escherichia coli
<220>
   <223> 3-oxoacyl-[acyl-carrier-protein] synthase I [Escherichia coli str. K-12 substr. MG1655]
<400> 9
   Met Lys Arg Ala Val Ile Thr Gly Leu Gly Ile Val Ser Ser Ile Gly
   1 5 10 15
   Asn Asn Gln Gln Glu Val Leu Ala Ser Leu Arg Glu Gly Arg Ser Gly
   20 25 30
   Ile Thr Phe Ser Gln Glu Leu Lys Asp Ser Gly Met Arg Ser His Val
   35 40 45
   Trp Gly Asn Val Lys Leu Asp Thr Thr Gly Leu Ile Asp Arg Lys Val
   50 55 60
   Val Arg Phe Met Ser Asp Ala Ser Ile Tyr Ala Phe Leu Ser Met Glu
   65 70 75 80
   Gln Ala Ile Ala Asp Ala Gly Leu Ser Pro Glu Ala Tyr Gln Asn Asn
   85 90 95
   Pro Arg Val Gly Leu Ile Ala Gly Ser Gly Gly Gly Ser Pro Arg Phe
   100 105 110
   Gln Val Phe Gly Ala Asp Ala Met Arg Gly Pro Arg Gly Leu Lys Ala
   115 120 125
   Val Gly Pro Tyr Val Val Thr Lys Ala Met Ala Ser Gly Val Ser Ala
   130 135 140
   Cys Leu Ala Thr Pro Phe Lys Ile His Gly Val Asn Tyr Ser Ile Ser
   145 150 155 160
   Ser Ala Cys Ala Thr Ser Ala His Cys Ile Gly Asn Ala Val Glu Gln
   165 170 175
   Ile Gln Leu Gly Lys Gln Asp Ile Val Phe Ala Gly Gly Gly Glu Glu
   180 185 190
   Leu Cys Trp Glu Met Ala Cys Glu Phe Asp Ala Met Gly Ala Leu Ser
   195 200 205
   Thr Lys Tyr Asn Asp Thr Pro Glu Lys Ala Ser Arg Thr Tyr Asp Ala
   210 215 220
   His Arg Asp Gly Phe Val Ile Ala Gly Gly Gly Gly Met Val Val Val
   225 230 235 240
   Glu Glu Leu Glu His Ala Leu Ala Arg Gly Ala His Ile Tyr Ala Glu
   245 250 255
   Ile Val Gly Tyr Gly Ala Thr Ser Asp Gly Ala Asp Met Val Ala Pro
   260 265 270
   Ser Gly Glu Gly Ala Val Arg Cys Met Lys Met Ala Met His Gly Val
   275 280 285
   Asp Thr Pro Ile Asp Tyr Leu Asn Ser His Gly Thr Ser Thr Pro Val
   290 295 300
   Gly Asp Val Lys Glu Leu Ala Ala Ile Arg Glu Val Phe Gly Asp Lys
   305 310 315 320
   Ser Pro Ala Ile Ser Ala Thr Lys Ala Met Thr Gly His Ser Leu Gly
   325 330 335
   Ala Ala Gly Val Gln Glu Ala Ile Tyr Ser Leu Leu Met Leu Glu His
   340 345 350
   Gly Phe Ile Ala Pro Ser Ile Asn Ile Glu Glu Leu Asp Glu Gln Ala
   355 360 365
   Ala Gly Leu Asn Ile Val Thr Glu Thr Thr Asp Arg Glu Leu Thr Thr
   370 375 380
   Val Met Ser Asn Ser Phe Gly Phe Gly Gly Thr Asn Ala Thr Leu Val
   385 390 395 400
   Met Arg Lys Leu Lys Asp
   405
<210> 10
   <211> 317
   <212> PRT
   <213> Escherichia coli
<220>
   <223> FABH-MONOMER FabH 1147982..1148935 Escherichia coli K-12 substr. MG1655
<400> 10
   Met Tyr Thr Lys Ile Ile Gly Thr Gly Ser Tyr Leu Pro Glu Gln Val
   1               5                   10                  15
   Arg Thr Asn Ala Asp Leu Glu Lys Met Val Asp Thr Ser Asp Glu Trp
               20                  25                  30
   Ile Val Thr Arg Thr Gly Ile Arg Glu Arg His Ile Ala Ala Pro Asn
           35                  40                  45
   Glu Thr Val Ser Thr Met Gly Phe Glu Ala Ala Thr Arg Ala Ile Glu
       50                  55                  60
   Met Ala Gly Ile Glu Lys Asp Gln Ile Gly Leu Ile Val Val Ala Thr
   65                  70                  75                  80
   Thr Ser Ala Thr His Ala Phe Pro Ser Ala Ala Cys Gln Ile Gln Ser
                   85                  90                  95
   Met Leu Gly Ile Lys Gly Cys Pro Ala Phe Asp Val Ala Ala Ala Cys
               100                 105                 110
   Ala Gly Phe Thr Tyr Ala Leu Ser Val Ala Asp Gln Tyr Val Lys Ser
           115                 120                 125
   Gly Ala Val Lys Tyr Ala Leu Val Val Gly Ser Asp Val Leu Ala Arg
       130                 135                 140
   Thr Cys Asp Pro Thr Asp Arg Gly Thr Ile Ile Ile Phe Gly Asp Gly
   145                 150                 155                 160
   Ala Gly Ala Ala Val Leu Ala Ala Ser Glu Glu Pro Gly Ile Ile Ser
                   165                 170                 175
   Thr His Leu His Ala Asp Gly Ser Tyr Gly Glu Leu Leu Thr Leu Pro
               180                 185                 190
   Asn Ala Asp Arg Val Asn Pro Glu Asn Ser Ile His Leu Thr Met Ala
           195                 200                 205
   Gly Asn Glu Val Phe Lys Val Ala Val Thr Glu Leu Ala His Ile Val
       210                 215                 220
   Asp Glu Thr Leu Ala Ala Asn Asn Leu Asp Arg Ser Gln Leu Asp Trp
   225                 230                 235                 240
   Leu Val Pro His Gln Ala Asn Leu Arg Ile Ile Ser Ala Thr Ala Lys
                   245                 250                 255
   Lys Leu Gly Met Ser Met Asp Asn Val Val Val Thr Leu Asp Arg His
               260                 265                 270
   Gly Asn Thr Ser Ala Ala Ser Val Pro Cys Ala Leu Asp Glu Ala Val
           275                 280                 285
   Arg Asp Gly Arg Ile Lys Pro Gly Gln Leu Val Leu Leu Glu Ala Phe
       290                 295                 300
   Gly Gly Gly Phe Thr Trp Gly Ser Ala Leu Val Arg Phe
   305                 310                 315
<210> 11
   <211> 244
   <212> PRT
   <213> Escherichia coli
<220>
   <223> 3-oxoacyl-[acyl-carrier-protein] reductase [Escherichia coli str. K-12 substr. MG1655]
<400> 11
   Met Asn Phe Glu Gly Lys Ile Ala Leu Val Thr Gly Ala Ser Arg Gly
   1               5                   10                  15
   Ile Gly Arg Ala Ile Ala Glu Thr Leu Ala Ala Arg Gly Ala Lys Val
               20                  25                  30
   Ile Gly Thr Ala Thr Ser Glu Asn Gly Ala Gln Ala Ile Ser Asp Tyr
           35                  40                  45
   Leu Gly Ala Asn Gly Lys Gly Leu Met Leu Asn Val Thr Asp Pro Ala
       50                  55                  60
   Ser Ile Glu Ser Val Leu Glu Lys Ile Arg Ala Glu Phe Gly Glu Val
   65                  70                  75                  80
   Asp Ile Leu Val Asn Asn Ala Gly Ile Thr Arg Asp Asn Leu Leu Met
                   85                  90                  95
   Arg Met Lys Asp Glu Glu Trp Asn Asp Ile Ile Glu Thr Asn Leu Ser
               100                 105                 110
   Ser Val Phe Arg Leu Ser Lys Ala Val Met Arg Ala Met Met Lys Lys
           115                 120                 125
   Arg His Gly Arg Ile Ile Thr Ile Gly Ser Val Val Gly Thr Met Gly
   130 135 140
   Asn Gly Gly Gln Ala Asn Tyr Ala Ala Ala Lys Ala Gly Leu Ile Gly
   145 150 155 160
   Phe Ser Lys Ser Leu Ala Arg Glu Val Ala Ser Arg Gly Ile Thr Val
   165 170 175
   Asn Val Val Ala Pro Gly Phe Ile Glu Thr Asp Met Thr Arg Ala Leu
   180 185 190
   Ser Asp Asp Gln Arg Ala Gly Ile Leu Ala Gln Val Pro Ala Gly Arg
   195 200 205
   Leu Gly Gly Ala Gln Glu Ile Ala Asn Ala Val Ala Phe Leu Ala Ser
   210 215 220
   Asp Glu Ala Ala Tyr Ile Thr Gly Glu Thr Leu His Val Asn Gly Gly
   225 230 235 240
   Met Tyr Met Val
<210> 12
   <211> 151
   <212> PRT
   <213> Escherichia coli
<220>
   <223> (3R)-hydroxymyristol acyl carrier protein dehydratase [Escherichia coli str. K-12 substr. MG1655
<400> 12
   Met Thr Thr Asn Thr His Thr Leu Gln Ile Glu Glu Ile Leu Glu Leu
   1               5                   10                  15
   Leu Pro His Arg Phe Pro Phe Leu Leu Val Asp Arg Val Leu Asp Phe
               20                  25                  30
   Glu Glu Gly Arg Phe Leu Arg Ala Val Lys Asn Val Ser Val Asn Glu
           35                  40                  45
   Pro Phe Phe Gln Gly His Phe Pro Gly Lys Pro Ile Phe Pro Gly Val
       50                  55                  60
   Leu Ile Leu Glu Ala Met Ala Gln Ala Thr Gly Ile Leu Ala Phe Lys
   65                  70                  75                  80
   Ser Val Gly Lys Leu Glu Pro Gly Glu Leu Tyr Tyr Phe Ala Gly Ile
                   85                  90                  95
   Asp Glu Ala Arg Phe Lys Arg Pro Val Val Pro Gly Asp Gln Met Ile
               100                 105                 110
   Met Glu Val Thr Phe Glu Lys Thr Arg Arg Gly Leu Thr Arg Phe Lys
           115                 120                 125
   Gly Val Ala Leu Val Asp Gly Lys Val Val Cys Glu Ala Thr Met Met
       130                 135                 140
   Cys Ala Arg Ser Arg Glu Ala
   145                 150
<210> 13
   <211> 172
   <212> PRT
   <213> Escherichia coli
<220>
   <223> beta-hydroxydecanoyl thioester dehydrase [Escherichia coli str. K-12 substr. MG1655]
<400> 13
   Met Val Asp Lys Arg Glu Ser Tyr Thr Lys Glu Asp Leu Leu Ala Ser
   1               5                   10                  15
   Gly Arg Gly Glu Leu Phe Gly Ala Lys Gly Pro Gln Leu Pro Ala Pro
               20                  25                  30
   Asn Met Leu Met Met Asp Arg Val Val Lys Met Thr Glu Thr Gly Gly
           35                  40                  45
   Asn Phe Asp Lys Gly Tyr Val Glu Ala Glu Leu Asp Ile Asn Pro Asp
       50                  55                  60
   Leu Trp Phe Phe Gly Cys His Phe Ile Gly Asp Pro Val Met Pro Gly
   65                  70                  75                  80
   Cys Leu Gly Leu Asp Ala Met Trp Gln Leu Val Gly Phe Tyr Leu Gly
                   85                  90                  95
   Trp Leu Gly Gly Glu Gly Lys Gly Arg Ala Leu Gly Val Gly Glu Val
               100                 105                 110
   Lys Phe Thr Gly Gln Val Leu Pro Thr Ala Lys Lys Val Thr Tyr Arg
           115                 120                 125
   Ile His Phe Lys Arg Ile Val Asn Arg Arg Leu Ile Met Gly Leu Ala
       130                 135                 140
   Asp Gly Glu Val Leu Val Asp Gly Arg Leu Ile Tyr Thr Ala Ser Asp
   145                 150                 155                 160
   Leu Lys Val Gly Leu Phe Gln Asp Thr Ser Ala Phe
                   165                 170
<210> 14
   <211> 262
   <212> PRT
   <213> Escherichia coli
<220>
   <223> enoyl-[acyl-carrier-protein] reductase, NADH-dependent [Escherichia coli str. K-12 substr. MG1655]
<400> 14
   Met Gly Phe Leu Ser Gly Lys Arg Ile Leu Val Thr Gly Val Ala Ser
   1               5                   10                  15
   Lys Leu Ser Ile Ala Tyr Gly Ile Ala Gln Ala Met His Arg Glu Gly
               20                  25                  30
   Ala Glu Leu Ala Phe Thr Tyr Gln Asn Asp Lys Leu Lys Gly Arg Val
           35                  40                  45
   Glu Glu Phe Ala Ala Gln Leu Gly Ser Asp Ile Val Leu Gln Cys Asp
       50                  55                  60
   Val Ala Glu Asp Ala Ser Ile Asp Thr Met Phe Ala Glu Leu Gly Lys
   65                  70                  75                  80
   Val Trp Pro Lys Phe Asp Gly Phe Val His Ser Ile Gly Phe Ala Pro
                   85                  90                  95
   Gly Asp Gln Leu Asp Gly Asp Tyr Val Asn Ala Val Thr Arg Glu Gly
               100                 105                 110
   Phe Lys Ile Ala His Asp Ile Ser Ser Tyr Ser Phe Val Ala Met Ala
           115                 120                 125
   Lys Ala Cys Arg Ser Met Leu Asn Pro Gly Ser Ala Leu Leu Thr Leu
       130                 135                 140
   Ser Tyr Leu Gly Ala Glu Arg Ala Ile Pro Asn Tyr Asn Val Met Gly
   145                 150                 155                 160
   Leu Ala Lys Ala Ser Leu Glu Ala Asn Val Arg Tyr Met Ala Asn Ala
                   165                 170                 175
   Met Gly Pro Glu Gly Val Arg Val Asn Ala Ile Ser Ala Gly Pro Ile
   180 185 190
   Arg Thr Leu Ala Ala Ser Gly Ile Lys Asp Phe Arg Lys Met Leu Ala
   195 200 205
   His Cys Glu Ala Val Thr Pro Ile Arg Arg Thr Val Thr Ile Glu Asp
   210 215 220
   Val Gly Asn Ser Ala Ala Phe Leu Cys Ser Asp Leu Ser Ala Gly Ile
   225 230 235 240
   Ser Gly Glu Val Val His Val Asp Gly Gly Phe Ser Ile Ala Ala Met
   245 250 255
   Asn Glu Leu Glu Leu Lys
   260
<210> 15
   <211> 1220
   <212> PRT
   <213> Chloroflexus aurantiacus
<400> 15
   Met Ser Gly Thr Gly Arg Leu Ala Gly Lys Ile Ala Leu Ile Thr Gly
   1               5                   10                  15
   Gly Ala Gly Asn Ile Gly Ser Glu Leu Thr Arg Arg Phe Leu Ala Glu
               20                  25                  30
   Gly Ala Thr Val Ile Ile Ser Gly Arg Asn Arg Ala Lys Leu Thr Ala
           35                  40                  45
   Leu Ala Glu Arg Met Gln Ala Glu Ala Gly Val Pro Ala Lys Arg Ile
       50                  55                  60
   Asp Leu Glu Val Met Asp Gly Ser Asp Pro Val Ala Val Arg Ala Gly
   65                  70                  75                  80
   Ile Glu Ala Ile Val Ala Arg His Gly Gln Ile Asp Ile Leu Val Asn
                   85                  90                  95
   Asn Ala Gly Ser Ala Gly Ala Gln Arg Arg Leu Ala Glu Ile Pro Leu
               100                 105                 110
   Thr Glu Ala Glu Leu Gly Pro Gly Ala Glu Glu Thr Leu His Ala Ser
           115                 120                 125
   Ile Ala Asn Leu Leu Gly Met Gly Trp His Leu Met Arg Ile Ala Ala
   130 135 140
   Pro His Met Pro Val Gly Ser Ala Val Ile Asn Val Ser Thr Ile Phe
   145 150 155 160
   Ser Arg Ala Glu Tyr Tyr Gly Arg Ile Pro Tyr Val Thr Pro Lys Ala
   165 170 175
   Ala Leu Asn Ala Leu Ser Gln Leu Ala Ala Arg Glu Leu Gly Ala Arg
   180 185 190
   Gly Ile Arg Val Asn Thr Ile Phe Pro Gly Pro Ile Glu Ser Asp Arg
   195 200 205
   Ile Arg Thr Val Phe Gln Arg Met Asp Gln Leu Lys Gly Arg Pro Glu
   210 215 220
   Gly Asp Thr Ala His His Phe Leu Asn Thr Met Arg Leu Cys Arg Ala
   225 230 235 240
   Asn Asp Gln Gly Ala Leu Glu Arg Arg Phe Pro Ser Val Gly Asp Val
   245 250 255
   Ala Asp Ala Ala Val Phe Leu Ala Ser Ala Glu Ser Ala Ala Leu Ser
   260 265 270
   Gly Glu Thr Ile Glu Val Thr His Gly Met Glu Leu Pro Ala Cys Ser
   275 280 285
   Glu Thr Ser Leu Leu Ala Arg Thr Asp Leu Arg Thr Ile Asp Ala Ser
   290 295 300
   Gly Arg Thr Thr Leu Ile Cys Ala Gly Asp Gln Ile Glu Glu Val Met
   305 310 315 320
   Ala Leu Thr Gly Met Leu Arg Thr Cys Gly Ser Glu Val Ile Ile Gly
   325 330 335
   Phe Arg Ser Ala Ala Ala Leu Ala Gln Phe Glu Gln Ala Val Asn Glu
   340 345 350
   Ser Arg Arg Leu Ala Gly Ala Asp Phe Thr Pro Pro Ile Ala Leu Pro
   355 360 365
   Leu Asp Pro Arg Asp Pro Ala Thr Ile Asp Ala Val Phe Asp Trp Gly
   370 375 380
   Ala Gly Glu Asn Thr Gly Gly Ile His Ala Ala Val Ile Leu Pro Ala
   385                 390                 395                 400
   Thr Ser His Glu Pro Ala Pro Cys Val Ile Glu Val Asp Asp Glu Arg
                   405                 410                 415
   Val Leu Asn Phe Leu Ala Asp Glu Ile Thr Gly Thr Ile Val Ile Ala
               420                 425                 430
   Ser Arg Leu Ala Arg Tyr Trp Gln Ser Gln Arg Leu Thr Pro Gly Ala
           435                 440                 445
   Arg Ala Arg Gly Pro Arg Val Ile Phe Leu Ser Asn Gly Ala Asp Gln
       450                 455                 460
   Asn Gly Asn Val Tyr Gly Arg Ile Gln Ser Ala Ala Ile Gly Gln Leu
   465                 470                 475                 480
   Ile Arg Val Trp Arg His Glu Ala Glu Leu Asp Tyr Gln Arg Ala Ser
                   485                 490                 495
   Ala Ala Gly Asp His Val Leu Pro Pro Val Trp Ala Asn Gln Ile Val
               500                 505                 510
   Arg Phe Ala Asn Arg Ser Leu Glu Gly Leu Glu Phe Ala Cys Ala Trp
           515                 520                 525
   Thr Ala Gln Leu Leu His Ser Gln Arg His Ile Asn Glu Ile Thr Leu
       530                 535                 540
   Asn Ile Pro Ala Asn Ile Ser Ala Thr Thr Gly Ala Arg Ser Ala Ser
   545                 550                 555                 560
   Val Gly Trp Ala Glu Ser Leu Ile Gly Leu His Leu Gly Lys Val Ala
                   565                 570                 575
   Leu Ile Thr Gly Gly Ser Ala Gly Ile Gly Gly Gln Ile Gly Arg Leu
               580                 585                 590
   Leu Ala Leu Ser Gly Ala Arg Val Met Leu Ala Ala Arg Asp Arg His
           595                 600                 605
   Lys Leu Glu Gln Met Gln Ala Met Ile Gln Ser Glu Leu Ala Glu Val
       610                 615                 620
   Gly Tyr Thr Asp Val Glu Asp Arg Val His Ile Ala Pro Gly Cys Asp
   625                 630                 635                 640
   Val Ser Ser Glu Ala Gln Leu Ala Asp Leu Val Glu Arg Thr Leu Ser
   645 650 655
   Ala Phe Gly Thr Val Asp Tyr Leu Ile Asn Asn Ala Gly Ile Ala Gly
   660 665 670
   Val Glu Glu Met Val Ile Asp Met Pro Val Glu Gly Trp Arg His Thr
   675 680 685
   Leu Phe Ala Asn Leu Ile Ser Asn Tyr Ser Leu Met Arg Lys Leu Ala
   690 695 700
   Pro Leu Met Lys Lys Gln Gly Ser Gly Tyr Ile Leu Asn Val Ser Ser
   705 710 715 720
   Tyr Phe Gly Gly Glu Lys Asp Ala Ala Ile Pro Tyr Pro Asn Arg Ala
   725 730 735
   Asp Tyr Ala Val Ser Lys Ala Gly Gln Arg Ala Met Ala Glu Val Phe
   740 745 750
   Ala Arg Phe Leu Gly Pro Glu Ile Gln Ile Asn Ala Ile Ala Pro Gly
   755 760 765
   Pro Val Glu Gly Asp Arg Leu Arg Gly Thr Gly Glu Arg Pro Gly Leu
   770 775 780
   Phe Ala Arg Arg Ala Arg Leu Ile Leu Glu Asn Lys Arg Leu Asn Glu
   785 790 795 800
   Leu His Ala Ala Leu Ile Ala Ala Ala Arg Thr Asp Glu Arg Ser Met
   805 810 815
   His Glu Leu Val Glu Leu Leu Leu Pro Asn Asp Val Ala Ala Leu Glu
   820 825 830
   Gln Asn Pro Ala Ala Pro Thr Ala Leu Arg Glu Leu Ala Arg Arg Phe
   835 840 845
   Arg Ser Glu Gly Asp Pro Ala Ala Ser Ser Ser Ser Ala Leu Leu Asn
   850 855 860
   Arg Ser Ile Ala Ala Lys Leu Leu Ala Arg Leu His Asn Gly Gly Tyr
   865 870 875 880
   Val Leu Pro Ala Asp Ile Phe Ala Asn Leu Pro Asn Pro Pro Asp Pro
   885 890 895
   Phe Phe Thr Arg Ala Gln Ile Asp Arg Glu Ala Arg Lys Val Arg Asp
               900                 905                 910
   Gly Ile Met Gly Met Leu Tyr Leu Gln Arg Met Pro Thr Glu Phe Asp
           915                 920                 925
   Val Ala Met Ala Thr Val Tyr Tyr Leu Ala Asp Arg Asn Val Ser Gly
       930                 935                 940
   Glu Thr Phe His Pro Ser Gly Gly Leu Arg Tyr Glu Arg Thr Pro Thr
   945                 950                 955                 960
   Gly Gly Glu Leu Phe Gly Leu Pro Ser Pro Glu Arg Leu Ala Glu Leu
                   965                 970                 975
   Val Gly Ser Thr Val Tyr Leu Ile Gly Glu His Leu Thr Glu His Leu
               980                 985                 990
   Asn Leu Leu Ala Arg Ala Tyr Leu  Glu Arg Tyr Gly Ala  Arg Gln Val
           995                 1000                 1005
   Val Met  Ile Val Glu Thr Glu  Thr Gly Ala Glu Thr  Met Arg Arg
       1010                 1015                 1020
   Leu Leu  His Asp His Val Glu  Ala Gly Arg Leu Met  Thr Ile Val
       1025                 1030                 1035
   Ala Gly  Asp Gln Ile Glu Ala  Ala Ile Asp Gln Ala  Ile Thr Arg
       1040                 1045                 1050
   Tyr Gly  Arg Pro Gly Pro Val  Val Cys Thr Pro Phe  Arg Pro Leu
       1055                 1060                 1065
   Pro Thr  Val Pro Leu Val Gly  Arg Lys Asp Ser Asp  Trp Ser Thr
       1070                 1075                 1080
   Val Leu  Ser Glu Ala Glu Phe  Ala Glu Leu Cys Glu  His Gln Leu
       1085                 1090                 1095
   Thr His  His Phe Arg Val Ala  Arg Lys Ile Ala Leu  Ser Asp Gly
       1100                 1105                 1110
   Ala Ser  Leu Ala Leu Val Thr  Pro Glu Thr Thr Ala  Thr Ser Thr
       1115                 1120                 1125
   Thr Glu  Gln Phe Ala Leu Ala  Asn Phe Ile Lys Thr  Thr Leu His
   1130 1135 1140
   Ala Phe Thr Ala Thr Ile Gly Val Glu Ser Glu Arg Thr Ala Gln
   1145 1150 1155
   Arg Ile Leu Ile Asn Gln Val Asp Leu Thr Arg Arg Ala Arg Ala
   1160 1165 1170
   Glu Glu Pro Arg Asp Pro His Glu Arg Gln Gln Glu Leu Glu Arg
   1175 1180 1185
   Phe Ile Glu Ala Val Leu Leu Val Thr Ala Pro Leu Pro Pro Glu
   1190 1195 1200
   Ala Asp Thr Arg Tyr Ala Gly Arg Ile His Arg Gly Arg Ala Ile
   1205 1210 1215
   Thr Val
   1220
<210> 16
   <211> 8252
   <212> DNA
   <213> Artificial Sequence
<220>
   <223> Description of Artificial Sequence: Synthetic polynucleotide
<400> 16
   gaattccgct agcaggagct aaggaagcta aaatgtccgg tacgggtcgt ttggctggta      60
   aaattgcatt gatcaccggt ggtgctggta acattggttc cgagctgacc cgccgttttc     120
   tggccgaggg tgcgacggtt attatcagcg gccgtaaccg tgcgaagctg accgcgctgg     180
   ccgagcgcat gcaagccgag gccggcgtgc cggccaagcg cattgatttg gaggtgatgg     240
   atggttccga ccctgtggct gtccgtgccg gtatcgaggc aatcgtcgct cgccacggtc     300
   agattgacat tctggttaac aacgcgggct ccgccggtgc ccaacgtcgc ttggcggaaa     360
   ttccgctgac ggaggcagaa ttgggtccgg gtgcggagga gactttgcac gcttcgatcg     420
   cgaatctgtt gggcatgggt tggcacctga tgcgtattgc ggctccgcac atgccagttg     480
   gctccgcagt tatcaacgtt tcgactattt tctcgcgcgc agagtactat ggtcgcattc     540
   cgtacgttac cccgaaggca gcgctgaacg ctttgtccca gctggctgcc cgcgagctgg     600
   gcgctcgtgg catccgcgtt aacactattt tcccaggtcc tattgagtcc gaccgcatcc     660
   gtaccgtgtt tcaacgtatg gatcaactga agggtcgccc ggagggcgac accgcccatc     720
   actttttgaa caccatgcgc ctgtgccgcg caaacgacca aggcgctttg gaacgccgct     780
   ttccgtccgt tggcgatgtt gctgatgcgg ctgtgtttct ggcttctgct gagagcgcgg 840
   cactgtcggg tgagacgatt gaggtcaccc acggtatgga actgccggcg tgtagcgaaa 900
   cctccttgtt ggcgcgtacc gatctgcgta ccatcgacgc gagcggtcgc actaccctga 960
   tttgcgctgg cgatcaaatt gaagaagtta tggccctgac gggcatgctg cgtacgtgcg 1020
   gtagcgaagt gattatcggc ttccgttctg cggctgccct ggcgcaattt gagcaggcag 1080
   tgaatgaatc tcgccgtctg gcaggtgcgg atttcacccc gccgatcgct ttgccgttgg 1140
   acccacgtga cccggccacc attgatgcgg ttttcgattg gggcgcaggc gagaatacgg 1200
   gtggcatcca tgcggcggtc attctgccgg caacctccca cgaaccggct ccgtgcgtga 1260
   ttgaagtcga tgacgaacgc gtcctgaatt tcctggccga tgaaattacc ggcaccatcg 1320
   ttattgcgag ccgtttggcg cgctattggc aatcccaacg cctgaccccg ggtgcccgtg 1380
   cccgcggtcc gcgtgttatc tttctgagca acggtgccga tcaaaatggt aatgtttacg 1440
   gtcgtattca atctgcggcg atcggtcaat tgattcgcgt ttggcgtcac gaggcggagt 1500
   tggactatca acgtgcatcc gccgcaggcg atcacgttct gccgccggtt tgggcgaacc 1560
   agattgtccg tttcgctaac cgctccctgg aaggtctgga gttcgcgtgc gcgtggaccg 1620
   cacagctgct gcacagccaa cgtcatatta acgaaattac gctgaacatt ccagccaata 1680
   ttagcgcgac cacgggcgca cgttccgcca gcgtcggctg ggccgagtcc ttgattggtc 1740
   tgcacctggg caaggtggct ctgattaccg gtggttcggc gggcatcggt ggtcaaatcg 1800
   gtcgtctgct ggccttgtct ggcgcgcgtg tgatgctggc cgctcgcgat cgccataaat 1860
   tggaacagat gcaagccatg attcaaagcg aattggcgga ggttggttat accgatgtgg 1920
   aggaccgtgt gcacatcgct ccgggttgcg atgtgagcag cgaggcgcag ctggcagatc 1980
   tggtggaacg tacgctgtcc gcattcggta ccgtggatta tttgattaat aacgccggta 2040
   ttgcgggcgt ggaggagatg gtgatcgaca tgccggtgga aggctggcgt cacaccctgt 2100
   ttgccaacct gatttcgaat tattcgctga tgcgcaagtt ggcgccgctg atgaagaagc 2160
   aaggtagcgg ttacatcctg aacgtttctt cctattttgg cggtgagaag gacgcggcga 2220
   ttccttatcc gaaccgcgcc gactacgccg tctccaaggc tggccaacgc gcgatggcgg 2280
   aagtgttcgc tcgtttcctg ggtccagaga ttcagatcaa tgctattgcc ccaggtccgg 2340
   ttgaaggcga ccgcctgcgt ggtaccggtg agcgtccggg cctgtttgct cgtcgcgccc 2400
   gtctgatctt ggagaataaa cgcctgaacg aattgcacgc ggctttgatt gctgcggccc 2460
   gcaccgatga gcgctcgatg cacgagttgg ttgaattgtt gctgccgaac gacgtggccg 2520
   cgttggagca gaacccagcg gcccctaccg cgctgcgtga gctggcacgc cgcttccgta 2580
   gcgaaggtga tccggcggca agctcctcgt ccgccttgct gaatcgctcc atcgctgcca 2640
   agctgttggc tcgcttgcat aacggtggct atgtgctgcc ggcggatatt tttgcaaatc 2700
   tgcctaatcc gccggacccg ttctttaccc gtgcgcaaat tgaccgcgaa gctcgcaagg 2760
   tgcgtgatgg tattatgggt atgctgtatc tgcagcgtat gccaaccgag tttgacgtcg 2820
   ctatggcaac cgtgtactat ctggccgatc gtaacgtgag cggcgaaact ttccatccgt 2880
   ctggtggttt gcgctacgag cgtaccccga ccggtggcga gctgttcggc ctgccatcgc 2940
   cggaacgtct ggcggagctg gttggtagca cggtgtacct gatcggtgaa cacctgaccg 3000
   agcacctgaa cctgctggct cgtgcctatt tggagcgcta cggtgcccgt caagtggtga 3060
   tgattgttga gacggaaacc ggtgcggaaa ccatgcgtcg tctgttgcat gatcacgtcg 3120
   aggcaggtcg cctgatgact attgtggcag gtgatcagat tgaggcagcg attgaccaag 3180
   cgatcacgcg ctatggccgt ccgggtccgg tggtgtgcac tccattccgt ccactgccaa 3240
   ccgttccgct ggtcggtcgt aaagactccg attggagcac cgttttgagc gaggcggaat 3300
   ttgcggaact gtgtgagcat cagctgaccc accatttccg tgttgctcgt aagatcgcct 3360
   tgtcggatgg cgcgtcgctg gcgttggtta ccccggaaac gactgcgact agcaccacgg 3420
   agcaatttgc tctggcgaac ttcatcaaga ccaccctgca cgcgttcacc gcgaccatcg 3480
   gtgttgagtc ggagcgcacc gcgcaacgta ttctgattaa ccaggttgat ctgacgcgcc 3540
   gcgcccgtgc ggaagagccg cgtgacccgc acgagcgtca gcaggaattg gaacgcttca 3600
   ttgaagccgt tctgctggtt accgctccgc tgcctcctga ggcagacacg cgctacgcag 3660
   gccgtattca ccgcggtcgt gcgattaccg tctaatagaa gcttggctgt tttggcggat 3720
   gagagaagat tttcagcctg atacagatta aatcagaacg cagaagcggt ctgataaaac 3780
   agaatttgcc tggcggcagt agcgcggtgg tcccacctga ccccatgccg aactcagaag 3840
   tgaaacgccg tagcgccgat ggtagtgtgg ggtctcccca tgcgagagta gggaactgcc 3900
   aggcatcaaa taaaacgaaa ggctcagtcg aaagactggg cctttcgttt tatctgttgt 3960
   ttgtcggtga acgctctcct gagtaggaca aatccgccgg gagcggattt gaacgttgcg 4020
   aagcaacggc ccggagggtg gcgggcagga cgcccgccat aaactgccag gcatcaaatt 4080
   aagcagaagg ccatcctgac ggatggcctt tttgcgtttc tacaaactct tttgtttatt 4140
   tttctaaata cattcaaata tgtatccgct catgagacaa taaccctgat aaatgcttca 4200
   ataatattga aaaaggaaga gtatgagtat tcaacatttc cgtgtcgccc ttattccctt 4260
   ttttgcggca ttttgccttc ctgtttttgc tcacccagaa acgctggtga aagtaaaaga 4320
   tgctgaagat cagttgggtg cacgagtggg ttacatcgaa ctggatctca acagcggtaa 4380
   gatccttgag agttttcgcc ccgaagaacg ttttccaatg atgagcactt ttaaagttct 4440
   gctatgtggc gcggtattat cccgtgttga cgccgggcaa gagcaactcg gtcgccgcat 4500
   acactattct cagaatgact tggttgagta ctcaccagtc acagaaaagc atcttacgga 4560
   tggcatgaca gtaagagaat tatgcagtgc tgccataacc atgagtgata acactgcggc 4620
   caacttactt ctgacaacga tcggaggacc gaaggagcta accgcttttt tgcacaacat 4680
   gggggatcat gtaactcgcc ttgatcgttg ggaaccggag ctgaatgaag ccataccaaa 4740
   cgacgagcgt gacaccacga tgctgtagca atggcaacaa cgttgcgcaa actattaact 4800
   ggcgaactac ttactctagc ttcccggcaa caattaatag actggatgga ggcggataaa 4860
   gttgcaggac cacttctgcg ctcggccctt ccggctggct ggtttattgc tgataaatct 4920
   ggagccggtg agcgtgggtc tcgcggtatc attgcagcac tggggccaga tggtaagccc 4980
   tcccgtatcg tagttatcta cacgacgggg agtcaggcaa ctatggatga acgaaataga 5040
   cagatcgctg agataggtgc ctcactgatt aagcattggt aactgtcaga ccaagtttac 5100
   tcatatatac tttagattga tttaaaactt catttttaat ttaaaaggat ctaggtgaag 5160
   atcctttttg ataatctcat gaccaaaatc ccttaacgtg agttttcgtt ccactgagcg 5220
   tcagaccccg tagaaaagat caaaggatct tcttgagatc ctttttttct gcgcgtaatc 5280
   tgctgcttgc aaacaaaaaa accaccgcta ccagcggtgg tttgtttgcc ggatcaagag 5340
   ctaccaactc tttttccgaa ggtaactggc ttcagcagag cgcagatacc aaatactgtc 5400
   cttctagtgt agccgtagtt aggccaccac ttcaagaact ctgtagcacc gcctacatac 5460
   ctcgctctgc taatcctgtt accagtggct gctgccagtg gcgataagtc gtgtcttacc 5520
   gggttggact caagacgata gttaccggat aaggcgcagc ggtcgggctg aacggggggt 5580
   tcgtgcacac agcccagctt ggagcgaacg acctacaccg aactgagata cctacagcgt 5640
   gagcattgag aaagcgccac gcttcccgaa gggagaaagg cggacaggta tccggtaagc 5700
   ggcagggtcg gaacaggaga gcgcacgagg gagcttccag ggggaaacgc ctggtatctt 5760
   tatagtcctg tcgggtttcg ccacctctga cttgagcgtc gatttttgtg atgctcgtca 5820
   ggggggcgga gcctatggaa aaacgccagc aacgcggcct ttttacggtt cctggccttt 5880
   tgctggcctt ttgctcacat gttctttcct gcgttatccc ctgattctgt ggataaccgt 5940
   attaccgcct ttgagtgagc tgataccgct cgccgcagcc gaacgaccga gcgcagcgag 6000
   tcagtgagcg aggaagcgga agagcgcctg atgcggtatt ttctccttac gcatctgtgc 6060
   ggtatttcac accgcatatg gtgcactctc agtacaatct gctctgatgc cgcatagtta 6120
   agccagtata cactccgcta tcgctacgtg actgggtcat ggctgcgccc cgacacccgc 6180
   caacacccgc tgacgcgccc tgacgggctt gtctgctccc ggcatccgct tacagacaag 6240
   ctgtgaccgt ctccgggagc tgcatgtgtc agaggttttc accgtcatca ccgaaacgcg 6300
   cgaggcagct gcggtaaagc tcatcagcgt ggtcgtgaag cgattcacag atgtctgcct 6360
   gttcatccgc gtccagctcg ttgagtttct ccagaagcgt taatgtctgg cttctgataa 6420
   agcgggccat gttaagggcg gttttttcct gtttggtcac tgatgcctcc gtgtaagggg 6480
   gatttctgtt catgggggta atgataccga tgaaacgaga gaggatgctc acgatacggg 6540
   ttactgatga tgaacatgcc cggttactgg aacgttgtga gggtaaacaa ctggcggtat 6600
   ggatgcggcg ggaccagaga aaaatcactc agggtcaatg ccagcgcttc gttaatacag 6660
   atgtaggtgt tccacagggt agccagcagc atcctgcgat gcagatccgg aacataatgg 6720
   tgcagggcgc tgacttccgc gtttccagac tttacgaaac acggaaaccg aagaccattc 6780
   atgttgttgc tcaggtcgca gacgttttgc agcagcagtc gcttcacgtt cgctcgcgta 6840
   tcggtgattc attctgctaa ccagtaaggc aaccccgcca gcctagccgg gtcctcaacg 6900
   acaggagcac gatcatgcgc acccgtggcc aggacccaac gctgcccgag atgcgccgcg 6960
   tgcggctgct ggagatggcg gacgcgatgg atatgttctg ccaagggttg gtttgcgcat 7020
   tcacagttct ccgcaagaat tgattggctc caattcttgg agtggtgaat ccgttagcga 7080
   ggtgccgccg gcttccattc aggtcgaggt ggcccggctc catgcaccgc gacgcaacgc 7140
   ggggaggcag acaaggtata gggcggcgcc tacaatccat gccaacccgt tccatgtgct 7200
   cgccgaggcg gcataaatcg ccgtgacgat cagcggtcca gtgatcgaag ttaggctggt 7260
   aagagccgcg agcgatcctt gaagctgtcc ctgatggtcg tcatctacct gcctggacag 7320
   catggcctgc aacgcgggca tcccgatgcc gccggaagcg agaagaatca taatggggaa 7380
   ggccatccag cctcgcgtcg cgaacgccag caagacgtag cccagcgcgt cggccgccat 7440
   gccggcgata atggcctgct tctcgccgaa acgtttggtg gcgggaccag tgacgaaggc 7500
   ttgagcgagg gcgtgcaaga ttccgaatac cgcaagcgac aggccgatca tcgtcgcgct 7560
   ccagcgaaag cggtcctcgc cgaaaatgac ccagagcgct gccggcacct gtcctacgag 7620
   ttgcatgata aagaagacag tcataagtgc ggcgacgata gtcatgcccc gcgcccaccg 7680
   gaaggagctg actgggttga aggctctcaa gggcatcggt cgacgctctc ccttatgcga 7740
   ctcctgcatt aggaagcagc ccagtagtag gttgaggccg ttgagcaccg ccgccgcaag 7800
   gaatggtgca tgcaaggaga tggcgcccaa cagtcccccg gccacggggc ctgccaccat 7860
   acccacgccg aaacaagcgc tcatgagccc gaagtggcga gcccgatctt ccccatcggt 7920
   gatgtcggcg atataggcgc cagcaaccgc acctgtggcg ccggtgatgc cggccacgat 7980
   gcgtccggcg tagaggatcc gggcttatcg actgcacggt gcaccaatgc ttctggcgtc 8040
   aggcagccat cggaagctgt ggtatggctg tgcaggtcgt aaatcactgc ataattcgtg 8100
   tcgctcaagg cgcactcccg ttctggataa tgttttttgc gccgacatca taacggttct 8160
   ggcaaatatt ctgaaatgag ctgttgacaa ttaatcatcg gctcgtataa tgtgtggaat 8220
   tgtgagcgga taacaatttc acacaggaaa ca 8252
<210> 17
   <211> 7988
   <212> DNA
   <213> Artificial Sequence
<220>
   <223> Description of Artificial Sequence: Synthetic polynucleotide
<400> 17
   ctcgagggta actagcctcg ccgatcccgc aagaggcccg gcagtcaggt ggcacttttc 60
   ggggaaatgt gcgcggaacc cctatttgtt tatttttcta aatacattca aatatgtatc 120
   cgctcatgag acaataaccc tgataaatgc ttcaataata ttgaaaaagg aagagtatga 180
   gtattcaaca tttccgtgtc gcccttattc ccttttttgc ggcattttgc cttcctgttt 240
   ttgctcaccc agaaacgctg gtgaaagtaa aagatgctga agatcagttg ggtgcacgag 300
   tgggttacat cgaactggat ctcaacagcg gtaagatcct tgagagtttt cgccccgaag 360
   aacgttttcc aatgatgagc acttttaaag ttctgctatg tggcgcggta ttatcccgta 420
   ttgacgccgg gcaagagcaa ctcggtcgcc gcatacacta ttctcagaat gacttggttg 480
   agtactcacc agtcacagaa aagcatctta cggatggcat gacagtaaga gaattatgca 540
   gtgctgccat aaccatgagt gataacactg cggccaactt acttctgaca acgatcggag 600
   gaccgaagga gctaaccgct tttttgcaca acatggggga tcatgtaact cgccttgatc 660
   gttgggaacc ggagctgaat gaagccatac caaacgacga gcgtgacacc acgatgcctg 720
   tagcaatggc aacaacgttg cgcaaactat taactggcga actacttact ctagcttccc 780
   ggcaacaatt aatagactgg atggaggcgg ataaagttgc aggaccactt ctgcgctcgg 840
   cccttccggc tggctggttt attgctgata aatctggagc cggtgagcgt gggtctcgcg 900
   gtatcattgc agcactgggg ccagatggta agccctcccg tatcgtagtt atctacacga 960
   cggggagtca ggcaactatg gatgaacgaa atagacagat cgctgagata ggtgcctcac 1020
   tgattaagca ttggtaactg tcagaccaag tttactcata tatactttag attgatttaa 1080
   aacttcattt ttaatttaaa aggatctagg tgaagatcct ttttgataat ctcatgacca 1140
   aaatccctta acgtgagttt tcgttccact gagcgtcaga ccccgtagaa aagatcaaag 1200
   gatcttcttg agatcctttt tttctgcgcg taatctgctg cttgcaaaca aaaaaaccac 1260
   cgctaccagc ggtggtttgt ttgccggatc aagagctacc aactcttttt ccgaaggtaa 1320
   ctggcttcag cagagcgcag ataccaaata ctgtccttct agtgtagccg tagttaggcc 1380
   accacttcaa gaactctgta gcaccgccta catacctcgc tctgctaatc ctgttaccag 1440
   tggctgctgc cagtggcgat aagtcgtgtc ttaccgggtt ggactcaaga cgatagttac 1500
   cggataaggc gcagcggtcg ggctgaacgg ggggttcgtg cacacagccc agcttggagc 1560
   gaacgaccta caccgaactg agatacctac agcgtgagct atgagaaagc gccacgcttc 1620
   ccgaagggag aaaggcggac aggtatccgg taagcggcag ggtcggaaca ggagagcgca 1680
   cgagggagct tccaggggga aacgcctggt atctttatag tcctgtcggg tttcgccacc 1740
   tctgacttga gcgtcgattt ttgtgatgct cgtcaggggg gcggagccta tggaaaaacg 1800
   ccagcaacgc ggccttttta cggttcctgg ccttttgctg gccttttgct cacatgttct 1860
   ttcctgcgtt atcccctgat tctgtggata accgtattac cgcctttgag tgagctgata 1920
   ccgctcgccg cagccgaacg accgagcgca gcgagtcagt gagcgaggaa gcggaagagc 1980
   gcccaatacg catgcttaag ttattggtat gactggtttt aagcgcaaaa aaagttgctt 2040
   tttcgtacct attaatgtat cgttttagaa aaccgactgt aaaaagtaca gtcggcatta 2100
   tctcatatta taaaagccag tcattaggcc tatctgacaa ttcctgaata gagttcataa 2160
   acaatcctgc atgataacca tcacaaacag aatgatgtac ctgtaaagat agcggtaaat 2220
   atattgaatt acctttatta atgaattttc ctgctgtaat aatgggtaga aggtaattac 2280
   tattattatt gatatttaag ttaaacccag taaatgaagt ccatggaata atagaaagag 2340
   aaaaagcatt ttcaggtata ggtgttttgg gaaacaattt ccccgaacca ttatatttct 2400
   ctacatcaga aaggtataaa tcataaaact ctttgaagtc attctttaca ggagtccaaa 2460
   taccagagaa tgttttagat acaccatcaa aaattgtata aagtggctct aacttatccc 2520
   aataacctaa ctctccgtcg ctattgtaac cagttctaaa agctgtattt gagtttatca 2580
   cccttgtcac taagaaaata aatgcagggt aaaatttata tccttcttgt tttatgtttc 2640
   ggtataaaac actaatatca atttctgtgg ttatactaaa agtcgtttgt tggttcaaat 2700
   aatgattaaa tatctctttt ctcttccaat tgtctaaatc aattttatta aagttcattt 2760
   gatatgcctc ctaaattttt atctaaagtg aatttaggag gcttacttgt ctgctttctt 2820
   cattagaatc aatccttttt taaaagtcaa tattactgta acataaatat atattttaaa 2880
   aatatcccac tttatccaat tttcgtttgt tgaactaatg ggtgctttag ttgaagaata 2940
   aagaccacat taaaaaatgt ggtcttttgt gtttttttaa aggatttgag cgtagcgaaa 3000
   aatccttttc tttcttatct tgataataag ggtaactatt gccgatcgtc cattccgaca 3060
   gcatcgccag tcactatggc gtgctgctag cgccattcgc cattcaggct gcgcaactgt 3120
   tgggaagggc gatcggtgcg ggcctcttcg ctattacgcc agctggcgaa agggggatgt 3180
   gctgcaaggc gattaagttg ggtaacgcca gggttttccc agtcacgacg ttgtaaaacg 3240
   acggccagtg aattcgagct caggccttaa ctcacattaa ttgcgttgcg ctcactgccc 3300
   gctttccagt cgggaaacct gtcgtgccag ctgcattaat gaatcggcca acgcgcgggg 3360
   agaggcggtt tgcgtattgg gcgccagggt ggtttttctt ttcaccagtg agacgggcaa 3420
   cagctgattg cccttcaccg cctggccctg agagagttgc agcaagcggt ccacgctggt 3480
   ttgccccagc aggcgaaaat cctgtttgat ggtggttgac ggcgggatat aacatgagct 3540
   gtcttcggta tcgtcgtatc ccactaccga gatatccgca ccaacgcgca gcccggactc 3600
   ggtaatggcg cgcattgcgc ccagcgccat ctgatcgttg gcaaccagca tcgcagtggg 3660
   aacgatgccc tcattcagca tttgcatggt ttgttgaaaa ccggacatgg cactccagtc 3720
   gccttcccgt tccgctatcg gctgaatttg attgcgagtg agatatttat gccagccagc 3780
   cagacgcaga cgcgccgaga cagaacttaa tgggcccgct aacagcgcga tttgctggtg 3840
   acccaatgcg accagatgct ccacgcccag tcgcgtaccg tcttcatggg agaaaataat 3900
   actgttgatg ggtgtctggt cagagacatc aagaaataac gccggaacat tagtgcaggc 3960
   agcttccaca gcaatggcat cctggtcatc cagcggatag ttaatgatca gcccactgac 4020
   gcgttgcgcg agaagattgt gcaccgccgc tttacaggct tcgacgccgc ttcgttctac 4080
   catcgacacc accacgctgg cacccagttg atcggcgcga gatttaatcg ccgcgacaat 4140
   ttgcgacggc gcgtgcaggg ccagactgga ggtggcaacg ccaatcagca acgactgttt 4200
   gcccgccagt tgttgtgcca cgcggttggg aatgtaattc agctccgcca tcgccgcttc 4260
   cacttttccc gcgtttgcag aaacgtggct ggcctggttc accacgcggg aaacggtctg 4320
   ataagagaca ccggcatact ctgcgacatc gtataacgtt actggtttca tcaaaatcgt 4380
   ctccctccgt ttgaatattt gattgatcgt aaccagatga agcactcttt ccactatccc 4440
   tacagtgtta tggcttgaac aatcacgaaa caataattgg tacgtacgat ctttcagccg 4500
   actcaaacat caaatcttac aaatgtagtc tttgaaagta ttacatatgt aagatttaaa 4560
   tgcaaccgtt ttttcggaag gaaatgatga cctcgtttcc accggaatta gcttggtacc 4620
   agctattgta acataatcgg tacgggggtg aaaaagctaa cggaaaaggg agcggaaaag 4680
   aatgatgtaa gcgtgaaaaa tttttatctt atcacttgaa aattggaagg gagattcttt 4740
   attataagaa ttgtggaatt gtgagcggat aacaattccc aattaaagga ggaaggatct 4800
   atgcgcggaa gccatcacca tcaccatcac catcacggat cctctagagt cgacgtcccc 4860
   ggggcagccc gcctaatgag cgggcttttt tcacgtcacg cgtccatgga gatctttgtc 4920
   tgcaactgaa aagtttatac cttacctgga acaaatggtt gaaacatacg aggctaatat 4980
   cggcttatta ggaatagtcc ctgtactaat aaaatcaggt ggatcagttg atcagtatat 5040
   tttggacgaa gctcggaaag aatttggaga tgacttgctt aattccacaa ttaaattaag 5100
   ggaaagaata aagcgatttg atgttcaagg aatcacggaa gaagatactc atgataaaga 5160
   agctctaaac tattcataac cttacatgga attgatcgaa gggtggaagg ttaatggtac 5220
   gaaattaggg gatctaccta gaaagcacaa ggcgataggt caagcttaaa gaacccttac 5280
   atggatctta cagattctga aagtaaagaa acaacagagg ttaaacaaac agaaccaaaa 5340
   agaaaaaaag cattgttgaa aacaatgaaa gttgatgttt caatccataa taagattaaa 5400
   tcgctgcacg aaattctggc agcatccgaa gggaattcat attacttaga ggatactatt 5460
   gagagagcta ttgataagat ggttgagaca ttacctgaga gccaaaaaac tttttatgaa 5520
   tatgaattaa aaaaaagaac caacaaaggc tgagacagac tccaaacgag tctgtttttt 5580
   taaaaaaaat attaggagca ttgaatatat attagagaat taagaaagac atgggaataa 5640
   aaatatttta aatccagtaa aaatatgata agattatttc agaatatgaa gaactctgtt 5700
   tgtttttgat gaaaaaacaa acaaaaaaaa tccacctaac ggaatctcaa tttaactaac 5760
   agcggccaaa ctgagaagtt aaatttgaga aggggaaaag gcggatttat acttgtattt 5820
   aactatctcc attttaacat tttattaaac cccatacaag tgaaaatcct cttttacact 5880
   gttcctttag gtgatcgcgg agggacatta tgagtgaagt aaacctaaaa ggaaatacag 5940
   atgaattagt gtattatcga cagcaaacca ctggaaataa aatcgccagg aagagaatca 6000
   aaaaagggaa agaagaagtt tattatgttg ctgaaacgga agagaagata tggacagaag 6060
   agcaaataaa aaacttttct ttagacaaat ttggtacgca tataccttac atagaaggtc 6120
   attatacaat cttaaataat tacttctttg atttttgggg ctatttttta ggtgctgaag 6180
   gaattgcgct ctatgctcac ctaactcgtt atgcatacgg cagcaaagac ttttgctttc 6240
   ctagtctaca aacaatcgct aaaaaaatgg acaagactcc tgttacagtt agaggctact 6300
   tgaaactgct tgaaaggtac ggttttattt ggaaggtaaa cgtccgtaat aaaaccaagg 6360
   ataacacaga ggaatccccg atttttaaga ttagacgtaa ggttcctttg ctttcagaag 6420
   aacttttaaa tggaaaccct aatattgaaa ttccagatga cgaggaagca catgtaaaga 6480
   aggctttaaa aaaggaaaaa gagggtcttc caaaggtttt gaaaaaagag cacgatgaat 6540
   ttgttaaaaa aatgatggat gagtcagaaa caattaatat tccagaggcc ttacaatatg 6600
   acacaatgta tgaagatata ctcagtaaag gagaaattcg aaaagaaatc aaaaaacaaa 6660
   tacctaatcc tacaacatct tttgagagta tatcaatgac aactgaagag gaaaaagtcg 6720
   acagtacttt aaaaagcgaa atgcaaaatc gtgtctctaa gccttctttt gatacctggt 6780
   ttaaaaacac taagatcaaa attgaaaata aaaattgttt attacttgta ccgagtgaat 6840
   ttgcatttga atggattaag aaaagatatt tagaaacaat taaaacagtc cttgaagaag 6900
   ctggatatgt tttcgaaaaa atcgaactaa gaaaagtgca ataaactgct gaagtatttc 6960
   agcagttttt tttatttaga aatagtgaaa aaaatataat cagggaggta tcaatattta 7020
   atgagtactg atttaaattt atttagactg gaattaataa ttaacacgta gactaattaa 7080
   aatttaatga gggataaaga ggatacaaaa atattaattt caatccctat taaattttaa 7140
   caaggggggg attaaaattt aattagaggt ttatccacaa gaaaagaccc taataaaatt 7200
   tttactaggg ttataacact gattaatttc ttaatggggg agggattaaa atttaatgac 7260
   aaagaaaaca atcttttaag aaaagctttt aaaagataat aataaaaaga gctttgcgat 7320
   taagcaaaac tctttacttt ttcattgaca ttatcaaatt catcgatttc aaattgttgt 7380
   tgtatcataa agttaattct gttttgcaca accttttcag gaatataaaa cacatctgag 7440
   gcttgtttta taaactcagg gtcgctaaag tcaatgtaac gtagcatatg atatggtata 7500
   gcttccaccc aagttagcct ttctgcttct tctgaatgtt tttcatatac ttccatgggt 7560
   atctctaaat gattttcctc atgtagcaag gtatgagcaa aaagtttatg gaattgatag 7620
   ttcctctctt tttcttcaac ttttttatct aaaacaaaca ctttaacatc tgagtcaatg 7680
   taagcataag atgtttttcc agtcataatt tcaatcccaa atcttttaga cagaaattct 7740
   ggacgtaaat cttttggtga aagaattttt ttatgtagca atatatccga tacagcacct 7800
   tctaaaagcg ttggtgaata gggcatttta cctatctcct ctcattttgt ggaataaaaa 7860
   tagtcatatt cgtccatcta cctatcctat tatcgaacag ttgaactttt taatcaagga 7920
   tcagtccttt ttttcattat tcttaaactg tgctcttaac tttaacaact cgatttgttt 7980
   ttccagat 7988
<210> 18
   <211> 27
   <212> DNA
   <213> Artificial Sequence
<220>
   <223> Description of Artificial Sequence: Synthetic primer
<400> 18
   ggaaggatcc atgtccggta cgggtcg 27
<210> 19
   <211> 26
   <212> DNA
   <213> Artificial Sequence
<220>
   <223> Description of Artificial Sequence: Synthetic primer
<400> 19
   gggattagac ggtaatcgca cgaccg 26
<210> 20
   <211> 7794
   <212> DNA
   <213> Artificial Sequence
<220>
   <223> Description of Artificial Sequence: Synthetic polynucleotide
<400> 20
   ggtggcggta cttgggtcga tatcaaagtg catcacttct tcccgtatgc ccaactttgt 60
   atagagagcc actgcgggat cgtcaccgta atctgcttgc acgtagatca cataagcacc 120
   aagcgcgttg gcctcatgct tgaggagatt gatgagcgcg gtggcaatgc cctgcctccg 180
   gtgctcgccg gagactgcga gatcatagat atagatctca ctacgcggct gctcaaactt 240
   gggcagaacg taagccgcga gagcgccaac aaccgcttct tggtcgaagg cagcaagcgc 300
   gatgaatgtc ttactacgga gcaagttccc gaggtaatcg gagtccggct gatgttggga 360
   gtaggtggct acgtcaccga actcacgacc gaaaagatca agagcagccc gcatggattt 420
   gacttggtca gggccgagcc tacatgtgcg aatgatgccc atacttgagc cacctaactt 480
   tgttttaggg cgactgccct gctgcgtaac atcgttgctg ctccataaca tcaaacatcg 540
   acccacggcg taacgcgctt gctgcttgga tgcccgaggc atagactgta caaaaaaaca 600
   gtcataacaa gccatgaaaa ccgccactgc gccgttacca ccgctgcgtt cggtcaaggt 660
   tctggaccag ttgcgtgagc gcattttttt ttcctcctcg gcgtttacgc cccgccctgc 720
   cactcatcgc agtactgttg taattcatta agcattctgc cgacatggaa gccatcacag 780
   acggcatgat gaacctgaat cgccagcggc atcagcacct tgtcgccttg cgtataatat 840
   ttgcccatag tgaaaacggg ggcgaagaag ttgtccatat tggccacgtt taaatcaaaa 900
   ctggtgaaac tcacccaggg attggcgctg acgaaaaaca tattctcaat aaacccttta 960
   gggaaatagg ccaggttttc accgtaacac gccacatctt gcgaatatat gtgtagaaac 1020
   tgccggaaat cgtcgtggta ttcactccag agcgatgaaa acgtttcagt ttgctcatgg 1080
   aaaacggtgt aacaagggtg aacactatcc catatcacca gctcaccgtc tttcattgcc 1140
   atacggaact ccggatgagc attcatcagg cgggcaagaa tgtgaataaa ggccggataa 1200
   aacttgtgct tatttttctt tacggtcttt aaaaaggccg taatatccag ctgaacggtc 1260
   tggttatagg tacattgagc aactgactga aatgcctcaa aatgttcttt acgatgccat 1320
   tgggatatat caacggtggt atatccagtg atttttttct ccattttttt ttcctccttt 1380
   agaaaaactc atcgagcatc aaatgaaact gcaatttatt catatcagga ttatcaatac 1440
   catatttttg aaaaagccgt ttctgtaatg aaggagaaaa ctcaccgagg cagttccata 1500
   ggatggcaag atcctggtat cggtctgcga ttccgactcg tccaacatca atacaaccta 1560
   ttaatttccc ctcgtcaaaa ataaggttat caagtgagaa atcaccatga gtgacgactg 1620
   aatccggtga gaatggcaaa agtttatgca tttctttcca gacttgttca acaggccagc 1680
   cattacgctc gtcatcaaaa tcactcgcat caaccaaacc gttattcatt cgtgattgcg 1740
   cctgagcgag gcgaaatacg cgatcgctgt taaaaggaca attacaaaca ggaatcgagt 1800
   gcaaccggcg caggaacact gccagcgcat caacaatatt ttcacctgaa tcaggatatt 1860
   cttctaatac ctggaacgct gtttttccgg ggatcgcagt ggtgagtaac catgcatcat 1920
   caggagtacg gataaaatgc ttgatggtcg gaagtggcat aaattccgtc agccagttta 1980
   gtctgaccat ctcatctgta acatcattgg caacgctacc tttgccatgt ttcagaaaca 2040
   actctggcgc atcgggcttc ccatacaagc gatagattgt cgcacctgat tgcccgacat 2100
   tatcgcgagc ccatttatac ccatataaat cagcatccat gttggaattt aatcgcggcc 2160
   tcgacgtttc ccgttgaata tggctcattt ttttttcctc ctttaccaat gcttaatcag 2220
   tgaggcacct atctcagcga tctgtctatt tcgttcatcc atagttgcct gactccccgt 2280
   cgtgtagata actacgatac gggagggctt accatctggc cccagcgctg cgatgatacc 2340
   gcgagaacca cgctcaccgg ctccggattt atcagcaata aaccagccag ccggaagggc 2400
   cgagcgcaga agtggtcctg caactttatc cgcctccatc cagtctatta attgttgccg 2460
   ggaagctaga gtaagtagtt cgccagttaa tagtttgcgc aacgttgttg ccatcgctac 2520
   aggcatcgtg gtgtcacgct cgtcgtttgg tatggcttca ttcagctccg gttcccaacg 2580
   atcaaggcga gttacatgat cccccatgtt gtgcaaaaaa gcggttagct ccttcggtcc 2640
   tccgatcgtt gtcagaagta agttggccgc agtgttatca ctcatggtta tggcagcact 2700
   gcataattct cttactgtca tgccatccgt aagatgcttt tctgtgactg gtgagtactc 2760
   aaccaagtca ttctgagaat agtgtatgcg gcgaccgagt tgctcttgcc cggcgtcaat 2820
   acgggataat accgcgccac atagcagaac tttaaaagtg ctcatcattg gaaaacgttc 2880
   ttcggggcga aaactctcaa ggatcttacc gctgttgaga tccagttcga tgtaacccac 2940
   tcgtgcaccc aactgatctt cagcatcttt tactttcacc agcgtttctg ggtgagcaaa 3000
   aacaggaagg caaaatgccg caaaaaaggg aataagggcg acacggaaat gttgaatact 3060
   catattcttc ctttttcaat attattgaag catttatcag ggttattgtc tcatgagcgg 3120
   atacatattt gaatgtattt agaaaaataa acaaataggg gtcagtgtta caaccaatta 3180
   accaattctg aacattatcg cgagcccatt tatacctgaa tatggctcat aacacccctt 3240
   gtttgcctgg cggcagtagc gcggtggtcc cacctgaccc catgccgaac tcagaagtga 3300
   aacgccgtag cgccgatggt agtgtgggga ctccccatgc gagagtaggg aactgccagg 3360
   catcaaataa aacgaaaggc tcagtcgaaa gactgggcct ttcgcccggg ctaattgagg 3420
   ggtgtcgccc ttattcgact ctatagtgaa gttcctattc tctagaaagt ataggaactt 3480
   ctgaagtggg gtttaaactc cctctgccct tccctcccgc ttcatcctta tttttggaca 3540
   ataaactaga gaacaatttg aacttgaatt ggaattcaga ttcagagcaa gagacaagaa 3600
   acttcccttt ttcttctcca catattatta tttattcgtg tattttcttt taacgatacg 3660
   atacgatacg acacgatacg atacgacacg ctactataca gtgacgtcag attgtactga 3720
   gagtgcagat tgtactgaga gtgcaccata aattcccgtt ttaagagctt ggtgagcgct 3780
   aggagtcact gccaggtatc gtttgaacac ggcattagtc agggaagtca taacacagtc 3840
   ctttcccgca attttctttt tctattactc ttggcctcct ctagtacact ctatattttt 3900
   ttatgcctcg gtaatgattt tcattttttt ttttccccta gcggatgact cttttttttt 3960
   cttagcgatt ggcattatca cataatgaat tatacattat ataaagtaat gtgatttctt 4020
   cgaagaatat actaaaaaat gagcaggcaa gataaacgaa ggcaaagatg acagagcaga 4080
   aagccctagt aaagcgtatt acaaatgaaa ccaagattca gattgcgatc tctttaaagg 4140
   gtggtcccct agcgatagag cactcgatct tcccagaaaa agaggcagaa gcagtagcag 4200
   aacaggccac acaatcgcaa gtgattaacg tccacacagg tatagggttt ctggaccata 4260
   tgatacatgc tctggccaag cattccggct ggtcgctaat cgttgagtgc attggtgact 4320
   tacacataga cgaccatcac accactgaag actgcgggat tgctctcggt caagctttta 4380
   aagaggccct actggcgcgt ggagtaaaaa ggtttggatc aggatttgcg cctttggatg 4440
   aggcactttc cagagcggtg gtagatcttt cgaacaggcc gtacgcagtt gtcgaacttg 4500
   gtttgcaaag ggagaaagta ggagatctct cttgcgagat gatcccgcat tttcttgaaa 4560
   gctttgcaga ggctagcaga attaccctcc acgttgattg tctgcgaggc aagaatgatc 4620
   atcaccgtag tgagagtgcg ttcaaggctc ttgcggttgc cataagagaa gccacctcgc 4680
   ccaatggtac caacgatgtt ccctccacca aaggtgttct tatgtagtga caccgattat 4740
   ttaaagctgc agcatacgat atatatacat gtgtatatat gtatacctat gaatgtcagt 4800
   aagtatgtat acgaacagta tgatactgaa gatgacaagg taatgcatca ttctatacgt 4860
   gtcattctga acgaggcgcg ctttcctttt ttctttttgc tttttctttt tttttctctt 4920
   gaactcgacg gatctatgcg gtgtgaaata ccgcacaggt gtgaaatacc gcacagtcat 4980
   gagatccgat aacttctttt cttttttttt cttttctctc tcccccgttg ttgtctcacc 5040
   atatccgcaa tgacaaaaaa aatgatggaa gacactaaag gaaaaaatta acgacaaaga 5100
   cagcaccaac agatgtcgtt gttccagagc tgatgagggg tatcttcgaa cacacgaaac 5160
   tttttccttc cttcattcac gcacactact ctctaatgag caacggtata cggccttcct 5220
   tccagttact tgaatttgaa ataaaaaaag tttgccgctt tgctatcaag tataaataga 5280
   cctgcaatta ttaatctttt gtttcctcgt cattgttctc gttccctttc ttccttgttt 5340
   ctttttctgc acaatatttc aagctatacc aagcatacaa tcaactccaa cggatccgaa 5400
   tactagttgg ccaatcatgt aattagttat gtcacgctta cattcacgcc ctccccccac 5460
   atccgctcta accgaaaagg aaggagttag acaacctgaa gtctaggtcc ctatttattt 5520
   ttttatagtt atgttagtat taagaacgtt atttatattt caaatttttc ttttttttct 5580
   gtacagacgc gtgtacgcat gtaacattat actgaaaacc ttgcttgaga aggttttggg 5640
   acgctcgaag gctttaattt gcaagcttgg ccaccacaca ccatagcttc aaaatgtttc 5700
   tactcctttt ttactcttcc agattttctc ggactccgcg catcgccgta ccacttcaaa 5760
   acacccaagc acagcatact aaattttccc tctttcttcc tctagggtgt cgttaattac 5820
   ccgtactaaa ggtttggaaa agaaaaaaga gaccgcctcg tttctttttc ttcgtcgaaa 5880
   aaggcaataa aaatttttat cacgtttctt tttcttgaaa tttttttttt tagttttttt 5940
   ctctttcagt gacctccatt gatatttaag ttaataaacg gtcttcaatt tctcaagttt 6000
   cagtttcatt tttcttgttc tattacaact ttttttactt cttgttcatt agaaagaaag 6060
   catagcaatc taatctaagg gatgagcgaa gaaagcttat tcgagtcttc tccacagaag 6120
   atggagtacg aaattacaaa ctactcagaa agacatacag aacttccagg tcatttcatt 6180
   ggcctcaata cagtagataa actagaggag tccccgttaa gggactttgt taagagtcac 6240
   ggtggtcaca cggtcatatc caagatcctg atagcaaata agtttaaaca aaatgaagtg 6300
   aagttcctat actttctaga gaataggaac ttctatagtg agtcgaataa gggcgacaca 6360
   aaatttattc taaatgcata ataaatactg ataacatctt atagtttgta ttatattttg 6420
   tattatcgtt gacatgtata attttgatat caaaaactga ttttcccttt attattttcg 6480
   agatttattt tcttaattct ctttaacaaa ctagaaatat tgtatataca aaaaatcata 6540
   aataatagat gaatagttta attataggtg ttcatcaatc gaaaaagcaa cgtatcttat 6600
   ttaaagtgcg ttgctttttt ctcatttata aggttaaata attctcatat atcaagcaaa 6660
   gtgacaggcg cccttaaata ttctgacaaa tgctctttcc ctaaactccc cccataaaaa 6720
   aacccgccga agcgggtttt tacgttattt gcggattaac gattactcgt tatcagaacc 6780
   gcccaggggg cccgagctta agactggccg tcgttttaca acacagaaag agtttgtaga 6840
   aacgcaaaaa ggccatccgt caggggcctt ctgcttagtt tgatgcctgg cagttcccta 6900
   ctctcgcctt ccgcttcctc gctcactgac tcgctgcgct cggtcgttcg gctgcggcga 6960
   gcggtatcag ctcactcaaa ggcggtaata cggttatcca cagaatcagg ggataacgca 7020
   ggaaagaaca tgtgagcaaa aggccagcaa aaggccagga accgtaaaaa ggccgcgttg 7080
   ctggcgtttt tccataggct ccgcccccct gacgagcatc acaaaaatcg acgctcaagt 7140
   cagaggtggc gaaacccgac aggactataa agataccagg cgtttccccc tggaagctcc 7200
   ctcgtgcgct ctcctgttcc gaccctgccg cttaccggat acctgtccgc ctttctccct 7260
   tcgggaagcg tggcgctttc tcatagctca cgctgtaggt atctcagttc ggtgtaggtc 7320
   gttcgctcca agctgggctg tgtgcacgaa ccccccgttc agcccgaccg ctgcgcctta 7380
   tccggtaact atcgtcttga gtccaacccg gtaagacacg acttatcgcc actggcagca 7440
   gccactggta acaggattag cagagcgagg tatgtaggcg gtgctacaga gttcttgaag 7500
   tggtgggcta actacggcta cactagaaga acagtatttg gtatctgcgc tctgctgaag 7560
   ccagttacct tcggaaaaag agttggtagc tcttgatccg gcaaacaaac caccgctggt 7620
   agcggtggtt tttttgtttg caagcagcag attacgcgca gaaaaaaagg atctcaagaa 7680
   gatcctttga tcttttctac ggggtctgac gctcagtgga acgacgcgcg cgtaactcac 7740
   gttaagggat tttggtcatg agcttgcgcc gtcccgtcaa gtcagcgtaa tgct 7794
<210> 21
   <211> 3710
   <212> DNA
   <213> Artificial Sequence
<220>
   <223> Description of Artificial Sequence: Synthetic polynucleotide
<400> 21
   gatcggatcc atggccggta cgggtcgttt ggctggtaaa attgcattga tcaccggtgg 60
   tgctggtaac attggttccg agctgacccg ccgttttctg gccgagggtg cgacggttat 120
   tatcagcggc cgtaaccgtg cgaagctgac cgcgctggcc gagcgcatgc aagccgaggc 180
   cggcgtgccg gccaagcgca ttgatttgga ggtgatggat ggttccgacc ctgtggctgt 240
   ccgtgccggt atcgaggcaa tcgtcgctcg ccacggtcag attgacattc tggttaacaa 300
   cgcgggctcc gccggtgccc aacgtcgctt ggcggaaatt ccgctgacgg aggcagaatt 360
   gggtccgggt gcggaggaga ctttgcacgc ttcgatcgcg aatctgttgg gcatgggttg 420
   gcacctgatg cgtattgcgg ctccgcacat gccagttggc tccgcagtta tcaacgtttc 480
   gactattttc tcgcgcgcag agtactatgg tcgcattccg tacgttaccc cgaaggcagc 540
   gctgaacgct ttgtcccagc tggctgcccg cgagctgggc gctcgtggca tccgcgttaa 600
   cactattttc ccaggtccta ttgagtccga ccgcatccgt accgtgtttc aacgtatgga 660
   tcaactgaag ggtcgcccgg agggcgacac cgcccatcac tttttgaaca ccatgcgcct 720
   gtgccgcgca aacgaccaag gcgctttgga acgccgcttt ccgtccgttg gcgatgttgc 780
   tgatgcggct gtgtttctgg cttctgctga gagcgcggca ctgtcgggtg agacgattga 840
   ggtcacccac ggtatggaac tgccggcgtg tagcgaaacc tccttgttgg cgcgtaccga 900
   tctgcgtacc atcgacgcga gcggtcgcac taccctgatt tgcgctggcg atcaaattga 960
   agaagttatg gccctgacgg gcatgctgcg tacgtgcggt agcgaagtga ttatcggctt 1020
   ccgttctgcg gctgccctgg cgcaatttga gcaggcagtg aatgaatctc gccgtctggc 1080
   aggtgcggat ttcaccccgc cgatcgcttt gccgttggac ccacgtgacc cggccaccat 1140
   tgatgcggtt ttcgattggg gcgcaggcga gaatacgggt ggcatccatg cggcggtcat 1200
   tctgccggca acctcccacg aaccggctcc gtgcgtgatt gaagtcgatg acgaacgcgt 1260
   cctgaatttc ctggccgatg aaattaccgg caccatcgtt attgcgagcc gtttggcgcg 1320
   ctattggcaa tcccaacgcc tgaccccggg tgcccgtgcc cgcggtccgc gtgttatctt 1380
   tctgagcaac ggtgccgatc aaaatggtaa tgtttacggt cgtattcaat ctgcggcgat 1440
   cggtcaattg attcgcgttt ggcgtcacga ggcggagttg gactatcaac gtgcatccgc 1500
   cgcaggcgat cacgttctgc cgccggtttg ggcgaaccag attgtccgtt tcgctaaccg 1560
   ctccctggaa ggtctggagt tcgcgtgcgc gtggaccgca cagctgctgc acagccaacg 1620
   tcatattaac gaaattacgc tgaacattcc agccaatatt agcgcgacca cgggcgcacg 1680
   ttccgccagc gtcggctggg ccgagtcctt gattggtctg cacctgggca aggtggctct 1740
   gattaccggt ggttcggcgg gcatcggtgg tcaaatcggt cgtctgctgg ccttgtctgg 1800
   cgcgcgtgtg atgctggccg ctcgcgatcg ccataaattg gaacagatgc aagccatgat 1860
   tcaaagcgaa ttggcggagg ttggttatac cgatgtggag gaccgtgtgc acatcgctcc 1920
   gggttgcgat gtgagcagcg aggcgcagct ggcagatctg gtggaacgta cgctgtccgc 1980
   attcggtacc gtggattatt tgattaataa cgccggtatt gcgggcgtgg aggagatggt 2040
   gatcgacatg ccggtggaag gctggcgtca caccctgttt gccaacctga tttcgaatta 2100
   ttcgctgatg cgcaagttgg cgccgctgat gaagaagcaa ggtagcggtt acatcctgaa 2160
   cgtttcttcc tattttggcg gtgagaagga cgcggcgatt ccttatccga accgcgccga 2220
   ctacgccgtc tccaaggctg gccaacgcgc gatggcggaa gtgttcgctc gtttcctggg 2280
   tccagagatt cagatcaatg ctattgcccc aggtccggtt gaaggcgacc gcctgcgtgg 2340
   taccggtgag cgtccgggcc tgtttgctcg tcgcgcccgt ctgatcttgg agaataaacg 2400
   cctgaacgaa ttgcacgcgg ctttgattgc tgcggcccgc accgatgagc gctcgatgca 2460
   cgagttggtt gaattgttgc tgccgaacga cgtggccgcg ttggagcaga acccagcggc 2520
   ccctaccgcg ctgcgtgagc tggcacgccg cttccgtagc gaaggtgatc cggcggcaag 2580
   ctcctcgtcc gccttgctga atcgctccat cgctgccaag ctgttggctc gcttgcataa 2640
   cggtggctat gtgctgccgg cggatatttt tgcaaatctg cctaatccgc cggacccgtt 2700
   ctttacccgt gcgcaaattg accgcgaagc tcgcaaggtg cgtgatggta ttatgggtat 2760
   gctgtatctg cagcgtatgc caaccgagtt tgacgtcgct atggcaaccg tgtactatct 2820
   ggccgatcgt aacgtgagcg gcgaaacttt ccatccgtct ggtggtttgc gctacgagcg 2880
   taccccgacc ggtggcgagc tgttcggcct gccatcgccg gaacgtctgg cggagctggt 2940
   tggtagcacg gtgtacctga tcggtgaaca cctgaccgag cacctgaacc tgctggctcg 3000
   tgcctatttg gagcgctacg gtgcccgtca agtggtgatg attgttgaga cggaaaccgg 3060
   tgcggaaacc atgcgtcgtc tgttgcatga tcacgtcgag gcaggtcgcc tgatgactat 3120
   tgtggcaggt gatcagattg aggcagcgat tgaccaagcg atcacgcgct atggccgtcc 3180
   gggtccggtg gtgtgcactc cattccgtcc actgccaacc gttccgctgg tcggtcgtaa 3240
   agactccgat tggagcaccg ttttgagcga ggcggaattt gcggaactgt gtgagcatca 3300
   gctgacccac catttccgtg ttgctcgtaa gatcgccttg tcggatggcg cgtcgctggc 3360
   gttggttacc ccggaaacga ctgcgactag caccacggag caatttgctc tggcgaactt 3420
   catcaagacc accctgcacg cgttcaccgc gaccatcggt gttgagtcgg agcgcaccgc 3480
   gcaacgtatt ctgattaacc aggttgatct gacgcgccgc gcccgtgcgg aagagccgcg 3540
   tgacccgcac gagcgtcagc aggaattgga acgcttcatt gaagccgttc tgctggttac 3600
   cgctccgctg cctcctgagg cagacacgcg ctacgcaggc cgtattcacc gcggtcgtgc 3660
   gattaccgtc ggatctagat ctcaccatca ccaccattaa actagtgatc 3710
<210> 22
   <211> 6477
   <212> DNA
   <213> Artificial Sequence
<220>
   <223> Description of Artificial Sequence: Synthetic polynucleotide
<400> 22
   aaactccctc tgcccttccc tcccgcttca tccttatttt tggacaataa actagagaac 60
   aatttgaact tgaattggaa ttcagattca gagcaagaga caagaaactt ccctttttct 120
   tctccacata ttattattta ttcgtgtatt ttcttttaac gatacgatac gatacgacac 180
   gatacgatac gacacgctac tatacagtga cgtcagattg tactgagagt gcagattgta 240
   ctgagagtgc accataaatt cccgttttaa gagcttggtg agcgctagga gtcactgcca 300
   ggtatcgttt gaacacggca ttagtcaggg aagtcataac acagtccttt cccgcaattt 360
   tctttttcta ttactcttgg cctcctctag tacactctat atttttttat gcctcggtaa 420
   tgattttcat tttttttttt cccctagcgg atgactcttt ttttttctta gcgattggca 480
   ttatcacata atgaattata cattatataa agtaatgtga tttcttcgaa gaatatacta 540
   aaaaatgagc aggcaagata aacgaaggca aagatgacag agcagaaagc cctagtaaag 600
   cgtattacaa atgaaaccaa gattcagatt gcgatctctt taaagggtgg tcccctagcg 660
   atagagcact cgatcttccc agaaaaagag gcagaagcag tagcagaaca ggccacacaa 720
   tcgcaagtga ttaacgtcca cacaggtata gggtttctgg accatatgat acatgctctg 780
   gccaagcatt ccggctggtc gctaatcgtt gagtgcattg gtgacttaca catagacgac 840
   catcacacca ctgaagactg cgggattgct ctcggtcaag cttttaaaga ggccctactg 900
   gcgcgtggag taaaaaggtt tggatcagga tttgcgcctt tggatgaggc actttccaga 960
   gcggtggtag atctttcgaa caggccgtac gcagttgtcg aacttggttt gcaaagggag 1020
   aaagtaggag atctctcttg cgagatgatc ccgcattttc ttgaaagctt tgcagaggct 1080
   agcagaatta ccctccacgt tgattgtctg cgaggcaaga atgatcatca ccgtagtgag 1140
   agtgcgttca aggctcttgc ggttgccata agagaagcca cctcgcccaa tggtaccaac 1200
   gatgttccct ccaccaaagg tgttcttatg tagtgacacc gattatttaa agctgcagca 1260
   tacgatatat atacatgtgt atatatgtat acctatgaat gtcagtaagt atgtatacga 1320
   acagtatgat actgaagatg acaaggtaat gcatcattct atacgtgtca ttctgaacga 1380
   ggcgcgcttt ccttttttct ttttgctttt tctttttttt tctcttgaac tcgacggatc 1440
   tatgcggtgt gaaataccgc acaggtgtga aataccgcac agtcatgaga tccgataact 1500
   tcttttcttt ttttttcttt tctctctccc ccgttgttgt ctcaccatat ccgcaatgac 1560
   aaaaaaaatg atggaagaca ctaaaggaaa aaattaacga caaagacagc accaacagat 1620
   gtcgttgttc cagagctgat gaggggtatc ttcgaacaca cgaaactttt tccttccttc 1680
   attcacgcac actactctct aatgagcaac ggtatacggc cttccttcca gttacttgaa 1740
   tttgaaataa aaaaagtttg ccgctttgct atcaagtata aatagacctg caattattaa 1800
   tcttttgttt cctcgtcatt gttctcgttc cctttcttcc ttgtttcttt ttctgcacaa 1860
   tatttcaagc tataccaagc atacaatcaa ctccaacgga tccatggccg gtacgggtcg 1920
   tttggctggt aaaattgcat tgatcaccgg tggtgctggt aacattggtt ccgagctgac 1980
   ccgccgtttt ctggccgagg gtgcgacggt tattatcagc ggccgtaacc gtgcgaagct 2040
   gaccgcgctg gccgagcgca tgcaagccga ggccggcgtg ccggccaagc gcattgattt 2100
   ggaggtgatg gatggttccg accctgtggc tgtccgtgcc ggtatcgagg caatcgtcgc 2160
   tcgccacggt cagattgaca ttctggttaa caacgcgggc tccgccggtg cccaacgtcg 2220
   cttggcggaa attccgctga cggaggcaga attgggtccg ggtgcggagg agactttgca 2280
   cgcttcgatc gcgaatctgt tgggcatggg ttggcacctg atgcgtattg cggctccgca 2340
   catgccagtt ggctccgcag ttatcaacgt ttcgactatt ttctcgcgcg cagagtacta 2400
   tggtcgcatt ccgtacgtta ccccgaaggc agcgctgaac gctttgtccc agctggctgc 2460
   ccgcgagctg ggcgctcgtg gcatccgcgt taacactatt ttcccaggtc ctattgagtc 2520
   cgaccgcatc cgtaccgtgt ttcaacgtat ggatcaactg aagggtcgcc cggagggcga 2580
   caccgcccat cactttttga acaccatgcg cctgtgccgc gcaaacgacc aaggcgcttt 2640
   ggaacgccgc tttccgtccg ttggcgatgt tgctgatgcg gctgtgtttc tggcttctgc 2700
   tgagagcgcg gcactgtcgg gtgagacgat tgaggtcacc cacggtatgg aactgccggc 2760
   gtgtagcgaa acctccttgt tggcgcgtac cgatctgcgt accatcgacg cgagcggtcg 2820
   cactaccctg atttgcgctg gcgatcaaat tgaagaagtt atggccctga cgggcatgct 2880
   gcgtacgtgc ggtagcgaag tgattatcgg cttccgttct gcggctgccc tggcgcaatt 2940
   tgagcaggca gtgaatgaat ctcgccgtct ggcaggtgcg gatttcaccc cgccgatcgc 3000
   tttgccgttg gacccacgtg acccggccac cattgatgcg gttttcgatt ggggcgcagg 3060
   cgagaatacg ggtggcatcc atgcggcggt cattctgccg gcaacctccc acgaaccggc 3120
   tccgtgcgtg attgaagtcg atgacgaacg cgtcctgaat ttcctggccg atgaaattac 3180
   cggcaccatc gttattgcga gccgtttggc gcgctattgg caatcccaac gcctgacccc 3240
   gggtgcccgt gcccgcggtc cgcgtgttat ctttctgagc aacggtgccg atcaaaatgg 3300
   taatgtttac ggtcgtattc aatctgcggc gatcggtcaa ttgattcgcg tttggcgtca 3360
   cgaggcggag ttggactatc aacgtgcatc cgccgcaggc gatcacgttc tgccgccggt 3420
   ttgggcgaac cagattgtcc gtttcgctaa ccgctccctg gaaggtctgg agttcgcgtg 3480
   cgcgtggacc gcacagctgc tgcacagcca acgtcatatt aacgaaatta cgctgaacat 3540
   tccagccaat attagcgcga ccacgggcgc acgttccgcc agcgtcggct gggccgagtc 3600
   cttgattggt ctgcacctgg gcaaggtggc tctgattacc ggtggttcgg cgggcatcgg 3660
   tggtcaaatc ggtcgtctgc tggccttgtc tggcgcgcgt gtgatgctgg ccgctcgcga 3720
   tcgccataaa ttggaacaga tgcaagccat gattcaaagc gaattggcgg aggttggtta 3780
   taccgatgtg gaggaccgtg tgcacatcgc tccgggttgc gatgtgagca gcgaggcgca 3840
   gctggcagat ctggtggaac gtacgctgtc cgcattcggt accgtggatt atttgattaa 3900
   taacgccggt attgcgggcg tggaggagat ggtgatcgac atgccggtgg aaggctggcg 3960
   tcacaccctg tttgccaacc tgatttcgaa ttattcgctg atgcgcaagt tggcgccgct 4020
   gatgaagaag caaggtagcg gttacatcct gaacgtttct tcctattttg gcggtgagaa 4080
   ggacgcggcg attccttatc cgaaccgcgc cgactacgcc gtctccaagg ctggccaacg 4140
   cgcgatggcg gaagtgttcg ctcgtttcct gggtccagag attcagatca atgctattgc 4200
   cccaggtccg gttgaaggcg accgcctgcg tggtaccggt gagcgtccgg gcctgtttgc 4260
   tcgtcgcgcc cgtctgatct tggagaataa acgcctgaac gaattgcacg cggctttgat 4320
   tgctgcggcc cgcaccgatg agcgctcgat gcacgagttg gttgaattgt tgctgccgaa 4380
   cgacgtggcc gcgttggagc agaacccagc ggcccctacc gcgctgcgtg agctggcacg 4440
   ccgcttccgt agcgaaggtg atccggcggc aagctcctcg tccgccttgc tgaatcgctc 4500
   catcgctgcc aagctgttgg ctcgcttgca taacggtggc tatgtgctgc cggcggatat 4560
   ttttgcaaat ctgcctaatc cgccggaccc gttctttacc cgtgcgcaaa ttgaccgcga 4620
   agctcgcaag gtgcgtgatg gtattatggg tatgctgtat ctgcagcgta tgccaaccga 4680
   gtttgacgtc gctatggcaa ccgtgtacta tctggccgat cgtaacgtga gcggcgaaac 4740
   tttccatccg tctggtggtt tgcgctacga gcgtaccccg accggtggcg agctgttcgg 4800
   cctgccatcg ccggaacgtc tggcggagct ggttggtagc acggtgtacc tgatcggtga 4860
   acacctgacc gagcacctga acctgctggc tcgtgcctat ttggagcgct acggtgcccg 4920
   tcaagtggtg atgattgttg agacggaaac cggtgcggaa accatgcgtc gtctgttgca 4980
   tgatcacgtc gaggcaggtc gcctgatgac tattgtggca ggtgatcaga ttgaggcagc 5040
   gattgaccaa gcgatcacgc gctatggccg tccgggtccg gtggtgtgca ctccattccg 5100
   tccactgcca accgttccgc tggtcggtcg taaagactcc gattggagca ccgttttgag 5160
   cgaggcggaa tttgcggaac tgtgtgagca tcagctgacc caccatttcc gtgttgctcg 5220
   taagatcgcc ttgtcggatg gcgcgtcgct ggcgttggtt accccggaaa cgactgcgac 5280
   tagcaccacg gagcaatttg ctctggcgaa cttcatcaag accaccctgc acgcgttcac 5340
   cgcgaccatc ggtgttgagt cggagcgcac cgcgcaacgt attctgatta accaggttga 5400
   tctgacgcgc cgcgcccgtg cggaagagcc gcgtgacccg cacgagcgtc agcaggaatt 5460
   ggaacgcttc attgaagccg ttctgctggt taccgctccg ctgcctcctg aggcagacac 5520
   gcgctacgca ggccgtattc accgcggtcg tgcgattacc gtcggatcta gatctcacca 5580
   tcaccaccat taaactagtt ggccaatcat gtaattagtt atgtcacgct tacattcacg 5640
   ccctcccccc acatccgctc taaccgaaaa ggaaggagtt agacaacctg aagtctaggt 5700
   ccctatttat ttttttatag ttatgttagt attaagaacg ttatttatat ttcaaatttt 5760
   tctttttttt ctgtacagac gcgtgtacgc atgtaacatt atactgaaaa ccttgcttga 5820
   gaaggttttg ggacgctcga aggctttaat ttgcaagctt ggccaccaca caccatagct 5880
   tcaaaatgtt tctactcctt ttttactctt ccagattttc tcggactccg cgcatcgccg 5940
   taccacttca aaacacccaa gcacagcata ctaaattttc cctctttctt cctctagggt 6000
   gtcgttaatt acccgtacta aaggtttgga aaagaaaaaa gagaccgcct cgtttctttt 6060
   tcttcgtcga aaaaggcaat aaaaattttt atcacgtttc tttttcttga aatttttttt 6120
   tttagttttt ttctctttca gtgacctcca ttgatattta agttaataaa cggtcttcaa 6180
   tttctcaagt ttcagtttca tttttcttgt tctattacaa ctttttttac ttcttgttca 6240
   ttagaaagaa agcatagcaa tctaatctaa gggatgagcg aagaaagctt attcgagtct 6300
   tctccacaga agatggagta cgaaattaca aactactcag aaagacatac agaacttcca 6360
   ggtcatttca ttggcctcaa tacagtagat aaactagagg agtccccgtt aagggacttt 6420
   gttaagagtc acggtggtca cacggtcata tccaagatcc tgatagcaaa taagttt 6477
<210> 23
   <211> 6233
   <212> DNA
   <213> Artificial Sequence
<220>
   <223> Description of Artificial Sequence: Synthetic polynucleotide
<400> 23
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatagcca tcctcatgaa aactgtgtaa cataataacc gaagtgtcga aaaggtggca     240
   ccttgtccaa ttgaacacgc tcgatgaaaa aaataagata tatataaggt taagtaaagc     300
   gtctgttaga aaggaagttt ttcctttttc ttgctctctt gtcttttcat ctactatttc     360
   cttcgtgtaa tacagggtcg tcagatacat agatacaatt ctattacccc catccataca     420
   atgccatctc atttcgatac tgttcaacta cacgccggcc aagagaaccc tggtgacaat     480
   gctcacagat ccagagctgt accaatttac gccaccactt cttatgtttt cgaaaactct     540
   aagcatggtt cgcaattgtt tggtctagaa gttccaggtt acgtctattc ccgtttccaa     600
   aacccaacca gtaatgtttt ggaagaaaga attgctgctt tagaaggtgg tgctgctgct     660
   ttggctgttt cctccggtca agccgctcaa acccttgcca tccaaggttt ggcacacact     720
   ggtgacaaca tcgtttccac ttcttactta tacggtggta cttataacca gttcaaaatc     780
   tcgttcaaaa gatttggtat cgaggctaga tttgttgaag gtgacaatcc agaagaattc     840
   gaaaaggtct ttgatgaaag aaccaaggct gtttatttgg aaaccattgg taatccaaag     900
   tacaatgttc cggattttga aaaaattgtt gcaattgctc acaaacacgg tattccagtt     960
   gtcgttgaca acacatttgg tgccggtggt tacttctgtc agccaattaa atacggtgct    1020
   gatattgtaa cacattctgc taccaaatgg attggtggtc atggtactac tatcggtggt    1080
   attattgttg actctggtaa gttcccatgg aaggactacc cagaaaagtt ccctcaattc    1140
   tctcaacctg ccgaaggata tcacggtact atctacaatg aagcctacgg taacttggca    1200
   tacatcgttc atgttagaac tgaactatta agagatttgg gtccattgat gaacccattt    1260
   gcctctttct tgctactaca aggtgttgaa acattatctt tgagagctga aagacacggt    1320
   gaaaatgcat tgaagttagc caaatggtta gaacaatccc catacgtatc ttgggtttca    1380
   taccctggtt tagcatctca ttctcatcat gaaaatgcta agaagtatct atctaacggt    1440
   ttcggtggtg tcttatcttt cggtgtaaaa gacttaccaa atgccgacaa ggaaactgac    1500
   ccattcaaac tttctggtgc tcaagttgtt gacaatttaa agcttgcctc taacttggcc    1560
   aatgttggtg atgccaagac cttagtcatt gctccatact tcactaccca caaacaatta    1620
   aatgacaaag aaaagttggc atctggtgtt accaaggact taattcgtgt ctctgttggt    1680
   atcgaattta ttgatgacat tattgcagac ttccagcaat cttttgaaac tgttttcgct    1740
   ggccaaaaac catgagtgtg cgtaatgagt tgtaaaatta tgtataaacc tactttctct    1800
   cacaagttat gcggtgtgaa ataccgcaca gatgcgtaag gagaaaatac cgcatcagga    1860
   aattgtaaac gttaatattt tgttaaaatt cgcgttaaat ttttgttaaa tcagctcatt 1920
   ttttaaccaa taggccgaaa tcggcaaaat cccttataaa tcaaaagaat agaccgagat 1980
   agggttgagt gttgttccag tttggaacaa gagtccacta ttaaagaacg tggactccaa 2040
   cgtcaaaggg cgaaaaaccg tctatcaggg cgatggccca ctacgtgaac catcacccta 2100
   atcaagtttt ttggggtcga ggtgccgtaa agcactaaat cggaacccta aagggagccc 2160
   ccgatttaga gcttgacggg gaaagccggc gaacgtggcg agaaaggaag ggaagaaagc 2220
   gaaaggagcg ggcgctaggg cgctggcaag tgtagcggtc acgctgcgcg taaccaccac 2280
   acccgccgcg cttaatgcgc cgctacaggg cgcgtcgcgc cattcgccat tcaggctgcg 2340
   caactgttgg gaagggcgat cggtgcgggc ctcttcgcta ttacgccagc tggcgaaagg 2400
   gggatgtgct gcaaggcgat taagttgggt aacgccaggg ttttcccagt cacgacgttg 2460
   taaaacgacg gccagtgagc gcgcgtaata cgactcacta tagggcgaat tgggtaccgg 2520
   gccccccctc gaggtcgacg gtatcgataa gcttgatatc gaattcctgc agcccggggg 2580
   atccactagt tctagagcgg ccgccaccgc ggtggagctc cagcttttgt tccctttagt 2640
   gagggttaat tgcgcgcttg gcgtaatcat ggtcatagct gtttcctgtg tgaaattgtt 2700
   atccgctcac aattccacac aacatacgag ccggaagcat aaagtgtaaa gcctggggtg 2760
   cctaatgagt gagctaactc acattaattg cgttgcgctc actgcccgct ttccagtcgg 2820
   gaaacctgtc gtgccagctg cattaatgaa tcggccaacg cgcggggaga ggcggtttgc 2880
   gtattgggcg ctcttccgct tcctcgctca ctgactcgct gcgctcggtc gttcggctgc 2940
   ggcgagcggt atcagctcac tcaaaggcgg taatacggtt atccacagaa tcaggggata 3000
   acgcaggaaa gaacatgtga gcaaaaggcc agcaaaaggc caggaaccgt aaaaaggccg 3060
   cgttgctggc gtttttccat aggctccgcc cccctgacga gcatcacaaa aatcgacgct 3120
   caagtcagag gtggcgaaac ccgacaggac tataaagata ccaggcgttt ccccctggaa 3180
   gctccctcgt gcgctctcct gttccgaccc tgccgcttac cggatacctg tccgcctttc 3240
   tcccttcggg aagcgtggcg ctttctcata gctcacgctg taggtatctc agttcggtgt 3300
   aggtcgttcg ctccaagctg ggctgtgtgc acgaaccccc cgttcagccc gaccgctgcg 3360
   ccttatccgg taactatcgt cttgagtcca acccggtaag acacgactta tcgccactgg 3420
   cagcagccac tggtaacagg attagcagag cgaggtatgt aggcggtgct acagagttct 3480
   tgaagtggtg gcctaactac ggctacacta gaaggacagt atttggtatc tgcgctctgc 3540
   tgaagccagt taccttcgga aaaagagttg gtagctcttg atccggcaaa caaaccaccg 3600
   ctggtagcgg tggttttttt gtttgcaagc agcagattac gcgcagaaaa aaaggatctc 3660
   aagaagatcc tttgatcttt tctacggggt ctgacgctca gtggaacgaa aactcacgtt 3720
   aagggatttt ggtcatgaga ttatcaaaaa ggatcttcac ctagatcctt ttaaattaaa 3780
   aatgaagttt taaatcaatc taaagtatat atgagtaaac ttggtctgac agttaccaat 3840
   gcttaatcag tgaggcacct atctcagcga tctgtctatt tcgttcatcc atagttgcct 3900
   gactccccgt cgtgtagata actacgatac gggagggctt accatctggc cccagtgctg 3960
   caatgatacc gcgagaccca cgctcaccgg ctccagattt atcagcaata aaccagccag 4020
   ccggaagggc cgagcgcaga agtggtcctg caactttatc cgcctccatc cagtctatta 4080
   attgttgccg ggaagctaga gtaagtagtt cgccagttaa tagtttgcgc aacgttgttg 4140
   ccattgctac aggcatcgtg gtgtcacgct cgtcgtttgg tatggcttca ttcagctccg 4200
   gttcccaacg atcaaggcga gttacatgat cccccatgtt gtgcaaaaaa gcggttagct 4260
   ccttcggtcc tccgatcgtt gtcagaagta agttggccgc agtgttatca ctcatggtta 4320
   tggcagcact gcataattct cttactgtca tgccatccgt aagatgcttt tctgtgactg 4380
   gtgagtactc aaccaagtca ttctgagaat agtgtatgcg gcgaccgagt tgctcttgcc 4440
   cggcgtcaat acgggataat accgcgccac atagcagaac tttaaaagtg ctcatcattg 4500
   gaaaacgttc ttcggggcga aaactctcaa ggatcttacc gctgttgaga tccagttcga 4560
   tgtaacccac tcgtgcaccc aactgatctt cagcatcttt tactttcacc agcgtttctg 4620
   ggtgagcaaa aacaggaagg caaaatgccg caaaaaaggg aataagggcg acacggaaat 4680
   gttgaatact catactcttc ctttttcaat attattgaag catttatcag ggttattgtc 4740
   tcatgagcgg atacatattt gaatgtattt agaaaaataa acaaataggg gttccgcgca 4800
   catttccccg aaaagtgcca cctgaacgaa gcatctgtgc ttcattttgt agaacaaaaa 4860
   tgcaacgcga gagcgctaat ttttcaaaca aagaatctga gctgcatttt tacagaacag 4920
   aaatgcaacg cgaaagcgct attttaccaa cgaagaatct gtgcttcatt tttgtaaaac 4980
   aaaaatgcaa cgcgagagcg ctaatttttc aaacaaagaa tctgagctgc atttttacag 5040
   aacagaaatg caacgcgaga gcgctatttt accaacaaag aatctatact tcttttttgt 5100
   tctacaaaaa tgcatcccga gagcgctatt tttctaacaa agcatcttag attacttttt 5160
   ttctcctttg tgcgctctat aatgcagtct cttgataact ttttgcactg taggtccgtt 5220
   aaggttagaa gaaggctact ttggtgtcta ttttctcttc cataaaaaaa gcctgactcc 5280
   acttcccgcg tttactgatt actagcgaag ctgcgggtgc attttttcaa gataaaggca 5340
   tccccgatta tattctatac cgatgtggat tgcgcatact ttgtgaacag aaagtgatag 5400
   cgttgatgat tcttcattgg tcagaaaatt atgaacggtt tcttctattt tgtctctata 5460
   tactacgtat aggaaatgtt tacattttcg tattgttttc gattcactct atgaatagtt 5520
   cttactacaa tttttttgtc taaagagtaa tactagagat aaacataaaa aatgtagagg 5580
   tcgagtttag atgcaagttc aaggagcgaa aggtggatgg gtaggttata tagggatata 5640
   gcacagagat atatagcaaa gagatacttt tgagcaatgt ttgtggaagc ggtattcgca 5700
   atattttagt agctcgttac agtccggtgc gtttttggtt ttttgaaagt gcgtcttcag 5760
   agcgcttttg gttttcaaaa gcgctctgaa gttcctatac tttctagaga ataggaactt 5820
   cggaatagga acttcaaagc gtttccgaaa acgagcgctt ccgaaaatgc aacgcgagct 5880
   gcgcacatac agctcactgt tcacgtcgca cctatatctg cgtgttgcct gtatatatat 5940
   atacatgaga agaacggcat agtgcgtgtt tatgcttaaa tgcgtactta tatgcgtcta 6000
   tttatgtagg atgaaaggta gtctagtacc tcctgtgata ttatcccatt ccatgcgggg 6060
   tatcgtatgc ttccttcagc actacccttt agctgttcta tatgctgcca ctcctcaatt 6120
   ggattagtct catccttcaa tgctatcatt tcctttgata ttggatcact aagaaaccat 6180
   tattatcatg acattaacct ataaaaatag gcgtatcacg aggccctttc gtc 6233
<210> 24
   <211> 12710
   <212> DNA
   <213> Artificial Sequence
<220>
   <223> Description of Artificial Sequence: Synthetic polynucleotide
<400> 24
   tcgcgcgttt cggtgatgac ggtgaaaacc tctgacacat gcagctcccg gagacggtca       60
   cagcttgtct gtaagcggat gccgggagca gacaagcccg tcagggcgcg tcagcgggtg      120
   ttggcgggtg tcggggctgg cttaactatg cggcatcaga gcagattgta ctgagagtgc      180
   accatagcca tcctcatgaa aactgtgtaa cataataacc gaagtgtcga aaaggtggca      240
   ccttgtccaa ttgaacacgc tcgatgaaaa aaataagata tatataaggt taagtaaagc      300
   gtctgttaga aaggaagttt ttcctttttc ttgctctctt gtcttttcat ctactatttc      360
   cttcgtgtaa tacagggtcg tcagatacat agatacaatt ctattacccc catccataca      420
   atgccatctc atttcgatac tgttcaacta cacgccggcc aagagaaccc tggtgacaat      480
   gctcacagat ccagagctgt accaatttac gccaccactt cttatgtttt cgaaaactct      540
   aagcatggtt cgcaattgtt tggtctagaa gttccaggtt acgtctattc ccgtttccaa      600
   aacccaacca gtaatgtttt ggaagaaaga attgctgctt tagaaggtgg tgctgctgct      660
   ttggctgttt cctccggtca agccgctcaa acccttgcca tccaaggttt ggcacacact      720
   ggtgacaaca tcgtttccac ttcttactta tacggtggta cttataacca gttcaaaatc      780
   tcgttcaaaa gatttggtat cgaggctaga tttgttgaag gtgacaatcc agaagaattc      840
   gaaaaggtct ttgatgaaag aaccaaggct gtttatttgg aaaccattgg taatccaaag      900
   tacaatgttc cggattttga aaaaattgtt gcaattgctc acaaacacgg tattccagtt      960
   gtcgttgaca acacatttgg tgccggtggt tacttctgtc agccaattaa atacggtgct 1020
   gatattgtaa cacattctgc taccaaatgg attggtggtc atggtactac tatcggtggt 1080
   attattgttg actctggtaa gttcccatgg aaggactacc cagaaaagtt ccctcaattc 1140
   tctcaacctg ccgaaggata tcacggtact atctacaatg aagcctacgg taacttggca 1200
   tacatcgttc atgttagaac tgaactatta agagatttgg gtccattgat gaacccattt 1260
   gcctctttct tgctactaca aggtgttgaa acattatctt tgagagctga aagacacggt 1320
   gaaaatgcat tgaagttagc caaatggtta gaacaatccc catacgtatc ttgggtttca 1380
   taccctggtt tagcatctca ttctcatcat gaaaatgcta agaagtatct atctaacggt 1440
   ttcggtggtg tcttatcttt cggtgtaaaa gacttaccaa atgccgacaa ggaaactgac 1500
   ccattcaaac tttctggtgc tcaagttgtt gacaatttaa agcttgcctc taacttggcc 1560
   aatgttggtg atgccaagac cttagtcatt gctccatact tcactaccca caaacaatta 1620
   aatgacaaag aaaagttggc atctggtgtt accaaggact taattcgtgt ctctgttggt 1680
   atcgaattta ttgatgacat tattgcagac ttccagcaat cttttgaaac tgttttcgct 1740
   ggccaaaaac catgagtgtg cgtaatgagt tgtaaaatta tgtataaacc tactttctct 1800
   cacaagttat gcggtgtgaa ataccgcaca gatgcgtaag gagaaaatac cgcatcagga 1860
   aattgtaaac gttaatattt tgttaaaatt cgcgttaaat ttttgttaaa tcagctcatt 1920
   ttttaaccaa taggccgaaa tcggcaaaat cccttataaa tcaaaagaat agaccgagat 1980
   agggttgagt gttgttccag tttggaacaa gagtccacta ttaaagaacg tggactccaa 2040
   cgtcaaaggg cgaaaaaccg tctatcaggg cgatggccca ctacgtgaac catcacccta 2100
   atcaagtttt ttggggtcga ggtgccgtaa agcactaaat cggaacccta aagggagccc 2160
   ccgatttaga gcttgacggg gaaagccggc gaacgtggcg agaaaggaag ggaagaaagc 2220
   gaaaggagcg ggcgctaggg cgctggcaag tgtagcggtc acgctgcgcg taaccaccac 2280
   acccgccgcg cttaatgcgc cgctacaggg cgcgtcgcgc cattcgccat tcaggctgcg 2340
   caactgttgg gaagggcgat cggtgcgggc ctcttcgcta ttacgccagc tggcgaaagg 2400
   gggatgtgct gcaaggcgat taagttgggt aacgccaggg ttttcccagt cacgacgttg 2460
   taaaacgacg gccagtgagc gcgcgtaata cgactcacta tagggcgaat tgggtaccgg 2520
   gccccccctc gaggtcgacg gtatcgataa gcttgatatc gaattcctgc agcccaaact 2580
   ccctctgccc ttccctcccg cttcatcctt atttttggac aataaactag agaacaattt 2640
   gaacttgaat tggaattcag attcagagca agagacaaga aacttccctt tttcttctcc 2700
   acatattatt atttattcgt gtattttctt ttaacgatac gatacgatac gacacgatac 2760
   gatacgacac gctactatac agtgacgtca gattgtactg agagtgcaga ttgtactgag 2820
   agtgcaccat aaattcccgt tttaagagct tggtgagcgc taggagtcac tgccaggtat 2880
   cgtttgaaca cggcattagt cagggaagtc ataacacagt cctttcccgc aattttcttt 2940
   ttctattact cttggcctcc tctagtacac tctatatttt tttatgcctc ggtaatgatt 3000
   ttcatttttt tttttcccct agcggatgac tctttttttt tcttagcgat tggcattatc 3060
   acataatgaa ttatacatta tataaagtaa tgtgatttct tcgaagaata tactaaaaaa 3120
   tgagcaggca agataaacga aggcaaagat gacagagcag aaagccctag taaagcgtat 3180
   tacaaatgaa accaagattc agattgcgat ctctttaaag ggtggtcccc tagcgataga 3240
   gcactcgatc ttcccagaaa aagaggcaga agcagtagca gaacaggcca cacaatcgca 3300
   agtgattaac gtccacacag gtatagggtt tctggaccat atgatacatg ctctggccaa 3360
   gcattccggc tggtcgctaa tcgttgagtg cattggtgac ttacacatag acgaccatca 3420
   caccactgaa gactgcggga ttgctctcgg tcaagctttt aaagaggccc tactggcgcg 3480
   tggagtaaaa aggtttggat caggatttgc gcctttggat gaggcacttt ccagagcggt 3540
   ggtagatctt tcgaacaggc cgtacgcagt tgtcgaactt ggtttgcaaa gggagaaagt 3600
   aggagatctc tcttgcgaga tgatcccgca ttttcttgaa agctttgcag aggctagcag 3660
   aattaccctc cacgttgatt gtctgcgagg caagaatgat catcaccgta gtgagagtgc 3720
   gttcaaggct cttgcggttg ccataagaga agccacctcg cccaatggta ccaacgatgt 3780
   tccctccacc aaaggtgttc ttatgtagtg acaccgatta tttaaagctg cagcatacga 3840
   tatatataca tgtgtatata tgtataccta tgaatgtcag taagtatgta tacgaacagt 3900
   atgatactga agatgacaag gtaatgcatc attctatacg tgtcattctg aacgaggcgc 3960
   gctttccttt tttctttttg cttttctttt ttttttctct tgaactcgac ggatctatgc 4020
   ggtgtgaaat accgcacagg tgtgaaatac cgcacagtca tgagatccga taacttcttt 4080
   tctttttttt tcttttctct ctcccccgtt gttgtctcac catatccgca atgacaaaaa 4140
   aaatgatgga agacactaaa ggaaaaaatt aacgacaaag acagcaccaa cagatgtcgt 4200
   tgttccagag ctgatgaggg gtatcttcga acacacgaaa ctttttcctt ccttcattca 4260
   cgcacactac tctctaatga gcaacggtat acggccttcc ttccagttac ttgaatttga 4320
   aataaaaaaa gtttgccgct ttgctatcaa gtataaatag acctgcaatt attaatcttt 4380
   tgtttcctcg tcattgttct cgttcccttt cttccttgtt tctttttctg cacaatattt 4440
   caagctatac caagcataca atcaactcca acggatccat ggccggtacg ggtcgtttgg 4500
   ctggtaaaat tgcattgatc accggtggtg ctggtaacat tggttccgag ctgacccgcc 4560
   gttttctggc cgagggtgcg acggttatta tcagcggccg taaccgtgcg aagctgaccg 4620
   cgctggccga gcgcatgcaa gccgaggccg gcgtgccggc caagcgcatt gatttggagg 4680
   tgatggatgg ttccgaccct gtggctgtcc gtgccggtat cgaggcaatc gtcgctcgcc 4740
   acggtcagat tgacattctg gttaacaacg cgggctccgc cggtgcccaa cgtcgcttgg 4800
   cggaaattcc gctgacggag gcagaattgg gtccgggtgc ggaggagact ttgcacgctt 4860
   cgatcgcgaa tctgttgggc atgggttggc acctgatgcg tattgcggct ccgcacatgc 4920
   cagttggctc cgcagttatc aacgtttcga ctattttctc gcgcgcagag tactatggtc 4980
   gcattccgta cgttaccccg aaggcagcgc tgaacgcttt gtcccagctg gctgcccgcg 5040
   agctgggcgc tcgtggcatc cgcgttaaca ctattttccc aggtcctatt gagtccgacc 5100
   gcatccgtac cgtgtttcaa cgtatggatc aactgaaggg tcgcccggag ggcgacaccg 5160
   cccatcactt tttgaacacc atgcgcctgt gccgcgcaaa cgaccaaggc gctttggaac 5220
   gccgctttcc gtccgttggc gatgttgctg atgcggctgt gtttctggct tctgctgaga 5280
   gcgcggcact gtcgggtgag acgattgagg tcacccacgg tatggaactg ccggcgtgta 5340
   gcgaaacctc cttgttggcg cgtaccgatc tgcgtaccat cgacgcgagc ggtcgcacta 5400
   ccctgatttg cgctggcgat caaattgaag aagttatggc cctgacgggc atgctgcgta 5460
   cgtgcggtag cgaagtgatt atcggcttcc gttctgcggc tgccctggcg caatttgagc 5520
   aggcagtgaa tgaatctcgc cgtctggcag gtgcggattt cacccccgcg atcgctttgc 5580
   cgttggaccc acgtgacccg gccaccattg atgcggtttt cgattggggc gcaggcgaga 5640
   atacgggtgg catccatgcg gcggtcattc tgccggcaac ctcccacgaa ccggctccgt 5700
   gcgtgattga agtcgatgac gaacgcgtcc tgaatttcct ggccgatgaa attaccggca 5760
   ccatcgttat tgcgagccgt ttggcgcgct attggcaatc ccaacgcctg accccgggtg 5820
   cccgtgcccg cggtccgcgt gttatctttc tgagcaacgg tgccgatcaa aatggtaatg 5880
   tttacggtcg tattcaatct gcggcgatcg gtcaattgat tcgcgtttgg cgtcacgagg 5940
   cggagttgga ctatcaacgt gcatccgccg caggcgatca cgttctgccg ccggtttggg 6000
   cgaaccagat tgtccgtttc gctaaccgct ccctggaagg tctggagttc gcgtgcgcgt 6060
   ggaccgcaca gctgctgcac agccaacgtc atattaacga aattacgctg aacattccag 6120
   ccaatattag cgcgaccacg ggcgcacgtt ccgccagcgt cggctgggcc gagtccttga 6180
   ttggtctgca cctgggcaag gtggctctga ttaccggtgg ttcggcgggc atcggtggtc 6240
   aaatcggtcg tctgctggcc ttgtctggcg cgcgtgtgat gctggccgct cgcgatcgcc 6300
   ataaattgga acagatgcaa gccatgattc aaagcgaatt ggcggaggtt ggttataccg 6360
   atgtggagga ccgtgtgcac atcgctccgg gttgcgatgt gagcagcgag gcgcagctgg 6420
   cagatctggt ggaacgtacg ctgtccgcat tcggtaccgt ggattatttg attaataacg 6480
   ccggtattgc gggcgtggag gagatggtga tcgacatgcc ggtggaaggc tggcgtcaca 6540
   ccctgtttgc caacctgatt tcgaattatt cgctgatgcg caagttggcg ccgctgatga 6600
   agaagcaagg tagcggttac atcctgaacg tttcttccta ttttggcggt gagaaggacg 6660
   cggcgattcc ttatccgaac cgcgccgact acgccgtctc caaggctggc caacgcgcga 6720
   tggcggaagt gttcgctcgt ttcctgggtc cagagattca gatcaatgct attgccccag 6780
   gtccggttga aggcgaccgc ctgcgtggta ccggtgagcg tccgggcctg tttgctcgtc 6840
   gcgcccgtct gatcttggag aataaacgcc tgaacgaatt gcacgcggct ttgattgctg 6900
   cggcccgcac cgatgagcgc tcgatgcacg agttggttga attgttgctg ccgaacgacg 6960
   tggccgcgtt ggagcagaac ccagcggccc ctaccgcgct gcgtgagctg gcacgccgct 7020
   tccgtagcga aggtgatccg gcggcaagct cctcgtccgc cttgctgaat cgctccatcg 7080
   ctgccaagct gttggctcgc ttgcataacg gtggctatgt gctgccggcg gatatttttg 7140
   caaatctgcc taatccgccg gacccgttct ttacccgtgc gcaaattgac cgcgaagctc 7200
   gcaaggtgcg tgatggtatt atgggtatgc tgtatctgca gcgtatgcca accgagtttg 7260
   acgtcgctat ggcaaccgtg tactatctgg ccgatcgtaa cgtgagcggc gaaactttcc 7320
   atccgtctgg tggtttgcgc tacgagcgta ccccgaccgg tggcgagctg ttcggcctgc 7380
   catcgccgga acgtctggcg gagctggttg gtagcacggt gtacctgatc ggtgaacacc 7440
   tgaccgagca cctgaacctg ctggctcgtg cctatttgga gcgctacggt gcccgtcaag 7500
   tggtgatgat tgttgagacg gaaaccggtg cggaaaccat gcgtcgtctg ttgcatgatc 7560
   acgtcgaggc aggtcgcctg atgactattg tggcaggtga tcagattgag gcagcgattg 7620
   accaagcgat cacgcgctat ggccgtccgg gtccggtggt gtgcactcca ttccgtccac 7680
   tgccaaccgt tccgctggtc ggtcgtaaag actccgattg gagcaccgtt ttgagcgagg 7740
   cggaatttgc ggaactgtgt gagcatcagc tgacccacca tttccgtgtt gctcgtaaga 7800
   tcgccttgtc ggatggcgcg tcgctggcgt tggttacccc ggaaacgact gcgactagca 7860
   ccacggagca atttgctctg gcgaacttca tcaagaccac cctgcacgcg ttcaccgcga 7920
   ccatcggtgt tgagtcggag cgcaccgcgc aacgtattct gattaaccag gttgatctga 7980
   cgcgccgcgc ccgtgcggaa gagccgcgtg acccgcacga gcgtcagcag gaattggaac 8040
   gcttcattga agccgttctg ctggttaccg ctccgctgcc tcctgaggca gacacgcgct 8100
   acgcaggccg tattcaccgc ggtcgtgcga ttaccgtcgg atctagatct caccatcacc 8160
   accattaaac tagttggcca atcatgtaat tagttatgtc acgcttacat tcacgccctc 8220
   cccccacatc cgctctaacc gaaaaggaag gagttagaca acctgaagtc taggtcccta 8280
   tttatttttt tatagttatg ttagtattaa gaacgttatt tatatttcaa atttttcttt 8340
   tttttctgta cagacgcgtg tacgcatgta acattatact gaaaaccttg cttgagaagg 8400
   ttttgggacg ctcgaaggct ttaatttgca agcttggcca ccacacacca tagcttcaaa 8460
   atgtttctac tcctttttta ctcttccaga ttttctcgga ctccgcgcat cgccgtacca 8520
   cttcaaaaca cccaagcaca gcatactaaa ttttccctct ttcttcctct agggtgtcgt 8580
   taattacccg tactaaaggt ttggaaaaga aaaaagagac cgcctcgttt ctttttcttc 8640
   gtcgaaaaag gcaataaaaa tttttatcac gtttcttttt cttgaaattt ttttttttag 8700
   tttttttctc tttcagtgac ctccattgat atttaagtta ataaacggtc ttcaatttct 8760
   caagtttcag tttcattttt cttgttctat tacaactttt tttacttctt gttcattaga 8820
   aagaaagcat agcaatctaa tctaagggat gagcgaagaa agcttattcg agtcttctcc 8880
   acagaagatg gagtacgaaa ttacaaacta ctcagaaaga catacagaac ttccaggtca 8940
   tttcattggc ctcaatacag tagataaact agaggagtcc ccgttaaggg actttgttaa 9000
   gagtcacggt ggtcacacgg tcatatccaa gatcctgata gcaaataagt ttgggggatc 9060
   cactagttct agagcggccg ccaccgcggt ggagctccag cttttgttcc ctttagtgag 9120
   ggttaattgc gcgcttggcg taatcatggt catagctgtt tcctgtgtga aattgttatc 9180
   cgctcacaat tccacacaac atacgagccg gaagcataaa gtgtaaagcc tggggtgcct 9240
   aatgagtgag ctaactcaca ttaattgcgt tgcgctcact gcccgctttc cagtcgggaa 9300
   acctgtcgtg ccagctgcat taatgaatcg gccaacgcgc ggggagaggc ggtttgcgta 9360
   ttgggcgctc ttccgcttcc tcgctcactg actcgctgcg ctcggtcgtt cggctgcggc 9420
   gagcggtatc agctcactca aaggcggtaa tacggttatc cacagaatca ggggataacg 9480
   caggaaagaa catgtgagca aaaggccagc aaaaggccag gaaccgtaaa aaggccgcgt 9540
   tgctggcgtt tttccatagg ctccgccccc ctgacgagca tcacaaaaat cgacgctcaa 9600
   gtcagaggtg gcgaaacccg acaggactat aaagatacca ggcgtttccc cctggaagct 9660
   ccctcgtgcg ctctcctgtt ccgaccctgc cgcttaccgg atacctgtcc gcctttctcc 9720
   cttcgggaag cgtggcgctt tctcatagct cacgctgtag gtatctcagt tcggtgtagg 9780
   tcgttcgctc caagctgggc tgtgtgcacg aaccccccgt tcagcccgac cgctgcgcct 9840
   tatccggtaa ctatcgtctt gagtccaacc cggtaagaca cgacttatcg ccactggcag 9900
   cagccactgg taacaggatt agcagagcga ggtatgtagg cggtgctaca gagttcttga 9960
   agtggtggcc taactacggc tacactagaa ggacagtatt tggtatctgc gctctgctga 10020
   agccagttac cttcggaaaa agagttggta gctcttgatc cggcaaacaa accaccgctg 10080
   gtagcggtgg tttttttgtt tgcaagcagc agattacgcg cagaaaaaaa ggatctcaag 10140
   aagatccttt gatcttttct acggggtctg acgctcagtg gaacgaaaac tcacgttaag 10200
   ggattttggt catgagatta tcaaaaagga tcttcaccta gatcctttta aattaaaaat 10260
   gaagttttaa atcaatctaa agtatatatg agtaaacttg gtctgacagt taccaatgct 10320
   taatcagtga ggcacctatc tcagcgatct gtctatttcg ttcatccata gttgcctgac 10380
   tccccgtcgt gtagataact acgatacggg agggcttacc atctggcccc agtgctgcaa 10440
   tgataccgcg agacccacgc tcaccggctc cagatttatc agcaataaac cagccagccg 10500
   gaagggccga gcgcagaagt ggtcctgcaa ctttatccgc ctccatccag tctattaatt 10560
   gttgccggga agctagagta agtagttcgc cagttaatag tttgcgcaac gttgttgcca 10620
   ttgctacagg catcgtggtg tcacgctcgt cgtttggtat ggcttcattc agctccggtt 10680
   cccaacgatc aaggcgagtt acatgatccc ccatgttgtg caaaaaagcg gttagctcct 10740
   tcggtcctcc gatcgttgtc agaagtaagt tggccgcagt gttatcactc atggttatgg 10800
   cagcactgca taattctctt actgtcatgc catccgtaag atgcttttct gtgactggtg 10860
   agtactcaac caagtcattc tgagaatagt gtatgcggcg accgagttgc tcttgcccgg 10920
   cgtcaatacg ggataatacc gcgccacata gcagaacttt aaaagtgctc atcattggaa 10980
   aacgttcttc ggggcgaaaa ctctcaagga tcttaccgct gttgagatcc agttcgatgt 11040
   aacccactcg tgcacccaac tgatcttcag catcttttac tttcaccagc gtttctgggt 11100
   gagcaaaaac aggaaggcaa aatgccgcaa aaaagggaat aagggcgaca cggaaatgtt 11160
   gaatactcat actcttcctt tttcaatatt attgaagcat ttatcagggt tattgtctca 11220
   tgagcggata catatttgaa tgtatttaga aaaataaaca aataggggtt ccgcgcacat 11280
   ttccccgaaa agtgccacct gaacgaagca tctgtgcttc attttgtaga acaaaaatgc 11340
   aacgcgagag cgctaatttt tcaaacaaag aatctgagct gcatttttac agaacagaaa 11400
   tgcaacgcga aagcgctatt ttaccaacga agaatctgtg cttcattttt gtaaaacaaa 11460
   aatgcaacgc gagagcgcta atttttcaaa caaagaatct gagctgcatt tttacagaac 11520
   agaaatgcaa cgcgagagcg ctattttacc aacaaagaat ctatacttct tttttgttct 11580
   acaaaaatgc atcccgagag cgctattttt ctaacaaagc atcttagatt actttttttc 11640
   tcctttgtgc gctctataat gcagtctctt gataactttt tgcactgtag gtccgttaag 11700
   gttagaagaa ggctactttg gtgtctattt tctcttccat aaaaaaagcc tgactccact 11760
   tcccgcgttt actgattact agcgaagctg cgggtgcatt ttttcaagat aaaggcatcc 11820
   ccgattatat tctataccga tgtggattgc gcatactttg tgaacagaaa gtgatagcgt 11880
   tgatgattct tcattggtca gaaaattatg aacggtttct tctattttgt ctctatatac 11940
   tacgtatagg aaatgtttac attttcgtat tgttttcgat tcactctatg aatagttctt 12000
   actacaattt ttttgtctaa agagtaatac tagagataaa cataaaaaat gtagaggtcg 12060
   agtttagatg caagttcaag gagcgaaagg tggatgggta ggttatatag ggatatagca 12120
   cagagatata tagcaaagag atacttttga gcaatgtttg tggaagcggt attcgcaata 12180
   ttttagtagc tcgttacagt ccggtgcgtt tttggttttt tgaaagtgcg tcttcagagc 12240
   gcttttggtt ttcaaaagcg ctctgaagtt cctatacttt ctagagaata ggaacttcgg 12300
   aataggaact tcaaagcgtt tccgaaaacg agcgcttccg aaaatgcaac gcgagctgcg 12360
   cacatacagc tcactgttca cgtcgcacct atatctgcgt gttgcctgta tatatatata 12420
   catgagaaga acggcatagt gcgtgtttat gcttaaatgc gtacttatat gcgtctattt 12480
   atgtaggatg aaaggtagtc tagtacctcc tgtgatatta tcccattcca tgcggggtat 12540
   cgtatgcttc cttcagcact accctttagc tgttctatat gctgccactc ctcaattgga 12600
   ttagtctcat ccttcaatgc tatcatttcc tttgatattg gatcactaag aaaccattat 12660
   tatcatgaca ttaacctata aaaataggcg tatcacgagg ccctttcgtc 12710
<210> 25
   <211> 23
   <212> DNA
   <213> Artificial Sequence
<220>
   <223> Description of Artificial Sequence: Synthetic primer
<400> 25
   gacaatatac aaacgcgatt ctc 23
<210> 26
   <211> 23
   <212> DNA
   <213> Artificial Sequence
<220>
   <223> Description of Artificial Sequence: Synthetic primer
<400> 26
   tgagactgaa atattgacgt tga 23
<210> 27
   <211> 660
   <212> DNA
   <213> Escherichia coli
<400> 27
   gtgaaagaga ttattgatgg attccttaaa ttccagcgcg aggcatttcc gaagcgggaa 60
   gccttgttta aacagctggc gacacagcaa agcccgcgca cactttttat ctcctgctcc 120
   gacagccgtc tggtccctga gctggtgacg caacgtgagc ctggcgatct gttcgttatt 180
   cgcaacgcgg gcaatatcgt cccttcctac gggccggaac ccggtggcgt ttctgcttcg 240
   gtggagtatg ccgtcgctgc gcttcgggta tctgacattg tgatttgtgg tcattccaac 300
   tgtggcgcga tgaccgccat tgccagctgt cagtgcatgg accatatgcc tgccgtctcc 360
   cactggctgc gttatgccga ttcagcccgc gtcgttaatg aggcgcgccc gcattccgat 420
   ttaccgtcaa aagctgcggc gatggtacgt gaaaacgtca ttgctcagtt ggctaatttg 480
   caaactcatc catcggtgcg cctggcgctc gaagaggggc ggatcgccct gcacggctgg 540
   gtctacgaca ttgaaagcgg cagcatcgca gcttttgacg gcgcaacccg ccagtttgtg 600
   ccactggccg ctaatcctcg cgtttgtgcc ataccgctac gccaaccgac cgcagcgtaa 660
<210> 28
   <211> 780
   <212> DNA
   <213> Escherichia coli
<400> 28
   atgggttttc tttccggtaa gcgcattctg gtaaccggtg ttgccagcaa actatccatc 60
   gcctacggta tcgctcaggc gatgcaccgc gaaggagctg aactggcatt cacctaccag 120
   aacgacaaac tgaaaggccg cgtagaagaa tttgccgctc aattgggttc tgacatcgtt 180
   ctgcagtgcg atgttgcaga agatgccagc atcgacacca tgttcgctga actggggaaa 240
   gtttggccga aatttgacgg tttcgtacac tctattggtt ttgcacctgg cgatcagctg 300
   gatggtgact atgttaacgc cgttacccgt gaaggcttca aaattgccca cgacatcagc 360
   tcctacagct tcgttgcaat ggcaaaagct tgccgctcca tgctgaatcc gggttctgcc 420
   ctgctgaccc tttcctacct tggcgctgag cgcgctatcc cgaactacaa cgttatgggt 480
   ctggcaaaag cgtctctgga agcgaacgtg cgctatatgg cgaacgcgat gggtccggaa 540
   ggtgtgcgtg ttaacgccat ctctgctggt ccgatccgta ctctggcggc ctccggtatc 600
   aaagacttcc gcaaaatgct ggctcattgc gaagccgtta ccccgattcg ccgtaccgtt 660
   actattgaag atgtgggtaa ctctgcggca ttcctgtgct ccgatctctc tgccggtatc 720
   ttcggtgaag tggtccacgt tgacggcggt ttcagcattg ctgcaatgaa cgaactcgaa 780
<210> 29
   <211> 262
   <212> PRT
   <213> Escherichia coli
<400> 29
   Met Gly Phe Leu Ser Gly Lys Arg Ile Leu Val Thr Gly Val Ala Ser
   1               5                   10                  15
   Lys Leu Ser Ile Ala Tyr Gly Ile Ala Gln Ala Met His Arg Glu Gly
               20                  25                  30
   Ala Glu Leu Ala Phe Thr Tyr Gln Asn Asp Lys Leu Lys Gly Arg Val
           35                  40                  45
   Glu Glu Phe Ala Ala Gln Leu Gly Ser Asp Ile Val Leu Gln Cys Asp
       50                  55                  60
   Val Ala Glu Asp Ala Ser Ile Asp Thr Met Phe Ala Glu Leu Gly Lys
   65                  70                  75                  80
   Val Trp Pro Lys Phe Asp Gly Phe Val His Ser Ile Gly Phe Ala Pro
                   85                  90                  95
   Gly Asp Gln Leu Asp Gly Asp Tyr Val Asn Ala Val Thr Arg Glu Gly
               100                 105                 110
   Phe Lys Ile Ala His Asp Ile Ser Ser Tyr Ser Phe Val Ala Met Ala
           115                 120                 125
   Lys Ala Cys Arg Ser Met Leu Asn Pro Gly Ser Ala Leu Leu Thr Leu
       130                 135                 140
   Ser Tyr Leu Gly Ala Glu Arg Ala Ile Pro Asn Tyr Asn Val Met Gly
   145                 150                 155                 160
   Leu Ala Lys Ala Ser Leu Glu Ala Asn Val Arg Tyr Met Ala Asn Ala
                   165                 170                 175
   Met Gly Pro Glu Gly Val Arg Val Asn Ala Ile Ser Ala Gly Pro Ile
               180                 185                 190
   Arg Thr Leu Ala Ala Ser Gly Ile Lys Asp Phe Arg Lys Met Leu Ala
           195                 200                 205
   His Cys Glu Ala Val Thr Pro Ile Arg Arg Thr Val Thr Ile Glu Asp
       210                 215                 220
   Val Gly Asn Ser Ala Ala Phe Leu Cys Ser Asp Leu Ser Ala Gly Ile
   225                 230                 235                 240
   Phe Gly Glu Val Val His Val Asp Gly Gly Phe Ser Ile Ala Ala Met
                   245                 250                 255
   Asn Glu Leu Glu Leu Lys
               260
<210> 30
   <211> 960
   <212> DNA
   <213> Escherichia coli
<400> 30
   atgagtctga atttccttga ttttgaacag ccgattgcag agctggaagc gaaaatcgat 60
   tctctgactg cggttagccg tcaggatgag aaactggata ttaacatcga tgaagaagtg 120
   catcgtctgc gtgaaaaaag cgtagaactg acacgtaaaa tcttcgccga tctcggtgca 180
   tggcagattg cgcaactggc acgccatcca cagcgtcctt ataccctgga ttacgttcgc 240
   ctggcatttg atgaatttga cgaactggct ggcgaccgcg cgtatgcaga cgataaagct 300
   atcgtcggtg gtatcgcccg tctcgatggt cgtccggtga tgatcattgg tcatcaaaaa 360
   ggtcgtgaaa ccaaagaaaa aattcgccgt aactttggta tgccagcgcc agaaggttac 420
   cgcaaagcac tgcgtctgat gcaaatggct gaacgcttta agatgcctat catcaccttt 480
   atcgacaccc cgggggctta tcctggcgtg ggcgcagaag agcgtggtca gtctgaagcc 540
   attgcacgca acctgcgtga aatgtctcgc ctcggcgtac cggtagtttg tacggttatc 600
   ggtgaaggtg gttctggcgg tgcgctggcg attggcgtgg gcgataaagt gaatatgctg 660
   caatacagca cctattccgt tatctcgccg gaaggttgtg cgtccattct gtggaagagc 720
   gccgacaaag cgccgctggc ggctgaagcg atgggtatca ttgctccgcg tctgaaagaa 780
   ctgaaactga tcgactccat catcccggaa ccactgggtg gtgctcaccg taacccggaa 840
   gcgatggcgg catcgttgaa agcgcaactg ctggcggatc tggccgatct cgacgtgtta 900
   agcactgaag atttaaaaaa tcgtcgttat cagcgcctga tgagctacgg ttacgcgtaa 960
<210> 31
   <211> 319
   <212> PRT
   <213> Escherichia coli
<400> 31
   Met Ser Leu Asn Phe Leu Asp Phe Glu Gln Pro Ile Ala Glu Leu Glu
   1               5                   10                  15
   Ala Lys Ile Asp Ser Leu Thr Ala Val Ser Arg Gln Asp Glu Lys Leu
               20                  25                  30
   Asp Ile Asn Ile Asp Glu Glu Val His Arg Leu Arg Glu Lys Ser Val
           35                  40                  45
   Glu Leu Thr Arg Lys Ile Phe Ala Asp Leu Gly Ala Trp Gln Ile Ala
       50                  55                  60
   Gln Leu Ala Arg His Pro Gln Arg Pro Tyr Thr Leu Asp Tyr Val Arg
   65                  70                  75                  80
   Leu Ala Phe Asp Glu Phe Asp Glu Leu Ala Gly Asp Arg Ala Tyr Ala
                   85                  90                  95
   Asp Asp Lys Ala Ile Val Gly Gly Ile Ala Arg Leu Asp Gly Arg Pro
               100                 105                 110
   Val Met Ile Ile Gly His Gln Lys Gly Arg Glu Thr Lys Glu Lys Ile
                   115                 120                 125
   Arg Arg Asn Phe Gly Met Pro Ala Pro Glu Gly Tyr Arg Lys Ala Leu
       130                 135                 140
   Arg Leu Met Gln Met Ala Glu Arg Phe Lys Met Pro Ile Ile Thr Phe
   145                 150                 155                 160
   Ile Asp Thr Pro Gly Ala Tyr Pro Gly Val Gly Ala Glu Glu Arg Gly
                   165                 170                 175
   Gln Ser Glu Ala Ile Ala Arg Asn Leu Arg Glu Met Ser Arg Leu Gly
               180                 185                 190
   Val Pro Val Val Cys Thr Val Ile Gly Glu Gly Gly Ser Gly Gly Ala
           195                 200                 205
   Leu Ala Ile Gly Val Gly Asp Lys Val Asn Met Leu Gln Tyr Ser Thr
       210                 215                 220
   Tyr Ser Val Ile Ser Pro Glu Gly Cys Ala Ser Ile Leu Trp Lys Ser
   225                 230                 235                 240
   Ala Asp Lys Ala Pro Leu Ala Ala Glu Ala Met Gly Ile Ile Ala Pro
                   245                 250                 255
   Arg Leu Lys Glu Leu Lys Leu Ile Asp Ser Ile Ile Pro Glu Pro Leu
               260                 265                 270
   Gly Gly Ala His Arg Asn Pro Glu Ala Met Ala Ala Ser Leu Lys Ala
           275                 280                 285
   Gln Leu Leu Ala Asp Leu Ala Asp Leu Asp Val Leu Ser Thr Glu Asp
       290                 295                 300
   Leu Lys Asn Arg Arg Tyr Gln Arg Leu Met Ser Tyr Gly Tyr Ala
   305                 310                 315
<210> 32
   <211> 471
   <212> DNA
   <213> Escherichia coli
<400> 32
   atggatattc gtaagattaa aaaactgatc gagctggttg aagaatcagg catctccgaa        60
   ctggaaattt ctgaaggcga agagtcagta cgcattagcc gtgcagctcc tgccgcaagt       120
   ttccctgtga tgcaacaagc ttacgctgca ccaatgatgc agcagccagc tcaatctaac       180
   gcagccgctc cggcgaccgt tccttccatg gaagcgccag cagcagcgga aatcagtggt       240
   cacatcgtac gttccccgat ggttggtact ttctaccgca ccccaagccc ggacgcaaaa       300
   gcgttcatcg aagtgggtca gaaagtcaac gtgggcgata ccctgtgcat cgttgaagcc       360
   atgaaaatga tgaaccagat cgaagcggac aaatccggta ccgtgaaagc aattctggtc       420
   gaaagtggac aaccggtaga atttgacgag ccgctggtcg tcatcgagta a                471
<210> 33
   <211> 156
   <212> PRT
   <213> Escherichia coli
<400> 33
   Met Asp Ile Arg Lys Ile Lys Lys Leu Ile Glu Leu Val Glu Glu Ser
   1               5                   10                  15
   Gly Ile Ser Glu Leu Glu Ile Ser Glu Gly Glu Glu Ser Val Arg Ile
               20                  25                  30
   Ser Arg Ala Ala Pro Ala Ala Ser Phe Pro Val Met Gln Gln Ala Tyr
           35                  40                  45
   Ala Ala Pro Met Met Gln Gln Pro Ala Gln Ser Asn Ala Ala Ala Pro
       50                  55                  60
   Ala Thr Val Pro Ser Met Glu Ala Pro Ala Ala Ala Glu Ile Ser Gly
   65                  70                  75                  80
   His Ile Val Arg Ser Pro Met Val Gly Thr Phe Tyr Arg Thr Pro Ser
                   85                  90                  95
   Pro Asp Ala Lys Ala Phe Ile Glu Val Gly Gln Lys Val Asn Val Gly
               100                 105                 110
   Asp Thr Leu Cys Ile Val Glu Ala Met Lys Met Met Asn Gln Ile Glu
           115                 120                 125
   Ala Asp Lys Ser Gly Thr Val Lys Ala Ile Leu Val Glu Ser Gly Gln
       130                 135                 140
   Pro Val Glu Phe Asp Glu Pro Leu Val Val Ile Glu
   145                 150                 155
<210> 34
   <211> 1350
   <212> DNA
   <213> Escherichia coli
<400> 34
   atgctggata aaattgttat tgccaaccgc ggcgagattg cattgcgtat tcttcgtgcc      60
   tgtaaagaac tgggcatcaa gactgtcgct gtgcactcca gcgcggatcg cgatctaaaa     120
   cacgtattac tggcagatga aacggtctgt attggccctg ctccgtcagt aaaaagttat     180
   ctgaacatcc cggcaatcat cagcgccgct gaaatcaccg gcgcagtagc aatccatccg     240
   ggttacggct tcctctccga gaacgccaac tttgccgagc aggttgaacg ctccggcttt     300
   atcttcattg gcccgaaagc agaaaccatt cgcctgatgg gcgacaaagt atccgcaatc     360
   gcggcgatga aaaaagcggg cgtcccttgc gtaccgggtt ctgacggccc gctgggcgac     420
   gatatggata aaaaccgtgc cattgctaaa cgcattggtt atccggtgat tatcaaagcc     480
   tccggcggcg gcggcggtcg cggtatgcgc gtagtgcgcg gcgacgctga actggcacaa     540
   tccatctcca tgacccgtgc ggaagcgaaa gctgctttca gcaacgatat ggtttacatg     600
   gagaaatacc tggaaaatcc tcgccacgtc gagattcagg tactggctga cggtcagggc     660
   aacgctatct atctggcgga acgtgactgc tccatgcaac gccgccacca gaaagtggtc     720
   gaagaagcgc cagcaccggg cattaccccg gaactgcgtc gctacatcgg cgaacgttgc     780
   gctaaagcgt gtgttgatat cggctatcgc ggtgcaggta ctttcgagtt cctgttcgaa     840
   aacggcgagt tctatttcat cgaaatgaac acccgtattc aggtagaaca cccggttaca     900
   gaaatgatca ccggcgttga cctgatcaaa gaacagctgc gtatcgctgc cggtcaaccg     960
   ctgtcgatca agcaagaaga agttcacgtt cgcggccatg cggtggaatg tcgtatcaac    1020
   gccgaagatc cgaacacctt cctgccaagt ccgggcaaaa tcacccgttt ccacgcacct    1080
   ggcggttttg gcgtacgttg ggagtctcat atctacgcgg gctacaccgt accgccgtac    1140
   tatgactcaa tgatcggtaa gctgatttgc tacggtgaaa accgtgacgt ggcgattgcc    1200
   cgcatgaaga atgcgctgca ggagctgatc atcgacggta tcaaaaccaa cgttgatctg    1260
   cagatccgca tcatgaatga cgagaacttc cagcatggtg gcactaacat ccactatctg    1320
   gagaaaaaac tcggtcttca ggaaaaataa                                      1350
<210> 35
   <211> 449
   <212> PRT
   <213> Escherichia coli
<400> 35
   Met Leu Asp Lys Ile Val Ile Ala Asn Arg Gly Glu Ile Ala Leu Arg
   1               5                   10                  15
   Ile Leu Arg Ala Cys Lys Glu Leu Gly Ile Lys Thr Val Ala Val His
               20                  25                  30
   Ser Ser Ala Asp Arg Asp Leu Lys His Val Leu Leu Ala Asp Glu Thr
           35                  40                  45
   Val Cys Ile Gly Pro Ala Pro Ser Val Lys Ser Tyr Leu Asn Ile Pro
       50                  55                  60
   Ala Ile Ile Ser Ala Ala Glu Ile Thr Gly Ala Val Ala Ile His Pro
   65                  70                  75                  80
   Gly Tyr Gly Phe Leu Ser Glu Asn Ala Asn Phe Ala Glu Gln Val Glu
                   85                  90                  95
   Arg Ser Gly Phe Ile Phe Ile Gly Pro Lys Ala Glu Thr Ile Arg Leu
               100                 105                 110
   Met Gly Asp Lys Val Ser Ala Ile Ala Ala Met Lys Lys Ala Gly Val
           115                 120                 125
   Pro Cys Val Pro Gly Ser Asp Gly Pro Leu Gly Asp Asp Met Asp Lys
       130                 135                 140
   Asn Arg Ala Ile Ala Lys Arg Ile Gly Tyr Pro Val Ile Ile Lys Ala
   145                 150                 155                 160
   Ser Gly Gly Gly Gly Gly Arg Gly Met Arg Val Val Arg Gly Asp Ala
                   165                 170                 175
   Glu Leu Ala Gln Ser Ile Ser Met Thr Arg Ala Glu Ala Lys Ala Ala
               180                 185                 190
   Phe Ser Asn Asp Met Val Tyr Met Glu Lys Tyr Leu Glu Asn Pro Arg
           195                 200                 205
   His Val Glu Ile Gln Val Leu Ala Asp Gly Gln Gly Asn Ala Ile Tyr
       210                 215                 220
   Leu Ala Glu Arg Asp Cys Ser Met Gln Arg Arg His Gln Lys Val Val
   225                 230                 235                 240
   Glu Glu Ala Pro Ala Pro Gly Ile Thr Pro Glu Leu Arg Arg Tyr Ile
                   245                 250                 255
   Gly Glu Arg Cys Ala Lys Ala Cys Val Asp Ile Gly Tyr Arg Gly Ala
               260                 265                 270
   Gly Thr Phe Glu Phe Leu Phe Glu Asn Gly Glu Phe Tyr Phe Ile Glu
           275                 280                 285
   Met Asn Thr Arg Ile Gln Val Glu His Pro Val Thr Glu Met Ile Thr
   290 295 300
   Gly Val Asp Leu Ile Lys Glu Gln Leu Arg Ile Ala Ala Gly Gln Pro
   305 310 315 320
   Leu Ser Ile Lys Gln Glu Glu Val His Val Arg Gly His Ala Val Glu
   325 330 335
   Cys Arg Ile Asn Ala Glu Asp Pro Asn Thr Phe Leu Pro Ser Pro Gly
   340 345 350
   Lys Ile Thr Arg Phe His Ala Pro Gly Gly Phe Gly Val Arg Trp Glu
   355 360 365
   Ser His Ile Tyr Ala Gly Tyr Thr Val Pro Pro Tyr Tyr Asp Ser Met
   370 375 380
   Ile Gly Lys Leu Ile Cys Tyr Gly Glu Asn Arg Asp Val Ala Ile Ala
   385 390 395 400
   Arg Met Lys Asn Ala Leu Gln Glu Leu Ile Ile Asp Gly Ile Lys Thr
   405 410 415
   Asn Val Asp Leu Gln Ile Arg Ile Met Asn Asp Glu Asn Phe Gln His
   420 425 430
   Gly Gly Thr Asn Ile His Tyr Leu Glu Lys Lys Leu Gly Leu Gln Glu
   435 440 445
   Lys
<210> 36
   <211> 915
   <212> DNA
   <213> Escherichia coli
<400> 36
   atgagctgga ttgaacgaat taaaagcaac attactccca cccgcaaggc gagcattcct 60
   gaaggggtgt ggactaagtg tgatagctgc ggtcaggttt tataccgcgc tgagctggaa 120
   cgtaatcttg aggtctgtcc gaagtgtgac catcacatgc gtatgacagc gcgtaatcgc 180
   ctgcatagcc tgttagatga aggaagcctt gtggagctgg gtagcgagct tgagccgaaa 240
   gatgtgctga agtttcgtga ctccaagaag tataaagacc gtctggcatc tgcgcagaaa 300
   gaaaccggcg aaaaagatgc gctggtggtg atgaaaggca ctctgtatgg aatgccggtt 360
   gtcgctgcgg cattcgagtt cgcctttatg ggcggttcaa tggggtctgt tgtgggtgca 420
   cgtttcgtgc gtgccgttga gcaggcgctg gaagataact gcccgctgat ctgcttctcc 480
   gcctctggtg gcgcacgtat gcaggaagca ctgatgtcgc tgatgcagat ggcgaaaacc 540
   tctgcggcac tggcaaaaat gcaggagcgc ggcttgccgt acatctccgt gctgaccgac 600
   ccgacgatgg gcggtgtttc tgcaagtttc gccatgctgg gcgatctcaa catcgctgaa 660
   ccgaaagcgt taatcggctt tgccggtccg cgtgttatcg aacagaccgt tcgcgaaaaa 720
   ctgccgcctg gattccagcg cagtgaattc ctgatcgaga aaggcgcgat cgacatgatc 780
   gtccgtcgtc cggaaatgcg cctgaaactg gcgagcattc tggcgaagtt gatgaatctg 840
   ccagcgccga atcctgaagc gccgcgtgaa ggcgtagtgg tacccccggt accggatcag 900
   gaacctgagg cctga 915
<210> 37
   <211> 304
   <212> PRT
   <213> Escherichia coli
<400> 37
   Met Ser Trp Ile Glu Arg Ile Lys Ser Asn Ile Thr Pro Thr Arg Lys
   1               5                   10                  15
   Ala Ser Ile Pro Glu Gly Val Trp Thr Lys Cys Asp Ser Cys Gly Gln
               20                  25                  30
   Val Leu Tyr Arg Ala Glu Leu Glu Arg Asn Leu Glu Val Cys Pro Lys
           35                  40                  45
   Cys Asp His His Met Arg Met Thr Ala Arg Asn Arg Leu His Ser Leu
       50                  55                  60
   Leu Asp Glu Gly Ser Leu Val Glu Leu Gly Ser Glu Leu Glu Pro Lys
   65                  70                  75                  80
   Asp Val Leu Lys Phe Arg Asp Ser Lys Lys Tyr Lys Asp Arg Leu Ala
                   85                  90                  95
   Ser Ala Gln Lys Glu Thr Gly Glu Lys Asp Ala Leu Val Val Met Lys
               100                 105                 110
   Gly Thr Leu Tyr Gly Met Pro Val Val Ala Ala Ala Phe Glu Phe Ala
           115                 120                 125
   Phe Met Gly Gly Ser Met Gly Ser Val Val Gly Ala Arg Phe Val Arg
       130                 135                 140
   Ala Val Glu Gln Ala Leu Glu Asp Asn Cys Pro Leu Ile Cys Phe Ser
   145                 150                 155                 160
   Ala Ser Gly Gly Ala Arg Met Gln Glu Ala Leu Met Ser Leu Met Gln
   165 170 175
   Met Ala Lys Thr Ser Ala Ala Leu Ala Lys Met Gln Glu Arg Gly Leu
   180 185 190
   Pro Tyr Ile Ser Val Leu Thr Asp Pro Thr Met Gly Gly Val Ser Ala
   195 200 205
   Ser Phe Ala Met Leu Gly Asp Leu Asn Ile Ala Glu Pro Lys Ala Leu
   210 215 220
   Ile Gly Phe Ala Gly Pro Arg Val Ile Glu Gln Thr Val Arg Glu Lys
   225 230 235 240
   Leu Pro Pro Gly Phe Gln Arg Ser Glu Phe Leu Ile Glu Lys Gly Ala
   245 250 255
   Ile Asp Met Ile Val Arg Arg Pro Glu Met Arg Leu Lys Leu Ala Ser
   260 265 270
   Ile Leu Ala Lys Leu Met Asn Leu Pro Ala Pro Asn Pro Glu Ala Pro
   275 280 285
   Arg Glu Gly Val Val Val Pro Pro Val Pro Asp Gln Glu Pro Glu Ala
   290 295 300
<210> 38
   <211> 1533
   <212> DNA
   <213> Escherichia coli
<400> 38
   atgcgaattg gcataccaag agaacggtta accaatgaaa cccgtgttgc agcaacgcca 60
   aaaacagtgg aacagctgct gaaactgggt tttaccgtcg cggtagagag cggcgcgggt 120
   caactggcaa gttttgacga taaagcgttt gtgcaagcgg gcgctgaaat tgtagaaggg 180
   aatagcgtct ggcagtcaga gatcattctg aaggtcaatg cgccgttaga tgatgaaatt 240
   gcgttactga atcctgggac aacgctggtg agttttatct ggcctgcgca gaatccggaa 300
   ttaatgcaaa aacttgcgga acgtaacgtg accgtgatgg cgatggactc tgtgccgcgt 360
   atctcacgcg cacaatcgct ggacgcacta agctcgatgg cgaacatcgc cggttatcgc 420
   gccattgttg aagcggcaca tgaatttggg cgcttcttta ccgggcaaat tactgcggcc 480
   gggaaagtgc caccggcaaa agtgatggtg attggtgcgg gtgttgcagg tctggccgcc 540
   attggcgcag caaacagtct cggcgcgatt gtgcgtgcat tcgacacccg cccggaagtg 600
   aaagaacaag ttcaaagtat gggcgcggaa ttcctcgagc tggattttaa agaggaagct 660
   ggcagcggcg atggctatgc caaagtgatg tcggacgcgt tcatcaaagc ggaaatggaa 720
   ctctttgccg cccaggcaaa agaggtcgat atcattgtca ccaccgcgct tattccaggc 780
   aaaccagcgc cgaagctaat tacccgtgaa atggttgact ccatgaaggc gggcagtgtg 840
   attgtcgacc tggcagccca aaacggcggc aactgtgaat acaccgtgcc gggtgaaatc 900
   ttcactacgg aaaatggtgt caaagtgatt ggttataccg atcttccggg ccgtctgccg 960
   acgcaatcct cacagcttta cggcacaaac ctcgttaatc tgctgaaact gttgtgcaaa 1020
   gagaaagacg gcaatatcac tgttgatttt gatgatgtgg tgattcgcgg cgtgaccgtg 1080
   atccgtgcgg gcgaaattac ctggccggca ccgccgattc aggtatcagc tcagccgcag 1140
   gcggcacaaa aagcggcacc ggaagtgaaa actgaggaaa aatgtacctg ctcaccgtgg 1200
   cgtaaatacg cgttgatggc gctggcaatc attctttttg gctggatggc aagcgttgcg 1260
   ccgaaagaat tccttgggca cttcaccgtt ttcgcgctgg cctgcgttgt cggttattac 1320
   gtggtgtgga atgtatcgca cgcgctgcat acaccgttga tgtcggtcac caacgcgatt 1380
   tcagggatta ttgttgtcgg agcactgttg cagattggcc agggcggctg ggttagcttc 1440
   cttagtttta tcgcggtgct tatagccagc attaatattt tcggtggctt caccgtgact 1500
   cagcgcatgc tgaaaatgtt ccgcaaaaat taa 1533
<210> 39
   <211> 510
   <212> PRT
   <213> Escherichia coli
<400> 39
   Met Arg Ile Gly Ile Pro Arg Glu Arg Leu Thr Asn Glu Thr Arg Val
   1               5                   10                  15
   Ala Ala Thr Pro Lys Thr Val Glu Gln Leu Leu Lys Leu Gly Phe Thr
               20                  25                  30
   Val Ala Val Glu Ser Gly Ala Gly Gln Leu Ala Ser Phe Asp Asp Lys
           35                  40                  45
   Ala Phe Val Gln Ala Gly Ala Glu Ile Val Glu Gly Asn Ser Val Trp
       50                  55                  60
   Gln Ser Glu Ile Ile Leu Lys Val Asn Ala Pro Leu Asp Asp Glu Ile
   65                  70                  75                  80
   Ala Leu Leu Asn Pro Gly Thr Thr Leu Val Ser Phe Ile Trp Pro Ala
                   85                  90                  95
   Gln Asn Pro Glu Leu Met Gln Lys Leu Ala Glu Arg Asn Val Thr Val
   100 105 110
   Met Ala Met Asp Ser Val Pro Arg Ile Ser Arg Ala Gln Ser Leu Asp
   115 120 125
   Ala Leu Ser Ser Met Ala Asn Ile Ala Gly Tyr Arg Ala Ile Val Glu
   130 135 140
   Ala Ala His Glu Phe Gly Arg Phe Phe Thr Gly Gln Ile Thr Ala Ala
   145 150 155 160
   Gly Lys Val Pro Pro Ala Lys Val Met Val Ile Gly Ala Gly Val Ala
   165 170 175
   Gly Leu Ala Ala Ile Gly Ala Ala Asn Ser Leu Gly Ala Ile Val Arg
   180 185 190
   Ala Phe Asp Thr Arg Pro Glu Val Lys Glu Gln Val Gln Ser Met Gly
   195 200 205
   Ala Glu Phe Leu Glu Leu Asp Phe Lys Glu Glu Ala Gly Ser Gly Asp
   210 215 220
   Gly Tyr Ala Lys Val Met Ser Asp Ala Phe Ile Lys Ala Glu Met Glu
   225 230 235 240
   Leu Phe Ala Ala Gln Ala Lys Glu Val Asp Ile Ile Val Thr Thr Ala
   245 250 255
   Leu Ile Pro Gly Lys Pro Ala Pro Lys Leu Ile Thr Arg Glu Met Val
   260 265 270
   Asp Ser Met Lys Ala Gly Ser Val Ile Val Asp Leu Ala Ala Gln Asn
   275 280 285
   Gly Gly Asn Cys Glu Tyr Thr Val Pro Gly Glu Ile Phe Thr Thr Glu
   290 295 300
   Asn Gly Val Lys Val Ile Gly Tyr Thr Asp Leu Pro Gly Arg Leu Pro
   305 310 315 320
   Thr Gln Ser Ser Gln Leu Tyr Gly Thr Asn Leu Val Asn Leu Leu Lys
   325 330 335
   Leu Leu Cys Lys Glu Lys Asp Gly Asn Ile Thr Val Asp Phe Asp Asp
   340 345 350
   Val Val Ile Arg Gly Val Thr Val Ile Arg Ala Gly Glu Ile Thr Trp
           355                 360                 365
   Pro Ala Pro Pro Ile Gln Val Ser Ala Gln Pro Gln Ala Ala Gln Lys
       370                 375                 380
   Ala Ala Pro Glu Val Lys Thr Glu Glu Lys Cys Thr Cys Ser Pro Trp
   385                 390                 395                 400
   Arg Lys Tyr Ala Leu Met Ala Leu Ala Ile Ile Leu Phe Gly Trp Met
                   405                 410                 415
   Ala Ser Val Ala Pro Lys Glu Phe Leu Gly His Phe Thr Val Phe Ala
               420                 425                 430
   Leu Ala Cys Val Val Gly Tyr Tyr Val Val Trp Asn Val Ser His Ala
           435                 440                 445
   Leu His Thr Pro Leu Met Ser Val Thr Asn Ala Ile Ser Gly Ile Ile
       450                 455                 460
   Val Val Gly Ala Leu Leu Gln Ile Gly Gln Gly Gly Trp Val Ser Phe
   465                 470                 475                 480
   Leu Ser Phe Ile Ala Val Leu Ile Ala Ser Ile Asn Ile Phe Gly Gly
                   485                 490                 495
   Phe Thr Val Thr Gln Arg Met Leu Lys Met Phe Arg Lys Asn
               500                 505                 510
<210> 40
   <211> 1389
   <212> DNA
   <213> Escherichia coli
<400> 40
   atgtctggag gattagttac agctgcatac attgttgccg cgatcctgtt tatcttcagt      60
   ctggccggtc tttcgaaaca tgaaacgtct cgccagggta acaacttcgg tatcgccggg     120
   atggcgattg cgttaatcgc aaccattttt ggaccggata cgggtaatgt tggctggatc     180
   ttgctggcga tggtcattgg tggggcaatt ggtatccgtc tggcgaagaa agttgaaatg     240
   accgaaatgc cagaactggt ggcgatcctg catagcttcg tgggtctggc ggcagtgctg     300
   gttggcttta acagctatct gcatcatgac gcgggaatgg caccgattct ggtcaatatt     360
   cacctgacgg aagtgttcct cggtatcttc atcggggcgg taacgttcac gggttcggtg     420
   gtggcgttcg gcaaactgtg tggcaagatt tcgtctaaac cattgatgct gccaaaccgt     480
   cacaaaatga acctggcggc tctggtcgtt tccttcctgc tgctgattgt atttgttcgc     540
   acggacagcg tcggcctgca agtgctggca ttgctgataa tgaccgcaat tgcgctggta     600
   ttcggctggc atttagtcgc ctccatcggt ggtgcagata tgccagtggt ggtgtcgatg     660
   ctgaactcgt actccggctg ggcggctgcg gctgcgggct ttatgctcag caacgacctg     720
   ctgattgtga ccggtgcgct ggtcggttct tcgggggcta tcctttctta cattatgtgt     780
   aaggcgatga accgttcctt tatcagcgtt attgcgggtg gtttcggcac cgacggctct     840
   tctactggcg atgatcagga agtgggtgag caccgcgaaa tcaccgcaga agagacagcg     900
   gaactgctga aaaactccca ttcagtgatc attactccgg ggtacggcat ggcagtcgcg     960
   caggcgcaat atcctgtcgc tgaaattact gagaaattgc gcgctcgtgg tattaatgtg    1020
   cgtttcggta tccacccggt cgcggggcgt ttgcctggac atatgaacgt attgctggct    1080
   gaagcaaaag taccgtatga catcgtgctg gaaatggacg agatcaatga tgactttgct    1140
   gataccgata ccgtactggt gattggtgct aacgatacgg ttaacccggc ggcgcaggat    1200
   gatccgaaga gtccgattgc tggtatgcct gtgctggaag tgtggaaagc gcagaacgtg    1260
   attgtcttta aacgttcgat gaacactggc tatgctggtg tgcaaaaccc gctgttcttc    1320
   aaggaaaaca cccacatgct gtttggtgac gccaaagcca gcgtggatgc aatcctgaaa    1380
   gctctgtaa                                                            1389
<210> 41
   <211> 462
   <212> PRT
   <213> Escherichia coli
<400> 41
   Met Ser Gly Gly Leu Val Thr Ala Ala Tyr Ile Val Ala Ala Ile Leu
   1               5                   10                  15
   Phe Ile Phe Ser Leu Ala Gly Leu Ser Lys His Glu Thr Ser Arg Gln
               20                  25                  30
   Gly Asn Asn Phe Gly Ile Ala Gly Met Ala Ile Ala Leu Ile Ala Thr
           35                  40                  45
   Ile Phe Gly Pro Asp Thr Gly Asn Val Gly Trp Ile Leu Leu Ala Met
       50                  55                  60
   Val Ile Gly Gly Ala Ile Gly Ile Arg Leu Ala Lys Lys Val Glu Met
   65                  70                  75                  80
   Thr Glu Met Pro Glu Leu Val Ala Ile Leu His Ser Phe Val Gly Leu
                   85                  90                  95
   Ala Ala Val Leu Val Gly Phe Asn Ser Tyr Leu His His Asp Ala Gly
   100 105 110
   Met Ala Pro Ile Leu Val Asn Ile His Leu Thr Glu Val Phe Leu Gly
   115 120 125
   Ile Phe Ile Gly Ala Val Thr Phe Thr Gly Ser Val Val Ala Phe Gly
   130 135 140
   Lys Leu Cys Gly Lys Ile Ser Ser Lys Pro Leu Met Leu Pro Asn Arg
   145 150 155 160
   His Lys Met Asn Leu Ala Ala Leu Val Val Ser Phe Leu Leu Leu Ile
   165 170 175
   Val Phe Val Arg Thr Asp Ser Val Gly Leu Gln Val Leu Ala Leu Leu
   180 185 190
   Ile Met Thr Ala Ile Ala Leu Val Phe Gly Trp His Leu Val Ala Ser
   195 200 205
   Ile Gly Gly Ala Asp Met Pro Val Val Val Ser Met Leu Asn Ser Tyr
   210 215 220
   Ser Gly Trp Ala Ala Ala Ala Ala Gly Phe Met Leu Ser Asn Asp Leu
   225 230 235 240
   Leu Ile Val Thr Gly Ala Leu Val Gly Ser Ser Gly Ala Ile Leu Ser
   245 250 255
   Tyr Ile Met Cys Lys Ala Met Asn Arg Ser Phe Ile Ser Val Ile Ala
   260 265 270
   Gly Gly Phe Gly Thr Asp Gly Ser Ser Thr Gly Asp Asp Gln Glu Val
   275 280 285
   Gly Glu His Arg Glu Ile Thr Ala Glu Glu Thr Ala Glu Leu Leu Lys
   290 295 300
   Asn Ser His Ser Val Ile Ile Thr Pro Gly Tyr Gly Met Ala Val Ala
   305 310 315 320
   Gln Ala Gln Tyr Pro Val Ala Glu Ile Thr Glu Lys Leu Arg Ala Arg
   325 330 335
   Gly Ile Asn Val Arg Phe Gly Ile His Pro Val Ala Gly Arg Leu Pro
   340 345 350
   Gly His Met Asn Val Leu Leu Ala Glu Ala Lys Val Pro Tyr Asp Ile
   355 360 365
   Val Leu Glu Met Asp Glu Ile Asn Asp Asp Phe Ala Asp Thr Asp Thr
   370 375 380
   Val Leu Val Ile Gly Ala Asn Asp Thr Val Asn Pro Ala Ala Gln Asp
   385 390 395 400
   Asp Pro Lys Ser Pro Ile Ala Gly Met Pro Val Leu Glu Val Trp Lys
   405 410 415
   Ala Gln Asn Val Ile Val Phe Lys Arg Ser Met Asn Thr Gly Tyr Ala
   420 425 430
   Gly Val Gln Asn Pro Leu Phe Phe Lys Glu Asn Thr His Met Leu Phe
   435 440 445
   Gly Asp Ala Lys Ala Ser Val Asp Ala Ile Leu Lys Ala Leu
   450 455 460
<210> 42
   <211> 1219
   <212> PRT
   <213> Chloroflexus aurantiacus
<400> 42
   Met Ser Gly Thr Gly Arg Leu Ala Gly Lys Ile Ala Leu Ile Thr Gly
   1 5 10 15
   Gly Ala Gly Asn Ile Gly Ser Glu Leu Thr Arg Arg Phe Leu Ala Glu
   20 25 30
   Gly Ala Thr Val Ile Ile Ser Gly Arg Asn Arg Ala Lys Leu Thr Ala
   35 40 45
   Leu Ala Glu Arg Met Gln Ala Glu Ala Gly Val Pro Ala Lys Arg Ile
   50 55 60
   Asp Leu Glu Val Met Asp Gly Ser Asp Pro Val Ala Val Arg Ala Gly
   65 70 75 80
   Ile Glu Ala Ile Val Ala Arg His Gly Gln Ile Asp Ile Leu Val Asn
   85 90 95
   Asn Ala Gly Ser Ala Gly Ala Gln Arg Arg Leu Ala Glu Ile Pro Leu
   100 105 110
   Thr Glu Ala Glu Leu Gly Pro Gly Ala Glu Glu Thr Leu His Ala Ser
           115                 120                 125
   Ile Ala Asn Leu Leu Gly Met Gly Trp His Leu Met Arg Ile Ala Ala
       130                 135                 140
   Pro His Met Pro Val Gly Ser Ala Val Ile Asn Val Ser Thr Ile Phe
   145                 150                 155                 160
   Ser Arg Ala Glu Tyr Tyr Gly Arg Ile Pro Tyr Val Thr Pro Lys Ala
                   165                 170                 175
   Ala Leu Asn Ala Leu Ser Gln Leu Ala Ala Arg Glu Leu Gly Ala Arg
               180                 185                 190
   Gly Ile Arg Val Asn Thr Ile Phe Pro Gly Pro Ile Glu Ser Asp Arg
           195                 200                 205
   Ile Arg Thr Val Phe Gln Arg Met Asp Gln Leu Lys Gly Arg Pro Glu
       210                 215                 220
   Gly Asp Thr Ala His His Phe Leu Asn Thr Met Arg Leu Cys Arg Ala
   225                 230                 235                 240
   Asn Asp Gln Gly Ala Leu Glu Arg Arg Phe Pro Ser Val Gly Asp Val
                   245                 250                 255
   Ala Asp Ala Ala Val Phe Leu Ala Ser Ala Glu Ser Ala Ala Leu Ser
               260                 265                 270
   Gly Glu Thr Ile Glu Val Thr His Gly Met Glu Leu Pro Ala Cys Ser
           275                 280                 285
   Glu Thr Ser Leu Leu Ala Arg Thr Asp Leu Arg Thr Ile Asp Ala Ser
       290                 295                 300
   Gly Arg Thr Thr Leu Ile Cys Ala Gly Asp Gln Ile Glu Glu Val Met
   305                 310                 315                 320
   Ala Leu Thr Gly Met Leu Arg Thr Cys Gly Ser Glu Val Ile Ile Gly
                   325                 330                 335
   Phe Arg Ser Ala Ala Ala Leu Ala Gln Phe Glu Gln Ala Val Asn Glu
               340                 345                 350
   Ser Arg Arg Leu Ala Gly Ala Asp Phe Thr Pro Pro Ile Ala Leu Pro
           355                 360                 365
   Leu Asp Pro Arg Asp Pro Ala Thr Ile Asp Ala Val Phe Asp Trp Ala
   370 375 380
   Gly Glu Asn Thr Gly Gly Ile His Ala Ala Val Ile Leu Pro Ala Thr
   385 390 395 400
   Ser His Glu Pro Ala Pro Cys Val Ile Glu Val Asp Asp Glu Arg Val
   405 410 415
   Leu Asn Phe Leu Ala Asp Glu Ile Thr Gly Thr Ile Val Ile Ala Ser
   420 425 430
   Arg Leu Ala Arg Tyr Trp Gln Ser Gln Arg Leu Thr Pro Gly Ala Arg
   435 440 445
   Ala Arg Gly Pro Arg Val Ile Phe Leu Ser Asn Gly Ala Asp Gln Asn
   450 455 460
   Gly Asn Val Tyr Gly Arg Ile Gln Ser Ala Ala Ile Gly Gln Leu Ile
   465 470 475 480
   Arg Val Trp Arg His Glu Ala Glu Leu Asp Tyr Gln Arg Ala Ser Ala
   485 490 495
   Ala Gly Asp His Val Leu Pro Pro Val Trp Ala Asn Gln Ile Val Arg
   500 505 510
   Phe Ala Asn Arg Ser Leu Glu Gly Leu Glu Phe Ala Cys Ala Trp Thr
   515 520 525
   Ala Gln Leu Leu His Ser Gln Arg His Ile Asn Glu Ile Thr Leu Asn
   530 535 540
   Ile Pro Ala Asn Ile Ser Ala Thr Thr Gly Ala Arg Ser Ala Ser Val
   545 550 555 560
   Gly Trp Ala Glu Ser Leu Ile Gly Leu His Leu Gly Lys Val Ala Leu
   565 570 575
   Ile Thr Gly Gly Ser Ala Gly Ile Gly Gly Gln Ile Gly Arg Leu Leu
   580 585 590
   Ala Leu Ser Gly Ala Arg Val Met Leu Ala Ala Arg Asp Arg His Lys
   595 600 605
   Leu Glu Gln Met Gln Ala Met Ile Gln Ser Glu Leu Ala Glu Val Gly
   610 615 620
   Tyr Thr Asp Val Glu Asp Arg Val His Ile Ala Pro Gly Cys Asp Val
   625 630 635 640
   Ser Ser Glu Ala Gln Leu Ala Asp Leu Val Glu Arg Thr Leu Ser Ala
   645 650 655
   Phe Gly Thr Val Asp Tyr Leu Ile Asn Asn Ala Gly Ile Ala Gly Val
   660 665 670
   Glu Glu Met Val Ile Asp Met Pro Val Glu Gly Trp Arg His Thr Leu
   675 680 685
   Phe Ala Asn Leu Ile Ser Asn Tyr Ser Leu Met Arg Lys Leu Ala Pro
   690 695 700
   Leu Met Lys Lys Gln Gly Ser Gly Tyr Ile Leu Asn Val Ser Ser Tyr
   705 710 715 720
   Phe Gly Gly Glu Lys Asp Ala Ala Ile Pro Tyr Pro Asn Arg Ala Asp
   725 730 735
   Tyr Ala Val Ser Lys Ala Gly Gln Arg Ala Met Ala Glu Val Phe Ala
   740 745 750
   Arg Phe Leu Gly Pro Glu Ile Gln Ile Asn Ala Ile Ala Pro Gly Pro
   755 760 765
   Val Glu Gly Asp Arg Leu Arg Gly Thr Gly Glu Arg Pro Gly Leu Phe
   770 775 780
   Ala Arg Arg Ala Arg Leu Ile Leu Glu Asn Lys Arg Leu Asn Glu Leu
   785 790 795 800
   His Ala Ala Leu Ile Ala Ala Ala Arg Thr Asp Glu Arg Ser Met His
   805 810 815
   Glu Leu Val Glu Leu Leu Leu Pro Asn Asp Val Ala Ala Leu Glu Gln
   820 825 830
   Asn Pro Ala Ala Pro Thr Ala Leu Arg Glu Leu Ala Arg Arg Phe Arg
   835 840 845
   Ser Glu Gly Asp Pro Ala Ala Ser Ser Ser Ser Ala Leu Leu Asn Arg
   850 855 860
   Ser Ile Ala Ala Lys Leu Leu Ala Arg Leu His Asn Gly Gly Tyr Val
   865                 870                 875                 880
   Leu Pro Ala Asp Ile Phe Ala Asn Leu Pro Asn Pro Pro Asp Pro Phe
                   885                 890                 895
   Phe Thr Arg Ala Gln Ile Asp Arg Glu Ala Arg Lys Val Arg Asp Gly
               900                 905                 910
   Ile Met Gly Met Leu Tyr Leu Gln Arg Met Pro Thr Glu Phe Asp Val
           915                 920                 925
   Ala Met Ala Thr Val Tyr Tyr Leu Ala Asp Arg Asn Val Ser Gly Glu
       930                 935                 940
   Thr Phe His Pro Ser Gly Gly Leu Arg Tyr Glu Arg Thr Pro Thr Gly
   945                 950                 955                 960
   Gly Glu Leu Phe Gly Leu Pro Ser Pro Glu Arg Leu Ala Glu Leu Val
                   965                 970                 975
   Gly Ser Thr Val Tyr Leu Ile Gly Glu His Leu Thr Glu His Leu Asn
               980                 985                 990
   Leu Leu Ala Arg Ala Tyr Leu Glu  Arg Tyr Gly Ala Arg  Gln Val Val
           995                 1000                 1005
   Met Ile  Val Glu Thr Glu Thr  Gly Ala Glu Thr Met  Arg Arg Leu
       1010                 1015                 1020
   Leu His  Asp His Val Glu Ala  Gly Arg Leu Met Thr  Ile Val Ala
       1025                 1030                 1035
   Gly Asp  Gln Ile Glu Ala Ala  Ile Asp Gln Ala Ile  Thr Arg Tyr
       1040                 1045                 1050
   Gly Arg  Pro Gly Pro Val Val  Cys Thr Pro Phe Arg  Pro Leu Pro
       1055                 1060                 1065
   Thr Val  Pro Leu Val Gly Arg  Lys Asp Ser Asp Trp  Ser Thr Val
       1070                 1075                 1080
   Leu Ser  Glu Ala Glu Phe Ala  Glu Leu Cys Glu His  Gln Leu Thr
       1085                 1090                 1095
   His His  Phe Arg Val Ala Arg  Lys Ile Ala Leu Ser  Asp Gly Ala
       1100                 1105                 1110
   Ser Leu Ala Leu Val Thr Pro Glu Thr Thr Ala Thr Ser Thr Thr
   1115 1120 1125
   Glu Gln Phe Ala Leu Ala Asn Phe Ile Lys Thr Thr Leu His Ala
   1130 1135 1140
   Phe Thr Ala Thr Ile Gly Val Glu Ser Glu Arg Thr Ala Gln Arg
   1145 1150 1155
   Ile Leu Ile Asn Gln Val Asp Leu Thr Arg Arg Ala Arg Ala Glu
   1160 1165 1170
   Glu Pro Arg Asp Pro His Glu Arg Gln Gln Glu Leu Glu Arg Phe
   1175 1180 1185
   Ile Glu Ala Val Leu Leu Val Thr Ala Pro Leu Pro Pro Glu Ala
   1190 1195 1200
   Asp Thr Arg Tyr Ala Gly Arg Ile His Arg Gly Arg Ala Ile Thr
   1205 1210 1215
   Val
<210> 43
   <211> 1219
   <212> PRT
   <213> Chloroflexus aggregans
<400> 43
   Met Ser Val Thr Gly Arg Leu Ala Gly Lys Ile Ala Leu Ile Thr Gly
   1 5 10 15
   Gly Ala Gly Asn Ile Gly Ser Glu Met Thr Arg Arg Phe Leu Ala Glu
   20 25 30
   Gly Ala Thr Val Ile Ile Ser Gly Arg Asn Ser Ala Lys Leu Ala Ala
   35 40 45
   Leu Ala Glu Arg Leu Arg Ser Glu Ala Gly Val Pro Ala Lys Arg Ile
   50 55 60
   Asp Leu Glu Val Met Asp Gly Ser Asp Pro Ala Ala Val Arg Ala Gly
   65 70 75 80
   Val Ala Ala Ile Ile Gly Arg His Gly His Ile Asp Ile Leu Val Asn
   85 90 95
   Asn Ala Gly Ser Thr Gly Ala Gln Arg Arg Leu Ala Glu Ile Pro Leu
               100                 105                 110
   Asn Glu Thr Asp Arg Asp Leu Asp Asp Glu Glu Ala Leu Ser Thr Ser
           115                 120                 125
   Val Ala Asn Leu Leu Gly Met Ala Trp His Leu Met Arg Ile Leu Ser
       130                 135                 140
   Pro His Met Pro Pro Gly Ser Ala Ile Ile Asn Ile Ser Thr Ile Phe
   145                 150                 155                 160
   Ser Arg Ala Glu Tyr Tyr Gly Arg Ile Pro Tyr Val Val Pro Lys Ala
                   165                 170                 175
   Ala Leu Asn Thr Leu Thr Gln Ile Ala Ala Arg Glu Leu Gly Ile Arg
               180                 185                 190
   Gly Ile Arg Val Asn Thr Ile Phe Pro Gly Pro Ile Glu Ser Glu Arg
           195                 200                 205
   Ile Gln Thr Val Phe Gln Arg Met Asp Gln Leu Lys Gly Arg Pro Glu
       210                 215                 220
   Gly Asp Thr Ala Ser Gln Phe Leu Ala Thr Met Arg Leu Tyr Arg Ala
   225                 230                 235                 240
   Asn Asp Gln Gly Gln Leu Glu Arg Arg Phe Pro Thr Ile Cys Asp Val
                   245                 250                 255
   Ala Asp Ala Ala Val Phe Leu Ala Ser Asp Glu Ala Ala Ala Leu Thr
               260                 265                 270
   Gly Glu Thr Ile Glu Val Thr His Gly Met Glu Leu Pro Thr Ser Ser
           275                 280                 285
   Glu Thr Ser Leu Leu Ala Arg Thr Asp Leu Arg Thr Ile Asp Ala Asn
       290                 295                 300
   Gly Arg Thr Thr Leu Ile Cys Ala Gly Asp Gln Ile Glu Glu Val Met
   305                 310                 315                 320
   Ala Leu Thr Gly Met Leu Arg Thr Cys Gly Ser Glu Val Ile Ile Gly
                   325                 330                 335
   Phe Arg Ser Glu Ala Ala Leu Ala Gln Phe Glu Gln Ala Ile Gly Glu
               340                 345                 350
   Ser Arg Arg Leu Ala Gly Glu Ser Phe Ile Pro Pro Ile Ala Leu Pro
   355 360 365
   Ile Asp Leu Arg Asn Pro Ser Thr Ile Asp Ala Leu Phe Asp Trp Ala
   370 375 380
   Gly Glu Asn Thr Gly Gly Ile His Ala Ala Val Ile Leu Pro Ala Ser
   385 390 395 400
   Gly Arg Glu Pro Ala Thr Gln Val Ile Asp Ile Asp Asp Ala His Val
   405 410 415
   Gln Ala Phe Leu Asn Asp Glu Ile Val Gly Ser Ile Ile Ile Ala Ser
   420 425 430
   Arg Leu Ala Arg Tyr Trp Gln Ala Gln Arg Ile Ala Pro Gly Ala Arg
   435 440 445
   Ala Arg Glu Pro Arg Val Ile Phe Leu Ser Asn Gly Ala Ser Thr Ala
   450 455 460
   Gly Asn Pro Tyr Gly Arg Ile Gln Ser Ala Ala Ile Glu Gln Leu Ile
   465 470 475 480
   Arg Val Trp Arg His Glu Ala Ala Leu Asp Tyr Glu Arg Ala Thr Ala
   485 490 495
   Ala Gly Glu Arg Val Leu Pro Ala Val Trp Ala Ser Gln Ile Val Arg
   500 505 510
   Phe Ala Asn Arg Ser Leu Glu Gly Leu Glu Phe Ala Cys Ala Trp Thr
   515 520 525
   Ala Gln Leu Leu His Ser Gln Arg Arg Ile Asn Glu Ile Thr Leu Thr
   530 535 540
   Ile Pro Ala Asp Ile Ser Ala Thr Thr Gly Ala Arg Ser Ala Ser Val
   545 550 555 560
   Gly Trp Ala Glu Ser Leu Ile Gly Leu His Leu Gly Lys Val Ala Leu
   565 570 575
   Ile Thr Gly Gly Ser Ala Gly Ile Gly Gly Gln Ile Gly Arg Leu Leu
   580 585 590
   Ala Leu Ser Gly Ala Arg Val Met Leu Ala Ala Arg Asp Pro His Lys
   595 600 605
   Leu Glu Gln Ile Gln Ala Thr Ile Arg Ala Glu Leu Ala Glu Val Gly
   610 615 620
   Tyr Thr Asp Val Glu Glu Arg Val Gln Ile Ala Pro Gly Cys Asp Val
   625 630 635 640
   Ser Ser Glu Glu Gln Leu Val Asp Leu Val Glu Arg Thr Leu Ala Ala
   645 650 655
   Phe Gly Thr Val Asp Tyr Leu Ile Asn Asn Ala Gly Ile Ala Gly Val
   660 665 670
   Glu Glu Met Val Ile Asp Met Pro Val Glu Gly Trp Arg Asn Thr Leu
   675 680 685
   Tyr Ala Asn Leu Ile Ser Asn Tyr Ser Leu Met Arg Lys Leu Ala Pro
   690 695 700
   Leu Met Lys Lys Gln Gly Ser Gly Tyr Val Leu Asn Val Ser Ser Tyr
   705 710 715 720
   Phe Gly Gly Glu Lys Asp Ala Ala Ile Pro Tyr Pro Asn Arg Ala Asp
   725 730 735
   Tyr Ala Val Ser Lys Ala Gly Gln Arg Ala Met Ala Glu Val Phe Ala
   740 745 750
   Arg Phe Leu Gly Pro Glu Ile Gln Ile Asn Ala Ile Ala Pro Gly Pro
   755 760 765
   Val Glu Gly Asp Arg Leu Arg Gly Thr Gly Glu Arg Pro Gly Leu Phe
   770 775 780
   Ala Arg Arg Ala Arg Leu Ile Leu Glu Asn Lys Arg Leu Asn Glu Leu
   785 790 795 800
   His Ala Ala Leu Ile Thr Ala Ala Arg Thr Asp Asn Arg Pro Met Arg
   805 810 815
   Glu Leu Val Glu Leu Leu Leu Pro Asn Asp Val Ala Ala Leu Ala Gln
   820 825 830
   His Pro Ala Ala Pro Asp Val Leu Arg Thr Leu Ala Lys Arg Phe Gln
   835 840 845
   Ser Glu Gly Asp Pro Ala Ala Ser Ser Ser Ser Phe Leu Leu Asn Arg
   850 855 860
   Ser Ile Ala Ala Lys Leu Leu Ala Arg Leu Ile Asn Gly Gly Tyr Asp
   865 870 875 880
   Leu Pro Ala Asp Ile Phe Ala Asn Leu Ala Val Pro Pro Asp Pro Phe
   885 890 895
   Phe Thr Arg Ala Gln Ile Asp Arg Glu Ala Arg Lys Val Arg Asp Gly
   900 905 910
   Ile Met Gly Met Leu Tyr Leu Gln Arg Met Pro Thr Glu Phe Asp Val
   915 920 925
   Ala Met Ala Thr Val Tyr Tyr Leu Ala Asp Arg Asn Val Ser Gly Glu
   930 935 940
   Thr Phe His Pro Ser Gly Gly Leu Arg Tyr Glu Arg Thr Pro Thr Gly
   945 950 955 960
   Gly Glu Leu Phe Gly Leu Pro Ala Pro Glu Arg Leu Ala Glu Leu Val
   965 970 975
   Gly Ser Thr Val Tyr Leu Ile Gly Glu His Leu Thr Glu His Leu Asn
   980 985 990
   Leu Leu Ala Arg Ala Tyr Leu Glu Arg Tyr Gly Ala Arg Gln Val Val
   995 1000 1005
   Met Ile Val Glu Thr Glu Ala Gly Ala Glu Lys Met Arg His Leu
   1010 1015 1020
   Leu His Asp His Val Glu Ala Gly Arg Leu Pro Ile Ile Val Ala
   1025 1030 1035
   Gly Asp Gln Ile Glu Ala Ala Ile Asp Gln Ala Ile Ala Asn Tyr
   1040 1045 1050
   Gly Arg Pro Gly Pro Val Val Cys Thr Pro Phe Arg Pro Leu Pro
   1055 1060 1065
   Ser Ala Pro Leu Val Gly Arg Lys Asp Ser Asp Trp Ser Thr Val
   1070 1075 1080
   Leu Ser Glu Ala Glu Phe Ala Glu Leu Cys Glu His Gln Leu Thr
   1085 1090 1095
   His His Phe Arg Val Ala Arg Lys Ile Ala Leu Ser Asp Gly Ala
       1100                1105                1110
   Ser Leu Ala Leu Val Thr Pro Glu Thr Thr Ala Thr Ser Ser Thr
       1115                1120                1125
   Glu Gln Phe Ala Leu Ala Asn Phe Val Lys Thr Thr Leu His Ala
       1130                1135                1140
   Phe Thr Ala Thr Ile Gly Val Glu Ser Glu Arg Thr Ala Gln Arg
       1145                1150                1155
   Ile Leu Ile Asn Gln Val Asp Leu Thr Arg Arg Ala Arg Ala Glu
       1160                1165                1170
   Glu Pro Arg Asp Pro Arg Glu Arg Gln Gln Glu Leu Glu Arg Phe
       1175                1180                1185
   Ile Glu Ala Val Leu Leu Val Thr Ala Pro Leu Pro Pro Glu Ala
       1190                1195                1200
   Asp Thr Arg Tyr Ala Gly Arg Ile His Arg Gly Arg Ala Ile Thr
       1205                1210                1215
   Val
<210> 44
   <211> 1229
   <212> PRT
   <213> Roseiflexus castenholzii
<400> 44
   Met Ser Thr Val Arg Arg Leu Glu Gly Lys Val Ala Leu Ile Thr Gly
   1               5                   10                  15
   Gly Ala Gly Asn Ile Gly Glu Val Ile Thr Arg Arg Phe Leu Ala Glu
               20                  25                  30
   Gly Ala Thr Val Val Ile Thr Gly Arg Asn Ala Glu Lys Leu Ala Val
           35                  40                  45
   Tyr Arg Arg Arg Leu Ile Asp Glu Glu Arg Val Ala Pro Glu Arg Val
       50                  55                  60
   Val Ala Leu Arg Met Asp Gly Ser Asp Ile Ala Gln Val Arg Ala Gly
   65                  70                  75                  80
   Val Ala Gln Ile Val His Gly Gly Thr Asp Val Pro Ile Pro Leu His
   85 90 95
   Arg Ile Asp Ile Leu Val Asn Asn Ala Gly Ser Ala Gly Pro Arg Arg
   100 105 110
   Arg Leu Val Asp Ile Pro Leu Glu Pro Ser Glu Val Gln Pro Pro Asp
   115 120 125
   Ser Glu Thr Leu Ala Gln Ala Val Gly Asn Leu Val Gly Ile Thr Trp
   130 135 140
   Asn Leu Thr Arg Ala Ala Ala Pro His Met Pro Ser Gly Ser Ser Val
   145 150 155 160
   Ile Asn Ile Ser Thr Ile Phe Ser Arg Thr Asp Tyr Tyr Gly Arg Ile
   165 170 175
   Ala Tyr Val Ala Pro Lys Ala Ala Leu Asn Ala Leu Ser Asp Gly Leu
   180 185 190
   Ala Arg Glu Leu Gly Val Arg Gly Ile Arg Val Asn Thr Ile Tyr Pro
   195 200 205
   Gly Pro Ile Glu Ser Glu Arg Ile Tyr Thr Met Phe Gln Ala Met Asp
   210 215 220
   Ala Leu Lys Gly Gln Pro Glu Gly Asp Thr Ala Ser Gly Phe Leu Arg
   225 230 235 240
   Met Met Arg Leu Ser Arg Ile Asp Gln Asn Gly Glu Val Val Lys Arg
   245 250 255
   Phe Pro Ser Pro Val Asp Val Ala Asn Thr Ala Val Phe Leu Ala Ser
   260 265 270
   Asp Glu Ser Ala Ala Phe Thr Gly His Ala Phe Glu Val Thr His Gly
   275 280 285
   Met Glu Val Pro Thr Glu Ser Arg Thr Thr Phe Val Ser Arg Pro Gly
   290 295 300
   Leu Arg Ser Val Asp Ala Thr Gly Lys Val Ile Leu Ile Cys Ala Gly
   305 310 315 320
   Asp Gln Val Asp Asp Ala Val Ala Leu Ala Asp Thr Leu Arg Ser Cys
   325 330 335
   Arg Ala Thr Val Val Ile Gly Phe Arg Asp Pro Arg Ala Leu Glu Lys
               340                 345                 350
   Ala Ser Val Leu Leu Arg Glu Pro Arg His Ala Leu Ala Ala Asp Met
           355                 360                 365
   Tyr Gly Arg Pro Thr Met Thr Ala Glu Ala Arg Leu Val Arg Leu Asp
       370                 375                 380
   Pro Leu Asp Pro Arg Ala Ala Ala Gln Thr Leu Glu Gln Ile His Ala
   385                 390                 395                 400
   Glu Leu Gly Ala Ile His His Ala Val Val Leu Pro Gly Gln Ser Arg
                   405                 410                 415
   His Ala Pro Ser Ala Ser Leu Ile Glu Val Asp Asp Gln Val Val Glu
               420                 425                 430
   Arg Phe Leu His Gln Glu Leu Val Gly Thr Ile Ala Leu Ala Arg Glu
           435                 440                 445
   Leu Ala Arg Phe Trp Glu Glu Tyr Pro Ser Gly Ser Ser Met His Arg
       450                 455                 460
   Val Leu Phe Val Ser Asn Pro Asp Asp Gln Gln Gly Asn Gln Tyr Ser
   465                 470                 475                 480
   His Ile Leu Arg Ala Ala Val Glu Gln Leu Val Arg Val Trp Arg His
                   485                 490                 495
   Glu Ser Glu Tyr Asp Ser Val Asn Pro Ala His Gln Gln Glu Gly Gln
               500                 505                 510
   Ser Ser Ala Ala Val Trp Ala Asn Gln Leu Ile Arg Tyr Val Asn Asn
           515                 520                 525
   Glu Met Ala Asn Leu Asp Phe Thr Cys Ala Trp Val Ala Lys Leu Leu
       530                 535                 540
   Gly Ser Asp Arg Arg Ile Ala Glu Ile Asn Leu Tyr Leu Pro Glu Glu
   545                 550                 555                 560
   Ile Val Gly Thr Ile Gly Val His Asn Pro Gly Phe Gly Trp Ala Glu
                   565                 570                 575
   Ser Leu Phe Gly Leu His Met Gly Lys Val Ala Leu Ile Thr Gly Gly
               580                 585                 590
   Ser Ala Gly Ile Gly Gly Gln Ile Gly Arg Leu Leu Ala Leu Ser Gly
   595 600 605
   Ala His Val Met Leu Ala Ala Arg Asn Ala Asp Gln Leu Glu Gln Met
   610 615 620
   Arg Ala Ser Ile Val Arg Glu Val Arg Asp Ala Ser Tyr Pro Asp Ala
   625 630 635 640
   Glu Ser Arg Val Ala Ile Phe Pro Gly Ser Asp Val Ser Asp Ile Asp
   645 650 655
   Gly Leu Glu Arg Leu Val Asn His Thr Val Arg Val Phe Gly Lys Val
   660 665 670
   Asp Tyr Leu Ile Asn Asn Ala Gly Ile Ala Gly Ala Glu Glu Met Val
   675 680 685
   Ile Asp Met Pro Val Asp Ala Trp Arg His Thr Leu Arg Ala Asn Leu
   690 695 700
   Ile Ser Asn Tyr Ala Leu Leu Arg Arg Leu Ala Pro Gln Met Lys Ala
   705 710 715 720
   Ala Gly Gly Ala Tyr Val Leu Asn Val Ser Ser Tyr Phe Gly Gly Glu
   725 730 735
   Lys Tyr Val Ala Ile Pro Tyr Pro Asn Arg Ser Asp Tyr Ala Val Ser
   740 745 750
   Lys Ala Gly Gln Arg Ala Met Val Glu Ser Leu Ala Arg Phe Leu Gly
   755 760 765
   Pro Glu Ile Gln Ile Asn Ala Ile Ala Pro Gly Pro Val Glu Gly Glu
   770 775 780
   Arg Leu Lys Gly Ala Gly Ser Arg Pro Gly Leu Phe Met Arg Arg Ala
   785 790 795 800
   Arg Leu Ile Leu Glu Asn Lys Arg Leu Asn Glu Val Phe Ala Ala Leu
   805 810 815
   Leu Ala Ala Arg His Glu Gly Ala Thr Ile Ala Asp Leu Leu Pro Asp
   820 825 830
   Leu Phe Ala Asn Asp Ile Gln Ser Ile Ala Asn Ser Ala Ala Met Pro
   835 840 845
   Ala Pro Leu Arg Arg Leu Ala Thr Met Leu Arg Glu Thr Ser Asp Ala
   850 855 860
   Gly Gly Ser Ala Gln Ser Tyr Leu Met Asn Ala Thr Ile Ala Arg Lys
   865 870 875 880
   Leu Leu Asn Arg Leu Glu Asn Gly Gly Tyr Ile Thr Leu His Asp Arg
   885 890 895
   Arg Ala Leu Thr Val Glu Pro Pro Glu Pro Phe Phe Thr Glu Ala Gln
   900 905 910
   Ile Glu Arg Glu Ala Ile Lys Val Arg Asp Gly Ile Leu Gly Met Leu
   915 920 925
   His Leu Gln Arg Met Pro Thr Glu Phe Asp Val Ala Leu Ala Thr Val
   930 935 940
   Phe Tyr Leu Ala Asp Arg Asn Val Thr Gly Glu Thr Phe His Pro Ser
   945 950 955 960
   Gly Gly Leu Arg Phe Glu Arg Thr Val Thr Glu Gly Glu Leu Phe Gly
   965 970 975
   Lys Pro Gly Gln Gln Arg Leu Glu Arg Leu Lys Gly Ser Val Val Tyr
   980 985 990
   Leu Ile Gly Glu His Leu Arg Gln His Leu Val Leu Leu Ala Arg Thr
   995 1000 1005
   Phe Leu Asp Glu Ile His Val Ala Arg Val Val Leu Leu Thr Glu
   1010 1015 1020
   Thr Thr Gln Ala Ala Thr Asp Leu Ala Ala Glu Leu Ser Asp Tyr
   1025 1030 1035
   Glu Ala Ala Gly Arg Phe Val Val Ile Pro Thr Cys Gly Asp Ile
   1040 1045 1050
   Glu Gly Gly Ile Asp Arg Ala Met Ala Glu Tyr Gly Arg Pro Gly
   1055 1060 1065
   Pro Val Ile Ser Thr Pro Phe Arg Pro Leu Pro Asp Arg Ala Leu
   1070 1075 1080
   Ser Ala Arg Asn Gly Asp Trp Ser Ser Val Leu Thr Thr Ala Glu
   1085 1090 1095
   Phe Glu Glu Leu Val Glu Gln Gln Ile Thr His His Phe Arg Val
   1100 1105 1110
   Ala Arg Lys Ala Gly Leu Ile Glu Gly Ala Asn Val Thr Leu Val
   1115 1120 1125
   Thr Pro Pro Thr Ser Ala Arg Ser Thr Ser Glu Glu Phe Ala Leu
   1130 1135 1140
   Ala Asn Phe Val Lys Thr Thr Leu His Ala Leu Thr Ala Thr Ala
   1145 1150 1155
   Gly Ala Glu Ser Glu Arg Thr Val Pro His Val Pro Val Asn Gln
   1160 1165 1170
   Val Asp Leu Thr Arg Arg Ala Arg Ser Glu Glu Pro Arg Thr Pro
   1175 1180 1185
   Ser Glu Glu Glu Glu Glu Leu Gln Arg Phe Val Asn Ala Val Leu
   1190 1195 1200
   Leu Thr Ser Ala Pro Leu Pro Thr Pro Leu Glu Ser Arg Tyr Arg
   1205 1210 1215
   Ala Arg Ile Tyr Arg Gly Asn Ala Ile Thr Val
   1220 1225
<210> 45
   <211> 1232
   <212> PRT
   <213> Roseiflexus sp.
<400> 45
   Met Ser Thr Thr Arg Arg His Asp Arg Leu Glu Gly Lys Val Ala Leu
   1               5                   10                  15
   Ile Thr Gly Gly Ala Gly Asn Ile Gly Glu Val Ile Thr Arg Arg Phe
               20                  25                  30
   Leu Ala Glu Gly Ala Thr Val Val Ile Thr Gly Arg Asn Val Glu Lys
           35                  40                  45
   Leu Ala Ala Tyr Arg Arg Arg Leu Ile Asp Glu Glu Arg Ile Ala Pro
       50                  55                  60
   Asp Arg Val Val Ala Leu Arg Met Asp Gly Ser Asp Met Ala Gln Val
   65                  70                  75                  80
   Arg Ala Ala Ile Ala Gln Ile Val His Gly Gly Ala Asp Val Pro Thr
   85 90 95
   Pro Leu Lys Arg Ile Asp Ile Leu Val Asn Asn Ala Gly Ser Ala Gly
   100 105 110
   Pro Arg Arg Arg Leu Val Asp Ile Pro Leu Glu Pro Ser Glu Val His
   115 120 125
   Pro Pro Asp Thr Glu Thr Leu Ala Gln Ala Val Gly Asn Leu Val Gly
   130 135 140
   Val Ala Trp Asn Leu Thr Arg Ala Ala Ala Pro Tyr Met Pro Pro Gly
   145 150 155 160
   Ser Ser Ile Ile Asn Val Ser Thr Ile Phe Ser Arg Thr Asp Tyr Tyr
   165 170 175
   Gly Arg Ile Ala Tyr Val Val Pro Lys Ala Ala Leu Asn Ala Leu Ser
   180 185 190
   Gln Gly Leu Ala Arg Glu Leu Gly Val Arg Gly Ile Arg Val Asn Thr
   195 200 205
   Ile Tyr Pro Gly Pro Ile Glu Ser Glu Arg Ile His Thr Val Phe Gln
   210 215 220
   Ala Met Asp Ala Leu Lys Gly Gln Pro Glu Gly Glu Thr Ala Arg Ser
   225 230 235 240
   Phe Leu Arg Leu Met Arg Leu Ser Arg Ala Asp Thr Gln Gly Glu Val
   245 250 255
   Thr Lys Arg Phe Pro Leu Pro Val Asp Val Ala Asn Thr Ala Val Phe
   260 265 270
   Leu Ala Ser Glu Glu Ser Ala Ala Phe Thr Gly His Ala Phe Glu Val
   275 280 285
   Thr His Gly Met Glu Ala Pro Val Glu Ser Arg Thr Thr Phe Val Ser
   290 295 300
   Arg Pro Gly Leu Arg Ser Val Asp Ala Thr Gly Asn Val Ile Leu Ile
   305 310 315 320
   Cys Ala Gly Asp Gln Val Asp Asp Ala Ile Ala Leu Ser Asp Thr Leu
   325 330 335
   Arg Ser Cys Arg Ala Thr Val Val Val Gly Phe Arg Asp Val Gln Ala
   340 345 350
   Leu Glu Lys Ala Ser Ala Leu Leu Arg Glu Pro Arg His Val Ala Pro
   355 360 365
   Ala Asp Met Tyr Gly Arg Pro Thr Met Val Ala Glu Thr Arg Leu Val
   370 375 380
   His Leu Asp Pro Leu Asp Ser Arg Ala Ala Ala Gln Thr Leu Glu Arg
   385 390 395 400
   Ile Arg Ala Glu Leu Gly Thr Leu His His Ala Val Ile Leu Pro Ala
   405 410 415
   Ser Leu Arg His Ala Pro Thr Glu Ser Leu Ile Asp Val Asp Asp Arg
   420 425 430
   Ile Ile Asp Arg Phe Leu Glu Gln Glu Leu Val Gly Ala Ile Ala Leu
   435 440 445
   Ala Arg Glu Leu Ala Arg Phe Trp Glu Glu His Pro Ala Gly Ser Arg
   450 455 460
   Ala His Arg Val Leu Phe Val Ser Asn Pro Asp Asp Gln Gln Gly Asn
   465 470 475 480
   Arg Tyr Ala Asp Ile Leu Arg Ala Ala Val Glu Gln Leu Ala Arg Val
   485 490 495
   Trp Arg His Glu Ser Glu Tyr Asp Ala Ala Asn Pro Ala His Arg His
   500 505 510
   Glu Asp Gln Pro Gly Ala Ala Val Trp Ala Asn Gln Leu Ile Arg Tyr
   515 520 525
   Val Asn Ser Glu Ser Ala Asn Leu Asp Phe Thr Cys Ala Trp Val Ala
   530 535 540
   Lys Leu Leu Gly Ser Asp Arg Arg Ile Ala Glu Ile Asn Leu Tyr Leu
   545 550 555 560
   Pro Glu Gln Ile Val Ser Thr Ile Gly Val His Asn Pro Gly Phe Gly
   565 570 575
   Trp Ala Glu Ser Leu Phe Gly Leu His Met Gly Lys Val Ala Leu Ile
   580 585 590
   Thr Gly Gly Ser Ala Gly Ile Gly Ala Gln Ile Gly Arg Leu Leu Ala
   595 600 605
   Leu Ser Gly Ala His Val Met Leu Ala Ala Arg Asn Ala Glu Gln Leu
   610 615 620
   Glu Gln Met Arg Ala Leu Ile Val Arg Glu Val Arg Asp Ala Ser Tyr
   625 630 635 640
   Pro Asp Ala Glu Ser Arg Val Ala Ile Phe Pro Asn Ser Asp Val Ser
   645 650 655
   Asp Ile Asp Gly Leu Glu Arg Leu Val Asn His Thr Leu Arg Val Phe
   660 665 670
   Gly Lys Val Asp Tyr Leu Ile Asn Asn Ala Gly Ile Ala Gly Ala Glu
   675 680 685
   Glu Met Val Ile Asp Met Pro Val Asp Ala Trp Arg His Thr Leu Arg
   690 695 700
   Ala Asn Leu Ile Ser Asn Tyr Ala Leu Leu Arg Arg Leu Ala Pro Gln
   705 710 715 720
   Met Lys Ala Ala Gly Gly Ala Tyr Val Leu Asn Val Ser Ser Tyr Phe
   725 730 735
   Gly Gly Glu Lys Tyr Val Ala Ile Pro Tyr Pro Asn Arg Ala Asp Tyr
   740 745 750
   Ala Val Ser Lys Ala Gly Gln Arg Ala Met Val Glu Ser Leu Ala Arg
   755 760 765
   Phe Leu Gly Pro Glu Ile Gln Ile Asn Ala Ile Ala Pro Gly Pro Val
   770 775 780
   Glu Gly Glu Arg Leu Lys Gly Ala Ala Asn Arg Pro Gly Leu Phe Met
   785 790 795 800
   Arg Arg Ala Arg Leu Ile Leu Glu Asn Lys Arg Leu Asn Glu Val Phe
   805 810 815
   Ala Ala Leu Leu Ala Ala Arg His Glu Gly Ala Val Ile Ala Ala Leu
   820 825 830
   Leu Pro Asp Leu Phe Val Asn Asp Leu Gln Thr Ile Ala Asp Asn Pro
   835 840 845
   Ala Met Pro Ala Pro Val Arg Arg Leu Ala Thr Met Leu Arg Glu Thr
   850 855 860
   Thr Asp Ala Gly Gly Ser Ala Gln Ser Tyr Leu Met Asn Ala Thr Ile
   865 870 875 880
   Ala Arg Lys Leu Leu Asn Arg Leu Glu Asn Gly Gly Tyr Ile Thr Ala
   885 890 895
   Gly Asp Arg Arg Ala Leu Thr Asn Glu Pro Pro Asp Pro Phe Phe Thr
   900 905 910
   Glu Ala Gln Ile Glu Arg Glu Ala Ile Lys Val Arg Asp Gly Ile Leu
   915 920 925
   Gly Met Leu His Leu Gln Arg Met Pro Thr Glu Phe Asp Val Ala Leu
   930 935 940
   Ala Thr Val Phe Tyr Leu Ala Asp Arg Asn Val Thr Gly Glu Thr Phe
   945 950 955 960
   His Pro Ser Gly Gly Leu Arg Phe Glu Arg Thr Val Thr Glu Gly Glu
   965 970 975
   Leu Phe Gly Lys Pro Gly Arg Gln Arg Leu Glu Arg Met Ala Gly Ser
   980 985 990
   Val Val Tyr Leu Ile Gly Glu His Leu Arg Gln His Leu Leu Leu Leu
   995 1000 1005
   Ala Arg Thr Phe Leu Asp Glu Ile His Val Ala Arg Val Val Leu
   1010 1015 1020
   Leu Thr Glu Thr Glu Gln Ala Ala Ala Glu Leu Ala Thr Val Phe
   1025 1030 1035
   Ala Asp Glu Glu Ala Ala Gly Arg Phe Val Ile Ile Pro Thr Gly
   1040 1045 1050
   Gly Asp Ile Glu Gly Gly Ile Asp Arg Ala Met Ala Glu Tyr Gly
   1055 1060 1065
   Arg Pro Gly Pro Val Ile Ser Thr Pro Phe Arg Pro Leu Pro Ser
   1070 1075 1080
   Arg Ala Leu Ser Ala Gln Asn Gly Asp Trp Ser Asn Val Leu Thr
   1085 1090 1095
   Thr Pro Glu Phe Glu Glu Leu Val Glu Gln His Ile Thr His His
   1100 1105 1110
   Phe Arg Val Val Arg Lys Ala Gly Leu Ile Glu Gly Ala Asn Val
   1115 1120 1125
   Thr Leu Val Thr Pro Pro Thr Ser Ala Arg Ser Thr Ala Glu Glu
   1130 1135 1140
   Phe Ala Leu Ala Asn Phe Ile Lys Thr Thr Leu His Ala Leu Thr
   1145 1150 1155
   Ala Thr Ala Gly Ala Glu Ser Glu Arg Thr Met Pro His Val Pro
   1160 1165 1170
   Val Asn Gln Val Asp Leu Thr Arg Arg Ala Arg Ser Glu Glu Pro
   1175 1180 1185
   Arg Thr Pro Ala Glu Glu Glu Glu Glu Leu Gln Arg Phe Val Asn
   1190 1195 1200
   Ala Val Leu Leu Thr Ser Ala Pro Leu Pro Thr Pro Leu Glu Ser
   1205 1210 1215
   Arg Tyr Arg Ala Arg Ile Tyr Arg Gly Asn Ala Ile Thr Val
   1220 1225 1230
<210> 46
   <211> 1217
   <212> PRT
   <213> Erythrobacter sp.
<400> 46
   Met Ser Lys Glu Gly Asn Ala Ala Lys Gly Arg Leu Glu Gly Lys Val
   1 5 10 15
   Ala Leu Ile Thr Gly Ala Ala Gly Asn Leu Gly Asn Glu Ile Ser Arg
   20 25 30
   Ala Phe Ala Arg Glu Gly Ala Phe Val Val Met Thr Gly Arg Thr Glu
   35 40 45
   Glu Arg Ile Ser Ala Ala Arg Glu Gln Leu Ile Ala Asp Thr Gly Val
   50 55 60
   Ala Pro Glu Arg Ile Asp Thr Ala Val Leu Asp Gly Gly Asn Pro Asp
   65 70 75 80
   Ser Ile Arg Ala Ala Met Ala Lys Leu Arg Lys Glu Tyr Gly Arg Ile
   85 90 95
   Asp Ile Leu Ile Asn Asn Ala Gly Ser Ala Gly Pro Lys Gln Pro Leu
   100 105 110
   His Asn Val Pro Leu Ser Pro Gln Glu Met Glu Ala Cys Gly Asp Thr
   115 120 125
   Glu Thr Val Arg Asp Ala Met Leu Asn Ile Leu Gly Val Thr Trp Asn
   130 135 140
   Met Ala Arg Ile Val Ala Pro Met Met Pro Val Gly Gly Ala Met Val
   145 150 155 160
   Asn Ile Ser Thr Ile Phe Ser His Thr Arg Tyr Tyr Gly Arg Thr Ala
   165 170 175
   Tyr Val Val Pro Lys Ala Ala Leu Asn Ala Leu Ser Asn Gln Leu Ala
   180 185 190
   Ser Glu Leu Gly Pro Arg Gly Ile Arg Val Asn Thr Val Phe Pro Gly
   195 200 205
   Pro Ile Glu Ser Asp Arg Ile Arg Thr Val Phe Ala Ala Met Asp Glu
   210 215 220
   Val Gln Ser Gln Pro Lys Asp Thr Thr Ala Asn Tyr Phe Thr Gly Arg
   225 230 235 240
   Met Ala Leu Thr Arg Ser Val Asn Gly Lys Val Asp Gly Lys Pro Leu
   245 250 255
   Pro Asn Pro Lys Asp Ile Ala Gly Thr Cys Leu Phe Leu Ala Ser Glu
   260 265 270
   Glu Ala Ala Gly Ile Ala Gly Glu Glu Val Asp Val Thr His Gly Leu
   275 280 285
   Ser Ala Asn Arg Thr Ser Ala Ser Thr Tyr Met Thr Arg Pro Ser Met
   290 295 300
   Arg Ser Leu Asp Gly Ala Gly Leu Asn Ile Phe Ile Val Ser Gly Glu
   305 310 315 320
   Asn Trp Asp Asp Ala Leu Val Ala Ala His Thr Leu Ile Gly Ser Gly
                   325                 330                 335
   Ala Lys Val Arg Leu Gly Leu Ala Arg Asn Ala Asp Val Ala Gln Ala
               340                 345                 350
   Asn Ala Arg Leu Lys Ala Gln Gly Ile Gly Glu Glu Leu Thr Val Thr
           355                 360                 365
   Arg Phe Asn Arg Ala Glu Pro Asp Ala Met Glu Asp Ala Leu Ala Ala
       370                 375                 380
   Phe Ser Gly Asp Val Asp Gly Ala Ile Thr Gly Ala Ile Ile Leu Pro
   385                 390                 395                 400
   Val Lys Pro Ser Gly His Phe Thr Gly Ser Leu Leu Ala Ala Asp Asp
                   405                 410                 415
   Asp Thr Val Thr Lys Phe Met Asp Thr Glu Leu Val Gly Ala Ile Ala
               420                 425                 430
   Val Ser Arg Ser Leu Ala Arg Tyr Trp His Gly Arg Glu Asp Leu Gln
           435                 440                 445
   Ser Pro Pro Arg Cys Val Phe Met Thr Asn Pro Gly Asp Pro Leu Gly
       450                 455                 460
   Asn Ser Phe Ala Ser Val Leu Ser Ala Gly Ile Thr Gln Leu Ile Arg
   465                 470                 475                 480
   Ile Trp Arg Asp Glu Glu Arg Val Gln Ala Gly Asn Gly Ser Thr Glu
                   485                 490                 495
   His Ala Val Trp Ser Asn Gln Ile Val Arg His Thr Asn Thr Glu Asp
               500                 505                 510
   Glu Asn Thr Arg Phe Ala Ser Gly His Ala Thr Arg Val Leu Phe Arg
           515                 520                 525
   Glu Gln His Ile Ala Glu Ile Asp Leu Lys Leu Pro Ala Asn Ile Ser
       530                 535                 540
   Glu Glu Thr Gly Ser Arg Lys Ala Met Val Gly Phe Ala Glu Asn Ile
   545                 550                 555                 560
   Thr Gly Leu His Leu Gly Lys Val Ala Phe Ile Thr Gly Gly Ser Ala
   565 570 575
   Gly Ile Gly Gly Gln Val Ala Arg Leu Leu Ala Leu Ala Gly Ala Lys
   580 585 590
   Val Met Met Val Ala Arg Arg Glu Ser Glu Leu Val Ala Ala Arg Asp
   595 600 605
   Arg Ile Val Gly Glu Leu Gln Asp Ile Gly Phe Ala Gly Val Glu Arg
   610 615 620
   Arg Val Lys Tyr Met Ala Asp Ile Asp Val Ser Asp Phe Ala Ser Leu
   625 630 635 640
   Asp Lys Ala Val Asp Ala Thr Leu Glu Glu Phe Gly Arg Ile Asp Tyr
   645 650 655
   Leu Ile Asn Asn Ala Gly Val Ala Gly Ala Glu Asp Met Val Ile Asp
   660 665 670
   Met Glu Pro Glu Ala Trp Arg Phe Thr Leu Asp Ala Asn Leu Ile Ser
   675 680 685
   Asn Tyr His Leu Met Gln Arg Val Val Pro Leu Met Lys Glu Gln Gly
   690 695 700
   Ser Gly Tyr Val Leu Asn Val Ser Ser Tyr Phe Gly Gly Glu Lys Phe
   705 710 715 720
   Leu Ala Val Ala Tyr Pro Asn Arg Ala Asp Tyr Gly Leu Ser Lys Ala
   725 730 735
   Gly Gln Arg Ala Met Val Glu Ala Phe Ser Pro Phe Leu Gly Pro Glu
   740 745 750
   Val Gln Cys Asn Ala Ile Ala Pro Gly Pro Val Asp Gly Asp Arg Leu
   755 760 765
   Ser Gly Thr Gly Gly Lys Pro Gly Leu Phe Gln Arg Arg Ala Lys Leu
   770 775 780
   Ile Leu Glu Asn Lys Arg Leu Asn Ala Val Tyr Ser Ala Val Ile His
   785 790 795 800
   Ala Ile Arg Glu Gly Gly Asp Ala Ala Lys Ile Leu Thr Arg Leu Ser
   805 810 815
   Arg Asn Ser Thr Ser Thr Leu Ser His Asp Ala Glu Ala Pro Glu Glu
   820 825 830
   Leu Arg Lys Leu Ala Leu Asp Phe Ala Ser Gln Gly Asp Gly Leu Cys
   835 840 845
   Thr Trp Asp Gln Tyr Leu Leu Thr Asp Ala Met Ala Gln Arg Leu Leu
   850 855 860
   Val Arg Leu Gln Leu Gly Gly Phe Leu Leu Gly Ser Asn Glu Trp Ala
   865 870 875 880
   Ser Leu Ser Ser Ser Glu Gln Thr Trp Leu Lys Leu Ser Pro Pro Asp
   885 890 895
   Asp Lys Pro Phe Leu Pro Ala Ala Gln Val Asp Lys Val Ala Asn Gly
   900 905 910
   Val Gly Lys Gly Val Ile Ser Gln Leu His Leu Gly Ala Met Pro Thr
   915 920 925
   Glu Ala Glu Val Ala Gln Ala Thr Val Phe Phe Leu Ala Asp Arg Ala
   930 935 940
   Val Ser Gly Glu Thr Phe Met Pro Ser Gly Gly Leu Arg Val Glu Arg
   945 950 955 960
   Ser Asn Thr Glu Arg Glu Met Phe Gly Ser Pro Lys Gln Glu Arg Ile
   965 970 975
   Asp Lys Met Lys Gly Lys Thr Val Trp Ile Ile Gly Glu His Leu Ser
   980 985 990
   Asp Tyr Val Ala Ala Thr Ile Glu Glu Leu Val Ser Gly Cys Gly Val
   995 1000 1005
   Ala Lys Val Val Leu Ile Ala Lys Asp Lys Ser Gly Glu Lys Ala
   1010 1015 1020
   Val Arg Asp Gln Leu Pro Asn Asp Leu Ser Lys Asp Ala Leu Glu
   1025 1030 1035
   Val Leu Ile Ala Gly Asp Gly Leu Glu Glu Ala Met Asp Glu Ala
   1040 1045 1050
   Leu Gly His Trp Gly Lys Pro Thr Thr Val Leu Ser Met Pro Gly
   1055 1060 1065
   Glu Pro Leu Pro Asp His Leu Phe Glu Gly Gly Asn Pro Leu Ser
   1070 1075 1080
   Thr Lys Asp Phe Ala His Met Val Glu Ala Asn Ile Thr Arg His
   1085 1090 1095
   Tyr Arg Val Thr Arg Lys Ala Ser Leu Tyr Asp Gly Cys Gln Val
   1100 1105 1110
   Val Leu Val Ser Pro Asp Val Pro Tyr Gly Ser Asp Gly Pro Gly
   1115 1120 1125
   Val Ala Leu Ala Asn Phe Val Lys Thr Ser Leu His Ala Phe Thr
   1130 1135 1140
   Ala Thr Val Ala Val Glu Asn Glu Arg Leu Val His Asp Val Pro
   1145 1150 1155
   Val Asn Gln Ile Asn Leu Thr Arg Arg Val Ser Ser Glu Glu Pro
   1160 1165 1170
   Arg Asp Ala Asp Glu His Ala Glu Glu Leu Arg Arg Phe Thr Arg
   1175 1180 1185
   Ala Val Leu Leu Val Gly Ala Pro Leu Pro Asp Ala Gln Asp Ser
   1190 1195 1200
   Arg Tyr Arg Ser Lys Ile Tyr Arg Gly Thr Ser Met Thr Val
   1205 1210 1215
<210> 47
   <211> 1210
   <212> PRT
   <213> Unknown
<220>
   <223> Description of Unknown: Gamma proteobacterium NOR51-B polypeptide
<400> 47
   Met Asn Thr Glu Thr Arg Thr Thr Ser Gly Gly Arg Leu His Asp Lys
   1 5 10 15
   Val Val Ile Leu Thr Gly Ala Ala Gly Asn Ile Gly Ser Tyr Ile Ser
   20 25 30
   Arg Ser Leu Leu Arg Glu Gly Ala Asn Leu Val Met Thr Gly Arg Asn
   35 40 45
   Glu Pro Lys Leu Gln Ala Phe Val Glu Gly Leu Val Glu Glu Gly Phe
       50                  55                  60
   Asp Arg Asp Asn Ile Leu Ile Ala Ile Gly Asp Ser Ala Lys Ala Asp
   65                  70                  75                  80
   Ile Cys Arg Glu Ile Val Lys Ala Thr Val Asn His Phe Gly Asn Ile
                   85                  90                  95
   Asp Val Leu Val Asn Asn Ala Gly Gly Ala Gly Pro Arg Arg Thr Leu
               100                 105                 110
   Arg Asp Ile Pro Phe Ser Glu Ser Glu Arg Leu Ala Arg Gly Asp Asp
           115                 120                 125
   Glu Thr Met Leu Asp Ala Ala Met Asn Leu Leu Ala Gly Ala Trp Asn
       130                 135                 140
   Met Thr Arg Ala Ala Val Pro His Met Ser Glu Gly Gly Ser Ile Val
   145                 150                 155                 160
   Asn Val Ser Thr Ile Phe Ser Arg Thr His Tyr Tyr Gly Arg Ile Pro
                   165                 170                 175
   Tyr Val Val Pro Lys Ser Gly Leu Asn Ala Leu Ser Ile Gly Leu Ala
               180                 185                 190
   Lys Glu Leu Gly Glu Glu His Gly Ile Arg Val Asn Thr Leu Phe Pro
           195                 200                 205
   Gly Pro Ile Glu Ser Glu Arg Ile Asp Thr Val Phe Gly Asn Met Asp
       210                 215                 220
   Ala Leu Gln Ser Ala Pro Ala Gly Ala Thr Ser Gln Glu Phe Arg Asp
   225                 230                 235                 240
   Leu Met Ile Thr Arg Arg Glu Asn Pro Asp Gly Glu Tyr Glu Tyr Arg
                   245                 250                 255
   Tyr Pro Thr Pro Asn Asp Val Ala Ser Thr Val Thr Trp Leu Ala Ser
               260                 265                 270
   Glu Glu Ser Ala Ala Leu Ser Gly His His Ile Glu Val Thr Asn Gly
           275                 280                 285
   Met Gln Val Pro Ala Gln Ser Arg Ser Lys Leu Val Ser Trp Pro Asp
       290                 295                 300
   Lys Arg Leu Glu Asp Leu Ser Gly Gln Val Val Phe Leu Leu Ala Gly
   305 310 315 320
   Ser Asp Tyr Glu Asp Ala Leu Ala Phe Ala Glu Arg His Met Val Ser
   325 330 335
   Gly Ala Lys Val Val Leu Ala Phe Arg Ser Leu Glu Ser Leu Gly Leu
   340 345 350
   Ala Arg Ser Leu Cys Ala Ser Arg Asp Leu Glu Ser Ile His Leu Leu
   355 360 365
   His Leu Glu Pro Leu Arg Arg Glu Ser Ala Asp Arg Cys Phe Asp Tyr
   370 375 380
   Ile Arg Asp His Phe Gly Arg Leu Asp Gly Ile Val Val Leu Pro Arg
   385 390 395 400
   Ser Gly Asn Gly Glu His Gly Tyr Ser Leu Ser Thr Ala Gly Asp Asp
   405 410 415
   Asp Val Glu Ala Phe Val Arg Asp Glu Ile Ile Ser Pro Val Ala Phe
   420 425 430
   Ala Ala Ala Leu Ala Ile Asn Leu Asp Arg Trp Gly Ile Leu Glu Glu
   435 440 445
   Ala Pro Ala Leu Thr Tyr Val Thr Asn Pro Thr Asp Gly His Gly Asp
   450 455 460
   Tyr Leu Asn Glu Val Lys Arg Ala Ala Ile Glu Ala Leu Ile Arg Ile
   465 470 475 480
   Trp Arg His Glu Asp Arg Gln Met Arg Lys Lys Gly Glu Arg Glu Trp
   485 490 495
   Ala Met Leu Pro Asn Gln Leu Val Arg Tyr Asp Asn Asn Glu Glu Asp
   500 505 510
   Asn Leu Thr Phe Thr Ala Asp Trp Ala Ala Thr Leu Thr Asn Arg Val
   515 520 525
   Arg Arg Met Asp Pro Ile Asn Leu Trp Val Pro Glu Ser Ile Met Arg
   530 535 540
   Ala Thr Gly Lys Ser Gly Met Pro Gln Ser Ile Gln Arg Val Leu Pro
   545 550 555 560
   Gly Leu His Lys Gly Arg Thr Ala Val Ile Thr Gly Gly Ser Leu Gly
   565 570 575
   Ile Gly Leu Gln Leu Gly Arg Phe Leu Ala Ile Ala Gly Ala Arg Val
   580 585 590
   Leu Leu Ser Ala Arg Ser Lys Glu Lys Leu Glu Glu Ala Arg His Glu
   595 600 605
   Ile Val Glu Glu Leu Arg Gly Val Gly Tyr Pro Asn Ala His Gln Arg
   610 615 620
   Val His Ile Leu Pro Asp Ile Asp Val Gly Asp Glu Glu Ala Leu Glu
   625 630 635 640
   Arg Leu Tyr Asn His Ser Ile Glu Leu Phe Gly Asn Val Asp Phe Leu
   645 650 655
   Ile Asn Asn Ala Gly Ile Ser Gly Ala Glu Glu Met Val Val Asp Met
   660 665 670
   Ser Leu Glu Ala Trp Asn Arg Thr Met Tyr Ala Asn Leu Ile Ser Asn
   675 680 685
   Tyr Ser Leu Ile Arg Lys Tyr Ala Pro Lys Met Lys Ala Asn Gly Tyr
   690 695 700
   Gly Val Val Leu Asn Val Ser Ser Tyr Phe Gly Gly Glu Lys Tyr Val
   705 710 715 720
   Ala Val Ala Tyr Pro Asn Arg Ala Asp Tyr Ala Val Ser Lys Ala Gly
   725 730 735
   Gln Arg Val Leu Ala Glu Ile Leu Ser Arg His Leu Gly Pro Glu Ile
   740 745 750
   Arg Ile Asn Ala Leu Ala Pro Gly Pro Val Asp Gly Ala Arg Leu Arg
   755 760 765
   Gly Leu Gly Gly Ala Pro Gly Leu Phe Glu Arg Arg Gly Arg Leu Val
   770 775 780
   Leu Glu Asn Lys Arg Leu Asn Ser Val His Lys Ala Val Leu Ala Ala
   785 790 795 800
   Leu Arg Glu Gly Ala Thr Pro Glu Val Ile Met Ala Leu Ser Arg Asn
   805 810 815
   Ala Leu Gly Asp Ala Lys Pro Thr Ala Gly Gln Ser Lys Ala Leu Asp
   820 825 830
   Lys Leu Phe Ala Gln Val Glu Asp Ser Pro Glu Gly Gly Asn Ser Thr
   835 840 845
   Ala Phe Leu Leu Asn Arg Asp Leu Ala Glu Lys Leu Met Asn Arg Leu
   850 855 860
   Val Thr Gly Gly Leu Phe Thr Pro Glu Ser Ala Thr Gln Phe Met Glu
   865 870 875 880
   Gly Phe Val Asp Ala Pro Ala Ile Phe Phe Asp Glu Lys Ser Val Asn
   885 890 895
   Lys Ala Ala Ala Gly Ile Glu Ala Gly Ile Leu Asn Arg Leu His Leu
   900 905 910
   His Lys Met Pro Thr Asp Glu Gln Ile Gly Leu Ser Thr Val Phe His
   915 920 925
   Leu Ala Asp Asp Ile Ala Ser Gly Glu Thr Phe His Pro Ser Gly Gly
   930 935 940
   Leu Lys Phe Asp Arg Ser Val Thr Glu Gly Glu Leu Leu Leu Pro Pro
   945 950 955 960
   Asp Arg Asp Ser Leu Ala Lys Leu Lys Gly Lys Arg Val Val Leu Ile
   965 970 975
   Gly Asp Ser Met Arg Glu Glu Leu Ser Ala Ile Gly Asn Gly Phe Ile
   980 985 990
   Asn Gln Gly Val Ala Ser Leu Thr Val Leu Thr Arg Ser Pro Glu Ala
   995 1000 1005
   Cys Glu Glu Val Gln His Ser Leu Gln Lys Ser Asn Ser Val Thr
   1010 1015 1020
   Leu Asp Val Arg Cys Ile Glu Asp Asn Ile Glu Asp Ala Leu Asp
   1025 1030 1035
   Asp Leu Leu Gln Asn Gln Gly Gly Phe Asp Val Val Val Ser Ala
   1040 1045 1050
   Pro Phe  Ser Arg Leu Pro Tyr  Asn Pro Leu Ala Ala  Glu Arg Glu
       1055                 1060                 1065
   Gly Ser  Trp Asn Arg Val Leu  Ser His Thr Asp Phe  Ala Arg Leu
       1070                 1075                 1080
   Ile Asp  Glu Gln Leu Thr His  His Phe Arg Val Ala  Arg Arg Ala
       1085                 1090                 1095
   Ala Leu  Val Pro Asn Cys Gln  Ile Val Leu Leu Thr  Pro Asp Thr
       1100                 1105                 1110
   Ser Phe  Val Ser Ser Arg Glu  Glu Phe Ala Leu Ala  Leu Phe Val
       1115                 1120                 1125
   Lys Asn  Ser Leu His Ala Phe  Thr Val Thr Leu Gly  Val Glu Thr
       1130                 1135                 1140
   Glu Arg  Leu Pro Thr Val Pro  Ala Val Asn Gln Val  Gln Leu Thr
       1145                 1150                 1155
   Arg Arg  Ala Arg Ala Glu Glu  Pro Ala Thr Glu Ser  Glu Leu Gln
       1160                 1165                 1170
   Glu Glu  Met Glu Arg Leu Val  Ser Ala Val Leu Gln  Cys Ala Val
       1175                 1180                 1185
   Pro Ala  Pro Ser Pro Ser Glu  Ser Arg Tyr Leu Ala  Arg Ile Phe
       1190                 1195                 1200
   Arg Gly  Asn Ala Val Thr Val
       1205                 1210
<210> 48
   <211> 1214
   <212> PRT
   <213> Unknown
<220>
   <223> Description of Unknown: Gamma proteobacterium NOR5-3 polypeptide
<400> 48
   Met Asn Asn Gln Asn Thr Thr Gln Thr His Ser Thr Ser Gly Ala Arg
   1               5                   10                  15
   Leu Asp Asn Lys Val Val Ile Leu Thr Gly Ala Ala Gly Asn Ile Gly
               20                  25                  30
   Ala Phe Val Ser Arg Asn Leu Leu Arg Glu Gly Ala Lys Leu Val Met
           35                  40                  45
   Thr Gly Arg Asn Glu Pro Lys Leu Lys Ser Phe Val Glu Glu Leu Val
       50                  55                  60
   Gly Glu Gly Phe Asp Arg Glu Lys Ile Leu Ile Ala Ile Gly Asp Ser
   65                  70                  75                  80
   Ala Lys Ala Asp Val Cys Arg Arg Ile Val Ala Ser Ala Val Glu Gln
                   85                  90                  95
   Phe Gly Lys Ile Asp Val Leu Val Asn Asn Ala Gly Gly Ala Gly Pro
               100                 105                 110
   Arg Arg Thr Leu Arg Glu Ile Pro Phe Ser Glu Ser Glu Arg Leu Ala
           115                 120                 125
   Arg Gly Asp Asp Glu Thr Met Phe Asp Ala Ala Met Asn Leu Leu Ala
       130                 135                 140
   Gly Pro Trp Asn Met Thr Arg Ala Ala Val Pro His Met Val Arg Gly
   145                 150                 155                 160
   Gly Ser Ile Val Asn Val Ser Thr Ile Phe Ser Arg Thr His Tyr Tyr
                   165                 170                 175
   Gly Arg Ile Pro Tyr Val Val Pro Lys Ser Gly Leu Asn Ala Leu Ser
               180                 185                 190
   Ile Gly Leu Ala Lys Glu Leu Gly Glu Glu His Gly Ile Arg Val Asn
           195                 200                 205
   Thr Leu Phe Pro Gly Pro Ile Glu Ser Glu Arg Ile Asp Thr Val Phe
       210                 215                 220
   Gly Asn Met Asp Ala Leu Gln Ser Ala Pro Ala Gly Thr Thr Ser Gln
   225                 230                 235                 240
   Glu Phe Arg Asp Leu Met Ile Thr Arg Arg Glu Asn Pro Glu Gly Glu
                   245                 250                 255
   Phe Glu Tyr Arg Tyr Pro Thr Pro Asn Asp Val Ala Ser Thr Val Thr
               260                 265                 270
   Trp Leu Ala Cys Gly Glu Ser Ala Ala Leu Ser Gly His His Ile Glu
           275                 280                 285
   Val Thr Asn Gly Met Gln Val Pro Ala Gln Ser Arg Ser Lys Leu Val
   290 295 300
   Ser Trp Pro Asp Lys Arg Leu Glu Asp Leu Ser Gly Gln Val Val Phe
   305 310 315 320
   Val Leu Ala Gly Ser Asp Tyr Glu Asp Ala Leu Ala Phe Ser Glu Arg
   325 330 335
   His Ile Val Ser Gly Ala Lys Val Val Leu Ala Phe Arg Ser Leu Glu
   340 345 350
   Ser Leu Gly Tyr Ala Arg Ser Leu Ile Ala Ser Arg Gly Leu Asp Ser
   355 360 365
   Ile His Leu Leu His Leu Glu Pro Leu Arg Arg Glu Ser Ala Asp Arg
   370 375 380
   Cys Phe Asp Tyr Ile Arg Asp His Phe Gly Arg Leu Asp Gly Ile Val
   385 390 395 400
   Val Leu Pro Arg Ser Gly Asn Gly Gln His Gly Tyr Ser Leu Ser Thr
   405 410 415
   Ala Gly Asp Asp Asp Val Glu Ala Phe Val Arg Asp Glu Ile Val Ser
   420 425 430
   Pro Val Ala Phe Ala Ala Gln Leu Ala Ile Asn Leu Asp Arg Trp Gly
   435 440 445
   Met Leu Asp Glu Ala Pro Ala Leu Thr Tyr Val Thr Asn Pro Thr Asp
   450 455 460
   Gly His Gly Asp Tyr Leu Asn Glu Val Lys Arg Ala Ala Ile Glu Ala
   465 470 475 480
   Leu Ile Arg Ile Trp Arg His Glu Asp Arg Gln Met Arg Lys Lys Gly
   485 490 495
   Glu Arg Gln Trp Ala Met Leu Pro Asn Gln Leu Val Arg Tyr Asp Asn
   500 505 510
   Asp Glu Glu Asp Asn Leu Thr Phe Thr Ala Asp Trp Ala Ala Thr Leu
   515 520 525
   Thr Asn Arg Val Arg Arg Met Asp Pro Ile Asn Leu Trp Val Pro Glu
   530 535 540
   Ser Ile Lys Arg Ala Thr Gly Lys Gly Ser Met Pro Gln Ser Ile Gln
   545                 550                 555                 560
   Arg Val Leu Pro Gly Leu His Lys Gly Arg Thr Ala Val Ile Thr Gly
                   565                 570                 575
   Gly Ser Leu Gly Ile Gly Leu Gln Leu Gly Arg Phe Leu Ala Ile Ala
               580                 585                 590
   Gly Ala Arg Val Leu Leu Ser Ala Arg Ser Lys Glu Lys Leu Glu Glu
           595                 600                 605
   Ala Arg Asn Gly Ile Val Glu Glu Leu Arg Ala Val Gly Tyr Pro Asn
       610                 615                 620
   Ala His Gln Arg Val His Ile Leu Pro Asp Ile Asp Val Gly Asp Glu
   625                 630                 635                 640
   Glu Ala Leu Val Arg Leu Tyr Asp His Ser Ile Glu Leu Phe Gly His
                   645                 650                 655
   Val Asp Phe Leu Ile Asn Asn Ala Gly Ile Ser Gly Ala Glu Glu Met
               660                 665                 670
   Val Val Asp Met Thr Leu Glu Ala Trp Asn Arg Thr Met Tyr Ala Asn
           675                 680                 685
   Leu Ile Ser Asn Tyr Ser Leu Ile Arg Lys Tyr Ala Pro Lys Met Lys
       690                 695                 700
   Ala Asn Gly Tyr Gly Ile Val Leu Asn Val Ser Ser Tyr Phe Gly Gly
   705                 710                 715                 720
   Glu Lys Tyr Val Ala Val Ala Tyr Pro Asn Arg Ala Asp Tyr Ala Val
                   725                 730                 735
   Ser Lys Ala Gly Gln Arg Val Leu Ala Glu Ile Leu Ser Arg His Leu
               740                 745                 750
   Gly Pro Glu Ile Arg Ile Asn Ala Leu Ala Pro Gly Pro Val Asp Gly
           755                 760                 765
   Ala Arg Leu Arg Gly Leu Gly Gly Ala Pro Gly Leu Phe Glu Arg Arg
       770                 775                 780
   Gly Arg Leu Val Leu Glu Asn Lys Arg Leu Asn Phe Val His Lys Ala
   785                 790                 795                 800
   Ile Leu Ala Ala Ile Asp Glu Lys Val Ser Asp Glu Ser Ile Leu Ala
                   805                 810                 815
   Leu Ala Lys Asn Ala Leu Ser Glu Met Lys Arg Val Ala Gly Gln Ser
               820                 825                 830
   Lys Ala Leu Gly Lys Leu Cys Ala Gln Val Glu Asp Ser Arg Glu Gly
           835                 840                 845
   Gly Asn Ser Ala Ala Tyr Leu Leu Asn Lys Asp Leu Ala Asp Lys Leu
       850                 855                 860
   Leu Lys Arg Leu Val Thr Gly Gly Leu Phe Lys Pro Glu Ser Ala Glu
   865                 870                 875                 880
   Ala Phe Met Glu Ala Phe Val Asp Ala Pro Thr Pro Phe Phe Asp Glu
                   885                 890                 895
   Lys Ala Val Lys Lys Ala Ala Asp Gly Ile Glu Ala Gly Ile Leu Asn
               900                 905                 910
   Arg Leu His Leu His Lys Met Pro Thr Asp Glu Gln Ile Gly Leu Ser
           915                 920                 925
   Thr Val Phe His Leu Ala Asp Asp Ile Ala Ser Gly Glu Thr Phe His
       930                 935                 940
   Pro Ser Gly Gly Leu Lys Phe Asp Arg Ser Val Thr Glu Gly Glu Leu
   945                 950                 955                 960
   Leu Leu Pro Pro Asp Arg Glu Ser Leu Ala Arg Leu Gly Gly Lys Arg
                   965                 970                 975
   Val Val Leu Ile Gly Asp Ala Met Arg Glu Glu Met Ser Ala Ile Gly
               980                 985                 990
   Asn Gly Phe Ile Glu Arg Glu Val  Ala Ser Leu Thr Val  Leu Thr Arg
           995                 1000                1005
   Thr Ala  Glu Ala Gln Glu Glu  Ile Leu His Ser Leu  Pro Gly Thr
       1010                1015                1020
   Gly Lys  Thr Lys Val Asp Val  Arg Val Ile Gly Asp  Asn Leu Glu
       1025                1030                1035
   Asp Ala  Leu Asp Asp Leu Val  Gln Asn Gln Gly Gly  Phe Asp Val
                   1040                1045                1050
   Val Val  Ser Ala Pro Phe Ser  Arg Leu Pro Tyr Asn  Pro Leu Ala
       1055                1060                1065
   Ala Glu  Arg Glu Gly Ser Trp  Glu Arg Val Leu Ser  His Thr Asp
       1070                1075                1080
   Phe Ala  Lys Leu Val Asp Glu  Gln Leu Thr Gln His  Phe Arg Val
       1085                1090                1095
   Ala Arg  Arg Ala Ala Leu Val  Pro Asn Cys Gln Ile  Val Leu Leu
       1100                1105                1110
   Thr Pro  Asp Thr Ser Phe Val  Ser Ser Arg Glu Glu  Phe Ala Leu
       1115                1120                1125
   Ala Leu  Phe Val Lys Asn Ser  Leu His Ala Phe Thr  Val Thr Leu
       1130                1135                1140
   Gly Val  Glu Thr Glu Arg Leu  Pro Thr Val Pro Ala  Val Asn Gln
       1145                1150                1155
   Val Gln  Leu Thr Arg Arg Ala  Arg Thr Glu Glu Pro  Ala Ser Glu
       1160                1165                1170
   Ser Glu  Leu Glu Glu Glu Leu  Glu Arg Leu Val Tyr  Ala Val Leu
       1175                1180                1185
   Gln Cys  Ala Val Pro Ala Pro  Ser Pro Thr Glu Ser  Arg Tyr Leu
       1190                1195                1200
   Ala Arg  Ile Phe Arg Gly Asn  Ala Val Thr Val
       1205                1210
<210> 49
   <211> 1216
   <212> PRT
   <213> Unknown
<220>
   <223> Description of Unknown: Marine gamma proteobacterium HTCC2080 polypeptide
<400> 49
   Met Asn Asp Phe Val Gln Phe Thr Asp Asp Met Thr Ser Gln Ser Lys
   1               5                   10                  15
   Ser Gly Lys Arg Leu Asp Asn Lys Ser Ile Ile Leu Thr Gly Ala Ala
               20                  25                  30
   Gly Ser Ile Gly Arg Phe Ile Thr Arg Gln Leu Leu Cys Glu Gly Ala
           35                  40                  45
   Arg Val Met Met Thr Gly Arg Asp Ile Ser Lys Leu Glu Glu Phe Val
       50                  55                  60
   Asp Ser Leu Cys Asp Asp Gly Phe Asp Arg Glu Asn Met Val Val Thr
   65                  70                  75                  80
   Val Gly Asp Cys Ala Asp Pro Glu Val Cys Arg Arg Ile Val Ala Asp
                   85                  90                  95
   Thr Val Glu Ala Phe Gly Thr Ile Asp Val Leu Val Asn Asn Ala Gly
               100                 105                 110
   Ala Ala Gly Pro Lys Tyr Thr Leu Arg Asp Ile Pro Phe Ser Asp Val
           115                 120                 125
   Glu Met Lys Thr Ala Gly Ser Asp Gln Thr Met Phe Asp Ser Ala Met
       130                 135                 140
   Asn Leu Leu Gly Ala Pro Trp Asn Met Ala Arg Ala Ala Ala Pro His
   145                 150                 155                 160
   Met Ser Val Gly Ala Ser Ile Ile Asn Val Ser Thr Ile Phe Ser Arg
                   165                 170                 175
   Thr His Tyr Phe Gly Arg Ile Pro Tyr Val Val Pro Lys Ser Gly Leu
               180                 185                 190
   Asn Ala Leu Ser Lys Gly Leu Ala Leu Glu Leu Gly Glu Glu Gln Gly
           195                 200                 205
   Ile Arg Val Asn Thr Val Phe Pro Gly Pro Ile Glu Ser Glu Arg Ile
       210                 215                 220
   Asp Thr Val Phe Ala Arg Met Asp Glu Leu Gln Asn Leu Glu Pro Gly
   225                 230                 235                 240
   Ser Thr Gly Arg Glu Phe Arg Asp Leu Met Ile Thr Thr Arg Glu Gly
                   245                 250                 255
   Glu Glu Gly Leu Glu Tyr Arg Tyr Pro Thr Pro Thr Asp Val Ala Ser
               260                 265                 270
   Ser Ile Thr Trp Leu Ala Ser Gly Glu Ser Ala Ala Val Ser Gly His
   275 280 285
   Ala Val Glu Val Thr Asn Gly Met Gln Val Pro Ala Gln Ser Arg Ser
   290 295 300
   Gln Leu Val Ser Trp Pro Asp Lys Arg Leu Glu Asp Leu Ser Asp His
   305 310 315 320
   Ile Val Leu Ile Leu Gly Gly Ser Asp Tyr Glu Glu Ala Val Thr Phe
   325 330 335
   Ala Glu Arg His Thr Glu Ser Gly Ala Arg Val Leu Leu Ala Phe Arg
   340 345 350
   Asn Leu Glu Ser Val Gly His Ala Arg Ser Ile Ile Gln Ala Arg Glu
   355 360 365
   Leu Glu Ser Val Gln Leu Ser His Leu Asp Pro Leu Arg Arg Glu Ser
   370 375 380
   Val Asp Arg Thr Met Gln Phe Ile Asp Asp His Phe Gly Arg Leu Asp
   385 390 395 400
   Gly Val Ile Val Leu Pro Gln Lys Lys Asn Gly Gln Tyr Gly Tyr Ser
   405 410 415
   Ile Cys Ser Ala Thr Asp Asp Asp Val Glu Asn Phe Val Lys Asp Glu
   420 425 430
   Val Val Ala Pro Val Ala Phe Ala Ser Thr Leu Ala Thr Asn Leu Ser
   435 440 445
   Arg Trp Phe Gly Lys Cys Asp Pro Pro Ala Ile Thr Tyr Val Thr Asn
   450 455 460
   Ala Ser Asp Gly His Gly Asn Leu Leu Asn Glu Val Ile Arg Ala Ser
   465 470 475 480
   Asn Glu Ala Leu Ile Arg Gly Trp Arg His Glu Asp Glu Thr Leu Lys
   485 490 495
   Ala Ala Gly Glu Leu Ser Trp Ser Val Gln Pro Asn Gln Leu Val Arg
   500 505 510
   Tyr Asp Thr Glu Asp Arg Asp Ala Leu Pro Phe Ala Ala Asp Trp Ala
   515 520 525
   Ala Thr Leu Thr Asn Arg Val Arg Gln Met Asp Pro Ile Asn Leu Trp
       530                 535                 540
   Ile Pro Lys Asp Ile Lys Arg Ala Thr Gly Lys Gly Ala Met Pro Thr
   545                 550                 555                 560
   Ser Leu Met Arg Val Leu Pro Gly Leu His Lys Gly Lys Thr Ala Val
                   565                 570                 575
   Ile Thr Gly Gly Ser Leu Gly Ile Gly Leu Gln Leu Gly Arg Tyr Leu
               580                 585                 590
   Ala Ile Ala Gly Ala Arg Val Leu Leu Ser Ala Arg Ser Glu Ala Lys
           595                 600                 605
   Leu Ile Glu Ala Lys Ala Glu Ile Val Ala Glu Leu Ser Gly Ile Gly
       610                 615                 620
   Tyr Pro Asn Ala Asp Asn Arg Ile His Val Leu Ala Asn Ile Asp Val
   625                 630                 635                 640
   Gly Asp Pro Ala Ala Leu Glu Thr Leu His Gln His Ala Val Asp Leu
                   645                 650                 655
   Phe Gly Gln Val Asp Phe Leu Ile Asn Asn Ala Gly Ile Ser Gly Ala
               660                 665                 670
   Glu Glu Met Val Val Asp Met Thr Leu Lys Asp Trp Asp Arg Thr Met
           675                 680                 685
   Glu Ala Asn Leu Ile Ser Asn Tyr Ser Leu Ile Arg Lys Phe Gly Pro
       690                 695                 700
   Leu Met Lys Asp Lys Gly Arg Gly Ser Ile Leu Asn Val Ser Ser Tyr
   705                 710                 715                 720
   Phe Gly Gly Glu Lys Tyr Val Ala Val Ala Tyr Pro Asn Arg Ala Asp
                   725                 730                 735
   Tyr Ala Val Ser Lys Ala Gly Gln Arg Val Leu Ala Glu Ile Leu Ser
               740                 745                 750
   Arg His Leu Gly Pro Glu Ile Gln Ile Asn Ala Leu Ala Pro Gly Pro
           755                 760                 765
   Val Asp Gly Ala Arg Leu Arg Gly Leu Gly Asp Ala Pro Gly Leu Phe
       770                 775                 780
   Asp Arg Arg Gly Arg Leu Val Leu Glu Asn Lys Arg Leu Asn Gln Val
   785                 790                 795                 800
   His Ala Ala Ile Ile Ser Ala Val Ala Asp Gly Tyr Pro Ile Glu Glu
                   805                 810                 815
   Ile Arg Lys Leu Ser Ala Asn Ala Val Glu Val Leu Pro Thr His Asn
               820                 825                 830
   Leu Pro Ser Val Leu Ser Arg Leu Tyr Ser Gln Val Lys Asp Ser Gly
           835                 840                 845
   Gly Thr Gly Ser Ser Ser Lys Cys Leu Leu His Met Gly Met Ala Val
       850                 855                 860
   Lys Leu Val Glu Arg Leu Val Asn Ala Gly Ile Phe Thr Thr Glu Asp
   865                 870                 875                 880
   Lys Asp Glu Phe Leu Gly Ser Phe Val Asp Ala Pro Ser Pro Phe Phe
                   885                 890                 895
   Asp Lys Glu Ala Cys Lys Arg Ser Ala Thr Gln Ile Glu Ser Gly Ile
               900                 905                 910
   Leu Asn Arg Leu His Leu His Lys Met Pro Thr Asp Glu Gln Val Gly
           915                 920                 925
   Leu Ser Thr Val Phe His Leu Ala Asp Glu Ile Val Ser Gly Glu Thr
       930                 935                 940
   Phe His Pro Ser Gly Gly Leu Lys Phe Asp Arg Ser Val Thr Glu Gly
   945                 950                 955                 960
   Glu Leu Leu Leu Ser Pro Ser Glu Lys Asp Leu Ala Arg Leu Ser Gly
                   965                 970                 975
   Lys Arg Val Val Ile Leu Gly Asp Cys Met Arg Asn Glu Ile Thr Glu
               980                 985                 990
   Ile Ala Lys Gly Phe Lys Ser Asn  Gly Val Glu Lys Leu  Trp Ile Leu
           995                 1000                1005
   Thr Arg  Ser Glu Glu Thr Lys  Thr Thr Leu Tyr His  Ala Leu Glu
       1010                1015                1020
   Cys Asp  Ser Val Glu Asn Ile  Asp Val Arg Cys Ile  Gly Asp Asp
       1025                1030                1035
   Ile Glu Gly Ala Leu Asp Asn Leu Leu Arg His Asp Gly Gly Phe
   1040 1045 1050
   Asp Val Val Val Ser Ser Pro Phe Glu Arg Leu Pro Leu Asn Ala
   1055 1060 1065
   Leu Ala Gly Asp Arg Gly Gly Asp Trp Asp Arg Val Leu Ser Asp
   1070 1075 1080
   Glu Gln Phe Arg Gln Leu Val His Gln Gln Leu Thr His His Phe
   1085 1090 1095
   Arg Ser Ala Arg Ile Ala Ala Leu Ile Pro Ser Cys Gln Ile Val
   1100 1105 1110
   Leu Leu Thr Pro Glu Thr Ser Leu Ala Ser Thr Arg Glu Glu Phe
   1115 1120 1125
   Ala Leu Ala Leu Phe Val Lys Asn Ser Leu His Ala Phe Thr Val
   1130 1135 1140
   Thr Leu Gly Val Glu Gly Glu Arg Leu Pro Thr Val Pro Ala Val
   1145 1150 1155
   Asn Gln Val Gln Leu Thr Arg Arg Ala His Thr Glu Glu Pro Ser
   1160 1165 1170
   Asn Asp Gln Glu Leu Ser Glu Glu Met Glu Arg Leu Val Ala Ala
   1175 1180 1185
   Val Met Gln Cys Ser Val Pro Ala Pro Ser Pro Lys Glu Ser Arg
   1190 1195 1200
   Tyr Leu Ser Lys Ile Phe Arg Gly Asn Ala Val Thr Val
   1205 1210 1215
<210> 50
   <211> 219
   <212> PRT
   <213> Psuedomonas fluorescens
<220>
   <223> Carbonic anhydrase P. fluorescens Pf-5
<400> 50
   Met Gln Asn Ile Ile Asp Gly Phe Leu Lys Phe Gln Arg Glu Ala Phe
   1 5 10 15
   Pro Gln Arg Ser Glu Leu Phe Lys Gln Leu Ala Ser Thr Gln Asn Pro
               20                  25                  30
   Gly Thr Leu Phe Val Thr Cys Ser Asp Ser Arg Val Val Pro Glu Leu
           35                  40                  45
   Leu Thr Gln Gln Glu Pro Gly Asp Leu Phe Val Ile Arg Asn Ala Gly
       50                  55                  60
   Asn Ile Val Pro Ser Tyr Gly Pro Glu Pro Gly Gly Val Ser Ala Thr
   65                  70                  75                  80
   Val Glu Tyr Ala Val Ala Val Leu Gly Val Ser Asp Ile Val Ile Cys
                   85                  90                  95
   Gly His Ser Asp Cys Gly Ala Met Thr Ala Ile Ser Thr Cys Lys Cys
               100                 105                 110
   Leu Asp His Leu Pro Ala Val Ala Asn Trp Leu Arg His Ala Glu Ser
           115                 120                 125
   Ala Lys Val Ile Asn Ala Ala Arg Gln His Ala Ser Pro Ala Glu His
       130                 135                 140
   Leu Asp Ala Leu Val Arg Asp Asn Val Ile Ala Gln Leu Ala Asn Leu
   145                 150                 155                 160
   Lys Thr His Pro Ser Val Ala Leu Ala Leu Glu Gln Gly Arg Leu Asn
                   165                 170                 175
   Leu His Gly Trp Val Tyr Asp Ile Glu Ser Gly Ala Ile Val Ala Leu
               180                 185                 190
   Asp Gly Asn Thr Gln Arg Phe Val Ser Leu Ala Glu Tyr Pro His Thr
           195                 200                 205
   Cys Ala Leu Ala Ser Gln Ala Ser Ser Ala Ala
       210                 215
<210> 51
   <211> 213
   <212> PRT
   <213> Yersinia ruckeri
<220>
   <223> Carbonic anhydrase Y. ruckeri ATCC 29473
<400> 51
   Met Gln Asp Ile Ile Asp Gly Phe Leu Lys Phe Gln Arg Glu Val Phe
   1 5 10 15
   Pro Gln Arg Ser Glu Leu Phe Lys Arg Leu Ala Asp Thr Gln His Pro
   20 25 30
   Gly Ala Leu Phe Val Thr Cys Ser Asp Ser Arg Val Val Pro Glu Leu
   35 40 45
   Leu Thr Gln Arg Glu Pro Gly Glu Leu Phe Val Ile Arg Asn Ala Gly
   50 55 60
   Asn Ile Val Pro Ser Tyr Gly Pro Glu Pro Gly Gly Val Ser Ala Thr
   65 70 75 80
   Val Glu Tyr Ala Val Ala Val Leu Gly Val Thr Asp Val Val Ile Cys
   85 90 95
   Gly His Ser Asn Cys Gly Ala Met Ser Ala Ile Ala Glu Cys Gln Cys
   100 105 110
   Leu Asp His Leu Pro Ala Val Ala Ala Trp Leu Arg His Ala Asp Ser
   115 120 125
   Ala Lys Leu Val Asn Ala Ala Leu Pro His Ala Ser Pro Lys Asp Arg
   130 135 140
   Leu Asn Ser Leu Val Arg Glu Asn Val Ile Ala Gln Leu Ala Asn Ile
   145 150 155 160
   Lys Thr His Pro Ser Val Ala Leu Ala Cys Ala Gln Gly Arg Leu Arg
   165 170 175
   Leu His Gly Trp Val Tyr Asp Ile Glu Thr Gly Ser Ile Asp Val Leu
   180 185 190
   Asp Glu Leu Thr Arg Thr Phe Ser Pro Leu Ser Ala Tyr Ser Val Val
   195 200 205
   Ser Lys Pro Thr Glu
   210
<210> 52
   <211> 219
   <212> PRT
   <213> Hahella chejuensis
<220>
   <223> Carbonic anhydrase Hahella chejuensis KCTC 2396
<400> 52
   Met Lys Asp Ile Ile Glu Gly Phe Leu Lys Phe Gln Arg Glu Ala Phe
   1               5                   10                  15
   Pro Glu Arg Lys Glu Leu Phe Lys Asp Leu Ala Asn Gln Gln Gln Pro
               20                  25                  30
   Arg Thr Leu Phe Ile Ser Cys Ser Asp Ser Arg Leu Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Pro Tyr Gly Pro Glu Pro Gly Gly Val Ser Ala Ser
   65                  70                  75                  80
   Val Glu Tyr Ala Val Ala Ala Leu Arg Val Ala Asp Ile Val Val Cys
                   85                  90                  95
   Gly His Ser Asn Cys Gly Ala Met Thr Ala Val Ala Thr Cys Gln Cys
               100                 105                 110
   Ile Asp His Met Pro Ala Val Ala His Trp Leu Arg Tyr Ala Asp Ser
           115                 120                 125
   Ala Lys Val Val Asn Gln Ala Arg Lys His Ala Ser Glu Arg Ala Lys
       130                 135                 140
   Ile Glu Asp Met Val Arg Glu Asn Val Ile Ala Gln Leu Ala Asn Leu
   145                 150                 155                 160
   Gln Thr His Pro Ser Val Arg Leu Ala Leu Gln Glu Gly Arg Leu Thr
                   165                 170                 175
   Met His Gly Trp Phe Tyr Asp Ile Glu Ser Gly Gly Ile Asp Ala Tyr
               180                 185                 190
   Asp Gly Ser Arg His Ala Phe Val Pro Leu Ala Glu His Pro Glu Ala
           195                 200                 205
   Arg Ala Ile Pro Gly Lys Leu Ser His Ala Val
       210                 215
<210> 53
   <211> 219
   <212> PRT
   <213> Burkholderia cenocepacia
<220>
   <223> Carbonic anhydrase Burkholderia cenocepacia J23152
<400> 53
   Met Lys Asp Ile Ile Glu Gly Phe Leu Lys Phe Gln Arg Asp Ala Tyr
   1               5                   10                  15
   Pro Ala Arg Ala Ala Leu Phe Arg Asp Leu Ala Arg Ser Gln Asn Pro
               20                  25                  30
   Arg Ala Leu Phe Ile Ser Cys Ser Asp Ser Arg Leu Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Ser Tyr Gly Pro Glu Pro Gly Gly Val Ser Ala Ser
   65                  70                  75                  80
   Val Glu Tyr Ala Val Ala Ala Leu Arg Val Thr Asp Val Val Ile Cys
                   85                  90                  95
   Gly His Ser Asp Cys Gly Ala Met Thr Ala Ile Ala Thr Cys Gln Cys
               100                 105                 110
   Met Asp His Met Pro Ala Val Gly His Trp Leu Arg Tyr Ala Asp Ser
           115                 120                 125
   Ala Arg Val Val Asn Glu Ala Arg Thr His Arg Ser Glu Arg Glu Arg
       130                 135                 140
   Ile Asp Ser Met Val Arg Glu Asn Val Val Ala Gln Leu Ala Asn Leu
   145                 150                 155                 160
   Lys Thr His Pro Ala Val Arg Leu Ala Leu Glu Glu Gly Arg Leu Ala
                   165                 170                 175
   Leu His Gly Trp Val Tyr Asp Ile Glu Ser Gly Cys Ile Asp Ala Tyr
               180                 185                 190
   Asp Gly Ala Thr Gly Arg Phe Val Ser Leu Ala Asp His Pro Gly Val
           195                 200                 205
   Arg Ala Thr Pro Ala Thr Leu Pro Val Ala Ala
       210                 215
<210> 54
   <211> 215
   <212> **PRT**
   <213> Yersinia frederiksenii
<220>
   <223> Carbonic anhydrase Yersinia frederiksenii ATCC 33461
<400> 54
   Met Lys Glu Ile Ile Asp Gly Phe Leu Lys Phe Gln Arg Asp Ala Phe
   1               5                   10                  15
   Pro Glu Arg Ala Glu Leu Phe Arg Ser Leu Ala Thr Gln Gln Ser Pro
               20                  25                  30
   Lys Thr Leu Phe Ile Ser Cys Ser Asp Ser Arg Met Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Ser Tyr Gly Pro Glu Pro Gly Gly Ile Ser Ala Ser
   65                  70                  75                  80
   Val Glu Tyr Ala Val Thr Ala Leu Lys Val Thr Asp Ile Val Ile Cys
                   85                  90                  95
   Gly His Ser Asp Cys Gly Ala Met Thr Ala Ile Ala Lys Cys His Cys
               100                 105                 110
   Leu Asp His Met Pro Ala Val Lys His Trp Leu Gln Tyr Ala Asp Ser
           115                 120                 125
   Ala Lys Val Val Asn Glu Ser Arg Glu Tyr Lys Asn Ile His Asp Lys
       130                 135                 140
   Thr Ile Ser Met Val His Glu Asn Val Val Ala Gln Leu Ala Asn Ile
   145                 150                 155                 160
   Gln Thr His Pro Ser Val Arg Leu Ala Leu Glu Glu Gly Arg Leu Thr
                   165                 170                 175
   Ile His Gly Trp Val Tyr Asp Ile Glu Ser Gly Leu Ile Ser Ala Phe
               180                 185                 190
   Asp Arg Ala Ser Arg Gln Phe Val Ser Leu Ala Ala Asn Pro Asn Val
           195                 200                 205
   Arg Ala Val Pro Ala His Asn
       210                 215
<210> 55
   <211> 219
   <212> PRT
   <213> Pseudomonas entomophila
<220>
   <223> Carbonic anhydrase Pseudomonas entomophila L48
<400> 55
   Met Gln Asp Ile Ile Asp Gly Phe Leu Lys Phe Gln Arg Asp Ala Phe
   1               5                   10                  15
   Pro Glu Arg Val Lys Leu Phe Lys Asp Leu Ala Thr Gln Gln Ser Pro
               20                  25                  30
   Arg Ala Leu Phe Ile Ser Cys Ser Asp Ser Arg Leu Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Ser Tyr Gly Pro Glu Pro Gly Gly Val Ser Ala Ser
   65                  70                  75                  80
   Val Glu Tyr Ala Val Ala Ala Leu Gln Val Ala Asp Ile Val Ile Cys
                   85                  90                  95
   Gly His Ser Asp Cys Gly Ala Met Thr Ala Ile Ala Thr Cys Lys Cys
               100                 105                 110
   Leu Asp His Met Pro Ala Val Ala Gly Trp Leu Arg Tyr Ala Asp Ser
           115                 120                 125
   Ala Arg Val Val Asn Glu Ala Arg Gln His Gln Ser Pro His Ala Lys
       130                 135                 140
   Val Glu Ala Met Val Arg Glu Asn Val Ile Ala Gln Leu Ala Asn Ile
   145                 150                 155                 160
   Gln Thr His Pro Ser Val Arg Leu Ala Leu Glu Glu Gly Arg Val Ala
                   165                 170                 175
   Leu His Gly Trp Ile Tyr Asp Ile Glu Ser Gly Arg Ile Asp Ala Phe
               180                 185                 190
   Asp Gly Arg Thr Gly Gln Phe Val Ser Leu Ala Asp Asn Pro Glu Val
           195                 200                 205
   Arg Ala Val Ser His Ala Ser Arg His Val Ala
       210                 215
<210> 56
   <211> 219
   <212> PRT
   <213> Pseudomonas putida
<220>
   <223> Carbonate dehydratase Pseudomonas putida W619
<400> 56
   Met Lys Ala Ile Ile Asp Gly Phe Leu Lys Phe Gln Lys Asn Ala Phe
   1               5                   10                  15
   Pro Glu Arg Val Lys Leu Phe Lys Asp Leu Ala Asn Gln Gln Ala Pro
               20                  25                  30
   Lys Ala Leu Phe Ile Ser Cys Ser Asp Ser Arg Leu Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Ser Tyr Gly Pro Glu Pro Gly Gly Val Ser Ala Ser
   65                  70                  75                  80
   Val Glu Tyr Ala Val Ala Gly Leu Asn Val Ala Asp Ile Val Ile Cys
                   85                  90                  95
   Gly His Ser Asp Cys Gly Ala Met Thr Ala Ile Ala Thr Cys Lys Cys
               100                 105                 110
   Leu Asp His Met Pro Ala Val Ala Gly Trp Leu Arg Tyr Ala Asp Ser
           115                 120                 125
   Ala Lys Val Val Asn Glu Ala Arg His His Val Asp Lys Pro Ser Lys
       130                 135                 140
   Val Ala Ser Met Val Arg Glu Asn Val Ile Ala Gln Leu Ala Asn Ile
   145                 150                 155                 160
   Gln Thr His Pro Ser Val Arg Leu Ala Leu Glu Glu Gly Arg Val Thr
                   165                 170                 175
   Leu His Gly Trp Ile Tyr Asp Ile Glu Thr Gly Gly Ile Asp Ala Phe
               180                 185                 190
   Asp Gly Ser Thr Gly Thr Phe Val Ser Leu Ala Glu Asn Pro Glu Val
           195                 200                 205
   His Ala Val Ser Gln Gln Ala Arg His Val Ala
       210                 215
<210> 57
   <211> 217
   <212> PRT
   <213> Serratia proteamaculans
<220>
   <223> Carbonate dehydratase Serratia proteamaculans 568
<400> 57
   Met Lys Glu Val Ile Glu Gly Phe Leu Lys Phe Gln Arg Glu Ala Phe
   1               5                   10                  15
   Val Glu Arg Thr Ala Leu Phe Gln Arg Leu Ala Thr Gln Gln Ser Pro
               20                  25                  30
   Arg Thr Leu Phe Ile Ser Cys Ser Asp Ser Arg Leu Val Pro Glu Leu
           35                  40                  45
   Ile Thr Gln Arg Glu Pro Gly Asp Leu Phe Val Ile Arg Asn Ala Gly
       50                  55                  60
   Asn Ile Val Pro Ser Phe Gly Pro Glu Pro Gly Gly Val Ser Ala Ser
   65                  70                  75                  80
   Val Glu Tyr Ala Val Ser Ala Leu Gly Val Glu Asp Ile Val Ile Cys
                   85                  90                  95
   Gly His Ser Asp Cys Gly Ala Met Thr Ala Ile Ala Thr Cys Gln Cys
               100                 105                 110
   Leu Gln His Met Pro Thr Val Ala Asn Trp Leu Arg Tyr Ala Asp Ser
           115                 120                 125
   Ala Lys Val Val Asn Gln Ala Tyr Gln His Ala Ser Glu Asn Glu Lys
       130                 135                 140
   Val Ser Ser Met Val Arg Glu Asn Val Ile Ala Gln Leu Asn Asn Ile
   145                 150                 155                 160
   Lys Thr His Pro Ser Val Ala Leu Ala Leu Glu Gln Gly Arg Leu Lys
                   165                 170                 175
   Leu His Gly Trp Val Tyr Asp Ile Ala Ser Gly Gly Ile Glu Ala Leu
               180                 185                 190
   Asp Gly Glu Thr Arg Arg Phe Ile Pro Leu Ala Thr Asn Pro Glu Val
           195                 200                 205
   Thr Ala Thr Pro Ala Val Ser Arg Phe
                       210                 215
<210> 58
   <211> 211
   <212> PRT
   <213> Erwinia tasmaniensis
<220>
   <223> Carbonate dehydratase Erwinia tasmaniensis Et1/99
<400> 58
   Met Gln His Ile Val Glu Gly Phe Leu Asn Phe Gln Lys Asp Ile Phe
   1               5                   10                  15
   Pro Glu Gln Lys Glu Leu Phe Arg Ser Leu Ala Ser Ser Gln Asn Pro
               20                  25                  30
   Lys Ala Leu Phe Ile Ser Cys Ser Asp Ser Arg Leu Val Pro Glu Leu
           35                  40                  45
   Val Thr Gln Gln Asp Pro Gly Gln Leu Phe Val Ile Arg Asn Ala Gly
       50                  55                  60
   Asn Ile Val Pro Ser Phe Gly Pro Glu Pro Gly Gly Val Ser Ala Thr
   65                  70                  75                  80
   Ile Glu Tyr Ala Val Val Ala Leu Gly Val Ser Asp Ile Val Ile Cys
                   85                  90                  95
   Gly His Ser Asn Cys Gly Ala Met Lys Ala Ile Ala Thr Cys Gln Cys
               100                 105                 110
   Leu Ala Pro Met Pro Ala Val Glu His Trp Leu Arg Tyr Ala Asp Ala
           115                 120                 125
   Ala Lys Ala Val Val Glu Lys Lys Asn Tyr Asp Thr Glu Glu Asp Lys
       130                 135                 140
   Val Asn Ala Met Val Gln Glu Asn Val Ile Ala Gln Leu Asn Asn Ile
   145                 150                 155                 160
   Lys Thr His Pro Ser Val Ala Val Gly Leu Arg Asn Asn Ala Leu Arg
                   165                 170                 175
   Leu His Gly Trp Val Tyr Asp Ile Glu Ser Gly Ala Ile Arg Ala Leu
               180                 185                 190
   Asp Lys Asp Ser Lys Lys Phe Val Leu Leu Ser Asp Asn Pro Gln Val
           195                 200                 205
   His Phe Glu
       210
<210> 59
   <211> 220
   <212> PRT
   <213> Pseudomonas aeruginosa
<220>
   <223> Carbonate dehydratase Pseudomonas aeruginosa PA7
<400> 59
   Met Arg Asp Ile Ile Asp Gly Phe Leu Arg Phe Gln Arg Asp Ala Tyr
   1               5                   10                  15
   Pro Ala Arg Ser Gln Leu Phe Lys Ser Leu Ala Thr Arg Gln Ala Pro
               20                  25                  30
   Lys Ala Leu Phe Ile Ala Cys Ser Asp Ser Arg Val Val Pro Glu Leu
           35                  40                  45
   Leu Thr Gln Arg Glu Pro Gly Glu Leu Phe Val Ile Arg Asn Ala Gly
       50                  55                  60
   Asn Ile Val Pro Gly Tyr Gly Pro Gln Pro Gly Gly Val Ser Ala Ser
   65                  70                  75                  80
   Val Glu Tyr Ala Val Ala Val Leu Gly Val Ala Asp Ile Val Val Cys
                   85                  90                  95
   Gly His Ser Asp Cys Gly Ala Met Gly Ala Ile Ala Ser Cys Ala Cys
               100                 105                 110
   Leu Asp His Leu Pro Ala Val Ala Gly Trp Leu Arg His Ala Glu Ala
           115                 120                 125
   Ala Arg Ala Met Asn Ser Ala His Glu His Ser Ser Asp Ala Ala Arg
       130                 135                 140
   Leu Asp Ala Leu Val Arg His Asn Val Ile Ala Gln Leu Ala Asn Leu
   145                 150                 155                 160
   Arg Thr His Pro Ser Val Ala Arg Ala Leu Glu Gln Gly Arg Leu Asn
                   165                 170                 175
   Leu His Gly Trp Val Tyr Asp Ile Glu Ser Gly Arg Ile Asp Ala Leu
               180                 185                 190
   Asp Gly Ala Ser Arg Arg Phe Val Ser Leu Ala Glu His Pro Gly Val
                 195                 200                 205
   Arg Ala Val Gly Gly Glu Pro Gly Gln Ala Val Ala
       210                 215                 220
<210> 60
   <211> 202
   <212> PRT
   <213> Streptosporangium roseum
<220>
   <223> Carbonate dehydratase Streptosporangium roseum DSM 43021
<400> 60
   Met Gln Asp Leu Glu Glu Gly Val Ala Arg Phe Gln Arg Asp Val Phe
   1               5                   10                  15
   Pro Ala Lys Thr Glu Leu Phe Thr Arg Leu Ala Thr Ala His Gln Pro
               20                  25                  30
   Ala Thr Leu Phe Ile Ser Cys Ser Asp Ala Arg Val Val Pro Glu Leu
           35                  40                  45
   Ile Thr Gln Ser Glu Pro Gly Glu Leu Phe Val Ile Arg Thr Ala Gly
       50                  55                  60
   Asn Leu Val Pro Ala Tyr Ala Pro Gly Ser Ala Asp Gly Val Ala Ala
   65                  70                  75                  80
   Gly Ile Glu Tyr Ala Val Ala Val Leu Gly Val Ser Asp Ile Val Val
                   85                  90                  95
   Cys Gly His Ser Gly Cys Gly Ala Met Thr Ala Val Ala Asp Gly Leu
               100                 105                 110
   Asp Pro Ala Ala Leu Pro Ala Val Ala Gly Trp Leu Arg His Ala Asp
           115                 120                 125
   Ala Ser Arg Ala Arg Val Thr Thr Thr Glu Thr Gly Thr Gly Glu Val
       130                 135                 140
   Ala Ala Leu Val Arg Gln Asn Val Leu Thr Gln Leu Ala Asn Leu Ala
   145                 150                 155                 160
   Thr His Pro Ser Val Ala His Ala Leu Ala Gly Lys Thr Val Thr Leu
                   165                 170                 175
   His Gly Trp Ile Tyr Asp Ile Gly Thr Gly Thr Val Ala Glu Leu Asp
               180                 185                 190
   Ala Thr Gly Arg Pro Ser Ala Leu Ala Val
           195                 200
<210> 61
   <211> 3704
   <212> DNA
   <213> Artificial Sequence
<220>
   <223> Description of Artificial Sequence: Synthetic mcr codon optimized polynucleotide from C. aurantiacus
<400> 61
   gaattccgct agcaggagct aaggaagcta aaatgtccgg tacgggtcgt ttggctggta      60
   aaattgcatt gatcaccggt ggtgctggta acattggttc cgagctgacc cgccgttttc     120
   tggccgaggg tgcgacggtt attatcagcg gccgtaaccg tgcgaagctg accgcgctgg     180
   ccgagcgcat gcaagccgag gccggcgtgc cggccaagcg cattgatttg gaggtgatgg     240
   atggttccga ccctgtggct gtccgtgccg gtatcgaggc aatcgtcgct cgccacggtc     300
   agattgacat tctggttaac aacgcgggct ccgccggtgc ccaacgtcgc ttggcggaaa     360
   ttccgctgac ggaggcagaa ttgggtccgg gtgcggagga gactttgcac gcttcgatcg     420
   cgaatctgtt gggcatgggt tggcacctga tgcgtattgc ggctccgcac atgccagttg     480
   gctccgcagt tatcaacgtt tcgactattt tctcgcgcgc agagtactat ggtcgcattc     540
   cgtacgttac cccgaaggca gcgctgaacg ctttgtccca gctggctgcc cgcgagctgg     600
   gcgctcgtgg catccgcgtt aacactattt tcccaggtcc tattgagtcc gaccgcatcc     660
   gtaccgtgtt tcaacgtatg gatcaactga agggtcgccc ggagggcgac accgcccatc     720
   actttttgaa caccatgcgc ctgtgccgcg caaacgacca aggcgctttg gaacgccgct     780
   ttccgtccgt tggcgatgtt gctgatgcgg ctgtgtttct ggcttctgct gagagcgcgg     840
   cactgtcggg tgagacgatt gaggtcaccc acggtatgga actgccggcg tgtagcgaaa     900
   cctccttgtt ggcgcgtacc gatctgcgta ccatcgacgc gagcggtcgc actaccctga     960
   tttgcgctgg cgatcaaatt gaagaagtta tggccctgac gggcatgctg cgtacgtgcg    1020
   gtagcgaagt gattatcggc ttccgttctg cggctgccct ggcgcaattt gagcaggcag    1080
   tgaatgaatc tcgccgtctg gcaggtgcgg atttcacccc gccgatcgct ttgccgttgg    1140
   acccacgtga cccggccacc attgatgcgg ttttcgattg gggcgcaggc gagaatacgg    1200
   gtggcatcca tgcggcggtc attctgccgg caacctccca cgaaccggct ccgtgcgtga    1260
   ttgaagtcga tgacgaacgc gtcctgaatt tcctggccga tgaaattacc ggcaccatcg    1320
   ttattgcgag ccgtttggcg cgctattggc aatcccaacg cctgaccccg ggtgcccgtg    1380
   cccgcggtcc gcgtgttatc tttctgagca acggtgccga tcaaaatggt aatgtttacg    1440
   gtcgtattca atctgcggcg atcggtcaat tgattcgcgt ttggcgtcac gaggcggagt 1500
   tggactatca acgtgcatcc gccgcaggcg atcacgttct gccgccggtt tgggcgaacc 1560
   agattgtccg tttcgctaac cgctccctgg aaggtctgga gttcgcgtgc gcgtggaccg 1620
   cacagctgct gcacagccaa cgtcatatta acgaaattac gctgaacatt ccagccaata 1680
   ttagcgcgac cacgggcgca cgttccgcca gcgtcggctg ggccgagtcc ttgattggtc 1740
   tgcacctggg caaggtggct ctgattaccg gtggttcggc gggcatcggt ggtcaaatcg 1800
   gtcgtctgct ggccttgtct ggcgcgcgtg tgatgctggc cgctcgcgat cgccataaat 1860
   tggaacagat gcaagccatg attcaaagcg aattggcgga ggttggttat accgatgtgg 1920
   aggaccgtgt gcacatcgct ccgggttgcg atgtgagcag cgaggcgcag ctggcagatc 1980
   tggtggaacg tacgctgtcc gcattcggta ccgtggatta tttgattaat aacgccggta 2040
   ttgcgggcgt ggaggagatg gtgatcgaca tgccggtgga aggctggcgt cacaccctgt 2100
   ttgccaacct gatttcgaat tattcgctga tgcgcaagtt ggcgccgctg atgaagaagc 2160
   aaggtagcgg ttacatcctg aacgtttctt cctattttgg cggtgagaag gacgcggcga 2220
   ttccttatcc gaaccgcgcc gactacgccg tctccaaggc tggccaacgc gcgatggcgg 2280
   aagtgttcgc tcgtttcctg ggtccagaga ttcagatcaa tgctattgcc ccaggtccgg 2340
   ttgaaggcga ccgcctgcgt ggtaccggtg agcgtccggg cctgtttgct cgtcgcgccc 2400
   gtctgatctt ggagaataaa cgcctgaacg aattgcacgc ggctttgatt gctgcggccc 2460
   gcaccgatga gcgctcgatg cacgagttgg ttgaattgtt gctgccgaac gacgtggccg 2520
   cgttggagca gaacccagcg gcccctaccg cgctgcgtga gctggcacgc cgcttccgta 2580
   gcgaaggtga tccggcggca agctcctcgt ccgccttgct gaatcgctcc atcgctgcca 2640
   agctgttggc tcgcttgcat aacggtggct atgtgctgcc ggcggatatt tttgcaaatc 2700
   tgcctaatcc gccggacccg ttctttaccc gtgcgcaaat tgaccgcgaa gctcgcaagg 2760
   tgcgtgatgg tattatgggt atgctgtatc tgcagcgtat gccaaccgag tttgacgtcg 2820
   ctatggcaac cgtgtactat ctggccgatc gtaacgtgag cggcgaaact ttccatccgt 2880
   ctggtggttt gcgctacgag cgtaccccga ccggtggcga gctgttcggc ctgccatcgc 2940
   cggaacgtct ggcggagctg gttggtagca cggtgtacct gatcggtgaa cacctgaccg 3000
   agcacctgaa cctgctggct cgtgcctatt tggagcgcta cggtgcccgt caagtggtga 3060
   tgattgttga gacggaaacc ggtgcggaaa ccatgcgtcg tctgttgcat gatcacgtcg 3120
   aggcaggtcg cctgatgact attgtggcag gtgatcagat tgaggcagcg attgaccaag 3180
   cgatcacgcg ctatggccgt ccgggtccgg tggtgtgcac tccattccgt ccactgccaa 3240
   ccgttccgct ggtcggtcgt aaagactccg attggagcac cgttttgagc gaggcggaat 3300
   ttgcggaact gtgtgagcat cagctgaccc accatttccg tgttgctcgt aagatcgcct 3360
   tgtcggatgg cgcgtcgctg gcgttggtta ccccggaaac gactgcgact agcaccacgg 3420
   agcaatttgc tctggcgaac ttcatcaaga ccaccctgca cgcgttcacc gcgaccatcg 3480
   gtgttgagtc ggagcgcacc gcgcaacgta ttctgattaa ccaggttgat ctgacgcgcc 3540
   gcgcccgtgc ggaagagccg cgtgacccgc acgagcgtca gcaggaattg gaacgcttca 3600
   ttgaagccgt tctgctggtt accgctccgc tgcctcctga ggcagacacg cgctacgcag 3660
   gccgtattca ccgcggtcgt gcgattaccg tctaatagaa gctt 3704
<210> 62
   <211> 4911
   <212> DNA
   <213> Artificial Sequence
<220>
   <223> Description of Artificial Sequence: Synthetic pJ206 plasmid polynucleotide
<400> 62
   ggtggcggta cttgggtcga tatcaaagtg catcacttct tcccgtatgc ccaactttgt 60
   atagagagcc actgcgggat cgtcaccgta atctgcttgc acgtagatca cataagcacc 120
   aagcgcgttg gcctcatgct tgaggagatt gatgagcgcg gtggcaatgc cctgcctccg 180
   gtgctcgccg gagactgcga gatcatagat atagatctca ctacgcggct gctcaaactt 240
   gggcagaacg taagccgcga gagcgccaac aaccgcttct tggtcgaagg cagcaagcgc 300
   gatgaatgtc ttactacgga gcaagttccc gaggtaatcg gagtccggct gatgttggga 360
   gtaggtggct acgtcaccga actcacgacc gaaaagatca agagcagccc gcatggattt 420
   gacttggtca gggccgagcc tacatgtgcg aatgatgccc atacttgagc cacctaactt 480
   tgttttaggg cgactgccct gctgcgtaac atcgttgctg ctccataaca tcaaacatcg 540
   acccacggcg taacgcgctt gctgcttgga tgcccgaggc atagactgta caaaaaaaca 600
   gtcataacaa gccatgaaaa ccgccactgc gccgttacca ccgctgcgtt cggtcaaggt 660
   tctggaccag ttgcgtgagc gcattttttt ttcctcctcg gcgtttacgc cccgccctgc 720
   cactcatcgc agtactgttg taattcatta agcattctgc cgacatggaa gccatcacag 780
   acggcatgat gaacctgaat cgccagcggc atcagcacct tgtcgccttg cgtataatat 840
   ttgcccatag tgaaaacggg ggcgaagaag ttgtccatat tggccacgtt taaatcaaaa 900
   ctggtgaaac tcacccaggg attggcgctg acgaaaaaca tattctcaat aaacccttta 960
   gggaaatagg ccaggttttc accgtaacac gccacatctt gcgaatatat gtgtagaaac 1020
   tgccggaaat cgtcgtggta ttcactccag agcgatgaaa acgtttcagt ttgctcatgg 1080
   aaaacggtgt aacaagggtg aacactatcc catatcacca gctcaccgtc tttcattgcc 1140
   atacggaact ccggatgagc attcatcagg cgggcaagaa tgtgaataaa ggccggataa 1200
   aacttgtgct tatttttctt tacggtcttt aaaaaggccg taatatccag ctgaacggtc 1260
   tggttatagg tacattgagc aactgactga aatgcctcaa aatgttcttt acgatgccat 1320
   tgggatatat caacggtggt atatccagtg atttttttct ccattttttt ttcctccttt 1380
   agaaaaactc atcgagcatc aaatgaaact gcaatttatt catatcagga ttatcaatac 1440
   catatttttg aaaaagccgt ttctgtaatg aaggagaaaa ctcaccgagg cagttccata 1500
   ggatggcaag atcctggtat cggtctgcga ttccgactcg tccaacatca atacaaccta 1560
   ttaatttccc ctcgtcaaaa ataaggttat caagtgagaa atcaccatga gtgacgactg 1620
   aatccggtga gaatggcaaa agtttatgca tttctttcca gacttgttca acaggccagc 1680
   cattacgctc gtcatcaaaa tcactcgcat caaccaaacc gttattcatt cgtgattgcg 1740
   cctgagcgag gcgaaatacg cgatcgctgt taaaaggaca attacaaaca ggaatcgagt 1800
   gcaaccggcg caggaacact gccagcgcat caacaatatt ttcacctgaa tcaggatatt 1860
   cttctaatac ctggaacgct gtttttccgg ggatcgcagt ggtgagtaac catgcatcat 1920
   caggagtacg gataaaatgc ttgatggtcg gaagtggcat aaattccgtc agccagttta 1980
   gtctgaccat ctcatctgta acatcattgg caacgctacc tttgccatgt ttcagaaaca 2040
   actctggcgc atcgggcttc ccatacaagc gatagattgt cgcacctgat tgcccgacat 2100
   tatcgcgagc ccatttatac ccatataaat cagcatccat gttggaattt aatcgcggcc 2160
   tcgacgtttc ccgttgaata tggctcattt ttttttcctc ctttaccaat gcttaatcag 2220
   tgaggcacct atctcagcga tctgtctatt tcgttcatcc atagttgcct gactccccgt 2280
   cgtgtagata actacgatac gggagggctt accatctggc cccagcgctg cgatgatacc 2340
   gcgagaacca cgctcaccgg ctccggattt atcagcaata aaccagccag ccggaagggc 2400
   cgagcgcaga agtggtcctg caactttatc cgcctccatc cagtctatta attgttgccg 2460
   ggaagctaga gtaagtagtt cgccagttaa tagtttgcgc aacgttgttg ccatcgctac 2520
   aggcatcgtg gtgtcacgct cgtcgtttgg tatggcttca ttcagctccg gttcccaacg 2580
   atcaaggcga gttacatgat cccccatgtt gtgcaaaaaa gcggttagct ccttcggtcc 2640
   tccgatcgtt gtcagaagta agttggccgc agtgttatca ctcatggtta tggcagcact 2700
   gcataattct cttactgtca tgccatccgt aagatgcttt tctgtgactg gtgagtactc 2760
   aaccaagtca ttctgagaat agtgtatgcg gcgaccgagt tgctcttgcc cggcgtcaat 2820
   acgggataat accgcgccac atagcagaac tttaaaagtg ctcatcattg gaaaacgttc 2880
   ttcggggcga aaactctcaa ggatcttacc gctgttgaga tccagttcga tgtaacccac 2940
   tcgtgcaccc aactgatctt cagcatcttt tactttcacc agcgtttctg ggtgagcaaa 3000
   aacaggaagg caaaatgccg caaaaaaggg aataagggcg acacggaaat gttgaatact 3060
   catattcttc ctttttcaat attattgaag catttatcag ggttattgtc tcatgagcgg 3120
   atacatattt gaatgtattt agaaaaataa acaaataggg gtcagtgtta caaccaatta 3180
   accaattctg aacattatcg cgagcccatt tatacctgaa tatggctcat aacacccctt 3240
   gtttgcctgg cggcagtagc gcggtggtcc cacctgaccc catgccgaac tcagaagtga 3300
   aacgccgtag cgccgatggt agtgtgggga ctccccatgc gagagtaggg aactgccagg 3360
   catcaaataa aacgaaaggc tcagtcgaaa gactgggcct ttcgcccggg ctaattgagg 3420
   ggtgtcgccc ttgtcgctga gatatcgata tccgtcaaaa gggcgacaca aaatttattc 3480
   taaatgcata ataaatactg ataacatctt atagtttgta ttatattttg tattatcgtt 3540
   gacatgtata attttgatat caaaaactga ttttcccttt attattttcg agatttattt 3600
   tcttaattct ctttaacaaa ctagaaatat tgtatataca aaaaatcata aataatagat 3660
   gaatagttta attataggtg ttcatcaatc gaaaaagcaa cgtatcttat ttaaagtgcg 3720
   ttgctttttt ctcatttata aggttaaata attctcatat atcaagcaaa gtgacaggcg 3780
   cccttaaata ttctgacaaa tgctctttcc ctaaactccc cccataaaaa aacccgccga 3840
   agcgggtttt tacgttattt gcggattaac gattactcgt tatcagaacc gcccaggggg 3900
   cccgagctta agactggccg tcgttttaca acacagaaag agtttgtaga aacgcaaaaa 3960
   ggccatccgt caggggcctt ctgcttagtt tgatgcctgg cagttcccta ctctcgcctt 4020
   ccgcttcctc gctcactgac tcgctgcgct cggtcgttcg gctgcggcga gcggtatcag 4080
   ctcactcaaa ggcggtaata cggttatcca cagaatcagg ggataacgca ggaaagaaca 4140
   tgtgagcaaa aggccagcaa aaggccagga accgtaaaaa ggccgcgttg ctggcgtttt 4200
   tccataggct ccgcccccct gacgagcatc acaaaaatcg acgctcaagt cagaggtggc 4260
   gaaacccgac aggactataa agataccagg cgtttccccc tggaagctcc ctcgtgcgct 4320
   ctcctgttcc gaccctgccg cttaccggat acctgtccgc ctttctccct tcgggaagcg 4380
   tggcgctttc tcatagctca cgctgtaggt atctcagttc ggtgtaggtc gttcgctcca 4440
   agctgggctg tgtgcacgaa ccccccgttc agcccgaccg ctgcgcctta tccggtaact 4500
   atcgtcttga gtccaacccg gtaagacacg acttatcgcc actggcagca gccactggta 4560
   acaggattag cagagcgagg tatgtaggcg gtgctacaga gttcttgaag tggtggccta 4620
   actacggcta cactagaaga acagtatttg gtatctgcgc tctgctgaag ccagttacct 4680
   tcggaaaaag agttggtagc tcttgatccg gcaaacaaac caccgctggt agcggtggtt 4740
   tttttgtttg caagcagcag attacgcgca gaaaaaaagg atctcaagaa gatcctttga 4800
   tcttttctac ggggtctgac gctcagtgga acgacgcgcg cgtaactcac gttaagggat 4860
   tttggtcatg agcttgcgcc gtcccgtcaa gtcagcgtaa tgctctgctt a 4911
<210> 63
   <211> 298
   <212> DNA
   <213> Artificial Sequence
<220>
   <223> Description of Artificial Sequence: Synthetic P(talA) promoter polynucleotide based on E. coli
<400> 63
   ggtcatccat ttggttgttc ctttcacgta acgttcacaa ataaagtgtg tgggcaacag      60
   cccctgccca caacgtggcg cacattatta ccctgccgga gtctacagac tttgagcaag     120
   tccaaactct caccattaat ataatgtttt ggtaataatc ctataacact gatgttacct     180
   gcttaatcca gcaataccat gcctgtctgc tatgcttttt tgatgcgttt agcgaaattt     240
   ctcagaagtg tgaattaacg cactcatcta acactttact tttcaaggag tatttccc       298
<210> 64
   <211> 5794
   <212> DNA
   <213> Artificial Sequence
<220>
   <223> Description of Artificial Sequence: Synthetic plasmid pSMART(HC)Amp-PtalA-mcr polynucleotide
<400> 64
   ggtcatccat ttggttgttc ctttcacgta acgttcacaa ataaagtgtg tgggcaacag 60
   cccctgccca caacgtggcg cacattatta ccctgccgga gtctacagac tttgagcaag 120
   tccaaactct caccattaat ataatgtttt ggtaataatc ctataacact gatgttacct 180
   gcttaatcca gcaataccat gcctgtctgc tatgcttttt tgatgcgttt agcgaaattt 240
   ctcagaagtg tgaattaacg cactcatcta acactttact tttcaaggag tatttcccat 300
   ggccggtacg ggtcgtttgg ctggtaaaat tgcattgatc accggtggtg ctggtaacat 360
   tggttccgag ctgacccgcc gttttctggc cgagggtgcg acggttatta tcagcggccg 420
   taaccgtgcg aagctgaccg cgctggccga gcgcatgcaa gccgaggccg gcgtgccggc 480
   caagcgcatt gatttggagg tgatggatgg ttccgaccct gtggctgtcc gtgccggtat 540
   cgaggcaatc gtcgctcgcc acggtcagat tgacattctg gttaacaacg cgggctccgc 600
   cggtgcccaa cgtcgcttgg cggaaattcc gctgacggag gcagaattgg gtccgggtgc 660
   ggaggagact ttgcacgctt cgatcgcgaa tctgttgggc atgggttggc acctgatgcg 720
   tattgcggct ccgcacatgc cagttggctc cgcagttatc aacgtttcga ctattttctc 780
   gcgcgcagag tactatggtc gcattccgta cgttaccccg aaggcagcgc tgaacgcttt 840
   gtcccagctg gctgcccgcg agctgggcgc tcgtggcatc cgcgttaaca ctattttccc 900
   aggtcctatt gagtccgacc gcatccgtac cgtgtttcaa cgtatggatc aactgaaggg 960
   tcgcccggag ggcgacaccg cccatcactt tttgaacacc atgcgcctgt gccgcgcaaa 1020
   cgaccaaggc gctttggaac gccgctttcc gtccgttggc gatgttgctg atgcggctgt 1080
   gtttctggct tctgctgaga gcgcggcact gtcgggtgag acgattgagg tcacccacgg 1140
   tatggaactg ccggcgtgta gcgaaacctc cttgttggcg cgtaccgatc tgcgtaccat 1200
   cgacgcgagc ggtcgcacta ccctgatttg cgctggcgat caaattgaag aagttatggc 1260
   cctgacgggc atgctgcgta cgtgcggtag cgaagtgatt atcggcttcc gttctgcggc 1320
   tgccctggcg caatttgagc aggcagtgaa tgaatctcgc cgtctggcag gtgcggattt 1380
   caccccgccg atcgctttgc cgttggaccc acgtgacccg gccaccattg atgcggtttt 1440
   cgattggggc gcaggcgaga atacgggtgg catccatgcg gcggtcattc tgccggcaac 1500
   ctcccacgaa ccggctccgt gcgtgattga agtcgatgac gaacgcgtcc tgaatttcct 1560
   ggccgatgaa attaccggca ccatcgttat tgcgagccgt ttggcgcgct attggcaatc 1620
   ccaacgcctg accccgggtg cccgtgcccg cggtccgcgt gttatctttc tgagcaacgg 1680
   tgccgatcaa aatggtaatg tttacggtcg tattcaatct gcggcgatcg gtcaattgat 1740
   tcgcgtttgg cgtcacgagg cggagttgga ctatcaacgt gcatccgccg caggcgatca 1800
   cgttctgccg ccggtttggg cgaaccagat tgtccgtttc gctaaccgct ccctggaagg 1860
   tctggagttc gcgtgcgcgt ggaccgcaca gctgctgcac agccaacgtc atattaacga 1920
   aattacgctg aacattccag ccaatattag cgcgaccacg ggcgcacgtt ccgccagcgt 1980
   cggctgggcc gagtccttga ttggtctgca cctgggcaag gtggctctga ttaccggtgg 2040
   ttcggcgggc atcggtggtc aaatcggtcg tctgctggcc ttgtctggcg cgcgtgtgat 2100
   gctggccgct cgcgatcgcc ataaattgga acagatgcaa gccatgattc aaagcgaatt 2160
   ggcggaggtt ggttataccg atgtggagga ccgtgtgcac atcgctccgg gttgcgatgt 2220
   gagcagcgag gcgcagctgg cagatctggt ggaacgtacg ctgtccgcat tcggtaccgt 2280
   ggattatttg attaataacg ccggtattgc gggcgtggag gagatggtga tcgacatgcc 2340
   ggtggaaggc tggcgtcaca ccctgtttgc caacctgatt tcgaattatt cgctgatgcg 2400
   caagttggcg ccgctgatga agaagcaagg tagcggttac atcctgaacg tttcttccta 2460
   ttttggcggt gagaaggacg cggcgattcc ttatccgaac cgcgccgact acgccgtctc 2520
   caaggctggc caacgcgcga tggcggaagt gttcgctcgt ttcctgggtc cagagattca 2580
   gatcaatgct attgccccag gtccggttga aggcgaccgc ctgcgtggta ccggtgagcg 2640
   tccgggcctg tttgctcgtc gcgcccgtct gatcttggag aataaacgcc tgaacgaatt 2700
   gcacgcggct ttgattgctg cggcccgcac cgatgagcgc tcgatgcacg agttggttga 2760
   attgttgctg ccgaacgacg tggccgcgtt ggagcagaac ccagcggccc ctaccgcgct 2820
   gcgtgagctg gcacgccgct tccgtagcga aggtgatccg gcggcaagct cctcgtccgc 2880
   cttgctgaat cgctccatcg ctgccaagct gttggctcgc ttgcataacg gtggctatgt 2940
   gctgccggcg gatatttttg caaatctgcc taatccgccg gacccgttct ttacccgtgc 3000
   gcaaattgac cgcgaagctc gcaaggtgcg tgatggtatt atgggtatgc tgtatctgca 3060
   gcgtatgcca accgagtttg acgtcgctat ggcaaccgtg tactatctgg ccgatcgtaa 3120
   cgtgagcggc gaaactttcc atccgtctgg tggtttgcgc tacgagcgta ccccgaccgg 3180
   tggcgagctg ttcggcctgc catcgccgga acgtctggcg gagctggttg gtagcacggt 3240
   gtacctgatc ggtgaacacc tgaccgagca cctgaacctg ctggctcgtg cctatttgga 3300
   gcgctacggt gcccgtcaag tggtgatgat tgttgagacg gaaaccggtg cggaaaccat 3360
   gcgtcgtctg ttgcatgatc acgtcgaggc aggtcgcctg atgactattg tggcaggtga 3420
   tcagattgag gcagcgattg accaagcgat cacgcgctat ggccgtccgg gtccggtggt 3480
   gtgcactcca ttccgtccac tgccaaccgt tccgctggtc ggtcgtaaag actccgattg 3540
   gagcaccgtt ttgagcgagg cggaatttgc ggaactgtgt gagcatcagc tgacccacca 3600
   tttccgtgtt gctcgtaaga tcgccttgtc ggatggcgcg tcgctggcgt tggttacccc 3660
   ggaaacgact gcgactagca ccacggagca atttgctctg gcgaacttca tcaagaccac 3720
   cctgcacgcg ttcaccgcga ccatcggtgt tgagtcggag cgcaccgcgc aacgtattct 3780
   gattaaccag gttgatctga cgcgccgcgc ccgtgcggaa gagccgcgtg acccgcacga 3840
   gcgtcagcag gaattggaac gcttcattga agccgttctg ctggttaccg ctccgctgcc 3900
   tcctgaggca gacacgcgct acgcaggccg tattcaccgc ggtcgtgcga ttaccgtcta 3960
   agacgaattc tctagatatc gctcaatact gaccatttaa atcatacctg acctccatag 4020
   cagaaagtca aaagcctccg accggaggct tttgacttga tcggcacgta agaggttcca 4080
   actttcacca taatgaaata agatcactac cgggcgtatt ttttgagtta tcgagatttt 4140
   caggagctaa ggaagctaaa atgagtattc aacatttccg tgtcgccctt attccctttt 4200
   ttgcggcatt ttgccttcct gtttttgctc acccagaaac gctggtgaaa gtaaaagatg 4260
   ctgaagatca gttgggtgca cgagtgggtt acatcgaact ggatctcaac agcggtaaga 4320
   tccttgagag tttacgcccc gaagaacgtt ttccaatgat gagcactttt aaagttctgc 4380
   tatgtggcgc ggtattatcc cgtattgacg ccgggcaaga gcaactcggt cgccgcatac 4440
   actattctca gaatgacttg gttgagtact caccagtcac agaaaagcat ctcacggatg 4500
   gcatgacagt aagagaatta tgcagtgctg ccataaccat gagtgataac actgcggcca 4560
   acttacttct ggcaacgatc ggaggaccga aggagctaac cgcttttttg cacaacatgg 4620
   gggatcatgt aactcgcctt gatcgttggg aaccggagct gaatgaagcc ataccaaacg 4680
   acgagcgtga caccacgatg cctgtagcaa tggcaacaac gttgcgcaaa ctattaactg 4740
   gcgaactact tactctagct tcccggcaac aattaataga ctggatggag gcggataaag 4800
   ttgcaggatc acttctgcgc tcggccctcc cggctggctg gtttattgct gataaatctg 4860
   gagccggtga gcgtgggtct cgcggtatca ttgcagcact ggggccagat ggtaagccct 4920
   cccgcatcgt agttatctac acgacgggga gtcaggcaac tatggatgaa cgaaatagac 4980
   agatcgctga gataggtgcc tcactgatta agcattggta atgagggccc aaatgtaatc 5040
   acctggctca ccttcgggtg ggcctttctg cgttgctggc gtttttccat aggctccgcc 5100
   cccctgacga gcatcacaaa aatcgatgct caagtcagag gtggcgaaac ccgacaggac 5160
   tataaagata ccaggcgttt ccccctggaa gctccctcgt gcgctctcct gttccgaccc 5220
   tgccgcttac cggatacctg tccgcctttc tcccttcggg aagcgtggcg ctttctcata 5280
   gctcacgctg taggtatctc agttcggtgt aggtcgttcg ctccaagctg ggctgtgtgc 5340
   acgaaccccc cgttcagccc gaccgctgcg ccttatccgg taactatcgt cttgagtcca 5400
   acccggtaag acacgactta tcgccactgg cagcagccac tggtaacagg attagcagag 5460
   cgaggtatgt aggcggtgct acagagttct tgaagtggtg gcctaactac ggctacacta 5520
   gaagaacagt atttggtatc tgcgctctgc tgaagccagt tacctcggaa aaagagttgg 5580
   tagctcttga tccggcaaac aaaccaccgc tggtagcggt ggtttttttg tttgcaagca 5640
   gcagattacg cgcagaaaaa aaggatctca agaagatcct ttgattttct accgaagaaa 5700
   ggcccacccg tgaaggtgag ccagtgagtt gattgcagtc cagttacgct ggagtctgag 5760
   gctcgtcctg aatgatatca agcttgaatt cgtt 5794
<210> 65
   <211> 27
   <212> DNA
   <213> Artificial Sequence
<220>
   <223> Description of Artificial Sequence: Synthetic primer 1
<400> 65
   gggaaccatg gcaattggca taccaag 27
<210> 66
   <211> 28
   <212> DNA
   <213> Artificial Sequence
<220>
   <223> Description of Artificial Sequence: Synthetic primer 2
<400> 66
   gggttacaga gctttcagga ttgcatcc 28
<210> 67
   <211> 26
   <212> DNA
   <213> Artificial Sequence
<220>
   <223> Description of Artificial Sequence: Synthetic primer 3
<400> 67
   gggaacggcg gggaaaaaca aacgtt 26
<210> 68
   <211> 30
   <212> DNA
   <213> Artificial Sequence
<220>
   <223> Description of Artificial Sequence: Synthetic primer 4
<400> 68
   ggtccatggt aattctccac gcttataagc 30
<210> 69
   <211> 2404
   <212> DNA
   <213> Artificial Sequence
<220>
   <223> Description of Artificial Sequence: Synthetic pBT-3 vector polynucleotide
<400> 69
   aacgaattca agcttgatat cattcaggac gagcctcaga ctccagcgta actggactga 60
   aaacaaacta aagcgccctt gtggcgcttt agttttgttc cgcggccacc ggctggctcg 120
   cttcgctcgg cccgtggaca accctgctgg acaagctgat ggacaggctg cgcctgccca 180
   cgagcttgac cacagggatt gcccaccggc tacccagcct tcgaccacat acccaccggc 240
   tccaactgcg cggcctgcgg ccttgcccca tcaatttttt taattttctc tggggaaaag 300
   cctccggcct gcggcctgcg cgcttcgctt gccggttgga caccaagtgg aaggcgggtc 360
   aaggctcgcg cagcgaccgc gcagcggctt ggccttgacg cgcctggaac gacccaagcc 420
   tatgcgagtg ggggcagtcg aaggcgaagc ccgcccgcct gccccccgag cctcacggcg 480
   gcgagtgcgg gggttccaag ggggcagcgc caccttgggc aaggccgaag gccgcgcagt 540
   cgatcaacaa gccccggagg ggccactttt tgccggaggg ggagccgcgc cgaaggcgtg 600
   ggggaacccc gcaggggtgc ccttctttgg gcaccaaaga actagatata gggcgaaatg 660
   cgaaagactt aaaaatcaac aacttaaaaa aggggggtac gcaacagctc attgcggcac 720
   cccccgcaat agctcattgc gtaggttaaa gaaaatctgt aattgactgc cacttttacg 780
   caacgcataa ttgttgtcgc gctgccgaaa agttgcagct gattgcgcat ggtgccgcaa 840
   ccgtgcggca ccctaccgca tggagataag catggccacg cagtccagag aaatcggcat 900
   tcaagccaag aacaagcccg gtcactgggt gcaaacggaa cgcaaagcgc atgaggcgtg 960
   ggccgggctt attgcgagga aacccacggc ggcaatgctg ctgcatcacc tcgtggcgca 1020
   gatgggccac cagaacgccg tggtggtcag ccagaagaca ctttccaagc tcatcggacg 1080
   ttctttgcgg acggtccaat acgcagtcaa ggacttggtg gccgagcgct ggatctccgt 1140
   cgtgaagctc aacggccccg gcaccgtgtc ggcctacgtg gtcaatgacc gcgtggcgtg 1200
   gggccagccc cgcgaccagt tgcgcctgtc ggtgttcagt gccgccgtgg tggttgatca 1260
   cgacgaccag gacgaatcgc tgttggggca tggcgacctg cgccgcatcc cgaccctgta 1320
   tccgggcgag cagcaactac cgaccggccc cggcgaggag ccgcccagcc agcccggcat 1380
   tccgggcatg gaaccagacc tgccagcctt gaccgaaacg gaggaatggg aacggcgcgg 1440
   gcagcagcgc ctgccgatgc ccgatgagcc gtgttttctg gacgatggcg agccgttgga 1500
   gccgccgaca cgggtcacgc tgccgcgccg gtagtacgta agaggttcca actttcacca 1560
   taatgaaata agatcactac cgggcgtatt ttttgagtta tcgagatttt caggagctaa 1620
   ggaagctaaa atggagaaaa aaatcactgg atataccacc gttgatatat cccaatggca 1680
   tcgtaaagaa cattttgagg catttcagtc agttgctcaa tgtacctata accagaccgt 1740
   tcagctggat attacggcct ttttaaagac cgtaaagaaa aataagcaca agttttatcc 1800
   ggcctttatt cacattcttg cccgcctgat gaatgctcat ccggaattcc gtatggcaat 1860
   gaaagacggt gagctggtga tatgggatag tgttcaccct tgttacaccg ttttccatga 1920
   gcaaactgaa acgttttcat cgctctggag tgaataccac gacgatttcc ggcagtttct 1980
   acacatatat tcgcaagatg tggcgtgtta cggtgaaaac ctggcctatt tccctaaagg 2040
   gtttattgag aatatgtttt tcgtctcagc caatccctgg gtgagtttca ccagttttga 2100
   tttaaacgtg gccaatatgg acaacttctt cgcccccgtt ttcaccatgg gcaaatatta 2160
   tacgcaaggc gacaaggtgc tgatgccgct ggcgattcag gttcatcatg ccgtttgtga 2220
   tggcttccat gtcggcagaa tgcttaatga attacaacag tactgcgatg agtggcaggg 2280
   cggggcgtaa acgcgtggat ccccctcaag tcaaaagcct ccggtcggag gcttttgact 2340
   ttctgctatg gaggtcaggt atgatttaaa tggtcagtat tgagcgatat ctagagaatt 2400
   cgtc 2404
<210> 70
   <211> 21
   <212> DNA
   <213> Artificial Sequence
<220>
   <223> Description of Artificial Sequence: Synthetic primer 7
<400> 70
   aacgaattca agcttgatat c 21
<210> 71
   <211> 21
   <212> DNA
   <213> Artificial Sequence
<220>
   <223> Description of Artificial Sequence: Synthetic primer 8
<400> 71
   gaattcgttg acgaattctc t 21
<210> 72
   <211> 24
   <212> DNA
   <213> Artificial Sequence
<220>
   <223> Description of Artificial Sequence: Synthetic primer 9
<400> 72
   ggaaacagct atgaccatga ttac 24
<210> 73
   <211> 26
   <212> DNA
   <213> Artificial Sequence
<220>
   <223> Description of Artificial Sequence: Synthetic primer 10
<400> 73
   ttgtaaaacg acggccagtg agcgcg 26
<210> 74
   <211> 7263
   <212> DNA
   <213> Artificial Sequence
<220>
   <223> Description of Artificial Sequence: Synthetic pj251:26385 plasmid polynucleotide
<400> 74
   gcttttagaa aaactcatcg agcatcaaat gaaactgcaa tttattcata tcaggattat 60
   caataccata tttttgaaaa agccgtttct gtaatgaagg agaaaactca ccgaggcagt 120
   tccataggat ggcaagatcc tggtatcggt ctgcgattcc gactcgtcca acatcaatac 180
   aacctattaa tttcccctcg tcaaaaataa ggttatcaag tgagaaatca ccatgagtga 240
   cgactgaatc cggtgagaat ggcaaaagtt tatgcatttc tttccagact tgttcaacag 300
   gccagccatt acgctcgtca tcaaaatcac tcgcatcaac caaaccgtta ttcattcgtg 360
   attgcgcctg agcgaggcga aatacgcgat cgctgttaaa aggacaatta caaacaggaa 420
   tcgagtgcaa ccggcgcagg aacactgcca gcgcatcaac aatattttca cctgaatcag 480
   gatattcttc taatacctgg aacgctgttt ttccggggat cgcagtggtg agtaaccatg 540
   catcatcagg agtacggata aaatgcttga tggtcggaag tggcataaat tccgtcagcc 600
   agtttagtct gaccatctca tctgtaacat cattggcaac gctacctttg ccatgtttca 660
   gaaacaactc tggcgcatcg ggcttcccat acaagcgata gattgtcgca cctgattgcc 720
   cgacattatc gcgagcccat ttatacccat ataaatcagc atccatgttg gaatttaatc 780
   gcggcctcga cgtttcccgt tgaatatggc tcatattctt cctttttcaa tattattgaa 840
   gcatttatca gggttattgt ctcatgagcg gatacatatt tgaatgtatt tagaaaaata 900
   aacaaatagg ggtcagtgtt acaaccaatt aaccaattct gaacattatc gcgagcccat 960
   ttatacctga atatggctca taacacccct tgtttgcctg gcggcagtag cgcggtggtc 1020
   ccacctgacc ccatgccgaa ctcagaagtg aaacgccgta gcgccgatgg tagtgtgggg 1080
   actccccatg cgagagtagg gaactgccag gcatcaaata aaacgaaagg ctcagtcgaa 1140
   agactgggcc tttcgcccgg gctaattagg gggtgtcgcc cttattcgac tctataggga 1200
   agttcctatt ctctagaaag tataggaact tctgaagggg ggacagctgt ctcaaatgtc 1260
   tcggaatcgc tgacgattcc caggtttctc cggcaagcat agcgcatggc gtctccatgc 1320
   gagaatgtcg cgcttgccgg ataaaagggg agccgctatc ggaatggacg caagccacgg 1380
   ccgcagcagg tgcggtcgag ggcttccagc cagttccagg gcagatgtgc cggcagaccc 1440
   tcccgctttg ggggaggcgc aagccgggtc cattcggata gcatctcccc atgcaaagtg 1500
   ccggccaggg caatgcccgg agccggttcg aggcgccggt ttgaataaat gacaaaaagc 1560
   aaagcctttg tgccgatgaa tctctatact gtttcacaga cctgctgccc tgcggggcag 1620
   gaggaaaaac atgtccctga acttcctgga cttcgagcag ccgattgcag aactggaagc 1680
   gaagattgac agcctgaccg cggttagccg tcaagatgag aaactggaca ttaacatcga 1740
   cgaagaggtc caccgtttgc gtgagaagtc tgttgaactg actcgcaaaa tctttgctga 1800
   tttgggcgca tggcagattg cccagttggc tcgccaccca caacgcccat ataccctgga 1860
   ctacgtgcgc ctggcgtttg acgagttcga cgaactggca ggcgaccgcg cctatgcgga 1920
   cgataaagca attgtcggcg gtattgctcg tttggatggc cgtccggtga tgattatcgg 1980
   ccatcaaaaa ggccgcgaga cgaaagaaaa gattcgtcgt aactttggta tgccggcacc 2040
   ggagggctac cgcaaggccc tgcgtctgat gcaaatggcc gaacgcttta agatgccgat 2100
   tatcacgttc attgatacgc cgggtgcgta cccaggcgtt ggtgcggaag agcgtggtca 2160
   gagcgaggcc atcgcacgta acctgcgtga gatgtctcgt ctgggtgtgc cggtcgtttg 2220
   caccgtgatt ggcgagggcg gtagcggtgg tgcgttggcg atcggtgtcg gtgataaggt 2280
   caacatgctg caatacagca cgtacagcgt cattagcccg gaaggttgcg cttccattct 2340
   gtggaagagc gcggataaag caccattggc agcggaagcg atgggtatca tcgcaccgcg 2400
   tctgaaagaa ctgaagttga ttgattctat catcccggaa ccgctgggcg gtgctcaccg 2460
   taatccggag gcgatggcag ccagcctgaa ggcccagctg ctggcggacc tggcggatct 2520
   ggacgtgctg agcacggagg atctgaaaaa ccgtcgctat cagcgcttga tgagctatgg 2580
   ctacgcgtag taaaggagga aaatcatgag ctggattgaa cgcattaagt ccaatatcac 2640
   cccgacccgc aaggcgagca tccctgaagg cgtctggacc aaatgcgata gctgcggtca 2700
   ggttttgtat cgtgcggagc tggagcgtaa cctggaagtg tgcccgaaat gcgaccatca 2760
   catgcgtatg accgctcgta atcgtctgca tagcctgctg gatgagggca gcctggtcga 2820
   gctgggtagc gaactggaac cgaaagatgt tctgaaattc cgtgattcca agaagtataa 2880
   ggatcgtttg gcatctgcac aaaaagaaac cggtgagaag gacgcactgg ttgttatgaa 2940
   aggcaccctg tatggtatgc cggttgttgc tgcggcgttc gagtttgcgt ttatgggtgg 3000
   cagcatgggt tccgtggtgg gcgcacgctt tgtgcgtgcc gtggagcagg cgctggagga 3060
   taactgtcct ctgatttgtt tcagcgcgag cggtggtgcg cgtatgcaag aggccctgat 3120
   gagcctgatg cagatggcaa aaacctcggc agccctggcg aagatgcaag aacgcggcct 3180
   gccgtacatt tccgtcctga ccgaccctac gatgggcggt gtcagcgcca gctttgcgat 3240
   gctgggtgat ttgaacatcg cagagccgaa ggctctgatt ggttttgctg gtccgcgtgt 3300
   tattgaacag acggttcgcg aaaagttgcc gcctggtttc cagcgcagcg agttcctgat 3360
   tgagaaaggt gccatcgaca tgatcgttcg ccgtccagaa atgcgtctga aactggcgag 3420
   cattctggcg aaattgatga atctgccggc tccgaatcct gaagcaccgc gtgagggtgt 3480
   cgtggttccg ccggtcccgg accaagagcc ggaggcataa tagcacgtgg agttaaccac 3540
   gcggcttgcc aacggggtct gaatcgcttt ttttgtatat aatgcgtgtg aaatttcata 3600
   ccacaggcga aggaggaaaa accatggaca ttcgtaagat caagaaactg attgaactgg 3660
   ttgaagaaag cggcatcagc gagctggaga tcagcgaagg tgaagagagc gtccgtattt 3720
   cccgtgcggc accggcagcg agctttccgg ttatgcagca agcatacgcc gctccgatga 3780
   tgcaacagcc ggcacagagc aacgccgctg caccggcgac cgttccaagc atggaggcac 3840
   cggcagcggc cgagatttcg ggtcatatcg tgcgtagccc gatggtgggc accttctatc 3900
   gcacgccgtc gccggacgca aaagccttca tcgaagtcgg ccagaaggtc aatgtcggcg 3960
   acacgctgtg tatcgttgag gcaatgaaaa tgatgaacca gattgaagcg gataagagcg 4020
   gtactgttaa agcgatcctg gtggaatccg gccagcctgt tgagttcgat gaaccgctgg 4080
   ttgtgatcga gtaacgaggc gaacatgttg gacaagatcg tgattgcaaa ccgcggtgaa 4140
   atcgcgctgc gtatcttgcg cgcgtgtaaa gagctgggca ttaagactgt tgccgtgcat 4200
   tccagcgcag accgcgacct gaagcatgtt ctgctggccg acgaaacggt ttgcatcggt 4260
   ccggcaccga gcgtgaaaag ctatctgaac atcccggcca tcatctctgc ggcagagatc 4320
   accggtgcag tggcgattca tccgggctac ggtttcctga gcgagaacgc taactttgct 4380
   gaacaagtgg agcgtagcgg tttcatcttc attggcccta aggcggagac gattcgcctg 4440
   atgggcgaca aagtgagcgc cattgcagcg atgaaaaagg ccggtgtgcc gtgtgttccg 4500
   ggcagcgatg gtccgctggg tgacgatatg gacaagaacc gtgccatcgc taaacgtatt 4560
   ggctacccgg tcattatcaa agcctctggt ggtggcggtg gccgtggtat gcgtgtcgtc 4620
   cgtggtgatg cggaactggc gcaaagcatc agcatgaccc gtgcggaagc caaagcggcg 4680
   ttctctaacg atatggtgta tatggagaag tatctggaga atccgcgcca cgttgaaatc 4740
   caagttctgg cggatggtca gggcaatgcg atctacttgg cagaacgtga ttgctccatg 4800
   caacgccgtc atcagaaggt ggtggaagag gcaccggctc cgggtattac gccggaactg 4860
   cgtcgctaca tcggtgagcg ctgtgcgaaa gcgtgtgtgg acattggtta ccgtggtgcg 4920
   ggtacgtttg agttcctgtt cgaaaatggt gagttttact tcattgaaat gaatacccgc 4980
   atccaggttg agcacccggt gaccgagatg attactggcg ttgatctgat caaagagcaa 5040
   ctgcgcattg cggctggtca gccgctgtcg atcaagcaag aagaggtgca cgttcgtggt 5100
   cacgcggtcg agtgccgtat caatgcggag gacccgaata cctttctgcc gagccctggt 5160
   aagatcacgc gttttcacgc gccaggtggt tttggcgttc gttgggagtc tcacatctac 5220
   gccggttaca ccgtgccgcc gtactatgac agcatgattg gtaaactgat ctgctatggc 5280
   gaaaatcgtg atgtcgcgat cgcccgcatg aaaaacgcgc tgcaagagct gatcattgat 5340
   ggcattaaga ccaatgtgga tttgcagatc cgcattatga acgacgagaa tttccagcac 5400
   ggcggtacga acattcacta cctggaaaag aaactgggcc tgcaagagaa ataatagggt 5460
   accctagcat aaccccttgg ggcctctaaa cgggtcttga ggggtttttt gaatcccccg     5520
   ggatgtctat cttaaggtag ccaaagttta aactttggct accttaagat agacatactg 5580
   ccggcctggt tcaaccagtc ggcagccggc gctggcgccc gcgtattgcg gtgcagccag 5640
   cgcggcgcac aaggcggcgg gcgtttcgtt tcgccgcccg tttcgcgggc cgtcaaggcc 5700
   cgcgaatcgt ttctgcccgc gcggcattcc tcgctttttg cgccaattca ccgggttttc 5760
   cttaagcccc gtcgcttttc ttagtgcctt gttgggcata gaatcagggc agcggcgcag 5820
   ccagcaccat gttcgtgcag cgcggccctc gcgggggcga ggctgcagac agctgtcaaa 5880
   atgaagggaa gttcctatac tttctagaga ataggaactt ctatagggag tcgaataagg 5940
   gcgacacaaa aggtattcta aatgcataat aaatactgat aacatcttat agtttgtatt 6000
   atattttgta ttatcgttga catgtataat tttgatatca aaaactgatt ttccctttat 6060
   tattttcgag atttattttc ttaattctct ttaacaaact agaaatattg tatatacaaa 6120
   aaatcataaa taatagatga atagtttaat tataggtgtt catcaatcga aaaagcaacg 6180
   tatcttattt aaagtgcgtt gcttttttct catttataag gttaaataat tctcatatat 6240
   caagcaaagt gacaggcgcc cttaaatatt ctgacaaatg ctctttccct aaactccccc 6300
   cataaaaaaa cccgccgaag cgggttttta cgttatttgc ggattaacga ttactcgtta 6360
   tcagaaccgc ccaggatgcc tggcagttcc ctactctcgc cgctgcgctc ggtcgttcgg 6420
   ctgcgggacc tcagcgctag cggagtgtat actggcttac tatgttggca ctgatgaggg 6480
   tgtcagtgaa gtgcttcatg tggcaggaga aaaaaggctg caccggtgcg tcagcagaat 6540
   atgtgataca ggatatattc cgcttcctcg ctcactgact cgctacgctc ggtcgttcga 6600
   ctgcggcgag cggaaatggc ttacgaacgg ggcggagatt tcctggaaga tgccaggaag 6660
   atacttaaca gggaagtgag agggccgcgg caaagccgtt tttccatagg ctccgccccc 6720
   ctgacaagca tcacgaaatc tgacgctcaa atcagtggtg gcgaaacccg acaggactat 6780
   aaagatacca ggcgtttccc cctggcggct ccctcgtgcg ctctcctgtt cctgcctttc 6840
   ggtttaccgg tgtcattccg ctgttatggc cgcgtttgtc tcattccacg cctgacactc 6900
   agttccgggt aggcagttcg ctccaagctg gactgtatgc acgaaccccc cgttcagtcc 6960
   gaccgctgcg ccttatccgg taactatcgt cttgagtcca acccggaaag acatgcaaaa 7020
   gcaccactgg cagcagccac tggtaattga tttagaggag ttagtcttga agtcatgcgc 7080
   cggttaaggc taaactgaaa ggacaagttt tggtgactgc gctcctccaa gccagttacc 7140
   tcggttcaaa gagttggtag ctcagagaac cttcgaaaaa ccgccctgca aggcggtttt 7200
   ttcgttttca gagcaagaga ttacgcgcag accaaaacga tctcaagaag atcatcttat 7260
   taa 7263
<210> 75
   <211> 22
   <212> DNA
   <213> Artificial Sequence
<220>
   <223> Description of Artificial Sequence: Synthetic pcr primer 1
<400> 75
   gcggggcagg aggaaaaaca tg 22
<210> 76
   <211> 45
   <212> DNA
   <213> Artificial Sequence
<220>
   <223> Description of Artificial Sequence: Synthetic primer 1
<400> 76
   gcttataagc gaataaagga agatggccgc cccgcagggc agcag 45
<210> 77
   <211> 7298
   <212> DNA
   <213> Artificial Sequence
<220>
   <223> Description of Artificial Sequence: Synthetic pJ251:PtpiA:accAD-PrpiA:accBC polynucleotide
<400> 77
   gcttttagaa aaactcatcg agcatcaaat gaaactgcaa tttattcata tcaggattat      60
   caataccata tttttgaaaa agccgtttct gtaatgaagg agaaaactca ccgaggcagt     120
   tccataggat ggcaagatcc tggtatcggt ctgcgattcc gactcgtcca acatcaatac     180
   aacctattaa tttcccctcg tcaaaaataa ggttatcaag tgagaaatca ccatgagtga     240
   cgactgaatc cggtgagaat ggcaaaagtt tatgcatttc tttccagact tgttcaacag     300
   gccagccatt acgctcgtca tcaaaatcac tcgcatcaac caaaccgtta ttcattcgtg     360
   attgcgcctg agcgaggcga aatacgcgat cgctgttaaa aggacaatta caaacaggaa     420
   tcgagtgcaa ccggcgcagg aacactgcca gcgcatcaac aatattttca cctgaatcag     480
   gatattcttc taatacctgg aacgctgttt ttccggggat cgcagtggtg agtaaccatg     540
   catcatcagg agtacggata aaatgcttga tggtcggaag tggcataaat tccgtcagcc     600
   agtttagtct gaccatctca tctgtaacat cattggcaac gctacctttg ccatgtttca     660
   gaaacaactc tggcgcatcg ggcttcccat acaagcgata gattgtcgca cctgattgcc     720
   cgacattatc gcgagcccat ttatacccat ataaatcagc atccatgttg gaatttaatc     780
   gcggcctcga cgtttcccgt tgaatatggc tcatattctt cctttttcaa tattattgaa     840
   gcatttatca gggttattgt ctcatgagcg gatacatatt tgaatgtatt tagaaaaata     900
   aacaaatagg ggtcagtgtt acaaccaatt aaccaattct gaacattatc gcgagcccat     960
   ttatacctga atatggctca taacacccct tgtttgcctg gcggcagtag cgcggtggtc    1020
   ccacctgacc ccatgccgaa ctcagaagtg aaacgccgta gcgccgatgg tagtgtgggg    1080
   actccccatg cgagagtagg gaactgccag gcatcaaata aaacgaaagg ctcagtcgaa    1140
   agactgggcc tttcgcccgg gctaattagg gggtgtcgcc cttattcgac tctataggga    1200
   agttcctatt ctctagaaag tataggaact tctgaagggg ggacagctgt ctcaaatgtc    1260
   tcggaatcgc tgacgattcc caggtttctc cggcaagcat agcgcatggc gtctccatgc 1320
   gagaatgtcg cgcttgccgg ataaaagggg agccgctatc ggaatggacg caagccacgg 1380
   ccgcagcagg tgcggtcgag ggcttccagc cagttccagg gcagatgtgc cggcagaccc 1440
   tcccgctttg ggggaggcgc aagccgggtc cattcggata gcatctcccc atgcaaagtg 1500
   ccggccaggg caatgcccgg agccggttcg aggcgccggt ttgaataaat gacaaaaagc 1560
   aaagcctttg tgccgatgaa tctctatact gtttcacaga cctgctgccc tgcggggcgg 1620
   ccatcttcct ttattcgctt ataagcgcgg ggcaggagga aaaacatgtc cctgaacttc 1680
   ctggacttcg agcagccgat tgcagaactg gaagcgaaga ttgacagcct gaccgcggtt 1740
   agccgtcaag atgagaaact ggacattaac atcgacgaag aggtccaccg tttgcgtgag 1800
   aagtctgttg aactgactcg caaaatcttt gctgatttgg gcgcatggca gattgcccag 1860
   ttggctcgcc acccacaacg cccatatacc ctggactacg tgcgcctggc gtttgacgag 1920
   ttcgacgaac tggcaggcga ccgcgcctat gcggacgata aagcaattgt cggcggtatt 1980
   gctcgtttgg atggccgtcc ggtgatgatt atcggccatc aaaaaggccg cgagacgaaa 2040
   gaaaagattc gtcgtaactt tggtatgccg gcaccggagg gctaccgcaa ggccctgcgt 2100
   ctgatgcaaa tggccgaacg ctttaagatg ccgattatca cgttcattga tacgccgggt 2160
   gcgtacccag gcgttggtgc ggaagagcgt ggtcagagcg aggccatcgc acgtaacctg 2220
   cgtgagatgt ctcgtctggg tgtgccggtc gtttgcaccg tgattggcga gggcggtagc 2280
   ggtggtgcgt tggcgatcgg tgtcggtgat aaggtcaaca tgctgcaata cagcacgtac 2340
   agcgtcatta gcccggaagg ttgcgcttcc attctgtgga agagcgcgga taaagcacca 2400
   ttggcagcgg aagcgatggg tatcatcgca ccgcgtctga aagaactgaa gttgattgat 2460
   tctatcatcc cggaaccgct gggcggtgct caccgtaatc cggaggcgat ggcagccagc 2520
   ctgaaggccc agctgctggc ggacctggcg gatctggacg tgctgagcac ggaggatctg 2580
   aaaaaccgtc gctatcagcg cttgatgagc tatggctacg cgtagtaaag gaggaaaatc 2640
   atgagctgga ttgaacgcat taagtccaat atcaccccga cccgcaaggc gagcatccct 2700
   gaaggcgtct ggaccaaatg cgatagctgc ggtcaggttt tgtatcgtgc ggagctggag 2760
   cgtaacctgg aagtgtgccc gaaatgcgac catcacatgc gtatgaccgc tcgtaatcgt 2820
   ctgcatagcc tgctggatga gggcagcctg gtcgagctgg gtagcgaact ggaaccgaaa 2880
   gatgttctga aattccgtga ttccaagaag tataaggatc gtttggcatc tgcacaaaaa 2940
   gaaaccggtg agaaggacgc actggttgtt atgaaaggca ccctgtatgg tatgccggtt 3000
   gttgctgcgg cgttcgagtt tgcgtttatg ggtggcagca tgggttccgt ggtgggcgca 3060
   cgctttgtgc gtgccgtgga gcaggcgctg gaggataact gtcctctgat ttgtttcagc 3120
   gcgagcggtg gtgcgcgtat gcaagaggcc ctgatgagcc tgatgcagat ggcaaaaacc 3180
   tcggcagccc tggcgaagat gcaagaacgc ggcctgccgt acatttccgt cctgaccgac 3240
   cctacgatgg gcggtgtcag cgccagcttt gcgatgctgg gtgatttgaa catcgcagag 3300
   ccgaaggctc tgattggttt tgctggtccg cgtgttattg aacagacggt tcgcgaaaag 3360
   ttgccgcctg gtttccagcg cagcgagttc ctgattgaga aaggtgccat cgacatgatc 3420
   gttcgccgtc cagaaatgcg tctgaaactg gcgagcattc tggcgaaatt gatgaatctg 3480
   ccggctccga atcctgaagc accgcgtgag ggtgtcgtgg ttccgccggt cccggaccaa 3540
   gagccggagg cataatagca cgtggagtta accacgcggc ttgccaacgg ggtctgaatc 3600
   gctttttttg tatataatgc gtgtgaaatt tcataccaca ggcgaaggag gaaaaaccat 3660
   ggacattcgt aagatcaaga aactgattga actggttgaa gaaagcggca tcagcgagct 3720
   ggagatcagc gaaggtgaag agagcgtccg tatttcccgt gcggcaccgg cagcgagctt 3780
   tccggttatg cagcaagcat acgccgctcc gatgatgcaa cagccggcac agagcaacgc 3840
   cgctgcaccg gcgaccgttc caagcatgga ggcaccggca gcggccgaga tttcgggtca 3900
   tatcgtgcgt agcccgatgg tgggcacctt ctatcgcacg ccgtcgccgg acgcaaaagc 3960
   cttcatcgaa gtcggccaga aggtcaatgt cggcgacacg ctgtgtatcg ttgaggcaat 4020
   gaaaatgatg aaccagattg aagcggataa gagcggtact gttaaagcga tcctggtgga 4080
   atccggccag cctgttgagt tcgatgaacc gctggttgtg atcgagtaac gaggcgaaca 4140
   tgttggacaa gatcgtgatt gcaaaccgcg gtgaaatcgc gctgcgtatc ttgcgcgcgt 4200
   gtaaagagct gggcattaag actgttgccg tgcattccag cgcagaccgc gacctgaagc 4260
   atgttctgct ggccgacgaa acggtttgca tcggtccggc accgagcgtg aaaagctatc 4320
   tgaacatccc ggccatcatc tctgcggcag agatcaccgg tgcagtggcg attcatccgg 4380
   gctacggttt cctgagcgag aacgctaact ttgctgaaca agtggagcgt agcggtttca 4440
   tcttcattgg ccctaaggcg gagacgattc gcctgatggg cgacaaagtg agcgccattg 4500
   cagcgatgaa aaaggccggt gtgccgtgtg ttccgggcag cgatggtccg ctgggtgacg 4560
   atatggacaa gaaccgtgcc atcgctaaac gtattggcta cccggtcatt atcaaagcct 4620
   ctggtggtgg cggtggccgt ggtatgcgtg tcgtccgtgg tgatgcggaa ctggcgcaaa 4680
   gcatcagcat gacccgtgcg gaagccaaag cggcgttctc taacgatatg gtgtatatgg 4740
   agaagtatct ggagaatccg cgccacgttg aaatccaagt tctggcggat ggtcagggca 4800
   atgcgatcta cttggcagaa cgtgattgct ccatgcaacg ccgtcatcag aaggtggtgg 4860
   aagaggcacc ggctccgggt attacgccgg aactgcgtcg ctacatcggt gagcgctgtg 4920
   cgaaagcgtg tgtggacatt ggttaccgtg gtgcgggtac gtttgagttc ctgttcgaaa 4980
   atggtgagtt ttacttcatt gaaatgaata cccgcatcca ggttgagcac ccggtgaccg 5040
   agatgattac tggcgttgat ctgatcaaag agcaactgcg cattgcggct ggtcagccgc 5100
   tgtcgatcaa gcaagaagag gtgcacgttc gtggtcacgc ggtcgagtgc cgtatcaatg 5160
   cggaggaccc gaataccttt ctgccgagcc ctggtaagat cacgcgtttt cacgcgccag 5220
   gtggttttgg cgttcgttgg gagtctcaca tctacgccgg ttacaccgtg ccgccgtact 5280
   atgacagcat gattggtaaa ctgatctgct atggcgaaaa tcgtgatgtc gcgatcgccc 5340
   gcatgaaaaa cgcgctgcaa gagctgatca ttgatggcat taagaccaat gtggatttgc 5400
   agatccgcat tatgaacgac gagaatttcc agcacggcgg tacgaacatt cactacctgg 5460
   aaaagaaact gggcctgcaa gagaaataat agggtaccct agcataaccc cttggggcct 5520
   ctaaacgggt cttgaggggt tttttgaatc ccccgggatg tctatcttaa ggtagccaaa 5580
   gtttaaactt tggctacctt aagatagaca tactgccggc ctggttcaac cagtcggcag 5640
   ccggcgctgg cgcccgcgta ttgcggtgca gccagcgcgg cgcacaaggc ggcgggcgtt 5700
   tcgtttcgcc gcccgtttcg cgggccgtca aggcccgcga atcgtttctg cccgcgcggc 5760
   attcctcgct ttttgcgcca attcaccggg ttttccttaa gccccgtcgc ttttcttagt 5820
   gccttgttgg gcatagaatc agggcagcgg cgcagccagc accatgttcg tgcagcgcgg 5880
   ccctcgcggg ggcgaggctg cagacagctg tcaaaatgaa gggaagttcc tatactttct 5940
   agagaatagg aacttctata gggagtcgaa taagggcgac acaaaaggta ttctaaatgc 6000
   ataataaata ctgataacat cttatagttt gtattatatt ttgtattatc gttgacatgt 6060
   ataattttga tatcaaaaac tgattttccc tttattattt tcgagattta ttttcttaat 6120
   tctctttaac aaactagaaa tattgtatat acaaaaaatc ataaataata gatgaatagt 6180
   ttaattatag gtgttcatca atcgaaaaag caacgtatct tatttaaagt gcgttgcttt 6240
   tttctcattt ataaggttaa ataattctca tatatcaagc aaagtgacag gcgcccttaa 6300
   atattctgac aaatgctctt tccctaaact ccccccataa aaaaacccgc cgaagcgggt 6360
   ttttacgtta tttgcggatt aacgattact cgttatcaga accgcccagg atgcctggca 6420
   gttccctact ctcgccgctg cgctcggtcg ttcggctgcg ggacctcagc gctagcggag 6480
   tgtatactgg cttactatgt tggcactgat gagggtgtca gtgaagtgct tcatgtggca 6540
   ggagaaaaaa ggctgcaccg gtgcgtcagc agaatatgtg atacaggata tattccgctt 6600
   cctcgctcac tgactcgcta cgctcggtcg ttcgactgcg gcgagcggaa atggcttacg 6660
   aacggggcgg agatttcctg gaagatgcca ggaagatact taacagggaa gtgagagggc 6720
   cgcggcaaag ccgtttttcc ataggctccg cccccctgac aagcatcacg aaatctgacg 6780
   ctcaaatcag tggtggcgaa acccgacagg actataaaga taccaggcgt ttccccctgg 6840
   cggctccctc gtgcgctctc ctgttcctgc ctttcggttt accggtgtca ttccgctgtt 6900
   atggccgcgt ttgtctcatt ccacgcctga cactcagttc cgggtaggca gttcgctcca 6960
   agctggactg tatgcacgaa ccccccgttc agtccgaccg ctgcgcctta tccggtaact 7020
   atcgtcttga gtccaacccg gaaagacatg caaaagcacc actggcagca gccactggta 7080
   attgatttag aggagttagt cttgaagtca tgcgccggtt aaggctaaac tgaaaggaca 7140
   agttttggtg actgcgctcc tccaagccag ttacctcggt tcaaagagtt ggtagctcag 7200
   agaaccttcg aaaaaccgcc ctgcaaggcg gttttttcgt tttcagagca agagattacg 7260
   cgcagaccaa aacgatctca agaagatcat cttattaa 7298
<210> 78
   <211> 17
   <212> DNA
   <213> Artificial Sequence
<220>
   <223> Description of Artificial Sequence: Synthetic pcr primer FWO43
<400> 78
   atgggttttc tttccgg 17
<210> 79
   <211> 19
   <212> DNA
   <213> Artificial Sequence
<220>
   <223> Description of Artificial Sequence: Synthetic pcr primer FWO47
<400> 79
   ttatttcagt tcgagttcg 19
<210> 80
   <211> 21
   <212> DNA
   <213> Artificial Sequence
<220>
   <223> Description of Artificial Sequence: Synthetic pcr primer FWO44
<400> 80
   ctatccatcg cctacggtat c 21
<210> 81
   <211> 18
   <212> DNA
   <213> Artificial Sequence
<220>
   <223> Description of Artificial Sequence: Synthetic pcr primer FWO45
<400> 81
   cgttgcaatg gcaaaagc 18
<210> 82
   <211> 18
   <212> DNA
   <213> Artificial Sequence
<220>
   <223> Description of Artificial Sequence: Synthetic pcr primer FWO46
<400> 82
   cggcggtttc agcattgc 18
<210> 83
   <211> 359
   <212> PRT
   <213> Sulfolobus tokodaii
<400> 83
   Met Ile Leu Met Arg Arg Thr Leu Lys Ala Ala Ile Leu Gly Ala Thr
   1               5                   10                  15
   Gly Leu Val Gly Ile Glu Tyr Val Arg Met Leu Ser Asn His Pro Tyr
               20                  25                  30
   Ile Lys Pro Ala Tyr Leu Ala Gly Lys Gly Ser Val Gly Lys Pro Tyr
           35                  40                  45
   Gly Glu Val Val Arg Trp Gln Thr Val Gly Gln Val Pro Lys Glu Ile
       50                  55                  60
   Ala Asp Met Glu Ile Lys Pro Thr Asp Pro Lys Leu Met Asp Asp Val
   65                  70                  75                  80
   Asp Ile Ile Phe Ser Pro Leu Pro Gln Gly Ala Ala Gly Pro Val Glu
                   85                  90                  95
   Glu Gln Phe Ala Lys Glu Gly Phe Pro Val Ile Ser Asn Ser Pro Asp
               100                 105                 110
   His Arg Phe Asp Pro Asp Val Pro Leu Leu Val Pro Glu Leu Asn Pro
           115                 120                 125
   His Thr Ile Ser Leu Ile Asp Glu Gln Arg Lys Arg Arg Glu Trp Lys
       130                 135                 140
   Gly Phe Ile Val Thr Thr Pro Leu Cys Thr Ala Gln Gly Ala Ala Ile
   145                 150                 155                 160
   Pro Leu Gly Ala Ile Phe Lys Asp Tyr Lys Met Asp Gly Ala Phe Ile
   165 170 175
   Thr Thr Ile Gln Ser Leu Ser Gly Ala Gly Tyr Pro Gly Ile Pro Ser
   180 185 190
   Leu Asp Val Val Asp Asn Ile Leu Pro Leu Gly Asp Gly Tyr Asp Ala
   195 200 205
   Lys Thr Ile Lys Glu Ile Phe Arg Ile Leu Ser Glu Val Lys Arg Asn
   210 215 220
   Val Asp Glu Pro Lys Leu Glu Asp Val Ser Leu Ala Ala Thr Thr His
   225 230 235 240
   Arg Ile Ala Thr Ile His Gly His Tyr Glu Val Leu Tyr Val Ser Phe
   245 250 255
   Lys Glu Glu Thr Ala Ala Glu Lys Val Lys Glu Thr Leu Glu Asn Phe
   260 265 270
   Arg Gly Glu Pro Gln Asp Leu Lys Leu Pro Thr Ala Pro Ser Lys Pro
   275 280 285
   Ile Ile Val Met Asn Glu Asp Thr Arg Pro Gln Val Tyr Phe Asp Arg
   290 295 300
   Trp Ala Gly Asp Ile Pro Gly Met Ser Val Val Val Gly Arg Leu Lys
   305 310 315 320
   Gln Val Asn Lys Arg Met Ile Arg Leu Val Ser Leu Ile His Asn Thr
   325 330 335
   Val Arg Gly Ala Ala Gly Gly Gly Ile Leu Ala Ala Glu Leu Leu Val
   340 345 350
   Glu Lys Gly Tyr Ile Glu Lys
   355
<210> 84
   <211> 780
   <212> DNA
   <213> Escherichia coli
<400> 84
   atgggttttc tttccggtaa gcgcattctg gtaaccggtg ttgccagcaa actatccatc 60
   gcctacggta tcgctcaggc gatgcaccgc gaaggagctg aactggcatt cacctaccag 120
   aacgacaaac tgaaaggccg cgtagaagaa tttgccgctc aattgggttc tgacatcgtt 180
   ctgcagtgcg atgttgcaga agatgccagc atcgacacca tgttcgctga actggggaaa 240
   gtttggccga aatttgacgg tttcgtacac tctattggtt ttgcacctgg cgatcagctg 300
   gatggtgact atgttaacgc cgttacccgt gaaggcttca aaattgccca cgacatcagc 360
   tcctacagct tcgttgcaat ggcaaaagct tgccgctcca tgctgaatcc gggttctgcc 420
   ctgctgaccc tttcctacct tggcgctgag cgcgctatcc cgaactacaa cgttatgggt 480
   ctggcaaaag cgtctctgga agcgaacgtg cgctatatgg cgaacgcgat gggtccggaa 540
   ggtgtgcgtg ttaacgccat ctctgctggt ccgatccgta ctctggcggc ctccggtatc 600
   aaagacttcc gcaaaatgct ggctcattgc gaagccgtta ccccgattcg ccgtaccgtt 660
   actattgaag atgtgggtaa ctctgcggca ttcctgtgct ccgatctctc tgccggtatc 720
   tccggtgaag tggtccacgt tgacggcggt ttcagcattg ctgcaatgaa cgaactcgaa 780
<210> 85
   <211> 262
   <212> PRT
   <213> Escherichia coli
<400> 85
   Met Gly Phe Leu Ser Gly Lys Arg Ile Leu Val Thr Gly Val Ala Ser
   1               5                   10                  15
   Lys Leu Ser Ile Ala Tyr Gly Ile Ala Gln Ala Met His Arg Glu Gly
               20                  25                  30
   Ala Glu Leu Ala Phe Thr Tyr Gln Asn Asp Lys Leu Lys Gly Arg Val
           35                  40                  45
   Glu Glu Phe Ala Ala Gln Leu Gly Ser Asp Ile Val Leu Gln Cys Asp
       50                  55                  60
   Val Ala Glu Asp Ala Ser Ile Asp Thr Met Phe Ala Glu Leu Gly Lys
   65                  70                  75                  80
   Val Trp Pro Lys Phe Asp Gly Phe Val His Ser Ile Gly Phe Ala Pro
                   85                  90                  95
   Gly Asp Gln Leu Asp Gly Asp Tyr Val Asn Ala Val Thr Arg Glu Gly
               100                 105                 110
   Phe Lys Ile Ala His Asp Ile Ser Ser Tyr Ser Phe Val Ala Met Ala
           115                 120                 125
   Lys Ala Cys Arg Ser Met Leu Asn Pro Gly Ser Ala Leu Leu Thr Leu
       130                 135                 140
   Ser Tyr Leu Gly Ala Glu Arg Ala Ile Pro Asn Tyr Asn Val Met Gly
   145 150 155 160
   Leu Ala Lys Ala Ser Leu Glu Ala Asn Val Arg Tyr Met Ala Asn Ala
   165 170 175
   Met Gly Pro Glu Gly Val Arg Val Asn Ala Ile Ser Ala Gly Pro Ile
   180 185 190
   Arg Thr Leu Ala Ala Ser Gly Ile Lys Asp Phe Arg Lys Met Leu Ala
   195 200 205
   His Cys Glu Ala Val Thr Pro Ile Arg Arg Thr Val Thr Ile Glu Asp
   210 215 220
   Val Gly Asn Ser Ala Ala Phe Leu Cys Ser Asp Leu Ser Ala Gly Ile
   225 230 235 240
   Ser Gly Glu Val Val His Val Asp Gly Gly Phe Ser Ile Ala Ala Met
   245 250 255
   Asn Glu Leu Glu Leu Lys
   260
<210> 86
   <211> 21
   <212> DNA
   <213> Artificial Sequence
<220>
   <223> Description of Artificial Sequence: Synthetic primer
<400> 86
   gagcacagta tcgcaaacat g 21
<210> 87
   <211> 18
   <212> DNA
   <213> Artificial Sequence
<220>
   <223> Description of Artificial Sequence: Synthetic primer
<400> 87
   ctgccagcct ggaaaaag 18
<210> 88
   <211> 20
   <212> DNA
   <213> Artificial Sequence
<220>
   <223> Description of Artificial Sequence: Synthetic primer
<400> 88
   tctatgggtt gcggttgaat 20
<210> 89
   <211> 23
   <212> DNA
   <213> Artificial Sequence
<220>
   <223> Description of Artificial Sequence: Synthetic primer
<400> 89
   tgagactgac tgttaataag cgc 23
<210> 90
   <211> 23
   <212> DNA
   <213> Artificial Sequence
<220>
   <223> Description of Artificial Sequence: Synthetic primer
<400> 90
   tagttttatt gggtgtccgg ccc 23
<210> 91
   <211> 23
   <212> DNA
   <213> Artificial Sequence
<220>
   <223> Description of Artificial Sequence: Synthetic primer
<400> 91
   agcagcttat aacgccggac cag 23
<210> 92
   <211> 23
   <212> DNA
   <213> Artificial Sequence
<220>
   <223> Description of Artificial Sequence: Synthetic primer
<400> 92
   tggaactatt ggccaaacta atg 23
<210> 93
   <211> 23
   <212> DNA
   <213> Artificial Sequence
<220>
   <223> Description of Artificial Sequence: Synthetic primer
<400> 93
   cgttgatttt ccggcagaag tca 23
<210> 94
   <211> 16
   <212> DNA
   <213> Artificial Sequence
<220>
   <223> Description of Artificial Sequence: Synthetic primer
<400> 94
   gcgcccatac gcccac 16
<210> 95
   <211> 21
   <212> DNA
   <213> Artificial Sequence
<220>
   <223> Description of Artificial Sequence: Synthetic primer
<400> 95
   gccgtgggcg aaaaacggct t 21
<210> 96
   <211> 70
   <212> DNA
   <213> Artificial Sequence
<220>
   <223> Description of Artificial Sequence: Synthetic primer
<400> 96
   cgtgcataac gtgttgggtt cataaaacat taatttcggt ttcgggtgac gtgtaggctg 60
   gagctgcttc 70
<210> 97
   <211> 75
   <212> DNA
   <213> Artificial Sequence
<220>
   <223> Description of Artificial Sequence: Synthetic primer
<400> 97
   ggctccctga cgttttttta gccacgtatc aattataggt acttccatgt cgagtgtgta 60
   ggctggagct gcttc 75
<210> 98
   <211> 24
   <212> DNA
   <213> Artificial Sequence
<220>
   <223> Description of Artificial Sequence: Synthetic primer
<400> 98
   aatcttgctt acgccacctg gaag 24
<210> 99
   <211> 25
   <212> DNA
   <213> Artificial Sequence
<220>
   <223> Description of Artificial Sequence: Synthetic primer
<400> 99
   caggcagcgc atcaggcagc cctgg 25
<210> 100
   <211> 20
   <212> DNA
   <213> Artificial Sequence
<220>
   <223> Description of Artificial Sequence: Synthetic primer
<400> 100
   ccattacctc aacggcaaaa 20
<210> 101
   <211> 20
   <212> DNA
   <213> Artificial Sequence
<220>
   <223> Description of Artificial Sequence: Synthetic primer
<400> 101
   aagcaataac gttccggttg 20
<210> 102
   <211> 20
   <212> DNA
   <213> Artificial Sequence
<220>
   <223> Description of Artificial Sequence: Synthetic primer
<400> 102
   gcgctgattg gcacacctga 20
<210> 103
   <211> 28
   <212> DNA
   <213> Artificial Sequence
<220>
   <223> Description of Artificial Sequence: Synthetic primer
<400> 103
   attgaaaggt tggtgcaaca gcatgttg 28
<210> 104
   <211> 24
   <212> DNA
   <213> Artificial Sequence
<220>
   <223> Description of Artificial Sequence: Synthetic primer
<400> 104
   tggtcccgtg atgtcgcgtt atac 24
<210> 105
   <211> 20
   <212> DNA
   <213> Artificial Sequence
<220>
   <223> Description of Artificial Sequence: Synthetic primer
<400> 105
   gaactggcga agaaagtgga 20
<210> 106
   <211> 21
   <212> DNA
   <213> Artificial Sequence
<220>
   <223> Description of Artificial Sequence: Synthetic primer
<400> 106
   tgccgaagag gtgaataacg c 21
<210> 107
   <211> 20
   <212> DNA
   <213> Artificial Sequence
<220>
   <223> Description of Artificial Sequence: Synthetic primer
<400> 107
   atcatgctgc cgagcatacg 20
<210> 108
   <211> 22
   <212> DNA
   <213> Artificial Sequence
<220>
   <223> Description of Artificial Sequence: Synthetic primer
<400> 108
   ctccccagtc gatattctcg tg 22
<210> 109
   <211> 70
   <212> DNA
   <213> Artificial Sequence
<220>
   <223> Description of Artificial Sequence: Synthetic primer
<400> 109
   ttaccgctat tgctgaaggt cgtcgttaat cacaaagcgg gtcgcccttg catatgaata 60
   tcctccttag 70
<210> 110
   <211> 75
   <212> DNA
   <213> Artificial Sequence
<220>
   <223> Description of Artificial Sequence: Synthetic primer
<400> 110
   ttccggttca gatatccgca gcgcaaagct gcggatgatg acgagattac tgctgcatat 60
   gaatatcctc cttag 75
<210> 111
   <211> 23
   <212> DNA
   <213> Artificial Sequence
<220>
   <223> Description of Artificial Sequence: Synthetic primer
<400> 111
   gcaaatagtt gtgcagaggg cgg 23
<210> 112
   <211> 21
   <212> DNA
   <213> Artificial Sequence
<220>
   <223> Description of Artificial Sequence: Synthetic primer
<400> 112
   ccagtgggga gctacattct c 21
<210> 113
   <211> 24
   <212> DNA
   <213> Artificial Sequence
<220>
   <223> Description of Artificial Sequence: Synthetic primer
<400> 113
   cgtcattcag atgctggcgc gate 24
<210> 114
   <211> 17
   <212> DNA
   <213> Artificial Sequence
<220>
   <223> Description of Artificial Sequence: Synthetic primer
<400> 114
   atgggttttc tttccgg 17
<210> 115
   <211> 80
   <212> DNA
   <213> Artificial Sequence
<220>
   <223> Description of Artificial Sequence: Synthetic primer
<400> 115
   caggtttgcg gcgtccagcg gttatgtaac tactattcgg cgcgacttac gccgctcccc 60
   gctcgcgata atgtggtagc 80
<210> 116
   <211> 80
   <212> DNA
   <213> Artificial Sequence
<220>
   <223> Description of Artificial Sequence: Synthetic primer
<400> 116
   aataaaacca atgatttggc taatgatcac acagtcccag gcagtaagac cgacgtcatt 60
   ctatcatgcc ataccgcgaa 80
<210> 117
   <211> 21
   <212> DNA
   <213> Artificial Sequence
<220>
   <223> Description of Artificial Sequence: Synthetic primer
<400> 117
   gagataagcc tgaaatgtcg c 21
<210> 118
   <211> 21
   <212> DNA
   <213> Artificial Sequence
<220>
   <223> Description of Artificial Sequence: Synthetic primer
<400> 118
   cagtccagtt acgctggagt c 21
<210> 119
   <211> 26
   <212> DNA
   <213> Artificial Sequence
<220>
   <223> Description of Artificial Sequence: Synthetic primer
<400> 119
   actgaccatt taaatcatac ctgacc 26
<210> 120
   <211> 1207
   <212> DNA
   <213> Artificial Sequence
<220>
   <223> Description of Artificial Sequence: Synthetic polynucleotide
<400> 120
   cagtccagtt acgctggagt ctgctaaggt gtatggagcc gatgttggcc cagaatgtcc 60
   gccagaactt cgaattgctc tattcgcgtc gcgagatgct gccgctggtg agtagtcacc 120
   gcacccgcat atttgaagcg attatggccg gtaagccgga agaagcgcgc gaagcatcgc 180
   atcgccatct ggcctttatc gaagaaattt tgctcgacag aagtcgtgaa gagagccgcc 240
   gtgagcgttc tctgcgtcgt ctggagcaac gaaagaatta gtgtttaaac ataacttcgt 300
   atagcataca ttatacgaag ttattgttga caattaatca tcggcatagt atatcggcat 360
   agtataatac gacaaggtga ggaactaaac catggccaag ttgaccagtg ccgttccggt 420
   gctcaccgcg cgcgacgtcg ccggagcggt cgagttctgg accgaccggc tcgggttctc 480
   ccgggacttc gtggaggacg acttcgccgg tgtggtccgg gacgacgtga ccctgttcat 540
   cagcgcggtc caggaccagg tggtgccgga caacaccctg gcctgggtgt gggtgcgcgg 600
   cctggacgag ctgtacgccg agtggtcgga ggtcgtgtcc acgaacttcc gggacgcctc 660
   cgggccggcc atgaccgaga tcggcgagca gccgtggggg cgggagttcg ccctgcgcga 720
   cccggccggc aactgcgtgc acttcgtggc cgaggagcag gactgaataa cttcgtatag 780
   catacattat acgaagttat agatcttaac tacttaaaaa tttcagttgc ttaatcctac 840
   aattcttgat ataatattct catagtttga aagaactatt cagatagata aggaataacc 900
   catgtcagaa cgtttcccaa atgacgtgga tccgatcgaa actcgcgact ggctccaggc 960
   gatcgaatcg gtcatccgtg aagaaggtgt tgagcgtgct cagtatctga tcgaccaact 1020
   gcttgctgaa gcccgcaaag gcggtgtaaa cgtagccgca ggcacaggta tcagcaacta 1080
   catcaacacc atccccgttg aagaacaacc ggagtatccg ggtaatctgg aactggaacg 1140
   ccgtattcgt tcagctatcc gctggaacgc catcatgacg gactgaccat ttaaatcata 1200
   cctgacc 1207
<210> 121
   <211> 1290
   <212> DNA
   <213> Artificial Sequence
<220>
   <223> Description of Artificial Sequence: Synthetic polynucleotide
<400> 121
   cagtccagtt acgctggagt cgtaactgtc tgcgtcattt ttcatatcac attccttaag 60
   ccaattttaa tcctgctcaa atgaccgtct atgcttaaaa aacagccgta tcagcatcat 120
   tactactgaa gcaactgaat tgtataagtt aatttaatgt taagtagtga ttcgtgccgg 180
   ggcgatgtct cgttttaccc gaccgtcgaa gacaattatc agtctttatc cggcgttcta 240
   aggtgtttat cccactatca cggctgaatc gttaatattt tgcgagttca cgccgaaata 300
   ctgatttttg gcgctagatc agtttaaaca taacttcgta tagcatacat tatacgaagt 360
   tattgttgac aattaatcat cggcatagta tatcggcata gtataatacg acaaggtgag 420
   gaactaaacc atggccaagc ctttgtctca agaagaatcc accctcattg aaagagcaac 480
   ggctacaatc aacagcatcc ccatctctga agactacagc gtcgccagcg cagctctctc 540
   tagcgacggc cgcatcttca ctggtgtcaa tgtatatcat tttactgggg gaccttgtgc 600
   agaactcgtg gtgctgggca ctgctgctgc tgcggcagct ggcaacctga cttgtatcgt 660
   cgcgatcgga aatgagaaca ggggcatctt gagcccctgc ggacggtgcc gacaggtgct 720
   tctcgatctg catcctggga tcaaagccat agtgaaggac agtgatggac agccgacggc 780
   agttgggatt cgtgaattgc tgccctctgg ttatgtgtgg gagggctaaa taacttcgta 840
   tagcatacat tatacgaagt tatagatctt aactacttaa aaatttcagt tgcttaatcc 900
   tacaattctt gatataatat tctcatagtt tgaaagaact attcaaccga tggaagggaa 960
   tatcatgcga attggcatac caagagaacg gttaaccaat gaaacccgtg ttgcagcaac 1020
   gccaaaaaca gtggaacagc tgctgaaact gggttttacc gtcgcggtag agagcggcgc 1080
   gggtcaactg gcaagttttg acgataaagc gtttgtgcaa gcgggcgctg aaattgtaga 1140
   agggaatagc gtctggcagt cagagatcat tctgaaggtc aatgcgccgt tagatgatga 1200
   aattgcgtta ctgaatcctg ggacaacgct ggtgagtttt atctggcctg cgcagaatcc 1260
   ggaaactgac catttaaatc atacctgacc 1290
<210> 122
   <211> 1210
   <212> DNA
   <213> Artificial Sequence
<220>
   <223> Description of Artificial Sequence: Synthetic polynucleotide
<400> 122
   cagtccagtt acgctggagt cggcactata ccagagaatg aacatactgg attgctccag      60
   tattgtgaat gaacggtaac gcaataaaac atatttactg caattctttg gccggttctt     120
   ttacgtacag cggaaacctg ccgcttaaac ggagagtatc gtcgataaaa atccaataaa     180
   acgtcagggc aaaagtaaga aacagacaaa gcaaaggccg ctcaggatat agccagataa     240
   atgacgggga tcaattggct tacccgcgat aaaatgttac cattctgttg cttttatgta     300
   tgtttaaaca taacttcgta tagcatacat tatacgaagt tattgttgac aattaatcat     360
   cggcatagta tatcggcata gtataatacg acaaggtgag gaactaaacc atggccaagc     420
   ctttgtctca agaagaatcc accctcattg aaagagcaac ggctacaatc aacagcatcc     480
   ccatctctga agactacagc gtcgccagcg cagctctctc tagcgacggc cgcatcttca     540
   ctggtgtcaa tgtatatcat tttactgggg gaccttgtgc agaactcgtg gtgctgggca     600
   ctgctgctgc tgcggcagct ggcaacctga cttgtatcgt cgcgatcgga aatgagaaca     660
   ggggcatctt gagcccctgc ggacggtgcc gacaggtgct tctcgatctg catcctggga     720
   tcaaagccat agtgaaggac agtgatggac agccgacggc agttgggatt cgtgaattgc     780
   tgccctctgg ttatgtgtgg gagggctaaa taacttcgta tagcatacat tatacgaagt     840
   tatagatctt aactacttaa aaatttcagt tgcttaatcc tacaattctt gatataatat     900
   tctcatagtt tgaaagaact attcaagaac aggtaagccc taccatgcca cattcctacg     960
   attacgatgc catagtaata ggttccggcc ccggcggcga aggcgctgca atgggcctgg    1020
   ttaagcaagg tgcgcgcgtc gcagttatcg agcgttatca aaatgttggc ggcggttgca    1080
   cccactgggg caccatcccg tcgaaagctc tccgtcacgc cgtcagccgc attatagaat    1140
   tcaatcaaaa cccactttac agcgaccatt cccgactgct ccgcactgac catttaaatc    1200
   atacctgacc                                                            1210
<210> 123
   <211> 1216
   <212> DNA
   <213> Artificial Sequence
<220>
   <223> Description of Artificial Sequence: Synthetic polynucleotide
<400> 123
   cagtccagtt acgctggagt cctctctcga catatcaatt attttcttgc cgtggctgaa 60
   catggcagct tcacccgtgc cgccagtgcg ttgcacgtct cccaacctgc gctttcccag 120
   cagattcgcc agttagagga gagtttaggc gtgccgctgt ttgaccgtag cgggcgaacg 180
   attcgtctca ctgatgcagg agaagtctgg cgacagtacg ccagccgggc gttacaggaa 240
   ctgggggcgg gtaaacgggt ttaaactacc gttcgtataa tgtatgctat acgaagttat 300
   gatatctact acgacactgc atgcatcatc agatcttgtt gacaattaat catcggcata 360
   gtatatcggc atagtataat acgacaaggt gaggaactaa accatggcca agcctttgtc 420
   tcaagaagaa tccaccctca ttgaaagagc aacggctaca atcaacagca tccccatctc 480
   tgaagactac agcgtcgcca gcgcagctct ctctagcgac ggccgcatct tcactggtgt 540
   caatgtatat cattttactg ggggaccttg tgcagaactc gtggtgctgg gcactgctgc 600
   tgctgcggca gctggcaacc tgacttgtat cgtcgcgatc ggaaatgaga acaggggcat 660
   cttgagcccc tgcggacggt gccgacaggt gcttctcgat ctgcatcctg ggatcaaagc 720
   catagtgaag gacagtgatg gacagccgac ggcagttggg attcgtgaat tgcataactt 780
   cgtataatgt atgctatacg aacggtaaga tcttaactac ttaaaaattt cagttgctta 840
   atcctacaat tcttgatata atattctcat agtttgaaag aactattcaa aattaaagag 900
   gtatatatta atgaaagaga ttattgatgg attccttaaa ttccagcgcg aggcatttcc 960
   gaagcgggaa gccttgttta aacagctggc gacacagcaa agcccgcgca cactttttat 1020
   ctcctgctcc gacagccgtc tggtccctga gctggtgacg caacgtgagc ctggcgatct 1080
   gttcgttatt cgcaacgcgg gcaatatcgt cccttcctac gggccggaac ccggtggcgt 1140
   ttctgcttcg gtggagtatg ccgtcgctgc gcttcgggta tctgacattg actgaccatt 1200
   taaatcatac ctgacc 1216
<210> 124
   <211> 2172
   <212> DNA
   <213> Artificial Sequence
<220>
   <223> Description of Artificial Sequence: Synthetic polynucleotide
<400> 124
   atggcaggcg atcacgttct gccgccggtt tgggcgaacc agattgtccg tttcgctaac      60
   cgctccctgg aaggtctgga gttcgcgtgc gcgtggaccg cacagctgct gcacagccaa     120
   cgtcatatta acgaaattac gctgaacatt ccagccaata ttagcgcgac cacgggcgca     180
   cgttccgcca gcgtcggctg ggccgagtcc ttgattggtc tgcacctggg caaggtggct     240
   ctgattaccg gtggttcggc gggcatcggt ggtcaaatcg gtcgtctgct ggccttgtct     300
   ggcgcgcgtg tgatgctggc cgctcgcgat cgccataaat tggaacagat gcaagccatg     360
   attcaaagcg aattggcgga ggttggttat accgatgtgg aggaccgtgt gcacatcgct     420
   ccgggttgcg atgtgagcag cgaggcgcag ctggcagatc tggtggaacg tacgctgtcc     480
   gcattcggta ccgtggatta tttgattaat aacgccggta ttgcgggcgt ggaggagatg     540
   gtgatcgaca tgccggtgga aggctggcgt cacaccctgt ttgccaacct gatttcgaat     600
   tattcgctga tgcgcaagtt ggcgccgctg atgaagaagc aaggtagcgg ttacatcctg     660
   aacgtttctt cctattttgg cggtgagaag gacgcggcga ttccttatcc gaaccgcgcc     720
   gactacgccg tctccaaggc tggccaacgc gcgatggcgg aagtgttcgc tcgtttcctg     780
   ggtccagaga ttcagatcaa tgctattgcc ccaggtccgg ttgaaggcga ccgcctgcgt     840
   ggtaccggtg agcgtccggg cctgtttgct cgtcgcgccc gtctgatctt ggagaataaa     900
   cgcctgaacg aattgcacgc ggctttgatt gctgcggccc gcaccgatga gcgctcgatg     960
   cacgagttgg ttgaattgtt gctgccgaac gacgtggccg cgttggagca gaacccagcg    1020
   gcccctaccg cgctgcgtga gctggcacgc cgcttccgta gcgaaggtga tccggcggca    1080
   agctcctcgt ccgccttgct gaatcgctcc atcgctgcca agctgttggc tcgcttgcat    1140
   aacggtggct atgtgctgcc ggcggatatt tttgcaaatc tgcctaatcc gccggacccg    1200
   ttctttaccc gtgcgcaaat tgaccgcgaa gctcgcaagg tgcgtgatgg tattatgggt    1260
   atgctgtatc tgcagcgtat gccaaccgag tttgacgtcg ctatggcaac cgtgtactat    1320
   ctggccgatc gtaacgtgag cggcgaaact ttccatccgt ctggtggttt gcgctacgag    1380
   cgtaccccga ccggtggcga gctgttcggc ctgccatcgc cggaacgtct ggcggagctg     1440
   gttggtagca cggtgtacct gatcggtgaa cacctgaccg agcacctgaa cctgctggct     1500
   cgtgcctatt tggagcgcta cggtgcccgt caagtggtga tgattgttga gacggaaacc     1560
   ggtgcggaaa ccatgcgtcg tctgttgcat gatcacgtcg aggcaggtcg cctgatgact     1620
   attgtggcag gtgatcagat tgaggcagcg attgaccaag cgatcacgcg ctatggccgt     1680
   ccgggtccgg tggtgtgcac tccattccgt ccactgccaa ccgttccgct ggtcggtcgt     1740
   aaagactccg attggagcac cgttttgagc gaggcggaat ttgcggaact gtgtgagcat     1800
   cagctgaccc accatttccg tgttgctcgt aagatcgcct tgtcggatgg cgcgtcgctg     1860
   gcgttggtta ccccggaaac gactgcgact agcaccacgg agcaatttgc tctggcgaac     1920
   ttcatcaaga ccaccctgca cgcgttcacc gcgaccatcg gtgttgagtc ggagcgcacc     1980
   gcgcaacgta ttctgattaa ccaggttgat ctgacgcgcc gcgcccgtgc ggaagagccg     2040
   cgtgacccgc acgagcgtca gcaggaattg gaacgcttca ttgaagccgt tctgctggtt     2100
   accgctccgc tgcctcctga ggcagacacg cgctacgcag gccgtattca ccgcggtcgt     2160
   gcgattaccg tc                                                         2172
<210> 125
   <211> 2568
   <212> DNA
   <213> Artificial Sequence
<220>
   <223> Description of Artificial Sequence: Synthetic polynucleotide
<400> 125
   atggctttgc cgttggaccc acgtgacccg gccaccattg atgcggtttt cgattggggc 60
   gcaggcgaga atacgggtgg catccatgcg gcggtcattc tgccggcaac ctcccacgaa 120
   ccggctccgt gcgtgattga agtcgatgac gaacgcgtcc tgaatttcct ggccgatgaa 180
   attaccggca ccatcgttat tgcgagccgt ttggcgcgct attggcaatc ccaacgcctg 240
   accccgggtg cccgtgcccg cggtccgcgt gttatctttc tgagcaacgg tgccgatcaa 300
   aatggtaatg tttacggtcg tattcaatct gcggcgatcg gtcaattgat tcgcgtttgg 360
   cgtcacgagg cggagttgga ctatcaacgt gcatccgccg caggcgatca cgttctgccg 420
   ccggtttggg cgaaccagat tgtccgtttc gctaaccgct ccctggaagg tctggagttc 480
   gcgtgcgcgt ggaccgcaca gctgctgcac agccaacgtc atattaacga aattacgctg 540
   aacattccag ccaatattag cgcgaccacg ggcgcacgtt ccgccagcgt cggctgggcc 600
   gagtccttga ttggtctgca cctgggcaag gtggctctga ttaccggtgg ttcggcgggc 660
   atcggtggtc aaatcggtcg tctgctggcc ttgtctggcg cgcgtgtgat gctggccgct 720
   cgcgatcgcc ataaattgga acagatgcaa gccatgattc aaagcgaatt ggcggaggtt     780
   ggttataccg atgtggagga ccgtgtgcac atcgctccgg gttgcgatgt gagcagcgag     840
   gcgcagctgg cagatctggt ggaacgtacg ctgtccgcat tcggtaccgt ggattatttg     900
   attaataacg ccggtattgc gggcgtggag gagatggtga tcgacatgcc ggtggaaggc     960
   tggcgtcaca ccctgtttgc caacctgatt tcgaattatt cgctgatgcg caagttggcg    1020
   ccgctgatga agaagcaagg tagcggttac atcctgaacg tttcttccta ttttggcggt    1080
   gagaaggacg cggcgattcc ttatccgaac cgcgccgact acgccgtctc caaggctggc    1140
   caacgcgcga tggcggaagt gttcgctcgt ttcctgggtc cagagattca gatcaatgct    1200
   attgccccag gtccggttga aggcgaccgc ctgcgtggta ccggtgagcg tccgggcctg    1260
   tttgctcgtc gcgcccgtct gatcttggag aataaacgcc tgaacgaatt gcacgcggct    1320
   ttgattgctg cggcccgcac cgatgagcgc tcgatgcacg agttggttga attgttgctg    1380
   ccgaacgacg tggccgcgtt ggagcagaac ccagcggccc ctaccgcgct gcgtgagctg    1440
   gcacgccgct tccgtagcga aggtgatccg gcggcaagct cctcgtccgc cttgctgaat    1500
   cgctccatcg ctgccaagct gttggctcgc ttgcataacg gtggctatgt gctgccggcg    1560
   gatatttttg caaatctgcc taatccgccg gacccgttct ttacccgtgc gcaaattgac    1620
   cgcgaagctc gcaaggtgcg tgatggtatt atgggtatgc tgtatctgca gcgtatgcca    1680
   accgagtttg acgtcgctat ggcaaccgtg tactatctgg ccgatcgtaa cgtgagcggc    1740
   gaaactttcc atccgtctgg tggtttgcgc tacgagcgta ccccgaccgg tggcgagctg    1800
   ttcggcctgc catcgccgga acgtctggcg gagctggttg gtagcacggt gtacctgatc    1860
   ggtgaacacc tgaccgagca cctgaacctg ctggctcgtg cctatttgga gcgctacggt    1920
   gcccgtcaag tggtgatgat tgttgagacg gaaaccggtg cggaaaccat gcgtcgtctg    1980
   ttgcatgatc acgtcgaggc aggtcgcctg atgactattg tggcaggtga tcagattgag    2040
   gcagcgattg accaagcgat cacgcgctat ggccgtccgg gtccggtggt gtgcactcca    2100
   ttccgtccac tgccaaccgt tccgctggtc ggtcgtaaag actccgattg gagcaccgtt    2160
   ttgagcgagg cggaatttgc ggaactgtgt gagcatcagc tgacccacca tttccgtgtt    2220
   gctcgtaaga tcgccttgtc ggatggcgcg tcgctggcgt tggttacccc ggaaacgact    2280
   gcgactagca ccacggagca atttgctctg gcgaacttca tcaagaccac cctgcacgcg    2340
   ttcaccgcga ccatcggtgt tgagtcggag cgcaccgcgc aacgtattct gattaaccag    2400
   gttgatctga cgcgccgcgc ccgtgcggaa gagccgcgtg acccgcacga gcgtcagcag    2460
   gaattggaac gcttcattga agccgttctg ctggttaccg ctccgctgcc tcctgaggca    2520
   gacacgcgct acgcaggccg tattcaccgc ggtcgtgcga ttaccgtc                2568
<210> 126
   <211> 3135
   <212> DNA
   <213> Artificial Sequence
<220>
   <223> Description of Artificial Sequence: Synthetic polynucleotide
<400> 126
   atggcgctga acgctttgtc ccagctggct gcccgcgagc tgggcgctcg tggcatccgc 60
   gttaacacta ttttcccagg tcctattgag tccgaccgca tccgtaccgt gtttcaacgt 120
   atggatcaac tgaagggtcg cccggagggc gacaccgccc atcacttttt gaacaccatg 180
   cgcctgtgcc gcgcaaacga ccaaggcgct ttggaacgcc gctttccgtc cgttggcgat 240
   gttgctgatg cggctgtgtt tctggcttct gctgagagcg cggcactgtc gggtgagacg 300
   attgaggtca cccacggtat ggaactgccg gcgtgtagcg aaacctcctt gttggcgcgt 360
   accgatctgc gtaccatcga cgcgagcggt cgcactaccc tgatttgcgc tggcgatcaa 420
   attgaagaag ttatggccct gacgggcatg ctgcgtacgt gcggtagcga agtgattatc 480
   ggcttccgtt ctgcggctgc cctggcgcaa tttgagcagg cagtgaatga atctcgccgt 540
   ctggcaggtg cggatttcac cccgccgatc gctttgccgt tggacccacg tgacccggcc 600
   accattgatg cggttttcga ttggggcgca ggcgagaata cgggtggcat ccatgcggcg 660
   gtcattctgc cggcaacctc ccacgaaccg gctccgtgcg tgattgaagt cgatgacgaa 720
   cgcgtcctga atttcctggc cgatgaaatt accggcacca tcgttattgc gagccgtttg 780
   gcgcgctatt ggcaatccca acgcctgacc ccgggtgccc gtgcccgcgg tccgcgtgtt 840
   atctttctga gcaacggtgc cgatcaaaat ggtaatgttt acggtcgtat tcaatctgcg 900
   gcgatcggtc aattgattcg cgtttggcgt cacgaggcgg agttggacta tcaacgtgca 960
   tccgccgcag gcgatcacgt tctgccgccg gtttgggcga accagattgt ccgtttcgct 1020
   aaccgctccc tggaaggtct ggagttcgcg tgcgcgtgga ccgcacagct gctgcacagc 1080
   caacgtcata ttaacgaaat tacgctgaac attccagcca atattagcgc gaccacgggc 1140
   gcacgttccg ccagcgtcgg ctgggccgag tccttgattg gtctgcacct gggcaaggtg 1200
   gctctgatta ccggtggttc ggcgggcatc ggtggtcaaa tcggtcgtct gctggccttg 1260
   tctggcgcgc gtgtgatgct ggccgctcgc gatcgccata aattggaaca gatgcaagcc 1320
   atgattcaaa gcgaattggc ggaggttggt tataccgatg tggaggaccg tgtgcacatc 1380
   gctccgggtt gcgatgtgag cagcgaggcg cagctggcag atctggtgga acgtacgctg 1440
   tccgcattcg gtaccgtgga ttatttgatt aataacgccg gtattgcggg cgtggaggag 1500
   atggtgatcg acatgccggt ggaaggctgg cgtcacaccc tgtttgccaa cctgatttcg 1560
   aattattcgc tgatgcgcaa gttggcgccg ctgatgaaga agcaaggtag cggttacatc 1620
   ctgaacgttt cttcctattt tggcggtgag aaggacgcgg cgattcctta tccgaaccgc 1680
   gccgactacg ccgtctccaa ggctggccaa cgcgcgatgg cggaagtgtt cgctcgtttc 1740
   ctgggtccag agattcagat caatgctatt gccccaggtc cggttgaagg cgaccgcctg 1800
   cgtggtaccg gtgagcgtcc gggcctgttt gctcgtcgcg cccgtctgat cttggagaat 1860
   aaacgcctga acgaattgca cgcggctttg attgctgcgg cccgcaccga tgagcgctcg 1920
   atgcacgagt tggttgaatt gttgctgccg aacgacgtgg ccgcgttgga gcagaaccca 1980
   gcggccccta ccgcgctgcg tgagctggca cgccgcttcc gtagcgaagg tgatccggcg 2040
   gcaagctcct cgtccgcctt gctgaatcgc tccatcgctg ccaagctgtt ggctcgcttg 2100
   cataacggtg gctatgtgct gccggcggat atttttgcaa atctgcctaa tccgccggac 2160
   ccgttcttta cccgtgcgca aattgaccgc gaagctcgca aggtgcgtga tggtattatg 2220
   ggtatgctgt atctgcagcg tatgccaacc gagtttgacg tcgctatggc aaccgtgtac 2280
   tatctggccg atcgtaacgt gagcggcgaa actttccatc cgtctggtgg tttgcgctac 2340
   gagcgtaccc cgaccggtgg cgagctgttc ggcctgccat cgccggaacg tctggcggag 2400
   ctggttggta gcacggtgta cctgatcggt gaacacctga ccgagcacct gaacctgctg 2460
   gctcgtgcct atttggagcg ctacggtgcc cgtcaagtgg tgatgattgt tgagacggaa 2520
   accggtgcgg aaaccatgcg tcgtctgttg catgatcacg tcgaggcagg tcgcctgatg 2580
   actattgtgg caggtgatca gattgaggca gcgattgacc aagcgatcac gcgctatggc 2640
   cgtccgggtc cggtggtgtg cactccattc cgtccactgc caaccgttcc gctggtcggt 2700
   cgtaaagact ccgattggag caccgttttg agcgaggcgg aatttgcgga actgtgtgag 2760
   catcagctga cccaccattt ccgtgttgct cgtaagatcg ccttgtcgga tggcgcgtcg 2820
   ctggcgttgg ttaccccgga aacgactgcg actagcacca cggagcaatt tgctctggcg 2880
   aacttcatca agaccaccct gcacgcgttc accgcgacca tcggtgttga gtcggagcgc 2940
   accgcgcaac gtattctgat taaccaggtt gatctgacgc gccgcgcccg tgcggaagag 3000
   ccgcgtgacc cgcacgagcg tcagcaggaa ttggaacgct tcattgaagc cgttctgctg 3060
   gttaccgctc cgctgcctcc tgaggcagac acgcgctacg caggccgtat tcaccgcggt 3120
   cgtgcgatta ccgtc 3135
<210> 127
   <211> 1217
   <212> PRT
   <213> Erythrobacter sp.
<220>
   <223> Erythrobacter sp. NAP1
<400> 127
   Met Ser Lys Glu Gly Asn Ala Ala Lys Gly Arg Leu Glu Gly Lys Val
   1               5                   10                  15
   Ala Leu Ile Thr Gly Ala Ala Gly Asn Leu Gly Asn Glu Ile Ser Arg
               20                  25                  30
   Ala Phe Ala Arg Glu Gly Ala Phe Val Val Met Thr Gly Arg Thr Glu
           35                  40                  45
   Glu Arg Ile Ser Ala Ala Arg Glu Gln Leu Ile Ala Asp Thr Gly Val
       50                  55                  60
   Ala Pro Glu Arg Ile Asp Thr Ala Val Leu Asp Gly Gly Asn Pro Asp
   65                  70                  75                  80
   Ser Ile Arg Ala Ala Met Ala Lys Leu Arg Lys Glu Tyr Gly Arg Ile
                   85                  90                  95
   Asp Ile Leu Ile Asn Asn Ala Gly Ser Ala Gly Pro Lys Gln Pro Leu
               100                 105                 110
   His Asn Val Pro Leu Ser Pro Gln Glu Met Glu Ala Cys Gly Asp Thr
           115                 120                 125
   Glu Thr Val Arg Asp Ala Met Leu Asn Ile Leu Gly Val Thr Trp Asn
       130                 135                 140
   Met Ala Arg Ile Val Ala Pro Met Met Pro Val Gly Gly Ala Met Val
   145                 150                 155                 160
   Asn Ile Ser Thr Ile Phe Ser His Thr Arg Tyr Tyr Gly Arg Thr Ala
                   165                 170                 175
   Tyr Val Val Pro Lys Ala Ala Leu Asn Ala Leu Ser Asn Gln Leu Ala
               180                 185                 190
   Ser Glu Leu Gly Pro Arg Gly Ile Arg Val Asn Thr Val Phe Pro Gly
           195                 200                 205
   Pro Ile Glu Ser Asp Arg Ile Arg Thr Val Phe Ala Ala Met Asp Glu
       210                 215                 220
   Val Gln Ser Gln Pro Lys Asp Thr Thr Ala Asn Tyr Phe Thr Gly Arg
   225                 230                 235                 240
   Met Ala Leu Thr Arg Ser Val Asn Gly Lys Val Asp Gly Lys Pro Leu
                   245                 250                 255
   Pro Asn Pro Lys Asp Ile Ala Gly Thr Cys Leu Phe Leu Ala Ser Glu
   260 265 270
   Glu Ala Ala Gly Ile Ala Gly Glu Glu Val Asp Val Thr His Gly Leu
   275 280 285
   Ser Ala Asn Arg Thr Ser Ala Ser Thr Tyr Met Thr Arg Pro Ser Met
   290 295 300
   Arg Ser Leu Asp Gly Ala Gly Leu Asn Ile Phe Ile Val Ser Gly Glu
   305 310 315 320
   Asn Trp Asp Asp Ala Leu Val Ala Ala His Thr Leu Ile Gly Ser Gly
   325 330 335
   Ala Lys Val Arg Leu Gly Leu Ala Arg Asn Ala Asp Val Ala Gln Ala
   340 345 350
   Asn Ala Arg Leu Lys Ala Gln Gly Ile Gly Glu Glu Leu Thr Val Thr
   355 360 365
   Arg Phe Asn Arg Ala Glu Pro Asp Ala Met Glu Asp Ala Leu Ala Ala
   370 375 380
   Phe Ser Gly Asp Val Asp Gly Ala Ile Thr Gly Ala Ile Ile Leu Pro
   385 390 395 400
   Val Lys Pro Ser Gly His Phe Thr Gly Ser Leu Leu Ala Ala Asp Asp
   405 410 415
   Asp Thr Val Thr Lys Phe Met Asp Thr Glu Leu Val Gly Ala Ile Ala
   420 425 430
   Val Ser Arg Ser Leu Ala Arg Tyr Trp His Gly Arg Glu Asp Leu Gln
   435 440 445
   Ser Pro Pro Arg Cys Val Phe Met Thr Asn Pro Gly Asp Pro Leu Gly
   450 455 460
   Asn Ser Phe Ala Ser Val Leu Ser Ala Gly Ile Thr Gln Leu Ile Arg
   465 470 475 480
   Ile Trp Arg Asp Glu Glu Arg Val Gln Ala Gly Asn Gly Ser Thr Glu
   485 490 495
   His Ala Val Trp Ser Asn Gln Ile Val Arg His Thr Asn Thr Glu Asp
   500 505 510
   Glu Asn Thr Arg Phe Ala Ser Gly His Ala Thr Arg Val Leu Phe Arg
   515 520 525
   Glu Gln His Ile Ala Glu Ile Asp Leu Lys Leu Pro Ala Asn Ile Ser
   530 535 540
   Glu Glu Thr Gly Ser Arg Lys Ala Met Val Gly Phe Ala Glu Asn Ile
   545 550 555 560
   Thr Gly Leu His Leu Gly Lys Val Ala Phe Ile Thr Gly Gly Ser Ala
   565 570 575
   Gly Ile Gly Gly Gln Val Ala Arg Leu Leu Ala Leu Ala Gly Ala Lys
   580 585 590
   Val Met Met Val Ala Arg Arg Glu Ser Glu Leu Val Ala Ala Arg Asp
   595 600 605
   Arg Ile Val Gly Glu Leu Gln Asp Ile Gly Phe Ala Gly Val Glu Arg
   610 615 620
   Arg Val Lys Tyr Met Ala Asp Ile Asp Val Ser Asp Phe Ala Ser Leu
   625 630 635 640
   Asp Lys Ala Val Asp Ala Thr Leu Glu Glu Phe Gly Arg Ile Asp Tyr
   645 650 655
   Leu Ile Asn Asn Ala Gly Val Ala Gly Ala Glu Asp Met Val Ile Asp
   660 665 670
   Met Glu Pro Glu Ala Trp Arg Phe Thr Leu Asp Ala Asn Leu Ile Ser
   675 680 685
   Asn Tyr His Leu Met Gln Arg Val Val Pro Leu Met Lys Glu Gln Gly
   690 695 700
   Ser Gly Tyr Val Leu Asn Val Ser Ser Tyr Phe Gly Gly Glu Lys Phe
   705 710 715 720
   Leu Ala Val Ala Tyr Pro Asn Arg Ala Asp Tyr Gly Leu Ser Lys Ala
   725 730 735
   Gly Gln Arg Ala Met Val Glu Ala Phe Ser Pro Phe Leu Gly Pro Glu
   740 745 750
   Val Gln Cys Asn Ala Ile Ala Pro Gly Pro Val Asp Gly Asp Arg Leu
   755 760 765
   Ser Gly Thr Gly Gly Lys Pro Gly Leu Phe Gln Arg Arg Ala Lys Leu
   770 775 780
   Ile Leu Glu Asn Lys Arg Leu Asn Ala Val Tyr Ser Ala Val Ile His
   785 790 795 800
   Ala Ile Arg Glu Gly Gly Asp Ala Ala Lys Ile Leu Thr Arg Leu Ser
   805 810 815
   Arg Asn Ser Thr Ser Thr Leu Ser His Asp Ala Glu Ala Pro Glu Glu
   820 825 830
   Leu Arg Lys Leu Ala Leu Asp Phe Ala Ser Gln Gly Asp Gly Leu Cys
   835 840 845
   Thr Trp Asp Gln Tyr Leu Leu Thr Asp Ala Met Ala Gln Arg Leu Leu
   850 855 860
   Val Arg Leu Gln Leu Gly Gly Phe Leu Leu Gly Ser Asn Glu Trp Ala
   865 870 875 880
   Ser Leu Ser Ser Ser Glu Gln Thr Trp Leu Lys Leu Ser Pro Pro Asp
   885 890 895
   Asp Lys Pro Phe Leu Pro Ala Ala Gln Val Asp Lys Val Ala Asn Gly
   900 905 910
   Val Gly Lys Gly Val Ile Ser Gln Leu His Leu Gly Ala Met Pro Thr
   915 920 925
   Glu Ala Glu Val Ala Gln Ala Thr Val Phe Phe Leu Ala Asp Arg Ala
   930 935 940
   Val Ser Gly Glu Thr Phe Met Pro Ser Gly Gly Leu Arg Val Glu Arg
   945 950 955 960
   Ser Asn Thr Glu Arg Glu Met Phe Gly Ser Pro Lys Gln Glu Arg Ile
   965 970 975
   Asp Lys Met Lys Gly Lys Thr Val Trp Ile Ile Gly Glu His Leu Ser
   980 985 990
   Asp Tyr Val Ala Ala Thr Ile Glu Glu Leu Val Ser Gly Cys Gly Val
   995 1000 1005
   Ala Lys Val Val Leu Ile Ala Lys Asp Lys Ser Gly Glu Lys Ala
       1010                1015                1020
   Val Arg Asp Gln Leu Pro Asn Asp Leu Ser Lys Asp Ala Leu Glu
       1025                1030                1035
   Val Leu Ile Ala Gly Asp Gly Leu Glu Glu Ala Met Asp Glu Ala
       1040                1045                1050
   Leu Gly His Trp Gly Lys Pro Thr Thr Val Leu Ser Met Pro Gly
       1055                1060                1065
   Glu Pro Leu Pro Asp His Leu Phe Glu Gly Gly Asn Pro Leu Ser
       1070                1075                1080
   Thr Lys Asp Phe Ala His Met Val Glu Ala Asn Ile Thr Arg His
       1085                1090                1095
   Tyr Arg Val Thr Arg Lys Ala Ser Leu Tyr Asp Gly Cys Gln Val
       1100                1105                1110
   Val Leu Val Ser Pro Asp Val Pro Tyr Gly Ser Asp Gly Pro Gly
       1115                1120                1125
   Val Ala Leu Ala Asn Phe Val Lys Thr Ser Leu His Ala Phe Thr
       1130                1135                1140
   Ala Thr Val Ala Val Glu Asn Glu Arg Leu Val His Asp Val Pro
       1145                1150                1155
   Val Asn Gln Ile Asn Leu Thr Arg Arg Val Ser Ser Glu Glu Pro
       1160                1165                1170
   Arg Asp Ala Asp Glu His Ala Glu Glu Leu Arg Arg Phe Thr Arg
       1175                1180                1185
   Ala Val Leu Leu Val Gly Ala Pro Leu Pro Asp Ala Gln Asp Ser
       1190                1195                1200
   Arg Tyr Arg Ser Lys Ile Tyr Arg Gly Thr Ser Met Thr Val
       1205                1210                1215
<210> 128
   <211> 7999
   <212> DNA
   <213> Artificial Sequence
<220>
   <223> Description of Artificial Sequence: Synthetic polynucleotide
<400> 128
   gtttgacagc ttatcatcga ctgcacggtg caccaatgct tctggcgtca ggcagccatc 60
   ggaagctgtg gtatggctgt gcaggtcgta aatcactgca taattcgtgt cgctcaaggc 120
   gcactcccgt tctggataat gttttttgcg ccgacatcat aacggttctg gcaaatattc 180
   tgaaatgagc tgttgacaat taatcatccg gctcgtataa tgtgtggaat tgtgagcgga 240
   taacaatttc acacaggaaa cagcgccgct gagaaaaagc gaagcggcac tgctctttaa 300
   caatttatca gacaatctgt gtgggcactc gaccggaatt atcgattaac tttattatta 360
   aaaattaaag aggtatatat taatgtatcg attaaataag gaggaataaa ccatgagcaa 420
   agaaggtaat gcggccaaag gccgcctgga gggtaaagtg gccctgatca ccggtgcagc 480
   tggcaacctg ggtaatgaaa ttagccgcgc gtttgcccgt gagggtgcgt tcgtggttat 540
   gaccggccgc acggaagaac gtatctctgc ggcccgcgaa cagctgattg cggataccgg 600
   tgtcgccccg gaacgtatcg acacggcggt gctggatggc ggtaacccgg actctattcg 660
   tgcagctatg gccaaactgc gcaaagaata tggccgtatt gatatcctga ttaacaatgc 720
   aggttcagca ggtccgaaac agccgctgca taatgttccg ctgtcgccgc aagaaatgga 780
   agcatgcggt gataccgaaa cggtgcgcga cgcgatgctg aacatcctgg gcgtgacctg 840
   gaatatggct cgtattgttg cgccgatgat gccggtgggc ggtgcaatgg ttaatatcag 900
   caccattttt agccacacgc gctattacgg ccgtaccgcg tatgtcgtgc cgaaagcggc 960
   cctgaacgca ctgagcaatc agctggcttc tgaactgggc ccgcgtggta ttcgcgttaa 1020
   caccgtcttt ccgggtccga tcgaaagtga tcgtattcgc acggtgttcg cagctatgga 1080
   tgaagttcag tcccaaccga aagacaccac ggcgaattac tttaccggtc gtatggcact 1140
   gacgcgtagc gtcaacggca aagtggatgg taaaccgctg ccgaatccga aagacatcgc 1200
   gggtacctgt ctgttcctgg ccagtgaaga agcggccggc attgccggtg aagaagttga 1260
   tgtcacccat ggcctgtccg caaaccgcac gtctgctagt acctatatga cgcgcccgtc 1320
   tatgcgtagt ctggatggcg cgggtctgaa catcttcatc gtttcaggtg aaaactggga 1380
   tgacgccctg gtcgcagctc acaccctgat cggctcgggt gcaaaagtgc gcctgggcct 1440
   ggcacgtaac gctgatgttg cacaggctaa tgcgcgtctg aaagcgcaag gcattggtga 1500
   agaactgacc gttacgcgtt ttaatcgcgc cgaaccggac gcaatggaag atgctctggc 1560
   ggccttcagt ggcgacgttg atggtgcgat caccggcgcc attatcctgc cggtcaaacc 1620
   gagcggtcat tttacgggct ctctgctggc agctgatgac gataccgtga cgaaattcat 1680
   ggataccgaa ctggtcggtg ccattgcagt gtcccgttca ctggcacgtt actggcacgg 1740
   tcgtgaagat ttgcagagcc cgccgcgttg cgtttttatg accaacccgg gtgacccgct 1800
   gggcaattcc ttcgcgtcag tgctgtcggc cggtatcacg cagctgatcc gtatttggcg 1860
   cgatgaagaa cgcgttcaag caggcaacgg tagtaccgaa catgcggtct ggtccaatca 1920
   gattgtgcgc cacaccaaca cggaagatga aaatacccgt tttgcaagcg gccatgctac 1980
   gcgcgtgctg ttccgtgaac aacacatcgc cgaaattgat ctgaaactgc cggcaaacat 2040
   cagtgaagaa accggttccc gcaaagcaat ggtgggcttt gctgaaaata ttaccggtct 2100
   gcatctgggc aaagttgcat tcatcacggg cggtagtgct ggtattggcg gtcaggtggc 2160
   acgtctgctg gctctggcag gtgccaaagt gatgatggtt gcacgtcgcg aatccgaact 2220
   ggtcgcggca cgtgatcgca tcgtgggtga actgcaagac attggctttg cgggtgttga 2280
   acgtcgcgtc aaatatatgg cggacattga tgtcagtgat tttgcctccc tggacaaagc 2340
   ggtggatgcc accctggaag aattcggccg tatcgactac ctgattaaca atgcgggcgt 2400
   tgcaggtgct gaagacatgg tcatcgatat ggaaccggaa gcctggcgct tcaccctgga 2460
   tgcaaacctg attagcaatt atcatctgat gcagcgtgtt gtcccgctga tgaaagaaca 2520
   aggctctggt tatgtcctga acgtgagctc ttactttggc ggtgaaaaat tcctggcggt 2580
   tgcctatccg aatcgcgccg attacggtct gagcaaagcg ggccagcgtg ccatggtgga 2640
   agcattttct ccgttcctgg gcccggaagt tcaatgcaac gcaattgctc cgggtccggt 2700
   cgacggtgat cgtctgtctg gtaccggcgg taaaccgggc ctgtttcagc gtcgcgcgaa 2760
   actgatcctg gaaaacaaac gcctgaatgc tgtgtatagt gcggttatcc atgccattcg 2820
   tgaaggcggt gatgcagcta aaattctgac ccgtctgagt cgcaattcga ccagcacgct 2880
   gtcccacgat gcagaagcac cggaagaact gcgtaaactg gcactggatt ttgctagcca 2940
   gggcgacggt ctgtgtacct gggatcaata cctgctgacg gacgcaatgg ctcagcgtct 3000
   gctggtgcgt ctgcaactgg gcggttttct gctgggttca aacgaatggg cctcgctgag 3060
   ttcctcagaa cagacctggc tgaaactgtc accgccggac gataaaccgt tcctgccggc 3120
   ggcccaagtt gataaagtcg cgaatggcgt cggtaaaggc gtgatttcgc agctgcatct 3180
   gggtgcaatg ccgaccgaag ccgaagttgc gcaagccacg gtctttttcc tggcagatcg 3240
   cgctgtgtca ggtgaaacct ttatgccgtc gggcggtctg cgtgttgaac gctcaaacac 3300
   cgaacgcgaa atgttcggct cgccgaaaca ggaacgtatc gataaaatga aaggtaaaac 3360
   cgtctggatt atcggcgaac acctgagtga ctatgtggca gctacgatcg aagaactggt 3420
   gtccggttgc ggcgttgcga aagtggttct gattgccaaa gataaaagcg gtgaaaaagc 3480
   ggttcgtgac cagctgccga atgacctgtc taaagatgca ctggaagtgc tgatcgctgg 3540
   tgatggcctg gaagaagcga tggacgaagc cctgggccat tggggtaaac cgaccacggt 3600
   gctgtcaatg ccgggcgaac cgctgccgga tcacctgttt gaaggcggta acccgctgtc 3660
   gaccaaagac ttcgcacata tggtggaagc taatattacc cgtcactatc gcgttacgcg 3720
   taaagcgtcc ctgtacgatg gttgtcaggt cgtgctggtt agcccggatg tcccgtacgg 3780
   ttctgacggt ccgggtgttg cactggctaa ctttgtcaaa acctctctgc atgcgttcac 3840
   cgccacggtg gcagttgaaa atgaacgcct ggttcacgat gtcccggtga accagatcaa 3900
   tctgacgcgt cgcgtgtcga gcgaagaacc gcgtgacgca gatgaacatg ctgaagaact 3960
   gcgtcgcttt acccgtgcag tgctgctggt tggtgcaccg ctgccggacg cacaggattc 4020
   acgttatcgc tcgaaaattt accgcggcac cagcatgacg gtgtaataac cgcggcacga 4080
   gcatgacggt ccatcaccat catcactaat agagatctgc agctggtacc atatgggaat 4140
   tcgaagcttg gctgttttgg cggatgagag aagattttca gcctgataca gattaaatca 4200
   gaacgcagaa gcggtctgat aaaacagaat ttgcctggcg gcagtagcgc ggtggtccca 4260
   cctgacccca tgccgaactc agaagtgaaa cgccgtagcg ccgatggtag tgtggggtct 4320
   ccccatgcga gagtagggaa ctgccaggca tcaaataaaa cgaaaggctc agtcgaaaga 4380
   ctgggccttt cgttttatct gttgtttgtc ggtgaacgct ctcctgagta ggacaaatcc 4440
   gccgggagcg gatttgaacg ttgcgaagca acggcccgga gggtggcggg caggacgccc 4500
   gccataaact gccaggcatc aaattaagca gaaggccatc ctgacggatg gcctttttgc 4560
   gtttctacaa actctttttg tttatttttc taaatacatt caaatatgta tccgctcatg 4620
   agacaataac cctgataaat gcttcaataa tattgaaaaa ggaagagtat gagtattcaa 4680
   catttccgtg tcgcccttat tccctttttt gcggcatttt gccttcctgt ttttgctcac 4740
   ccagaaacgc tggtgaaagt aaaagatgct gaagatcagt tgggtgcacg agtgggttac 4800
   atcgaactgg atctcaacag cggtaagatc cttgagagtt ttcgccccga agaacgtttt 4860
   ccaatgatga gcacttttaa agttctgcta tgtggcgcgg tattatcccg tgttgacgcc 4920
   gggcaagagc aactcggtcg ccgcatacac tattctcaga atgacttggt tgagtactca 4980
   ccagtcacag aaaagcatct tacggatggc atgacagtaa gagaattatg cagtgctgcc 5040
   ataaccatga gtgataacac tgcggccaac ttacttctga caacgatcgg aggaccgaag 5100
   gagctaaccg cttttttgca caacatgggg gatcatgtaa ctcgccttga tcgttgggaa 5160
   ccggagctga atgaagccat accaaacgac gagcgtgaca ccacgatgcc tgtagcaatg 5220
   gcaacaacgt tgcgcaaact attaactggc gaactactta ctctagcttc ccggcaacaa 5280
   ttaatagact ggatggaggc ggataaagtt gcaggaccac ttctgcgctc ggcccttccg 5340
   gctggctggt ttattgctga taaatctgga gccggtgagc gtgggtctcg cggtatcatt 5400
   gcagcactgg ggccagatgg taagccctcc cgtatcgtag ttatctacac gacggggagt 5460
   caggcaacta tggatgaacg aaatagacag atcgctgaga taggtgcctc actgattaag 5520
   cattggtaac tgtcagacca agtttactca tatatacttt agattgattt aaaacttcat 5580
   ttttaattta aaaggatcta ggtgaagatc ctttttgata atctcatgac caaaatccct 5640
   taacgtgagt tttcgttcca ctgagcgtca gaccccgtag aaaagatcaa aggatcttct 5700
   tgagatcctt tttttctgcg cgtaatctgc tgcttgcaaa caaaaaaacc accgctacca 5760
   gcggtggttt gtttgccgga tcaagagcta ccaactcttt ttccgaaggt aactggcttc 5820
   agcagagcgc agataccaaa tactgtcctt ctagtgtagc cgtagttagg ccaccacttc 5880
   aagaactctg tagcaccgcc tacatacctc gctctgctaa tcctgttacc agtggctgct 5940
   gccagtggcg ataagtcgtg tcttaccggg ttggactcaa gacgatagtt accggataag 6000
   gcgcagcggt cgggctgaac ggggggttcg tgcacacagc ccagcttgga gcgaacgacc 6060
   tacaccgaac tgagatacct acagcgtgag ctatgagaaa gcgccacgct tcccgaaggg 6120
   agaaaggcgg acaggtatcc ggtaagcggc agggtcggaa caggagagcg cacgagggag 6180
   cttccagggg gaaacgcctg gtatctttat agtcctgtcg ggtttcgcca cctctgactt 6240
   gagcgtcgat ttttgtgatg ctcgtcaggg gggcggagcc tatggaaaaa cgccagcaac 6300
   gcggcctttt tacggttcct ggccttttgc tggccttttg ctcacatgtt ctttcctgcg 6360
   ttatcccctg attctgtgga taaccgtatt accgcctttg agtgagctga taccgctcgc 6420
   cgcagccgaa cgaccgagcg cagcgagtca gtgagcgagg aagcggaaga gcgcctgatg 6480
   cggtattttc tccttacgca tctgtgcggt atttcacacc gcatatggtg cactctcagt 6540
   acaatctgct ctgatgccgc atagttaagc cagtatacac tccgctatcg ctacgtgact 6600
   gggtcatggc tgcgccccga cacccgccaa cacccgctga cgcgccctga cgggcttgtc 6660
   tgctcccggc atccgcttac agacaagctg tgaccgtctc cgggagctgc atgtgtcaga 6720
   ggttttcacc gtcatcaccg aaacgcgcga ggcagcagat caattcgcgc gcgaaggcga 6780
   agcggcatgc atttacgttg acaccatcga atggtgcaaa acctttcgcg gtatggcatg 6840
   atagcgcccg gaagagagtc aattcagggt ggtgaatgtg aaaccagtaa cgttatacga 6900
   tgtcgcagag tatgccggtg tctcttatca gaccgtttcc cgcgtggtga accaggccag 6960
   ccacgtttct gcgaaaacgc gggaaaaagt ggaagcggcg atggcggagc tgaattacat 7020
   tcccaaccgc gtggcacaac aactggcggg caaacagtcg ttgctgattg gcgttgccac 7080
   ctccagtctg gccctgcacg cgccgtcgca aattgtcgcg gcgattaaat ctcgcgccga 7140
   tcaactgggt gccagcgtgg tggtgtcgat ggtagaacga agcggcgtcg aagcctgtaa 7200
   agcggcggtg cacaatcttc tcgcgcaacg cgtcagtggg ctgatcatta actatccgct 7260
   ggatgaccag gatgccattg ctgtggaagc tgcctgcact aatgttccgg cgttatttct 7320
   tgatgtctct gaccagacac ccatcaacag tattattttc tcccatgaag acggtacgcg 7380
   actgggcgtg gagcatctgg tcgcattggg tcaccagcaa atcgcgctgt tagcgggccc 7440
   attaagttct gtctcggcgc gtctgcgtct ggctggctgg cataaatatc tcactcgcaa 7500
   tcaaattcag ccgatagcgg aacgggaagg cgactggagt gccatgtccg gttttcaaca 7560
   aaccatgcaa atgctgaatg agggcatcgt tcccactgcg atgctggttg ccaacgatca 7620
   gatggcgctg ggcgcaatgc gcgccattac cgagtccggg ctgcgcgttg gtgcggatat 7680
   ctcggtagtg ggatacgacg ataccgaaga cagctcatgt tatatcccgc cgtcaaccac 7740
   catcaaacag gattttcgcc tgctggggca aaccagcgtg gaccgcttgc tgcaactctc 7800
   tcagggccag gcggtgaagg gcaatcagct gttgcccgtc tcactggtga aaagaaaaac 7860
   caccctggcg cccaatacgc aaaccgcctc tccccgcgcg ttggccgatt cattaatgca 7920
   gctggcacga caggtttccc gactggaaag cgggcagtga gcgcaacgca attaatgtga 7980
   gttagcgcga attgatctg 7999
<210> 129
   <211> 7837
   <212> DNA
   <213> Artificial Sequence
<220>
   <223> Description of Artificial Sequence: Synthetic polynucleotide
<400> 129
   catggctttg ccgttggacc cacgtgaccc ggccaccatt gatgcggttt tcgattgggg      60
   cgcaggcgag aatacgggtg gcatccatgc ggcggtcatt ctgccggcaa cctcccacga     120
   accggctccg tgcgtgattg aagtcgatga cgaacgcgtc ctgaatttcc tggccgatga     180
   aattaccggc accatcgtta ttgcgagccg tttggcgcgc tattggcaat cccaacgcct     240
   gaccccgggt gcccgtgccc gcggtccgcg tgttatcttt ctgagcaacg gtgccgatca     300
   aaatggtaat gtttacggtc gtattcaatc tgcggcgatc ggtcaattga ttcgcgtttg     360
   gcgtcacgag gcggagttgg actatcaacg tgcatccgcc gcaggcgatc acgttctgcc     420
   gccggtttgg gcgaaccaga ttgtccgttt cgctaaccgc tccctggaag gtctggagtt     480
   cgcgtgcgcg tggaccgcac agctgctgca cagccaacgt catattaacg aaattacgct     540
   gaacattcca gccaatatta gcgcgaccac gggcgcacgt tccgccagcg tcggctgggc     600
   cgagtccttg attggtctgc acctgggcaa ggtggctctg attaccggtg gttcggcggg     660
   catcggtggt caaatcggtc gtctgctggc cttgtctggc gcgcgtgtga tgctggccgc     720
   tcgcgatcgc cataaattgg aacagatgca agccatgatt caaagcgaat tggcggaggt     780
   tggttatacc gatgtggagg accgtgtgca catcgctccg ggttgcgatg tgagcagcga     840
   ggcgcagctg gcagatctgg tggaacgtac gctgtccgca ttcggtaccg tggattattt     900
   gattaataac gccggtattg cgggcgtgga ggagatggtg atcgacatgc cggtggaagg     960
   ctggcgtcac accctgtttg ccaacctgat ttcgaattat tcgctgatgc gcaagttggc 1020
   gccgctgatg aagaagcaag gtagcggtta catcctgaac gtttcttcct attttggcgg 1080
   tgagaaggac gcggcgattc cttatccgaa ccgcgccgac tacgccgtct ccaaggctgg 1140
   ccaacgcgcg atggcggaag tgttcgctcg tttcctgggt ccagagattc agatcaatgc 1200
   tattgcccca ggtccggttg aaggcgaccg cctgcgtggt accggtgagc gtccgggcct 1260
   gtttgctcgt cgcgcccgtc tgatcttgga gaataaacgc ctgaacgaat tgcacgcggc 1320
   tttgattgct gcggcccgca ccgatgagcg ctcgatgcac gagttggttg aattgttgct 1380
   gccgaacgac gtggccgcgt tggagcagaa cccagcggcc cctaccgcgc tgcgtgagct 1440
   ggcacgccgc ttccgtagcg aaggtgatcc ggcggcaagc tcctcgtccg ccttgctgaa 1500
   tcgctccatc gctgccaagc tgttggctcg cttgcataac ggtggctatg tgctgccggc 1560
   ggatattttt gcaaatctgc ctaatccgcc ggacccgttc tttacccgtg cgcaaattga 1620
   ccgcgaagct cgcaaggtgc gtgatggtat tatgggtatg ctgtatctgc agcgtatgcc 1680
   aaccgagttt gacgtcgcta tggcaaccgt gtactatctg gccgatcgta acgtgagcgg 1740
   cgaaactttc catccgtctg gtggtttgcg ctacgagcgt accccgaccg gtggcgagct 1800
   gttcggcctg ccatcgccgg aacgtctggc ggagctggtt ggtagcacgg tgtacctgat 1860
   cggtgaacac ctgaccgagc acctgaacct gctggctcgt gcctatttgg agcgctacgg 1920
   tgcccgtcaa gtggtgatga ttgttgagac ggaaaccggt gcggaaacca tgcgtcgtct 1980
   gttgcatgat cacgtcgagg caggtcgcct gatgactatt gtggcaggtg atcagattga 2040
   ggcagcgatt gaccaagcga tcacgcgcta tggccgtccg ggtccggtgg tgtgcactcc 2100
   attccgtcca ctgccaaccg ttccgctggt cggtcgtaaa gactccgatt ggagcaccgt 2160
   tttgagcgag gcggaatttg cggaactgtg tgagcatcag ctgacccacc atttccgtgt 2220
   tgctcgtaag atcgccttgt cggatggcgc gtcgctggcg ttggttaccc cggaaacgac 2280
   tgcgactagc accacggagc aatttgctct ggcgaacttc atcaagacca ccctgcacgc 2340
   gttcaccgcg accatcggtg ttgagtcgga gcgcaccgcg caacgtattc tgattaacca 2400
   ggttgatctg acgcgccgcg cccgtgcgga agagccgcgt gacccgcacg agcgtcagca 2460
   ggaattggaa cgcttcattg aagccgttct gctggttacc gctccgctgc ctcctgaggc 2520
   agacacgcgc tacgcaggcc gtattcaccg cggtcgtgcg attaccgtct aatagaagct 2580
   tggctgtttt ggcggatgag agaagatttt cagcctgata cagattaaat cagaacgcag 2640
   aagcggtctg ataaaacaga atttgcctgg cggcagtagc gcggtggtcc cacctgaccc 2700
   catgccgaac tcagaagtga aacgccgtag cgccgatggt agtgtggggt ctccccatct 2760
   gtggtatggc tgtgcaggtc gtaaatcact gcataattcg tgtcgctcaa ggcgcactcc 2820
   cgttctggat aatgtttttt gcgccgacat cataacggtt ctggcaaata ttctgaaatg 2880
   agctgttgac aattaatcat ccggctcgta taatgtgtgg aattgtgagc ggataacaat 2940
   ttcacacagg aaacagcgcc gctgagaaaa agcgaagcgg cactgctctt taacaattta 3000
   tcagacaatc tgtgtgggca ctcgaccgga attatcgatt aactttatta ttaaaaatta 3060
   aagaggtata tattaatgta tcgattaaat aaggaggaat aaaccatggt cgttttagta 3120
   actggagcaa cggcaggttt tggtgaatgc attactcgtc gttttattca acaagggcat 3180
   aaagttatcg ccactggccg tcgccaggaa cggttgcagg agttaaaaga cgaactggga 3240
   gataatctgt atatcgccca actggacgtt cgcaaccgcg ccgctattga agagatgctg 3300
   gcatcgcttc ctgccgagtg gtgcaatatt gatatcctgg taaataatgc cggcctggcg 3360
   ttgggcatgg agcctgcgca taaagccagc gttgaagact gggaaacgat gattgatacc 3420
   aacaacaaag gcctggtata tatgacgcgc gccgtcttac cgggtatggt tgaacgtaat 3480
   catggtcata ttattaacat tggctcaacg gcaggtagct ggccgtatgc cggtggtaac 3540
   gtttacggtg cgacgaaagc gtttgttcgt cagtttagcc tgaatctgcg tacggatctg 3600
   catggtacgg cggtgcgcgt caccgacatc gaaccgggtc tggtgggtgg taccgagttt 3660
   tccaatgtcc gctttaaagg cgatgacggt aaagcagaaa aaacctatca aaataccgtt 3720
   gcattgacgc cagaagatgt cagcgaagcc gtctggtggg tgtcaacgct gcctgctcac 3780
   gtcaatatca ataccctgga aatgatgccg gttacccaaa gctatgccgg actgaatgtc 3840
   caccgtcagt aaggaggcgg tatcattgca gcactggggc cagatggtaa gccctcccgt 3900
   atcgtagtta tctacacgac gggattagaa aaactcatcg agcatcaaat gaaactgcaa 3960
   tttattcata tcaggattat caataccata tttttgaaaa agccgtttct gtaatgaagg 4020
   agaaaactca ccgaggcagt tccataggat ggcaagatcc tggtatcggt ctgcgattcc 4080
   gactcgtcca acatcaatac aacctattaa tttcccctcg tcaaaaataa ggttatcaag 4140
   tgagaaatca ccatgagtga cgactgaatc cggtgagaat ggcaaaagct tatgcatttc 4200
   tttccagact tgttcaacag gccagccatt acgctcgtca tcaaaatcac tcgcatcaac 4260
   caaaccgtta ttcattcgtg attgcgcctg agcgagacga aatacgcgat cgctgttaaa 4320
   aggacaatta caaacaggaa tcgaatgcaa ccggcgcagg aacactgcca gcgcatcaac 4380
   aatattttca cctgaatcag gatattcttc taatacctgg aatgctgttt tcccggggat 4440
   cgcagtggtg agtaaccatg catcatcagg agtacggata aaatgcttga tggtcggaag 4500
   aggcataaat tccgtcagcc agtttagtct gaccatctca tctgtaacat cattggcaac 4560
   gctacctttg ccatgtttca gaaacaactc tggcgcatcg ggcttcccat acaatcgata 4620
   gattgtcgca cctgattgcc cgacattatc gcgagcccat ttatacccat ataaatcagc 4680
   atccatgttg gaatttaatc gcggcctcga gcaagacgtt tcccgttgaa tatggctcat 4740
   aacacccctt gtattactgt ttatgtaagc agacagtttt attgttcatg atgatatatt 4800
   tttatcttgt gcaatgtaac atcagagatt ttgagacaca acgtggcttt gttgaataaa 4860
   tcgaactttt gctgagttga agggagtcag gcaactatgg atgaacgaaa tagacagatc 4920
   gctgagatag gtgcctcact gattaagcat tggtaactgt cagaccaagt ttactcatat 4980
   atactttaga ttgatttaaa acttcatttt taatttaaaa ggatctaggt gaagatcctt 5040
   tttgataatc tcatgaccaa aatcccttaa cgtgagtttt cgttccactg agcgtcagac 5100
   cccgtagaaa agatcaaagg atcttcttga gatccttttt ttctgcgcgt aatctgctgc 5160
   ttgcaaacaa aaaaaccacc gctaccagcg gtggtttgtt tgccggatca agagctacca 5220
   actctttttc cgaaggtaac tggcttcagc agagcgcaga taccaaatac tgtccttcta 5280
   gtgtagccgt agttaggcca ccacttcaag aactctgtag caccgcctac atacctcgct 5340
   ctgctaatcc tgttaccagt ggctgctgcc agtggcgata agtcgtgtct taccgggttg 5400
   gactcaagac gatagttacc ggataaggcg cagcggtcgg gctgaacggg gggttcgtgc 5460
   acacagccca gcttggagcg aacgacctac accgaactga gatacctaca gcgtgagcta 5520
   tgagaaagcg ccacgcttcc cgaagggaga aaggcggaca ggtatccggt aagcggcagg 5580
   gtcggaacag gagagcgcac gagggagctt ccagggggaa acgcctggta tctttatagt 5640
   cctgtcgggt ttcgccacct ctgacttgag cgtcgatttt tgtgatgctc gtcagggggg 5700
   cggagcctat ggaaaaacgc cagcaacgcg gcctttttac ggttcctggc cttttgctgg 5760
   ccttttgctc acatgttctt tcctgcgtta tcccctgatt ctgtggataa ccgtattacc 5820
   gcctttgagt gagctgatac cgctcgccgc agccgaacga ccgagcgcag cgagtcagtg 5880
   agcgaggaag cggaagagcg cctgatgcgg tattttctcc ttacgcatct gtgcggtatt 5940
   tcacaccgca tatggtgcac tctcagtaca atctgctctg atgccgcata gttaagccag 6000
   tatacactcc gctatcgcta cgtgactggg tcatggctgc gccccgacac ccgccaacac 6060
   ccgctgacgc gccctgacgg gcttgtctgc tcccggcatc cgcttacaga caagctgtga 6120
   ccgtctccgg gagctgcatg tgtcagaggt tttcaccgtc atcaccgaaa cgcgcgaggc 6180
   agcagatcaa ttcgcgcgcg aaggcgaagc ggcatgcatt tacgttgaca ccatcgaatg 6240
   gtgcaaaacc tttcgcggta tggcatgata gcgcccggaa gagagtcaat tcagggtggt 6300
   gaatgtgaaa ccagtaacgt tatacgatgt cgcagagtat gccggtgtct cttatcagac 6360
   cgtttcccgc gtggtgaacc aggccagcca cgtttctgcg aaaacgcggg aaaaagtgga 6420
   agcggcgatg gcggagctga attacattcc caaccgcgtg gcacaacaac tggcgggcaa 6480
   acagtcgttg ctgattggcg ttgccacctc cagtctggcc ctgcacgcgc cgtcgcaaat 6540
   tgtcgcggcg attaaatctc gcgccgatca actgggtgcc agcgtggtgg tgtcgatggt 6600
   agaacgaagc ggcgtcgaag cctgtaaagc ggcggtgcac aatcttctcg cgcaacgcgt 6660
   cagtgggctg atcattaact atccgctgga tgaccaggat gccattgctg tggaagctgc 6720
   ctgcactaat gttccggcgt tatttcttga tgtctctgac cagacaccca tcaacagtat 6780
   tattttctcc catgaagacg gtacgcgact gggcgtggag catctggtcg cattgggtca 6840
   ccagcaaatc gcgctgttag cgggcccatt aagttctgtc tcggcgcgtc tgcgtctggc 6900
   tggctggcat aaatatctca ctcgcaatca aattcagccg atagcggaac gggaaggcga 6960
   ctggagtgcc atgtccggtt ttcaacaaac catgcaaatg ctgaatgagg gcatcgttcc 7020
   cactgcgatg ctggttgcca acgatcagat ggcgctgggc gcaatgcgcg ccattaccga 7080
   gtccgggctg cgcgttggtg cggatatctc ggtagtggga tacgacgata ccgaagacag 7140
   ctcatgttat atcccgccgt caaccaccat caaacaggat tttcgcctgc tggggcaaac 7200
   cagcgtggac cgcttgctgc aactctctca gggccaggcg gtgaagggca atcagctgtt 7260
   gcccgtctca ctggtgaaaa gaaaaaccac cctggcgccc aatacgcaaa ccgcctctcc 7320
   ccgcgcgttg gccgattcat taatgcagct ggcacgacag gtttcccgac tggaaagcgg 7380
   gcagtgagcg caacgcaatt aatgtgagtt agcgcgaatt gatctggttt gacagcttat 7440
   catcgactgc acggtgcacc aatgcttctg gcgtcaggca gccatcggaa gctgtggtat 7500
   ggctgtgcag gtcgtaaatc actgcataat tcgtgtcgct caaggcgcac tcccgttctg 7560
   gataatgttt tttgcgccga catcataacg gttctggcaa atattctgaa atgagctgtt 7620
   gacaattaat catccggctc gtataatgtg tggaattgtg agcggataac aatttcacac 7680
   aggaaacagc gccgctgaga aaaagcgaag cggcactgct ctttaacaat ttatcagaca 7740
   atctgtgtgg gcactcgacc ggaattatcg attaacttta ttattaaaaa ttaaagaggt 7800
   atatattaat gtatcgatta aataaggagg aataaac 7837
<210> 130
   <211> 7946
   <212> DNA
   <213> Artificial Sequence
<220>
   <223> Description of Artificial Sequence: Synthetic polynucleotide
<400> 130
   gtttgacagc ttatcatcga ctgcacggtg caccaatgct tctggcgtca ggcagccatc      60
   ggaagctgtg gtatggctgt gcaggtcgta aatcactgca taattcgtgt cgctcaaggc     120
   gcactcccgt tctggataat gttttttgcg ccgacatcat aacggttctg gcaaatattc     180
   tgaaatgagc tgttgacaat taatcatccg gctcgtataa tgtgtggaat tgtgagcgga     240
   taacaatttc acacaggaaa cagcgccgct gagaaaaagc gaagcggcac tgctctttaa     300
   caatttatca gacaatctgt gtgggcactc gaccggaatt atcgattaac tttattatta     360
   aaaattaaag aggtatatat taatgtatcg attaaataag gaggaataaa ccatggtcgt 420
   tttagtaact ggagcaacgg caggttttgg tgaatgcatt actcgtcgtt ttattcaaca 480
   agggcataaa gttatcgcca ctggccgtcg ccaggaacgg ttgcaggagt taaaagacga 540
   actgggagat aatctgtata tcgcccaact ggacgttcgc aaccgcgccg ctattgaaga 600
   gatgctggca tcgcttcctg ccgagtggtg caatattgat atcctggtaa ataatgccgg 660
   cctggcgttg ggcatggagc ctgcgcataa agccagcgtt gaagactggg aaacgatgat 720
   tgataccaac aacaaaggcc tggtatatat gacgcgcgcc gtcttaccgg gtatggttga 780
   acgtaatcat ggtcatatta ttaacattgg ctcaacggca ggtagctggc cgtatgccgg 840
   tggtaacgtt tacggtgcga cgaaagcgtt tgttcgtcag tttagcctga atctgcgtac 900
   ggatctgcat ggtacggcgg tgcgcgtcac cgacatcgaa ccgggtctgg tgggtggtac 960
   cgagttttcc aatgtccgct ttaaaggcga tgacggtaaa gcagaaaaaa cctatcaaaa 1020
   taccgttgca ttgacgccag aagatgtcag cgaagccgtc tggtgggtgt caacgctgcc 1080
   tgctcacgtc aatatcaata ccctggaaat gatgccggtt acccaaagct atgccggact 1140
   gaatgtccac cgtcagtaaa ttaaagaggt ggatccgaga tctcggtagt gggatacgac 1200
   gataccgaag acagctcatg ttatatcccg ccgtcaacca ccatcaaaca ggattttcgc 1260
   ctgctggggc aaaccagcgt ggaccgcttg ctgcaactct ctcagggcca ggcggtgaag 1320
   ggcaatcagc tgttgcccgt ctcactggtg aaaagaaaaa ccaccctggc gcccaatacg 1380
   caaaccgcct ctccccgcgc gttggccgat tcattaatgc agctggcacg acaggtttcc 1440
   cgactggaaa gcgggcagtg agcgcaacgc aattaatgtg agttagcgcg aattgatctg 1500
   gtttgacagc ttatcatcga ctgcacggtg caccaatgct tctggcgtca ggcagccatc 1560
   ggaagctgtg gtatggctgt gcaggtcgta aatcactgca taattcgtgt cgctcaaggc 1620
   gcactcccgt tctggataat gttttttgcg ccgacatcat aacggttctg gcaaatattc 1680
   tgaaatgagc tgttgacaat taatcatccg gctcgtataa tgtgtggaat tgtgagcgga 1740
   taacaatttc acacaggaaa cagcgccgct gagaaaaagc gaagcggcac tgctctttaa 1800
   caatttatca gacaatctgt gtgggcactc gaccggaatt atcgattaac tttattatta 1860
   aaaattaaag aggtatatat taatgtatcg attaaataag gaggaataaa ccatggcagg 1920
   cgatcacgtt ctgccgccgg tttgggcgaa ccagattgtc cgtttcgcta accgctccct 1980
   ggaaggtctg gagttcgcgt gcgcgtggac cgcacagctg ctgcacagcc aacgtcatat 2040
   taacgaaatt acgctgaaca ttccagccaa tattagcgcg accacgggcg cacgttccgc 2100
   cagcgtcggc tgggccgagt ccttgattgg tctgcacctg ggcaaggtgg ctctgattac 2160
   cggtggttcg gcgggcatcg gtggtcaaat cggtcgtctg ctggccttgt ctggcgcgcg 2220
   tgtgatgctg gccgctcgcg atcgccataa attggaacag atgcaagcca tgattcaaag 2280
   cgaattggcg gaggttggtt ataccgatgt ggaggaccgt gtgcacatcg ctccgggttg 2340
   cgatgtgagc agcgaggcgc agctggcaga tctggtggaa cgtacgctgt ccgcattcgg 2400
   taccgtggat tatttgatta ataacgccgg tattgcgggc gtggaggaga tggtgatcga 2460
   catgccggtg gaaggctggc gtcacaccct gtttgccaac ctgatttcga attattcgct 2520
   gatgcgcaag ttggcgccgc tgatgaagaa gcaaggtagc ggttacatcc tgaacgtttc 2580
   ttcctatttt ggcggtgaga aggacgcggc gattccttat ccgaaccgcg ccgactacgc 2640
   cgtctccaag gctggccaac gcgcgatggc ggaagtgttc gctcgtttcc tgggtccaga 2700
   gattcagatc aatgctattg ccccaggtcc ggttgaaggc gaccgcctgc gtggtaccgg 2760
   tgagcgtccg ggcctgtttg ctcgtcgcgc ccgtctgatc ttggagaata aacgcctgaa 2820
   cgaattgcac gcggctttga ttgctgcggc ccgcaccgat gagcgctcga tgcacgagtt 2880
   ggttgaattg ttgctgccga acgacgtggc cgcgttggag cagaacccag cggcccctac 2940
   cgcgctgcgt gagctggcac gccgcttccg tagcgaaggt gatccggcgg caagctcctc 3000
   gtccgccttg ctgaatcgct ccatcgctgc caagctgttg gctcgcttgc ataacggtgg 3060
   ctatgtgctg ccggcggata tttttgcaaa tctgcctaat ccgccggacc cgttctttac 3120
   ccgtgcgcaa attgaccgcg aagctcgcaa ggtgcgtgat ggtattatgg gtatgctgta 3180
   tctgcagcgt atgccaaccg agtttgacgt cgctatggca accgtgtact atctggccga 3240
   tcgtaacgtg agcggcgaaa ctttccatcc gtctggtggt ttgcgctacg agcgtacccc 3300
   gaccggtggc gagctgttcg gcctgccatc gccggaacgt ctggcggagc tggttggtag 3360
   cacggtgtac ctgatcggtg aacacctgac cgagcacctg aacctgctgg ctcgtgccta 3420
   tttggagcgc tacggtgccc gtcaagtggt gatgattgtt gagacggaaa ccggtgcgga 3480
   aaccatgcgt cgtctgttgc atgatcacgt cgaggcaggt cgcctgatga ctattgtggc 3540
   aggtgatcag attgaggcag cgattgacca agcgatcacg cgctatggcc gtccgggtcc 3600
   ggtggtgtgc actccattcc gtccactgcc aaccgttccg ctggtcggtc gtaaagactc 3660
   cgattggagc accgttttga gcgaggcgga atttgcggaa ctgtgtgagc atcagctgac 3720
   ccaccatttc cgtgttgctc gtaagatcgc cttgtcggat ggcgcgtcgc tggcgttggt 3780
   taccccggaa acgactgcga ctagcaccac ggagcaattt gctctggcga acttcatcaa 3840
   gaccaccctg cacgcgttca ccgcgaccat cggtgttgag tcggagcgca ccgcgcaacg 3900
   tattctgatt aaccaggttg atctgacgcg ccgcgcccgt gcggaagagc cgcgtgaccc 3960
   gcacgagcgt cagcaggaat tggaacgctt cattgaagcc gttctgctgg ttaccgctcc 4020
   gctgcctcct gaggcagaca cgcgctacgc aggccgtatt caccgcggtc gtgcgattac 4080
   cgtctaatag aagcttggct gttttggcgg atgagagaag attttcagcc tgatacagat 4140
   taaatcagaa cgcagaagcg gtctgataaa acagaatttg cctggcggca gtagcgcggt 4200
   ggtcccacct gaccccatgc cgaactcaga agtgaaacgc cgtagcgccg atggtagtgt 4260
   ggggtctccc catgcgagag tagggaactg ccaggcatca aataaaacga aaggctcagt 4320
   cgaaagactg ggcctttcgt tttatctgtt gtttgtcggt gaacgctctc ctgagtagga 4380
   caaatccgcc gggagcggat ttgaacgttg cgaagcaacg gcccggaggg tggcgggcag 4440
   gacgcccgcc ataaactgcc aggcatcaaa ttaagcagaa ggccatcctg acggatggcc 4500
   tttttgcgtt tctacaaact ctttttgttt atttttctaa atacattcaa atatgtatcc 4560
   gctcatgaga caataaccct gataaatgct tcaataatat tgaaaaagga agagtatgag 4620
   tattcaacat ttccgtgtcg cccttattcc cttttttgcg gcattttgcc ttcctgtttt 4680
   tgctcaccca gaaacgctgg tgaaagtaaa agatgctgaa gatcagttgg gtgcacgagt 4740
   gggttacatc gaactggatc tcaacagcgg taagatcctt gagagttttc gccccgaaga 4800
   acgttttcca atgatgagca cttttaaagt tctgctatgt ggcgcggtat tatcccgtgt 4860
   tgacgccggg caagagcaac tcggtcgccg catacactat tctcagaatg acttggttga 4920
   gtactcacca gtcacagaaa agcatcttac ggatggcatg acagtaagag aattatgcag 4980
   tgctgccata accatgagtg ataacactgc ggccaactta cttctgacaa cgatcggagg 5040
   accgaaggag ctaaccgctt ttttgcacaa catgggggat catgtaactc gccttgatcg 5100
   ttgggaaccg gagctgaatg aagccatacc aaacgacgag cgtgacacca cgatgcctgt 5160
   agcaatggca acaacgttgc gcaaactatt aactggcgaa ctacttactc tagcttcccg 5220
   gcaacaatta atagactgga tggaggcgga taaagttgca ggaccacttc tgcgctcggc 5280
   ccttccggct ggctggttta ttgctgataa atctggagcc ggtgagcgtg ggtctcgcgg 5340
   tatcattgca gcactggggc cagatggtaa gccctcccgt atcgtagtta tctacacgac 5400
   ggggagtcag gcaactatgg atgaacgaaa tagacagatc gctgagatag gtgcctcact 5460
   gattaagcat tggtaactgt cagaccaagt ttactcatat atactttaga ttgatttaaa 5520
   acttcatttt taatttaaaa ggatctaggt gaagatcctt tttgataatc tcatgaccaa 5580
   aatcccttaa cgtgagtttt cgttccactg agcgtcagac cccgtagaaa agatcaaagg 5640
   atcttcttga gatccttttt ttctgcgcgt aatctgctgc ttgcaaacaa aaaaaccacc 5700
   gctaccagcg gtggtttgtt tgccggatca agagctacca actctttttc cgaaggtaac 5760
   tggcttcagc agagcgcaga taccaaatac tgtccttcta gtgtagccgt agttaggcca 5820
   ccacttcaag aactctgtag caccgcctac atacctcgct ctgctaatcc tgttaccagt 5880
   ggctgctgcc agtggcgata agtcgtgtct taccgggttg gactcaagac gatagttacc 5940
   ggataaggcg cagcggtcgg gctgaacggg gggttcgtgc acacagccca gcttggagcg 6000
   aacgacctac accgaactga gatacctaca gcgtgagcta tgagaaagcg ccacgcttcc 6060
   cgaagggaga aaggcggaca ggtatccggt aagcggcagg gtcggaacag gagagcgcac 6120
   gagggagctt ccagggggaa acgcctggta tctttatagt cctgtcgggt ttcgccacct 6180
   ctgacttgag cgtcgatttt tgtgatgctc gtcagggggg cggagcctat ggaaaaacgc 6240
   cagcaacgcg gcctttttac ggttcctggc cttttgctgg ccttttgctc acatgttctt 6300
   tcctgcgtta tcccctgatt ctgtggataa ccgtattacc gcctttgagt gagctgatac 6360
   cgctcgccgc agccgaacga ccgagcgcag cgagtcagtg agcgaggaag cggaagagcg 6420
   cctgatgcgg tattttctcc ttacgcatct gtgcggtatt tcacaccgca tatggtgcac 6480
   tctcagtaca atctgctctg atgccgcata gttaagccag tatacactcc gctatcgcta 6540
   cgtgactggg tcatggctgc gccccgacac ccgccaacac ccgctgacgc gccctgacgg 6600
   gcttgtctgc tcccggcatc cgcttacaga caagctgtga ccgtctccgg gagctgcatg 6660
   tgtcagaggt tttcaccgtc atcaccgaaa cgcgcgaggc agcagatcaa ttcgcgcgcg 6720
   aaggcgaagc ggcatgcatt tacgttgaca ccatcgaatg gtgcaaaacc tttcgcggta 6780
   tggcatgata gcgcccggaa gagagtcaat tcagggtggt gaatgtgaaa ccagtaacgt 6840
   tatacgatgt cgcagagtat gccggtgtct cttatcagac cgtttcccgc gtggtgaacc 6900
   aggccagcca cgtttctgcg aaaacgcggg aaaaagtgga agcggcgatg gcggagctga 6960
   attacattcc caaccgcgtg gcacaacaac tggcgggcaa acagtcgttg ctgattggcg 7020
   ttgccacctc cagtctggcc ctgcacgcgc cgtcgcaaat tgtcgcggcg attaaatctc 7080
   gcgccgatca actgggtgcc agcgtggtgg tgtcgatggt agaacgaagc ggcgtcgaag 7140
   cctgtaaagc ggcggtgcac aatcttctcg cgcaacgcgt cagtgggctg atcattaact 7200
   atccgctgga tgaccaggat gccattgctg tggaagctgc ctgcactaat gttccggcgt 7260
   tatttcttga tgtctctgac cagacaccca tcaacagtat tattttctcc catgaagacg 7320
   gtacgcgact gggcgtggag catctggtcg cattgggtca ccagcaaatc gcgctgttag 7380
   cgggcccatt aagttctgtc tcggcgcgtc tgcgtctggc tggctggcat aaatatctca 7440
   ctcgcaatca aattcagccg atagcggaac gggaaggcga ctggagtgcc atgtccggtt 7500
   ttcaacaaac catgcaaatg ctgaatgagg gcatcgttcc cactgcgatg ctggttgcca 7560
   acgatcagat ggcgctgggc gcaatgcgcg ccattaccga gtccgggctg cgcgttggtg 7620
   cggatatctc ggtagtggga tacgacgata ccgaagacag ctcatgttat atcccgccgt 7680
   caaccaccat caaacaggat tttcgcctgc tggggcaaac cagcgtggac cgcttgctgc 7740
   aactctctca gggccaggcg gtgaagggca atcagctgtt gcccgtctca ctggtgaaaa 7800
   gaaaaaccac cctggcgccc aatacgcaaa ccgcctctcc ccgcgcgttg gccgattcat 7860
   taatgcagct ggcacgacag gtttcccgac tggaaagcgg gcagtgagcg caacgcaatt 7920
   aatgtgagtt agcgcgaatt gatctg 7946
<210> 131
   <211> 7934
   <212> DNA
   <213> Artificial Sequence
<220>
   <223> Description of Artificial Sequence: Synthetic polynucleotide
<400> 131
   catggccggt acgggtcgtt tggctggtaa aattgcattg atcaccggtg gtgctggtaa 60
   cattggttcc gagctgaccc gccgttttct ggccgagggt gcgacggtta ttatcagcgg 120
   ccgtaaccgt gcgaagctga ccgcgctggc cgagcgcatg caagccgagg ccggcgtgcc 180
   ggccaagcgc attgatttgg aggtgatgga tggttccgac cctgtggctg tccgtgccgg 240
   tatcgaggca atcgtcgctc gccacggtca gattgacatt ctggttaaca acgcgggctc 300
   cgccggtgcc caacgtcgct tggcggaaat tccgctgacg gaggcagaat tgggtccggg 360
   tgcggaggag actttgcacg cttcgatcgc gaatctgttg ggcatgggtt ggcacctgat 420
   gcgtattgcg gctccgcaca tgccagttgg ctccgcagtt atcaacgttt cgactatttt 480
   ctcgcgcgca gagtactatg gtcgcattcc gtacgttacc ccgaaggcag cgctgaacgc 540
   tttgtcccag ctggctgccc gcgagctggg cgctcgtggc atccgcgtta acactatttt 600
   cccaggtcct attgagtccg accgcatccg taccgtgttt caacgtatgg atcaactgaa 660
   gggtcgcccg gagggcgaca ccgcccatca ctttttgaac accatgcgcc tgtgccgcgc 720
   aaacgaccaa ggcgctttgg aacgccgctt tccgtccgtt ggcgatgttg ctgatgcggc 780
   tgtgtttctg gcttctgctg agagcgcggc actgtcgggt gagacgattg aggtcaccca 840
   cggtatggaa ctgccggcgt gtagcgaaac ctccttgttg gcgcgtaccg atctgcgtac 900
   catcgacgcg agcggtcgca ctaccctgat ttgcgctggc gatcaaattg aagaagttat 960
   ggccctgacg ggcatgctgc gtacgtgcgg tagcgaagtg attatcggct tccgttctgc 1020
   ggctgccctg gcgcaatttg agcaggcagt gaatgaatct cgccgtctgg caggtgcgga 1080
   tttcaccccg ccgatcgctt tgccgttgga cccacgtgac ccggccacca ttgatgcggt 1140
   tttcgattgg ggcgcaggcg agaatacggg tggcatccat gcggcggtca ttctgccggc 1200
   aacctcccac gaaccggctc cgtgcgtgat tgaagtcgat gacgaacgcg tcctgaattt 1260
   cctggccgat gaaattaccg gcaccatcgt tattgcgagc cgtttggcgc gctattggca 1320
   atcccaacgc ctgaccccgg gtgcccgtgc ccgcggtccg cgtgttatct ttctgagcaa 1380
   cggtgccgat caaaatggta atgtttacgg tcgtattcaa tctgcggcga tcggtcaatt 1440
   gattcgcgtt tggcgtcacg aggcggagtt ggactatcaa cgtgcatccg ccgcaggcga 1500
   tcacgttctg ccgccggttt gggcgaacca gattgtccgt ttcgctaacc gctccctgga 1560
   aggtctggag ttcgcgtgcg cgtggaccgc acagctgctg cacagccaac gtcatattaa 1620
   cgaaattacg ctgaacattc cagccaatat tagcgcgacc acgggcgcac gttccgccag 1680
   cgtcggctgg gccgagtcct tgattggtct gcacctgggc aaggtggctc tgattaccgg 1740
   tggttcggcg ggcatcggtg gtcaaatcgg tcgtctgctg gccttgtctg gcgcgcgtgt 1800
   gatgctggcc gctcgcgatc gccataaatt ggaacagatg caagccatga ttcaaagcga 1860
   attggcggag gttggttata ccgatgtgga ggaccgtgtg cacatcgctc cgggttgcga 1920
   tgtgagcagc gaggcgcagc tggcagatct ggtggaacgt acgctgtccg cattcggtac 1980
   cgtggattat ttgattaata acgccggtat tgcgggcgtg gaggagatgg tgatcgacat 2040
   gccggtggaa ggctggcgtc acaccctgtt tgccaacctg atttcgaatt attcgctgat 2100
   gcgcaagttg gcgccgctga tgaagaagca aggtagcggt tacatcctga acgtttcttc 2160
   ctattttggc ggtgagaagg acgcggcgat tccttatccg aaccgcgccg actacgccgt 2220
   ctccaaggct ggccaacgcg cgatggcgga agtgttcgct cgtttcctgg gtccagagat 2280
   tcagatcaat gctattgccc caggtccggt tgaaggcgac cgcctgcgtg gtaccggtga 2340
   gcgtccgggc ctgtttgctc gtcgcgcccg tctgatcttg gagaataaac gcctgaacga 2400
   attgcacgcg gctttgattg ctgcggcccg caccgatgag cgctcgatgc acgagttggt 2460
   tgaattgttg ctgccgaacg acgtggccgc gttggagcag aacccagcgg cccctaccgc 2520
   gctgcgtgag ctggcacgcc gcttccgtag cgaaggtgat ccggcggcaa gctcctcgtc 2580
   cgccttgctg aatcgctcca tcgctgccaa gctgttggct cgcttgcata acggtggcta 2640
   tgtgctgccg gcggatattt ttgcaaatct gcctaatccg ccggacccgt tctttacccg 2700
   tgcgcaaatt gaccgcgaag ctcgcaaggt gcgtgatggt attatgggta tgctgtatct 2760
   gcagcgtatg ccaaccgagt ttgacgtcgc tatggcaacc gtgtactatc tggccgatcg 2820
   taacgtgagc ggcgaaactt tccatccgtc tggtggtttg cgctacgagc gtaccccgac 2880
   cggtggcgag ctgttcggcc tgccatcgcc ggaacgtctg gcggagctgg ttggtagcac 2940
   ggtgtacctg atcggtgaac acctgaccga gcacctgaac ctgctggctc gtgcctattt 3000
   ggagcgctac ggtgcccgtc aagtggtgat gattgttgag acggaaaccg gtgcggaaac 3060
   catgcgtcgt ctgttgcatg atcacgtcga ggcaggtcgc ctgatgacta ttgtggcagg 3120
   tgatcagatt gaggcagcga ttgaccaagc gatcacgcgc tatggccgtc cgggtccggt 3180
   ggtgtgcact ccattccgtc cactgccaac cgttccgctg gtcggtcgta aagactccga 3240
   ttggagcacc gttttgagcg aggcggaatt tgcggaactg tgtgagcatc agctgaccca 3300
   ccatttccgt gttgctcgta agatcgcctt gtcggatggc gcgtcgctgg cgttggttac 3360
   cccggaaacg actgcgacta gcaccacgga gcaatttgct ctggcgaact tcatcaagac 3420
   caccctgcac gcgttcaccg cgaccatcgg tgttgagtcg gagcgcaccg cgcaacgtat 3480
   tctgattaac caggttgatc tgacgcgccg cgcccgtgcg gaagagccgc gtgacccgca 3540
   cgagcgtcag caggaattgg aacgcttcat tgaagccgtt ctgctggtta ccgctccgct 3600
   gcctcctgag gcagacacgc gctacgcagg ccgtattcac cgcggtcgtg cgattaccgt 3660
   ctaatagaag cttggctgtt ttggcggatg agagaagatt ttcagcctga tacagattaa 3720
   atcagaacgc agaagcggtc tgataaaaca gaatttgcct ggcggcagta gcgcggtggt 3780
   cccacctgac cccatgccga actcagaagt gaaacgccgt agcgccgatg gtagtgtggg 3840
   gtctccccat gcgagagtag ggaactgcca ggcatcaaat aaaacgaaag gctcagtcga 3900
   aagactgggc ctttcgtttt atctgttgtt tgtcggtgaa cgctctcctg agtaggacaa 3960
   atccgccggg agcggatttg aacgttgcga agcaacggcc cggagggtgg cgggcaggac 4020
   gcccgccata aactgccagg catcaaatta agcagaaggc catcctgacg gatggccttt 4080
   ttgcgtttct acaaactctt tttgtttatt tttctaaata cattcaaata tgtatccgct 4140
   catgagacaa taaccctgat aaatgcttca ataatattga aaaaggaaga gtatgagtat 4200
   tcaacatttc cgtgtcgccc ttattccctt ttttgcggca ttttgccttc ctgtttttgc 4260
   tcacccagaa acgctggtga aagtaaaaga tgctgaagat cagttgggtg cacgagtggg 4320
   ttacatcgaa ctggatctca acagcggtaa gatccttgag agttttcgcc ccgaagaacg 4380
   ttttccaatg atgagcactt ttaaagttct gctatgtggc gcggtattat cccgtgttga 4440
   cgccgggcaa gagcaactcg gtcgccgcat acactattct cagaatgact tggttgagta 4500
   ctcaccagtc acagaaaagc atcttacgga tggcatgaca gtaagagaat tatgcagtgc 4560
   tgccataacc atgagtgata acactgcggc caacttactt ctgacaacga tcggaggacc 4620
   gaaggagcta accgcttttt tgcacaacat gggggatcat gtaactcgcc ttgatcgttg 4680
   ggaaccggag ctgaatgaag ccataccaaa cgacgagcgt gacaccacga tgcctgtagc 4740
   aatggcaaca acgttgcgca aactattaac tggcgaacta cttactctag cttcccggca 4800
   acaattaata gactggatgg aggcggataa agttgcagga ccacttctgc gctcggccct 4860
   tccggctggc tggtttattg ctgataaatc tggagccggt gagcgtgggt ctcgcggtat 4920
   cattgcagca ctggggccag atggtaagcc ctcccgtatc gtagttatct acacgacggg 4980
   gagtcaggca actatggatg aacgaaatag acagatcgct gagataggtg cctcactgat 5040
   taagcattgg taactgtcag accaagttta ctcatatata ctttagattg atttaaaact 5100
   tcatttttaa tttaaaagga tctaggtgaa gatccttttt gataatctca tgaccaaaat 5160
   cccttaacgt gagttttcgt tccactgagc gtcagacccc gtagaaaaga tcaaaggatc 5220
   ttcttgagat cctttttttc tgcgcgtaat ctgctgcttg caaacaaaaa aaccaccgct 5280
   accagcggtg gtttgtttgc cggatcaaga gctaccaact ctttttccga aggtaactgg 5340
   cttcagcaga gcgcagatac caaatactgt ccttctagtg tagccgtagt taggccacca 5400
   cttcaagaac tctgtagcac cgcctacata cctcgctctg ctaatcctgt taccagtggc 5460
   tgctgccagt ggcgataagt cgtgtcttac cgggttggac tcaagacgat agttaccgga 5520
   taaggcgcag cggtcgggct gaacgggggg ttcgtgcaca cagcccagct tggagcgaac 5580
   gacctacacc gaactgagat acctacagcg tgagctatga gaaagcgcca cgcttcccga 5640
   agggagaaag gcggacaggt atccggtaag cggcagggtc ggaacaggag agcgcacgag 5700
   ggagcttcca gggggaaacg cctggtatct ttatagtcct gtcgggtttc gccacctctg 5760
   acttgagcgt cgatttttgt gatgctcgtc aggggggcgg agcctatgga aaaacgccag 5820
   caacgcggcc tttttacggt tcctggcctt ttgctggcct tttgctcaca tgttctttcc 5880
   tgcgttatcc cctgattctg tggataaccg tattaccgcc tttgagtgag ctgataccgc 5940
   tcgccgcagc cgaacgaccg agcgcagcga gtcagtgagc gaggaagcgg aagagcgcct 6000
   gatgcggtat tttctcctta cgcatctgtg cggtatttca caccgcatat ggtgcactct 6060
   cagtacaatc tgctctgatg ccgcatagtt aagccagtat acactccgct atcgctacgt 6120
   gactgggtca tggctgcgcc ccgacacccg ccaacacccg ctgacgcgcc ctgacgggct 6180
   tgtctgctcc cggcatccgc ttacagacaa gctgtgaccg tctccgggag ctgcatgtgt 6240
   cagaggtttt caccgtcatc accgaaacgc gcgaggcagc agatcaattc gcgcgcgaag 6300
   gcgaagcggc atgcatttac gttgacacca tcgaatggtg caaaaccttt cgcggtatgg 6360
   catgatagcg cccggaagag agtcaattca gggtggtgaa tgtgaaacca gtaacgttat 6420
   acgatgtcgc agagtatgcc ggtgtctctt atcagaccgt ttcccgcgtg gtgaaccagg 6480
   ccagccacgt ttctgcgaaa acgcgggaaa aagtggaagc ggcgatggcg gagctgaatt 6540
   acattcccaa ccgcgtggca caacaactgg cgggcaaaca gtcgttgctg attggcgttg 6600
   ccacctccag tctggccctg cacgcgccgt cgcaaattgt cgcggcgatt aaatctcgcg 6660
   ccgatcaact gggtgccagc gtggtggtgt cgatggtaga acgaagcggc gtcgaagcct 6720
   gtaaagcggc ggtgcacaat cttctcgcgc aacgcgtcag tgggctgatc attaactatc 6780
   cgctggatga ccaggatgcc attgctgtgg aagctgcctg cactaatgtt ccggcgttat 6840
   ttcttgatgt ctctgaccag acacccatca acagtattat tttctcccat gaagacggta 6900
   cgcgactggg cgtggagcat ctggtcgcat tgggtcacca gcaaatcgcg ctgttagcgg 6960
   gcccattaag ttctgtctcg gcgcgtctgc gtctggctgg ctggcataaa tatctcactc 7020
   gcaatcaaat tcagccgata gcggaacggg aaggcgactg gagtgccatg tccggttttc 7080
   aacaaaccat gcaaatgctg aatgagggca tcgttcccac tgcgatgctg gttgccaacg 7140
   atcagatggc gctgggcgca atgcgcgcca ttaccgagtc cgggctgcgc gttggtgcgg 7200
   atatctcggt agtgggatac gacgataccg aagacagctc atgttatatc ccgccgtcaa 7260
   ccaccatcaa acaggatttt cgcctgctgg ggcaaaccag cgtggaccgc ttgctgcaac 7320
   tctctcaggg ccaggcggtg aagggcaatc agctgttgcc cgtctcactg gtgaaaagaa 7380
   aaaccaccct ggcgcccaat acgcaaaccg cctctccccg cgcgttggcc gattcattaa 7440
   tgcagctggc acgacaggtt tcccgactgg aaagcgggca gtgagcgcaa cgcaattaat 7500
   gtgagttagc gcgaattgat ctggtttgac agcttatcat cgactgcacg gtgcaccaat 7560
   gcttctggcg tcaggcagcc atcggaagct gtggtatggc tgtgcaggtc gtaaatcact 7620
   gcataattcg tgtcgctcaa ggcgcactcc cgttctggat aatgtttttt gcgccgacat 7680
   cataacggtt ctggcaaata ttctgaaatg agctgttgac aattaatcat ccggctcgta 7740
   taatgtgtgg aattgtgagc ggataacaat ttcacacagg aaacagcgcc gctgagaaaa 7800
   agcgaagcgg cactgctctt taacaattta tcagacaatc tgtgtgggca ctcgaccgga 7860
   attatcgatt aactttatta ttaaaaatta aagaggtata tattaatgta tcgattaaat 7920
   aaggaggaat aaac 7934
<210> 132
   <211> 11744
   <212> DNA
   <213> Artificial Sequence
<220>
   <223> Description of Artificial Sequence: Synthetic polynucleotide
<400> 132
   ccttcaactc agcaaaagtt cgatttattc aacaaagcca cgttgtgtct caaaatctct 60
   gatgttacat tgcacaagat aaaaatatat catcatgaac aataaaactg tctgcttaca 120
   taaacagtaa tacaaggggt gttatgagcc atattcaacg ggaaacgtct tgctcgaggc 180
   cgcgattaaa ttccaacatg gatgctgatt tatatgggta taaatgggct cgcgataatg 240
   tcgggcaatc aggtgcgaca atctatcgat tgtatgggaa gcccgatgcg ccagagttgt 300
   ttctgaaaca tggcaaaggt agcgttgcca atgatgttac agatgagatg gtcagactaa 360
   actggctgac ggaatttatg cctcttccga ccatcaagca ttttatccgt actcctgatg 420
   atgcatggtt actcaccact gcgatccccg ggaaaacagc attccaggta ttagaagaat 480
   atcctgattc aggtgaaaat attgttgatg cgctggcagt gttcctgcgc cggttgcatt 540
   cgattcctgt ttgtaattgt ccttttaaca gcgatcgcgt atttcgtctc gctcaggcgc 600
   aatcacgaat gaataacggt ttggttgatg cgagtgattt tgatgacgag cgtaatggct 660
   ggcctgttga acaagtctgg aaagaaatgc ataagctttt gccattctca ccggattcag 720
   tcgtcactca tggtgatttc tcacttgata accttatttt tgacgagggg aaattaatag 780
   gttgtattga tgttggacga gtcggaatcg cagaccgata ccaggatctt gccatcctat 840
   ggaactgcct cggtgagttt tctccttcat tacagaaacg gctttttcaa aaatatggta 900
   ttgataatcc tgatatgaat aaattgcagt ttcatttgat gctcgatgag tttttctaat 960
   cgagtcaggc aactatggat gaacgaaata gacagatcgc tgagataggt gcctcactga 1020
   ttaagcattg gtaactgtca gaccaagttt actcatatat actttagatt gatttaaaac 1080
   ttcattttta atttaaaagg atctaggtga agatcctttt tgataatctc atgaccaaaa 1140
   tcccttaacg tgagttttcg ttccactgag cgtcagaccc cgtagaaaag atcaaaggat 1200
   cttcttgaga tccttttttt ctgcgcgtaa tctgctgctt gcaaacaaaa aaaccaccgc 1260
   taccagcggt ggtttgtttg ccggatcaag agctaccaac tctttttccg aaggtaactg 1320
   gcttcagcag agcgcagata ccaaatactg tccttctagt gtagccgtag ttaggccacc 1380
   acttcaagaa ctctgtagca ccgcctacat acctcgctct gctaatcctg ttaccagtgg 1440
   ctgctgccag tggcgataag tcgtgtctta ccgggttgga ctcaagacga tagttaccgg 1500
   ataaggcgca gcggtcgggc tgaacggggg gttcgtgcac acagcccagc ttggagcgaa 1560
   cgacctacac cgaactgaga tacctacagc gtgagctatg agaaagcgcc acgcttcccg 1620
   aagggagaaa ggcggacagg tatccggtaa gcggcagggt cggaacagga gagcgcacga 1680
   gggagcttcc agggggaaac gcctggtatc tttatagtcc tgtcgggttt cgccacctct 1740
   gacttgagcg tcgatttttg tgatgctcgt caggggggcg gagcctatgg aaaaacgcca 1800
   gcaacgcggc ctttttacgg ttcctggcct tttgctggcc ttttgctcac atgttctttc 1860
   ctgcgttatc ccctgattct gtggataacc gtattaccgc ctttgagtga gctgataccg 1920
   ctcgccgcag ccgaacgacc gagcgcagcg agtcagtgag cgaggaagcg gaagagcgcc 1980
   tgatgcggta ttttctcctt acgcatctgt gcggtatttc acaccgcata tcgtggcggc 2040
   tggcgttcct tgtcgggtta ttcgcgaaat aaacgaccgg gataagcact attatttcaa 2100
   agattataaa gttgaatcgt cagtttaaat tataaaaatt gcctgatacg ctgcgcttat 2160
   caggcctaca agttcagcga tctacattag ccgcatccgg catgaacaaa gcgcaggaac 2220
   aagcgtcgca tcatgcctct ttgacccaca gctgcggaaa acgtactggt gcaaaacgca 2280
   gggttatgat catcagccca acgacgcaca gcgcatgaaa tgcccagtcc atcaggtaat 2340
   tgccgctgat actacgcagc acgccagaaa accacggggc aagcccggcg atgataaaac 2400
   cgattccctg cataaacgcc accagcttgc cagcaatagc cggttgcaca gagtgatcga 2460
   gcgccagcag caaacagagc ggaaacgcgc cgcccagacc taacccacac accatcgccc 2520
   acaataccgg caattgcatc ggcagccaga taaagccgca gaaccccacc agttgtaaca 2580
   ccagcgccag cattaacagt ttgcgccgat cctgatggcg agccatagca ggcatcagca 2640
   aagctcctgc ggcttgccca agcgtcatca atgccagtaa ggaaccgctg tactgcgcgc 2700
   tggcaccaat ctcaatatag aaagcgggta accaggcaat caggctggcg taaccgccgt 2760
   taatcagacc gaagtaaaca cccagcgtcc acgcgcgggg agtgaatacc acgcgaaccg 2820
   gagtggttgt tgtcttgtgg gaagaggcga cctcgcgggc gctttgccac caccaggcaa 2880
   agagcgcaac aacggcaggc agcgcccacc aggcgagtgt ttgataccag gtttcgctat 2940
   gttgaactaa ccagggcgtt atggcggcac caagcccacc gccgcccatc agagccgcgg 3000
   accacagccc catcaccagt ggcgtgcgct gctgaaaccg ccgtttaatc accgaaggca 3060
   tcaccgcctg aatgatgccg atccccaccc caccaagcag tgcgctgcta agcagcagcg 3120
   cactttgcgg gtaaagctca cgcatcaatg caccgacggc aatcagcaac agactgatgg 3180
   cgacactgcg acgttcgctg acatgctgat gaagccagct tccggccagc gccagcccgc 3240
   ccatggtaac caccggcaga gcggtcaaca gggcagccac gctaaagctc attccgctcg 3300
   cctggcgcaa ttgcggtagc agtggcccga cggaggtgag cagtggtcgc atattaagac 3360
   caatcagcac cagtaccagc agcatcgtga acgtcttttg atggttgcgc atggctgtca 3420
   gaacggtttg gtcggcagat atttaccatc taaggtgatg accgcacgtt cgccaccttc 3480
   cgggtccgcc actttcttaa cgtcgagttt gaagttaatc gcgctaataa tgccatcgcc 3540
   aaatttctca tgaaccaacg ctttcagggt tgtaccgtac acctgcaaca tttcatagaa 3600
   acgatacatc gttgggtcag ttggaatacg gtcatcaatg cagccacgca gtggaatcat 3660
   ctgcaacagt agaatggagt cttcgtcgag atccagcttc gccccgacca ggcgggcggc 3720
   gtcggcagga agcgcctgct gacccagcaa agccgcggtt acaaaggctt ctgccagacc 3780
   ggtgccgtcg gcaatctcgg caaatgagag atctttttta gctttgctga gcaaaatggc 3840
   atcggcaaga tcaagacgaa tattgcggtt aatttgtgac tgaatcatgg tggaactcct 3900
   gatggtttaa aaataaggtt acgctgcggt cggttggcgt agcggtatgg cacaaacgcg 3960
   aggattagcg gccagtggca caaactggcg ggttgcgccg tcaaaagctg cgatgctgcc 4020
   gctttcaatg tcgtagaccc agccgtgcag ggcgatccgc ccctcttcga gcgccaggcg 4080
   caccgatgga tgagtttgca aattagccaa ctgagcaatg acgttttcac gtaccatcgc 4140
   cgcagctttt gacggtaaat cggaatgcgg gcgcgcctca ttaacgacgc gggctgaatc 4200
   ggcataacgc agccagtggg agacggcagg catatggtcc atgcactgac agctggcaat 4260
   ggcggtcatc gcgccacagt tggaatgacc acaaatcaca atgtcagata cccgaagcgc 4320
   agcgacggca tactccaccg aagcagaaac gccaccgggt tccggcccgt aggaagggac 4380
   gatattgccc gcgttgcgaa taacgaacag atcgccaggc tcacgttgcg tcaccagctc 4440
   agggaccaga cggctgtcgg agcaggagat aaaaagtgtg cgcgggcttt gctgtgtcgc 4500
   cagctgttta aacaaggctt cccgcttcgg aaatgcctcg cgctggaatt taaggaatcc 4560
   atcaataatc tctttcaccg ttaacctctg tctgtctctg gaatgagagg cagactactc 4620
   gccgattgtc ataaggtaaa agtctcattt atgatgagtt ccattggatt tacttatagg 4680
   ttgcgaatgc tctctcgaca tatcaattat tttcttgccg tggctgaaca tggcagcttc 4740
   acccgtgccg ccagtgcgtt gcacgtctcc caacctgcgc tttcccagca gattcgccag 4800
   ttagaggaga gtttaggcgt gccgctgttt gaccgtagcg ggcgaacgat tcgtctcact 4860
   gatgcaggag aagtctggta tggtgcactc tcagtacaat ctgctctgat gccgcatagt 4920
   taagccagta tacactccgc tatcgctacg tgactgggtc atggctgcgc cccgacaccc 4980
   gccaacaccc gctgacgcgc cctgacgggc ttgtctgctc ccggcatccg cttacagaca 5040
   agctgtgacc gtctccggga gctgcatgtg tcagaggttt tcaccgtcat caccgaaacg 5100
   cgcgaggcag cagatcaatt cgcgcgcgaa ggcgaagcgg catgcattta cgttgacacc 5160
   atcgaatggt gcaaaacctt tcgcggtatg gcatgatagc gcccggaaga gagtcaattc 5220
   agggtggtga atgtgaaacc agtaacgtta tacgatgtcg cagagtatgc cggtgtctct 5280
   tatcagaccg tttcccgcgt ggtgaaccag gccagccacg tttctgcgaa aacgcgggaa 5340
   aaagtggaag cggcgatggc ggagctgaat tacattccca accgcgtggc acaacaactg 5400
   gcgggcaaac agtcgttgct gattggcgtt gccacctcca gtctggccct gcacgcgccg 5460
   tcgcaaattg tcgcggcgat taaatctcgc gccgatcaac tgggtgccag cgtggtggtg 5520
   tcgatggtag aacgaagcgg cgtcgaagcc tgtaaagcgg cggtgcacaa tcttctcgcg 5580
   caacgcgtca gtgggctgat cattaactat ccgctggatg accaggatgc cattgctgtg 5640
   gaagctgcct gcactaatgt tccggcgtta tttcttgatg tctctgacca gacacccatc 5700
   aacagtatta ttttctccca tgaagacggt acgcgactgg gcgtggagca tctggtcgca 5760
   ttgggtcacc agcaaatcgc gctgttagcg ggcccattaa gttctgtctc ggcgcgtctg 5820
   cgtctggctg gctggcataa atatctcact cgcaatcaaa ttcagccgat agcggaacgg 5880
   gaaggcgact ggagtgccat gtccggtttt caacaaacca tgcaaatgct gaatgagggc 5940
   atcgttccca ctgcgatgct ggttgccaac gatcagatgg cgctgggcgc aatgcgcgcc 6000
   attaccgagt ccgggctgcg cgttggtgcg gatatctcgg tagtgggata cgacgatacc 6060
   gaagacagct catgttatat cccgccgtca accaccatca aacaggattt tcgcctgctg 6120
   gggcaaacca gcgtggaccg cttgctgcaa ctctctcagg gccaggcggt gaagggcaat 6180
   cagctgttgc ccgtctcact ggtgaaaaga aaaaccaccc tggcgcccaa tacgcaaacc 6240
   gcctctcccc gcgcgttggc cgattcatta atgcagctgg cacgacaggt ttcccgactg 6300
   gaaagcgggc agtgagcgca acgcaattaa tgtgagttag cgcgaattga tctggtttga 6360
   cagcttatca tcgactgcac ggtgcaccaa tgcttctggc gtcaggcagc catcggaagc 6420
   tgtggtatgg ctgtgcaggt cgtaaatcac tgcataattc gtgtcgctca aggcgcactc 6480
   ccgttctgga taatgttttt tgcgccgaca tcataacggt tctggcaaat attctgaaat 6540
   gagctgttga caattaatca tccggctcgt ataatgtgtg gaattgtgag cggataacaa 6600
   tttcacacag gaaacagcgc cgctgagaaa aagcgaagcg gcactgctct ttaacaattt 6660
   atcagacaat ctgtgtgggc actcgaccgg aattatcgat taactttatt attaaaaatt 6720
   aaagaggtat atattaatgt atcgattaaa taaggaggaa taaaccatgg ccggtacggg 6780
   tcgtttggct ggtaaaattg cattgatcac cggtggtgct ggtaacattg gttccgagct 6840
   gacccgccgt tttctggccg agggtgcgac ggttattatc agcggccgta accgtgcgaa 6900
   gctgaccgcg ctggccgagc gcatgcaagc cgaggccggc gtgccggcca agcgcattga 6960
   tttggaggtg atggatggtt ccgaccctgt ggctgtccgt gccggtatcg aggcaatcgt 7020
   cgctcgccac ggtcagattg acattctggt taacaacgcg ggctccgccg gtgcccaacg 7080
   tcgcttggcg gaaattccgc tgacggaggc agaattgggt ccgggtgcgg aggagacttt 7140
   gcacgcttcg atcgcgaatc tgttgggcat gggttggcac ctgatgcgta ttgcggctcc 7200
   gcacatgcca gttggctccg cagttatcaa cgtttcgact attttctcgc gcgcagagta 7260
   ctatggtcgc attccgtacg ttaccccgaa ggcagcgctg aacgctttgt cccagctggc 7320
   tgcccgcgag ctgggcgctc gtggcatccg cgttaacact attttcccag gtcctattga 7380
   gtccgaccgc atccgtaccg tgtttcaacg tatggatcaa ctgaagggtc gcccggaggg 7440
   cgacaccgcc catcactttt tgaacaccat gcgcctgtgc cgcgcaaacg accaaggcgc 7500
   tttggaacgc cgctttccgt ccgttggcga tgttgctgat gcggctgtgt ttctggcttc 7560
   tgctgagagc gcggcactgt cgggtgagac gattgaggtc acccacggta tggaactgcc 7620
   ggcgtgtagc gaaacctcct tgttggcgcg taccgatctg cgtaccatcg acgcgagcgg 7680
   tcgcactacc ctgatttgcg ctggcgatca aattgaagaa gttatggccc tgacgggcat 7740
   gctgcgtacg tgcggtagcg aagtgattat cggcttccgt tctgcggctg ccctggcgca 7800
   atttgagcag gcagtgaatg aatctcgccg tctggcaggt gcggatttca ccccgccgat 7860
   cgctttgccg ttggacccac gtgacccggc caccattgat gcggttttcg attggggcgc 7920
   aggcgagaat acgggtggca tccatgcggc ggtcattctg ccggcaacct cccacgaacc 7980
   ggctccgtgc gtgattgaag tcgatgacga acgcgtcctg aatttcctgg ccgatgaaat 8040
   taccggcacc atcgttattg cgagccgttt ggcgcgctat tggcaatccc aacgcctgac 8100
   cccgggtgcc cgtgcccgcg gtccgcgtgt tatctttctg agcaacggtg ccgatcaaaa 8160
   tggtaatgtt tacggtcgta ttcaatctgc ggcgatcggt caattgattc gcgtttggcg 8220
   tcacgaggcg gagttggact atcaacgtgc atccgccgca ggcgatcacg ttctgccgcc 8280
   ggtttgggcg aaccagattg tccgtttcgc taaccgctcc ctggaaggtc tggagttcgc 8340
   gtgcgcgtgg accgcacagc tgctgcacag ccaacgtcat attaacgaaa ttacgctgaa 8400
   cattccagcc aatattagcg cgaccacggg cgcacgttcc gccagcgtcg gctgggccga 8460
   gtccttgatt ggtctgcacc tgggcaaggt ggctctgatt accggtggtt cggcgggcat 8520
   cggtggtcaa atcggtcgtc tgctggcctt gtctggcgcg cgtgtgatgc tggccgctcg 8580
   cgatcgccat aaattggaac agatgcaagc catgattcaa agcgaattgg cggaggttgg 8640
   ttataccgat gtggaggacc gtgtgcacat cgctccgggt tgcgatgtga gcagcgaggc 8700
   gcagctggca gatctggtgg aacgtacgct gtccgcattc ggtaccgtgg attatttgat 8760
   taataacgcc ggtattgcgg gcgtggagga gatggtgatc gacatgccgg tggaaggctg 8820
   gcgtcacacc ctgtttgcca acctgatttc gaattattcg ctgatgcgca agttggcgcc 8880
   gctgatgaag aagcaaggta gcggttacat cctgaacgtt tcttcctatt ttggcggtga 8940
   gaaggacgcg gcgattcctt atccgaaccg cgccgactac gccgtctcca aggctggcca 9000
   acgcgcgatg gcggaagtgt tcgctcgttt cctgggtcca gagattcaga tcaatgctat 9060
   tgccccaggt ccggttgaag gcgaccgcct gcgtggtacc ggtgagcgtc cgggcctgtt 9120
   tgctcgtcgc gcccgtctga tcttggagaa taaacgcctg aacgaattgc acgcggcttt 9180
   gattgctgcg gcccgcaccg atgagcgctc gatgcacgag ttggttgaat tgttgctgcc 9240
   gaacgacgtg gccgcgttgg agcagaaccc agcggcccct accgcgctgc gtgagctggc 9300
   acgccgcttc cgtagcgaag gtgatccggc ggcaagctcc tcgtccgcct tgctgaatcg 9360
   ctccatcgct gccaagctgt tggctcgctt gcataacggt ggctatgtgc tgccggcgga 9420
   tatttttgca aatctgccta atccgccgga cccgttcttt acccgtgcgc aaattgaccg 9480
   cgaagctcgc aaggtgcgtg atggtattat gggtatgctg tatctgcagc gtatgccaac 9540
   cgagtttgac gtcgctatgg caaccgtgta ctatctggcc gatcgtaacg tgagcggcga 9600
   aactttccat ccgtctggtg gtttgcgcta cgagcgtacc ccgaccggtg gcgagctgtt 9660
   cggcctgcca tcgccggaac gtctggcgga gctggttggt agcacggtgt acctgatcgg 9720
   tgaacacctg accgagcacc tgaacctgct ggctcgtgcc tatttggagc gctacggtgc 9780
   ccgtcaagtg gtgatgattg ttgagacgga aaccggtgcg gaaaccatgc gtcgtctgtt 9840
   gcatgatcac gtcgaggcag gtcgcctgat gactattgtg gcaggtgatc agattgaggc 9900
   agcgattgac caagcgatca cgcgctatgg ccgtccgggt ccggtggtgt gcactccatt 9960
   ccgtccactg ccaaccgttc cgctggtcgg tcgtaaagac tccgattgga gcaccgtttt 10020
   gagcgaggcg gaatttgcgg aactgtgtga gcatcagctg acccaccatt tccgtgttgc 10080
   tcgtaagatc gccttgtcgg atggcgcgtc gctggcgttg gttaccccgg aaacgactgc 10140
   gactagcacc acggagcaat ttgctctggc gaacttcatc aagaccaccc tgcacgcgtt 10200
   caccgcgacc atcggtgttg agtcggagcg caccgcgcaa cgtattctga ttaaccaggt 10260
   tgatctgacg cgccgcgccc gtgcggaaga gccgcgtgac ccgcacgagc gtcagcagga 10320
   attggaacgc ttcattgaag ccgttctgct ggttaccgct ccgctgcctc ctgaggcaga 10380
   cacgcgctac gcaggccgta ttcaccgcgg tcgtgcgatt accgtctaat agaagcttgg 10440
   ctgttttggc ggatgagaga agattttcag cctgatacag attaaatcag aacgcagaag 10500
   cggtctgata aaacagaatt tgcctggcgg cagtagcgcg gtggtcccac ctgaccccat 10560
   gccgaactca gaagtgaaac gccgtagcgc cgatggtagt gtggggtctc cccatgcgag 10620
   agtagggaac tgccaggcat caaataaaac gaaaggctca gtcgaaagac tgggcctttc 10680
   gttttatctg ttgtttgtcg gtgaacgctc tcctgagtag gacaaatccg ccgggagcgg 10740
   atttgaacgt tgcgaagcaa cggcccggag ggtggcgggc aggacgcccg ccataaactg 10800
   ccaggcatca aattaagcag aaggccatcc tgacggatgg cctttttgcg tttctacaaa 10860
   ctctttttgt ttatttttct aaatacattc aaatatgtat ccgctcatga gacaataacc 10920
   ctgataaatg cttcaataat attgaaaaag gaagagtatg agtattcaac atttccgtgt 10980
   cgcccttatt cccttttttg cggcattttg ccttcctgtt tttgctcacc cagaaacgct 11040
   ggtgaaagta aaagatgctg aagatcagtt gggtgcacga gtgggttaca tcgaactgga 11100
   tctcaacagc ggtaagatcc ttgagagttt tcgccccgaa gaacgttttc caatgatgag 11160
   cacttttaaa gttctgctat gtggcgcggt attatcccgt gttgacgccg ggcaagagca 11220
   actcggtcgc cgcatacact attctcagaa tgacttggtt gagtactcac cagtcacaga 11280
   aaagcatctt acggatggca tgacagtaag agaattatgc agtgctgcca taaccatgag 11340
   tgataacact gcggccaact tacttctgac aacgatcgga ggaccgaagg agctaaccgc 11400
   ttttttgcac aacatggggg atcatgtaac tcgccttgat cgttgggaac cggagctgaa 11460
   tgaagccata ccaaacgacg agcgtgacac cacgatgcct gtagcaatgg caacaacgtt 11520
   gcgcaaacta ttaactggcg aactacttac tctagcttcc cggcaacaat taatagactg 11580
   gatggaggcg gataaagttg caggaccact tctgcgctcg gcccttccgg ctggctggtt 11640
   tattgctgat aaatctggag ccggtgagcg tgggtctcgc ggtatcattg cagcactggg 11700
   gccagatggt aagccctccc gtatcgtagt tatctacacg acgg 11744
<210> 133
   <211> 6915
   <212> DNA
   <213> Artificial Sequence
<220>
   <223> Description of Artificial Sequence: Synthetic polynucleotide
<400> 133
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatgggtt 2040
   acagagcttt caggattgca tccacgctgg ctttggcgtc accaaacagc atgtgggtgt 2100
   tttccttgaa gaacagcggg ttttgcacac cagcatagcc agtgttcatc gaacgtttaa 2160
   agacaatcac gttctgcgct ttccacactt ccagcacagg cataccagca atcggactct 2220
   tcggatcatc ctgcgccgcc gggttaaccg tatcgttagc accaatcacc agtacggtat 2280
   cggtatcagc aaagtcatca ttgatctcgt ccatttccag cacgatgtca tacggtactt 2340
   ttgcttcagc cagcaatacg ttcatatgtc caggcaaacg ccccgcgacc gggtggatac 2400
   cgaaacgcac attaatacca cgagcgcgca atttctcagt aatttcagcg acaggatatt 2460
   gcgcctgcgc gactgccatg ccgtaccccg gagtaatgat cactgaatgg gagtttttca 2520
   gcagttccgc tgtctcttct gcggtgattt cgcggtgctc acccacttcc tgatcatcgc 2580
   cagtagaaga gccgtcggtg ccgaaaccac ccgcaataac gctgataaag gaacggttca 2640
   tcgcctcaca cataatgtaa gaaaggatag cccccgaaga accgaccagc gcaccggtca 2700
   caatcagcag gtcgttgctg agcataaagc ccgcagccgc agccgcccag ccggagtacg 2760
   agttcagcat cgacaccacc actggcatat ctgcaccacc gatggaggcg actaaatgcc 2820
   agccgaatac cagcgcaatt gcggtcatta tcagcaatgc cagcacttgc aggccgacgc 2880
   tgtccgtgcg aacaaataca atcagcagca ggaaggaaac gaccagagcc gccaggttca 2940
   ttttgtgacg gtttggcagc atcaatggtt tagacgaaat cttgccacac agtttgccga 3000
   acgccaccac cgaacccgtg aacgttaccg ccccgatgaa gataccgagg aacacttccg 3060
   tcaggtgaat attgaccaga atcggtgcca ttcccgcgtc atgatgcaga tagctgttaa 3120
   agccaaccag cactgccgcc agacccacga agctatgcag gatcgccacc agttctggca 3180
   tttcggtcat ttcaactttc ttcgccagac ggataccaat tgccccacca atgaccatcg 3240
   ccagcaagat ccagccaaca ttacccgtat ccggtccaaa aatggttgcg attaacgcaa 3300
   tcgccatccc ggcgataccg aagttgttac cctggcgaga cgtttcatgt ttcgaaagac 3360
   cggccagact gaagataaac aggatcgcgg caacaatgta tgcagctgta actaatcctc 3420
   cagacatatg ttacccctta atttttgcgg aacattttca gcatgcgctg agtcacggtg 3480
   aagccaccga aaatattaat gctggctata agcaccgcga taaaactaag gaagctaacc 3540
   cagccgccct ggccaatctg caacagtgct ccgacaacaa taatccctga aatcgcgttg 3600
   gtgaccgaca tcaacggtgt atgcagcgcg tgcgatacat tccacaccac gtaataaccg 3660
   acaacgcagg ccagcgcgaa aacggtgaag tgcccaagga attctttcgg cgcaacgctt 3720
   gccatccagc caaaaagaat gattgccagc gccatcaacg cgtatttacg ccacggtgag 3780
   caggtacatt tttcctcagt tttcacttcc ggtgccgctt tttgtgccgc ctgcggctga 3840
   gctgatacct gaatcggcgg tgccggccag gtaatttcgc ccgcacggat cacggtcacg 3900
   ccgcgaatca ccacatcatc aaaatcaaca gtgatattgc cgtctttctc tttgcacaac 3960
   agtttcagca gattaacgag gtttgtgccg taaagctgtg aggattgcgt cggcagacgg 4020
   cccggaagat cggtataacc aatcactttg acaccatttt ccgtagtgaa gatttcaccc 4080
   ggcacggtgt attcacagtt gccgccgttt tgggctgcca ggtcgacaat cacactgccc 4140
   gccttcatgg agtcaaccat ttcacgggta attagcttcg gcgctggttt gcctggaata 4200
   agcgcggtgg tgacaatgat atcgacctct tttgcctggg cggcaaagag ttccatttcc 4260
   gctttgatga acgcgtccga catcactttg gcatagccat cgccgctgcc agcttcctct 4320
   ttaaaatcca gctcgaggaa ttccgcgccc atactttgaa cttgttcttt cacttccggg 4380
   cgggtgtcga atgcacgcac aatcgcgccg agactgtttg ctgcgccaat ggcggccaga 4440
   cctgcaacac ccgcaccaat caccatcact tttgccggtg gcactttccc ggccgcagta 4500
   atttgcccgg taaagaagcg cccaaattca tgtgccgctt caacaatggc gcgataaccg 4560
   gcgatgttcg ccatcgagct tagtgcgtcc agcgattgtg cgcgtgagat acgcggcaca 4620
   gagtccatcg ccatcacggt cacgttacgt tccgcaagtt tttgcattaa ttccggattc 4680
   tgcgcaggcc agataaaact caccagcgtt gtcccaggat tcagtaacgc aatttcatca 4740
   tctaacggcg cattgacctt cagaatgatc tctgactgcc agacgctatt cccttctaca 4800
   atttcagcgc ccgcttgcac aaacgcttta tcgtcaaaac ttgccagttg acccgcgccg 4860
   ctctctaccg cgacggtaaa acccagtttc agcagctgtt ccactgtttt tggcgttgct 4920
   gcaacacggg tttcattggt taaccgttct cttggtatgc caattgccat ggtaaatact 4980
   ccttgaaaag taaagtgtta gatgagtgcg ttaattcaca cttctgagaa atttcgctaa 5040
   acgcatcaaa aaagcatagc agacaggcat ggtattgctg gattaagcag gtaacatcag 5100
   tgttatagga ttattaccaa aacattatat tcacaggtgt atcgctcaat actgaccatt 5160
   taaatcatac ctgacctcca tagcagaaag tcaaaagcct ccgaccggag gcttttgact 5220
   tgagggggat cagcttctat tatttctctt gcaggcccag tttcttttcc aggtagtgaa 5280
   tgttcgtacc gccgtgctgg aaattctcgt cgttcataat gcggatctgc aaatccacat 5340
   tggtcttaat gccatcaatg atcagctctt gcagcgcgtt tttcatgcgg gcgatcgcat 5400
   gtttttcctc ctgccccgcg cttataagcg aataaaggaa gatggccgcc ccgcagggca 5460
   gcaggtctgt gaaacagtat agagattcat cggcacaaag gctttgcttt ttgtcattta 5520
   ttcaaaccgg cgcctcgaac cggctccggg cattgccctg gccggcactt tgcatgggga 5580
   gatgcggatc tcgggcagcg ttgggtcctg gccacgggtg cgcatgatcg tgctcctgtc 5640
   gttgaggacc cggctaggct ggcggggttg ccttactggt tagcagaatg aatcaccgat 5700
   acgcgagcga acgtgaagcg actgctgctg caaaacgtct gcgacctgag caacaacatg 5760
   aatggtcttc ggtttccgtg tttcgtaaag tctggaaacg cggaagtccc ctacgtgctg 5820
   ctgaagttgc ccgcaacaga gagtggaacc aaccggtgat accacgatac tatgactgag 5880
   agtcaacgcc atgagcggcc tcatttctta ttctgagtta caacagtccg caccgctgtc 5940
   cggtagctcc ttccggtggg cgcggggcat gactatcgtc gccgcactta tgactgtctt 6000
   ctttatcatg caactcgtag gacaggtgcc ggcagcgccc aacagtcccc cggccacggg 6060
   gcctgccacc atacccacgc cgaaacaagc gccctgcacc attatgttcc ggatctgcat 6120
   cgcaggatgc tgctggctac cctgtggaac acctacatct gtattaacga agcgctaacc 6180
   gtttttatca ggctctggga ggcagaataa atgatcatat cgtcaattat tacctccacg 6240
   gggagagcct gagcaaactg gcctcaggca tttgagaagc acacggtcac actgcttccg 6300
   gtagtcaata aaccggtaaa ccagcaatag acataagcgg ctatttaacg accctgccct 6360
   gaaccgacga ccgggtcgaa tttgctttcg aatttctgcc attcatccgc ttattatcac 6420
   ttattcaggc gtagcaccag gcgtttaagg gcaccaataa ctgccttaaa aaaattacgc 6480
   cccgccctgc cactcatcgc agtactgttg taattcatta agcattctgc cgacatggaa 6540
   gccatcacag acggcatgat gaacctgaat cgccagcggc atcagcacct tgtcgccttg 6600
   cgtataatat ttgcccatgg tgaaaacggg ggcgaagaag ttgtccatat tggccacgtt 6660
   taaatcaaaa ctggtgaaac tcacccaggg attggctgag acgaaaaaca tattctcaat 6720
   aaacccttta gggaaatagg ccaggttttc accgtaacac gccacatctt gcgaatatat 6780
   gtgtagaaac tgccggaaat cgtcgtggta ttcactccag agcgatgaaa acgtttcagt 6840
   ttgctcatgg aaaacggtgt aacaagggtg aacactatcc catatcacca gctcaccgtc 6900
   tttcattgcc atacg 6915
<210> 134
   <211> 8995
   <212> DNA
   <213> Artificial Sequence
<220>
   <223> Description of Artificial Sequence: Synthetic polynucleotide
<400> 134
   gaattccgga tgagcattca tcaggcgggc aagaatgtga ataaaggccg gataaaactt     60
   gtgcttattt ttctttacgg tctttaaaaa ggccgtaata tccagctgaa cggtctggtt    120
   ataggtacat tgagcaactg actgaaatgc ctcaaaatgt tctttacgat gccattggga    180
   tatatcaacg gtggtatatc cagtgatttt tttctccatt ttagcttcct tagctcctga    240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatttaaa 2040
   acaggcggtt taaaccgttt aacgcagcta cccgataggc ttccgccatc gtcgggtagt 2100
   taaaggtggt gttgacgaag tactcaatag tgttgccgcc acctttctgt tccataatcg 2160
   cctgaccgat atgaataatt tcggcagcgc gctcgccaaa gcagtgaata cccagaatct 2220
   cttttgtttc ccgatggaac aaaattttca gcgtgcccac gttcatgccg acgatttgtg 2280
   cgcgtgccag atgtttaaac tgggcgcggc ccacttcata tggcactttc attgcggtca 2340
   gctgctgttc ggttttgccc acagagctga tttccgggat ggtgtaaata ccggtaggga 2400
   tatcttcaat cagatgtgcg gtggcttcgc cttttaccag cgcctgcgcg gcaatgcgcc 2460
   cctggtcata ggccgccgac gccaggctcg gataaccaat cacgtcgccc accgcgtaaa 2520
   cgtgtggctg tgcggtctga tacatgctgt tgaccttcag ctgtccgcgg ctgtcagttt 2580
   ctagcccaat gttctgtaac gccagcgaat cggtattacc ggtgcgaccg ttggcataga 2640
   gcaggcagtc agctttcagt tttttacccg acttcagatg catgatcaca ccatcgtcac 2700
   agccttcgat cttctcgtac tcttcgttgt gacgaatcac tacgccactg ttccagaagt 2760
   gataggagag agaatctgac atctcttgat cgagaaatgc cagcaggcga tcgcgggtgt 2820
   tgatcagatc cacttttaca tccataccgc ggaagatcga cgcatattca cagccgatca 2880
   ctccagcacc atagataagt acatggcgcg gttcgtggtg catgctgaga attgagtcgc 2940
   tgtcgtaaat gcgtggatgg gtgaaatcaa catctgttgg atgatatgga cgagagccgc 3000
   aggcaataac aaatttttca gcggttagtg tttcaacgct gccgtccggg caatccagcg 3060
   ccaacgtatg ctcgtcaaca aagcgagcgt ttccctgcaa tatttcacag tgattacgtt 3120
   cgtaaaatcc ctgacgcatg cgcgtttgtt gattaatcac gttatcggca tggttaagga 3180
   tatcggcaaa agaagagcgg agcagtcggg aatggtcgct gtaaagtggg ttttgattga 3240
   attctataat gcggctgacg gcgtgacgga gagctttcga cgggatggtg ccccagtggg 3300
   tgcaaccgcc gccaacattt tgataacgct cgataactgc gacgcgcgca ccttgcttaa 3360
   ccaggcccat tgcagcgcct tcgccgccgg ggccggaacc tattactatg gcatcgtaat 3420
   cgtaggaatg tggggccatg gtagggctta cctgttcttg aatagttctt tcaaactatg 3480
   agaatattat atcaagaatt gtaggattaa gcaactgaaa tttttaagta gttaagatct 3540
   ataacttcgt ataatgtatg ctatacgaag ttatgtttaa atcatacctg acctccatag 3600
   cagaaagtca aaagcctccg accggaggct tttgacttga gggggatcag cttctattat 3660
   ttctcttgca ggcccagttt cttttccagg tagtgaatgt tcgtaccgcc gtgctggaaa 3720
   ttctcgtcgt tcataatgcg gatctgcaaa tccacattgg tcttaatgcc atcaatgatc 3780
   agctcttgca gcgcgttttt catgcgggcg atcgcgacat cacgattttc gccatagcag 3840
   atcagtttac caatcatgct gtcatagtac ggcggcacgg tgtaaccggc gtagatgtga 3900
   gactcccaac gaacgccaaa accacctggc gcgtgaaaac gcgtgatctt accagggctc 3960
   ggcagaaagg tattcgggtc ctccgcattg atacggcact cgaccgcgtg accacgaacg 4020
   tgcacctctt cttgcttgat cgacagcggc tgaccagccg caatgcgcag ttgctctttg 4080
   atcagatcaa cgccagtaat catctcggtc accgggtgct caacctggat gcgggtattc 4140
   atttcaatga agtaaaactc accattttcg aacaggaact caaacgtacc cgcaccacgg 4200
   taaccaatgt ccacacacgc tttcgcacag cgctcaccga tgtagcgacg cagttccggc 4260
   gtaatacccg gagccggtgc ctcttccacc accttctgat gacggcgttg catggagcaa 4320
   tcacgttctg ccaagtagat cgcattgccc tgaccatccg ccagaacttg gatttcaacg 4380
   tggcgcggat tctccagata cttctccata tacaccatat cgttagagaa cgccgctttg 4440
   gcttccgcac gggtcatgct gatgctttgc gccagttccg catcaccacg gacgacacgc 4500
   ataccacggc caccgccacc accagaggct ttgataatga ccgggtagcc aatacgttta 4560
   gcgatggcac ggttcttgtc catatcgtca cccagcggac catcgctgcc cggaacacac 4620
   ggcacaccgg cctttttcat cgctgcaatg gcgctcactt tgtcgcccat caggcgaatc 4680
   gtctccgcct tagggccaat gaagatgaaa ccgctacgct ccacttgttc agcaaagtta 4740
   gcgttctcgc tcaggaaacc gtagcccgga tgaatcgcca ctgcaccggt gatctctgcc 4800
   gcagagatga tggccgggat gttcagatag cttttcacgc tcggtgccgg accgatgcaa 4860
   accgtttcgt cggccagcag aacatgcttc aggtcgcggt ctgcgctgga atgcacggca 4920
   acagtcttaa tgcccagctc tttacacgcg cgcaagatac gcagcgcgat ttcaccgcgg 4980
   tttgcaatca cgatcttgtc caacatgttc gcctcgttac tcgatcacaa ccagcggttc 5040
   atcgaactca acaggctggc cggattccac caggatcgct ttaacagtac cgctcttatc 5100
   cgcttcaatc tggttcatca ttttcattgc ctcaacgata cacagcgtgt cgccgacatt 5160
   gaccttctgg ccgacttcga tgaaggcttt tgcgtccggc gacggcgtgc gatagaaggt 5220
   gcccaccatc gggctacgca cgatatgacc cgaaatctcg gccgctgccg gtgcctccat 5280
   gcttggaacg gtcgccggtg cagcggcgtt gctctgtgcc ggctgttgca tcatcggagc 5340
   ggcgtatgct tgctgcataa ccggaaagct cgctgccggt gccgcacggg aaatacggac 5400
   gctctcttca ccttcgctga tctccagctc gctgatgccg ctttcttcaa ccagttcaat 5460
   cagtttcttg atcttacgaa tgtccatggt ttttcctcct tcgcctgtgg tatgaaattt 5520
   cacacgcatt atatacaaaa aaagcgattc agaccccgtt ggcaagccgc gtggttaact 5580
   ccacgtgcta ttatgcctcc ggctcttggt ccgggaccgg cggaaccacg acaccctcac 5640
   gcggtgcttc aggattcgga gccggcagat tcatcaattt cgccagaatg ctcgccagtt 5700
   tcagacgcat ttctggacgg cgaacgatca tgtcgatggc acctttctca atcaggaact 5760
   cgctgcgctg gaaaccaggc ggcaactttt cgcgaaccgt ctgttcaata acacgcggac 5820
   cagcaaaacc aatcagagcc ttcggctctg cgatgttcaa atcacccagc atcgcaaagc 5880
   tggcgctgac accgcccatc gtagggtcgg tcaggacgga aatgtacggc aggccgcgtt 5940
   cttgcatctt cgccagggct gccgaggttt ttgccatctg catcaggctc atcagggcct 6000
   cttgcatacg cgcaccaccg ctcgcgctga aacaaatcag aggacagtta tcctccagcg 6060
   cctgctccac ggcacgcaca aagcgtgcgc ccaccacgga acccatgctg ccacccataa 6120
   acgcaaactc gaacgccgca gcaacaaccg gcataccata cagggtgcct ttcataacaa 6180
   ccagtgcgtc cttctcaccg gtttcttttt gtgcagatgc caaacgatcc ttatacttct 6240
   tggaatcacg gaatttcaga acatctttcg gttccagttc gctacccagc tcgaccaggc 6300
   tgccctcatc cagcaggcta tgcagacgat tacgagcggt catacgcatg tgatggtcgc 6360
   atttcgggca cacttccagg ttacgctcca gctccgcacg atacaaaacc tgaccgcagc 6420
   tatcgcattt ggtccagacg ccttcaggga tgctcgcctt gcgggtcggg gtgatattgg 6480
   acttaatgcg ttcaatccag ctcatgattt tcctccttta ctacgcgtag ccatagctca 6540
   tcaagcgctg atagcgacgg tttttcagat cctccgtgct cagcacgtcc agatccgcca 6600
   ggtccgccag cagctgggcc ttcaggctgg ctgccatcgc ctccggatta cggtgagcac 6660
   cgcccagcgg ttccgggatg atagaatcaa tcaacttcag ttctttcaga cgcggtgcga 6720
   tgatacccat cgcttccgct gccaatggtg ctttatccgc gctcttccac agaatggaag 6780
   cgcaaccttc cgggctaatg acgctgtacg tgctgtattg cagcatgttg accttatcac 6840
   cgacaccgat cgccaacgca ccaccgctac cgccctcgcc aatcacggtg caaacgaccg 6900
   gcacacccag acgagacatc tcacgcaggt tacgtgcgat ggcctcgctc tgaccacgct 6960
   cttccgcacc aacgcctggg tacgcacccg gcgtatcaat gaacgtgata atcggcatct 7020
   taaagcgttc ggccatttgc atcagacgca gggccttgcg gtagccctcc ggtgccggca 7080
   taccaaagtt acgacgaatc ttttctttcg tctcgcggcc tttttgatgg ccgataatca 7140
   tcaccggacg gccatccaaa cgagcaatac cgccgacaat tgctttatcg tccgcatagg 7200
   cgcggtcgcc tgccagttcg tcgaactcgt caaacgccag gcgcacgtag tccagggtat 7260
   atgggcgttg tgggtggcga gccaactggg caatctgcca tgcgcccaaa tcagcaaaga 7320
   ttttgcgagt cagttcaaca gacttctcac gcaaacggtg gacctcttcg tcgatgttaa 7380
   tgtccagttt ctcatcttga cggctaaccg cggtcaggct gtcaatcttc gcttccagtt 7440
   ctgcaatcgg ctgctcgaag tccaggaagt tcagggacat gtttttcctc ctgccccgcg 7500
   cttataagcg aataaaggaa gatggccgcc ccgcagggca gcaggtctgt gaaacagtat 7560
   agagattcat cggcacaaag gctttgcttt ttgtcattta ttcaaaccgg cgcctcgaac 7620
   cggctccggg cattgccctg gccggcactt tgcatgggga gatgcggatc tcgggcagcg 7680
   ttgggtcctg gccacgggtg cgcatgatcg tgctcctgtc gttgaggacc cggctaggct 7740
   ggcggggttg ccttactggt tagcagaatg aatcaccgat acgcgagcga acgtgaagcg 7800
   actgctgctg caaaacgtct gcgacctgag caacaacatg aatggtcttc ggtttccgtg 7860
   tttcgtaaag tctggaaacg cggaagtccc ctacgtgctg ctgaagttgc ccgcaacaga 7920
   gagtggaacc aaccggtgat accacgatac tatgactgag agtcaacgcc atgagcggcc 7980
   tcatttctta ttctgagtta caacagtccg caccgctgtc cggtagctcc ttccggtggg 8040
   cgcggggcat gactatcgtc gccgcactta tgactgtctt ctttatcatg caactcgtag 8100
   gacaggtgcc ggcagcgccc aacagtcccc cggccacggg gcctgccacc atacccacgc 8160
   cgaaacaagc gccctgcacc attatgttcc ggatctgcat cgcaggatgc tgctggctac 8220
   cctgtggaac acctacatct gtattaacga agcgctaacc gtttttatca ggctctggga 8280
   ggcagaataa atgatcatat cgtcaattat tacctccacg gggagagcct gagcaaactg 8340
   gcctcaggca tttgagaagc acacggtcac actgcttccg gtagtcaata aaccggtaaa 8400
   ccagcaatag acataagcgg ctatttaacg accctgccct gaaccgacga ccgggtcgaa 8460
   tttgctttcg aatttctgcc attcatccgc ttattatcac ttattcaggc gtagcaccag 8520
   gcgtttaagg gcaccaataa ctgccttaaa aaaattacgc cccgccctgc cactcatcgc 8580
   agtactgttg taattcatta agcattctgc cgacatggaa gccatcacag acggcatgat 8640
   gaacctgaat cgccagcggc atcagcacct tgtcgccttg cgtataatat ttgcccatgg 8700
   tgaaaacggg ggcgaagaag ttgtccatat tggccacgtt taaatcaaaa ctggtgaaac 8760
   tcacccaggg attggctgag acgaaaaaca tattctcaat aaacccttta gggaaatagg 8820
   ccaggttttc accgtaacac gccacatctt gcgaatatat gtgtagaaac tgccggaaat 8880
   cgtcgtggta ttcactccag agcgatgaaa acgtttcagt ttgctcatgg aaaacggtgt 8940
   aacaagggtg aacactatcc catatcacca gctcaccgtc tttcattgcc atacg 8995
<210> 135
   <211> 10319
   <212> DNA
   <213> Artificial Sequence
<220>
   <223> Description of Artificial Sequence: Synthetic polynucleotide
<400> 135
   ccttcaactc agcaaaagtt cgatttattc aacaaagcca cgttgtgtct caaaatctct 60
   gatgttacat tgcacaagat aaaaatatat catcatgaac aataaaactg tctgcttaca 120
   taaacagtaa tacaaggggt gttatgagcc atattcaacg ggaaacgtct tgctcgaggc 180
   cgcgattaaa ttccaacatg gatgctgatt tatatgggta taaatgggct cgcgataatg 240
   tcgggcaatc aggtgcgaca atctatcgat tgtatgggaa gcccgatgcg ccagagttgt 300
   ttctgaaaca tggcaaaggt agcgttgcca atgatgttac agatgagatg gtcagactaa 360
   actggctgac ggaatttatg cctcttccga ccatcaagca ttttatccgt actcctgatg 420
   atgcatggtt actcaccact gcgatccccg ggaaaacagc attccaggta ttagaagaat 480
   atcctgattc aggtgaaaat attgttgatg cgctggcagt gttcctgcgc cggttgcatt 540
   cgattcctgt ttgtaattgt ccttttaaca gcgatcgcgt atttcgtctc gctcaggcgc 600
   aatcacgaat gaataacggt ttggttgatg cgagtgattt tgatgacgag cgtaatggct 660
   ggcctgttga acaagtctgg aaagaaatgc ataagctttt gccattctca ccggattcag 720
   tcgtcactca tggtgatttc tcacttgata accttatttt tgacgagggg aaattaatag 780
   gttgtattga tgttggacga gtcggaatcg cagaccgata ccaggatctt gccatcctat 840
   ggaactgcct cggtgagttt tctccttcat tacagaaacg gctttttcaa aaatatggta 900
   ttgataatcc tgatatgaat aaattgcagt ttcatttgat gctcgatgag tttttctaat 960
   cgagtcaggc aactatggat gaacgaaata gacagatcgc tgagataggt gcctcactga 1020
   ttaagcattg gtaactgtca gaccaagttt actcatatat actttagatt gatttaaaac 1080
   ttcattttta atttaaaagg atctaggtga agatcctttt tgataatctc atgaccaaaa 1140
   tcccttaacg tgagttttcg ttccactgag cgtcagaccc cgtagaaaag atcaaaggat 1200
   cttcttgaga tccttttttt ctgcgcgtaa tctgctgctt gcaaacaaaa aaaccaccgc 1260
   taccagcggt ggtttgtttg ccggatcaag agctaccaac tctttttccg aaggtaactg 1320
   gcttcagcag agcgcagata ccaaatactg tccttctagt gtagccgtag ttaggccacc 1380
   acttcaagaa ctctgtagca ccgcctacat acctcgctct gctaatcctg ttaccagtgg 1440
   ctgctgccag tggcgataag tcgtgtctta ccgggttgga ctcaagacga tagttaccgg 1500
   ataaggcgca gcggtcgggc tgaacggggg gttcgtgcac acagcccagc ttggagcgaa 1560
   cgacctacac cgaactgaga tacctacagc gtgagctatg agaaagcgcc acgcttcccg 1620
   aagggagaaa ggcggacagg tatccggtaa gcggcagggt cggaacagga gagcgcacga 1680
   gggagcttcc agggggaaac gcctggtatc tttatagtcc tgtcgggttt cgccacctct 1740
   gacttgagcg tcgatttttg tgatgctcgt caggggggcg gagcctatgg aaaaacgcca 1800
   gcaacgcggc ctttttacgg ttcctggcct tttgctggcc ttttgctcac atggtacttg 1860
   gatcctcatt agtacagcag acgggcgcga atggtacccg gaatagcttt cattgcctgc 1920
   agcgcttttt cggcaacgtc ttcgtcggct tcaatatcaa taaccacata acccatctgg 1980
   gcggaagttt gcagatattg cgcggcgatg ttgacgccct gctcggcgaa gattttgttc 2040
   agcgcagtta gcacgcccgg acggttttcg tggatgtgca tcagacgacg cccaccgtgc 2100
   agtggcagcg agacttccgg gaagttcacc gcagagagcg ttgagccatt gtcagaatac 2160
   ttgatcaatt tacccgcaac ttccaggccg atattctcct gcgcttcctg agtcgaaccg 2220
   ccaatgtgtg gcgtcagaag gacgttgtcg aattcacaca gcggagaggt aaatggatcg 2280
   ctattggtcg ccggttccgt cgggaatacg tcgattgccg cccccgccag atgtttgctc 2340
   gccagcgcat cacacagcgc cggaatatcc accacagtac cgcgcgaagc attaatcagc 2400
   agcgagccgg gcttcattag tgaaatttct ttcgcgccca tcatattttt ggtggacgga 2460
   ttctctggta catgcagact caccacatcg ctcatattca gcaggtcaga aagatgctgt 2520
   acctgagtgg cgttgcccag cggcagttta ttttcaatat cataaaagta aacatacatt 2580
   cccagcgatt cagccagaat gcccaattgc gtaccaatat gaccgtagcc gatgataccc 2640
   agctttttgc cgcgcgcttc aaaagaaccc gccgccagtt tgttccacac gccacggtgc 2700
   gctttagcat tggcttccgg cacgccgcgc aatagcagca gcagttcgcc aatcaccagc 2760
   tccgcaacag agcgcgtatt tgagaacggt gcgttaaata ccgggatccc gcgctttgcc 2820
   gccgcatcca gatcaacctg gtttgttccg atacagaaac agccaatagc gaccagtttt 2880
   tctgcggcgt tgatcacgtc ttcagtcaga tgggtacggg atcgcaggcc gatgaagtgg 2940
   gcatcgcgga tggattcttt taattgttca tcatccagcg cgcctttgtg aaattcgatg 3000
   ttggtgtaac cagctgcacg aaggctttcc agcgcctttt ggtgcacgcc ttctaccagc 3060
   agaaacttaa tcttgtcttt ctccagcgat acctttgcca tggtaattct ccacgcttat 3120
   aagcgaataa aggaagatgg ccgccccgca gggcagcagg tctgtgaaac agtatagaga 3180
   ttcatcggca caaaggcttt gctttttgtc atttattcaa acctgtacga aagggtgtat 3240
   cgctcaatac tgaccattta aatcatacct gacccatgtt ctttcctgcg ttatcccctg 3300
   attctgtgga taaccgtatt accgcctttg agtgagctga taccgctcgc cgcagccgaa 3360
   cgaccgagcg cagcgagtca gtgagcgagg aagcggaaga gcgcctgatg cggtattttc 3420
   tccttacgca tctgtgcggt atttcacacc gcatatggtg cactctcagt acaatctgct 3480
   ctgatgccgc atagttaagc cagtatacac tccgctatcg ctacgtgact gggtcatggc 3540
   tgcgccccga cacccgccaa cacccgctga cgcgccctga cgggcttgtc tgctcccggc 3600
   atccgcttac agacaagctg tgaccgtctc cgggagctgc atgtgtcaga ggttttcacc 3660
   gtcatcaccg aaacgcgcga ggcagcagat caattcgcgc gcgaaggcga agcggcatgc 3720
   atttacgttg acaccatcga atggtgcaaa acctttcgcg gtatggcatg atagcgcccg 3780
   gaagagagtc aattcagggt ggtgaatgtg aaaccagtaa cgttatacga tgtcgcagag 3840
   tatgccggtg tctcttatca gaccgtttcc cgcgtggtga accaggccag ccacgtttct 3900
   gcgaaaacgc gggaaaaagt ggaagcggcg atggcggagc tgaattacat tcccaaccgc 3960
   gtggcacaac aactggcggg caaacagtcg ttgctgattg gcgttgccac ctccagtctg 4020
   gccctgcacg cgccgtcgca aattgtcgcg gcgattaaat ctcgcgccga tcaactgggt 4080
   gccagcgtgg tggtgtcgat ggtagaacga agcggcgtcg aagcctgtaa agcggcggtg 4140
   cacaatcttc tcgcgcaacg cgtcagtggg ctgatcatta actatccgct ggatgaccag 4200
   gatgccattg ctgtggaagc tgcctgcact aatgttccgg cgttatttct tgatgtctct 4260
   gaccagacac ccatcaacag tattattttc tcccatgaag acggtacgcg actgggcgtg 4320
   gagcatctgg tcgcattggg tcaccagcaa atcgcgctgt tagcgggccc attaagttct 4380
   gtctcggcgc gtctgcgtct ggctggctgg cataaatatc tcactcgcaa tcaaattcag 4440
   ccgatagcgg aacgggaagg cgactggagt gccatgtccg gttttcaaca aaccatgcaa 4500
   atgctgaatg agggcatcgt tcccactgcg atgctggttg ccaacgatca gatggcgctg 4560
   ggcgcaatgc gcgccattac cgagtccggg ctgcgcgttg gtgcggatat ctcggtagtg 4620
   ggatacgacg ataccgaaga cagctcatgt tatatcccgc cgtcaaccac catcaaacag 4680
   gattttcgcc tgctggggca aaccagcgtg gaccgcttgc tgcaactctc tcagggccag 4740
   gcggtgaagg gcaatcagct gttgcccgtc tcactggtga aaagaaaaac caccctggcg 4800
   cccaatacgc aaaccgcctc tccccgcgcg ttggccgatt cattaatgca gctggcacga 4860
   caggtttccc gactggaaag cgggcagtga gcgcaacgca attaatgtaa gttagcgcga 4920
   attgatctgg tttgacagct tatcatcgac tgcacggtgc accaatgctt ctggcgtcag 4980
   gcagccatcg gaagctgtgg tatggctgtg caggtcgtaa atcactgcat aattcgtgtc 5040
   gctcaaggcg cactcccgtt ctggataatg ttttttgcgc cgacatcata acggttctgg 5100
   caaatattct gaaatgagct gttgacaatt aatcatccgg ctcgtataat gtgtggaatt 5160
   gtgagcggat aacaatttca cacaggaaac agcgccgctg agaaaaagcg aagcggcact 5220
   gctctttaac aatttatcag acaatctgtg tgggcactcg accggaatta tcgattaact 5280
   ttattattaa aaattaaaga ggtatatatt aatgtatcga ttaaataagg aggaataaac 5340
   catggccggt acgggtcgtt tggctggtaa aattgcattg atcaccggtg gtgctggtaa 5400
   cattggttcc gagctgaccc gccgttttct ggccgagggt gcgacggtta ttatcagcgg 5460
   ccgtaaccgt gcgaagctga ccgcgctggc cgagcgcatg caagccgagg ccggcgtgcc 5520
   ggccaagcgc attgatttgg aggtgatgga tggttccgac cctgtggctg tccgtgccgg 5580
   tatcgaggca atcgtcgctc gccacggtca gattgacatt ctggttaaca acgcgggctc 5640
   cgccggtgcc caacgtcgct tggcggaaat tccgctgacg gaggcagaat tgggtccggg 5700
   tgcggaggag actttgcacg cttcgatcgc gaatctgttg ggcatgggtt ggcacctgat 5760
   gcgtattgcg gctccgcaca tgccagttgg ctccgcagtt atcaacgttt cgactatttt 5820
   ctcgcgcgca gagtactatg gtcgcattcc gtacgttacc ccgaaggcag cgctgaacgc 5880
   tttgtcccag ctggctgccc gcgagctggg cgctcgtggc atccgcgtta acactatttt 5940
   cccaggtcct attgagtccg accgcatccg taccgtgttt caacgtatgg atcaactgaa 6000
   gggtcgcccg gagggcgaca ccgcccatca ctttttgaac accatgcgcc tgtgccgcgc 6060
   aaacgaccaa ggcgctttgg aacgccgctt tccgtccgtt ggcgatgttg ctgatgcggc 6120
   tgtgtttctg gcttctgctg agagcgcggc actgtcgggt gagacgattg aggtcaccca 6180
   cggtatggaa ctgccggcgt gtagcgaaac ctccttgttg gcgcgtaccg atctgcgtac 6240
   catcgacgcg agcggtcgca ctaccctgat ttgcgctggc gatcaaattg aagaagttat 6300
   ggccctgacg ggcatgctgc gtacgtgcgg tagcgaagtg attatcggct tccgttctgc 6360
   ggctgccctg gcgcaatttg agcaggcagt gaatgaatct cgccgtctgg caggtgcgga 6420
   tttcaccccg ccgatcgctt tgccgttgga cccacgtgac ccggccacca ttgatgcggt 6480
   tttcgattgg ggcgcaggcg agaatacggg tggcatccat gcggcggtca ttctgccggc 6540
   aacctcccac gaaccggctc cgtgcgtgat tgaagtcgat gacgaacgcg tcctgaattt 6600
   cctggccgat gaaattaccg gcaccatcgt tattgcgagc cgtttggcgc gctattggca 6660
   atcccaacgc ctgaccccgg gtgcccgtgc ccgcggtccg cgtgttatct ttctgagcaa 6720
   cggtgccgat caaaatggta atgtttacgg tcgtattcaa tctgcggcga tcggtcaatt 6780
   gattcgcgtt tggcgtcacg aggcggagtt ggactatcaa cgtgcatccg ccgcaggcga 6840
   tcacgttctg ccgccggttt gggcgaacca gattgtccgt ttcgctaacc gctccctgga 6900
   aggtctggag ttcgcgtgcg cgtggaccgc acagctgctg cacagccaac gtcatattaa 6960
   cgaaattacg ctgaacattc cagccaatat tagcgcgacc acgggcgcac gttccgccag 7020
   cgtcggctgg gccgagtcct tgattggtct gcacctgggc aaggtggctc tgattaccgg 7080
   tggttcggcg ggcatcggtg gtcaaatcgg tcgtctgctg gccttgtctg gcgcgcgtgt 7140
   gatgctggcc gctcgcgatc gccataaatt ggaacagatg caagccatga ttcaaagcga 7200
   attggcggag gttggttata ccgatgtgga ggaccgtgtg cacatcgctc cgggttgcga 7260
   tgtgagcagc gaggcgcagc tggcagatct ggtggaacgt acgctgtccg cattcggtac 7320
   cgtggattat ttgattaata acgccggtat tgcgggcgtg gaggagatgg tgatcgacat 7380
   gccggtggaa ggctggcgtc acaccctgtt tgccaacctg atttcgaatt attcgctgat 7440
   gcgcaagttg gcgccgctga tgaagaagca aggtagcggt tacatcctga acgtttcttc 7500
   ctattttggc ggtgagaagg acgcggcgat tccttatccg aaccgcgccg actacgccgt 7560
   ctccaaggct ggccaacgcg cgatggcgga agtgttcgct cgtttcctgg gtccagagat 7620
   tcagatcaat gctattgccc caggtccggt tgaaggcgac cgcctgcgtg gtaccggtga 7680
   gcgtccgggc ctgtttgctc gtcgcgcccg tctgatcttg gagaataaac gcctgaacga 7740
   attgcacgcg gctttgattg ctgcggcccg caccgatgag cgctcgatgc acgagttggt 7800
   tgaattgttg ctgccgaacg acgtggccgc gttggagcag aacccagcgg cccctaccgc 7860
   gctgcgtgag ctggcacgcc gcttccgtag cgaaggtgat ccggcggcaa gctcctcgtc 7920
   cgccttgctg aatcgctcca tcgctgccaa gctgttggct cgcttgcata acggtggcta 7980
   tgtgctgccg gcggatattt ttgcaaatct gcctaatccg ccggacccgt tctttacccg 8040
   tgcgcaaatt gaccgcgaag ctcgcaaggt gcgtgatggt attatgggta tgctgtatct 8100
   gcagcgtatg ccaaccgagt ttgacgtcgc tatggcaacc gtgtactatc tggccgatcg 8160
   taacgtgagc ggcgaaactt tccatccgtc tggtggtttg cgctacgagc gtaccccgac 8220
   cggtggcgag ctgttcggcc tgccatcgcc ggaacgtctg gcggagctgg ttggtagcac 8280
   ggtgtacctg atcggtgaac acctgaccga gcacctgaac ctgctggctc gtgcctattt 8340
   ggagcgctac ggtgcccgtc aagtggtgat gattgttgag acggaaaccg gtgcggaaac 8400
   catgcgtcgt ctgttgcatg atcacgtcga ggcaggtcgc ctgatgacta ttgtggcagg 8460
   tgatcagatt gaggcagcga ttgaccaagc gatcacgcgc tatggccgtc cgggtccggt 8520
   ggtgtgcact ccattccgtc cactgccaac cgttccgctg gtcggtcgta aagactccga 8580
   ttggagcacc gttttgagcg aggcggaatt tgcggaactg tgtgagcatc agctgaccca 8640
   ccatttccgt gttgctcgta agatcgcctt gtcggatggc gcgtcgctgg cgttggttac 8700
   cccggaaacg actgcgacta gcaccacgga gcaatttgct ctggcgaact tcatcaagac 8760
   caccctgcac gcgttcaccg cgaccatcgg tgttgagtcg gagcgcaccg cgcaacgtat 8820
   tctgattaac caggttgatc tgacgcgccg cgcccgtgcg gaagagccgc gtgacccgca 8880
   cgagcgtcag caggaattgg aacgcttcat tgaagccgtt ctgctggtta ccgctccgct 8940
   gcctcctgag gcagacacgc gctacgcagg ccgtattcac cgcggtcgtg cgattaccgt 9000
   ctaatagaag cttggctgtt ttggcggatg agagaagatt ttcagcctga tacagattaa 9060
   atcagaacgc agaagcggtc tgataaaaca gaatttgcct ggcggcagta gcgcggtggt 9120
   cccacctgac cccatgccga actcagaagt gaaacgccgt agcgccgatg gtagtgtggg 9180
   gtctccccat gcgagagtag ggaactgcca ggcatcaaat aaaacgaaag gctcagtcga 9240
   aagactgggc ctttcgtttt atctgttgtt tgtcggtgaa cgctctcctg agtaggacaa 9300
   atccgccggg agcggatttg aacgttgcga agcaacggcc cggagggtgg cgggcaggac 9360
   gcccgccata aactgccagg catcaaatta agcagaaggc catcctgacg gatggccttt 9420
   ttgcgtttct acaaactctt tttgtttatt tttctaaata cattcaaata tgtatccgct 9480
   catgagacaa taaccctgat aaatgcttca ataatattga aaaaggaaga gtatgagtat 9540
   tcaacatttc cgtgtcgccc ttattccctt ttttgcggca ttttgccttc ctgtttttgc 9600
   tcacccagaa acgctggtga aagtaaaaga tgctgaagat cagttgggtg cacgagtggg 9660
   ttacatcgaa ctggatctca acagcggtaa gatccttgag agttttcgcc ccgaagaacg 9720
   ttttccaatg atgagcactt ttaaagttct gctatgtggc gcggtattat cccgtgttga 9780
   cgccgggcaa gagcaactcg gtcgccgcat acactattct cagaatgact tggttgagta 9840
   ctcaccagtc acagaaaagc atcttacgga tggcatgaca gtaagagaat tatgcagtgc 9900
   tgccataacc atgagtgata acactgcggc caacttactt ctgacaacga tcggaggacc 9960
   gaaggagcta accgcttttt tgcacaacat gggggatcat gtaactcgcc ttgatcgttg 10020
   ggaaccggag ctgaatgaag ccataccaaa cgacgagcgt gacaccacga tgcctgtagc 10080
   aatggcaaca acgttgcgca aactattaac tggcgaacta cttactctag cttcccggca 10140
   acaattaata gactggatgg aggcggataa agttgcagga ccacttctgc gctcggccct 10200
   tccggctggc tggtttattg ctgataaatc tggagccggt gagcgtgggt ctcgcggtat 10260
   cattgcagca ctggggccag atggtaagcc ctcccgtatc gtagttatct acacgacgg 10319
<210> 136
   <211> 5543
   <212> DNA
   <213> Artificial Sequence
<220>
   <223> Description of Artificial Sequence: Synthetic polynucleotide
<400> 136
   gtttgacagc ttatcatcga ctgcacggtg caccaatgct tctggcgtca ggcagccatc 60
   ggaagctgtg gtatggctgt gcaggtcgta aatcactgca taattcgtgt cgctcaaggc 120
   gcactcccgt tctggataat gttttttgcg ccgacatcat aacggttctg gcaaatattc 180
   tgaaatgagc tgttgacaat taatcatccg gctcgtataa tgtgtggaat tgtgagcgga 240
   taacaatttc acacaggaaa cagcgccgct gagaaaaagc gaagcggcac tgctctttaa 300
   caatttatca gacaatctgt gtgggcactc gaccggaatt atcgattaac tttattatta 360
   aaaattaaag aggtatatat taatgtatcg attaaataag gaggaataaa ccatgggatc 420
   taagcgtcgt gtagttgtga ccggactggg catgttgtct cctgtcggca ataccgtaga 480
   gtctacctgg aaagctctgc ttgccggtca gagtggcatc agcctaatcg accatttcga 540
   tactagcgcc tatgcaacga aatttgctgg cttagtaaag gattttaact gtgaggacat 600
   tatctcgcgc aaagaacagc gcaagatgga tgccttcatt caatatggaa ttgtcgctgg 660
   cgttcaggcc atgcaggatt ctggccttga aataacggaa gagaacgcaa cccgcattgg 720
   tgccgcaatt ggctccggga ttggcggcct cggactgatc gaagaaaacc acacatctct 780
   gatgaacggt ggtccacgta agatcagccc attcttcgtt ccgtcaacga ttgtgaacat 840
   ggtggcaggt catctgacta tcatgtatgg cctgcgtggc ccgagcatct ctatcgcgac 900
   tgcctgtact tccggcgtgc acaacattgg ccatgctgcg cgtattatcg cgtatggcga 960
   tgctgacgtg atggttgcag gtggcgcaga gaaagccagt acgccgctgg gcgttggtgg 1020
   ttttggcgcg gcacgtgcat tatctacccg caatgataac ccgcaagcgg cgagccgccc 1080
   gtgggataaa gagcgtgatg gtttcgtact gggcgatggt gccggtatgc tggtacttga 1140
   agagtacgaa cacgcgaaaa aacgcggtgc gaaaatttac gctgaactcg tcggctttgg 1200
   tatgagcagc gatgcttatc atatgacgtc accgccagaa aatggcgcag gcgcagctct 1260
   ggcgatggca aatgctctgc gtgatgcagg cattgaagcg agtcagattg gctacgttaa 1320
   cgcgcacggt acttctacgc cggctggcga taaagctgaa gcgcaggcgg tgaaaaccat 1380
   cttcggtgaa gctgcaagcc gtgtgttggt aagctccacg aaatctatga ccggtcacct 1440
   gttaggtgcg gcgggtgcag tagaatctat ctactccatc ctggcgctgc gcgatcaggc 1500
   tgttccgcca accatcaacc tggataaccc ggatgaaggt tgcgatctgg atttcgtacc 1560
   gcacgaagcg cgtcaggtta gcggaatgga atacactctg tgtaactcct tcggcttcgg 1620
   tggcactaat ggttctttga tctttaaaaa gatctaatta ctgcagctgg taccatatgg 1680
   gaattcgaag cttggctgtt ttggcggatg agagaagatt ttcagcctga tacagattaa 1740
   atcagaacgc agaagcggtc tgataaaaca gaatttgcct ggcggcagta gcgcggtggt 1800
   cccacctgac cccatgccga actcagaagt gaaacgccgt agcgccgatg gtagtgtggg 1860
   gtctccccat gcgagagtag ggaactgcca ggcatcaaat aaaacgaaag gctcagtcga 1920
   aagactgggc ctttcgtttt atctgttgtt tgtcggtgaa cgctctcctg agtaggacaa 1980
   atccgccggg agcggatttg aacgttgcga agcaacggcc cggagggtgg cgggcaggac 2040
   gcccgccata aactgccagg catcaaatta agcagaaggc catcctgacg gatggccttt 2100
   ttgcgtttct acaaactctt tttgtttatt tttctaaata cattcaaata tgtatccgct 2160
   catgagacaa taaccctgat aaatgcttca ataatattga aaaaggaaga gtatgagtat 2220
   tcaacatttc cgtgtcgccc ttattccctt ttttgcggca ttttgccttc ctgtttttgc 2280
   tcacccagaa acgctggtga aagtaaaaga tgctgaagat cagttgggtg cacgagtggg 2340
   ttacatcgaa ctggatctca acagcggtaa gatccttgag agttttcgcc ccgaagaacg 2400
   ttttccaatg atgagcactt ttaaagttct gctatgtggc gcggtattat cccgtgttga 2460
   cgccgggcaa gagcaactcg gtcgccgcat acactattct cagaatgact tggttgagta 2520
   ctcaccagtc acagaaaagc atcttacgga tggcatgaca gtaagagaat tatgcagtgc 2580
   tgccataacc atgagtgata acactgcggc caacttactt ctgacaacga tcggaggacc 2640
   gaaggagcta accgcttttt tgcacaacat gggggatcat gtaactcgcc ttgatcgttg 2700
   ggaaccggag ctgaatgaag ccataccaaa cgacgagcgt gacaccacga tgcctgtagc 2760
   aatggcaaca acgttgcgca aactattaac tggcgaacta cttactctag cttcccggca 2820
   acaattaata gactggatgg aggcggataa agttgcagga ccacttctgc gctcggccct 2880
   tccggctggc tggtttattg ctgataaatc tggagccggt gagcgtgggt ctcgcggtat 2940
   cattgcagca ctggggccag atggtaagcc ctcccgtatc gtagttatct acacgacggg 3000
   gagtcaggca actatggatg aacgaaatag acagatcgct gagataggtg cctcactgat 3060
   taagcattgg taactgtcag accaagttta ctcatatata ctttagattg atttaaaact 3120
   tcatttttaa tttaaaagga tctaggtgaa gatccttttt gataatctca tgaccaaaat 3180
   cccttaacgt gagttttcgt tccactgagc gtcagacccc gtagaaaaga tcaaaggatc 3240
   ttcttgagat cctttttttc tgcgcgtaat ctgctgcttg caaacaaaaa aaccaccgct 3300
   accagcggtg gtttgtttgc cggatcaaga gctaccaact ctttttccga aggtaactgg 3360
   cttcagcaga gcgcagatac caaatactgt ccttctagtg tagccgtagt taggccacca 3420
   cttcaagaac tctgtagcac cgcctacata cctcgctctg ctaatcctgt taccagtggc 3480
   tgctgccagt ggcgataagt cgtgtcttac cgggttggac tcaagacgat agttaccgga 3540
   taaggcgcag cggtcgggct gaacgggggg ttcgtgcaca cagcccagct tggagcgaac 3600
   gacctacacc gaactgagat acctacagcg tgagctatga gaaagcgcca cgcttcccga 3660
   agggagaaag gcggacaggt atccggtaag cggcagggtc ggaacaggag agcgcacgag 3720
   ggagcttcca gggggaaacg cctggtatct ttatagtcct gtcgggtttc gccacctctg 3780
   acttgagcgt cgatttttgt gatgctcgtc aggggggcgg agcctatgga aaaacgccag 3840
   caacgcggcc tttttacggt tcctggcctt ttgctggcct tttgctcaca tgttctttcc 3900
   tgcgttatcc cctgattctg tggataaccg tattaccgcc tttgagtgag ctgataccgc 3960
   tcgccgcagc cgaacgaccg agcgcagcga gtcagtgagc gaggaagcgg aagagcgcct 4020
   gatgcggtat tttctcctta cgcatctgtg cggtatttca caccgcatat ggtgcactct 4080
   cagtacaatc tgctctgatg ccgcatagtt aagccagtat acactccgct atcgctacgt 4140
   gactgggtca tggctgcgcc ccgacacccg ccaacacccg ctgacgcgcc ctgacgggct 4200
   tgtctgctcc cggcatccgc ttacagacaa gctgtgaccg tctccgggag ctgcatgtgt 4260
   cagaggtttt caccgtcatc accgaaacgc gcgaggcagc agatcaattc gcgcgcgaag 4320
   gcgaagcggc atgcatttac gttgacacca tcgaatggtg caaaaccttt cgcggtatgg 4380
   catgatagcg cccggaagag agtcaattca gggtggtgaa tgtgaaacca gtaacgttat 4440
   acgatgtcgc agagtatgcc ggtgtctctt atcagaccgt ttcccgcgtg gtgaaccagg 4500
   ccagccacgt ttctgcgaaa acgcgggaaa aagtggaagc ggcgatggcg gagctgaatt 4560
   acattcccaa ccgcgtggca caacaactgg cgggcaaaca gtcgttgctg attggcgttg 4620
   ccacctccag tctggccctg cacgcgccgt cgcaaattgt cgcggcgatt aaatctcgcg 4680
   ccgatcaact gggtgccagc gtggtggtgt cgatggtaga acgaagcggc gtcgaagcct 4740
   gtaaagcggc ggtgcacaat cttctcgcgc aacgcgtcag tgggctgatc attaactatc 4800
   cgctggatga ccaggatgcc attgctgtgg aagctgcctg cactaatgtt ccggcgttat 4860
   ttcttgatgt ctctgaccag acacccatca acagtattat tttctcccat gaagacggta 4920
   cgcgactggg cgtggagcat ctggtcgcat tgggtcacca gcaaatcgcg ctgttagcgg 4980
   gcccattaag ttctgtctcg gcgcgtctgc gtctggctgg ctggcataaa tatctcactc 5040
   gcaatcaaat tcagccgata gcggaacggg aaggcgactg gagtgccatg tccggttttc 5100
   aacaaaccat gcaaatgctg aatgagggca tcgttcccac tgcgatgctg gttgccaacg 5160
   atcagatggc gctgggcgca atgcgcgcca ttaccgagtc cgggctgcgc gttggtgcgg 5220
   atatctcggt agtgggatac gacgataccg aagacagctc atgttatatc ccgccgtcaa 5280
   ccaccatcaa acaggatttt cgcctgctgg ggcaaaccag cgtggaccgc ttgctgcaac 5340
   tctctcaggg ccaggcggtg aagggcaatc agctgttgcc cgtctcactg gtgaaaagaa 5400
   aaaccaccct ggcgcccaat acgcaaaccg cctctccccg cgcgttggcc gattcattaa 5460
   tgcagctggc acgacaggtt tcccgactgg aaagcgggca gtgagcgcaa cgcaattaat 5520
   gtgagttagc gcgaattgat ctg 5543
<210> 137
   <211> 6620
   <212> DNA
   <213> Artificial Sequence
<220>
   <223> Description of Artificial Sequence: Synthetic polynucleotide
<400> 137
   cgcggtatca ttgcagcact ggggccagat ggtaagccct cccgtatcgt agttatctac 60
   acgacgggga gtcaggcaac tatggatgaa cgaaatagac agatcgctga gataggtgcc 120
   tcactgatta agcattggta actgtcagac caagtttact catatatact ttagattgat 180
   ttaaaacttc atttttaatt taaaaggatc taggtgaaga tcctttttga taatctcatg 240
   accaaaatcc cttaacgtga gttttcgttc cactgagcgt cagacccctt aataagatga 300
   tcttcttgag atcgttttgg tctgcgcgta atctcttgct ctgaaaacga aaaaaccgcc 360
   ttgcagggcg gtttttcgaa ggttctctga gctaccaact ctttgaaccg aggtaactgg 420
   cttggaggag cgcagtcacc aaaacttgtc ctttcagttt agccttaacc ggcgcatgac 480
   ttcaagacta actcctctaa atcaattacc agtggctgct gccagtggtg cttttgcatg 540
   tctttccggg ttggactcaa gacgatagtt accggataag gcgcagcggt cggactgaac 600
   ggggggttcg tgcatacagt ccagcttgga gcgaactgcc tacccggaac tgagtgtcag 660
   gcgtggaatg agacaaacgc ggccataaca gcggaatgac accggtaaac cgaaaggcag 720
   gaacaggaga gcgcacgagg gagccgccag ggggaaacgc ctggtatctt tatagtcctg 780
   tcgggtttcg ccaccactga tttgagcgtc agatttcgtg atgcttgtca ggggggcgga 840
   gcctatggaa aaacggcttt gccgcggccc tctcacttcc ctgttaagta tcttcctggc 900
   atcttccagg aaatctccgc cccgttcgta agccatttcc gctcgccgca gtcgaacgac 960
   cgagcgtagc gagtcagtga gcgaggaagc ggaatatatc ctgtatcaca tattctgctg 1020
   acgcaccggt gcagcctttt ttctcctgcc acatgaagca cttcactgac accctcatca 1080
   gtgccaacat agtaagccag tatacactcc gctagcgctg aggtctgcct cgtgaagaag 1140
   gtgttgctga ctcataccag gcctgaatcg ccccatcatc cagccagaaa gtgagggagc 1200
   cacggttgat gagagctttg ttgtaggtgg accagttggt gattttgaac ttttgctttg 1260
   ccacggaacg gtctgcgttg tcgggaagat gcgtgatctg atccttcaac tcagcaaaag 1320
   ttcgatttat tcaacaaagc cacgttgtgt ctcaaaatct ctgatgttac attgcacaag 1380
   ataaaaatat atcatcatga acaataaaac tgtctgctta cataaacagt aatacaaggg 1440
   gtgttatgag ccatattcaa cgggaaacgt cttgctcgag gccgcgatta aattccaaca 1500
   tggatgctga tttatatggg tataaatggg ctcgcgataa tgtcgggcaa tcaggtgcga 1560
   caatctatcg attgtatggg aagcccgatg cgccagagtt gtttctgaaa catggcaaag 1620
   gtagcgttgc caatgatgtt acagatgaga tggtcagact aaactggctg acggaattta 1680
   tgcctcttcc gaccatcaag cattttatcc gtactcctga tgatgcatgg ttactcacca 1740
   ctgcgatccc cgggaaaaca gcattccagg tattagaaga atatcctgat tcaggtgaaa 1800
   atattgttga tgcgctggca gtgttcctgc gccggttgca ttcgattcct gtttgtaatt 1860
   gtccttttaa cagcgatcgc gtatttcgtc tcgctcaggc gcaatcacga atgaataacg 1920
   gtttggttga tgcgagtgat tttgatgacg agcgtaatgg ctggcctgtt gaacaagtct 1980
   ggaaagaaat gcataagctt ttgccattct caccggattc agtcgtcact catggtgatt 2040
   tctcacttga taaccttatt tttgacgagg ggaaattaat aggttgtatt gatgttggac 2100
   gagtcggaat cgcagaccga taccaggatc ttgccatcct atggaactgc ctcggtgagt 2160
   tttctccttc attacagaaa cggctttttc aaaaatatgg tattgataat cctgatatga 2220
   ataaattgca gtttcatttg atgctcgatg agtttttcta atcagaattg gttaattggt 2280
   tgtaacactg gcagagcatt acgctgactt gacgcggaag agccgatgct tgacgcggaa 2340
   gagccgatgc cagtccagtt acgctggagt ctgaggctcg tcctgaatga tatcaagctt 2400
   gattcgtctg tggtatggct gtgcaggtcg taaatcactg cataattcgt gtcgctcaag 2460
   gcgcactccc gttctggata atgttttttg cgccgacatc ataacggttc tggcaaatat 2520
   tctgaaatga gctgttgaca attaatcatc cggctcgtat aatgtgtgga attgtgagcg 2580
   gataacaatt tcacacagga aacagcgccg ctgagaaaaa gcgaagcggc actgctcttt 2640
   aacaatttat cagacaatct gtgtgggcac tcgaccggaa ttatcgatta actttattat 2700
   taaaaattaa agaggtatat attaatgtat cgattaaata aggaggaata aaccatggcc 2760
   ggtacgggtc gtttggctgg taaaattgca ttgatcaccg gtggtgctgg taacattggt 2820
   tccgagctga cccgccgttt tctggccgag ggtgcgacgg ttattatcag cggccgtaac 2880
   cgtgcgaagc tgaccgcgct ggccgagcgc atgcaagccg aggccggcgt gccggccaag 2940
   cgcattgatt tggaggtgat ggatggttcc gaccctgtgg ctgtccgtgc cggtatcgag 3000
   gcaatcgtcg ctcgccacgg tcagattgac attctggtta acaacgcggg ctccgccggt 3060
   gcccaacgtc gcttggcgga aattccgctg acggaggcag aattgggtcc gggtgcggag 3120
   gagactttgc acgcttcgat cgcgaatctg ttgggcatgg gttggcacct gatgcgtatt 3180
   gcggctccgc acatgccagt tggctccgca gttatcaacg tttcgactat tttctcgcgc 3240
   gcagagtact atggtcgcat tccgtacgtt accccgaagg cagcgctgaa cgctttgtcc 3300
   cagctggctg cccgcgagct gggcgctcgt ggcatccgcg ttaacactat tttcccaggt 3360
   cctattgagt ccgaccgcat ccgtaccgtg tttcaacgta tggatcaact gaagggtcgc 3420
   ccggagggcg acaccgccca tcactttttg aacaccatgc gcctgtgccg cgcaaacgac 3480
   caaggcgctt tggaacgccg ctttccgtcc gttggcgatg ttgctgatgc ggctgtgttt 3540
   ctggcttctg ctgagagcgc ggcactgtcg ggtgagacga ttgaggtcac ccacggtatg 3600
   gaactgccgg cgtgtagcga aacctccttg ttggcgcgta ccgatctgcg taccatcgac 3660
   gcgagcggtc gcactaccct gatttgcgct ggcgatcaaa ttgaagaagt tatggccctg 3720
   acgggcatgc tgcgtacgtg cggtagcgaa gtgattatcg gcttccgttc tgcggctgcc 3780
   ctggcgcaat ttgagcaggc agtgaatgaa tctcgccgtc tggcaggtgc ggatttcacc 3840
   ccgccgatcg ctttgccgtt ggacccacgt gacccggcca ccattgatgc ggttttcgat 3900
   tggggcgcag gcgagaatac gggtggcatc catgcggcgg tcattctgcc ggcaacctcc 3960
   cacgaaccgg ctccgtgcgt gattgaagtc gatgacgaac gcgtcctgaa tttcctggcc 4020
   gatgaaatta ccggcaccat cgttattgcg agccgtttgg cgcgctattg gcaatcccaa 4080
   cgcctgaccc cgggtgcccg tgcccgcggt ccgcgtgtta tctttctgag caacggtgcc 4140
   gatcaaaatg gtaatgttta cggtcgtatt caatctgcgg cgatcggtca attgattcgc 4200
   gtttggcgtc acgaggcgga gttggactat caacgtgcat ccgccgcagg cgatcacgtt 4260
   ctgccgccgg tttgggcgaa ccagattgtc cgtttcgcta accgctccct ggaaggtctg 4320
   gagttcgcgt gcgcgtggac cgcacagctg ctgcacagcc aacgtcatat taacgaaatt 4380
   acgctgaaca ttccagccaa tattagcgcg accacgggcg cacgttccgc cagcgtcggc 4440
   tgggccgagt ccttgattgg tctgcacctg ggcaaggtgg ctctgattac cggtggttcg 4500
   gcgggcatcg gtggtcaaat cggtcgtctg ctggccttgt ctggcgcgcg tgtgatgctg 4560
   gccgctcgcg atcgccataa attggaacag atgcaagcca tgattcaaag cgaattggcg 4620
   gaggttggtt ataccgatgt ggaggaccgt gtgcacatcg ctccgggttg cgatgtgagc 4680
   agcgaggcgc agctggcaga tctggtggaa cgtacgctgt ccgcattcgg taccgtggat 4740
   tatttgatta ataacgccgg tattgcgggc gtggaggaga tggtgatcga catgccggtg 4800
   gaaggctggc gtcacaccct gtttgccaac ctgatttcga attattcgct gatgcgcaag 4860
   ttggcgccgc tgatgaagaa gcaaggtagc ggttacatcc tgaacgtttc ttcctatttt 4920
   ggcggtgaga aggacgcggc gattccttat ccgaaccgcg ccgactacgc cgtctccaag 4980
   gctggccaac gcgcgatggc ggaagtgttc gctcgtttcc tgggtccaga gattcagatc 5040
   aatgctattg ccccaggtcc ggttgaaggc gaccgcctgc gtggtaccgg tgagcgtccg 5100
   ggcctgtttg ctcgtcgcgc ccgtctgatc ttggagaata aacgcctgaa cgaattgcac 5160
   gcggctttga ttgctgcggc ccgcaccgat gagcgctcga tgcacgagtt ggttgaattg 5220
   ttgctgccga acgacgtggc cgcgttggag cagaacccag cggcccctac cgcgctgcgt 5280
   gagctggcac gccgcttccg tagcgaaggt gatccggcgg caagctcctc gtccgccttg 5340
   ctgaatcgct ccatcgctgc caagctgttg gctcgcttgc ataacggtgg ctatgtgctg 5400
   ccggcggata tttttgcaaa tctgcctaat ccgccggacc cgttctttac ccgtgcgcaa 5460
   attgaccgcg aagctcgcaa ggtgcgtgat ggtattatgg gtatgctgta tctgcagcgt 5520
   atgccaaccg agtttgacgt cgctatggca accgtgtact atctggccga tcgtaacgtg 5580
   agcggcgaaa ctttccatcc gtctggtggt ttgcgctacg agcgtacccc gaccggtggc 5640
   gagctgttcg gcctgccatc gccggaacgt ctggcggagc tggttggtag cacggtgtac 5700
   ctgatcggtg aacacctgac cgagcacctg aacctgctgg ctcgtgccta tttggagcgc 5760
   tacggtgccc gtcaagtggt gatgattgtt gagacggaaa ccggtgcgga aaccatgcgt 5820
   cgtctgttgc atgatcacgt cgaggcaggt cgcctgatga ctattgtggc aggtgatcag 5880
   attgaggcag cgattgacca agcgatcacg cgctatggcc gtccgggtcc ggtggtgtgc 5940
   actccattcc gtccactgcc aaccgttccg ctggtcggtc gtaaagactc cgattggagc 6000
   accgttttga gcgaggcgga atttgcggaa ctgtgtgagc atcagctgac ccaccatttc 6060
   cgtgttgctc gtaagatcgc cttgtcggat ggcgcgtcgc tggcgttggt taccccggaa 6120
   acgactgcga ctagcaccac ggagcaattt gctctggcga acttcatcaa gaccaccctg 6180
   cacgcgttca ccgcgaccat cggtgttgag tcggagcgca ccgcgcaacg tattctgatt 6240
   aaccaggttg atctgacgcg ccgcgcccgt gcggaagagc cgcgtgaccc gcacgagcgt 6300
   cagcaggaat tggaacgctt cattgaagcc gttctgctgg ttaccgctcc gctgcctcct 6360
   gaggcagaca cgcgctacgc aggccgtatt caccgcggtc gtgcgattac cgtctaatag 6420
   aagcttggct gttttggcgg atgagagaag attttcagcc tgatacagat taaatcagaa 6480
   cgcagaagcg gtctgataaa acagaatttg cctggcggca gtagcgcggt ggtcccacct 6540
   gaccccatgc cgaactcaga agtgaaacga cgaattctct agatatcgct caatactgac 6600
   catttaaatc atacctgacc 6620
<210> 138
   <211> 10944
   <212> DNA
   <213> Artificial Sequence
<220>
   <223> Description of Artificial Sequence: Synthetic polynucleotide
<400> 138
   cgcggtatca ttgcagcact ggggccagat ggtaagccct cccgtatcgt agttatctac      60
   acgacgggga gtcaggcaac tatggatgaa cgaaatagac agatcgctga gataggtgcc     120
   tcactgatta agcattggta actgtcagac caagtttact catatatact ttagattgat     180
   ttaaaacttc atttttaatt taaaaggatc taggtgaaga tcctttttga taatctcatg     240
   accaaaatcc cttaacgtga gttttcgttc cactgagcgt cagacccctt aataagatga     300
   tcttcttgag atcgttttgg tctgcgcgta atctcttgct ctgaaaacga aaaaaccgcc     360
   ttgcagggcg gtttttcgaa ggttctctga gctaccaact ctttgaaccg aggtaactgg     420
   cttggaggag cgcagtcacc aaaacttgtc ctttcagttt agccttaacc ggcgcatgac     480
   ttcaagacta actcctctaa atcaattacc agtggctgct gccagtggtg cttttgcatg     540
   tctttccggg ttggactcaa gacgatagtt accggataag gcgcagcggt cggactgaac     600
   ggggggttcg tgcatacagt ccagcttgga gcgaactgcc tacccggaac tgagtgtcag     660
   gcgtggaatg agacaaacgc ggccataaca gcggaatgac accggtaaac cgaaaggcag     720
   gaacaggaga gcgcacgagg gagccgccag ggggaaacgc  ctggtatctt tatagtcctg     780
   tcgggtttcg ccaccactga tttgagcgtc agatttcgtg atgcttgtca ggggggcgga     840
   gcctatggaa aaacggcttt gccgcggccc tctcacttcc ctgttaagta tcttcctggc     900
   atcttccagg aaatctccgc cccgttcgta agccatttcc gctcgccgca gtcgaacgac     960
   cgagcgtagc gagtcagtga gcgaggaagc ggaatatatc ctgtatcaca tattctgctg    1020
   acgcaccggt gcagcctttt ttctcctgcc acatgaagca cttcactgac accctcatca    1080
   gtgccaacat agtaagccag tatacactcc gctagcgctg aggtctgcct cgtgaagaag    1140
   gtgttgctga ctcataccag gcctgaatcg ccccatcatc cagccagaaa gtgagggagc    1200
   cacggttgat gagagctttg ttgtaggtgg accagttggt gattttgaac ttttgctttg    1260
   ccacggaacg gtctgcgttg tcgggaagat gcgtgatctg atccttcaac tcagcaaaag    1320
   ttcgatttat tcaacaaagc cacgttgtgt ctcaaaatct ctgatgttac attgcacaag    1380
   ataaaaatat atcatcatga acaataaaac tgtctgctta cataaacagt aatacaaggg 1440
   gtgttatgag ccatattcaa cgggaaacgt cttgctcgag gccgcgatta aattccaaca 1500
   tggatgctga tttatatggg tataaatggg ctcgcgataa tgtcgggcaa tcaggtgcga 1560
   caatctatcg attgtatggg aagcccgatg cgccagagtt gtttctgaaa catggcaaag 1620
   gtagcgttgc caatgatgtt acagatgaga tggtcagact aaactggctg acggaattta 1680
   tgcctcttcc gaccatcaag cattttatcc gtactcctga tgatgcatgg ttactcacca 1740
   ctgcgatccc cgggaaaaca gcattccagg tattagaaga atatcctgat tcaggtgaaa 1800
   atattgttga tgcgctggca gtgttcctgc gccggttgca ttcgattcct gtttgtaatt 1860
   gtccttttaa cagcgatcgc gtatttcgtc tcgctcaggc gcaatcacga atgaataacg 1920
   gtttggttga tgcgagtgat tttgatgacg agcgtaatgg ctggcctgtt gaacaagtct 1980
   ggaaagaaat gcataagctt ttgccattct caccggattc agtcgtcact catggtgatt 2040
   tctcacttga taaccttatt tttgacgagg ggaaattaat aggttgtatt gatgttggac 2100
   gagtcggaat cgcagaccga taccaggatc ttgccatcct atggaactgc ctcggtgagt 2160
   tttctccttc attacagaaa cggctttttc aaaaatatgg tattgataat cctgatatga 2220
   ataaattgca gtttcatttg atgctcgatg agtttttcta atcagaattg gttaattggt 2280
   tgtaacactg gcagagcatt acgctgactt gacgcggaag agccgatgcc agtccagtta 2340
   cgctggagtc tgaggctcgt cctgaatgat atcaagcttg attcgtctgt ggtatggctg 2400
   tgcaggtcgt aaatcactgc ataattcgtg tcgctcaagg cgcactcccg ttctggataa 2460
   tgttttttgc gccgacatca taacggttct ggcaaatatt ctgaaatgag ctgttgacaa 2520
   ttaatcatcc ggctcgtata atgtgtggaa ttgtgagcgg ataacaattt cacacaggaa 2580
   acagcgccgc tgagaaaaag cgaagcggca ctgctcttta acaatttatc agacaatctg 2640
   tgtgggcact cgaccggaat tatcgattaa ctttattatt aaaaattaaa gaggtatata 2700
   ttaatgtatc gattaaataa ggaggaataa accatggccg gtacgggtcg tttggctggt 2760
   aaaattgcat tgatcaccgg tggtgctggt aacattggtt ccgagctgac ccgccgtttt 2820
   ctggccgagg gtgcgacggt tattatcagc ggccgtaacc gtgcgaagct gaccgcgctg 2880
   gccgagcgca tgcaagccga ggccggcgtg ccggccaagc gcattgattt ggaggtgatg 2940
   gatggttccg accctgtggc tgtccgtgcc ggtatcgagg caatcgtcgc tcgccacggt 3000
   cagattgaca ttctggttaa caacgcgggc tccgccggtg cccaacgtcg cttggcggaa 3060
   attccgctga cggaggcaga attgggtccg ggtgcggagg agactttgca cgcttcgatc 3120
   gcgaatctgt tgggcatggg ttggcacctg atgcgtattg cggctccgca catgccagtt 3180
   ggctccgcag ttatcaacgt ttcgactatt ttctcgcgcg cagagtacta tggtcgcatt 3240
   ccgtacgtta ccccgaaggc agcgctgaac gctttgtccc agctggctgc ccgcgagctg 3300
   ggcgctcgtg gcatccgcgt taacactatt ttcccaggtc ctattgagtc cgaccgcatc 3360
   cgtaccgtgt ttcaacgtat ggatcaactg aagggtcgcc cggagggcga caccgcccat 3420
   cactttttga acaccatgcg cctgtgccgc gcaaacgacc aaggcgcttt ggaacgccgc 3480
   tttccgtccg ttggcgatgt tgctgatgcg gctgtgtttc tggcttctgc tgagagcgcg 3540
   gcactgtcgg gtgagacgat tgaggtcacc cacggtatgg aactgccggc gtgtagcgaa 3600
   acctccttgt tggcgcgtac cgatctgcgt accatcgacg cgagcggtcg cactaccctg 3660
   atttgcgctg gcgatcaaat tgaagaagtt atggccctga cgggcatgct gcgtacgtgc 3720
   ggtagcgaag tgattatcgg cttccgttct gcggctgccc tggcgcaatt tgagcaggca 3780
   gtgaatgaat ctcgccgtct ggcaggtgcg gatttcaccc cgccgatcgc tttgccgttg 3840
   gacccacgtg acccggccac cattgatgcg gttttcgatt ggggcgcagg cgagaatacg 3900
   ggtggcatcc atgcggcggt cattctgccg gcaacctccc acgaaccggc tccgtgcgtg 3960
   attgaagtcg atgacgaacg cgtcctgaat ttcctggccg atgaaattac cggcaccatc 4020
   gttattgcga gccgtttggc gcgctattgg caatcccaac gcctgacccc gggtgcccgt 4080
   gcccgcggtc cgcgtgttat ctttctgagc aacggtgccg atcaaaatgg taatgtttac 4140
   ggtcgtattc aatctgcggc gatcggtcaa ttgattcgcg tttggcgtca cgaggcggag 4200
   ttggactatc aacgtgcatc cgccgcaggc gatcacgttc tgccgccggt ttgggcgaac 4260
   cagattgtcc gtttcgctaa ccgctccctg gaaggtctgg agttcgcgtg cgcgtggacc 4320
   gcacagctgc tgcacagcca acgtcatatt aacgaaatta cgctgaacat tccagccaat 4380
   attagcgcga ccacgggcgc acgttccgcc agcgtcggct gggccgagtc cttgattggt 4440
   ctgcacctgg gcaaggtggc tctgattacc ggtggttcgg cgggcatcgg tggtcaaatc 4500
   ggtcgtctgc tggccttgtc tggcgcgcgt gtgatgctgg ccgctcgcga tcgccataaa 4560
   ttggaacaga tgcaagccat gattcaaagc gaattggcgg aggttggtta taccgatgtg 4620
   gaggaccgtg tgcacatcgc tccgggttgc gatgtgagca gcgaggcgca gctggcagat 4680
   ctggtggaac gtacgctgtc cgcattcggt accgtggatt atttgattaa taacgccggt 4740
   attgcgggcg tggaggagat ggtgatcgac atgccggtgg aaggctggcg tcacaccctg 4800
   tttgccaacc tgatttcgaa ttattcgctg atgcgcaagt tggcgccgct gatgaagaag 4860
   caaggtagcg gttacatcct gaacgtttct tcctattttg gcggtgagaa ggacgcggcg 4920
   attccttatc cgaaccgcgc cgactacgcc gtctccaagg ctggccaacg cgcgatggcg 4980
   gaagtgttcg ctcgtttcct gggtccagag attcagatca atgctattgc cccaggtccg 5040
   gttgaaggcg accgcctgcg tggtaccggt gagcgtccgg gcctgtttgc tcgtcgcgcc 5100
   cgtctgatct tggagaataa acgcctgaac gaattgcacg cggctttgat tgctgcggcc 5160
   cgcaccgatg agcgctcgat gcacgagttg gttgaattgt tgctgccgaa cgacgtggcc 5220
   gcgttggagc agaacccagc ggcccctacc gcgctgcgtg agctggcacg ccgcttccgt 5280
   agcgaaggtg atccggcggc aagctcctcg tccgccttgc tgaatcgctc catcgctgcc 5340
   aagctgttgg ctcgcttgca taacggtggc tatgtgctgc cggcggatat ttttgcaaat 5400
   ctgcctaatc cgccggaccc gttctttacc cgtgcgcaaa ttgaccgcga agctcgcaag 5460
   gtgcgtgatg gtattatggg tatgctgtat ctgcagcgta tgccaaccga gtttgacgtc 5520
   gctatggcaa ccgtgtacta tctggccgat cgtaacgtga gcggcgaaac tttccatccg 5580
   tctggtggtt tgcgctacga gcgtaccccg accggtggcg agctgttcgg cctgccatcg 5640
   ccggaacgtc tggcggagct ggttggtagc acggtgtacc tgatcggtga acacctgacc 5700
   gagcacctga acctgctggc tcgtgcctat ttggagcgct acggtgcccg tcaagtggtg 5760
   atgattgttg agacggaaac cggtgcggaa accatgcgtc gtctgttgca tgatcacgtc 5820
   gaggcaggtc gcctgatgac tattgtggca ggtgatcaga ttgaggcagc gattgaccaa 5880
   gcgatcacgc gctatggccg tccgggtccg gtggtgtgca ctccattccg tccactgcca 5940
   accgttccgc tggtcggtcg taaagactcc gattggagca ccgttttgag cgaggcggaa 6000
   tttgcggaac tgtgtgagca tcagctgacc caccatttcc gtgttgctcg taagatcgcc 6060
   ttgtcggatg gcgcgtcgct ggcgttggtt accccggaaa cgactgcgac tagcaccacg 6120
   gagcaatttg ctctggcgaa cttcatcaag accaccctgc acgcgttcac cgcgaccatc 6180
   ggtgttgagt cggagcgcac cgcgcaacgt attctgatta accaggttga tctgacgcgc 6240
   cgcgcccgtg cggaagagcc gcgtgacccg cacgagcgtc agcaggaatt ggaacgcttc 6300
   attgaagccg ttctgctggt taccgctccg ctgcctcctg aggcagacac gcgctacgca 6360
   ggccgtattc accgcggtcg tgcgattacc gtctaataga agcttggctg ttttggcgga 6420
   tgagagaaga ttttcagcct gatacagatt aaatcagaac gcagaagcgg tctgataaaa 6480
   cagaatttgc ctggcggcag tagcgcggtg gtcccacctg accccatgcc gaactcagaa 6540
   gtgaaacgac gaattctcta gaaagtatag gaacttctga aggggggaca gctgtctcaa 6600
   atgtctcgga atcgctgacg attcccaggt ttctccggca agcatagcgc atggcgtctc 6660
   catgcgagaa tgtcgcgctt gccggataaa aggggagccg ctatcggaat ggacgcaagc 6720
   cacggccgca gcaggtgcgg tcgagggctt ccagccagtt ccagggcaga tgtgccggca     6780
   gaccctcccg ctttggggga ggcgcaagcc gggtccattc ggatagcatc tccccatgca     6840
   aagtgccggc cagggcaatg cccggagccg gttcgaggcg ccggtttgaa taaatgacaa 6900
   aaagcaaagc ctttgtgccg atgaatctct atactgtttc acagacctgc tgccctgcgg 6960
   ggcggccatc ttcctttatt cgcttataag cgcggggcag gaggaaaaac atgtccctga 7020
   acttcctgga cttcgagcag ccgattgcag aactggaagc gaagattgac agcctgaccg 7080
   cggttagccg tcaagatgag aaactggaca ttaacatcga cgaagaggtc caccgtttgc 7140
   gtgagaagtc tgttgaactg actcgcaaaa tctttgctga tttgggcgca tggcagattg 7200
   cccagttggc tcgccaccca caacgcccat ataccctgga ctacgtgcgc ctggcgtttg 7260
   acgagttcga cgaactggca ggcgaccgcg cctatgcgga cgataaagca attgtcggcg 7320
   gtattgctcg tttggatggc cgtccggtga tgattatcgg ccatcaaaaa ggccgcgaga 7380
   cgaaagaaaa gattcgtcgt aactttggta tgccggcacc ggagggctac cgcaaggccc 7440
   tgcgtctgat gcaaatggcc gaacgcttta agatgccgat tatcacgttc attgatacgc 7500
   cgggtgcgta cccaggcgtt ggtgcggaag agcgtggtca gagcgaggcc atcgcacgta 7560
   acctgcgtga gatgtctcgt ctgggtgtgc cggtcgtttg caccgtgatt ggcgagggcg 7620
   gtagcggtgg tgcgttggcg atcggtgtcg gtgataaggt caacatgctg caatacagca 7680
   cgtacagcgt cattagcccg gaaggttgcg cttccattct gtggaagagc gcggataaag 7740
   caccattggc agcggaagcg atgggtatca tcgcaccgcg tctgaaagaa ctgaagttga 7800
   ttgattctat catcccggaa ccgctgggcg gtgctcaccg taatccggag gcgatggcag 7860
   ccagcctgaa ggcccagctg ctggcggacc tggcggatct ggacgtgctg agcacggagg 7920
   atctgaaaaa ccgtcgctat cagcgcttga tgagctatgg ctacgcgtag taaaggagga 7980
   aaatcatgag ctggattgaa cgcattaagt ccaatatcac cccgacccgc aaggcgagca 8040
   tccctgaagg cgtctggacc aaatgcgata gctgcggtca ggttttgtat cgtgcggagc 8100
   tggagcgtaa cctggaagtg tgcccgaaat gcgaccatca catgcgtatg accgctcgta 8160
   atcgtctgca tagcctgctg gatgagggca gcctggtcga gctgggtagc gaactggaac 8220
   cgaaagatgt tctgaaattc cgtgattcca agaagtataa ggatcgtttg gcatctgcac 8280
   aaaaagaaac cggtgagaag gacgcactgg ttgttatgaa aggcaccctg tatggtatgc 8340
   cggttgttgc tgcggcgttc gagtttgcgt ttatgggtgg cagcatgggt tccgtggtgg 8400
   gcgcacgctt tgtgcgtgcc gtggagcagg cgctggagga taactgtcct ctgatttgtt 8460
   tcagcgcgag cggtggtgcg cgtatgcaag aggccctgat gagcctgatg cagatggcaa 8520
   aaacctcggc agccctggcg aagatgcaag aacgcggcct gccgtacatt tccgtcctga 8580
   ccgaccctac gatgggcggt gtcagcgcca gctttgcgat gctgggtgat ttgaacatcg 8640
   cagagccgaa ggctctgatt ggttttgctg gtccgcgtgt tattgaacag acggttcgcg 8700
   aaaagttgcc gcctggtttc cagcgcagcg agttcctgat tgagaaaggt gccatcgaca 8760
   tgatcgttcg ccgtccagaa atgcgtctga aactggcgag cattctggcg aaattgatga 8820
   atctgccggc tccgaatcct gaagcaccgc gtgagggtgt cgtggttccg ccggtcccgg 8880
   accaagagcc ggaggcataa tagcacgtgg agttaaccac gcggcttgcc aacggggtct 8940
   gaatcgcttt ttttgtatat aatgcgtgtg aaatttcata ccacaggcga aggaggaaaa 9000
   accatggaca ttcgtaagat caagaaactg attgaactgg ttgaagaaag cggcatcagc 9060
   gagctggaga tcagcgaagg tgaagagagc gtccgtattt cccgtgcggc accggcagcg 9120
   agctttccgg ttatgcagca agcatacgcc gctccgatga tgcaacagcc ggcacagagc 9180
   aacgccgctg caccggcgac cgttccaagc atggaggcac cggcagcggc cgagatttcg 9240
   ggtcatatcg tgcgtagccc gatggtgggc accttctatc gcacgccgtc gccggacgca 9300
   aaagccttca tcgaagtcgg ccagaaggtc aatgtcggcg acacgctgtg tatcgttgag 9360
   gcaatgaaaa tgatgaacca gattgaagcg gataagagcg gtactgttaa agcgatcctg 9420
   gtggaatccg gccagcctgt tgagttcgat gaaccgctgg ttgtgatcga gtaacgaggc 9480
   gaacatgttg gacaagatcg tgattgcaaa ccgcggtgaa atcgcgctgc gtatcttgcg 9540
   cgcgtgtaaa gagctgggca ttaagactgt tgccgtgcat tccagcgcag accgcgacct 9600
   gaagcatgtt ctgctggccg acgaaacggt ttgcatcggt ccggcaccga gcgtgaaaag 9660
   ctatctgaac atcccggcca tcatctctgc ggcagagatc accggtgcag tggcgattca 9720
   tccgggctac ggtttcctga gcgagaacgc taactttgct gaacaagtgg agcgtagcgg 9780
   tttcatcttc attggcccta aggcggagac gattcgcctg atgggcgaca aagtgagcgc 9840
   cattgcagcg atgaaaaagg ccggtgtgcc gtgtgttccg ggcagcgatg gtccgctggg 9900
   tgacgatatg gacaagaacc gtgccatcgc taaacgtatt ggctacccgg tcattatcaa 9960
   agcctctggt ggtggcggtg gccgtggtat gcgtgtcgtc cgtggtgatg cggaactggc 10020
   gcaaagcatc agcatgaccc gtgcggaagc caaagcggcg ttctctaacg atatggtgta 10080
   tatggagaag tatctggaga atccgcgcca cgttgaaatc caagttctgg cggatggtca 10140
   gggcaatgcg atctacttgg cagaacgtga ttgctccatg caacgccgtc atcagaaggt 10200
   ggtggaagag gcaccggctc cgggtattac gccggaactg cgtcgctaca tcggtgagcg 10260
   ctgtgcgaaa gcgtgtgtgg acattggtta ccgtggtgcg ggtacgtttg agttcctgtt 10320
   cgaaaatggt gagttttact tcattgaaat gaatacccgc atccaggttg agcacccggt 10380
   gaccgagatg attactggcg ttgatctgat caaagagcaa ctgcgcattg cggctggtca 10440
   gccgctgtcg atcaagcaag aagaggtgca cgttcgtggt cacgcggtcg agtgccgtat 10500
   caatgcggag gacccgaata cctttctgcc gagccctggt aagatcacgc gttttcacgc 10560
   gccaggtggt tttggcgttc gttgggagtc tcacatctac gccggttaca ccgtgccgcc 10620
   gtactatgac agcatgattg gtaaactgat ctgctatggc gaaaatcgtg atgtcgcgat 10680
   cgcccgcatg aaaaacgcgc tgcaagagct gatcattgat ggcattaaga ccaatgtgga 10740
   tttgcagatc cgcattatga acgacgagaa tttccagcac ggcggtacga acattcacta 10800
   cctggaaaag aaactgggcc tgcaagagaa ataatagggt accctagcat aaccccttgg 10860
   ggcctctaaa cgggtcttga ggggtttttt gaatcccccg ggatgtctat cttaaggtag 10920
   ccaaagttta aatcatacct gacc 10944
<210> 139
   <211> 1401
   <212> DNA
   <213> Escherichia coli
<400> 139
   atgccacatt cctacgatta cgatgccata gtaataggtt ccggccccgg cggcgaaggc 60
   gctgcaatgg gcctggttaa gcaaggtgcg cgcgtcgcag ttatcgagcg ttatcaaaat 120
   gttggcggcg gttgcaccca ctggggcacc atcccgtcga aagctctccg tcacgccgtc 180
   agccgcatta tagaattcaa tcaaaaccca ctttacagcg accattcccg actgctccgc 240
   tcttcttttg ccgatatcct taaccatgcc gataacgtga ttaatcaaca aacgcgcatg 300
   cgtcagggat tttacgaacg taatcactgt gaaatattgc agggaaacgc tcgctttgtt 360
   gacgagcata cgttggcgct ggattgcccg gacggcagcg ttgaaacact aaccgctgaa 420
   aaatttgtta ttgcctgcgg ctctcgtcca tatcatccaa cagatgttga tttcacccat 480
   ccacgcattt acgacagcga ctcaattctc agcatgcacc acgaaccgcg ccatgtactt 540
   atctatggtg ctggagtgat cggctgtgaa tatgcgtcga tcttccgcgg tatggatgta 600
   aaagtggatc tgatcaacac ccgcgatcgc ctgctggcat ttctcgatca agagatgtca 660
   gattctctct cctatcactt ctggaacagt ggcgtagtga ttcgtcacaa cgaagagtac 720
   gagaagatcg aaggctgtga cgatggtgtg atcatgcatc tgaagtcggg taaaaaactg 780
   aaagctgact gcctgctcta tgccaacggt cgcaccggta ataccgattc gctggcgtta 840
   cagaacattg ggctagaaac tgacagccgc ggacagctga aggtcaacag catgtatcag 900
   accgcacagc cacacgttta cgcggtgggc gacgtgattg gttatccgag cctggcgtcg 960
   gcggcctatg accaggggcg cattgccgcg caggcgctgg taaaaggcga agccaccgca 1020
   catctgattg aagatatccc taccggtatt tacaccatcc cggaaatcag ctctgtgggc 1080
   aaaaccgaac agcagctgac cgcaatgaaa gtgccatatg aagtgggccg cgcccagttt 1140
   aaacatctgg cacgcgcaca aatcgtcggc atgaacgtgg gcacgctgaa aattttgttc 1200
   catcgggaaa caaaagagat tctgggtatt cactgctttg gcgagcgcgc tgccgaaatt 1260
   attcatatcg gtcaggcgat tatggaacag aaaggtggcg gcaacactat tgagtacttc 1320
   gtcaacacca cctttaacta cccgacgatg gcggaagcct atcgggtagc tgcgttaaac 1380
   ggtttaaacc gcctgtttta a 1401
<210> 140
   <211> 8995
   <212> DNA
   <213> Artificial Sequence
<220>
   <223> Description of Artificial Sequence: Synthetic polynucleotide
<400> 140
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatttaaa 2040
   acaggcggtt taaaccgttt aacgcagcta cccgataggc ttccgccatc gtcgggtagt 2100
   taaaggtggt gttgacgaag tactcaatag tgttgccgcc acctttctgt tccataatcg 2160
   cctgaccgat atgaataatt tcggcagcgc gctcgccaaa gcagtgaata cccagaatct 2220
   cttttgtttc ccgatggaac aaaattttca gcgtgcccac gttcatgccg acgatttgtg 2280
   cgcgtgccag atgtttaaac tgggcgcggc ccacttcata tggcactttc attgcggtca 2340
   gctgctgttc ggttttgccc acagagctga tttccgggat ggtgtaaata ccggtaggga 2400
   tatcttcaat cagatgtgcg gtggcttcgc cttttaccag cgcctgcgcg gcaatgcgcc 2460
   cctggtcata ggccgccgac gccaggctcg gataaccaat cacgtcgccc accgcgtaaa 2520
   cgtgtggctg tgcggtctga tacatgctgt tgaccttcag ctgtccgcgg ctgtcagttt 2580
   ctagcccaat gttctgtaac gccagcgaat cggtattacc ggtgcgaccg ttggcataga 2640
   gcaggcagtc agctttcagt tttttacccg acttcagatg catgatcaca ccatcgtcac 2700
   agccttcgat cttctcgtac tcttcgttgt gacgaatcac tacgccactg ttccagaagt 2760
   gataggagag agaatctgac atctcttgat cgagaaatgc cagcaggcga tcgcgggtgt 2820
   tgatcagatc cacttttaca tccataccgc ggaagatcga cgcatattca cagccgatca 2880
   ctccagcacc atagataagt acatggcgcg gttcgtggtg catgctgaga attgagtcgc 2940
   tgtcgtaaat gcgtggatgg gtgaaatcaa catctgttgg atgatatgga cgagagccgc 3000
   aggcaataac aaatttttca gcggttagtg tttcaacgct gccgtccggg caatccagcg 3060
   ccaacgtatg ctcgtcaaca aagcgagcgt ttccctgcaa tatttcacag tgattacgtt 3120
   cgtaaaatcc ctgacgcatg cgcgtttgtt gattaatcac gttatcggca tggttaagga 3180
   tatcggcaaa agaagagcgg agcagtcggg aatggtcgct gtaaagtggg ttttgattga 3240
   attctataat gcggctgacg gcgtgacgga gagctttcga cgggatggtg ccccagtggg 3300
   tgcaaccgcc gccaacattt tgataacgct cgataactgc gacgcgcgca ccttgcttaa 3360
   ccaggcccat tgcagcgcct tcgccgccgg ggccggaacc tattactatg gcatcgtaat 3420
   cgtaggaatg tggggccatg gtagggctta cctgttcttg aatagttctt tcaaactatg 3480
   agaatattat atcaagaatt gtaggattaa gcaactgaaa tttttaagta gttaagatct 3540
   ataacttcgt ataatgtatg ctatacgaag ttatgtttaa atcatacctg acctccatag 3600
   cagaaagtca aaagcctccg accggaggct tttgacttga gggggatcag cttctattat 3660
   ttctcttgca ggcccagttt cttttccagg tagtgaatgt tcgtaccgcc gtgctggaaa 3720
   ttctcgtcgt tcataatgcg gatctgcaaa tccacattgg tcttaatgcc atcaatgatc 3780
   agctcttgca gcgcgttttt catgcgggcg atcgcgacat cacgattttc gccatagcag 3840
   atcagtttac caatcatgct gtcatagtac ggcggcacgg tgtaaccggc gtagatgtga 3900
   gactcccaac gaacgccaaa accacctggc gcgtgaaaac gcgtgatctt accagggctc 3960
   ggcagaaagg tattcgggtc ctccgcattg atacggcact cgaccgcgtg accacgaacg 4020
   tgcacctctt cttgcttgat cgacagcggc tgaccagccg caatgcgcag ttgctctttg 4080
   atcagatcaa cgccagtaat catctcggtc accgggtgct caacctggat gcgggtattc 4140
   atttcaatga agtaaaactc accattttcg aacaggaact caaacgtacc cgcaccacgg 4200
   taaccaatgt ccacacacgc tttcgcacag cgctcaccga tgtagcgacg cagttccggc 4260
   gtaatacccg gagccggtgc ctcttccacc accttctgat gacggcgttg catggagcaa 4320
   tcacgttctg ccaagtagat cgcattgccc tgaccatccg ccagaacttg gatttcaacg 4380
   tggcgcggat tctccagata cttctccata tacaccatat cgttagagaa cgccgctttg 4440
   gcttccgcac gggtcatgct gatgctttgc gccagttccg catcaccacg gacgacacgc 4500
   ataccacggc caccgccacc accagaggct ttgataatga ccgggtagcc aatacgttta 4560
   gcgatggcac ggttcttgtc catatcgtca cccagcggac catcgctgcc cggaacacac 4620
   ggcacaccgg cctttttcat cgctgcaatg gcgctcactt tgtcgcccat caggcgaatc 4680
   gtctccgcct tagggccaat gaagatgaaa ccgctacgct ccacttgttc agcaaagtta 4740
   gcgttctcgc tcaggaaacc gtagcccgga tgaatcgcca ctgcaccggt gatctctgcc 4800
   gcagagatga tggccgggat gttcagatag cttttcacgc tcggtgccgg accgatgcaa 4860
   accgtttcgt cggccagcag aacatgcttc aggtcgcggt ctgcgctgga atgcacggca 4920
   acagtcttaa tgcccagctc tttacacgcg cgcaagatac gcagcgcgat ttcaccgcgg 4980
   tttgcaatca cgatcttgtc caacatgttc gcctcgttac tcgatcacaa ccagcggttc 5040
   atcgaactca acaggctggc cggattccac caggatcgct ttaacagtac cgctcttatc 5100
   cgcttcaatc tggttcatca ttttcattgc ctcaacgata cacagcgtgt cgccgacatt 5160
   gaccttctgg ccgacttcga tgaaggcttt tgcgtccggc gacggcgtgc gatagaaggt 5220
   gcccaccatc gggctacgca cgatatgacc cgaaatctcg gccgctgccg gtgcctccat 5280
   gcttggaacg gtcgccggtg cagcggcgtt gctctgtgcc ggctgttgca tcatcggagc 5340
   ggcgtatgct tgctgcataa ccggaaagct cgctgccggt gccgcacggg aaatacggac 5400
   gctctcttca ccttcgctga tctccagctc gctgatgccg ctttcttcaa ccagttcaat 5460
   cagtttcttg atcttacgaa tgtccatggt ttttcctcct tcgcctgtgg tatgaaattt 5520
   cacacgcatt atatacaaaa aaagcgattc agaccccgtt ggcaagccgc gtggttaact 5580
   ccacgtgcta ttatgcctcc ggctcttggt ccgggaccgg cggaaccacg acaccctcac 5640
   gcggtgcttc aggattcgga gccggcagat tcatcaattt cgccagaatg ctcgccagtt 5700
   tcagacgcat ttctggacgg cgaacgatca tgtcgatggc acctttctca atcaggaact 5760
   cgctgcgctg gaaaccaggc ggcaactttt cgcgaaccgt ctgttcaata acacgcggac 5820
   cagcaaaacc aatcagagcc ttcggctctg cgatgttcaa atcacccagc atcgcaaagc 5880
   tggcgctgac accgcccatc gtagggtcgg tcaggacgga aatgtacggc aggccgcgtt 5940
   cttgcatctt cgccagggct gccgaggttt ttgccatctg catcaggctc atcagggcct 6000
   cttgcatacg cgcaccaccg ctcgcgctga aacaaatcag aggacagtta tcctccagcg 6060
   cctgctccac ggcacgcaca aagcgtgcgc ccaccacgga acccatgctg ccacccataa 6120
   acgcaaactc gaacgccgca gcaacaaccg gcataccata cagggtgcct ttcataacaa 6180
   ccagtgcgtc cttctcaccg gtttcttttt gtgcagatgc caaacgatcc ttatacttct 6240
   tggaatcacg gaatttcaga acatctttcg gttccagttc gctacccagc tcgaccaggc 6300
   tgccctcatc cagcaggcta tgcagacgat tacgagcggt catacgcatg tgatggtcgc 6360
   atttcgggca cacttccagg ttacgctcca gctccgcacg atacaaaacc tgaccgcagc 6420
   tatcgcattt ggtccagacg ccttcaggga tgctcgcctt gcgggtcggg gtgatattgg 6480
   acttaatgcg ttcaatccag ctcatgattt tcctccttta ctacgcgtag ccatagctca 6540
   tcaagcgctg atagcgacgg tttttcagat cctccgtgct cagcacgtcc agatccgcca 6600
   ggtccgccag cagctgggcc ttcaggctgg ctgccatcgc ctccggatta cggtgagcac 6660
   cgcccagcgg ttccgggatg atagaatcaa tcaacttcag ttctttcaga cgcggtgcga 6720
   tgatacccat cgcttccgct gccaatggtg ctttatccgc gctcttccac agaatggaag 6780
   cgcaaccttc cgggctaatg acgctgtacg tgctgtattg cagcatgttg accttatcac 6840
   cgacaccgat cgccaacgca ccaccgctac cgccctcgcc aatcacggtg caaacgaccg 6900
   gcacacccag acgagacatc tcacgcaggt tacgtgcgat ggcctcgctc tgaccacgct 6960
   cttccgcacc aacgcctggg tacgcacccg gcgtatcaat gaacgtgata atcggcatct 7020
   taaagcgttc ggccatttgc atcagacgca gggccttgcg gtagccctcc ggtgccggca 7080
   taccaaagtt acgacgaatc ttttctttcg tctcgcggcc tttttgatgg ccgataatca 7140
   tcaccggacg gccatccaaa cgagcaatac cgccgacaat tgctttatcg tccgcatagg 7200
   cgcggtcgcc tgccagttcg tcgaactcgt caaacgccag gcgcacgtag tccagggtat 7260
   atgggcgttg tgggtggcga gccaactggg caatctgcca tgcgcccaaa tcagcaaaga 7320
   ttttgcgagt cagttcaaca gacttctcac gcaaacggtg gacctcttcg tcgatgttaa 7380
   tgtccagttt ctcatcttga cggctaaccg cggtcaggct gtcaatcttc gcttccagtt 7440
   ctgcaatcgg ctgctcgaag tccaggaagt tcagggacat gtttttcctc ctgccccgcg 7500
   cttataagcg aataaaggaa gatggccgcc ccgcagggca gcaggtctgt gaaacagtat 7560
   agagattcat cggcacaaag gctttgcttt ttgtcattta ttcaaaccgg cgcctcgaac 7620
   cggctccggg cattgccctg gccggcactt tgcatgggga gatgcggatc tcgggcagcg 7680
   ttgggtcctg gccacgggtg cgcatgatcg tgctcctgtc gttgaggacc cggctaggct 7740
   ggcggggttg ccttactggt tagcagaatg aatcaccgat acgcgagcga acgtgaagcg 7800
   actgctgctg caaaacgtct gcgacctgag caacaacatg aatggtcttc ggtttccgtg 7860
   tttcgtaaag tctggaaacg cggaagtccc ctacgtgctg ctgaagttgc ccgcaacaga 7920
   gagtggaacc aaccggtgat accacgatac tatgactgag agtcaacgcc atgagcggcc 7980
   tcatttctta ttctgagtta caacagtccg caccgctgtc cggtagctcc ttccggtggg 8040
   cgcggggcat gactatcgtc gccgcactta tgactgtctt ctttatcatg caactcgtag 8100
   gacaggtgcc ggcagcgccc aacagtcccc cggccacggg gcctgccacc atacccacgc 8160
   cgaaacaagc gccctgcacc attatgttcc ggatctgcat cgcaggatgc tgctggctac 8220
   cctgtggaac acctacatct gtattaacga agcgctaacc gtttttatca ggctctggga 8280
   ggcagaataa atgatcatat cgtcaattat tacctccacg gggagagcct gagcaaactg 8340
   gcctcaggca tttgagaagc acacggtcac actgcttccg gtagtcaata aaccggtaaa 8400
   ccagcaatag acataagcgg ctatttaacg accctgccct gaaccgacga ccgggtcgaa 8460
   tttgctttcg aatttctgcc attcatccgc ttattatcac ttattcaggc gtagcaccag 8520
   gcgtttaagg gcaccaataa ctgccttaaa aaaattacgc cccgccctgc cactcatcgc 8580
   agtactgttg taattcatta agcattctgc cgacatggaa gccatcacag acggcatgat 8640
   gaacctgaat cgccagcggc atcagcacct tgtcgccttg cgtataatat ttgcccatgg 8700
   tgaaaacggg ggcgaagaag ttgtccatat tggccacgtt taaatcaaaa ctggtgaaac 8760
   tcacccaggg attggctgag acgaaaaaca tattctcaat aaacccttta gggaaatagg 8820
   ccaggttttc accgtaacac gccacatctt gcgaatatat gtgtagaaac tgccggaaat 8880
   cgtcgtggta ttcactccag agcgatgaaa acgtttcagt ttgctcatgg aaaacggtgt 8940
   aacaagggtg aacactatcc catatcacca gctcaccgtc tttcattgcc atacg 8995
<210> 141
   <211> 415
   <212> PRT
   <213> Escherichia coli
<400> 141
   Met Ala Leu Asn Ile Pro Phe Arg Asn Ala Tyr Tyr Arg Phe Ala Ser
   1               5                   10                  15
   Ser Tyr Ser Phe Leu Phe Phe Ile Ser Trp Ser Leu Trp Trp Ser Leu
               20                  25                  30
   Tyr Ala Ile Trp Leu Lys Gly His Leu Gly Leu Thr Gly Thr Glu Leu
           35                  40                  45
   Gly Thr Leu Tyr Ser Val Asn Gln Phe Thr Ser Ile Leu Phe Met Met
       50                  55                  60
   Phe Tyr Gly Ile Val Gln Asp Lys Leu Gly Leu Lys Lys Pro Leu Ile
   65                  70                  75                  80
   Trp Cys Met Ser Phe Ile Leu Val Leu Thr Gly Pro Phe Met Ile Tyr
                   85                  90                  95
   Val Tyr Glu Pro Leu Leu Gln Ser Asn Phe Ser Val Gly Leu Ile Leu
               100                 105                 110
   Gly Ala Leu Phe Phe Gly Leu Gly Tyr Leu Ala Gly Cys Gly Leu Leu
           115                 120                 125
   Asp Ser Phe Thr Glu Lys Met Ala Arg Asn Phe His Phe Glu Tyr Gly
       130                 135                 140
   Thr Ala Arg Ala Trp Gly Ser Phe Gly Tyr Ala Ile Gly Ala Phe Phe
   145                 150                 155                 160
   Ala Gly Ile Phe Phe Ser Ile Ser Pro His Ile Asn Phe Trp Leu Val
                   165                 170                 175
   Ser Leu Phe Gly Ala Val Phe Met Met Ile Asn Met Arg Phe Lys Asp
               180                 185                 190
   Lys Asp His Gln Cys Ile Ala Ala Asp Ala Gly Gly Val Lys Lys Glu
           195                 200                 205
   Asp Phe Ile Ala Val Phe Lys Asp Arg Asn Phe Trp Val Phe Val Ile
       210                 215                 220
   Phe Ile Val Gly Thr Trp Ser Phe Tyr Asn Ile Phe Asp Gln Gln Leu
   225                 230                 235                 240
   Phe Pro Val Phe Tyr Ala Gly Leu Phe Glu Ser His Asp Val Gly Thr
                   245                 250                 255
   Arg Leu Tyr Gly Tyr Leu Asn Ser Phe Gln Val Val Leu Glu Ala Leu
   260 265 270
   Cys Met Ala Ile Ile Pro Phe Phe Val Asn Arg Val Gly Pro Lys Asn
   275 280 285
   Ala Leu Leu Ile Gly Val Val Ile Met Ala Leu Arg Ile Leu Ser Cys
   290 295 300
   Ala Leu Phe Val Asn Pro Trp Ile Ile Ser Leu Val Lys Leu Leu His
   305 310 315 320
   Ala Ile Glu Val Pro Leu Cys Val Ile Ser Val Phe Lys Tyr Ser Val
   325 330 335
   Ala Asn Phe Asp Lys Arg Leu Ser Ser Thr Ile Phe Leu Ile Gly Phe
   340 345 350
   Gln Ile Ala Ser Ser Leu Gly Ile Val Leu Leu Ser Thr Pro Thr Gly
   355 360 365
   Ile Leu Phe Asp His Ala Gly Tyr Gln Thr Val Phe Phe Ala Ile Ser
   370 375 380
   Gly Ile Val Cys Leu Met Leu Leu Phe Gly Ile Phe Phe Leu Ser Lys
   385 390 395 400
   Lys Arg Glu Gln Ile Val Met Glu Thr Pro Val Pro Ser Ala Ile
   405 410 415
<210> 142
   <211> 307
   <212> PRT
   <213> Escherichia coli
<400> 142
   Met Ser Ala Lys Val Trp Val Leu Gly Asp Ala Val Val Asp Leu Leu
   1 5 10 15
   Pro Glu Ser Asp Gly Arg Leu Leu Pro Cys Pro Gly Gly Ala Pro Ala
   20 25 30
   Asn Val Ala Val Gly Ile Ala Arg Leu Gly Gly Thr Ser Gly Phe Ile
   35 40 45
   Gly Arg Val Gly Asp Asp Pro Phe Gly Ala Leu Met Gln Arg Thr Leu
   50 55 60
   Leu Thr Glu Gly Val Asp Ile Thr Tyr Leu Lys Gln Asp Glu Trp His
   65                  70                  75                  80
   Arg Thr Ser Thr Val Leu Val Asp Leu Asn Asp Gln Gly Glu Arg Ser
                   85                  90                  95
   Phe Thr Phe Met Val Arg Pro Ser Ala Asp Leu Phe Leu Glu Thr Thr
               100                 105                 110
   Asp Leu Pro Cys Trp Arg His Gly Glu Trp Leu His Leu Cys Ser Ile
           115                 120                 125
   Ala Leu Ser Ala Glu Pro Ser Arg Thr Ser Ala Phe Thr Ala Met Thr
       130                 135                 140
   Ala Ile Arg His Ala Gly Gly Phe Val Ser Phe Asp Pro Asn Ile Arg
   145                 150                 155                 160
   Glu Asp Leu Trp Gln Asp Glu His Leu Leu Arg Leu Cys Leu Arg Gln
                   165                 170                 175
   Ala Leu Gln Leu Ala Asp Val Val Lys Leu Ser Glu Glu Glu Trp Arg
               180                 185                 190
   Leu Ile Ser Gly Lys Thr Gln Asn Asp Arg Asp Ile Cys Ala Leu Ala
           195                 200                 205
   Lys Glu Tyr Glu Ile Ala Met Leu Leu Val Thr Lys Gly Ala Glu Gly
       210                 215                 220
   Val Val Val Cys Tyr Arg Gly Gln Val His His Phe Ala Gly Met Ser
   225                 230                 235                 240
   Val Asn Cys Val Asp Ser Thr Gly Ala Gly Asp Ala Phe Val Ala Gly
                   245                 250                 255
   Leu Leu Thr Gly Leu Ser Ser Thr Gly Leu Ser Thr Asp Glu Arg Glu
               260                 265                 270
   Met Arg Arg Ile Ile Asp Leu Ala Gln Arg Cys Gly Ala Leu Ala Val
           275                 280                 285
   Thr Ala Lys Gly Ala Met Thr Ala Leu Pro Cys Arg Gln Glu Leu Glu
       290                 295                 300
   Ser Glu Lys
   305
<210> 143
   <211> 477
   <212> PRT
   <213> Escherichia coli
<400> 143
   Met Thr Gln Ser Arg Leu His Ala Ala Gln Asn Ala Leu Ala Lys Leu
   1               5                   10                  15
   His Glu Arg Arg Gly Asn Thr Phe Tyr Pro His Phe His Leu Ala Pro
               20                  25                  30
   Pro Ala Gly Trp Met Asn Asp Pro Asn Gly Leu Ile Trp Phe Asn Asp
           35                  40                  45
   Arg Tyr His Ala Phe Tyr Gln His His Pro Met Ser Glu His Trp Gly
       50                  55                  60
   Pro Met His Trp Gly His Ala Thr Ser Asp Asp Met Ile His Trp Gln
   65                  70                  75                  80
   His Glu Pro Ile Ala Leu Ala Pro Gly Asp Glu Asn Asp Lys Asp Gly
                   85                  90                  95
   Cys Phe Ser Gly Ser Ala Val Asp Asp Asn Gly Val Leu Ser Leu Ile
               100                 105                 110
   Tyr Thr Gly His Val Trp Leu Asp Gly Ala Gly Asn Asp Asp Ala Ile
           115                 120                 125
   Arg Glu Val Gln Cys Leu Ala Thr Ser Arg Asp Gly Ile His Phe Glu
       130                 135                 140
   Lys Gln Gly Val Ile Leu Thr Pro Pro Glu Gly Ile Met His Phe Arg
   145                 150                 155                 160
   Asp Pro Lys Val Trp Arg Glu Ala Asp Thr Trp Trp Met Val Val Gly
                   165                 170                 175
   Ala Lys Asp Pro Gly Asn Thr Gly Gln Ile Leu Leu Tyr Arg Gly Ser
               180                 185                 190
   Ser Leu Arg Glu Trp Thr Phe Asp Arg Val Leu Ala His Ala Asp Ala
           195                 200                 205
   Gly Glu Ser Tyr Met Trp Glu Cys Pro Asp Phe Phe Ser Leu Gly Asp
       210                 215                 220
   Gln His Tyr Leu Met Phe Ser Pro Gln Gly Met Asn Ala Glu Gly Tyr
   225 230 235 240
   Ser Tyr Arg Asn Arg Phe Gln Ser Gly Val Ile Pro Gly Met Trp Ser
   245 250 255
   Pro Gly Arg Leu Phe Ala Gln Ser Gly His Phe Thr Glu Leu Asp Asn
   260 265 270
   Gly His Asp Phe Tyr Ala Pro Gln Ser Phe Val Ala Lys Asp Gly Arg
   275 280 285
   Arg Ile Val Ile Gly Trp Met Asp Met Trp Glu Ser Pro Met Pro Ser
   290 295 300
   Lys Arg Glu Gly Trp Ala Gly Cys Met Thr Leu Ala Arg Glu Leu Ser
   305 310 315 320
   Glu Ser Asn Gly Lys Leu Leu Gln Arg Pro Val His Glu Ala Glu Ser
   325 330 335
   Leu Arg Gln Gln His Gln Ser Ile Ser Pro Arg Thr Ile Ser Asn Lys
   340 345 350
   Tyr Val Leu Gln Glu Asn Ala Gln Ala Val Glu Ile Gln Leu Gln Trp
   355 360 365
   Ala Leu Lys Asn Ser Asp Ala Glu His Tyr Gly Leu Gln Leu Gly Ala
   370 375 380
   Gly Met Arg Leu Tyr Ile Asp Asn Gln Ser Glu Arg Leu Val Leu Trp
   385 390 395 400
   Arg Tyr Tyr Pro His Glu Asn Leu Asp Gly Tyr Arg Ser Ile Pro Leu
   405 410 415
   Pro Gln Gly Asp Met Leu Ala Leu Arg Ile Phe Ile Asp Thr Ser Ser
   420 425 430
   Val Glu Val Phe Ile Asn Asp Gly Glu Ala Val Met Ser Ser Arg Ile
   435 440 445
   Tyr Pro Gln Pro Glu Glu Arg Glu Leu Ser Leu Tyr Ala Ser His Gly
   450 455 460
   Val Ala Val Leu Gln His Gly Ala Leu Trp Gln Leu Gly
   465 470 475
<210> 144
   <211> 3847
   <212> DNA
   <213> Artificial Sequence
<220>
   <223> Description of Artificial Sequence: Synthetic polynucleotide
<400> 144
   tcacctccta actacttaaa attgctatca ttcgttattg ttatctagtt gtgcaaaaca      60
   ttgacatccc tatcagtgat agatataatg agcacatgcc caccggcagc tgccggtggg     120
   ccgtattttg gatgataaca ggaggcaaaa ccatggccct gaacatccca ttccgcaacg     180
   catactaccg cttcgcaagc agctacagct tcctgttctt catttcctgg agcttgtggt     240
   ggtcgctgta tgctatctgg ctgaaaggtc atctgggctt gacgggtacg gaactgggta     300
   ccctgtattc cgttaatcaa tttacgtcca tcctgtttat gatgttttac ggcatcgtgc     360
   aggataagct gggcctgaag aaaccgctga tctggtgtat gagcttcatc ctggttctga     420
   ccggtccgtt tatgatttac gtgtacgaac cgctgctgca gtccaacttt agcgtgggcc     480
   tgatcctggg tgcgttgttt ttcggcttgg gttacctggc cggttgtggt ctgctggata     540
   gctttacgga aaagatggcg cgtaactttc actttgaata tggtaccgcg cgtgcgtggg     600
   gttcgttcgg ctacgctatc ggcgcgttct tcgccggcat tttcttcagc attagcccgc     660
   acatcaattt ctggctggtt agcctgttcg gtgcggtctt tatgatgatc aacatgcgtt     720
   ttaaggacaa ggatcaccag tgtattgcag cggatgctgg tggtgttaag aaagaggact     780
   ttattgcagt tttcaaagat cgcaacttct gggtgttcgt gatcttcatt gtcggtacct     840
   ggtcctttta caatatcttc gatcagcaac tgtttccggt tttctatgcg ggcctgttcg     900
   aaagccacga tgttggcacg cgtctgtacg gttatctgaa tagctttcaa gtcgtcctgg     960
   aagctctgtg tatggcgatc attccgttct ttgttaaccg tgtaggcccg aaaaatgcgt    1020
   tgctgattgg cgttgtgatt atggcactgc gtatcctgag ctgcgcactg tttgtgaatc    1080
   cgtggatcat tagcctggtg aaactgctgc acgctattga agtcccgctg tgtgtcatct    1140
   ctgttttcaa gtattccgtc gcgaacttcg acaagcgttt gtccagcacc atcttcctga    1200
   tcggtttcca aattgcaagc agcctgggta ttgttctgtt gagcacgccg actggtattt    1260
   tgtttgatca cgctggttat cagactgtgt tctttgcgat ctcgggtatc gtttgcttga    1320
   tgctgctgtt cggtatcttt ttcctgtcta agaaacgcga gcaaattgtt atggaaaccc    1380
   cggttcctag cgcgatctaa ggaggtaata cacatgtcag cgaaagtatg ggtcctgggc    1440
   gacgcagtag tagatctgtt gccggaaagc gacggtcgtc tgctgccgtg tccgggtggt    1500
   gcaccggcta atgttgcggt gggcatcgcg cgtctgggtg gtacctcggg ttttatcggt    1560
   cgcgtgggtg acgacccttt cggtgcgctg atgcagcgca ctttgctgac cgagggtgtg 1620
   gatatcacgt atctgaagca ggatgagtgg catcgtacct ctaccgtcct ggtggacctg 1680
   aatgaccagg gtgaacgtag cttcaccttc atggtgcgcc cgagcgccga tttgtttctg 1740
   gaaacgaccg atctgccgtg ttggcgtcac ggcgagtggc tgcacttgtg tagcattgca 1800
   ctgtctgcgg aaccgtcgcg cacgagcgcc ttcaccgcga tgacggcaat ccgccatgca 1860
   ggtggctttg tctccttcga cccgaacatt cgtgaggatc tgtggcagga cgagcacctg 1920
   ttgcgcctgt gcctgcgtca agcactgcag ttggccgacg ttgtaaaact gtccgaagaa 1980
   gagtggcgtc tgatctctgg taaaacgcag aatgaccgcg atatttgtgc gctggctaaa 2040
   gagtatgaga tcgccatgtt gctggtgacg aagggtgccg aaggtgttgt tgtgtgctat 2100
   cgtggtcagg tgcaccattt tgcaggcatg tccgtgaatt gcgtggattc tacgggcgca 2160
   ggcgatgcgt tcgtcgcggg tctgttgacc ggtctgagca gcaccggcct gtcgaccgat 2220
   gaacgtgaaa tgcgtcgtat cattgacctg gcgcagcgtt gcggtgcctt ggcggttacc 2280
   gcaaagggtg ccatgaccgc attgccgtgc cgtcaggagc tggaaagcga gaaataagga 2340
   ggtaatactc atgactcaat cccgcctgca cgcagcacaa aacgcactgg caaagctgca 2400
   tgagcgtcgt ggtaacacct tctacccaca cttccacctg gcgccacctg cgggttggat 2460
   gaacgatccg aacggcctga tttggttcaa cgaccgttac catgcgttct atcaacacca 2520
   ccctatgtcc gagcattggg gtccgatgca ttggggccac gctaccagcg atgacatgat 2580
   ccattggcag cacgagccga ttgccctggc accgggtgat gagaacgaca aggatggttg 2640
   cttcagcggt tctgctgtcg atgataacgg tgtcctgtct ctgatctaca ccggtcatgt 2700
   ctggctggat ggcgctggca atgacgacgc aatccgtgaa gttcaatgcc tggcgacctc 2760
   gcgtgacggc attcacttcg agaaacaggg cgtcattttg acgccgcctg agggtatcat 2820
   gcactttcgc gacccaaaag tttggcgtga agcggacacg tggtggatgg tcgtcggcgc 2880
   gaaagacccg ggtaacacgg gtcagattct gctgtatcgt ggcagcagcc tgcgtgagtg 2940
   gaccttcgac cgcgtcctgg cccatgctga tgcgggcgag agctatatgt gggaatgccc 3000
   agacttcttt tctctgggcg accaacatta tctgatgttc agcccgcaag gcatgaatgc 3060
   ggagggctac agctaccgta accgctttca gagcggcgtt atcccgggta tgtggagccc 3120
   aggtcgcctg tttgctcagt ccggtcattt taccgagctg gacaatggtc acgatttcta 3180
   tgcaccgcaa tcgttcgtgg cgaaagatgg ccgtcgcatc gttattggtt ggatggatat 3240
   gtgggaaagc ccgatgccga gcaagcgtga gggttgggcg ggttgcatga ccctggcccg 3300
   tgagctgagc gagagcaatg gcaaattgct gcaacgcccg gtgcacgagg cagaaagcct 3360
   gcgccagcaa caccaaagca tcagccctcg taccattagc aacaaatacg ttctgcagga 3420
   aaatgcgcag gccgttgaga ttcaactgca atgggccctg aaaaacagcg acgcggagca 3480
   ttacggcctg cagctgggtg cgggcatgcg tttgtacatt gataatcaaa gcgagcgttt 3540
   ggttttgtgg cgctactacc cgcatgagaa cctggacggc taccgtagca tcccgctgcc 3600
   gcagggtgac atgctggcgc tgcgtatttt cattgatact agcagcgtcg aggtattcat 3660
   caatgacggc gaagccgtga tgtctagccg tatttacccg cagccggagg aacgcgagct 3720
   gagcttgtat gccagccacg gtgtcgcggt gctgcaacac ggtgcactgt ggcaactggg 3780
   ttaagcttca gtagcgctgt ctggcaacat aaacggcccc ttctgggcaa tgccgatcag 3840
   ttaagga 3847
<210> 145
   <211> 1242
   <212> DNA
   <213> Escherichia coli
<400> 145
   gtgtctaagc gtcgtgtagt tgtgaccgga ctgggcatgt tgtctcctgt cggcaatacc        60
   gtagagtcta cctggaaagc tctgcttgcc ggtcagagtg gcatcagcct aatcgaccat       120
   ttcgatacta gcgcctatgc aacgaaattt gctggcttag taaaggattt taactgtgag       180
   gacattatct cgcgcaaaga acagcgcaag atggatgcct tcattcaata tggaattgtc       240
   gctggcgttc aggccatgca ggattctggc cttgaaataa cggaagagaa cgcaacccgc       300
   attggtgccg caattggctc cgggattggc ggcctcggac tgatcgaaga aaaccacaca       360
   tctctgatga acggtggtcc acgtaagatc agcccattct tcgttccgtc aacgattgtg       420
   aacatggtgg caggtcatct gactatcatg tatggcctgc gtggcccgag catctctatc       480
   gcgactgcct gtacttccgg cgtgcacaac attggccatg ctgcgcgtat tatcgcgtat       540
   ggcgatgctg acgtgatggt tgcaggtggc gcagagaaag ccagtacgcc gctgggcgtt       600
   ggtggttttg gcgcggcacg tgcattatct acccgcaatg ataacccgca agcggcgagc       660
   cgcccgtggg ataaagagcg tgatggtttc gtactgggcg atggtgccgg tatgctggta       720
   cttgaagagt acgaacacgc gaaaaaacgc ggtgcgaaaa tttacgctga actcgtcggc       780
   tttggtatga gcagcgatgc ttatcatatg acgtcaccgc cagaaaatgg cgcaggcgca       840
   gctctggcga tggcaaatgc tctgcgtgat gcaggcattg aagcgagtca gattggctac       900
   gttaacgcgc acggtacttc tacgccggct ggcgataaag ctgaagcgca ggcggtgaaa       960
   accatcttcg gtgaagctgc aagccgtgtg ttggtaagct ccacgaaatc tatgaccggt      1020
   cacctgttag gtgcggcggg tgcagtagaa tctatctact ccatcctggc gctgcgcgat      1080
   caggctgttc cgccaaccat caacctggat aacccggatg aaggttgcga tctggatttc      1140
   gtaccgcacg aagcgcgtca ggttagcgga atggaataca ctctgtgtaa ctccttcggc      1200
   ttcggtggca ctaatggttc tttgatcttt aaaaagatct aa                          1242
<210> 146
   <211> 1221
   <212> DNA
   <213> Artificial Sequence
<220>
   <223> Description of Artificial Sequence: Synthetic polynucleotide
<400> 146
   atgaaacgtg cagtgattac tggcctgggc attgtttcca gcatcggtaa taaccagcag      60
   gaagtcctgg catctctgcg tgaaggacgt tcagggatca ctttctctca ggagctgaag     120
   gattccggca tgcgtagcca cgtctggggc aacgtaaaac tggataccac tggcctcatt     180
   gaccgcaaag ttgtgcgctt tatgagcgac gcatccattt atgcattcct ttctatggag     240
   caggcaatcg ctgatgcggg cctctctccg gaagcttacc agaataaccc gcgcgttggc     300
   ctgattgcag gttccggcgg cggctccccg cgtttccagg tgttcggcgc tgacgcaatg     360
   cgcggcccgc gcggcctgaa agcggttggc ccgtatgtgg tcaccaaagc gatggcatcc     420
   ggcgtttctg cctgcctcgc caccccgttt aaaattcatg gcgttaacta ctccatcagc     480
   tccgcgtgtg cgacttccgc acactgtatc ggtaacgcag tagagcagat ccaactgggc     540
   aaacaggaca tcgtgtttgc tggcggcggc gaagagctgt gctgggaaat ggcttgcgaa     600
   ttcgacgcaa tgggtgcgct gtctactaaa tacaacgaca ccccggaaaa agcctcccgt     660
   acttacgacg ctcaccgtga cggtttcgtt atcgctggcg gcggcggtat ggtagtggtt     720
   gaagagctgg aacacgcgct ggcgcgtggt gctcacatct atgctgaaat cgttggctac     780
   ggcgcaacct ctgatggtgc agacatggtt gctccgtctg gcgaaggcgc agtacgctgc     840
   atgaagatgg cgatgcatgg cgttgatacc ccaatcgatt acctgaactc ccacggtact     900
   tcgactccgg ttggcgacgt gaaagagctg gcagctatcc gtgaagtgtt cggcgataag     960
   agcccggcga tttctgcaac caaagtcatg accggtcact ctctgggcgc tgctggcgta    1020
   caggaagcta tctactctct gctgatgctg gaacacggct ttatcgcccc gagcatcaac    1080
   attgaagagc tggacgagca ggctgcgggt ctgaacatcg tgaccgaaac gaccgatcgc    1140
   gaactgacca ccgttatgtc taacagcttc ggcttcggcg gcaccaacgc cacgctggta    1200
   atgcgcaagc tgaaagatta a                                              1221
<210> 147
   <211> 2503
   <212> DNA
   <213> Artificial Sequence
<220>
   <223> Description of Artificial Sequence: Synthetic polynucleotide
<400> 147
   cttacaggcg gtggctcgat cttagcgatg tgtgtaaggc tgcgcaaatt tctctattaa 60
   atggctgatc ggacttgttc ggcgtacaag tgtacgctat tgtgcattcg aaacttactc 120
   tatgtgcgac ttacagaggt attgaatgaa acgtgcagtg attactggcc tgggcattgt 180
   ttccagcatc ggtaataacc agcaggaagt cctggcatct ctgcgtgaag gacgttcagg 240
   gatcactttc tctcaggagc tgaaggattc cggcatgcgt agccacgtct ggggcaacgt 300
   aaaactggat accactggcc tcattgaccg caaagttgtg cgctttatga gcgacgcatc 360
   catttatgca ttcctttcta tggagcaggc aatcgctgat gcgggcctct ctccggaagc 420
   ttaccagaat aacccgcgcg ttggcctgat tgcaggttcc ggcggcggct ccccgcgttt 480
   ccaggtgttc ggcgctgacg caatgcgcgg cccgcgcggc ctgaaagcgg ttggcccgta 540
   tgtggtcacc aaagcgatgg catccggcgt ttctgcctgc ctcgccaccc cgtttaaaat 600
   tcatggcgtt aactactcca tcagctccgc gtgtgcgact tccgcacact gtatcggtaa 660
   cgcagtagag cagatccaac tgggcaaaca ggacatcgtg tttgctggcg gcggcgaaga 720
   gctgtgctgg gaaatggctt gcgaattcga cgcaatgggt gcgctgtcta ctaaatacaa 780
   cgacaccccg gaaaaagcct cccgtactta cgacgctcac cgtgacggtt tcgttatcgc 840
   tggcggcggc ggtatggtag tggttgaaga gctggaacac gcgctggcgc gtggtgctca 900
   catctatgct gaaatcgttg gctacggcgc aacctctgat ggtgcagaca tggttgctcc 960
   gtctggcgaa ggcgcagtac gctgcatgaa gatggcgatg catggcgttg ataccccaat 1020
   cgattacctg aactcccacg gtacttcgac tccggttggc gacgtgaaag agctggcagc 1080
   tatccgtgaa gtgttcggcg ataagagccc ggcgatttct gcaaccaaag tcatgaccgg 1140
   tcactctctg ggcgctgctg gcgtacagga agctatctac tctctgctga tgctggaaca 1200
   cggctttatc gccccgagca tcaacattga agagctggac gagcaggctg cgggtctgaa 1260
   catcgtgacc gaaacgaccg atcgcgaact gaccaccgtt atgtctaaca gcttcggctt 1320
   cggcggcacc aacgccacgc tggtaatgcg caagctgaaa gattaattcg cagtaggtcg 1380
   gagtagacgc gccagcgtcg catccgacgt tacgcgccaa tgcggcctcc ggcactaacg 1440
   catttaaata ggcctggcgc tagatcagtt taaacataac ttcgtatagc atacattata 1500
   cgaagttatt gttgacaatt aatcatcggc atagtatatc ggcatagtat aatacgacaa 1560
   ggtgaggaac taaaccatgg ccaagccttt gtctcaagaa gaatccaccc tcattgaaag 1620
   agcaacggct acaatcaaca gcatccccat ctctgaagac tacagcgtcg ccagcgcagc 1680
   tctctctagc gacggccgca tcttcactgg tgtcaatgta tatcatttta ctgggggacc 1740
   ttgtgcagaa ctcgtggtgc tgggcactgc tgctgctgcg gcagctggca acctgacttg 1800
   tatcgtcgcg atcggaaatg agaacagggg catcttgagc ccctgcggac ggtgccgaca 1860
   ggtgcttctc gatctgcatc ctgggatcaa agccatagtg aaggacagtg atggacagcc 1920
   gacggcagtt gggattcgtg aattgctgcc ctctggttat gtgtgggagg gctaaataac 1980
   ttcgtatagc atacattata cgaagttata gatctgtcga cgggaacctg atggttccct 2040
   ttttcacatc attgacaatc gccgccagtt ccaggcaaac ttcccgcttt gtcgatttcc 2100
   ttctgaaaag acgtacgcgt taaatcctgc caacgcaccg taaccctgaa accagagaga 2160
   tgagacgggg atactcctcg ccttgcgctg cattctggag taatgcatga ctgctgtaag 2220
   ccaaaccgaa acacgatctt ctgccaattt ttcgctcttc cgcatcgctt ttgcggtttt 2280
   tctcacctac atgaccgtag ggttgccgtt gccggttatc ccgctgtttg ttcatcatga 2340
   actgggctat ggcaatacca tggtcggcat tgccgtcggg attcagtttc tggctacggt 2400
   gctgacgcgt ggttacgccg ggcgactggc cgatcaatat ggtgcaaaac gttcggcgct 2460
   tcagggaatg ttagcttgtg gtctggctgg cggcgcgttg ctg 2503
<210> 148
   <211> 3651
   <212> DNA
   <213> Artificial Sequence
<220>
   <223> Description of Artificial Sequence: Synthetic polynucleotide
<400> 148
   atgagcaaag aaggtaatgc ggccaaaggc cgcctggagg gtaaagtggc cctgatcacc      60
   ggtgcagctg gcaacctggg taatgaaatt agccgcgcgt ttgcccgtga gggtgcgttc     120
   gtggttatga ccggccgcac ggaagaacgt atctctgcgg cccgcgaaca gctgattgcg     180
   gataccggtg tcgccccgga acgtatcgac acggcggtgc tggatggcgg taacccggac     240
   tctattcgtg cagctatggc caaactgcgc aaagaatatg gccgtattga tatcctgatt     300
   aacaatgcag gttcagcagg tccgaaacag ccgctgcata atgttccgct gtcgccgcaa     360
   gaaatggaag catgcggtga taccgaaacg gtgcgcgacg cgatgctgaa catcctgggc     420
   gtgacctgga atatggctcg tattgttgcg ccgatgatgc cggtgggcgg tgcaatggtt     480
   aatatcagca ccatttttag ccacacgcgc tattacggcc gtaccgcgta tgtcgtgccg     540
   aaagcggccc tgaacgcact gagcaatcag ctggcttctg aactgggccc gcgtggtatt     600
   cgcgttaaca ccgtctttcc gggtccgatc gaaagtgatc gtattcgcac ggtgttcgca     660
   gctatggatg aagttcagtc ccaaccgaaa gacaccacgg cgaattactt taccggtcgt     720
   atggcactga cgcgtagcgt caacggcaaa gtggatggta aaccgctgcc gaatccgaaa     780
   gacatcgcgg gtacctgtct gttcctggcc agtgaagaag cggccggcat tgccggtgaa     840
   gaagttgatg tcacccatgg cctgtccgca aaccgcacgt ctgctagtac ctatatgacg     900
   cgcccgtcta tgcgtagtct ggatggcgcg ggtctgaaca tcttcatcgt ttcaggtgaa 960
   aactgggatg acgccctggt cgcagctcac accctgatcg gctcgggtgc aaaagtgcgc 1020
   ctgggcctgg cacgtaacgc tgatgttgca caggctaatg cgcgtctgaa agcgcaaggc 1080
   attggtgaag aactgaccgt tacgcgtttt aatcgcgccg aaccggacgc aatggaagat 1140
   gctctggcgg ccttcagtgg cgacgttgat ggtgcgatca ccggcgccat tatcctgccg 1200
   gtcaaaccga gcggtcattt tacgggctct ctgctggcag ctgatgacga taccgtgacg 1260
   aaattcatgg ataccgaact ggtcggtgcc attgcagtgt cccgttcact ggcacgttac 1320
   tggcacggtc gtgaagattt gcagagcccg ccgcgttgcg tttttatgac caacccgggt 1380
   gacccgctgg gcaattcctt cgcgtcagtg ctgtcggccg gtatcacgca gctgatccgt 1440
   atttggcgcg atgaagaacg cgttcaagca ggcaacggta gtaccgaaca tgcggtctgg 1500
   tccaatcaga ttgtgcgcca caccaacacg gaagatgaaa atacccgttt tgcaagcggc 1560
   catgctacgc gcgtgctgtt ccgtgaacaa cacatcgccg aaattgatct gaaactgccg 1620
   gcaaacatca gtgaagaaac cggttcccgc aaagcaatgg tgggctttgc tgaaaatatt 1680
   accggtctgc atctgggcaa agttgcattc atcacgggcg gtagtgctgg tattggcggt 1740
   caggtggcac gtctgctggc tctggcaggt gccaaagtga tgatggttgc acgtcgcgaa 1800
   tccgaactgg tcgcggcacg tgatcgcatc gtgggtgaac tgcaagacat tggctttgcg 1860
   ggtgttgaac gtcgcgtcaa atatatggcg gacattgatg tcagtgattt tgcctccctg 1920
   gacaaagcgg tggatgccac cctggaagaa ttcggccgta tcgactacct gattaacaat 1980
   gcgggcgttg caggtgctga agacatggtc atcgatatgg aaccggaagc ctggcgcttc 2040
   accctggatg caaacctgat tagcaattat catctgatgc agcgtgttgt cccgctgatg 2100
   aaagaacaag gctctggtta tgtcctgaac gtgagctctt actttggcgg tgaaaaattc 2160
   ctggcggttg cctatccgaa tcgcgccgat tacggtctga gcaaagcggg ccagcgtgcc 2220
   atggtggaag cattttctcc gttcctgggc ccggaagttc aatgcaacgc aattgctccg 2280
   ggtccggtcg acggtgatcg tctgtctggt accggcggta aaccgggcct gtttcagcgt 2340
   cgcgcgaaac tgatcctgga aaacaaacgc ctgaatgctg tgtatagtgc ggttatccat 2400
   gccattcgtg aaggcggtga tgcagctaaa attctgaccc gtctgagtcg caattcgacc 2460
   agcacgctgt cccacgatgc agaagcaccg gaagaactgc gtaaactggc actggatttt 2520
   gctagccagg gcgacggtct gtgtacctgg gatcaatacc tgctgacgga cgcaatggct 2580
   cagcgtctgc tggtgcgtct gcaactgggc ggttttctgc tgggttcaaa cgaatgggcc 2640
   tcgctgagtt cctcagaaca gacctggctg aaactgtcac cgccggacga taaaccgttc 2700
   ctgccggcgg cccaagttga taaagtcgcg aatggcgtcg gtaaaggcgt gatttcgcag 2760
   ctgcatctgg gtgcaatgcc gaccgaagcc gaagttgcgc aagccacggt ctttttcctg 2820
   gcagatcgcg ctgtgtcagg tgaaaccttt atgccgtcgg gcggtctgcg tgttgaacgc 2880
   tcaaacaccg aacgcgaaat gttcggctcg ccgaaacagg aacgtatcga taaaatgaaa 2940
   ggtaaaaccg tctggattat cggcgaacac ctgagtgact atgtggcagc tacgatcgaa 3000
   gaactggtgt ccggttgcgg cgttgcgaaa gtggttctga ttgccaaaga taaaagcggt 3060
   gaaaaagcgg ttcgtgacca gctgccgaat gacctgtcta aagatgcact ggaagtgctg 3120
   atcgctggtg atggcctgga agaagcgatg gacgaagccc tgggccattg gggtaaaccg 3180
   accacggtgc tgtcaatgcc gggcgaaccg ctgccggatc acctgtttga aggcggtaac 3240
   ccgctgtcga ccaaagactt cgcacatatg gtggaagcta atattacccg tcactatcgc 3300
   gttacgcgta aagcgtccct gtacgatggt tgtcaggtcg tgctggttag cccggatgtc 3360
   ccgtacggtt ctgacggtcc gggtgttgca ctggctaact ttgtcaaaac ctctctgcat 3420
   gcgttcaccg ccacggtggc agttgaaaat gaacgcctgg ttcacgatgt cccggtgaac 3480
   cagatcaatc tgacgcgtcg cgtgtcgagc gaagaaccgc gtgacgcaga tgaacatgct 3540
   gaagaactgc gtcgctttac ccgtgcagtg ctgctggttg gtgcaccgct gccggacgca 3600
   caggattcac gttatcgctc gaaaatttac cgcggcacca gcatgacggt g 3651
<210> 149
   <211> 2568
   <212> DNA
   <213> Artificial Sequence
<220>
   <223> Description of Artificial Sequence: Synthetic polynucleotide
<400> 149
   atggctttgc cgttggaccc acgtgacccg gccaccattg atgcggtttt cgattggggc      60
   gcaggcgaga atacgggtgg catccatgcg gcggtcattc tgccggcaac ctcccacgaa     120
   ccggctccgt gcgtgattga agtcgatgac gaacgcgtcc tgaatttcct ggccgatgaa     180
   attaccggca ccatcgttat tgcgagccgt ttggcgcgct attggcaatc ccaacgcctg     240
   accccgggtg cccgtgcccg cggtccgcgt gttatctttc tgagcaacgg tgccgatcaa     300
   aatggtaatg tttacggtcg tattcaatct gcggcgatcg gtcaattgat tcgcgtttgg     360
   cgtcacgagg cggagttgga ctatcaacgt gcatccgccg caggcgatca cgttctgccg     420
   ccggtttggg cgaaccagat tgtccgtttc gctaaccgct ccctggaagg tctggagttc     480
   gcgtgcgcgt ggaccgcaca gctgctgcac agccaacgtc atattaacga aattacgctg     540
   aacattccag ccaatattag cgcgaccacg ggcgcacgtt ccgccagcgt cggctgggcc     600
   gagtccttga ttggtctgca cctgggcaag gtggctctga ttaccggtgg ttcggcgggc     660
   atcggtggtc aaatcggtcg tctgctggcc ttgtctggcg cgcgtgtgat gctggccgct 720
   cgcgatcgcc ataaattgga acagatgcaa gccatgattc aaagcgaatt ggcggaggtt 780
   ggttataccg atgtggagga ccgtgtgcac atcgctccgg gttgcgatgt gagcagcgag 840
   gcgcagctgg cagatctggt ggaacgtacg ctgtccgcat tcggtaccgt ggattatttg 900
   attaataacg ccggtattgc gggcgtggag gagatggtga tcgacatgcc ggtggaaggc 960
   tggcgtcaca ccctgtttgc caacctgatt tcgaattatt cgctgatgcg caagttggcg 1020
   ccgctgatga agaagcaagg tagcggttac atcctgaacg tttcttccta ttttggcggt 1080
   gagaaggacg cggcgattcc ttatccgaac cgcgccgact acgccgtctc caaggctggc 1140
   caacgcgcga tggcggaagt gttcgctcgt ttcctgggtc cagagattca gatcaatgct 1200
   attgccccag gtccggttga aggcgaccgc ctgcgtggta ccggtgagcg tccgggcctg 1260
   tttgctcgtc gcgcccgtct gatcttggag aataaacgcc tgaacgaatt gcacgcggct 1320
   ttgattgctg cggcccgcac cgatgagcgc tcgatgcacg agttggttga attgttgctg 1380
   ccgaacgacg tggccgcgtt ggagcagaac ccagcggccc ctaccgcgct gcgtgagctg 1440
   gcacgccgct tccgtagcga aggtgatccg gcggcaagct cctcgtccgc cttgctgaat 1500
   cgctccatcg ctgccaagct gttggctcgc ttgcataacg gtggctatgt gctgccggcg 1560
   gatatttttg caaatctgcc taatccgccg gacccgttct ttacccgtgc gcaaattgac 1620
   cgcgaagctc gcaaggtgcg tgatggtatt atgggtatgc tgtatctgca gcgtatgcca 1680
   accgagtttg acgtcgctat ggcaaccgtg tactatctgg ccgatcgtaa cgtgagcggc 1740
   gaaactttcc atccgtctgg tggtttgcgc tacgagcgta ccccgaccgg tggcgagctg 1800
   ttcggcctgc catcgccgga acgtctggcg gagctggttg gtagcacggt gtacctgatc 1860
   ggtgaacacc tgaccgagca cctgaacctg ctggctcgtg cctatttgga gcgctacggt 1920
   gcccgtcaag tggtgatgat tgttgagacg gaaaccggtg cggaaaccat gcgtcgtctg 1980
   ttgcatgatc acgtcgaggc aggtcgcctg atgactattg tggcaggtga tcagattgag 2040
   gcagcgattg accaagcgat cacgcgctat ggccgtccgg gtccggtggt gtgcactcca 2100
   ttccgtccac tgccaaccgt tccgctggtc ggtcgtaaag actccgattg gagcaccgtt 2160
   ttgagcgagg cggaatttgc ggaactgtgt gagcatcagc tgacccacca tttccgtgtt 2220
   gctcgtaaga tcgccttgtc ggatggcgcg tcgctggcgt tggttacccc ggaaacgact 2280
   gcgactagca ccacggagca atttgctctg gcgaacttca tcaagaccac cctgcacgcg 2340
   ttcaccgcga ccatcggtgt tgagtcggag cgcaccgcgc aacgtattct gattaaccag 2400
   gttgatctga cgcgccgcgc ccgtgcggaa gagccgcgtg acccgcacga gcgtcagcag 2460
   gaattggaac gcttcattga agccgttctg ctggttaccg ctccgctgcc tcctgaggca 2520
   gacacgcgct acgcaggccg tattcaccgc ggtcgtgcga ttaccgtc 2568
<210> 150
   <211> 2172
   <212> DNA
   <213> Artificial Sequence
<220>
   <223> Description of Artificial Sequence: Synthetic polynucleotide
<400> 150
   atggcaggcg atcacgttct gccgccggtt tgggcgaacc agattgtccg tttcgctaac      60
   cgctccctgg aaggtctgga gttcgcgtgc gcgtggaccg cacagctgct gcacagccaa     120
   cgtcatatta acgaaattac gctgaacatt ccagccaata ttagcgcgac cacgggcgca     180
   cgttccgcca gcgtcggctg ggccgagtcc ttgattggtc tgcacctggg caaggtggct     240
   ctgattaccg gtggttcggc gggcatcggt ggtcaaatcg gtcgtctgct ggccttgtct     300
   ggcgcgcgtg tgatgctggc cgctcgcgat cgccataaat tggaacagat gcaagccatg     360
   attcaaagcg aattggcgga ggttggttat accgatgtgg aggaccgtgt gcacatcgct     420
   ccgggttgcg atgtgagcag cgaggcgcag ctggcagatc tggtggaacg tacgctgtcc     480
   gcattcggta ccgtggatta tttgattaat aacgccggta ttgcgggcgt ggaggagatg     540
   gtgatcgaca tgccggtgga aggctggcgt cacaccctgt ttgccaacct gatttcgaat     600
   tattcgctga tgcgcaagtt ggcgccgctg atgaagaagc aaggtagcgg ttacatcctg     660
   aacgtttctt cctattttgg cggtgagaag gacgcggcga ttccttatcc gaaccgcgcc     720
   gactacgccg tctccaaggc tggccaacgc gcgatggcgg aagtgttcgc tcgtttcctg     780
   ggtccagaga ttcagatcaa tgctattgcc ccaggtccgg ttgaaggcga ccgcctgcgt     840
   ggtaccggtg agcgtccggg cctgtttgct cgtcgcgccc gtctgatctt ggagaataaa     900
   cgcctgaacg aattgcacgc ggctttgatt gctgcggccc gcaccgatga gcgctcgatg     960
   cacgagttgg ttgaattgtt gctgccgaac gacgtggccg cgttggagca gaacccagcg    1020
   gcccctaccg cgctgcgtga gctggcacgc cgcttccgta gcgaaggtga tccggcggca    1080
   agctcctcgt ccgccttgct gaatcgctcc atcgctgcca agctgttggc tcgcttgcat    1140
   aacggtggct atgtgctgcc ggcggatatt tttgcaaatc tgcctaatcc gccggacccg    1200
   ttctttaccc gtgcgcaaat tgaccgcgaa gctcgcaagg tgcgtgatgg tattatgggt    1260
   atgctgtatc tgcagcgtat gccaaccgag tttgacgtcg ctatggcaac cgtgtactat    1320
   ctggccgatc gtaacgtgag cggcgaaact ttccatccgt ctggtggttt gcgctacgag    1380
   cgtaccccga ccggtggcga gctgttcggc ctgccatcgc cggaacgtct ggcggagctg    1440
   gttggtagca cggtgtacct gatcggtgaa cacctgaccg agcacctgaa cctgctggct    1500
   cgtgcctatt tggagcgcta cggtgcccgt caagtggtga tgattgttga gacggaaacc    1560
   ggtgcggaaa ccatgcgtcg tctgttgcat gatcacgtcg aggcaggtcg cctgatgact    1620
   attgtggcag gtgatcagat tgaggcagcg attgaccaag cgatcacgcg ctatggccgt    1680
   ccgggtccgg tggtgtgcac tccattccgt ccactgccaa ccgttccgct ggtcggtcgt    1740
   aaagactccg attggagcac cgttttgagc gaggcggaat ttgcggaact gtgtgagcat    1800
   cagctgaccc accatttccg tgttgctcgt aagatcgcct tgtcggatgg cgcgtcgctg    1860
   gcgttggtta ccccggaaac gactgcgact agcaccacgg agcaatttgc tctggcgaac    1920
   ttcatcaaga ccaccctgca cgcgttcacc gcgaccatcg gtgttgagtc ggagcgcacc    1980
   gcgcaacgta ttctgattaa ccaggttgat ctgacgcgcc gcgcccgtgc ggaagagccg    2040
   cgtgacccgc acgagcgtca gcaggaattg gaacgcttca ttgaagccgt tctgctggtt    2100
   accgctccgc tgcctcctga ggcagacacg cgctacgcag gccgtattca ccgcggtcgt    2160
   gcgattaccg tc                                                        2172
<210> 151
   <211> 657
   <212> DNA
   <213> Escherichia coli
<400> 151
   gtgaaagaga ttattgatgg attccttaaa ttccagcgcg aggcatttcc gaagcgggaa      60
   gccttgttta aacagctggc gacacagcaa agcccgcgca cactttttat ctcctgctcc     120
   gacagccgtc tggtccctga gctggtgacg caacgtgagc ctggcgatct gttcgttatt     180
   cgcaacgcgg gcaatatcgt cccttcctac gggccggaac ccggtggcgt ttctgcttcg     240
   gtggagtatg ccgtcgctgc gcttcgggta tctgacattg tgatttgtgg tcattccaac     300
   tgtggcgcga tgaccgccat tgccagctgt cagtgcatgg accatatgcc tgccgtctcc     360
   cactggctgc gttatgccga ttcagcccgc gtcgttaatg aggcgcgccc gcattccgat     420
   ttaccgtcaa aagctgcggc gatggtacgt gaaaacgtca ttgctcagtt ggctaatttg     480
   caaactcatc catcggtgcg cctggcgctc gaagaggggc ggatcgccct gcacggctgg     540
   gtctacgaca ttgaaagcgg cagcatcgca gcttttgacg gcgcaacccg ccagtttgtg     600
   ccactggccg ctaatcctcg cgtttgtgcc ataccgctac gccaaccgac cgcagcg        657
<210> 152
   <211> 468
   <212> DNA
   <213> Escherichia coli
<400> 152
   atgattcagt cacaaattaa ccgcaatatt cgtcttgatc ttgccgatgc cattttgctc 60
   agcaaagcta aaaaagatct ctcatttgcc gagattgccg acggcaccgg tctggcagaa 120
   gcctttgtaa ccgcggcttt gctgggtcag caggcgcttc ctgccgacgc cgcccgcctg 180
   gtcggggcga agctggatct cgacgaagac tccattctac tgttgcagat gattccactg 240
   cgtggctgca ttgatgaccg tattccaact gacccaacga tgtatcgttt ctatgaaatg 300
   ttgcaggtgt acggtacaac cctgaaagcg ttggttcatg agaaatttgg cgatggcatt 360
   attagcgcga ttaacttcaa actcgacgtt aagaaagtgg cggacccgga aggtggcgaa 420
   cgtgcggtca tcaccttaga tggtaaatat ctgccgacca aaccgttc 468
<210> 153
   <211> 1230
   <212> DNA
   <213> Escherichia coli
<400> 153
   atggcaaagg tatcgctgga gaaagacaag attaagtttc tgctggtaga aggcgtgcac 60
   caaaaggcgc tggaaagcct tcgtgcagct ggttacacca acatcgaatt tcacaaaggc 120
   gcgctggatg atgaacaatt aaaagaatcc atccgcgatg cccacttcat cggcctgcga 180
   tcccgtaccc atctgactga agacgtgatc aacgccgcag aaaaactggt cgctattggc 240
   tgtttctgta tcggaacaaa ccaggttgat ctggatgcgg cggcaaagcg cgggatcccg 300
   gtatttaacg caccgttctc aaatacgcgc tctgttgcgg agctggtgat tggcgaactg 360
   ctgctgctat tgcgcggcgt gccggaagcc aatgctaaag cgcaccgtgg cgtgtggaac 420
   aaactggcgg cgggttcttt tgaagcgcgc ggcaaaaagc tgggtatcat cggctacggt 480
   catattggta cgcaattggg cattctggct gaatcgctgg gaatgtatgt ttacttttat 540
   gatattgaaa ataaactgcc gctgggcaac gccactcagg tacagcatct ttctgacctg 600
   ctgaatatga gcgatgtggt gagtctgcat gtaccagaga atccgtccac caaaaatatg 660
   atgggcgcga aagaaatttc actaatgaag cccggctcgc tgctgattaa tgcttcgcgc 720
   ggtactgtgg tggatattcc ggcgctgtgt gatgcgctgg cgagcaaaca tctggcgggg 780
   gcggcaatcg acgtattccc gacggaaccg gcgaccaata gcgatccatt tacctctccg 840
   ctgtgtgaat tcgacaacgt ccttctgacg ccacacattg gcggttcgac tcaggaagcg 900
   caggagaata tcggcctgga agttgcgggt aaattgatca agtattctga caatggctca 960
   acgctctctg cggtgaactt cccggaagtc tcgctgccac tgcacggtgg gcgtcgtctg 1020
   atgcacatcc acgaaaaccg tccgggcgtg ctaactgcgc tgaacaaaat cttcgccgag 1080
   cagggcgtca acatcgccgc gcaatatctg caaacttccg cccagatggg ttatgtggtt 1140
   attgatattg aagccgacga agacgttgcc gaaaaagcgc tgcaggcaat gaaagctatt 1200
   ccgggtacca ttcgcgcccg tctgctgtac 1230
<210> 154
   <211> 1530
   <212> DNA
   <213> Escherichia coli
<400> 154
   atggcaattg gcataccaag agaacggtta accaatgaaa cccgtgttgc agcaacgcca 60
   aaaacagtgg aacagctgct gaaactgggt tttaccgtcg cggtagagag cggcgcgggt 120
   caactggcaa gttttgacga taaagcgttt gtgcaagcgg gcgctgaaat tgtagaaggg 180
   aatagcgtct ggcagtcaga gatcattctg aaggtcaatg cgccgttaga tgatgaaatt 240
   gcgttactga atcctgggac aacgctggtg agttttatct ggcctgcgca gaatccggaa 300
   ttaatgcaaa aacttgcgga acgtaacgtg accgtgatgg cgatggactc tgtgccgcgt 360
   atctcacgcg cacaatcgct ggacgcacta agctcgatgg cgaacatcgc cggttatcgc 420
   gccattgttg aagcggcaca tgaatttggg cgcttcttta ccgggcaaat tactgcggcc 480
   gggaaagtgc caccggcaaa agtgatggtg attggtgcgg gtgttgcagg tctggccgcc 540
   attggcgcag caaacagtct cggcgcgatt gtgcgtgcat tcgacacccg cccggaagtg 600
   aaagaacaag ttcaaagtat gggcgcggaa ttcctcgagc tggattttaa agaggaagct 660
   ggcagcggcg atggctatgc caaagtgatg tcggacgcgt tcatcaaagc ggaaatggaa 720
   ctctttgccg cccaggcaaa agaggtcgat atcattgtca ccaccgcgct tattccaggc 780
   aaaccagcgc cgaagctaat tacccgtgaa atggttgact ccatgaaggc gggcagtgtg 840
   attgtcgacc tggcagccca aaacggcggc aactgtgaat acaccgtgcc gggtgaaatc 900
   ttcactacgg aaaatggtgt caaagtgatt ggttataccg atcttccggg ccgtctgccg 960
   acgcaatcct cacagcttta cggcacaaac ctcgttaatc tgctgaaact gttgtgcaaa 1020
   gagaaagacg gcaatatcac tgttgatttt gatgatgtgg tgattcgcgg cgtgaccgtg 1080
   atccgtgcgg gcgaaattac ctggccggca ccgccgattc aggtatcagc tcagccgcag 1140
   gcggcacaaa aagcggcacc ggaagtgaaa actgaggaaa aatgtacctg ctcaccgtgg 1200
   cgtaaatacg cgttgatggc gctggcaatc attctttttg gctggatggc aagcgttgcg 1260
   ccgaaagaat tccttgggca cttcaccgtt ttcgcgctgg cctgcgttgt cggttattac 1320
   gtggtgtgga atgtatcgca cgcgctgcat acaccgttga tgtcggtcac caacgcgatt 1380
   tcagggatta ttgttgtcgg agcactgttg cagattggcc agggcggctg ggttagcttc 1440
   cttagtttta tcgcggtgct tatagccagc attaatattt tcggtggctt caccgtgact 1500
   cagcgcatgc tgaaaatgtt ccgcaaaaat 1530
<210> 155
   <211> 1386
   <212> DNA
   <213> Escherichia coli
<400> 155
   atgtctggag gattagttac agctgcatac attgttgccg cgatcctgtt tatcttcagt 60
   ctggccggtc tttcgaaaca tgaaacgtct cgccagggta acaacttcgg tatcgccggg 120
   atggcgattg cgttaatcgc aaccattttt ggaccggata cgggtaatgt tggctggatc 180
   ttgctggcga tggtcattgg tggggcaatt ggtatccgtc tggcgaagaa agttgaaatg 240
   accgaaatgc cagaactggt ggcgatcctg catagcttcg tgggtctggc ggcagtgctg 300
   gttggcttta acagctatct gcatcatgac gcgggaatgg caccgattct ggtcaatatt 360
   cacctgacgg aagtgttcct cggtatcttc atcggggcgg taacgttcac gggttcggtg 420
   gtggcgttcg gcaaactgtg tggcaagatt tcgtctaaac cattgatgct gccaaaccgt 480
   cacaaaatga acctggcggc tctggtcgtt tccttcctgc tgctgattgt atttgttcgc 540
   acggacagcg tcggcctgca agtgctggca ttgctgataa tgaccgcaat tgcgctggta 600
   ttcggctggc atttagtcgc ctccatcggt ggtgcagata tgccagtggt ggtgtcgatg 660
   ctgaactcgt actccggctg ggcggctgcg gctgcgggct ttatgctcag caacgacctg 720
   ctgattgtga ccggtgcgct ggtcggttct tcgggggcta tcctttctta cattatgtgt 780
   gaggcgatga accgttcctt tatcagcgtt attgcgggtg gtttcggcac cgacggctct 840
   tctactggcg atgatcagga agtgggtgag caccgcgaaa tcaccgcaga agagacagcg 900
   gaactgctga aaaactccca ttcagtgatc attactccgg ggtacggcat ggcagtcgcg 960
   caggcgcaat atcctgtcgc tgaaattact gagaaattgc gcgctcgtgg tattaatgtg 1020
   cgtttcggta tccacccggt cgcggggcgt ttgcctggac atatgaacgt attgctggct 1080
   gaagcaaaag taccgtatga catcgtgctg gaaatggacg agatcaatga tgactttgct 1140
   gataccgata ccgtactggt gattggtgct aacgatacgg ttaacccggc ggcgcaggat 1200
   gatccgaaga gtccgattgc tggtatgcct gtgctggaag tgtggaaagc gcagaacgtg 1260
   attgtcttta aacgttcgat gaacactggc tatgctggtg tgcaaaaccc gctgttcttc 1320
   aaggaaaaca cccacatgct gtttggtgac gccaaagcca gcgtggatgc aatcctgaaa 1380
   gctctg 1386
<210> 156
   <211> 1401
   <212> DNA
   <213> Escherichia coli
<400> 156
   atggccccac attcctacga ttacgatgcc atagtaatag gttccggccc cggcggcgaa 60
   ggcgctgcaa tgggcctggt taagcaaggt gcgcgcgtcg cagttatcga gcgttatcaa 120
   aatgttggcg gcggttgcac ccactggggc accatcccgt cgaaagctct ccgtcacgcc 180
   gtcagccgca ttatagaatt caatcaaaac ccactttaca gcgaccattc ccgactgctc 240
   cgctcttctt ttgccgatat ccttaaccat gccgataacg tgattaatca acaaacgcgc 300
   atgcgtcagg gattttacga acgtaatcac tgtgaaatat tgcagggaaa cgctcgcttt 360
   gttgacgagc atacgttggc gctggattgc ccggacggca gcgttgaaac actaaccgct 420
   gaaaaatttg ttattgcctg cggctctcgt ccatatcatc caacagatgt tgatttcacc 480
   catccacgca tttacgacag cgactcaatt ctcagcatgc accacgaacc gcgccatgta 540
   cttatctatg gtgctggagt gatcggctgt gaatatgcgt cgatcttccg cggtatggat 600
   gtaaaagtgg atctgatcaa cacccgcgat cgcctgctgg catttctcga tcaagagatg 660
   tcagattctc tctcctatca cttctggaac agtggcgtag tgattcgtca caacgaagag 720
   tacgagaaga tcgaaggctg tgacgatggt gtgatcatgc atctgaagtc gggtaaaaaa 780
   ctgaaagctg actgcctgct ctatgccaac ggtcgcaccg gtaataccga ttcgctggcg 840
   ttacagaaca ttgggctaga aactgacagc cgcggacagc tgaaggtcaa cagcatgtat 900
   cagaccgcac agccacacgt ttacgcggtg ggcgacgtga ttggttatcc gagcctggcg 960
   tcggcggcct atgaccaggg gcgcattgcc gcgcaggcgc tggtaaaagg cgaagccacc 1020
   gcacatctga ttgaagatat ccctaccggt atttacacca tcccggaaat cagctctgtg 1080
   ggcaaaaccg aacagcagct gaccgcaatg aaagtgccat atgaagtggg ccgcgcccag 1140
   tttaaacatc tggcacgcgc acaaatcgtc ggcatgaacg tgggcacgct gaaaattttg 1200
   ttccatcggg aaacaaaaga gattctgggt attcactgct ttggcgagcg cgctgccgaa 1260
   attattcata tcggtcaggc gattatggaa cagaaaggtg gcggcaacac tattgagtac 1320
   ttcgtcaaca ccacctttaa ctacccgacg atggcggaag cctatcgggt agctgcgtta 1380
   aacggtttaa accgcctgtt t 1401
<210> 157
   <211> 1242
   <212> DNA
   <213> Escherichia coli
<400> 157
   atgggatcta agcgtcgtgt agttgtgacc ggactgggca tgttgtctcc tgtcggcaat 60
   accgtagagt ctacctggaa agctctgctt gccggtcaga gtggcatcag cctaatcgac 120
   catttcgata ctagcgccta tgcaacgaaa tttgctggct tagtaaagga ttttaactgt 180
   gaggacatta tctcgcgcaa agaacagcgc aagatggatg ccttcattca atatggaatt 240
   gtcgctggcg ttcaggccat gcaggattct ggccttgaaa taacggaaga gaacgcaacc 300
   cgcattggtg ccgcaattgg ctccgggatt ggcggcctcg gactgatcga agaaaaccac 360
   acatctctga tgaacggtgg tccacgtaag atcagcccat tcttcgttcc gtcaacgatt 420
   gtgaacatgg tggcaggtca tctgactatc atgtatggcc tgcgtggccc gagcatctct 480
   atcgcgactg cctgtacttc cggcgtgcac aacattggcc atgctgcgcg tattatcgcg 540
   tatggcgatg ctgacgtgat ggttgcaggt ggcgcagaga aagccagtac gccgctgggc 600
   gttggtggtt ttggcgcggc acgtgcatta tctacccgca atgataaccc gcaagcggcg 660
   agccgcccgt gggataaaga gcgtgatggt ttcgtactgg gcgatggtgc cggtatgctg 720
   gtacttgaag agtacgaaca cgcgaaaaaa cgcggtgcga aaatttacgc tgaactcgtc 780
   ggctttggta tgagcagcga tgcttatcat atgacgtcac cgccagaaaa tggcgcaggc 840
   gcagctctgg cgatggcaaa tgctctgcgt gatgcaggca ttgaagcgag tcagattggc 900
   tacgttaacg cgcacggtac ttctacgccg gctggcgata aagctgaagc gcaggcggtg 960
   aaaaccatct tcggtgaagc tgcaagccgt gtgttggtaa gctccacgaa atctatgacc 1020
   ggtcacctgt taggtgcggc gggtgcagta gaatctatct actccatcct ggcgctgcgc 1080
   gatcaggctg ttccgccaac catcaacctg gataacccgg atgaaggttg cgatctggat 1140
   ttcgtaccgc acgaagcgcg tcaggttagc ggaatggaat acactctgtg taactccttc 1200
   ggcttcggtg gcactaatgg ttctttgatc tttaaaaaga tc 1242
<210> 158
   <211> 7999
   <212> DNA
   <213> Artificial Sequence
<220>
   <223> Description of Artificial Sequence: Synthetic polynucleotide
<400> 158
   gtttgacagc ttatcatcga ctgcacggtg caccaatgct tctggcgtca ggcagccatc 60
   ggaagctgtg gtatggctgt gcaggtcgta aatcactgca taattcgtgt cgctcaaggc 120
   gcactcccgt tctggataat gttttttgcg ccgacatcat aacggttctg gcaaatattc 180
   tgaaatgagc tgttgacaat taatcatccg gctcgtataa tgtgtggaat tgtgagcgga 240
   taacaatttc acacaggaaa cagcgccgct gagaaaaagc gaagcggcac tgctctttaa 300
   caatttatca gacaatctgt gtgggcactc gaccggaatt atcgattaac tttattatta 360
   aaaattaaag aggtatatat taatgtatcg attaaataag gaggaataaa ccatgagcaa 420
   agaaggtaat gcggccaaag gccgcctgga gggtaaagtg gccctgatca ccggtgcagc 480
   tggcaacctg ggtaatgaaa ttagccgcgc gtttgcccgt gagggtgcgt tcgtggttat 540
   gaccggccgc acggaagaac gtatctctgc ggcccgcgaa cagctgattg cggataccgg 600
   tgtcgccccg gaacgtatcg acacggcggt gctggatggc ggtaacccgg actctattcg 660
   tgcagctatg gccaaactgc gcaaagaata tggccgtatt gatatcctga ttaacaatgc 720
   aggttcagca ggtccgaaac agccgctgca taatgttccg ctgtcgccgc aagaaatgga 780
   agcatgcggt gataccgaaa cggtgcgcga cgcgatgctg aacatcctgg gcgtgacctg 840
   gaatatggct cgtattgttg cgccgatgat gccggtgggc ggtgcaatgg ttaatatcag 900
   caccattttt agccacacgc gctattacgg ccgtaccgcg tatgtcgtgc cgaaagcggc 960
   cctgaacgca ctgagcaatc agctggcttc tgaactgggc ccgcgtggta ttcgcgttaa 1020
   caccgtcttt ccgggtccga tcgaaagtga tcgtattcgc acggtgttcg cagctatgga 1080
   tgaagttcag tcccaaccga aagacaccac ggcgaattac tttaccggtc gtatggcact 1140
   gacgcgtagc gtcaacggca aagtggatgg taaaccgctg ccgaatccga aagacatcgc 1200
   gggtacctgt ctgttcctgg ccagtgaaga agcggccggc attgccggtg aagaagttga 1260
   tgtcacccat ggcctgtccg caaaccgcac gtctgctagt acctatatga cgcgcccgtc 1320
   tatgcgtagt ctggatggcg cgggtctgaa catcttcatc gtttcaggtg aaaactggga 1380
   tgacgccctg gtcgcagctc acaccctgat cggctcgggt gcaaaagtgc gcctgggcct 1440
   ggcacgtaac gctgatgttg cacaggctaa tgcgcgtctg aaagcgcaag gcattggtga 1500
   agaactgacc gttacgcgtt ttaatcgcgc cgaaccggac gcaatggaag atgctctggc 1560
   ggccttcagt ggcgacgttg atggtgcgat caccggcgcc attatcctgc cggtcaaacc 1620
   gagcggtcat tttacgggct ctctgctggc agctgatgac gataccgtga cgaaattcat 1680
   ggataccgaa ctggtcggtg ccattgcagt gtcccgttca ctggcacgtt actggcacgg 1740
   tcgtgaagat ttgcagagcc cgccgcgttg cgtttttatg accaacccgg gtgacccgct 1800
   gggcaattcc ttcgcgtcag tgctgtcggc cggtatcacg cagctgatcc gtatttggcg 1860
   cgatgaagaa cgcgttcaag caggcaacgg tagtaccgaa catgcggtct ggtccaatca 1920
   gattgtgcgc cacaccaaca cggaagatga aaatacccgt tttgcaagcg gccatgctac 1980
   gcgcgtgctg ttccgtgaac aacacatcgc cgaaattgat ctgaaactgc cggcaaacat 2040
   cagtgaagaa accggttccc gcaaagcaat ggtgggcttt gctgaaaata ttaccggtct 2100
   gcatctgggc aaagttgcat tcatcacggg cggtagtgct ggtattggcg gtcaggtggc 2160
   acgtctgctg gctctggcag gtgccaaagt gatgatggtt gcacgtcgcg aatccgaact 2220
   ggtcgcggca cgtgatcgca tcgtgggtga actgcaagac attggctttg cgggtgttga 2280
   acgtcgcgtc aaatatatgg cggacattga tgtcagtgat tttgcctccc tggacaaagc 2340
   ggtggatgcc accctggaag aattcggccg tatcgactac ctgattaaca atgcgggcgt 2400
   tgcaggtgct gaagacatgg tcatcgatat ggaaccggaa gcctggcgct tcaccctgga 2460
   tgcaaacctg attagcaatt atcatctgat gcagcgtgtt gtcccgctga tgaaagaaca 2520
   aggctctggt tatgtcctga acgtgagctc ttactttggc ggtgaaaaat tcctggcggt 2580
   tgcctatccg aatcgcgccg attacggtct gagcaaagcg ggccagcgtg ccatggtgga 2640
   agcattttct ccgttcctgg gcccggaagt tcaatgcaac gcaattgctc cgggtccggt 2700
   cgacggtgat cgtctgtctg gtaccggcgg taaaccgggc ctgtttcagc gtcgcgcgaa 2760
   actgatcctg gaaaacaaac gcctgaatgc tgtgtatagt gcggttatcc atgccattcg 2820
   tgaaggcggt gatgcagcta aaattctgac ccgtctgagt cgcaattcga ccagcacgct 2880
   gtcccacgat gcagaagcac cggaagaact gcgtaaactg gcactggatt ttgctagcca 2940
   gggcgacggt ctgtgtacct gggatcaata cctgctgacg gacgcaatgg ctcagcgtct 3000
   gctggtgcgt ctgcaactgg gcggttttct gctgggttca aacgaatggg cctcgctgag 3060
   ttcctcagaa cagacctggc tgaaactgtc accgccggac gataaaccgt tcctgccggc 3120
   ggcccaagtt gataaagtcg cgaatggcgt cggtaaaggc gtgatttcgc agctgcatct 3180
   gggtgcaatg ccgaccgaag ccgaagttgc gcaagccacg gtctttttcc tggcagatcg 3240
   cgctgtgtca ggtgaaacct ttatgccgtc gggcggtctg cgtgttgaac gctcaaacac 3300
   cgaacgcgaa atgttcggct cgccgaaaca ggaacgtatc gataaaatga aaggtaaaac 3360
   cgtctggatt atcggcgaac acctgagtga ctatgtggca gctacgatcg aagaactggt 3420
   gtccggttgc ggcgttgcga aagtggttct gattgccaaa gataaaagcg gtgaaaaagc 3480
   ggttcgtgac cagctgccga atgacctgtc taaagatgca ctggaagtgc tgatcgctgg 3540
   tgatggcctg gaagaagcga tggacgaagc cctgggccat tggggtaaac cgaccacggt 3600
   gctgtcaatg ccgggcgaac cgctgccgga tcacctgttt gaaggcggta acccgctgtc 3660
   gaccaaagac ttcgcacata tggtggaagc taatattacc cgtcactatc gcgttacgcg 3720
   taaagcgtcc ctgtacgatg gttgtcaggt cgtgctggtt agcccggatg tcccgtacgg 3780
   ttctgacggt ccgggtgttg cactggctaa ctttgtcaaa acctctctgc atgcgttcac 3840
   cgccacggtg gcagttgaaa atgaacgcct ggttcacgat gtcccggtga accagatcaa 3900
   tctgacgcgt cgcgtgtcga gcgaagaacc gcgtgacgca gatgaacatg ctgaagaact 3960
   gcgtcgcttt acccgtgcag tgctgctggt tggtgcaccg ctgccggacg cacaggattc 4020
   acgttatcgc tcgaaaattt accgcggcac cagcatgacg gtgtaataac cgcggcacga 4080
   gcatgacggt ccatcaccat catcactaat agagatctgc agctggtacc atatgggaat 4140
   tcgaagcttg gctgttttgg cggatgagag aagattttca gcctgataca gattaaatca 4200
   gaacgcagaa gcggtctgat aaaacagaat ttgcctggcg gcagtagcgc ggtggtccca 4260
   cctgacccca tgccgaactc agaagtgaaa cgccgtagcg ccgatggtag tgtggggtct 4320
   ccccatgcga gagtagggaa ctgccaggca tcaaataaaa cgaaaggctc agtcgaaaga 4380
   ctgggccttt cgttttatct gttgtttgtc ggtgaacgct ctcctgagta ggacaaatcc 4440
   gccgggagcg gatttgaacg ttgcgaagca acggcccgga gggtggcggg caggacgccc 4500
   gccataaact gccaggcatc aaattaagca gaaggccatc ctgacggatg gcctttttgc 4560
   gtttctacaa actctttttg tttatttttc taaatacatt caaatatgta tccgctcatg 4620
   agacaataac cctgataaat gcttcaataa tattgaaaaa ggaagagtat gagtattcaa 4680
   catttccgtg tcgcccttat tccctttttt gcggcatttt gccttcctgt ttttgctcac 4740
   ccagaaacgc tggtgaaagt aaaagatgct gaagatcagt tgggtgcacg agtgggttac 4800
   atcgaactgg atctcaacag cggtaagatc cttgagagtt ttcgccccga agaacgtttt 4860
   ccaatgatga gcacttttaa agttctgcta tgtggcgcgg tattatcccg tgttgacgcc 4920
   gggcaagagc aactcggtcg ccgcatacac tattctcaga atgacttggt tgagtactca 4980
   ccagtcacag aaaagcatct tacggatggc atgacagtaa gagaattatg cagtgctgcc 5040
   ataaccatga gtgataacac tgcggccaac ttacttctga caacgatcgg aggaccgaag 5100
   gagctaaccg cttttttgca caacatgggg gatcatgtaa ctcgccttga tcgttgggaa 5160
   ccggagctga atgaagccat accaaacgac gagcgtgaca ccacgatgcc tgtagcaatg 5220
   gcaacaacgt tgcgcaaact attaactggc gaactactta ctctagcttc ccggcaacaa 5280
   ttaatagact ggatggaggc ggataaagtt gcaggaccac ttctgcgctc ggcccttccg 5340
   gctggctggt ttattgctga taaatctgga gccggtgagc gtgggtctcg cggtatcatt 5400
   gcagcactgg ggccagatgg taagccctcc cgtatcgtag ttatctacac gacggggagt 5460
   caggcaacta tggatgaacg aaatagacag atcgctgaga taggtgcctc actgattaag 5520
   cattggtaac tgtcagacca agtttactca tatatacttt agattgattt aaaacttcat 5580
   ttttaattta aaaggatcta ggtgaagatc ctttttgata atctcatgac caaaatccct 5640
   taacgtgagt tttcgttcca ctgagcgtca gaccccgtag aaaagatcaa aggatcttct 5700
   tgagatcctt tttttctgcg cgtaatctgc tgcttgcaaa caaaaaaacc accgctacca 5760
   gcggtggttt gtttgccgga tcaagagcta ccaactcttt ttccgaaggt aactggcttc 5820
   agcagagcgc agataccaaa tactgtcctt ctagtgtagc cgtagttagg ccaccacttc 5880
   aagaactctg tagcaccgcc tacatacctc gctctgctaa tcctgttacc agtggctgct 5940
   gccagtggcg ataagtcgtg tcttaccggg ttggactcaa gacgatagtt accggataag 6000
   gcgcagcggt cgggctgaac ggggggttcg tgcacacagc ccagcttgga gcgaacgacc 6060
   tacaccgaac tgagatacct acagcgtgag ctatgagaaa gcgccacgct tcccgaaggg 6120
   agaaaggcgg acaggtatcc ggtaagcggc agggtcggaa caggagagcg cacgagggag 6180
   cttccagggg gaaacgcctg gtatctttat agtcctgtcg ggtttcgcca cctctgactt 6240
   gagcgtcgat ttttgtgatg ctcgtcaggg gggcggagcc tatggaaaaa cgccagcaac 6300
   gcggcctttt tacggttcct ggccttttgc tggccttttg ctcacatgtt ctttcctgcg 6360
   ttatcccctg attctgtgga taaccgtatt accgcctttg agtgagctga taccgctcgc 6420
   cgcagccgaa cgaccgagcg cagcgagtca gtgagcgagg aagcggaaga gcgcctgatg 6480
   cggtattttc tccttacgca tctgtgcggt atttcacacc gcatatggtg cactctcagt 6540
   acaatctgct ctgatgccgc atagttaagc cagtatacac tccgctatcg ctacgtgact 6600
   gggtcatggc tgcgccccga cacccgccaa cacccgctga cgcgccctga cgggcttgtc 6660
   tgctcccggc atccgcttac agacaagctg tgaccgtctc cgggagctgc atgtgtcaga 6720
   ggttttcacc gtcatcaccg aaacgcgcga ggcagcagat caattcgcgc gcgaaggcga 6780
   agcggcatgc atttacgttg acaccatcga atggtgcaaa acctttcgcg gtatggcatg 6840
   atagcgcccg gaagagagtc aattcagggt ggtgaatgtg aaaccagtaa cgttatacga 6900
   tgtcgcagag tatgccggtg tctcttatca gaccgtttcc cgcgtggtga accaggccag 6960
   ccacgtttct gcgaaaacgc gggaaaaagt ggaagcggcg atggcggagc tgaattacat 7020
   tcccaaccgc gtggcacaac aactggcggg caaacagtcg ttgctgattg gcgttgccac 7080
   ctccagtctg gccctgcacg cgccgtcgca aattgtcgcg gcgattaaat ctcgcgccga 7140
   tcaactgggt gccagcgtgg tggtgtcgat ggtagaacga agcggcgtcg aagcctgtaa 7200
   agcggcggtg cacaatcttc tcgcgcaacg cgtcagtggg ctgatcatta actatccgct 7260
   ggatgaccag gatgccattg ctgtggaagc tgcctgcact aatgttccgg cgttatttct 7320
   tgatgtctct gaccagacac ccatcaacag tattattttc tcccatgaag acggtacgcg 7380
   actgggcgtg gagcatctgg tcgcattggg tcaccagcaa atcgcgctgt tagcgggccc 7440
   attaagttct gtctcggcgc gtctgcgtct ggctggctgg cataaatatc tcactcgcaa 7500
   tcaaattcag ccgatagcgg aacgggaagg cgactggagt gccatgtccg gttttcaaca 7560
   aaccatgcaa atgctgaatg agggcatcgt tcccactgcg atgctggttg ccaacgatca 7620
   gatggcgctg ggcgcaatgc gcgccattac cgagtccggg ctgcgcgttg gtgcggatat 7680
   ctcggtagtg ggatacgacg ataccgaaga cagctcatgt tatatcccgc cgtcaaccac 7740
   catcaaacag gattttcgcc tgctggggca aaccagcgtg gaccgcttgc tgcaactctc 7800
   tcagggccag gcggtgaagg gcaatcagct gttgcccgtc tcactggtga aaagaaaaac 7860
   caccctggcg cccaatacgc aaaccgcctc tccccgcgcg ttggccgatt cattaatgca 7920
   gctggcacga caggtttccc gactggaaag cgggcagtga gcgcaacgca attaatgtga 7980
   gttagcgcga attgatctg 7999
<210> 159
   <211> 329
   <212> PRT
   <213> Streptomyces sp.
<220>
   <223> Streptomyces sp. NphT7
<400> 159
   Met Thr Asp Val Arg Phe Arg Ile Ile Gly Thr Gly Ala Tyr Val Pro
   1               5                   10                  15
   Glu Arg Ile Val Ser Asn Asp Glu Val Gly Ala Pro Ala Gly Val Asp
               20                  25                  30
   Asp Asp Trp Ile Thr Arg Lys Thr Gly Ile Arg Gln Arg Arg Trp Ala
           35                  40                  45
   Ala Asp Asp Gln Ala Thr Ser Asp Leu Ala Thr Ala Ala Gly Arg Ala
       50                  55                  60
   Ala Leu Lys Ala Ala Gly Ile Thr Pro Glu Gln Leu Thr Val Ile Ala
   65                  70                  75                  80
   Val Ala Thr Ser Thr Pro Asp Arg Pro Gln Pro Pro Thr Ala Ala Tyr
                   85                  90                  95
   Val Gln His His Leu Gly Ala Thr Gly Thr Ala Ala Phe Asp Val Asn
               100                 105                 110
   Ala Val Cys Ser Gly Thr Val Phe Ala Leu Ser Ser Val Ala Gly Thr
           115                 120                 125
   Leu Val Tyr Arg Gly Gly Tyr Ala Leu Val Ile Gly Ala Asp Leu Tyr
       130                 135                 140
   Ser Arg Ile Leu Asn Pro Ala Asp Arg Lys Thr Val Val Leu Phe Gly
   145                 150                 155                 160
   Asp Gly Ala Gly Ala Met Val Leu Gly Pro Thr Ser Thr Gly Thr Gly
                   165                 170                 175
   Pro Ile Val Arg Arg Val Ala Leu His Thr Phe Gly Gly Leu Thr Asp
               180                 185                 190
   Leu Ile Arg Val Pro Ala Gly Gly Ser Arg Gln Pro Leu Asp Thr Asp
           195                 200                 205
   Gly Leu Asp Ala Gly Leu Gln Tyr Phe Ala Met Asp Gly Arg Glu Val
       210                 215                 220
   Arg Arg Phe Val Thr Glu His Leu Pro Gln Leu Ile Lys Gly Phe Leu
   225                 230                 235                 240
   His Glu Ala Gly Val Asp Ala Ala Asp Ile Ser His Phe Val Pro His
                   245                 250                 255
   Gln Ala Asn Gly Val Met Leu Asp Glu Val Phe Gly Glu Leu His Leu
               260                 265                 270
   Pro Arg Ala Thr Met His Arg Thr Val Glu Thr Tyr Gly Asn Thr Gly
           275                 280                 285
   Ala Ala Ser Ile Pro Ile Thr Met Asp Ala Ala Val Arg Ala Gly Ser
       290                 295                 300
   Phe Arg Pro Gly Glu Leu Val Leu Leu Ala Gly Phe Gly Gly Gly Met
   305                 310                 315                 320
   Ala Ala Ser Phe Ala Leu Ile Glu Trp
                   325
<210> 160
   <211> 2137
   <212> DNA
   <213> Streptomyces sp.
<220>
   <223> Streptomyces sp. NphT7
<400> 160
   cctgcaggcc gtcgagggcg cctggaagga ctacgcggag caggacggcc ggtcgctgga      60
   ggagttcgcg gcgttcgtct accaccagcc gttcacgaag atggcctaca aggcgcaccg     120
   ccacctgctg aacttcaacg gctacgacac cgacaaggac gccatcgagg gcgccctcgg     180
   ccagacgacg gcgtacaaca acgtcatcgg caacagctac accgcgtcgg tgtacctggg     240
   cctggccgcc ctgctcgacc aggcggacga cctgacgggc cgttccatcg gcttcctgag     300
   ctacggctcg ggcagcgtcg ccgagttctt ctcgggcacc gtcgtcgccg ggtaccgcga     360
   gcgtctgcgc accgaggcga accaggaggc gatcgcccgg cgcaagagcg tcgactacgc     420
   cacctaccgc gagctgcacg agtacacgct cccgtccgac ggcggcgacc acgccacccc     480
   ggtgcagacc accggcccct tccggctggc cgggatcaac gaccacaagc gcatctacga     540
   ggcgcgctag cgacacccct cggcaacggg gtgcgccact gttcggcgca ccccgtgccg     600
   ggctttcgca cagctattca cgaccatttg aggggcgggc agccgcatga ccgacgtccg     660
   attccgcatt atcggtacgg gtgcctacgt accggaacgg atcgtctcca acgatgaagt     720
   cggcgcgccg gccggggtgg acgacgactg gatcacccgc aagaccggta tccggcagcg     780
   tcgctgggcc gccgacgacc aggccacctc ggacctggcc acggccgcgg ggcgggcagc     840
   gctgaaagcg gcgggcatca cgcccgagca gctgaccgtg atcgcggtcg ccacctccac     900
   gccggaccgg ccgcagccgc ccacggcggc ctatgtccag caccacctcg gtgcgaccgg     960
   cactgcggcg ttcgacgtca acgcggtctg ctccggcacc gtgttcgcgc tgtcctcggt 1020
   ggcgggcacc ctcgtgtacc ggggcggtta cgcgctggtc atcggcgcgg acctgtactc 1080
   gcgcatcctc aacccggccg accgcaagac ggtcgtgctg ttcggggacg gcgccggcgc 1140
   aatggtcctc gggccgacct cgaccggcac gggccccatc gtccggcgcg tcgccctgca 1200
   caccttcggc ggcctcaccg acctgatccg tgtgcccgcg ggcggcagcc gccagccgct 1260
   ggacacggat ggcctcgacg cgggactgca gtacttcgcg atggacgggc gtgaggtgcg 1320
   ccgcttcgtc acggagcacc tgccgcagct gatcaagggc ttcctgcacg aggccggggt 1380
   cgacgccgcc gacatcagcc acttcgtgcc gcatcaggcc aacggtgtca tgctcgacga 1440
   ggtcttcggc gagctgcatc tgccgcgggc gaccatgcac cggacggtcg agacctacgg 1500
   caacacggga gcggcctcca tcccgatcac catggacgcg gccgtgcgcg ccggttcctt 1560
   ccggccgggc gagctggtcc tgctggccgg gttcggcggc ggcatggccg cgagcttcgc 1620
   cctgatcgag tggtagtcgc ccgtaccacc acagcggtcc ggcgccacct gttccctgcg 1680
   ccgggccgcc ctcggggcct ttaggcccca caccgcccca gccgacggat tcagtcgcgg 1740
   cagtacctca gatgtccgct gcgacggcgt cccggagagc ccgggcgaga tcgcgggccc 1800
   ccttctgctc gtccccggcc cctcccgcga gcaccacccg cggcggacgg ccgccgtcct 1860
   ccgcgatacg ccgggcgagg tcgcaggcga gcacgccgga cccggagaag ccccccagca 1920
   ccagcgaccg gccgactccg tgcgcggcca gggcaggctg cgcgccgtcg acgtcggtga 1980
   gcagcaccag gagctcctgc ggcccggcgt agaggtcggc cagccggtcg tagcaggtcg 2040
   cgggcgcgcc cggcggcggg atcagacaga tcgtgcccgc ccgctcgtgc ctcgccgccc 2100
   gcagcgtgac cagcggaatg tcccgcccag ctccgga 2137
<210> 161
   <211> 349
   <212> PRT
   <213> Pseudomonas fluorescens
<220>
   <223> phld Pseudomonas fluorescens Pf-5
<400> 161
   Met Ser Thr Leu Cys Leu Pro His Val Met Phe Pro Gln His Lys Ile
   1               5                   10                  15
   Thr Gln Gln Gln Met Val Asp His Leu Glu Asn Leu His Ala Asp His
               20                  25                  30
   Pro Arg Met Ala Leu Ala Lys Arg Met Ile Ala Asn Thr Glu Val Asn
           35                  40                  45
   Glu Arg His Leu Val Leu Pro Ile Asp Glu Leu Ala Val His Thr Gly
   50 55 60
   Phe Thr His Arg Ser Ile Val Tyr Glu Arg Glu Ala Arg Gln Met Ser
   65 70 75 80
   Ser Ala Ala Ala Arg Gln Ala Ile Glu Asn Ala Gly Leu Gln Ile Ser
   85 90 95
   Asp Ile Arg Met Val Ile Val Thr Ser Cys Thr Gly Phe Met Met Pro
   100 105 110
   Ser Leu Thr Ala His Leu Ile Asn Asp Leu Ala Leu Pro Thr Ser Thr
   115 120 125
   Val Gln Leu Pro Ile Ala Gln Leu Gly Cys Val Ala Gly Ala Ala Ala
   130 135 140
   Ile Asn Arg Ala Asn Asp Phe Ala Arg Leu Asp Ala Arg Asn His Val
   145 150 155 160
   Leu Ile Val Ser Leu Glu Phe Ser Ser Leu Cys Tyr Gln Pro Asp Asp
   165 170 175
   Thr Lys Leu His Ala Phe Ile Ser Ala Ala Leu Phe Gly Asp Ala Val
   180 185 190
   Ser Ala Cys Val Leu Arg Ala Asp Asp Gln Ala Gly Gly Phe Lys Ile
   195 200 205
   Lys Lys Thr Glu Ser Tyr Phe Leu Pro Lys Ser Glu His Tyr Ile Lys
   210 215 220
   Tyr Asp Val Lys Asp Thr Gly Phe His Phe Thr Leu Asp Lys Ala Val
   225 230 235 240
   Met Asn Ser Ile Lys Asp Val Ala Pro Val Met Glu Arg Leu Asn Tyr
   245 250 255
   Glu Ser Phe Glu Gln Asn Cys Ala His Asn Asp Phe Phe Ile Phe His
   260 265 270
   Thr Gly Gly Arg Lys Ile Leu Asp Glu Leu Val Met His Leu Asp Leu
   275 280 285
   Ala Ser Asn Arg Val Ser Gln Ser Arg Ser Ser Leu Ser Glu Ala Gly
   290 295 300
   Asn Ile Ala Ser Val Val Val Phe Asp Val Leu Lys Arg Gln Phe Asp
   305                 310                 315                 320
   Ser Asn Leu Asn Arg Gly Asp Ile Gly Leu Leu Ala Ala Phe Gly Pro
                   325                 330                 335
   Gly Phe Thr Ala Glu Met Ala Val Gly Glu Trp Thr Ala
               340                 345
<210> 162
   <211> 5385
   <212> DNA
   <213> Artificial Sequence
<220>
   <223> Description of Artificial Sequence: Synthetic pTRC-THNS polynucleotide
<400> 162
   gtttgacagc ttatcatcga ctgcacggtg caccaatgct tctggcgtca ggcagccatc 60
   ggaagctgtg gtatggctgt gcaggtcgta aatcactgca taattcgtgt cgctcaaggc 120
   gcactcccgt tctggataat gttttttgcg ccgacatcat aacggttctg gcaaatattc 180
   tgaaatgagc tgttgacaat taatcatccg gctcgtataa tgtgtggaat tgtgagcgga 240
   taacaatttc acacaggaaa cagcgccgct gagaaaaagc gaagcggcac tgctctttaa 300
   caatttatca gacaatctgt gtgggcactc gaccggaatt atcgattaac tttattatta 360
   aaaattaaag aggtatatat taatgtatcg attaaataag gaggaataaa ccatgaccac 420
   tctgtgccgc cctagcgtta gcgtcccgga acacgtgatt accatggagg aaacgctgga 480
   actggcgcgt cgtcgtcata cggatcatcc gcagctgccg ctggctttgc gtctgatcga 540
   gaataccggc gtgcgcactc gtcacattgt tcagccaatt gaggacacgc tggagcatcc 600
   gggtttcgag gaccgcaaca aagtctatga acgtgaagcc aagagccgcg ttccggctgt 660
   gatccagcgt gccctggacg atgcagaact gctggctacg gatattgatg ttatcatcta 720
   cgtgagctgc acgggtttta tgatgccgag cctgaccgcg tggttgatta acgagatggg 780
   tttcgactcc accacccgtc aaatcccgat tgcgcagctg ggttgtgcag cgggtggtgc 840
   ggcgatcaac cgtgcccatg atttttgcac cgcgtacccg gaagccaatg cactgatcgt 900
   cgcttgcgaa ttctgtagcc tgtgctatca gccgaccgac ttgggtgtgg gtagcttgct 960
   gtgcaacggt ctgttcggcg atggcattgc agcggccgtg gttcgcggtc gtggtggcac 1020
   cggtgttcgc ctggaacgta atggttctta cctgattccg aaaaccgaag attggattat 1080
   gtatgatgtg aaggcgacgg gtttccattt tctgctggac aagcgtgtcc cggcgaccat 1140
   ggaaccgttg gctccggcac tgaaagagct ggctggtgag cacggctggg acgccagcga 1200
   tctggacttc tacattgtgc acgcgggtgg tccgcgtatt ctggatgacc tgagcacctt 1260
   tctggaagtc gaccctcacg cattccgctt ttcccgtgca acgctgacgg agtacggcaa 1320
   tatcgcaagc gcggttgttt tggacgcact gcgtcgtctg ttcgacgagg gtggtgttga 1380
   ggaaggcgca cgcggcttgc tggcgggttt tggtccgggt attactgcag aaatgagcct 1440
   gggttgttgg cagaccgccg atgttcgtcg cggcatccgc caagatgtga cccgcacggc 1500
   ggcacgtggc gttagccgcc gtgtgcgtca agcttggctg ttttggcgga tgagagaaga 1560
   ttttcagcct gatacagatt aaatcagaac gcagaagcgg tctgataaaa cagaatttgc 1620
   ctggcggcag tagcgcggtg gtcccacctg accccatgcc gaactcagaa gtgaaacgcc 1680
   gtagcgccga tggtagtgtg gggtctcccc atgcgagagt agggaactgc caggcatcaa 1740
   ataaaacgaa aggctcagtc gaaagactgg gcctttcgtt ttatctgttg tttgtcggtg 1800
   aacgctctcc tgagtaggac aaatccgccg ggagcggatt tgaacgttgc gaagcaacgg 1860
   cccggagggt ggcgggcagg acgcccgcca taaactgcca ggcatcaaat taagcagaag 1920
   gccatcctga cggatggcct ttttgcgttt ctacaaactc tttttgttta tttttctaaa 1980
   tacattcaaa tatgtatccg ctcatgagac aataaccctg ataaatgctt caataatatt 2040
   gaaaaaggaa gagtatgagt attcaacatt tccgtgtcgc ccttattccc ttttttgcgg 2100
   cattttgcct tcctgttttt gctcacccag aaacgctggt gaaagtaaaa gatgctgaag 2160
   atcagttggg tgcacgagtg ggttacatcg aactggatct caacagcggt aagatccttg 2220
   agagttttcg ccccgaagaa cgttttccaa tgatgagcac ttttaaagtt ctgctatgtg 2280
   gcgcggtatt atcccgtgtt gacgccgggc aagagcaact cggtcgccgc atacactatt 2340
   ctcagaatga cttggttgag tactcaccag tcacagaaaa gcatcttacg gatggcatga 2400
   cagtaagaga attatgcagt gctgccataa ccatgagtga taacactgcg gccaacttac 2460
   ttctgacaac gatcggagga ccgaaggagc taaccgcttt tttgcacaac atgggggatc 2520
   atgtaactcg ccttgatcgt tgggaaccgg agctgaatga agccatacca aacgacgagc 2580
   gtgacaccac gatgcctgta gcaatggcaa caacgttgcg caaactatta actggcgaac 2640
   tacttactct agcttcccgg caacaattaa tagactggat ggaggcggat aaagttgcag 2700
   gaccacttct gcgctcggcc cttccggctg gctggtttat tgctgataaa tctggagccg 2760
   gtgagcgtgg gtctcgcggt atcattgcag cactggggcc agatggtaag ccctcccgta 2820
   tcgtagttat ctacacgacg gggagtcagg caactatgga tgaacgaaat agacagatcg 2880
   ctgagatagg tgcctcactg attaagcatt ggtaactgtc agaccaagtt tactcatata 2940
   tactttagat tgatttaaaa cttcattttt aatttaaaag gatctaggtg aagatccttt 3000
   ttgataatct catgaccaaa atcccttaac gtgagttttc gttccactga gcgtcagacc 3060
   ccgtagaaaa gatcaaagga tcttcttgag atcctttttt tctgcgcgta atctgctgct 3120
   tgcaaacaaa aaaaccaccg ctaccagcgg tggtttgttt gccggatcaa gagctaccaa 3180
   ctctttttcc gaaggtaact ggcttcagca gagcgcagat accaaatact gtccttctag 3240
   tgtagccgta gttaggccac cacttcaaga actctgtagc accgcctaca tacctcgctc 3300
   tgctaatcct gttaccagtg gctgctgcca gtggcgataa gtcgtgtctt accgggttgg 3360
   actcaagacg atagttaccg gataaggcgc agcggtcggg ctgaacgggg ggttcgtgca 3420
   cacagcccag cttggagcga acgacctaca ccgaactgag atacctacag cgtgagctat 3480
   gagaaagcgc cacgcttccc gaagggagaa aggcggacag gtatccggta agcggcaggg 3540
   tcggaacagg agagcgcacg agggagcttc cagggggaaa cgcctggtat ctttatagtc 3600
   ctgtcgggtt tcgccacctc tgacttgagc gtcgattttt gtgatgctcg tcaggggggc 3660
   ggagcctatg gaaaaacgcc agcaacgcgg cctttttacg gttcctggcc ttttgctggc 3720
   cttttgctca catgttcttt cctgcgttat cccctgattc tgtggataac cgtattaccg 3780
   cctttgagtg agctgatacc gctcgccgca gccgaacgac cgagcgcagc gagtcagtga 3840
   gcgaggaagc ggaagagcgc ctgatgcggt attttctcct tacgcatctg tgcggtattt 3900
   cacaccgcat atggtgcact ctcagtacaa tctgctctga tgccgcatag ttaagccagt 3960
   atacactccg ctatcgctac gtgactgggt catggctgcg ccccgacacc cgccaacacc 4020
   cgctgacgcg ccctgacggg cttgtctgct cccggcatcc gcttacagac aagctgtgac 4080
   cgtctccggg agctgcatgt gtcagaggtt ttcaccgtca tcaccgaaac gcgcgaggca 4140
   gcagatcaat tcgcgcgcga aggcgaagcg gcatgcattt acgttgacac catcgaatgg 4200
   tgcaaaacct ttcgcggtat ggcatgatag cgcccggaag agagtcaatt cagggtggtg 4260
   aatgtgaaac cagtaacgtt atacgatgtc gcagagtatg ccggtgtctc ttatcagacc 4320
   gtttcccgcg tggtgaacca ggccagccac gtttctgcga aaacgcggga aaaagtggaa 4380
   gcggcgatgg cggagctgaa ttacattccc aaccgcgtgg cacaacaact ggcgggcaaa 4440
   cagtcgttgc tgattggcgt tgccacctcc agtctggccc tgcacgcgcc gtcgcaaatt 4500
   gtcgcggcga ttaaatctcg cgccgatcaa ctgggtgcca gcgtggtggt gtcgatggta 4560
   gaacgaagcg gcgtcgaagc ctgtaaagcg gcggtgcaca atcttctcgc gcaacgcgtc 4620
   agtgggctga tcattaacta tccgctggat gaccaggatg ccattgctgt ggaagctgcc 4680
   tgcactaatg ttccggcgtt atttcttgat gtctctgacc agacacccat caacagtatt 4740
   attttctccc atgaagacgg tacgcgactg ggcgtggagc atctggtcgc attgggtcac 4800
   cagcaaatcg cgctgttagc gggcccatta agttctgtct cggcgcgtct gcgtctggct 4860
   ggctggcata aatatctcac tcgcaatcaa attcagccga tagcggaacg ggaaggcgac 4920
   tggagtgcca tgtccggttt tcaacaaacc atgcaaatgc tgaatgaggg catcgttccc 4980
   actgcgatgc tggttgccaa cgatcagatg gcgctgggcg caatgcgcgc cattaccgag 5040
   tccgggctgc gcgttggtgc ggatatctcg gtagtgggat acgacgatac cgaagacagc 5100
   tcatgttata tcccgccgtc aaccaccatc aaacaggatt ttcgcctgct ggggcaaacc 5160
   agcgtggacc gcttgctgca actctctcag ggccaggcgg tgaagggcaa tcagctgttg 5220
   cccgtctcac tggtgaaaag aaaaaccacc ctggcgccca atacgcaaac cgcctctccc 5280
   cgcgcgttgg ccgattcatt aatgcagctg gcacgacagg tttcccgact ggaaagcggg 5340
   cagtgagcgc aacgcaatta atgtgagtta gcgcgaattg atctg 5385
<210> 163
   <211> 8145
   <212> DNA
   <213> Artificial Sequence
<220>
   <223> Description of Artificial Sequence: Synthetic pACYC-accABCD/ pACYC-Ptpia-accAD-PrpiA-accCD polynucleotide
<400> 163
   gcttttagaa aaactcatcg agcatcaaat gaaactgcaa tttattcata tcaggattat 60
   caataccata tttttgaaaa agccgtttct gtaatgaagg agaaaactca ccgaggcagt 120
   tccataggat ggcaagatcc tggtatcggt ctgcgattcc gactcgtcca acatcaatac 180
   aacctattag gaaacagcta tgaccatgat tacgccaagc gcgcaattaa ccctcactaa 240
   agggaacaaa agctgggtac cgggcccccc ctcgaggtcg acggtatcga taagcttgat 300
   atccactgtg gaattcgccc ttggcacgtg cgaagttcct attctctaga aagtatagga 360
   acttcgtaag aggttccaac tttcaccata atgaaataag atcactaccg ggcgtatttt 420
   ttgagttatc gagattttca ggagctaagg aagctaaaat ggagaaaaaa atcactggat 480
   ataccaccgt tgatatatcc caatggcatc gtaaagaaca ttttgaggca tttcagtcag 540
   ttgctcaatg tacctataac cagaccgttc agctggatat tacggccttt ttaaagaccg 600
   taaagaaaaa taagcacaag ttttatccgg cctttattca cattcttgcc cgcctgatga 660
   atgctcatcc ggaattccgt atggcaatga aagacggtga gctggtgata tgggatagtg 720
   ttcacccttg ttacaccgtt ttccatgagc aaactgaaac gttttcatcg ctctggagtg 780
   aataccacga cgatttccgg cagtttctac acatatattc gcaagatgtg gcgtgttacg 840
   gtgaaaacct ggcctatttc cctaaagggt ttattgagaa tatgtttttc gtctcagcca 900
   atccctgggt gagtttcacc agttttgatt taaacgtggc caatatggac aacttcttcg 960
   cccccgtttt caccatgggc aaatattata cgcaaggcga caaggtgctg atgccgctgg 1020
   cgattcaggt tcatcatgcc gtttgtgatg gcttccatgt cggcagaatg cttaatgaat 1080
   tacaacagta ctgcgatgag tggcagggcg gggcgtaaac gcgtggatcc ccctcaagtc 1140
   aaaagcctcg aagttcctat actttcctag agaataggaa cttcgcacgt gccaagggcg 1200
   aattccacat tggtcgctgc agcccggggg atccactagt tctagagcgg ccgcaccgcg 1260
   ggagctccaa ttcgccctat agtgagtcgt attacgcgcg ctcactggcc gtcgttttac 1320
   aaatcaggat attcttctaa tacctggaac gctgtttttc cggggatcgc agtggtgagt 1380
   aaccatgcat catcaggagt acggataaaa tgcttgatgg tcggaagtgg cataaattcc 1440
   gtcagccagt ttagtctgac catctcatct gtaacatcat tggcaacgct acctttgcca 1500
   tgtttcagaa acaactctgg cgcatcgggc ttcccataca agcgatagat tgtcgcacct 1560
   gattgcccga cattatcgcg agcccattta tacccatata aatcagcatc catgttggaa 1620
   tttaatcgcg gcctcgacgt ttcccgttga atatggctca tattcttcct ttttcaatat 1680
   tattgaagca tttatcaggg ttattgtctc atgagcggat acatatttga atgtatttag 1740
   aaaaataaac aaataggggt cagtgttaca accaattaac caattctgaa cattatcgcg 1800
   agcccattta tacctgaata tggctcataa caccccttgt ttgcctggcg gcagtagcgc 1860
   ggtggtccca cctgacccca tgccgaactc agaagtgaaa cgccgtagcg ccgatggtag 1920
   tgtggggact ccccatgcga gagtagggaa ctgccaggca tcaaataaaa cgaaaggctc 1980
   agtcgaaaga ctgggccttt cgcccgggct aattaggggg tgtcgccctt attcgactct 2040
   atagggaagt tcctattctc tagaaagtat aggaacttct gaagggggga cagctgtctc 2100
   aaatgtctcg gaatcgctga cgattcccag gtttctccgg caagcatagc gcatggcgtc 2160
   tccatgcgag aatgtcgcgc ttgccggata aaaggggagc cgctatcgga atggacgcaa 2220
   gccacggccg cagcaggtgc ggtcgagggc ttccagccag ttccagggca gatgtgccgg 2280
   cagaccctcc cgctttgggg gaggcgcaag ccgggtccat tcggatagca tctccccatg 2340
   caaagtgccg gccagggcaa tgcccggagc cggttcgagg cgccggtttg aataaatgac 2400
   aaaaagcaaa gcctttgtgc cgatgaatct ctatactgtt tcacagacct gctgccctgc 2460
   ggggcggcca tcttccttta ttcgcttata agcgcggggc aggaggaaaa acatgtccct 2520
   gaacttcctg gacttcgagc agccgattgc agaactggaa gcgaagattg acagcctgac 2580
   cgcggttagc cgtcaagatg agaaactgga cattaacatc gacgaagagg tccaccgttt 2640
   gcgtgagaag tctgttgaac tgactcgcaa aatctttgct gatttgggcg catggcagat 2700
   tgcccagttg gctcgccacc cacaacgccc atataccctg gactacgtgc gcctggcgtt 2760
   tgacgagttc gacgaactgg caggcgaccg cgcctatgcg gacgataaag caattgtcgg 2820
   cggtattgct cgtttggatg gccgtccggt gatgattatc ggccatcaaa aaggccgcga 2880
   gacgaaagaa aagattcgtc gtaactttgg tatgccggca ccggagggct accgcaaggc 2940
   cctgcgtctg atgcaaatgg ccgaacgctt taagatgccg attatcacgt tcattgatac 3000
   gccgggtgcg tacccaggcg ttggtgcgga agagcgtggt cagagcgagg ccatcgcacg 3060
   taacctgcgt gagatgtctc gtctgggtgt gccggtcgtt tgcaccgtga ttggcgaggg 3120
   cggtagcggt ggtgcgttgg cgatcggtgt cggtgataag gtcaacatgc tgcaatacag 3180
   cacgtacagc gtcattagcc cggaaggttg cgcttccatt ctgtggaaga gcgcggataa 3240
   agcaccattg gcagcggaag cgatgggtat catcgcaccg cgtctgaaag aactgaagtt 3300
   gattgattct atcatcccgg aaccgctggg cggtgctcac cgtaatccgg aggcgatggc 3360
   agccagcctg aaggcccagc tgctggcgga cctggcggat ctggacgtgc tgagcacgga 3420
   ggatctgaaa aaccgtcgct atcagcgctt gatgagctat ggctacgcgt agtaaaggag 3480
   gaaaatcatg agctggattg aacgcattaa gtccaatatc accccgaccc gcaaggcgag 3540
   catccctgaa ggcgtctgga ccaaatgcga tagctgcggt caggttttgt atcgtgcgga 3600
   gctggagcgt aacctggaag tgtgcccgaa atgcgaccat cacatgcgta tgaccgctcg 3660
   taatcgtctg catagcctgc tggatgaggg cagcctggtc gagctgggta gcgaactgga 3720
   accgaaagat gttctgaaat tccgtgattc caagaagtat aaggatcgtt tggcatctgc 3780
   acaaaaagaa accggtgaga aggacgcact ggttgttatg aaaggcaccc tgtatggtat 3840
   gccggttgtt gctgcggcgt tcgagtttgc gtttatgggt ggcagcatgg gttccgtggt 3900
   gggcgcacgc tttgtgcgtg ccgtggagca ggcgctggag gataactgtc ctctgatttg 3960
   tttcagcgcg agcggtggtg cgcgtatgca agaggccctg atgagcctga tgcagatggc 4020
   aaaaacctcg gcagccctgg cgaagatgca agaacgcggc ctgccgtaca tttccgtcct 4080
   gaccgaccct acgatgggcg gtgtcagcgc cagctttgcg atgctgggtg atttgaacat 4140
   cgcagagccg aaggctctga ttggttttgc tggtccgcgt gttattgaac agacggttcg 4200
   cgaaaagttg ccgcctggtt tccagcgcag cgagttcctg attgagaaag gtgccatcga 4260
   catgatcgtt cgccgtccag aaatgcgtct gaaactggcg agcattctgg cgaaattgat 4320
   gaatctgccg gctccgaatc ctgaagcacc gcgtgagggt gtcgtggttc cgccggtccc 4380
   ggaccaagag ccggaggcat aatagcacgt ggagttaacc acgcggcttg ccaacggggt 4440
   ctgaatcgct ttttttgtat ataatgcgtg tgaaatttca taccacaggc gaaggaggaa 4500
   aaaccatgga cattcgtaag atcaagaaac tgattgaact ggttgaagaa agcggcatca 4560
   gcgagctgga gatcagcgaa ggtgaagaga gcgtccgtat ttcccgtgcg gcaccggcag 4620
   cgagctttcc ggttatgcag caagcatacg ccgctccgat gatgcaacag ccggcacaga 4680
   gcaacgccgc tgcaccggcg accgttccaa gcatggaggc accggcagcg gccgagattt 4740
   cgggtcatat cgtgcgtagc ccgatggtgg gcaccttcta tcgcacgccg tcgccggacg 4800
   caaaagcctt catcgaagtc ggccagaagg tcaatgtcgg cgacacgctg tgtatcgttg 4860
   aggcaatgaa aatgatgaac cagattgaag cggataagag cggtactgtt aaagcgatcc 4920
   tggtggaatc cggccagcct gttgagttcg atgaaccgct ggttgtgatc gagtaacgag 4980
   gcgaacatgt tggacaagat cgtgattgca aaccgcggtg aaatcgcgct gcgtatcttg 5040
   cgcgcgtgta aagagctggg cattaagact gttgccgtgc attccagcgc agaccgcgac 5100
   ctgaagcatg ttctgctggc cgacgaaacg gtttgcatcg gtccggcacc gagcgtgaaa 5160
   agctatctga acatcccggc catcatctct gcggcagaga tcaccggtgc agtggcgatt 5220
   catccgggct acggtttcct gagcgagaac gctaactttg ctgaacaagt ggagcgtagc 5280
   ggtttcatct tcattggccc taaggcggag acgattcgcc tgatgggcga caaagtgagc 5340
   gccattgcag cgatgaaaaa ggccggtgtg ccgtgtgttc cgggcagcga tggtccgctg 5400
   ggtgacgata tggacaagaa ccgtgccatc gctaaacgta ttggctaccc ggtcattatc 5460
   aaagcctctg gtggtggcgg tggccgtggt atgcgtgtcg tccgtggtga tgcggaactg 5520
   gcgcaaagca tcagcatgac ccgtgcggaa gccaaagcgg cgttctctaa cgatatggtg 5580
   tatatggaga agtatctgga gaatccgcgc cacgttgaaa tccaagttct ggcggatggt 5640
   cagggcaatg cgatctactt ggcagaacgt gattgctcca tgcaacgccg tcatcagaag 5700
   gtggtggaag aggcaccggc tccgggtatt acgccggaac tgcgtcgcta catcggtgag 5760
   cgctgtgcga aagcgtgtgt ggacattggt taccgtggtg cgggtacgtt tgagttcctg 5820
   ttcgaaaatg gtgagtttta cttcattgaa atgaataccc gcatccaggt tgagcacccg 5880
   gtgaccgaga tgattactgg cgttgatctg atcaaagagc aactgcgcat tgcggctggt 5940
   cagccgctgt cgatcaagca agaagaggtg cacgttcgtg gtcacgcggt cgagtgccgt 6000
   atcaatgcgg aggacccgaa tacctttctg ccgagccctg gtaagatcac gcgttttcac 6060
   gcgccaggtg gttttggcgt tcgttgggag tctcacatct acgccggtta caccgtgccg 6120
   ccgtactatg acagcatgat tggtaaactg atctgctatg gcgaaaatcg tgatgtcgcg 6180
   atcgcccgca tgaaaaacgc gctgcaagag ctgatcattg atggcattaa gaccaatgtg 6240
   gatttgcaga tccgcattat gaacgacgag aatttccagc acggcggtac gaacattcac 6300
   tacctggaaa agaaactggg cctgcaagag aaataatagg gtaccctagc ataacccctt 6360
   ggggcctcta aacgggtctt gaggggtttt ttgaatcccc cgggatgtct atcttaaggt 6420
   agccaaagtt taaactttgg ctaccttaag atagacatac tgccggcctg gttcaaccag 6480
   tcggcagccg gcgctggcgc ccgcgtattg cggtgcagcc agcgcggcgc acaaggcggc 6540
   gggcgtttcg tttcgccgcc cgtttcgcgg gccgtcaagg cccgcgaatc gtttctgccc 6600
   gcgcggcatt cctcgctttt tgcgccaatt caccgggttt tccttaagcc ccgtcgcttt 6660
   tcttagtgcc ttgttgggca tagaatcagg gcagcggcgc agccagcacc atgttcgtgc 6720
   agcgcggccc tcgcgggggc gaggctgcag acagctgtca aaatgaaggg aagttcctat 6780
   actttctaga gaataggaac ttctataggg agtcgaataa gggcgacaca aaaggtattc 6840
   taaatgcata ataaatactg ataacatctt atagtttgta ttatattttg tattatcgtt 6900
   gacatgtata attttgatat caaaaactga ttttcccttt attattttcg agatttattt 6960
   tcttaattct ctttaacaaa ctagaaatat tgtatataca aaaaatcata aataatagat 7020
   gaatagttta attataggtg ttcatcaatc gaaaaagcaa cgtatcttat ttaaagtgcg 7080
   ttgctttttt ctcatttata aggttaaata attctcatat atcaagcaaa gtgacaggcg 7140
   cccttaaata ttctgacaaa tgctctttcc ctaaactccc cccataaaaa aacccgccga 7200
   agcgggtttt tacgttattt gcggattaac gattactcgt tatcagaacc gcccaggatg 7260
   cctggcagtt ccctactctc gccgctgcgc tcggtcgttc ggctgcggga cctcagcgct 7320
   agcggagtgt atactggctt actatgttgg cactgatgag ggtgtcagtg aagtgcttca 7380
   tgtggcagga gaaaaaaggc tgcaccggtg cgtcagcaga atatgtgata caggatatat 7440
   tccgcttcct cgctcactga ctcgctacgc tcggtcgttc gactgcggcg agcggaaatg 7500
   gcttacgaac ggggcggaga tttcctggaa gatgccagga agatacttaa cagggaagtg 7560
   agagggccgc ggcaaagccg tttttccata ggctccgccc ccctgacaag catcacgaaa 7620
   tctgacgctc aaatcagtgg tggcgaaacc cgacaggact ataaagatac caggcgtttc 7680
   cccctggcgg ctccctcgtg cgctctcctg ttcctgcctt tcggtttacc ggtgtcattc 7740
   cgctgttatg gccgcgtttg tctcattcca cgcctgacac tcagttccgg gtaggcagtt 7800
   cgctccaagc tggactgtat gcacgaaccc cccgttcagt ccgaccgctg cgccttatcc 7860
   ggtaactatc gtcttgagtc caacccggaa agacatgcaa aagcaccact ggcagcagcc 7920
   actggtaatt gatttagagg agttagtctt gaagtcatgc gccggttaag gctaaactga 7980
   aaggacaagt tttggtgact gcgctcctcc aagccagtta cctcggttca aagagttggt 8040
   agctcagaga accttcgaaa aaccgccctg caaggcggtt ttttcgtttt cagagcaaga 8100
   gattacgcgc agaccaaaac gatctcaaga agatcatctt attaa 8145
<210> 164
   <211> 8894
   <212> DNA
   <213> Artificial Sequence
<220>
   <223> Description of Artificial Sequence: Synthetic Ptrc-mcr/ pTRC-Ptrc-mcr polynucleotide
<400> 164
   ccttcaactc agcaaaagtt cgatttattc aacaaagcca cgttgtgtct caaaatctct 60
   gatgttacat tgcacaagat aaaaatatat catcatgaac aataaaactg tctgcttaca 120
   taaacagtaa tacaaggggt gttatgagcc atattcaacg ggaaacgtct tgctcgaggc 180
   cgcgattaaa ttccaacatg gatgctgatt tatatgggta taaatgggct cgcgataatg 240
   tcgggcaatc aggtgcgaca atctatcgat tgtatgggaa gcccgatgcg ccagagttgt 300
   ttctgaaaca tggcaaaggt agcgttgcca atgatgttac agatgagatg gtcagactaa 360
   actggctgac ggaatttatg cctcttccga ccatcaagca ttttatccgt actcctgatg 420
   atgcatggtt actcaccact gcgatccccg ggaaaacagc attccaggta ttagaagaat 480
   atcctgattc aggtgaaaat attgttgatg cgctggcagt gttcctgcgc cggttgcatt 540
   cgattcctgt ttgtaattgt ccttttaaca gcgatcgcgt atttcgtctc gctcaggcgc 600
   aatcacgaat gaataacggt ttggttgatg cgagtgattt tgatgacgag cgtaatggct 660
   ggcctgttga acaagtctgg aaagaaatgc ataagctttt gccattctca ccggattcag 720
   tcgtcactca tggtgatttc tcacttgata accttatttt tgacgagggg aaattaatag 780
   gttgtattga tgttggacga gtcggaatcg cagaccgata ccaggatctt gccatcctat 840
   ggaactgcct cggtgagttt tctccttcat tacagaaacg gctttttcaa aaatatggta 900
   ttgataatcc tgatatgaat aaattgcagt ttcatttgat gctcgatgag tttttctaat 960
   cgagtcaggc aactatggat gaacgaaata gacagatcgc tgagataggt gcctcactga 1020
   ttaagcattg gtaactgtca gaccaagttt actcatatat actttagatt gatttaaaac 1080
   ttcattttta atttaaaagg atctaggtga agatcctttt tgataatctc atgaccaaaa 1140
   tcccttaacg tgagttttcg ttccactgag cgtcagaccc cgtagaaaag atcaaaggat 1200
   cttcttgaga tccttttttt ctgcgcgtaa tctgctgctt gcaaacaaaa aaaccaccgc 1260
   taccagcggt ggtttgtttg ccggatcaag agctaccaac tctttttccg aaggtaactg 1320
   gcttcagcag agcgcagata ccaaatactg tccttctagt gtagccgtag ttaggccacc 1380
   acttcaagaa ctctgtagca ccgcctacat acctcgctct gctaatcctg ttaccagtgg 1440
   ctgctgccag tggcgataag tcgtgtctta ccgggttgga ctcaagacga tagttaccgg 1500
   ataaggcgca gcggtcgggc tgaacggggg gttcgtgcac acagcccagc ttggagcgaa 1560
   cgacctacac cgaactgaga tacctacagc gtgagctatg agaaagcgcc acgcttcccg 1620
   aagggagaaa ggcggacagg tatccggtaa gcggcagggt cggaacagga gagcgcacga 1680
   gggagcttcc agggggaaac gcctggtatc tttatagtcc tgtcgggttt cgccacctct 1740
   gacttgagcg tcgatttttg tgatgctcgt caggggggcg gagcctatgg aaaaacgcca 1800
   gcaacgcggc ctttttacgg ttcctggcct tttgctggcc ttttgctcac atgttctttc 1860
   ctgcgttatc ccctgattct gtggataacc gtattaccgc ctttgagtga gctgataccg 1920
   ctcgccgcag ccgaacgacc gagcgcagcg agtcagtgag cgaggaagcg gaagagcgcc 1980
   tgatgcggta ttttctcctt acgcatctgt gcggtatttc acaccgcata tggtgcactc 2040
   tcagtacaat ctgctctgat gccgcatagt taagccagta tacactccgc tatcgctacg 2100
   tgactgggtc atggctgcgc cccgacaccc gccaacaccc gctgacgcgc cctgacgggc 2160
   ttgtctgctc ccggcatccg cttacagaca agctgtgacc gtctccggga gctgcatgtg 2220
   tcagaggttt tcaccgtcat caccgaaacg cgcgaggcag cagatcaatt cgcgcgcgaa 2280
   ggcgaagcgg catgcattta cgttgacacc atcgaatggt gcaaaacctt tcgcggtatg 2340
   gcatgatagc gcccggaaga gagtcaattc agggtggtga atgtgaaacc agtaacgtta 2400
   tacgatgtcg cagagtatgc cggtgtctct tatcagaccg tttcccgcgt ggtgaaccag 2460
   gccagccacg tttctgcgaa aacgcgggaa aaagtggaag cggcgatggc ggagctgaat 2520
   tacattccca accgcgtggc acaacaactg gcgggcaaac agtcgttgct gattggcgtt 2580
   gccacctcca gtctggccct gcacgcgccg tcgcaaattg tcgcggcgat taaatctcgc 2640
   gccgatcaac tgggtgccag cgtggtggtg tcgatggtag aacgaagcgg cgtcgaagcc 2700
   tgtaaagcgg cggtgcacaa tcttctcgcg caacgcgtca gtgggctgat cattaactat 2760
   ccgctggatg accaggatgc cattgctgtg gaagctgcct gcactaatgt tccggcgtta 2820
   tttcttgatg tctctgacca gacacccatc aacagtatta ttttctccca tgaagacggt 2880
   acgcgactgg gcgtggagca tctggtcgca ttgggtcacc agcaaatcgc gctgttagcg 2940
   ggcccattaa gttctgtctc ggcgcgtctg cgtctggctg gctggcataa atatctcact 3000
   cgcaatcaaa ttcagccgat agcggaacgg gaaggcgact ggagtgccat gtccggtttt 3060
   caacaaacca tgcaaatgct gaatgagggc atcgttccca ctgcgatgct ggttgccaac 3120
   gatcagatgg cgctgggcgc aatgcgcgcc attaccgagt ccgggctgcg cgttggtgcg 3180
   gatatctcgg tagtgggata cgacgatacc gaagacagct catgttatat cccgccgtca 3240
   accaccatca aacaggattt tcgcctgctg gggcaaacca gcgtggaccg cttgctgcaa 3300
   ctctctcagg gccaggcggt gaagggcaat cagctgttgc ccgtctcact ggtgaaaaga 3360
   aaaaccaccc tggcgcccaa tacgcaaacc gcctctcccc gcgcgttggc cgattcatta 3420
   atgcagctgg cacgacaggt ttcccgactg gaaagcgggc agtgagcgca acgcaattaa 3480
   tgtgagttag cgcgaattga tctggtttga cagcttatca tcgactgcac ggtgcaccaa 3540
   tgcttctggc gtcaggcagc catcggaagc tgtggtatgg ctgtgcaggt cgtaaatcac 3600
   tgcataattc gtgtcgctca aggcgcactc ccgttctgga taatgttttt tgcgccgaca 3660
   tcataacggt tctggcaaat attctgaaat gagctgttga caattaatca tccggctcgt 3720
   ataatgtgtg gaattgtgag cggataacaa tttcacacag gaaacagcgc cgctgagaaa 3780
   aagcgaagcg gcactgctct ttaacaattt atcagacaat ctgtgtgggc actcgaccgg 3840
   aattatcgat taactttatt attaaaaatt aaagaggtat atattaatgt atcgattaaa 3900
   taaggaggaa taaaccatgg ccggtacggg tcgtttggct ggtaaaattg cattgatcac 3960
   cggtggtgct ggtaacattg gttccgagct gacccgccgt tttctggccg agggtgcgac 4020
   ggttattatc agcggccgta accgtgcgaa gctgaccgcg ctggccgagc gcatgcaagc 4080
   cgaggccggc gtgccggcca agcgcattga tttggaggtg atggatggtt ccgaccctgt 4140
   ggctgtccgt gccggtatcg aggcaatcgt cgctcgccac ggtcagattg acattctggt 4200
   taacaacgcg ggctccgccg gtgcccaacg tcgcttggcg gaaattccgc tgacggaggc 4260
   agaattgggt ccgggtgcgg aggagacttt gcacgcttcg atcgcgaatc tgttgggcat 4320
   gggttggcac ctgatgcgta ttgcggctcc gcacatgcca gttggctccg cagttatcaa 4380
   cgtttcgact attttctcgc gcgcagagta ctatggtcgc attccgtacg ttaccccgaa 4440
   ggcagcgctg aacgctttgt cccagctggc tgcccgcgag ctgggcgctc gtggcatccg 4500
   cgttaacact attttcccag gtcctattga gtccgaccgc atccgtaccg tgtttcaacg 4560
   tatggatcaa ctgaagggtc gcccggaggg cgacaccgcc catcactttt tgaacaccat 4620
   gcgcctgtgc cgcgcaaacg accaaggcgc tttggaacgc cgctttccgt ccgttggcga 4680
   tgttgctgat gcggctgtgt ttctggcttc tgctgagagc gcggcactgt cgggtgagac 4740
   gattgaggtc acccacggta tggaactgcc ggcgtgtagc gaaacctcct tgttggcgcg 4800
   taccgatctg cgtaccatcg acgcgagcgg tcgcactacc ctgatttgcg ctggcgatca 4860
   aattgaagaa gttatggccc tgacgggcat gctgcgtacg tgcggtagcg aagtgattat 4920
   cggcttccgt tctgcggctg ccctggcgca atttgagcag gcagtgaatg aatctcgccg 4980
   tctggcaggt gcggatttca ccccgccgat cgctttgccg ttggacccac gtgacccggc 5040
   caccattgat gcggttttcg attggggcgc aggcgagaat acgggtggca tccatgcggc 5100
   ggtcattctg ccggcaacct cccacgaacc ggctccgtgc gtgattgaag tcgatgacga 5160
   acgcgtcctg aatttcctgg ccgatgaaat taccggcacc atcgttattg cgagccgttt 5220
   ggcgcgctat tggcaatccc aacgcctgac cccgggtgcc cgtgcccgcg gtccgcgtgt 5280
   tatctttctg agcaacggtg ccgatcaaaa tggtaatgtt tacggtcgta ttcaatctgc 5340
   ggcgatcggt caattgattc gcgtttggcg tcacgaggcg gagttggact atcaacgtgc 5400
   atccgccgca ggcgatcacg ttctgccgcc ggtttgggcg aaccagattg tccgtttcgc 5460
   taaccgctcc ctggaaggtc tggagttcgc gtgcgcgtgg accgcacagc tgctgcacag 5520
   ccaacgtcat attaacgaaa ttacgctgaa cattccagcc aatattagcg cgaccacggg 5580
   cgcacgttcc gccagcgtcg gctgggccga gtccttgatt ggtctgcacc tgggcaaggt 5640
   ggctctgatt accggtggtt cggcgggcat cggtggtcaa atcggtcgtc tgctggcctt 5700
   gtctggcgcg cgtgtgatgc tggccgctcg cgatcgccat aaattggaac agatgcaagc 5760
   catgattcaa agcgaattgg cggaggttgg ttataccgat gtggaggacc gtgtgcacat 5820
   cgctccgggt tgcgatgtga gcagcgaggc gcagctggca gatctggtgg aacgtacgct 5880
   gtccgcattc ggtaccgtgg attatttgat taataacgcc ggtattgcgg gcgtggagga 5940
   gatggtgatc gacatgccgg tggaaggctg gcgtcacacc ctgtttgcca acctgatttc 6000
   gaattattcg ctgatgcgca agttggcgcc gctgatgaag aagcaaggta gcggttacat 6060
   cctgaacgtt tcttcctatt ttggcggtga gaaggacgcg gcgattcctt atccgaaccg 6120
   cgccgactac gccgtctcca aggctggcca acgcgcgatg gcggaagtgt tcgctcgttt 6180
   cctgggtcca gagattcaga tcaatgctat tgccccaggt ccggttgaag gcgaccgcct 6240
   gcgtggtacc ggtgagcgtc cgggcctgtt tgctcgtcgc gcccgtctga tcttggagaa 6300
   taaacgcctg aacgaattgc acgcggcttt gattgctgcg gcccgcaccg atgagcgctc 6360
   gatgcacgag ttggttgaat tgttgctgcc gaacgacgtg gccgcgttgg agcagaaccc 6420
   agcggcccct accgcgctgc gtgagctggc acgccgcttc cgtagcgaag gtgatccggc 6480
   ggcaagctcc tcgtccgcct tgctgaatcg ctccatcgct gccaagctgt tggctcgctt 6540
   gcataacggt ggctatgtgc tgccggcgga tatttttgca aatctgccta atccgccgga 6600
   cccgttcttt acccgtgcgc aaattgaccg cgaagctcgc aaggtgcgtg atggtattat 6660
   gggtatgctg tatctgcagc gtatgccaac cgagtttgac gtcgctatgg caaccgtgta 6720
   ctatctggcc gatcgtaacg tgagcggcga aactttccat ccgtctggtg gtttgcgcta 6780
   cgagcgtacc ccgaccggtg gcgagctgtt cggcctgcca tcgccggaac gtctggcgga 6840
   gctggttggt agcacggtgt acctgatcgg tgaacacctg accgagcacc tgaacctgct 6900
   ggctcgtgcc tatttggagc gctacggtgc ccgtcaagtg gtgatgattg ttgagacgga 6960
   aaccggtgcg gaaaccatgc gtcgtctgtt gcatgatcac gtcgaggcag gtcgcctgat 7020
   gactattgtg gcaggtgatc agattgaggc agcgattgac caagcgatca cgcgctatgg 7080
   ccgtccgggt ccggtggtgt gcactccatt ccgtccactg ccaaccgttc cgctggtcgg 7140
   tcgtaaagac tccgattgga gcaccgtttt gagcgaggcg gaatttgcgg aactgtgtga 7200
   gcatcagctg acccaccatt tccgtgttgc tcgtaagatc gccttgtcgg atggcgcgtc 7260
   gctggcgttg gttaccccgg aaacgactgc gactagcacc acggagcaat ttgctctggc 7320
   gaacttcatc aagaccaccc tgcacgcgtt caccgcgacc atcggtgttg agtcggagcg 7380
   caccgcgcaa cgtattctga ttaaccaggt tgatctgacg cgccgcgccc gtgcggaaga 7440
   gccgcgtgac ccgcacgagc gtcagcagga attggaacgc ttcattgaag ccgttctgct 7500
   ggttaccgct ccgctgcctc ctgaggcaga cacgcgctac gcaggccgta ttcaccgcgg 7560
   tcgtgcgatt accgtctaat agaagcttgg ctgttttggc ggatgagaga agattttcag 7620
   cctgatacag attaaatcag aacgcagaag cggtctgata aaacagaatt tgcctggcgg 7680
   cagtagcgcg gtggtcccac ctgaccccat gccgaactca gaagtgaaac gccgtagcgc 7740
   cgatggtagt gtggggtctc cccatgcgag agtagggaac tgccaggcat caaataaaac 7800
   gaaaggctca gtcgaaagac tgggcctttc gttttatctg ttgtttgtcg gtgaacgctc 7860
   tcctgagtag gacaaatccg ccgggagcgg atttgaacgt tgcgaagcaa cggcccggag 7920
   ggtggcgggc aggacgcccg ccataaactg ccaggcatca aattaagcag aaggccatcc 7980
   tgacggatgg cctttttgcg tttctacaaa ctctttttgt ttatttttct aaatacattc 8040
   aaatatgtat ccgctcatga gacaataacc ctgataaatg cttcaataat attgaaaaag 8100
   gaagagtatg agtattcaac atttccgtgt cgcccttatt cccttttttg cggcattttg 8160
   ccttcctgtt tttgctcacc cagaaacgct ggtgaaagta aaagatgctg aagatcagtt 8220
   gggtgcacga gtgggttaca tcgaactgga tctcaacagc ggtaagatcc ttgagagttt 8280
   tcgccccgaa gaacgttttc caatgatgag cacttttaaa gttctgctat gtggcgcggt 8340
   attatcccgt gttgacgccg ggcaagagca actcggtcgc cgcatacact attctcagaa 8400
   tgacttggtt gagtactcac cagtcacaga aaagcatctt acggatggca tgacagtaag 8460
   agaattatgc agtgctgcca taaccatgag tgataacact gcggccaact tacttctgac 8520
   aacgatcgga ggaccgaagg agctaaccgc ttttttgcac aacatggggg atcatgtaac 8580
   tcgccttgat cgttgggaac cggagctgaa tgaagccata ccaaacgacg agcgtgacac 8640
   cacgatgcct gtagcaatgg caacaacgtt gcgcaaacta ttaactggcg aactacttac 8700
   tctagcttcc cggcaacaat taatagactg gatggaggcg gataaagttg caggaccact 8760
   tctgcgctcg gcccttccgg ctggctggtt tattgctgat aaatctggag ccggtgagcg 8820
   tgggtctcgc ggtatcattg cagcactggg gccagatggt aagccctccc gtatcgtagt 8880
   tatctacacg acgg 8894
<210> 165
   <211> 10578
   <212> DNA
   <213> Artificial Sequence
<220>
   <223> Description of Artificial Sequence: Synthetic pACYC-accABCD-pntAB/ pACYC-Ptal-pntAB-Ptpia-accAD-PrpiA-accCD polynucleotide
<400> 165
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatgggtt 2040
   acagagcttt caggattgca tccacgctgg ctttggcgtc accaaacagc atgtgggtgt 2100
   tttccttgaa gaacagcggg ttttgcacac cagcatagcc agtgttcatc gaacgtttaa 2160
   agacaatcac gttctgcgct ttccacactt ccagcacagg cataccagca atcggactct 2220
   tcggatcatc ctgcgccgcc gggttaaccg tatcgttagc accaatcacc agtacggtat 2280
   cggtatcagc aaagtcatca ttgatctcgt ccatttccag cacgatgtca tacggtactt 2340
   ttgcttcagc cagcaatacg ttcatatgtc caggcaaacg ccccgcgacc gggtggatac 2400
   cgaaacgcac attaatacca cgagcgcgca atttctcagt aatttcagcg acaggatatt 2460
   gcgcctgcgc gactgccatg ccgtaccccg gagtaatgat cactgaatgg gagtttttca 2520
   gcagttccgc tgtctcttct gcggtgattt cgcggtgctc acccacttcc tgatcatcgc 2580
   cagtagaaga gccgtcggtg ccgaaaccac ccgcaataac gctgataaag gaacggttca 2640
   tcgcctcaca cataatgtaa gaaaggatag cccccgaaga accgaccagc gcaccggtca 2700
   caatcagcag gtcgttgctg agcataaagc ccgcagccgc agccgcccag ccggagtacg 2760
   agttcagcat cgacaccacc actggcatat ctgcaccacc gatggaggcg actaaatgcc 2820
   agccgaatac cagcgcaatt gcggtcatta tcagcaatgc cagcacttgc aggccgacgc 2880
   tgtccgtgcg aacaaataca atcagcagca ggaaggaaac gaccagagcc gccaggttca 2940
   ttttgtgacg gtttggcagc atcaatggtt tagacgaaat cttgccacac agtttgccga 3000
   acgccaccac cgaacccgtg aacgttaccg ccccgatgaa gataccgagg aacacttccg 3060
   tcaggtgaat attgaccaga atcggtgcca ttcccgcgtc atgatgcaga tagctgttaa 3120
   agccaaccag cactgccgcc agacccacga agctatgcag gatcgccacc agttctggca 3180
   tttcggtcat ttcaactttc ttcgccagac ggataccaat tgccccacca atgaccatcg 3240
   ccagcaagat ccagccaaca ttacccgtat ccggtccaaa aatggttgcg attaacgcaa 3300
   tcgccatccc ggcgataccg aagttgttac cctggcgaga cgtttcatgt ttcgaaagac 3360
   cggccagact gaagataaac aggatcgcgg caacaatgta tgcagctgta actaatcctc 3420
   cagacatatg ttacccctta atttttgcgg aacattttca gcatgcgctg agtcacggtg 3480
   aagccaccga aaatattaat gctggctata agcaccgcga taaaactaag gaagctaacc 3540
   cagccgccct ggccaatctg caacagtgct ccgacaacaa taatccctga aatcgcgttg 3600
   gtgaccgaca tcaacggtgt atgcagcgcg tgcgatacat tccacaccac gtaataaccg 3660
   acaacgcagg ccagcgcgaa aacggtgaag tgcccaagga attctttcgg cgcaacgctt 3720
   gccatccagc caaaaagaat gattgccagc gccatcaacg cgtatttacg ccacggtgag 3780
   caggtacatt tttcctcagt tttcacttcc ggtgccgctt tttgtgccgc ctgcggctga 3840
   gctgatacct gaatcggcgg tgccggccag gtaatttcgc ccgcacggat cacggtcacg 3900
   ccgcgaatca ccacatcatc aaaatcaaca gtgatattgc cgtctttctc tttgcacaac 3960
   agtttcagca gattaacgag gtttgtgccg taaagctgtg aggattgcgt cggcagacgg 4020
   cccggaagat cggtataacc aatcactttg acaccatttt ccgtagtgaa gatttcaccc 4080
   ggcacggtgt attcacagtt gccgccgttt tgggctgcca ggtcgacaat cacactgccc 4140
   gccttcatgg agtcaaccat ttcacgggta attagcttcg gcgctggttt gcctggaata 4200
   agcgcggtgg tgacaatgat atcgacctct tttgcctggg cggcaaagag ttccatttcc 4260
   gctttgatga acgcgtccga catcactttg gcatagccat cgccgctgcc agcttcctct 4320
   ttaaaatcca gctcgaggaa ttccgcgccc atactttgaa cttgttcttt cacttccggg 4380
   cgggtgtcga atgcacgcac aatcgcgccg agactgtttg ctgcgccaat ggcggccaga 4440
   cctgcaacac ccgcaccaat caccatcact tttgccggtg gcactttccc ggccgcagta 4500
   atttgcccgg taaagaagcg cccaaattca tgtgccgctt caacaatggc gcgataaccg 4560
   gcgatgttcg ccatcgagct tagtgcgtcc agcgattgtg cgcgtgagat acgcggcaca 4620
   gagtccatcg ccatcacggt cacgttacgt tccgcaagtt tttgcattaa ttccggattc 4680
   tgcgcaggcc agataaaact caccagcgtt gtcccaggat tcagtaacgc aatttcatca 4740
   tctaacggcg cattgacctt cagaatgatc tctgactgcc agacgctatt cccttctaca 4800
   atttcagcgc ccgcttgcac aaacgcttta tcgtcaaaac ttgccagttg acccgcgccg 4860
   ctctctaccg cgacggtaaa acccagtttc agcagctgtt ccactgtttt tggcgttgct 4920
   gcaacacggg tttcattggt taaccgttct cttggtatgc caattgccat ggtaaatact 4980
   ccttgaaaag taaagtgtta gatgagtgcg ttaattcaca cttctgagaa atttcgctaa 5040
   acgcatcaaa aaagcatagc agacaggcat ggtattgctg gattaagcag gtaacatcag 5100
   tgttatagga ttattaccaa aacattatat tcacaggtgt atcgctcaat actgaccatt 5160
   taaatcatac ctgacctcca tagcagaaag tcaaaagcct ccgaccggag gcttttgact 5220
   tgagggggat cagcttctat tatttctctt gcaggcccag tttcttttcc aggtagtgaa 5280
   tgttcgtacc gccgtgctgg aaattctcgt cgttcataat gcggatctgc aaatccacat 5340
   tggtcttaat gccatcaatg atcagctctt gcagcgcgtt tttcatgcgg gcgatcgcga 5400
   catcacgatt ttcgccatag cagatcagtt taccaatcat gctgtcatag tacggcggca 5460
   cggtgtaacc ggcgtagatg tgagactccc aacgaacgcc aaaaccacct ggcgcgtgaa 5520
   aacgcgtgat cttaccaggg ctcggcagaa aggtattcgg gtcctccgca ttgatacggc 5580
   actcgaccgc gtgaccacga acgtgcacct cttcttgctt gatcgacagc ggctgaccag 5640
   ccgcaatgcg cagttgctct ttgatcagat caacgccagt aatcatctcg gtcaccgggt 5700
   gctcaacctg gatgcgggta ttcatttcaa tgaagtaaaa ctcaccattt tcgaacagga 5760
   actcaaacgt acccgcacca cggtaaccaa tgtccacaca cgctttcgca cagcgctcac 5820
   cgatgtagcg acgcagttcc ggcgtaatac ccggagccgg tgcctcttcc accaccttct 5880
   gatgacggcg ttgcatggag caatcacgtt ctgccaagta gatcgcattg ccctgaccat 5940
   ccgccagaac ttggatttca acgtggcgcg gattctccag atacttctcc atatacacca 6000
   tatcgttaga gaacgccgct ttggcttccg cacgggtcat gctgatgctt tgcgccagtt 6060
   ccgcatcacc acggacgaca cgcataccac ggccaccgcc accaccagag gctttgataa 6120
   tgaccgggta gccaatacgt ttagcgatgg cacggttctt gtccatatcg tcacccagcg 6180
   gaccatcgct gcccggaaca cacggcacac cggccttttt catcgctgca atggcgctca 6240
   ctttgtcgcc catcaggcga atcgtctccg ccttagggcc aatgaagatg aaaccgctac 6300
   gctccacttg ttcagcaaag ttagcgttct cgctcaggaa accgtagccc ggatgaatcg 6360
   ccactgcacc ggtgatctct gccgcagaga tgatggccgg gatgttcaga tagcttttca 6420
   cgctcggtgc cggaccgatg caaaccgttt cgtcggccag cagaacatgc ttcaggtcgc 6480
   ggtctgcgct ggaatgcacg gcaacagtct taatgcccag ctctttacac gcgcgcaaga 6540
   tacgcagcgc gatttcaccg cggtttgcaa tcacgatctt gtccaacatg ttcgcctcgt 6600
   tactcgatca caaccagcgg ttcatcgaac tcaacaggct ggccggattc caccaggatc 6660
   gctttaacag taccgctctt atccgcttca atctggttca tcattttcat tgcctcaacg 6720
   atacacagcg tgtcgccgac attgaccttc tggccgactt cgatgaaggc ttttgcgtcc 6780
   ggcgacggcg tgcgatagaa ggtgcccacc atcgggctac gcacgatatg acccgaaatc 6840
   tcggccgctg ccggtgcctc catgcttgga acggtcgccg gtgcagcggc gttgctctgt 6900
   gccggctgtt gcatcatcgg agcggcgtat gcttgctgca taaccggaaa gctcgctgcc 6960
   ggtgccgcac gggaaatacg gacgctctct tcaccttcgc tgatctccag ctcgctgatg 7020
   ccgctttctt caaccagttc aatcagtttc ttgatcttac gaatgtccat ggtttttcct 7080
   ccttcgcctg tggtatgaaa tttcacacgc attatataca aaaaaagcga ttcagacccc 7140
   gttggcaagc cgcgtggtta actccacgtg ctattatgcc tccggctctt ggtccgggac 7200
   cggcggaacc acgacaccct cacgcggtgc ttcaggattc ggagccggca gattcatcaa 7260
   tttcgccaga atgctcgcca gtttcagacg catttctgga cggcgaacga tcatgtcgat 7320
   ggcacctttc tcaatcagga actcgctgcg ctggaaacca ggcggcaact tttcgcgaac 7380
   cgtctgttca ataacacgcg gaccagcaaa accaatcaga gccttcggct ctgcgatgtt 7440
   caaatcaccc agcatcgcaa agctggcgct gacaccgccc atcgtagggt cggtcaggac 7500
   ggaaatgtac ggcaggccgc gttcttgcat cttcgccagg gctgccgagg tttttgccat 7560
   ctgcatcagg ctcatcaggg cctcttgcat acgcgcacca ccgctcgcgc tgaaacaaat 7620
   cagaggacag ttatcctcca gcgcctgctc cacggcacgc acaaagcgtg cgcccaccac 7680
   ggaacccatg ctgccaccca taaacgcaaa ctcgaacgcc gcagcaacaa ccggcatacc 7740
   atacagggtg cctttcataa caaccagtgc gtccttctca ccggtttctt tttgtgcaga 7800
   tgccaaacga tccttatact tcttggaatc acggaatttc agaacatctt tcggttccag 7860
   ttcgctaccc agctcgacca ggctgccctc atccagcagg ctatgcagac gattacgagc 7920
   ggtcatacgc atgtgatggt cgcatttcgg gcacacttcc aggttacgct ccagctccgc 7980
   acgatacaaa acctgaccgc agctatcgca tttggtccag acgccttcag ggatgctcgc 8040
   cttgcgggtc ggggtgatat tggacttaat gcgttcaatc cagctcatga ttttcctcct 8100
   ttactacgcg tagccatagc tcatcaagcg ctgatagcga cggtttttca gatcctccgt 8160
   gctcagcacg tccagatccg ccaggtccgc cagcagctgg gccttcaggc tggctgccat 8220
   cgcctccgga ttacggtgag caccgcccag cggttccggg atgatagaat caatcaactt 8280
   cagttctttc agacgcggtg cgatgatacc catcgcttcc gctgccaatg gtgctttatc 8340
   cgcgctcttc cacagaatgg aagcgcaacc ttccgggcta atgacgctgt acgtgctgta 8400
   ttgcagcatg ttgaccttat caccgacacc gatcgccaac gcaccaccgc taccgccctc 8460
   gccaatcacg gtgcaaacga ccggcacacc cagacgagac atctcacgca ggttacgtgc 8520
   gatggcctcg ctctgaccac gctcttccgc accaacgcct gggtacgcac ccggcgtatc 8580
   aatgaacgtg ataatcggca tcttaaagcg ttcggccatt tgcatcagac gcagggcctt 8640
   gcggtagccc tccggtgccg gcataccaaa gttacgacga atcttttctt tcgtctcgcg 8700
   gcctttttga tggccgataa tcatcaccgg acggccatcc aaacgagcaa taccgccgac 8760
   aattgcttta tcgtccgcat aggcgcggtc gcctgccagt tcgtcgaact cgtcaaacgc 8820
   caggcgcacg tagtccaggg tatatgggcg ttgtgggtgg cgagccaact gggcaatctg 8880
   ccatgcgccc aaatcagcaa agattttgcg agtcagttca acagacttct cacgcaaacg 8940
   gtggacctct tcgtcgatgt taatgtccag tttctcatct tgacggctaa ccgcggtcag 9000
   gctgtcaatc ttcgcttcca gttctgcaat cggctgctcg aagtccagga agttcaggga 9060
   catgtttttc ctcctgcccc gcgcttataa gcgaataaag gaagatggcc gccccgcagg 9120
   gcagcaggtc tgtgaaacag tatagagatt catcggcaca aaggctttgc tttttgtcat 9180
   ttattcaaac cggcgcctcg aaccggctcc gggcattgcc ctggccggca ctttgcatgg 9240
   ggagatgcgg atctcgggca gcgttgggtc ctggccacgg gtgcgcatga tcgtgctcct 9300
   gtcgttgagg acccggctag gctggcgggg ttgccttact ggttagcaga atgaatcacc 9360
   gatacgcgag cgaacgtgaa gcgactgctg ctgcaaaacg tctgcgacct gagcaacaac 9420
   atgaatggtc ttcggtttcc gtgtttcgta aagtctggaa acgcggaagt cccctacgtg 9480
   ctgctgaagt tgcccgcaac agagagtgga accaaccggt gataccacga tactatgact 9540
   gagagtcaac gccatgagcg gcctcatttc ttattctgag ttacaacagt ccgcaccgct 9600
   gtccggtagc tccttccggt gggcgcgggg catgactatc gtcgccgcac ttatgactgt 9660
   cttctttatc atgcaactcg taggacaggt gccggcagcg cccaacagtc ccccggccac 9720
   ggggcctgcc accataccca cgccgaaaca agcgccctgc accattatgt tccggatctg 9780
   catcgcagga tgctgctggc taccctgtgg aacacctaca tctgtattaa cgaagcgcta 9840
   accgttttta tcaggctctg ggaggcagaa taaatgatca tatcgtcaat tattacctcc 9900
   acggggagag cctgagcaaa ctggcctcag gcatttgaga agcacacggt cacactgctt 9960
   ccggtagtca ataaaccggt aaaccagcaa tagacataag cggctattta acgaccctgc 10020
   cctgaaccga cgaccgggtc gaatttgctt tcgaatttct gccattcatc cgcttattat 10080
   cacttattca ggcgtagcac caggcgttta agggcaccaa taactgcctt aaaaaaatta 10140
   cgccccgccc tgccactcat cgcagtactg ttgtaattca ttaagcattc tgccgacatg 10200
   gaagccatca cagacggcat gatgaacctg aatcgccagc ggcatcagca ccttgtcgcc 10260
   ttgcgtataa tatttgccca tggtgaaaac gggggcgaag aagttgtcca tattggccac 10320
   gtttaaatca aaactggtga aactcaccca gggattggct gagacgaaaa acatattctc 10380
   aataaaccct ttagggaaat aggccaggtt ttcaccgtaa cacgccacat cttgcgaata 10440
   tatgtgtaga aactgccgga aatcgtcgtg gtattcactc cagagcgatg aaaacgtttc 10500
   agtttgctca tggaaaacgg tgtaacaagg gtgaacacta tcccatatca ccagctcacc 10560
   gtctttcatt gccatacg 10578
<210> 166
   <211> 9047
   <212> DNA
   <213> Artificial Sequence
<220>
   <223> Description of Artificial Sequence: Synthetic pACYC-CAT-accADBC-udhA/ pACYC-Ptal-udhA-Ptpia-accAD-PrpiA-accCD polynucleotide
<400> 166
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatttaaa 2040
   acaggcggtt taaaccgttt aacgcagcta cccgataggc ttccgccatc gtcgggtagt 2100
   taaaggtggt gttgacgaag tactcaatag tgttgccgcc acctttctgt tccataatcg 2160
   cctgaccgat atgaataatt tcggcagcgc gctcgccaaa gcagtgaata cccagaatct 2220
   cttttgtttc ccgatggaac aaaattttca gcgtgcccac gttcatgccg acgatttgtg 2280
   cgcgtgccag atgtttaaac tgggcgcggc ccacttcata tggcactttc attgcggtca 2340
   gctgctgttc ggttttgccc acagagctga tttccgggat ggtgtaaata ccggtaggga 2400
   tatcttcaat cagatgtgcg gtggcttcgc cttttaccag cgcctgcgcg gcaatgcgcc 2460
   cctggtcata ggccgccgac gccaggctcg gataaccaat cacgtcgccc accgcgtaaa 2520
   cgtgtggctg tgcggtctga tacatgctgt tgaccttcag ctgtccgcgg ctgtcagttt 2580
   ctagcccaat gttctgtaac gccagcgaat cggtattacc ggtgcgaccg ttggcataga 2640
   gcaggcagtc agctttcagt tttttacccg acttcagatg catgatcaca ccatcgtcac 2700
   agccttcgat cttctcgtac tcttcgttgt gacgaatcac tacgccactg ttccagaagt 2760
   gataggagag agaatctgac atctcttgat cgagaaatgc cagcaggcga tcgcgggtgt 2820
   tgatcagatc cacttttaca tccataccgc ggaagatcga cgcatattca cagccgatca 2880
   ctccagcacc atagataagt acatggcgcg gttcgtggtg catgctgaga attgagtcgc 2940
   tgtcgtaaat gcgtggatgg gtgaaatcaa catctgttgg atgatatgga cgagagccgc 3000
   aggcaataac aaatttttca gcggttagtg tttcaacgct gccgtccggg caatccagcg 3060
   ccaacgtatg ctcgtcaaca aagcgagcgt ttccctgcaa tatttcacag tgattacgtt 3120
   cgtaaaatcc ctgacgcatg cgcgtttgtt gattaatcac gttatcggca tggttaagga 3180
   tatcggcaaa agaagagcgg agcagtcggg aatggtcgct gtaaagtggg ttttgattga 3240
   attctataat gcggctgacg gcgtgacgga gagctttcga cgggatggtg ccccagtggg 3300
   tgcaaccgcc gccaacattt tgataacgct cgataactgc gacgcgcgca ccttgcttaa 3360
   ccaggcccat tgcagcgcct tcgccgccgg ggccggaacc tattactatg gcatcgtaat 3420
   cgtaggaatg tggggccatg gtaaatactc cttgaaaagt aaagtgttag atgagtgcgt 3480
   taattcacac ttctgagaaa tttcgctaaa cgcatcaaaa aagcatagca gacaggcatg 3540
   gtattgctgg attaagcagg taacatcagt gttataggat tattaccaaa acattatatt 3600
   cacaggtgta tcgctcaata ctgaccattt aaatcatacc tgacctccat agcagaaagt 3660
   caaaagcctc cgaccggagg cttttgactt gagggggatc agcttctatt atttctcttg 3720
   caggcccagt ttcttttcca ggtagtgaat gttcgtaccg ccgtgctgga aattctcgtc 3780
   gttcataatg cggatctgca aatccacatt ggtcttaatg ccatcaatga tcagctcttg 3840
   cagcgcgttt ttcatgcggg cgatcgcgac atcacgattt tcgccatagc agatcagttt 3900
   accaatcatg ctgtcatagt acggcggcac ggtgtaaccg gcgtagatgt gagactccca 3960
   acgaacgcca aaaccacctg gcgcgtgaaa acgcgtgatc ttaccagggc tcggcagaaa 4020
   ggtattcggg tcctccgcat tgatacggca ctcgaccgcg tgaccacgaa cgtgcacctc 4080
   ttcttgcttg atcgacagcg gctgaccagc cgcaatgcgc agttgctctt tgatcagatc 4140
   aacgccagta atcatctcgg tcaccgggtg ctcaacctgg atgcgggtat tcatttcaat 4200
   gaagtaaaac tcaccatttt cgaacaggaa ctcaaacgta cccgcaccac ggtaaccaat 4260
   gtccacacac gctttcgcac agcgctcacc gatgtagcga cgcagttccg gcgtaatacc 4320
   cggagccggt gcctcttcca ccaccttctg atgacggcgt tgcatggagc aatcacgttc 4380
   tgccaagtag atcgcattgc cctgaccatc cgccagaact tggatttcaa cgtggcgcgg 4440
   attctccaga tacttctcca tatacaccat atcgttagag aacgccgctt tggcttccgc 4500
   acgggtcatg ctgatgcttt gcgccagttc cgcatcacca cggacgacac gcataccacg 4560
   gccaccgcca ccaccagagg ctttgataat gaccgggtag ccaatacgtt tagcgatggc 4620
   acggttcttg tccatatcgt cacccagcgg accatcgctg cccggaacac acggcacacc 4680
   ggcctttttc atcgctgcaa tggcgctcac tttgtcgccc atcaggcgaa tcgtctccgc 4740
   cttagggcca atgaagatga aaccgctacg ctccacttgt tcagcaaagt tagcgttctc 4800
   gctcaggaaa ccgtagcccg gatgaatcgc cactgcaccg gtgatctctg ccgcagagat 4860
   gatggccggg atgttcagat agcttttcac gctcggtgcc ggaccgatgc aaaccgtttc 4920
   gtcggccagc agaacatgct tcaggtcgcg gtctgcgctg gaatgcacgg caacagtctt 4980
   aatgcccagc tctttacacg cgcgcaagat acgcagcgcg atttcaccgc ggtttgcaat 5040
   cacgatcttg tccaacatgt tcgcctcgtt actcgatcac aaccagcggt tcatcgaact 5100
   caacaggctg gccggattcc accaggatcg ctttaacagt accgctctta tccgcttcaa 5160
   tctggttcat cattttcatt gcctcaacga tacacagcgt gtcgccgaca ttgaccttct 5220
   ggccgacttc gatgaaggct tttgcgtccg gcgacggcgt gcgatagaag gtgcccacca 5280
   tcgggctacg cacgatatga cccgaaatct cggccgctgc cggtgcctcc atgcttggaa 5340
   cggtcgccgg tgcagcggcg ttgctctgtg ccggctgttg catcatcgga gcggcgtatg 5400
   cttgctgcat aaccggaaag ctcgctgccg gtgccgcacg ggaaatacgg acgctctctt 5460
   caccttcgct gatctccagc tcgctgatgc cgctttcttc aaccagttca atcagtttct 5520
   tgatcttacg aatgtccatg gtttttcctc cttcgcctgt ggtatgaaat ttcacacgca 5580
   ttatatacaa aaaaagcgat tcagaccccg ttggcaagcc gcgtggttaa ctccacgtgc 5640
   tattatgcct ccggctcttg gtccgggacc ggcggaacca cgacaccctc acgcggtgct 5700
   tcaggattcg gagccggcag attcatcaat ttcgccagaa tgctcgccag tttcagacgc 5760
   atttctggac ggcgaacgat catgtcgatg gcacctttct caatcaggaa ctcgctgcgc 5820
   tggaaaccag gcggcaactt ttcgcgaacc gtctgttcaa taacacgcgg accagcaaaa 5880
   ccaatcagag ccttcggctc tgcgatgttc aaatcaccca gcatcgcaaa gctggcgctg 5940
   acaccgccca tcgtagggtc ggtcaggacg gaaatgtacg gcaggccgcg ttcttgcatc 6000
   ttcgccaggg ctgccgaggt ttttgccatc tgcatcaggc tcatcagggc ctcttgcata 6060
   cgcgcaccac cgctcgcgct gaaacaaatc agaggacagt tatcctccag cgcctgctcc 6120
   acggcacgca caaagcgtgc gcccaccacg gaacccatgc tgccacccat aaacgcaaac 6180
   tcgaacgccg cagcaacaac cggcatacca tacagggtgc ctttcataac aaccagtgcg 6240
   tccttctcac cggtttcttt ttgtgcagat gccaaacgat ccttatactt cttggaatca 6300
   cggaatttca gaacatcttt cggttccagt tcgctaccca gctcgaccag gctgccctca 6360
   tccagcaggc tatgcagacg attacgagcg gtcatacgca tgtgatggtc gcatttcggg 6420
   cacacttcca ggttacgctc cagctccgca cgatacaaaa cctgaccgca gctatcgcat 6480
   ttggtccaga cgccttcagg gatgctcgcc ttgcgggtcg gggtgatatt ggacttaatg 6540
   cgttcaatcc agctcatgat tttcctcctt tactacgcgt agccatagct catcaagcgc 6600
   tgatagcgac ggtttttcag atcctccgtg ctcagcacgt ccagatccgc caggtccgcc 6660
   agcagctggg ccttcaggct ggctgccatc gcctccggat tacggtgagc accgcccagc 6720
   ggttccggga tgatagaatc aatcaacttc agttctttca gacgcggtgc gatgataccc 6780
   atcgcttccg ctgccaatgg tgctttatcc gcgctcttcc acagaatgga agcgcaacct 6840
   tccgggctaa tgacgctgta cgtgctgtat tgcagcatgt tgaccttatc accgacaccg 6900
   atcgccaacg caccaccgct accgccctcg ccaatcacgg tgcaaacgac cggcacaccc 6960
   agacgagaca tctcacgcag gttacgtgcg atggcctcgc tctgaccacg ctcttccgca 7020
   ccaacgcctg ggtacgcacc cggcgtatca atgaacgtga taatcggcat cttaaagcgt 7080
   tcggccattt gcatcagacg cagggccttg cggtagccct ccggtgccgg cataccaaag 7140
   ttacgacgaa tcttttcttt cgtctcgcgg cctttttgat ggccgataat catcaccgga 7200
   cggccatcca aacgagcaat accgccgaca attgctttat cgtccgcata ggcgcggtcg 7260
   cctgccagtt cgtcgaactc gtcaaacgcc aggcgcacgt agtccagggt atatgggcgt 7320
   tgtgggtggc gagccaactg ggcaatctgc catgcgccca aatcagcaaa gattttgcga 7380
   gtcagttcaa cagacttctc acgcaaacgg tggacctctt cgtcgatgtt aatgtccagt 7440
   ttctcatctt gacggctaac cgcggtcagg ctgtcaatct tcgcttccag ttctgcaatc 7500
   ggctgctcga agtccaggaa gttcagggac atgtttttcc tcctgccccg cgcttataag 7560
   cgaataaagg aagatggccg ccccgcaggg cagcaggtct gtgaaacagt atagagattc 7620
   atcggcacaa aggctttgct ttttgtcatt tattcaaacc ggcgcctcga accggctccg 7680
   ggcattgccc tggccggcac tttgcatggg gagatgcgga tctcgggcag cgttgggtcc 7740
   tggccacggg tgcgcatgat cgtgctcctg tcgttgagga cccggctagg ctggcggggt 7800
   tgccttactg gttagcagaa tgaatcaccg atacgcgagc gaacgtgaag cgactgctgc 7860
   tgcaaaacgt ctgcgacctg agcaacaaca tgaatggtct tcggtttccg tgtttcgtaa 7920
   agtctggaaa cgcggaagtc ccctacgtgc tgctgaagtt gcccgcaaca gagagtggaa 7980
   ccaaccggtg ataccacgat actatgactg agagtcaacg ccatgagcgg cctcatttct 8040
   tattctgagt tacaacagtc cgcaccgctg tccggtagct ccttccggtg ggcgcggggc 8100
   atgactatcg tcgccgcact tatgactgtc ttctttatca tgcaactcgt aggacaggtg 8160
   ccggcagcgc ccaacagtcc cccggccacg gggcctgcca ccatacccac gccgaaacaa 8220
   gcgccctgca ccattatgtt ccggatctgc atcgcaggat gctgctggct accctgtgga 8280
   acacctacat ctgtattaac gaagcgctaa ccgtttttat caggctctgg gaggcagaat 8340
   aaatgatcat atcgtcaatt attacctcca cggggagagc ctgagcaaac tggcctcagg 8400
   catttgagaa gcacacggtc acactgcttc cggtagtcaa taaaccggta aaccagcaat 8460
   agacataagc ggctatttaa cgaccctgcc ctgaaccgac gaccgggtcg aatttgcttt 8520
   cgaatttctg ccattcatcc gcttattatc acttattcag gcgtagcacc aggcgtttaa 8580
   gggcaccaat aactgcctta aaaaaattac gccccgccct gccactcatc gcagtactgt 8640
   tgtaattcat taagcattct gccgacatgg aagccatcac agacggcatg atgaacctga 8700
   atcgccagcg gcatcagcac cttgtcgcct tgcgtataat atttgcccat ggtgaaaacg 8760
   ggggcgaaga agttgtccat attggccacg tttaaatcaa aactggtgaa actcacccag 8820
   ggattggctg agacgaaaaa catattctca ataaaccctt tagggaaata ggccaggttt 8880
   tcaccgtaac acgccacatc ttgcgaatat atgtgtagaa actgccggaa atcgtcgtgg 8940
   tattcactcc agagcgatga aaacgtttca gtttgctcat ggaaaacggt gtaacaaggg 9000
   tgaacactat cccatatcac cagctcaccg tctttcattg ccatacg 9047
<210> 167
   <211> 5592
   <212> DNA
   <213> Artificial Sequence
<220>
   <223> Description of Artificial Sequence: Synthetic pBT3_tpiA-pntAB/ pBT3-PtpiA-pntAB polynucleotide
<400> 167
   aacgaattca agcttgatat ccactgtgga attcgccctt ttacagagct ttcaggattg      60
   catccacgct ggctttggcg tcaccaaaca gcatgtgggt gttttccttg aagaacagcg     120
   ggttttgcac accagcatag ccagtgttca tcgaacgttt aaagacaatc acgttctgcg     180
   ctttccacac ttccagcaca ggcataccag caatcggact cttcggatca tcctgcgccg     240
   ccgggttaac cgtatcgtta gcaccaatca ccagtacggt atcggtatca gcaaagtcat     300
   cattgatctc gtccatttcc agcacgatgt catacggtac ttttgcttca gccagcaata     360
   cgttcatatg tccaggcaaa cgccccgcga ccgggtggat accgaaacgc acattaatac     420
   cacgagcgcg caatttctca gtaatttcag cgacaggata ttgcgcctgc gcgactgcca     480
   tgccgtaccc cggagtaatg atcactgaat gggagttttt cagcagttcc gctgtctctt     540
   ctgcggtgat ttcgcggtgc tcacccactt cctgatcatc gccagtagaa gagccgtcgg     600
   tgccgaaacc acccgcaata acgctgataa aggaacggtt catcgcctta cacataatgt     660
   aagaaaggat agcccccgaa gaaccgacca gcgcaccggt cacaatcagc aggtcgttgc     720
   tgagcataaa gcccgcagcc gcagccgccc agccggagta cgagttcagc atcgacacca     780
   ccactggcat atctgcacca ccgatggagg cgactaaatg ccagccgaat accagcgcaa 840
   ttgcggtcat tatcagcaat gccagcactt gcaggccgac gctgtccgtg cgaacaaata 900
   caatcagcag caggaaggaa acgaccagag ccgccaggtt cattttgtga cggtttggca 960
   gcatcaatgg tttagacgaa atcttgccac acagtttgcc gaacgccacc accgaacccg 1020
   tgaacgttac cgccccgatg aagataccga ggaacacttc cgtcaggtga atattgacca 1080
   gaatcggtgc cattcccgcg tcatgatgca gatagctgtt aaagccaacc agcactgccg 1140
   ccagacccac gaagctatgc aggatcgcca ccagttctgg catttcggtc atttcaactt 1200
   tcttcgccag acggatacca attgccccac caatgaccat cgccagcaag atccagccaa 1260
   cattacccgt atccggtcca aaaatggttg cgattaacgc aatcgccatc ccggcgatac 1320
   cgaagttgtt accctggcga gacgtttcat gtttcgaaag accggccaga ctgaagataa 1380
   acaggatcgc ggcaacaatg tatgcagctg taactaatcc tccagacata tgttacccct 1440
   taatttttgc ggaacatttt cagcatgcgc tgagtcacgg tgaagccacc gaaaatatta 1500
   atgctggcta taagcaccgc gataaaacta aggaagctaa cccagccgcc ctggccaatc 1560
   tgcaacagtg ctccgacaac aataatccct gaaatcgcgt tggtgaccga catcaacggt 1620
   gtatgcagcg cgtgcgatac attccacacc acgtaataac cgacaacgca ggccagcgcg 1680
   aaaacggtga agtgcccaag gaattctttc ggcgcaacgc ttgccatcca gccaaaaaga 1740
   atgattgcca gcgccatcaa cgcgtattta cgccacggtg agcaggtaca tttttcctca 1800
   gttttcactt ccggtgccgc tttttgtgcc gcctgcggct gagctgatac ctgaatcggc 1860
   ggtgccggcc aggtaatttc gcccgcacgg atcacggtca cgccgcgaat caccacatca 1920
   tcaaaatcaa cagtgatatt gccgtctttc tctttgcaca acagtttcag cagattaacg 1980
   aggtttgtgc cgtaaagctg tgaggattgc gtcggcagac ggcccggaag atcggtataa 2040
   ccaatcactt tgacaccatt ttccgtagtg aagatttcac ccggcacggt gtattcacag 2100
   ttgccgccgt tttgggctgc caggtcgaca atcacactgc ccgccttcat ggagtcaacc 2160
   atttcacggg taattagctt cggcgctggt ttgcctggaa taagcgcggt ggtgacaatg 2220
   atatcgacct cttttgcctg ggcggcaaag agttccattt ccgctttgat gaacgcgtcc 2280
   gacatcactt tggcatagcc atcgccgctg ccagcttcct ctttaaaatc cagctcgagg 2340
   aattccgcgc ccatactttg aacttgttct ttcacttccg ggcgggtgtc gaatgcacgc 2400
   acaatcgcgc cgagactgtt tgctgcgcca atggcggcca gacctgcaac acccgcacca 2460
   atcaccatca cttttgccgg tggcactttc ccggccgcag taatttgccc ggtaaagaag 2520
   cgcccaaatt catgtgccgc ttcaacaatg gcgcgataac cggcgatgtt cgccatcgag 2580
   cttagtgcgt ccagcgattg tgcgcgtgag atacgcggca cagagtccat cgccatcacg 2640
   gtcacgttac gttccgcaag tttttgcatt aattccggat tctgcgcagg ccagataaaa 2700
   ctcaccagcg ttgtcccagg attcagtaac gcaatttcat catctaacgg cgcattgacc 2760
   ttcagaatga tctctgactg ccagacgcta ttcccttcta caatttcagc gcccgcttgc 2820
   acaaacgctt tatcgtcaaa acttgccagt tgacccgcgc cgctctctac cgcgacggta 2880
   aaacccagtt tcagcagctg ttccactgtt tttggcgttg ctgcaacacg ggtttcattg 2940
   gttaaccgtt ctcttggtat gccaattcgc attttaattc tccacgctta taagcgaata 3000
   aaggaagatg gccgccccgc agggcagcag gtctgtgaaa cagtatagag attcatcggc 3060
   acaaaggctt tgctttttgt catttattca aaccaagggc gaattccaca ttggtcgctg 3120
   cagcccgggg gatccactag ttctagagcg gccgcaccgc gggagctaga gaattcgtca 3180
   acgaattcaa cgaattcaag cttgatatca ttcaggacga gcctcagact ccagcgtaac 3240
   tggactgaaa acaaactaaa gcgcccttgt ggcgctttag ttttgttccg cggccaccgg 3300
   ctggctcgct tcgctcggcc cgtggacaac cctgctggac aagctgatgg acaggctgcg 3360
   cctgcccacg agcttgacca cagggattgc ccaccggcta cccagccttc gaccacatac 3420
   ccaccggctc caactgcgcg gcctgcggcc ttgccccatc aattttttta attttctctg 3480
   gggaaaagcc tccggcctgc ggcctgcgcg cttcgcttgc cggttggaca ccaagtggaa 3540
   ggcgggtcaa ggctcgcgca gcgaccgcgc agcggcttgg ccttgacgcg cctggaacga 3600
   cccaagccta tgcgagtggg ggcagtcgaa ggcgaagccc gcccgcctgc cccccgagcc 3660
   tcacggcggc gagtgcgggg gttccaaggg ggcagcgcca ccttgggcaa ggccgaaggc 3720
   cgcgcagtcg atcaacaagc cccggagggg ccactttttg ccggaggggg agccgcgccg 3780
   aaggcgtggg ggaaccccgc aggggtgccc ttctttgggc accaaagaac tagatatagg 3840
   gcgaaatgcg aaagacttaa aaatcaacaa cttaaaaaag gggggtacgc aacagctcat 3900
   tgcggcaccc cccgcaatag ctcattgcgt aggttaaaga aaatctgtaa ttgactgcca 3960
   cttttacgca acgcataatt gttgtcgcgc tgccgaaaag ttgcagctga ttgcgcatgg 4020
   tgccgcaacc gtgcggcacc ctaccgcatg gagataagca tggccacgca gtccagagaa 4080
   atcggcattc aagccaagaa caagcccggt cactgggtgc aaacggaacg caaagcgcat 4140
   gaggcgtggg ccgggcttat tgcgaggaaa cccacggcgg caatgctgct gcatcacctc 4200
   gtggcgcaga tgggccacca gaacgccgtg gtggtcagcc agaagacact ttccaagctc 4260
   atcggacgtt ctttgcggac ggtccaatac gcagtcaagg acttggtggc cgagcgctgg 4320
   atctccgtcg tgaagctcaa cggccccggc accgtgtcgg cctacgtggt caatgaccgc 4380
   gtggcgtggg gccagccccg cgaccagttg cgcctgtcgg tgttcagtgc cgccgtggtg 4440
   gttgatcacg acgaccagga cgaatcgctg ttggggcatg gcgacctgcg ccgcatcccg 4500
   accctgtatc cgggcgagca gcaactaccg accggccccg gcgaggagcc gcccagccag 4560
   cccggcattc cgggcatgga accagacctg ccagccttga ccgaaacgga ggaatgggaa 4620
   cggcgcgggc agcagcgcct gccgatgccc gatgagccgt gttttctgga cgatggcgag 4680
   ccgttggagc cgccgacacg ggtcacgctg ccgcgccggt agtacgtaag aggttccaac 4740
   tttcaccata atgaaataag atcactaccg ggcgtatttt ttgagttatc gagattttca 4800
   ggagctaagg aagctaaaat ggagaaaaaa atcactggat ataccaccgt tgatatatcc 4860
   caatggcatc gtaaagaaca ttttgaggca tttcagtcag ttgctcaatg tacctataac 4920
   cagaccgttc agctggatat tacggccttt ttaaagaccg taaagaaaaa taagcacaag 4980
   ttttatccgg cctttattca cattcttgcc cgcctgatga atgctcatcc ggaattccgt 5040
   atggcaatga aagacggtga gctggtgata tgggatagtg ttcacccttg ttacaccgtt 5100
   ttccatgagc aaactgaaac gttttcatcg ctctggagtg aataccacga cgatttccgg 5160
   cagtttctac acatatattc gcaagatgtg gcgtgttacg gtgaaaacct ggcctatttc 5220
   cctaaagggt ttattgagaa tatgtttttc gtctcagcca atccctgggt gagtttcacc 5280
   agttttgatt taaacgtggc caatatggac aacttcttcg cccccgtttt caccatgggc 5340
   aaatattata cgcaaggcga caaggtgctg atgccgctgg cgattcaggt tcatcatgcc 5400
   gtttgtgatg gcttccatgt cggcagaatg cttaatgaat tacaacagta ctgcgatgag 5460
   tggcagggcg gggcgtaaac gcgtggatcc ccctcaagtc aaaagcctcc ggtcggaggc 5520
   ttttgacttt ctgctatgga ggtcaggtat gatttaaatg gtcagtattg agcgatatct 5580
   agagaattcg tc 5592
<210> 168
   <211> 7059
   <212> DNA
   <213> Artificial Sequence
<220>
   <223> Description of Artificial Sequence: Synthetic pTRC-Ptrc-mcr digest polynucleotide
<400> 168
   catggccggt acgggtcgtt tggctggtaa aattgcattg atcaccggtg gtgctggtaa      60
   cattggttcc gagctgaccc gccgttttct ggccgagggt gcgacggtta ttatcagcgg     120
   ccgtaaccgt gcgaagctga ccgcgctggc cgagcgcatg caagccgagg ccggcgtgcc     180
   ggccaagcgc attgatttgg aggtgatgga tggttccgac cctgtggctg tccgtgccgg     240
   tatcgaggca atcgtcgctc gccacggtca gattgacatt ctggttaaca acgcgggctc     300
   cgccggtgcc caacgtcgct tggcggaaat tccgctgacg gaggcagaat tgggtccggg     360
   tgcggaggag actttgcacg cttcgatcgc gaatctgttg ggcatgggtt ggcacctgat     420
   gcgtattgcg gctccgcaca tgccagttgg ctccgcagtt atcaacgttt cgactatttt     480
   ctcgcgcgca gagtactatg gtcgcattcc gtacgttacc ccgaaggcag cgctgaacgc 540
   tttgtcccag ctggctgccc gcgagctggg cgctcgtggc atccgcgtta acactatttt 600
   cccaggtcct attgagtccg accgcatccg taccgtgttt caacgtatgg atcaactgaa 660
   gggtcgcccg gagggcgaca ccgcccatca ctttttgaac accatgcgcc tgtgccgcgc 720
   aaacgaccaa ggcgctttgg aacgccgctt tccgtccgtt ggcgatgttg ctgatgcggc 780
   tgtgtttctg gcttctgctg agagcgcggc actgtcgggt gagacgattg aggtcaccca 840
   cggtatggaa ctgccggcgt gtagcgaaac ctccttgttg gcgcgtaccg atctgcgtac 900
   catcgacgcg agcggtcgca ctaccctgat ttgcgctggc gatcaaattg aagaagttat 960
   ggccctgacg ggcatgctgc gtacgtgcgg tagcgaagtg attatcggct tccgttctgc 1020
   ggctgccctg gcgcaatttg agcaggcagt gaatgaatct cgccgtctgg caggtgcgga 1080
   tttcaccccg ccgatcgctt tgccgttgga cccacgtgac ccggccacca ttgatgcggt 1140
   tttcgattgg ggcgcaggcg agaatacggg tggcatccat gcggcggtca ttctgccggc 1200
   aacctcccac gaaccggctc cgtgcgtgat tgaagtcgat gacgaacgcg tcctgaattt 1260
   cctggccgat gaaattaccg gcaccatcgt tattgcgagc cgtttggcgc gctattggca 1320
   atcccaacgc ctgaccccgg gtgcccgtgc ccgcggtccg cgtgttatct ttctgagcaa 1380
   cggtgccgat caaaatggta atgtttacgg tcgtattcaa tctgcggcga tcggtcaatt 1440
   gattcgcgtt tggcgtcacg aggcggagtt ggactatcaa cgtgcatccg ccgcaggcga 1500
   tcacgttctg ccgccggttt gggcgaacca gattgtccgt ttcgctaacc gctccctgga 1560
   aggtctggag ttcgcgtgcg cgtggaccgc acagctgctg cacagccaac gtcatattaa 1620
   cgaaattacg ctgaacattc cagccaatat tagcgcgacc acgggcgcac gttccgccag 1680
   cgtcggctgg gccgagtcct tgattggtct gcacctgggc aaggtggctc tgattaccgg 1740
   tggttcggcg ggcatcggtg gtcaaatcgg tcgtctgctg gccttgtctg gcgcgcgtgt 1800
   gatgctggcc gctcgcgatc gccataaatt ggaacagatg caagccatga ttcaaagcga 1860
   attggcggag gttggttata ccgatgtgga ggaccgtgtg cacatcgctc cgggttgcga 1920
   tgtgagcagc gaggcgcagc tggcagatct ggtggaacgt acgctgtccg cattcggtac 1980
   cgtggattat ttgattaata acgccggtat tgcgggcgtg gaggagatgg tgatcgacat 2040
   gccggtggaa ggctggcgtc acaccctgtt tgccaacctg atttcgaatt attcgctgat 2100
   gcgcaagttg gcgccgctga tgaagaagca aggtagcggt tacatcctga acgtttcttc 2160
   ctattttggc ggtgagaagg acgcggcgat tccttatccg aaccgcgccg actacgccgt 2220
   ctccaaggct ggccaacgcg cgatggcgga agtgttcgct cgtttcctgg gtccagagat 2280
   tcagatcaat gctattgccc caggtccggt tgaaggcgac cgcctgcgtg gtaccggtga 2340
   gcgtccgggc ctgtttgctc gtcgcgcccg tctgatcttg gagaataaac gcctgaacga 2400
   attgcacgcg gctttgattg ctgcggcccg caccgatgag cgctcgatgc acgagttggt 2460
   tgaattgttg ctgccgaacg acgtggccgc gttggagcag aacccagcgg cccctaccgc 2520
   gctgcgtgag ctggcacgcc gcttccgtag cgaaggtgat ccggcggcaa gctcctcgtc 2580
   cgccttgctg aatcgctcca tcgctgccaa gctgttggct cgcttgcata acggtggcta 2640
   tgtgctgccg gcggatattt ttgcaaatct gcctaatccg ccggacccgt tctttacccg 2700
   tgcgcaaatt gaccgcgaag ctcgcaaggt gcgtgatggt attatgggta tgctgtatct 2760
   gcagcgtatg ccaaccgagt ttgacgtcgc tatggcaacc gtgtactatc tggccgatcg 2820
   taacgtgagc ggcgaaactt tccatccgtc tggtggtttg cgctacgagc gtaccccgac 2880
   cggtggcgag ctgttcggcc tgccatcgcc ggaacgtctg gcggagctgg ttggtagcac 2940
   ggtgtacctg atcggtgaac acctgaccga gcacctgaac ctgctggctc gtgcctattt 3000
   ggagcgctac ggtgcccgtc aagtggtgat gattgttgag acggaaaccg gtgcggaaac 3060
   catgcgtcgt ctgttgcatg atcacgtcga ggcaggtcgc ctgatgacta ttgtggcagg 3120
   tgatcagatt gaggcagcga ttgaccaagc gatcacgcgc tatggccgtc cgggtccggt 3180
   ggtgtgcact ccattccgtc cactgccaac cgttccgctg gtcggtcgta aagactccga 3240
   ttggagcacc gttttgagcg aggcggaatt tgcggaactg tgtgagcatc agctgaccca 3300
   ccatttccgt gttgctcgta agatcgcctt gtcggatggc gcgtcgctgg cgttggttac 3360
   cccggaaacg actgcgacta gcaccacgga gcaatttgct ctggcgaact tcatcaagac 3420
   caccctgcac gcgttcaccg cgaccatcgg tgttgagtcg gagcgcaccg cgcaacgtat 3480
   tctgattaac caggttgatc tgacgcgccg cgcccgtgcg gaagagccgc gtgacccgca 3540
   cgagcgtcag caggaattgg aacgcttcat tgaagccgtt ctgctggtta ccgctccgct 3600
   gcctcctgag gcagacacgc gctacgcagg ccgtattcac cgcggtcgtg cgattaccgt 3660
   ctaatagaag cttggctgtt ttggcggatg agagaagatt ttcagcctga tacagattaa 3720
   atcagaacgc agaagcggtc tgataaaaca gaatttgcct ggcggcagta gcgcggtggt 3780
   cccacctgac cccatgccga actcagaagt gaaacgccgt agcgccgatg gtagtgtggg 3840
   gtctccccat gcgagagtag ggaactgcca ggcatcaaat aaaacgaaag gctcagtcga 3900
   aagactgggc ctttcgtttt atctgttgtt tgtcggtgaa cgctctcctg agtaggacaa 3960
   atccgccggg agcggatttg aacgttgcga agcaacggcc cggagggtgg cgggcaggac 4020
   gcccgccata aactgccagg catcaaatta agcagaaggc catcctgacg gatggccttt 4080
   ttgcgtttct acaaactctt tttgtttatt tttctaaata cattcaaata tgtatccgct 4140
   catgagacaa taaccctgat aaatgcttca ataatattga aaaaggaaga gtatgagtat 4200
   tcaacatttc cgtgtcgccc ttattccctt ttttgcggca ttttgccttc ctgtttttgc 4260
   tcacccagaa acgctggtga aagtaaaaga tgctgaagat cagttgggtg cacgagtggg 4320
   ttacatcgaa ctggatctca acagcggtaa gatccttgag agttttcgcc ccgaagaacg 4380
   ttttccaatg atgagcactt ttaaagttct gctatgtggc gcggtattat cccgtgttga 4440
   cgccgggcaa gagcaactcg gtcgccgcat acactattct cagaatgact tggttgagta 4500
   ctcaccagtc acagaaaagc atcttacgga tggcatgaca gtaagagaat tatgcagtgc 4560
   tgccataacc atgagtgata acactgcggc caacttactt ctgacaacga tcggaggacc 4620
   gaaggagcta accgcttttt tgcacaacat gggggatcat gtaactcgcc ttgatcgttg 4680
   ggaaccggag ctgaatgaag ccataccaaa cgacgagcgt gacaccacga tgcctgtagc 4740
   aatggcaaca acgttgcgca aactattaac tggcgaacta cttactctag cttcccggca 4800
   acaattaata gactggatgg aggcggataa agttgcagga ccacttctgc gctcggccct 4860
   tccggctggc tggtttattg ctgataaatc tggagccggt gagcgtgggt ctcgcggtat 4920
   cattgcagca ctggggccag atggtaagcc ctcccgtatc gtagttatct acacgacggc 4980
   cttcaactca gcaaaagttc gatttattca acaaagccac gttgtgtctc aaaatctctg 5040
   atgttacatt gcacaagata aaaatatatc atcatgaaca ataaaactgt ctgcttacat 5100
   aaacagtaat acaaggggtg ttatgagcca tattcaacgg gaaacgtctt gctcgaggcc 5160
   gcgattaaat tccaacatgg atgctgattt atatgggtat aaatgggctc gcgataatgt 5220
   cgggcaatca ggtgcgacaa tctatcgatt gtatgggaag cccgatgcgc cagagttgtt 5280
   tctgaaacat ggcaaaggta gcgttgccaa tgatgttaca gatgagatgg tcagactaaa 5340
   ctggctgacg gaatttatgc ctcttccgac catcaagcat tttatccgta ctcctgatga 5400
   tgcatggtta ctcaccactg cgatccccgg gaaaacagca ttccaggtat tagaagaata 5460
   tcctgattca ggtgaaaata ttgttgatgc gctggcagtg ttcctgcgcc ggttgcattc 5520
   gattcctgtt tgtaattgtc cttttaacag cgatcgcgta tttcgtctcg ctcaggcgca 5580
   atcacgaatg aataacggtt tggttgatgc gagtgatttt gatgacgagc gtaatggctg 5640
   gcctgttgaa caagtctgga aagaaatgca taagcttttg ccattctcac cggattcagt 5700
   cgtcactcat ggtgatttct cacttgataa ccttattttt gacgagggga aattaatagg 5760
   ttgtattgat gttggacgag tcggaatcgc agaccgatac caggatcttg ccatcctatg 5820
   gaactgcctc ggtgagtttt ctccttcatt acagaaacgg ctttttcaaa aatatggtat 5880
   tgataatcct gatatgaata aattgcagtt tcatttgatg ctcgatgagt ttttctaatc 5940
   gagtcaggca actatggatg aacgaaatag acagatcgct gagataggtg cctcactgat 6000
   taagcattgg taactgtcag accaagttta ctcatatata ctttagattg atttaaaact 6060
   tcatttttaa tttaaaagga tctaggtgaa gatccttttt gataatctca tgaccaaaat 6120
   cccttaacgt gagttttcgt tccactgagc gtcagacccc gtagaaaaga tcaaaggatc 6180
   ttcttgagat cctttttttc tgcgcgtaat ctgctgcttg caaacaaaaa aaccaccgct 6240
   accagcggtg gtttgtttgc cggatcaaga gctaccaact ctttttccga aggtaactgg 6300
   cttcagcaga gcgcagatac caaatactgt ccttctagtg tagccgtagt taggccacca 6360
   cttcaagaac tctgtagcac cgcctacata cctcgctctg ctaatcctgt taccagtggc 6420
   tgctgccagt ggcgataagt cgtgtcttac cgggttggac tcaagacgat agttaccgga 6480
   taaggcgcag cggtcgggct gaacgggggg ttcgtgcaca cagcccagct tggagcgaac 6540
   gacctacacc gaactgagat acctacagcg tgagctatga gaaagcgcca cgcttcccga 6600
   agggagaaag gcggacaggt atccggtaag cggcagggtc ggaacaggag agcgcacgag 6660
   ggagcttcca gggggaaacg cctggtatct ttatagtcct gtcgggtttc gccacctctg 6720
   acttgagcgt cgatttttgt gatgctcgtc aggggggcgg agcctatgga aaaacgccag 6780
   caacgcggcc tttttacggt tcctggcctt ttgctggcct tttgctcaca tgttctttcc 6840
   tgcgttatcc cctgattctg tggataaccg tattaccgcc tttgagtgag ctgataccgc 6900
   tcgccgcagc cgaacgaccg agcgcagcga gtcagtgagc gaggaagcgg aagagcgcct 6960
   gatgcggtat tttctcctta cgcatctgtg cggtatttca caccgcatat ggtgcactct 7020
   cagtacaatc tgctctgatg ccgcatagtt aagccagta 7059
<210> 169
   <211> 2172
   <212> DNA
   <213> Artificial Sequence
<220>
   <223> Description of Artificial Sequence: Synthetic pIDTSMART-PyibD polynucleotide
<400> 169
   cccgtgtaaa acgacggcca gtttatctag tcagcttgat tctagctgat cgtggaccgg 60
   aaggtgagcc agtgagttga ttgcagtcca gttacgctgg agtctgaggc tcgtcctgaa 120
   tgatatgcgg cctccacgtg cgtaattgtg ctgatctctt atatagctgc tctcattatc 180
   tctctaccct gaagtgactc tctcacctgt aaaaataata tctcacaggc ttaatagttt 240
   cttaatacaa agcctgtaaa acgtcaggat aacttctgtg taggaggata atccatggaa 300
   ttccgcacgt ggcgcgtgat cttacggcat tatacgtatg atcggtccac gatcagctag 360
   attatctagt cagcttgatg tcatagctgt ttcctgaggc tcaatactga ccatttaaat 420
   catacctgac ctccatagca gaaagtcaaa agcctccgac cggaggcttt tgacttgatc 480
   ggcacgtaag aggttccaac tttcaccata atgaaataag atcactaccg ggcgtatttt 540
   ttgagttatc gagattttca ggagctaagg aagctaaaat gagtattcaa catttccgtg 600
   tcgcccttat tccctttttt gcggcatttt gccttcctgt ttttgctcac ccagaaacgc 660
   tggtgaaagt aaaagatgct gaagatcagt tgggtgcacg agtgggttac atcgaactgg 720
   atctcaacag cggtaagatc cttgagagtt tacgccccga agaacgtttt ccaatgatga 780
   gcacttttaa agttctgcta tgtggcgcgg tattatcccg tattgacgcc gggcaagagc 840
   aactcggtcg ccgcatacac tattctcaga atgacttggt tgagtactca ccagtcacag 900
   aaaagcatct cacggatggc atgacagtaa gagaattatg cagtgctgcc ataaccatga 960
   gtgataacac tgcggccaac ttacttctgg caacgatcgg aggaccgaag gagctaaccg 1020
   cttttttgca caacatgggg gatcatgtaa ctcgccttga tcgttgggaa ccggagctga 1080
   atgaagccat accaaacgac gagcgtgaca ccacgatgcc tgtagcaatg gcaacaacgt 1140
   tgcgcaaact attaactggc gaactactta ctctagcttc ccggcaacaa ttaatagact 1200
   ggatggaggc ggataaagtt gcaggatcac ttctgcgctc ggccctcccg gctggctggt 1260
   ttattgctga taaatctgga gccggtgagc gtgggtctcg cggtatcatt gcagcactgg 1320
   ggccagatgg taagccctcc cgcatcgtag ttatctacac gacggggagt caggcaacta 1380
   tggatgaacg aaatagacag atcgctgaga taggtgcctc actgattaag cattggtaat 1440
   gagggcccaa atgtaatcac ctggctcacc ttcgggtggg cctttcttga ggacctaaat 1500
   gtaatcacct ggctcacctt cgggtgggcc tttctgcgtt gctggcgttt ttccataggc 1560
   tccgcccccc tgacgagcat cacaaaaatc gatgctcaag tcagaggtgg cgaaacccga 1620
   caggactata aagataccag gcgtttcccc ctggaagctc cctcgtgcgc tctcctgttc 1680
   cgaccctgcc gcttaccgga tacctgtccg cctttctccc ttcgggaagc gtggcgcttt 1740
   ctcatagctc acgctgtagg tatctcagtt cggtgtaggt cgttcgctcc aagctgggct 1800
   gtgtgcacga accccccgtt cagcccgacc gctgcgcctt atccggtaac tatcgtcttg 1860
   agtccaaccc ggtaagacac gacttatcgc cactggcagc agccactggt aacaggatta 1920
   gcagagcgag gtatgtaggc ggtgctacag agttcttgaa gtggtggcct aactacggct 1980
   acactagaag aacagtattt ggtatctgcg ctctgctgaa gccagttacc tcggaaaaag 2040
   agttggtagc tcttgatccg gcaaacaaac caccgctggt agcggtggtt tttttgtttg 2100
   caagcagcag attacgcgca gaaaaaaagg atctcaagaa gatcctttga ttttctaccg 2160
   aagaaaggcc ca 2172
<210> 170
   <211> 7215
   <212> DNA
   <213> Artificial Sequence
<220>
   <223> Description of Artificial Sequence: Synthetic pTRC-PyibD-mcr polynucleotide
<400> 170
   ccttcaactc agcaaaagtt cgatttattc aacaaagcca cgttgtgtct caaaatctct 60
   gatgttacat tgcacaagat aaaaatatat catcatgaac aataaaactg tctgcttaca 120
   taaacagtaa tacaaggggt gttatgagcc atattcaacg ggaaacgtct tgctcgaggc 180
   cgcgattaaa ttccaacatg gatgctgatt tatatgggta taaatgggct cgcgataatg 240
   tcgggcaatc aggtgcgaca atctatcgat tgtatgggaa gcccgatgcg ccagagttgt 300
   ttctgaaaca tggcaaaggt agcgttgcca atgatgttac agatgagatg gtcagactaa 360
   actggctgac ggaatttatg cctcttccga ccatcaagca ttttatccgt actcctgatg 420
   atgcatggtt actcaccact gcgatccccg ggaaaacagc attccaggta ttagaagaat 480
   atcctgattc aggtgaaaat attgttgatg cgctggcagt gttcctgcgc cggttgcatt 540
   cgattcctgt ttgtaattgt ccttttaaca gcgatcgcgt atttcgtctc gctcaggcgc 600
   aatcacgaat gaataacggt ttggttgatg cgagtgattt tgatgacgag cgtaatggct 660
   ggcctgttga acaagtctgg aaagaaatgc ataagctttt gccattctca ccggattcag 720
   tcgtcactca tggtgatttc tcacttgata accttatttt tgacgagggg aaattaatag 780
   gttgtattga tgttggacga gtcggaatcg cagaccgata ccaggatctt gccatcctat 840
   ggaactgcct cggtgagttt tctccttcat tacagaaacg gctttttcaa aaatatggta 900
   ttgataatcc tgatatgaat aaattgcagt ttcatttgat gctcgatgag tttttctaat 960
   cgagtcaggc aactatggat gaacgaaata gacagatcgc tgagataggt gcctcactga 1020
   ttaagcattg gtaactgtca gaccaagttt actcatatat actttagatt gatttaaaac 1080
   ttcattttta atttaaaagg atctaggtga agatcctttt tgataatctc atgaccaaaa 1140
   tcccttaacg tgagttttcg ttccactgag cgtcagaccc cgtagaaaag atcaaaggat 1200
   cttcttgaga tccttttttt ctgcgcgtaa tctgctgctt gcaaacaaaa aaaccaccgc 1260
   taccagcggt ggtttgtttg ccggatcaag agctaccaac tctttttccg aaggtaactg 1320
   gcttcagcag agcgcagata ccaaatactg tccttctagt gtagccgtag ttaggccacc 1380
   acttcaagaa ctctgtagca ccgcctacat acctcgctct gctaatcctg ttaccagtgg 1440
   ctgctgccag tggcgataag tcgtgtctta ccgggttgga ctcaagacga tagttaccgg 1500
   ataaggcgca gcggtcgggc tgaacggggg gttcgtgcac acagcccagc ttggagcgaa 1560
   cgacctacac cgaactgaga tacctacagc gtgagctatg agaaagcgcc acgcttcccg 1620
   aagggagaaa ggcggacagg tatccggtaa gcggcagggt cggaacagga gagcgcacga 1680
   gggagcttcc agggggaaac gcctggtatc tttatagtcc tgtcgggttt cgccacctct 1740
   gacttgagcg tcgatttttg tgatgctcgt caggggggcg gagcctatgg aaaaacgcca 1800
   gcaacgcggc ctttttacgg ttcctggcct tttgctggcc ttttgctcac atgttctttc 1860
   ctgcgttatc ccctgattct gtggataacc gtattaccgc ctttgagtga gctgataccg 1920
   ctcgccgcag ccgaacgacc gagcgcagcg agtcagtgag cgaggaagcg gaagagcgcc 1980
   tgatgcggta ttttctcctt acgcatctgt gcggtatttc acaccgcata tggtgcactc 2040
   tcagtacaat ctgctctgat gccgcatagt taagccagta gtgcgtaatt gtgctgatct 2100
   cttatatagc tgctctcatt atctctctac cctgaagtga ctctctcacc tgtaaaaata 2160
   atatctcaca ggcttaatag tttcttaata caaagcctgt aaaacgtcag gataacttct 2220
   gtgtaggagg ataatccatg gccggtacgg gtcgtttggc tggtaaaatt gcattgatca 2280
   ccggtggtgc tggtaacatt ggttccgagc tgacccgccg ttttctggcc gagggtgcga 2340
   cggttattat cagcggccgt aaccgtgcga agctgaccgc gctggccgag cgcatgcaag 2400
   ccgaggccgg cgtgccggcc aagcgcattg atttggaggt gatggatggt tccgaccctg 2460
   tggctgtccg tgccggtatc gaggcaatcg tcgctcgcca cggtcagatt gacattctgg 2520
   ttaacaacgc gggctccgcc ggtgcccaac gtcgcttggc ggaaattccg ctgacggagg 2580
   cagaattggg tccgggtgcg gaggagactt tgcacgcttc gatcgcgaat ctgttgggca 2640
   tgggttggca cctgatgcgt attgcggctc cgcacatgcc agttggctcc gcagttatca 2700
   acgtttcgac tattttctcg cgcgcagagt actatggtcg cattccgtac gttaccccga 2760
   aggcagcgct gaacgctttg tcccagctgg ctgcccgcga gctgggcgct cgtggcatcc 2820
   gcgttaacac tattttccca ggtcctattg agtccgaccg catccgtacc gtgtttcaac 2880
   gtatggatca actgaagggt cgcccggagg gcgacaccgc ccatcacttt ttgaacacca 2940
   tgcgcctgtg ccgcgcaaac gaccaaggcg ctttggaacg ccgctttccg tccgttggcg 3000
   atgttgctga tgcggctgtg tttctggctt ctgctgagag cgcggcactg tcgggtgaga 3060
   cgattgaggt cacccacggt atggaactgc cggcgtgtag cgaaacctcc ttgttggcgc 3120
   gtaccgatct gcgtaccatc gacgcgagcg gtcgcactac cctgatttgc gctggcgatc 3180
   aaattgaaga agttatggcc ctgacgggca tgctgcgtac gtgcggtagc gaagtgatta 3240
   tcggcttccg ttctgcggct gccctggcgc aatttgagca ggcagtgaat gaatctcgcc 3300
   gtctggcagg tgcggatttc accccgccga tcgctttgcc gttggaccca cgtgacccgg 3360
   ccaccattga tgcggttttc gattggggcg caggcgagaa tacgggtggc atccatgcgg 3420
   cggtcattct gccggcaacc tcccacgaac cggctccgtg cgtgattgaa gtcgatgacg 3480
   aacgcgtcct gaatttcctg gccgatgaaa ttaccggcac catcgttatt gcgagccgtt 3540
   tggcgcgcta ttggcaatcc caacgcctga ccccgggtgc ccgtgcccgc ggtccgcgtg 3600
   ttatctttct gagcaacggt gccgatcaaa atggtaatgt ttacggtcgt attcaatctg 3660
   cggcgatcgg tcaattgatt cgcgtttggc gtcacgaggc ggagttggac tatcaacgtg 3720
   catccgccgc aggcgatcac gttctgccgc cggtttgggc gaaccagatt gtccgtttcg 3780
   ctaaccgctc cctggaaggt ctggagttcg cgtgcgcgtg gaccgcacag ctgctgcaca 3840
   gccaacgtca tattaacgaa attacgctga acattccagc caatattagc gcgaccacgg 3900
   gcgcacgttc cgccagcgtc ggctgggccg agtccttgat tggtctgcac ctgggcaagg 3960
   tggctctgat taccggtggt tcggcgggca tcggtggtca aatcggtcgt ctgctggcct 4020
   tgtctggcgc gcgtgtgatg ctggccgctc gcgatcgcca taaattggaa cagatgcaag 4080
   ccatgattca aagcgaattg gcggaggttg gttataccga tgtggaggac cgtgtgcaca 4140
   tcgctccggg ttgcgatgtg agcagcgagg cgcagctggc agatctggtg gaacgtacgc 4200
   tgtccgcatt cggtaccgtg gattatttga ttaataacgc cggtattgcg ggcgtggagg 4260
   agatggtgat cgacatgccg gtggaaggct ggcgtcacac cctgtttgcc aacctgattt 4320
   cgaattattc gctgatgcgc aagttggcgc cgctgatgaa gaagcaaggt agcggttaca 4380
   tcctgaacgt ttcttcctat tttggcggtg agaaggacgc ggcgattcct tatccgaacc 4440
   gcgccgacta cgccgtctcc aaggctggcc aacgcgcgat ggcggaagtg ttcgctcgtt 4500
   tcctgggtcc agagattcag atcaatgcta ttgccccagg tccggttgaa ggcgaccgcc 4560
   tgcgtggtac cggtgagcgt ccgggcctgt ttgctcgtcg cgcccgtctg atcttggaga 4620
   ataaacgcct gaacgaattg cacgcggctt tgattgctgc ggcccgcacc gatgagcgct 4680
   cgatgcacga gttggttgaa ttgttgctgc cgaacgacgt ggccgcgttg gagcagaacc 4740
   cagcggcccc taccgcgctg cgtgagctgg cacgccgctt ccgtagcgaa ggtgatccgg 4800
   cggcaagctc ctcgtccgcc ttgctgaatc gctccatcgc tgccaagctg ttggctcgct 4860
   tgcataacgg tggctatgtg ctgccggcgg atatttttgc aaatctgcct aatccgccgg 4920
   acccgttctt tacccgtgcg caaattgacc gcgaagctcg caaggtgcgt gatggtatta 4980
   tgggtatgct gtatctgcag cgtatgccaa ccgagtttga cgtcgctatg gcaaccgtgt 5040
   actatctggc cgatcgtaac gtgagcggcg aaactttcca tccgtctggt ggtttgcgct 5100
   acgagcgtac cccgaccggt ggcgagctgt tcggcctgcc atcgccggaa cgtctggcgg 5160
   agctggttgg tagcacggtg tacctgatcg gtgaacacct gaccgagcac ctgaacctgc 5220
   tggctcgtgc ctatttggag cgctacggtg cccgtcaagt ggtgatgatt gttgagacgg 5280
   aaaccggtgc ggaaaccatg cgtcgtctgt tgcatgatca cgtcgaggca ggtcgcctga 5340
   tgactattgt ggcaggtgat cagattgagg cagcgattga ccaagcgatc acgcgctatg 5400
   gccgtccggg tccggtggtg tgcactccat tccgtccact gccaaccgtt ccgctggtcg 5460
   gtcgtaaaga ctccgattgg agcaccgttt tgagcgaggc ggaatttgcg gaactgtgtg 5520
   agcatcagct gacccaccat ttccgtgttg ctcgtaagat cgccttgtcg gatggcgcgt 5580
   cgctggcgtt ggttaccccg gaaacgactg cgactagcac cacggagcaa tttgctctgg 5640
   cgaacttcat caagaccacc ctgcacgcgt tcaccgcgac catcggtgtt gagtcggagc 5700
   gcaccgcgca acgtattctg attaaccagg ttgatctgac gcgccgcgcc cgtgcggaag 5760
   agccgcgtga cccgcacgag cgtcagcagg aattggaacg cttcattgaa gccgttctgc 5820
   tggttaccgc tccgctgcct cctgaggcag acacgcgcta cgcaggccgt attcaccgcg 5880
   gtcgtgcgat taccgtctaa tagaagcttg gctgttttgg cggatgagag aagattttca 5940
   gcctgataca gattaaatca gaacgcagaa gcggtctgat aaaacagaat ttgcctggcg 6000
   gcagtagcgc ggtggtccca cctgacccca tgccgaactc agaagtgaaa cgccgtagcg 6060
   ccgatggtag tgtggggtct ccccatgcga gagtagggaa ctgccaggca tcaaataaaa 6120
   cgaaaggctc agtcgaaaga ctgggccttt cgttttatct gttgtttgtc ggtgaacgct 6180
   ctcctgagta ggacaaatcc gccgggagcg gatttgaacg ttgcgaagca acggcccgga 6240
   gggtggcggg caggacgccc gccataaact gccaggcatc aaattaagca gaaggccatc 6300
   ctgacggatg gcctttttgc gtttctacaa actctttttg tttatttttc taaatacatt 6360
   caaatatgta tccgctcatg agacaataac cctgataaat gcttcaataa tattgaaaaa 6420
   ggaagagtat gagtattcaa catttccgtg tcgcccttat tccctttttt gcggcatttt 6480
   gccttcctgt ttttgctcac ccagaaacgc tggtgaaagt aaaagatgct gaagatcagt 6540
   tgggtgcacg agtgggttac atcgaactgg atctcaacag cggtaagatc cttgagagtt 6600
   ttcgccccga agaacgtttt ccaatgatga gcacttttaa agttctgcta tgtggcgcgg 6660
   tattatcccg tgttgacgcc gggcaagagc aactcggtcg ccgcatacac tattctcaga 6720
   atgacttggt tgagtactca ccagtcacag aaaagcatct tacggatggc atgacagtaa 6780
   gagaattatg cagtgctgcc ataaccatga gtgataacac tgcggccaac ttacttctga 6840
   caacgatcgg aggaccgaag gagctaaccg cttttttgca caacatgggg gatcatgtaa 6900
   ctcgccttga tcgttgggaa ccggagctga atgaagccat accaaacgac gagcgtgaca 6960
   ccacgatgcc tgtagcaatg gcaacaacgt tgcgcaaact attaactggc gaactactta 7020
   ctctagcttc ccggcaacaa ttaatagact ggatggaggc ggataaagtt gcaggaccac 7080
   ttctgcgctc ggcccttccg gctggctggt ttattgctga taaatctgga gccggtgagc 7140
   gtgggtctcg cggtatcatt gcagcactgg ggccagatgg taagccctcc cgtatcgtag 7200
   ttatctacac gacgg 7215
<210> 171
   <211> 75
   <212> DNA
   <213> Artificial Sequence
<220>
   <223> Description of Artificial Sequence: Synthetic PT5 oligonucleotide
<400> 171
   taactactta aaaatttcag ttgcttaatc ctacaattct tgatataata ttctcatagt 60
   ttgaaagaac tattc 75
<210> 172
   <211> 6421
   <212> DNA
   <213> Artificial Sequence
<220>
   <223> Description of Artificial Sequence: Synthetic T5-aceEF-lpd polynucleotide
<400> 172
   agatcttaac tacttaaaaa tttcagttgc ttaatcctac aattcttgat ataatattct      60
   catagtttga aagaactatt cagatagata aggaataacc catgtcagaa cgtttcccaa     120
   atgacgtgga tccgatcgaa actcgcgact ggctccaggc gatcgaatcg gtcatccgtg     180
   aagaaggtgt tgagcgtgct cagtatctga tcgaccaact gcttgctgaa gcccgcaaag     240
   gcggtgtaaa cgtagccgca ggcacaggta tcagcaacta catcaacacc atccccgttg     300
   aagaacaacc ggagtatccg ggtaatctgg aactggaacg ccgtattcgt tcagctatcc     360
   gctggaacgc catcatgacg gtgctgcgtg cgtcgaaaaa agacctcgaa ctgggcggcc     420
   atatggcgtc cttccagtct tccgcaacca tttatgatgt gtgctttaac cacttcttcc     480
   gtgcacgcaa cgagcaggat ggcggcgacc tggtttactt ccagggccac atctccccgg     540
   gcgtgtacgc tcgtgctttc ctggaaggtc gtctgactca ggagcagctg gataacttcc     600
   gtcaggaagt tcacggcaat ggcctctctt cctatccgca cccgaaactg atgccggaat     660
   tctggcagtt cccgaccgta tctatgggtc tgggtccgat tggtgctatt taccaggcta     720
   aattcctgaa atatctggaa caccgtggcc tgaaagatac ctctaaacaa accgtttacg     780
   cgttcctcgg tgacggtgaa atggacgaac cggaatccaa aggtgcgatc accatcgcta     840
   cccgtgaaaa actggataac ctggtcttcg ttatcaactg taacctgcag cgtcttgacg     900
   gcccggtcac cggtaacggc aagatcatca acgaactgga aggcatcttc gaaggtgctg     960
   gctggaacgt gatcaaagtg atgtggggta gccgttggga tgaactgctg cgtaaggata    1020
   ccagcggtaa actgatccag ctgatgaacg aaaccgttga cggcgactac cagaccttca    1080
   aatcgaaaga tggtgcgtac gttcgtgaac acttcttcgg taaatatcct gaaaccgcag    1140
   cactggttgc agactggact gacgagcaga tctgggcact gaaccgtggt ggtcacgatc    1200
   cgaagaaaat ctacgctgca ttcaagaaag cgcaggaaac caaaggcaaa gcgacagtaa    1260
   tccttgctca taccattaaa ggttacggca tgggcgacgc ggctgaaggt aaaaacatcg    1320
   cgcaccaggt taagaaaatg aacatggacg gtgtgcgtca tatccgcgac cgtttcaatg    1380
   tgccggtgtc tgatgcagat atcgaaaaac tgccgtacat caccttcccg gaaggttctg 1440
   aagagcatac ctatctgcac gctcagcgtc agaaactgca cggttatctg ccaagccgtc 1500
   agccgaactt caccgagaag cttgagctgc cgagcctgca agacttcggc gcgctgttgg 1560
   aagagcagag caaagagatc tctaccacta tcgctttcgt tcgtgctctg aacgtgatgc 1620
   tgaagaacaa gtcgatcaaa gatcgtctgg taccgatcat cgccgacgaa gcgcgtactt 1680
   tcggtatgga aggtctgttc cgtcagattg gtatttacag cccgaacggt cagcagtaca 1740
   ccccgcagga ccgcgagcag gttgcttact ataaagaaga cgagaaaggt cagattctgc 1800
   aggaagggat caacgagctg ggcgcaggtt gttcctggct ggcagcggcg acctcttaca 1860
   gcaccaacaa tctgccgatg atcccgttct acatctatta ctcgatgttc ggcttccagc 1920
   gtattggcga tctgtgctgg gcggctggcg accagcaagc gcgtggcttc ctgatcggcg 1980
   gtacttccgg tcgtaccacc ctgaacggcg aaggtctgca gcacgaagat ggtcacagcc 2040
   acattcagtc gctgactatc ccgaactgta tctcttacga cccggcttac gcttacgaag 2100
   ttgctgtcat catgcatgac ggtctggagc gtatgtacgg tgaaaaacaa gagaacgttt 2160
   actactacat cactacgctg aacgaaaact accacatgcc ggcaatgccg gaaggtgctg 2220
   aggaaggtat ccgtaaaggt atctacaaac tcgaaactat tgaaggtagc aaaggtaaag 2280
   ttcagctgct cggctccggt tctatcctgc gtcacgtccg tgaagcagct gagatcctgg 2340
   cgaaagatta cggcgtaggt tctgacgttt atagcgtgac ctccttcacc gagctggcgc 2400
   gtgatggtca ggattgtgaa cgctggaaca tgctgcaccc gctggaaact ccgcgcgttc 2460
   cgtatatcgc tcaggtgatg aacgacgctc cggcagtggc atctaccgac tatatgaaac 2520
   tgttcgctga gcaggtccgt acttacgtac cggctgacga ctaccgcgta ctgggtactg 2580
   atggcttcgg tcgttccgac agccgtgaga acctgcgtca ccacttcgaa gttgatgctt 2640
   cttatgtcgt ggttgcggcg ctgggcgaac tggctaaacg tggcgaaatc gataagaaag 2700
   tggttgctga cgcaatcgcc aaattcaaca tcgatgcaga taaagttaac ccgcgtctgg 2760
   cgtaagaggt aaaagaataa tggctatcga aatcaaagta ccggacatcg gggctgatga 2820
   agttgaaatc accgagatcc tggtcaaagt gggcgacaaa gttgaagccg aacagtcgct 2880
   gatcaccgta gaaggcgaca aagcctctat ggaagttccg tctccgcagg cgggtatcgt 2940
   taaagagatc aaagtctctg ttggcgataa aacccagacc ggcgcactga ttatgatttt 3000
   cgattccgcc gacggtgcag cagacgctgc acctgctcag gcagaagaga agaaagaagc 3060
   agctccggca gcagcaccag cggctgcggc ggcaaaagac gttaacgttc cggatatcgg 3120
   cagcgacgaa gttgaagtga ccgaaatcct ggtgaaagtt ggcgataaag ttgaagctga 3180
   acagtcgctg atcaccgtag aaggcgacaa ggcttctatg gaagttccgg ctccgtttgc 3240
   tggcaccgtg aaagagatca aagtgaacgt gggtgacaaa gtgtctaccg gctcgctgat 3300
   tatggtcttc gaagtcgcgg gtgaagcagg cgcggcagct ccggccgcta aacaggaagc 3360
   agctccggca gcggcccctg caccagcggc tggcgtgaaa gaagttaacg ttccggatat 3420
   cggcggtgac gaagttgaag tgactgaagt gatggtgaaa gtgggcgaca aagttgccgc 3480
   tgaacagtca ctgatcaccg tagaaggcga caaagcttct atggaagttc cggcgccgtt 3540
   tgcaggcgtc gtgaaggaac tgaaagtcaa cgttggcgat aaagtgaaaa ctggctcgct 3600
   gattatgatc ttcgaagttg aaggcgcagc gcctgcggca gctcctgcga aacaggaagc 3660
   ggcagcgccg gcaccggcag caaaagctga agccccggca gcagcaccag ctgcgaaagc 3720
   ggaaggcaaa tctgaatttg ctgaaaacga cgcttatgtt cacgcgactc cgctgatccg 3780
   ccgtctggca cgcgagtttg gtgttaacct tgcgaaagtg aagggcactg gccgtaaagg 3840
   tcgtatcctg cgcgaagacg ttcaggctta cgtgaaagaa gctatcaaac gtgcagaagc 3900
   agctccggca gcgactggcg gtggtatccc tggcatgctg ccgtggccga aggtggactt 3960
   cagcaagttt ggtgaaatcg aagaagtgga actgggccgc atccagaaaa tctctggtgc 4020
   gaacctgagc cgtaactggg taatgatccc gcatgttact cacttcgaca aaaccgatat 4080
   caccgagttg gaagcgttcc gtaaacagca gaacgaagaa gcggcgaaac gtaagctgga 4140
   tgtgaagatc accccggttg tcttcatcat gaaagccgtt gctgcagctc ttgagcagat 4200
   gcctcgcttc aatagttcgc tgtcggaaga cggtcagcgt ctgaccctga agaaatacat 4260
   caacatcggt gtggcggtgg ataccccgaa cggtctggtt gttccggtat tcaaagacgt 4320
   caacaagaaa ggcatcatcg agctgtctcg cgagctgatg actatttcta agaaagcgcg 4380
   tgacggtaag ctgactgcgg gcgaaatgca gggcggttgc ttcaccatct ccagcatcgg 4440
   cggcctgggt actacccact tcgcgccgat tgtgaacgcg ccggaagtgg ctatcctcgg 4500
   cgtttccaag tccgcgatgg agccggtgtg gaatggtaaa gagttcgtgc cgcgtctgat 4560
   gctgccgatt tctctctcct tcgaccaccg cgtgatcgac ggtgctgatg gtgcccgttt 4620
   cattaccatc attaacaaca cgctgtctga cattcgccgt ctggtgatgt aagtaaaaga 4680
   gccggcccaa cggccggctt ttttctggta atctcatgaa tgtattgagg ttattagcga 4740
   atagacaaat cggttgccgt ttgttgttta aaaattgtta acaattttgt aaaataccga 4800
   cggatagaac gacccggtgg tggttagggt attacttcac ataccctatg gatttctggg 4860
   tgcagcaagg tagcaagcgc cagaatcccc aggagcttac ataagtaagt gactggggtg 4920
   agggcgtgaa gctaacgccg ctgcggcctg aaagacgacg ggtatgaccg ccggagataa 4980
   atatatagag gtcatgatga gtactgaaat caaaactcag gtcgtggtac ttggggcagg 5040
   ccccgcaggt tactccgctg ccttccgttg cgctgattta ggtctggaaa ccgtaatcgt 5100
   agaacgttac aacacccttg gcggtgtttg cctgaacgtc ggctgtatcc cttctaaagc 5160
   actgctgcac gtagcaaaag ttatcgaaga agccaaagcg ctggctgaac acggtatcgt 5220
   cttcggcgaa ccgaaaaccg atatcgacaa gattcgtacc tggaaagaga aagtgatcaa 5280
   tcagctgacc ggtggtctgg ctggtatggc gaaaggccgc aaagtcaaag tggtcaacgg 5340
   tctgggtaaa ttcaccgggg ctaacaccct ggaagttgaa ggtgagaacg gcaaaaccgt 5400
   gatcaacttc gacaacgcga tcattgcagc gggttctcgc ccgatccaac tgccgtttat 5460
   tccgcatgaa gatccgcgta tctgggactc cactgacgcg ctggaactga aagaagtacc 5520
   agaacgcctg ctggtaatgg gtggcggtat catcggtctg gaaatgggca ccgtttacca 5580
   cgcgctgggt tcacagattg acgtggttga aatgttcgac caggttatcc cggcagctga 5640
   caaagacatc gttaaagtct tcaccaagcg tatcagcaag aaattcaacc tgatgctgga 5700
   aaccaaagtt accgccgttg aagcgaaaga agacggcatt tatgtgacga tggaaggcaa 5760
   aaaagcaccc gctgaaccgc agcgttacga cgccgtgctg gtagcgattg gtcgtgtgcc 5820
   gaacggtaaa aacctcgacg caggcaaagc aggcgtggaa gttgacgacc gtggtttcat 5880
   ccgcgttgac aaacagctgc gtaccaacgt accgcacatc tttgctatcg gcgatatcgt 5940
   cggtcaaccg atgctggcac acaaaggtgt tcacgaaggt cacgttgccg ctgaagttat 6000
   cgccggtaag aaacactact tcgatccgaa agttatcccg tccatcgcct ataccgaacc 6060
   agaagttgca tgggtgggtc tgactgagaa agaagcgaaa gagaaaggca tcagctatga 6120
   aaccgccacc ttcccgtggg ctgcttctgg tcgtgctatc gcttccgact gcgcagacgg 6180
   tatgaccaag ctgattttcg acaaagaatc tcaccgtgtg atcggtggtg cgattgtcgg 6240
   tactaacggc ggcgagctgc tgggtgaaat cggcctggca atcgaaatgg gttgtgatgc 6300
   tgaagacatc gcactgacca tccacgcgca cccgactctg cacgagtctg tgggcctggc 6360
   ggcagaagtg ttcgaaggta gcattaccga cctgccgaac ccgaaagcga agaagaagta 6420
   a 6421
<210> 173
   <211> 951
   <212> DNA
   <213> Artificial Sequence
<220>
   <223> Description of Artificial Sequence: Synthetic coaA polynucleotide
<400> 173
   atgagtataa aagagcaaac gttaatgacg ccttacctac agtttgaccg caaccagtgg 60
   gcagctctgc gtgattccgt acctatgacg ttatcggaag atgagatcgc ccgtctcaaa 120
   ggtattaatg aagatctctc gttagaagaa gttgccgaga tctatttacc tttgtcacgt 180
   ttgctgaact tctatataag ctcgaatctg cgccgtcagg cagttctgga acagtttctt 240
   ggtaccaacg ggcaacgcat tccttacatt atcagtattg ctggcagtgt cgcggtgggg 300
   aaaagtacaa cggcggctgt gctccaggcg ctattaagcc gttggccgga acatcgtcgt 360
   gttgaactga tcactacaga tggcttcctt caccctaatc aggttctgaa agaacgtggt 420
   ctgatgaaga agaaaggctt cccggaatcg tatgatatgc atcgcctggt gaagtttgtt 480
   tccgatctca aatccggcgt gccaaacgtt acagcacctg tttactcaca tcttatttat 540
   gatgtgatcc cggatggaga taaaacggtt gttcagcctg atattttaat tcttgaaggg 600
   ttaaatgtct tacagagcgg gatggattat ccacacgatc cacatcatgt atttgtttct 660
   gattttgtcg atttttcgat atatgttgat gcaccggaag acttacttca gacatggtat 720
   atcaaccgtt ttctgaaatt ccgcgaaggg gcttttaccg acccggattc ctattttcat 780
   aactacgcga aattaactaa agaagaagcg attaagactg ccatgacatt gtggaaagag 840
   atcaactggc tgaacttaaa gcaaaatatt ctacctactc gtgagcgcgc cagtttaatc 900
   ctgacgaaaa gtgctaatca tgcggtagaa gaggtcagac tacgcaaata a 951
<210> 174
   <211> 10138
   <212> DNA
   <213> Artificial Sequence
<220>
   <223> Description of Artificial Sequence: Synthetic pTRC-Ptrc-mcr-Pkan-gapA polynucleotide
<400> 174
   ccttcaactc agcaaaagtt cgatttattc aacaaagcca cgttgtgtct caaaatctct      60
   gatgttacat tgcacaagat aaaaatatat catcatgaac aataaaactg tctgcttaca     120
   taaacagtaa tacaaggggt gttatgagcc atattcaacg ggaaacgtct tgctcgaggc     180
   cgcgattaaa ttccaacatg gatgctgatt tatatgggta taaatgggct cgcgataatg     240
   tcgggcaatc aggtgcgaca atctatcgat tgtatgggaa gcccgatgcg ccagagttgt     300
   ttctgaaaca tggcaaaggt agcgttgcca atgatgttac agatgagatg gtcagactaa     360
   actggctgac ggaatttatg cctcttccga ccatcaagca ttttatccgt actcctgatg     420
   atgcatggtt actcaccact gcgatccccg ggaaaacagc attccaggta ttagaagaat     480
   atcctgattc aggtgaaaat attgttgatg cgctggcagt gttcctgcgc cggttgcatt     540
   cgattcctgt ttgtaattgt ccttttaaca gcgatcgcgt atttcgtctc gctcaggcgc     600
   aatcacgaat gaataacggt ttggttgatg cgagtgattt tgatgacgag cgtaatggct     660
   ggcctgttga acaagtctgg aaagaaatgc ataagctttt gccattctca ccggattcag     720
   tcgtcactca tggtgatttc tcacttgata accttatttt tgacgagggg aaattaatag     780
   gttgtattga tgttggacga gtcggaatcg cagaccgata ccaggatctt gccatcctat     840
   ggaactgcct cggtgagttt tctccttcat tacagaaacg gctttttcaa aaatatggta 900
   ttgataatcc tgatatgaat aaattgcagt ttcatttgat gctcgatgag tttttctaat 960
   cgagtcaggc aactatggat gaacgaaata gacagatcgc tgagataggt gcctcactga 1020
   ttaagcattg gtaactgtca gaccaagttt actcatatat actttagatt gatttaaaac 1080
   ttcattttta atttaaaagg atctaggtga agatcctttt tgataatctc atgaccaaaa 1140
   tcccttaacg tgagttttcg ttccactgag cgtcagaccc cgtagaaaag atcaaaggat 1200
   cttcttgaga tccttttttt ctgcgcgtaa tctgctgctt gcaaacaaaa aaaccaccgc 1260
   taccagcggt ggtttgtttg ccggatcaag agctaccaac tctttttccg aaggtaactg 1320
   gcttcagcag agcgcagata ccaaatactg tccttctagt gtagccgtag ttaggccacc 1380
   acttcaagaa ctctgtagca ccgcctacat acctcgctct gctaatcctg ttaccagtgg 1440
   ctgctgccag tggcgataag tcgtgtctta ccgggttgga ctcaagacga tagttaccgg 1500
   ataaggcgca gcggtcgggc tgaacggggg gttcgtgcac acagcccagc ttggagcgaa 1560
   cgacctacac cgaactgaga tacctacagc gtgagctatg agaaagcgcc acgcttcccg 1620
   aagggagaaa ggcggacagg tatccggtaa gcggcagggt cggaacagga gagcgcacga 1680
   gggagcttcc agggggaaac gcctggtatc tttatagtcc tgtcgggttt cgccacctct 1740
   gacttgagcg tcgatttttg tgatgctcgt caggggggcg gagcctatgg aaaaacgcca 1800
   gcaacgcggc ctttttacgg ttcctggcct tttgctggcc ttttgctcac atgttctttc 1860
   ctgcgttatc ccctgattct gtggataacc gtattaccgc ctttgagtga gctgataccg 1920
   ctcgccgcag ccgaacgacc gagcgcagcg agtcagtgag cgaggaagcg gaagagcgcc 1980
   tgatgcggta ttttctcctt acgcatctgt gcggtatttc acaccgcata cagtccagtt 2040
   acgctggagt ctgaggctcg tcctgaatga tatcaagctt ccggaattgc cagctggggc 2100
   gccctctggt aaggttggga agccctgcaa agtaaactgg atggctttct tgccgccaag 2160
   gatctgatgg cgcaggggat caagctctga tcaagagaca ggatgaggat cgtttcgatg 2220
   actatcaaag taggtatcaa cggttttggc cgtatcggtc gcattgtttt ccgtgctgct 2280
   cagaaacgtt ctgacatcga gatcgttgca atcaacgacc tgttagacgc tgattacatg 2340
   gcatacatgc tgaaatatga ctccactcac ggccgtttcg acggtaccgt tgaagtgaaa 2400
   gacggtcatc tgatcgttaa cggtaaaaaa atccgtgtta ccgctgaacg tgatccggct 2460
   aacctgaaat gggacgaagt tggtgttgac gttgtcgctg aagcaactgg tctgttcctg 2520
   actgacgaaa ctgctcgtaa acacatcacc gctggtgcga agaaagtggt tatgactggt 2580
   ccgtctaaag acaacactcc gatgttcgtt aaaggcgcta acttcgacaa atatgctggc 2640
   caggacatcg tttccaacgc ttcctgcacc accaactgcc tggctccgct ggctaaagtt 2700
   atcaacgata acttcggcat catcgaaggt ctgatgacca ccgttcacgc tactaccgct 2760
   actcagaaaa ccgttgatgg cccgtctcac aaagactggc gcggcggccg cggcgcttcc 2820
   cagaacatca tcccgtcctc taccggtgct gctaaagctg taggtaaagt actgccagaa 2880
   ctgaatggca aactgactgg tatggcgttc cgcgttccga ccccgaacgt atctgtagtt 2940
   gacctgaccg ttcgtctgga aaaagctgca acttacgagc agatcaaagc tgccgttaaa 3000
   gctgctgctg aaggcgaaat gaaaggcgtt ctgggctaca ccgaagatga cgtagtatct 3060
   accgatttca acggcgaagt ttgcacttcc gtgttcgatg ctaaagctgg tatcgctctg 3120
   aacgacaact tcgtgaaact ggtatcctgg tacgacaacg aaaccggtta ctccaacaaa 3180
   gttctggacc tgatcgctca catctccaaa taagatccta gacgaattct ctagatatcg 3240
   ctcaatactg accatttaaa tcatacctga cctatggtgc actctcagta caatctgctc 3300
   tgatgccgca tagttaagcc agtatacact ccgctatcgc tacgtgactg ggtcatggct 3360
   gcgccccgac acccgccaac acccgctgac gcgccctgac gggcttgtct gctcccggca 3420
   tccgcttaca gacaagctgt gaccgtctcc gggagctgca tgtgtcagag gttttcaccg 3480
   tcatcaccga aacgcgcgag gcagcagatc aattcgcgcg cgaaggcgaa gcggcatgca 3540
   tttacgttga caccatcgaa tggtgcaaaa cctttcgcgg tatggcatga tagcgcccgg 3600
   aagagagtca attcagggtg gtgaatgtga aaccagtaac gttatacgat gtcgcagagt 3660
   atgccggtgt ctcttatcag accgtttccc gcgtggtgaa ccaggccagc cacgtttctg 3720
   cgaaaacgcg ggaaaaagtg gaagcggcga tggcggagct gaattacatt cccaaccgcg 3780
   tggcacaaca actggcgggc aaacagtcgt tgctgattgg cgttgccacc tccagtctgg 3840
   ccctgcacgc gccgtcgcaa attgtcgcgg cgattaaatc tcgcgccgat caactgggtg 3900
   ccagcgtggt ggtgtcgatg gtagaacgaa gcggcgtcga agcctgtaaa gcggcggtgc 3960
   acaatcttct cgcgcaacgc gtcagtgggc tgatcattaa ctatccgctg gatgaccagg 4020
   atgccattgc tgtggaagct gcctgcacta atgttccggc gttatttctt gatgtctctg 4080
   accagacacc catcaacagt attattttct cccatgaaga cggtacgcga ctgggcgtgg 4140
   agcatctggt cgcattgggt caccagcaaa tcgcgctgtt agcgggccca ttaagttctg 4200
   tctcggcgcg tctgcgtctg gctggctggc ataaatatct cactcgcaat caaattcagc 4260
   cgatagcgga acgggaaggc gactggagtg ccatgtccgg ttttcaacaa accatgcaaa 4320
   tgctgaatga gggcatcgtt cccactgcga tgctggttgc caacgatcag atggcgctgg 4380
   gcgcaatgcg cgccattacc gagtccgggc tgcgcgttgg tgcggatatc tcggtagtgg 4440
   gatacgacga taccgaagac agctcatgtt atatcccgcc gtcaaccacc atcaaacagg 4500
   attttcgcct gctggggcaa accagcgtgg accgcttgct gcaactctct cagggccagg 4560
   cggtgaaggg caatcagctg ttgcccgtct cactggtgaa aagaaaaacc accctggcgc 4620
   ccaatacgca aaccgcctct ccccgcgcgt tggccgattc attaatgcag ctggcacgac 4680
   aggtttcccg actggaaagc gggcagtgag cgcaacgcaa ttaatgtgag ttagcgcgaa 4740
   ttgatctggt ttgacagctt atcatcgact gcacggtgca ccaatgcttc tggcgtcagg 4800
   cagccatcgg aagctgtggt atggctgtgc aggtcgtaaa tcactgcata attcgtgtcg 4860
   ctcaaggcgc actcccgttc tggataatgt tttttgcgcc gacatcataa cggttctggc 4920
   aaatattctg aaatgagctg ttgacaatta atcatccggc tcgtataatg tgtggaattg 4980
   tgagcggata acaatttcac acaggaaaca gcgccgctga gaaaaagcga agcggcactg 5040
   ctctttaaca atttatcaga caatctgtgt gggcactcga ccggaattat cgattaactt 5100
   tattattaaa aattaaagag gtatatatta atgtatcgat taaataagga ggaataaacc 5160
   atggccggta cgggtcgttt ggctggtaaa attgcattga tcaccggtgg tgctggtaac 5220
   attggttccg agctgacccg ccgttttctg gccgagggtg cgacggttat tatcagcggc 5280
   cgtaaccgtg cgaagctgac cgcgctggcc gagcgcatgc aagccgaggc cggcgtgccg 5340
   gccaagcgca ttgatttgga ggtgatggat ggttccgacc ctgtggctgt ccgtgccggt 5400
   atcgaggcaa tcgtcgctcg ccacggtcag attgacattc tggttaacaa cgcgggctcc 5460
   gccggtgccc aacgtcgctt ggcggaaatt ccgctgacgg aggcagaatt gggtccgggt 5520
   gcggaggaga ctttgcacgc ttcgatcgcg aatctgttgg gcatgggttg gcacctgatg 5580
   cgtattgcgg ctccgcacat gccagttggc tccgcagtta tcaacgtttc gactattttc 5640
   tcgcgcgcag agtactatgg tcgcattccg tacgttaccc cgaaggcagc gctgaacgct 5700
   ttgtcccagc tggctgcccg cgagctgggc gctcgtggca tccgcgttaa cactattttc 5760
   ccaggtccta ttgagtccga ccgcatccgt accgtgtttc aacgtatgga tcaactgaag 5820
   ggtcgcccgg agggcgacac cgcccatcac tttttgaaca ccatgcgcct gtgccgcgca 5880
   aacgaccaag gcgctttgga acgccgcttt ccgtccgttg gcgatgttgc tgatgcggct 5940
   gtgtttctgg cttctgctga gagcgcggca ctgtcgggtg agacgattga ggtcacccac 6000
   ggtatggaac tgccggcgtg tagcgaaacc tccttgttgg cgcgtaccga tctgcgtacc 6060
   atcgacgcga gcggtcgcac taccctgatt tgcgctggcg atcaaattga agaagttatg 6120
   gccctgacgg gcatgctgcg tacgtgcggt agcgaagtga ttatcggctt ccgttctgcg 6180
   gctgccctgg cgcaatttga gcaggcagtg aatgaatctc gccgtctggc aggtgcggat 6240
   ttcaccccgc cgatcgcttt gccgttggac ccacgtgacc cggccaccat tgatgcggtt 6300
   ttcgattggg gcgcaggcga gaatacgggt ggcatccatg cggcggtcat tctgccggca 6360
   acctcccacg aaccggctcc gtgcgtgatt gaagtcgatg acgaacgcgt cctgaatttc 6420
   ctggccgatg aaattaccgg caccatcgtt attgcgagcc gtttggcgcg ctattggcaa 6480
   tcccaacgcc tgaccccggg tgcccgtgcc cgcggtccgc gtgttatctt tctgagcaac 6540
   ggtgccgatc aaaatggtaa tgtttacggt cgtattcaat ctgcggcgat cggtcaattg 6600
   attcgcgttt ggcgtcacga ggcggagttg gactatcaac gtgcatccgc cgcaggcgat 6660
   cacgttctgc cgccggtttg ggcgaaccag attgtccgtt tcgctaaccg ctccctggaa 6720
   ggtctggagt tcgcgtgcgc gtggaccgca cagctgctgc acagccaacg tcatattaac 6780
   gaaattacgc tgaacattcc agccaatatt agcgcgacca cgggcgcacg ttccgccagc 6840
   gtcggctggg ccgagtcctt gattggtctg cacctgggca aggtggctct gattaccggt 6900
   ggttcggcgg gcatcggtgg tcaaatcggt cgtctgctgg ccttgtctgg cgcgcgtgtg 6960
   atgctggccg ctcgcgatcg ccataaattg gaacagatgc aagccatgat tcaaagcgaa 7020
   ttggcggagg ttggttatac cgatgtggag gaccgtgtgc acatcgctcc gggttgcgat 7080
   gtgagcagcg aggcgcagct ggcagatctg gtggaacgta cgctgtccgc attcggtacc 7140
   gtggattatt tgattaataa cgccggtatt gcgggcgtgg aggagatggt gatcgacatg 7200
   ccggtggaag gctggcgtca caccctgttt gccaacctga tttcgaatta ttcgctgatg 7260
   cgcaagttgg cgccgctgat gaagaagcaa ggtagcggtt acatcctgaa cgtttcttcc 7320
   tattttggcg gtgagaagga cgcggcgatt ccttatccga accgcgccga ctacgccgtc 7380
   tccaaggctg gccaacgcgc gatggcggaa gtgttcgctc gtttcctggg tccagagatt 7440
   cagatcaatg ctattgcccc aggtccggtt gaaggcgacc gcctgcgtgg taccggtgag 7500
   cgtccgggcc tgtttgctcg tcgcgcccgt ctgatcttgg agaataaacg cctgaacgaa 7560
   ttgcacgcgg ctttgattgc tgcggcccgc accgatgagc gctcgatgca cgagttggtt 7620
   gaattgttgc tgccgaacga cgtggccgcg ttggagcaga acccagcggc ccctaccgcg 7680
   ctgcgtgagc tggcacgccg cttccgtagc gaaggtgatc cggcggcaag ctcctcgtcc 7740
   gccttgctga atcgctccat cgctgccaag ctgttggctc gcttgcataa cggtggctat 7800
   gtgctgccgg cggatatttt tgcaaatctg cctaatccgc cggacccgtt ctttacccgt 7860
   gcgcaaattg accgcgaagc tcgcaaggtg cgtgatggta ttatgggtat gctgtatctg 7920
   cagcgtatgc caaccgagtt tgacgtcgct atggcaaccg tgtactatct ggccgatcgt 7980
   aacgtgagcg gcgaaacttt ccatccgtct ggtggtttgc gctacgagcg taccccgacc 8040
   ggtggcgagc tgttcggcct gccatcgccg gaacgtctgg cggagctggt tggtagcacg 8100
   gtgtacctga tcggtgaaca cctgaccgag cacctgaacc tgctggctcg tgcctatttg 8160
   gagcgctacg gtgcccgtca agtggtgatg attgttgaga cggaaaccgg tgcggaaacc 8220
   atgcgtcgtc tgttgcatga tcacgtcgag gcaggtcgcc tgatgactat tgtggcaggt 8280
   gatcagattg aggcagcgat tgaccaagcg atcacgcgct atggccgtcc gggtccggtg 8340
   gtgtgcactc cattccgtcc actgccaacc gttccgctgg tcggtcgtaa agactccgat 8400
   tggagcaccg ttttgagcga ggcggaattt gcggaactgt gtgagcatca gctgacccac 8460
   catttccgtg ttgctcgtaa gatcgccttg tcggatggcg cgtcgctggc gttggttacc 8520
   ccggaaacga ctgcgactag caccacggag caatttgctc tggcgaactt catcaagacc 8580
   accctgcacg cgttcaccgc gaccatcggt gttgagtcgg agcgcaccgc gcaacgtatt 8640
   ctgattaacc aggttgatct gacgcgccgc gcccgtgcgg aagagccgcg tgacccgcac 8700
   gagcgtcagc aggaattgga acgcttcatt gaagccgttc tgctggttac cgctccgctg 8760
   cctcctgagg cagacacgcg ctacgcaggc cgtattcacc gcggtcgtgc gattaccgtc 8820
   taatagaagc ttggctgttt tggcggatga gagaagattt tcagcctgat acagattaaa 8880
   tcagaacgca gaagcggtct gataaaacag aatttgcctg gcggcagtag cgcggtggtc 8940
   ccacctgacc ccatgccgaa ctcagaagtg aaacgccgta gcgccgatgg tagtgtgggg 9000
   tctccccatg cgagagtagg gaactgccag gcatcaaata aaacgaaagg ctcagtcgaa 9060
   agactgggcc tttcgtttta tctgttgttt gtcggtgaac gctctcctga gtaggacaaa 9120
   tccgccggga gcggatttga acgttgcgaa gcaacggccc ggagggtggc gggcaggacg 9180
   cccgccataa actgccaggc atcaaattaa gcagaaggcc atcctgacgg atggcctttt 9240
   tgcgtttcta caaactcttt ttgtttattt ttctaaatac attcaaatat gtatccgctc 9300
   atgagacaat aaccctgata aatgcttcaa taatattgaa aaaggaagag tatgagtatt 9360
   caacatttcc gtgtcgccct tattcccttt tttgcggcat tttgccttcc tgtttttgct 9420
   cacccagaaa cgctggtgaa agtaaaagat gctgaagatc agttgggtgc acgagtgggt 9480
   tacatcgaac tggatctcaa cagcggtaag atccttgaga gttttcgccc cgaagaacgt 9540
   tttccaatga tgagcacttt taaagttctg ctatgtggcg cggtattatc ccgtgttgac 9600
   gccgggcaag agcaactcgg tcgccgcata cactattctc agaatgactt ggttgagtac 9660
   tcaccagtca cagaaaagca tcttacggat ggcatgacag taagagaatt atgcagtgct 9720
   gccataacca tgagtgataa cactgcggcc aacttacttc tgacaacgat cggaggaccg 9780
   aaggagctaa ccgctttttt gcacaacatg ggggatcatg taactcgcct tgatcgttgg 9840
   gaaccggagc tgaatgaagc cataccaaac gacgagcgtg acaccacgat gcctgtagca 9900
   atggcaacaa cgttgcgcaa actattaact ggcgaactac ttactctagc ttcccggcaa 9960
   caattaatag actggatgga ggcggataaa gttgcaggac cacttctgcg ctcggccctt 10020
   ccggctggct ggtttattgc tgataaatct ggagccggtg agcgtgggtc tcgcggtatc 10080
   attgcagcac tggggccaga tggtaagccc tcccgtatcg tagttatcta cacgacgg 10138
<210> 175
   <211> 11352
   <212> DNA
   <213> Artificial Sequence
<220>
   <223> Description of Artificial Sequence: Synthetic pACYC-Ptal-pntAB-Ptpia-accAD-PrpiA-accCD-Pkan-ccdAB polynucleotide
<400> 175
   gaattccgga tgagcattca tcaggcgggc aagaatgtga ataaaggccg gataaaactt     60
   gtgcttattt ttctttacgg tctttaaaaa ggccgtaata tccagctgaa cggtctggtt    120
   ataggtacat tgagcaactg actgaaatgc ctcaaaatgt tctttacgat gccattggga    180
   tatatcaacg gtggtatatc cagtgatttt tttctccatt ttagcttcct tagctcctga    240
   aaatctcgat aactcaaaaa atacgcccgg tagtgatctt atttcattat ggtgaaagtt    300
   ggaacctctt acgtgccgat caacgtctca ttttcgccaa aagttggccc agggcttccc    360
   ggtatcaaca gggacaccag gatttattta ttctgcgaag tgatcttccg tcacaggtat    420
   ttattcggcg caaagtgcgt cgggtgatgc tgccaactta ctgatttagt gtatgatggt    480
   gtttttgagg tgctccagtg gcttctgttt ctatcagctg tccctcctgt tcagctactg    540
   acggggtggt gcgtaacggc aaaagcaccg ccggacatca gcgctagcgg agtgtatact    600
   ggcttactat gttggcactg atgagggtgt cagtgaagtg cttcatgtgg caggagaaaa    660
   aaggctgcac cggtgcgtca gcagaatatg tgatacagga tatattccgc ttcctcgctc    720
   actgactcgc tacgctcggt cgttcgactg cggcgagcgg aaatggctta cgaacggggc    780
   ggagatttcc tggaagatgc caggaagata cttaacaggg aagtgagagg gccgcggcaa    840
   agccgttttt ccataggctc cgcccccctg acaagcatca cgaaatctga cgctcaaatc    900
   agtggtggcg aaacccgaca ggactataaa gataccaggc gtttccccct ggcggctccc    960
   tcgtgcgctc tcctgttcct gcctttcggt ttaccggtgt cattccgctg ttatggccgc   1020
   gtttgtctca ttccacgcct gacactcagt tccgggtagg cagttcgctc caagctggac   1080
   tgtatgcacg aaccccccgt tcagtccgac cgctgcgcct tatccggtaa ctatcgtctt   1140
   gagtccaacc cggaaagaca tgcaaaagca ccactggcag cagccactgg taattgattt   1200
   agaggagtta gtcttgaagt catgcgccgg ttaaggctaa actgaaagga caagttttgg   1260
   tgactgcgct cctccaagcc agttacctcg gttcaaagag ttggtagctc agagaacctt   1320
   cgaaaaaccg ccctgcaagg cggttttttc gttttcagag caagagatta cgcgcagacc   1380
   aaaacgatct caagaagatc atcttattaa tcagataaaa tatttctaga tttcagtgca   1440
   atttatctct tcaaatgtag cacctgaagt cagccccata cgatataagt tgtaattctc   1500
   atgtttgaca gcttatcatc gataagcttt aatgcggtag tttatcacag ttaaattgct   1560
   aacgcagtca ggcaccgtgt atgaaatcta acaatgcgct catcgtcatc ctcggcaccg   1620
   tcaccctgga tgctgtaggc ataggcttgg ttatgccggt actgccgggc ctcttgcggg   1680
   atttaagctc gggccccctg ggcggttctg ataacgagta atcgttaatc cgcaaataac 1740
   gtaaaaaccc gcttcggcgg gtttttttat ggggggagtt tagggaaaga gcatttgtca 1800
   gaatatttaa gggcgcctgt cactttgctt gatatatgag aattatttaa ccttataaat 1860
   gagaaaaaag caacgcactt taaataagat acgttgcttt ttcgattgat gaacacctat 1920
   aattaaacta ttcatctatt atttatgatt ttttgtatat acaatatttc tagtttgtta 1980
   aagagaatta agaaaataaa tctcgaaaat aataaaggga aaatcagttt ttgatgggtt 2040
   acagagcttt caggattgca tccacgctgg ctttggcgtc accaaacagc atgtgggtgt 2100
   tttccttgaa gaacagcggg ttttgcacac cagcatagcc agtgttcatc gaacgtttaa 2160
   agacaatcac gttctgcgct ttccacactt ccagcacagg cataccagca atcggactct 2220
   tcggatcatc ctgcgccgcc gggttaaccg tatcgttagc accaatcacc agtacggtat 2280
   cggtatcagc aaagtcatca ttgatctcgt ccatttccag cacgatgtca tacggtactt 2340
   ttgcttcagc cagcaatacg ttcatatgtc caggcaaacg ccccgcgacc gggtggatac 2400
   cgaaacgcac attaatacca cgagcgcgca atttctcagt aatttcagcg acaggatatt 2460
   gcgcctgcgc gactgccatg ccgtaccccg gagtaatgat cactgaatgg gagtttttca 2520
   gcagttccgc tgtctcttct gcggtgattt cgcggtgctc acccacttcc tgatcatcgc 2580
   cagtagaaga gccgtcggtg ccgaaaccac ccgcaataac gctgataaag gaacggttca 2640
   tcgcctcaca cataatgtaa gaaaggatag cccccgaaga accgaccagc gcaccggtca 2700
   caatcagcag gtcgttgctg agcataaagc ccgcagccgc agccgcccag ccggagtacg 2760
   agttcagcat cgacaccacc actggcatat ctgcaccacc gatggaggcg actaaatgcc 2820
   agccgaatac cagcgcaatt gcggtcatta tcagcaatgc cagcacttgc aggccgacgc 2880
   tgtccgtgcg aacaaataca atcagcagca ggaaggaaac gaccagagcc gccaggttca 2940
   ttttgtgacg gtttggcagc atcaatggtt tagacgaaat cttgccacac agtttgccga 3000
   acgccaccac cgaacccgtg aacgttaccg ccccgatgaa gataccgagg aacacttccg 3060
   tcaggtgaat attgaccaga atcggtgcca ttcccgcgtc atgatgcaga tagctgttaa 3120
   agccaaccag cactgccgcc agacccacga agctatgcag gatcgccacc agttctggca 3180
   tttcggtcat ttcaactttc ttcgccagac ggataccaat tgccccacca atgaccatcg 3240
   ccagcaagat ccagccaaca ttacccgtat ccggtccaaa aatggttgcg attaacgcaa 3300
   tcgccatccc ggcgataccg aagttgttac cctggcgaga cgtttcatgt ttcgaaagac 3360
   cggccagact gaagataaac aggatcgcgg caacaatgta tgcagctgta actaatcctc 3420
   cagacatatg ttacccctta atttttgcgg aacattttca gcatgcgctg agtcacggtg 3480
   aagccaccga aaatattaat gctggctata agcaccgcga taaaactaag gaagctaacc 3540
   cagccgccct ggccaatctg caacagtgct ccgacaacaa taatccctga aatcgcgttg 3600
   gtgaccgaca tcaacggtgt atgcagcgcg tgcgatacat tccacaccac gtaataaccg 3660
   acaacgcagg ccagcgcgaa aacggtgaag tgcccaagga attctttcgg cgcaacgctt 3720
   gccatccagc caaaaagaat gattgccagc gccatcaacg cgtatttacg ccacggtgag 3780
   caggtacatt tttcctcagt tttcacttcc ggtgccgctt tttgtgccgc ctgcggctga 3840
   gctgatacct gaatcggcgg tgccggccag gtaatttcgc ccgcacggat cacggtcacg 3900
   ccgcgaatca ccacatcatc aaaatcaaca gtgatattgc cgtctttctc tttgcacaac 3960
   agtttcagca gattaacgag gtttgtgccg taaagctgtg aggattgcgt cggcagacgg 4020
   cccggaagat cggtataacc aatcactttg acaccatttt ccgtagtgaa gatttcaccc 4080
   ggcacggtgt attcacagtt gccgccgttt tgggctgcca ggtcgacaat cacactgccc 4140
   gccttcatgg agtcaaccat ttcacgggta attagcttcg gcgctggttt gcctggaata 4200
   agcgcggtgg tgacaatgat atcgacctct tttgcctggg cggcaaagag ttccatttcc 4260
   gctttgatga acgcgtccga catcactttg gcatagccat cgccgctgcc agcttcctct 4320
   ttaaaatcca gctcgaggaa ttccgcgccc atactttgaa cttgttcttt cacttccggg 4380
   cgggtgtcga atgcacgcac aatcgcgccg agactgtttg ctgcgccaat ggcggccaga 4440
   cctgcaacac ccgcaccaat caccatcact tttgccggtg gcactttccc ggccgcagta 4500
   atttgcccgg taaagaagcg cccaaattca tgtgccgctt caacaatggc gcgataaccg 4560
   gcgatgttcg ccatcgagct tagtgcgtcc agcgattgtg cgcgtgagat acgcggcaca 4620
   gagtccatcg ccatcacggt cacgttacgt tccgcaagtt tttgcattaa ttccggattc 4680
   tgcgcaggcc agataaaact caccagcgtt gtcccaggat tcagtaacgc aatttcatca 4740
   tctaacggcg cattgacctt cagaatgatc tctgactgcc agacgctatt cccttctaca 4800
   atttcagcgc ccgcttgcac aaacgcttta tcgtcaaaac ttgccagttg acccgcgccg 4860
   ctctctaccg cgacggtaaa acccagtttc agcagctgtt ccactgtttt tggcgttgct 4920
   gcaacacggg tttcattggt taaccgttct cttggtatgc caattgccat ggtaaatact 4980
   ccttgaaaag taaagtgtta gatgagtgcg ttaattcaca cttctgagaa atttcgctaa 5040
   acgcatcaaa aaagcatagc agacaggcat ggtattgctg gattaagcag gtaacatcag 5100
   tgttatagga ttattaccaa aacattatat tcacaggtgt atcgctcaat actgaccatt 5160
   taaatcatac ctgacctcca tagcagaaag tcaaaagcct ccgaccggag gcttttgact 5220
   tgagggggat cagcttctat tatttctctt gcaggcccag tttcttttcc aggtagtgaa 5280
   tgttcgtacc gccgtgctgg aaattctcgt cgttcataat gcggatctgc aaatccacat 5340
   tggtcttaat gccatcaatg atcagctctt gcagcgcgtt tttcatgcgg gcgatcgcga 5400
   catcacgatt ttcgccatag cagatcagtt taccaatcat gctgtcatag tacggcggca 5460
   cggtgtaacc ggcgtagatg tgagactccc aacgaacgcc aaaaccacct ggcgcgtgaa 5520
   aacgcgtgat cttaccaggg ctcggcagaa aggtattcgg gtcctccgca ttgatacggc 5580
   actcgaccgc gtgaccacga acgtgcacct cttcttgctt gatcgacagc ggctgaccag 5640
   ccgcaatgcg cagttgctct ttgatcagat caacgccagt aatcatctcg gtcaccgggt 5700
   gctcaacctg gatgcgggta ttcatttcaa tgaagtaaaa ctcaccattt tcgaacagga 5760
   actcaaacgt acccgcacca cggtaaccaa tgtccacaca cgctttcgca cagcgctcac 5820
   cgatgtagcg acgcagttcc ggcgtaatac ccggagccgg tgcctcttcc accaccttct 5880
   gatgacggcg ttgcatggag caatcacgtt ctgccaagta gatcgcattg ccctgaccat 5940
   ccgccagaac ttggatttca acgtggcgcg gattctccag atacttctcc atatacacca 6000
   tatcgttaga gaacgccgct ttggcttccg cacgggtcat gctgatgctt tgcgccagtt 6060
   ccgcatcacc acggacgaca cgcataccac ggccaccgcc accaccagag gctttgataa 6120
   tgaccgggta gccaatacgt ttagcgatgg cacggttctt gtccatatcg tcacccagcg 6180
   gaccatcgct gcccggaaca cacggcacac cggccttttt catcgctgca atggcgctca 6240
   ctttgtcgcc catcaggcga atcgtctccg ccttagggcc aatgaagatg aaaccgctac 6300
   gctccacttg ttcagcaaag ttagcgttct cgctcaggaa accgtagccc ggatgaatcg 6360
   ccactgcacc ggtgatctct gccgcagaga tgatggccgg gatgttcaga tagcttttca 6420
   cgctcggtgc cggaccgatg caaaccgttt cgtcggccag cagaacatgc ttcaggtcgc 6480
   ggtctgcgct ggaatgcacg gcaacagtct taatgcccag ctctttacac gcgcgcaaga 6540
   tacgcagcgc gatttcaccg cggtttgcaa tcacgatctt gtccaacatg ttcgcctcgt 6600
   tactcgatca caaccagcgg ttcatcgaac tcaacaggct ggccggattc caccaggatc 6660
   gctttaacag taccgctctt atccgcttca atctggttca tcattttcat tgcctcaacg 6720
   atacacagcg tgtcgccgac attgaccttc tggccgactt cgatgaaggc ttttgcgtcc 6780
   ggcgacggcg tgcgatagaa ggtgcccacc atcgggctac gcacgatatg acccgaaatc 6840
   tcggccgctg ccggtgcctc catgcttgga acggtcgccg gtgcagcggc gttgctctgt 6900
   gccggctgtt gcatcatcgg agcggcgtat gcttgctgca taaccggaaa gctcgctgcc 6960
   ggtgccgcac gggaaatacg gacgctctct tcaccttcgc tgatctccag ctcgctgatg 7020
   ccgctttctt caaccagttc aatcagtttc ttgatcttac gaatgtccat ggtttttcct 7080
   ccttcgcctg tggtatgaaa tttcacacgc attatataca aaaaaagcga ttcagacccc 7140
   gttggcaagc cgcgtggtta actccacgtg ctattatgcc tccggctctt ggtccgggac 7200
   cggcggaacc acgacaccct cacgcggtgc ttcaggattc ggagccggca gattcatcaa 7260
   tttcgccaga atgctcgcca gtttcagacg catttctgga cggcgaacga tcatgtcgat 7320
   ggcacctttc tcaatcagga actcgctgcg ctggaaacca ggcggcaact tttcgcgaac 7380
   cgtctgttca ataacacgcg gaccagcaaa accaatcaga gccttcggct ctgcgatgtt 7440
   caaatcaccc agcatcgcaa agctggcgct gacaccgccc atcgtagggt cggtcaggac 7500
   ggaaatgtac ggcaggccgc gttcttgcat cttcgccagg gctgccgagg tttttgccat 7560
   ctgcatcagg ctcatcaggg cctcttgcat acgcgcacca ccgctcgcgc tgaaacaaat 7620
   cagaggacag ttatcctcca gcgcctgctc cacggcacgc acaaagcgtg cgcccaccac 7680
   ggaacccatg ctgccaccca taaacgcaaa ctcgaacgcc gcagcaacaa ccggcatacc 7740
   atacagggtg cctttcataa caaccagtgc gtccttctca ccggtttctt tttgtgcaga 7800
   tgccaaacga tccttatact tcttggaatc acggaatttc agaacatctt tcggttccag 7860
   ttcgctaccc agctcgacca ggctgccctc atccagcagg ctatgcagac gattacgagc 7920
   ggtcatacgc atgtgatggt cgcatttcgg gcacacttcc aggttacgct ccagctccgc 7980
   acgatacaaa acctgaccgc agctatcgca tttggtccag acgccttcag ggatgctcgc 8040
   cttgcgggtc ggggtgatat tggacttaat gcgttcaatc cagctcatga ttttcctcct 8100
   ttactacgcg tagccatagc tcatcaagcg ctgatagcga cggtttttca gatcctccgt 8160
   gctcagcacg tccagatccg ccaggtccgc cagcagctgg gccttcaggc tggctgccat 8220
   cgcctccgga ttacggtgag caccgcccag cggttccggg atgatagaat caatcaactt 8280
   cagttctttc agacgcggtg cgatgatacc catcgcttcc gctgccaatg gtgctttatc 8340
   cgcgctcttc cacagaatgg aagcgcaacc ttccgggcta atgacgctgt acgtgctgta 8400
   ttgcagcatg ttgaccttat caccgacacc gatcgccaac gcaccaccgc taccgccctc 8460
   gccaatcacg gtgcaaacga ccggcacacc cagacgagac atctcacgca ggttacgtgc 8520
   gatggcctcg ctctgaccac gctcttccgc accaacgcct gggtacgcac ccggcgtatc 8580
   aatgaacgtg ataatcggca tcttaaagcg ttcggccatt tgcatcagac gcagggcctt 8640
   gcggtagccc tccggtgccg gcataccaaa gttacgacga atcttttctt tcgtctcgcg 8700
   gcctttttga tggccgataa tcatcaccgg acggccatcc aaacgagcaa taccgccgac 8760
   aattgcttta tcgtccgcat aggcgcggtc gcctgccagt tcgtcgaact cgtcaaacgc 8820
   caggcgcacg tagtccaggg tatatgggcg ttgtgggtgg cgagccaact gggcaatctg 8880
   ccatgcgccc aaatcagcaa agattttgcg agtcagttca acagacttct cacgcaaacg 8940
   gtggacctct tcgtcgatgt taatgtccag tttctcatct tgacggctaa ccgcggtcag 9000
   gctgtcaatc ttcgcttcca gttctgcaat cggctgctcg aagtccagga agttcaggga 9060
   catgtttttc ctcctgcccc gcgcttataa gcgaataaag gaagatggcc gccccgcagg 9120
   gcagcaggtc tgtgaaacag tatagagatt catcggcaca aaggctttgc tttttgtcat 9180
   ttattcaaac cggcgcctcg aaccggctcc gggcattgcc ctggccggca ctttgcatgg 9240
   ggagatgcgg atctcgggca gcgttgggtc ctggccacgg gtgcgcatga tcgtgctcct 9300
   gtcgttgagg acccggctag gctggcgggg ttgccttact ggttagcaga atgaatcacc 9360
   gatacgcgag cgaacgtgaa gcgactgctg ctgcaaaacg tctgcgacct gagcaacaac 9420
   atgaatggtc ttcggtttcc gtgtttcgta aagtctggaa acgcggaagt cccctacgtg 9480
   ctgctgaagt tgcccgcaac agagagtgga accaaccggt gataccacga tactatgact 9540
   gagagtcaac gccatgagcg gcctcatttc ttattctgag ttacaacagt ccgcaccgct 9600
   gtccggtagc tccttccggt gggcgcgggg catgactagg tcaggtatga tttaaatggt 9660
   cagtattgag cgatatctag agaattcgtc taggatcctt atattcccca gaacatcagg 9720
   ttaatggcgt ttttgatgtc attttcgcgg tggctgagat cagccacttc ttccccgata 9780
   acggagaccg gcacactggc catatcggtg gtcatcatgc gccagctttc atccccgata 9840
   tgcaccaccg ggtaaagttc acgggagact ttatctgaca gcagacgtgc actggccagg 9900
   gggatcacca tccgtcgccc gggcgtgtca ataatatcac tctgtacatc cacaaacaga 9960
   cgataacggc tctctctttt ataggtgtaa accttaaact gcatttcacc agtccctgtt 10020
   ctcgtcagca aaagagccgt tcatttcaat aaaccgggcg acctcagcca tcccttcctg 10080
   attttccgct ttccagcgtt cggcacgcag acgacgggct tcattctgca tggttgtgct 10140
   taccagaccg gagatattga catcatatgc cttgagcaac tgatagctgt cgctgtcaac 10200
   tgtcactgta atacgctgct tcatgcgaaa cgatcctcat cctgtctctt gatcagagct 10260
   tgatcccctg cgccatcaga tccttggcgg cgagaaagcc atccagttta ctttgcaggg 10320
   cttcccaacc ttaccagagg gcgccccagc tggcaattcc ggaagcttga tatcattcag 10380
   gacgagcctc agactccagc gtaactggac tgtcgtcgcc gcacttatga ctgtcttctt 10440
   tatcatgcaa ctcgtaggac aggtgccggc agcgcccaac agtcccccgg ccacggggcc 10500
   tgccaccata cccacgccga aacaagcgcc ctgcaccatt atgttccgga tctgcatcgc 10560
   aggatgctgc tggctaccct gtggaacacc tacatctgta ttaacgaagc gctaaccgtt 10620
   tttatcaggc tctgggaggc agaataaatg atcatatcgt caattattac ctccacgggg 10680
   agagcctgag caaactggcc tcaggcattt gagaagcaca cggtcacact gcttccggta 10740
   gtcaataaac cggtaaacca gcaatagaca taagcggcta tttaacgacc ctgccctgaa 10800
   ccgacgaccg ggtcgaattt gctttcgaat ttctgccatt catccgctta ttatcactta 10860
   ttcaggcgta gcaccaggcg tttaagggca ccaataactg ccttaaaaaa attacgcccc 10920
   gccctgccac tcatcgcagt actgttgtaa ttcattaagc attctgccga catggaagcc 10980
   atcacagacg gcatgatgaa cctgaatcgc cagcggcatc agcaccttgt cgccttgcgt 11040
   ataatatttg cccatggtga aaacgggggc gaagaagttg tccatattgg ccacgtttaa 11100
   atcaaaactg gtgaaactca cccagggatt ggctgagacg aaaaacatat tctcaataaa 11160
   cccttTaggg aaataggcca ggttttcacc gtaacacgcc acatcttgcg aatatatgtg 11220
   tagaaactgc cggaaatcgt cgtggtattc actccagagc gatgaaaacg tttcagtttg 11280
   ctcatggaaa acggtgtaac aagggtgaac actatcccat atcaccagct caccgtcttt 11340
   cattgccata cg 11352
<210> 176
   <211> 1245
   <212> DNA
   <213> Artificial Sequence
<220>
   <223> Description of Artificial Sequence: Synthetic cscB polynucleotide
<400> 176
   atggccctga acatcccatt ccgcaacgca tactaccgct tcgcaagcag ctacagcttc 60
   ctgttcttca tttcctggag cttgtggtgg tcgctgtatg ctatctggct gaaaggtcat 120
   ctgggcttga cgggtacgga actgggtacc ctgtattccg ttaatcaatt tacgtccatc 180
   ctgtttatga tgttttacgg catcgtgcag gataagctgg gcctgaagaa accgctgatc 240
   tggtgtatga gcttcatcct ggttctgacc ggtccgttta tgatttacgt gtacgaaccg 300
   ctgctgcagt ccaactttag cgtgggcctg atcctgggtg cgttgttttt cggcttgggt 360
   tacctggccg gttgtggtct gctggatagc tttacggaaa agatggcgcg taactttcac 420
   tttgaatatg gtaccgcgcg tgcgtggggt tcgttcggct acgctatcgg cgcgttcttc 480
   gccggcattt tcttcagcat tagcccgcac atcaatttct ggctggttag cctgttcggt 540
   gcggtcttta tgatgatcaa catgcgtttt aaggacaagg atcaccagtg tattgcagcg 600
   gatgctggtg gtgttaagaa agaggacttt attgcagttt tcaaagatcg caacttctgg 660
   gtgttcgtga tcttcattgt cggtacctgg tccttttaca atatcttcga tcagcaactg 720
   tttccggttt tctatgcggg cctgttcgaa agccacgatg ttggcacgcg tctgtacggt 780
   tatctgaata gctttcaagt cgtcctggaa gctctgtgta tggcgatcat tccgttcttt 840
   gttaaccgtg taggcccgaa aaatgcgttg ctgattggcg ttgtgattat ggcactgcgt 900
   atcctgagct gcgcactgtt tgtgaatccg tggatcatta gcctggtgaa actgctgcac 960
   gctattgaag tcccgctgtg tgtcatctct gttttcaagt attccgtcgc gaacttcgac 1020
   aagcgtttgt ccagcaccat cttcctgatc ggtttccaaa ttgcaagcag cctgggtatt 1080
   gttctgttga gcacgccgac tggtattttg tttgatcacg ctggttatca gactgtgttc 1140
   tttgcgatct cgggtatcgt ttgcttgatg ctgctgttcg gtatcttttt cctgtctaag 1200
   aaacgcgagc aaattgttat ggaaaccccg gttcctagcg cgatc 1245
<210> 177
   <211> 1431
   <212> DNA
   <213> Artificial Sequence
<220>
   <223> Description of Artificial Sequence: Synthetic cscA polynucleotide
<400> 177
   atgactcaat cccgcctgca cgcagcacaa aacgcactgg caaagctgca tgagcgtcgt      60
   ggtaacacct tctacccaca cttccacctg gcgccacctg cgggttggat gaacgatccg     120
   aacggcctga tttggttcaa cgaccgttac catgcgttct atcaacacca ccctatgtcc     180
   gagcattggg gtccgatgca ttggggccac gctaccagcg atgacatgat ccattggcag     240
   cacgagccga ttgccctggc accgggtgat gagaacgaca aggatggttg cttcagcggt     300
   tctgctgtcg atgataacgg tgtcctgtct ctgatctaca ccggtcatgt ctggctggat     360
   ggcgctggca atgacgacgc aatccgtgaa gttcaatgcc tggcgacctc gcgtgacggc     420
   attcacttcg agaaacaggg cgtcattttg acgccgcctg agggtatcat gcactttcgc     480
   gacccaaaag tttggcgtga agcggacacg tggtggatgg tcgtcggcgc gaaagacccg     540
   ggtaacacgg gtcagattct gctgtatcgt ggcagcagcc tgcgtgagtg gaccttcgac     600
   cgcgtcctgg cccatgctga tgcgggcgag agctatatgt gggaatgccc agacttcttt     660
   tctctgggcg accaacatta tctgatgttc agcccgcaag gcatgaatgc ggagggctac     720
   agctaccgta accgctttca gagcggcgtt atcccgggta tgtggagccc aggtcgcctg     780
   tttgctcagt ccggtcattt taccgagctg gacaatggtc acgatttcta tgcaccgcaa     840
   tcgttcgtgg cgaaagatgg ccgtcgcatc gttattggtt ggatggatat gtgggaaagc     900
   ccgatgccga gcaagcgtga gggttgggcg ggttgcatga ccctggcccg tgagctgagc     960
   gagagcaatg gcaaattgct gcaacgcccg gtgcacgagg cagaaagcct gcgccagcaa    1020
   caccaaagca tcagccctcg taccattagc aacaaatacg ttctgcagga aaatgcgcag    1080
   gccgttgaga ttcaactgca atgggccctg aaaaacagcg acgcggagca ttacggcctg    1140
   cagctgggtg cgggcatgcg tttgtacatt gataatcaaa gcgagcgttt ggttttgtgg    1200
   cgctactacc cgcatgagaa cctggacggc taccgtagca tcccgctgcc gcagggtgac    1260
   atgctggcgc tgcgtatttt cattgatact agcagcgtcg aggtattcat caatgacggc    1320
   gaagccgtga tgtctagccg tatttacccg cagccggagg aacgcgagct gagcttgtat    1380
   gccagccacg gtgtcgcggt gctgcaacac ggtgcactgt ggcaactggg t             1431
<210> 178
   <211> 921
   <212> DNA
   <213> Artificial Sequence
<220>
   <223> Description of Artificial Sequence: Synthetic cscK polynucleotide
<400> 178
   atgtcagcga aagtatgggt cctgggcgac gcagtagtag atctgttgcc ggaaagcgac      60
   ggtcgtctgc tgccgtgtcc gggtggtgca ccggctaatg ttgcggtggg catcgcgcgt     120
   ctgggtggta cctcgggttt tatcggtcgc gtgggtgacg accctttcgg tgcgctgatg     180
   cagcgcactt tgctgaccga gggtgtggat atcacgtatc tgaagcagga tgagtggcat     240
   cgtacctcta ccgtcctggt ggacctgaat gaccagggtg aacgtagctt caccttcatg     300
   gtgcgcccga gcgccgattt gtttctggaa acgaccgatc tgccgtgttg gcgtcacggc     360
   gagtggctgc acttgtgtag cattgcactg tctgcggaac cgtcgcgcac gagcgccttc     420
   accgcgatga cggcaatccg ccatgcaggt ggctttgtct ccttcgaccc gaacattcgt     480
   gaggatctgt ggcaggacga gcacctgttg cgcctgtgcc tgcgtcaagc actgcagttg     540
   gccgacgttg taaaactgtc cgaagaagag tggcgtctga tctctggtaa aacgcagaat     600
   gaccgcgata tttgtgcgct ggctaaagag tatgagatcg ccatgttgct ggtgacgaag     660
   ggtgccgaag gtgttgttgt gtgctatcgt ggtcaggtgc accattttgc aggcatgtcc     720
   gtgaattgcg tggattctac gggcgcaggc gatgcgttcg tcgcgggtct gttgaccggt     780
   ctgagcagca ccggcctgtc gaccgatgaa cgtgaaatgc gtcgtatcat tgacctggcg     840
   cagcgttgcg gtgccttggc ggttaccgca aagggtgcca tgaccgcatt gccgtgccgt     900
   caggagctgg aaagcgagaa a                                               921
<210> 179
   <211> 4500
   <212> DNA
   <213> Artificial Sequence
<220>
   <223> Description of Artificial Sequence: Synthetic delta-adhE:cscBAK polynucleotide
<400> 179
   gaagcaaaag ccggataatg ttagccataa ataaggttga aaagacgcgc tgacaatacg      60
   ccttttgaca gcatttttca cctcctaact acttaaaatt gctatcattc gttattgtta     120
   tctagttgtg caaaacattg acatccctat cagtgataga tataatgagc acatgcccac     180
   cggcagctgc cggtgggccg tattttggat gataacagga ggcaaaacca tggccctgaa     240
   catcccattc cgcaacgcat actaccgctt cgcaagcagc tacagcttcc tgttcttcat     300
   ttcctggagc ttgtggtggt cgctgtatgc tatctggctg aaaggtcatc tgggcttgac     360
   gggtacggaa ctgggtaccc tgtattccgt taatcaattt acgtccatcc tgtttatgat     420
   gttttacggc atcgtgcagg ataagctggg cctgaagaaa ccgctgatct ggtgtatgag      480
   cttcatcctg gttctgaccg gtccgtttat gatttacgtg tacgaaccgc tgctgcagtc      540
   caactttagc gtgggcctga tcctgggtgc gttgtttttc ggcttgggtt acctggccgg      600
   ttgtggtctg ctggatagct ttacggaaaa gatggcgcgt aactttcact ttgaatatgg      660
   taccgcgcgt gcgtggggtt cgttcggcta cgctatcggc gcgttcttcg ccggcatttt      720
   cttcagcatt agcccgcaca tcaatttctg gctggttagc ctgttcggtg cggtctttat      780
   gatgatcaac atgcgtttta aggacaagga tcaccagtgt attgcagcgg atgctggtgg      840
   tgttaagaaa gaggacttta ttgcagtttt caaagatcgc aacttctggg tgttcgtgat      900
   cttcattgtc ggtacctggt ccttttacaa tatcttcgat cagcaactgt ttccggtttt      960
   ctatgcgggc ctgttcgaaa gccacgatgt tggcacgcgt ctgtacggtt atctgaatag     1020
   ctttcaagtc gtcctggaag ctctgtgtat ggcgatcatt ccgttctttg ttaaccgtgt     1080
   aggcccgaaa aatgcgttgc tgattggcgt tgtgattatg gcactgcgta tcctgagctg     1140
   cgcactgttt gtgaatccgt ggatcattag cctggtgaaa ctgctgcacg ctattgaagt     1200
   cccgctgtgt gtcatctctg ttttcaagta ttccgtcgcg aacttcgaca agcgtttgtc     1260
   cagcaccatc ttcctgatcg gtttccaaat tgcaagcagc ctgggtattg ttctgttgag     1320
   cacgccgact ggtattttgt ttgatcacgc tggttatcag actgtgttct ttgcgatctc     1380
   gggtatcgtt tgcttgatgc tgctgttcgg tatctttttc ctgtctaaga aacgcgagca     1440
   aattgttatg gaaaccccgg ttcctagcgc gatctaagga ggtaatacac atgtcagcga     1500
   aagtatgggt cctgggcgac gcagtagtag atctgttgcc ggaaagcgac ggtcgtctgc     1560
   tgccgtgtcc gggtggtgca ccggctaatg ttgcggtggg catcgcgcgt ctgggtggta     1620
   cctcgggttt tatcggtcgc gtgggtgacg accctttcgg tgcgctgatg cagcgcactt     1680
   tgctgaccga gggtgtggat atcacgtatc tgaagcagga tgagtggcat cgtacctcta     1740
   ccgtcctggt ggacctgaat gaccagggtg aacgtagctt caccttcatg gtgcgcccga     1800
   gcgccgattt gtttctggaa acgaccgatc tgccgtgttg gcgtcacggc gagtggctgc     1860
   acttgtgtag cattgcactg tctgcggaac cgtcgcgcac gagcgccttc accgcgatga     1920
   cggcaatccg ccatgcaggt ggctttgtct ccttcgaccc gaacattcgt gaggatctgt     1980
   ggcaggacga gcacctgttg cgcctgtgcc tgcgtcaagc actgcagttg gccgacgttg     2040
   taaaactgtc cgaagaagag tggcgtctga tctctggtaa aacgcagaat gaccgcgata     2100
   tttgtgcgct ggctaaagag tatgagatcg ccatgttgct ggtgacgaag ggtgccgaag     2160
   gtgttgttgt gtgctatcgt ggtcaggtgc accattttgc aggcatgtcc gtgaattgcg     2220
   tggattctac gggcgcaggc gatgcgttcg tcgcgggtct gttgaccggt ctgagcagca     2280
   ccggcctgtc gaccgatgaa cgtgaaatgc gtcgtatcat tgacctggcg cagcgttgcg     2340
   gtgccttggc ggttaccgca aagggtgcca tgaccgcatt gccgtgccgt caggagctgg 2400
   aaagcgagaa ataaggaggt aatactcatg actcaatccc gcctgcacgc agcacaaaac 2460
   gcactggcaa agctgcatga gcgtcgtggt aacaccttct acccacactt ccacctggcg 2520
   ccacctgcgg gttggatgaa cgatccgaac ggcctgattt ggttcaacga ccgttaccat 2580
   gcgttctatc aacaccaccc tatgtccgag cattggggtc cgatgcattg gggccacgct 2640
   accagcgatg acatgatcca ttggcagcac gagccgattg ccctggcacc gggtgatgag 2700
   aacgacaagg atggttgctt cagcggttct gctgtcgatg ataacggtgt cctgtctctg 2760
   atctacaccg gtcatgtctg gctggatggc gctggcaatg acgacgcaat ccgtgaagtt 2820
   caatgcctgg cgacctcgcg tgacggcatt cacttcgaga aacagggcgt cattttgacg 2880
   ccgcctgagg gtatcatgca ctttcgcgac ccaaaagttt ggcgtgaagc ggacacgtgg 2940
   tggatggtcg tcggcgcgaa agacccgggt aacacgggtc agattctgct gtatcgtggc 3000
   agcagcctgc gtgagtggac cttcgaccgc gtcctggccc atgctgatgc gggcgagagc 3060
   tatatgtggg aatgcccaga cttcttttct ctgggcgacc aacattatct gatgttcagc 3120
   ccgcaaggca tgaatgcgga gggctacagc taccgtaacc gctttcagag cggcgttatc 3180
   ccgggtatgt ggagcccagg tcgcctgttt gctcagtccg gtcattttac cgagctggac 3240
   aatggtcacg atttctatgc accgcaatcg ttcgtggcga aagatggccg tcgcatcgtt 3300
   attggttgga tggatatgtg ggaaagcccg atgccgagca agcgtgaggg ttgggcgggt 3360
   tgcatgaccc tggcccgtga gctgagcgag agcaatggca aattgctgca acgcccggtg 3420
   cacgaggcag aaagcctgcg ccagcaacac caaagcatca gccctcgtac cattagcaac 3480
   aaatacgttc tgcaggaaaa tgcgcaggcc gttgagattc aactgcaatg ggccctgaaa 3540
   aacagcgacg cggagcatta cggcctgcag ctgggtgcgg gcatgcgttt gtacattgat 3600
   aatcaaagcg agcgtttggt tttgtggcgc tactacccgc atgagaacct ggacggctac 3660
   cgtagcatcc cgctgccgca gggtgacatg ctggcgctgc gtattttcat tgatactagc 3720
   agcgtcgagg tattcatcaa tgacggcgaa gccgtgatgt ctagccgtat ttacccgcag 3780
   ccggaggaac gcgagctgag cttgtatgcc agccacggtg tcgcggtgct gcaacacggt 3840
   gcactgtggc aactgggtta agcttcagta gcgctgtctg gcaacataaa cggccccttc 3900
   tgggcaatgc cgatcagtta aggattagtt gaccgatcct taaactgagg cactataacg 3960
   gcttccacaa cagggagccg ttttcttatg ccacttctca atgatctgct cgatttcagt 4020
   gaccatccgc ttatgcctcc gccctctgca caactatttg cagaacacct tcccaccgag 4080
   tggatacaac actgcctgac gctttctgct catgcgaccg ttcgccgccg tcgtttaccg 4140
   ggggacatgg ttatctggat ggtggtgcaa tgagccaatt accgatgttg ttcgccgtct 4200
   gaacctgagc gcggatggcg aagcggggat gaacctgctg gcccgcagcg ctgtcaccca 4260
   ggcgcgtcag cgcgtggggg ccgccccagt ggaatggctc ttccgccaga ccgcacagac 4320
   aggggcgcgg aacgttacct gaaggatgac tggcacggcc tgcaactttt tgccattgat 4380
   ggcgcacagt tcaggacacc tgataaaccc gagctgcgtg aatattatgg gtctgccaac 4440
   acatccacta agcggcagaa cgcctacccg gttatgcgtc tggtggcctt aatgaacctg 4500
<210> 180
   <211> 1248
   <212> DNA
   <213> Artificial Sequence
<220>
   <223> Description of Artificial Sequence: Synthetic cscB(1A) polynucleotide
<400> 180
   atggccctga acatcccatt ccgcaacgca tactaccgct tcgcaagcag ctacagcttc 60
   ctgttcttca tttcctggag cttgtggtgg tcgctgtatg ctatctggct gaaaggtcat 120
   ctgggcttga cgggtacgga actgggtacc ctgtattccg ttaatcaatt tacgtccatc 180
   ctgtttatga tgttttacgg catcgtgcag gataagctgg gcctgaagaa accgctgatc 240
   tggtgtatga gcttcatcct ggttctgacc ggtccgttta tgatttacgt gtacgaaccg 300
   ctgctgcagt ccaactttag cgtgggcctg atcctgggtg cgttgttttt cggcttgggt 360
   tacctggccg gttgtggtct gctggatagc tttacggaaa agatggcgcg taactttcac 420
   tttgaatatg gtaccgcgcg tgcgtggggt tcgttcggct acgctatcgg cgcgttcttc 480
   gccggcattt tcttcagcat tagcccgcac atcaatttct ggctggttag cctgttcggt 540
   gcggtcttta tgatgatcaa catgcgtttt aaggacaagg atcaccagtg tattgcagcg 600
   gatgctggtg gtgttaagaa agaggacttt attgcagtct tcaaagatcg caacttctgg 660
   gtgttcgtga tcttcattgt cggtacctgg tccttttacg atatcttcga tcagcaactg 720
   tttccggttt tctatgcggg cctgttcgaa agccacgatg ttggcacgcg tctgtacggt 780
   tatctgaata gctttcaggt cgtcctggaa gctctgtgta tggcgatcat tccgttcttt 840
   gttaaccgtg taggcccgaa aaatgcgttg ctgattggcg ttgtgattat ggcactgcgt 900
   atcctgagct gcgcactgtt tgtgaatccg tgggtcatta gcctggtgaa actgctgcac 960
   gctattgaag tcccgctgtg tgtcatctct gttttcaagt attccgtcgc gaacttcgac 1020
   aagcgtttgt ccagcaccat cttcctgatc ggtttccaaa ttgcaagcag cctgggtatt 1080
   gttctgttga gcacgccgac tggtgttttg tttgatcacg ctggttatca gactgtgttc 1140
   tttgcgatct cgggtatcgt ttgcttgatg ctgctgttcg gtatcttttt cctgtctaag 1200
   aaacgcgagc aaattgttat ggaaaccccg gttcctagcg cgatctaa 1248
<210> 181
   <211> 9045
   <212> DNA
   <213> Artificial Sequence
<220>
   <223> Description of Artificial Sequence: Synthetic pTRC-Ptrc-mcr-glk polynucleotide
<400> 181
   ccttcaactc agcaaaagtt cgatttattc aacaaagcca cgttgtgtct caaaatctct      60
   gatgttacat tgcacaagat aaaaatatat catcatgaac aataaaactg tctgcttaca     120
   taaacagtaa tacaaggggt gttatgagcc atattcaacg ggaaacgtct tgctcgaggc     180
   cgcgattaaa ttccaacatg gatgctgatt tatatgggta taaatgggct cgcgataatg     240
   tcgggcaatc aggtgcgaca atctatcgat tgtatgggaa gcccgatgcg ccagagttgt     300
   ttctgaaaca tggcaaaggt agcgttgcca atgatgttac agatgagatg gtcagactaa     360
   actggctgac ggaatttatg cctcttccga ccatcaagca ttttatccgt actcctgatg     420
   atgcatggtt actcaccact gcgatccccg ggaaaacagc attccaggta ttagaagaat     480
   atcctgattc aggtgaaaat attgttgatg cgctggcagt gttcctgcgc cggttgcatt     540
   cgattcctgt ttgtaattgt ccttttaaca gcgatcgcgt atttcgtctc gctcaggcgc     600
   aatcacgaat gaataacggt ttggttgatg cgagtgattt tgatgacgag cgtaatggct     660
   ggcctgttga acaagtctgg aaagaaatgc ataagctttt gccattctca ccggattcag     720
   tcgtcactca tggtgatttc tcacttgata accttatttt tgacgagggg aaattaatag     780
   gttgtattga tgttggacga gtcggaatcg cagaccgata ccaggatctt gccatcctat     840
   ggaactgcct cggtgagttt tctccttcat tacagaaacg gctttttcaa aaatatggta     900
   ttgataatcc tgatatgaat aaattgcagt ttcatttgat gctcgatgag tttttctaat     960
   ccggtatcat tgcagcactg gggccagatg gtaagccctc ccgtatcgta gttatctaca    1020
   cgacggggag tcaggcaact atggatgaac gaaatagaca gatcgctgag ataggtgcct    1080
   cactgattaa gcattggtaa ctgtcagacc aagtttactc atatatactt tagattgatt    1140
   taaaacttca tttttaattt aaaaggatct aggtgaagat cctttttgat aatctcatga    1200
   ccaaaatccc ttaacgtgag ttttcgttcc actgagcgtc agaccccgta gaaaagatca    1260
   aaggatcttc ttgagatcct ttttttctgc gcgtaatctg ctgcttgcaa acaaaaaaac    1320
   caccgctacc agcggtggtt tgtttgccgg atcaagagct accaactctt tttccgaagg    1380
   taactggctt cagcagagcg cagataccaa atactgtcct tctagtgtag ccgtagttag    1440
   gccaccactt caagaactct gtagcaccgc ctacatacct cgctctgcta atcctgttac    1500
   cagtggctgc tgccagtggc gataagtcgt gtcttaccgg gttggactca agacgatagt    1560
   taccggataa ggcgcagcgg tcgggctgaa cggggggttc gtgcacacag cccagcttgg 1620
   agcgaacgac ctacaccgaa ctgagatacc tacagcgtga gctatgagaa agcgccacgc 1680
   ttcccgaagg gagaaaggcg gacaggtatc cggtaagcgg cagggtcgga acaggagagc 1740
   gcacgaggga gcttccaggg ggaaacgcct ggtatcttta tagtcctgtc gggtttcgcc 1800
   acctctgact tgagcgtcga tttttgtgat gctcgtcagg ggggcggagc ctatggaaaa 1860
   acgccagcaa cgcggccttt ttacggttcc tggccttttg ctggcctttt gctcacatgt 1920
   tctttcctgc gttatcccct gattctgtgg ataaccgtat taccgccttt gagtgagctg 1980
   ataccgctcg ccgcagccga acgaccgagc gcagcgagtc agtgagcgag gaagcggaag 2040
   agcgcctgat gcggtatttt ctccttacgc atctgtgcgg tatttcacac cgcatatggt 2100
   gcactctcag tacaatctgc tctgatgccg catagttaag ccagtataca ctccgctatc 2160
   gctacgtgac tgggtcatgg ctgcgccccg acacccgcca acacccgctg acgcgccctg 2220
   acgggcttgt ctgctcccgg catccgctta cagacaagct gtgaccgtct ccgggagctg 2280
   catgtgtcag aggttttcac cgtcatcacc gaaacgcgcg aggcagcaga tcaattcgcg 2340
   cgcgaaggcg aagcggcatg catttacgtt gacaccatcg aatggtgcaa aacctttcgc 2400
   ggtatggcat gatagcgccc ggaagagagt caattcaggg tggtgaatgt gaaaccagta 2460
   acgttatacg atgtcgcaga gtatgccggt gtctcttatc agaccgtttc ccgcgtggtg 2520
   aaccaggcca gccacgtttc tgcgaaaacg cgggaaaaag tggaagcggc gatggcggag 2580
   ctgaattaca ttcccaaccg cgtggcacaa caactggcgg gcaaacagtc gttgctgatt 2640
   ggcgttgcca cctccagtct ggccctgcac gcgccgtcgc aaattgtcgc ggcgattaaa 2700
   tctcgcgccg atcaactggg tgccagcgtg gtggtgtcga tggtagaacg aagcggcgtc 2760
   gaagcctgta aagcggcggt gcacaatctt ctcgcgcaac gcgtcagtgg gctgatcatt 2820
   aactatccgc tggatgacca ggatgccatt gctgtggaag ctgcctgcac taatgttccg 2880
   gcgttatttc ttgatgtctc tgaccagaca cccatcaaca gtattatttt ctcccatgaa 2940
   gacggtacgc gactgggcgt ggagcatctg gtcgcattgg gtcaccagca aatcgcgctg 3000
   ttagcgggcc cattaagttc tgtctcggcg cgtctgcgtc tggctggctg gcataaatat 3060
   ctcactcgca atcaaattca gccgatagcg gaacgggaag gcgactggag tgccatgtcc 3120
   ggttttcaac aaaccatgca aatgctgaat gagggcatcg ttcccactgc gatgctggtt 3180
   gccaacgatc agatggcgct gggcgcaatg cgcgccatta ccgagtccgg gctgcgcgtt 3240
   ggtgcggata tctcggtagt gggatacgac gataccgaag acagctcatg ttatatcccg 3300
   ccgtcaacca ccatcaaaca ggattttcgc ctgctggggc aaaccagcgt ggaccgcttg 3360
   ctgcaactct ctcagggcca ggcggtgaag ggcaatcagc tgttgcccgt ctcactggtg 3420
   aaaagaaaaa ccaccctggc gcccaatacg caaaccgcct ctccccgcgc gttggccgat 3480
   tcattaatgc agctggcacg acaggtttcc cgactggaaa gcgggcagtg agcgcaacgc 3540
   aattaatgtg agttagcgcg aattgatctg gtttgacagc ttatcatcga ctgcacggtg 3600
   caccaatgct tctggcgtca ggcagccatc ggaagctgtg gtatggctgt gcaggtcgta 3660
   aatcactgca taattcgtgt cgctcaaggc gcactcccgt tctggataat gttttttgcg 3720
   ccgacatcat aacggttctg gcaaatattc tgaaatgagc tgttgacaat taatcatccg 3780
   gctcgtataa tgtgtggaat tgtgagcgga taacaatttc acacaggaaa cagcgccgct 3840
   gagaaaaagc gaagcggcac tgctctttaa caatttatca gacaatctgt gtgggcactc 3900
   gaccggaatt atcgattaac tttattatta aaaattaaag aggtatatat taatgtatcg 3960
   attaaataag gaggaataaa ccatggccgg tacgggtcgt ttggctggta aaattgcatt 4020
   gatcaccggt ggtgctggta acattggttc cgagctgacc cgccgttttc tggccgaggg 4080
   tgcgacggtt attatcagcg gccgtaaccg tgcgaagctg accgcgctgg ccgagcgcat 4140
   gcaagccgag gccggcgtgc cggccaagcg cattgatttg gaggtgatgg atggttccga 4200
   ccctgtggct gtccgtgccg gtatcgaggc aatcgtcgct cgccacggtc agattgacat 4260
   tctggttaac aacgcgggct ccgccggtgc ccaacgtcgc ttggcggaaa ttccgctgac 4320
   ggaggcagaa ttgggtccgg gtgcggagga gactttgcac gcttcgatcg cgaatctgtt 4380
   gggcatgggt tggcacctga tgcgtattgc ggctccgcac atgccagttg gctccgcagt 4440
   tatcaacgtt tcgactattt tctcgcgcgc agagtactat ggtcgcattc cgtacgttac 4500
   cccgaaggca gcgctgaacg ctttgtccca gctggctgcc cgcgagctgg gcgctcgtgg 4560
   catccgcgtt aacactattt tcccaggtcc tattgagtcc gaccgcatcc gtaccgtgtt 4620
   tcaacgtatg gatcaactga agggtcgccc ggagggcgac accgcccatc actttttgaa 4680
   caccatgcgc ctgtgccgcg caaacgacca aggcgctttg gaacgccgct ttccgtccgt 4740
   tggcgatgtt gctgatgcgg ctgtgtttct ggcttctgct gagagcgcgg cactgtcggg 4800
   tgagacgatt gaggtcaccc acggtatgga actgccggcg tgtagcgaaa cctccttgtt 4860
   ggcgcgtacc gatctgcgta ccatcgacgc gagcggtcgc actaccctga tttgcgctgg 4920
   cgatcaaatt gaagaagtta tggccctgac gggcatgctg cgtacgtgcg gtagcgaagt 4980
   gattatcggc ttccgttctg cggctgccct ggcgcaattt gagcaggcag tgaatgaatc 5040
   tcgccgtctg gcaggtgcgg atttcacccc gccgatcgct ttgccgttgg acccacgtga 5100
   cccggccacc attgatgcgg ttttcgattg gggcgcaggc gagaatacgg gtggcatcca 5160
   tgcggcggtc attctgccgg caacctccca cgaaccggct ccgtgcgtga ttgaagtcga 5220
   tgacgaacgc gtcctgaatt tcctggccga tgaaattacc ggcaccatcg ttattgcgag 5280
   ccgtttggcg cgctattggc aatcccaacg cctgaccccg ggtgcccgtg cccgcggtcc 5340
   gcgtgttatc tttctgagca acggtgccga tcaaaatggt aatgtttacg gtcgtattca 5400
   atctgcggcg atcggtcaat tgattcgcgt ttggcgtcac gaggcggagt tggactatca 5460
   acgtgcatcc gccgcaggcg atcacgttct gccgccggtt tgggcgaacc agattgtccg 5520
   tttcgctaac cgctccctgg aaggtctgga gttcgcgtgc gcgtggaccg cacagctgct 5580
   gcacagccaa cgtcatatta acgaaattac gctgaacatt ccagccaata ttagcgcgac 5640
   cacgggcgca cgttccgcca gcgtcggctg ggccgagtcc ttgattggtc tgcacctggg 5700
   caaggtggct ctgattaccg gtggttcggc gggcatcggt ggtcaaatcg gtcgtctgct 5760
   ggccttgtct ggcgcgcgtg tgatgctggc cgctcgcgat cgccataaat tggaacagat 5820
   gcaagccatg attcaaagcg aattggcgga ggttggttat accgatgtgg aggaccgtgt 5880
   gcacatcgct ccgggttgcg atgtgagcag cgaggcgcag ctggcagatc tggtggaacg 5940
   tacgctgtcc gcattcggta ccgtggatta tttgattaat aacgccggta ttgcgggcgt 6000
   ggaggagatg gtgatcgaca tgccggtgga aggctggcgt cacaccctgt ttgccaacct 6060
   gatttcgaat tattcgctga tgcgcaagtt ggcgccgctg atgaagaagc aaggtagcgg 6120
   ttacatcctg aacgtttctt cctattttgg cggtgagaag gacgcggcga ttccttatcc 6180
   gaaccgcgcc gactacgccg tctccaaggc tggccaacgc gcgatggcgg aagtgttcgc 6240
   tcgtttcctg ggtccagaga ttcagatcaa tgctattgcc ccaggtccgg ttgaaggcga 6300
   ccgcctgcgt ggtaccggtg agcgtccggg cctgtttgct cgtcgcgccc gtctgatctt 6360
   ggagaataaa cgcctgaacg aattgcacgc ggctttgatt gctgcggccc gcaccgatga 6420
   gcgctcgatg cacgagttgg ttgaattgtt gctgccgaac gacgtggccg cgttggagca 6480
   gaacccagcg gcccctaccg cgctgcgtga gctggcacgc cgcttccgta gcgaaggtga 6540
   tccggcggca agctcctcgt ccgccttgct gaatcgctcc atcgctgcca agctgttggc 6600
   tcgcttgcat aacggtggct atgtgctgcc ggcggatatt tttgcaaatc tgcctaatcc 6660
   gccggacccg ttctttaccc gtgcgcaaat tgaccgcgaa gctcgcaagg tgcgtgatgg 6720
   tattatgggt atgctgtatc tgcagcgtat gccaaccgag tttgacgtcg ctatggcaac 6780
   cgtgtactat ctggccgatc gtaacgtgag cggcgaaact ttccatccgt ctggtggttt 6840
   gcgctacgag cgtaccccga ccggtggcga gctgttcggc ctgccatcgc cggaacgtct 6900
   ggcggagctg gttggtagca cggtgtacct gatcggtgaa cacctgaccg agcacctgaa 6960
   cctgctggct cgtgcctatt tggagcgcta cggtgcccgt caagtggtga tgattgttga 7020
   gacggaaacc ggtgcggaaa ccatgcgtcg tctgttgcat gatcacgtcg aggcaggtcg 7080
   cctgatgact attgtggcag gtgatcagat tgaggcagcg attgaccaag cgatcacgcg 7140
   ctatggccgt ccgggtccgg tggtgtgcac tccattccgt ccactgccaa ccgttccgct 7200
   ggtcggtcgt aaagactccg attggagcac cgttttgagc gaggcggaat ttgcggaact 7260
   gtgtgagcat cagctgaccc accatttccg tgttgctcgt aagatcgcct tgtcggatgg 7320
   cgcgtcgctg gcgttggtta ccccggaaac gactgcgact agcaccacgg agcaatttgc 7380
   tctggcgaac ttcatcaaga ccaccctgca cgcgttcacc gcgaccatcg gtgttgagtc 7440
   ggagcgcacc gcgcaacgta ttctgattaa ccaggttgat ctgacgcgcc gcgcccgtgc 7500
   ggaagagccg cgtgacccgc acgagcgtca gcaggaattg gaacgcttca ttgaagccgt 7560
   tctgctggtt accgctccgc tgcctcctga ggcagacacg cgctacgcag gccgtattca 7620
   ccgcggtcgt gcgattaccg tctaatagaa gctaaacaca tgtaggtttg aatttgggag 7680
   ttaaccacgc ggcttgccaa cggggtctga atcgcttttt ttgtatataa tgcgtgtgaa 7740
   atttcatacc acaggcgaag gaggaaaaat catgaccaag tatgcactgg tgggcgatgt 7800
   gggtggcacc aatgcgcgtc tggcgctgtg cgacattgcc tcgggtgaga ttagccaagc 7860
   gaaaacctat agcggcttgg actacccgtc cctggaggcg gtgatccgtg tctacctgga 7920
   agaacataaa gtcgaggtga aggacggctg tatcgccatt gcgtgcccga ttaccggcga 7980
   ctgggtggcg atgacgaatc acacctgggc attcagcatt gcagagatga agaaaaacct 8040
   gggctttagc cacctggaga ttatcaacga cttcacggcc gtttcgatgg cgattccgat 8100
   gctgaagaaa gaacatctga ttcaatttgg tggtgcggag ccggtggagg gtaagccgat 8160
   cgcggtctac ggcgctggca cgggcctggg cgtggcgcac ctggtgcatg ttgacaagcg 8220
   ttgggttagc ctgccgggtg aaggtggcca cgttgatttt gcgccaaata gcgaagaaga 8280
   agcgatcatt ctggaaatcc tgcgtgcgga aattggtcac gtttccgcag agcgtgttct 8340
   gagcggtcct ggtttggtga acctgtatcg tgcaattgtg aaagcagata atcgcttgcc 8400
   ggagaacttg aaaccgaagg acatcaccga acgcgcgctg gcagatagct gcaccgattg 8460
   ccgccgtgcg ctgtctttgt tctgcgtcat catgggtcgt tttggtggca atctggcact 8520
   gaacctgggc acctttggtg gcgtgttcat cgcaggcggt attgtcccgc gttttctgga 8580
   gttcttcaag gcttcgggct tccgcgcagc gttcgaagat aaaggtcgtt tcaaagaata 8640
   cgttcacgat attccggtct atctgatcgt ccatgacaac cctggcctgt tgggcagcgg 8700
   tgcccatctg cgtcagaccc tgggtcacat tctgtaatag cccgggatgt ctatcttaag 8760
   gtagccaaag tttaaacttt ggctacctta agatagacat attcttgccg cactagaggc 8820
   ctgtcaggcc ggcgccattg ggtcatcgga tcccgggccc gtcagcttgg ctgttttggc 8880
   ggatgagaga agattttcag cctgatacag attaaatcag aacgcagaag cggtctgata 8940
   aaacagaatt tgcctggcgg cagtagcgcg gtggtcccac ctgaccccat gccgaactca 9000
   gaagtgaaac gccgtagcgc cgatggtagt gtggggtctc cccat 9045
<210> 182
   <211> 3822
   <212> DNA
   <213> Artificial Sequence
<220>
   <223> Description of Artificial Sequence: Synthetic rbs accADBC polynucleotide
<400> 182
   aggaggaaat acatatgtct ctgaacttcc tggacttcga gcagccgatc gccgaactgg 60
   aggcgaagat tgacagcctg accgcggtta gccgtcaaga tgagaaactg gacattaaca 120
   tcgacgaaga ggtccaccgt ttgcgtgaga agtctgttga actgactcgc aaaatctttg 180
   ctgatttggg cgcatggcag attgcccagt tggctcgcca cccacaacgc ccatataccc 240
   tggactacgt gcgcctggcg tttgacgagt tcgacgaact ggcaggcgac cgcgcctatg 300
   cggacgataa agcaattgtc ggcggtattg ctcgtttgga tggccgtccg gtgatgatta 360
   tcggccatca aaaaggccgc gagacaaagg aaaagattcg tcgtaatttc ggaatgccgg 420
   caccggaggg ctaccgcaag gccctgcgtc tgatgcaaat ggccgaacgc tttaagatgc 480
   cgattatcac gttcattgat acgcctggtg cgtacccagg cgttggtgcg gaagagcgtg 540
   gtcagagcga ggccatcgca cgtaacctgc gtgagatgtc tcgtctgggt gtgccggtcg 600
   tttgcaccgt gattggcgag ggcggtagcg gtggtgcgtt ggcgatcggt gtcggtgata 660
   aggtcaacat gctgcaatac agcacgtaca gcgtcattag cccggaaggt tgcgcttcca 720
   ttctgtggaa gagcgcggat aaagcaccat tggcagcgga agcgatgggt atcatcgcac 780
   cgcgtctgaa agaactgaag ttgattgatt ctatcatccc ggaaccgctg ggcggtgctc 840
   accgtaatcc ggaggcgatg gcagccagcc tgaaggccca gctgctggcg gacctggcgg 900
   atctggacgt gctgagcacg gaggatctga aaaaccgtcg ctatcagcgc ttgatgagct 960
   atggctacgc ctaaggtacc tttatcaatt tatttaagga ggactcttaa gatgagctgg 1020
   atcgagcgca tcaagagcaa catcaccccg acccgcaagg cgagcatccc tgaaggcgtc 1080
   tggaccaaat gcgatagctg cggtcaggtt ttgtatcgtg cggagctgga gcgtaacctg 1140
   gaagtgtgcc cgaaatgcga ccatcacatg cgtatgaccg ctcgtaatcg tctgcatagc 1200
   ctgctggatg agggcagcct ggtcgagctg ggtagcgaac tggaaccgaa agatgttctg 1260
   aaattccgtg attccaagaa gtataaggat cgtttggcat ctgcacaaaa agaaaccggt 1320
   gagaaggacg cactggttgt tatgaaaggc accctgtatg gtatgccggt tgttgctgcg 1380
   gcgttcgagt ttgcgtttat gggtggcagc atgggttccg tggtgggcgc acgctttgtg 1440
   cgtgccgtgg agcaggcgct ggaggataac tgtcctctga tttgtttcag cgcgagcggt 1500
   ggtgcgcgta tgcaagaggc cctgatgagc ctgatgcaga tggcaaaaac ctcggcagcc 1560
   ctggcgaaga tgcaagaacg cggcctgccg tacatttccg tcctgaccga ccctacgatg 1620
   ggcggtgtca gcgccagctt tgcgatgctg ggtgatttga acatcgcaga gccgaaggct 1680
   ctgattggtt ttgctggtcc gcgtgttatt gaacagacgg ttcgcgaaaa gttgccgcct 1740
   ggtttccagc gcagcgagtt cctgattgag aaaggtgcca tcgacatgat cgttcgccgt 1800
   ccagaaatgc gtctgaaact ggcgagcatt ctggcgaaat tgatgaatct gccggctccg 1860
   aatcctgaag caccgcgtga gggtgtcgtg gttccgccgg tcccggacca agagccggag 1920
   gcttaattaa ccaaattaga agtattttat ttagaggagg tagccatgga catccgcaag 1980
   atcaagaagc tgatcgaact ggtggaagag tctggcatca gcgagctgga gatcagcgaa 2040
   ggtgaagaga gcgtccgtat ttcccgtgcg gcaccggcag cgagctttcc ggttatgcag 2100
   caagcatacg ccgctccgat gatgcaacag ccggcacaga gcaacgccgc tgcaccggcg 2160
   accgttccaa gcatggaggc accggcagcg gccgagattt cgggtcatat cgtgcgtagc 2220
   ccgatggtgg gcaccttcta tcgcacgccg tcgccggacg caaaagcctt catcgaagtc 2280
   ggccagaagg tcaatgtcgg cgacacgctg tgtatcgttg aggcaatgaa aatgatgaac 2340
   cagattgaag cggataagag cggtactgtt aaagcgatcc tggtggaatc cggccagcct 2400
   gttgagttcg atgaaccgct ggttgtgatc gagtaagagc tccggatata cggggaagaa 2460
   caaggggtgt acatgctgga caagatcgtc atcgccaacc gcggcgaaat cgccctgcgc 2520
   atcttgcgcg cgtgtaaaga gctgggcatt aagactgttg ccgtgcattc cagcgcagac 2580
   cgcgacctga agcatgttct gctggccgac gaaacggttt gcatcggtcc ggcaccgagc 2640
   gtgaaaagct atctgaacat cccggccatc atctctgcgg cagagatcac cggtgcagtg 2700
   gcgattcatc cgggctacgg tttcctgagc gagaacgcta actttgctga acaagtggag 2760
   cgtagcggtt tcatcttcat tggccctaag gcggagacga ttcgcctgat gggcgacaaa 2820
   gtgagcgcca ttgcagcgat gaaaaaggcc ggtgtgccgt gtgttccggg cagcgatggt 2880
   ccgctgggtg acgatatgga caagaaccgt gccatcgcta aacgtattgg ctacccggtc 2940
   attatcaaag cctctggtgg tggcggtggc cgtggtatgc gtgtcgtccg tggtgatgcg 3000
   gaactggcgc aaagcatcag catgacccgt gcggaagcca aagcggcgtt ctctaacgat 3060
   atggtgtata tggagaagta tctggagaat ccgcgccacg ttgaaatcca agttctggcg 3120
   gatggtcagg gcaatgcgat ctacttggca gaacgtgatt gctccatgca acgccgtcat 3180
   cagaaggtgg tggaagaggc accggctccg ggtattacgc cggaactgcg tcgctacatc 3240
   ggtgagcgct gtgcgaaagc gtgtgtggac attggttacc gtggtgcggg tacgtttgag 3300
   ttcctgttcg aaaatggtga gttttacttc attgaaatga atacccgcat ccaggttgag 3360
   cacccggtga ccgagatgat tactggcgtt gatctgatca aagagcaact gcgcattgcg 3420
   gctggtcagc cgctgtcgat caagcaagaa gaggtgcacg ttcgtggtca cgcggtcgag 3480
   tgccgtatca atgcggagga cccgaatacc tttctgccga gccctggtaa gatcactcgt 3540
   tttcacgcgc caggtggttt tggcgttcgt tgggagtctc acatctacgc cggttacacc 3600
   gtgccgccgt actatgacag catgattggt aaactgatct gctatggcga aaatcgtgat 3660
   gtcgcgatcg cccgcatgaa aaacgcgctg caagagctga tcattgatgg cattaagacc 3720
   aatgtggatt tgcagatccg cattatgaac gacgagaatt tccagcacgg cggtacgaac 3780
   attcactacc tggaaaagaa actgggcctg caagagaaat aa 3822
<210> 183
   <211> 7721
   <212> DNA
   <213> Artificial Sequence
<220>
   <223> Description of Artificial Sequence: Synthetic pACYC-T7-rbs accADBC polynucleotide
<400> 183
   gacgaattct ctagatatcg gccggccacg cgatcgctga cgtcggtacc ctcgagtctg      60
   gtaaagaaac cgctgctgcg aaatttgaac gccagcacat ggactcgtct actagcgcag     120
   cttaattaac ctaggctgct gccaccgctg agcaataact agcataaccc cttggggcct     180
   ctaaacgggt cttgaggggt tttttgctga aacctcaggc atttgagaag cacacggtca     240
   cactgcttcc ggtagtcaat aaaccggtaa accagcaata gacataagcg gctatttaac     300
   gaccctgccc tgaaccgacg accgggtcga atttgctttc gaatttctgc cattcatccg     360
   cttattatca cttattcagg cgtagcacca ggcgtttaag ggcaccaata actgccttaa     420
   aaaaattacg ccccgccctg ccactcatcg cagtactgtt gtaattcatt aagcattctg     480
   ccgacatgga agccatcaca gacggcatga tgaacctgaa tcgccagcgg catcagcacc     540
   ttgtcgcctt gcgtataata tttgcccata gtgaaaacgg gggcgaagaa gttgtccata     600
   ttggccacgt ttaaatcaaa actggtgaaa ctcacccagg gattggctga gacgaaaaac     660
   atattctcaa taaacccttt agggaaatag gccaggtttt caccgtaaca cgccacatct     720
   tgcgaatata tgtgtagaaa ctgccggaaa tcgtcgtggt attcactcca gagcgatgaa     780
   aacgtttcag tttgctcatg gaaaacggtg taacaagggt gaacactatc ccatatcacc     840
   agctcaccgt ctttcattgc catacggaac tccggatgag cattcatcag gcgggcaaga     900
   atgtgaataa aggccggata aaacttgtgc ttatttttct ttacggtctt taaaaaggcc     960
   gtaatatcca gctgaacggt ctggttatag gtacattgag caactgactg aaatgcctca    1020
   aaatgttctt tacgatgcca ttgggatata tcaacggtgg tatatccagt gatttttttc    1080
   tccattttag cttccttagc tcctgaaaat ctcgataact caaaaaatac gcccggtagt    1140
   gatcttattt cattatggtg aaagttggaa cctcttacgt gccgatcaac gtctcatttt    1200
   cgccaaaagt tggcccaggg cttcccggta tcaacaggga caccaggatt tatttattct    1260
   gcgaagtgat cttccgtcac aggtatttat tcggcgcaaa gtgcgtcggg tgatgctgcc 1320
   aacttactga tttagtgtat gatggtgttt ttgaggtgct ccagtggctt ctgtttctat 1380
   cagctgtccc tcctgttcag ctactgacgg ggtggtgcgt aacggcaaaa gcaccgccgg 1440
   acatcagcgc tagcggagtg tatactggct tactatgttg gcactgatga gggtgtcagt 1500
   gaagtgcttc atgtggcagg agaaaaaagg ctgcaccggt gcgtcagcag aatatgtgat 1560
   acaggatata ttccgcttcc tcgctcactg actcgctacg ctcggtcgtt cgactgcggc 1620
   gagcggaaat ggcttacgaa cggggcggag atttcctgga agatgccagg aagatactta 1680
   acagggaagt gagagggccg cggcaaagcc gtttttccat aggctccgcc cccctgacaa 1740
   gcatcacgaa atctgacgct caaatcagtg gtggcgaaac ccgacaggac tataaagata 1800
   ccaggcgttt cccctggcgg ctccctcgtg cgctctcctg ttcctgcctt tcggtttacc 1860
   ggtgtcattc cgctgttatg gccgcgtttg tctcattcca cgcctgacac tcagttccgg 1920
   gtaggcagtt cgctccaagc tggactgtat gcacgaaccc cccgttcagt ccgaccgctg 1980
   cgccttatcc ggtaactatc gtcttgagtc caacccggaa agacatgcaa aagcaccact 2040
   ggcagcagcc actggtaatt gatttagagg agttagtctt gaagtcatgc gccggttaag 2100
   gctaaactga aaggacaagt tttggtgact gcgctcctcc aagccagtta cctcggttca 2160
   aagagttggt agctcagaga accttcgaaa aaccgccctg caaggcggtt ttttcgtttt 2220
   cagagcaaga gattacgcgc agaccaaaac gatctcaaga agatcatctt attaatcaga 2280
   taaaatattt ctagatttca gtgcaattta tctcttcaaa tgtagcacct gaagtcagcc 2340
   ccatacgata taagttgtaa ttctcatgtt agtcatgccc cgcgcccacc ggaaggagct 2400
   gactgggttg aaggctctca agggcatcgg tcgagatccc ggtgcctaat gagtgagcta 2460
   acttacatta attgcgttgc gctcactgcc cgctttccag tcgggaaacc tgtcgtgcca 2520
   gctgcattaa tgaatcggcc aacgcgcggg gagaggcggt ttgcgtattg ggcgccaggg 2580
   tggtttttct tttcaccagt gagacgggca acagctgatt gcccttcacc gcctggccct 2640
   gagagagttg cagcaagcgg tccacgctgg tttgccccag caggcgaaaa tcctgtttga 2700
   tggtggttaa cggcgggata taacatgagc tgtcttcggt atcgtcgtat cccactaccg 2760
   agatgtccgc accaacgcgc agcccggact cggtaatggc gcgcattgcg cccagcgcca 2820
   tctgatcgtt ggcaaccagc atcgcagtgg gaacgatgcc ctcattcagc atttgcatgg 2880
   tttgttgaaa accggacatg gcactccagt cgccttcccg ttccgctatc ggctgaattt 2940
   gattgcgagt gagatattta tgccagccag ccagacgcag acgcgccgag acagaactta 3000
   atgggcccgc taacagcgcg atttgctggt gacccaatgc gaccagatgc tccacgccca 3060
   gtcgcgtacc gtcttcatgg gagaaaataa tactgttgat gggtgtctgg tcagagacat 3120
   caagaaataa cgccggaaca ttagtgcagg cagcttccac agcaatggca tcctggtcat 3180
   ccagcggata gttaatgatc agcccactga cgcgttgcgc gagaagattg tgcaccgccg 3240
   ctttacaggc ttcgacgccg cttcgttcta ccatcgacac caccacgctg gcacccagtt 3300
   gatcggcgcg agatttaatc gccgcgacaa tttgcgacgg cgcgtgcagg gccagactgg 3360
   aggtggcaac gccaatcagc aacgactgtt tgcccgccag ttgttgtgcc acgcggttgg 3420
   gaatgtaatt cagctccgcc atcgccgctt ccactttttc ccgcgttttc gcagaaacgt 3480
   ggctggcctg gttcaccacg cgggaaacgg tctgataaga gacaccggca tactctgcga 3540
   catcgtataa cgttactggt ttcacattca ccaccctgaa ttgactctct tccgggcgct 3600
   atcatgccat accgcgaaag gttttgcgcc attcgatggt gtccgggatc tcgacgctct 3660
   cccttatgcg actcctgcat taggaaatta atacgactca ctatagggga attgtgagcg 3720
   gataacaatt cccctgtaga aataattttg tttaacttta ataaggagat ataccatggg 3780
   cagcagccat caccatcatc accacagcca ggatccgaat tcgagatcaa gcttgaattc 3840
   gttcgaatgc atctagatcc aattctagac gtcgaacctc tctaaaacta aggaggaaat 3900
   acatatgtct ctgaacttcc tggacttcga gcagccgatc gccgaactgg aggcgaagat 3960
   tgacagcctg accgcggtta gccgtcaaga tgagaaactg gacattaaca tcgacgaaga 4020
   ggtccaccgt ttgcgtgaga agtctgttga actgactcgc aaaatctttg ctgatttggg 4080
   cgcatggcag attgcccagt tggctcgcca cccacaacgc ccatataccc tggactacgt 4140
   gcgcctggcg tttgacgagt tcgacgaact ggcaggcgac cgcgcctatg cggacgataa 4200
   agcaattgtc ggcggtattg ctcgtttgga tggccgtccg gtgatgatta tcggccatca 4260
   aaaaggccgc gagacaaagg aaaagattcg tcgtaatttc ggaatgccgg caccggaggg 4320
   ctaccgcaag gccctgcgtc tgatgcaaat ggccgaacgc tttaagatgc cgattatcac 4380
   gttcattgat acgcctggtg cgtacccagg cgttggtgcg gaagagcgtg gtcagagcga 4440
   ggccatcgca cgtaacctgc gtgagatgtc tcgtctgggt gtgccggtcg tttgcaccgt 4500
   gattggcgag ggcggtagcg gtggtgcgtt ggcgatcggt gtcggtgata aggtcaacat 4560
   gctgcaatac agcacgtaca gcgtcattag cccggaaggt tgcgcttcca ttctgtggaa 4620
   gagcgcggat aaagcaccat tggcagcgga agcgatgggt atcatcgcac cgcgtctgaa 4680
   agaactgaag ttgattgatt ctatcatccc ggaaccgctg ggcggtgctc accgtaatcc 4740
   ggaggcgatg gcagccagcc tgaaggccca gctgctggcg gacctggcgg atctggacgt 4800
   gctgagcacg gaggatctga aaaaccgtcg ctatcagcgc ttgatgagct atggctacgc 4860
   ctaaggtacc tttatcaatt tatttaagga ggactcttaa gatgagctgg atcgagcgca 4920
   tcaagagcaa catcaccccg acccgcaagg cgagcatccc tgaaggcgtc tggaccaaat 4980
   gcgatagctg cggtcaggtt ttgtatcgtg cggagctgga gcgtaacctg gaagtgtgcc 5040
   cgaaatgcga ccatcacatg cgtatgaccg ctcgtaatcg tctgcatagc ctgctggatg 5100
   agggcagcct ggtcgagctg ggtagcgaac tggaaccgaa agatgttctg aaattccgtg 5160
   attccaagaa gtataaggat cgtttggcat ctgcacaaaa agaaaccggt gagaaggacg 5220
   cactggttgt tatgaaaggc accctgtatg gtatgccggt tgttgctgcg gcgttcgagt 5280
   ttgcgtttat gggtggcagc atgggttccg tggtgggcgc acgctttgtg cgtgccgtgg 5340
   agcaggcgct ggaggataac tgtcctctga tttgtttcag cgcgagcggt ggtgcgcgta 5400
   tgcaagaggc cctgatgagc ctgatgcaga tggcaaaaac ctcggcagcc ctggcgaaga 5460
   tgcaagaacg cggcctgccg tacatttccg tcctgaccga ccctacgatg ggcggtgtca 5520
   gcgccagctt tgcgatgctg ggtgatttga acatcgcaga gccgaaggct ctgattggtt 5580
   ttgctggtcc gcgtgttatt gaacagacgg ttcgcgaaaa gttgccgcct ggtttccagc 5640
   gcagcgagtt cctgattgag aaaggtgcca tcgacatgat cgttcgccgt ccagaaatgc 5700
   gtctgaaact ggcgagcatt ctggcgaaat tgatgaatct gccggctccg aatcctgaag 5760
   caccgcgtga gggtgtcgtg gttccgccgg tcccggacca agagccggag gcttaattaa 5820
   ccaaattaga agtattttat ttagaggagg tagccatgga catccgcaag atcaagaagc 5880
   tgatcgaact ggtggaagag tctggcatca gcgagctgga gatcagcgaa ggtgaagaga 5940
   gcgtccgtat ttcccgtgcg gcaccggcag cgagctttcc ggttatgcag caagcatacg 6000
   ccgctccgat gatgcaacag ccggcacaga gcaacgccgc tgcaccggcg accgttccaa 6060
   gcatggaggc accggcagcg gccgagattt cgggtcatat cgtgcgtagc ccgatggtgg 6120
   gcaccttcta tcgcacgccg tcgccggacg caaaagcctt catcgaagtc ggccagaagg 6180
   tcaatgtcgg cgacacgctg tgtatcgttg aggcaatgaa aatgatgaac cagattgaag 6240
   cggataagag cggtactgtt aaagcgatcc tggtggaatc cggccagcct gttgagttcg 6300
   atgaaccgct ggttgtgatc gagtaagagc tccggatata cggggaagaa caaggggtgt 6360
   acatgctgga caagatcgtc atcgccaacc gcggcgaaat cgccctgcgc atcttgcgcg 6420
   cgtgtaaaga gctgggcatt aagactgttg ccgtgcattc cagcgcagac cgcgacctga 6480
   agcatgttct gctggccgac gaaacggttt gcatcggtcc ggcaccgagc gtgaaaagct 6540
   atctgaacat cccggccatc atctctgcgg cagagatcac cggtgcagtg gcgattcatc 6600
   cgggctacgg tttcctgagc gagaacgcta actttgctga acaagtggag cgtagcggtt 6660
   tcatcttcat tggccctaag gcggagacga ttcgcctgat gggcgacaaa gtgagcgcca 6720
   ttgcagcgat gaaaaaggcc ggtgtgccgt gtgttccggg cagcgatggt ccgctgggtg 6780
   acgatatgga caagaaccgt gccatcgcta aacgtattgg ctacccggtc attatcaaag 6840
   cctctggtgg tggcggtggc cgtggtatgc gtgtcgtccg tggtgatgcg gaactggcgc 6900
   aaagcatcag catgacccgt gcggaagcca aagcggcgtt ctctaacgat atggtgtata 6960
   tggagaagta tctggagaat ccgcgccacg ttgaaatcca agttctggcg gatggtcagg 7020
   gcaatgcgat ctacttggca gaacgtgatt gctccatgca acgccgtcat cagaaggtgg 7080
   tggaagaggc accggctccg ggtattacgc cggaactgcg tcgctacatc ggtgagcgct 7140
   gtgcgaaagc gtgtgtggac attggttacc gtggtgcggg tacgtttgag ttcctgttcg 7200
   aaaatggtga gttttacttc attgaaatga atacccgcat ccaggttgag cacccggtga 7260
   ccgagatgat tactggcgtt gatctgatca aagagcaact gcgcattgcg gctggtcagc 7320
   cgctgtcgat caagcaagaa gaggtgcacg ttcgtggtca cgcggtcgag tgccgtatca 7380
   atgcggagga cccgaatacc tttctgccga gccctggtaa gatcactcgt tttcacgcgc 7440
   caggtggttt tggcgttcgt tgggagtctc acatctacgc cggttacacc gtgccgccgt 7500
   actatgacag catgattggt aaactgatct gctatggcga aaatcgtgat gtcgcgatcg 7560
   cccgcatgaa aaacgcgctg caagagctga tcattgatgg cattaagacc aatgtggatt 7620
   tgcagatccg cattatgaac gacgagaatt tccagcacgg cggtacgaac attcactacc 7680
   tggaaaagaa actgggcctg caagagaaat aactgcagag g 7721
<210> 184
   <211> 6862
   <212> DNA
   <213> Artificial Sequence
<220>
   <223> Description of Artificial Sequence: Synthetic pACYC-pyibD-rbsaccADBC polynucleotide
<400> 184
   gacgaattct ctagatgcat gatcgtgctc ctgtcgttga ggacccggct aggctggcgg 60
   ggttgcctta ctggttagca gaatgaatca ccgatacgcg agcgaacgtg aagcgactgc 120
   tgctgcaaaa cgtctgcgac ctgagcaaca acatgaatgg tcttcggttt ccgtgtttcg 180
   taaagtctgg aaacgcggaa gtcccctacg tgctgctgaa gttgcccgca acagagagtg 240
   gaaccaaccg gtgataccac gatactatga ctgagagtca acgccatgag cggcctcatt 300
   tcttattctg agttacaaca gtccgcaccg ctgtccggta gctccttccg gtgggcgcgg 360
   ggcatgacta tcgtcgccgc acttatgact gtcttcttta tcatgcaact cgtaggacag 420
   gtgccggcag cgcccaacag tcccccggcc acggggcctg ccaccatacc cacgccgaaa 480
   caagcgccct gcaccattat gttccggatc tgcatcgcag gatgctgctg gctaccctgt 540
   ggaacaccta catctgtatt aacgaagcgc taaccgtttt tatcaggctc tgggaggcag 600
   aataaatgat catatcgtca attattacct ccacggggag agcctgagca aactggcctc 660
   aggcatttga gaagcacacg gtcacactgc ttccggtagt caataaaccg gtaaaccagc 720
   aatagacata agcggctatt taacgaccct gccctgaacc gacgaccggg tcgaatttgc 780
   tttcgaattt ctgccattca tccgcttatt atcacttatt caggcgtagc accaggcgtt 840
   taagggcacc aataactgcc ttaaaaaaat tacgccccgc cctgccactc atcgcagtac 900
   tgttgtaatt cattaagcat tctgccgaca tggaagccat cacagacggc atgatgaacc 960
   tgaatcgcca gcggcatcag caccttgtcg ccttgcgtat aatatttgcc catagtgaaa 1020
   acgggggcga agaagttgtc catattggcc acgtttaaat caaaactggt gaaactcacc 1080
   cagggattgg ctgagacgaa aaacatattc tcaataaacc ctttagggaa ataggccagg 1140
   ttttcaccgt aacacgccac atcttgcgaa tatatgtgta gaaactgccg gaaatcgtcg 1200
   tggtattcac tccagagcga tgaaaacgtt tcagtttgct catggaaaac ggtgtaacaa 1260
   gggtgaacac tatcccatat caccagctca ccgtctttca ttgccatacg gaactccgga 1320
   tgagcattca tcaggcgggc aagaatgtga ataaaggccg gataaaactt gtgcttattt 1380
   ttctttacgg tctttaaaaa ggccgtaata tccagctgaa cggtctggtt ataggtacat 1440
   tgagcaactg actgaaatgc ctcaaaatgt tctttacgat gccattggga tatatcaacg 1500
   gtggtatatc cagtgatttt tttctccatt ttagcttcct tagctcctga aaatctcgat 1560
   aactcaaaaa atacgcccgg tagtgatctt atttcattat ggtgaaagtt ggaacctctt 1620
   acgtgccgat caacgtctca ttttcgccaa aagttggccc agggcttccc ggtatcaaca 1680
   gggacaccag gatttattta ttctgcgaag tgatcttccg tcacaggtat ttattcggcg 1740
   caaagtgcgt cgggtgatgc tgccaactta ctgatttagt gtatgatggt gtttttgagg 1800
   tgctccagtg gcttctgttt ctatcagctg tccctcctgt tcagctactg acggggtggt 1860
   gcgtaacggc aaaagcaccg ccggacatca gcgctagcgg agtgtatact ggcttactat 1920
   gttggcactg atgagggtgt cagtgaagtg cttcatgtgg caggagaaaa aaggctgcac 1980
   cggtgcgtca gcagaatatg tgatacagga tatattccgc ttcctcgctc actgactcgc 2040
   tacgctcggt cgttcgactg cggcgagcgg aaatggctta cgaacggggc ggagatttcc 2100
   tggaagatgc caggaagata cttaacaggg aagtgagagg gccgcggcaa agccgttttt 2160
   ccataggctc cgcccccctg acaagcatca cgaaatctga cgctcaaatc agtggtggcg 2220
   aaacccgaca ggactataaa gataccaggc gtttccccct ggcggctccc tcgtgcgctc 2280
   tcctgttcct gcctttcggt ttaccggtgt cattccgctg ttatggccgc gtttgtctca 2340
   ttccacgcct gacactcagt tccgggtagg cagttcgctc caagctggac tgtatgcacg 2400
   aaccccccgt tcagtccgac cgctgcgcct tatccggtaa ctatcgtctt gagtccaacc 2460
   cggaaagaca tgcaaaagca ccactggcag cagccactgg taattgattt agaggagtta 2520
   gtcttgaagt catgcgccgg ttaaggctaa actgaaagga caagttttgg tgactgcgct 2580
   cctccaagcc agttacctcg gttcaaagag ttggtagctc agagaacctt cgaaaaaccg 2640
   ccctgcaagg cggttttttc gttttcagag caagagatta cgcgcagacc aaaacgatct 2700
   caagaagatc atcttattaa tcagataaaa tatttctaga tttcagtgca atttatctct 2760
   tcaaatgtag cacctgaagt cagccccata cgatataagt tgtaattctc atgtttgaca 2820
   gcttatcatc gatacagtcc agttacgctg gagtctgagg ctcgtcctga atgatatgcg 2880
   gcctccacgt gcgtaattgt gctgatctct tatatagctg ctctcattat ctctctaccc 2940
   tgaagtgact ctctcacctg taaaaataat atctcacagg cttaatagtt tcttaataca 3000
   aagcctgtaa aacgtcagga taacttctgt gtaggaggat aatccatgag tctgaacttc 3060
   ctggacttcg agcagccgat cgccgaactg gaggcgaaga ttgacagcct gaccgcggtt 3120
   agccgtcaag atgagaaact ggacattaac atcgacgaag aggtccaccg tttgcgtgag 3180
   aagtctgttg aactgactcg caaaatcttt gctgatttgg gcgcatggca gattgcccag 3240
   ttggctcgcc acccacaacg cccatatacc ctggactacg tgcgcctggc gtttgacgag 3300
   ttcgacgaac tggcaggcga ccgcgcctat gcggacgata aagcaattgt cggcggtatt 3360
   gctcgtttgg atggccgtcc ggtgatgatt atcggccatc aaaaaggccg cgagacaaag 3420
   gaaaagattc gtcgtaattt cggaatgccg gcaccggagg gctaccgcaa ggccctgcgt 3480
   ctgatgcaaa tggccgaacg ctttaagatg ccgattatca cgttcattga tacgcctggt 3540
   gcgtacccag gcgttggtgc ggaagagcgt ggtcagagcg aggccatcgc acgtaacctg 3600
   cgtgagatgt ctcgtctggg tgtgccggtc gtttgcaccg tgattggcga gggcggtagc 3660
   ggtggtgcgt tggcgatcgg tgtcggtgat aaggtcaaca tgctgcaata cagcacgtac 3720
   agcgtcatta gcccggaagg ttgcgcttcc attctgtgga agagcgcgga taaagcacca 3780
   ttggcagcgg aagcgatggg tatcatcgca ccgcgtctga aagaactgaa gttgattgat 3840
   tctatcatcc cggaaccgct gggcggtgct caccgtaatc cggaggcgat ggcagccagc 3900
   ctgaaggccc agctgctggc ggacctggcg gatctggacg tgctgagcac ggaggatctg 3960
   aaaaaccgtc gctatcagcg cttgatgagc tatggctacg cctaaggtac ctttatcaat 4020
   ttatttaagg aggactctta agatgagctg gatcgagcgc atcaagagca acatcacccc 4080
   gacccgcaag gcgagcatcc ctgaaggcgt ctggaccaaa tgcgatagct gcggtcaggt 4140
   tttgtatcgt gcggagctgg agcgtaacct ggaagtgtgc ccgaaatgcg accatcacat 4200
   gcgtatgacc gctcgtaatc gtctgcatag cctgctggat gagggcagcc tggtcgagct 4260
   gggtagcgaa ctggaaccga aagatgttct gaaattccgt gattccaaga agtataagga 4320
   tcgtttggca tctgcacaaa aagaaaccgg tgagaaggac gcactggttg ttatgaaagg 4380
   caccctgtat ggtatgccgg ttgttgctgc ggcgttcgag tttgcgttta tgggtggcag 4440
   catgggttcc gtggtgggcg cacgctttgt gcgtgccgtg gagcaggcgc tggaggataa 4500
   ctgtcctctg atttgtttca gcgcgagcgg tggtgcgcgt atgcaagagg ccctgatgag 4560
   cctgatgcag atggcaaaaa cctcggcagc cctggcgaag atgcaagaac gcggcctgcc 4620
   gtacatttcc gtcctgaccg accctacgat gggcggtgtc agcgccagct ttgcgatgct 4680
   gggtgatttg aacatcgcag agccgaaggc tctgattggt tttgctggtc cgcgtgttat 4740
   tgaacagacg gttcgcgaaa agttgccgcc tggtttccag cgcagcgagt tcctgattga 4800
   gaaaggtgcc atcgacatga tcgttcgccg tccagaaatg cgtctgaaac tggcgagcat 4860
   tctggcgaaa ttgatgaatc tgccggctcc gaatcctgaa gcaccgcgtg agggtgtcgt 4920
   ggttccgccg gtcccggacc aagagccgga ggcttaatta accaaattag aagtatttta 4980
   tttagaggag gtagccatgg acatccgcaa gatcaagaag ctgatcgaac tggtggaaga 5040
   gtctggcatc agcgagctgg agatcagcga aggtgaagag agcgtccgta tttcccgtgc 5100
   ggcaccggca gcgagctttc cggttatgca gcaagcatac gccgctccga tgatgcaaca 5160
   gccggcacag agcaacgccg ctgcaccggc gaccgttcca agcatggagg caccggcagc 5220
   ggccgagatt tcgggtcata tcgtgcgtag cccgatggtg ggcaccttct atcgcacgcc 5280
   gtcgccggac gcaaaagcct tcatcgaagt cggccagaag gtcaatgtcg gcgacacgct 5340
   gtgtatcgtt gaggcaatga aaatgatgaa ccagattgaa gcggataaga gcggtactgt 5400
   taaagcgatc ctggtggaat ccggccagcc tgttgagttc gatgaaccgc tggttgtgat 5460
   cgagtaagag ctccggatat acggggaaga acaaggggtg tacatgctgg acaagatcgt 5520
   catcgccaac cgcggcgaaa tcgccctgcg catcttgcgc gcgtgtaaag agctgggcat 5580
   taagactgtt gccgtgcatt ccagcgcaga ccgcgacctg aagcatgttc tgctggccga 5640
   cgaaacggtt tgcatcggtc cggcaccgag cgtgaaaagc tatctgaaca tcccggccat 5700
   catctctgcg gcagagatca ccggtgcagt ggcgattcat ccgggctacg gtttcctgag 5760
   cgagaacgct aactttgctg aacaagtgga gcgtagcggt ttcatcttca ttggccctaa 5820
   ggcggagacg attcgcctga tgggcgacaa agtgagcgcc attgcagcga tgaaaaaggc 5880
   cggtgtgccg tgtgttccgg gcagcgatgg tccgctgggt gacgatatgg acaagaaccg 5940
   tgccatcgct aaacgtattg gctacccggt cattatcaaa gcctctggtg gtggcggtgg 6000
   ccgtggtatg cgtgtcgtcc gtggtgatgc ggaactggcg caaagcatca gcatgacccg 6060
   tgcggaagcc aaagcggcgt tctctaacga tatggtgtat atggagaagt atctggagaa 6120
   tccgcgccac gttgaaatcc aagttctggc ggatggtcag ggcaatgcga tctacttggc 6180
   agaacgtgat tgctccatgc aacgccgtca tcagaaggtg gtggaagagg caccggctcc 6240
   gggtattacg ccggaactgc gtcgctacat cggtgagcgc tgtgcgaaag cgtgtgtgga 6300
   cattggttac cgtggtgcgg gtacgtttga gttcctgttc gaaaatggtg agttttactt 6360
   cattgaaatg aatacccgca tccaggttga gcacccggtg accgagatga ttactggcgt 6420
   tgatctgatc aaagagcaac tgcgcattgc ggctggtcag ccgctgtcga tcaagcaaga     6480
   agaggtgcac gttcgtggtc acgcggtcga gtgccgtatc aatgcggagg acccgaatac     6540
   ctttctgccg agccctggta agatcactcg ttttcacgcg ccaggtggtt ttggcgttcg     6600
   ttgggagtct cacatctacg ccggttacac cgtgccgccg tactatgaca gcatgattgg     6660
   taaactgatc tgctatggcg aaaatcgtga tgtcgcgatc gcccgcatga aaaacgcgct     6720
   gcaagagctg atcattgatg gcattaagac caatgtggat ttgcagatcc gcattatgaa     6780
   cgacgagaat ttccagcacg gcggtacgaa cattcactac ctggaaaaga aactgggcct     6840
   gcaagagaaa taactgcaga gg                                              6862
<210> 185
   <211> 25
   <212> DNA
   <213> Artificial Sequence
<220>
   <223> Description of Artificial Sequence: Synthetic CPM1205 oligonucleotide
<400> 185
   gcgggttagc cagtgcaatt aatgg 25
<210> 186
   <211> 26
   <212> DNA
   <213> Artificial Sequence
<220>
   <223> Description of Artificial Sequence: Synthetic CPM1207 oligonucleotide
<400> 186
   gcacgagttg ttcgaaagcg aagaag 26
<210> 187
   <211> 26
   <212> DNA
   <213> Artificial Sequence
<220>
   <223> Description of Artificial Sequence: Synthetic CPM2011 oligonucleotide
<400> 187
   gtcgtcagtt caaggcagga taaggg 26
<210> 188
   <211> 24
   <212> DNA
   <213> Artificial Sequence
<220>
   <223> Description of Artificial Sequence: Synthetic CPM2017 oligonucleotide
<400> 188
   gagcttcagc cagttcggga tgta 24
<210> 189
   <211> 25
   <212> DNA
   <213> Artificial Sequence
<220>
   <223> Description of Artificial Sequence: Synthetic CPM2013 oligonucleotide
<400> 189
   ggtgcgggca tgaccggcgg tttcg 25
<210> 190
   <211> 25
   <212> DNA
   <213> Artificial Sequence
<220>
   <223> Description of Artificial Sequence: Synthetic CPM2015 oligonucleotide
<400> 190
   atatcgagac aatttacatg gaatg 25
<210> 191
   <211> 5902
   <212> DNA
   <213> Artificial Sequence
<220>
   <223> Description of Artificial Sequence: Synthetic pUC57-gdhA replace polynucleotide
<400> 191
   atttaaatgt ttgacagctt atcatcgact gcacggtgca ccaatgcttc tggcgtcagg 60
   cagccatcgg aagctgtggt atggctgtgc aggtcgtaaa tcactgcata attcgtgtcg 120
   ctcaaggcgc actcccgttc tggataatgt tttttgcgcc gacatcataa cggttctggc 180
   aaatattctg aaatgagctg ttgacaatta atcatccggc tcgtataatg tgtggaattg 240
   tgagcggata acaatttcac acaggaaaca gcgccgctga gaaaaagcga agcggcactg 300
   ctctttaaca atttatcaga caatctgtgt gggcactcga ccggaattat cgattaactt 360
   tattattaaa aattaaagag gtatatatta atgtatcgat taaataagga ggttataaga 420
   caattgtcct gataaacgca gcactgagta cagggctgcg ctgactgccc gcctgaactg 480
   aacacttctg gcggtacgtt tacctccacg tccggacgat aatgcgggtt agccagtgca 540
   attaatggaa atgctaatac tacggcgaac aatgctcgac tcacagggaa ctccttaacg 600
   ttattgtctc tgctactgat aacggtagcc gggtggcaaa actttagcgt ctgagttatc 660
   gcatttggtt atgagattac tctcgttatt aatttgcttt cctgggtcat ttttttcttg 720
   cttaccgtca cattcttgat ggtatagtcg aaaactgcaa aagcacatga cataaacaac 780
   ataagcacaa tcgtattaat atataagggt tttataacta tgagcatcag caaagcaatc 840
   gaaaaagttg aagcccgtta cgcacatcag ccggaattta tccaggcagt gaaagaagtg 900
   gcaatcacga ttaaaccgct gtatgatgcg catccggaat acgacaaact gaaagttttt 960
   gaacgtctgg tcgaaccgga tcgtgtgttt ggcttccgcg ttaactggga agatgacaat 1020
   ggcgaaattc agatcaaccg tggttggcgc gttcaatttt caaatgcact gggtccgtat 1080
   aaaggcggtc tgcgcttcca cccgaccgtc aaccagtcgg tgctgaaatt cctgggcttc 1140
   gaacaaatct tcaaaaatgc tctgacgggt ctgccgatcg gcggtggcaa aggtggctca 1200
   gatttcgacc cgaaaggcaa aaccgattcg gaaattcgtc gcttttgcta tgccttcatg 1260
   cgtgaactgc atcactacgt taacaaagat atggacgtcc cggcaggtga tattggtgtc 1320
   ggtggccgtg aagtgagcta tatgtttgcc atgtacaaaa atctgacccg cgaatctacg 1380
   ggtgtgatca ccggtaaagg cgttggtttt ggtggctccc tgatgcgcac cgaagcaacg 1440
   ggctatggtg ctgtttactt cctgcagaac atgctggcgg cccaaaatga aagcatcgaa 1500
   ggcaaaaaag tgctggttag cggcgccggt aacgtgtctc tgcatgcagc tgaaaaagcg 1560
   accctgattg gtgccatcgt gctgaccgtt agtgattcca aaggcaccat ttatgacgcg 1620
   aaaggtctga atcaggaaaa aatcgattgg ctgaaagttc agaaagacca acacaaaccg 1680
   ctggcagatt acgtcgaagt gtttggtggc gaatggatgg ctgaccagaa accgtggagt 1740
   attaaagcag atattgctat cccgtccgcg acgcagaacg aaatcaatga agaagatgcc 1800
   aaactgctgg ttgacaacgg cgtcaaatat attgtggaag gtgccaatat gccgctgacc 1860
   gcagaagcta ttgattatat ccgtctgcat cgtgtgcact acgcaccggg taaagcagca 1920
   aacgcgggtg gcgttgcagt cagcgccctg gaaatgtcac agaattcggt ccgtcagtat 1980
   caaacgttcg aacaggtgga tgaacgcctg caaggcatta tgaaagatat tcatgacagc 2040
   tctgcgcagg ccagtgaaat gtacggccaa accgatgaag gttatatcga ctacatgtcc 2100
   ggtgcgaaca tggtcggctt caaacgtgtc gcagacgcac tggttgcctt cggtatcctg 2160
   aactaataag ttgtaaatgc ctgatggcgc tacgcttatc aggcctacaa atgggcacaa 2220
   ttcattgcag ttacgctcta atgtaggccg ggcaagcgca gcgcccggcg gcggatccgg 2280
   cggcaggcct ggcgctagat caggttaaac ataacttcgt atagcataca ttatacgaag 2340
   ttattgttga caattaatca tcggcatagt atatcggcat agtataatac gacaaggtga 2400
   ggaactaaac catggccaag cctttgtctc aagaagaatc caccctcatt gaaagagcaa 2460
   cggctacaat caacagcatc cccatctctg aagactacag cgtcgccagc gcagctctct 2520
   ctagcgacgg ccgcatcttc actggtgtca atgtatatca ttttactggg ggaccttgtg 2580
   cagaactcgt ggtgctgggc actgctgctg ctgcggcagc tggcaacctg acttgtatcg 2640
   tcgcgatcgg aaatgagaac aggggcatct tgagcccctg cggacggtgc cgacaggtgc 2700
   ttctcgatct gcatcctggg atcaaagcca tagtgaagga cagtgatgga cagccgacgg 2760
   cagttgggat tcgtgaattg ctgccctctg gttatgtgtg ggagggctaa ataacttcgt 2820
   atagcataca ttatacgaag ttatagatct taacgtcgac gccgccggat ccgccgcccc 2880
   ggcaaaattt caggcgttta tgagtattta acggatgatg ctccccacgg aacatttctt 2940
   atgggccaac ggcatttctt actgtagtgc tcccaaaact gcttgtcgta acgataacac 3000
   gcttcaagtt cagcatccgt taactttctg cgatagcagc agatatgcca gtaaagaaat 3060
   cccatttgac tatttttttg ataatcttct tcgctttcga acaactcgtg cgcctttcga 3120
   gaagcaagca ttatataatg ccaggccagt tcttcttcaa ttgtcccgtt ttgaaaagct 3180
   gtgcatttaa atatcggatc ccgggcccgt cgactgcaga ggcctgcatg caagcttggc 3240
   gtaatcatgg tcatagctgt ttcctgtgtg aaattgttat ccgctcacaa ttccacacaa 3300
   catacgagcc ggaagcataa agtgtaaagc ctggggtgcc taatgagtga gctaactcac 3360
   attaattgcg ttgcgctcac tgcccgcttt ccagtcggga aacctgtcgt gccagctgca 3420
   ttaatgaatc ggccaacgcg cggggagagg cggtttgcgt attgggcgct cttccgcttc 3480
   ctcgctcact gactcgctgc gctcggtcgt tcggctgcgg cgagcggtat cagctcactc 3540
   aaaggcggta atacggttat ccacagaatc aggggataac gcaggaaaga acatgtgagc 3600
   aaaaggccag caaaaggcca ggaaccgtaa aaaggccgcg ttgctggcgt ttttccatag 3660
   gctccgcccc cctgacgagc atcacaaaaa tcgacgctca agtcagaggt ggcgaaaccc 3720
   gacaggacta taaagatacc aggcgtttcc ccctggaagc tccctcgtgc gctctcctgt 3780
   tccgaccctg ccgcttaccg gatacctgtc cgcctttctc ccttcgggaa gcgtggcgct 3840
   ttctcatagc tcacgctgta ggtatctcag ttcggtgtag gtcgttcgct ccaagctggg 3900
   ctgtgtgcac gaaccccccg ttcagcccga ccgctgcgcc ttatccggta actatcgtct 3960
   tgagtccaac ccggtaagac acgacttatc gccactggca gcagccactg gtaacaggat 4020
   tagcagagcg aggtatgtag gcggtgctac agagttcttg aagtggtggc ctaactacgg 4080
   ctacactaga agaacagtat ttggtatctg cgctctgctg aagccagtta ccttcggaaa 4140
   aagagttggt agctcttgat ccggcaaaca aaccaccgct ggtagcggtg gtttttttgt 4200
   ttgcaagcag cagattacgc gcagaaaaaa aggatctcaa gaagatcctt tgatcttttc 4260
   tacggggtct gacgctcagt ggaacgaaaa ctcacgttaa gggattttgg tcatgagatt 4320
   atcaaaaagg atcttcacct agatcctttt aaattaaaaa tgaagtttta aatcaatcta 4380
   aagtatatat gagtaaactt ggtctgacag ttaccaatgc ttaatcagtg aggcacctat 4440
   ctcagcgatc tgtctatttc gttcatccat agttgcctga ctccccgtcg tgtagataac 4500
   tacgatacgg gagggcttac catctggccc cagtgctgca atgataccgc gagacccacg 4560
   ctcaccggct ccagatttat cagcaataaa ccagccagcc ggaagggccg agcgcagaag 4620
   tggtcctgca actttatccg cctccatcca gtctattaat tgttgccggg aagctagagt 4680
   aagtagttcg ccagttaata gtttgcgcaa cgttgttgcc attgctacag gcatcgtggt 4740
   gtcacgctcg tcgtttggta tggcttcatt cagctccggt tcccaacgat caaggcgagt 4800
   tacatgatcc cccatgttgt gcaaaaaagc ggttagctcc ttcggtcctc cgatcgttgt 4860
   cagaagtaag ttggccgcag tgttatcact catggttatg gcagcactgc ataattctct 4920
   tactgtcatg ccatccgtaa gatgcttttc tgtgactggt gagtactcaa ccaagtcatt 4980
   ctgagaatag tgtatgcggc gaccgagttg ctcttgcccg gcgtcaatac gggataatac 5040
   cgcgccacat agcagaactt taaaagtgct catcattgga aaacgttctt cggggcgaaa 5100
   actctcaagg atcttaccgc tgttgagatc cagttcgatg taacccactc gtgcacccaa 5160
   ctgatcttca gcatctttta ctttcaccag cgtttctggg tgagcaaaaa caggaaggca 5220
   aaatgccgca aaaaagggaa taagggcgac acggaaatgt tgaatactca tactcttcct 5280
   ttttcaatat tattgaagca tttatcaggg ttattgtctc atgagcggat acatatttga 5340
   atgtatttag aaaaataaac aaataggggt tccgcgcaca tttccccgaa aagtgccacc 5400
   tgacgtctaa gaaaccatta ttatcatgac attaacctat aaaaataggc gtatcacgag 5460
   gccctttcgt ctcgcgcgtt tcggtgatga cggtgaaaac ctctgacaca tgcagctccc 5520
   ggagacggtc acagcttgtc tgtaagcgga tgccgggagc agacaagccc gtcagggcgc 5580
   gtcagcgggt gttggcgggt gtcggggctg gcttaactat gcggcatcag agcagattgt 5640
   actgagagtg caccatatgc ggtgtgaaat accgcacaga tgcgtaagga gaaaataccg 5700
   catcaggcgc cattcgccat tcaggctgcg caactgttgg gaagggcgat cggtgcgggc 5760
   ctcttcgcta ttacgccagc tggcgaaagg gggatgtgct gcaaggcgat taagttgggt 5820
   aacgccaggg ttttcccagt cacgacgttg taaaacgacg gccagtgaat tcgagctcgg 5880
   tacctcgcga atgcatctag at 5902
<210> 192
   <211> 4092
   <212> DNA
   <213> Artificial Sequence
<220>
   <223> Description of Artificial Sequence: Synthetic pUC57_Ptrc-StMCR polynucleotide
<400> 192
   ctagatataa tttaaatgcg gccgcttatt atttttcaat gtaacctttt tcaaccagca 60
   gttcagcagc caggatgcca ccgcccgccg caccgcgaac ggtattgtga atcagagaca 120
   ccaggcggat catacgtttg ttcacctgtt tcagacggcc cacgacaacg ctcatacccg 180
   gaatgtcgcc agcccagcga tcaaagtaca cttgcggacg cgtatcttca ttcatgacga 240
   taatcggttt ggacggtgcg gtcggcagtt tcagatcctg cggttcaccg cggaagtttt 300
   ccagcgtttc tttcactttt tcagctgcgg tttcttcttt aaaactaacg tacagcactt 360
   catagtggcc atgaatggtg gcgatacgat gcgtggtggc cgccagcgaa acgtcttcca 420
   gtttcggttc atcgacattg cgtttaactt ctgacagaat acggaagatt tctttaatgg 480
   ttttcgcgtc ataaccatcg cccagcggca gaatgttgtc cacgacatcc agagacggga 540
   tacccgggta acctgcaccg gacagactct ggatcgtggt aatgaacgcg ccgtccattt 600
   tgtaatcttt gaagatagca cccagcggga tagctgcacc ttgtgccgtg cacagcggcg 660
   tggtaacaat aaaacctttc cattcgcgac gtttgcgctg ttcatcgatc agactaatgg 720
   tgtgcggatt cagttccggc accagcagcg ggacgtccgg atcgaaacga tgatccggag 780
   agttgctgat gaccgggaag ccttcttttg caaattgttc ttcaaccgga ccggccgcac 840
   cctgcggcag cggcgaaaag ataatatcca cgtcatccat cagtttcggg tccgtcggtt 900
   taatttccat atcggcgatt tctttcggga cttggccaac ggtctgccaa cgaaccactt 960
   caccgtacgg tttgccaact gaacctttgc cggccagata cgccggtttg atatacggat 1020
   gatttgacag catacggaca tattcgatac caaccaggcc ggtggcgccc aggatagccg 1080
   ctttcagggt gcgacgcatc aattgtttcc tgtgtgaaat tgttatccgc tcacaattcc 1140
   acacattata cgagccggat gattaattgt caacagctca tttcagaata tttgccagaa 1200
   ccgttatgat gtcggcgcaa aaaacattat ccagaacggg agtgcgcctt gagcgacacg 1260
   aattatgcag tgatttacga cctgcacagc cataccacag cttccgatgg ctgcctgacg 1320
   ccagaagcat tggtgcaccg tgcagtcgat gataagctgt caaaccgcta gcatttaaat 1380
   attgggtcat cggatcccgg gcccgtcgac tgcagaggcc tgcatgcaag cttggcgtaa 1440
   tcatggtcat agctgtttcc tgtgtgaaat tgttatccgc tcacaattcc acacaacata 1500
   cgagccggaa gcataaagtg taaagcctgg ggtgcctaat gagtgagcta actcacatta 1560
   attgcgttgc gctcactgcc cgctttccag tcgggaaacc tgtcgtgcca gctgcattaa 1620
   tgaatcggcc aacgcgcggg gagaggcggt ttgcgtattg ggcgctcttc cgcttcctcg 1680
   ctcactgact cgctgcgctc ggtcgttcgg ctgcggcgag cggtatcagc tcactcaaag 1740
   gcggtaatac ggttatccac agaatcaggg gataacgcag gaaagaacat gtgagcaaaa 1800
   ggccagcaaa aggccaggaa ccgtaaaaag gccgcgttgc tggcgttttt ccataggctc 1860
   cgcccccctg acgagcatca caaaaatcga cgctcaagtc agaggtggcg aaacccgaca 1920
   ggactataaa gataccaggc gtttccccct ggaagctccc tcgtgcgctc tcctgttccg 1980
   accctgccgc ttaccggata cctgtccgcc tttctccctt cgggaagcgt ggcgctttct 2040
   catagctcac gctgtaggta tctcagttcg gtgtaggtcg ttcgctccaa gctgggctgt 2100
   gtgcacgaac cccccgttca gcccgaccgc tgcgccttat ccggtaacta tcgtcttgag 2160
   tccaacccgg taagacacga cttatcgcca ctggcagcag ccactggtaa caggattagc 2220
   agagcgaggt atgtaggcgg tgctacagag ttcttgaagt ggtggcctaa ctacggctac 2280
   actagaagaa cagtatttgg tatctgcgct ctgctgaagc cagttacctt cggaaaaaga 2340
   gttggtagct cttgatccgg caaacaaacc accgctggta gcggtggttt ttttgtttgc 2400
   aagcagcaga ttacgcgcag aaaaaaagga tctcaagaag atcctttgat cttttctacg 2460
   gggtctgacg ctcagtggaa cgaaaactca cgttaaggga ttttggtcat gagattatca 2520
   aaaaggatct tcacctagat ccttttaaat taaaaatgaa gttttaaatc aatctaaagt 2580
   atatatgagt aaacttggtc tgacagttac caatgcttaa tcagtgaggc acctatctca 2640
   gcgatctgtc tatttcgttc atccatagtt gcctgactcc ccgtcgtgta gataactacg 2700
   atacgggagg gcttaccatc tggccccagt gctgcaatga taccgcgaga cccacgctca 2760
   ccggctccag atttatcagc aataaaccag ccagccggaa gggccgagcg cagaagtggt 2820
   cctgcaactt tatccgcctc catccagtct attaattgtt gccgggaagc tagagtaagt 2880
   agttcgccag ttaatagttt gcgcaacgtt gttgccattg ctacaggcat cgtggtgtca 2940
   cgctcgtcgt ttggtatggc ttcattcagc tccggttccc aacgatcaag gcgagttaca 3000
   tgatccccca tgttgtgcaa aaaagcggtt agctccttcg gtcctccgat cgttgtcaga 3060
   agtaagttgg ccgcagtgtt atcactcatg gttatggcag cactgcataa ttctcttact 3120
   gtcatgccat ccgtaagatg cttttctgtg actggtgagt actcaaccaa gtcattctga 3180
   gaatagtgta tgcggcgacc gagttgctct tgcccggcgt caatacggga taataccgcg 3240
   ccacatagca gaactttaaa agtgctcatc attggaaaac gttcttcggg gcgaaaactc 3300
   tcaaggatct taccgctgtt gagatccagt tcgatgtaac ccactcgtgc acccaactga 3360
   tcttcagcat cttttacttt caccagcgtt tctgggtgag caaaaacagg aaggcaaaat 3420
   gccgcaaaaa agggaataag ggcgacacgg aaatgttgaa tactcatact cttccttttt 3480
   caatattatt gaagcattta tcagggttat tgtctcatga gcggatacat atttgaatgt 3540
   atttagaaaa ataaacaaat aggggttccg cgcacatttc cccgaaaagt gccacctgac 3600
   gtctaagaaa ccattattat catgacatta acctataaaa ataggcgtat cacgaggccc 3660
   tttcgtctcg cgcgtttcgg tgatgacggt gaaaacctct gacacatgca gctcccggag 3720
   acggtcacag cttgtctgta agcggatgcc gggagcagac aagcccgtca gggcgcgtca 3780
   gcgggtgttg gcgggtgtcg gggctggctt aactatgcgg catcagagca gattgtactg      3840
   agagtgcacc atatgcggtg tgaaataccg cacagatgcg taaggagaaa ataccgcatc      3900
   aggcgccatt cgccattcag gctgcgcaac tgttgggaag ggcgatcggt gcgggcctct      3960
   tcgctattac gccagctggc gaaaggggga tgtgctgcaa ggcgattaag ttgggtaacg      4020
   ccagggtttt cccagtcacg acgttgtaaa acgacggcca gtgaattcga gctcggtacc      4080
   tcgcgaatgc at                                                          4092
<210> 193
   <211> 4008
   <212> DNA
   <213> Artificial Sequence
<220>
   <223> Description of Artificial Sequence: Synthetic pACYC duet polynucleotide
<400> 193
   ggggaattgt gagcggataa caattcccct gtagaaataa ttttgtttaa ctttaataag 60
   gagatatacc atgggcagca gccatcacca tcatcaccac agccaggatc cgaattcgag 120
   ctcggcgcgc ctgcaggtcg acaagcttgc ggccgcataa tgcttaagtc gaacagaaag 180
   taatcgtatt gtacacggcc gcataatcga aattaatacg actcactata ggggaattgt 240
   gagcggataa caattcccca tcttagtata ttagttaagt ataagaagga gatatacata 300
   tggcagatct caattggata tcggccggcc acgcgatcgc tgacgtcggt accctcgagt 360
   ctggtaaaga aaccgctgct gcgaaatttg aacgccagca catggactcg tctactagcg 420
   cagcttaatt aacctaggct gctgccaccg ctgagcaata actagcataa ccccttgggg 480
   cctctaaacg ggtcttgagg ggttttttgc tgaaacctca ggcatttgag aagcacacgg 540
   tcacactgct tccggtagtc aataaaccgg taaaccagca atagacataa gcggctattt 600
   aacgaccctg ccctgaaccg acgaccgggt cgaatttgct ttcgaatttc tgccattcat 660
   ccgcttatta tcacttattc aggcgtagca ccaggcgttt aagggcacca ataactgcct 720
   taaaaaaatt acgccccgcc ctgccactca tcgcagtact gttgtaattc attaagcatt 780
   ctgccgacat ggaagccatc acagacggca tgatgaacct gaatcgccag cggcatcagc 840
   accttgtcgc cttgcgtata atatttgccc atagtgaaaa cgggggcgaa gaagttgtcc 900
   atattggcca cgtttaaatc aaaactggtg aaactcaccc agggattggc tgagacgaaa 960
   aacatattct caataaaccc tttagggaaa taggccaggt tttcaccgta acacgccaca 1020
   tcttgcgaat atatgtgtag aaactgccgg aaatcgtcgt ggtattcact ccagagcgat 1080
   gaaaacgttt cagtttgctc atggaaaacg gtgtaacaag ggtgaacact atcccatatc 1140
   accagctcac cgtctttcat tgccatacgg aactccggat gagcattcat caggcgggca 1200
   agaatgtgaa taaaggccgg ataaaacttg tgcttatttt tctttacggt ctttaaaaag 1260
   gccgtaatat ccagctgaac ggtctggtta taggtacatt gagcaactga ctgaaatgcc 1320
   tcaaaatgtt ctttacgatg ccattgggat atatcaacgg tggtatatcc agtgattttt 1380
   ttctccattt tagcttcctt agctcctgaa aatctcgata actcaaaaaa tacgcccggt 1440
   agtgatctta tttcattatg gtgaaagttg gaacctctta cgtgccgatc aacgtctcat 1500
   tttcgccaaa agttggccca gggcttcccg gtatcaacag ggacaccagg atttatttat 1560
   tctgcgaagt gatcttccgt cacaggtatt tattcggcgc aaagtgcgtc gggtgatgct 1620
   gccaacttac tgatttagtg tatgatggtg tttttgaggt gctccagtgg cttctgtttc 1680
   tatcagctgt ccctcctgtt cagctactga cggggtggtg cgtaacggca aaagcaccgc 1740
   cggacatcag cgctagcgga gtgtatactg gcttactatg ttggcactga tgagggtgtc 1800
   agtgaagtgc ttcatgtggc aggagaaaaa aggctgcacc ggtgcgtcag cagaatatgt 1860
   gatacaggat atattccgct tcctcgctca ctgactcgct acgctcggtc gttcgactgc 1920
   ggcgagcgga aatggcttac gaacggggcg gagatttcct ggaagatgcc aggaagatac 1980
   ttaacaggga agtgagaggg ccgcggcaaa gccgtttttc cataggctcc gcccccctga 2040
   caagcatcac gaaatctgac gctcaaatca gtggtggcga aacccgacag gactataaag 2100
   ataccaggcg tttcccctgg cggctccctc gtgcgctctc ctgttcctgc ctttcggttt 2160
   accggtgtca ttccgctgtt atggccgcgt ttgtctcatt ccacgcctga cactcagttc 2220
   cgggtaggca gttcgctcca agctggactg tatgcacgaa ccccccgttc agtccgaccg 2280
   ctgcgcctta tccggtaact atcgtcttga gtccaacccg gaaagacatg caaaagcacc 2340
   actggcagca gccactggta attgatttag aggagttagt cttgaagtca tgcgccggtt 2400
   aaggctaaac tgaaaggaca agttttggtg actgcgctcc tccaagccag ttacctcggt 2460
   tcaaagagtt ggtagctcag agaaccttcg aaaaaccgcc ctgcaaggcg gttttttcgt 2520
   tttcagagca agagattacg cgcagaccaa aacgatctca agaagatcat cttattaatc 2580
   agataaaata tttctagatt tcagtgcaat ttatctcttc aaatgtagca cctgaagtca 2640
   gccccatacg atataagttg taattctcat gttagtcatg ccccgcgccc accggaagga 2700
   gctgactggg ttgaaggctc tcaagggcat cggtcgagat cccggtgcct aatgagtgag 2760
   ctaacttaca ttaattgcgt tgcgctcact gcccgctttc cagtcgggaa acctgtcgtg 2820
   ccagctgcat taatgaatcg gccaacgcgc ggggagaggc ggtttgcgta ttgggcgcca 2880
   gggtggtttt tcttttcacc agtgagacgg gcaacagctg attgcccttc accgcctggc 2940
   cctgagagag ttgcagcaag cggtccacgc tggtttgccc cagcaggcga aaatcctgtt 3000
   tgatggtggt taacggcggg atataacatg agctgtcttc ggtatcgtcg tatcccacta 3060
   ccgagatgtc cgcaccaacg cgcagcccgg actcggtaat ggcgcgcatt gcgcccagcg 3120
   ccatctgatc gttggcaacc agcatcgcag tgggaacgat gccctcattc agcatttgca 3180
   tggtttgttg aaaaccggac atggcactcc agtcgccttc ccgttccgct atcggctgaa 3240
   tttgattgcg agtgagatat ttatgccagc cagccagacg cagacgcgcc gagacagaac 3300
   ttaatgggcc cgctaacagc gcgatttgct ggtgacccaa tgcgaccaga tgctccacgc 3360
   ccagtcgcgt accgtcttca tgggagaaaa taatactgtt gatgggtgtc tggtcagaga 3420
   catcaagaaa taacgccgga acattagtgc aggcagcttc cacagcaatg gcatcctggt 3480
   catccagcgg atagttaatg atcagcccac tgacgcgttg cgcgagaaga ttgtgcaccg 3540
   ccgctttaca ggcttcgacg ccgcttcgtt ctaccatcga caccaccacg ctggcaccca 3600
   gttgatcggc gcgagattta atcgccgcga caatttgcga cggcgcgtgc agggccagac 3660
   tggaggtggc aacgccaatc agcaacgact gtttgcccgc cagttgttgt gccacgcggt 3720
   tgggaatgta attcagctcc gccatcgccg cttccacttt ttcccgcgtt ttcgcagaaa 3780
   cgtggctggc ctggttcacc acgcgggaaa cggtctgata agagacaccg gcatactctg 3840
   cgacatcgta taacgttact ggtttcacat tcaccaccct gaattgactc tcttccgggc 3900
   gctatcatgc cataccgcga aaggttttgc gccattcgat ggtgtccggg atctcgacgc 3960
   tctcccttat gcgactcctg cattaggaaa ttaatacgac tcactata 4008
<210> 194
   <211> 5944
   <212> DNA
   <213> Artificial Sequence
<220>
   <223> Description of Artificial Sequence: Synthetic pET28b::mAID polynucleotide
<400> 194
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattggat ccctatcagt ggtggtggtg gtggtggtgg tggtggtggg 240
   gcccctggaa cagaacttcc agccctccgc ctccaaatcc caacatacga aatgcatctc 300
   gcaagtcatc gacttcgtac aagggcaaaa ggatgcgccg aagttgtctg gttagccgga 360
   cagaattttc atgtagccct tcccaggctt tgaaagttct ttcacgattt tctacaaatg 420
   tattccagca gtaaaaatag tctttgaagg tcatgatccc gatctggacc ccagcgcggt 480
   gcagtctccg cagcccctca ggctcagcct tgcggtcttc acagaagtag aggcgcgcgg 540
   tgaaaatcct caggctgagg ttagggttcc atctcagaaa ctcagccacg tgccgggcac 600
   agtcatagca cgggctccag gaggtgaacc aggtgacgcg gtaacaccgg cccgggtcca 660
   ggtcccagtc tgagatgtag cgtaggaaca acaattccac gtggcagcca gacttgttgc 720
   gaaggtggcc gaagtccagt gagcaggagg tggcactatc tctcctcttc accacgtagc 780
   agaggtaggt ctcatgccgt cccttggccc agcggacatt tttgaaatgg taaagaaact 840
   tcttttgctt catcagaagg ctgtccattc ccatggtata tctccttctt aaagttaaac 900
   aaaattattt ctagagggga attgttatcc gctcacaatt cccctatagt gagtcgtatt 960
   aatttcgcgg gatcgagatc tcgatcctct acgccggacg catcgtggcc ggcatcaccg 1020
   gcgccacagg tgcggttgct ggcgcctata tcgccgacat caccgatggg gaagatcggg 1080
   ctcgccactt cgggctcatg agcgcttgtt tcggcgtggg tatggtggca ggccccgtgg 1140
   ccgggggact gttgggcgcc atctccttgc atgcaccatt ccttgcggcg gcggtgctca 1200
   acggcctcaa cctactactg ggctgcttcc taatgcagga gtcgcataag ggagagcgtc 1260
   gagatcccgg acaccatcga atggcgcaaa acctttcgcg gtatggcatg atagcgcccg 1320
   gaagagagtc aattcagggt ggtgaatgtg aaaccagtaa cgttatacga tgtcgcagag 1380
   tatgccggtg tctcttatca gaccgtttcc cgcgtggtga accaggccag ccacgtttct 1440
   gcgaaaacgc gggaaaaagt ggaagcggcg atggcggagc tgaattacat tcccaaccgc 1500
   gtggcacaac aactggcggg caaacagtcg ttgctgattg gcgttgccac ctccagtctg 1560
   gccctgcacg cgccgtcgca aattgtcgcg gcgattaaat ctcgcgccga tcaactgggt 1620
   gccagcgtgg tggtgtcgat ggtagaacga agcggcgtcg aagcctgtaa agcggcggtg 1680
   cacaatcttc tcgcgcaacg cgtcagtggg ctgatcatta actatccgct ggatgaccag 1740
   gatgccattg ctgtggaagc tgcctgcact aatgttccgg cgttatttct tgatgtctct 1800
   gaccagacac ccatcaacag tattattttc tcccatgaag acggtacgcg actgggcgtg 1860
   gagcatctgg tcgcattggg tcaccagcaa atcgcgctgt tagcgggccc attaagttct 1920
   gtctcggcgc gtctgcgtct ggctggctgg cataaatatc tcactcgcaa tcaaattcag 1980
   ccgatagcgg aacgggaagg cgactggagt gccatgtccg gttttcaaca aaccatgcaa 2040
   atgctgaatg agggcatcgt tcccactgcg atgctggttg ccaacgatca gatggcgctg 2100
   ggcgcaatgc gcgccattac cgagtccggg ctgcgcgttg gtgcggatat ctcggtagtg 2160
   ggatacgacg ataccgaaga cagctcatgt tatatcccgc cgttaaccac catcaaacag 2220
   gattttcgcc tgctggggca aaccagcgtg gaccgcttgc tgcaactctc tcagggccag 2280
   gcggtgaagg gcaatcagct gttgcccgtc tcactggtga aaagaaaaac caccctggcg 2340
   cccaatacgc aaaccgcctc tccccgcgcg ttggccgatt cattaatgca gctggcacga 2400
   caggtttccc gactggaaag cgggcagtga gcgcaacgca attaatgtaa gttagctcac 2460
   tcattaggca ccgggatctc gaccgatgcc cttgagagcc ttcaacccag tcagctcctt 2520
   ccggtgggcg cggggcatga ctatcgtcgc cgcacttatg actgtcttct ttatcatgca 2580
   actcgtagga caggtgccgg cagcgctctg ggtcattttc ggcgaggacc gctttcgctg 2640
   gagcgcgacg atgatcggcc tgtcgcttgc ggtattcgga atcttgcacg ccctcgctca 2700
   agccttcgtc actggtcccg ccaccaaacg tttcggcgag aagcaggcca ttatcgccgg 2760
   catggcggcc ccacgggtgc gcatgatcgt gctcctgtcg ttgaggaccc ggctaggctg 2820
   gcggggttgc cttactggtt agcagaatga atcaccgata cgcgagcgaa cgtgaagcga 2880
   ctgctgctgc aaaacgtctg cgacctgagc aacaacatga atggtcttcg gtttccgtgt 2940
   ttcgtaaagt ctggaaacgc ggaagtcagc gccctgcacc attatgttcc ggatctgcat 3000
   cgcaggatgc tgctggctac cctgtggaac acctacatct gtattaacga agcgctggca 3060
   ttgaccctga gtgatttttc tctggtcccg ccgcatccat accgccagtt gtttaccctc 3120
   acaacgttcc agtaaccggg catgttcatc atcagtaacc cgtatcgtga gcatcctctc 3180
   tcgtttcatc ggtatcatta cccccatgaa cagaaatccc ccttacacgg aggcatcagt 3240
   gaccaaacag gaaaaaaccg cccttaacat ggcccgcttt atcagaagcc agacattaac 3300
   gcttctggag aaactcaacg agctggacgc ggatgaacag gcagacatct gtgaatcgct 3360
   tcacgaccac gctgatgagc tttaccgcag ctgcctcgcg cgtttcggtg atgacggtga 3420
   aaacctctga cacatgcagc tcccggagac ggtcacagct tgtctgtaag cggatgccgg 3480
   gagcagacaa gcccgtcagg gcgcgtcagc gggtgttggc gggtgtcggg gcgcagccat 3540
   gacccagtca cgtagcgata gcggagtgta tactggctta actatgcggc atcagagcag 3600
   attgtactga gagtgcacca tatatgcggt gtgaaatacc gcacagatgc gtaaggagaa 3660
   aataccgcat caggcgctct tccgcttcct cgctcactga ctcgctgcgc tcggtcgttc 3720
   ggctgcggcg agcggtatca gctcactcaa aggcggtaat acggttatcc acagaatcag 3780
   gggataacgc aggaaagaac atgtgagcaa aaggccagca aaaggccagg aaccgtaaaa 3840
   aggccgcgtt gctggcgttt ttccataggc tccgcccccc tgacgagcat cacaaaaatc 3900
   gacgctcaag tcagaggtgg cgaaacccga caggactata aagataccag gcgtttcccc 3960
   ctggaagctc cctcgtgcgc tctcctgttc cgaccctgcc gcttaccgga tacctgtccg 4020
   cctttctccc ttcgggaagc gtggcgcttt ctcatagctc acgctgtagg tatctcagtt 4080
   cggtgtaggt cgttcgctcc aagctgggct gtgtgcacga accccccgtt cagcccgacc 4140
   gctgcgcctt atccggtaac tatcgtcttg agtccaaccc ggtaagacac gacttatcgc 4200
   cactggcagc agccactggt aacaggatta gcagagcgag gtatgtaggc ggtgctacag 4260
   agttcttgaa gtggtggcct aactacggct acactagaag gacagtattt ggtatctgcg 4320
   ctctgctgaa gccagttacc ttcggaaaaa gagttggtag ctcttgatcc ggcaaacaaa 4380
   ccaccgctgg tagcggtggt ttttttgttt gcaagcagca gattacgcgc agaaaaaaag 4440
   gatctcaaga agatcctttg atcttttcta cggggtctga cgctcagtgg aacgaaaact 4500
   cacgttaagg gattttggtc atgaacaata aaactgtctg cttacataaa cagtaataca 4560
   aggggtgtta tgagccatat tcaacgggaa acgtcttgct ctaggccgcg attaaattcc 4620
   aacatggatg ctgatttata tgggtataaa tgggctcgcg ataatgtcgg gcaatcaggt 4680
   gcgacaatct atcgattgta tgggaagccc gatgcgccag agttgtttct gaaacatggc 4740
   aaaggtagcg ttgccaatga tgttacagat gagatggtca gactaaactg gctgacggaa 4800
   tttatgcctc ttccgaccat caagcatttt atccgtactc ctgatgatgc atggttactc 4860
   accactgcga tccccgggaa aacagcattc caggtattag aagaatatcc tgattcaggt 4920
   gaaaatattg ttgatgcgct ggcagtgttc ctgcgccggt tgcattcgat tcctgtttgt 4980
   aattgtcctt ttaacagcga tcgcgtattt cgtctcgctc aggcgcaatc acgaatgaat 5040
   aacggtttgg ttgatgcgag tgattttgat gacgagcgta atggctggcc tgttgaacaa 5100
   gtctggaaag aaatgcataa acttttgcca ttctcaccgg attcagtcgt cactcatggt 5160
   gatttctcac ttgataacct tatttttgac gaggggaaat taataggttg tattgatgtt 5220
   ggacgagtcg gaatcgcaga ccgataccag gatcttgcca tcctatggaa ctgcctcggt 5280
   gagttttctc cttcattaca gaaacggctt tttcaaaaat atggtattga taatcctgat 5340
   atgaataaat tgcagtttca tttgatgctc gatgagtttt tctaagaatt aattcatgag 5400
   cggatacata tttgaatgta tttagaaaaa taaacaaata ggggttccgc gcacatttcc 5460
   ccgaaaagtg ccacctgaaa ttgtaaacgt taatattttg ttaaaattcg cgttaaattt 5520
   ttgttaaatc agctcatttt ttaaccaata ggccgaaatc ggcaaaatcc cttataaatc 5580
   aaaagaatag accgagatag ggttgagtgt tgttccagtt tggaacaaga gtccactatt 5640
   aaagaacgtg gactccaacg tcaaagggcg aaaaaccgtc tatcagggcg atggcccact 5700
   acgtgaacca tcaccctaat caagtttttt ggggtcgagg tgccgtaaag cactaaatcg 5760
   gaaccctaaa gggagccccc gatttagagc ttgacgggga aagccggcga acgtggcgag 5820
   aaaggaaggg aagaaagcga aaggagcggg cgctagggcg ctggcaagtg tagcggtcac 5880
   gctgcgcgta accaccacac ccgccgcgct taatgcgccg ctacagggcg cgtcccattc 5940
   gcca 5944
<210> 195
   <211> 7868
   <212> DNA
   <213> Artificial Sequence
<220>
   <223> Description of Artificial Sequence: Synthetic pACYC-T7-phaA-hbd-crt-ter polynucleotide
<400> 195
<210> 196
   <211> 7687
   <212> DNA
   <213> Artificial Sequence
<220>
   <223> Description of Artificial Sequence: Synthetic pACYC-T7-nphT07_hbd_crt_ter polynucleotide
<400> 196
   cgctgacgtc ggtaccctcg agtctggtaa agaaaccgct gctgcgaaat ttgaacgcca 60
   gcacatggac tcgtctacta gcgcagctta attaacctag gctgctgcca ccgctgagca 120
   ataactagca taaccccttg gggcctctaa acgggtcttg aggggttttt tgctgaaacc 180
   tcaggcattt gagaagcaca cggtcacact gcttccggta gtcaataaac cggtaaacca 240
   gcaatagaca taagcggcta tttaacgacc ctgccctgaa ccgacgaccg ggtcgaattt 300
   gctttcgaat ttctgccatt catccgctta ttatcactta ttcaggcgta gcaccaggcg 360
   tttaagggca ccaataactg ccttaaaaaa attacgcccc gccctgccac tcatcgcagt 420
   actgttgtaa ttcattaagc attctgccga catggaagcc atcacagacg gcatgatgaa 480
   cctgaatcgc cagcggcatc agcaccttgt cgccttgcgt ataatatttg cccatagtga 540
   aaacgggggc gaagaagttg tccatattgg ccacgtttaa atcaaaactg gtgaaactca 600
   cccagggatt ggctgagacg aaaaacatat tctcaataaa ccctttaggg aaataggcca 660
   ggttttcacc gtaacacgcc acatcttgcg aatatatgtg tagaaactgc cggaaatcgt 720
   cgtggtattc actccagagc gatgaaaacg tttcagtttg ctcatggaaa acggtgtaac 780
   aagggtgaac actatcccat atcaccagct caccgtcttt cattgccata cggaactccg 840
   gatgagcatt catcaggcgg gcaagaatgt gaataaaggc cggataaaac ttgtgcttat 900
   ttttctttac ggtctttaaa aaggccgtaa tatccagctg aacggtctgg ttataggtac 960
   attgagcaac tgactgaaat gcctcaaaat gttctttacg atgccattgg gatatatcaa 1020
   cggtggtata tccagtgatt tttttctcca ttttagcttc cttagctcct gaaaatctcg 1080
   ataactcaaa aaatacgccc ggtagtgatc ttatttcatt atggtgaaag ttggaacctc 1140
   ttacgtgccg atcaacgtct cattttcgcc aaaagttggc ccagggcttc ccggtatcaa 1200
   cagggacacc aggatttatt tattctgcga agtgatcttc cgtcacaggt atttattcgg 1260
   cgcaaagtgc gtcgggtgat gctgccaact tactgattta gtgtatgatg gtgtttttga 1320
   ggtgctccag tggcttctgt ttctatcagc tgtccctcct gttcagctac tgacggggtg 1380
   gtgcgtaacg gcaaaagcac cgccggacat cagcgctagc ggagtgtata ctggcttact 1440
   atgttggcac tgatgagggt gtcagtgaag tgcttcatgt ggcaggagaa aaaaggctgc 1500
   accggtgcgt cagcagaata tgtgatacag gatatattcc gcttcctcgc tcactgactc 1560
   gctacgctcg gtcgttcgac tgcggcgagc ggaaatggct tacgaacggg gcggagattt 1620
   cctggaagat gccaggaaga tacttaacag ggaagtgaga gggccgcggc aaagccgttt 1680
   ttccataggc tccgcccccc tgacaagcat cacgaaatct gacgctcaaa tcagtggtgg 1740
   cgaaacccga caggactata aagataccag gcgtttcccc tggcggctcc ctcgtgcgct 1800
   ctcctgttcc tgcctttcgg tttaccggtg tcattccgct gttatggccg cgtttgtctc 1860
   attccacgcc tgacactcag ttccgggtag gcagttcgct ccaagctgga ctgtatgcac 1920
   gaaccccccg ttcagtccga ccgctgcgcc ttatccggta actatcgtct tgagtccaac 1980
   ccggaaagac atgcaaaagc accactggca gcagccactg gtaattgatt tagaggagtt 2040
   agtcttgaag tcatgcgccg gttaaggcta aactgaaagg acaagttttg gtgactgcgc 2100
   tcctccaagc cagttacctc ggttcaaaga gttggtagct cagagaacct tcgaaaaacc 2160
   gccctgcaag gcggtttttt cgttttcaga gcaagagatt acgcgcagac caaaacgatc 2220
   tcaagaagat catcttatta atcagataaa atatttctag atttcagtgc aatttatctc 2280
   ttcaaatgta gcacctgaag tcagccccat acgatataag ttgtaattct catgttagtc 2340
   atgccccgcg cccaccggaa ggagctgact gggttgaagg ctctcaaggg catcggtcga 2400
   gatcccggtg cctaatgagt gagctaactt acattaattg cgttgcgctc actgcccgct 2460
   ttccagtcgg gaaacctgtc gtgccagctg cattaatgaa tcggccaacg cgcggggaga 2520
   ggcggtttgc gtattgggcg ccagggtggt ttttcttttc accagtgaga cgggcaacag 2580
   ctgattgccc ttcaccgcct ggccctgaga gagttgcagc aagcggtcca cgctggtttg 2640
   ccccagcagg cgaaaatcct gtttgatggt ggttaacggc gggatataac atgagctgtc 2700
   ttcggtatcg tcgtatccca ctaccgagat gtccgcacca acgcgcagcc cggactcggt 2760
   aatggcgcgc attgcgccca gcgccatctg atcgttggca accagcatcg cagtgggaac 2820
   gatgccctca ttcagcattt gcatggtttg ttgaaaaccg gacatggcac tccagtcgcc 2880
   ttcccgttcc gctatcggct gaatttgatt gcgagtgaga tatttatgcc agccagccag 2940
   acgcagacgc gccgagacag aacttaatgg gcccgctaac agcgcgattt gctggtgacc 3000
   caatgcgacc agatgctcca cgcccagtcg cgtaccgtct tcatgggaga aaataatact 3060
   gttgatgggt gtctggtcag agacatcaag aaataacgcc ggaacattag tgcaggcagc 3120
   ttccacagca atggcatcct ggtcatccag cggatagtta atgatcagcc cactgacgcg 3180
   ttgcgcgaga agattgtgca ccgccgcttt acaggcttcg acgccgcttc gttctaccat 3240
   cgacaccacc acgctggcac ccagttgatc ggcgcgagat ttaatcgccg cgacaatttg 3300
   cgacggcgcg tgcagggcca gactggaggt ggcaacgcca atcagcaacg actgtttgcc 3360
   cgccagttgt tgtgccacgc ggttgggaat gtaattcagc tccgccatcg ccgcttccac 3420
   tttttcccgc gttttcgcag aaacgtggct ggcctggttc accacgcggg aaacggtctg 3480
   ataagagaca ccggcatact ctgcgacatc gtataacgtt actggtttca cattcaccac 3540
   cctgaattga ctctcttccg ggcgctatca tgccataccg cgaaaggttt tgcgccattc 3600
   gatggtgtcc gggatctcga cgctctccct tatgcgactc ctgcattagg aaattaatac 3660
   gactcactat aggggaattg tgagcggata acaattcccc tgtagaaata attttgttta 3720
   actttaataa ggagatatac catgacggat gtccgctttc gtatcattgg cacgggcgct 3780
   tacgtcccgg aacgcattgt cagcaacgac gaagtcggcg caccggctgg tgtggatgac 3840
   gattggatta cgcgtaaaac cggtatccgc cagcgtcgct gggcggccga cgatcaagca 3900
   acgagcgatc tggctaccgc agctggtcgt gcagcactga aagcagctgg cattacgccg 3960
   gaacagctga ccgtcatcgc agtggctacc tctacgccgg accgtccgca gccgccgacc 4020
   gcggcctatg ttcaacatca cctgggtgca accggcacgg cagcttttga tgttaacgct 4080
   gtgtgcagcg gtacggtttt cgcgctgagc tctgttgccg gcaccctggt ctatcgtggc 4140
   ggttacgcac tggtcattgg tgctgatctg tactcacgta tcctgaatcc ggcggaccgc 4200
   aaaaccgtgg ttctgttcgg cgatggtgcg ggcgcgatgg tgctgggccc gaccagcacc 4260
   ggcaccggtc cgattgtgcg tcgcgttgca ctgcatacgt ttggcggtct gaccgatctg 4320
   atccgtgttc cggccggcgg ttcccgccag ccgctggaca ccgatggtct ggacgcaggc 4380
   ctgcaatatt ttgcgatgga tggccgcgaa gtccgtcgct tcgtgaccga acatctgccg 4440
   cagctgatta aaggttttct gcacgaagcg ggcgtggatg cggcggatat tagccatttc 4500
   gtgccgcacc aagccaacgg tgtgatgctg gacgaagttt ttggcgaact gcatctgccg 4560
   cgtgcaacca tgcaccgtac ggtggaaacc tacggtaata cgggcgcagc tagtattccg 4620
   atcacgatgg atgcggccgt tcgtgcaggt tccttccgtc cgggcgaact ggttctgctg 4680
   gcgggctttg gcggcggtat ggcggcttcg tttgctctga ttgaatggtg acctaatgca 4740
   ggctgcaggc ggatacgagg aggaataaac catgaaaaag gtatgtgtta taggtgcagg 4800
   tactatgggt tcaggaattg ctcaggcatt tgcagctaaa ggatttgaag tagtattaag 4860
   agatattaaa gatgaatttg ttgatagagg attagatttt atcaataaaa atctttctaa 4920
   attagttaaa aaaggaaaga tagaagaagc tactaaagtt gaaatcttaa ctagaatttc 4980
   cggaacagtt gaccttaata tggcagctga ttgcgattta gttatagaag cagctgttga 5040
   aagaatggat attaaaaagc agatttttgc tgacttagac aatatatgca agccagaaac 5100
   aattcttgca tcaaatacat catcactttc aataacagaa gtggcatcag caactaaaag 5160
   acctgataag gttataggta tgcatttctt taatccagct cctgttatga agcttgtaga 5220
   ggtaataaga ggaatagcta catcacaaga aacttttgat gcagttaaag agacatctat 5280
   agcaatagga aaagatcctg tagaagtagc agaagcacca ggatttgttg taaatagaat 5340
   attaatacca atgattaatg aagcagttgg tatattagca gaaggaatag cttcagtaga 5400
   agacatagat aaagctatga aacttggagc taatcaccca atgggaccat tagaattagg 5460
   tgattttata ggtcttgata tatgtcttgc tataatggat gttttatact cagaaactgg 5520
   agattctaag tatagaccac atacattact taagaagtat gtaagagcag gatggcttgg 5580
   aagaaaatca ggaaaaggtt tctacgatta ttcaaaataa gtttacagga tctgcaggga 5640
   ggaggaaatc atggagttga acaacgttat tctggagaaa gaaggcaagg tggcggttgt 5700
   caccattaac cgtccaaagg ccctgaacgc tctgaactcg gataccctga aagagatgga 5760
   ttacgttatt ggcgagattg agaatgacag cgaagtgctg gctgtgattc tgaccggtgc 5820
   gggtgagaag agctttgtcg cgggtgcgga catcagcgag atgaaagaaa tgaacaccat 5880
   cgaaggccgt aagttcggta ttctgggcaa caaggtgttt cgtcgtctgg aactgctgga 5940
   gaaacctgtc attgctgccg tgaacggttt cgcgctgggc ggtggttgcg agatcgctat 6000
   gagctgcgat attcgtatcg catcgtccaa cgcacgcttt ggtcaaccgg aggtcggtct 6060
   gggtatcact ccgggtttcg gcggtacgca acgtctgagc cgcctggttg gcatgggcat 6120
   ggcgaaacag ttgattttca cggcacagaa cattaaggcg gatgaggcgc tgcgtattgg 6180
   tctggtgaat aaggtcgttg agccaagcga actgatgaat accgcgaaag aaattgcgaa 6240
   caagatcgtt agcaatgccc cggtggccgt taagctgtcg aaacaggcaa tcaaccgtgg 6300
   catgcagtgt gacatcgaca ccgccctggc gtttgagagc gaggcgtttg gtgagtgctt 6360
   ctccaccgag gaccaaaagg atgcgatgac cgcgttcatt gagaaacgca agatcgaggg 6420
   tttcaagaat cgttaataga ggaggatagg aggttttcat atgattgtga aaccgatggt 6480
   ccgtaataat atctgtctga atgctcaccc gcagggctgt aaaaaaggcg tggaagatca 6540
   aattgaatat accaaaaaac gtattacggc agaagtgaaa gccggcgcaa aagctccgaa 6600
   aaacgtgctg gttctgggtt gcagcaatgg ctatggtctg gcttctcgca ttaccgcggc 6660
   ctttggctac ggtgcagcta cgatcggcgt tagtttcgaa aaagcaggtt ccgaaaccaa 6720
   atatggcacg ccgggttggt acaacaatct ggcttttgat gaagcggcca aacgtgaagg 6780
   cctgtatagt gtcaccattg atggtgacgc gttctccgat gaaattaaag cacaggtgat 6840
   cgaagaagcg aagaaaaaag gcattaaatt tgacctgatc gtttacagcc tggcatctcc 6900
   ggtccgtacc gatccggaca cgggtatcat gcataaatct gtgctgaaac cgtttggcaa 6960
   aaccttcacg ggtaaaaccg ttgatccgtt cacgggcgaa ctgaaagaaa ttagcgcgga 7020
   accggccaac gatgaagaag cagctgcgac cgtcaaagtg atgggcggtg aagactggga 7080
   acgttggatc aaacagctga gtaaagaagg cctgctggaa gaaggttgca ttaccctggc 7140
   gtattcctac atcggcccgg aagcaaccca agctctgtat cgcaaaggca cgattggtaa 7200
   agcgaaagaa catctggaag cgaccgccca ccgtctgaac aaagaaaatc cgtcaatccg 7260
   cgccttcgtt tcggtcaata aaggtctggt tacccgtgca tcagctgtga ttccggttat 7320
   cccgctgtac ctggcatcgc tgtttaaagt catgaaagaa aaaggcaacc atgaaggttg 7380
   tattgaacag atcacccgcc tgtatgccga acgtctgtac cgcaaagatg gtacgattcc 7440
   ggtggacgaa gaaaatcgta ttcgcatcga tgactgggaa ctggaagaag atgtccaaaa 7500
   agccgtgagc gccctgatgg aaaaagttac cggcgaaaac gcggaatctc tgacggatct 7560
   ggccggttat cgtcacgact ttctggcgag taatggtttt gatgttgaag gcattaacta 7620
   cgaagctgaa gtggaacgct ttgatcgcat ttgatctaga atatcggccg gccacgcgat 7680
   cgctgac 7687
<210> 197
   <211> 7376
   <212> DNA
   <213> Artificial Sequence
<220>
   <223> Description of Artificial Sequence: Synthetic pET28B-ptb-buk polynucleotide
<400> 197
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacc atggcgatta agagttttaa tgaaattatc 5100
   atgaaggtaa agagcaaaga aatgaaaaaa gttgctgttg ctgtagcaca agacgagcca 5160
   gtacttgaag cagtaagaga tgctaagaaa aatggtattg cagatgctat tcttgttgga 5220
   gaccatgacg aaatcgtgtc aatcgcgctt aaaataggaa tggatgtaaa tgattttgaa 5280
   atagtaaacg agcctaacgt taagaaagct gctttaaagg cagtagagct tgtatcaact 5340
   ggaaaagctg atatggtaat gaagggactt gtaaatacag caactttctt aagatctgta 5400
   ttaaacaaag aagttggact tagaacagga aaaactatgt ctcacgttgc agtatttgaa 5460
   actgagaaat ttgatagact attattttta acagatgttg ctttcaatac ttatcctgaa 5520
   ttaaaggaaa aaattgatat agtaaacaat tcagttaagg ttgcacatgc aataggaatt 5580
   gaaaatccaa aggttgctcc aatttgtgca gttgaggtta taaaccctaa aatgccatca 5640
   acacttgatg cagcaatgct ttcaaaaatg agtgacagag gacaaattaa aggttgtgta 5700
   gttgacggac ctttagcact tgatatagct ttatcagaag aagcagcaca tcataaggga 5760
   gtaacaggag aagttgctgg aaaagctgat atcttcttaa tgccaaacat agaaacagga 5820
   aatgtaatgt ataagacttt aacatataca actgattcaa aaaatggagg aatcttagtt 5880
   ggaacttctg caccagttgt tttaacttca agagctgaca gccatgaaac aaaaatgaac 5940
   tctatagcac ttgcagcttt agttgcaggc aataaataaa ttaaagttaa gtggaggaat 6000
   gttaacatgt atagattact aataatcaat cctggctcga cctcaactaa aattggtatt 6060
   tatgacgatg aaaaagagat atttgagaag actttaagac attcagctga agagatagaa 6120
   aaatataaca ctatatttga tcaatttcaa ttcagaaaga atgtaatttt agatgcgtta 6180
   aaagaagcaa acatagaagt aagttcttta aatgctgtag ttggaagagg cggactctta 6240
   aagccaatag taagtggaac ttatgcagta aatcaaaaaa tgcttgaaga ccttaaagta 6300
   ggagttcaag gtcagcatgc gtcaaatctt ggtggaatta ttgcaaatga aatagcaaaa 6360
   gaaataaatg ttccagcata catagttgat ccagttgttg tggatgagct tgatgaagtt 6420
   tcaagaatat caggaatggc tgacattcca agaaaaagta tattccatgc attaaatcaa 6480
   aaagcagttg ctagaagata tgcaaaagaa gttggaaaaa aatacgaaga tcttaattta 6540
   atcgtagtcc acatgggtgg aggtacttca gtaggtactc ataaagatgg tagagtaata 6600
   gaagttaata atacacttga tggagaaggt ccattctcac cagaaagaag tggtggagtt 6660
   ccaataggag atcttgtaag attgtgcttc agcaacaaat atacttatga agaagtaatg 6720
   aaaaagataa acggcaaagg cggagttgtt agttacttaa atactatcga ttttaaggct 6780
   gtagttgata aagctcttga aggagataag aaatgtgcac ttatatatga agctttcaca 6840
   ttccaggtag caaaagagat aggaaaatgt tcaaccgttt taaaaggaaa tgtagatgca 6900
   ataatcttaa caggcggaat tgcgtacaac gagcatgtat gtaatgccat agaggataga 6960
   gtaaaattca tagcacctgt agttagatat ggtggagaag atgaacttct tgcacttgca 7020
   gaaggtggac ttagagtttt aagaggagaa gaaaaagcta aggaatacaa ataataaagt 7080
   cataaataat ataatataac cagtacccat gtttataaaa cttttgccct ggtaccatat 7140
   ggctagcatg actggtggac agcaaatggg tcgggatccg aattcgagct ccgtcgacaa 7200
   gcttgcggcc gcactcgagc accaccacca ccaccactga gatccggctg ctaacaaagc 7260
   ccgaaaggaa gctgagttgg ctgctgccac cgctgagcaa taactagcat aaccccttgg 7320
   ggcctctaaa cgggtcttga ggggtttttt gctgaaagga ggaactatat ccggat 7376
<210> 198
   <211> 8467
   <212> DNA
   <213> Artificial Sequence
<220>
   <223> Description of Artificial Sequence: Synthetic pACYC-T7-nphT07-phaB-T7-ech2-crr polynucleotide
<400> 198
   cgctgacgtc ggtaccctcg agtctggtaa agaaaccgct gctgcgaaat ttgaacgcca       60
   gcacatggac tcgtctacta gcgcagctta attaacctag gctgctgcca ccgctgagca      120
   ataactagca taaccccttg gggcctctaa acgggtcttg aggggttttt tgctgaaacc      180
   tcaggcattt gagaagcaca cggtcacact gcttccggta gtcaataaac cggtaaacca      240
   gcaatagaca taagcggcta tttaacgacc ctgccctgaa ccgacgaccg ggtcgaattt      300
   gctttcgaat ttctgccatt catccgctta ttatcactta ttcaggcgta gcaccaggcg      360
   tttaagggca ccaataactg ccttaaaaaa attacgcccc gccctgccac tcatcgcagt      420
   actgttgtaa ttcattaagc attctgccga catggaagcc atcacagacg gcatgatgaa      480
   cctgaatcgc cagcggcatc agcaccttgt cgccttgcgt ataatatttg cccatagtga      540
   aaacgggggc gaagaagttg tccatattgg ccacgtttaa atcaaaactg gtgaaactca 600
   cccagggatt ggctgagacg aaaaacatat tctcaataaa ccctttaggg aaataggcca 660
   ggttttcacc gtaacacgcc acatcttgcg aatatatgtg tagaaactgc cggaaatcgt 720
   cgtggtattc actccagagc gatgaaaacg tttcagtttg ctcatggaaa acggtgtaac 780
   aagggtgaac actatcccat atcaccagct caccgtcttt cattgccata cggaactccg 840
   gatgagcatt catcaggcgg gcaagaatgt gaataaaggc cggataaaac ttgtgcttat 900
   ttttctttac ggtctttaaa aaggccgtaa tatccagctg aacggtctgg ttataggtac 960
   attgagcaac tgactgaaat gcctcaaaat gttctttacg atgccattgg gatatatcaa 1020
   cggtggtata tccagtgatt tttttctcca ttttagcttc cttagctcct gaaaatctcg 1080
   ataactcaaa aaatacgccc ggtagtgatc ttatttcatt atggtgaaag ttggaacctc 1140
   ttacgtgccg atcaacgtct cattttcgcc aaaagttggc ccagggcttc ccggtatcaa 1200
   cagggacacc aggatttatt tattctgcga agtgatcttc cgtcacaggt atttattcgg 1260
   cgcaaagtgc gtcgggtgat gctgccaact tactgattta gtgtatgatg gtgtttttga 1320
   ggtgctccag tggcttctgt ttctatcagc tgtccctcct gttcagctac tgacggggtg 1380
   gtgcgtaacg gcaaaagcac cgccggacat cagcgctagc ggagtgtata ctggcttact 1440
   atgttggcac tgatgagggt gtcagtgaag tgcttcatgt ggcaggagaa aaaaggctgc 1500
   accggtgcgt cagcagaata tgtgatacag gatatattcc gcttcctcgc tcactgactc 1560
   gctacgctcg gtcgttcgac tgcggcgagc ggaaatggct tacgaacggg gcggagattt 1620
   cctggaagat gccaggaaga tacttaacag ggaagtgaga gggccgcggc aaagccgttt 1680
   ttccataggc tccgcccccc tgacaagcat cacgaaatct gacgctcaaa tcagtggtgg 1740
   cgaaacccga caggactata aagataccag gcgtttcccc tggcggctcc ctcgtgcgct 1800
   ctcctgttcc tgcctttcgg tttaccggtg tcattccgct gttatggccg cgtttgtctc 1860
   attccacgcc tgacactcag ttccgggtag gcagttcgct ccaagctgga ctgtatgcac 1920
   gaaccccccg ttcagtccga ccgctgcgcc ttatccggta actatcgtct tgagtccaac 1980
   ccggaaagac atgcaaaagc accactggca gcagccactg gtaattgatt tagaggagtt 2040
   agtcttgaag tcatgcgccg gttaaggcta aactgaaagg acaagttttg gtgactgcgc 2100
   tcctccaagc cagttacctc ggttcaaaga gttggtagct cagagaacct tcgaaaaacc 2160
   gccctgcaag gcggtttttt cgttttcaga gcaagagatt acgcgcagac caaaacgatc 2220
   tcaagaagat catcttatta atcagataaa atatttctag atttcagtgc aatttatctc 2280
   ttcaaatgta gcacctgaag tcagccccat acgatataag ttgtaattct catgttagtc 2340
   atgccccgcg cccaccggaa ggagctgact gggttgaagg ctctcaaggg catcggtcga 2400
   gatcccggtg cctaatgagt gagctaactt acattaattg cgttgcgctc actgcccgct 2460
   ttccagtcgg gaaacctgtc gtgccagctg cattaatgaa tcggccaacg cgcggggaga 2520
   ggcggtttgc gtattgggcg ccagggtggt ttttcttttc accagtgaga cgggcaacag 2580
   ctgattgccc ttcaccgcct ggccctgaga gagttgcagc aagcggtcca cgctggtttg 2640
   ccccagcagg cgaaaatcct gtttgatggt ggttaacggc gggatataac atgagctgtc 2700
   ttcggtatcg tcgtatccca ctaccgagat gtccgcacca acgcgcagcc cggactcggt 2760
   aatggcgcgc attgcgccca gcgccatctg atcgttggca accagcatcg cagtgggaac 2820
   gatgccctca ttcagcattt gcatggtttg ttgaaaaccg gacatggcac tccagtcgcc 2880
   ttcccgttcc gctatcggct gaatttgatt gcgagtgaga tatttatgcc agccagccag 2940
   acgcagacgc gccgagacag aacttaatgg gcccgctaac agcgcgattt gctggtgacc 3000
   caatgcgacc agatgctcca cgcccagtcg cgtaccgtct tcatgggaga aaataatact 3060
   gttgatgggt gtctggtcag agacatcaag aaataacgcc ggaacattag tgcaggcagc 3120
   ttccacagca atggcatcct ggtcatccag cggatagtta atgatcagcc cactgacgcg 3180
   ttgcgcgaga agattgtgca ccgccgcttt acaggcttcg acgccgcttc gttctaccat 3240
   cgacaccacc acgctggcac ccagttgatc ggcgcgagat ttaatcgccg cgacaatttg 3300
   cgacggcgcg tgcagggcca gactggaggt ggcaacgcca atcagcaacg actgtttgcc 3360
   cgccagttgt tgtgccacgc ggttgggaat gtaattcagc tccgccatcg ccgcttccac 3420
   tttttcccgc gttttcgcag aaacgtggct ggcctggttc accacgcggg aaacggtctg 3480
   ataagagaca ccggcatact ctgcgacatc gtataacgtt actggtttca cattcaccac 3540
   cctgaattga ctctcttccg ggcgctatca tgccataccg cgaaaggttt tgcgccattc 3600
   gatggtgtcc gggatctcga cgctctccct tatgcgactc ctgcattagg aaattaatac 3660
   gactcactat aggggaattg tgagcggata acaattcccc tgtagaaata attttgttta 3720
   actttaataa ggagatatac atgacggatg tccgctttcg tatcattggc acgggcgctt 3780
   acgtcccgga acgcattgtc agcaacgacg aagtcggcgc accggctggt gtggatgacg 3840
   attggattac gcgtaaaacc ggtatccgcc agcgtcgctg ggcggccgac gatcaagcaa 3900
   cgagcgatct ggctaccgca gctggtcgtg cagcactgaa agcagctggc attacgccgg 3960
   aacagctgac cgtcatcgca gtggctacct ctacgccgga ccgtccgcag ccgccgaccg 4020
   cggcctatgt tcaacatcac ctgggtgcaa ccggcacggc agcttttgat gttaacgctg 4080
   tgtgcagcgg tacggttttc gcgctgagct ctgttgccgg caccctggtc tatcgtggcg 4140
   gttacgcact ggtcattggt gctgatctgt actcacgtat cctgaatccg gcggaccgca 4200
   aaaccgtggt tctgttcggc gatggtgcgg gcgcgatggt gctgggcccg accagcaccg 4260
   gcaccggtcc gattgtgcgt cgcgttgcac tgcatacgtt tggcggtctg accgatctga 4320
   tccgtgttcc ggccggcggt tcccgccagc cgctggacac cgatggtctg gacgcaggcc 4380
   tgcaatattt tgcgatggat ggccgcgaag tccgtcgctt cgtgaccgaa catctgccgc 4440
   agctgattaa aggttttctg cacgaagcgg gcgtggatgc ggcggatatt agccatttcg 4500
   tgccgcacca agccaacggt gtgatgctgg acgaagtttt tggcgaactg catctgccgc 4560
   gtgcaaccat gcaccgtacg gtggaaacct acggtaatac gggcgcagct agtattccga 4620
   tcacgatgga tgcggccgtt cgtgcaggtt ccttccgtcc gggcgaactg gttctgctgg 4680
   cgggctttgg cggcggtatg gcggcttcgt ttgctctgat tgaatggtga cctaatcgag 4740
   gcaaacgagg cgttcgccgc tcaagcctgt gccgtgaata aggatatggg ttgggacccg 4800
   agcatcgtta atgtgaatgg tggtgcaatc gcgatcggcc atccgatcgg cgcaagcggt 4860
   gcacgcgttc tgaacacgct gctgtttgaa atgcagcgcc gcaatgcgaa gaaaggtttg 4920
   gctaccctgt gcattggcgg tggtatgggt gtcgccatgt gcctggagcg tccgtagtaa 4980
   cacgtggagt taggaggaaa aatcatgagc aaagtggccc tggtgaccgg tggttcccgt 5040
   ggcatcggtg ccgcgatctc cgtggccctg aagaatgcag gctataccgt cgcggccaac 5100
   tacgcgggta atgacgaggc ggcacgtaag tttaccgaag agacgggcat caagacgtac 5160
   aaatggagcg tcgccgatta cgacgcgtgc gctgcgggca tcgcacaagt ggaagcagaa 5220
   ctgggtccgg ttgcagtcct ggtcaacaac gcgggtatta cccgtgatag catgtttcat 5280
   aagatgactc gtgaccaatg gaaagaggtc atcgacacga acctgagcgg cttgttcaac 5340
   atgactcatc cggtttggag cggtatgcgc gaccgcaagt ttggtcgtat catcaatatc 5400
   agcagcatta acggtcagaa gggccaggca ggtcaagcca actacagcgc ggcaaaagcc 5460
   ggcgacctgg gcttcacgaa agctctggcc caagagggtg cgcgtgcagg catcaccgtg 5520
   aatgcgatct gtccgggcta tatcgccacc gaaatggtta tggctgtccc ggagaaagtt 5580
   cgcgagagca ttatcgcaca gattcctacc ggccgtctgg gtgagccgga agagattgcg 5640
   cgttgtgtcg tttttctggc gagcgacgat gcgggctttg tcaccggttc taccatcacg 5700
   gcaaacggtg gtcagtactt tgtctaaatc gaaattaata cgactcacta taggggaatt 5760
   gtgagcggat aacaattccc catcttagta tattagttaa gtataagaag gagatatacc 5820
   atggccgatg caagtggctt tgcgggtgtc gtgggccata aactgccgtc tttcagttcc 5880
   tcatataccg aactgcagtg cattatgtac gctctgggtg ttggcgcgag tgtcaaaaac 5940
   ccgaaggacc tgaaatttgt gtatgaaggt tccgccgatt tttcatgtct gccgaccttc 6000
   ggcgttattg tcgcccagaa atctctgatg agtggcggtc tggcagaagt cccgggtctg 6060
   agcatcaatt tcgctaaagt gctgcacggc gaacaatatc tggaactgta caagccgctg 6120
   ccgcgttcgg gcgaactgaa atgcgaagcc gttattgcag acatcctgga taagggtagc 6180
   ggcattgtga tcgttatgga tgtgtattcc tactcaggca aggaactgat ttgttataac 6240
   cagttctcgg ttttcgttgt cggcagcggc ggttttggcg gtaaacgcac ctcggaaaaa 6300
   ctgaaggcag ctgtcgctgt gccgagccgc ccgccggacg cagtgctgcg tgataccacg 6360
   agcctgaacc aggcggccct gtaccgtctg tccggtgact caaatccgct gcatattgat 6420
   ccgtcgtttg ctagcatcgc gggcttcgaa aaaccgattc tgcatggtct gtgcaccttt 6480
   ggcttctctg cccgccacgt gctgcagcaa tttgcagaca atgatgttag tcgctttaag 6540
   gccattaaag tgcgtttcgc aaaaccggtt tatccgggtc agaccctgca aacggaaatg 6600
   tggaaggaag gcaaccgtat ccatttccag accaaagtcc aagaaacggg tgacattgtg 6660
   atcagcaatg cgtacgttga tctggtcccg acctctggtg tgagtgcaca gacgccgtct 6720
   gaaggcggtg ctctgcaaag tgcgctggtt tttggtgaaa ttggccgccg tctgaaagat 6780
   gtcggtcgtg aagtggttaa aaaggtgaac gcggttttcg aatggcacat taccaaaaac 6840
   ggcaatgtgg ccgctaagtg gacgatcgat ctgaaaaatg gtagtggcga agtttatcag 6900
   ggtccggcca aaggctccgc agataccacg attaccatct cagacgaaga ttttatggaa 6960
   gtcgtgctgg gcaagctgaa cccgcagaat gcgtttttca gcggccgtct gaaagcgcgt 7020
   ggcaacatta tgctgtcgca aaaactgcaa atgattctga aagattacgc caaactgtga 7080
   acgcgagagg aggaaaccat gactgtcaaa gacatcctgg atgcaatcca gtccaaagac 7140
   gcaacgagcg cggacttcgc cgccttgcaa ctgccggaaa gctaccgtgc aattaccgtg 7200
   cacaaagacg agactgagat gtttgccggc ctggaaacgc gcgataaaga cccgcgtaaa 7260
   agcattcatc tggatgaggt tccggtgccg gagctgggtc cgggcgaagc tctggttgcc 7320
   gttatggcaa gctctgtgaa ctacaacagc gtgtggacca gcatctttga accggtgtct 7380
   acgttcgctt ttctggagcg ctatggtaag ctgagcccgc tgaccaagcg tcacgatctg 7440
   ccgtaccaca ttatcggtag cgatctggca ggtgtggtgc tgcgtacggg tccgggtgtt 7500
   aatgcctggc agccaggtga cgaggtcgtg gcgcattgtc tgtctgttga gctggagagc 7560
   ccggatggcc acgacgacac catgctggat ccggaacagc gtatctgggg cttcgaaacc 7620
   aatttcggtg gtctggcaga gattgcgctg gtcaaaacga accaattgat gccgaagccg 7680
   aaacatctga cgtgggaaga agcggcagcg ccaggtctgg tcaattctac cgcgtatcgc 7740
   cagctggtga gccgtaatgg cgcagctatg aaacaaggtg acaatgttct gatttggggc 7800
   gctagcggtg gcctgggttc ctatgcgacc caatttgcat tggcaggcgg tgcgaatccg 7860
   atttgcgttg tgagcagccc gcaaaaagcg gaaatctgtc gtagcatggg tgctgaagcg 7920
   attatcgatc gtaatgcaga gggctacaag ttctggaagg acgaacacac tcaagatccg 7980
   aaagagtgga aacgcttcgg caagcgtatc cgcgaattga ccggtggtga ggacattgac 8040
   atcgtctttg aacaccctgg tcgcgaaacg ttcggtgcaa gcgtttacgt cacgcgcaaa 8100
   ggcggcacca tcaccacctg tgcgagcacg agcggctata tgcacgagta cgacaaccgt 8160
   tacttgtgga tgagcctgcg catcatcggt agccattttg cgaactaccg cgaagcatat 8220
   gaagccaatc gtttgattgc gaagggtaag attcacccta ccctgtccaa gacctattct 8280
   ctggaagaaa ccggccaggc cgcgtacgat gtccatcgta acttgcacca gggcaaagtc 8340
   ggcgtgttgt gcctggctcc ggaagagggc ctgggtgtgc gtgacgcgga gatgcgtgca 8400
   cagcatattg atgcgatcaa tcgtttccgc aatgtcaagc ttctcgagcg atcgatcgac 8460
   tgactga 8467
<210> 199
   <211> 915
   <212> DNA
   <213> Artificial Sequence
<220>
   <223> Description of Artificial Sequence: Synthetic ELO1 polynucleotide
<400> 199
   atgttcttca ccccgccgca actgcaaaaa ctggaacagg attggaatgg tctggctgtc 60
   cgtgactgga tgatcgcaaa tgtggatgtg gttctgtata tttcatttct gtacctgggc 120
   tttgtcttca tcggtccgaa actgttcgcc aaactggttg gcacgaaccc ggcggcggcg 180
   gcggcgggtg cacgtagcgc agatggcacc ggttctccga ttgtgcgtcg cagtatggtc 240
   gtgtggaatc tggcgctgag catttttagc atcttcggca cctccacggt taccccggtc 300
   ctgctgcgca acctggctaa taaaggcttt tatggtgcga cgtgcgattt taaagaaacc 360
   gaattttaca ccacgaacgt tggcttttgg atgggtattt tcgccctgag caaaatcccg 420
   gaactggtgg ataccatttt tctggtgctg caaggcaaac aagaactgcc gttcctgcat 480
   tggtatcatc acgtcacggt gctgctgttt tcttggcaca cctattgtgt tggtagctct 540
   gcgtacatct gggtcgccgc aatgaattat tcagtgcatt cggttatgta tctgtacttc 600
   gctctggctg cgctgggtta taagcgtgtg gtccgcccgc tggcgccgta cattacgatt 660
   atccagatcc tgcaaatggt ggttggctgc tatgtgacca tctttgctct gcaagaactg 720
   cacggtgaag gcggtcgtgg ttgtggtgtg agcccggcaa acatgcgcat tcaactggtt 780
   atgtatgcat cctatctgta cctgttttca aaaatgttcg tggcatcgta catccgtccg 840
   ccgaaacgcc cgaccgttgg tggtccgagc agcacggcgg gcgtctcaaa cggttctgtg 900
   gaaaagaaag tgaaa 915
<210> 200
   <211> 816
   <212> DNA
   <213> Artificial Sequence
<220>
   <223> Description of Artificial Sequence: Synthetic ELO2 polynucleotide
<400> 200
   atgtcttttc cgtatgtcac cgactactct ggttttgcta ttcgtaaatg gatgattgat      60
   aatgtggatg ttgcgggttt cctgtgtctg ctgtacctgg gtctggtgtg gaaaggtccg     120
   ggcgtggtta aaagcctgcg tgagaaaaac ctgattaatg caaccctgct gcaaggcgtc     180
   tttatcatgt ggaacctgtt tctgagtacg ttctccgtta ttggtatgat cgtcgtggtt     240
   ccggcggcca ttgcgcatat ctctaacaaa ggtctggtcc cggcactgtg cgaacgtgat     300
   gtgaatatga tttatgactc accggttggc ttttgggttg gtgtcttcgc cctgtcgaaa     360
   atcccggaac tgtttgatac cgtcctgctg gtgctgcaag gcaaacaacc gccgtttctg     420
   cattggtatc atcacaccac ggtgctgatt ttctcatggc agtcgtattg cgaaggcagc     480
   tctaccatct ttgtgttcgt tgcaatgaac ctgacggttc acgctgtcat gtatttttac     540
   ttcgcgatgt gtgcctcagg tttcaaagca attatgcgta ccatcgctcc ggttattacg     600
   atcatgcaga ttctgcaaat gatcgtgggc agcgcggtta ccatgtattc tgcctacgtg     660
   ctgtataatc cgcagccgga tggcccgcaa acctgtgacg ttacgaaagc aagtgctcgc     720
   atgggtgtcg tgatgtatct gtcctatctg tacctgtttg cggcactgtt cgttgaatct     780
   tacctgaaac cgaaaaaacg cacggaaaaa tctaaa                                816
<210> 201
   <211> 906
   <212> DNA
   <213> Artificial Sequence
<220>
   <223> Description of Artificial Sequence: Synthetic ELO3 polynucleotide
<400> 201
   atggacctga tgaacttcgg cggctcgtat gatgcttaca ttaacaactt tcaaggcacg 60
   tttctggcgg aatggatgct ggaccacccg tcggtcccgt atattgcagg cgtgatgtac 120
   ctgatcctgg ttctgtatgt cccgaaatca attatggctt cgcagccgcc gctgaacctg 180
   cgtgcagcaa acatcgtgtg gaacctgttt ctgacgctgt tcagcatgtg cggtgcgtat 240
   tacaccgttc cgtatctggt caaagccttt atgaacccgg aaattgttat ggcagcttct 300
   ggcatcaaac tggatgccaa tacgagcccg attatcaccc attctggttt ctacaccacg 360
   acctgtgcgc tggccgattc tttttatttc aacggcgacg tgggtttttg ggttgcactg 420
   ttcgctctga gtaaaattcc ggaaatgatc gacacggcgt ttctggtttt ccagaaaaag 480
   ccggtcattt ttctgcactg gtatcatcac ctgaccgtga tgctgttttg ctggttcgcg 540
   tatgttcaaa aaattagctc tggcctgtgg tttgccagca tgaattacag tgtgcattcc 600
   atcatgtatc tgtattactt tgtctgcgca tgtggtcacc gtcgcctggt tcgtccgttc 660
   gctccgatta tcacgtttgt gcagattttc caaatggtgg ttggcaccat tgtggtgtgc 720
   tatacgtaca ccgtgaagca tgtgctgggc cgttcgtgta cggttaccga ttttagcctg 780
   cacaccggtc tggtcatgta tgtgagttac ctgctgctgt tttcccaact gttctatcgc 840
   tcatacctgt ccccgcgtga caaagcgagt atcccgcatg ttgctgccga aatcaaaaag 900
   aaagaa 906
<210> 202
   <211> 1044
   <212> DNA
   <213> Artificial Sequence
<220>
   <223> Description of Artificial Sequence: Synthetic Sc_KCR polynucleotide
<400> 202
   atgactttta tgcaacagct tcaagaggct ggggaaagat ttaggtgtat caatggtctt      60
   ttatgggttg tttttgggct tggtgtctta aaatgtacaa cgttatcact tagattttta     120
   gctcttattt ttgatctttt tttactacca gcagtcaatt tcgacaagta tggtgctaaa     180
   actggtaaat actgtgctat cactggtgca agtgacggta ttggtaaaga atttgctaga     240
   caaatggcga aacgtggctt caacttggta ttgatctcga gaacgcaatc taaattggag     300
   gctttacaaa aagaactaga agatcaacat catgtcgttg taaagattct agccattgac     360
   attgcggagg ataaagaatc caactatgaa tccattaagg aattgtgtgc gcagttacca     420
   atcaccgttt tggtcaataa tgttggtcaa tcacactcca ttcctgttcc atttttggaa     480
   acagaagaga aggagcttag aaatattatc actatcaata acactgctac attattaatt     540
   acacaaatca ttgcaccaaa gattgtggaa actgtgaaag ctgaaaacaa gaagtcgggt     600
   actcgtggtt tgatcttaac catgggctca tttggtggtc tgattcccac cccacttttg     660
   gctacataca gtggttcgaa atcattctta caaggttggt ctaactcttt ggctggggaa     720
   ttatctaaag atgctatcga tgttgaatta atcatttcat atttggtcac tagctcgatg     780
   tctaaaatca gaagatcatc tttaatgatc ccaaatccac aacagtttgt aaaatccact     840
   ttaagaagcg ttggcagacg ctgcggctct caagaaagat acgctactat gaccccttac     900
   tgggcgcacg cagtgtatca atttgtaatt acagagacct ttggcgttta ctctaagatt     960
   gttaactcca ttaattattc cttccataaa tctatcagaa ttagagcctt aaaaaaagcc    1020
   gcaagacagg ttaaaaagga atag                                           1044
<210> 203
   <211> 654
   <212> DNA
   <213> Artificial Sequence
<220>
   <223> Description of Artificial Sequence: Synthetic Sc_3HCDh polynucleotide
<400> 203
   atgtcaaaaa aacttgcgtc accattgtcc ttcttacccc tttataattt gctttctgct        60
   gttggttggt cttatttgct ttacttggtc atctccttgt acccaaaagt tggacagcca       120
   gcattcttct accaaactaa aaatgtcgct acccttgttc aatgtggtgc tataatcgag       180
   atcataaact catttttagg agttgtacgt tccccattgc tgaccactgt tgcacaagtg       240
   tcttcaagac tactagttgt cctcggcatc ttccaattgt tgccaaacac aagtggtgtt       300
   caatcagttg tttacatatc attattactg gcatggtcta taactgagat cgtcagatac       360
   ttgtattatt ttttcatgtt ggtattcaag aatggcgcac caaagatctt aattctatta       420
   agatataatt tgttctggat tttgtacccc actggtgttg ccagcgaact acgcattatt       480
   tactgtgctt taaatgcagc tgaatctcag tattctttac tttacaaaag aattttaata       540
   gcggccatgc tcgcttatat cccaggcttc ccaatgctct tcctacacat ggtagcacag       600
   agaaagaaag tcatgaaaag tttaagatcc tctttcggga agaaactaat ttga             654
<210> 204
   <211> 933
   <212> DNA
   <213> Artificial Sequence
<220>
   <223> Description of Artificial Sequence: Synthetic Sc_EnCR polynucleotide
<400> 204
   atgcctatca ccataaaaag ccgctctaaa gggttaaggg acactgaaat tgacttatcc        60
   aaaaagccta ctttagatga tgttttgaaa aaaatctctg ctaataacca caatatcagc       120
   aagtacagga taagattaac ctacaaaaag gaatctaaac aagttccggt tatttcagaa       180
   tcgttttttc aagaagaggc tgatgactca atggaattct tcatcaaaga tttgggtccc       240
   caaatttcat ggagattagt cttcttttgt gagtatttgg gtccagtctt ggttcactcc       300
   cttttttatt atctatctac cattcccaca gttgttgata gatggcacag tgctagctcc       360
   gactataatc catttttaaa cagggttgca tattttttaa ttttaggaca ttatggaaag       420
   agattatttg aaaccttatt tgttcaccaa ttctctttag ctactatgcc aattttcaac       480
   ctgttcaaaa attgtttcca ttactgggtt ctaagcggtc tcatttcatt cggttacttt       540
   ggctacggct tcccctttgg gaatgctaag ttattcaaat actattcata tttgaaattg      600
   gatgacttga gtacattaat tggtcttttc gtgctttcag aactatggaa cttttattgc       660
   cacattaaat tgcgcctatg gggtgactat caaaagaagc atggtaacgc taagatccgt 720
   gtcccattga atcaaggtat tttcaatctt tttgttgctc ccaactatac ttttgaagtt 780
   tggtcttgga tttggtttac ttttgtgttc aagttcaatt tatttgccgt tttatttttg 840
   actgtttcaa cagctcaaat gtacgcatgg gctcaaaaga aaaacaaaaa gtatcatacc 900
   agaagagcat tcttgattcc atttgtattt tga 933
<210> 205
   <211> 936
   <212> DNA
   <213> Artificial Sequence
<220>
   <223> Description of Artificial Sequence: Synthetic Tb_KCR (codon-optimized) polynucleotide
<400> 205
   atgctgtttg attgtacgct gctgtcgctg ctgggcgcta tggttgcggc tattggtctg 60
   tatcgtgtcg ttcagtttgt ccacctgacc tttttcagca cgggccatga tctgaaacgt 120
   cgctattcta aagccggtga ctgggcagtg gttaccggcg gtacggaagg catcggtcgt 180
   gctgtggcgc tggatctggc aaaccgtggt tttaatgtgt gcgttattag tcgtacccag 240
   tccaaactgg atgaagtcgt ggcggaaatc gaaaaatgtg gcacccgcgg tcacagtatt 300
   gcctttgatt tcgccacggc aggtgaagca gaatataaaa tgctgtttgc taaactggac 360
   tcactggcgg tgggcctgct ggttaacaat gtcggtgtga actataccta cgccaattac 420
   ttcgatgaag cagacgttgt cgatgacctg cgtattatca aagtgaactg cgaagctacc 480
   acgcgtatga ccaaattttt cgcaccgcgt atgaaagcac gtcgcgcagg cggtatcgtc 540
   ctgctgggca gcttttctgc tgttacgccg gcaccgctgc tggctaccta tgcgggcacg 600
   aaagcgttta acgtgagctt cggtgatgcg ctgttttacg aactgaaaaa attcggtgtt 660
   gacgtcctgg tggttacccc gaatctggtc gtgtcacgta tgacccaagg tgcatcgacg 720
   cgcgcaccga aagaaacctt cctgacggtt ggtgcggcgg cgatggcgcg tcagaccctg 780
   aaccaactgg gcgttgtcaa tcgtacggcg ggtcatcgca atcacattat cattgaagct 840
   attgcacgtc tgctgccgga atccctgcgt ggtgaaaaaa tgctggcgat gcacgaatca 900
   atcaaaaaac gcgcagaacg caaagcgaaa caatga 936
<210> 206
   <211> 801
   <212> DNA
   <213> Artificial Sequence
<220>
   <223> Description of Artificial Sequence: Synthetic Tb_3HCDh (codon-optimized) polynucleotide
<400> 206
   atggtcctgt tcttcttctt ttgtctgtcc tcgggtcgca tggttgtgaa actgaaactg        60
   tccctgtatt cctgtagcac gacgttcctg accctggtgc atccgctgct gatgctgtca       120
   aaagtctatc tgctggcata caacggtgtg ctgctggcag gttggtcgac gattctgatg       180
   aaaatcgttc agcacctgtc aaccggcggt cgttttgcag atgtctattc gctgatcgct       240
   ccgctgctgg ttgtgagcca atctgcggcc gttctggaag tcctgcatgc gctgttcggt       300
   ctggtgcgta gcccggttgg caccacgctg ctgcaggttc tgtctcgcct gctggtcctg       360
   tacggtgccc tggaaattgg tccgacggca gctcgtatga gtccgtttgc aacccaaatg       420
   atcgtggcgt ggtccctggc cgaagttatt cgctatacgt tctacgcaag caatctggct       480
   ggcgtcaaac tgaaaccggt gacctggctg cgctattctg cgtttacggt gctgtacccg       540
   atgggcatta ccggtgaaat cgcgtgcttc atttcagccc tgccgtatat ccgtgagaaa       600
   aaaccgtgga cggttgaact gccgaaccgc ctgaatttta gtttctcctg gtattacacc       660
   gtgctgctgc tgctggcagt cgtgtatccg gctggttcct acgttatgta tacctacatg       720
   ctgcagcaac gtcgcaaagc cctgaaagcg acggacgatg cgagccaaac ggatgttagc       780
   aataagaaaa aagcctcata a                                                 801
<210> 207
   <211> 897
   <212> DNA
   <213> Artificial Sequence
<220>
   <223> Description of Artificial Sequence: Synthetic Tb_EnCR (codon-optimized) polynucleotide
<400> 207
   atgaaagtga ccgtgattag tggctcgtcc agcgaagtgg ttgaactgcc gtccaatgcg 60
   ggcctgaccg acctgaaaaa agtgtataaa ccgcgtgttg atattcatcg caaatctttt 120
   aaaatcctgc gtagtggcgg tgataaaaac gacaaatcgg catatattac cctggatgcc 180
   aaacgcgcac tgacggaaca gggcgtcaaa gatggtagcg aagtggttta caaagacctg 240
   ggcccgcaag tcggttatcg taccgtgttt gtcgtggaat acgccggccc gctggcaatc 300
   atgctggctt atgcggcccg cccgagcttc atttacggta gctctatcgt gaaagaatat 360
   tgctacacgc agaaactgta tattgcgctg ttttgtgccc acttcatcaa acgtgaactg 420
   gaaacctttt tcgtccataa attttctcac ccgacgatgc cgcgtcgcaa cattatcaaa 480
   aattgcgtgt attactggac cttcgcactg ggcattggtt acgctctgtg tagtccgtat 540
   tacaccgaac cggcatcccc gacgctggtg aacgcatcag ctgtggcgat ggttatcttt 600
   gaactgctga atttcgcggt tcatgtccag ctgagcggca tgcgtaaagg tgatggtgac 660
   gcaacccgtc cggtgccgaa aggtattctg tttagtctgg tttcctgccc gaactacctg 720
   tttgaaatcc tgtcttgggt tgcattctca ctgggcacct cgatgctgac gagctggggc 780
   tttaccttcg ctggtctggt tcaaatggct gaatgggcgg tcaaaaaaca caaaaattac 840
   attaaaaccg acccgagcgt tcgtaacaaa aaagcgatgc tgccgttcct gctgtga 897
<210> 208
   <211> 552
   <212> DNA
   <213> Artificial Sequence
<220>
   <223> Description of Artificial Sequence: Synthetic tesA' polynucleotide
<400> 208
   atggcagcgg acacgttatt gattctgggt gatagcctga gcgccgggta tcgaatgtct 60
   gccagcgcgg cctggcctgc cttgttgaat gataagtggc agagtaaaac gtcggtagtt 120
   aatgccagca tcagcggcga cacctcgcaa caaggactgg cgcgccttcc ggctctgctg 180
   aaacagcatc agccgcgttg ggtgctggtt gaactgggcg gcaatgacgg tttgcgtggt 240
   tttcagccac agcaaaccga gcaaacgctg cgccagattt tgcaggatgt caaagccgcc 300
   aacgctgaac cattgttaat gcaaatacgt ctgcctgcaa actatggtcg ccgttataat 360
   gaagccttta gcgccattta ccccaaactc gccaaagagt ttgatgttcc gctgctgccc 420
   ttttttatgg aagaggtcta cctcaagcca caatggatgc aggatgacgg tattcatccc 480
   aaccgcgacg cccagccgtt tattgccgac tggatggcga agcagttgca gcctttagta 540
   aatcatgact ca 552
<210> 209
   <211> 861
   <212> DNA
   <213> Artificial Sequence
<220>
   <223> Description of Artificial Sequence: Synthetic tesB polynucleotide
<400> 209
   atgagtcagg cgctaaaaaa tttactgaca ttgttaaatc tggaaaaaat tgaggaagga 60
   ctctttcgcg gccagagtga agatttaggt ttacgccagg tgtttggcgg ccaggtcgtg 120
   ggtcaggcct tgtatgctgc aaaagagacc gtccctgaag agcggctggt acattcgttt 180
   cacagctact ttcttcgccc tggcgatagt aagaagccga ttatttatga tgtcgaaacg 240
   ctgcgtgacg gtaacagctt cagcgcccgc cgggttgctg ctattcaaaa cggcaaaccg 300
   attttttata tgactgcctc tttccaggca ccagaagcgg gtttcgaaca tcaaaaaaca 360
   atgccgtccg cgccagcgcc tgatggcctc ccttcggaaa cgcaaatcgc ccaatcgctg 420
   gcgcacctgc tgccgccagt gctgaaagat aaattcatct gcgatcgtcc gctggaagtc 480
   cgtccggtgg agtttcataa cccactgaaa ggtcacgtcg cagaaccaca tcgtcaggtg 540
   tggatccgcg caaatggtag cgtgccggat gacctgcgcg ttcatcagta tctgctcggt 600
   tacgcttctg atcttaactt cctgccggta gctctacagc cgcacggcat cggttttctc 660
   gaaccgggga ttcagattgc caccattgac cattccatgt ggttccatcg cccgtttaat 720
   ttgaatgaat ggctgctgta tagcgtggag agcacctcgg cgtccagcgc acgtggcttt 780
   gtgcgcggtg agttttatac ccaagacggc gtactggttg cctcgaccgt tcaggaaggg 840
   gtgatgcgta atcacaatta a 861
<210> 210
   <211> 156
   <212> DNA
   <213> Artificial Sequence
<220>
   <223> Description of Artificial Sequence: Synthetic polynucleotide
<400> 210
   gtgcgtaatt gtgctgatct cttatatagc tgctctcatt atctctctac cctgaagtga 60
   ctctctcacc tgtaaaaata atatctcaca ggcttaatag tttcttaata caaagcctgt 120
   aaaacgtcag gataacttct gtgtaggagg ataatc 156
<210> 211
   <211> 177
   <212> DNA
   <213> Artificial Sequence
<220>
   <223> Description of Artificial Sequence: Synthetic polynucleotide
<400> 211
   cacgtgcgta attgtgctga tctcttatat agctgctctc attatctctc taccctgaag        60
   tgactctctc acctgtaaaa ataatatctc acaggcttaa tagtttctta atacaaagcc       120
   tgtaaaacgt caggataact tctgtgtagg aggataatcc atggaattcc gcacgtg          177
<210> 212
   <211> 8252
   <212> DNA
   <213> Artificial Sequence
<220>
   <223> Description of Artificial Sequence: Synthetic pKK223-mcr polynucleotide
<220>
   <221> modified base
   <222> (1)..(1)
   <223> Any nucleotide
<400> 212
   ngaattcgct agcaggagct aaggaagcta aaatgtccgg tacgggtcgt ttggctggta 60
   aaattgcatt gatcaccggt ggtgctggta acattggttc cgagctgacc cgccgttttc 120
   tggccgaggg tgcgacggtt attatcagcg gccgtaaccg tgcgaagctg accgcgctgg 180
   ccgagcgcat gcaagccgag gccggcgtgc cggccaagcg cattgatttg gaggtgatgg 240
   atggttccga ccctgtggct gtccgtgccg gtatcgaggc aatcgtcgct cgccacggtc 300
   agattgacat tctggttaac aacgcgggct ccgccggtgc ccaacgtcgc ttggcggaaa 360
   ttccgctgac ggaggcagaa ttgggtccgg gtgcggagga gactttgcac gcttcgatcg 420
   cgaatctgtt gggcatgggt tggcacctga tgcgtattgc ggctccgcac atgccagttg 480
   gctccgcagt tatcaacgtt tcgactattt tctcgcgcgc agagtactat ggtcgcattc 540
   cgtacgttac cccgaaggca gcgctgaacg ctttgtccca gctggctgcc cgcgagctgg 600
   gcgctcgtgg catccgcgtt aacactattt tcccaggtcc tattgagtcc gaccgcatcc 660
   gtaccgtgtt tcaacgtatg gatcaactga agggtcgccc ggagggcgac accgcccatc 720
   actttttgaa caccatgcgc ctgtgccgcg caaacgacca aggcgctttg gaacgccgct 780
   ttccgtccgt tggcgatgtt gctgatgcgg ctgtgtttct ggcttctgct gagagcgcgg 840
   cactgtcggg tgagacgatt gaggtcaccc acggtatgga actgccggcg tgtagcgaaa 900
   cctccttgtt ggcgcgtacc gatctgcgta ccatcgacgc gagcggtcgc actaccctga 960
   tttgcgctgg cgatcaaatt gaagaagtta tggccctgac gggcatgctg cgtacgtgcg 1020
   gtagcgaagt gattatcggc ttccgttctg cggctgccct ggcgcaattt gagcaggcag 1080
   tgaatgaatc tcgccgtctg gcaggtgcgg atttcacccc gccgatcgct ttgccgttgg 1140
   acccacgtga cccggccacc attgatgcgg ttttcgattg gggcgcaggc gagaatacgg 1200
   gtggcatcca tgcggcggtc attctgccgg caacctccca cgaaccggct ccgtgcgtga 1260
   ttgaagtcga tgacgaacgc gtcctgaatt tcctggccga tgaaattacc ggcaccatcg 1320
   ttattgcgag ccgtttggcg cgctattggc aatcccaacg cctgaccccg ggtgcccgtg 1380
   cccgcggtcc gcgtgttatc tttctgagca acggtgccga tcaaaatggt aatgtttacg 1440
   gtcgtattca atctgcggcg atcggtcaat tgattcgcgt ttggcgtcac gaggcggagt 1500
   tggactatca acgtgcatcc gccgcaggcg atcacgttct gccgccggtt tgggcgaacc 1560
   agattgtccg tttcgctaac cgctccctgg aaggtctgga gttcgcgtgc gcgtggaccg 1620
   cacagctgct gcacagccaa cgtcatatta acgaaattac gctgaacatt ccagccaata 1680
   ttagcgcgac cacgggcgca cgttccgcca gcgtcggctg ggccgagtcc ttgattggtc 1740
   tgcacctggg caaggtggct ctgattaccg gtggttcggc gggcatcggt ggtcaaatcg 1800
   gtcgtctgct ggccttgtct ggcgcgcgtg tgatgctggc cgctcgcgat cgccataaat 1860
   tggaacagat gcaagccatg attcaaagcg aattggcgga ggttggttat accgatgtgg 1920
   aggaccgtgt gcacatcgct ccgggttgcg atgtgagcag cgaggcgcag ctggcagatc 1980
   tggtggaacg tacgctgtcc gcattcggta ccgtggatta tttgattaat aacgccggta 2040
   ttgcgggcgt ggaggagatg gtgatcgaca tgccggtgga aggctggcgt cacaccctgt 2100
   ttgccaacct gatttcgaat tattcgctga tgcgcaagtt ggcgccgctg atgaagaagc 2160
   aaggtagcgg ttacatcctg aacgtttctt cctattttgg cggtgagaag gacgcggcga 2220
   ttccttatcc gaaccgcgcc gactacgccg tctccaaggc tggccaacgc gcgatggcgg 2280
   aagtgttcgc tcgtttcctg ggtccagaga ttcagatcaa tgctattgcc ccaggtccgg 2340
   ttgaaggcga ccgcctgcgt ggtaccggtg agcgtccggg cctgtttgct cgtcgcgccc 2400
   gtctgatctt ggagaataaa cgcctgaacg aattgcacgc ggctttgatt gctgcggccc 2460
   gcaccgatga gcgctcgatg cacgagttgg ttgaattgtt gctgccgaac gacgtggccg 2520
   cgttggagca gaacccagcg gcccctaccg cgctgcgtga gctggcacgc cgcttccgta 2580
   gcgaaggtga tccggcggca agctcctcgt ccgccttgct gaatcgctcc atcgctgcca 2640
   agctgttggc tcgcttgcat aacggtggct atgtgctgcc ggcggatatt tttgcaaatc 2700
   tgcctaatcc gccggacccg ttctttaccc gtgcgcaaat tgaccgcgaa gctcgcaagg 2760
   tgcgtgatgg tattatgggt atgctgtatc tgcagcgtat gccaaccgag tttgacgtcg 2820
   ctatggcaac cgtgtactat ctggccgatc gtaacgtgag cggcgaaact ttccatccgt 2880
   ctggtggttt gcgctacgag cgtaccccga ccggtggcga gctgttcggc ctgccatcgc 2940
   cggaacgtct ggcggagctg gttggtagca cggtgtacct gatcggtgaa cacctgaccg 3000
   agcacctgaa cctgctggct cgtgcctatt tggagcgcta cggtgcccgt caagtggtga 3060
   tgattgttga gacggaaacc ggtgcggaaa ccatgcgtcg tctgttgcat gatcacgtcg 3120
   aggcaggtcg cctgatgact attgtggcag gtgatcagat tgaggcagcg attgaccaag 3180
   cgatcacgcg ctatggccgt ccgggtccgg tggtgtgcac tccattccgt ccactgccaa 3240
   ccgttccgct ggtcggtcgt aaagactccg attggagcac cgttttgagc gaggcggaat 3300
   ttgcggaact gtgtgagcat cagctgaccc accatttccg tgttgctcgt aagatcgcct 3360
   tgtcggatgg cgcgtcgctg gcgttggtta ccccggaaac gactgcgact agcaccacgg 3420
   agcaatttgc tctggcgaac ttcatcaaga ccaccctgca cgcgttcacc gcgaccatcg 3480
   gtgttgagtc ggagcgcacc gcgcaacgta ttctgattaa ccaggttgat ctgacgcgcc 3540
   gcgcccgtgc ggaagagccg cgtgacccgc acgagcgtca gcaggaattg gaacgcttca 3600
   ttgaagccgt tctgctggtt accgctccgc tgcctcctga ggcagacacg cgctacgcag 3660
   gccgtattca ccgcggtcgt gcgattaccg tctaatagaa gcttggctgt tttggcggat 3720
   gagagaagat tttcagcctg atacagatta aatcagaacg cagaagcggt ctgataaaac 3780
   agaatttgcc tggcggcagt agcgcggtgg tcccacctga ccccatgccg aactcagaag 3840
   tgaaacgccg tagcgccgat ggtagtgtgg ggtctcccca tgcgagagta gggaactgcc 3900
   aggcatcaaa taaaacgaaa ggctcagtcg aaagactggg cctttcgttt tatctgttgt 3960
   ttgtcggtga acgctctcct gagtaggaca aatccgccgg gagcggattt gaacgttgcg 4020
   aagcaacggc ccggagggtg gcgggcagga cgcccgccat aaactgccag gcatcaaatt 4080
   aagcagaagg ccatcctgac ggatggcctt tttgcgtttc tacaaactct tttgtttatt 4140
   tttctaaata cattcaaata tgtatccgct catgagacaa taaccctgat aaatgcttca 4200
   ataatattga aaaaggaaga gtatgagtat tcaacatttc cgtgtcgccc ttattccctt 4260
   ttttgcggca ttttgccttc ctgtttttgc tcacccagaa acgctggtga aagtaaaaga 4320
   tgctgaagat cagttgggtg cacgagtggg ttacatcgaa ctggatctca acagcggtaa 4380
   gatccttgag agttttcgcc ccgaagaacg ttttccaatg atgagcactt ttaaagttct 4440
   gctatgtggc gcggtattat cccgtgttga cgccgggcaa gagcaactcg gtcgccgcat 4500
   acactattct cagaatgact tggttgagta ctcaccagtc acagaaaagc atcttacgga 4560
   tggcatgaca gtaagagaat tatgcagtgc tgccataacc atgagtgata acactgcggc 4620
   caacttactt ctgacaacga tcggaggacc gaaggagcta accgcttttt tgcacaacat 4680
   gggggatcat gtaactcgcc ttgatcgttg ggaaccggag ctgaatgaag ccataccaaa 4740
   cgacgagcgt gacaccacga tgctgtagca atggcaacaa cgttgcgcaa actattaact 4800
   ggcgaactac ttactctagc ttcccggcaa caattaatag actggatgga ggcggataaa 4860
   gttgcaggac cacttctgcg ctcggccctt ccggctggct ggtttattgc tgataaatct 4920
   ggagccggtg agcgtgggtc tcgcggtatc attgcagcac tggggccaga tggtaagccc 4980
   tcccgtatcg tagttatcta cacgacgggg agtcaggcaa ctatggatga acgaaataga 5040
   cagatcgctg agataggtgc ctcactgatt aagcattggt aactgtcaga ccaagtttac 5100
   tcatatatac tttagattga tttaaaactt catttttaat ttaaaaggat ctaggtgaag 5160
   atcctttttg ataatctcat gaccaaaatc ccttaacgtg agttttcgtt ccactgagcg 5220
   tcagaccccg tagaaaagat caaaggatct tcttgagatc ctttttttct gcgcgtaatc 5280
   tgctgcttgc aaacaaaaaa accaccgcta ccagcggtgg tttgtttgcc ggatcaagag 5340
   ctaccaactc tttttccgaa ggtaactggc ttcagcagag cgcagatacc aaatactgtc 5400
   cttctagtgt agccgtagtt aggccaccac ttcaagaact ctgtagcacc gcctacatac 5460
   ctcgctctgc taatcctgtt accagtggct gctgccagtg gcgataagtc gtgtcttacc 5520
   gggttggact caagacgata gttaccggat aaggcgcagc ggtcgggctg aacggggggt 5580
   tcgtgcacac agcccagctt ggagcgaacg acctacaccg aactgagata cctacagcgt 5640
   gagcattgag aaagcgccac gcttcccgaa gggagaaagg cggacaggta tccggtaagc 5700
   ggcagggtcg gaacaggaga gcgcacgagg gagcttccag ggggaaacgc ctggtatctt 5760
   tatagtcctg tcgggtttcg ccacctctga cttgagcgtc gatttttgtg atgctcgtca 5820
   ggggggcgga gcctatggaa aaacgccagc aacgcggcct ttttacggtt cctggccttt 5880
   tgctggcctt ttgctcacat gttctttcct gcgttatccc ctgattctgt ggataaccgt 5940
   attaccgcct ttgagtgagc tgataccgct cgccgcagcc gaacgaccga gcgcagcgag 6000
   tcagtgagcg aggaagcgga agagcgcctg atgcggtatt ttctccttac gcatctgtgc 6060
   ggtatttcac accgcatatg gtgcactctc agtacaatct gctctgatgc cgcatagtta 6120
   agccagtata cactccgcta tcgctacgtg actgggtcat ggctgcgccc cgacacccgc 6180
   caacacccgc tgacgcgccc tgacgggctt gtctgctccc ggcatccgct tacagacaag 6240
   ctgtgaccgt ctccgggagc tgcatgtgtc agaggttttc accgtcatca ccgaaacgcg 6300
   cgaggcagct gcggtaaagc tcatcagcgt ggtcgtgaag cgattcacag atgtctgcct 6360
   gttcatccgc gtccagctcg ttgagtttct ccagaagcgt taatgtctgg cttctgataa 6420
   agcgggccat gttaagggcg gttttttcct gtttggtcac tgatgcctcc gtgtaagggg 6480
   gatttctgtt catgggggta atgataccga tgaaacgaga gaggatgctc acgatacggg 6540
   ttactgatga tgaacatgcc cggttactgg aacgttgtga gggtaaacaa ctggcggtat 6600
   ggatgcggcg ggaccagaga aaaatcactc agggtcaatg ccagcgcttc gttaatacag 6660
   atgtaggtgt tccacagggt agccagcagc atcctgcgat gcagatccgg aacataatgg 6720
   tgcagggcgc tgacttccgc gtttccagac tttacgaaac acggaaaccg aagaccattc 6780
   atgttgttgc tcaggtcgca gacgttttgc agcagcagtc gcttcacgtt cgctcgcgta 6840
   tcggtgattc attctgctaa ccagtaaggc aaccccgcca gcctagccgg gtcctcaacg 6900
   acaggagcac gatcatgcgc acccgtggcc aggacccaac gctgcccgag atgcgccgcg 6960
   tgcggctgct ggagatggcg gacgcgatgg atatgttctg ccaagggttg gtttgcgcat 7020
   tcacagttct ccgcaagaat tgattggctc caattcttgg agtggtgaat ccgttagcga 7080
   ggtgccgccg gcttccattc aggtcgaggt ggcccggctc catgcaccgc gacgcaacgc 7140
   ggggaggcag acaaggtata gggcggcgcc tacaatccat gccaacccgt tccatgtgct 7200
   cgccgaggcg gcataaatcg ccgtgacgat cagcggtcca gtgatcgaag ttaggctggt 7260
   aagagccgcg agcgatcctt gaagctgtcc ctgatggtcg tcatctacct gcctggacag 7320
   catggcctgc aacgcgggca tcccgatgcc gccggaagcg agaagaatca taatggggaa 7380
   ggccatccag cctcgcgtcg cgaacgccag caagacgtag cccagcgcgt cggccgccat 7440
   gccggcgata atggcctgct tctcgccgaa acgtttggtg gcgggaccag tgacgaaggc 7500
   ttgagcgagg gcgtgcaaga ttccgaatac cgcaagcgac aggccgatca tcgtcgcgct 7560
   ccagcgaaag cggtcctcgc cgaaaatgac ccagagcgct gccggcacct gtcctacgag 7620
   ttgcatgata aagaagacag tcataagtgc ggcgacgata gtcatgcccc gcgcccaccg 7680
   gaaggagctg actgggttga aggctctcaa gggcatcggt cgacgctctc ccttatgcga 7740
   ctcctgcatt aggaagcagc ccagtagtag gttgaggccg ttgagcaccg ccgccgcaag 7800
   gaatggtgca tgcaaggaga tggcgcccaa cagtcccccg gccacggggc ctgccaccat 7860
   acccacgccg aaacaagcgc tcatgagccc gaagtggcga gcccgatctt ccccatcggt 7920
   gatgtcggcg atataggcgc cagcaaccgc acctgtggcg ccggtgatgc cggccacgat 7980
   gcgtccggcg tagaggatcc gggcttatcg actgcacggt gcaccaatgc ttctggcgtc 8040
   aggcagccat cggaagctgt ggtatggctg tgcaggtcgt aaatcactgc ataattcgtg 8100
   tcgctcaagg cgcactcccg ttctggataa tgttttttgc gccgacatca taacggttct 8160
   ggcaaatatt ctgaaatgag ctgttgacaa ttaatcatcg gctcgtataa tgtgtggaat 8220
   tgtgagcgga taacaatttc acacaggaaa ca 8252
<210> 213
   <211> 1440
   <212> DNA
   <213> Artificial Sequence
<220>
   <223> Description of Artificial Sequence: Synthetic pSUCR polynucleotide
<400> 213
   taagctttta ccaatgctta atcagtgagg cacctatctc agcgatctgt ctatttcgtt      60
   catccatagt tgcctgactc cccgtcgtgt agataactac gatacgggag ggcttaccat     120
   ctggccccag cgctgcgatg ataccgcgag aaccacgctc accggctccg gatttatcag     180
   caataaacca gccagccgga agggccgagc gcagaagtgg tcctgcaact ttatccgcct     240
   ccatccagtc tattaattgt tgccgggaag ctagagtaag tagttcgcca gttaatagtt     300
   tgcgcaacgt tgttgccatc gctacaggca tcgtggtgtc acgctcgtcg tttggtatgg     360
   cttcattcag ctccggttcc caacgatcaa ggcgagttac atgatccccc atgttgtgca     420
   aaaaagcggt tagctccttc ggtcctccga tcgttgtcag aagtaagttg gccgcagtgt     480
   tatcactcat ggttatggca gcactgcata attctcttac tgtcatgcca tccgtaagat     540
   gcttttctgt gactggtgag tactcaacca agtcattctg agaatagtgt atgcggcgac     600
   cgagttgctc ttgcccggcg tcaatacggg ataataccgc gccacatagc agaactttaa     660
   aagtgctcat cattggaaaa cgttcttcgg ggcgaaaact ctcaaggatc ttaccgctgt     720
   tgagatccag ttcgatgtaa cccactcgtg cacccaactg atcttcagca tcttttactt     780
   tcaccagcgt ttctgggtga gcaaaaacag gaaggcaaaa tgccgcaaaa aagggaataa     840
   gggcgacacg gaaatgttga atactcatat tcttcctttt tcaatattat tgaagcattt     900
   atcagggtta ttgtctcatg agcggataca tatttgaatg tatttagaaa aataaacaaa     960
   taggggtcag tgttacaacc aattaaccaa ttctgaacat tatcgcgagc ccatttatac    1020
   ctgaatatgg ctcataacac cccttgtttg cctggcggca gtagcgcggt ggtcccacct    1080
   gaccccatgc cgaactcaga agtgaaacgc cgtagcgccg atggtagtgt ggggactccc    1140
   catgcgagag tagggaactg ccaggcatca aataaaacga aaggctcagt cgaaagactg    1200
   ggcctttcgc ccgggctaat tatggggtgt cgcccttatt cgactctata gggaagttcc    1260
   tattctctag aaagtatagg aacttctgaa ggggggtcac ctcctaacta cttaaaattg    1320
   ctatcattcg ttattgttat ctagttgtgc aaaacattga catccctatc agtgatagat    1380
   ataatgagca catgcccacc ggcagctgcc ggtgggccgt attttggatg ataacaggag    1440
<210> 214
   <211> 90
   <212> DNA
   <213> Artificial Sequence
<220>
   <223> Description of Artificial Sequence: Synthetic HL021 polynucleotide
<400> 214
   atttctgcct tttattcctt ttacacttgt ttttatgaag cccttcacag aattgtcctt        60
   tcacgaaaac attgacatcc ctatcagtga                                         90
<210> 215
   <211> 90
   <212> DNA
   <213> Artificial Sequence
<220>
   <223> Description of Artificial Sequence: Synthetic HL022 polynucleotide
<400> 215
   cactcattaa gactgtaaat aaaccacctg ggtctgcaga tattcatgca agccatgttt        60
   accataagct taacccagtt gccacagtgc                                         90

## Description

### RELATED APPLICATIONS

This application claims priority to U.S. provisional patent applications US 61/466,363, filed on March 22, 2011; US 61/466,433, filed on March 22, 2011; US 61/539,162, filed September 26, 2011; and US 61/539,378, filed September 26, 2011.

### STATEMENT AS TO FEDERALLY SPONSORED RESEARCH

This invention was made with Government support under DE-AR0000088 awarded by the United States Department of Energy. The Government has certain rights in this invention.

### SEQUENCE LISTINGS

The instant application contains a Sequence Listing which has been submitted in ASCII format via EFS-Web. Said ASCII copy, created on March 22, 2012, is named 34246760.txt and is 848 kbytes in size.

### BACKGROUND OF THE INVENTION

There is increased emphasis on renewable production of fuels and industrial chemicals as spikes in petroleum costs occur and as petroleum finite resources are consumed. Notwithstanding advances in the general field, there remains a need to find metabolic solutions to renewable production so as to increase rate, productivity, yield, and overall cost-effectiveness for biosynthesis of various chemical products.

### SUMMARY OF THE INVENTION

The invention is defined in the claims. Disclosed herein is a method for producing a chemical, the method comprising combining a carbon source, a microorganism, and a cell culture to produce the chemical, wherein a) said cell culture comprises an inhibitor of fatty acid synthase and/or the microorganism is genetically modified for reduced enzymatic activity in at least two of the microorganism's fatty acid synthase pathway enzymes, providing for reduced conversion of malonyl-CoA to fatty acids; and b) the microorganism additionally has one or more genetic modifications relating to a metabolic production pathway from malonyl-CoA to the chemical. In various embodiments, the at least two fatty acid synthase pathway enzymes with reduced enzymatic activity are an enoyl-coA reductase and a beta-ketoacyl-ACP synthase. In one example, the enoyl-coA reductase is fabI or a peptide of 80% or more homology to SEQ ID NO: 14. In addition, the beta-ketoacyl-ACP synthase may be selected from the group consisting of fabB, fabF, a peptide of 80% or more homology to SEQ ID NO: 9, and a peptide of 80% or more homology to SEQ ID NO: 8. In various emdodiments, a third fatty acid synthase pathway enzyme is modified for reduced enzymatic activity. For example, the enoyl-coA reductase is fabI or a peptide of 80% or more homology to SEQ ID NO: 14, the beta-ketoacyl-ACP synthase is fabB or a peptide of 80% homology or more to SEQ ID NO: 9, and the third fatty acid synthase pathway enzyme is fabF or a peptide of 80% homology or more to SEQ ID NO: 8.

In various embodiments, the third fatty acid synthase pathway enzyme is a malonyl-coA-ACP transacylase. For example, the malonyl-coA-ACP transacylase is modified fabD or a peptide of 80% or more homology to SEQ ID NO: 7. In various embodiments, the enoyl-coA reductase is fabI or a peptide of 80% or more homology to SEQ ID NO: 14, the beta-ketoacyl-ACP synthase is selected from the group consisting of fabB or a peptide of 80% homology or more to SEQ ID NO: 9, and fabF or a peptide of 80% homology or more to SEQ ID NO: 8, and the malonyl-coA-ACP transacylase is fabD or a peptide of 80% or more homology to SEQ ID NO: 7.

In various emdodiments, the at least two fatty acid synthase pathway enzymes with reduced enzymatic activity are an enoyl-coA reductase and a malonyl-coA-ACP transacylase. For example, the enoyl-coA reductase is fabI or a peptide of 80% or more homology to SEQ ID NO: 14. As an additional example, the malonyl-coA-ACP transacylase is fabD or a peptide of 80% or more homology to SEQ ID NO: 7.

Also disclosed are methods as described above, where the microorganism is E. coli. In any of the embodiments described herein, the method may be performed at above room temperature. Preferably, the method is performed at a temperature between 25°C and 50°C.

Also disclosed are methods as described herein, where the microorganism has one or more genetic modifications to increase levels of pantothenate. As an example, the one or more genetic modifications to increase levels of pantothenate is a modification of panE or a peptide of 80% or more homology to panE.

In various embodiments, the microorganism has one or more genetic modifications to increase pyruvate dehydrogenase activity. For example, the one or more genetic modifications to increase pyruvate dehydrogenase activity is a modification of aceE or a peptide of 80% or more homology to SEQ ID NO: 172.

In various embodiments, the increased pyruvate dehydrogenase activity is resistant to inhibition by elevated NADH levels. In addition, the microorganism may be further genetically modified to have reduced alcohol dehydrogenase activity. As an example, the microorganism is genetically modified to have a modification of adhE or a peptide of 80% or more homology to adhE.

Also disclosed are methods as described herein, wherein the microorganism is further genetically modified to have one or more genetic modifications relating to a metabolic production pathway from malonyl-CoA to the chemical, wherein the pathway uses NADH as a reducing agent and wherein there is a mutation or deletion of an alcohol dehydrogenase gene.

In various embodiments, the microorganism is further genetically modified to have modified malonyl-coA dependant acetoacetyl-coA synthase activity. For example, the modified malonyl-coA dependant acetoacetyl-coA synthase is npth07 or a peptide of 80% or more homology to npth07.

In various embodiments, the microorganism is further genetically modified to have altered elongase activity. For example, the microorganism has one or more modifications in the group consisting of elo1, elo2, and elo3, or a peptide of 80% or more homology to a peptide from SEQ ID NOs: 199-201.

Disclosed herein are methods where acetoacetyl-coA is converted to (S)-3-hydroxybutyryl-coA, (R)-3-hydroxybutyryl-coA, 3-hydroxymethylglutaryl-coA, crotonyl-coA, butyryl-coA, isobutyryl-coA, methacrylyl-coA, or 2-hydroxyisobutyryl-coA. The conversion occurs in one step or the conversion occurs in more than one step. In various methods acetoacetyl-coA is converted to a linear acyl-coA with a chain length from 4-18 carbons.

In various embodiments, chemicals produced according to the methods disclosed herein are selected from the group consisting of butanol, isobutanol, 3-hydroxypropionic acid, methacrylic acid, 2-hydroxyisobutyrate, butyrate, isobutyrate, acetoacetate, polyhydroxybutyrate, (S)-3-hydroxybutyrate, (R)-3-hydroxybutyrate, mevalonate, an isoprenoid, a fatty acid, a fatty aldehyde, a fatty alcohol, a fatty acid ester, phloroglucinol, resorcinol, an alkylresorcinol, tetracycline, erythromycin, avermectin, macrolides, Vancomycin-group antibiotics, and Type II polyketides. Preferably, the chemical is produced at a concentration higher than a concentration of said chemical produced by a wild type microorganism.

Also disclosed herein are methods for producing a C4-C18 fatty acid, said methods comprising combining a carbon source, a microorganism, and a cell culture to produce the C4-C18 fatty acid, wherein a) said cell culture comprises an inhibitor of fatty acid synthase and/or the microorganism is genetically modified for reduced enzymatic activity in at least one of the microorganism's native fatty acid synthase pathway enzymes, providing for reduced conversion of malonyl-CoA to fatty acyl-ACPs; and b) the microorganism additionally has one or more genetic modifications increasing fatty acid production. The C4-C18 fatty acid may be selected from the group consisting of C4, C6, C8, C10, C12, C14, C16 and C18 fatty acids. An additional step comprises isolating a C4-C18 fatty acid from said cell culture. In various embodiments, the microorganism synthesizes fatty acyl-coAs of 4-18 carbon chain length. Preferably, the microorganism expresses at least one heterologous elongase enzyme. The elongase enzyme may be a peptide with a sequence corresponding to the group consisting of elo1, elo2, and elo3, or a peptide of 80% or more homology to a peptide from SEQ ID NOs: 199-201.

Also disclosed herein are genetically modified microorganisms for use according to any of the methods described herein. The microorganisms may be yeast or bacteria. In various embodiments, the microorganism is E. coli.

Microorganisms comprising a sequence selected from the group consisting of SEQ ID NO: 1-215 or a sequence of 80% or more homology to SEQ ID NO: 1-215 are disclosed. Further disclosed are geneically modified microorganisms comprising at least one, two, three, four, five, six, seven, eight, nine, ten, or more sequences selected from group consisting of SEQ ID NO: 1-215 or sequences of 80% or more homology to SEQ ID NO: 1-215. In addition, microorganisms further genetically modified to induce enzyme expression from the yibD gene promoter with a decreased environmental phosphate concentration are disclosed. In various emdobiments, the microorganism is further genetically modified to maintain plasmids with expression of a gapA gene on the plasmid and a deletion of the gapA gene in the chromosome.

In various embodiments, microorganisms as described herein have a genotype with 10, 11, 12, 13, 14, 15, or more features selected from the group consisting of F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabIts (S241F)-zeoR, fabB(ts), Δfabf:frt, coaA*, fabD(ts), and ΔaceBAK:frt.

In various embodiments, microorganisms as described herein have a genotype with 10, 11, 12, 13, 14, 15, or more features selected from the group consisting of F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC::T5-aceEF-lpd(E354K):loxP, ΔaceBAK:frt, lpd(E354K):loxP, ΔaldB:PyibD-T7pol:loxP, ΔadhE:frt, ΔaldA:cscBKA.

In various embodiments, the present disclosure provides for production of 3HP in cell culture with a plamid containing one or more genes as described herein. Accordingly, a mixture of 3HP and cell culture is disclosed, as well as mixtures of 3HP and cellular debris including plasmids or vectors containing such genes and genetic material.

### BRIEF DESCRIPTION OF DRAWINGS

The novel features of the invention are set forth with particularity in the claims. A better understanding of the features and advantages of the present invention will be obtained by reference to the following detailed description that sets forth illustrative embodiments, in which the principles of the invention are utilized, and the accompanying drawings of which
FIG. 1 Enzymatic conversion steps involved in production of malonyl-CoA.
FIG. 2A Metabolic pathways for production and utilization of malonyl-CoA.
FIGs. 2B A fatty acid biosynthesis initiation pathway.
FIG. 2C Two fatty acid biosynthesis initiation pathways.
FIG. 2D Superpathyway of fatty acid biosynthesis initiation (E. coli).
FIG. 3 Chemicals into which 3-HP may be converted for further commercial use.
FIGs. 4A-D Pathway involving production of coenzyme A.
FIG. 5 Chemical products produced from malonyl-CoA.
FIG. 6 Pathways toward production of fatty acid methyl esters.

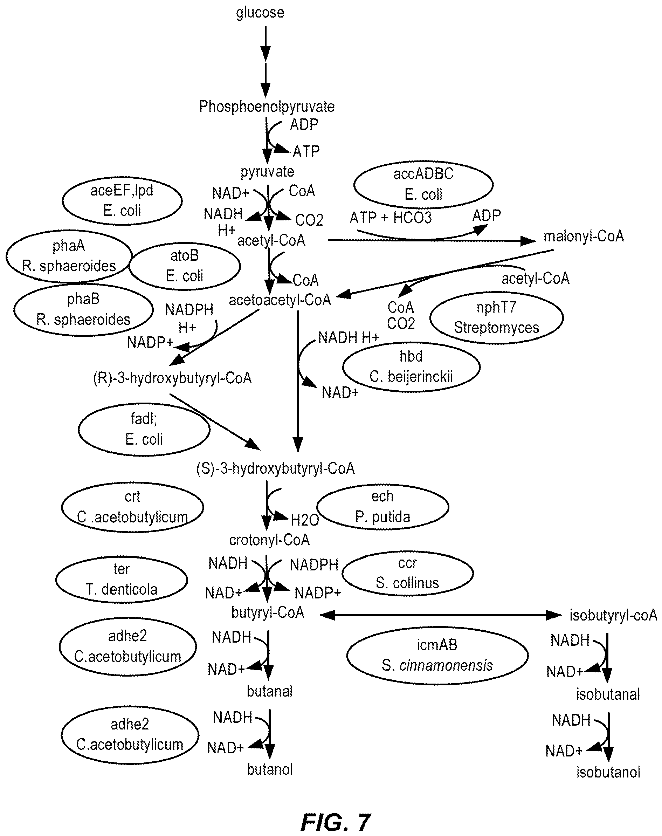

FIG. 7 Pathways toward production of butanol and isobutanol.

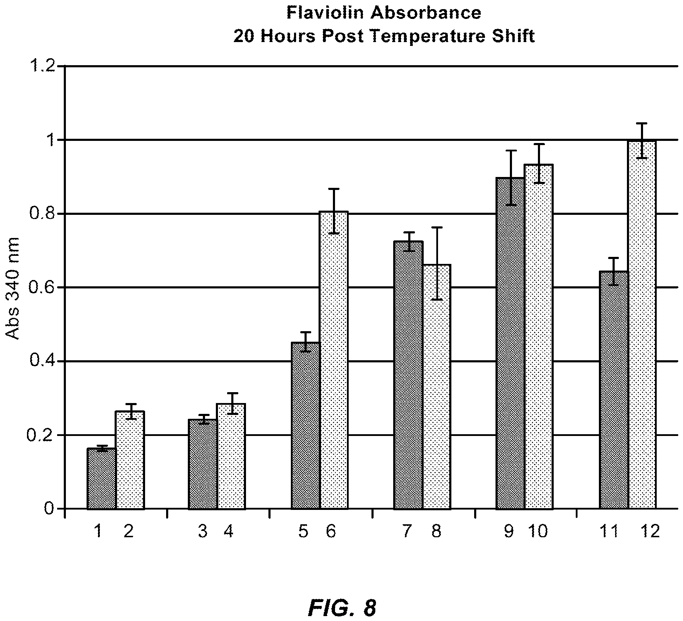

FIG. 8 Flaviolin asorbance at 24 hrs for FAS (Fatty Acid Synthesis pathway) mutants having a plasmid Ptrc-THNS comprising a nucleic acid sequence encoding THN synthase. Numbers along X-axis refer to strains.

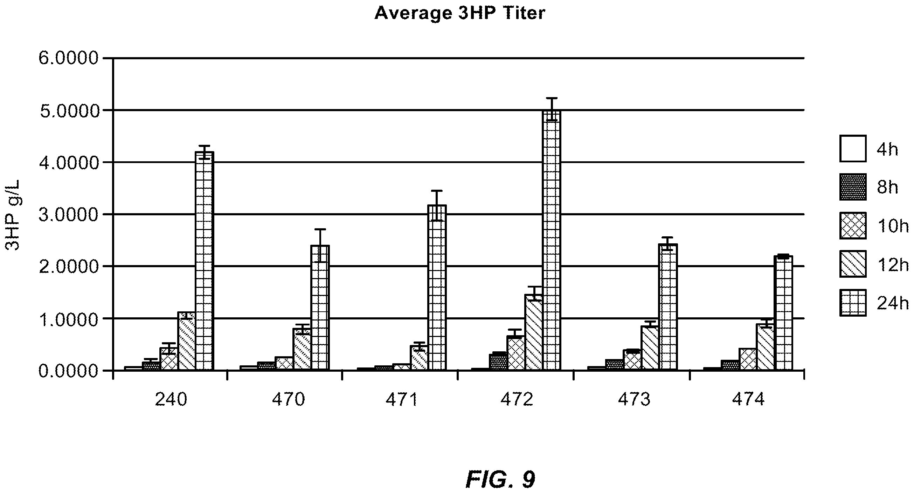

FIG. 9 3-HP Production by FAS mutant strains.

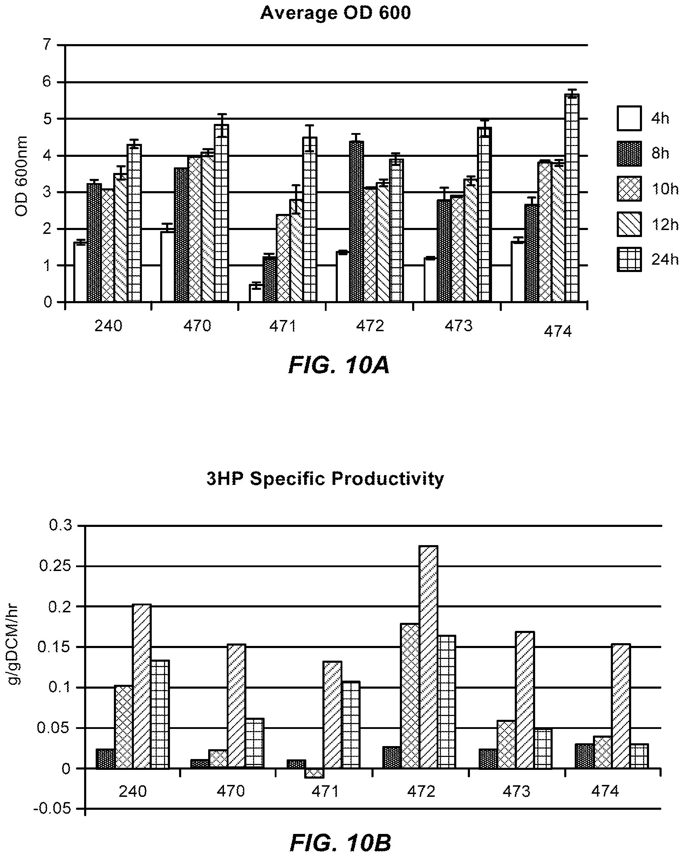

FIG.10A Growth of FAS mutant strains.
FIG.10B Productivity of FAS mutant strains.

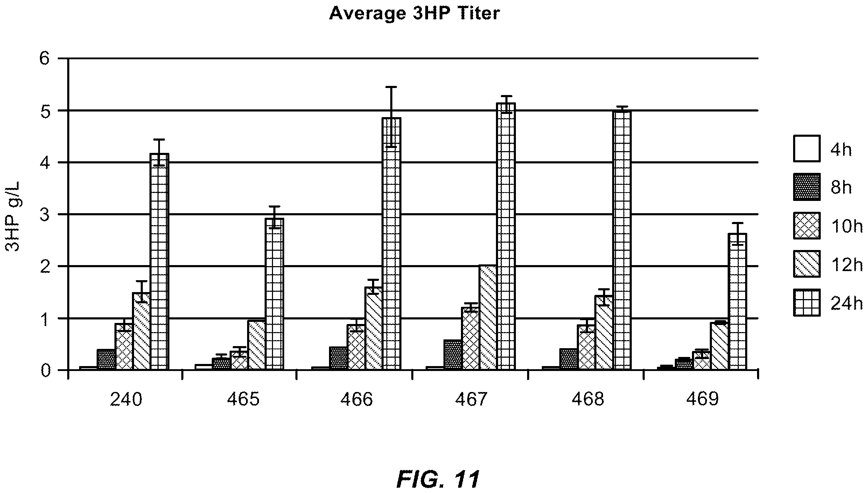

FIG. 11 Additional 3-HP Production of FAS mutants.

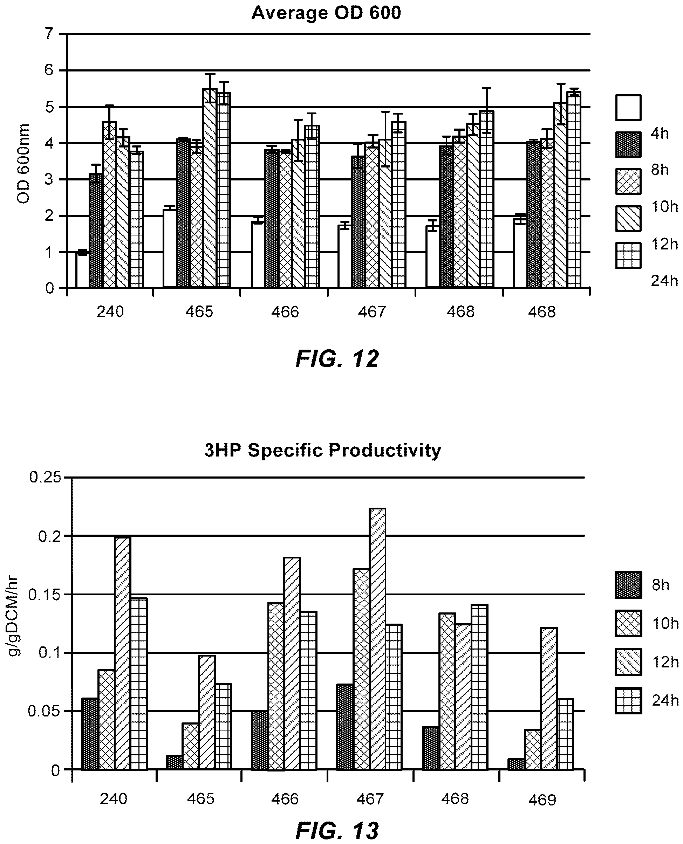

FIG. 12 Additional growth results of FAS mutants.
FIG.13 Additional Specific Productivity results of FAS mutants.

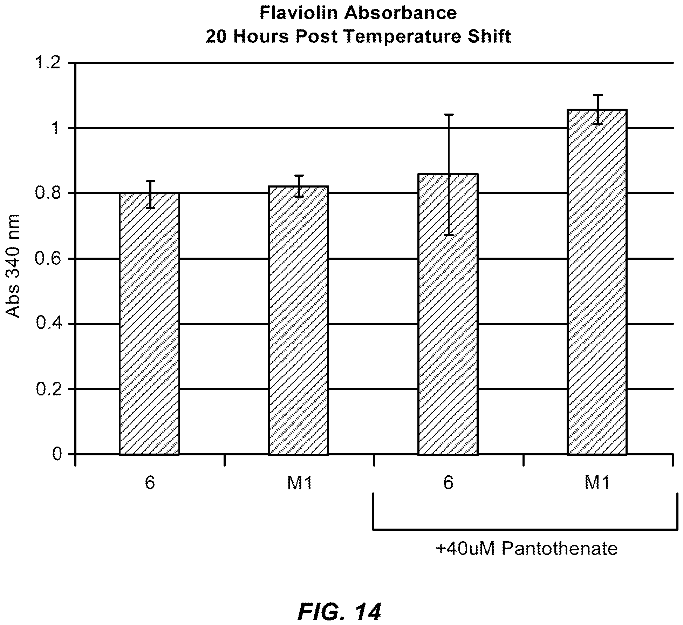

FIG.14 Flaviolin Absorbance results at 24 hours for FAS and CoA mutants

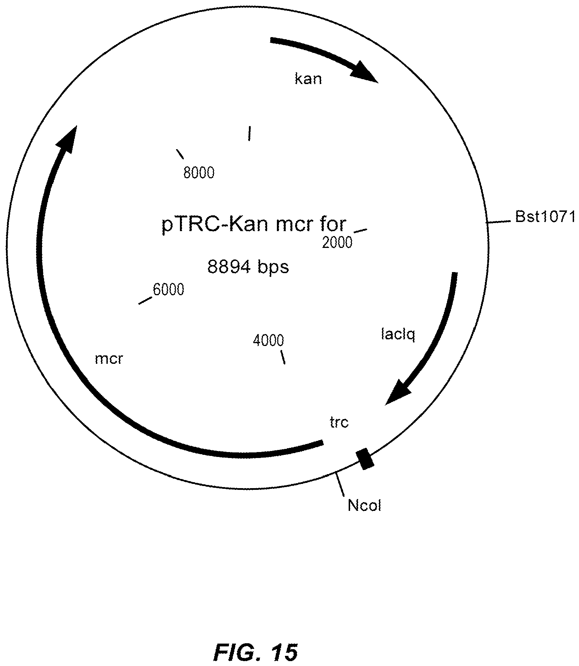

FIG. 15: Plasmid Map 1: Original pTrc-ptrc-MCR

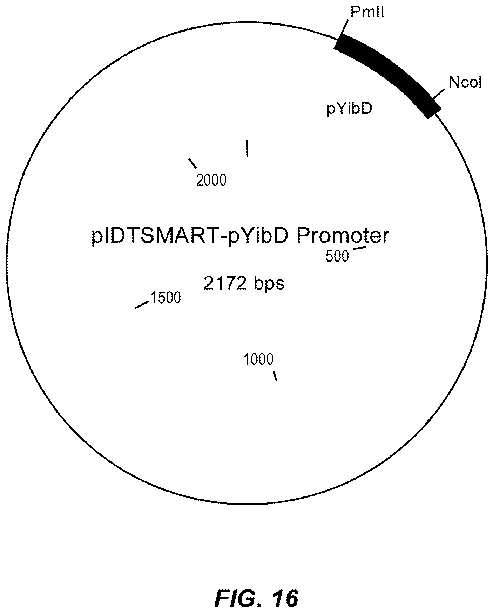

FIG 16: Plasmid Map 2: pIDTSMART-PyibD. Synthesized by Integrated DNA Technologies.

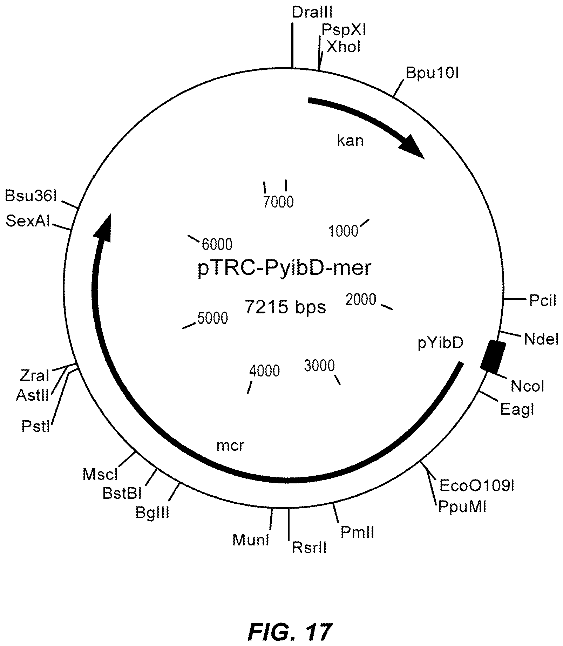

FIG. 17: Plasmid Map 3: New MCR construct. pTRC-PyibD-mcr (low phosphate induction) (SEQ ID NO:170).

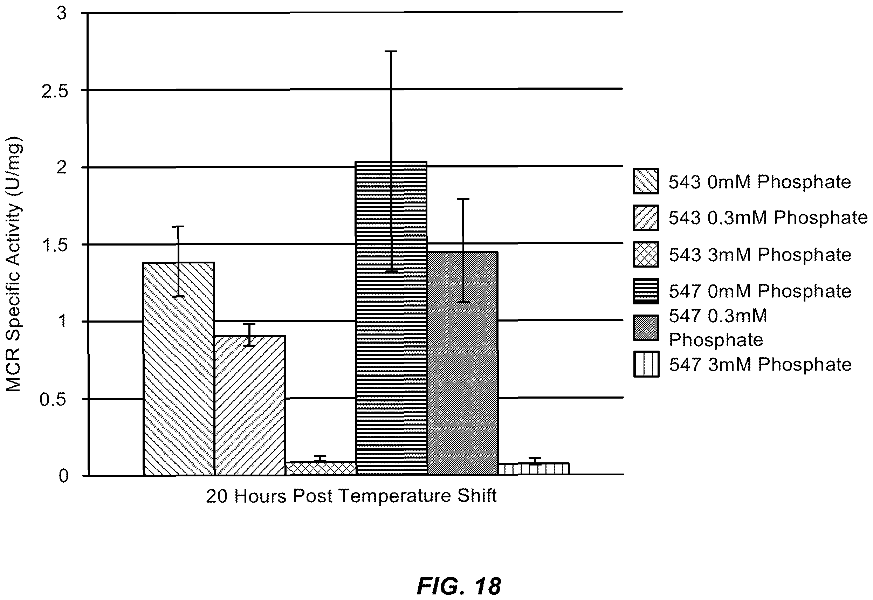

FIG. 18: mcr activity for PyibD-mcr measured at variable phosphate levels
FIG 19. Pyruvate dehydrogenase activity for various strains. Figure 19 shows that strains expressing one or two copies of the mutated lpdA gene (E354K) are less sensitive to inhibition by NADH compared to the wild type strains (BX775).

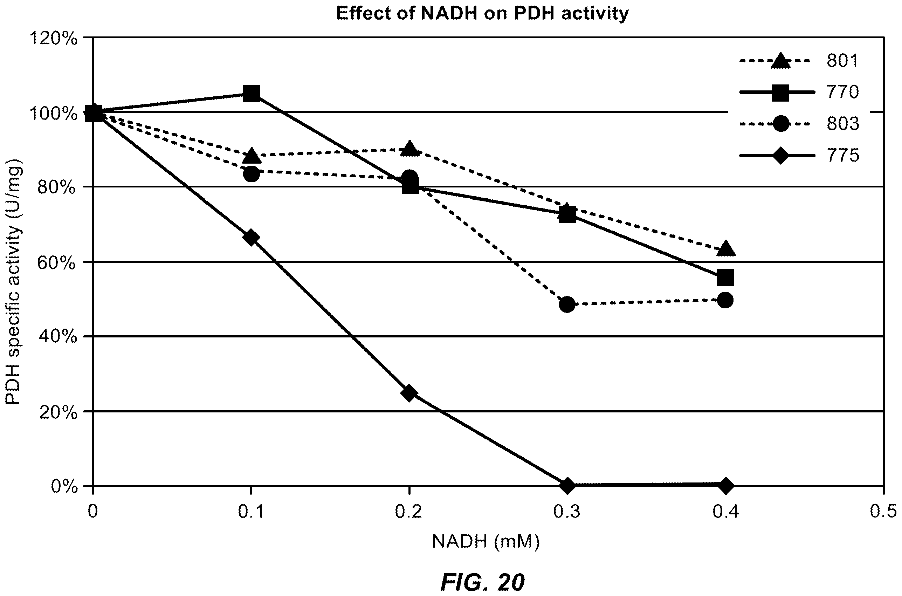

FIG. 20. Effect of NADH inhibition on pyruvate dehydrogenase activity.

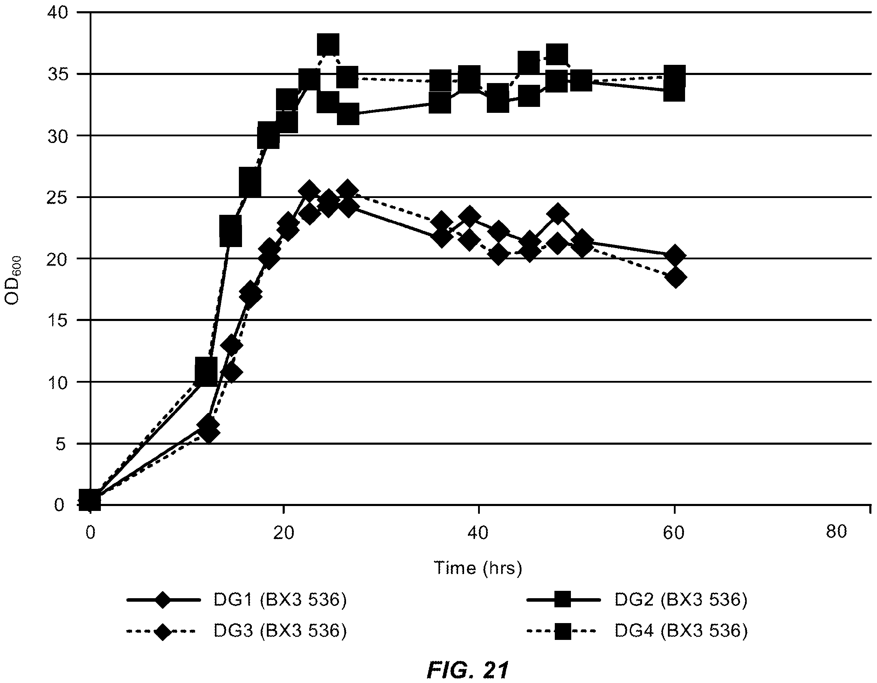

FIG 21. Growth profiles of 535 and 536 biocatalysts in fermentors

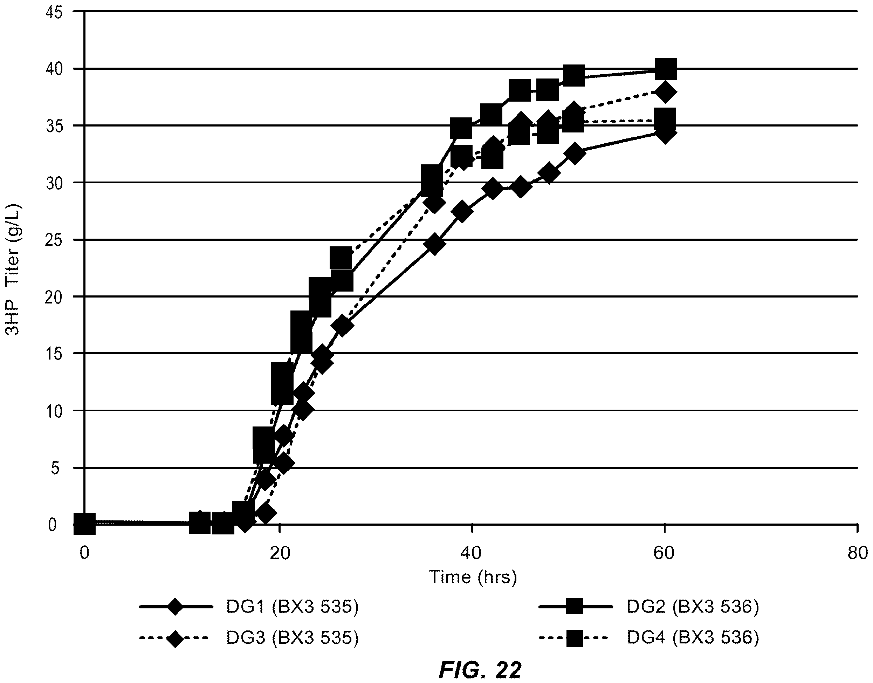

FIG. 22. 3-HP production from sucrose

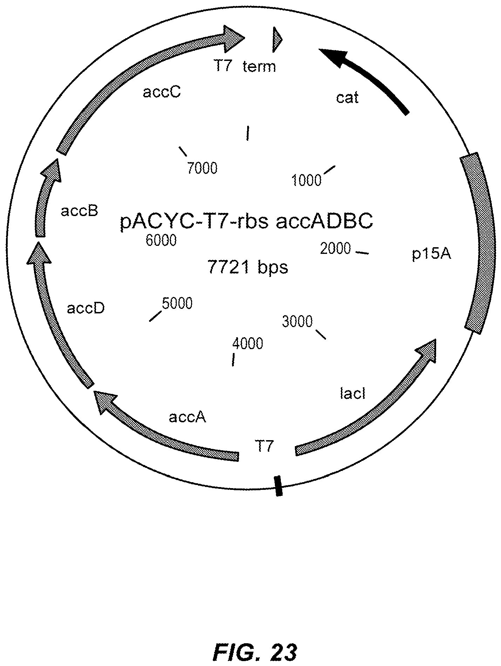

FIG. 23. Plasmid Map 4: pACYC-T7-rbs accADBC

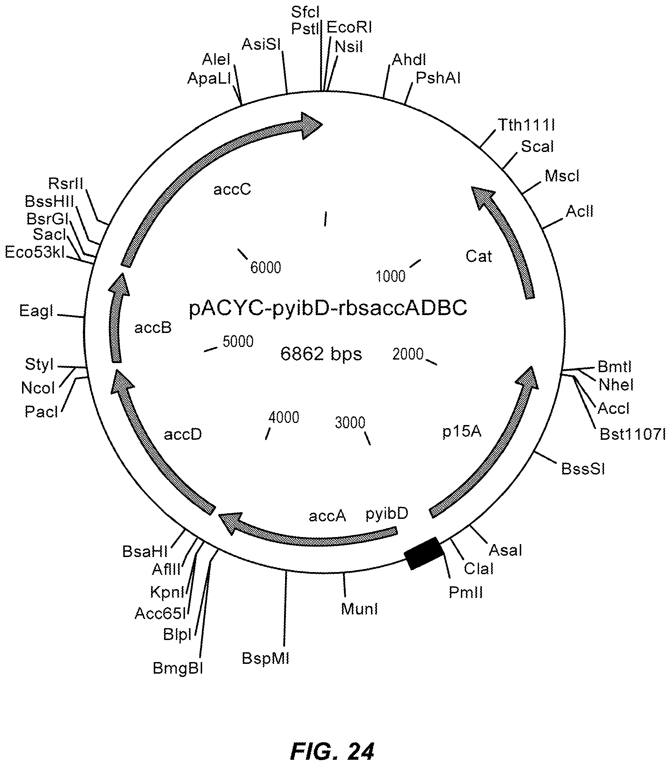

FIG. 24. Plasmid Map 5: map of pACYC-pyibD-rbsaccADBC

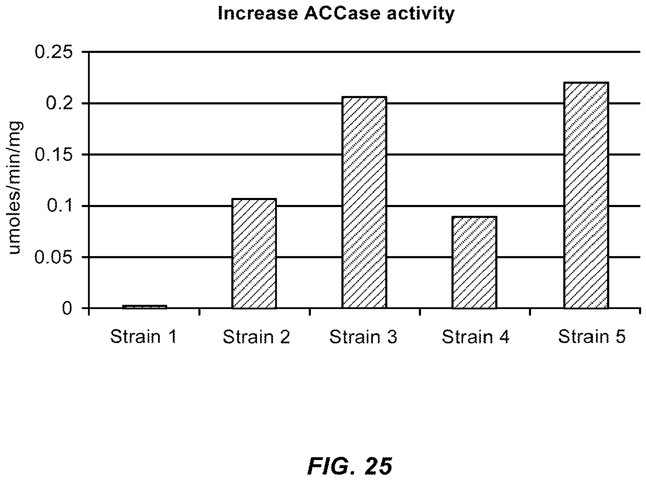

FIG. 25 shows increased ACCase activity for various strains.

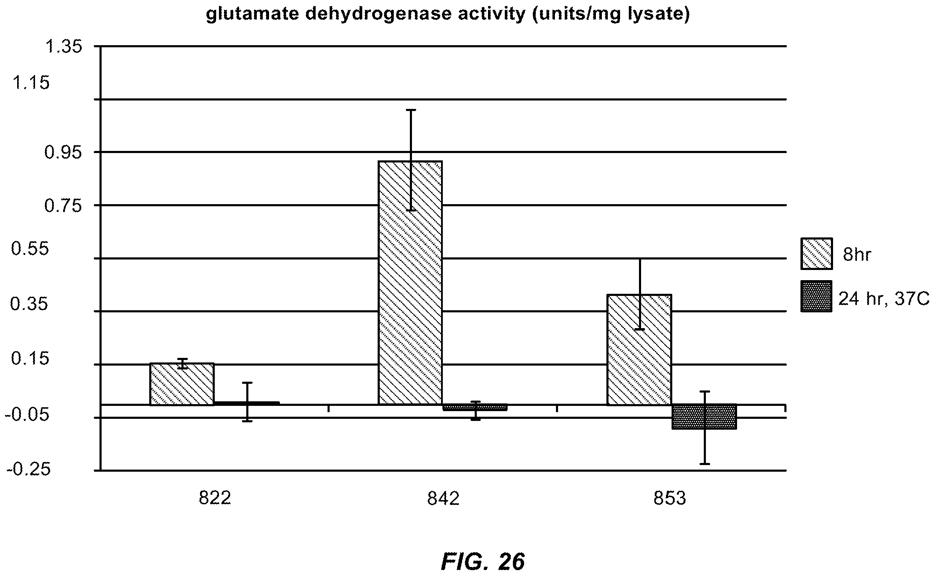

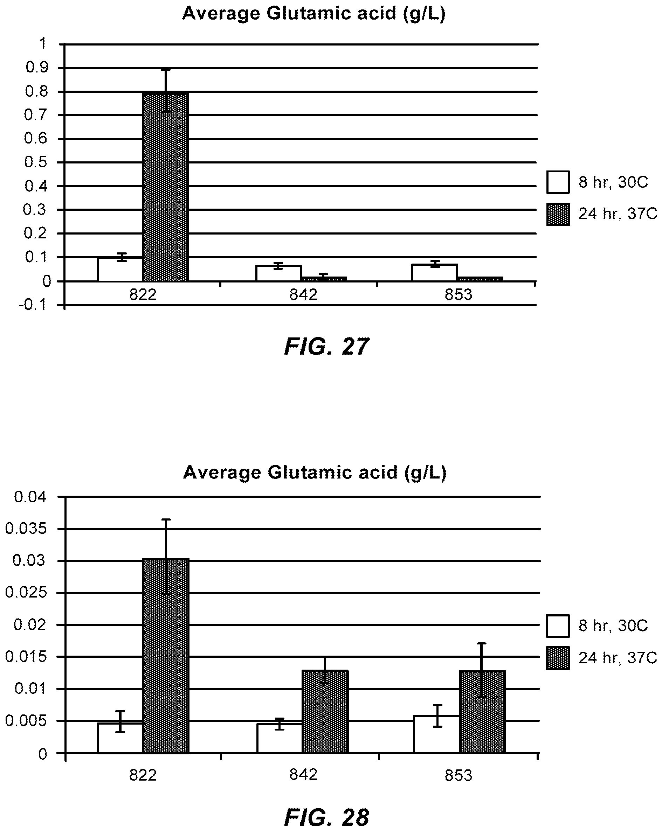

FIG. 26 shows glutmate dehydrogenase activity for strains with altered glutamate biosynthesis capabilities. In addition to the specific activity measurements, cultures grown at 30 degrees Celsius and shifted to 37 degree Celsius were also evaluated for their glutamate levels and there glutamine levels, a product derived from glutamate. Glutamate and glutamine levels can be determined in g/L using appropriate sensors such as those from YSI Incorporated using the manufactures instructions. Each sample was measured in triplicate. The glutamate and glutamine results are shown in Figures 27 and 28. These results show a significant decrease in glutamate and glutamine production from culture with strains carrying the *Psychrobacter sp. TAD1* gdh gene alone (853) or the gltB deletion and *Psychrobacter sp. TAD1* gdh gene in combination (842) upon shift to 37 Celsius when compared to the parent strain from which these two strains were derived (822).
FIG. 27: average glutamic acid production for strains with altered glutamate biosynthesis capabilities
FIG. 28: average glutamine production for strains with altered glutamate biosynthesis capabilities.
FIG. 29: Average specific productivity of 3-HP for a strain with altered glutamate biosynthesis
FIG. 30: Biochemical assays of the mcr from Sulfolobus tokodaii specific activity.

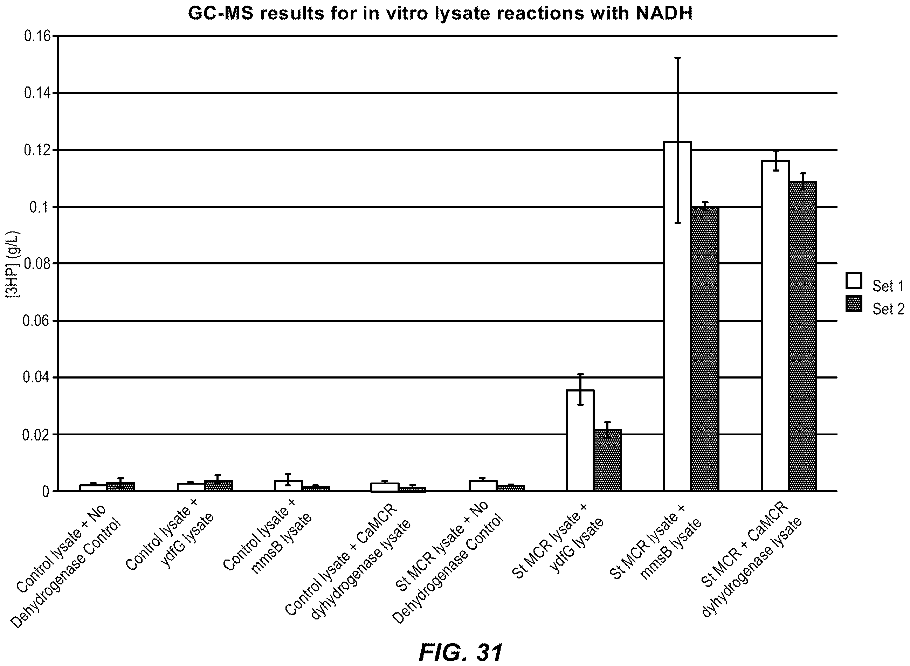

FIG. 31: GC-MS results for in vitro lysate reactions with NADH
FIG. 32: Flaviolin production measured in E. coli strains with fabI mutations complemented by *C*. *necator* fabI homologues

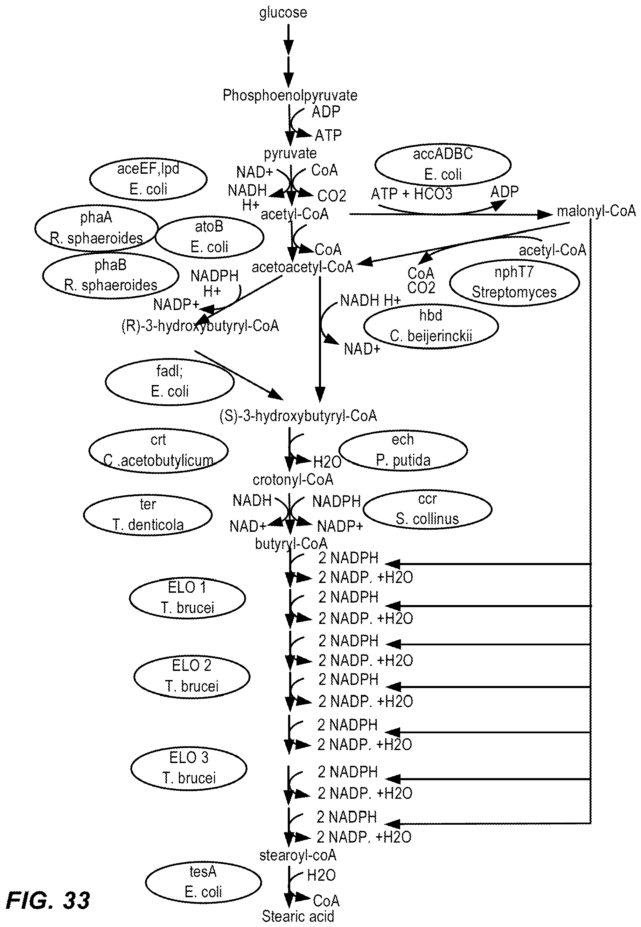

FIG. 33: elongase metabolic pathway
FIGs. 34A-C Process of converting biomass to a product.
FIGs. 35A-B Process of converting biomass to a product.

### DETAILED DESCRIPTION THE INVENTION

The invention is defined in the claims. The present invention provides compositions and methods for production of malonyl-CoA derived products from genetically modified microorganisms. The microorganisms described herein have been engineered to increase yields and productivities to desired malonyl-CoA dependent chemical products by reducing carbon flow through native fatty acyl-ACP biosynthesis. More specifically, by reducing flux through native fatty acid synthesis a proportionally greater number of malonyl-CoA molecules are 1) produced and/or 2) converted via the metabolic pathway from malonyl-CoA to the selected chemical product. Disclosed and exemplified herewith are combinations of genetic modifications that are shown to provide unexpectedly elevated increases in productivity of a desired chemical product that is biosynthesized in a host cell with such genetic modifications. It was shown that certain combinations of genetic modifications would result in increased flux through malonyl-CoA, where any single modification alone may be enough to block the competing fatty acid synthesis pathway.

### I. DEFINITIONS

Definitions and abbreviations are as follows:
As used in the specification and the claims, the singular forms "a," "an," and "the" include plural referents unless the context clearly dictates otherwise. Thus, for example, reference to an "expression vector" includes a single expression vector as well as a plurality of expression vectors, either the same (e.g., the same operon) or different; reference to "microorganism" includes a single microorganism as well as a plurality of microorganisms; and the like.

"C" means Celsius or degrees Celsius, as is clear from its usage, DCW means dry cell weight, "s" means second(s), "min" means minute(s), "h," "hr," or "hrs" means hour(s), "psi" means pounds per square inch, "nm" means nanometers, "d" means day(s), "µL" or "µL" or "ul" means microliter(s), "mL" means milliliter(s), "L" means liter(s), "mm" means millimeter(s), "nm" means nanometers, "mM" means millimolar, "µM" or "uM" means micromolar, "M" means molar, "mmol" means millimole(s), "µmol" or "uMol" means micromole(s)", "g" means gram(s), "µg" or "ug" means microgram(s) and "ng" means nanogram(s), "PCR" means polymerase chain reaction, "OD" means optical density, "OD₆₀₀ means the optical density measured at a photon wavelength of 600 nm, "kDa" means kilodaltons, "g" means the gravitation constant, "bp" means base pair(s), "kbp" means kilobase pair(s), "% w/v" means weight/volume percent,"% v/v" means volume/volume percent, "IPTG" means isopropyl-µ-D-thiogalactopyranoiside, "RBS" means ribosome binding site, "rpm" means revolutions per
minute, "HPLC" means high performance liquid chromatography, and "GC" means gas chromatography. Also, 10^5 and the like are taken to mean 10⁵ and the like.

As disclosed herein, "3-HP" and "3HP" means 3-hydroxypropionic acid.

As used herein, dry cell weight (DCW) for *E. coli* strains is calculated as 0.41 times the measured OD₆₀₀ value, based on baseline DCW to OD₆₀₀ determinations.

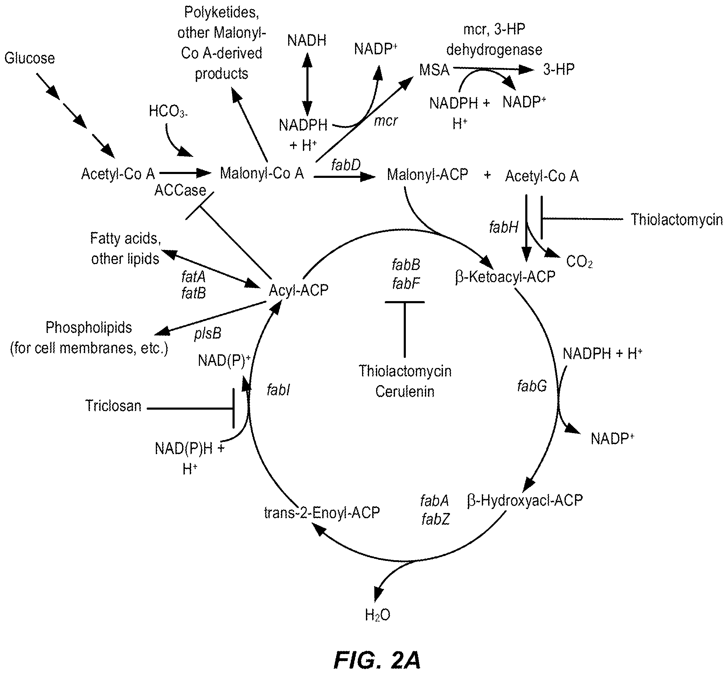

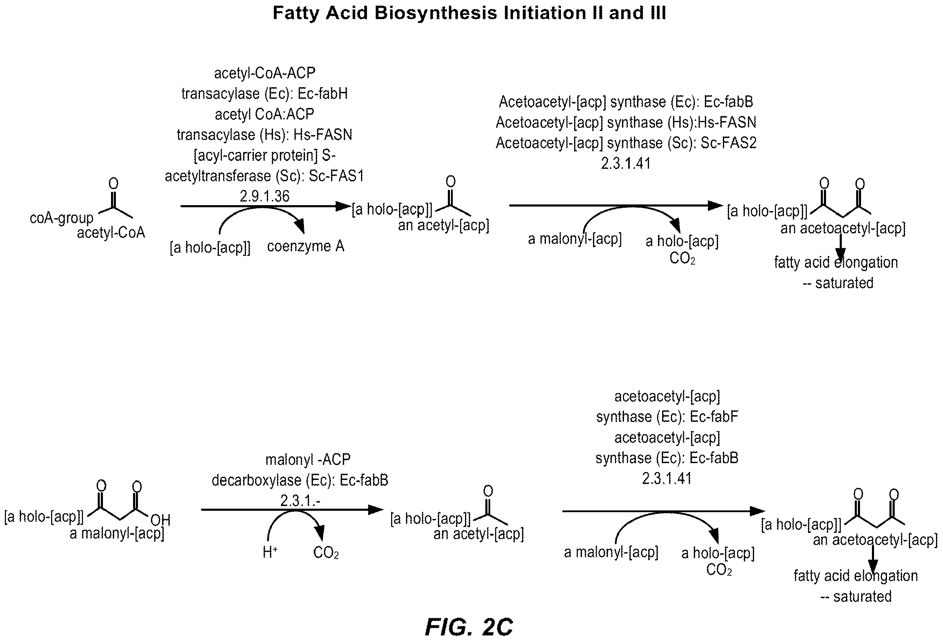

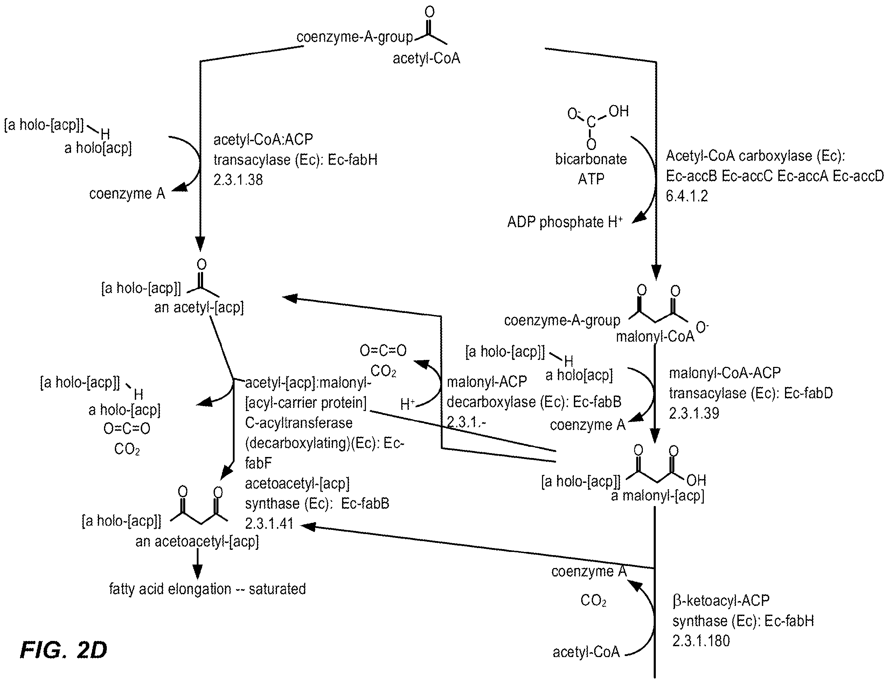

As used herein, "fatty acid synthase," whether followed by "pathway," "system," or "complex," is meant to refer to a metabolic pathway, often involving cyclic reactions to biosynthesize fatty acids in a host cell. For example, FIG. 2A depicts a fatty acid synthase pathway common to bacteria. It is noted that this may also be referred to as a "fatty acid synthesis," a "fatty acid biosynthesis," (or a "fatty acid synthetase") "pathway," "system," or "complex."

As used herein, "reduced enzymatic activity," "reducing enzymatic activity," and the like is meant to indicate that a microorganism cell's, or an isolated enzyme, exhibits a lower level of activity than that measured in a comparable cell of the same species or its native enzyme. That is, enzymatic conversion of the indicated substrate(s) to indicated product(s) under known standard conditions for that enzyme is at least 10, at least 20, at least 30, at least 40, at least 50, at least 60, at least 70, at least 80, or at least 90% less than the enzymatic activity for the same biochemical conversion by a native (non-modified) enzyme under a standard specified condition. This term also can include elimination of that enzymatic activity. A cell having reduced enzymatic activity of an enzyme can be identified using any method known in the art. For example, enzyme activity assays can be used to identify cells having reduced enzyme activity (see, for example, Enzyme Nomenclature, Academic Press, Inc., New York, NY 2007).

The term "reduction" or "to reduce" when used in such phrase and its grammatical equivalents are intended to encompass a complete elimination of such conversion(s).

The term "heterologous DNA," "heterologous nucleic acid sequence," and the like as used herein refers to a nucleic acid sequence wherein at least one of the following is true: (a) the sequence of nucleic acids is foreign to (i.e., not naturally found in) a given host microorganism; (b) the sequence may be naturally found in a given host microorganism, but in an unnatural (e.g., greater than expected) amount; or (c) the sequence of nucleic acids comprises two or more subsequences that are not found in the same relationship to each other in nature. For example, regarding instance (c), a heterologous nucleic acid sequence that is recombinantly produced will have two or more sequences from unrelated genes arranged to make a new functional nucleic acid.

The term "heterologous" is intended to include the term "exogenous" as the latter term is generally used in the art. With reference to the host microorganism's genome prior to the introduction of a heterologous nucleic acid sequence, the nucleic acid sequence that codes for the enzyme is heterologous (whether or not the heterologous nucleic acid sequence is introduced into that genome).

By "increase production," "increase the production," and like terms is meant to increase the quantity of one or more of enzymes, the enzymatic activity, the enzymatic specificity, and/or the overall flux through an enzymatic conversion step, biosynthetic pathway, or portion of a biosynthetic pathway. A discussion of non-limiting genetic modification techniques is discussed, infra, which may be used either for increasing or decreasing a particular enzyme's quantity, activity, specificity, flux, etc.

As used herein, the term "gene disruption," or grammatical equivalents thereof (and including "to disrupt enzymatic function," "disruption of enzymatic function," and the like), is intended to mean a genetic modification to a microorganism that renders the encoded gene product as having a reduced polypeptide activity compared with polypeptide activity in or from a microorganism cell not so modified. The genetic modification can be, for example, deletion of the entire gene, deletion or other modification of a regulatory sequence required for transcription or translation, deletion of a portion of the gene which results in a truncated gene product (e.g., enzyme) or by any of various mutation strategies that reduces activity (including to no detectable activity level) the encoded gene product. A disruption may broadly include a deletion of all or part of the nucleic acid sequence encoding the enzyme, and also includes, but is not limited to other types of genetic modifications, e.g., introduction of stop codons, frame shift mutations, introduction or removal of portions of the gene, and introduction of a degradation signal, those genetic modifications affecting mRNA transcription levels and/or stability, and altering the promoter or repressor upstream of the gene encoding the enzyme.

In various contexts, a gene disruption is taken to mean any genetic modification to the DNA, mRNA encoded from the DNA, and the corresponding amino acid sequence that results in reduced polypeptide activity. Many different methods can be used to make a cell having reduced polypeptide activity. For example, a cell can be engineered to have a disrupted regulatory sequence or polypeptide-encoding sequence using common mutagenesis or knock-out technology (see, e.g., Methods in Yeast Genetics (1997 edition), Adams et al., Cold Spring Harbor Press (1998)). One particularly useful method of gene disruption is complete gene deletion because it reduces or eliminates the occurrence of genetic reversions in the genetically modified microorganisms of the invention. Accordingly, a disruption of a gene whose product is an enzyme thereby disrupts enzymatic function. Alternatively, antisense technology can be used to reduce the activity of a particular polypeptide. For example, a cell can be engineered to contain an eDNA that encodes an antisense molecule that prevents a polypeptide from being translated. Further, gene silencing can be used to reduce the activity of a particular polypeptide.

The term "antisense molecule" as used herein encompasses any nucleic acid molecule or nucleic acid analog (e.g., peptide nucleic acids) that contains a sequence that corresponds to the coding strand of an endogenous polypeptide. An antisense molecule also can have flanking sequences (e.g., regulatory sequences). Thus, antisense molecules can be ribozymes or antisense oligonucleotides.

As used herein, a ribozyme can have any general structure including, without limitation, hairpin, hammerhead, or axhead structures, provided the molecule cleaves RNA.

Bio-production, as used herein, may be aerobic, microaerobic, or anaerobic.

As used herein, the language "sufficiently identical" refers to proteins or portions thereof that have amino acid sequences that include a minimum number of identical or equivalent amino acid residues when compared to an amino acid sequence of the amino acid sequences provided in this application (including the SEQ ID Nos./sequence listings) such that the protein or portion thereof is able to achieve the respective enzymatic reaction and/or other function. To determine whether a particular protein or portion thereof is sufficiently homologous may be determined by an assay of enzymatic activity, such as those commonly known in the art.

Descriptions and methods for sequence identity and homology are intended to be exemplary and it is recognized that these concepts are well-understood in the art. Further, it is appreciated that nucleic acid sequences may be varied and still encode an enzyme or other polypeptide exhibiting a desired functionality. Also, it is intended that the phrase "equivalents thereof is mean to indicate functional equivalents of a referred to gene, enzyme or the like. Such an equivalent may be for the same species or another species, such as another microorganism species.

Further to nucleic acid sequences, a nucleic acid is "hybridizable" to another nucleic acid when a single stranded form of the nucleic acid can anneal to the other nucleic acid under appropriate conditions of temperature and solution ionic strength. Hybridization and washing conditions are well known and exemplified in Sambrook (1989), supra, (see in particular Chapters 9 and 11). Low stringency hybridization conditions correspond to a Tm of 55°C (for example 5xSSC, 0.1% SDS, 0.25 milk and no formamide or 5xSSC, 0.5% SDS and 30% formamide). Moderate stringency hybridization conditions correspond for example, to Tm of 60°C (for example 6xSSC, 0.1% SDS, 0.05% milk with or without formamide, and stringent hybridization conditions correspond for example, to a Tm of 65°C. and 0.1xSSC and 0.1% SDS. For various embodiments of the invention a sequence of interest may be hybridizable under any such stringency condition - low, moderate or high.

The term "identified enzymatic functional variant" means a polypeptide that is determined to possess an enzymatic activity and specificity of an enzyme of interest but which has an amino acid sequence different from such enzyme of interest. A corresponding "variant nucleic acid sequence" may be constructed that is determined to encode such an identified enzymatic functional variant. These may be identified and/or developed from orthologs, paralogs, or nonorthologous gene displacements.

The use of the phrase "segment of interest" is meant to include both a gene and any other nucleic acid sequence segment of interest. One example of a method used to obtain a segment of interest is to acquire a culture of a microorganism, where that microorganism's genome includes the gene or nucleic acid sequence segment of interest.

When the genetic modification of a gene product, i.e., an enzyme, is referred to herein, including the claims, it is understood that the genetic modification is of a nucleic acid sequence, such as or including the gene, that normally encodes the stated gene product, i.e., the enzyme.

By "means for modulating" the conversion of malonyl-CoA to fatty acyl-ACP or fatty acyl-coA molecules, and to fatty acid molecules, is meant any one of the following: 1) providing in a microorganism cell at least one polynucleotide that encodes at least one polypeptide having activity of one of the fatty acid synthase system enzymes (such as recited herein), wherein the polypeptide so encoded has (such as by mutation and/or promoter substitution, etc., to lower enzymatic activity), or may be modulated to have (such as by temperature sensitivity, inducible promoter, etc.) a reduced enzymatic activity; 2) providing to a vessel comprising a microorganism cell or population an inhibitor that inhibits enzymatic activity of one or more of the fatty acid synthase system enzymes (such as recited herein), at a dosage effective to reduce enzymatic activity of one or more of these enzymes. These means may be provided in combination with one another. When a means for modulating involves a conversion, during a fermentation event, from a higher to a lower activity of the fatty acid synthetase system, such as by increasing temperature of a culture vessel comprising a population of genetically modified microorganism comprising a temperature-sensitive fatty acid synthetase system polypeptide (e.g., enoyl-ACP reductase), or by adding an inhibitor, there are conceived two modes - one during which there is higher activity, and a second during which there is lower activity, of such fatty acid synthetase system. During the lower activity mode, a shift to greater utilization of malonyl-CoA to a selected chemical product may proceed.

"CoA" means coenzyme-A, and "mcr" means malonyl-CoA reductase.

A "temperature-sensitive mutation" or "(ts)" refers to a modified gene product that can be induced to express the modified RNA at specific temperature.

### II. ORGANISMS

Features as described and claimed herein may be provided in a microorganism selected from the listing herein, or another suitable microorganism, that also comprises one or more natural, introduced, or enhanced chemical product biosynthesis pathway. Thus, in some embodiments the microorganism comprises an endogenous chemical product biosynthesis pathway (which may, in some such embodiments, be enhanced), whereas in other embodiments the microorganism does not comprise an endogenous biosynthesis pathway for the selected chemical product.

Varieties of these genetically modified microorganisms may comprise genetic modifications and/or other system alterations as may be described in other patent applications of one or more of the present inventor(s) and/or subject to assignment or license to the owner of the present patent application.

The examples describe specific modifications and evaluations to certain bacterial and yeast microorganisms. Generally, a microorganism used for the present invention may be selected from bacteria, cyanobacteria, filamentous fungi and yeasts.

For some embodiments, microbial hosts initially selected for a selected chemical product biosynthesis should also utilize sugars including glucose at a high rate. Most microbes are capable of utilizing carbohydrates. However, certain environmental microbes cannot utilize carbohydrates to high efficiency, and therefore would not be suitable hosts for such embodiments that are intended for glucose or other carbohydrates as the principal added carbon source.

As the genomes of various species become known, the present invention easily may be applied to an ever-increasing range of suitable microorganisms. Further, given the relatively low cost of genetic sequencing, the genetic sequence of a species of interest may readily be determined to make application of aspects of the present invention more readily obtainable (based on the ease of application of genetic modifications to an organism having a known genomic sequence). Public database sites, such as <<www.metacyc.org>>, <<www.ecocyc.org>>, <<www.biocyc.org>>, and <<www.ncbi.gov>>, << htpp://www.nchi.nlm.nih.gov/>> have various genetic and genomic information and associated tools to identify enzymes in various species that have desired function or that may be modified to achieve such desired function.

More particularly, based on the various criteria described herein, suitable microbial hosts for the biosynthesis of a chemical product generally may include, but are not limited to, any gram negative organisms, more particularly a member of the family Enterobacteriaceae, such as E. coli, or Oligotropha carboxidovorans, or Pseudomononas sp.; any gram positive microorganism, for example Bacillus subtilis, Lactobaccilus sp. or Lactococcus sp.; a yeast, for example Saccharomyces cerevisiae, Pichia pastoris or Pichia stipitis; and other groups or microbial species including those found in Actinomycetes (also referred to as Actinobacteria). More particularly, suitable microbial hosts for the biosynthesis of a chemical product generally include, but are not limited to, members of the genera Clostridium, Zymomonas, Escherichia, Salmonella, Rhodococcus, Pseudomonas, Bacillus, Lactobacillus, Enterococcus, Alcaligenes, Klebsiella, Paenibacillus,Arthrobacter, Corynebacterium, Brevibacterium, Pichia, Candida, Hansenula and Saccharomyces. Hosts that may be particularly of interest include: Oligotropha carboxidovorans (such as strain OMS), Escherichia coli, Alcaligenes eutrophus (Cupriavidus necator), Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis and Saccharomyces cerevisiae.

More particularly, suitable microbial hosts for the biosynthesis of selected chemical products generally include, but are not limited to, members of the genera Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula and Saccharomyces.

Hosts that may be particularly of interest include: Oligotropha carboxidovorans (such as strain 0M5T), Escherichia coli, Alcaligenes eutrophus (Cupriavidus necator), Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis and Saccharomyces cerevisiae. Also, any of the known strains of these species may be utilized as a starting microorganism, as may any of the following species including respective strains thereof- Cupriavidus basilensis, Cupriavidus campinensis, Cupriavidus gilardi, Cupriavidus laharsis, Cupriavidus metallidurans, Cupriavidus oxalaticus, Cupriavidus pauculus, Cupriavidus pinatubonensis, Cupriavidus respiraculi, and Cupriavidus taiwanensis.

In some embodiments, the recombinant microorganism is a gram-negative bacterium. In some embodiments, the recombinant microorganism is selected from the genera Zymomonas, Escherichia, Pseudomonas, Alcaligenes, and Klebsiella. In some embodiments, the recombinant microorganism is selected from the species Escherichia coli, Cupriavidus necator, Oligotropha carboxidovorans, and Pseudomonas putida. In some embodiments, the recombinant microorganism is an E. coli strain.

In some embodiments, the recombinant microorganism is a gram-positive bacterium. In some embodiments, the recombinant microorganism is selected from the genera Clostridium, Salmonella, Rhodococcus, Bacillus, Lactobacillus, Enterococcus, Paenibacillus, Arthrobacter, Corynebacterium, and Brevibacterium. In some embodiments, the recombinant microorganism is selected from the species Bacillus licheniformis, Paenibacillus macerans, Rhodococcus erythropolis, Lactobacillus plantarum, Enterococcus faecium, Enterococcus gallinarium, Enterococcus faecalis, and Bacillus subtilis. In particular embodiments, the recombinant microorganism is a B. subtilis strain.

In some embodiments, the recombinant microorganism is a yeast. In some embodiments, the recombinant microorganism is selected from the genera Pichia, Candida, Hansenula and Saccharomyces. In particular embodiments, the recombinant microorganism is Saccharomyces cerevisiae.

The ability to genetically modify the host is essential for the production of any recombinant microorganism. The mode of gene transfer technology may be by electroporation, conjugation, transduction or natural transformation. A broad range of host conjugative plasmids and drug resistance markers are available. The cloning vectors are tailored to the host organisms based on the nature of antibiotic resistance markers that can function in that host.

### III. CARBON SOURCES/GROWTH MEDIA/BIOREACTORS

### Carbon Sources

Bio-production media, which is used in the present disclosure with recombinant microorganisms having a biosynthetic pathway for 3-HP, must contain suitable carbon sources or substrates for the intended metabolic pathways. Suitable substrates may include, but are not limited to, monosaccharides such as glucose and fructose, oligosaccharides such as lactose or sucrose, polysaccharides such as starch or cellulose or mixtures thereof and unpurified mixtures from renewable feedstocks such as cheese whey permeate, cornsteep liquor, sugar beet molasses, and barley malt. Additionally the carbon substrate may also be one-carbon substrates such as carbon dioxide, carbon monoxide, or methanol for which metabolic conversion into key biochemical intermediates has been demonstrated. In addition to one and two carbon substrates methylotrophic organisms are also known to utilize a number of other carbon containing compounds such as methylamine, glucosamine and a variety of amino acids for metabolic activity. Although it is contemplated that all of the above mentioned carbon substrates and mixtures thereof are suitable in the present invention as a carbon source, common carbon substrates used as carbon sources are glucose, fructose, and sucrose, as well as mixtures of any of these sugars. Other suitable substrates include xylose, arabinose, other cellulose-based C-5
sugars, high-fructose corn syrup, and various other sugars and sugar mixtures as are available commercially. Sucrose may be obtained from feedstocks such as sugar cane, sugar beets, cassava, bananas or other fruit, and sweet sorghum. Glucose and dextrose may be obtained through saccharification of starch based feedstocks including grains such as corn, wheat, rye, barley, and oats. Also, in some embodiments all or a portion of the carbon source may be glycerol. Alternatively, glycerol may be excluded as an added carbon source.

In one embodiment, the carbon source is selected from glucose, fructose, sucrose, dextrose, lactose, glycerol, and mixtures thereof. Variously, the amount of these components in the carbon source may be greater than about 50%, greater than about 60%, greater than about 70%, greater than about 80%, greater than about 90%, or more, up to 100% or essentially 100% of the carbon source.

In addition, methylotrophic organisms are known to utilize a number of other carbon containing compounds such as methylamine, glucosamine and a variety of amino acids for metabolic activity. For example, methylotrophic yeast are known to utilize the carbon from methylamine to form trehalose or glycerol (Bellion et al., Microb. Growth Cl Compd. (Int. Symp.), 7th (1993), 415-32. Editor(s): Murrell, J. Collin; Kelly, Don P. Publisher: Intercept, Andover, UK). Similarly, various species of Candida will metabolize alanine or oleic acid (Sulter et al., Arch. Microbiol. 153:485-489 (1990)). Hence it is contemplated that the source of carbon utilized in embodiments of the present invention may encompass a wide variety of carbon-containing substrates.

In addition, fermentable sugars may be obtained from cellulosic and lignocellulosic biomass through processes of pretreatment and saccharification, as described, for example, in U.S. Patent Publication No. 2007/0031918A1. Biomass refers to any cellulosic or lignocellulosic material and includes materials comprising cellulose, and optionally further comprising hemicellulose, lignin, starch, oligosaccharides and/or monosaccharides. Biomass may also comprise additional components, such as protein and/or lipid. Biomass may be derived from a single source, or biomass can comprise a mixture derived from more than one source; for example, biomass could comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn grain, corn cobs, crop residues such as corn husks, corn stover, grasses, wheat, wheat straw, barley, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers and animal manure. Any such
biomass may be used in a bio-production method or system to provide a carbon source. Various approaches to breaking down cellulosic biomass to mixtures of more available and utilizable carbon molecules, including sugars, include: heating in the presence of concentrated or dilute acid (e.g., < 1% sulfuric acid); treating with ammonia; treatment with ionic salts; enzymatic degradation; and combinations of these. These methods normally follow mechanical separation and milling, and are followed by appropriate separation processes.

In various embodiments, any of a wide range of sugars, including, but not limited to sucrose, glucose, xylose, cellulose or hemicellulose, are provided to a microorganism, such as in an industrial system comprising a reactor vessel in which a defined media (such as a minimal salts media including but not limited to M9 minimal media, potassium sulfate minimal media, yeast synthetic minimal media and many others or variations of these), an inoculum of a microorganism providing one or more of the 3-HP biosynthetic pathway alternatives, and the a carbon source may be combined. The carbon source enters the cell and is cataboliized by well-known and common metabolic pathways to yield common metabolic intermediates, including phosphoenolpyruvate (PEP). (See Molecular Biology of the Cell, 3rd Ed., B. Alberts et al. Garland Publishing, New York, 1994, pp. 42-45, 66-74 Principles of Biochemistry, 3rd Ed., D. L. Nelson & M. M. Cox, Worth Publishers, New York, 2000, pp 527-658 and Biochemistry, 4th Ed., L. Stryer, W. H. Freeman and Co., New York, 1995, pp. 463-650.)

Bio-based carbon can be distinguished from petroleum-based carbon according to a variety of methods, including without limitation ASTM D6866, or various other techniques. For example, carbon-14 and carbon-12 ratios differ in bio-based carbon sources versus petroleum-based sources, where higher carbon-14 ratios are found in bio-based carbon sources. In various embodiments, the carbon source is not petroleum-based, or is not predominantly petroleum based. In various embodiments, the carbon source is greater than about 50% non-petroleum based, greater than about 60% non-petroleum based, greater than about 70% non-petroleum based, greater than about 80% non-petroleum based, greater than about 90% non-petroleum based, or more. In various embodiments, the carbon source has a carbon-14 to carbon-12 ratio of about 1.0 x 10-14 or greater.

Various components may be excluded from the carbon source. For example, in some embodiments, acrylic acid, 1,4-butanediol, and/or glycerol are excluded or essentially excluded from the carbon source. As such, the carbon source according to some embodiments of the invention may be less than about 50% glycerol, less than about 40% glycerol, less than about 30% glycerol, less than about 20% glycerol, less than about 10% glycerol, less than about 5% glycerol, less than about 1% glycerol, or less. For example, the carbon source may be essentially glycerol-free. By essentially glycerol-free is meant that any glycerol that may be present in a residual amount does not contribute substantially to the production of the target chemical compound.

### Growth Media

In addition to an appropriate carbon source, such as selected from one of the herein-disclosed types, bio-production media must contain suitable minerals, salts, cofactors, buffers and other components, known to those skilled in the art, suitable for the growth of the cultures and promotion of the enzymatic pathway necessary for 3-HP production, or other products made under the present invention.

Another aspect of the invention regards media and culture conditions that comprise genetically modified microorganisms of the invention and optionally supplements.

Typically cells are grown at a temperature in the range of about 25° C to about 40° C in an appropriate medium, as well as up to 70° C for thermophilic microorganisms. Suitable growth media in the present invention are common commercially prepared media such as Luria Bertani (LB) broth, M9 minimal media, Sabouraud Dextrose (SD) broth, Yeast medium (YM) broth, (Ymin) yeast synthetic minimal media, and minimal media as described herein, such as M9 minimal media. Other defined or synthetic growth media may also be used, and the appropriate medium for growth of the particular microorganism will be known by one skilled in the art of microbiology or bio-production science. In various embodiments a minimal media may be developed and used that does not comprise, or that has a low level of addition of various components, for example less than 10, 5, 2 or 1 g/L of a complex nitrogen source including but not limited to yeast extract, peptone, tryptone, soy flour, corn steep liquor, or casein. These minimal medias may also have limited supplementation of vitamin mixtures including biotin, vitamin B12 and derivatives of vitamin B12, thiamin, pantothenate and other vitamins. Minimal medias may also have limited simple inorganic nutrient sources containing less than 28, 17, or 2.5 mM phosphate, less than 25 or 4 mM sulfate, and less than 130 or 50mM total nitrogen.

Bio-production media, which is used in embodiments of the present invention with genetically modified microorganisms, must contain suitable carbon substrates for the intended metabolic pathways. As described hereinbefore, suitable carbon substrates include carbon monoxide, carbon dioxide, and various monomeric and oligomeric sugars.

Suitable pH ranges for the bio-production are between pH 3.0 to pH 10.0, where pH 6.0 to pH 8.0 is a typical pH range for the initial condition. However, the actual culture conditions for a particular embodiment are not meant to be limited by these pH ranges.

Bio-productions may be performed under aerobic, microaerobic, or anaerobic conditions, with or without agitation.

### Bioreactors

Fermentation systems utilizing methods and/or compositions according to the disclosure are also within the scope of the disclosure.

Any of the recombinant microorganisms as described and/or referred to herein may be introduced into an industrial bio-production system where the microorganisms convert a carbon source into a selected chemical product, such as 3-HP or a polyketide such as described herein (including in priority document(s)), in a commercially viable operation. The bio-production system includes the introduction of such a recombinant microorganism into a bioreactor vessel, with a carbon source substrate and bio-production media suitable for growing the recombinant microorganism, and maintaining the bio-production system within a suitable temperature range (and dissolved oxygen concentration range if the reaction is aerobic or microaerobic) for a suitable time to obtain a desired conversion of a portion of the substrate molecules to 3-HP. Industrial bio-production systems and their operation are well-known to those skilled in the arts of chemical engineering and bioprocess engineering.

Bio-productions may be performed under aerobic, microaerobic, or anaerobic conditions, with or without agitation. The operation of cultures and populations of microorganisms to achieve aerobic, microaerobic and anaerobic conditions are known in the art, and dissolved oxygen levels of a liquid culture comprising a nutrient media and such microorganism populations may be monitored to maintain or confirm a desired aerobic, microaerobic or anaerobic condition. When syngas is used as a feedstock, aerobic, microaerobic, or anaerobic conditions may be utilized. When sugars are used, anaerobic, aerobic or microaerobic conditions can be implemented in various embodiments.

Any of the recombinant microorganisms as described and/or referred to herein may be introduced into an industrial bio-production system where the microorganisms convert a carbon source into 3-HP, and optionally in various embodiments also to one or more downstream compounds of 3-HP in a commercially viable operation. The bio-production system includes the introduction of such a recombinant microorganism into a bioreactor vessel, with a carbon source substrate and bio-production media suitable for growing the recombinant microorganism, and maintaining the bio-production system within a suitable temperature range (and dissolved oxygen
concentration range if the reaction is aerobic or microaerobic) for a suitable time to obtain a desired conversion of a portion of the substrate molecules to 3-HP.

In various embodiments, syngas components or sugars are provided to a microorganism, such as in an industrial system comprising a reactor vessel in which a defined media (such as a minimal salts media including but not limited to M9 minimal media, potassium sulfate minimal media, yeast synthetic minimal media and many others or variations of these), an inoculum of a microorganism providing an embodiment of the biosynthetic pathway(s) taught herein, and the carbon source may be combined. The carbon source enters the cell and is catabolized by well-known and common metabolic pathways to yield common metabolic intermediates, including phosphoenolpyruvate (PEP). (See Molecular Biology of the Cell, 3rd Ed., B. Alberts et al. Garland Publishing, New York, 1994, pp. 42-45, 66-74; Principles of Biochemistry, 3'd Ed., D. L. Nelson & M. M. Cox, Worth Publishers, New York 2000, pp. 527-658; and Biochemistry, 4th Ed., L. Stryer, W. H. Freeman and Co., New York, 1995, pp. 463-650.).

Further to types of industrial bio-production, various embodiments of the present disclosure may employ a batch type of industrial bioreactor. A classical batch bioreactor system is considered "closed" meaning that the composition of the medium is established at the beginning of a respective bio-production event and not subject to artificial alterations and additions during the time period ending substantially with the end of the bio-production event. Thus, at the beginning of the bio-production event the medium is inoculated with the desired organism or organisms, and bio-production is permitted to occur without adding anything to the system. Typically, however, a "batch" type of bio-production event is batch with respect to the addition of carbon source and attempts are often made at controlling factors such as pH and oxygen concentration. In batch systems the metabolite and biomass compositions of the system change constantly up to the time the bio¬production event is stopped. Within batch cultures cells moderate through a static lag phase to a high growth log phase and finally to a stationary phase where growth rate is diminished or halted. If untreated, cells in the stationary phase will eventually die. Cells in log phase generally are responsible for the bulk of production of a desired end product or intermediate.

A variation on the standard batch system is the fed-batch system. Fed-batch bio-production processes are also suitable in the present disclosure and comprise a typical batch system with the exception that the nutrients, including the substrate, are added in increments as the bio-production progresses. Fed-Batch systems are useful when catabolite repression is apt to
inhibit the metabolism of the cells and where it is desirable to have limited amounts of substrate in the media. Measurement of the actual nutrient concentration in Fed-Batch systems may be measured directly, such as by sample analysis at different times, or estimated on the basis of the changes of measurable factors such as pH, dissolved oxygen and the partial pressure of waste gases such as CO2. Batch and fed-batch approaches are common and well known in the art and examples may be found in Thomas D. Brock in Biotechnology: A Textbook of Industrial Microbiology, Second Edition (1989) Sinauer Associates, Inc., Sunderland, Mass., Deshpande, Mukund V., Appl. Biochem. Biotechnol., 36:227, (1992), and Biochemical Engineering Fundamentals, 2'd Ed. J. E. Bailey and D. F. 011is, McGraw Hill, New York, 1986.

Although embodiments of the present disclosure may be performed in batch mode, or in fed-batch mode, it is contemplated that the disclosure would be adaptable to continuous bio-production methods. Continuous bio-production is considered an "open" system where a defined bio-production medium is added continuously to a bioreactor and an equal amount of conditioned media is removed simultaneously for processing. Continuous bio-production generally maintains the cultures within a controlled density range where cells are primarily in log phase growth. Two types of continuous bioreactor operation include a chemostat, wherein fresh media is fed to the vessel while simultaneously removing an equal rate of the vessel contents. The limitation of this approach is that cells are lost and high cell density generally is not achievable. In fact, typically one can obtain much higher cell density with a fed-batch process. Another continuous bioreactor utilizes perfusion culture, which is similar to the chemostat approach except that the stream that is removed from the vessel is subjected to a separation technique which recycles viable cells back to the vessel. This type of continuous bioreactor operation has been shown to yield significantly higher cell densities than fed-batch and can be operated continuously. Continuous bio-production is particularly advantageous for industrial operations because it has less down time associated with draining, cleaning and preparing the equipment for the next bio-production event. Furthermore, it is typically more economical to continuously operate downstream unit operations, such as distillation, than to run them in batch mode.

Continuous bio-production allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Methods of modulating nutrients and growth factors for continuous bio-production processes as
well as techniques for maximizing the rate of product formation are well known in the art of industrial microbiology and a variety of methods are detailed by Brock, supra.

It is contemplated that embodiments of the present disclosure may be practiced using either batch, fed-batch or continuous processes and that any known mode of bio-production would be suitable. It is contemplated that cells may be immobilized on an inert scaffold as whole cell catalysts and subjected to suitable bio¬production conditions for 3-HP production, or be cultured in liquid media in a vessel, such as a culture vessel. Thus, embodiments used in such processes, and in bio-production systems using these processes, include a population of genetically modified microorganisms of the present disclosure, a culture system comprising such population in a media comprising nutrients for the population, and methods of making 3-HP and thereafter, a downstream product of 3-HP.

Embodiments of the disclosure include methods of making 3-HP in a bio-production system, some of which methods may include obtaining 3-HP after such bio-production event. For example, a method of making 3-HP may comprise: providing to a culture vessel a media comprising suitable nutrients; providing to the culture vessel an inoculum of a genetically modified microorganism comprising genetic modifications described herein such that the microorganism produces 3-HP from syngas and/or a sugar molecule; and maintaining the culture vessel under suitable conditions for the genetically modified microorganism to produce 3-HP.

Also, it is within the scope of the present disclosure to produce, and to utilize in bio-production methods and systems, including industrial bio-production systems for production of a selected chemical product (chemical), a recombinant microorganism genetically engineered to modify one or more aspects effective to increase chemical product bio-production by at least 20 percent over control microorganism lacking the one or more modifications.

In various embodiments, the disclosure is directed to a system for bio-production of a chemical product as described herein, said system comprising: a fermentation tank suitable for microorganism cell culture; a line for discharging contents from the fermentation tank to an extraction and/or separation vessel; and an extraction and/or separation vessel suitable for removal of the chemical product from cell culture waste. In various embodiments, the system includes one or more pre-fermentation tanks, distillation columns, centrifuge vessels, back extraction columns, mixing vessels, or combinations thereof.

The following published resources indicate the level of skill in these relevant arts, and as needed to support a disclosure that teaches how to make and use methods of industrial bio-production of 3-HP, or other product(s) produced under the disclosure, from sugar sources, and also industrial systems that may be used to achieve such conversion with any of the recombinant microorganisms of the
present disclosure (Biochemical Engineering Fundamentals, 2nd Ed. J. E. Bailey and D. F. 011is, McGraw Hill, New York, 1986, entire book for purposes indicated and Chapter 9, pages 533-657 in particular for biological reactor design; Unit Operations of Chemical Engineering, 5th Ed., W. L. McCabe et al., McGraw Hill, New York 1993, entire book for purposes indicated, and particularly for process and separation technologies analyses; Equilibrium Staged Separations, P. C. Wankat, Prentice Hall, Englewood Cliffs, NJ USA, 1988, entire book for separation technologies teachings). Generally, it is appreciated, in view of the disclosure, that any of the above methods and systems may be used for production of various chemical products such as those disclosed herein.

### IV. PRODUCTS

In various embodiments the compositions, methods and systems of the present invention involve inclusion of a metabolic production pathway that converts malonyl-CoA to a chemical product of interest.

One chemical product is 3-hydroxypropionic acid (CAS No. 503-66-2, "3-HP"). Chemical products further include tetracycline, erythromycin, avermectin, macrolides, vanomycin-group antibiotics, Type II polyketides, (5R)-carbapenem, 6-methoxymellein, acridone, actinorhodin, aloesone, apigenin, barbaloin, biochanin A, maackiain, medicarpin, cannabinoid, cohumulone, daidzein, flavonoid, formononetin, genistein, humulone, hyperforin, mycolate, olivetol, pelargonidin, pentaketide chromone, pinobanksin, pinosylvin, plumbagin, raspberry ketone, resveratrol, rifamycin B, salvianin, shisonin, sorgoleone, stearate, anthocyanin, ternatin, tetrahydroxyxanthone, usnate, and xanthohumol. Particular polyketide chemical products include 1,3,6,8- tetrahydroxynaphthalene (THN) or its derivative flaviolin (CAS No. 479-05-0). The production of 3-HP, or of THN or flaviolin, may be used herein to demonstrate the features of the disclosure as they may be applied to other chemical products. Alternatively, any of the above compounds may be excluded from a group of chemical products.

Numerous products can be made from malonyl-coA precursors alone, by expressing enzyme functions to convert malonyl-coA into products. Several examples of these non-limiting products are shown below in Table 1A. Any of the strains discussed in the specification that increase flux through malonyl-coA can be used to produce these products. Hexaketide pyrone can be made by expressing a hexaketide pyrone synthase from either Aloe arborescens or Plumbago indica. Octaketide 4b pyrone can be made by expressing an octaketide 4b pyrone synthase from Aloe arborescens. Octaketide can be made by expressing an octaketide synthase from Hypericum perforatum. Pentaketide chromone can be made by expressing a pentaketide chromone synthase from Aloe arborescens. 3-hydroxypropionic acid can be made by expressing a malonyl-coA reductase and 3-hydroxypropionic acid dehydrogenase from various sources.

**Table 1A: Products requiring malonyl-coA precursors alone**

| **Chemical product** | **Biosynthesis** |
|---|---|
| 3,5,7,9,11,13,15-hepta-oxo-hexadecanoyl-[PKS acp] | 8 malonyl-CoA+a polyketide synthase containing an [acp] domain-> a 3,5,7,9,11,13,15-hepta-oxo-hexadecanoyl-[PKS acp] + 8 CO2 + 8 coenzyme A |
| octoketide 4b | 8 malonyl-CoA+ 4 H2 = octoketide 4b + 8 CO2 + 8 coenzyme A+ H2O + H+ |
| octoketide | 8 malonyl-CoA+ 4 H2 = octoketide + 8 CO2 + 8 coenzyme A+ H2O + H+ |
| heptaketide pyrone | 7 malonyl-CoA+ 4 H2 = heptaketide pyrone+ 7 CO2 + 7 coenzyme A+ H2O + 2 H+ |
| hexaketide pyrone | 6 malonyl-CoA+ 3 H2 = hexaketide pyrone+ 6 CO2 + 6 coenzyme A+ H2O + H+ |
| pentaketide chromone | 5 malonyl-CoA+ 5 H+ = 5,7-dihydroxy-2-methylchromone + 5 CO2+ 5 coenzyme A+ H2O |
| 3-hydroxypropionate | malonyl-CoA+ NADPH + H+ =malonate semialdehyde + NADP+ +coenzyme A |

Furthermore, numerous polyketide products can be made from malonyl-coA precursors in combination with acetyl-coA precursors. As acetyl-coA is a precursor of malonyl-coA, no additional modifications to a malonyl-coA producing strain are needed. Products can be made by expressing enzyme functions to convert malonyl-coA in addition to acetyl-coA into products. Several examples of these non-limiting products are shown below in Table 1B. Any of the strains discussed in the specification that increase flux through malonyl-coA can be used to produce these products. 6-hydroxymellein can be made by expressing a 6-hydroxymellein synthase from either Aloe or Daucus carota. Aloesone can be made by expressing an aloesone synthase from either Aloe arborescens or Rheum palmatum. Olivetolic acid can be made by expressing an olivetolic acid synthase. Naphthylisoquinoline alkaloid precursor (a precursor to plumbagine) can be made by expressing a polyketide pyrone synthase such as PKS G-11468 from Plumbago indica. 6-methylsalicylate can be made by expressing a 6-methylsalicylate synthase from Penicillium species. Triacetic acid lactone can be made by expressing a triacetic acid lactone synthase.

**Table 1B: Products requiring malonyl-coA and acetyl-coA precursors**

| **Chemical product** | **Biosynthesis** |
|---|---|
| 6-hydroxymellein | acetyl-CoA + 4 malonyl-CoA+ NADPH + 5 H+ = 6-hydroxymellein + 4 CO2 + NADP+ + 5 coenzyme A+ H2O |
| aloesone | acetyl-CoA + 6 malonyl-CoA+ 6 H+ = aloesone + 7 CO2 + 7 coenzyme A+ H2O |
| olivetolic acid (cannabinoid) | acetyl-CoA + 5 malonyl-CoA+ 12 H+ = olivetolic acid+ 5 CO2 + 6 coenzyme A+ 2 H20 |
| plumbagin | acetyl-CoA + 5 malonyl-CoA+ 3 H2+ H+ = naphthylisoquinoline alkaloid precursor+ 6 CO2+ 6 coenzyme A + 2 H20, acetyl-CoA + 5 malonyl-CoA+ 2 H2 = hexaketide pyrone+ 5 CO2+ 6 coenzyme A+ H2 0 |
| 6-methylsalicylate | acetyl-CoA + 3 malonyl-CoA+ NADPH + 3 H+ = 6-methylsalicylate + 3 CO2 + NADP+ + 4 coenzyme A |
| triacetic acid lactone | acetyl-CoA + 2 malonyl-CoA + H⁺ → triacetic acid lactone + 2 CO₂ + 3 coenzyme A |

Furthermore, numerous polyketide products can be made from malonyl-coA precursors in combination with isobutyryl-coA precursors. It is described elsewhere how to construct a genetically modified organism to produce butyryl-coA or isobutyryl-coA from malonyl-coA. Products can be made by expressing enzyme functions to convert malonyl-coA in addition to isobutyryl-coA into products. Several examples of these non-limiting products are shown below in Table 1C. Any of the strains discussed in the specification that increase flux through malonyl-coA and also produce isobutyryl-coA can be used to produce these products. Phlorisobutyrophenone can be produced by expressing a phlorisobutyrophenone synthase from Humulus lupus.

**Table 1C: Products requiring malonyl-coA and isobutyryl-coA precursors**

| **Chemical product** | **Biosynthesis** |
|---|---|
| cohumulone | 3 malonyl-CoA+ isobutyryl-CoA + 3 H+ = phlorisobutyrophenone + 3 CO2 + 4 coenzyme A |
| hyperforin | 3 malonyl-CoA+ isobutyryl-CoA + 3 H+ = phlorisobutyrophenone + 3 CO2 + 4 coenzyme A |

In addition, numerous polyketide products can be made from malonyl-coA precursors in combination with coumaryl-coA or cinnamoyl-coA precursors. Coumaryl-coA or cinnamoyl-coA production in genetically modified organisms has been described, for example, in Yohei Katsuyama et al.("Production of curcuminoids by Escherichia coli carrying an artificial biosynthesis pathway", Microbiology (2008), 154, 2620-2628). These genetic modifications can be made in combination with modifications described herein to increase malonyl-coA production. Products can be made by expressing enzyme functions to convert malonyl-coA in addition to coumaryl-coA or cinnamoyl-coA into products. Several examples of these non-limiting products are shown below in Table ID. p-coumaroyltriacetic acid lactone can be made by expressing a p-coumaroyltriacetic acid lactone synthase from humulus lupulus or rubeus idaeus. Naringenin chalcone can be made by expressing a naringenin chalcone synthase from either humulus lupulus or arabidopsis thaliana. Isoliquiritigenin or other flavonoids can be made by expressing a chalcone reductase from numerous sources. 4-hydroxybenzalacetone can be made by expressing a 4-hydroxybenzalacetone synthase from rubus idaeus. Pinocembrin chalcone can be made by expressing a pinocembrin chalcone synthase from pinus densiflora. Resvertrol can be made by expressing a resveratrol or stilbene synthase such as RS G-528 from arachis hypogaea or STS G-230 from rheum tatricum. Bis-noryangonin can be produced by expressing a styrylpyrone synthase such as from equisetum arvense. P-coumaroyltriacetate can be made by expressing a p-coumaroyltriacetic acid lactone synthase from hydrangea macrophylla. Pinosylvin can be made by expressing one of numeroues stilbene synthases such as stilbene synthase PDSTS2 from pinus denisflora.

**Table ID: Products requiring malonyl-coA and coumaryl-coA OR cinnamoyl-coA precursors**

| **Chemical product** | **Biosynthesis** |
|---|---|
| p-coumaroyltriacetic acid lactone (aromatic polyketide) | 4-coumaroyl-CoA + 3 malonyl-CoA+ 2 H+ = p-coumaroyltriacetic acid lactone+ 3 CO2 + 4 coenzyme A |
| naringenin chalcone (flavonoid) | 4-coumaroyl-CoA + 3 malonyl-CoA+ 3 H+ = naringenin chalcone+ 3 CO2 + 4 coenzyme A |
| isoliquiritigenin (flavonoid) | 4-coumaroyl-CoA + 3 malonyl-CoA+ NADPH + 4 H+ = isoliquiritigenin + 3 CO2 + NADP+ + 4 coenzyme A+ H20, |
| pinocembrin chalcone (pinobanksin) | 3 malonyl-CoA+ (E)-cinnamoyl-CoA + 3 H+ = pinocembrin chalcone+ 3 CO2+ 4 coenzyme A |
| 4 hydroxybenzalacetone (raspberry ketone) | 4-coumaroyl-CoA + malonyl-CoA+ H 20 + H+ = 4 hydroxybenzalacetone + 2 CO2+ 2 coenzyme A |
| p-coumaroyltriacetate (resveratrol) | 4-coumaroyl-CoA + 3 malonyl-CoA+ H2O + 2 H+ = 3 CO2+ p-coumaroyltriacetate + 4 coenzyme A |
| bis-noryangonin (resveratrol) | 4-coumaroyl-CoA + 2 malonyl-CoA+ H+ = bis-noryangonin + 2 CO2+ 3 coenzyme A |
| resveratrol | 4-coumaroyl-CoA + 3 malonyl-CoA+ 3 H+ = resveratrol + 4 CO2+ 4 coenzyme A |
| xanthohumol | 4-coumaroyl-CoA + 3 malonyl-CoA+ 3 H+ = naringenin chalcone+ 3 CO2 + 4 coenzyme A |
| pinosylvin | (E)-cinnamoyl-CoA + 3 malonyl-CoA+ 3 H+ = 4 CO2+ pinosylvin + 4 coenzyme A |

Numerous polyketide products can also be made from malonyl-coA precursors in combination with isovaleryl-coA precursors. As isovaleryl-coA is a product of leucine degradation pathways, many metabolic pathways are known to produce this product. These genetic modifications can be made in combination with modifications described herein to increase malonyl-coA production. Products can be made by expressing enzyme functions to convert malonyl-coA in addition to isovaleryl-coA into products. Several examples of these non-limiting products are shown below in Table IE. 6-isobutyl-4-hydroxy-2-pyrone can be made by expressing a 6-isobutyl-4-hydroxy-2-pyrone synthase from Humulus lupulus. 6-(4-methyl-2-oxopentyl)-4-hydroxy-2-pyrone can be made by expressing a 6-(4-methyl-2-oxopentyl)-4-hydroxy-2-pyrone synthase from Humulus lupulus.

**Table IE: Products requiring Malonyl-coA and isovaleryl-coA precursors**

| **Chemical product** | **Biosynthesis** |
|---|---|
| 6-isobutyl-4-hydroxy-2-pyrone | isovaleryl-CoA + 2 malonyl-CoA+ H+ = 6-isobutyl-4-hydroxy-2-pyrone + 2 CO2 + 3 coenzyme A |
| 6-(4-methyl-2-oxopentyl)-4-hydroxy-2-pyrone | isovaleryl-CoA + 3 malonyl-CoA+ 2 H+ = 6-(4-methyl-2-oxopentyl)-4-hydroxy-2-pyrone + 3 CO2 + 4 coenzyme A |

In addition, numerous polyketide products can be made from malonyl-coA precursors in combination with benzoyl-coA or 3-hydroxybenzoyl-coA precursors. Benzoyl-coA or 3-hydroxybenzoyl-coA production in genetically modified organisms has been described. In particular, one route to produce benzoyl-coA is from benzoate by expressing a benzoate-coA ligase such as from Hypericum androsaemum. In addition, 3-hydroxybenzoyl-coA can be made from 3-hydroxybenzoate by expressing a 3-hydroxybenzoate-coA ligase such as from Hypericum androsaemum. Products can be made by expressing enzyme functions to convert malonyl-coA in addition to benzoyl-coA or 3-hydroxybenzoyl-coA into products. Several examples of these non-limiting products are shown below in Table IF. 2,3',4,6-tetrahydroxybenzophenone can be made by expressing a benzophenone synthase from Centauraium erythraea. 2,4,6-trihydroxybenzophenone can be made by expressing a benzophenone synthase such as AF352395 from Hypericum androsaemum. 3,5-dihydroxybiphenyl can be made be expressing a 3,5-dihydroxybiphenyl synthase.

**Table IF: Products requiring Malonyl-coA and benzoyl-coA OR 3-hydroxy-benzoyl-coA precursors**

| **Chemical product** | **Biosynthesis** |
|---|---|
| 2,3',4,6-tetrahydroxybenzophenone (tetrahydroxyxanthone) | 3-hydroxybenzoyl-CoA + 3 malonyl-CoA+ 3 H+ = 3 CO2 + 2,3',4,6-tetrahydroxybenzophenone + 4 coenzyme A |
| 2,4,6-trihydroxybenzophenone (tetrahydroxyxanthone) | 3 malonyl-CoA+ benzoyl-CoA + 3 H+ = 2,4,6-trihydroxybenzophenone + 3 CO2 + 4 coenzyme A |
| 3,5-dihydroxybiphenyl | 3 malonyl-CoA+ benzoyl-CoA + 3 H+ = 3,5-dihydroxybiphenyl + 4 CO2 + 4 coenzyme A |

In addition to the above, numerous polyketide products can be made from malonyl-coA precursors in combination with other secondary metabolite precursors. For example, (2S,5S)-carboxymethylproline, the precursor to carbapenem can be made from malonyl-coA and (S)-1-pyrroline-5-carboxylate by expressing a carboxymethylproline synthase from Pectobacterium carotovorum. The (S)-1-pyrroline-5-carboxylate is a precursor to the amino acid proline, whose production is known. 1,3-dihydroxy-N-methylacridone can be made from malonyl-coA and the additional precursor N-methylanthraniloyl-CoA by expressing an acridone synthase such as ACSII from Ruta graveolens. N-methylanthraniloyl-CoA can be made metabolically from the aromatic amino acid precursor chorismate by expressing an anthranilate synthase such as Ruta graveolens to convert chorismate to anthranilate, additionally expressing an anthranilate N-methyltransferase from Ruta graveolens to produce N-methylanthranilate from anthranilate and finally expressing a N-methylanthranilate-coA ligase to produce N-methylanthraniloyl-coA from N-methylanthranilate. Several examples of non-limiting products are shown below in Table 1G.

**Table 1G: Products requiring Malonyl-coA and other secondary metabolites.**

| **Chemical product** | **Biosynthesis** |
|---|---|
| (2S,5S)-carboxymethylproline ((5R)-carbapenem) | (S)-1-pyrroline-5-carboxylate + malonyl-CoA+ H2O + H+ = (2S,5S)-carboxymethylproline + CO2 +coenzyme |
| 1,3-dihydroxy-N-methylacridone (acridone alkaloid) | N-methylanthraniloyl-CoA + 3 malonyl-CoA= 3 CO2 + 1,3-dihydroxy-N-methylacridone + 4 coenzyme A |

In addition to the above, several malonyl-coA conjugate products can be made by supplying a cell with an enzyme expressing a malonyl-transferase to add a malonyl group to a larger molecule. For example, an isoflavone-7-O-glucoside-6-O-malonyl-transferase can add malonyl group to biochanin glucosides to produce biochanin glucoside malonates, an isoflavone-7-O-glucoside-6-O-malonyl-transferase can add malonyl group to biochanin glucosides to produce biochanin glucoside malonates, or alternatively add a malonyl group to glucosyl-apegenins to produce apegenin malonyl-glucosides. Table 1H gives the reaction of several malonyltransferases and substrates and products. Enzymes that can be expressed to perform these conversions can be identified in numerous databases in the art such as <<www.metacyc.org>>.

**Table 1H: Products requiring Malonyl-coA and malonyltransferases**

| **Chemical product** | **Biosynthesis** |
|---|---|
| apigenin 7-0(6-malonyl-B-D-glucoside) (apigenin glycosides) | 7 0 B D glucosyl-apigenin+malonyl-CoA=apigenin 7-0(6-malonyl-B-D-glucoside)+coenzyme A |
| biochanin A 7 0 glucoside-6"-malonate (biochanin A conjugates interconversion) | biochanin A 7 0 glucoside + malonyl CoA + ATP + H2O = biochanin A 7 0 glucoside-6"-malonate +AMP+ diphosphate + coenzyme A + 2 H+ |
| maackiain 3 0 glucoside-6"-malonate (maackiain conjugates interconversion) | (-)-maackiain 3 0 glucoside + malonyl CoA + ATP + H2O = ( ) maackiain 3 0 glucoside-6"-malonate +AMP + diphosphate + coenzyme A + 2 H+ |
| medicarpin 3 0 glucoside-6"-malonate (medicarpin conjugates | (-)-medicarpin 3 0 glucoside + malonyl CoA + ATP + H2O = ( ) medicarpin 3 0 glucoside-6"-malonate + AMP + diphosphate + coenzyme A |
| interconversion) | + 2 H+ |
| Malonyldaidzin (daidzein conjugates interconversion) | daidzin + malonyl-CoA+ ATP + H2O = malonyldaidzin +AMP+ diphosphate+ coenzyme A+ 2 H+ |
| formononetin 7 0 glucoside-6"-malonate (formononetin conjugates interconversion) | ononin + malonyl-CoA+ ATP + H2O = formononetin 7 0 glucoside-6"-malonate +AMP+ diphosphate+ coenzyme A + 2 H+ |
| Malonylgenistin (genistein conjugates interconversion) | genistin + malonyl-CoA+ ATP + H2O = malonylgenistin +AMP+ diphosphate+ coenzyme A+ 2 H+ |
| pelargonidin 3 0 (6 0 malonyl B D glucoside) (pelargonidin conjugates) | pelargonidin 3 0 B D - glucoside + malonyl-CoA+ H+ = pelargonidin 3 0 (6 0 malonyl B D glucoside) + coenzyme A |
| salvianin | monodemalonylsalvianin + malonyl-CoA+ H+ = salvianin +coenzyme A, bisdemalonylsalvianin + malonyl-CoA+ H+ = monodemalonylsalvianin +coenzyme A |
| malonylshisonin | shisonin + malonyl-CoA+ H+ = malonylshisonin +coenzyme A |
| malonylshisonin | superpathway of anthocyanin biosynthesis (from cyanidin and cyanidin 3-0-glucoside) : shisonin + malonyl-CoA+ H+ = malonylshisonin +coenzyme A |
| delphinidin 3-0-(6"-0-malonyl)-B-glucoside (ternatin C5) | delphinidin 3 0 B D - glucoside + malonyl-CoA= delphinidin 3-0-(6"-0-malonyl)-B-glucoside +coenzyme A |
| N - Malonylanthranilate | anthranilate+ malonyl-CoA= N-Malonylanthranilate +coenzyme A |
| N-(3,4-dichlorophenyl)-malonamate | 3,4-dichloroaniline + malonyl-CoA= N-(3,4-dichlorophenyl)-malonamate +coenzyme A |
| anthocyanidin 3 0 (6 0 malonyl B D glucoside) | malonyl-CoA+an anthocyanidin 3 0 B D glucoside =an anthocyanidin 3 0 (6 0 malonyl B D glucoside) + coenzyme A |
| 7 hydroxyflavone 7 0 (6 malonyl-B-D-glucoside) | 7 0 B D glucosyl 7 hydroxyflavone + malonyl CoA = 7 hydroxyflavone 7 0 (6 malonyl-B-D-glucoside) + coenzyme A |

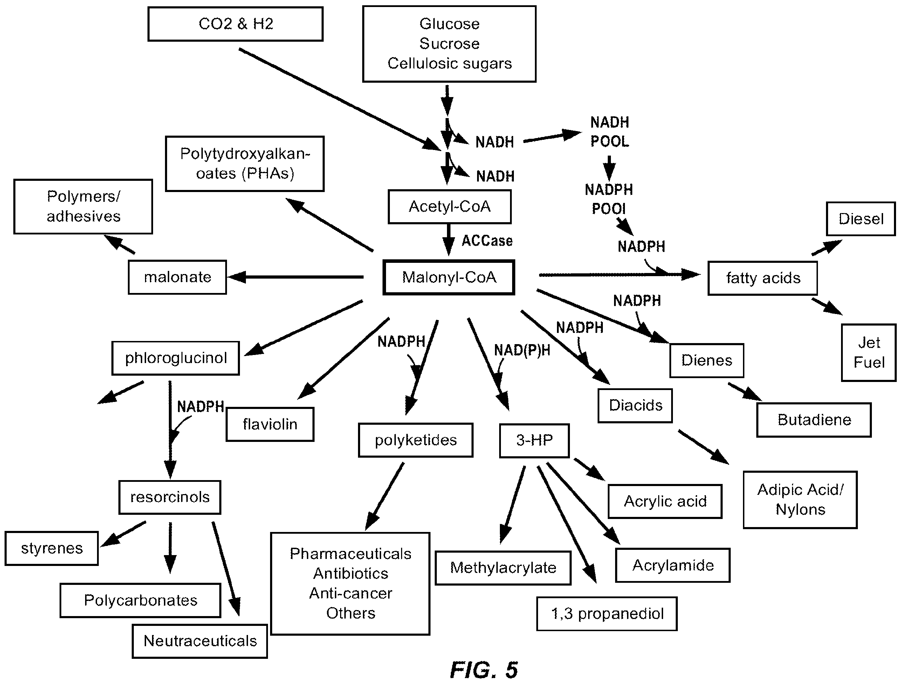

Also provided herein, in addition to 3-HP and chemcials and products made from it (including methylacrylate), and various polyketides provided in the tables, are teachings and other disclosures variously directed to production of phloroglucinol, resorcinol, malonic acid, diacids, dienes, flaviolin, malonate, chalcones, pyrones, type I, II and III polyketides more generally and other chemicals and other products made from these. FIG. 5 schematically depicts bioconversions from exemplary carbon sources to these various chemical products. It is noted that in various embodiments the invention may be applied to produce fatty acids and subsequently to obtain products made from these, such as jet fuel and diesel. In various embodiments, this is achieved by use of a pathway comprising an elongase as disclosed herein. Any of the chemicals described or otherwise disclosed herein, including in the figures, may be a selected chemical product, or a chemical product of interest. Also, any grouping, including any sub-group, of the above listing may be considered what is referred to by "selected chemical product," "chemical product of interest," and the like. For any of these chemical products a microorganism may inherently comprise a biosynthesis pathway to such chemical product and/or may require addition of one or more heterologous nucleic acid sequences to provide or complete such a biosynthesis pathway, in order to achieve a desired production of such chemical product.

U.S. Patent Publication US2009/0111151 A1, describes illustrative polyketide synthase (PKS) genes and corresponding enzymes that can be utilized in the construction of genetically modified microorganisms and related methods and systems. Any of these may be employed in the embodiments of the present invention, such as in microorganisms that produce polyketides and also comprise modifications to reduce activity of fatty acid synthase enzymatic conversions.

U.S. Patent Publication US2011/0171702 A1, describes production of 2-hydroxyisobutyric acid (2-HIBA) and corresponding enzymes that can be utilized in the construction of genetically modified microorganisms and related methods and systems. Any of these may be employed in the embodiments of the present invention, such as in microorganisms that produce 2-HIBA and also comprise modifications to reduce activity of fatty acid synthase enzymatic conversions.

U.S. Patent Publication US2010/0291644 A1, describes the production of 3-hydroxyisobutyric acid (3-HIBA) and corresponding enzymes that can be utilized in the construction of genetically modified microorganisms and related methods and systems and the dehydration to methacrylic. Any of these may be employed in the embodiments of the present invention, such as in microorganisms
that produce 3-HIBA and/or polyhydroxyalkanoates and also comprise modifications to reduce activity of fatty acid synthase enzymatic conversions.

U.S. Patent Publication US2008/0274523 A1, describes illustrative isoprenoid synthesis genes and corresponding enzymes that can be utilized in the construction of genetically modified microorganisms and related methods and systems. Any of these may be employed in the embodiments of the present invention, such as in microorganisms that produce mevalonate or other isoprenoids and also comprise modifications to reduce activity of fatty acid synthase enzymatic conversions.

U.S. Patent number 6593116, describes polyhydroxyalkanoate synthesis and corresponding enzymes that can be utilized in the construction of genetically modified microorganisms and related methods and systems. Any of these may be employed in the embodiments of the present invention, such as in microorganisms that produce polyhydroxybutyrate or other polyhydroxyalkanoates and also comprise modifications to reduce activity of fatty acid synthase enzymatic conversions.

Particular polyketide chemical products which are considered to be chemical product biosynthesis candidates under the present invention include the following: Amphotericin B; antimycin A; brefeldin A; candicidin; epothilones; erythromycin; azithromycin; clarithromycin; erythromycin estolate; erythromycin ethylsuccinate; roxithromycin; ivermectin; josamycin; ketolides; leucomycins; kitasamycin; spiramycin; lovastatin; lucensomycin; macrolides; maytansine; mepartricin; miocamycin; natamycin; nystatin; oleandomycin; troleandomycin; oligomycins; rutamycin; sirolimus; tacrolimus; tylosin; oleandomycin; deoxyoleandolide; narbonolide; narbomycin; and pikromycin.

### V. GENETIC MODIFICATIONS

Embodiments of the present invention may result from introduction of an expression vector into a host microorganism, wherein the expression vector contains a nucleic acid sequence coding for an enzyme that is, or is not, normally found in a host microorganism.

The ability to genetically modify a host cell is essential for the production of any genetically modified (recombinant) microorganism. The mode of gene transfer technology may be by electroporation, conjugation, transduction, or natural transformation. A broad range of host conjugative plasmids and drug resistance markers are available. The cloning vectors are tailored to the host organisms based on the nature of antibiotic resistance markers that can function in that host. Also, as disclosed herein, a genetically modified (recombinant) microorganism may comprise modifications other than via plasmid introduction, including modifications to its genomic DNA.

Those skilled in the art will understand that the genetic alterations, including metabolic modifications exemplified herein are described with reference to a suitable source organism such as E. coli, yeast, or other organisms disclosed herein and their corresponding metabolic enzymatic reactions or a suitable source organism for desired genetic material such as genes encoding enzymes for their corresponding metabolic enzymatic reactions. However, given the complete genome sequencing of a wide variety of organisms and the high level of skill in the area of genomics, those skilled in the art will readily be able to apply the teachings and guidance provided herein to essentially all other microorganisms. For example, the E. coli metabolic alterations exemplified herein can readily be applied to other species by incorporating the same or analogous encoding nucleic acid from species other than the referenced species. Such genetic alterations include, for example, genetic alterations of species homo logs, in general, and in particular, orthologs, paralogs or nonorthologous gene displacements.

An ortholog is a gene or genes that are related by vertical descent and are responsible for substantially the same or identical functions in different organisms. Genes are related by vertical descent when, for example, they share sequence similarity of sufficient amount to indicate they are homologous, or related by evolution from a common ancestor. Genes can also be considered orthologs if they share three-dimensional structure but not necessarily sequence similarity, of a sufficient amount to indicate that they have evolved from a common ancestor to the extent that the primary sequence similarity is not identifiable. Genes that are orthologous can encode proteins with sequence similarity of about 25% to 100% amino acid sequence identity. Genes encoding proteins sharing an amino acid similarity less that 25% can also be considered to have arisen by vertical descent if their three-dimensional structure also shows similarities. Members of the serine protease family of enzymes, including tissue plasminogen activator and elastase, are considered to have arisen by vertical descent from a common ancestor.

Orthologs include genes or their encoded gene products that through, for example, evolution, have diverged in structure or overall activity. For example, where one species encodes a gene product exhibiting two functions and where such functions have been separated into distinct genes in a second species, the three genes and their corresponding products are considered to be orthologs. An example of orthologs exhibiting separable activities is where distinct activities have been separated into distinct gene products between two or more species or within a single species. A specific example is the separation of elastase proteolysis and plasminogen proteolysis, two types of serine protease activity, into distinct molecules as plasminogen activator and elastase.

In contrast, paralogs are homologues related by, for example, duplication followed by evolutionary divergence and have similar or common, but not identical functions. Paralogs can originate or derive from, for example, the same species or from a different species. For example, microsomal epoxide hydrolase (epoxide hydrolase I) and soluble epoxide hydrolase (epoxide hydrolase II) can be considered paralogs because they represent two distinct enzymes, co-evolved from a common ancestor, that catalyze distinct reactions and have distinct functions in the same species. Paralogs are proteins from the same species with significant sequence similarity to each other suggesting that they are homologous, or related through co-evolution from a common ancestor. Groups of paralogous protein families include HipA homologs, luciferase genes, peptidases, and others. A nonorthologous gene displacement is a nonorthologous gene from one species that can substitute for a referenced gene function in a different species. Substitution includes, for example, being able to perform substantially the same or a similar function in the species of origin compared to the referenced function in the different species. Although generally, a nonorthologous gene displacement will be identifiable as structurally related to a known gene encoding the referenced function, less structurally related but functionally similar genes and their corresponding gene products nevertheless will still fall within the meaning of the term as it is used herein. In some cases, functional similarity requires at least some structural similarity in the active site or binding region of a nonorthologous gene compared to a gene encoding the function sought to be substituted. Therefore, a nonorthologous gene includes, for example, a paralog or an unrelated gene.

Therefore, in identifying and designing a genetically modified microorganism of the present invention, those skilled in the art will understand with applying the teaching and guidance provided herein to a particular species that the identification of genetic modifications can include identification and inclusion or inactivation or other modification of orthologs. To the extent that paralogs and/or nonorthologous gene displacements are present in the referenced microorganism that encode an enzyme catalyzing a similar or substantially similar metabolic reaction, those skilled in the art also can utilize these evolutionarily related genes. Orthologs, paralogs and nonorthologous gene displacements can be determined by methods well known to those skilled in the art. For example, inspection of nucleic acid or amino acid sequences for two polypeptides will reveal sequence identity and similarities between the compared sequences. Based on such similarities, one skilled in the art can determine if the similarity is sufficiently high to indicate the proteins are related through evolution from a common ancestor. Algorithms well known to those skilled in the art, such as Align, BLAST, Clustal Wand others compare and determine a raw sequence similarity or identity, and also determine the presence or significance of gaps in the sequence which can be assigned a weight or score. Such algorithms also are known in the art and are similarly applicable for determining nucleotide sequence similarity or identity. Parameters for sufficient similarity to determine relatedness are computed based on well known methods for calculating statistical similarity, or the chance of finding a similar match in a random polypeptide, and the significance of the match determined. A computer comparison of two or more sequences can, if desired, also be optimized visually by those skilled in the art. Related gene products or proteins can be expected to have a high similarity, for example, 25% to 100% sequence identity. Proteins that are unrelated can have an identity which is essentially the same as would be expected to occur by chance, if a database of sufficient size is scanned(about 5%). Sequences between 5% and 24% sequence identity may or may not represent sufficient homology to conclude that the compared sequences are related. Additional statistical analysis to determine the significance of such matches given the size of the data set can be carried out to determine the relevance of these sequences.

Exemplary parameters for determining relatedness of two or more sequences using the BLAST algorithm, for example, can be as set forth below. Briefly, amino acid sequence alignments can be performed using BLASTP version 2.0.8 (Jan-05-1999) and the following parameters: Matrix: 0 BLOSUM62; gap open: 11; gap extension: 1; x_dropoff: 50; expect: 10.0; wordsize: 3; filter: on. Nucleic acid sequence alignments can be performed using BLASTN version 2.0.6 (Sept- 16- 1998) and the following parameters: Match: 1; mismatch: -2; gap open: 5; gap extension: 2; x_dropoff: 50; expect: 10.0; wordsize: 11; filter: off. Those skilled in the art will know what modifications can be made to the above parameters to either increase or decrease the stringency of the comparison, for example, and determine the relatedness of two or more sequences. Through such comparisons and analyses, one skilled in the art may be able to obtain a desired polypeptide in a particular species that functions similarly to a polypeptide (enzyme) disclosed herein, and/or a functional variant that possesses a desired enzymatic activity.

Also, in various embodiments polypeptides, such as enzymes, obtained by the expression of the any of the various polynucleotide molecules (i.e., nucleic acid sequences) of the present invention may have at least approximately 50%, 60%, 70%, 80%, 90%, 95%, 96%, 97%, 98%, 99% or 100% identity to one or more amino acid sequences encoded by the genes and/or nucleic acid sequences described herein.

Embodiments of the present invention may involve various nucleic acid sequences, such as heterologous nucleic acid sequences introduced into a cell's genome or may be episomal, and also encompasses antisense nucleic acid molecules, i.e., molecules which are complementary to a sense nucleic acid encoding a protein, e.g., complementary to the coding strand of a doublestranded eDNA molecule or complementary to an mRNA sequence.

For various embodiments of the invention the genetic manipulations may be described to include various genetic manipulations, including those directed to change regulation of, and therefore ultimate activity of, an enzyme or enzymatic activity of an enzyme identified in any of the respective pathways. Such genetic modifications may be directed to transcriptional, translational, and post-translational modifications that result in a change of enzyme activity and/or selectivity under selected and/or identified culture conditions and/or to provision of additional nucleic acid sequences such as to increase copy number and/or mutants of an enzyme related to 3-HP production. Specific methodologies and approaches to achieve such genetic modification are well known to one skilled in the art, and include, but are not limited to: increasing expression of an endogenous genetic element; decreasing functionality of a repressor gene; introducing a heterologous genetic element; increasing copy number of a nucleic acid sequence encoding a polypeptide catalyzing an enzymatic conversion step to produce 3-HP; mutating a genetic element to provide a mutated protein to increase specific enzymatic activity; over-expressing; under-expressing; over-expressing a chaperone; knocking out a protease; altering or modifying feedback inhibition; providing an enzyme variant comprising one or more of an impaired binding site for a repressor and/or competitive inhibitor; knocking out a repressor gene; evolution, selection and/or other approaches to improve mRNA stability as well as use of plasmids having an effective copy number and promoters to achieve an effective level of improvement. Random mutagenesis may be practiced to provide genetic modifications that may fall into any of these or other stated approaches. The genetic modifications further broadly fall into additions (including insertions), deletions (such as by a mutation) and substitutions of one or more nucleic acids in a nucleic acid of interest. In various embodiments a genetic modification results in improved enzymatic specific activity and/or turnover number of an enzyme. Without being limited, changes may be measured by one or more of the following: KM; Kam; and In various embodiments, to function more efficiently, a microorganism may comprise one or more gene deletions. For example, in E. coli, the genes encoding the lactate dehydrogenase (ldhA), phosphate acetyltransferase (pta), pyruvate oxidase (poxB), and pyruvate-formate lyase (pflB) may be disrupted, including deleted. Such gene disruptions, including deletions, are not meant to be limiting, and may be implemented in various combinations in various embodiments. Gene deletions may be accomplished by mutational gene deletion approaches, and/or starting with a mutant strain having reduced or no expression of one or more of these enzymes, and/or other methods known to those skilled in the art. Gene deletions may be effectuated by any of a number of known specific methodologies, including but not limited to the RED/ET methods using kits and other reagents sold by Gene Bridges (Gene Bridges GmbH, Dresden, Germany, <<www.genebridges.com>>). More particularly as to the latter method, use of Red/ET recombination, is known to those of ordinary skill in the art and described in U.S. Patent Nos. 6,355,412 and 6,509,156, issued to Stewart et al. Material and kits for such method are available from Gene Bridges (Gene Bridges GmbH, Dresden, Germany, <<www.genebridges.com>>), and the method may proceed by following the manufacturer's instructions. The method involves replacement of the target gene by a selectable marker via homologous recombination performed by the recombinase from X-phage. The host organism expressing k-red recombinase is transformed with a linear DNA product coding for a selectable marker flanked by the terminal regions (generally -50 bp, and alternatively up to about -300 bp) homologous with the target gene. The marker could then be removed by another recombination step performed by a plasmid vector carrying the FLP-recombinase, or another recombinase, such as Cre. Targeted deletion of parts of microbial chromosomal DNA or the addition of foreign genetic material to microbial chromosomes may be practiced to alter a host cell's metabolism so as to reduce or eliminate production of undesired metabolic products. This may be used in combination with other genetic modifications such as described herein in this general example. In this detailed description, reference has been made to multiple embodiments and to the accompanying drawings in which is shown by way of illustration specific exemplary embodiments in which the invention may be practiced. These embodiments are described in sufficient detail to enable those skilled in the art to practice the invention, and it is to be understood that modifications to the various disclosed embodiments may be made by a skilled artisan. Further, for 3-HP production, such genetic modifications may be chosen and/or selected for to achieve a higher flux rate through certain enzymatic conversion steps within the respective 3-HP production pathway and so may affect general cellular metabolism in fundamental and/or major ways. It has long been recognized in the art that some amino acids in amino acid sequences can be varied without significant effect on the structure or function of proteins. Variants included can constitute deletions, insertions, inversions, repeats, and type substitutions so long as the indicated enzyme activity is not significantly adversely affected. Guidance concerning which amino acid changes are likely to be phenotypically silent can be found, inter alia, in Bowie, J. U., et al., "Deciphering the Message in Protein Sequences: Tolerance to Amino Acid Substitutions," Science 247:1306-1310 (1990). Further, it will be appreciated that amino acid "homology" includes conservative substitutions, i.e. those that substitute a given amino acid in a polypeptide by another amino acid of similar characteristics. Recognized conservative amino acid substitutions comprise (substitutable amino acids following each colon of a set): ala:ser; arg:lys; asn:gln or his; asp:glu; cys:ser; gln:asn; glu:asp; gly:pro; his:asn or gln; ile:leu or val; leu:ile or val; lys: arg or gln or glu; met:leu or ile; phe:met or leu or tyr; ser:thr; thr:ser; trp:tyr; tyr:trp or phe; val:ile or leu. Also generally recognized as conservative substitutions are the following replacements: replacements of an aliphatic amino acid such as Ala, Val, Leu and Ile with another aliphatic amino acid; replacement of a Ser with a Thr or vice versa; replacement of an acidic residue such as Asp or Glu with another acidic residue; replacement of a residue bearing an amide group, such as Asn or Gln, with another residue bearing an amide group; exchange of a basic residue such as Lys or Arg with another basic residue; and replacement of an aromatic residue such as Phe or Tyr with another aromatic residue.

For all polynucleotide (nucleic acid) and polypeptide (amino acid) sequences provided herein, it is appreciated that conservatively modified variants of these sequences are included, and are within the scope of the invention in its various embodiments. Conservatively modified variant include amino acid conservative substitutions such as those described in the previous paragraph as well as modified polynucleotide sequences such as based on codon degeneracy described in the following paragraph and table. Further, the following table also provides characteristics of amino acids that provide for additional conservative substitutions that may fall within the scope of conservatively modified variants, based on commonly shared properties of particular amino acids. Also, in various embodiments deletions and/or substitutions at either end, or in other regions, of a polynucleotide or polypeptide may be practiced for sequences, based on the present teachings and knowledge of those skilled in the art, and remain within the scope of conservatively modified variants.

Accordingly, functionally equivalent polynucleotides and polypeptides (functional variants), which may include conservatively modified variants as well as more extensively varied sequences, which are well within the skill of the person of ordinary skill in the art, and microorganisms comprising these, also are within the scope of various embodiments of the invention, as are methods and systems comprising such sequences and/or microorganisms. In various embodiments, nucleic acid sequences encoding sufficiently homologous proteins or portions thereof are within the scope of the invention. More generally, nucleic acids sequences that encode a particular amino acid sequence employed in the invention may vary due to the degeneracy of the genetic code, and nonetheless fall within the scope of the invention. The following table provides a summary of similarities among amino acids, upon which conservative substitutions may be based, and also various codon redundancies that reflect this degeneracy.

**TABLE 2**

| **Amino Acid** | **Relationships** | **DNA codons** |
|---|---|---|
| Alanine | - | GCT, GCC, GCA, GCG |
| Proline | N | CCT, CCC, CCA, CCG |
| Valine | N, Ali | GTT, GTC, GTA, GTG |
| Leucine | N, Ali | |
| Isoleucine | N, Ali | ATT, ATC, ATA |
| Methionine | N | ATG |
| Phenylalanine | N, Aro | |
| Tryptophan | | TTT, TTC |
| Glycine | N | TGG |
| Serine | PU | |
| Threonine | PU | |
| Asparagine | PU | ACT, ACC, ACA, ACG |
| Glutamine | PU, Ami | AAT, AAC |
| Cysteine | PU, Ami | CAA, CAG |
| Aspartic acid | | TGT, TGC |
| Glutamic acid | | GAT, GAC |
| Arginine | NEG, A | GAA, GAG |
| Lysine | POS, B | |
| Histidine | POS, B | AAA, AAG |
| Tyrosine | POS | CAT, CAC |
| *Stop Codons* | Aro | TAT, TAC |
| | | TAA, TAG, TGA |

Legend: side groups and other related properties: A=acidic; B=basic; Ali=aliphatic; Ami=amine; Aro= aromatic; N=nonpolar; PU=polar uncharged; NEG=negatively charged; POS=positively charged.

It is noted that codon preferences and codon usage tables for a particular species can be used to engineer isolated nucleic acid molecules that take advantage of the codon usage preferences of that particular species. For example, the isolated nucleic acid provided herein can be designed to have codons that are preferentially used by a particular organism of interest. Numerous software and sequencing services are available for such codon-optimizing of sequences. It also is noted that less conservative substitutions may be made and still provide a functional variant.

In various embodiments polypeptides obtained by the expression of the polynucleotide molecules of the present disclosure may have at least approximately 50%, 60%, 70%, 80%, 90%, 95%, 96%, 97%, 98%, 99% or 100% identity to one or more amino acid sequences encoded by the genes and/or nucleic acid sequences described herein for chemical product biosynthesis pathways.

As a practical matter, whether any particular polypeptide is at least 50%, 60%, 70%, 80%, 85%, 90%, 92%, 95%, 96%, 97%, 98% or 99% identical to any reference amino acid sequence of any polypeptide described herein (which may correspond with a particular nucleic acid sequence described herein), such particular polypeptide sequence can be determined
conventionally using known computer programs such the Bestfit program (Wisconsin Sequence Analysis Package, Version 8 for Unix, Genetics Computer Group, University Research Park, 575 Science Drive, Madison, Wis. 53711). When using Bestfit or any other sequence alignment program to determine whether a particular sequence is, for instance, 95% identical to a reference sequence according to the present disclosure, the parameters are set such that the percentage of identity is calculated over the full length of the reference amino acid sequence and that gaps in homology of up to 5% of the total number of amino acid residues in the reference sequence are allowed.

For example, in a specific embodiment the identity between a reference sequence (query sequence, i.e., a sequence of the present disclosure) and a subject sequence, also referred to as a global sequence alignment, may be determined using the FASTDB computer program based on the algorithm of Brutlag et al. (Comp. App. Biosci. 6:237-245 (1990)). Preferred parameters for a particular embodiment in which identity is narrowly construed, used in a FASTDB amino acid alignment, are: Scoring Scheme=PAM (Percent Accepted Mutations) 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter. According to this embodiment, if the subject sequence is shorter than the query sequence due to N- or C-terminal deletions, not because of internal deletions, a manual correction is made to the results to take into consideration the fact that the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are lateral to the N- and C-terminal of the subject sequence, which are not matched/aligned with a corresponding subject residue, as a percent of the total bases of the query sequence. A determination of whether a residue is matched/aligned is determined by results of the FASTDB sequence alignment. This percentage is then subtracted from the percent identity, calculated by the FASTDB program using the specified parameters, to arrive at a final percent identity score. This final percent identity score is what is used for the purposes of this embodiment. Only residues to the N- and C-termini of the subject sequence, which are not aligned with the query sequence, are considered for the purposes of manually adjusting the percent identity score. That is, only query residue positions outside the farthest N- and C-terminal residues of the subject sequence are considered for this manual correction. For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not aligned with the query sequence are manually corrected for.

More generally, nucleic acid constructs can be prepared comprising an isolated polynucleotide encoding a polypeptide having enzyme activity operably linked to one or more (several) control sequences that direct the expression of the coding sequence in a microorganism, such as E. coli, under conditions compatible with the control sequences. The isolated polynucleotide may be manipulated to provide for expression of the polypeptide. Manipulation of the polynucleotide's sequence prior to its insertion into a vector may be desirable or necessary depending on the expression vector. The techniques for modifying polynucleotide sequences utilizing recombinant DNA methods are well established in the art.

The control sequence may be an appropriate promoter sequence, a nucleotide sequence that is recognized by a host cell for expression of a polynucleotide encoding a polypeptide of the present invention. The promoter sequence contains transcriptional control sequences that mediate the expression of the polypeptide. The promoter may be any nucleotide sequence that shows transcriptional activity in the host cell of choice including mutant, truncated, and hybrid promoters, and may be obtained from genes encoding extracellular or intracellular polypeptides either homologous or heterologous to the host cell. Examples of suitable promoters for directing transcription of the nucleic acid constructs, especially in an E. coli host cell, are the lac promoter (Gronenborn, 1976, Mol. Gen. Genet. 148: 243-250), tac promoter (DeBoer et a/., 1983, Proceedings of the National Academy of Sciences USA 80: 21-25), trc promoter (Brosius et al, 1985, J. Biol. Chem. 260: 3539-3541), T7 RNA polymerase promoter (Studier and Moffatt, 1986, J. Mol. Biol. 189: 113¬130), phage promoter pL (Elvin et al., 1990, Gene 87: 123-126), tetA prmoter (Skerra, 1994, Gene 151 : 131¬135), araBAD promoter (Guzman et al., 1995, J. Bacteriol. 177: 4121-4130), and rhaPBAD promoter (Haldimann et al., 1998, J. Bacteriol. 180: 1277-1286). Other promoters are described in "Useful proteins from recombinant bacteria" in Scientific American, 1980, 242: 74-94; and in Sambrook and Russell, "Molecular Cloning: A Laboratory Manual," Third Edition 2001 (volumes 1-3), Cold Spring Harbor Laboratory Press, Cold Spring Harbor, N.Y.

The control sequence may also be a suitable transcription terminator sequence, a sequence recognized by a host cell to terminate transcription. The terminator sequence is operably linked to the 3' terminus of the nucleotide sequence encoding the polypeptide. Any terminator that is functional in an E. coli cell may be used in the present disclosure. It may also be desirable to add regulatory sequences that allow regulation of the expression of the polypeptide relative to the growth of the host cell. Examples of regulatory systems are those that cause the expression of the gene to be turned on or off in response to a chemical or physical stimulus, including the presence of a regulatory compound. Regulatory systems in prokaryotic systems include the lac, tac, and trp operator systems.

Also, variants and portions of particular nucleic acid sequences, and respective encoded amino acid sequences recited herein may be exhibit a desired functionality, e.g., enzymatic activity at a selected level, when such nucleic acid sequence variant and/or portion contains a 15 nucleotide sequence identical to any 15 nucleotide sequence set forth in the nucleic acid sequences recited herein including, without limitation, the sequence starting at nucleotide number 1 and ending at nucleotide number 15, the sequence starting at nucleotide number 2 and ending at nucleotide number 16, the sequence starting at nucleotide number 3 and ending at nucleotide number 17, and so forth. It will be appreciated that the disclosure also provides isolated nucleic acid that contains a nucleotide sequence that is greater than 15 nucleotides (e.g., 16, 17, 18, 19, 20, 21, 22, 23, 24, 25, 26, 27, 28, 29, 30 or more nucleotides) in length and identical to any portion of the sequence set forth in nucleic acid sequences recited herein. For example, the disclosure provides isolated nucleic acid that contains a 25 nucleotide sequence identical to any 25 nucleotide sequence set forth in any one or more (including any grouping of) nucleic acid sequences recited herein including, without limitation, the sequence starting at nucleotide number 1 and ending at nucleotide number 25, the sequence starting at nucleotide number 2 and ending at nucleotide number 26, the sequence starting at nucleotide number 3 and ending at nucleotide number 27, and so forth. Additional examples include, without limitation, isolated nucleic acids that contain a nucleotide sequence that is 50 or more nucleotides (e.g., 100, 150, 200, 250, 300, or more nucleotides) in length and identical to any portion of any of the sequences disclosed herein. Such isolated nucleic acids can include, without limitation, those isolated nucleic acids containing a nucleic acid sequence represented in any one section of discussion and/or examples, including nucleic acid sequences encoding enzymes of the fatty acid synthase system. For example, the disclosure provides an isolated nucleic acid containing a nucleic acid sequence listed herein that contains a single insertion, a single deletion, a single substitution, multiple insertions,
multiple deletions, multiple substitutions, or any combination thereof (e. g., single deletion together with multiple insertions). Such isolated nucleic acid molecules can share at least 60, 65, 70, 75, 80, 85, 90, 95, 97, 98, or 99 percent sequence identity with a nucleic acid sequence listed herein (i.e., in the sequence listing).

Additional examples include, without limitation, isolated nucleic acids that contain a nucleic acid sequence that encodes an amino acid sequence that is 50 or more amino acid residues (e.g., 100, 150, 200, 250, 300, or more amino acid residues) in length and identical to any portion of an amino acid sequence listed or otherwise disclosed herein.

In addition, the disclosure provides isolated nucleic acid that contains a nucleic acid sequence that encodes an amino acid sequence having a variation of an amino acid sequence listed or otherwise disclosed herein. For example, the invention provides isolated nucleic acid containing a nucleic acid sequence encoding an amino acid sequence listed or otherwise disclosed herein that contains a single insertion, a single deletion, a single substitution, multiple insertions, multiple deletions, multiple substitutions, or any combination thereof (e.g., single deletion together with multiple insertions). Such isolated nucleic acid molecules can contain a nucleic acid sequence encoding an amino acid sequence that shares at least 60, 65, 70, 75, 80, 85, 90, 95, 97, 98, or 99 percent sequence identity with an amino acid sequence listed or otherwise disclosed herein.

The disclosure provides polypeptides that contain the entire amino acid sequence of an amino acid sequence listed or otherwise disclosed herein. In addition, the disclosure provides polypeptides that contain a portion of an amino acid sequence listed or otherwise disclosed herein. For example, the disclosure provides polypeptides that contain a 15 amino acid sequence identical to any 15 amino acid sequence of an amino acid sequence listed or otherwise disclosed herein including, without limitation, the sequence starting at amino acid residue number 1 and ending at amino acid residue number 15, the sequence starting at amino acid residue number 2 and ending at amino acid residue number 16, the sequence starting at amino acid residue number 3 and ending at amino acid residue number 17, and so forth. It will be appreciated that the disclosure also provides polypeptides that contain an amino acid sequence that is greater than 15 amino acid residues (e. g., 16, 17, 18, 19, 20, 21, 22, 23, 24, 25, 26, 27, 28, 29, 30 or more amino acid residues) in length and identical to any portion of an amino acid sequence listed or otherwise disclosed herein For example, the disclosure provides polypeptides that contain a 25 amino acid sequence identical to any 25 amino acid sequence of an amino acid sequence listed or otherwise disclosed herein including, without limitation, the sequence starting at amino acid residue number 1 and ending at amino acid residue number 25, the sequence starting at amino acid residue number 2 and ending at amino acid residue number 26, the sequence starting at amino
acid residue number 3 and ending at amino acid residue number 27, and so forth. Additional examples include, without limitation, polypeptides that contain an amino acid sequence that is 50 or more amino acid residues (e.g., 100, 150, 200, 250, 300 or more amino acid residues) in length and identical to any portion of an amino acid sequence listed or otherwise disclosed herein. Further, it is appreciated that, per above, a 15 nucleotide sequence will provide a 5 amino acid sequence, so that the latter, and higher-length amino acid sequences, may be defined by the above-described nucleotide sequence lengths having identity with a sequence provided herein.

In addition, the disclosure provides polypeptides that an amino acid sequence having a variation of the amino acid sequence set forth in an amino acid sequence listed or otherwise disclosed herein. For example, the disclosure provides polypeptides containing an amino acid sequence listed or otherwise disclosed herein that contains a single insertion, a single deletion, a single substitution, multiple insertions, multiple deletions, multiple substitutions, or any combination thereof (e.g., single deletion together with multiple insertions). Such polypeptides can contain an amino acid sequence that shares at least 60, 65, 70, 75, 80, 85, 90, 95, 97, 98 or 99 percent sequence identity with an amino acid sequence listed or otherwise disclosed herein. A particular variant amino acid sequence may comprise any number of variations as well as any combination of types of variations.

As indicated herein, polypeptides having a variant amino acid sequence can retain enzymatic activity. Such polypeptides can be produced by manipulating the nucleotide sequence encoding a polypeptide using standard procedures such as site-directed mutagenesis or various PCR techniques. As noted herein, one type of modification includes the substitution of one or more amino acid residues for amino acid residues having a similar chemical and/or biochemical property. For example, a polypeptide can have an amino acid sequence set forth in an amino acid sequence listed or otherwise disclosed herein comprising one or more conservative substitutions.

More substantial changes can be obtained by selecting substitutions that are less conservative, and/or in areas of the sequence that may be more critical, for example selecting residues that differ more significantly in their effect on maintaining: (a) the structure of the polypeptide backbone in the area of the substitution, for example, as a sheet or helical conformation; (b) the charge or hydrophobicity of the polypeptide at the target site; or (c) the bulk of the side chain. The substitutions that in general are expected to produce the greatest changes in polypeptide function are those in which: (a) a hydrophilic residue, e.g., serine or threonine, is substituted for (or by) a hydrophobic residue, e.g., leucine, isoleucine, phenylalanine, valine or alanine; (b) a cysteine or proline is substituted for (or by) any other residue ; (c) a residue having an electropositive side chain, e.g., lysine, arginine, or histidine, is substituted for (or by) an electronegative residue, e.g., glutamic acid or aspartic acid; or (d) a
residue having a bulky side chain, e.g., phenylalanine, is substituted for (or by) one not having a side chain, e.g., glycine. The effects of these amino acid substitutions (or other deletions or additions) can be assessed for polypeptides having enzymatic activity by analyzing the ability of the polypeptide to catalyze the conversion of the same substrate as the related native polypeptide to the same product as the related native polypeptide. Accordingly, polypeptides having 5, 10, 20, 30, 40, 50 or less conservative substitutions are provided by the disclosure.

Polypeptides and nucleic acids encoding polypeptides can be produced by standard DNA mutagenesis techniques, for example, M13 primer mutagenesis. Details of these techniques are provided in Sambrook and Russell, 2001. Nucleic acid molecules can contain changes of a coding region to fit the codon usage bias of the particular organism into which the molecule is to be introduced.

The disclosure also provides an isolated nucleic acid that is at least about 12 bases in length (e.g., at least about 13, 14, 15, 16, 17, 18, 19, 20, 25, 30, 40, 50, 60, 100, 250, 500, 750, 1000, 1500, 2000, 3000, 4000, or 5000 bases in length) and hybridizes, under hybridization conditions, to the sense or antisense strand of a nucleic acid having a sequence listed or otherwise disclosed herein. The hybridization conditions can be moderately or highly stringent hybridization conditions. Also, in some embodiments the microorganism comprises an endogenous chemical productproduction pathway (which may, in some such embodiments, be enhanced), whereas in other embodiments the microorganism does not comprise a chemical product production pathway, but is provided with one or more nucleic acid sequences encoding polypeptides having enzymatic activity or activities to complete a pathway, described herein, resulting in production of a selected chemical product. In some embodiments, the particular sequences disclosed herein, or conservatively modified variants thereof, are provided to a selected microorganism, such as selected from one or more of the species and groups of species or other taxonomic groups listed herein.

### VI. METABOLIC PATHWAYS

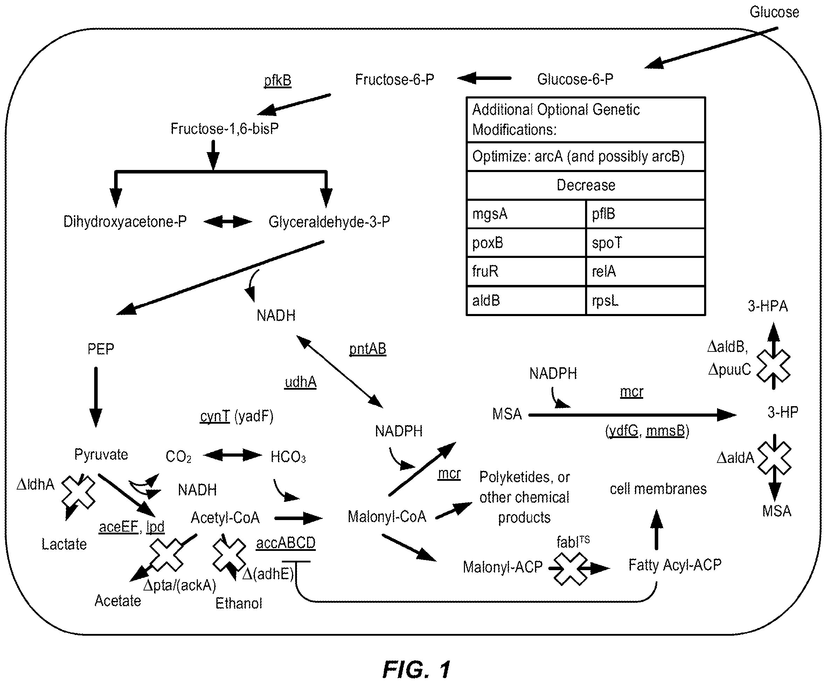

FIG. 1 diagrammatically depicts a cell showing key enzymatic conversion steps that include production of malonyl-CoA. In this figure enzyme functions are indicated by indicated enzymatic conversions and/or representative E. coli gene identifiers that encode proteins (polypeptides) having such enzyme functions (except that mcr indicates non-E. coli malonyl-CoA reductase), deletions are shown by the standard "A" before the respective gene identifier, and increased enzymatic activities are shown by underlining (noting that additional targets for modifications are as indicated in the embedded table of the figure). Genes in parentheses are possible substitutes for or supplements of an enzyme encoded by another gene also shown along the respective pathway step. Disrupted pathways also are shown by an "X" and the transient or partially disrupted step from malonyl-CoA to Fatty Acid-ACP is shown by a dashed "X." Also, the use of fabl(ts) represents a substitution for the native non-temperature-sensitive gene, thus the dashed X shape over the enzymatic conversion step is meant to indicate that a modification of this step may be made so that the enzymatic conversion is reduced, reduced under certain conditions, etc. This is not meant to be limiting; as described elsewhere there are a number of approaches to control and limit flux to fatty acyl-ACP and other intermediates or products of a fatty acid synthase pathway. Also, in this and various other figures, the use of "glucose" as a carbon source is exemplary and not meant to be limiting; pathways for other carbon sources to feed into the indicated pathways may be substituted.

The insert box of FIG. 1 provides a list, not to be limiting, of additional optional genetic modifications that may be made to the host cell to further improve chemical product biosynthesis under various culture conditions.

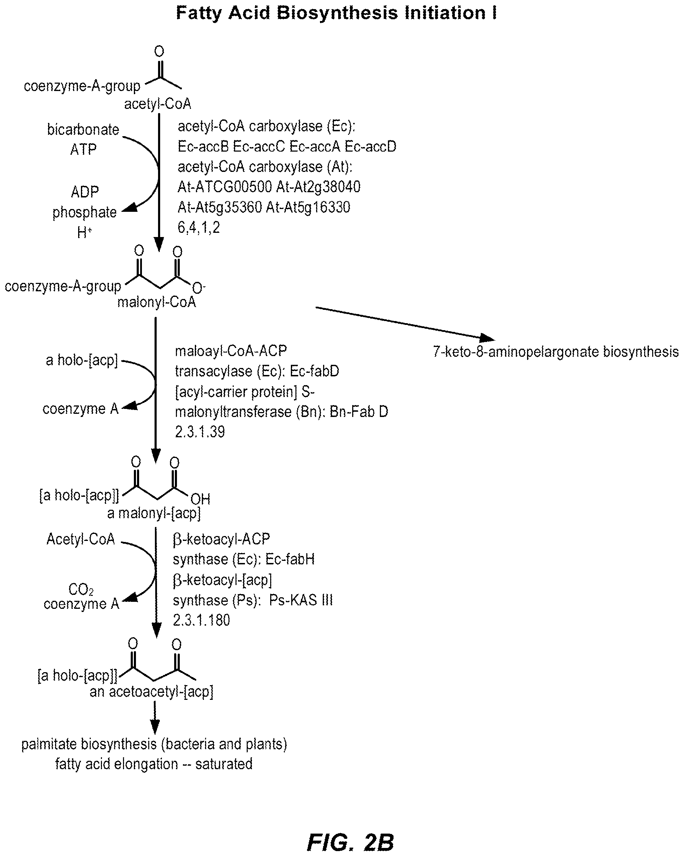

It is important to note that malonyl-CoA may be a substrate for a fatty acid synthase system such as that shown in FIG. 2A, which utilizes malonyl-CoA and produces fatty acids that are used for cell membranes and other cellular requirements. Also shown in FIG. 2A are E. coli gene names for genes that encode various steps of this representative fatty acid synthase (also referred to as fatty acid synthesis) pathway, the circular portion of which exemplifies fatty acid elongation. FIGs. 2B-D are provided to show fatty acid initiation reactions in greater detail (some of which are depicted in FIG. 2A). The gene names are not meant to be limiting, and are only exemplary. Table 3 provides enzyme function and corresponding EC numbers.

FIG. 2A also depicts a metabolic competition between utilization of malonyl-CoA for fatty acid synthesis (lower part of diagram) versus for production of polyketides and other malonyl-CoA-based chemical products, and also for production of 3-HP (which may be converted after biosynthesis to acrylic acid and other chemicals and products). Without being bound to a particular theory, when flux can be diverted from fatty acid synthesis that consumes malonyl-CoA, more malonyl-CoA may become available for biosynthesis of a desired chemical product of commercial interest (other than a fatty acid, a phospholipid, etc.). FIG. 2A also identifies certain inhibitors selected pathway steps, and certain feedback inhibition by metabolic intermediates (shown by a "Tee" shape extending from the inhibitor or metabolic intermediate to the inhibited step).

It is noteworthy that for purposes of the present invention, although traditionally fatty acid synthesis is often viewed to start with acetyl-CoA carboxylase, which produces malonyl-CoA, for the purposes of the present invention, as is clear from FIG. 2A and other teachings herein, modulation to decrease one or more, two or more, or three or more enzymatic functions of the fatty acid synthesis pathways downstream of malonyl-CoA are of interest to increase availability of malonyl-CoA for biosynthesis of the chemical products disclosed herein. Also, as taught herein, the genetic modification(s) and/or other system modifications (e.g., addition of an inhibitor) to modulate these enzymatic functions (i.e., enzymatic conversion steps) may be combined with various genetic modification(s) to increase production of and/or flux through malonyl-CoA, including increasing net activity (whether by increasing gene copy numbers, increasing activity, using a mutant form, etc.) of acetyl- coA carboxylase. The basis for this is observable in FIG. 2A, since malonyl-CoA is the product of this enzyme (ACCase being the abbreviation for acetyl-CoA carboxylase in FIG. 2A).

CoA means coenzyme A; MSA means malonate semialdehyde; mcr means malonyl-CoA reductase (which may be monofunctional, reacting only malonyl¬CoA to MSA, or bifunctional, reacting this and also MSA to 3-HP (one bifunctional form being from Chloroflexus auriantiacus)); and ACP means acyl carrier protein. Also, representative (not to be limiting) gene names for E. coli genes that encode enzymes that carry out the indicated enzymatic conversion steps are provided. Table 3 provides additional information about these enzymatic conversion steps, which is incorporated into FIG. 2A.

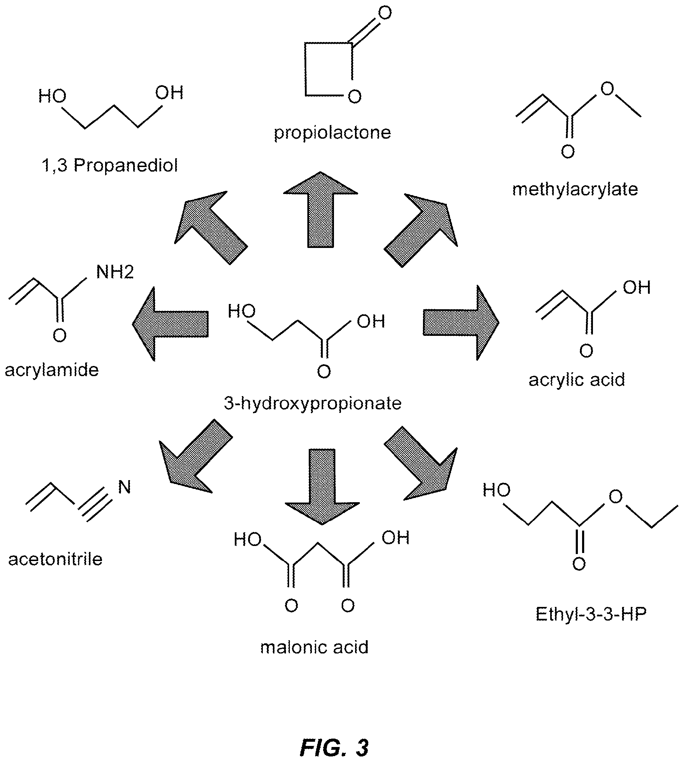

FIG. 3 shows various chemicals that may be derived from 3-HP. This is not meant to be limiting, and it is known that numerous products may be produced from each of these chemicals that may be derived from 3- HP.

Such products are referred to herein as 3-HP derivative products. Various reactions to obtain these and other chemical products from these are described in Ulmann's Encyclopedia of Industrial Chemistry, Acrylic Acid and Derivatives, Wiley VCH Verlag GmbH, Wienham (2005).

It is within the scope of the present invention to have a method of making any of the chemical compounds disclosed herein, including those in the preceding paragraph, that includes biosynthesis of malonyl- CoA as taught herein, wherein the malonyl-CoA is thereafter converted to a chemical product by the cell (e.g. 3-HP), and optionally thereafter converted to another chemical product such as those in or made from those in FIG. 3.

As indicated above, it was unexpected that particular combinations of modifications of fatty acid synthase pathway genes, such as modifications that transiently lower enzymatic function at elevated culture temperature or deletion of a gene encoding an enzyme of the pathway, would increase chemical product of mcA, 3-HP biosynthesis (such as measured by specific productivity). Particular combinations of such genetic modifications are described below, including in the examples, which are incorporated into this section.

Further, it has been found that combining genetic modifications directed to different metabolic effects in a host cell leads to greater production of a selected chemical product. In various embodiments, at least one genetic modification along a pathway to a selected chemical product is combined with one or more, two or more, or three or more, genetic modifications that are effective to reduce the overall activity and/or flux through one or more, two or more, or three or more, enzymatic conversion steps of a host cell's fatty acid synthase pathway. The enzymatic functions that catalyze the enzymatic conversions steps of the representative fatty acid synthase pathway, depicted in FIG. 2A, are summarized in Table 3, with reference to sequence listing numbers that correspond to representative DNA and amino acid sequences for these enzymatic functions in E. coli. These may be modified in accordance with embodiments of the invention.

**TABLE 3**

| **Enzyme Function(s) (with synonyms)** | **Enzyme Commission (EC) Number** | **Names of** | **SEQ ID NO. of** |
|---|---|---|---|
| malonyl-CoA-acyl carrier protein transacylase | 2.3.1.39 | fabD | 007 |
| βketoacyl-acyl carrier protein synthase II, β-ketoacyl-ACP synthase II, 3-oxoacyl-ACP synthas II, KASII, acyl-[acyl carrier protein]:malonyl-[acyl carrier protein] C-acyltransferase (decarboxylating), | 2.3.41.41 | fabF | 008 |
| β-ketoacyl-ACP synthase, -ketoacyl-acyl carrier protein synthase I, 3-oxoacyl-ACP-synthase I, KASI | | fabB | 009 |
| β-ketoacyl-acyl carrier protein synthase III, β-ketoacyl-ACP synthase III, 3-oxoacyl-ACP synthase III, KASIII | | fabH | 010 |
| β-ketoacyl-Eacyl-carrier protein] reductase; 3-oxoacyl-[acyl-carrier-protein] reductase | 1.1.1.100 | fabG | 011 |
| β-hydroxyacl-ACP dehydratase, 3-hydroxyacyl-[acp] dehydratase | 4.2.1.59 | fabZ | 012 |
| | | fabA | 013 |
| enoyl-ACP reductase (NADH), enoyl-acyl carrier protein (ACP) reductase | 1.3.1.9; 1.3.1.10 | fabI | 014 |

While not meant to be limiting one approach to modification is to use a temperature sensitive mutant of one or more of the above polypeptides. For example, a temperature-sensitive FabB is known, having the following mutation: A329V. Also, a temperature-sensitive FabI is known, having the following mutation: (S241F). Either or both of these may be combined with other modifications, such as a deletion of FabF. Non- limiting examples of such combinations are provided in the Examples section in strains having other indicated genetic modifications including introduction of specific plasmids.

It also is noted that any combination of modifications to reduce activity along the fatty acid synthesis pathway downstream of malonyl-CoA may include one or more modifications of the polypeptides that are responsible for enzymatic conversion steps shown in FIGs. 2B-D.

As noted herein, various aspects of the present invention are directed to a microorganism cell that comprises a metabolic pathway from malonyl-CoA to a chemical product of interest, such as those described above, and means for modulating conversion of malonyl-CoA to fatty acyl molecules (which thereafter may be converted to fatty acids) also are provided. Then, when the means for modulating modulate to decrease such conversion, a proportionally greater number of malonyl-CoA molecules are 1) produced and/or 2) converted via the metabolic pathway from malonyl-CoA to the chemical product. In various embodiments, additional genetic modifications may be made, such as to 1) increase intracellular bicarbonate levels, such as by increasing carbonic anhydrase, 2) increase enzymatic activity of acetyl-CoA carboxylase, and NADPH-dependent transhydrogenase, 3) increase production of coenzyme A.

In various embodiments the production of a selected chemical product is not linked to microorganism growth, that is to say, there are non-growth coupled embodiments in which production rate is not linked metabolically to cellular growth. For example, a microbial culture may be brought to a desired cell density, followed by a modulation (such as temperature shift of a temperature-sensitive protein) resulting in less malonyl-CoA being converted to fatty acids (generally needed for growth) and thus more to production of a selected chemical product.

Other additional genetic modifications are disclosed herein for various embodiments.

Included among additional genetic modifications are genetic modifications directed to reduce (including eliminate) flux through and/or activity of enzymes in the glyoxylate bypass of the microorganism, for example one or more of malate synthase, isocitrate lyase, and isocitrate dehydrogenase kinase-phosphatase. Such modification has been demonstrated to substantially reduce the flux of carbon through oxaloacetate generated via the glyoxylate bypass shunt. For example, in E. coli a deletion of the operon identified as aceBAK may be made.

More generally embodiments of the invention comprise a modification to reduce production of undesired metabolic products, which may be selected from various amino acids and other metabolic products, said modification resulting in a reduction (including elimination) of enzymatic activity of or an enzyme of or controlling flux through the glyoxylate bypass, optionally further selected from one or more of malate synthase A, isocitrate lyase, and kinase-phosphatase that controls activity of an isocitrate dehydrogenase.

As one example, not to be limiting, a deletion may be made to aceBAK in an E. coli strain that comprises other genetic modifications taught herein. One example of an E. coli strain comprising such deletion is:

**TABLE 4**

| **Strain** | **Parent** | **Genotype** | **Plasmid(s)** |
|---|---|---|---|
| BX3_547 | BX_0775.0 | *F-, Δ(araD-araB)567, ΔlacZ4787(::rrnB-3), LAM-, rph-1, Δ(rhaD-rhaB)568, hsdR514,* ΔldhA::frt, ΔpflB::frt, ΔmgsA::frt, ΔpoxB::frt, Δpta-ack::frt, fabI(ts) (S241F)-zeoR, fabB(ts), Δfabf::frt, coaA*, fabD(ts), ΔaceBAK::frt | pTRC-kan-PyibD-mcr, pACYC-CAT-accADBC/pntAB |

Genotype traits denoted in bold italic font are present in the BW25113 host strain (available from the Coli Genetic Stock Center, Yale University, New Haven, CT USA). Additional genotype traits denoted in normal font were engineered by the inventors using standard methods as described and/or referenced herein. Parent BX _0775.0 is E. coli BW25113 (modifications shown in bold italic font) to which the other genetic modifications in the above genotype were made by methods described elsewhere herein.

Strain BX3_547 also comprises the plasmids identified in the above table. In pTRC-kan-PyibD-mcr, a promoter for malonyl-CoA reductase that is operative under low phosphate conditions. Phosphate starvation inducible promoters located upstream of native E. coli yibD and ytfK genes have been identified (Yoshida, et al., J Microbiol, 49(2), pp285-289, 2010). The target promoter sequence was ordered (Integrated DNA Technologies, Coralville, Iowa USA) including with modifications to the native ribosome binding site to be compatible with existing expression vectors and to accommodate expression of key downstream gene(s) within the vector(s) (see examples).

Strain BX3_547 was evaluated under various fermentation conditions that modulated or otherwise controlled temperature, pH, oxygen concentration, glucose feed rate and concentration, and other media conditions. Among the parameters used to determine other process steps, low ambient phosphate concentration was used as a control point that lead to temperature shift from approximately 30°C to approximately 37°C. A promoter sensitive to low ambient phosphate was utilized to control expression of the gene encoding malonyl-CoA reductase in the plasmid identified as pTrc-PyibD-mcr (SEQ ID NO:170). Also, during one or more evaluations, any one or more of dissolved oxygen, redox potential, aeration rate, agitation rate, oxygen transfer rate, and oxygen utilization rate was/were used to control the system and/or measured.

Strain BX3_547 was evaluated over 36 fermentation events that were conducted over an 8 week period using FM11 medium (described in the Common Methods Section). Duration of the fermentation events were all less than 80 hours, of which a portion was after temperature increase to effectuate reduced enzymatic activity of fabl(ts), fabB(ts), and fabD(ts). While not meant to be particularly limiting in view of other genetic modifications and culture conditions that may be employed, these results demonstrated microbial performance over a range of reduced oxygen conditions, with final 3-HP titers ranging between 50 and 62 grams of 3-HP/liter of final culture media volume.

It is appreciated that the PyibD promoter, or a similar low-phosphate induction promoter, could be utilized in a genetic construct to induce any one or more of the sequences described and/or taught herein, so as to enable production of any other of the chemical products disclosed herein, including in the examples provided herein.

In various embodiments, expression of desired genes is induced when the environmental phosphate concentration is maintained or adjusted to a low, more particularly, an effectively low concentration. This may be achieved, for example, by introduction of a promoter induced by low phosphate concentration for promotion of one or more nucleic acid sequences of interest. For example, increased production of a desired chemical product may result when environmental phosphate concentration is at or less than a concentration effective for the increased production, including but not limited to when phosphate concentration is not detectable by standard analytical techniques. One example of a low phosphate promoter that may be used with such embodiments is the PyibD promoter, exemplified such as by SEQ ID NO:169.

Further, various embodiments of the disclosure accordingly are directed to methods, compositions and systems that regard culturing modified microorganisms, such as those comprising a low-phosphate inducible enzyme such as described above, in a two-phase approach, the first phase substantially to increase microorganism biomass and the second phase substantially to produce a desired product (such as but not limited to 3-HP).

To constructe pTRC-kan-PyibD-mcr, the promoter from *E. coli yibD,* with nearby native sequences and selected restriction sites, was synthesized (Integrated DNA Technologies). Also, changes to the native ribosome binding site were made to accommodate appropriate expression of MCR:

This sequence as provided in pTrc-PyibD-mcr to induce mcr is as follows:

The Examples further describes the construction of the plasmid pTrc-PyibD-mcr (SEQ ID NO:170).

Strain BX3_547 also comprises coaA*, a pantothenate kinase which is refractory to feedback inhibition. An exemplary sequence of such coaA* is SEQ ID NO: 173. CoaA* R106A Mutant Sequence (SEQ ID NO:173) is provided below. This coaA* mutant also is provided in other strains listed in Table 6.

It is appreciated that the particular constructs in these examples, and the product obtained (3-HP), are not meant to be limiting. In various embodiments genetic constructs, microorganisms, methods and systems are produced and/or employed that comprise and utilize induction under low environmental phosphate conditions. Any of the products described herein, including those in Tables 1A-1H may be produced using a microorganism that comprises a modification that leads to induction of one or more enzymes under low phosphate conditions. Increased production of a selected chemical product may be obtained when phosphate concentration is maintained or adjusted to a low, more particularly, an effectively low concentration. This may be achieved, for example, by introduction of a promoter induced by low phosphate concentration for promotion of one or more nucleic acid sequences of interest. For example, increased production of a desired chemical product may result when environmental phosphate concentration is at or less than a concentration effective for the increased production, including but not limited to when phosphate concentration is not detectable by standard analytical techniques. One example of a low phosphate promoter is the PyibD promoter, exemplified such as by SEQ ID NO:210. This aspect of the disclosure may be combined, in any combination, with any of the other aspects of the disclosure taught herein.

Unexpected increases in specific productivity by a population of a genetically modified microorganism may be achieved in methods and systems in which that microorganism has a microbial production pathway from malonyl-CoA to a selected chemical product as well as a reduction in the enzymatic activity of a selected enzyme of the microorganism's fatty acid synthase system (more particularly, its fatty acid elongation enzymes). In various embodiments, specific supplements to a bioreactor vessel comprising such microorganism population may also be provided to further improve the methods and systems. In various embodiments one or more, two or more, or three or more, enzymatic conversion steps of a host cell's pathway(s) to biosynthesize coenzyme-A ("CoA", "coA", "Co-A" or "co-A") are modified to increase cellular production of CoA. This further increases chemical product biosynthesis when combined with either or both of the two types of genetic modifications just described- regarding increasing production of a chemical product and toward reducing fatty acid synthase pathway activities/flux. Accordingly, in various embodiments of the disclosure, to more effectively redirect malonyl-CoA from fatty acid synthesis and toward the biosynthesis of such a desired chemical product, genetic modifications are made to:
(a) increase production along the biosynthetic pathway that includes malonyl-CoA and leads to a desired chemical product;
(b) reduce the activity of one or more, or of two or more, or of three or more of enoyl- acyl carrier protein (ACP) reductase, B-ketoacyl-acyl carrier protein synthase I (such as fabB), B-ketoacyl-acyl carrier protein synthase II, and malonyl-CoA-acyl carrier protein transacylase; and optionally to c. increase the production of coenzyme A in the microorganism cell.

In various microorganisms conversion of the metabolic intermediate malonyl-CoA to fatty acids via a fatty acid synthase (also referred to by "synthesis") system (also referred to as "pathway" or "complex") is the only or the major use of malonyl-CoA. A representative fatty acid synthase pathway known to function in microorganisms is depicted in FIG. 2A. This has a cyclic component by which fatty acid molecules are elongated to a final length; these may thereafter be further modified to phospholipids, etc., which are used in cell membranes and other cellular functions.

It has been determined that when a production pathway to an alternative chemical product exists in a microorganism, reducing such conversion of malonyl-CoA to fatty acids can improve metrics for production of that alternative chemical product (e.g., a polyketide or 3-HP). For example, as depicted in FIG. 2A and listed in Table 3, in many microorganism cells the fatty acid synthase system comprises polypeptides that have the following enzymatic activities: malonyl-CoA-acyl carrier protein (ACP) transacylase; B-ketoacyl-ACP synthase; B-ketoacyl-ACP reductase; B-hydroxyacyl-ACP dehydratase; 3-hydroxyacyl-(acp) dehydratase; and enoyl-acyl carrier protein reductase (enoyl-ACP reductase). In various embodiments nucleic acid sequences that encode temperature-sensitive forms of these polypeptides may be introduced in place of the native enzymes, and when such genetically modified microorganisms are cultured at elevated temperatures (at which these thermolabile polypeptides become inactivated, partially or completely, due to alterations in protein structure or complete denaturation), there is observed an increase in a product such as 3-HP, THN, or flaviolin. In other embodiments other types of genetic modifications may be made to otherwise modulate, such as lower, enzymatic activities of one or more of these polypeptides. In various embodiments a result of such genetic modifications is to shift malonyl-CoA utilization so that there is a reduced conversion of malonyl-CoA to fatty acids, overall biomass, and proportionally greater conversion of carbon source to a chemical product such as 3-HP. In various embodiments, the specific productivity for the microbially produced chemical product is unexpectedly high. Also, additional genetic modifications, such as to increase malonyl-CoA production, may be made for certain embodiments.

In various embodiments genetic modifications are made to reduce the overall function, whether measureable as enzymatic activity, enzyme concentration, and/or flux, of two or more, or of three or more, of the enzymatic functions of a host cell's fatty acid synthase pathway. These may be combined with other types of genetic modifications described herein, such as to a chemical product pathway and/or to genetic modifications that result in greater production of coenzyme A.

Accordingly, in some embodiments the present disclosure comprises a genetically modified microorganism that comprises at least one genetic modification that provides,
completes, or enhances a chemical production pathway effective to convert malonyl-CoA to a chemical product, and further comprises at least two, or at least three genetic modifications of at least two, or at least three enzymes of the fatty acid synthase system of a host cell, such as selected from enoyl-acyl carrier protein reductase (enoyl-ACP reductase) or enoyl-coenzyme A reductase (enoyl-CoA reductase), B-ketoacyl-ACP synthase or B-ketoacyl-CoA synthase, malonyl-CoA-ACP, where such latter genetic modifications have a cumulative effect to reduce conversion of malonyl-CoA to fatty acids. Other genetic modifications may be provided to such host cell as described elsewhere herein. The latter include those depicted in FIG. 1, such as but not limited to a genetic modification of carbonic anhydrase to increase bicarbonate levels in the microorganism cell (and/or a supplementation of its culture medium with bicarbonate and/or carbonate), and one or more genetic modifications to increase enzymatic activity of one or more of acetyl-CoA carboxylase and NADPH-dependent transhydrogenase. Related methods and systems utilize such any of such genetically modified microorganisms.

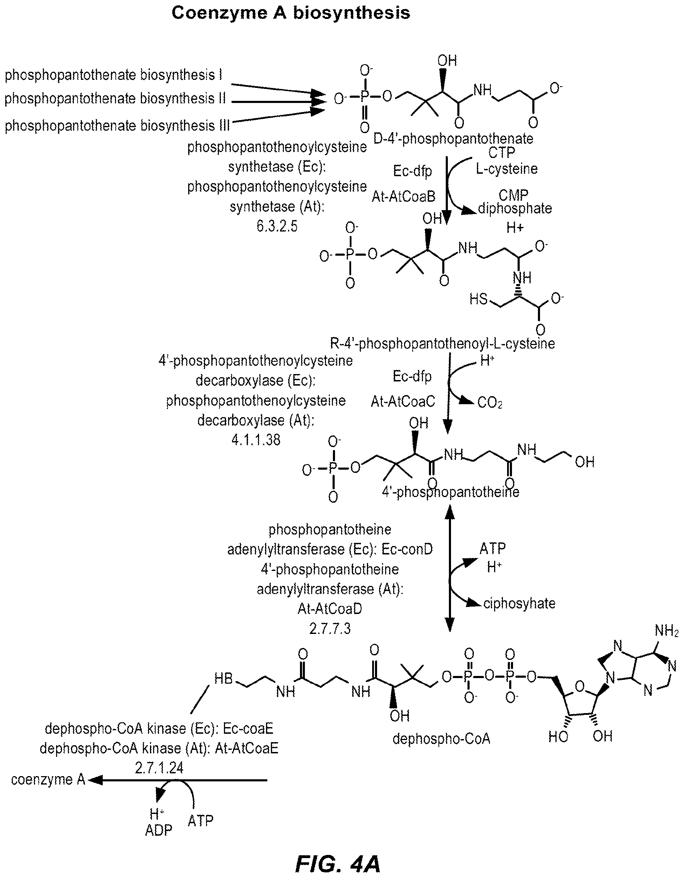

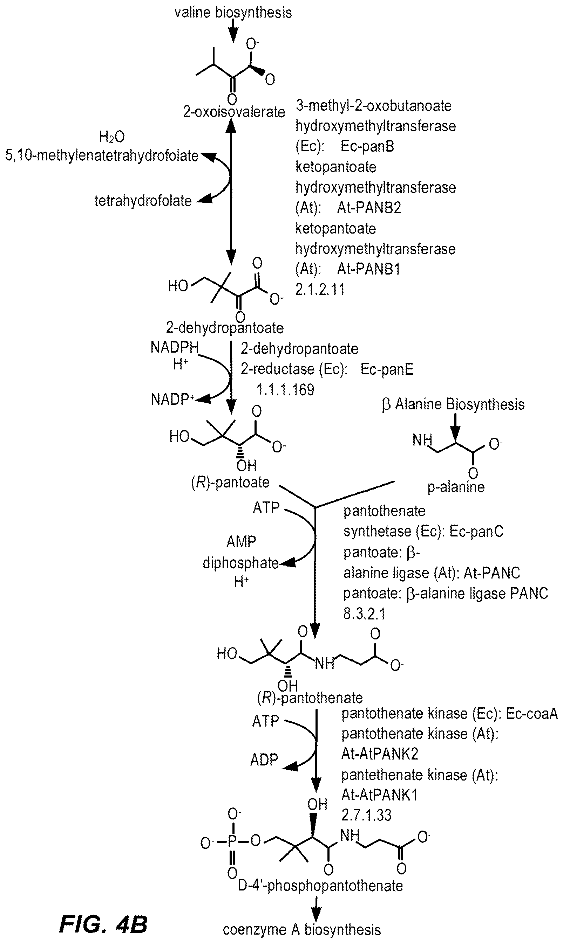

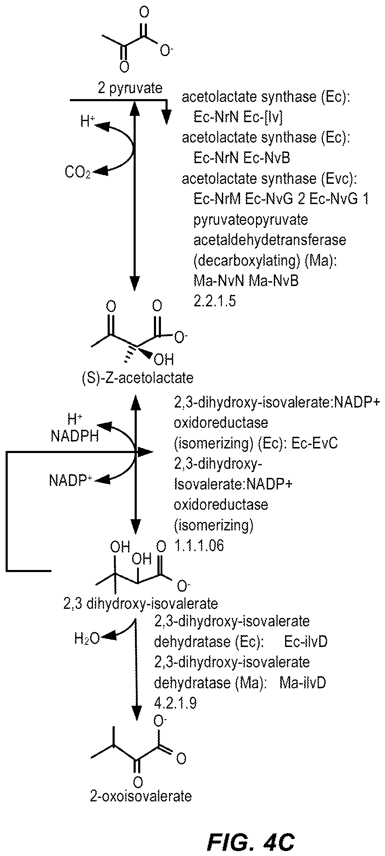

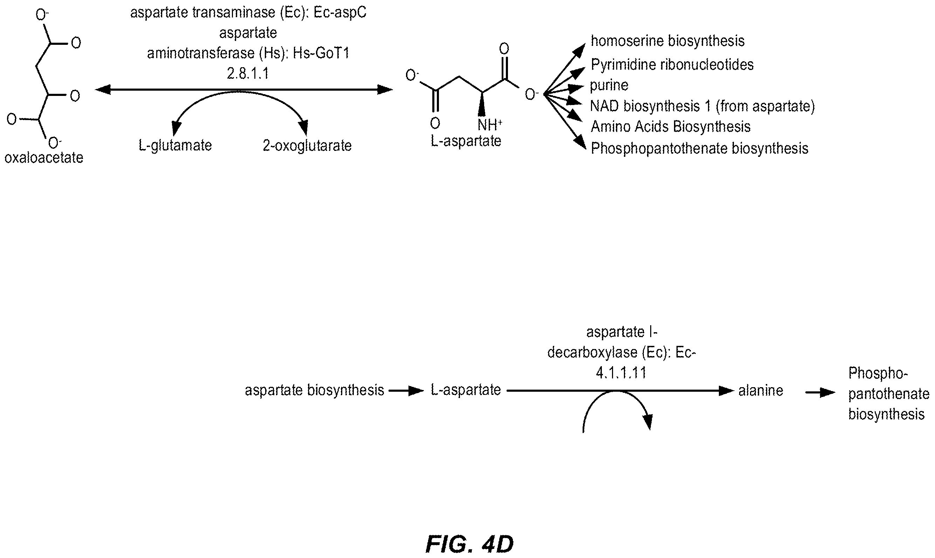

Also, as noted, the disclosure may comprise, in various embodiments, one or more genetic modifications that result in greater production of coenzyme A. Such genetic modification may be to any of the genes encoding enzymes along the pathways leading to production of coenzyme A. For example, FIGs. 4A-D depict representative biosynthetic pathways (and portions thereof) that lead to coenzyme A. Any of the genes encoding these enzymes may be modified to increase respective enzymatic conversion step activity and/or flux in order to increase coenzyme A production in a host cell. In a particular embodiment, one or more, two or more, or three or more genetic modifications may be made to achieve this end. Table 5 summarizes the enzymatic conversions shown in FIGs. 4A-D.

**TABLE 5**

| **Enzyme Function** | **E.C. Classification** | **Gene Name in E. coli** |
|---|---|---|
| aspartate transaminase | 2.6.1.1 | aspC |
| aspartate 1-decarboxylase | 4.1.1.11 | panD |
| acetolactate synthase | 2.2.1.6 | i1vH, ilvi |
| 2,3-dihydroxy-isovalerate:NADP+ oxidoreductase (isomerizing) | 1.1.1.86 | ilvC |
| 2,3-dihydroxy-isovalerate dehydratase | 4.2.1.9 | ilvD |
| 3-methyl-2-oxobutanoate | 2.1.2.11 | panB |
| 2-dehydropantoate | 1.1.1.169 | panE |
| pantothenate synthetase | 6.3.2.1 | pane |
| pantothenate kinase | 2.7.1.33 | coaA (panK) |
| phosphopantothenoylcysteine synthetase | 6.3.2.5 | dfp |
| 4'-phosphopantothenoylcysteine decarboxylase | 4.1.1.36 | dfp |
| phosphopantetheine adenylytransferase | 2.7.7.3 | coaD |
| Dephospho-CoA kinase | 2.7.1.24 | coaE |

A plurality of strains are described in the Examples section, hereby incorporated into this section. In addition, the following table lists additional strains of value in production of chemical products such as 3-HP and related chemicals. For construction of these strains, generally all plasmids were introduced at the same time via electroporation using standard methods. Transformed cells were grown on the appropriate media with antibiotic supplementation and colonies were selected based on their appropriate growth on the selective media. The base strains were derived from E. coli BW25113 (F-, Δ(araD-araB)567, ΔlacZ4787(::rrnB-3), lamba-, rph-1, Δ(rhaD-rhaB)568, hsdR514), these strains comprising additional chromosomal modifications generally introduced using Gene Bridges technology as described herein, such as in the Common Methods Section. Temperature-sensitive mutant forms are designated herein by "ts" or "(ts)," either of which may be as a superscript.

**TABLE 6**

| **Strain** | **Background** | **Genotype** | **Plasmids** |
|---|---|---|---|
| BX3_0451.0 | BX_0701.0 | F-, Δ(araD-araB)67, ΔlacZ4787(::rrnB-3), LAM-, rph-1, Δ(rhaD-rhaB)568, hsdR514, , ΔldhA:frt, ΔpflB:frt, ΔmgsA:frt,ΔpoxB:frt, Δpta-ack:frt, fabI^{ts} (S241F)-zeoR, Δalda::CSC | pTRC-KAN-mcr, pACYC-CAT-accADBC/pntAB |
| BX3_0467 | BX_00704.0 | F-, Δ(araD-araB)567, ΔlacZ4787(::rrnB-3), LAM-, rph-1, Δ(rhaD-rhaB)568, hsdR514,, ΔldhA::frt, ΔpflB::frt, ΔmgsA::frt, ΔpoxB::frt, Δpta-ack::frt, fabIts (S241F)-zeoR, ΔfabF::frt | pTRC-KAN-mcr, pACYC-CAT-accADBC/pntAB |
| BX3_0472 | BX_00706.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt | pTRC-KAN-mcr, pACYC-CAT-accADBC/pntAB |
| BX3_0478 | BX_00725.0 | F-, Δ(araD-araB)567, ΔlacZ4787(::rrnB-3), LAM-, rph-1, Δ(rhaD-rhaB)568, hsdR514, , ΔldhA:frt, ΔpflB:frt, ΔmgsA:frt,ΔpoxB:frt, Δpta-ack:frt, fabI^{ts} (S241F)-zeoR, coaA* | pTRC-KAN-mcr, pACYC-CAT-accADBC/pntAB |
| BX3_0491.0 | BX_0726.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt,ΔpoxB::frt, Δpta-ack::frt, fabIts (S241F)-zeoR, fabDts | pTRC-KAN-mcr, pACYC-CAT-accADBC/pntAB |
| BX3_0492.0 | BX_0735.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA* | pTRC-KAN-mcr, pACYC-CAT-accADBC/pntAB |
| BX3_0495.0 | BX0746.0 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, fabD(ts) | pTRC-KAN-mcr, pACYC-CAT-accADBC/pntAB |
| BX3_0494.0 | BX_0738.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA*, fabDts | pTRC-KAN-mcr, pACYC-CAT-accADBC/pntAB |
| BX3_0501.0 | BX_0728.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt,ΔpoxB::frt, Δpta-ack::frt, fabIts (S241F)-zeoR, ΔgapA::frt | pTRC- KAN-ptrc-mcr-gapA, pACYC-CAT-accADBC/pntAB-ccdAB |
| BX3_0537.0 | BX_0775.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA*, fabDts, ΔaceBAK:: frt | pTRC-KAN-ptrc-mcr, pACYC-CAT-accADBC/pntAB |
| BX3_0538.0 | BX_0775.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA*, fabDts, ΔaceBAK:: frt | pTRC-KAN-ptrc-mcr-gapA, pACYC-CAT-accADBC/pntAB-ccdAB |
| BX3_0547.0 | BX_0775.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA*, fabDts, ΔaceBAK:: frt | pTRC-KAN-PyibD--mcr, pACYC-CAT-accADBC/pntAB |

The genetic modifications described and exemplified herein may be provided in various combinations in a microorganism strain so as to achieve a desired improvement in production rate, yield, and final titer of a selected chemical product. Various embodiments of the disclosure additionally may comprise a genetic modification to increase the availability of the cofactor NADPH, which can increase the NADPH/NADP+ ratio as may be desired. Non-limiting examples for such genetic modification are pgi (E.C. 5.3.1.9, in a mutated form), pntAB (E.C. 1.6.1.2), overexpressed, gapA (E.C. 1.2.1.12):gapN (E.C. 1.2.1.9, from Streptococcus mutans) substitution/replacement (including replacement in a plasmid), and disrupting or modifying a soluble transhydrogenase such as sthA (E.C. 1.6.1.2), and/or genetic modifications of one or more of zwf (E.C. 1.1.1.49), gnd (E.C. 1.1.1.44), and edd (E.C. 4.2.1.12). Sequences of these genes are available at <<www.metacyc.org>>, and also are available at <<www.ncbi.gov>> as well as <<www.ecocyc.org>>.

Polypeptides, such as encoded by the various specified genes, may be NADH- or NADPH¬dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1): 87-96 (1999),
Hall and Tomsett, Microbiology, 146 (Pt 6): 1399-406 (2000), and Dohr et al., Proc. Natl. Acad. Sci., 98 (1) : 81-86 (2001))."

### Redirecting Malonyl-CoA from Fatty Acid Synthesis to a Chemical Product

Compositions of the present disclosure, such as genetically modified microorganisms, comprise a production pathway for a chemical product in which malonyl-CoA is a substrate, and may also comprise one or more genetic modifications to reduce the activity of enzymes encoded by one or more of the fatty acid synthetase system genes. The compositions may be used in the methods and systems of the present disclosure.

Regarding microbial fermentation of a number of chemical products in many microorganisms of commercial fermentation interest, malonyl-CoA is a metabolic intermediate that, under normal growth conditions, is converted to fatty acids and derivatives thereof, such as phospholipids, that are then used in cell membranes and for other key cellular functions. For example, in Escherichia coli, the fatty acid synthase system is a type II or dissociated fatty acid synthase system. In this system the enzymes of fatty acid production pathway are encoded by distinct genes, and, common for many critical metabolic pathways, is well-regulated, including by downstream products inhibiting upstream enzymes.

In various microorganisms conversion of the metabolic intermediate malonyl-CoA to fatty acids via a fatty acid synthesis system (i.e., pathway or complex) is the only or the major use of malonyl-CoA. It has been determined that when a production pathway to an alternative chemical product exists in a microorganism, reducing such conversion of malonyl-CoA to fatty acids can improve metrics for production of that alternative chemical product (e.g., a polyketide or 3-HP). For example, in many microorganism cells the fatty acid synthase system comprises polypeptides that have the following enzymatic activities: malonyl-CoA-acyl carrier protein (ACP) transacylase; β-ketoacyl-ACP synthase; β-ketoacyl-ACP reductase; β-hydroxyacyl-ACP dehydratase; 3-hydroxyacyl-(acp) dehydratase; and enoyl-acyl carrier protein reductase (enoyl-ACP reductase). In various embodiments nucleic acid sequences that encode temperature-sensitive forms of these polypeptides may be introduced in place of the native enzymes, and when such genetically modified microorganisms are cultured at elevated temperatures (at which these thermolabile polypeptides become inactivated, partially or completely, due to alterations in protein structure or complete denaturation), there is observed an increase in a product such as 3-HP THN or flaviolin. In other embodiments other types of genetic modifications may be made to otherwise modulate, such as lower, enzymatic activities of one or more of these polypeptides. In various embodiments a result of such genetic modifications is to shift malonyl-CoA utilization so that there is a reduced conversion of malonyl-CoA to fatty acids, overall biomass, and
proportionally greater conversion of carbon source to a chemical product such as 3-HP. In various embodiments, the specific productivity for the microbially produced chemical product is unexpectedly high. Also, additional genetic modifications, such as to increase malonyl-CoA production, may be made for certain embodiments.

One enzyme, enoyl(acyl carrier protein) reductase (EC No. 1.3.1.9, also referred to as enoyl-ACP reductase) is a key enzyme for fatty acid biosynthesis from malonyl-CoA. In Escherichia coli this enzyme, FabI, is encoded by the gene fabl (See "Enoyl-Acyl Carrier Protein (fabl) Plays a Determinant Role in Completing Cycles of Fatty Acid Elongation in Escherichia coli," Richard J. Heath and Charles 0. Rock, J. Biol. Chem. 270:44, pp. 26538-26543 (1995).

The present invention may utilize a microorganism that is provided with a nucleic acid sequence (polynucleotide) that encodes a polypeptide having enoyl-ACP reductase enzymatic activity that may be modulated during a fermentation event. For example, a nucleic acid sequence encoding a temperature-sensitive enoyl-ACP reductase may be provided in place of the native enoyl-ACP reductase, so that an elevated culture temperature results in reduced enzymatic activity, which then results in a shifting utilization of malonyl-CoA to production of a desired chemical product. At such elevated temperature the enzyme is considered non-permissive, as is the temperature. One such sequence is a mutant temperature-sensitive fabl (fabI(TS)) of E. coli, SEQ ID NO:28 for DNA, SEQ ID NO:29 for protein.

It is appreciated that nucleic acid and amino acid sequences for enoyl-ACP reductase in species other than E. coli are readily obtained by conducting homology searches in known genomics databases, such as BLASTN and BLASTP. Approaches to obtaining homologues in other species and functional equivalent sequences are described herein. Accordingly, it is appreciated that the present invention may be practiced by one skilled in the art for many microorganism species of commercial interest.

Other approaches than a temperature-sensitive enoyl-ACP reductase may be employed as known to those skilled in the art, such as, but not limited to, replacing a native enoyl-ACP or enoyl-CoA reductase with a nucleic acid sequence that includes an inducible promoter for this enzyme, so that an initial induction may be followed by no induction, thereby decreasing enoyl-ACP or enoyl-CoA reductase enzymatic activity after a selected cell density is attained.

In some aspects, compositions, methods and systems of the present disclosure shift utilization of malonyl-CoA in a genetic modified microorganism, which comprises at least one enzyme of the fatty acid synthase system, such as enoyl-acyl carrier protein reductase (enoyl-ACP reductase) or enoyl-coenzyme A reductase (enoyl-CoA reductase), β-ketoacyl-ACP synthase or β-ketoacyl-CoA synthase malonyl-CoA-ACP, and may further comprise at least one
genetic modification of nucleic acid sequence encoding carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. More generally, addition of carbonate and/or bicarbonate may be used to increase bicarbonate levels in a fermentation broth.

In some aspects, the present disclosure comprises a genetically modified microorganism that comprises at least one genetic modification that provides, completes, or enhances a 3-HP production pathway effective to convert malonyl-CoA to 3-HP, and further comprises a genetic modification of carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. Related methods and systems utilize such genetically modified microorganism.

In some aspects, the present disclosure comprises a genetically modified microorganism that comprises at least one genetic modification that provides, completes, or enhances a 3-HP production pathway effective to convert malonyl-CoA to 3-HP, and further comprises a genetic modification of at least one enzyme of the fatty acid synthase system, such as enoyl-acyl carrier protein reductase (enoyl-ACP reductase) or enoyl-coenzyme A reductase (enoyl-CoA reductase), β-ketoacyl-ACP synthase or β-ketoacyl-CoA synthase , malonyl-CoA-ACP, and may further comprise a genetic modification of carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. Related methods and systems utilize such genetically modified microorganism.

In some aspects, the present disclosure comprises a genetically modified microorganism that comprises at least one genetic modification that provides, completes, or enhances a 3-HP production pathway effective to convert malonyl-CoA to 3-HP, and further comprises a genetic modification of carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. In some aspects, the present disclosure comprises a genetically modified microorganism that comprises at least one genetic modification that provides, completes, or enhances a 3-HP production pathway effective to convert malonyl-CoA to 3-HP, and further comprises a genetic
modification of at least one enzyme of the fatty acid synthase system, such as enoyl-acyl carrier protein reductase (enoyl-ACP reductase) or enoyl-coenzyme A reductase (enoyl-CoA reductase), β-ketoacyl-ACP synthase or β-ketoacyl-CoA synthase , malonyl-CoA-ACP, and may further comprise a genetic modification of carbonic anhydrase to increase bicarbonate levels in the microorganism cell and/or a supplementation of its culture medium with bicarbonate and/or carbonate, and may
In various embodiments the present disclosure is directed to a method of making a chemical product comprising: providing a selected cell density of a genetically modified microorganism population in a vessel, wherein the genetically modified microorganism comprises a production pathway for production of a chemical product from malonyl-CoA; and reducing enzymatic activity of at least one enzyme of the genetically modified microorganism's fatty acid synthase pathway.

In various embodiments, reducing the enzymatic activity of an enoyl-ACP reductase in a microorganism host cell results in production of 3-HP at elevated specific and volumetric productivity. In still other embodiments, reducing the enzymatic activity of an enoyl-CoA reductase in a microorganism host cell results in production of 3-HP at elevated specific and volumetric productivity.

Another approach to genetic modification to reduce enzymatic activity of these enzymes is to provide an inducible promoter that promotes one such enzyme, such as the enoyl-ACP reductase gene (e.g., fabl in E. coli). In such example this promoter may be induced (such as with isopropyl-u-D-thiogalactopyranoiside (IPTG)) during a first phase of a method herein, and after the IPTG is exhausted, removed or diluted out the second step, of reducing enoyl-ACP reductase enzymatic activity, may begin. Other approaches may be applied to control enzyme expression and activity such as are described herein and/or known to those skilled in the art.

While enoyl-CoA reductase is considered an important enzyme of the fatty acid synthase system, genetic modifications may be made to any combination of the polynucleotides (nucleic acid sequences) encoding the polypeptides exhibiting the enzymatic activities of this system, such as are listed herein. For example, FabB, β-ketoacyl-acyl carrier protein synthase I, is an enzyme in E. coli that is essential for growth and the biosynthesis of both saturated and unsaturated fatty acids. Inactivation of FabB results in the inhibition of fatty acid elongation and diminished cell growth as well as eliminating a futile cycle that recycles the malonate moiety of malonyl-ACP back to acetyl-CoA. FabF, β-ketoacyl-acyl carrier protein synthase II, is required for the synthesis of saturated fatty acids and the control membrane fluidity in cells. Both enzymes are inhibited by cerulenin.

It is reported that overexpression of FabF results in diminished fatty acid biosynthesis. It is proposed that FabF outcompetes FabB for association with FabD, malonyl-CoA:ACP transacylase. The association of FabB with FabD is required for the condensation reaction that initiates fatty acid elongation. (See Microbiological Reviews, Sept. 1993, p. 522-542 Vol. 57, No. 3; K. Magnuson et al., "Regulation of Fatty Acid Biosynthesis in Escherichia coli," American Society for Microbiology; W. Zha et al., "Improving cellular malonyl-CoA level in Escherichia coli via metabolic engineering," Metabolic Engineering 11 (2009) 192-198). An alternative to genetic modification to reduce such fatty acid synthase enzymes is to provide into a culture system a suitable inhibitor of one or more such enzymes. This approach may be practiced independently or in combination with the genetic modification approach. Inhibitors, such as cerulenin, thiolactomycin, and triclosan (this list not limiting) or genetic modifications directed to reduce activity of enzymes encoded by one or more of the fatty acid synthetase system genes may be employed, singly or in combination.

Without being bound to a particular theory, it is believed that reducing the enzymatic activity of enoyl-ACP reductase (and/or of other enzymes of the fatty acid synthase system) in a microorganism leads to an accumulation and/or shunting of malonyl-CoA, a metabolic intermediate upstream of the enzyme, and such malonyl-CoA may then be converted to a chemical product for which the microorganism cell comprises a metabolic pathway that utilizes malonyl-CoA. In certain compositions, methods and systems of the present invention the reduction of enzymatic activity of enoyl-ACP reductase (or, more generally, of the fatty acid synthase system) is made to occur after a sufficient cell density of a genetically modified microorganism is attained. This bi-phasic culture approach balances a desired quantity of catalyst, in the cell biomass which supports a particular production rate, with yield, which may be partly attributed to having less carbon be directed to cell mass after the enoyl-ACP reductase activity (and/or activity of other enzymes of the fatty acid synthase system) is/are reduced. This results in a shifting net utilization of malonyl-CoA, thus providing for greater carbon flux to a desired chemical product.

In various embodiments of the present disclosure the specific productivity is elevated and this results in overall rapid and efficient microbial fermentation methods and systems. In various embodiments the volumetric productivity also is substantially elevated.

In various embodiments a genetically modified microorganism comprises a metabolic pathway that includes conversion of malonyl-CoA to a desired chemical product, 3-hydroxypropionic acid (3-HP). This is viewed as quite advantageous for commercial 3-HP production economics and is viewed as an advance having clear economic benefit.

In various embodiments a genetically modified microorganism comprises a metabolic pathway that includes conversion of malonyl-CoA to a selected chemical product, selected from various polyketides such as those described herein. This is viewed as quite advantageous for commercial production economics for such polyketide chemical products and is viewed as an advance having clear economic benefit. Other chemical products also are disclosed herein.

The improvements in both specific and volumetric productivity parameters are unexpected and advance the art.

The reduction of enoyl-ACP reductase activity and/or of other enzymes of the fatty acid synthase system may be achieved in a number of ways, as is discussed herein.

By "means for modulating" the conversion of malonyl-CoA to fatty acyl-ACP or fatty acyl-CoA molecules, and to fatty acid molecules, is meant any one of the following: 1) providing in a microorganism cell at least one polynucleotide that encodes at least one polypeptide having activity of one of the fatty acid synthase system enzymes (such as recited herein), wherein the polypeptide so encoded has (such as by mutation and/or promoter substitution, etc., to lower enzymatic activity), or may be modulated to have (such as by temperature sensitivity, inducible promoter, etc.) a reduced enzymatic activity; 2) providing to a vessel comprising a microorganism cell or population an inhibitor that inhibits enzymatic activity of one or more of the fatty acid synthase system enzymes (such as recited herein), at a dosage effective to reduce enzymatic activity of one or more of these enzymes. These means may be provided in combination with one another. When a means for modulating involves a conversion, during a fermentation event, from a higher to a lower activity of the fatty acid synthetase system, such as by increasing temperature of a culture vessel comprising a population of genetically modified microorganism comprising a temperature-sensitive fatty acid synthetase system polypeptide (e.g., enoyl-ACP reductase), or by adding an inhibitor, there are conceived two modes - one during which there is higher activity, and a second during which there is lower activity, of such fatty acid synthetase system. During the lower activity mode, a shift to greater utilization of malonyl-CoA to a selected chemical product may proceed.

Once the modulation is in effect to decrease the noted enzymatic activity(ies), each respective enzymatic activity so modulated may be reduced by at least 10, at least 20, at least 30, at least 40, at least 50, at least 60, at least 70, at least 80, or at least 90 percent compared with the activity of the native, non-modulated enzymatic activity (such as in a cell or isolated). Similarly, the conversion of malonyl-CoA to fatty acyl-ACP or fatty acyl-CoA molecules may be reduced by at least 10, at least 20, at least 30, at least 40, at least 50, at least 60, at least 70, at least 80, or at least 90 percent compared with such conversion in a non-modulated cell or other system. Likewise, the conversion of malonyl-CoA to fatty acid molecules may be reduced by at least 10, at least 20, at least 30, at least 40, at least 50, at least 60, at least 70, at least 80, or at least 90 percent compared with such conversion in a non-modulated cell or other system.

Also Table 7 below provides additional information regarding genes and sequences that may be used in various embodiments of the invention. Information in this table is incorporated into the respective examples below.

**TABLE 7**

| **Enzyme Function** | **E.C. Classification** | **Gene Name(s)** |
|---|---|---|
| Phloroglucinol reductase | 1.3.1.57 | phloroglucinol reductase Eubacterium oxidoreducens 041). |
| 6-methylsalicylic-acid synthase | 2.3.1.165 | 6-MSAS |
| crotonyl-CoA reductase | 1.3.1.86 | ccr |
| acyl-coA thioesterase I | 3.1.1.5 | tesA |
| butanol dehydrogenase/ butanal dehydrogenase | 1.2.1.- | adhE2 |
| isobutanol dehydrogenase/isobutanal | 1.2.1.- | adhE |
| Butyryl-CoA dehydrogenase | 1.3.8.1 | bed |
| Electron transfer flavoprotein | 1.3.8.1 | etfAB |
| phosphotransbutyrylase | 2.3.1.19 | ptb |
| butyrate kinase | 2.7.2.7 | bukl |
| (S)-3-hydroxybutyryl-CoA dehydrogenase | 1.1.1.35 | hbd |
| 3-hydroxybutyryl-CoA dehydratase | 4.2.1.55 | crt |
| (R)-3 -hydroxybutyryl-CoA dehydrogenase | 1.1.1.100 | phaB |
| acetyl-coA acetyltransferase | 2.3.1.9 | phaA |
| Polyhydroxybutyrate polymerase | 2.3.1.- | phaC |
| 3-ketoacyl-CoA thiolase | 2.3.1.16 | fall |
| *Enoyl-CoA hydratase*/*3-hydroxybutyryl-CoA epimerase* | 4.2.1.17/5.1.2.3 | fadJ |
| trans-2-enoyl-CoA reductase (NAD+) | 1.3.1.44 | ter |
| butanoyl-CoA/ | | |
| 2-methylpropanoyl-CoA mutase | 5.4.99.13 | icmA, icmB |
| phloroisovalerophenon e synthase | 2.3.1.156 | VPS |
| Acyl-coA dehydrogenase | 1.3.99 | acdH |
| Hydroxymethylglutaryl -CoA synthase | 2.3.3.10 | hmgS |
| HMG-CoA reductase 1 | 1.1.1.34 | HMG1 |
| HMG-CoA reductase 1 | 1.1.1.34 | HMG2 |
| short chain enoyl-CoA hydratase | 4.2.1.17 | ECHS1 |
| 3-hydroxyisobutyryl-CoA hydrolase | 3.1.2.4 | Hibch |
| enoyl-CoA hydratase | 4.2.1.17 | ech |
| Phosphoenolpyruvate carboxylase | 4.1.1.31 | ppc |
| citrate synthase | 2.3.3.1 | gltA |
| phosphoenolpyruvate carboxykinase | 4.1.1.49 | pck/ pckA |

As to ph1D, the sequence listing is provided below:

### Production Pathway from Malonyl-CoA to 3-HP

In various embodiments the compositions, methods and systems of the present invention involve inclusion of a metabolic production pathway that converts malonyl-CoA to a chemical product of interest.

As one example, 3-HP is selected as the chemical product of interest.

Further as to specific sequences for 3-HP production pathway, malonyl-CoA reductase (mcr) from C. aurantiacus was gene synthesized and codon optimized by the services of DNA 2.0. The FASTA sequence is shown in SEQ ID NO:15 (gi142561982IgbIAAS20429.11malonyl-CoA reductase (Chloroflexus aurantiacus)).

Mcr has very few sequence homologs in the NCBI data base. Blast searches finds 8 different sequences when searching over the entire protein. Hence development of a pile-up sequences comparison is expected to yield limited information. However, embodiments of the present invention nonetheless may comprise any of these eight sequences, shown herein and identified as SEQ ID NOs:42 to 49, which are expected to be but are not yet confirmed to be bi-functional as to this enzymatic activity. Other embodiments may comprise mutated and other variant forms of any of SEQ ID NOs:42 to 49, as well as polynucleotides (including variant forms with conservative and other substitutions), such as those introduced into a selected microorganism to provide or increase 3-HP production therein.

The portion of a CLUSTAL 2.0.11 multiple sequence alignment identifies these eight sequences with respective SEQ ID NOs: 15, 42-49, as shown in the following table.

**TABLE 8**

| **Reference Nos.** | **Seq ID No** | **Genus Species** |
|---|---|---|
| gi142561982IgbIAAS20429.1 | 15 | *Chloroflexus aurantiacus* |
| gi11638481651reflYP_001636209 | 42 | *Chloroflexus aurantiacus* J-10-fl |
| giI2198481671reflYP_002462600 | 43 | *Chloroflexus aggregans* DSM 9485 |
| gi11567428801reflYP_001433009 | 44 | *Roseiflexus castenholzii* DSM 13941 |
| gi11486573071reflYP_001277512 | 45 | *Roseiflexus* sp. RS-1 |
| gi185708113IreflZP_01039179.1 | 46 | *Erythrobacter* sp. NAP1 |
| gi1254282228IreflZP_04957196.1 | 47 | gamma proteobacterium NOR51 -B |
| gi1254513883IreflZP_05125944.1 | 48 | gamma proteobacterium NOR5-3 |
| gi11195043131reflZP_01626393.1 | 49 | 3marine gamma proteobacterium HTCC208 |

Malonyl-CoA may be converted to 3-HP in a microorganism that comprises one or more of the following:
A bi-functional malonyl-CoA reductase, such as may be obtained from Chloroflexus aurantiacus and other microorganism species. By bi-functional in this regard is meant that the malonyl-CoA reductase catalyzes both the conversion of malonyl-CoA to malonate semialdehyde, and of malonate semialdehyde to 3-HP.

A mono-functional malonyl-CoA reductase in combination with a 3-HP dehydrogenase. By mono-functional is meant that the malonyl-CoA reductase catalyzes the conversion of malonyl-CoA to malonate semialdehyde.

Any of the above polypeptides may be NADH- or NADPH-dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1) : 87-96 (1999), Hall and Tomsett, Microbiology, 146 (Pt 6): 1399-406 (2000), and Dohr et al., Proc. Natl. Acad. Sci., 98 (1) : 81-86 (2001))."

Without being limiting, a bi-functional malonyl-CoA reductase may be selected from the malonyl-CoA reductase of Chloroflexus aurantiacus (such as from ATCC 29365) and other sequences. Also without being limiting, a mono-functional malonyl-CoA reductase may be selected from the malonyl-CoA reductase of Sulfolobus tokodaii (SEQ ID NO:83). As to the malonyl-CoA reductase of C. aurantiacus, that sequence and other species' sequences may also be bi-functional as to this enzymatic activity.

When a mono-functional malonyl-CoA reductase is provided in a microorganism cell, 3-HP dehydrogenase enzymatic activity also may be provided to convert malonate semialdehyde to 3-HP. As shown in the examples, a mono-functional malonyl-CoA reductase may be obtained by truncation of a bi-functional mono-functional malonyl-CoA, and combined in a strain with an enzyme that converts malonate semialdehyde to 3-HP.

Also, it is noted that another malonyl-CoA reductase is known in Metallosphaera sedula (Msed_709, identified as malonyl-CoA reductase/succinyl-CoA reductase).

By providing nucleic acid sequences that encode polypeptides having the above enzymatic activities, a genetically modified microorganism may comprise an effective 3-HP pathway to convert malonyl-CoA to 3-HP in accordance with the embodiments of the present disclosure.

Other 3-HP pathways, such as those comprising an aminotransferase (see, e.g., WO 2010/011874, published January 28, 2010), may also be provided in embodiments of a genetically modified microorganism of the present disclosure.

Incorporated into this section, the present disclosure provides for elevated specific and volumetric productivity metrics as to production of a selected chemical product, such as 3-hydroxypropionic acid (3-HP). In various embodiments, production of a chemical product, such as 3-HP, is not linked to growth.

In various embodiments, production of 3-HP, or alternatively one of its downstream products such as described herein, may reach at least 1, at least 2, at least 5, at least 10, at least 20, at least 30, at least 40, and at least 50 g/liter titer, such as by using one of the methods disclosed herein.

As may be realized by appreciation of the advances disclosed herein as they relate to commercial fermentations of selected chemical products, embodiments of the present disclosure may be combined with other genetic modifications and/or method or system modulations so as to obtain a microorganism (and corresponding method) effective to produce at least 10, at least 20, at least 30, at least 40, at least 45, at least 50, at least 80, at least 100, or at least 120 grams of a chemical product, such as 3-HP, per liter of final (e.g., spent) fermentation broth while achieving this with specific and/or volumetric productivity rates as disclosed herein.

In some embodiments a microbial chemical production event (i.e., a fermentation event using a cultured population of a microorganism) proceeds using a genetically modified microorganism as described herein, wherein the specific productivity is between 0.01 and 0.60 grams of 3-HP produced per gram of microorganism cell on a dry weight basis per hour (g 3-HP/g DCW-hr). In various embodiments the specific productivity is greater than 0.01, greater than 0.05, greater than 0.10, greater than 0.15, greater than 0.20, greater than 0.25, greater than
0.30, greater than 0.35, greater than 0.40, greater than 0.45, or greater than 0.50 g 3-HP/g DCW-hr. Specific productivity may be assessed over a 2, 4, 6, 8, 12 or 24 hour period in a particular microbial chemical production event. More particularly, the specific productivity for 3-HP or other chemical product is between 0.05 and 0.10, 0.10 and 0.15, 0.15 and 0.20, 0.20 and 0.25, 0.25 and 0.30, 0.30 and 0.35, 0.35 and 0.40, 0.40 and 0.45, or 0.45 and 0.50 g 3-HP/g DCW-hr., 0.50 and 0.55, or 0.55 and 0.60 g 3-HP/g DCW-hr. Various embodiments comprise culture systems demonstrating such productivity. Also, in various embodiments of the present disclosure the volumetric productivity achieved may be 0.25 g 3-HP (or other chemical product) per liter per hour (g (chemical product)/L-hr), may be greater than 0.25 g 3-HP (or other chemical product)/L-hr, may be greater than 0.50 g 3-HP (or other chemical product)/L-hr, may be greater than 1.0 g 3-HP (or other chemical product)/L-hr, may be greater than 1.50 g 3-HP (or other chemical product)/L-hr, may be greater than 2.0 g 3-HP (or other chemical product)/L-hr, may be greater than 2.50 g 3-HP (or other chemical product)/L-hr, may be greater than 3.0 g 3-HP (or other chemical product)/L-hr, may be greater than 3.50 g 3-HP (or other chemical product)/L-hr, may be greater than 4.0 g 3-HP (or other chemical product)/L-hr, may be greater than 4.50 g 3-HP (or other chemical product)/L-hr, may be greater than 5.0 g 3-HP (or other chemical product)/L-hr, may be greater than 5.50 g 3-HP (or other chemical product)/L-hr, may be greater than 6.0 g 3-HP (or other chemical product)/L-hr, may be greater than 6.50 g 3-HP (or other chemical product)/L-hr, may be greater than 7.0 g 3-HP (or other chemical product)/L-hr, may be greater than 7.50 g 3-HP (or other chemical product)/L-hr, may be greater than 8.0 g 3-HP (or other chemical product)/L-hr, may be greater than 8.50 g 3-HP (or other chemical product)/L-hr, may be greater than 9.0 g 3-HP (or other chemical product)/L-hr, may be greater than 9.50 g 3-HP (or other chemical product)/L-hr, or may be greater than 10.0 g 3-HP (or other chemical product)/L-hr.

In some embodiments, specific productivity as measured over a 24-hour fermentation (culture) period may be greater than 0.01, 0.05, 0.10, 0.20, 0.5, 1.0, 2.0, 3.0, 4.0, 5.0, 6.0, 7.0, 8.0, 9.0, 10.0, 11.0 or 12.0 grams of chemical product per gram DCW of microorganisms (based on the final DCW at the end of the 24-hour period).

In various aspects and embodiments of the present disclosure, there is a resulting substantial increase in microorganism specific productivity that advances the fermentation art and commercial economic feasibility of microbial chemical production, such as of 3-HP (but not limited thereto).

Stated in another manner, in various embodiments the specific productivity exceeds (is at least) 0.01 g chemical product/g DCW-hr, exceeds (is at least) 0.05 g chemical product/g DCW-hr, exceeds (is at least) 0.10 g chemical product/g DCW-hr, exceeds (is at least) 0.15 g
chemical product/g DCW-hr, exceeds (is at least) 0.20 g chemical product/g DCW-hr, exceeds (is at least) 0.25 g chemical product/g DCW-hr, exceeds (is at least) 0.30 g chemical product/g DCW-hr, exceeds (is at least) 0.35 g chemical product/g DCW-hr, exceeds (is at least) 0.40 g chemical product/g DCW-hr, exceeds (is at least) 0.45 g chemical product/g DCW-hr, exceeds (is at least) 0.50 g chemical product/g DCW-hr, exceeds (is at least) 0.60 g chemical product/g DCW-hr.

More generally, based on various combinations of the genetic modifications described herein, optionally in combination with supplementations described herein, specific productivity values for 3-HP, and for other chemical products described herein, may exceed 0.01 g chemical product/g DCW-hr, may exceed 0.05 g chemical product/g DCW-hr, may exceed 0.10 g chemical product/g DCW-hr, may exceed 0.15 g chemical product/g DCW-hr, may exceed 0.20 g chemical product/g DCW-hr, may exceed 0.25 g chemical product/g DCW-hr, may exceed 0.30 g chemical product/g DCW-hr, may exceed 0.35 g chemical product/g DCW-hr, may exceed 0.40 g chemical product/g DCW-hr, may exceed 0.45 g chemical product/g DCW-hr, and may exceed 0.50 g or 0.60 chemical product/g DCW-hr. Such specific productivity may be assessed over a 2, 4, 6, 8, 12 or 24 hour period in a particular microbial chemical production event.

The improvements achieved by embodiments of the present disclosure may be determined by percentage increase in specific productivity, or by percentage increase in volumetric productivity, compared with an appropriate control microorganism lacking the particular genetic modification combinations taught herein (with or without the supplements taught herein, added to a vessel comprising the microorganism population). For particular embodiments and groups thereof, such specific productivity and/or volumetric productivity improvements is/are at least 10, at least 20, at least 30, at least 40, at least 50, at least 100, at least 200, at least 300, at least 400, and at least 500 percent over the respective specific productivity and/or volumetric productivity of such appropriate control microorganism.

The specific methods and teachings of the specification, and/or cited references, may be incorporated into the examples. Also, production of 3-HP, or one of its downstream products such as described herein, may reach at least 1, at least 2, at least 5, at least 10, at least 20, at least 30, at least 40, and at least 50 g/liter titer in various embodiments.

The metrics may be applicable to any of the compositions, e.g., genetically modified microorganisms, methods, e.g., of producing 3-HP or other chemical products, and systems, e.g., fermentation systems utilizing the genetically modified microorganisms and/or methods disclosed herein.

It is appreciated that iterative improvements using the strategies and methods provided herein, and based on the discoveries of the interrelationships of the pathways and pathway portions, may lead to even greater production of a selected chemical product.

Any number of strategies may lead to development of a suitable modified enzyme suitable for use in a chemical production pathway. With regard to malonyl-CoA-reductase, one may utilize or modify an enzyme such as encoded by the sequences in the table immediately above, to achieve a suitable level of chemical production capability in a microorganism strain.

### Production Pathway from Malonyl-CoA to 3-HP - Additional Aspects

In various embodiments the compositions, methods and systems of the present disclosure involve inclusion of a metabolic production pathway that converts malonyl-CoA to a chemical product of interest. As one example, 3-HP is selected as the chemical product of interest.

Further as to specific sequences for 3-HP production pathway, malonyl-CoA reductase (mcr) from C. aurantiacus was gene synthesized and codon optimized by the services of DNA 2.0. The FASTA sequence is shown in SEQ ID NO:015 (gi1425619821gbIAAS20429.1I malonyl-CoA reductase (Chloroflexus aurantiacus)).

Mcr has very few sequence homologs in the NCBI data base. Blast searches finds 8 different sequences when searching over the entire protein. Hence development of a pile-up sequences comparison is expected to yield limited information. However, embodiments of the present invention nonetheless may comprise any of these eight sequences, which are expected to be but are not yet confirmed to be bi-functional as to this enzymatic activity. Other embodiments may comprise mutated and other variant forms of any of these sequences, as well as polynucleotides (including variant forms with conservative and other substitutions), such as those introduced into a selected microorganism to provide or increase 3-HP production therein.

The portion of a CLUSTAL 2.0.11 multiple sequence alignment identifies these eight sequences as shown in the following table.

| **Reference Nos.** | **Genus Species** |
|---|---|
| gii425619821gb I AAS20429.1 | *Chloroflexus aurantiacus* |
| 811163848165Iref I YP_001636209 | *Chloroflexus aurantiacus* J-10-fl |
| 8112198481671ref I YP_002462600 | *Chloroflexus aggregans* DSM 9485 |
| 8111567428801ref I YP_001433009 | *Roseiflexus castenholzii* DSM 13941 |
| 8111486573071ref I YP_001277512 | *Roseiflexus* sp. RS-1 |
| 81185708113Iref I ZP_01039179.1 | *Erythrobacter* sp. NAP1 |
| 8112542822281ref I ZP_04957196.1 | gamma proteobacterium NOR51 -B |
| 811254513883Iref I ZP_ 05125944.1 | gamma proteobacterium NOR5-3 |
| 8¹1119504313 I ref I ZP_01626393.1 | 3 marine gamma proteobacterium HTCC208 |

Malonyl-CoA may be converted to 3-HP in a microorganism that comprises one or more of the following:

A bi-functional malonyl-CoA reductase, such as may be obtained from Chloroflexus aurantiacus and other microorganism species. By bi-functional in this regard is meant that the malonyl-CoA reductase catalyzes both the conversion of malonyl-CoA to malonate semialdehyde, and of malonate semialdehyde to 3-HP.

A mono-functional malonyl-CoA reductase may be used in combination with a 3-HP dehydrogenase. By mono-functional is meant that the malonyl-CoA reductase catalyzes the conversion of malonyl-CoA to malonate semialdehyde.

Any of the above polypeptides may be NADH- or NADPH-dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1) : 87-96 (1999), Hall and Tomsett, Microbiology, 146 (Pt 6): 1399-406 (2000), and Dohr et al., Proc. Natl. Acad. Sci., 98 (1) : 81-86 (2001))."

Without being limiting, a bi-functional malonyl-CoA reductase may be selected from the malonyl-CoA reductase of Chloroflexus aurantiacus (such as from ATCC 29365) and other sequences. Also without being limiting, a mono-functional malonyl-CoA reductase may be selected from the malonyl-CoA reductase of Sulfolobus tokodaii. As to the malonyl-CoA reductase of C. aurantiacus, that sequence and other species' sequences may also be bi-functional as to this enzymatic activity.

When a mono-functional malonyl-CoA reductase is provided in a microorganism cell, 3-HP dehydrogenase enzymatic activity also may be provided to convert malonate semialdehyde to 3-HP. A mono-functional malonyl-CoA reductase may be obtained by truncation of a bi-functional mono-functional malonyl-CoA, and combined in a strain with an enzyme that converts malonate semialdehyde to 3-HP.

Also, it is noted that another malonyl-CoA reductase is known in Metallosphaera sedula (Msed_709, identified as malonyl-CoA reductase/succinyl-CoA reductase).

By providing nucleic acid sequences that encode polypeptides having the above enzymatic activities, a genetically modified microorganism may comprise an effective 3-HP pathway to convert malonyl¬CoA to 3-HP in accordance with the embodiments of the present disclosure.

Other 3-HP pathways, such as those comprising an aminotransferase (see, e.g., WO 2010/011874, published January 28, 2010), may also be provided in embodiments of a genetically modified microorganism of the present disclosure.

Any number of strategies may lead to development of a suitable modified enzyme suitable for use in a 3-HP production pathway. With regard to malonyl-CoA-reductase, one may utilize or modify an enzyme such as encoded by the sequences in the table immediately above, to achieve a suitable level of 3-HP production capability in a microorganism strain.

### Combinations of Genetic Modifications

In some embodiments, the genetically modified microorganism additionally comprises at least one genetic modification to increase, in the genetically modified microorganism, a protein function selected from the protein functions of Table 9 (Glucose transporter function (such as by galP), pyruvate dehydrogenase Elp, dihydrolipoamide acetyltransferase, and pyruvate dehydrogenase E3). In certain embodiments, the genetically modified microorganism comprises at least one genetic modification to increase two, three, or four protein functions selected from the protein functions of Table 9.

In some embodiments, such genetically modified microorganism additionally comprises at least one genetic modification to decrease protein functions selected from the protein functions of Table 10, lactate dehydrogenase, pyruvate formate lyase, pyruvate oxidase, phosphate acetyltransferase, histidyl phosphorylatable protein (of PTS), phosphoryl transfer protein (of PTS), and the polypeptide chain (of PTS).

In various embodiments, such genetically modified microorganism comprises at least one genetic modification to decrease enzymatic activity of two, three, four, five, six, or seven protein functions selected from the protein functions of Table 10. Also, in various embodiments at least one, or more than one, genetic modification is made to modify the protein functions of Table 11 in accordance with the Comments therein.including in Table 11.

It will be appreciated that, in various embodiments, there can be many possible combinations of increases in one or more protein functions of Table 9, with reductions in one or more protein functions of Table 9 in the genetically modified microorganism comprising at least one genetic modification to provide or increase malonyl-CoA-reductase protein function (i.e,
enzymatic activity). Protein functions can be independently varied, and any combination (i.e., a full factorial) of genetic modifications of protein functions in Tables 9, 10, and 11 herein can be adjusted by the methods taught and provided into said genetically modified microorganism.

In some embodiments, at least one genetic modification to decrease enzymatic activity is a gene disruption. In some embodiments, at least one genetic modification to decrease enzymatic activity is a gene deletion.

Certain embodiments of the disclosure additionally comprise a genetic modification to increase the availability of the cofactor NADPH, which can increase the NADPH/NADP+ ratio as may be desired. Non-limiting examples for such genetic modification are pgi (E.C. 5.3.1.9, in a mutated form), pntAB (E.C. 1.6.1.2), overexpressed, gapA (E.C. 1.2.1.12):gapN (E.C. 1.2.1.9, from Streptococcus mutans) substitution/replacement, and disrupting or modifying a soluble transhydrogenase such as sthA (E.C. 1.6.1.2), and/or genetic modifications of one or more of zwf (E.C. 1.1.1.49), gnd (E.C. 1.1.1.44), and edd (E.C. 4.2.1.12). Sequences of these genes are available at <<www.metacyc.org>>. Also, the sequences for the genes and encoded proteins for the E. coli gene names shown in Tables 9, 10, and 11 are provided in U.S. Provisional Patent Application No.: 61/246,141 are available at <<www.ncbi.gov>> as well as <<www.metacyc.org>> or «www.ecocyc.org».

In some embodiments, the genetic modification increases microbial synthesis of a selected chemical product above a rate or titer of a control microorganism lacking said at least one genetic modification to produce the selected chemical product. In some embodiments, the genetic modification is effective to increase enzymatic conversions to the selected chemical product by at least about 5 percent, at least about 10 percent, at least about 20 percent, at least about 30 percent, or at least about 50 percent above the enzymatic conversion of a control microorganism lacking the genetic modification.

**TABLE 9**

| **Enzyme Function** | **E.C. Classification** | **Gene Name in E. coli** |
|---|---|---|
| Glucose transporter | N/A | galP |
| Pyruvate dehydrogenase Elp | 1.2.4.1 | aceE |
| lipoate acetyltransferase / dihydrolipoamide acetyltransferase | 2.3.1.12 | aceF |
| Pyruvate dehydrogenase E3 (lipoamide dehydrogenase) | 1.8.1.4 | 1pd |

**TABLE 10**

| **Enzyme Function** | **E.C. Classification** | **Gene Name in E. coli** |
|---|---|---|
| Lactate dehydrogenase | 1.1.1.28 | ldhA |
| Pyruvate formate lyase (B "inactive") | 2.3.1.- | pflB |
| Pyruvate oxidase | 1.2.2.2 | poxB |
| Phosphate acetyltransferase | 2.3.1.8 | Pta |
| Heat stable, histidyl phosphorylatable protein (of PTS) | N/A | ptsH (HPr) |
| Phosphoryl transfer protein (of PTS) | N/A | ptsl |
| Polypeptide chain (of PTS) | N/A | Crr |

**TABLE 11**

| **Enzyme Function** | **E.C. Classification** | **Gene Name in E. coli** | **Comments** |
|---|---|---|---|
| 0 ketoacyl-acyl carrier protein synthase I 3-OXOACYL- ACP-SYNTHASE II MONOMER | 2.3.1.179 | fabF | Decrease function, including by mutation |
| | 2.3.1.41 | | |
| β-ketoacyl-ACP synthase I, 3-oxoacyl-ACP-synthase I | 2.3.1.41 2.3.1.- | fabB | Decrease function, including by mutation |
| Malonyl-CoA-ACP transacylase | 2.3.1.39 | fabD | Decrease function, including by mutation |
| enoyl acyl carrier protein reductase | 1.3.1.9, 1.3.1.10 | fabl | Decrease function, including by mutation |
| β-ketoacyl-acyl carrier protein synthase III | 2.3.1.180 | fabH | Decrease function, including by mutation |
| Carboxyl transferase subunit a subunit | 6.4.1.2 | accA | Increase function |
| Biotin carboxyl carrier protein | 6.4.1.2 | accB | Increase function |
| Biotin carboxylase subunit | 6.3.4.14 | accC | Increase function |
| Carboxyl transferase subunit β subunit | 6.4.1.2 | accD | Increase function |
| long chain fatty acyl thioesterase I | 3.1.2.2, 3.1.1.5 | tesA | Increase function |
| GDP pyrophosphokinase / GTP pyrophosphokinase | 2.7.6.5 | relA | Decrease function, including by mutation |
| GDP diphosphokinase / guanosine-3',5'-bis(diphosphate)3'-diphosphatase | 2.7.6.5, 3.1.7.2 | Spot | Decrease function, including by mutation |

Further with regard to descrasing enzyme function based on Table 11's teachings, any one or a combination of enzyme functions of the following may be decreased in a particular embodiment combined with other genetic modifications described herein: β-ketoacyl-ACP synthase I, 3-oxoacyl-ACP-synthase I; Malonyl-CoA-ACP transacylase; enoyl acyl carrier protein reductase; and β-ketoacyl-acyl carrier protein synthase III.

Moreover, in particular embodiments to more effectively redirect malonyl-CoA from fatty acid synthesis and toward the biosynthesis of such a desired chemical product, genetic modifications are made to:
(a) increase production along the biosynthetic pathway that includes malonyl-CoA and leads to a desired chemical product;
(b) reduce the activity of one or more, or of two or more, or of three or more of enoyl acyl carrier protein (ACP) reductase, B-ketoacyl-acyl carrier protein synthase I (such as fabB), B-ketoacyl-acyl carrier protein synthase II, and malonyl-CoA-acyl carrier protein transacylase; and optionally
(c) increase the production of coenzyme A in the microorganism cell.

Accordingly, as described in various sections above, some compositions, methods and systems of the present disclosure comprise providing a genetically modified microorganism that comprises both a production pathway to a selected chemical product, and a modified polynucleotide that encodes an enzyme of the fatty acid synthase system that exhibits reduced activity, so that utilization of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such embodiments. Providing additional copies of polynucleotides that encode polypeptides exhibiting these enzymatic activities is shown to increase production of a selected chemical. Other ways to increase these respective enzymatic activities is known in the art and may be applied to various embodiments of the present disclosure. SEQ ID NOs for these polynucleotides and polypeptides of E. coli are: acetyl-CoA carboxylase (accABCD, SEQ ID NOs:30-37); and NADPH-dependent transhydrogenase (SEQ ID NOs:38-41), also referred to as pyridine nucleotide transhydrogenase, pntAB in E. coli).

Also, without being limiting, a first step in some multi-phase method embodiments of making a chemical product may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family Enterobacteriaceae, such as E. coli, where the genetically modified microorganism comprises a metabolic pathway that converts malonyl-CoA to molecules of a selected chemical. Also, in various embodiments an inoculum of a microorganism of the present disclosure is cultured in a vessel so that the cell density increases to a cell density suitable for reaching a production level of a selected chemical that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such embodiments provide the selected cell density according to the first noted step of the method.

Also without being limiting, a subsequent step may be exemplified by two approaches, which also may be practiced in combination in various embodiments. A first approach provides a genetic modification to the genetically modified microorganism such that its enoyl-ACP reductase enzymatic activity may be controlled. As one example, a genetic modification may be made to substitute for the native enoyl-ACP reductase a temperature-sensitive mutant enoyl-ACP reductase (e.g., fabEs in E. coli). The latter may exhibit reduced enzymatic activity at temperatures above 30 C but normal enzymatic activity at 30 C, so that elevating the culture temperature to, for example to 34C, 35C, 36C, 37C or even 42C, reduces enzymatic activity of enoyl¬ACP reductase. In such case, more malonyl-CoA is converted to 3-HP or another chemical product than at 30 C, where conversion of malonyl-CoA to fatty acids is not impeded by a less effective enoyl-ACP reductase.

For the second approach, an inhibitor of enoyl-ACP reductase, or another of the fatty acid synthase enzyme, is added to reduce conversion of malonyl-CoA to fatty acids. For example, the inhibitor cerulenin is added at a concentration that inhibits one or more enzymes of the fatty acid synthase system. FIG. 2A depicts relevant pathways and shows three inhibitors - thiolactomycin, triclosan, and cerulenin, next to the enzymes that they inhibit. Encircled E. coli gene names indicate a temperature-sensitive mutant is available for the polypeptide encoded by the gene. FIG. 2B provides a more detailed depiction of representative enzymatic conversions and exemplary E. coli genes of the fatty acid synthetase system that was more generally depicted in FIG. 2A. This listing of inhibitors of microorganism fatty acid synthetase enzymes is not
meant to be limiting. Other inhibitors, some of which are used as antibiotics, are known in the art and include, but are not limited to, diazaborines such as thienodiazaborine, and, isoniazid.

In some embodiments, the genetic modification increases microbial synthesis of a selected chemical above a rate or titer of a control microorganism lacking said at least one genetic modification to produce a selected chemical. In some embodiments, the genetic modification is effective to increase enzymatic conversions to a selected chemical by at least about 5 percent, at least about 10 percent, at least about 20 percent, at least about 30 percent, or at least about 50 percent above the enzymatic conversion of a control microorganism lacking the genetic modification.

Genetic modifications as described herein may include modifications to reduce enzymatic activity of any one or more of: β-ketoacyl-ACP synthase I, 3-oxoacyl-ACP-synthase I; Malonyl-CoA-ACP transacylase; enoyl acyl carrier protein reductase; and β-ketoacyl-acyl carrier protein synthase III.

Accordingly, as described in various sections above, some compositions, methods and systems of the present disclosure comprise providing a genetically modified microorganism that comprises both a production pathway to a selected chemical product, such as a selected chemical, and a modified polynucleotide that encodes an enzyme of the fatty acid synthase system that exhibits reduced activity, so that utilization of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such embodiments. Providing additional copies of polynucleotides that encode polypeptides exhibiting these enzymatic activities is shown to increase a selected chemical production. Other ways to increase these respective enzymatic activities is known in the art and may be applied to various embodiments of the present disclosure. SEQ ID NOs for these polynucleotides and polypeptides of E. coli are: acetyl-CoA carboxylase (accABCD, SEQ ID NOs:30-37); and NADPH-dependent transhydrogenase (SEQ ID NOs:38-41), also referred to as pyridine nucleotide transhydrogenase, pntAB in E. coli).

Also, without being limiting, a first step in some multi-phase method embodiments of making a chemical product may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family Enterobacteriaceae, such as E. coli, where the genetically modified microorganism comprises a
metabolic pathway that converts malonyl-CoA to a selected chemical molecules. For example, genetic modifications may include the provision of at least one nucleic acid sequence that encodes a gene encoding the enzyme malonyl-CoA reductase in one of its bi-functional forms, or that encodes genes encoding a mono-functional malonyl-CoA reductase and an NADH- or NADPH-dependent 3-hydroxypropionate dehydrogenase (e.g., ydfG or mmsB from E. coli, or mmsB from Pseudomonas aeruginosa). In either case, when provided into an E. coli host cell, these genetic modifications complete a metabolic pathway that converts malonyl-CoA to a selected chemical. This inoculum is cultured in the vessel so that the cell density increases to a cell density suitable for reaching a production level of a selected chemical that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such embodiments provide the selected cell density according to the first noted step of the method.

Thus, for various embodiments of the disclosure the genetic manipulations to any pathways described herein also may include various genetic manipulations, including those directed to change regulation of, and therefore ultimate activity of, an enzyme or enzymatic activity of an enzyme identified in any of the respective pathways. Such genetic modifications may be directed to transcriptional, translational, and post-translational modifications that result in a change of enzyme activity and/or selectivity under selected and/or identified culture conditions. Thus, in various embodiments, to function more efficiently, a microorganism may comprise one or more gene deletions. For example, for a particular embodiment in E. coli, the genes encoding lactate dehydrogenase (ldhA), phosphate acetyltransferase (pta), pyruvate oxidase (poxB) and pyruvate-formate lyase (pflB) may be deleted. Additionally, a further deletion or other modification to reduce enzymatic activity, of multifunctional 2-keto-3-deoxygluconate 6-phosphate aldolase and 2-keto-4-hydroxyglutarate aldolase and oxaloacetate decarboxylase (eda in E. coli), may be provided to various strains. Further to the latter, in various embodiments combined with such reduction of enzymatic activity of multifunctional 2-keto-3-deoxygluconate 6-phosphate aldolase and 2-keto-4-hydroxyglutarate aldolase and oxaloacetate decarboxylase (eda in E. coli), further genetic modifications may be made to increase a glucose transporter (e.g. galP in E. coli) and/or to decrease activity of one or more of heat stable, histidyl phosphorylatable protein (of PTS) (ptsH (HPr) in E. coli), phosphoryl transfer protein (of PTS) (ptsl in E. coli), and the polypeptide chain of PTS (Crr in E. coli).

Gene deletions may be accomplished by mutational gene deletion approaches, and/or starting with a mutant strain having reduced or no expression of one or more of these enzymes, and/or other methods known to those skilled in the art.

Aspects of the invention also regard provision of multiple genetic modifications to improve microorganism overall effectiveness in converting a selected carbon source into a chemical product such as 3-HP. Particular combinations are shown, such as in the Examples, to increase specific productivity, volumetric productivity, titer and yield substantially over more basic combinations of genetic modifications.

Further to FIG. 1 genetic modifications, appropriate additional genetic modifications can provide further improved production metrics. Various strains may comprise genetic modifications for a selected chemical, and additional genetic modifications as disclosed herein (including a particular genetic modification regarding the fatty acid synthase system, not to be limiting, such modifications more generally disclosed elsewhere herein). The embodiment of FIG. 1 depicts a number of genetic modifications in combination, however in various embodiments of the present invention other combinations of the genetic modifications of these enzymatic functions may be provided to achieve a desired level of increased rate, titer and yield as to bio-production of a chemical product.

Additional genetic modifications may be provided in a microorganism strain of the present invention. As one example, a deletion, of multifunctional 2-keto-3-deoxygluconate 6-phosphate aldolase and 2- keto-4-hydroxyglutarate aldolase and oxaloacetate decarboxylase (eda in E. coli), may be provided to various strains.

For example, the ability to utilize sucrose may be provided, and this would expand the range of feed stocks that can be utilized to produce a selected chemical or other chemical products. Common laboratory and industrial strains of E. coli, such as the strains described herein, are not capable of utilizing sucrose as the sole carbon source. Since sucrose, and sucrose-containing feed stocks such as molasses, are abundant and often used as feed stocks for the production by microbial fermentation, adding appropriate genetic modifications to permit uptake and use of sucrose may be practiced in strains having other features as provided herein. Various sucrose uptake and metabolism systems are known in the art (for example, U.S. Pat. No. 6,960,455). These and other approaches may be provided in strains of the present invention. The examples provide at least two approaches.

Also, genetic modifications may be provided to add functionality for breakdown of more complex carbon sources, such as cellulosic biomass or products thereof, for uptake, and/or for utilization of such carbon sources. For example, numerous cellulases and cellulase-based cellulose degradation systems have been studied and characterized (see, for example,
Beguin, P and Aubert, J-P (1994) FEMS Microbial. Rev. 13: 25-58; Ohima, K. et al. (1997) Biotechnol. Genet. Eng. Rev. 14: 365414).

In addition to the above-described genetic modifications, in various embodiments genetic modifications also are provided to increase the pool and availability of the cofactor NADPH, and/or, consequently, the NADPH/NADP+ ratio. For example, in various embodiments for E. coli, this may be done by increasing activity, such as by genetic modification, of one or more of the following genes- pgi (in a mutated form), pntAB, overexpressed, gapA:gapN substitution/replacement, and disrupting or modifying a soluble transhydrogenase such as sthA, and/or genetic modifications of one or more of zwf, gnd, and edd.

Any such genetic modifications may be provided to species not having such functionality, or having a less than desired level of such functionality.

More generally, and depending on the particular metabolic pathways of a microorganism selected for genetic modification, any subgroup of genetic modifications may be made to decrease cellular production of fermentation product(s) selected from the group consisting of acetate, acetoin, acetone, acrylic, malate, fatty acid ethyl esters, isoprenoids, glycerol, ethylene glycol, ethylene, propylene, butylene, isobutylene, ethyl acetate, vinyl acetate, other acetates, 1,4-butanediol, 2,3-butanediol, butanol, isobutanol, sec-butanol, butyrate, isobutyrate, 2-OH-isobutryate, 3-OH-butyrate, ethanol, isopropanol, D-lactate, L-lactate, pyruvate, itaconate, levulinate, glucarate, glutarate, caprolactam, adipic acid, propanol, isopropanol, fusel alcohols, and 1,2- propanediol, 1,3-propanediol, formate, fumaric acid, propionic acid, succinic acid, valeric acid, and maleic acid. Gene deletions may be made as disclosed generally herein, and other approaches may also be used to achieve a desired decreased cellular production of selected fermentation products.

### Elongase Pathway

In various embodiments an elongase is used in a metabolic pathway to produce a selected chemical product. The following paragraphs are provided to describe elongases and their utilization. Trypanosoma brucei, a eukaryotic human parasite, is known to evade the host immune response by synthesizing a specific surface coating comprised of glycoproteins tethered to fatty acids anchored into the membrane. To accommodate the surface coating, T brucei produces large quantities of the anchor molecule, which is composed exclusively of C14 saturated fatty acid (myristic acid). Myristic acid synthesis in T. brucei is initiated by the formation of butyryl-CoA and utilizes membrane-bound elongation enzymes, similar to those typically used to extend fatty acids, to condense malonyl-CoA with the growing acyl chain . This particular ELO system
esterifies the growing fatty acid chain to CoA rather than ACP like the bacterial and other microbial counterparts (see Lee et al, Cell 126, 691-699, 2006 and Cronan, Cell, 126, 2006). This is in contrast to typical bacterial fatty acid elongation which is initiated following the formation of acetoacetyl acyl-ACP from malonyl-ACP. Microbial fatty acid synthesis is then catalysed in the cytosol by multiple soluble proteins, each responsible for a single reaction where the growing fatty acid chain is esterified to an acyl carrier protein (ACP). The carbon-donor molecule is a malonyl-ACP which extends the growing carbon chain length by 2 carbons at a time.

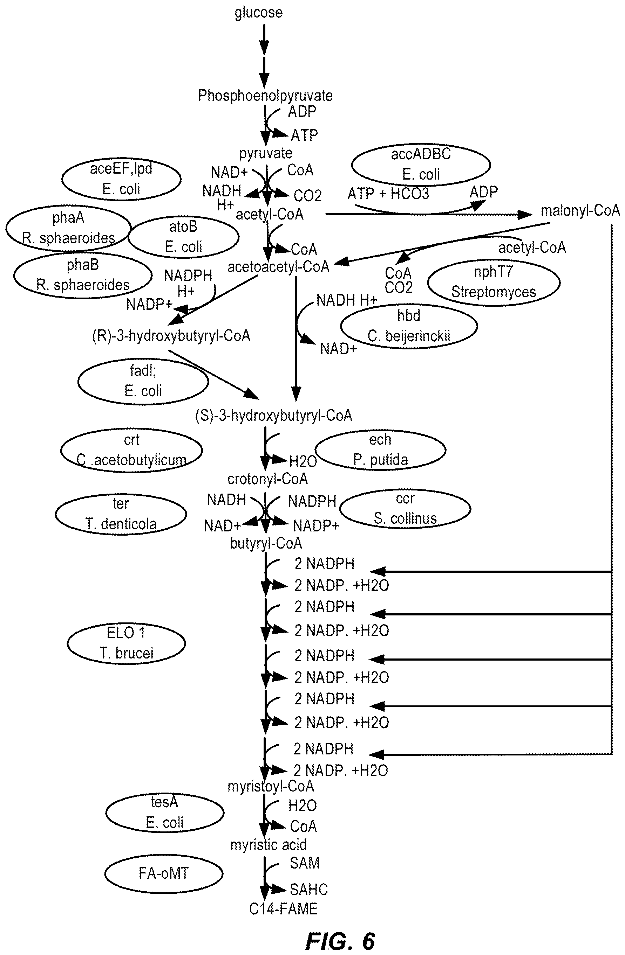

A proposed elongase-utilizing pathway (see FIG. 6) builds from PHB and n-butanol production pathways. The first committed step requires the formation of acetoacetyl-CoA, which is traditionally catalysed by either the b-ketothiolase (phaA) or acetyl-CoA acetyltransferase (atoB) using 2 acetyl-CoA molecules as a substrate. A recently reported malonyl-CoA dependent route for synthesis of acetoacetyl-CoA in Streptomyces sp. has been cloned and expressed in E. coli (Okamura, et al., PNAS, 107, 25, 2010). When considered with other aspects of the disclosure, including those increasing malonyl-CoA pools and fluxes, this reaction appears to provide an attractive alternative to the common route to acetoacetyl-CoA formation, which is an unfavorable reaction, preferring the hydrolysis of acetoacetyl-coA The novel enzyme, nphT7, catalyzes the irreversible reaction to form acetoacetyl-CoA from malonyl-CoA and acetyl-CoA and would allow for two driving forces towards acetoacetyl-CoA production (irreversibility and release of CO2). The protein and oligonucleotide sequences for NphT7 (AB540131.1 GI:299758081) are provided below (SEQ ID NOs:159; 160):

### SEQ ID NO:159

### SEQ ID NO:160

| | | | | | | |
|---|---|---|---|---|---|---|
| 1 | cctgcaggcc | gtcgagggcg | cctggaagga | ctacgcggag | caggacggcc | ggtcgctgga |
| 61 | ggagttcgcg | gcgttcgtct | accaccagcc | gttcacgaag | atggcctaca | aggcgcaccg |
| 121 | ccacctgctg | aacttcaacg | gctacgacac | cgacaaggac | gccatcgagg | gcgccctcgg |
| 181 | ccagacgacg | gcgtacaaca | acgtcatcgg | caacagctac | accgcgtcgg | tgtacctggg |
| 241 | cctggccgcc | ctgctcgacc | aggcggacga | cctgacgggc | cgttccatcg | gcttcctgag |
| 301 | ctacggctcg | ggcagcgtcg | ccgagttctt | ctcgggcacc | gtcgtcgccg | ggtaccgcga |
| 361 | gcgtctgcgc | accgaggcga | accaggaggc | gatcgcccgg | cgcaagagcg | tcgactacgc |
| 421 | cacctaccgc | gagctgcacg | agtacacgct | cccgtccgac | ggcggcgacc | acgccacccc |
| 481 | ggtgcagacc | accggcccct | tccggctggc | cgggatcaac | gaccacaagc | gcatctacga |
| 541 | ggcgcgctag | cgacacccct | cggcaacggg | gtgcgccact | gttcggcgca | ccccgtgccg |
| 601 | ggctttcgca | cagctattca | cgaccatttg | aggggcgggc | agccgcatga | ccgacgtccg |
| 661 | attccgcatt | atcggtacgg | gtgcctacgt | accggaacgg | atcgtctcca | acgatgaagt |
| 721 | cggcgcgccg | gccggggtgg | acgacgactg | gatcacccgc | aagaccggta | tccggcagcg |
| 781 | tcgctgggcc | gccgacgacc | aggccacctc | ggacctggcc | acggccgcgg | ggcgggcagc |
| 841 | gctgaaagcg | gcgggcatca | cgcccgagca | gctgaccgtg | atcgcggtcg | ccacctccac |
| 901 | gccggaccgg | ccgcagccgc | ccacggcggc | ctatgtccag | caccacctcg | gtgcgaccgg |
| 961 | cactgcggcg | ttcgacgtca | acgcggtctg | ctccggcacc | gtgttcgcgc | tgtcctcggt |
| 1021 | ggcgggcacc | ctcgtgtacc | ggggcggtta | cgcgctggtc | atcggcgcgg | acctgtactc |
| 1081 | gcgcatcctc | aacccggccg | accgcaagac | ggtcgtgctg | ttcggggacg | gcgccggcgc |
| 1141 | aatggtcctc | gggccgacct | cgaccggcac | gggccccatc | gtccggcgcg | tcgccctgca |
| 1201 | caccttcggc | ggcctcaccg | acctgatccg | tgtgcccgcg | ggcggcagcc | gccagccgct |
| 1261 | ggacacggat | ggcctcgacg | cgggactgca | gtacttcgcg | atggacgggc | gtgaggtgcg |
| 1321 | ccgcttcgtc | acggagcacc | tgccgcagct | gatcaagggc | ttcctgcacg | aggccggggt |
| 1381 | cgacgccgcc | gacatcagcc | acttcgtgcc | gcatcaggcc | aacggtgtca | tgctcgacga |
| 1441 | ggtcttcggc | gagctgcatc | tgccgcgggc | gaccatgcac | cggacggtcg | agacctacgg |
| 1501 | caacacggga | gcggcctcca | tcccgatcac | catggacgcg | gccgtgcgcg | ccggttcctt |
| 1561 | ccggccgggc | gagctggtcc | tgctggccgg | gttcggcggc | ggcatggccg | cgagcttcgc |
| 1621 | cctgatcgag | tggtagtcgc | ccgtaccacc | acagcggtcc | ggcgccacct | gttccctgcg |
| 1681 | ccgggccgcc | ctcggggcct | ttaggcccca | caccgcccca | gccgacggat | tcagtcgcgg |
| 1741 | cagtacctca | gatgtccgct | gcgacggcgt | cccggagagc | ccgggcgaga | tcgcgggccc |
| 1801 | ccttctgctc | gtccccggcc | cctcccgcga | gcaccacccg | cggcggacgg | ccgccgtcct |
| 1861 | ccgcgatacg | ccgggcgagg | tcgcaggcga | gcacgccgga | cccggagaag | ccccccagca |
| 1921 | ccagcgaccg | gccgactccg | tgcgcggcca | gggcaggctg | cgcgccgtcg | acgtcggtga |
| 1981 | gcagcaccag | gagctcctgc | ggcccggcgt | agaggtcggc | cagccggtcg | tagcaggtcg |
| 2041 | cgggcgcgcc | cggcggcggg | atcagacaga | tcgtgcccgc | ccgctcgtgc | ctcgccgccc |
| 2101 | gcagcgtgac | cagcggaatg | tcccgcccag | ctccgga | | |

The immediate next step in the pathway involves the reduction of acetoacetyl-CoA to 3-HB-CoA. There are two primary routes characterized to convert acetoacetyl-CoA to (S)3-HB-CoA. The first is catalysed by an NADH-dependent (S)-3HB-CoA dehydrogenase, which has been well characterized in C. beijerinckii.(Tseng, et al., AEM, 75, 10, 2009). The second is an NADPH-dependent (R)-3HB-CoA dehydrogenase followed by a non-specific epimerase reaction in E. coli to convert (R) to (S)-3HB-CoA. The NADH-dependent dehydrogenase is the more desirable activity to pursue due to higher reported specific activities, direct conversion to (S)-3HB-CoA, and more thermodynamically favourable reaction.

The crotonase reaction (3HB-CoA -> crotonyl-CoA) is immediately followed by a reduction to form butyryl-CoA, which is the substrate for the ELO1 elongase . The trans-2-enoyl-CoA reductase, ter, from T denticola has been characeterized as an NADH-dependent reductase specific for the conversion of crotonyl¬CoA to butyryl-CoA without flavoproteins (Tucci, et al. PEBS Letters, 581, 2007). Ter has been cloned and expressed in E. coli and in vitro enzyme characterization have suggested irreversible activity in the desired direction of butyryl-CoA production (Shen, et al., AEM, 77, 9, 2011). Ccr from S. collinus, which has been reported to catalyze the same flavoprotein-independent reaction, has also been expressed in E. coli. However, ccr requires NADPH as a cofactor.

### VII. COMPOSITIONS OF MATTER

In various embodiments, a method is provided for producing a C4-C18 fatty acid, the method comprising combining a carbon source and a microorganism cell culture to produce the C4-C18 fatty acid. For example, the method includes combing a caron source and a microorganism cell culture, where the cell culture comprises an inhibitor of fatty acid synthase and/or the microorganism of said cell culture is genetically modified for reduced enzymatic activity in at least one of an organism's native fatty acid synthase pathway enzymes, providing for reduced conversion of malonyl-CoA to fatty acyl-ACPs; and the microorganism of said cell culture additionally has one or more genetic modifications conferring fatty acid production. In various embodiments, the C4-C18 fatty acid is selected from the group consisting of C4, C6, C8, C10, C12, C14, C16 and C18 fatty acids. Preferably, the fatty acid production occurs through the synthesis of fatty acyl-coAs of chain length 4-18. Alternatively, the fatty acid production occurs through the synthesis of fatty acyl-coAs via the expression of at least one elongase enzyme.

The fatty acids produced according to the method exist as a mixture. In various embodiments, the mixture is processed to remove cellular debris. However, residual components may be present in the mixture. For example, the mixture may further contain E. coli DNA. Preferably, the mixture consists essentially of C4-C18 compounds. For example, preferably the mixture is essentially free of one or more additional components selected from the group consisting of opacifying agents, pearlescent aids, astringents anti-acne agents, anti-caking agents, antimicrobial agents, antioxidants, biocides, pH modifiers, skin treating agents, vitamins, and humectants. In various embodiments, the mixture is essentially free of one or more essential oils selected from the group consisting of almond, anise, basil, bay, bergamot, bitter almond, camphor, cassia, cedarwood, chamomile, cinnamon, citronella, clove, clove bud, coriander, cypress, eucalyptus, fennel, fir, frankincense, geranium, ginger, grapefruit, jasmine, lavender, lemon, lemongrass, lime, litsea, marigold, marjoram, myrtle, neroli, nutmeg, oakmoss, orange, palmorosa, patchouli, pennyroyal, peppermint, pine, rose, rosemary, sage, sandalwood, spearmint, spruce, sweet birch, tangerine, tea tree, vanilla, vetivert, white thyme, and wintergreen.

In various embodiments, the mixture is isolated from fermentation media, and processed as a component in a consumer product. For example, the consumer product is selected from the group consisting of a detergent, soap, resin, emulsifier, lubricant, grease, and wax. The mixture may be incorporated into the consumer product as the same chemical entity as produced by the genetically modified organism, or the mixture may be further processed as a mixture of fatty acids, fatty alcohols, fatty acid esters, fatty aldehydes, fatty amines, fatty amides, or fatty acid salts.

### VIII. SEPARATION AND PURIFICATION FROM FERMENTATION

A selected chemical product may be separated and purified by the approaches described in the following paragraphs, taking into account that many methods of separation and purification are known in the art and the following disclosure is not meant to be limiting. Osmotic shock, sonication, homogenization, and/or a repeated freeze-thaw cycle followed by filtration and/or centrifugation, among other methods, such as pH adjustment and heat treatment, may be used to produce a cell-free extract from intact cells. Any one or more of these methods also may be employed to release the selected chemical producxt from cells as an extraction step. Further as to general processing of a bio-production broth comprising a selected chemical product, various methods may be practiced to remove biomass and/or separate the chemical product from the culture broth and its components, including centrifugation, filtration, extraction, chemical conversion such as esterification, distillation, crystallization, chromatography, and ion-exchange, in various forms. Additionally, cell rupture may be conducted as needed to release a selected chemical from the cell mass, such as by sonication, homogenization, pH adjustment or heating. a selected chemical may be further separated and/or purified by methods known in the art, including any combination of one or more of centrifugation, liquid-liquid separations, including extractions such as solvent extraction, reactive extraction, two-phase aqueous extraction and two-phase solvent extraction, membrane separation technologies, distillation, evaporation, ion-exchange chromatography, adsorption chromatography, reverse phase chromatography and crystallization. Any of the above methods may be applied to a portion of a bio-production broth (i.e., a fermentation broth, whether made under aerobic, anaerobic, or microaerobic conditions), such as may be removed from a bio-production event gradually or periodically, or to the broth at termination of a bio-production event.

Polypeptides, such as encoded by the various specified genes, may be NADH- or NADPH-dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1) : 87-96 (1999), Hall and Tomsett, Microbiology, 146 (Pt 6): 1399-406 (2000), and Dohr et al., Proc. Natl. Acad. Sci., 98 (1) : 81-86 (2001))."

In various embodiments, bio-production of a selected chemical product may reach at least 1, at least 2, at least 5, at least 10, at least 20, at least 30, at least 40, and at least 50 g/liter titer, such as by using one of the methods disclosed herein.

As may be realized by appreciation of the advances disclosed herein as they relate to commercial fermentations of selected chemical products, embodiments of the present disclosure may be combined with other genetic modifications and/or method or system modulations so as to obtain a microorganism (and corresponding method) effective to produce at least 10, at least 20, at least 30, at least 40, at least 45, at least 50, at least 80, at least 100, or at least 120 grams of a chemical product per liter of final (e.g., spent) fermentation broth while achieving this with specific and/or volumetric productivity rates as disclosed herein.

In some embodiments a microbial chemical bio-production event (i.e., a fermentation event using a cultured population of a microorganism) proceeds using a genetically modified microorganism as described herein, wherein the specific productivity is between 0.01 and 0.60 grams of selected chemical product produced per gram of microorganism cell on a dry weight basis per hour (g chemical product/g DCW-hr). In various embodiments the specific productivity is greater than 0.01, greater than 0.05, greater than 0.10, greater than 0.15, greater than 0.20, greater than 0.25, greater than 0.30, greater than 0.35, greater than 0.40, greater than 0.45, or greater than 0.50 g chemical product/g DCW-hr. Specific productivity may be assessed over a 2, 4, 6, 8, 12 or 24 hour period in a particular microbial chemical production event. More particularly, the specific productivity for a chemical product is between 0.05 and 0.10, 0.10 and 0.15, 0.15 and 0.20, 0.20 and 0.25, 0.25 and 0.30, 0.30 and 0.35, 0.35 and 0.40, 0.40 and 0.45, or 0.45 and 0.50 g chemical product/g DCW-hr., 0.50 and 0.55, or 0.55 and 0.60 g chemical product/g DCW-hr. Various embodiments comprise culture systems demonstrating such productivity.

In some embodiments, specific productivity as measured over a 24-hour fermentation (culture) period may be greater than 0.01, 0.05, 0.10, 0.20, 0.5, 1.0, 2.0, 3.0, 4.0, 5.0, 6.0, 7.0, 8.0, 9.0, 10.0, 11.0 or 12.0 grams of chemical product per gram DCW of microorganisms (based on the final DCW at the end of the 24-hour period).

In various aspects and embodiments of the present disclosure, there is a resulting substantial increase in microorganism specific productivity that advances the fermentation art and commercial economic feasibility of microbial chemical production, such as of phloroglucinol or resorcinol.

Stated in another manner, in various embodiments the specific productivity exceeds (is at least) 0.01 g chemical product/g DCW-hr, exceeds (is at least) 0.05 g chemical product/g DCW-hr, exceeds (is at least) 0.10 g chemical product/g DCW-hr, exceeds (is at least) 0.15 g chemical product/g DCW-hr, exceeds (is at least) 0.20 g chemical product/g DCW-hr, exceeds (is at least) 0.25 g chemical product/g DCW-hr, exceeds (is at least) 0.30 g chemical product/g DCW-hr, exceeds (is at least) 0.35 g chemical product/g DCW-hr, exceeds (is at least) 0.40 g chemical product/g DCW-hr, exceeds (is at least) 0.45 g chemical product/g DCW-hr, exceeds (is at least) 0.50 g chemical product/g DCW-hr, exceeds (is at least) 0.60 g chemical product/g DCW-hr.

More generally, based on various combinations of the genetic modifications described herein, optionally in combination with supplementations described herein, specific productivity values for 3-HP, and for other chemical products described herein, may exceed 0.01 g chemical product/g DCW-hr, may exceed 0.05 g chemical product/g DCW-hr, may exceed 0.10 g chemical product/g DCW-hr, may exceed 0.15 g chemical product/g DCW-hr, may exceed 0.20 g chemical product/g DCW-hr, may exceed 0.25 g chemical product/g DCW-hr, may exceed 0.30 g chemical product/g DCW-hr, may exceed 0.35 g chemical product/g DCW-hr, may exceed 0.40 g chemical product/g DCW-hr, may exceed 0.45 g chemical product/g DCW-hr, and may exceed 0.50 g or 0.60 chemical product/g DCW-hr. Such specific productivity may be assessed over a 2, 4, 6, 8, 12 or 24 hour period in a particular microbial chemical production event.

In some embodiments a microbial chemical biosynthesis event (i.e., a fermentation event using a cultured population of a microorganism) proceeds using a genetically modified microorganism as described herein, wherein the specific productivity is between 0.01 and 0.60 grams of selected chemical product produced per gram of microorganism cell on a dry weight basis per hour (g chemical product/g DCW-hr). In various embodiments the specific productivity is greater than 0.01, greater than 0.05, greater than 0.10, greater than 0.15, greater than 0.20, greater than 0.25, greater than 0.30, greater than 0.35, greater than 0.40, greater than 0.45, or
greater than 0.50 g chemical product/g DCW-hr. Specific productivity may be assessed over a 2, 4, 6, 8, 12 or 24 hour period in a particular microbial chemical production event. More particularly, the specific productivity for a chemical product is between 0.05 and 0.10, 0.10 and 0.15, 0.15 and 0.20, 0.20 and 0.25, 0.25 and 0.30, 0.30 and 0.35, 0.35 and 0.40, 0.40 and 0.45, or 0.45 and 0.50 g chemical product/g DCW-hr., 0.50 and 0.55, or 0.55 and 0.60 g chemical product/g DCW-hr. Various embodiments comprise culture systems demonstrating such productivity.

Also, in various embodiments of the present disclosure the volumetric productivity achieved may be 0.25 g polyketide (or other chemical product) per liter per hour (g (chemical product)IL-hr), may be greater than 0.25 g polyketide (or other chemical product)/L-hr, may be greater than 0.50 g polyketide (or other chemical product)/L-hr, may be greater than 1.0 g polyketide (or other chemical product)IL-hr, may be greater than 1.50 g polyketide (or other chemical product)IL-hr, may be greater than 2.0 g polyketide (or other chemical product)IL- hr, may be greater than 2.50 g polyketide (or other chemical product)IL¬hr, may be greater than 3.0 g polyketide (or other chemical product)IL-hr, may be greater than 3.50 g polyketide (or other chemical product)IL-hr, may be greater than 4.0 g polyketide (or other chemical product)IL-hr, may be greater than 4.50 g polyketide (or other chemical product)/L-hr, may be greater than 5.0 g polyketide (or other chemical product)/L-hr, may be greater than 5.50 g polyketide (or other chemical product)IL-hr, may be greater than 6.0 g polyketide (or other chemical product)IL-hr, may be greater than 6.50 g polyketide (or other chemical product)/L-hr, may be greater than 7.0 g polyketide (or other chemical product)IL-hr, may be greater than 7.50 g polyketide (or other chemical product)/L-hr, may be greater than 8.0 g polyketide (or other chemical product)IL-hr, may be greater than 8.50 g polyketide (or other chemical product)IL-hr, may be greater than 9.0 g polyketide (or other chemical product)IL- hr, may be greater than 9.50 g polyketide (or other chemical product)IL-hr, or may be greater than 10.0 g polyketide (or other chemical product)IL-hr.

In some embodiments, specific productivity as measured over a 24-hour fermentation (culture) period may be greater than 0.01, 0.05, 0.10, 0.20, 0.5, 1.0, 2.0, 3.0, 4.0, 5.0, 6.0, 7.0, 8.0, 9.0, 10.0, 11.0 or 12.0 grams of chemical product per gram DCW of microorganisms (based on the final DCW at the end of the 24-hour period).

The improvements achieved by embodiments of the present disclosure may be determined by percentage increase in specific productivity, or by percentage increase in volumetric productivity, compared with an appropriate control microorganism lacking the particular genetic modification combinations taught herein (with or without the supplements taught herein, added to a vessel comprising the microorganism population). For particular
embodiments and groups thereof, such specific productivity and/or volumetric productivity improvements is/are at least 10, at least 20, at least 30, at least 40, at least 50, at least 100, at least 200, at least 300, at least 400, and at least 500 percent over the respective specific productivity and/or volumetric productivity of such appropriate control microorganism.

The specific methods and teachings of the specification, and/or cited references may be incorporated into the examples. Also, production of a chemical product may reach at least 1, at least 2, at least 5, at least 10, at least 20, at least 30, at least 40, and at least 50 g/liter titer in various embodiments.

The metrics may be applicable to any of the compositions, e.g., genetically modified microorganisms, methods, e.g., of producing chemical products, and systems, e.g., fermentation systems utilizing the genetically modified microorganisms and/or methods disclosed herein.

The amount of 3-HP or other product(s), including a polyketide, produced in a bio-production media generally can be determined using a number of methods known in the art, for example, high performance liquid chromatography (HPLC), gas chromatography (GC), GC/Mass Spectroscopy (MS), or spectrometry.

When 3-HP is the chemical product, the 3-HP may be separated and purified by the approaches described in the following paragraphs, taking into account that many methods of separation and purification are known in the art and the following disclosure is not meant to be limiting. Osmotic shock, sonication, homogenization, and/or a repeated freeze-thaw cycle followed by filtration and/or centrifugation, among other methods, such as pH adjustment and heat treatment, may be used to produce a cell-free extract from intact cells. Any one or more of these methods also may be employed to release 3-HP from cells as an extraction step.

Further as to general processing of a bio-production broth comprising 3-HP, various methods may be practiced to remove biomass and/or separate 3-HP from the culture broth and its components. Methods to separate and/or concentrate the 3-HP include centrifugation, filtration, extraction, chemical conversion such as esterification, distillation (which may result in chemical conversion, such as dehydration to acrylic acid, under some reactive-distillation conditions), crystallization, chromatography, and ion-exchange, in various forms. Additionally, cell rupture may be conducted as needed to release 3-HP from the cell mass, such as by sonication, homogenization, pH adjustment or heating. 3-HP may be further separated and/or purified by methods known in the art, including any combination of one or more of centrifugation, liquid-liquid separations, including extractions such as solvent extraction, reactive extraction, two-phase aqueous extraction and two-phase solvent extraction, membrane separation technologies, distillation, evaporation, ion-exchange chromatography, adsorption chromatography, reverse phase chromatography and crystallization. Any of the above methods may be applied to a portion
of a bio-production broth (i.e., a fermentation broth, whether made under aerobic, anaerobic, or microaerobic conditions), such as may be removed from a bio-production event gradually or periodically, or to the broth at termination of a bio-production event. Conversion of 3-HP to downstream products, such as described herein, may proceed after separation and purification, or, such as with distillation, thin-film evaporation, or wiped-film evaporation optionally also in part as a separation means.

For various of these approaches, one may apply a counter-current strategy, or a sequential or iterative strategy, such as multi-pass extractions. For example, a given aqueous solution comprising 3-HP may be repeatedly extracted with a non-polar phase comprising an amine to achieve multiple reactive extractions.

When a culture event (fermentation event) is at a point of completion, the spent broth may transferred to a separate tank, or remain in the culture vessel, and in either case the temperature may be elevated to at least 60°C for a minimum of one hour in order to kill the microorganisms. (Alternatively, as noted above other approaches to killing the microorganisms may be practiced, or centrifugation may occur prior to heating.) By spent broth is meant the final liquid volume comprising the initial nutrient media, cells grown from the microorganism inoculum (and possibly including some original cells of the inoculum), 3-HP, and optionally liquid additions made after providing the initial nutrient media, such as periodic additions to provide additional carbon source, etc. It is noted that the spent broth may comprise organic acids other than 3-HP, such as for example acetic acid and/or lactic acid.

A centrifugation step may then be practiced to filter out the biomass solids (e.g., microorganism cells). This may be achieved in a continuous or batch centrifuge, and solids removal may be at least about 80%, 85%, 90%, or 95% in a single pass, or cumulatively after two or more serial centrifugations.

An optional step is to polish the centrifuged liquid through a filter, such as microfiltration or ultrafiltration, or may comprise a filter press or other filter device to which is added a filter aid such as diatomaceous earth. Alternative or supplemental approaches to this and the centrifugation may include removal of cells by a flocculent, where the cells floc and are allowed to settle, and the liquid is drawn off or otherwise removed. A flocculent may be added to a fermentation broth after which settling of material is allowed for a time, and then separations may be applied, including but not limited to centrifugation.

After such steps, a spent broth comprising 3-HP and substantially free of solids is obtained for further processing. By "substantially free of solids" is meant that greater than 98%, 99%, or 99.5% of the solids have been removed.

In various embodiments this spent broth comprises various ions of salts, such as Na, Cl, SO4, and PO4. In some embodiments these ions may be removed by passing this spent broth through ion exchange columns, or otherwise contacting the spent broth with appropriate ion exchange material. Here and elsewhere in this document, "contacting" is taken to mean a contacting for the stated purpose by any way known to persons skilled in the art, such as, for example, in a column, under appropriate conditions that are well within the ability of persons of ordinary skill in the relevant art to determine. As but one example, these may comprise sequential contacting with anion and cation exchange materials (in any order), or with a mixed anion/cation material. This demineralization step should remove most such inorganic ions without removing the 3-HP. This may be achieved, for example, by lowering the pH sufficiently to protonate 3-HP and similar organic acids so that these acids are not bound to the anion exchange material, whereas anions, such as Cl and SO4, that remain charged at such pH are removed from the solution by binding to the resin. Likewise, positively charged ions are removed by contacting with cation exchange material. Such removal of ions may be assessed by a decrease in conductivity of the solution. Such ion exchange materials may be regenerated by methods known to those skilled in the art.

In some embodiments, the spent broth (such as but not necessarily after the previous demineralization step) is subjected to a pH elevation, after which it is passed through an ion exchange column, or otherwise contacted with an ion exchange resin, that comprises anionic groups, such as amines, to which organic acids, ionic at this pH, associate. Other organics that do not so associate with amines at this pH (which may be over 6.5, over 7.5, over 8.5, over 9.5, over 10.5, or higher pH) may be separated from the organic acids at this stage, such as by flushing with an elevated pH rinse. Thereafter elution with a lower pH and/or elevated salt content rinse may remove the organic acids. Eluting with a gradient of decreasing pH and/or increasing salt content rinses may allow more distinct separation of 3-HP from other organic acids, thereafter simplifying further processing.

This latter step of anion-exchange resin retention of organic acids may be practiced before or after the demineralization step. However, the following two approaches are alternatives to the anion-exchange resin step.

A first alternative approach comprises reactive extraction (a form of liquid-liquid extraction) as exemplified in this and the following paragraphs. The spent broth, which may be at a stage before or after the demineralization step above, is combined with a quantity of a tertiary amine such as Alamine336® (Cognis Corp., Cincinnati, OH USA) at low pH. Co-solvents for the Alamine336 or other tertiary amine may be added and include, but are not limited to benzene, carbon tetrachloride, chloroform, cyclohexane, disobutyl ketone, ethanol, #2 fuel oil, isopropanol, kerosene, n-butanol, isobutanol, octanol, and n-decanol that increase the partition coefficient when combined with the amine. After appropriate mixing a period of time for phase separation transpires, after which the non-polar phase, which comprises 3-HP associated with the Alamine336 or other tertiary amine, is separated from the aqueous phase.

When a co-solvent is used that has a lower boiling point than the 3-HP/tertiary amine, a distilling step may be used to remove the co-solvent, thereby leaving the 3-HP-tertiary amine complex in the non-polar phase.

Whether or not there is such a distillation step, a stripping or recovery step may be used to separate the 3-HP from the tertiary amine. An inorganic salt, such as ammonium sulfate, sodium chloride, or sodium carbonate, or a base such as sodium hydroxide or ammonium hydroxide, is added to the 3-HP/tertiary amine to reverse the amine protonation reaction, and a second phase is provided by addition of an aqueous solution (which may be the vehicle for provision of the inorganic salt). After suitable mixing, two phases result and this allows for tertiary amine regeneration and re-use, and provides the 3-HP in an aqueous solution. Alternatively, hot water may also be used without a salt or base to recover the 3HP from the amine.

In the above approach the phase separation and extraction of 3-HP to the aqueous phase can serve to concentrate the 3-HP. It is noted that chromatographic separation of respective organic acids also can serve to concentrate such acids, such as 3-HP. In similar approaches other suitable, non-polar amines, which may include primary, secondary and quaternary amines, may be used instead of and/or in combination with a tertiary amine.

A second alternative approach is crystallization. For example, the spent broth (such as free of biomass solids) may be contacted with a strong base such as ammonium hydroxide, which results in formation of an ammonium salt of 3-HP. This may be concentrated, and then ammonium-3-HP crystals are formed and may be separated, such as by filtration, from the aqueous phase. Once collected, ammonium-3-HP crystals may be treated with an acid, such as sulfuric acid, so that ammonium sulfate is regenerated, so that 3-HP and ammonium sulfate result.

Also, various aqueous two-phase extraction methods may be utilized to separate and/or concentrate a desired chemical product from a fermentation broth or later-obtained solution. It is known that the addition of polymers, such as dextran and glycol polymers, such as polyethylene glycol (PEG) and polypropylene glycol (PPG) to an aqueous solution may result in formation of two aqueous phases. In such systems a desired chemical product may segregate to one phase while cells and other chemicals partition to the other phase, thus providing for a separation without use of organic solvents. This approach has been demonstrated for some chemical products, but challenges associated with chemical product recovery from a polymer solution and low selectivities are recognized (See "Extractive Recovery of Products from Fermentation Broths," Joong Kyun Kim et al., Biotechnol. Bioprocess Eng., 1999(4)1-11.

Various substitutions and combinations of the above steps and processes may be made to obtain a relatively purified 3-HP solution. Also, methods of separation and purification disclosed in US 6,534,679, issued March 18, 2003 may be considered based on a particular processing scheme. Also, in some culture events periodic removal of a portion of the liquid volume may be made, and processing of such portion(s) may be made to recover the 3-HP, including by any combination of the approaches disclosed above.

As noted, solvent extraction is another alternative. This may use any of a number of and/or combinations of solvents, including alcohols, esters, ketones, and various organic solvents. Without being limiting, after phase separation a distillation step or a secondary extraction may be employed to separate 3-HP from the organic phase.

The following published resources indicate the level of skill in these relevant arts, and as needed to support a disclosure that teaches how to make and use methods of industrial bio-production of 3-HP, and also industrial systems that may be used to achieve such conversion with any of the recombinant microorganisms of the present disclosure (Biochemical Engineering Fundamentals, 2'd Ed. J. E. Bailey and D. F. 011is, McGraw Hill, New York, 1986, entire book for purposes indicated and Chapter 9, pp. 533-657 in particular for biological reactor design; Unit Operations of Chemical Engineering, 5th E a W. L. McCabe et al., McGraw Hill, New York 1993, entire book for purposes indicated, and particularly for process and separation technologies analyses; Equilibrium Staged Separations, P. C. Wankat, Prentice Hall, Englewood Cliffs, NJ USA, 1988, entire book for separation technologies teachings).

### IX. CONVERSION OF FERMENTATION PRODUCT TO DOWNSTREAM PRODUCT(S)

### Conversion of 3-HP to Acrylic Acid and Downstream Products

As discussed herein, various embodiments described herein are related to production of a particular chemical product, 3-hydroxypropionic acid (3-HP). This organic acid, 3-HP, may be converted to various other products having industrial uses, such as but not limited to acrylic acid, esters of acrylic acid, and other chemicals obtained from 3-HP, referred to as "downstream products." Under some approaches the 3-HP may be converted to acrylic acid, acrylamide, and/or
other downstream chemical products, in some instances the conversion being associated with the separation and/or purification steps. Many conversions to such downstream products are described herein. The methods of the disclosure include steps to produce downstream products of 3-HP.

As a C3 building block, 3-HP offers much potential in a variety of chemical conversions to commercially important intermediates, industrial end products, and consumer products. For example, 3-HP may be converted to acrylic acid, acrylates (e.g., acrylic acid salts and esters), 1,3-propanediol, malonic acid, ethyl-3- hydroxypropionate, ethyl ethoxy propionate, propiolactone, acrylamide, or acrylonitrile.

For example, methyl acrylate may be made from 3-HP via dehydration and esterification, the latter to add a methyl group (such as using methanol); acrylamide may be made from 3-HP via dehydration and amidation reactions; acrylonitrile may be made via a dehydration reaction and forming a nitrile moiety; propriolactone may be made from 3-HP via a ring-forming internal esterification reaction (eliminating a water molecule); ethyl-3-HP may be made from 3-HP via esterification with ethanol; malonic acid may be made from 3-HP via an oxidation reaction; and 1,3-propanediol may be made from 3-HP via a reduction reaction. Also, acrylic acid, first converted from 3-HP by dehydration, may be esterified with appropriate compounds to form a number of commercially important acrylate-based esters, including but not limited to methyl acrylate, ethyl acrylate, methyl acrylate, 2-ethylhexyl acrylate, butyl acrylate, and lauryl acrylate. Alternatively, 3HP may be esterified to form an ester of 3HP and then dehydrated to form the acrylate ester.

Additionally, 3-HP may be oligomerized or polymerized to form poly(3-hydroxypropionate) homopolymers, or co-polymerized with one or more other monomers to form various co-polymers. Because 3-HP has only a single stereoisomer, polymerization of 3-HP is not complicated by the stereospecificity of monomers during chain growth. This is in contrast to (S)-2-Hydroxypropanoic acid (also known as lactic acid), which has two (D, L) stereoisomers that must be considered during its polymerizations.

As will be further described, 3-HP can be converted into derivatives starting (i) substantially as the protonated form of 3-hydroxypropionic acid; (ii) substantially as the deprotonated form, 3-hydroxypropionate; or (iii) as mixtures of the protonated and deprotonated forms. Generally, the fraction of 3-HP present as the acid versus the salt will depend on the pH, the presence of other ionic species in solution, temperature (which changes the equilibrium constant relating the acid and salt forms), and to some extent pressure. Many chemical conversions may be carried out from either of the 3-HP forms, and overall process economics will typically dictate the form of 3-HP for downstream conversion.

Also, as an example of a conversion during separation, 3-HP in an amine salt form, such as in the extraction step herein disclosed using Alamine 336 as the amine, may be converted to acrylic acid by contacting a solution comprising the 3-HP amine salt with a dehydration catalyst, such as aluminum oxide, at an elevated temperature, such as 170 to 180 C, or 180 to 190 C, or 190 to 200 C, and passing the collected vapor phase over a low temperature condenser. Operating conditions, including 3-HP concentration, organic amine, co-solvent (if any), temperature, flow rates, dehydration catalyst, and condenser temperature, are evaluated and improved for commercial purposes. Conversion of 3-HP to acrylic acid is expected to exceed at least 80 percent, or at least 90 percent, in a single conversion event. The amine may be re-used, optionally after clean-up. Other dehydration catalysts, as provided herein, may be evaluated. It is noted that U.S. Patent No.7,186,856 discloses data regarding this conversion approach, albeit as part of an extractive salt-splitting conversion that differs from the teachings herein. Further as to embodiments in which the chemical product being synthesized by the microorganism host cell is 3-HP, made as provided herein and optionally purified to a selected purity prior to conversion, the methods of the present disclosure can also be used to produce "downstream" compounds derived from 3-HP, such as polymerized-3-HP (poly-3-HP), acrylic acid, polyacrylic acid (polymerized acrylic acid, in various forms), methyl acrylate, acrylamide, acrylonitrile, propiolactone, ethyl 3-HP, malonic acid, and 1,3- propanediol. Numerous approaches may be employed for such downstream conversions, generally falling into enzymatic, catalytic (chemical conversion process using a catalyst), thermal, and combinations thereof (including some wherein a desired pressure is applied to accelerate a reaction).

As noted, an important industrial chemical product that may be produced from 3-HP is acrylic acid. Chemically, one of the carbon-carbon single bonds in 3-HP must undergo a dehydration reaction, converting to a carbon-carbon double bond and rejecting a water molecule. Dehydration of 3-HP in principle can be carried out in the liquid phase or in the gas phase. In some embodiments, the dehydration takes place in the presence of a suitable homogeneous or heterogeneous catalyst. Suitable dehydration catalysts are both acid and alkaline catalysts. Following dehydration, an acrylic acid-containing phase is obtained and can be purified where appropriate by further purification steps, such as by distillation methods, extraction methods, or crystallization methods, or combinations thereof.

Making acrylic acid from 3-HP via a dehydration reaction may be achieved by a number of commercial methodologies including via a distillation process, which may be part of the
separation regime and which may include an acid and/or a metal ion as catalyst. More broadly, incorporated herein for its teachings of conversion of 3-HP, and other β-hydroxy carbonyl compounds, to acrylic acid and other related downstream compounds, is U.S. Patent Publication No. 2007/0219390 A1, published September 20, 2007, now abandoned. This publication lists numerous catalysts and provides examples of conversions, which are specifically incorporated herein. Also among the various specific methods to dehydrate 3-HP to produce acrylic acid is an older method, described in U.S. Patent No. 2,469,701 (Redmon). This reference teaches a method for the preparation of acrylic acid by heating 3-HP to a temperature between 130 and 190°C, in the presence of a dehydration catalyst, such as sulfuric acid or phosphoric acid, under reduced pressure. U.S. Patent Publication No. 2005/0222458 A1 (Craciun et al.) also provides a process for the preparation of acrylic acid by heating 3-HP or its derivatives. Vapor-phase dehydration of 3-HP occurs in the presence of dehydration catalysts, such as packed beds of silica, alumina, or titania.

The dehydration catalyst may comprise one or more metal oxides, such as A1203, Si02, or Ti02. In some embodiments, the dehydration catalyst is a high surface area A1203 or a high surface area silica wherein the silica is substantially Si02. High surface area for the purposes of the disclosure means a surface area of at least about 50, 75, 100 m2/g, or more. In some embodiments, the dehydration catalyst may comprise an aluminosilicate, such as a zeolite.

For example, including as exemplified from such incorporated references, 3-HP may be dehydrated to acrylic acid via various specific methods, each often involving one or more dehydration catalysts. One catalyst of particular apparent value is titanium, such as in the form of titanium oxide, TiO(2). A titanium dioxide catalyst may be provided in a dehydration system that distills an aqueous solution comprising 3-HP, wherein the 3-HP dehydrates, such as upon volatilization, converting to acrylic acid, and the acrylic acid is collected by condensation from the vapor phase.

As but one specific method, an aqueous solution of 3-HP is passed through a reactor column packed with a titanium oxide catalyst maintained at a temperature between 170 and 190 C and at ambient atmospheric pressure. Vapors leaving the reactor column are passed over a low temperature condenser, where acrylic acid is collected. The low temperature condenser may be cooled to 30 C or less, 2 C or less, or at any suitable temperature for efficient condensation based on the flow rate and design of the system. Also, the reactor column temperatures may be lower, for instance when operating at a pressure lower than ambient atmospheric pressure. It is noted that Example 1 of U.S. Patent Publication No. 2007/0219390, published September 20, 2007, now abandoned, provides specific parameters that employs the approach of this method. As
noted.

Further as to dehydration catalysts, the following table summarizes a number of catalysts (including chemical classes) that may be used in a dehydration reaction from 3-HP (or its esters) to acrylic acid (or acrylate esters). Such catalysts, some of which may be used in any of solid, liquid or gaseous forms, may be used individually or in any combination. This listing of catalysts is not intended to be limiting, and many specific catalysts not listed may be used for specific dehydration reactions. Further without being limiting, catalyst selection may depend on the solution pH and/or the form of 3-HP in a particular conversion, so that an acidic catalyst may be used when 3-HP is in acidic form, and a basic catalyst may be used when the ammonium salt of 3-HP is being converted to acrylic acid. Also, some catalysts may be in the form of ion exchange resins.

**Table 12: Dehydration Catalysts**

| Catalyst by Chemical Class | Non-limiting Examples |
|---|---|
| Acids (including weak and strong) | H2SO4,HCl, titanic acids, metal oxide hydrates, metal sulfates (MSO4,.where M=Zn, Sn, Ca, Ba, Ni, Co, or other transition metals), metal oxide sulfates, metal phosphates (e.g., M3,(PO4) 2, where M=Ca, Ba), metal phosphates, metal oxide phosphates, carbon (e.g., transition metals on a carbon support), mineral acids, carboxylic acids, salts thereof, acidic resins, acidic zeolites, clays, Si02/H3PO4, fluorinated A1203, Nb203/P05 3, N b203/SO4 2, Nb2O5H2O, phosphotungstic acids, phosphomolybdic acids, silicomolybdic acids, silicotungstic acids, carbon dioxide |
| Bases (including weak and strong) | NaOH, ammonia, polyvinylpyridine, metal hydroxides, Zr(OH)4, and substituted amines |
| Oxides (generally metal oxides) | TiO₂, Zr02, Al203, SiO2, Zn02, Sn02, WO3, Mn02, Fe2O3, V205 |

As to another specific method using one of these catalysts, concentrated sulfuric acid and an aqueous solution comprising 3-HP are separately flowed into a reactor maintained at 150 to 165°C at a reduced pressure of 100 mm Hg. Flowing from the reactor is a solution comprising acrylic acid. A specific embodiment of this method, disclosed in Example 1 of US2009/0076297 indicates a yield of acrylic acid exceeding 95 percent.

Based on the wide range of possible catalysts and knowledge in the art of dehydration reactions of this type, numerous other specific dehydration methods may be evaluated and implemented for commercial production.

The dehydration of 3-HP may also take place in the absence of a dehydration catalyst. For example, the reaction may be run in the vapor phase in the presence of a nominally inert packing such as glass, ceramic, a resin, porcelain, plastic, metallic or brick dust packing and still form acrylic acid in reasonable yields and purity. The catalyst particles can be sized and configured such that the chemistry is, in some embodiments, mass-transfer-limited or kinetically limited. The catalyst can take the form of powder, pellets, granules, beads, extrudates, and so on. When a catalyst support is optionally employed, the support may assume any physical form such as pellets, spheres, monolithic channels, etc. The supports may be co-precipitated with active metal species; or the support may be treated with the catalytic metal species and then used as is or formed into the aforementioned shapes; or the support may be formed into the aforementioned shapes and then treated with the catalytic species.

A reactor for dehydration of 3-HP may be engineered and operated in a wide variety of ways. The reactor operation can be continuous, semi-continuous, or batch. It is perceived that an operation that is substantially continuous and at steady state is advantageous from operations and economics perspectives. The flow pattern can be substantially plug flow, substantially well-mixed, or a flow pattern between these extremes. A "reactor" can actually be a series or network of several reactors in various arrangements. For example, without being limiting, acrylic acid may be made from 3-HP via a dehydration reaction, which may be achieved by a number of commercial methodologies including via a distillation process, which may be part of the separation regime and which may include an acid and/or a metal ion as catalyst. U.S. Patent Publication No. 2007/0219390 A1, published September 20, 2007, now abandoned, lists numerous catalysts and provides examples of conversions.

For example, including as exemplified from such incorporated references, 3-HP may be dehydrated to acrylic acid via various specific methods, each often involving one or more dehydration catalysts.

One catalyst of particular apparent value is titanium, such as in the form of titanium oxide, TiO2. A titanium dioxide catalyst may be provided in a dehydration system that distills an aqueous solution comprising 3-HP, wherein the 3-HP dehydrates, such as upon volatilization, converting to acrylic acid, and the acrylic acid is collected by condensation from the vapor phase.

As but one specific method, an aqueous solution of 3-HP is passed through a reactor column packed with a titanium oxide catalyst maintained at a temperature between 170 and 190°C and at ambient atmospheric pressure. Vapors leaving the reactor column are passed over a low temperature condenser, where acrylic acid is collected. The low temperature condenser may be cooled to 30°C or less, 20°C or less, 2°C or less, or at any suitable temperature for efficient condensation based on the flow rate and design of the system. Also, the reactor column temperatures may be lower, for instance when operating at a pressure lower than ambient atmospheric pressure. It is noted that Example 1 of U.S. Patent Publication No. 2007/0219390, published September 20, 2007, now abandoned, provides specific parameters that employs the approach of this method. Crystallization of the acrylic acid obtained by dehydration of 3-HP may be used as one of the final separation/purification steps. Various approaches to crystallization are known in the art, including crystallization of esters.

As noted above, in some embodiments, a salt of 3-HP is converted to acrylic acid or an ester or salt thereof. For example, U.S. Patent No. 7,186,856 (Meng et al.) teaches a process for producing acrylic acid from the ammonium salt of 3-HP, which involves a first step of heating the ammonium salt of 3-HP in the presence of an organic amine or solvent that is immiscible with water, to form a two-phase solution and split the 3-HP salt into its respective ionic constituents under conditions which transfer 3-HP from the aqueous phase to the organic phase of the solution, leaving ammonia and ammonium cations in the aqueous phase. The organic phase is then back-extracted to separate the 3-HP, followed by a second step of heating the 3-HP-containing solution in the presence of a dehydration catalyst to produce acrylic acid. Various alternatives to the particular approach disclosed in this patent may be developed for suitable extraction and conversion processes.

Methyl acrylate may be made from 3-HP via dehydration and esterification, the latter to add a methyl group (such as using methanol), acrylamide may be made from 3-HP via dehydration and amidation reactions, acrylonitrile may be made via a dehydration reaction and forming a nitrile moiety, propriolactone may be made from 3-HP via a ring-forming internal esterification reaction (eliminating a water molecule), ethyl-3¬HP may be made from 3-HP via esterification with ethanol, malonic acid may be made from 3-HP via an oxidation reaction, and 1,3-propanediol may be made from 3-HP via a reduction reaction.

Malonic acid may be produced from oxidation of 3-HP as produced herein. U.S. Patent No. 5,817,870 (Haas et al.) discloses catalytic oxidation of 3-HP by a precious metal selected from Ru, Rh, Pd, Os, Ir or Pt. These can be pure metal catalysts or supported catalysts. The catalytic oxidation can be carried out using a suspension catalyst in a suspension reactor or using a fixed-bed catalyst in a fixed-bed reactor. If the catalyst, such as a supported catalyst, is disposed in a fixed-bed reactor, the latter can be operated in a trickle-bed procedure as well as also in a liquid-phase procedure. In the trickle-bed procedure the aqueous phase comprising the 3-HP starting material, as well as the oxidation products of the same and means for the adjustment of pH, and oxygen or an oxygen-containing gas can be conducted in parallel flow or counter-flow. In the liquid-phase procedure the liquid phase and the gas phase are conveniently conducted in parallel flow.

In order to achieve a sufficiently short reaction time, the conversion is carried out at a pH equal or greater than 6, such as at least 7, and in particular between 7.5 and 9. According to a particular embodiment, during the oxidation reaction the pH is kept constant, such as at a pH in the range between 7.5 and 9, by adding a base, such as an alkaline or alkaline earth hydroxide solution. The oxidation is usefully carried out at a temperature of at least 10°C and maximally 70°C. The flow of oxygen is not limited. In the suspension method it is important that the liquid and the gaseous phase are brought into contact by stirring vigorously. Malonic acid can be obtained in nearly quantitative yields. 1,3-Propanediol may be produced from hydrogenation of 3-HP as produced herein. Possible catalysts include ruthenium metal, or compounds of ruthenium, supported or unsupported, alone or in combination with at least one or more additional metal(s) selected from molybdenum, tungsten, titanium, zirconium, niobium, vanadium or chromium. The ruthenium metal or compound thereof, and/or the additional metal(s), or compound thereof, may be utilized in supported or unsupported form. If utilized in supported form, the method of preparing the supported catalyst is not critical and can be any technique such as impregnation of the support or deposition on the support. Any suitable support may be utilized. Supports that may be used include, but are not limited to, alumina, titania, silica, zirconia, carbons, carbon blacks, graphites, silicates, zeolites, aluminosilicate zeolites, aluminosilicate clays, and the like.

The hydrogenation process may be carried out in liquid phase. The liquid phase includes water, organic solvents that are not hydrogenatable, such as any aliphatic or aromatic
hydrocarbon, alcohols, ethers, toluene, decalin, dioxane, diglyme, n-heptane, hexane, xylene, benzene, tetrahydrofuran, cyclohexane, methylcyclohexane, and the like, and mixtures of water and organic solvent(s). The hydrogenation process may be carried out batch wise, semi-continuously, or continuously. The hydrogenation process may be carried out in any suitable apparatus. Exemplary of such apparatus are stirred tank reactors, trickle-bed reactors, high pressure hydrogenation reactors, and the like.

The hydrogenation process is generally carried out at a temperature ranging from about 20 to about 250°C, more particularly from about 100 to about 200°C. Further, the hydrogenation process is generally carried out in a pressure range of from about 20 psi to about 4000 psi. The hydrogen containing gas utilized in the hydrogenation process is, optionally, commercially pure hydrogen. The hydrogen containing gas is usable if nitrogen, gaseous hydrocarbons, or oxides of carbon, and similar materials, are present in the hydrogen containing gas. For example, hydrogen from synthesis gas (hydrogen and carbon monoxide) may be employed, such synthesis gas potentially further including carbon dioxide, water, and various impurities.

As is known in the art, it is also possible to convert 3-HP to 1,3-propanediol using biological methods. For example, 1,3-propanediol can be created from either 3-HP-CoA or 3-HP via the use of polypeptides having enzymatic activity. These polypeptides can be used either in vitro or in vivo. When converting 3-HP-CoA to 1,3-propanediol, polypeptides having oxidoreductase activity or reductase activity (e.g., enzymes from the 1.1.1.-class of enzymes) can be used. Alternatively, when creating 1,3-propanediol from 3-HP, a combination of a polypeptide having aldyhyde dehydrogenase activity (e.g., an enzyme from the 1.1.1.34 class) and a polypeptide having alcohol dehydrogenase activity (e.g., an enzyme from the 1.1.1.32 class) can be used.

Another downstream production of 3-HP, acrylonitrile, may be converted from acrylic acid by various organic syntheses, including by not limited to the Sohio acrylonitrile process, a single-step method of production known in the chemical manufacturing industry

Also, addition reactions may yield acrylic acid or acrylate derivatives having alkyl or aryl groups at the carbonyl hydroxyl group. Such additions may be catalyzed chemically, such as by hydrogen, hydrogen halides, hydrogen cyanide, or Michael additions under alkaline conditions optionally in the presence of basic catalysts. Alcohols, phenols, hydrogen sulfide, and thiols are known to add under basic conditions. Aromatic amines or amides, and aromatic hydrocarbons, may be added under acidic conditions. These and other reactions are described in Ulmann's Encyclopedia of Industrial Chemistry, Acrylic Acid and Derivatives, Wi1eyVCH Verlag GmbH, Wienham (2005).

Acrylic acid obtained from 3-HP made by the present disclosure may be further converted to various chemicals, including polymers, which are also considered downstream products in some embodiments. Acrylic acid esters may be formed from acrylic acid (or directly from 3-HP) such as by condensation esterification reactions with an alcohol, releasing water. This chemistry is described in Monomeric Acrylic Esters, E. H. Riddle, Reinhold, NY (1954). Among esters that are formed are methyl acrylate, ethyl acrylate, n-butyl acrylate, hydroxypropyl acrylate, hydroxyethyl acrylate, isobutyl acrylate, and 2-ethylhexyl acrylate, and these and/or other acrylic acid and/or other acrylate esters may be combined, including with other compounds, to form various known acrylic acid-based polymers. Although acrylamide is produced in chemical syntheses by hydration of acrylonitrile, herein a conversion may convert acrylic acid to acrylamide by amidation.

Acrylic acid obtained from 3-HP made by the present disclosure may be further converted to various chemicals, including polymers, which are also considered downstream products in some embodiments. Acrylic acid esters may be formed from acrylic acid (or directly from 3-HP) such as by condensation esterification reactions with an alcohol, releasing water. This chemistry is described in Monomeric Acrylic Esters, E. H. Riddle, Reinhold, NY (1954). Among esters that are formed are methyl acrylate, ethyl acrylate, n-butyl acrylate, hydroxypropyl acrylate, hydroxyethyl acrylate, isobutyl acrylate, and 2-ethylhexyl acrylate, and these and/or other acrylic acid and/or other acrylate esters may be combined, including with other compounds, to form various known acrylic acid-based polymers. Although acrylamide is produced in chemical syntheses by hydration of acrylonitrile, herein a conversion may convert acrylic acid to acrylamide by amidation.

Direct esterification of acrylic acid can take place by esterification methods known to the person skilled in the art, by contacting the acrylic acid obtained from 3-HP dehydration with one or more alcohols, such as methanol, ethanol, 1-propanol, 2-propanol, n-butanol, tert-butanol or isobutanol, and heating to a temperature of at least 50, 75, 100, 125, or 150°C. The water formed during esterification may be removed from the reaction mixture, such as by azeotropic distillation through the addition of suitable separation aids, or by another means of separation. Conversions up to 95%, or more, may be realized, as is known in the art.

Several suitable esterification catalysts are commercially available, such as from Dow Chemical (Midland, Michigan US). For example, AmberlystTM 131 Wet Monodisperse gel catalyst confers enhanced hydraulic and reactivity properties and is suitable for fixed bed reactors. AmberlystTM 39Wet is a macroreticular catalyst suitable particularly for stirred and
slurry loop reactors. AmberlystTM 46 is a macroporous catalyst producing less ether byproducts than conventional catalyst (as described in U.S. Patent No. 5,426,199 to Rohm and Haas),.

Acrylic acid, and any of its esters, may be further converted into various polymers. Polymerization may proceed by any of heat, light, other radiation of sufficient energy, and free radical generating compounds, such as azo compounds or peroxides, to produce a desired polymer of acrylic acid or acrylic acid esters. As one example, an aqueous acrylic acid solution's temperature raised to a temperature known to start polymerization (in part based on the initial acrylic acid concentration), and the reaction proceeds, the process frequently involving heat removal given the high exothermicity of the reaction. Many other methods of polymerization are known in the art. Some are described in Ulmann's Encyclopedia of Industrial Chemistry, Polyacrylamides and Poly(Acrylic Acids), Wi1eyVCH Verlag GmbH, Wienham (2005).

For example, the free-radical polymerization of acrylic acid takes place by polymerization methods known to the skilled worker and can be carried out either in an emulsion or suspension in aqueous solution or another solvent. Initiators, such as but not limited to organic peroxides, often are added to aid in the polymerization. Among the classes of organic peroxides that may be used as initiators are diacyls, peroxydicarbonates, monoperoxycarbonates, peroxyketals, peroxyesters, dialkyls, and hydroperoxides. Another class of initiators is azo initiators, which may be used for acrylate polyermization as well as copolymerization with other monomers. U.S. Patent Nos. 5,470,928; 5,510,307; 6,709,919; and 7,678,869 teach various approaches to polymerization using a number of initiators, including organic peroxides, azo compounds, and other chemical types.

Accordingly, it is further possible for co-monomers, such as crosslinkers, to be present during the polymerization. The free-radical polymerization of the acrylic acid obtained from dehydration of 3-HP, as produced herein, in at least partly neutralized form and in the presence of crosslinkers is practiced in certain embodiments. This polymerization may result in hydrogels which can then be comminuted, ground and, where appropriate, surface-modified, by known techniques.

An important commercial use of polyacrylic acid is for superabsorbent polymers. Superabsorbent polymers are primarily used as absorbents for water and aqueous solutions for diapers, adult incontinence products, feminine hygiene products, and similar consumer products. In such consumer products, superabsorbent materials can replace traditional absorbent materials such as cloth, cotton, paper wadding, and cellulose fiber. Superabsorbent polymers absorb, and retain under a slight mechanical pressure, up to 25 times or their weight in liquid. The swollen gel holds the liquid in a solid, rubbery state and prevents the liquid from leaking. Superabsorbent polymer particles can be surface-modified to produce a shell structure with the shell being more highly crosslinked. This technique improves the balance of absorption, absorption under load, and resistance to gel-blocking. It is recognized that superabsorbent polymers have uses in fields other than consumer products, including agriculture, horticulture, and medicine. Superabsorbent polymers are prepared from acrylic acid (such as acrylic acid derived from 3 HP provided herein) and a crosslinker, by solution or suspension polymerization. Exemplary methods include U.S. Patent Nos. 5,145,906; 5,350,799; 5,342,899; 4,857,610; 4,985,518; 4,708, 997; 5,180,798; 4,666,983; 4,734,478; and 5,331,059.

Among consumer products, a diaper, a feminine hygiene product, and an adult incontinence product are made with superabsorbent polymer that itself is made substantially from acrylic acid converted from 3-HP made in accordance with the present disclosure.

Diapers and other personal hygiene products may be produced that incorporate superabsorbent polymer made from acrylic acid made from 3-HP which is bio-produced by the teachings of the present application. The following provides general guidance for making a diaper that incorporates such superabsorbent polymer. The superabsorbent polymer first is prepared into an absorbent pad that may be vacuum formed, and in which other materials, such as a fibrous material (e.g., wood pulp) are added. The absorbent pad then is assembled with sheet(s) of fabric, generally a nonwoven fabric (e.g., made from one or more of nylon, polyester, polyethylene, and polypropylene plastics) to form diapers.

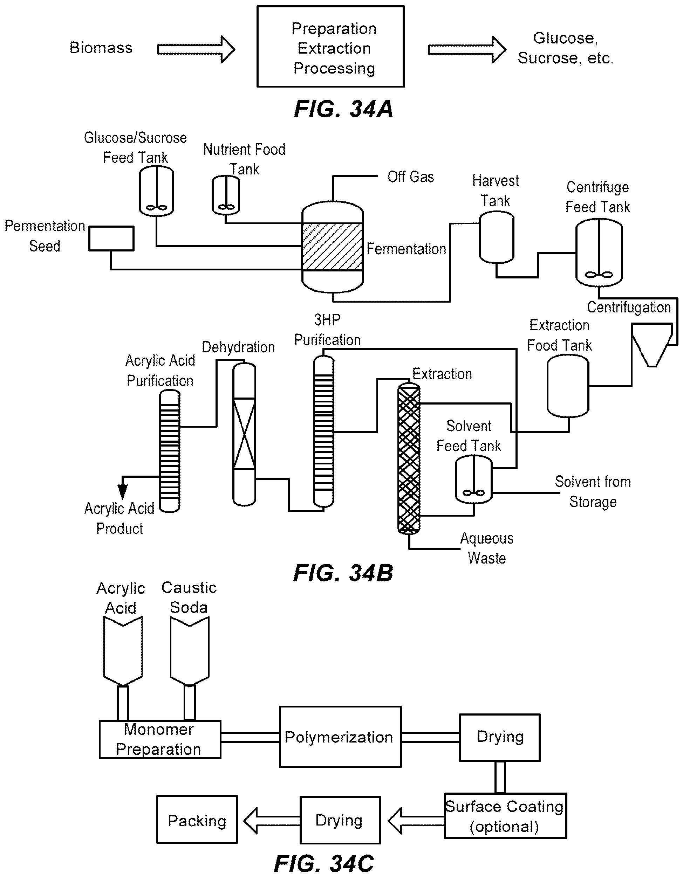

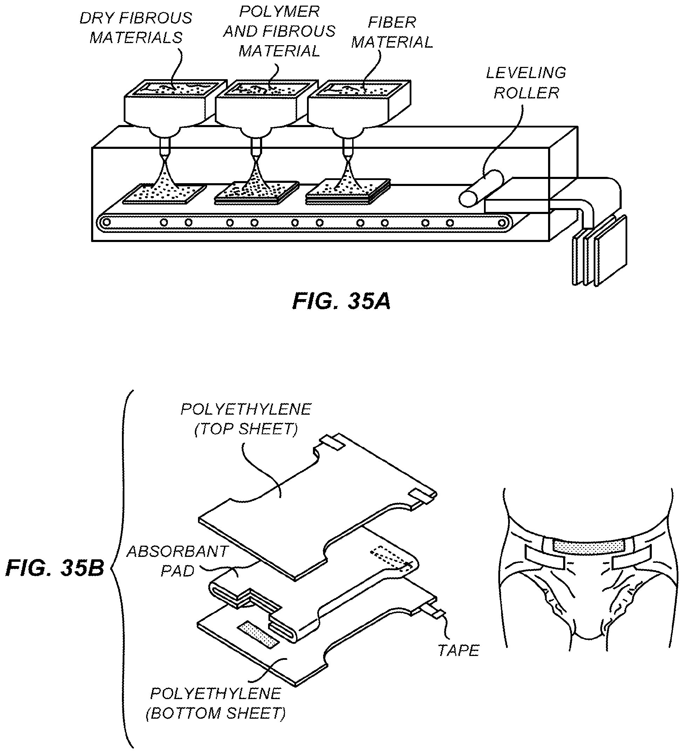

More particularly, in one non-limiting process, above a conveyer belt multiple pressurized nozzles spray superabsorbent polymer particles (such as about 400 micron size or larger), fibrous material, and/or a combination of these onto the conveyer belt at designated spaces/intervals. The conveyor belt is perforated and under vacuum from below, so that the sprayed on materials are pulled toward the belt surface to form a flat pad. In various embodiments, fibrous material is applied first on the belt, followed by a mixture of fibrous material and the superabsorbent polymer particles, followed by fibrous material, so that the superabsorbent polymer is concentrated in the middle of the pad. A leveling roller may be used toward the end of the belt path to yield pads of uniform thickness. Each pad thereafter may be further processed, such as to cut it to a proper shape for the diaper, or the pad may be in the form of a long roll sufficient for multiple diapers. Thereafter, the pad is sandwiched between a top sheet and a bottom sheet of fabric (one generally being liquid pervious, the other liquid impervious), such as on a conveyor belt, and these are attached together such as by gluing, heating or ultrasonic welding, and cut into diaper-sized units (if not previously so cut). Additional features may be provided, such as elastic components, strips of tape, etc., for fit and ease of wearing by a person. FIG. 34A, B, and C and FIG. 35A and B show a schematic of an entire process of converting biomass to a finished product such as a diaper. These are meant to be exemplary and not limiting.

The ratio of the fibrous material to polymer particles is known to effect performance characteristics. In some embodiments, this ratio is between 75:25 and 90:10 (see U.S. Patent No. 4,685,915. Other disposable absorbent articles may be constructed in a similar fashion, such as for adult incontinence, feminine hygiene (sanitary napkins), tampons, etc. (see, for example, U.S. Patent Nos. 5,009,653, 5,558,656, and 5,827,255.

Low molecular-weight polyacrylic acid has uses for water treatment, flocculants, and thickeners for various applications including cosmetics and pharmaceutical preparations. For these applications, the polymer may be uncrosslinked or lightly crosslinked, depending on the specific application. The molecular weights are typically from about 200 to about 1,000,000 g/mol. Preparation of these low molecular-weight polyacrylic acid polymers is described in U.S. Patent Nos. 3,904,685; 4,301,266; 2,798,053; and 5,093,472.

Acrylic acid may be co-polymerized with one or more other monomers selected from acrylamide, 2-acrylamido-2-methylpropanesulfonic acid, N,N-dimethylacrylamide, N-isopropylacrylamide, methacrylic acid, and methacrylamide, to name a few. The relative reactivities of the monomers affect the microstructure and thus the physical properties of the polymer. Co-monomers may be derived from 3-HP, or otherwise provided, to produce co-polymers. Ulmann's Encyclopedia of Industrial Chemistry, Polyacrylamides and Poly(Acrylic Acids), WileyVCH Verlag GmbH, Wienham (2005).

Acrylic acid can in principle be copolymerized with almost any free-radically polymerizable monomers including styrene, butadiene, acrylonitrile, acrylic esters, maleic acid, maleic anhydride, vinyl chloride, acrylamide, itaconic acid, and so on. End-use applications typically dictate the co-polymer composition, which influences properties. Acrylic acid also may
have a number of optional substitutions on it, and after such substitutions be used as a monomer for polymerization, or co-polymerization reactions. As a general rule, acrylic acid (or one of its co-polymerization monomers) may be substituted by any substituent that does not interfere with the polymerization process, such as alkyl, alkoxy, aryl, heteroaryl, benzyl, vinyl, allyl, hydroxy, epoxy, amide, ethers, esters, ketones, maleimides, succinimides, sulfoxides, glycidyl and silyl (see U.S. Patent No. 7,678,869 for further discussion). The following paragraphs provide a few non-limiting examples of copolymerization applications.

Paints that comprise polymers and copolymers of acrylic acid and its esters are in wide use as industrial and consumer products. Aspects of the technology for making such paints can be found in U.S. Patent Nos. 3,687,885 and 3,891,591 Generally, acrylic acid and its esters may form homopolymers or copolymers among themselves or with other monomers, such as amides, methacrylates, acrylonitrile, vinyl, styrene and butadiene. A desired mixture of homopolymers and/or copolymers, referred to in the paint industry as 'vehicle' (or 'binder') are added to an aqueous solution and agitated sufficiently to form an aqueous dispersion that includes sub-micrometer sized polymer particles. The paint cures by coalescence of these 'vehicle' particles as the water and any other solvent evaporate. Other additives to the aqueous dispersion may include pigment, filler (e.g., calcium carbonate, aluminum silicate), solvent (e.g., acetone, benzol, alcohols, etc., although these are not found in certain no VOC paints), thickener, and additional additives depending on the conditions, applications, intended surfaces, etc. In many paints, the weight percent of the vehicle portion may range from about nine to about 26 percent, but for other paints the weight percent may vary beyond this range.

Acrylic-based polymers are used for many coatings in addition to aints. For example, for paper coating latexes, acrylic acid is used from 0.1-5.0%, along with styrene and butadiene, to enhance binding to the paper and modify rheology, freeze-thaw stability and shear stability. Acrylate-based polymers also are used in many inks, particularly UV curable printing inks. For water treatment, acrylamide and/or hydroxy ethyl acrylate are commonly co-polymerized with acrylic acid to produce low molecular-weight linear polymers. Co-polymers of acrylic acid with maleic acid or itaconic acid are also produced for water-treatment applications, as described in U.S. Patent No. 5,135,677. Sodium acrylate (the sodium salt of glacial acrylic acid) can be co-polymerized with acrylamide (which may be derived from acrylic acid via amidation chemistry) to make an anionic co-polymer that is used as a flocculant in water treatment.

For thickening agents, a variety of co-monomers can be used, such as described in U.S. Patent Nos. 4,268,641 and 3,915,921. U.S. Patent No. 5,135,677 describes a number of co-monomers that can be used with acrylic acid to produce water-soluble polymers.

Also as noted, some conversions to downstream products may be made enzymatically. For example, 3-HP may be converted to 3-HP-CoA, which then may be converted into polymerized 3-HP with an enzyme having polyhydroxyacid synthase activity (EC 2.3.1.-). Also, 1,3-propanediol can be made using polypeptides having oxidoreductase activity or reductase activity (e.g. , enzymes in the EC 1.1.1.- class of enzymes). Alternatively, when creating 1,3-propanediol from 3HP, a combination of (1) a polypeptide having aldehyde dehydrogenase activity (e.g., an enzyme from the 1.1.1.34 class) and (2) a polypeptide having alcohol dehydrogenase activity (e.g., an enzyme from the 1.1.1.32 class) can be used. Polypeptides having lipase activity may be used to form esters. Enzymatic reactions such as these may be conducted in vitro, such as using cell-free extracts, or in vivo. Thus, various embodiments of the present disclosure, such as methods of making a chemical, include conversion steps to any such noted downstream products of microbially produced 3-HP, including but not limited to those chemicals described herein and in the incorporated references (the latter for jurisdictions allowing this). For example, one embodiment is making 3-HP molecules by the teachings herein and further converting the 3-HP molecules to polymerized-3-HP (poly-3-HP) or acrylic acid, and such as from acrylic acid then producing from the 3-HP molecules any one of polyacrylic acid (polymerized acrylic acid, in various forms), methyl acrylate, acrylamide, acrylonitrile, propiolactone, ethyl 3-HP, malonic acid, 1,3-propanediol, ethyl acrylate, n-butyl acrylate, hydroxypropyl acrylate, hydroxyethyl acrylate, isobutyl acrylate, 2-ethylhexyl acrylate, and acrylic acid or an acrylic acid ester to which an alkyl or aryl addition is made, and/or to which halogens, aromatic amines or amides, and aromatic hydrocarbons are added.

Also as noted, some conversions to downstream products may be made enzymatically. For example, 3-HP may be converted to 3-HP-CoA, which then may be converted into polymerized 3-HP with an enzyme having polyhydroxyacid synthase activity (EC 2.3.1.-). Also, 1,3-propanediol can be made using polypeptides having oxidoreductase activity or reductase activity (e.g. , enzymes in the EC 1.1.1.- class of enzymes). Alternatively, when creating 1,3-propanediol from 3HP, a combination of (1) a polypeptide having aldehyde dehydrogenase activity (e.g., an enzyme from the 1.1.1.34 class) and (2) a polypeptide having alcohol
dehydrogenase activity (e.g., an enzyme from the 1.1.1.32 class) can be used. Polypeptides having lipase activity may be used to form esters. Enzymatic reactions such as these may be conducted in vitro, such as using cell-free extracts, or in vivo.

Thus, various embodiments of the present disclosure, such as methods of making a chemical, include conversion steps to any such noted downstream products of microbially produced 3-HP, including but not limited to those chemicals described herein and in the incorporated references (the latter for jurisdictions allowing this). For example, one embodiment is making 3-HP molecules by the teachings herein and further converting the 3-HP molecules to polymerized-3-HP (poly-3-HP) or acrylic acid, and such as from acrylic acid then producing from the 3-HP molecules any one of polyacrylic acid (polymerized acrylic acid, in various forms), methyl acrylate, acrylamide, acrylonitrile, propiolactone, ethyl 3-HP, malonic acid, 1,3-propanediol, ethyl acrylate, n-butyl acrylate, hydroxypropyl acrylate, hydroxyethyl acrylate, isobutyl acrylate, 2-ethylhexyl acrylate, and acrylic acid or an acrylic acid ester to which an alkyl or aryl addition is made, and/or to which halogens, aromatic amines or amides, and aromatic hydrocarbons are added.

Reactions that form downstream compounds such as acrylates or acrylamides can be conducted in conjunction with use of suitable stabilizing agents or inhibiting agents reducing likelihood of polymer formation. See, for example, U.S. Patent Publication No. 2007/0219390 A1. Stabilizing agents and/or inhibiting agents include, but are not limited to, e.g., phenolic compounds (e.g., dimethoxyphenol (DMP) or alkylated phenolic compounds such as di-tert-butyl phenol), quinones (e.g., t-butyl hydroquinone or the monomethyl ether of hydroquinone (MEHQ)), and/or metallic copper or copper salts (e.g., copper sulfate, copper chloride, or copper acetate). Inhibitors and/or stabilizers can be used individually or in combinations as will be known by those of skill in the art. Also, in various embodiments, the one or more downstream compounds is/are recovered at a molar yield of up to about 100 percent, or a molar yield in the range from about 70 percent to about 90 percent, or a molar yield in the range from about 80 percent to about 100 percent, or a molar yield in the range from about 90 percent to about 100 percent. Such yields may be the result of single-pass (batch or continuous) or iterative separation and purification steps in a particular process.

Acrylic acid and other downstream products are useful as commodities in manufacturing, such as in the manufacture of consumer goods, including diapers, textiles, carpets, paint, adhesives, and acrylic glass.

### EXAMPLES

The following examples are given for the purpose of illustrating various embodiments of the disclosure and are not meant to limit the present invention in any fashion.

### COMMON METHODS

### Common Method Example 1: Microorganism Species and Strains

Bacterial species, that may be utilized as needed, are as follows:
*Acinetobacter calcoaceticus* (DSMZ # 1139) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL, USA). Serial dilutions of the resuspended *A. calcoaceticus* culture are made into BHI and are allowed to grow for aerobically for 48 hours at 37°C at 250 rpm until saturated.

*Bacillus subtilis* is a gift from the Gill lab (University of Colorado at Boulder, Boulder CO USA) and is obtained as an actively growing culture. Serial dilutions of the actively growing *B. subtilis* culture are made into Luria Broth (RPI Corp., Mt. Prospect, IL, USA) and are allowed to grow for aerobically for 24 hours at 37°C at 250 rpm until saturated.

*Chlorobium limicola* (DSMZ# 245) is obtained from the German Collection of Microorganisms and Cell Cultures (Braunschweig, Germany) as a vacuum dried culture. Cultures are then resuspended using Pfennig's Medium I and II (#28 and 29) as described per DSMZ instructions. *C*. *limicola* is grown at 25°C under constant vortexing.

*Citrobacter braakii* (DSMZ # 30040) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended C. *braakii* culture are made into BHI and are allowed to grow for aerobically for 48 hours at 30°C at 250 rpm until saturated.

*Clostridium acetobutylicum* (DSMZ # 792) is obtained from the German Collection of Microorganisms and Cell Cultures (Braunschweig, Germany) as a vacuum dried culture. Cultures are then resuspended in *Clostridium acetobutylicum* medium (#411) as described per DSMZ instructions. *C*. *acetobutylicum* is grown anaerobically at 37°C at 250 rpm until saturated.

*Clostridium aminobutyricum* (DSMZ # 2634) is obtained from the German Collection of Microorganisms and Cell Cultures (Braunschweig, Germany) as a vacuum dried culture. Cultures are then resuspended in *Clostridium aminobutyricum* medium (#286) as described per DSMZ instructions. *C*. *aminobutyricum* is grown anaerobically at 37°C at 250 rpm until saturated.

*Clostridium kluyveri* (DSMZ #555) is obtained from the German Collection of Microorganisms and Cell Cultures (Braunschweig, Germany) as an actively growing culture. Serial dilutions of *C*. *kluyveri* culture are made into *Clostridium kluyveri* medium (#286) as described per DSMZ instructions. *C. kluyveri* is grown anaerobically at 37°C at 250 rpm until saturated.

*Cupriavidus metallidurans* (DMSZ # 2839) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *C. metallidurans* culture are made into BHI and are allowed to grow for aerobically for 48 hours at 30°C at 250 rpm until saturated.

*Cupriavidus necator* (DSMZ # 428) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *C. necator* culture are made into BHI and are allowed to grow for aerobically for 48 hours at 30°C at 250 rpm until saturated. As noted elsewhere, previous names for this species are Alcaligenes eutrophus and Ralstonia eutrophus.

*Desulfovibrio fructosovorans* (DSMZ # 3604) is obtained from the German Collection of Microorganisms and Cell Cultures (Braunschweig, Germany) as a vacuum dried culture. Cultures are then resuspended in *Desufovibrio fructosovorans* medium (#63) as described per DSMZ instructions. *D.fructosovorans* is grown anaerobically at 37°C at 250 rpm until saturated.

*Escherichia coli Crooks* (DSMZ#1576) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *E. coli Crooks* culture are made into BHI and are allowed to grow for aerobically for 48 hours at 37°C at 250 rpm until saturated.

*Escherichia coli 12* a gift from the Gill lab (University of Colorado at Boulder, Boulder, CO USA) and is obtained as an actively growing culture. Serial dilutions of the actively growing *E. coli K12* culture are made into Luria Broth (RPI Corp., Mt. Prospect, IL USA) and are allowed to grow for aerobically for 24 hours at 37°C at 250 rpm until saturated.

*Halobacterium salinarum* (DSMZ# 1576) is obtained from the German Collection of Microorganisms and Cell Cultures (Braunschweig, Germany) as a vacuum dried culture. Cultures are then resuspended in *Halobacterium* medium (#97) as described per DSMZ instructions. *H. salinarum* is grown aerobically at 37°C at 250 rpm until saturated.

*Lactobacillus delbruecki* (#4335) is obtained from WYEAST USA (Odell, OR USA) as an actively growing culture. Serial dilutions of the actively growing *L. delbruecki* culture are made into Brain Heart Infusion (BHI) broth (RPI Corp., Mt. Prospect, IL USA) and are allowed to grow for aerobically for 24 hours at 30°C at 250 rpm until saturated.

*Metallosphaera sedula* (DSMZ #5348) is obtained from the German Collection of Microorganisms and Cell Cultures (Braunschweig, Germany) as an actively growing culture. Serial dilutions of *M. sedula* culture are made into *Metallosphaera* medium (#485) as described per DSMZ instructions. *M. sedula* is grown aerobically at 65°C at 250 rpm until saturated.

*Propionibacterium freudenreichii* subsp. *shermanii* (DSMZ# 4902) is obtained from the German Collection of Microorganisms and Cell Cultures (Braunschweig, Germany) as a vacuum dried culture. Cultures are then resuspended in PYG-medium (#104) as described per DSMZ instructions. *P. freudenreichii* subsp. *shermanii* is grown anaerobically at 30°C at 250 rpm until saturated.

*Pseudomonas putida* is a gift from the Gill Lab (University of Colorado at Boulder, Boulder, CO USA) and is obtained as an actively growing culture. Serial dilutions of the actively growing *P. putida* culture are made into Luria Broth (RPI Corp., Mt. Prospect, IL USA) and are allowed to grow for aerobically for 24 hours at 37°C at 250 rpm until saturated.

*Streptococcus mutans* (DSMZ# 6178) is obtained from the German Collection of Microorganisms and Cell Cultures (Braunschweig, Germany) as a vacuum dried culture. Cultures are then resuspended in Luria Broth (RPI Corp., Mt. Prospect, IL USA). *S. mutans* is grown aerobically at 37°C at 250 rpm until saturated.

The following non-limiting strains may also be used as starting strains in the Examples: DF40 Hfr(P02A), garB10, fhuA22, ompF627(T2R), fadL701(T2R), relA1, pitA10, spoTl, rrnB-2, pgi-2, mcrB1, creC510, BW25113 F-, Δ(araD-araB)567, ΔlacZ4787(::rrnB-3), &lambda·, rph-1, Δ(rhaD-rhaB)568, hsdR514, JP111 Hfr(POl), *galE45*(Ga1S), *&lambda⁻, fab*/*392*(ts), *relAl, spoTl, thi-1.* These strains possess recognized genetic modifications, and are available from public culture sources such as the Yale Coli Genetic Stock Collection (New Haven, CT USA). Strains developed from these strains are described in the Examples.

### Common Method Example 2: Cultures and Growth Media

Bacterial growth culture media and associated materials and conditions, are as follows:
Fed-batch medium contained (per liter): 10 g tryptone, 5 g yeast extract, 1.5 g NaC1, 2 g Na2HPO4.7 H2O, 1 g KH2PO4, and glucose as indicated.

AM2 medium contained (per liter): 2.87 g K₂HPO₄, 1.50g KH₂PI₄, 3.13g (NH4)₂SO₄, 0.15g KC1, 1.5 mM MgSO₄, 0.1M K⁺ MOPS pH 7.2, 30 g glucose, and 1 ml trace Mineral Stock prepared as described in Martinez et al. Biotechnol Lett 29:397-404 (2007). Concentration of glucose in glucose feed for AM2 vessels: 200 g/L glucose.

AM2 Medium used in Fermenters for Initial Batch Medium

| | |
|---|---|
| K2HP04 | 2.87 g/L |
| KH2P04 | 1.50 g/L |
| (NH4)2S04 | 3.13 g/L |
| KCl | 0.15 g/L |
| Glucose | 6.0 g/L |
| MgS04 | 0.18 g/L |
| AM2 Trace Metals Stock Solution | 1.0 ml/L |
| Calcium | 0.005 g/L |
| Ampicillin | 0.1 g/L |
| Kanamycin | 0.02 g/L |
| Chloramphenicol | 0.02 g/L |

Trace Metals Stock Solution for AM2 medium used in Fermenters

| | |
|---|---|
| Concentrated HCl | 10.0 ml/L |
| FeCl₃•6H₂O | 2.4 g/L |
| CoC₁₂•6 H₂O | 0.17 g/L |
| CuCb•2 H₂O | 0.15g/L |
| ZnCb | 0.3 g/L |
| Na₂MoO₄•2H₂O | 0.3 g/L |
| H₃BO₃ | 0.07 g/L |
| MnCb•4H₂O | 0.5 g/L |

Rich Medium used in Fermenters Initial Batch Medium

| | |
|---|---|
| Tryptone | 10 g/L |
| Yeast Extract | 5 g/L |
| Glucose | 4 g/L |
| Na₂HPO₄•7H₂O | 2 g/L |
| KH₂PO₄ | 1 g/L |
| MgSO₄ | 2 g/L |
| Ampicillin | 0.1 g/L |
| Kanamycin | 0.02 g/L |
| Chloramphenicol | 0.02 g/L |

Feed Formulation for additional glucose feed for rich media

| | |
|---|---|
| Glucose | 200 g/L |
| (NH₄)₂SO₄ | 30 g/L |
| KH₂PO₄ | 7.5 g/L |
| Citric Acid | 3 g/L |
| MgSO₄ | 2.93 g/L |
| FeSO₄•7H₂O | 0.05 g/L |

SM3 minimal medium for *E. coli* (Final phosphate concentration = 27.5 mM; Final N concentration = 47.4 mM NH₄⁺).

Components per liter: 700 mL DI water, 100 mL 10X SM3 Salts, 2 ml lM MgSO₄, 1 mL 100X Trace Mineral Stock, 60 mL 500 g/L glucose, 100 mL 0.1 M MOPS (pH 7.4), 0.1 mL of 1 M CaCl₂, Q.S. with DI water to 1000 mL, and 0.2 µm filter sterilize.

### To Make SM8 Minimal Media

SM8 minimal medium for *E. coli* (Final phosphate concentration= 3.2 mM; Final N concentration= 45 mM NH₄)+. Components per liter: 600 mL DI water, 100 mL 10X FM8 Salts, 2.26 mL 1M MgSO₄, 2 mL FM10 Trace Mineral Stock, 10 mL 100 g/L yeast extract, 60 mL 500 g/L glucose, 200 mL 1M MOPS (pH 7.4), Q.S. with DI water to 1000 mL, and 0.2 µm filter sterilize.

### Preparation of Stock Solutions:

To make 10X SM3 Salts (1 L): 800 mL DI water, 28.7 g K₂HPO₄, 15 g KH₂PO₄, 31.3 g (NH₄)₂SO₄, 1.5 g KCl, 0.5 g Citric Acid (anhydrous), and Q.S. with DI water to 1000 mL.

To make 100X Trace Mineral Stock (1L): save in 50-mL portions at room temp: Per liter in 0.12M HCl (dilute 10 mL cone HCl into 1 liter water):2.4 g FeCL₃ 6H₂O, 0.17 g CoCh₆H₂O, 0.15 g CuCl₂, 2H₂O, 0.3 g ZnCl₂, 0.3 g NaMoO₄.2H₂O (Molybdic acid, disodium salt, dihydrate), 0.07 g H₃BO₃, and 0.5 g MnCh₄H₂O.

To make 1M MOPS:209.3 g MOPS, dissolve in 700 mL water. Take 70-mL portions and adjust to desired pH with 50% KOH, adjust to 100 mL final volume, and 0.2 µm filter sterilize.

To make 1M MgSO₄:120.37 g dissolved in 1000 mL water.

To make 500 g/L (50%) glucose stock solution: 900 mL DI water, 500 g glucose, and Q.S. to 1000 mL.

To make 10X FM8 Salts (1 L): 800 mL DI water, 3.29 g K₂HPO₄, 1.73 g KH₂PO₄, 30 g (NH₄)₂SO₄, 1.5 g Citric Acid (anhydrous), and Q.S. with DI water to 1000 mL.

To make FM10 Trace Mineral Stock (100 mL): 50 mL DI water, 1 mL 13M HCl, 4.9 g CaCl₂*2H20, 0.97 g FeCL₃•6H₂O, 0.04 g CoCl₂•6H₂O, 0.27 g CuCl₂•H₂O, 0.02 g ZnCl₂, 0.024 g NaMoO₄•2H₂O, 0.007 g H₃BO₃, and 0.036 g MnCl₂.4H₂O, Q.S. with DI water to 100 mL.

To make 1M MOPS: 209.3 g MOPS, dissolve in 700 mL water, take 70-mL portions and adjust to desired pH with 50% KOH, adjust to 100 mL final volume, and 0.2 µm filter sterilize.

To make 1M MgSO₄: 120.37 g dissolved in 1000 mL water.

To make 500 g/L (50%) glucose stock solution: 900 mL DI water, 500 g glucose, and Q.S. to 1000 mL.

To make 100 g/L yeast extract: 900 mL water: 100 g yeast extract, and Q.S. to 1000 mL.

Additional Growth Media Formulation(s) is/are summarized as:

| | Ingredient | Concentration in FM11 |
|---|---|---|
| 1 | K₂HPO₄ | 0.329 g/L |
| 2 | KH₂PO₄ | 0.173 g/L |
| 3 | (NH₄)₂SO₄ | 3 g/L |
| 4 | NaCl | -------- |
| 5 | Citric Acid•H₂O | 0.15 g/L |
| 6 | Yeast Extract | 1 g/L |
| 7 | Antifoam 204 | 0.1 mL/L |
| 8 | Glucose | 30 g/L |
| 9 | MgSO₄ •7H₂O | 0.82 g/L |
| 10 | FM10 Trace | 2 mL/L |
| | Metals Stock Solution | |
| 11 | Kanamycin | 35 mg/L |
| 12 | Chloramphenicol | 20 mg/L |
| 13 | 1000x Vitamin Mixture | 1.25 mL/L |

FM10: Trace Metals Stock Solution formulation:

| Ingredient | Concentration |
|---|---|
| Concentrated HCl | 10.0 ml/L |
| CaCl₂.2H₂O | 49 g/L |
| FeCl₃•6H₂O | 9.7 g/L |
| CoCl₂•6H₂O | 0.4 g/L |
| CuCl₂•2H₂O | 2.7 g/L |
| ZnCl₂ | 0.2 g/L |
| Na₂MoO₄•2H₂O | 0.24 g/L |
| H₃BO₃ | 0.07 g/L |
| MnCl₂•4H₂O | 0.36 g/L |

1000x Vitamin Mixture:

| Component | Amount (g/L) |
|---|---|
| Thiamine Hydrochloride | 5.0 |
| D-Pantothenic Acid | 5.4 |
| Nicotinic Acid | 6.0 |
| Biotin | 0.06 |
| q.s. with DI water to final volume | |

### To make 1 L M9 minimal media:

*M9* minimal media was made by combining 5X M9 salts, 1M MgS0₄, 20% glucose, 1M CaCl₂ and sterile deionized water. The 5X M9 salts are made by dissolving the following salts in deionized water to a final volume of 1L: 64g Na₂HPO₄ • 7H₂O, 15g KH₂PO₄,2.5g NaCl, 5.0g NH₄Cl. The salt solution was divided into 200 mL aliquots and sterilized by autoclaving for 15 minutes at 15 psi on the liquid cycle. A 1M solution of MgSO₄ and 1M CaCl₂ were made separately, then sterilized by autoclaving. The glucose was filter sterilized by passing it thought a 0.22µm filter. All of the components are combined as follows to make 1L of M9: 750mL sterile water, 200 mL 5X M9 salts, 2mL of 1M MgSO₄, 20 mL 20% glucose, 0.1 mL CaCl₂, Q.S. to a final volume of 1L.

### To make EZ rich media:

All media components were obtained from TEKnova (Hollister, CA USA) and combined in the following volumes. 100 mL 10X MOPS mixture, 10 mL 0.132M K₂ HPO₄, 100 mL 10X ACGU, 200 mL 5X Supplement EX, 10 mL 20% glucose, 580 mL sterile water.

### To make FGN30 Medium:

FGN30 medium is made of the following components in the concentrations listed:

| Chemical | FGN Medium Final concentration | |
|---|---|---|
| K2HPO4 | 3.746 | g/L |
| KH2PO4 | 1.156 | g/L |
| NH4Cl | 0.962 | g/L |
| NaCl | 0.702 | g/L |
| Citric Acid | 66 | mg/L |
| FeSO4.7H2O | 16.68 | mg/L |
| ZnCl2 | 0.1 | mg/L |
| MnCl2.4H2O | 0.03 | mg/L |
| CoCl2.6H2O | 0.05 | mg/L |
| CuCl2.2H2O | 0.07 | mg/L |
| NiCl2.6H2O | 0.12 | mg/L |
| Na2MoO4.2H2O | 0.03 | mg/L |
| CrCl3.6H2O | 0.05 | mg/L |
| H3BO3 | 0.3 | mg/L |
| CaCl2 | 11 | mg/L |
| MgSO4 | 240 | mg/L |
| Fructose | 2 | g/L |
| Glycerol | 2 | g/L |

FGN30 medium can be made according to the following protocol:
Measure each stock solution in the order as listed below. Mix well after adding each stock solution before adding the next stock solution. Filter and sterilize using a 0.2 µm bottle top vacuum filter.

| Chemical Name | Quantity | |
|---|---|---|
| Water (DI) | 800 | mL |
| 10x Mineral Salts Solution | 100 | mL |
| 1000x Micronutrient stock solution | 1 | mL |
| 1 M MgSO₄ stock solution | 2 | mL |
| Fructose | 15 | g |
| Glycerol | 15 | g |
| 1 M CaCl₂ stock solution | 100 | µL |
| Q.S. using DI water to: | 1000 | mL |

### Preparation of FGN30 Stock solutions:

10x Mineral Salts Solution: Measure each chemical in the order as listed below. Allow each chemical to dissolve completely before adding the next chemical.

| Chemical Name | Quantity | |
|---|---|---|
| Water (DI) | 800 | mL |
| K2HPO4 | 37.46 | g |
| KH2PO4 | 11.56 | g |
| NH4Cl | 9.62 | g |
| NaCl | 7.02 | g |
| Citric Acid, anhydrous | 0.5 | g |
| Q.S. using DI water to: | 1000 | mL |

1000x Micronutrient Stock Solution: Measure each chemical in the order as listed below. Allow each chemical to dissolve completely before adding the next chemical.

| Chemical Name | Quantity | |
|---|---|---|
| Water (DI) | 800 | mL |

| | | |
|---|---|---|
| Citric Acid anhydrous | 16 | g |
| FeSO4.7H2O | 16.68 | g |
| ZnCl2 | 0.1 | g |
| MnCl2.4H2O | 0.03 | g |
| CoCl2.6H2O | 0.05 | g |
| CuCl2.2H2O | 0.07 | g |
| NiCl2.6H2O | 0.12 | g |
| Na2MoO4.2H2O | 0.03 | g |
| CrCl3.6H2O | 0.05 | g |
| H3BO3 | 0.3 | g |
| Q.S. using DI water to: | 1000 | mL |

### To make FGN30HN Medium:

FGN30HN medium can be made according to the following protocol: Measure each stock solution in the order as listed below. Mix well after adding each stock solution before adding the next stock solution. Filter sterilize using a 0.2 µm bottle top vacuum filter.

| Chemical Name | Quantity | |
|---|---|---|
| Water (DI) | 800 | mL |
| 10x Mineral Salts Solution | 100 | mL |
| 1000x Micronutrient stock solution | 1 | mL |
| 1 M MgSO₄ stock solution | 2 | mL |
| Fructose | 15 | g |
| Glycerol | 15 | g |
| 1 M CaCl₂ stock solution | 100 | µL |
| Q.S. using DI water to: | 1000 | mL |

### Preparation of FGN30HN Stock solutions:

10x High nitrogen Mineral Salts Solution: Measure each chemical in the order as listed below. Allow each chemical to dissolve completely before adding the next chemical.

| Chemical Name | Quantity | |
|---|---|---|
| Water (DI) | 800 | mL |

| | | |
|---|---|---|
| K2HPO4 | 7.492 | g |
| KH2PO4 | 2.312 | g |
| NH4Cl | 28.86 | g |
| NaCl | 7.02 | g |
| Citric Acid, anhydrous | 0.5 | g |
| Q.S. using DI water to: | 1000 | mL |

1000x Micronutrient Stock Solution: Measure each chemical in the order as listed below. Allow each chemical to dissolve completely before adding the next chemical.

| Chemical Name | Quantity | |
|---|---|---|
| Water (DI) | 800 | mL |
| Citric Acid anhydrous | 16 | g |
| FeSO4.7H2O | 16.68 | g |
| ZnCl2 | 0.1 | g |
| MnCl2.4H2O | 0.03 | g |
| CoCl2.6H2O | 0.05 | g |
| CuCl2.2H2O | 0.07 | g |
| NiCl2.6H2O | 0.12 | g |
| Na2MoO4.2H2O | 0.03 | g |
| CrCl3.6H2O | 0.05 | g |
| H3BO3 | 0.3 | g |
| Q.S. using DI water to: | 1000 | mL |

To make MSM Medium: MSM medium is made of the following components in the concentrations listed:

| Chemical | MSM Medium Final concentration | |
|---|---|---|
| K2HPO4 | 3.746 | g/L |
| KH2PO4 | 1.156 | g/L |
| NH4Cl | 0.962 | g/L |
| NaCl | 0.702 | g/L |
| Citric Acid | 66 | mg/L |

| | | |
|---|---|---|
| FeSO4.7H2O | 16.68 | mg/L |
| ZnCl2 | 0.1 | mg/L |
| MnCl2.4H2O | 0.03 | mg/L |
| CoCl2.6H2O | 0.05 | mg/L |
| CuCl2.2H2O | 0.07 | mg/L |
| NiCl2.6H2O | 0.12 | mg/L |
| Na2MoO4.2H2O | 0.03 | mg/L |
| CrCl3.6H2O | 0.05 | mg/L |
| H3BO3 | 0.3 | mg/L |
| CaCl2 | 11 | mg/L |
| MgSO₄ | 240 | mg/L |
| Fructose | NA | |
| Glycerol | NA | |

MSM Medium (for chemolithotropic growth of *C. necator*): Measure each stock solution in the order as listed below. Mix well after adding each stock solution before adding the next stock solution. Filter and sterilize using a 0.2 µm bottle top vacuum filter.

| Chemical Name | Quantity | |
|---|---|---|
| Water (DI) | 650 | mL |
| 1M MOPS pH 7.4 | | 150mL |
| 10x Mineral Salts Solution with 3x NH₄Cl and 0.2x PO₄ (HN mineral salts) | 100 | mL |
| 1000x Micronutrient stock solution | 1 | mL |
| 1 M MgSO₄ stock solution | 2 | mL |
| 1 M CaCl₂ stock solution | 100 | µL |
| Q.S. using DI water to: | 1000 | mL |

### Common Method Example 3: Gel Preparation, DNA Separation, Extraction, Ligation, and Transformation

Molecular biology grade agarose (RPI Corp., Mt. Prospect, IL USA) is added to 1x TAE to make a 1% Agarose in TAE. To obtain 50x TAE add the following to 900 mL distilled H₂O : 242g Tris base (RPI Corp., Mt. Prospect, IL USA), 57.1 mL Glacial Acetic Acid (Sigma-Aldrich, St. Louis, MO USA), 18.6 g EDTA (Fisher Scientific, Pittsburgh, PA USA), and adjust volume to 1L with additional distilled water. To obtain 1x TAE, add 20 mL of 50x TAE to 980 mL of distilled water. The agarose-TAE solution is then heated until boiling occurred and the agarose is fully dissolved. The solution is allowed to cool to 50°C before 10 mg/mL ethidium bromide (Acros Organics, Morris Plains, NJ USA) is added at a concentration of 5 µL per 100 mL of 1% agarose solution. Once the ethidium bromide is added, the solution is briefly mixed and poured into a gel casting tray with the appropriate number of combs (Idea Scientific Co., Minneapolis, MN USA) per sample analysis. DNA samples are then mixed accordingly with 5X TAE loading buffer. 5X TAE loading buffer consists of 5X TAE(diluted from 50X TAE as described herein), 20% glycerol (Acros Organics, Morris Plains, NJ USA), 0.125% Bromophenol Blue (Alfa Aesar, Ward Hill, MA USA), and adjust volume to 50 mL with distilled water. Loaded gels are then run in gel rigs (Idea Scientific Co., Minneapolis, MN USA) filled with 1X TAE at a constant voltage of 125 volts for 25-30 minutes. At this point, the gels are removed from the gel boxes with voltage and visualized under a UV transilluminator (FOTODYNE Inc., Hartland, WI USA).

The DNA isolated through gel extraction is then extracted using the QIAquick Gel Extraction Kit following manufacturer's instructions (Qiagen, Valencia, CA USA). Similar methods are known to those skilled in the art.

The thus-extracted DNA then may be ligated into pSMART (Lucigen Corp., Middleton, WI USA), StrataClone (Stratagene, La Jolla, CA USA) or pCR2.1-TOPO TA (Invitrogen Corp., Carlsbad, CA USA) according to manufacturer's instructions.

### Ligation Methods

### For ligations into pSMART vectors:

Gel extracted DNA is blunted using PCRTerminator (Lucigen Corp., Middleton, WI USA) according to manufacturer's instructions. Then 500 ng of DNA is added to 2.5 µL 4x CloneSmart vector premix, 1 µL CloneSmart DNA ligase (Lucigen Corp., Middleton, WI USA) and distilled water is added for a total volume of 10 µL. The reaction is then allowed to sit at room temperature for 30 minutes and then heat inactivated at 70°C for 15 minutes and then placed on ice. *E. coli* 10G Chemically Competent cells (Lucigen Corp., Middleton, WI USA) are thawed for 20 minutes on ice. 40 µL of chemically competent cells are placed into a microcentrifuge tube and 1 µL of heat inactivated CloneSmart Ligation is added to the tube. The whole reaction is stirred briefly with a pipette tip. The ligation and cells are incubated on ice for 30 minutes and then the cells are heat shocked for 45 seconds at 42°C and then put back onto ice for 2 minutes. 960 µL of room temperature Recovery media (Lucigen Corp., Middleton, WI USA) and places into microcentrifuge tubes. Shake tubes at 250 rpm for 1 hour at 37°C. Plate 100 µL of transformed cells on Luria Broth plates (RPI Corp., Mt. Prospect, IL USA) plus appropriate antibiotics depending on the pSMART vector used. Incubate plates overnight at 37°C.

### For Litigations into StrataClone:

Gel extracted DNA is blunted using PCRTerminator (Lucigen Corp., Middleton, WI USA) according to manufacturer's instructions. Then 2 µL of DNA is added to 3 µL StrataClone Blunt Cloning buffer and 1 µL StrataClone Blunt vector mix amplkan (Stratagene, La Jolla, CA USA) for a total of 6 µL. Mix the reaction by gently pipeting up and down and incubate the reaction at room temperature for 30 minutes then place onto ice. Thaw a tube of StrataClone chemically competent cells (Stratagene, La Jolla, CA USA) on ice for 20 minutes. Add 1 µL of the cloning reaction to the tube of chemically competent cells and gently mix with a pipette tip and incubate on ice for 20 minutes. Heat shock the transformation at 42°C for 45 seconds then put on ice for 2 minutes. Add 250 µL pre-warmed Luria Broth (RPI Corp., Mt. Prospect, IL USA) and shake at 250 rpm for 37°C for 2 hours. Plate 100 µL of the transformation mixture onto Luria Broth plates (RPI Corp., Mt. Prospect, IL USA) plus appropriate antibiotics. Incubate plates overnight at 37°C.

### For Ligations into pCR2.1-TOPO TA:

Add 1 µL TOPO vector, 1 µL Salt Solution (Invitrogen Corp., Carlsbad, CA USA) and 3 µL gel extracted DNA into a microcentrifuge tube. Allow the tube to incubate at room temperature for 30 minutes then place the reaction on ice. Thaw one tube of TOP10F chemically competent cells (Invitrogen Corp., Carlsbad, CA USA) per reaction. Add 1 µL of reaction mixture into the thawed TOP10F cells and mix gently by swirling the cells with a pipette tip and incubate on ice for 20 minutes. Heat shock the transformation at 42°C for 45 seconds then put on ice for 2 minutes. Add 250 µL pre-warmed SOC media (Invitrogen Corp., Carlsbad, CA USA) and shake at 250 rpm for 37°C for 1 hour. Plate 100 µL of the transformation mixture onto Luria Broth plates (RPI Corp., Mt. Prospect, IL USA) plus appropriate antibiotics. Incubate plates overnight at 37°C.

### General Transformation and Related Culture Methodologies:

Chemically competent transformation protocols are carried out according to the manufacturer's instructions or according to the literature contained in Molecular Cloning (Sambrook and Russell, 2001). Generally, plasmid DNA or ligation products are chilled on ice for 5 to 30 minutes in solution with chemically competent cells. Chemically competent cells are a widely used product in the field of biotechnology and are available from multiple vendors, such as those indicated in this Subsection. Following the chilling period cells generally are heat-shocked for 30 seconds at 42°C without shaking, re-chilled and combined with 250 microliters of rich media, such as SOC. Cells are then incubated at 37°C while shaking at 250 rpm for 1 hour. Finally, the cells are screened for successful transformations by plating on media containing the appropriate antibiotics.

Alternatively, selected cells may be transformed by electroporation methods such as are known to those skilled in the art.

The choice of an *E. coli* host strain for plasmid transformation is determined by considering factors such as plasmid stability, plasmid compatibility, plasmid screening methods and protein expression. Strain backgrounds can be changed by simply purifying plasmid DNA as described herein and transforming the plasmid into a desired or otherwise appropriate *E. coli* host strain such as determined by experimental necessities, such as any commonly used cloning strain (e.g., DH5α, Top10F', *E. coli* 10G, etc.).

Plasmid DNA was prepared using the commercial miniprep kit from Qiagen (Valencia, CA USA) according to manufacturer's instructions.

### Common Method Example 4: 3-HP Preparation and Analysis

A 3-HP stock solution was prepared as follows. A vial of β-propiolactone (Sigma-Aldrich, St. Louis, MO USA) was opened under a fume hood and the entire bottle contents was transferred to a new container sequentially using a 25-mL glass pipette. The vial was rinsed with 50 mL of HPLC grade water and this rinse was poured into the new container. Two additional rinses were performed and added to the new container. Additional HPLC grade water was added to the new container to reach a ratio of 50 mL water per 5 mL β-propiolactone. The new container was capped tightly and allowed to remain in the fume hood at room temperature for 72 hours. After 72 hours the contents were transferred to centrifuge tubes and centrifuged for 10 minutes at 4,000 rpm. Then the solution was filtered to remove particulates and, as needed, concentrated by use of a rotary evaporator at room temperature. Assay for concentration was conducted, and dilution to make a standard concentration stock solution was made as needed.

### Analytical Methods for 3-HP Detection

Analysis of Cultures for 3-HP Production: For HPLC analysis of 3-HP, the Waters Chromatography System (Milford, MA USA) consisted of the following: 600S Controller, 616 Pump, 717 Plus Autosampler, 486 Tunable UV Detector, and an in-line mobile phase Degasser. In addition, an Eppendorf (Hamburg, Germany) external column heater is used and the data are collected using an SRI (Torrance, CA USA) analog-to-digital converter linked to a standard desk top computer. Data are analyzed using the SRI Peak Simple software. A Coregel 64H ion exclusion column (Transgenomic, Inc., San Jose, CA USA) is employed. The column resin is a sulfonated polystyrene divinyl benzene with a particle size of 10µm and column dimensions are 300 x 7.8 mm. The mobile phase consisted of sulfuric acid (Fisher Scientific, Pittsburgh, PA USA) diluted with deionized (18 MΩcm) water to a concentration of 0.02 N and vacuum filtered through a 0.2 µm nylon filter. The flow rate of the mobile phase is 0.6 mL/min. The UV detector is operated at a wavelength of 210 nm and the column is heated to 60°C. The same equipment and method as described herein is used for 3-HP analyses for relevant examples.

The following method is used for GC-MS analysis of 3-HP. Soluble monomeric 3-HP is quantified using GC-MS after a single extraction of the fermentation media with ethyl acetate. Once the 3-HP has been extracted into the ethyl acetate, the active hydrogens on the 3-HP are replaced with trimethylsilyl groups using N,O-Bis-(*Trimethylsilyl)* trifluoroacetamide to make the compound volatile for GC analysis. A standard curve of known 3-HP concentrations is prepared at the beginning of the run and a known quantity of ketohexanoic acid (1 g/L) is added to both the standards and the samples to act as an internal standard for Quantitation, with tropic acid as an additional internal standard. The 3-HP content of individual samples is then assayed by examining the ratio of the ketohexanoic acid ion (m/z = 247) to the 3-HP ion (219) and compared to the standard curve. 3-HP is quantified using a 3HP standard curve at the beginning of the run and the data are analyzed using HP Chemstation. The GC-MS system consists of a Hewlett Packard model 5890 GC and Hewlett Packard model5972 MS. The column is Supelco SPB-1 (60m X 0.32mm X 0.25 flm film thickness). The capillary coating is a non-polar methylsilicone. The carrier gas is helium at a flow rate of 1 mL/min. The 3-HP as derivatized is separated from other components in the ethyl acetate extract using either of two similar temperature regimes. In a first temperature gradient regime, the column temperature starts with 40°C for 1 minute, then is raised at a rate of 10°C/minute to 235°C, and then is raised at a rate of 50°C/minute to 300°C. In a second temperature regime, which was demonstrated to process samples more quickly, the column temperature starts with 70°C which is held for 1 minute, followed by a ramp-up of 10°C/minute to 235°C which is followed by a ramp-up of 50°C/minute to 300°C.

A bioassay for detection of 3-HP also was used in various examples. This determination of 3-HP concentration was carried out based on the activity of the *E. coli* 3-HP dehydrogenase encoded by the *ydfG* gene (the YDFG protein). Reactions of 200-µl were carried out in 96-well microtiter plates, and contained 100 mM Tris-HCl, pH 8.8, 2.5 mM MgCl₂, 2.625 mM NADP⁺, 3 µg g purified YDFG and 20 µL culture supernatant. Culture supernatants were prepared by centrifugation in a microfuge (14,000 rpm, 5 min) to remove cells. A standard curve of 3-HP (containing from 0.025 to 2 g/L) was used in parallel reactions to quantitate the amount of 3-HP in culture supernatants. Uninoculated medium was used as the reagent blank. Where necessary, the culture supernatant was diluted in medium to obtain a solution with 3-HP concentrations within that of the standard curve.

The reactions were incubated at 37°C for 1 hr, and 20 µL of color developer containing 1.43 mM nitroblue tetrazolium, 0.143 phenazine methosulfate, and 2.4% bovine serum albumin were added to each reaction. Color development was allowed to proceed at 37°C for an additional hour, and the absorbance at 580 nm was measured. 3-HP concentration in the culture supernatants was quantitated by comparison with the values obtained from the standard curve generated on the same microtiter plate. The results obtained with the enzymatic assay were verified to match those obtained by one of the analytical methods described above.

### Common Method Example 5: Enzyme assay methods for the quantification of enzyme activities

### Pyruvate dehydrogenase assay

Strains to be evaluated were started in 5ml TB overnights, and 1 ml were diluted into 100 ml SM8 medium and grown at 30°C for ~10 hr. The SM8 cultures was harvested in 2 x 50-ml aliquots by centrifugation and the cell pellet washed into Eppendorf tubes with 1 ml Butterfield's diluent. After recentrifugation, the diluent was removed and the cell pellets stored at -80°C until lysis. Cell pellets were resuspended with 1 ml 50 mM Tris-HCl, pH 8.0, 25 mM NaCl, 2 mM EDTA, 1 mM DTT, 250 U/ml Benzonase and transferred to a 2-ml screw cap Eppendorf tube half-filled with glass beads. A lysate was prepared by disruption of the cells in the BeadBeater for 90s, and clarified by centrifugation (13.2 krpm, 5 min, 4°C). PDH assays were carried out according to the SOP using the supernatant, and protein concentrations in the lysates determined using the Pierce660 reagent. Lysate protein was varied between 0.01 and 0.04 mg total protein per 200 µL reaction, and the specific activity calculated from the slope of the linear curve fitted to the data. The effect of NADH was measured by adding varying amounts of NADH to an assay containing 1 mM NAD+, and monitoring the increase in A340.

### St-Mcr

Cell line carrying plasmids able to over express malonyl CoA reductases were grown with antibiotic selection in LB media overnight as starter cultures. These overnight starter cultures were used to inoculate either 50 mL to 100 mL expression cultures grown with antibiotic selection in LB media supplemented with 1mM Isopropyl β-D-1-thiogalactopyranoside (IPTG) to induce protein production. Cultures were grown 24 hr, after which the cells were collected by centrifugation. Cell pellets were lysed using a mixture of Bugbuster, benzonase nuclease, and rLysozyme (all from Novagen). Once lysed, the lysate mixture was centrifuged at 14000 RPM in a standard table top centrifuge. The resulting supernatant was removed to another tube. The clarified supernatant was measure for protein concentration using a Biorad Total Protein determination kit (BioRad). For each measurement, 20 uL of lysate was added to a reaction buffer filled well of the 96-well plate used to perform the assay. All samples were performed in duplicates. The assay was initiated by addition of malonyl CoA to a final concentration of 0.3 mM or 1mM, which is well above the reported Km binding constant for these enzymes. Once the reaction time course was read and the slopes of each well were calculated, the specific activities were compared to a negative control to determine a background rate. All values reported are the average specific activities measured in triplicate.

### MmsB

The coupled assay uses lysates overexpressing various dehydrogenases (YdfG, MmsB, and the dehydrogenase domain of *Chloroflexus aurantiacus* MCR) able to convert malonate semialdehyde formed by the *Sulfolobus tokodaii* MCR to 3-hydroxypropionate. The formation of 3-hydroxypropionate was assessed using gas chromatography - mass spectrometry (GC-MS). The reactions for these assays were performed as 750 uL reactions containing 20 uL of clarified whole cell lysates from *Sulfolobus tokodaii* MCR over expressing cultures and 20 uL of clarified whole cell lysates from cells expressing one of three dehydrogenases (ydfG, mmsB, the *Chloroflexus aurantiacus).* The buffer conditions consisted of 1 mM malonyl CoA, 2 mM NADH or 2mM NADH, 5 mM dithiothreitol, 3 mM magnesium chloride, 100 mM Trizma-HCl pH7.6 buffer. Lysates for these assays were prepared as follows. Cell line carrying plasmids able to over express ydfG, mmsB, the *Chloroflexus aurantiacus* dehydrogenase domain, or the *Sulfolobus tokodaii* malonyl CoA reductases were grown with antibiotic selection in LB media overnight as starter cultures. These overnight starter cultures were used to inoculate either 50 mL to 100 mL expression cultures grown with antibiotic selection in LB media supplemented with 1mM Isopropyl β-D-1-thiogalactopyranoside (IPTG) to induce protein production. Cultures were grown 24 hr, after which the cells were collected by centrifugation. Cell pellets were lysed using a mixture of Bugbuster, benzonase nuclease, and rLysozyme (all from Novagen). Once lysed, the lysate mixtures were centrifuged at 14000 RPM in a standard table top centrifuge. The resulting supernatants were removed to another tube. Various combination dehydrogenases with and without the *Sulfolobus tokodaii* malonyl CoA reductases were evaluated. Reactions were incubated at 37 degrees Celsius for 12 to 15 hours. With each assay set, negative control samples for of the proteins overexpressed were included to make sure no lysate had the ability to form 3-hydroxypropionate with a combination of a CoA reductase domain and a dehydrogenase domain. After incubation, all samples were submitted for GS-MS analysis as described elsewhere herein.

### Acetyl-CoA carboxylase assay

Acetyl-CoA carboxylase assay (AccADBC) activities were determined by a coupled enzymatic assay with mcr. Cells were resuspended in ∼1 mL of Lysis/Assay Buffer (50 mM Tris, pH 8.0, 25 mM NaCl, 2 mM EDTA, 2% PEG and 1 mM DTT) containing 250 U/mL Benzonase (EMD Chemicals). Cells were lysed using Bead Beater at ∼75% intensity for ∼1.5 minutes and spin to remove cellular debris. The assay reaction mixture was made as follows: 25 mM Trizma, pH 8.0, 2.5 mM MgCl₂, 1 mM DTT, 15 mM Ammonium Sulfate, 7.5 mM NaHCO₃, 1 mM NADPH, 1 mM ATP, 0.005 mM Biotin, 2% PEG and titrated the cell lysate to a final volume of 200 ul. Reactions were initiated with 1 mM Acetyl CoA and kinetic reads were performed at Abs 340nm. (Note: for strains which are not overexpressing mcr, the addition of purified mcr is required for the assay to work).

### Glutamate dehydrogenase assay

The reaction was performed in a 96 well plate format with samples for each lysate performed in duplicate. Each reaction was carried out in a 200 uL volume, and the buffer conditions for the assay were 50 mM Trizma buffer pH7.5, 15mM ammonium chloride, 1 mM NADPH. For lysates, samples of each culture were pelleted and then lysed using a mixture of Bugbuster, benzonase nuclease, and rLysozyme (all from Novagen). To each tech well, 10 microliters of lysate was added. A baseline activity for each well was measured for 10 minutes using a Molecular Dynamics SpectraMax 384 microplate reader with SoftmaxPro software (Molecular Dynamics, Sunnyvale CA) to quantitate the rate of change in the 340 nm absorbance. All assays were conducted at 30°C, and the progress of each reaction was monitored for 30 minutes during which measurements were made every 20 seconds. To initiate the glutamate dehydrogenase specific activity, 2-ketoglutarate was added to a 15 mM concentration and again the progress of each reaction was monitored for 30 minutes during which measurements were made every 20 seconds. From these two sets of reading, a rate was calculated by subtracting the baseline rate from the 2-ketoglutarate rate. The specific glutamate dehydrogenase activity was calculated by adjusting the observed rates by the amount of total lysate protein added to each well. The total lysate protein of each prepared lysate was determined using the Pierce 660nm Protein Assay (Rockville, Illinois).

### Common Method Example 6: Strain evaluation methods for evaluating 3-HP production

### Low Phosphate Shake Flask Method

3-HP production using production strains was demonstrated at 100-mL scale in SM11 (minimal salts) media without phosphate. Cultures were started from freezer stocks by standard practice (Sambrook and Russell, 2001) into 50 mL of SM11 (minimal salts) media containing 30 mM phosphate plus 35 µg/mL kanamycin and 20 µg/mL chloramphenicol and grown to stationary phase overnight at 30°C with rotation at 250 rpm. Three mL of this culture were transferred to 100 ml of SM11 No Phosphate media plus 30 g/L glucose, 35 µg/ml kanamycin, and 20 µg/mL chloramphenicol in triplicate 250-ml baffled flasks and incubated at 30°C, 250 rpm. To monitor cell growth by these cultures, samples (2 ml) were withdrawn at designated time points for optical density measurements at 600nm (OD₆₀₀, 1 cm pathlength). Cultures were shifted to production by transferring the cultures to 37°C at 6 hours post-inoculation. A sample was collected at this time for analysis of 3HP and enzyme activities. Samples were also collected at 10 and 22 hours post-inoculation for monitoring 3HP production and enzyme activity. To monitor 3HP production by these cultures, samples (10 mL) were withdrawn at the designated time points and pelleted by centrifugation at 12,000rpm for 10 min and the supernatant collected for analysis of 3-HP production as described elsewhere herein. The pellet was frozen at -80°C for analysis of enzyme activity as described under elsewhere herein. Dry cell weight (DCW) is calculated as 0.40 times the measured OD₆₀₀ value, based on baseline DCW to OD₆₀₀ determinations. All data are the average of triplicate cultures. For comparison purposes, the specific productivity is calculated from the averaged data at the 24-h time point and expressed as g 3-HP produced per gDCW.

### Syngas Fermentation Method

3HP production using syngas feed stocks is demonstrated at 0.6 L scale in SM11 (minimal salts) media. Cultures are started from freezer stocks by standard practice (Sambrook and Russell, 2001) into 49 mL of FGN30 medium supplemented with appropriate antibiotics and incubated at 30°C for 24 hours with rotation at 250 rpm. 5 mL of this culture is transferred to 45 ml of FGN30HN with appropriate antibiotics and is incubated at 30°C for 24 hours with rotation at 250 rpm. Columns are setup with appropriate gas flow rates (132ml/min for 600ml columns and 20 ml/min for 20 ml columns - 70% H₂, 20% O₂, 10% CO₂). Water baths are warmed to 30C and columns are filled with MSM-HN media with appropriate antibiotics. FGN30HN overnight cultures are diluted to OD600 = 10-20 and are inoculated into gas-fed columns (60 mL inoculumn into 540 mL of MSM-HN medium for 600 mL cultures and 2 mL into 18 mL MSM-HN for 20 mL cultures). Columns are operated for approximately 72 hours at 30C and samples are removed for 3-HP quantification.

### Common Method Example 7: Minimum Inhibitory Concentration Evaluation (MIC) Protocols

For MIC evaluations, the final results are expressed in chemical agent concentrations determined by analysis of the stock solution by HPLC.

### E. coli aerobic MIC.

The (MIC) was determined aerobically in a 96 well-plate format. Plates were setup such that each individual well, when brought to a final volume of 100 uL following inoculation, had the following component levels (corresponding to standard M9 media): 47.7 mM Na2HPO4, 22 mM KH2PO4, 8.6 mM NaCl, 18.7 mM NH4C1, 2 mM MgS04, 0.1 mM CaC12, and 0.4% glucose. Overnight cultures of strains were grown in triplicate in 5 mL LB (with antibiotic where appropriate). A 1% (v/v) inoculum was introduced into a 5 ml culture of M9 minimal media. After the cells reached mid-exponential phase, the culture was diluted to an OD600 of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were incubated for 24 hours at 37C. The minimum inhibitory 3-HP concentration and maximum 3-HP concentration corresponding to visible cell growth (OD-0.1) was recorded after 24 hours. For cases when MIC > 60 g/L, assessments were performed in plates with extended 3-HP concentrations (0-100 g/L, in 5 g/L increments). (OD-0.1) was recorded after 24 hours. For cases when MIC > 60 g/L, assessments were performed in plates with extended 3-HP concentrations (0-100 g/L, in 5 g/L increments) E. *coli* anaerobic. The minimum inhibitory concentration (MIC) was determined anaerobically in a 96 well-plate format. Plates were setup such that each individual well, when brought to a final volume of 100 uL following inoculation, had the following component levels (corresponding to standard M9 media): 47.7 mM Na2HPO4, 22 mM KH2PO4, 8.6 mM NaC1, 18.7 mM NH4C1, 2 mM MgS04, 0.1 mM CaC12, and 0.4% glucose. Overnight cultures of strains were grown in triplicate in 5 mL LB (with antibiotic where appropriate). A 1% (v/v) inoculum was introduced into a 5 ml culture of M9 minimal media. After the cells reached mid-exponential phase, the culture was diluted to an OD600 of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were sealed in biobag anaerobic chambers that contained gas generators for anaerobic conditions and incubated for 24 hours at 37C. The minimum inhibitory 3-HP concentration and maximum 3-HP concentration corresponding to visible cell growth (OD-0.1) was recorded after 24 hours. For cases when MIC > 60 g/L, assessments were performed in plates with extended 3-HP concentrations (0-100 g/L, in 5 g/L increments).

### B. subtilis aerobic MIC

The minimum inhibitory concentration (MIC) was determined aerobically in a 96 well-plate format. Plates were setup such that each individual well, when brought to a final volume of 100 uL following inoculation, had the following component levels (corresponding to standard M9 media + supplemental glutamate): 47.7 mM Na2HPO4, 22 mM KH2PO4, 8.6 mM NaC1, 18.7 mM NH4C1, 2 mM MgS04, 0.1 mM CaC12, 10 mM glutamate and 0.4% glucose. Overnight cultures of strains were grown in triplicate in 5 mL LB (with antibiotic where appropriate). A 1% (v/v) inoculum was introduced into a 5 ml culture of M9 minimal media + glutamate. After the cells reached mid-exponential phase, the culture was diluted to an OD₆₀₀ of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were incubated for 24 hours at 37C. The minimum inhibitory 3-HP concentration and maximum 3-HP concentration corresponding to visible cell growth (0D-0.1) was recorded after 24 hours. For cases when MIC > 60 g/L, assessments were performed in plates with extended 3-HP concentrations (0-100 g/L, in 5 g/L increment).

### C. necator (R. eutropha) aerobic MIC

The minimum inhibitory concentration (MIC) was determined aerobically in a 96 well-plate format. Plates were setup such that each individual well, when brought to a final volume of 100 uL following inoculation, had the following component levels (corresponding to FGN media): 21.5 mM K2HPO4, 8.5 mM KH2PO4, 18 mM NH4C1, 12 mM NaCl, 7.3 uM ZnCl, 0.15 uM MnC12, 4.85 uM H3B03, 0.21 uM CoC12, 0.41 uM CuC12, 0.50 uM NiC12, 0.12 uM Na2MoO4, 0.19 uM CrC13, 0.06 mM CaC12, 0.5 mM MgS04, 0.06 mM FeS04, 0.2% glycerol, 0.2% fructose. Overnight cultures of strains were grown in triplicate in 5 mL LB (with antibiotic where appropriate). A 1% (v/v) inoculum was introduced into a 5 ml culture of FGN media. After the cells reached mid-exponential phase, the culture was diluted to an OD600 of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were incubated for 24 hours at 30C. The minimum inhibitory 3-HP concentration and maximum 3-HP concentration corresponding to visible cell growth (0D-0.1) was recorded after 24 hours. For cases when MIC > 60 g/L, assessments were performed in plates with extended 3-HP concentrations (0-100 g/L, in 5 g/L increments).

### Example 1: Construction of plasmids expressing malonyl-CoA reductase (mcr)

The nucleotide sequence for the malonyl-CoA reductase gene from Chloroflexus aurantiacus was codon-optimized for E. coli according to a service from DNA2.0 (Menlo Park, CA USA), a commercial DNA gene synthesis provider. This gene sequence (SEQ ID NO:61) incorporated an EcoRI restriction site before the start codon and was followed by a HindIII restriction site. In addition, a ribosomal binding site was placed in front of the start codon. This gene construct was synthesized by DNA2.0 and provided in a pJ206 vector backbone (SEQ ID NO:62). Plasmid DNA pJ206 containing the synthesized mcr gene was subjected to enzymatic restriction digestion with the enzymes EcoRI and HindIII obtained from New England BioLabs (Ipswich, MA USA) according to manufacturer's instructions. The digestion mixture was separated by agarose gel electrophoresis and the appropriate DNA fragment recovered as described in the Common Methods Section. An E. coli cloning strain bearing pKK223-aroH was obtained as a kind gift from the laboratory of Prof. Ryan T. Gill from the University of Colorado at Boulder. Cultures of this strain bearing the plasmid were grown and plasmid DNA prepared as described in the Common Methods Section. Plasmid DNA was digested with the restriction endonucleases EcoRI and HindIII obtained from New England Biolabs (Ipswich, MA USA) according to manufacturer's instructions. This digestion served to separate the aroH reading frame from the pKK223 backbone. The digestion mixture was separated by agarose gel electrophoresis, and he agarose gel slice containing the DNA piece corresponding to the backbone of the pKK223 plasmid was recovered as described in the Common Methods Section.

Purified DNA fragments corresponding to the mcr gene and pK223 vector backbone were ligated and the ligation product was transformed and electroporated according to manufacturer's instructions. The sequence of the resulting vector termed pKK223-mcr was confirmed by routine sequencing performed by a commercial provider (SEQ ID NO:212). pKK223-mcr confers resistance to ampicillin and contains the mcr gene of C. aurantiacus under control of a Piac promoter inducible in E. coli hosts by IPTG.

To express the mcr gene under the regulation of other promoters besides the Piac on pKK223, the synthetic mcr gene was transferred to other plasmids. Plasmid pTrc-Pfrc-mcr was based on pTrcHisA (Invitrogen, Carlsbad, CA; Catalog Number V360-20) and the expression of mcr is directed by the Pfrc IPTG-inducible promoter. The inducer-independent Ptau promoter is based on sequences upstream of the E. coli talA gene. The nucleotide sequence of this promoter, placed immediately upstream of the initiator ATG codon of the synthetic mcr gene, is listed as SEQ ID NO:63.

The P,AA:mcr construct was incorporated by PCR into a pSC-B vector (Stratagene Corporation ,La Jolla, CA, USA), which was propagated in an E. coli stock, the plasmid DNA purified according to methods described elsewhere herein. The P,AA:mcr region in pSC-B-P,AA:mcr was transferred to a plasmid vector, pSMART-HCamp (Lucigen Corporation, Middleton, WI, catalog number 40041-2, GenBank AF399742) by PCR using vector primers, M13F and M13R. The fragment generated by PCR was cloned into pSMART-HCamp according to the manufacturer's protocol resulting in plasmid pSMART(HC)Amp-PwA-mcr (SEQ ID NO:64) in which mcr expression does not require induction with IPTG.

### Example 2: Construction of a plasmid expressing transhydrogenase (pntAB)

A fusion of the inducer-independent E. coli promoter derived from the tpiA gene (P,p,A) and the pyridine nucleotide transhydrogenase genes, pntAB, (SEQ ID NO:38 and SEQ ID NO:40) was created by amplifying the tpiA promoter region and pntAB region from genomic E. coli K12 DNA by polymerase chain reactions. For the pntAB genes, the region was amplified using the pntAB forward primer GGGAACCATGGCAATTGGCATACCAAG (SEQ ID NO:65), noting that all primers disclosed herein are artificial sequences) containing a Ncol site that incorporates the initiator Met for the protein sequence of pntA and the pntAB reverse primer GGGTTACAGAGCTTTCAGGATTGCATCC (SEQ ID NO:66). Likewise, the PtpiA region was amplified using the forward primer GGGAACGGCGGGGAAAAACAAACGTT (SEQ ID NO:67) and the reverse primer GGTCCATGGTAATTCTCCACGCTTATAAGC (SEQ ID NO:68) containing a Ncol restriction site. Polymerase chain reaction products were purified using a PCR purification kit from Qiagen Corporation (Valencia, CA, USA) using the manufacturer's instructions. Following purification, the products were subjected to enzymatic restriction digestion with the enzyme Ncol. Restriction enzymes were obtained from New England BioLabs (Ipswich, MA USA), and used according to manufacturer's instructions. The digestion mixtures were separated by agarose gel electrophoresis, and visualized under UV transillumination as described in the Common Methods Section. Agarose gel slices containing the DNA fragment corresponding to the amplified pntAB gene product and the P,,,,A product were excised from the gel and the DNA recovered with a gel extraction kit from Qiagen used according to manufacturer's instructions. The recovered products were ligated together with T4 DNA ligase (New England BioLabs, Ipswich, MA USA) according to manufacturer's instructions.

Because the ligation reaction can result in several different products, the desired product corresponding to the P,,,,A fragment ligated to the pntAB genes was amplified by polymerase chain reaction and isolated by a second gel purification. For this polymerase chain reaction, the forward primer was GGGAACGGCGGGGAAAAACAAACGTT (SEQ ID NO:67), and the reverse primer was GGGTTACAGAGCTTTCAGGATTGCATCC (SEQ ID NO:66), and the ligation mixture was used as template. The digestion mixtures were separated by agarose gel electrophoresis, and visualized under UV transillumination as described the Common Methods Section. Agarose gel slices containing the DNA piece corresponding to the amplified PJ,iA - pntAB fusion was cut from the gel and the DNA recovered with a standard gel extraction protocol and components from Qiagen according to manufacturer's instructions. This extracted DNA was inserted into a pSC-B vector using the Blunt PCR Cloning kit obtained from Stratagene Corporation (La Jolla, CA, USA) using the manufacturer's instructions. Colonies were screened by colony polymerase chain reactions. Plasmid DNA from colonies showing inserts of correct size were cultured and miniprepped using a standard miniprep protocol and components from Qiagen according to the manufacturer's instruction. Isolated plasmids were checked by restriction digests and confirmed by sequencing. The sequenced-verified isolated plasmids produced with this procedure were designated pSC-B-P,p,A:pntAB.

The Pti,,A:pntAB region in pSC-B-P,i,,A:pntAB was transferred to a pBT-3 vector (SEQ ID NO:69) which provides a broad host range origin of replication and a chloramphenicol selection marker. To achieve this construct, a fragment from pBT-3 vector was produced by polymerase chain amplification using the forward primer AACGAATTCAAGCTTGATATC (SEQ ID NO:70), and the reverse primer GAATTCGTTGACGAATTCTCT (SEQ ID NO:71), using pBT-3 as template. The amplified product was subjected to treatment with DpnI to restrict the methylated template DNA, and the mixture was separated by agarose gel electrophoresis, and visualized under UV transillumination as described in the Common Methods Section. The agarose gel slice containing the DNA fragment corresponding to amplified pBT-3 vector product was cut from the gel and the DNA recovered with a standard gel extraction protocol and components from Qiagen according to manufacturer's instructions. The P,p,A:pntAB insert in pSC-B-P,p,A:pntAB was amplified using a polymerase chain reaction with the forward primer GGAAACAGCTATGACCATGATTAC (SEQ ID NO:72) and the reverse primer TTGTAAAACGACGGCCAGTGAGCGCG (SEQ ID NO:73. Both primers were 5' phosphorylated.

The PCR product was separated by agarose gel electrophoresis, and visualized under UV transillumination as described in the Common Methods Section. Agarose gel slices containing the DNA fragment corresponding to the amplified Pti,,A:pntAB insert was excised from the gel and the DNA recovered with a standard gel extraction protocol and components from Qiagen according to manufacturer's instructions. This insert DNA was ligated into the pBT-3 vector prepared as described herein with T4 DNA ligase obtained from New England Biolabs (Bedford, MA, USA), following the manufacturer's instructions. Ligation mixtures were transformed into E. coli 100 cells obtained from Lucigen Corp according to the manufacturer's instructions. Colonies were screened by colony polymerase chain reactions. Plasmid DNA from colonies showing inserts of correct size were cultured and purified using a standard miniprep protocol and components from Qiagen according to the manufacturer's instruction. Isolated plasmids were checked by restriction digests and confirmed by sequencing. The sequenced-verified isolated plasmid produced with this procedure was designated pBT-3-P,i,,A:pntAB (SEQ ID NO:6).

### Example 3A: Construction of a plasmid expressing acetyl-CoA carboxylase (accABCD).

A plasmid carrying two operons able to express the components the acetyl-CoA carboxyltransferase complex from E. coli was constructed by DNA2.0 (Menlo Park, CA USA), a commercial DNA gene synthesis provider. This construct incorporated the DNA sequences of the accA and accD genes under control of an inducer-independent promoter derived from the E. coli tpiA gene, and the DNA sequences of.the accB and accC genes under control of an inducer-independent promoter derived from the E. coli rpiA genes. Each coding sequence was preceded by a ribosome-binding sequence. The designed operons were provided in a pJ251 vector backbone and was designated pJ251:26385 (SEQ ID NO:74).

The tpiA promoter of the pJ251:26385 plasmid was altered to provide better expression. This modification was incorporated by amplifying the pJ251:26385 plasmid with the forward primer GCGGGGCAGGAGGAAAAACATG (SEQ ID NO:75) and the reverse primer GCTTATAAGCGAATAAAGGAAGATGGCCGCCCCGCAGGGCAG (SEQ ID NO:76). Each of these primers were synthesized with a 5' phosphorylation modification. The resulting PCR product was separated by agarose gel electrophoresis, and the appropriate DNA fragment recovered as described in the Common Methods Section. The recovered product was self-ligated with T4 DNA ligase obtained from New England BioLabs (Ipswich, MA USA) and digested with Dpnl according to manufacturer's instructions. Plasmid DNA from colonies showing inserts of correct size were cultured and purified using a standard miniprep protocol and components from Qiagen according to the manufacturer's instruction. Isolated plasmids were checked by restrictions digests and confirmed by sequencing. The sequenced-verified isolated plasmids produced with this procedure were designated pJ251(26385)-P,,,A:accAD-P,,A:accBC (SEQ ID NO:77).

### Example 3B: Construction of pTrc-PyibD-mcr.

Plasmid maps are shown below. Plasmid 1 was digested with NcoI/Bst1107. A fragment size of 7059 bases was excised from a gel and purified (SEQ ID NO:168). The target promoter sequence was ordered (Integrated DNA Technologies, Coralville, Iowa USA) including with modifications to the native ribosome binding site and subsequently changed to be compatible with existing expression vectors and to accommodate expression of key downstream gene(s) within the vector(s), in this example malonyl CoA reductase (MCR, mcr). Plasmid 2, synthesized by (Integrated DNA Technologies, Coralville, IA USA) to comprise this low-phosphate promoter (see discussion above regarding SEQ ID NOs: 210 and 211), was digested with NcoI/PmlI. A fragment size of 156 bases was excised from a gel and purified, this fragment is the pYibD promoter (SEQ ID NO:210). Fragments were ligated overnight using T4 DNA ligase to create Plasmid 3, identified as pTrc-PyibD-mcr (SEQ ID NO:170).
Plasmid Map 1: Original pTrc-ptrc-MCR
Plasmid Map 2: pIDTSMART-pYibD promoter. Synthesized by Integrated DNA Technologies.
Plasmid Map 3: New MCR construct. pTrc-PyibD-mcr (low phosphate induction) (SEQ ID NO:170).

### Example 4: Construction of specific strains that produce 3-hydroxypropionic acid

According to the respective combinations indicated in the following table, the plasmids described herein w ere introduced into the respective base strains. All plasmids were introduced at the same time via electroporation using standard methods. Transformed cells were grown on the appropriate media with antibiotic supplementation and colonies were selected based on their appropriate growth on the selective media. The mcr expression plasmid pKK223-mcr was transformed into E. coli DF40 (Hfr, garB10, fhuA22, ompF627, fadL701, relAl, pitA10, spoT1, rrnB-2, pgi-2, mcrB1, creC527) or E. coli JP1111 (Hfr, galE45(GalS), LAM-, fabI392(ts, temperature-sensitive), relAl, spoT1, thi-1) as described in the Common Methods Section. As is known in the art, the strains DF40 and JP1111 are generally available E. coli strains, available from sources including the Yale Coli Genetic Stock Collection (New Haven, CT USA). Strains carrying multiple compatible plasmids were constructed from these mcr transformants by preparing cells competent for transformation by electroporation as described in the Common Methods Section and transforming with the additional plasmids. Transformants were subsequently selected for on media containing the appropriate combination of antibiotics.

**Table: Strain names and characteristics**

| Strain name | Host | Plasmids |
|---|---|---|
| KX3_0001 | DF40 | pKK223-mcr |
| JX3_0077 | JP1111 | pKK223-mcr |
| JX3_0087 | JP1111 | pkk223-mcr + pBT-3-PtpiA:pntAB |
| JX3_0097 | JP1111 | pkk223-mcr +pJ251(26385)PtpiA:accAD-PrpiA:accBC |
| JX3_0098 | JP1111 | pKK223-mcr + pJ251(26385)PtpiA:accAD-PrpiA:accBC + pBT-3-PtpiA:pntAB |

### Example 5: Production of 3-hydroxypropionic acid

3-HP production by KX3 0001 was demonstrated at 100-mL scale in fed-batch (rich) or AM2 (minimal salts) media. Cultures were started from freezer stocks by standard practice (Sambrook and Russell, 2001) into 50 mL of LB media plus 100 µg/mL ampicillin and grown to stationary phase overnight at 37°C with rotation at 225 rpm. Five ml of this culture were transferred to 100 ml of fed-batch or AM2 media plus 40 g/L glucose, 100 kg/m1 ampicillin, 1 mM IPTG in triplicate 250-ml baffled flasks, and incubated at 37°C, 225 rpm. To monitor cell growth and 3-HP production by these cultures, samples (2 ml) were withdrawn at designated time points for optical density measurements at 600nm (0D600, 1 cm pathlength) and pelleted by centrifugation at 12,000rpm for 5 min and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Production of 3-HP by strain KX3_0001 in fed-batch medium is shown in the following table. Under these conditions, the specific productivity after 24 h is 0.0041 g 3-HP per gDCW.

**Table: Production of 3-HP by KX3_0001 in fed-batch medium**

| Time (hr) | 3HP (g/L) | OD600 |
|---|---|---|
| 0 | 0.002 | 0.118 |
| 3 | 0.002 | 0.665 |
| 4 | 0.005 | 1.44 |
| 6 | 0.008 | 2.75 |
| 8 | 0.009 | 3.35 |
| 24 | 0.008 | 5.87 |

### Example 6: Effect on 3-HP production of increased malonyl-CoA precursor pools by inhibition of fatty acid synthesis

As described herein, certain chemicals are known to inhibit various enzymes of the fatty acid synthase system, some of which are used as antibiotics given the role of fatty acid synthesis in membrane maintenance and growth, and microorganism growth. Among these inhibitors is cerulenin, which inhibits the KASI ketoacyl-ACP synthase (e.g., fabB in E. coli). To further evaluate approaches to modulate and shift malonyl-CoA utilization in microorganisms that comprise production pathways to a selected chemical product, here 3-HP, wherein malonyl-CoA is a substrate in that pathway, addition of cerulenin during a culture was evaluated.

Pathways downstream of malonyl-CoA are limited to fatty acid biosynthesis and 3HP production (when a pathway to the latter via malonyl-CoA exists or is provided in a cell). This experiment is designed to determine how to control the use of malonyl-CoA pools in 3HP production strains and further improve the rate of 3HP production. It is hypothesized that by inhibiting fatty acid biosynthesis and regulating malonyl-CoA pools, flux through the pathway will be shifted toward 3HP production. A representative inhibitor has been selected to both interrupt fatty acid elongation and disrupt a futile cycle that recaptures the malonate moiety back to the acetyl-CoA pool. Production by strain KX3_0001 in fed-batch medium in the presence of 10 kg/m1 cerulenin is shown in the following table.

In the presence of the inhibitor, internal pools of the malonyl-CoA precursor are proposed to increase thus leading to increased production of 3-HP. As may be seen by comparison to the results without cerulenin (Example 5), substantially more 3-HP is produced at every time point, and the specific productivity at 24 h is 0.128 g 3-HP per gDCW, a 31-fold increase relative to the results without cerulenin.

**Table: Production of 3-HP by KX3_0001 in fed-batch medium and the presence of 10 kg/m1 cerulenin**

| | |
|---|---|
| 3HP (g/L) | OD600 |
| 0.002 | 0.118 |
| 0.002 | 0.724 |
| 0.020 | 1.59 |
| 0.060 | 2.80 |
| 0.090 | 3.45 |
| 0.200 | 4.73 |

### Example 7: Effect on 3-HP production of increased malonyl-CoA precursor pools using temperature-sensitive fatty acid synthesis mutant.

An alternative approach to increasing internal malonyl-CoA pools is to use genetic mutations rather than chemical inhibitors. While inactivating mutations in the genes encoding fatty acid synthesis functions are usually lethal and thus not obtainable, conditional mutants, such as temperature-sensitive mutants, have been described (de Mendoza, D., and Cronan, J. E., Jr. (1983) Trends Biochem. Sci., 8, 49-52). For example, a temperature-sensitive mutation in the fabl gene, encoding enoyl-ACP reductase, of strain JP1111 (genotype fab1392(ts)) has relatively normal activity at reduced temperature, such as 30C, and becomes non-permissive, likely through denaturation and inactivation, at elevated temperature, such that when cultured at 37 to 42C a microorganism only comprising this temperature-sensitive mutant as its enoyl-ACP reductase will produce substantially less fatty acids and phospholipids. This leads to decreased or no growth. However, it was hypothesized that when such mutant is provided in a genetically modified microorganism that also comprises a production pathway, such as to 3-HP, from malonyl-CoA, effective culture methods involving elevating culture temperature can result in increased 3-HP specific productivity.

Production of 3-HP by strain JX3_0077 in fed-batch medium at a constant temperature of 30°C and by a culture subjected to a temperature shift from 30°C to 42°C is shown in the following Table. The temperature shift is designed to inactivate the enoyl-ACP reductase, hence eliminating the accumulation of fatty acid which in turn increases the internal malonyl-CoA pool. Substantially more 3-HP is produced at every time point, and the specific productivity at 24 h by the temperature-shifted culture is 1.15 g 3-HP per gDCW, a greater than 100-fold increase over the specific productivity of 0.011 g 3-HP per gDCW by the culture maintained constantly at 30°C. This increased productivity of 3-HP by the culture in which the enoyl-ACP reductase is inactivated by elevated temperature supports the view that shifting of malonyl-CoA utilization leads to increased 3-HP production.

**Table: Production of 3-HP by JX3_0077 in fed-batch medium**

| | Constant 30°C | | Shifted to 42°C | |
|---|---|---|---|---|
| Time (hr) | 3HP (g/L) | OD600 | 3HP (g/L) | OD600 |
| 0 | 0 | 0.065 | 0.0007 | 0.068 |
| 3 | 0.003 | 0.273 | 0.004 | 0.25 |
| 4 | 0.010 | 0.409 | 0.037 | 0.79 |
| 6 | 0.030 | 1.09 | 0.096 | 0.91 |
| 8 | 0.016 | 1.81 | 0.193 | 0.81 |
| 24 | 0.014 | 3.8 | 0.331 | 0.87 |

The following Table shows the 3-HP production by strain JX3_0087 which carried a plasmid overexpressing the transhydrogenase gene in addition to a plasmid carrying the mcr gene. In the culture maintained at a constant temperature of 30°C, a specific productivity of 0.085 g 3-HP per gDCW in 24 h was attained. This is significantly higher than the specific productivity of JX3_0077 which does not carry the overexpressed transhydrogenase gene (above table). The specific productivity of the temperature-shifted culture of JX3_0087 was 1.68 g 3-HP per gDCW, a 20-fold increase over the specific productivity of the culture maintained constantly at 30°C in which the enoyl-ACP reductase was not inactivated.

**Table: Production of 3-HP by JX3_0087 in fed-batch medium**

| | Constant 30°C | | Shifted to 42°C | |
|---|---|---|---|---|
| Time (hr) | 3HP (g/L) | OD600 | 3HP (g/L) | OD600 |
| 0 | 0 | 0.008 | 0 | 0.004 |
| 3 | 0.0007 | 0.008 | 0.0007 | 0.011 |
| 4 | 0 | 0.04 | 0.002 | 0.063 |
| 6 | 0.0007 | 0.05 | 0.009 | 0.193 |
| 8 | 0.003 | 0.157 | 0.050 | 0.257 |
| 24 | 0.003 | 0.107 | 0.455 | 0.820 |

The following Table shows the 3-HP production by strain JX3_0097 which carried a plasmid overexpressing genes encoding the acetyl-CoA carboxylase complex in addition to a plasmid carrying the mcr gene. In the culture maintained at a constant temperature of 30°C, a specific productivity of 0.0068 g 3-HP per gDCW in 24 h was attained. This specific productivity is similar to that attained by strain JX3_0077 in which acetyl-CoA carboxylase is not overexpressed. The specific productivity of the temperature-shifted culture of JX3_0097 was 0.29 g 3-HP per gDCW, a 42-fold increase over the specific productivity of the culture maintained constantly at 30°C in which the enoyl-ACP reductase was not inactivated.

**Table 13: Production of 3-HP by JX3_0097 in fed-batch medium**

| | Constant 30°C* | | Shifted to 42°C* | |
|---|---|---|---|---|
| Time (hr) | 3HP (g/L) | OD600 | 3HP (g/L) | OD600 |
| 0 | | 0.016 | 0 | 0.014 |
| 4 | 0.004 | 0.3 | 0.004 | 0.31 |
| 5 | | 0.36 | 0.006 | 0.59 |
| 6 | | 0.65 | 0.062 | 1.51 |
| 8 | 0.006 | 1.46 | 0.178 | 1.91 |
| 24 | 0.006 | 2.66 | 0.176 | 1.87 |

Fed-batch medium, a rich medium, may contain components that serve as fatty acid precursors and thus may reduce the demand for malonyl-CoA. Thus the production of 3-HP by the strains derived from JP1111 in AM2, a minimal medium was verified. As shown in Table 14, 3-HP was produced by JX3_0077 in AM2 medium. A specific productivity of 0.024 g 3-HP per gDCW in 24 h was obtained by the culture maintained constantly at 30°C, approximately twice the value obtained in fed-batch medium. The temperature-shifted culture attained a specific productivity of 1.04 g 3-HP per gDCW over 24 h, a 44-fold increase compared to the specific productivity of the culture maintained constantly at 30°C, again indicating that conditional inactivation of the enoyl-ACP reductase increased the internal malonyl-CoA pool and hence increased the 3-HP production, as envisioned by the inventors.

**Table 14. Production of 3-HP by JX3_0077 in AM2 medium**

| | Constant 30°C | | Shifted to 42°C | |
|---|---|---|---|---|
| Time (hr) | 3HP (g/L) | OD600 | 3HP (g/L) | OD600 |
| 0 | 0 | 0.066 | 0 | 0.063 |
| 4 | 0.002 | 0.360 | 0.002 | 0.40 |
| 5 | 0.004 | 0.253 | 0.015 | 0.39 |
| 6 | 0.004 | 0.413 | 0.1 | 0.68 |
| 8 | 0.005 | 0.476 | 0.2 | 0.71 |
| 24 | 0.008 | 1.03 | 0.25 | 0.73 |

Production of 3-HP in AM2 medium by strain JX3_0087, which carried a plasmid overexpressing the transhydrogenase gene in addition to a plasmid carrying the mcr gene, is shown in Table 15. In the JX3_0087 culture maintained at a constant temperature of 30°C, a specific productivity of 0.018 g 3-HP per gDCW in 24 h was attained. In contrast to results obtained in fed-batch medium, this value is not higher than the specific productivity obtained in AM2 with strain JX3_0077 which does not carry the overexpressed transhydrogenase gene (Table 14). The specific productivity of the temperature-shifted culture of JX3_0087 was 0.50 g 3-HP per gDCW, a 27-fold increase over the specific productivity of the culture maintained constantly at 30°C in which the enoyl-ACP reductase was not inactivated.

**Table 15. Production of 3-HP by JX3_0087 in AM2**

| | Constant 30°C | | Shifted to 42°C | |
|---|---|---|---|---|
| Time (hr) | 3HP (g/L) | OD600 | 3HP (g/L) | OD600 |
| 0 | 0 | 0.08 | 0 | 0.086 |
| 4 | 0.002 | 0.363 | 0.002 | 0.380 |
| 5 | 0.002 | 0.273 | 0.011 | 0.360 |
| 6 | 0.003 | 0.297 | 0.050 | 0.520 |
| 8 | 0.005 | 0.467 | 0.100 | 0.607 |
| 24 | 0.006 | 1.0 | 0.112 | 0.683 |

Table 16 shows the 3-HP production in AM2 medium by strain JX3_0097 which carried a plasmid overexpressing genes encoding the acetyl-CoA carboxylase complex in addition to a plasmid carrying the mcr gene. In the culture maintained at a constant temperature of 30°C, a specific productivity of 0.021 g 3-HP per gDCW in 24 h was attained. This specific productivity is similar to that attained by strain JX3_0077 in which acetyl-CoA carboxylase is not overexpressed. The specific productivity of the temperature-shifted culture of JX3_0097 was 0.94 g 3-HP per gDCW in 24 h, a 45-fold increase over the specific productivity of the culture maintained constantly at 30°C in which the enoyl-ACP reductase was not inactivated.

**Table 16. Production of 3-HP by JX3_0097.0 in AM2**

| | Constant 30°C | | Shifted to 42°C | |
|---|---|---|---|---|
| Time (hr) | 3HP (g/L) | OD600 | 3HP (g/L) | OD600 |
| 0 | 0 | 0.085 | 0.001 | 0.085 |
| 4 | 0.002 | 0.500 | 0.003 | 0.483 |
| 5 | 0.003 | 0.287 | 0.015 | 0.473 |
| 6 | 0.005 | 0.417 | 0.073 | 0.510 |
| 8 | 0.005 | 0.520 | 0.198 | 0.590 |
| 24 | 0.013 | 1.91 | 0.192 | 0.620 |

The effect of combining the plasmids expressing mcr (malonyl-CoA reductase), pntAB (transhydrogenase), and accABCD (acetyl-CoA carboxylase complex) in the same organism was tested by constructing strain JX3_0098. The Table below shows the production of 3-HP by this strain in AM2 medium. A specific productivity of 0.54 g 3-HP per gDCW in 24 h was obtained in the culture maintained constantly at 30°C, representing a >20-fold increase over strains carrying mcr alone or mcr with either pntAB or accABCD, but not both. Shifting the temperature to inactivate enoyl-ACP reductase resulted in a specific productivity of 2.01 g 3-HP per gDCW in 24 h, a further 3.8-fold increase. Thus the combination of overexpression of pntAB and of accABCD, plus the inactivation of enoyl-ACP reductase via the temperature-sensitive fabr' allele, resulted in an approximately 500-fold increase in specific productivity of 3-HP by mcr-bearing cells (specific productivity of 2.01 vs. 0.0041 g 3-HP per gDCW in 24 h).

**Table 17. Production of 3-HP by JX3_0098.0 in AM2 medium**

| | Constant 30°C | | Shifted to 42°C | |
|---|---|---|---|---|
| Time (hr) | 3HP (g/L) | OD600 | 3HP (g/L) | OD600 |
| 0 | 0.007 | 0.117 | 0 | 0.13 |
| 4 | 0.013 | 0.303 | 0.017 | 0.47 |
| 5 | 0.017 | 0.600 | 0.060 | 0.75 |
| 6 | 0.033 | 0.730 | 0.107 | 0.87 |
| 8 | 0.053 | 0.9107 | 0.263 | 0.81 |
| 24 | 0.670 | 3.790 | 0.577 | 0.81 |

### Example 8: Sequence of the fabits mutation

The nature of the exact sequence change in the fabr' allele carried by strains JP1111 was reconfirmed. Confirmation of this change allows targeted mutagenesis to generate alternative strains with different temperature sensitivities and mutants with stabilities intermediate between wild type and the fabI392 temperature-sensitive allele, allowing growth at a constant temperature higher than 30°C while providing the benefit of increased internal malonyl-CoA pools. To confirm the DNA sequence of this segment of the chromosome of a wild type (BW25113) and the JP1111 mutant E. coli, chromosomal DNA was prepared from these strains. These DNA were used as templates in a PCR reaction with primers:
FWO43 ATGGGTTTTCTTTCCGG SEQ ID NO:78
FWO47 TTATTTCAGTTCGAGTTCG SEQ ID NO:79

Thermocyler conditions for the PCR were: 95°C, 10 min; 30 cycles of 95°C, 10s; 47°C increasing to 58°C, 30s; 72°C, 1 min; followed by a final incubation at 72°C for 5 min. The PCR product was separated on an agarose gel and the appropriate sized fragment recovered as described in the Common Methods Section, and sequenced using primers:
FWO44 CTATCCATCGCCTACGGTATC SEQ ID NO:80
FWO45 CGTTGCAATGGCAAAAGC SEQ ID NO:81
FWO46 CGGCGGTTTCAGCATTGC SEQ ID NO:82

A comparison of the DNA sequence obtained from the fabI392 (SEQ ID NO:28) and wild type strains reveals a single difference between the alleles of C at position 722 of the wild type gene to T, leading to a protein change of Ser at codon 241 to Phe.

The identification of the affected residue at codon 241 indicates that targeted mutagenesis at this codon, for example to amino acid residues such as Trp, Tyr, His, Ile, or other amino acids other than Ser or Phe, may result in fah./ alleles with different properties than the fabI392 originally isolated in JP1111. Targeted mutagenesis at codons near to codon 241 may also be contemplated to obtain the desired fah./ mutants with altered properties.

### Example 9: Effect on volumetric 3-HP production in 1L fermentations, of increased malonyl-CoA precursor pools using temperature sensitive fatty acid synthesis mutants.

Four 1 L fed batch fermentation experiments were carried out using the strain JX3_0098. Briefly, seed cultures were started and grown overnight in LB media (Luria Broth) and used to inoculate four 1 L New Brunswick fermentation vessels. The first vessel contained defined AM2 medium at 30°C, IPTG induction was added at 2mM at an OD600nm of 2, additional glucose feed was initiated when glucose was depleted to between 1-2 g/L. The temperature was shifted 37°C over 1 hr at target OD of 10. A high glucose feed rate was maintained at > 3g/L/hr until glucose began to accumulate at concentrations greater than 1g/L at which time feed rate was varied to maintain residual glucose between 1 and 10g/L. The second vessel contained defined AM2 medium at 30°C, IPTG induction was added at 2mM at an OD600nm of 2, additional glucose feed was initiated when glucose was depleted to 0 g/L. The temperature was shifted 37°C over 1 hr at target OD of 10. The glucose feed rate was maintained less than or equal to 3g/L/hr. The third vessel contained rich medium at 30°C, IPTG induction was added at 2mM at an OD600nm of 2, additional glucose feed was initiated when glucose was depleted to 1-2 g/L. The temperature was shifted 37°C over 1 hr at target OD of 10. A high glucose feed rate was maintained at > 3g/L/hr until glucose began to accumulate at concentrations greater than 1g/L at which time feed rate was varied to maintain residual glucose between 1 and 10g/L. The fourth vessel contained rich medium at 30°C, IPTG induction was added at 2mM at an OD600nm of 2, additional glucose feed was initiated when glucose was depleted to 0 g/L. The temperature was shifted 37°C over 1 hr at target OD of 10. The glucose feed rate was maintained less than or equal to 3g/L/hr.

All fermentation vessels were maintained at pH = 7.4 by the controlled addition of 50% v/v ammonium hydroxide (Fisher Scientific). All vessels were maintained at least 20% dissolved oxygen by aeration with sparged filtered air. Samples were taken for optical density measurements as well as HPLC analysis for 3-HP concentration. (Refer to common methods). Maximum volumetric productivities reached 2.99 g/L/hr. In addition, the figures demonstrate the correlation between the 3-4 hour average biomass concentration and 3-4 hr average volumetric productivity rates in these 4 vessels.

### Example 10A: Production of 3-HP in 250 liter fermentations

Examples of two fed batch fermentations in a 250 liter volume stainless steel fermentor were carried out using the strain BX3_0240, the genotype of which is described elsewhere herein. A two stage seed process was used to generate inoculum for the 250 L fermentor. In the first stage, one ml of glycerol stock of the strain was inoculated into 100 ml of TB medium (Terrific Broth) in a shake flask and incubated at 30°C until the OD600 was between 3 and 4. In the second stage, 85 ml of the shake flask culture was aseptically transferred to a 14 L New Brunswick fermentor containing 8 L of TB medium and grown at 30°C and 500 rpm agitation until the OD600 was between 5 and 6. The culture from the 14 L fermentor was used to aseptically inoculate the 250 L volume bioreactor containing defined FM5 medium (see Common Methods Section) at 30°C so that the post-inoculation volume was 155 L.

In the first fermentation, induction was effected by adding IPTG to a final concentration of 2 mM at an OD600 of 20. Glucose feed (consisting of a 700 g/L glucose solution) was initiated when the residual glucose in the fermentor was 10-15 g/L. The feed rate was adjusted to maintain the residual glucose between 10 and 15 g/L until about the last 6 hours of the fermentation when the feed rate was reduced so that the residual glucose at harvest was <1 g/L to facilitate 3-HP recovery. Three hours after induction, the temperature was shifted to 37°C over 1 hour. At the time the temperature shift was initiated, the dissolved oxygen (DO) set point was changed from 20% of air saturation to a point where the DO was maintained between 2-4% of air saturation. The fermentation broth was harvested 48 hours after inoculation. The final broth volume was 169.5 liters.

The second fermentation was run identically to the first example fermentation described above except for the following differences: induction with IPTG was effected at an OD₆₀₀ of 15, the residual glucose (after the glucose feed was started) ranged between 3-30 g/L, and the fermentation broth was harvested at 38.5 hours after inoculation so that the final residual glucose concentration was 25 g/L. The final broth volume was 167 liters.

Each fermentation broth was maintained at a pH of approximately 7.4 by the controlled addition of anhydrous ammonia gas. Dissolved oxygen was maintained at the desired levels by aeration with sparged, sterile-filtered air. Samples were taken for optical density measurements as well as HPLC analysis for 3-HP concentration. In the first fermentation, the maximum biomass concentration was 12.0 g dry cell weight/L and the biomass concentration at harvest was 11.4 g dry cell weight/L. The maximum 3-HP titer in this fermentation was 20.7 g/L. In the second fermentation, the maximum biomass concentration was 10.2 g dry cell weight/L and the biomass concentration at harvest was 9.5 g dry cell weight/L. The maximum 3-HP titer in this fermentation was 20.7 g/L.

### Example 10B: Effect of growth medium on 3-HP production in 1 L fermentations

Eight 1 L fed batch fermentation experiments were carried out using the strain BX3_0240. Seed culture was started from 1 ml of glycerol stock of the strain inoculated into 400 ml of TB medium (Terrific Broth) in a shake flask and incubated at 30°C until the OD600 was between 5 and 6. The shake flask culture was used to aseptically inoculate each 1 L volume bioreactor so that the post-inoculation volume was 653 ml in each vessel.

Fermentors 1 and 2 contained defined FM3 medium. Fermentors 3-5 contained defined FM4 medium. Fermentors 6-8 contained defined FM5 medium. All media formulations are listed in the Common Methods Section. In each fermentor, the initial temperature was 30°C.

Induction was effected by adding IPTG to a final concentration of 2 mM at OD600 values of 15-16. Glucose feed (consisting of a 500 g/L glucose solution for FM3 and FM5 media and 500 g/L glucose plus 75 mM MgS04 for FM4) was initiated when the residual glucose in the fermentor was about 10 g/L. The feed rate was adjusted to maintain the residual glucose >3 g/L (the exception was fermentor 8 in which the residual glucose temporarily reached 0.1 g/L before the feed rate was increased). Three hours after induction, the temperature was shifted to 37°C over 1 hour. At the time the temperature shift was initiated, the dissolved oxygen (DO) set point was changed from 20% of air saturation to 1% of air saturation. The fermentations were stopped 48 hours after inoculation.

The broth of each fermentor was maintained at a pH of approximately 7.4 by the controlled addition of a pH titrant. The pH titrant for FM3 medium was 5 M NaOH and for FM4 and FM5 it was a 50:50 mixture of concentrated ammonium hydroxide and water. Dissolved oxygen was maintained at the desired levels by sparging with sterile-filtered air. Samples were taken for optical density measurements as well as HPLC analysis for 3-HP concentration. The maximum biomass concentration and the biomass concentration at harvest as well as the maximum 3-HP titer in each fermentor are summarized in the Table 18 below.

**Table 18**

| **Fermentor No.** | **Growth Medium** | **Maximum Biomass Conc. (g DCW/L)** | **Biomass Conc. at Harvest (g DCW/L)** | **Maximum 3HP Titer (g/L)** |
|---|---|---|---|---|
| 1 | FM3 | 8.7 | 8.7 | 12.3 |
| 2 | FM3 | 9.6 | 9.5 | 16.7 |
| 3 | FM4 | 10.9 | 10.9 | 20.7 |
| 4 | FM4 | 11.5 | 11.5 | 18.3 |
| 5 | FM4 | 11.3 | 11.3 | 22.1 |
| 6 | FM5 | 11.3 | 11.3 | 35.2 |
| 7 | FM5 | 11.2 | 11.0 | 34.0 |
| 8 | FM5 | 11.6 | 10.6 | 31.2 |

### Example 10C: Effect of batch phosphate concentration on 3-HP production in 1 L fermentations.

Four 1 L fed batch fermentation experiments were carried out using the strain BX3_0240. Seed culture was started from 1 ml of glycerol stock of the strain inoculated into 400 ml of TB medium (Terrific Broth) in a shake flask and incubated at 30°C until the OD600 was between 5 and 7. The shake flask culture was used to aseptically inoculate each 1 L volume bioreactor so that the post-inoculation volume was 653 ml in each vessel.

All fermentors contained defined FM5 growth medium, but each had different initial concentrations of monobasic and dibasic potassium phosphate. The phosphate concentrations in the batch medium in each fermentor are summarized in the Table 19.

**Table 19**

| **Fermentor No.** | **K2HPO4 conc. in batch medium (g/L)** | **KH2PO4 conc. in batch medium (g/L)** |
|---|---|---|
| 1 | 6.1 | 1.92 |
| 2 | 2.63 | 1.38 |
| 3 | 0.87 | 0.14 |
| 4 | 0.043 | 0.070 |

In each fermentor, the initial temperature was 30°C. Induction was effected by adding IPTG to a final concentration of 2 mM when the OD600 values were at the following values: fermentor 1, 15.3; fermentor 2, 16.0; fermentor 3, 18.1; fermentor 4, 18.4. Glucose feed (consisting of a 500 g/L glucose solution for FM3 and FM5 media and 500 g/L glucose plus 75 mM MgS04 for FM4) was initiated when the residual glucose in the fermentor was about 10 g/L. The feed rate was adjusted to maintain the residual glucose >6.5 g/L. Three hours after induction, the temperature was shifted to 37°C over 1 hour. At the time the temperature shift was initiated, the dissolved oxygen (DO) set point was changed from 20% of air saturation to 1% of air saturation. The fermentations were stopped 48 hours after inoculation.

The broth of each fermentor was maintained at a pH of 7.4 by the controlled addition of a 50:50 mixture of concentrated ammonium hydroxide and water. Dissolved oxygen was maintained at the desired levels by sparging with sterile-filtered air. Samples were taken for optical density measurements as well as HPLC analysis for 3-HP concentration. The maximum biomass concentration and the biomass concentration at harvest as well as the maximum 3-HP titer in each fermentor are summarized in the Table 20 below.

**Table 20**

| **Fermentor No.** | **Maximum Biomass Conc. (g DCW/L)** | **Biomass Conc. at Harvest (g DCW/L)** | **Maximum 3HP Titer (g/L)** |
|---|---|---|---|
| 1 | 9.6 | 8.4 | 23.7 |
| 2 | 11.3 | 11.3 | 27.8 |
| 3 | 14.8 | 12.9 | 39.8 |
| 4 | 12.3 | 10.9 | 44.1 |

### Example 10D: 3-HP production in 1 L fermentations

Two 1 L fed batch fermentation experiments were carried out using the strain BX3_0240. Seed culture was started from 1 mL of glycerol stock of the strain inoculated into 100 mL of TB medium (Terrific Broth) in a shake flask and incubated at 30°C until the OD600 was between 5 and 6. The shake flask culture was used to aseptically inoculate (5% volume/volume) each 1 L volume bioreactor so that the post-inoculation volume was 800 mL in each vessel. The fermentors used in this experiment were Das Gip fed-batch pro parallel fermentation system (DASGIP AG, Julich, Germany, model SR07000DLS). The fermentation system included real-time monitoring and control of dissolved oxygen (%D0), pH, temperature, agitation, and feeding. Fermentors 1 and 2 contained defined FM5 medium, made as shown in the Common Methods Section except that Citric Acid was added at 2.0 g/L and MgS04 was added at 0.40 g/L. In each fermentor, the initial temperature was 30°C. Induction was effected by adding IPTG to a final concentration of 2 mM at OD600 values of 17-19, which corresponded to a time post-inoculation of 14.5 hr. Glucose feed (consisting of a 500 g/L glucose solution) was initiated when the residual glucose in the fermentor was about 1 g/L. The feed rate was adjusted to maintain the residual glucose >3 g/L. Three hours after induction, the temperature was shifted to 37°C over 1 hour. At the time the temperature shift was initiated, the OTR was set to 40mmol/L-hr by setting airflow and agitation to 1.08 vvm and 1000 rpm respectively. Compressed air at 2 bar was used as the air feed. The broth of each fermentor was maintained at a pH of approximately 7.4 by the controlled addition of a pH titrant. Two hours subsequent to IPTG induction, the pH titrant was changed from 50% NH₄(OH) to 7.4 M NaOH . Samples were taken for optical density measurements as well as HPLC analysis for 3-HP concentration. The maximum biomass concentration and the biomass concentration at harvest as well as the maximum 3-HP titer in each fermentor are summarized in the Table 21 below.

**Table 21**

| **Fermentor No.** | **Maximum Biomass Conc. (g DCW/L)** | **Biomass Conc. at Harvest (g DCW/L)** | **Total 3-HP (g) at 69hrs** | **Yield of 3-HP At (g3-HP/g glucose)** |
|---|---|---|---|---|
| 1 | 10.5 | 8.7 | 49.0 | 0.46 |
| 2 | 10.5 | 8.7 | 47.8 | 0.46 |

The following Table 22 provides a summary of concentrations of metabolic products obtained in the fermentation broth at the indicated time in hours.

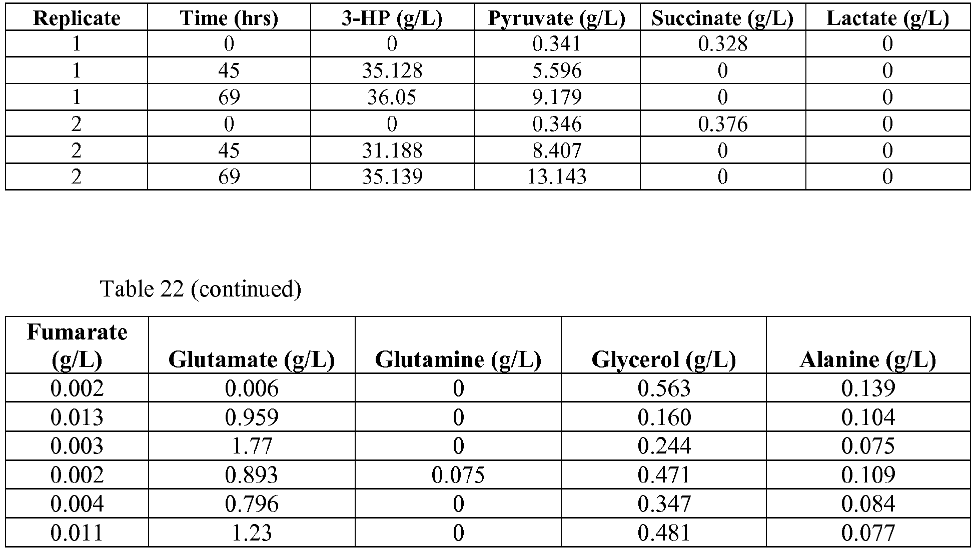

### Example 10E: 3-HP production in 1 L fermentations

Four 1 L fed batch fermentation experiments were carried out using the strain BX3_0240. Seed culture was started from 1 ml of glycerol stock of the strain inoculated into 100 mL of TB medium (Terrific Broth) in a shake flask and incubated at 30°C until the OD₆₀₀ was between 5 and 6. The shake flask culture was used to aseptically inoculate (5% volume/volume) each 1 L volume bioreactor so that the post-inoculation volume was 800 ml in each vessel. The fermentors used in this experiment were Das Gip fed-batch pro parallel fermentation system (DASGIP AG, Julich, Germany, model SR07000DLS). The fermentation system included real-time monitoring and control of dissolved oxygen (%D0), pH, temperature, agitation, and feeding. All fermentors contained defined FM5 medium, made as shown in the Common Methods Section except that Citric Acid was added at 2.0 g/L and MgS04 was added at 0.40 g/L. In each fermentor, the initial temperature was 30°C. Induction was effected by adding IPTG to a final concentration of 2 mM at OD600 values of 15-19, which corresponded to a time post-inoculation of 15.75 hr. Glucose feed (consisting of a 500 g/L glucose solution) was initiated when the residual glucose in the fermentor was about 3 g/L. The feed rate was adjusted to maintain the residual glucose >3 g/L. Three hours after induction, the temperature was shifted to 37°C over 1 hour.The broth of each fermentor was maintained at a pH of approximately 7.4 by the controlled addition of a pH titrant 50% NH4(OH). At the time the temperature shift was initiated, the OTR was changed for each fermentor by varying the agitation and airflow according to Table 23. Compressed air at 2 bar was used as the air feed) Samples were taken for optical density measurements as well as HPLC analysis for 3-HP concentration. The maximum biomass concentration and the biomass concentration at harvest as well as the maximum 3-HP titer in each fermentor are summarized in the Table 23 below.

**Table 23**

| **Fermentor No.** | **Airflow (vvm)** | **Agitation during Production (rpm)** | **Biomass Conc. at Harvest (g DCW/L)** | **3HP Titer (g/L) at 37 hrs** |
|---|---|---|---|---|
| 1 | 1.08 | 1000 | 8.6 | 14.9 |
| 2 | 1.08 | 800 | 9.0 | 7.9 |
| 3 | 1.08 | 600 | 8.2 | 0.5 |
| 4 | 1.08 | 400 | 5.9 | 0.5 |

### Example 10F: 3-HP production in 1.8 L fermentation

A 1.8 L fed batch fermentation experiment was carried out using the strain BX3_0240. Seed culture was started from 1 ml of glycerol stock of the strain inoculated into 105 ml of TB medium (Terrific Broth) in a shake flask and incubated at 30°C until the OD600 was between 5 and 7.90 ml of the shake flask culture was used to aseptically inoculate 1.71 L of FM5 growth medium, except that the phosphate concentrations were 0.33 g/L K2HPO4 and 0.17 g/L KH2PO4 in batch medium. The other ingredients in the FM5 media formulation are as listed in the Common Methods Section. The initial temperature in the fermentor was 30°C. Induction was effected by adding IPTG to a final concentration of 2 mM when the OD600 value was at 15.46. Glucose feed (consisting of a 500 g/L glucose solution) was initiated when the residual glucose in the fermentor was about 10 g/L. The feed rate was adjusted to maintain the residual glucose >6.5 g/L. Three hours after induction, the temperature was shifted to 37°C over 1 hour. At the time the temperature shift was initiated, the dissolved oxygen (DO) set point was changed from 20% of air saturation to 1% of air saturation. The broth of each fermentor was maintained at a pH of 7.4 by the controlled addition of a 50:50 mixture of concentrated ammonium hydroxide and water. Dissolved oxygen was maintained at the desired levels by sparging with sterile-filtered air. Samples were taken for optical density measurements as well as HPLC analysis for 3-HP concentration. The maximum final biomass concentration was 9.84 g/L, the maximum 3-HP titer was 48.4g/L with a final yield from glucose of 0.53g 3-HP/ g glucose.

### Example 11

### Part 1: Strain Construction for Further Evaluations of 3-HP Production

According to the respective combinations indicated in Table 24 below, the plasmids described herein were introduced into the respective strains. All plasmids were introduced at the same time via electroporation using standard methods. Transformed cells were grown on the appropriate media with antibiotic supplementation and colonies were selected based on their appropriate growth on the selective media. As summarized in Table 24, the mcr expression plasmids pTrc-ptrc-mcr or pACYC(kan)-ptalA-mcr were transformed into two strains derived from E. coli BW25113 (F-, A(araD-araB)567, AlacZ4787(::rrnB-3), lamba-, rph-1, A(rhaD-rhaB)568, hsdR514), these strains comprising additional chromosomal modifications introduced using Gene Bridges technology as described in the Common Methods Section. Strain BX_0590 comprises additional deletions of the 1dhA, pflB, mgsA, and poxB genes. Strain BX_0591 comprises the additional deletions of Strain BX_0590 and an additional deletion of the ack_pta genes. Transformants were subsequently selected for on media containing the appropriate combination of antibiotics.

**Table 24**

| **Strain name** | **Host** | **Plasmids** |
|---|---|---|
| BX3_0194 | BX_0590 | PTrc-ptrc-mcr |
| BX3_0195 | BX_0591 | PTrc-ptrc-mcr |
| BX3_0206 | BX_0590 | pACYC(kan)-ptalA-mcr |

### Example 11A: Construction of Additional Strains for Evaluation

### Part 1: Gene Deletions

The homologous recombination method using Red/ET recombination, as described elsewhere herein, was employed for gene deletion in E. coli strains. This method is known to those of ordinary skill in the art and described in U.S. Patent Nos. 6,355,412 and 6,509,156, issued to Stewart et al. Material and kits for such method are available from Gene Bridges (Gene Bridges GmbH, Heidelberg (formerly Dresden), Germany, <<www.genebridges.com>>), and the method proceeded by following the manufacturer's instructions. The method replaces the target gene by a selectable marker via homologous recombination performed by the recombinase from X-phage. The host organism expressing k-red recombinase is transformed with a linear DNA product coding for a selectable marker flanked by the terminal regions (generally -50 bp, and alternatively up to about -300 bp) homologous with the target gene or promoter sequence. The marker is thereafter removed by another recombination step performed by a plasmid vector carrying the FLP-recombinase, or another recombinase, such as Cre.

Specific deletions were constructed by amplification using PCR from the Keio strains carrying particular deletions using primers as specified below. The Keio collection was obtained from Open Biosystems (Huntsville, AL USA 35806). Individual clones may be purchased from the Yale Genetic Stock Center (New Haven, CT USA 06520). These strains each contain a kanamycin marker in place of the deleted gene. In cases where the desired deletion was not in a Keio strain, for example ackA-pta, the deletion was constructed by the above-noted
recombination method using the kanamycin resistance marker to replace the deleted sequence, followed by selection of a kanamycin resistance clone having the deletion. The PCR products were introduced into targeted strains using the above-noted recombination method. Combinations of deletions were generated sequentially to obtain strains as described in the following parts of this example.

**Table 25**

| **Plasmid template** | **Keio Clone Number** | **Gene Deletion** | **Forward Primer SEQ ID NO:** | **Reverse Primer SEQ ID NO:** |
|---|---|---|---|---|
| | JW1375 | ldhA | | |
| | JWO886 | pflB | 86 | 99 |
| | JW5129 | mgsA | 87 | 100 |
| | JWO855 | poxB | 88 | 101 |
| | JW2880 | serA | 89 | 102 |
| | JW4364 | arcA | 90 | 103 |
| | JW4356 | trpR | 91 | 104 |
| | JW3561 | aldB | 92 | 105 |
| | JW1412 | aldA | 93 | 106 |
| | JW1293 | puuC | 94 | 107 |
| | JW2755 | relA | 95 | 108 |
| pKD4 | | spoT | 96 | 109 |
| pKD4 | | ackA-pta | 97 | 110 |
| | JW1228 | adhE | 98 | 111 |

### Part 2: Construction of strains BW_595 and BW_651 having a fabl mutation

The fads mutation (Ser241->Phe) in E. coli strain JP1111 significantly increases the malonyl-CoA concentration when cells are grown at the nonpermissive temperature (37°C) and thus produces more 3-HP at this temperature. However, JP1111 is not an ideal strain for transitioning into pilot and commercial scale, since it is the product of NTG mutagenesis and thus may harbor unknown mutations, carries mutations in the stringency regulatory factors relA and spoT, and has enhanced conjugation propensity due to the presence of an Hfr factor. Thus the fads mutation was moved into strain BX_591, a strain developed from the well-characterized BW23115 carrying the additional mutations AldhA, ApflB, AmgsA, ApoxB, Apta-ack. These mutations were generated by the sequential application of the gene deletion method described in Part 1 above. The fads gene with 600 by of upstream and downstream DNA sequence was isolated from JP1111 genomic DNA by PCR using primers:
SEQ ID NO: 112 FWO56: 5'-CCAGTGGGGAGCTACATTCTC; and
SEQ ID NO: 113 FWO57: 5'-CGTCATTCAGATGCTGGCGCGATC.

The FRT::kan::FRT cassette was then inserted at a Smal site downstream of the fads to generate plasmid pSMART(HC)amp_fabr_FRT::kan::FRT. This plasmid was used as template DNA and the region between primers:
SEQ ID NO: 114 FWO43: 5'- ATGGGTTTTCTTTCCGG and
FWO57 (SEQ ID NO: 113)
was amplified in a PCR using KOD HS DNA polymerase (Novagen). The reaction was treated with Dpnl to fragment the plasmid template and the amplification fragment was gel-purified and recovered using the DNA Clean and Concentrator kit (Zymo Research, Orange, CA). Strain BX_591 was transformed with pSIM5 (Datta, S., et al., Gene 379:109-115, 2006) and expression of the lambda red genes carried on this plasmid were induced by incubation at 42°C for 15 min.

Electrocompetent cells were made by standard methods. These cells were transformed with the amplification fragment bearing the fabr_FRT::kan::FRT cassette and transformant colonies isolated on LB plates containing 35 jig/mlkanamycin at 30°C. Individual colonies were purified by restreaking, and tested for temperature sensitivity by growth in liquid medium at 30°C and 42°C. Compared to wildtype parental strain, the strain bearing the fabI allele grows poorly at 42°C but exhibited comparable growth at 30°C. Correct insertion of the FRT::kan::FRT marker was verified by colony PCR, and the fabr kanR strain was designated BX_594.

To allow use of the kanR marker on plasmids, the marker incorporated in the chromosome adjacent to fabr was replaced with a DNA fragment encoding resistance to zeocin. The zeoR gene was amplified by PCR from plasmid pJ402 (DNA 2.0, Menlo Park, CA) using primers:
SEQ ID NO: 115 HL018:
5'-CAGGTTTGCGGCGTCCAGCGGTTATGTAACTACTATTCGGC GCGACTTACGCCGCTCCCCGCTCGCGATAATGTGGTAGC; and
SEQ ID NO: 116 HL019:

The reaction was treated with Dpnl and gel-purified as above. Strain BX_594 was transformed with pKD46 (Datsenko and Wanner, Proc. Natl. Acad. Sci. USA 96: 6640-6645, 2000) and the lambda red genes carried on this plasmid were induced by the addition of L-arabinose to 1 mM for 2 hr. Electrocompetent cells were made by standard methods (e.g, Sambrook and Russell, 2001). These cells were transformed with the zeoR fragment and transformants selected for on LB plates formulated without NaCl and with 25 jig/m1 zeocin. Plates were kept in the dark by wrapping in aluminum foil, and incubated at 30°C. A zeocin-resistant, kanamycin-sensitive strain isolated by this method was designated BX_595. Retention of the fablts allele was confirmed by growth as above.

Strain BX_651 was constructed by transferring the fabr -zeoR cassette from BX_595 to strain BW25113 which does not carry mutations in metabolic genes. A DNA fragment carrying this cassette was obtained by PCR using BX_595 chromosomal DNA and primers FWO43 (see above) and SEQ ID NO:117 FW65: 5'-GAGATAAGCCTGAAATGTCGC. The PCR product was purified and concentrated using the DNA Clean and Concentrator kit (Zymo Research, Orange, CA). Strain BW25113 was transformed with pRedD/ET (Gene Bridges GmBH, Heidelberg, Germany) and the lambda red genes carried on this plasmid were induced by the addition of L-arabinose to 5 mM for 2 hr. Electrocompetent cells were made by standard methods, and transformed with the fabr-zeoR DNA fragment. Transformants were plated as above on zeocin, and clones bearing the temperature-sensitive allele verified by growth at 30°C and 42°C as described above.

### Part 3: Promoter Replacement for Selected Genes in Chromosome

The homologous recombination method described elsewhere herein was employed to replace promoters of various genes. As noted, use of Red/ET recombination is known to those of ordinary skill in the art and described in U.S. Patent Nos. 6,355,412 and 6,509,156, issued to Stewart et al. Material and kits for such method are available from Gene Bridges (Gene Bridges GmbH, Heidelberg, Germany, <<www.genebridges.com>>), and the method may proceed by following the manufacturer's instructions. The method involves replacement of the target gene (or, in this case, a promoter region) by a selectable marker via homologous recombination performed by the recombinase from X-phage. The host organism expressing X-red recombinase is transformed with a linear DNA product coding for a selectable marker flanked by the terminal regions (generally -50 bp, and alternatively up to about -300 bp) homologous with the target gene or promoter sequence. The marker can then be removed by another recombination step performed by a plasmid vector carrying the FLP-recombinase, or another recombinase, such as Cre. This method was used according to manufacturer's instructions. Template sequences, each comprising end sequences to achieve the recombination to replace a native promoter for the indicated gene of interest, the desired replacement promoter, and an antibiotic marker sequence, were synthesized by an outside manufacturer (Integrated DNA Technologies, Coralville, IA). These sequences are designed to replace the native promoter in front of these genes with a T5 promoter. The T5-aceEF cassette (SEQ ID NO:120) also includes a zeocin resistance cassette flanked by loxP sites. The T5-pntAB (SEQ ID NO:121), T5-udhA (SEQ ID NO:122) and T5-cynTS (SEQ ID NO:123) cassettes each include a blasticidin resistance cassette flanked by loxP sites. Also, T5-cynTS (SEQ ID NO:123) comprises modified loxP sites in accordance with Lambert et al., AEM 73(4) p1126-1135.

Each cassette first is used as a template for PCR amplification to generate a PCR product using the primers CAGTCCAGTTACGCTGGAGTC (SEQ ID NO:118), and ACTGACCATTTAAATCATACCTGACC (SEQ ID NO:119). This PCR product is used for electroporation (using standard methods such as described elsewhere herein) and recombination into the genome following the Red/ET recombination method of Gene Bridges described above. After transformation positive recombinants are selected on media containing zeocin or blasticidin antibiotics. Curing of the resistance marker is accomplished by expression of the Cre-recombinase according to standard methods. Table 27 shows strains having genotypes that comprise replaced promoters. These are shown as "T5" followed by the affected gene(s).

### Part 4: Construction of Plasmids

The following table summarizes the construction of plasmids that were used in strains described below. To make the plasmids, a respective gene or gene region of interest was isolated by either PCR amplification and restriction enzyme (RE) digestion or direct restriction enzyme digestion of an appropriate source carrying the gene. The isolated gene was then ligated into the desired vector, transformed into E. coli 10G (Lucigen, Middleton, WI) competent cells, screened by restriction mapping and confirmed by DNA sequencing using standard molecular biology procedures (e.g., Sambrook and Russell, 2001).

It is noted that among these plasmids are those that comprise mono-functional malonyl-CoA reductase activity. Particularly, truncated portions of malonyl-CoA reductase from C. aurantiacus were constructed by use of PCR primers adjacent, respectively, to nucleotide bases encoding amino acid residues 366 and 1220, and 496 and 1220, of the codon-optimized malonyl-CoA reductase from pTRC-ptrc-mcr-amp. Also, a malonyl¬CoA reductase from Erythrobacter sp. was incorporated into another plasmid. As for other plasmids, these were incorporated into strains and evaluated as described below.

**Table 26**

| **Gene(s) or Region Name** | **Vector and *Supplier** | **Catalog Number** | **Cloning Method/Gene(s) Source** | **Plasmid Name** | **Plasmid SEQ ID NO:** |
|---|---|---|---|---|---|
| *Erythrobacter sp* MCR | pTRCHisA *A | V360-20 | RE (Ncol/BgIII) / pUC *57-Eb* mcr (SEQ ID NO:905) | pTrc-ptrc-Ebmcr-amp | 128 |
| Truncated C. *aurantiacus mcr* (366-1220) | pTRCHisA *A | V360-20 | PCR, RE (Ncol/HindIII)/ pTRC-ptrc mcr-amp | pTrc-ptrc-(366-1220)mcr-ptrc-ydfG-kan | 129 |
| Truncated C. *aurantiacus mcr* (496-1220) | pTRCHisA *A | V360-20 | PCR, RE (Ncol/HindIII)/ pTRC-ptrc mcr-amp | pTrc-ptrc-ydfG-ptrc-(496-1220)mcr-amp | 130 |
| Mcr | pTRCHisA *A | V360-20 | PCR, RE (Ncol/HindIII)/ SEQ ID No. 003 | pTrc-ptrc-mcr-amp | 131 |
| Mcr | pTRCHisA *A | V360-20 | RE (Ahdl, blunted) for Kan insertion/pTRC-ptrc mcr-amp | pTrc-ptrc-mcr-kan | 3 |
| mcr/cynTS | pTRCHisA *A | V360-20 | RE/ (Ndel, blunted: pTRC ptrc-mcr ) kan, (EcoRV: pSMARTHC am pcynTS) | pTrc-ptrc-mcr-kan-cynTS | 132 |
| AccABCD | pJ251 *C | N/A | RE (EcoNI, Asel, blunted) for Cat insertion/ SEQ ID No 820 | pJ251-cat-PtpiA-accAD-PrpiA-accBC | 2 |
| PntAB | pACYC 184 cat *B | E4152S | RE (Nrul, Pcil, blunted) self-ligate/ pACYC1 84-cat-PtpiA-accAD-PrpiA-accBCptalA-pntAB | pACYC1 84-cat-PtalA-pntAB | 133 |
| acccABCD/pntAB | pACYC 184 cat *B | E41525 | RE/( EcoRV,Aval, BseB1, blunted: pACYC184), (BamBl, blunted: pJ244-pntAB-accABCD) | pACYC184-cat-PtpiA-accAD-PrpiA-accBC-ptalA-pntAB | 4 |
| accABCD/udhA | pACYC 184 cat *B | E4152S | RE (Swal, Apal)/ pJ244-pTal-udhA | pACYC1 84-cat-PtpiA-accAD-PrpiA-accBC-ptaIA-udhA | 5 |
| accABCD/T5- | pACYC 184 | E4152S | RE (Swal, Ndel)/ | pACYC 184- | 134 |
| udhA | cat *B | | pACYC184-cat-PtpiA-accAD-PrpiA-accBC PCR, RE (Pmel, Ndel)/ BX 00635 | cat-PtpiA-accADPrpiA-accBC-T5-udhA | |
| mcr/serA | pTRCHisA *A | V360-20 | RE (Pcil, blunted) for pTpiA serA insertion/ SEQ ID No. 0047 | pTrc-ptrc-mcr-kan-PtpiA-serA | 135 |
| FabF | pTRCHisA *A | V360-20 | PCR, RE (Ncol/Pstl)/ *E.coli* K12 genome | pTrc-ptrc-fabF-amp | 136 |
| Mcr | pACYC177 kan *B | E41515 | PCR (blunt)/ pTRC-ptrc mcr-amp | pACYC177-kan-ptrc-mcr | 137 |
| mcr/accABCD | pACYC177 kan *B | E41515 | RE / (Swal, Xbal: pACYC 177 kan ptrc-mcr), (Pmel. Xbal: pJ251-cat-PtpiA-accAD-PrpiA-accBC | pACYC177-kan-ptrc-mcr-ptPIA accAD-PrpiA-accBC | 138 |

| | | | | | |
|---|---|---|---|---|---|
| *A: Invitrogen, Carlsbad, CA *B: New England Biolabs, Ipswich, MA *C: DNA 2.0, Menlo Park | | | | | |

### Part 5: Cloning of pACYC-cat-accABCD-PT5-udhA.

The Pfau promoter driving expression of udhA in pACYC-cat-accABCD-udhA was replaced with the stronger T5 promoter. The genomic PT5-udhA construct from strain BX_00635 - was amplified using primer AS1170 (udhA 300 by upstream). See SEQ ID NO:139 for sequence of udhA). PCR fragments of PT5-udhA obtained above were digested with PmeI and NdeI (New England BioLabs, Ipswich, MA). Vector pACYC-cat¬accABCD-P,rudhA was similarly digested with SwaI and NdeI (New England BioLabs). The two digested DNA fragments were ligated and transformed to create pACYC-cat-accABCD-PT5-udhA (SEQ ID NO:140). Plasmid digests were used to confirm the correct sequence. This plasmid is incorporated into strains shown in Table 27.

### Part 6: Strain Construction

Using constructs made by the above methods, strains shown in Table 27, given the indicated Strain Names, were produced providing the genotypes. This is not meant to be limiting, and other strains may be made using these methods and following the teachings provided in this application, including providing different genes and gene regions for tolerance, and/or 3-HP production and modifications to modulate the fatty acid synthase system. Further to the latter, such strains may be produced by chromosomal modifications and/or introduction of non-chromosomal introductions, such as plasmids.

As to the latter, according to the respective combinations indicated in Table 27 below, the plasmids described above were introduced into the respective strains. All plasmids were introduced at the same time via electroporation using standard methods. Transformed cells were grown on the appropriate media with antibiotic supplementation and colonies were selected based on their appropriate growth on the selective media.

**Table 27**

| **Strain Name** | **Strain Genotype** |
|---|---|
| BW25113 | F-, A(araD-araB)567, AlacZ4787(::rrnB-3), LAM-, rph-1, A(rhaD-rhaB)568, hsdR514 |
| BX 0591 - | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt |
| 6X_0595 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR |
| BX_0619 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB¬BSD |
| BX_0634 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF |
| BX_0635 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA-BSD |
| 6X_0636 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-aceEF |
| BX_0637 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-aceEF, T5-udhA-BSD |
| BX_0638 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, AaldB::frt |
| BX_0639 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, AtrpR::kan |
| BX 0651 - | F-, A(araD-araB)567, AlacZ4787(::rrnB-3), LAM-, rph-1, A(rhaD-rhaB)568, hsdR514, fablts (S241F)-zeoR |
| BX_0652 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA, AarcA::kan |
| BX_0653 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA, ApuuC::kan |
| BX_0654 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA, AaldA::kan |

### Example 11B: Preparing a Genetically Modified E. coli Host Cell Comprising malonyl-CoA-reductase (Mcr) in Combination with Other Genetic Modifications to Increase 3-HP Production Relative to a Control E. coli Cell

Genetic modifications are made to introduce a vector comprising mmsB such as from Pseudomonas auruginos, which further is codon-optimized for E. coli. Vectors comprising galP and a native or mutated ppc also may be introduced by methods known to those skilled in the art (see, e.g., Sambrook and Russell, Molecular Cloning: A Laboratory Manual, Third Edition 2001 (volumes 1-3), Cold Spring Harbor Laboratory Press, Cold Spring Harbor, N.Y., "Sambrook and Russell, 2001"), additionally recognizing that mutations may be made by a method using the XL1-Red mutator strain, using appropriate materials following a manufacturer's instructions (Stratagene QuikChange Mutagenesis Kit, Stratagene, La Jolla, CA USA) and selected for or screened under standard protocols.

Also, genetic modifications are made to reduce or eliminate the enzymatic activities of E.coli genes as desired. These genetic modifications are achieved by using the RED/ET homologous recombination method with kits supplied by Gene Bridges (Gene Bridges GmbH, Dresden, Germany, <<www.genebridges.com>>) according to manufacturer's instructions.

Also, in some embodiments genetic modifications are made to increase the NADPH cellular pool. Non-limiting examples of some targets for genetic modification are provided herein. These are pgi (in a mutated form), pntAB, overexpressed, gapA:gapN substitution/replacement, and disrupting or modifying a soluble transhydrogenase such as sthA, and genetic modifications of one or more of zwf, gnd, and edd.

The so-genetically modified microorganism of any such engineered embodiment is evaluated and found to exhibit higher productivity of 3-HP compared with a control E. coli lacking said genetic modifications. Productivity is measured by standard metrics, such as volumetric productivity (grams of 3-HP/hour) under similar culture conditions.

### Example 11C: Mutational Development of Selected Polynucleotides

A selected gene sequence, such as a nucleic acid sequence that encodes for any of SEQ ID NOs:15 and 42-49, is subjected to a mutation development protocol, starting by constructing a mutant library of a native or previously evolved and/or codon-optimized polynucleotide by use of an error-inducing PCR site-directed mutagenesis method.

A polynucleotide exhibiting enzymatic activity of the selected gene (which may be any disclosed herein, e.g., an aminotransferase or mmsB) is cloned into an appropriate expression system for E. coli. This sequence may be codon optimized Cloning of a codon-optimized polynucleotide and its adequate expression will be accomplished via gene synthesis supplied from a commercial supplier using standard techniques. The gene will be synthesized with an eight amino acid C-terminal tag to enable affinity based protein purification. Once obtained using standard methodology, the gene will be cloned into an expression system using standard techniques.

The plasmid containing the above-described polynucleotide will be mutated by standard methods resulting in a large library of mutants (>106). The mutant sequences will be excised from these plasmids and again cloned into an expression vector, generating a final library of greater than 106 clones for subsequent screening. These numbers ensure a greater than 99% probability that the library will contain a mutation in every amino acid encoded by sequence. It is acknowledged that each method of creating a mutational library has its own biases, including transformation into mutator strains of E. coli, error prone PCR, and in addition more site directed muagenesis.

In some embodiments, various methods may be considered and possibly several explored in parallel. One such method is the use of the XL1-Red mutator strain, which is deficient in several repair mechanisms necessary for accurate DNA replication and generates mutations in plasmids at a rate 5,000 times that of the wild-type mutation rate, may be employed using appropriate materials following a manufacturer's instructions (See Stratagene QuikChange Mutagenesis Kit, Stratagene, La Jolla, CA USA). This technique or other techniques known to those skilled in the art, may be employed and then a population of such mutants, e.g., in a library, is evaluated, such as by a screening or selection method, to identify clones having a suitable or favorable mutation.

With the successful construction of a mutant library, it will be possible to screen this library for increased activity, such as increased malonyl-CoA reductase activity. The screening process will be designed to screen the entire library of greater than 106 mutants. This is done by screening methods suited to the particular enzymatic reaction.

### Example 12A: Evaluation of 3-HP Production

3-HP production by BX3_0194 was demonstrated at 100-mL scale in SM3 (minimal salts) media. Cultures were started from freezer stocks by standard practice (Sambrook and Russell, 2001) into 50 mL of LB media plus 100 µg/mL ampicillin and grown to stationary phase overnight at 37°C with rotation at 225 rpm. Five ml of this culture were transferred to 100 ml of SM3 media plus 40 g/L glucose, 100 kg/ml ampicillin, and 1 mM IPTG in triplicate 250-ml baffled flasks and incubated at 37°C, 225 rpm. To monitor cell growth and 3-HP production by these cultures, samples (2 ml) were withdrawn at designated time points for optical density measurements at 600nm (OD600, 1 cm pathlength) and pelleted by centrifugation at 12,000rpm for 5 min and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Under these conditions, no 3HP is produced after 24 hours in a culture growing to an OD600 that corresponds to approximately 1.0 g DCW. Production of 3-HP by strain BX3_0194 in SM3 medium is shown in Table 28.

**Table 28. Production of 3-HP by BX3_0194 in SM3 medium**

| **Time (hr)** | **3HP (gIL)** | **OD600** |
|---|---|---|
| 4 | 0 | 1.3 |
| 6 | 0 | 2.3 |
| 8 | 0 | 2.8 |
| 24 | 0 | 3.4 |

Production by strain BX3_0194 in SM3 medium in the presence of 10.1,g/ml cerulenin is shown in Table 29. In the presence of cerulenin, an inhibitor of the fatty acid synthase system, internal pools of the malonyl-CoA precursor are proposed to increase thus leading to increased production of 3-HP. As may be seen by comparison to the results without cerulenin (Table 28), substantially more 3-HP is produced at every time point. Under these conditions, the specific productivity after 24 hours is 1.3 g 3HP per gDCW.

**Table 29. Production of 3-HP by BX3_0194 in SM3 medium and the presence of 10 .1,g/ml cerulenin**

| **Time (hr)** | **3HP (gIL)** | **OD600** |
|---|---|---|
| 4 | 0.003 | 1.3 |
| 6 | 0.14 | 2.6 |
| 8 | 0.43 | 3.1 |
| 24 | 1.43 | 3.3 |

3-HP production by BX3_0195 was demonstrated at 100-mL scale in SM3 (minimal salts) media. Cultures were started from freezer stocks by standard practice (Sambrook and Russell, 2001) into 50 mL of LB media plus 100 µg/mL ampicillin and grown to stationary phase overnight at 37°C with rotation at 225 rpm. Five ml of this culture were transferred to 100 ml of SM3 media plus 40 g/L glucose, 100 .1,g/ml ampicillin, and 1 mM IPTG in triplicate 250-ml baffled flasks and incubated at 37°C, 225 rpm. To monitor cell growth and 3-HP production by these cultures, samples (2 ml) were withdrawn at designated time points for optical density measurements at 600nm (OD600, 1 cm pathlength) and pelleted by centrifugation at 12,000rpm for 5 mM and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Under these conditions, no 3HP is produced after 24 hours in a culture growing to and OD 600 that corresponds to approximately 1.65 g DCW. Production of 3-HP by strain BX3_0195 in SM3 medium is shown in Table 30.

**Table 30. Production of 3-HP by BX3_0195 in SM3 medium**

| **Time (hr)** | **3HP (gIL)** | **OD600** |
|---|---|---|
| 4 | 0 | 0.92 |
| 6 | 0 | 1.35 |
| 8 | 0 | 2.36 |
| 24 | 0 | 5.00 |

Production by strain BX3_0195 in SM3 medium in the presence of 10 u.g/mlcerulenin is shown in Table 31. In the presence of cerulenin, an inhibitor of the fatty acid synthase system, internal pools of the malonyl-CoA precursor are proposed to increase thus leading to increased production of 3-HP. As may be seen by comparison to the results without cerulenin (Table 30), substantially more 3-HP is produced at every time point. Under these conditions, the specific productivity after 24 hours is 0.54 g 3HP per gDCW.

**Table 31. Production of 3-HP by BX3_0195 in SM3 medium and the presence of 10 .1,g/ml cerulenin**

| **Time (hr)** | **3HP (gIL)** | **OD600** |
|---|---|---|
| 4 | 0.003 | 0.97 |
| 6 | 0.07 | 1.57 |
| 8 | 0.31 | 2.36 |
| 24 | 1.17 | 6.59 |

3-HP production by BX3_0206 was demonstrated at 100-mL scale in SM3 (minimal salts) media. Cultures were started from freezer stocks by standard practice (Sambrook and Russell, 2001) into 50 mL of LB media plus 35 µg/mL kanamycin and grown to stationary phase overnight at 37°C with rotation at 225 rpm. Five ml of this culture were transferred to 100 ml of SM3 media plus 40 g/L glucose and 35 .1,g/ml kanamycin in triplicate 250-ml baffled flasks and incubated at 37°C, 225 rpm. To monitor cell growth and 3-HP production by these cultures, samples (2 ml) were withdrawn at designated time points for optical density measurements at 600nm (OD600, 1 cm pathlength) and pelleted by centrifugation at 12,000rpm for 5 min and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Under these conditions, the specific productivity after 24 hours is 0.05 g 3HP per gDCW. Production of 3-HP by strain BX3_0206 in SM3 medium is shown in Table 32.

**Table 32. Production of 3-HP by BX3_0206 in SM3 medium**

| **Time (hr)** | **3HP (gIL)** | **OD600** |
|---|---|---|
| 24 | 0.01 | 6.5 |

Production by strain BX3_0206 in SM3 medium in the presence of 10 .1,g/m1 cerulenin is shown in Table 33. In the presence of cerulenin, an inhibitor of the fatty acid synthase system internal pools of the malonyl-CoA precursor are proposed to increase thus leading to increased production of 3-HP. As may be seen by comparison to the results without cerulenin (Table 32), substantially more 3-HP is produced after 24 hours. Under these conditions, the specific productivity after 24 hours is 0.20 g 3HP per gDCW, an approximately 40-fold increase relative to the results without cerulenin.

**Table 33. Production of 3-HP by BX3_0195 in SM3 medium and the presence of 10 ug/ml cerulenin**

| **Time (hr)** | **3HP (gIL)** | **OD600** |
|---|---|---|
| 24 | 0.43 | 6.4 |

### Example 12B: Evaluation of Strains for 3-HP Production

3-HP production in biocatalysts (strains) listed in the following table was demonstrated at 100-mL scale in SM3 (minimal salts) media. SM3 used is described under the Common Methods Section, but was supplemented with 200 mM MOPS. Cultures were started from LB plates containing antibiotics by standard practice (Sambrook and Russell, 2001) into 50 mL of TB media plus the appropriate antibiotic as indicated and grown to stationary phase overnight at 30°C with rotation at 250 rpm. Five ml of this culture were transferred to 100 ml of SM3 media plus 30 g/L glucose, antibiotic, and 1 mM IPTG (identified as "yes" under the "Induced" column) in triplicate 250-ml baffled flasks and incubated at 30°C, 250 rpm. Flasks were shifted to 37°C, 250 rpm after 4 hours. To monitor cell growth and 3-HP production by these cultures, samples (2 ml) were withdrawn at 24 hours for optical density measurements at 600nm (0D600, 1 cm pathlength) and pelleted by centrifugation at 14000 rpm for 5 mins and the supernatant collected for analysis of 3-HP production. 3-HP titer and standard deviation is expressed as g/L. Dry cell weight (DCW) is calculated as 0.33 times the measured OD600 value, based on baseline DCW per OD600 determinations. All data are the average of triplicate cultures. For comparison purposes, product to cell ratio is calculated from the averaged data over 24 hours and is expressed as g 3-HP produced per gDCW. The specific productivity is calculated from the cell/product ratio obtained over the 20 hours of production and expressed as g 3-HP produced per gDCW per hour.

**Table 34**

| **Strain Name** | **Strain Host** | **Plasmids** | **Induced** | **Average 24 Hour Titer** | **Standard Deviation** | **20 Hour Specific Productivity** | **24 Hour Product/Cell Ratio** |
|---|---|---|---|---|---|---|---|
| BX3_0274 | BW25113 | 1) pTrc-ptrc-mcr-kan | yes | <0.001 | 0.000 | <0.001 | <0.001 |
| BX3_0282 | BW25113 | 1)pTrc-ptrc-mcr-kan 2)pJ251-cat-PtpiA-accAD-PrpiA-accBC | yes | <0.001 | 0.000 | <0.001 | <0.001 |
| BX3_0283 | BW25113 | 1)pTrc-ptrc-mcr-kan 2)pACYC18 4-cat-Pta1A-pntAB | yes | <0.001 | 0.000 | <0.001 | <0.001 |
| BX3_0275 | BW25113 | 1)pTrc-ptrc-mcr-kan 2)pACYC18 4-cat¬PtpiA-accAD-PrpiA¬accB C-ptalA-pntAB | yes | <0.001 | 0.000 | <0.001 | <0.001 |
| BX3_0284 | BW25113 | 1)pTrc-ptrc-mcr-kan 2)pACYC18 4-cat¬PtpiA-accAD-PrpiA¬accB C-ptalA-udhA | yes | <0.001 | 0.000 | <0.001 | <0.001 |
| BX3_0285 | BX_00591 | 1) pTrc-ptrc-mcr-kan | yes | <0.001 | 0.000 | <0.001 | <0.001 |
| BX3_0286 | BX_00591 | 1)pTrc-ptrc-mcr-kan 2) pJ251-cat-PtpiA-accAD-PrpiA-accBC | yes | <0.001 | <0.001 | 0.000 | <0.001 |
| BX3_0287 | BX_00591 | 1)pTrc-ptrc-mcr-kan 2)pACYC18 4-cat-Pta1A-pntAB | yes | <0.001 | 0.000 | <0.001 | <0.001 |
| BX3_0288 | BX_00591 | 1)pTrc-ptrc-mcr-kan 2)pACYC18 4-cat¬PtpiA-accAD-PrpiA¬accB C-ptalA-pntAB | yes | <0.001 | 0.000 | <0.001 | <0.001 |
| BX3_0289 | BX_00591 | 1)pTrc-ptrc-mcr-kan 2)pACYC18 4-cat¬PtpiA-accAD-PrpiA¬accB C-ptalA-udhA | yes | <0.001 | 0.000 | <0.001 | <0.001 |
| BX3_0239 | BX_00595 | 1) pTrc-ptrc-mcr-kan | yes | 2.317 | 0.001 | 0.067 | 1.335 |
| BX3_0261 | BX_00595 | 1)pTrc-ptrc-mcr-kan 2)pJ251-cat-PtpiA | yes | 4.576 | 0.327 | 0.187 | 3.748 |
| | | accAD-PrpiA-accBC | | | | | |
| BX3_0290 | BX_00595 | 1) pIrc-ptrc-mcr-kan 2) pACYC1 84-cat-Pta1A-pntAB | yes | 1.706 | 0.396 | 0.060 | 1.194 |
| BX3_0240 | BX_00595 | 1) pIrc-ptrc-mcr-kan 2) pACYC1 84-cat¬PtpiA-accAD-PrpiA¬accB C-ptalA-pntAB | yes | 5.878 | 0.684 | 0.228 | 4.563 |
| BX3_0267 | BX_00595 | 1) pIrc-ptrc-mcr-kan, 2)pACYC18 4-cat-PtpiA-accAD-PrpiA-accBC-ptalA-udhA | yes | 3.440 | 0.205 | 0.160 | 2.912 |
| BX3_0253 | BX_00619 | 1) pIrc-ptrc-mcr-kan | yes | 1.327 | 0.575 | 0.034 | 0.670 |
| BX3_0254 | BX_00619 | 1) pIrc-ptrc-mcr-kan 2)pJ251-cat-PtpiA-accAD-PrpiA-accBC | yes | 3.131 | 0.058 | 0.136 | 2.711 |
| BX3_0263 | BX_00619 | 1)pIrc-ptrc-mcr-kan | | | | | |
| 2) pACYC 18 4-cat¬PtpiA-accAD-PrpiA¬acc BC-ptalA-pntAB | yes | 2.376 | 0.717 | 0.060 | 1.200 | | |
| BX3_0268 | BX_00619 | 1)pIrc-ptrc-mcr-kan 2) pACYC1 84-cat¬PtpiA-accAD-PrpiA¬accB C-ptalA-udhA | yes | 5.555 | 0.265 | 0.240 | 4.809 |
| BX3_0279 | BX_00637 | 1) pIrc-ptrc-mcr-kan 2) pJ251-cat-PtpiA-accAD-PrpiA-accBC | yes | 3.640 | 0.210 | 0.154 | 3.073 |
| BX3_0303 | BX_00637 | 1) pIrc-ptrc-mcr-kan 2) pACYC1 84-cat-Pta1A-pntAB | yes | 2.620 | 0.085 | 0.065 | 1.297 |
| BX3_0281 | BX_00637 | 1) pIrc-ptrc-mcr-kan 2) pACYC1 84-cat¬PtpiA-accAD-PrpiA¬accB C-ptalA-pntAB | yes | 4.700 | 0.271 | 0.209 | 4.177 |
| BX3_0280 | BX_00637 | 1) pIrc-ptrc-mcr-kan 2) pACYC1 84-cat¬PtpiA-accAD-PrpiA¬accB C-ptalA-udhA | yes | 4.270 | 0.314 | 0.175 | 3.507 |
| BX3_0276 | BX_00635 | 1) pIrc-ptrc-mcr-kan 2) pJ251-cat-PtpiA-accAD-PrpiA-accBC | yes | 5.110 | 0.542 | 0.210 | 4.196 |
| BX3_0304 | BX_00635 | 1) pIrc-ptrc-mcr-kan 2) pACYC1 84-cat-Pta1A-pntAB | yes | 2.430 | 0.147 | 0.076 | 1.512 |
| BX3_0278 | BX_00635 | 1) pIrc-ptrc-mcr-kan 2) pACYC1 84-cat¬PtpiA-accAD-PrpiA¬accB C-ptalA-pntAB | yes | 0.790 | 0.015 | 0.034 | 0.672 |
| BX3_0277 | BX_006 35 | 1) pIrc-ptrc-mcr-kan 2) pACYC1 84-cat¬PtpiA-accAD-PrpiA¬accB C-ptalA-udhA | yes | 6.340 | 0.580 | 0.260 | 5.207 |
| BX3_029 | BX_006 | 1) pIrc-ptrc-mcr-kan | yes | 3.400 | 0.139 | 0.102 | 2.032 |
| BX3_0297 | BX_006 BX 36 | 1) pIrc-ptrc-mcr-kan 2) pJ251-cat-PtpiA-accAD-PrpiA-accBC | yes | 1.830 | 0.144 | 0.069 | 1.376 |
| BX3_0298 | BX_006 BX 36 | 1) pIrc-ptrc-mcr-kan 2) pACYC1 84-cat-PtalA-pntAB | yes | 2.670 | 0.065 | 0.081 | 1.628 |
| BX3_0299 | BX_00636 | 1) pIrc-ptrc-mcr-kan 2) pACYC1 84-cat¬PtpiA-accAD-PrpiA-accBC-pta1A-pntAB | yes | 3.200 | 0.418 | 0.121 | 2.412 |
| BX3_0300 | BX_006 36 | 1) pIrc-ptrc-mcr-kan 2) pACYC1 84-cat¬PtpiA-accAD-PrpiA-accBC-ptalA-udhA | yes | 4.930 | 0.638 | 0.184 | 3.671 |
| BX3_0291 | BX_00634 | 1) pIrc-ptrc-mcr-kan | yes | 1.330 | 0.138 | 0.039 | 0.783 |
| BX3_0292 | BX_00634 | 1) pIrc-ptrc-mcr-kan 2) pJ251-cat-PtpiA-accAD-PrpiA-accBC | yes | 1.209 | 0.087 | 0.030 | 0.599 |
| BX3_0293 | BX_00634 | 1) pIrc-ptrc-mcr-kan 2) pACYC1 84-cat-PtalA-pntAB | yes | 0.269 | 0.035 | 0.006 | 0.124 |
| BX3_0294 | BX_00634 | 1) pIrc-ptrc-mcr-kan 2) pACYC1 84-cat¬PtpiA-accAD-PrpiA-accBC-pta1A-pntAB | yes | 1.588 | 0.136 | 0.046 | 0.927 |
| BX3_0295 | BX_00634 | 1) pIrc-ptrc-mcr-kan 2) pACYC1 84-cat¬PtpiA-accAD-PrpiA-accBC-ptalA-udhA | yes | 1.054 | 0.048 | 0.028 | 0.552 |
| BX3_0302 | BX_00637 | 1) pIrc-ptrc-mcr-kan | yes | 3.710 | 0.221 | 0.118 | 2.352 |
| BX3_0301 | BX_00635 | 1) pIrc-ptrc-mcr-kan | yes | 3.150 | 0.576 | 0.101 | 2.027 |
| BX3 0305 | BW25113 | 1) pIrc-ptrc-mcr-kan-cynTS 2) pACYC1 84-cat-PtpiA-accAD-PrpiA-accBC-ptalA-pntAB | yes | 0.006 | 0.006 | 0.000 | 0.003 |
| BX3 0306 | BX 00591 | 1) pIrc-ptrc-mcr-kan-cynTS 2) pACYC1 84-cat-PtpiA-accAD-PrpiA-accBC-ptalA-pntAB | yes | 0.035 | 0.035 | 0.001 | 0.014 |
| BX3_0258 | BX 005 9-5 | 1) pIrc-ptrc-mcr-kan-cynTS 2) pACYC1 84-cat-PtpiA-accAD-PrpiA-accBC-ptalA-pntAB | yes | 1.190 | 0.046 | 0.039 | 0.771 |
| BX3_0308 | BX_00634 | 1) pIrc-ptrc-mcr-kan-cynTS 2) pACYC1 84-cat-PtpiA-accAD-PrpiA accBC-ptalA-udhA | yes | 0.401 | 0.006 | 0.011 | 0.211 |
| BX3 0310 | BX_00637 | 1)pIrc-ptrc-mcr-kan-cynTS 2)pACYC18 4-cat-PtpiA-accAD-PrpiA-accBC-ptalA-udhA | yes | 1.450 | 0.072 | 0.045 | 0.897 |
| BX3 0309 | BX_00635 | 1) pIrc-ptrc-mcr-kan-cynTS 2) pACYC1 84-cat-PtpiA-accAD-PrpiA-accBC-ptalA-udhA | yes | 4.079 | 0.054 | 0.155 | 3.098 |
| BX3_0311 | BX_00638 | 1) pIrc-ptrc-mcr-kan 2) pACYC1 84-cat¬PtpiA-accAD-PrpiA¬accB C-ptalA-udhA | yes | 3.040 | 0.227 | 0.119 | 2.387 |
| BX3_0312 | BX_00639 | 1) pIrc-ptrc-mcr-amp 2) pACYC1 84-cat¬PtpiA-accAD-PrpiA¬accB C-ptalA-udhA | yes | 2.850 | 0.071 | 0.152 | 3.030 |
| BX3_0352 | BX_0651 | 1) pIrc-ptrc-mcr-kan | yes | <0.001 | 0.000 | <0.001 | NA |
| BX3_0353 | BX_0651 | 1) pIrc-ptrc-mcr-kan 2) pJ251-cat-PtpiA-accAD-PrpiA-accBC | yes | <0.001 | 0.000 | <0.001 | NA |
| BX3_0313 | BX_00635 | 1) pACYC 177-kan-ptrc-mcr | no | 0.037 | 0.009 | 0.001 | 0.027 |
| BX3_0313 | BX_00635 | 1) pACYC177-kan-ptrc-mcr | yes | 0.031 | 0.009 | 0.001 | 0.023 |
| BX3 0335 | BX BX_00635 | 1) pACYC177-kan- ptrc-mcr-PtpiA-accAD-PrpiA-accBC | no | 0.037 | 0.021 | 0.001 | 0.020 |
| BX3 0335 | BX_006 BX 35 | 1) pACYC177-kan- ptrc-mcr-PtpiA-accAD-PrpiA-accBC | yes | 0.037 | 0.021 | 0.001 | 0.020 |
| BX3_0349 | BX_00591 | 1) pIrc-ptrc-(366-1220)mcr-ptrc-ydfG-kan | yes | 0.057 | 0.006 | 0.001 | 0.025 |
| BX3_0350 | BX_00595 | 1) pIrc-ptrc-(366-1220)mcr-ptrc-ydfG-kan | yes | 1.163 | 0.045 | 0.023 | 0.457 |
| BX3_ 0351 | BX_00635 | 1) pTrc-ptrc-(366-1220)mcr-ptrc-ydfG-kan 2) pACYC1 84-cat-PtpiA-accAD-PrpiA-accBC-pta1A-udhA | yes | 0.658 | 0.060 | 0.020 | 0.390 |
| BX3_0358 | BX_00591 | 1) pIrc-ptrc-ydfG-ptrc-(496-1220)mcr-amp | yes | 0.040 | 0.000 | 0.001 | 0.015 |
| BX3_0360 | BX_00635 | 1) pTrc-ptrc-ydfG-ptrc-(496-1220)mcr-amp 2) pACYC184-cat- PtpiA-accAD-PrpiA-accBC-ptalA-udhA | yes | 4.027 | 0.185 | 0.138 | 2.761 |
| BX3_031 | BX_006 | 1) pIrc-ptrc-mcr-kan- | yes | 1.170 | 0.118 | 0.055 | 1.101 |
| 4 | 35 | PtpiA-serA 2) pACYC1 84-cat-PtpiA-accAD-PrpiA-accBC-ptalA-udhA | | | | | |
| BX3_0315 | BX_00591 | 1) pACYC177-kan-ptrc-mcr | no | 0.013 | 0.006 | 0.000 | 0.008 |
| BX3_0316 | BX_00595 | 1) pACYC177-kan-ptrc-mcr | no | 0.010 | 0.012 | 0.000 | 0.007 |
| BX3_0333 | BX_00591 | 1) pACYC177-kan-ptrc-mcr-PtpiA-accAD-PrpiA-accBC | no | 0.005 | 0.004 | 0.000 | 0.002 |
| BX3_0334 | BX_005 BX 9-5 | 1) pACYC177-kan- ptrc-mcr-PtpiA-accAD-PrpiA-accBC | no | 0.300 | 0.013 | 0.007 | 0.134 |
| BX3_0317 | BX_00591 | 1) pACYC177-kan-ptrc-mcr 2) pIrc-ptrc- fabF-amp | no | <0.001 | 0.000 | <0.2 | <0.2 |
| BX3_0317 | BX_00591 | 1) pACYC177-kan-ptrc-mcr 2) pIrc-ptrc-fabF-amp | yes | 0.033 | 0.024 | 0.001 | 0.021 |
| BX3_0338 | BX_00591 | 1) pACYC177-kan-ptrc-mcr-PtpiA-accAD¬Prpi A-accBC 2) pIrc-ptrc-fabF-amp | no | 0.010 | 0.005 | 0.000 | 0.004 |
| BX3_0338 | BX_00591 | 1) pACYC177-kan¬ptrc-mcr-PtpiA-accAD¬Prpi A-accBC 2) pIrc-ptrc-fabF-amp | yes | 1.580 | 0.142 | 0.006 | 0.116 |
| BX3_0318 | BX_00595 | 1) pACYC177-kan-ptrc-mcr 2) pIrc-ptrc-fabF-amp | no | 0.161 | 0.013 | 0.005 | 0.097 |
| BX3_0318 | BX_00595 | 1) pACYC177-kan-ptrc-mcr 2) pIrc-ptrc-fabF-amp | yes | 1.330 | 0.101 | 0.049 | 0.976 |
| BX3_0339 | BX_00595 | 1) pACYC177-kan-ptrc-mcr-PtpiA-accAD¬Prpi A-accBC 2) pIrc-ptrc-fabF-amp | no | 0.083 | 0.015 | 0.007 | 0.149 |
| BX3_0339 | BX_00595 | 1) pACYC177-kan-ptrc-mcr-PtpiA-accAD¬Prpi A-accBC 2) pIrc-ptrc-fabF-amp | yes | 0.010 | 0.009 | 0.000 | 0.007 |
| BX3_0319 | BX_00635 | 1) pACYC177-kan-ptrc-mcr 2) pIrc-ptrc-fabF-amp | no | 0.120 | 0.008 | 0.005 | 0.094 |
| BX3_0319 | BX 0063-5 | 1) pACYC177-kan- ptrc-mcr 2) pIrc-ptrc-fabF-amp | yes | 1.068 | 0.450 | 0.043 | 0.854 |
| BX3 0341 | BX_00635 | 1) pACYC177-kan¬ptrc-mcr-PtpiA-accAD¬Prpi A-accBC 2) pIrc-ptrc-fabF-amp | no | 0.327 | 0.021 | 0.009 | 0.171 |
| BX3_0341 | BX_00635 | 1) pACYC177-kan-ptrc-mcr-PtpiA-accAD PrpiA-accBC 2) pTrc-ptrc-fabF-amp | yes | 0.140 | 0.017 | 0.015 | 0.293 |
| BX3_0342 | BX_00635 | 1) pTrc-ptrc-mcr-kan 2) pACYC18 4-cat¬PtpiA-accAD-PrpiA¬acc BC-T5-udhA | yes | 0.341 | 0.055 | 0.009 | 0.188 |
| BX3 0343 | BX_00635 | 1) pTrc-ptrc-mcr-kan-cynTS 2) pACYC18 4-cat-PtpiA-accAD-PrpiA-accBC- T5-udhA | yes | 1.927 | 0.047 | 0.077 | 1.536 |
| BX3_0344 | BX_00652 | 1) pTrc-ptrc-mcr-amp | yes | 1.562 | 0.280 | 0.040 | 0.797 |
| BX3_0345 | BX_00652 | 1) pTrc-ptrc-mcr-amp 2) pJ251-cat-PtpiA-accAD-PrpiA-accBC | yes | 5.195 | 0.229 | 0.184 | 3.678 |
| BX3_0346 | BX_00652 | 1) pTrc-ptrc-mcr-amp 2) pACYC18 4-cat¬PtpiA-accAD-PrpiA¬acc BC-ptalA-udhA | yes | 1.781 | 0.132 | 0.056 | 1.119 |
| BX3_0347 | BX_00653 | 1) pTrc-ptrc-mcr-amp 2) pACYC18 4-cat¬PtpiA-accAD-PrpiA¬acc BC-ptalA-udhA | yes | 1.370 | 0.307 | 0.049 | 0.977 |
| BX3_0348 | BX_00654 | 1) pTrc-ptrc-mcr-amp 2) pACYC18 4-cat¬PtpiA-accAD-PrpiA¬acc BC-ptalA-udhA | yes | 1.387 | 0.184 | 0.049 | 0.982 |
| BX3_0324 | BX_00591 | 1) pTrc-ptrc-Ebmcr-amp | yes | 0.009 | 0.002 | 0.000 | 0.004 |
| BX3_0328 | BX_00595 | 1) pTrc-ptrc-Ebmcr-amp | yes | 0.011 | 0.005 | 0.000 | 0.006 |

### Example 12C: Evaluation of BX3_240 Strain with Carbonate Addition

3-HP production in E. coli BX3_240 (made by methods above) was evaluated at 100-mL scale in SM3 (minimal salts) media having added sodium carbonate. SM3 used is described under the Common Methods Section, to which was added 10mM, 20mM and 50mM Na2CO3 as treatments. Cultures were started from LB plates containing antibiotics by standard practice (Sambrook and Russell, 2001) into 50 mL of TB media plus the appropriate antibiotics kan and cat and grown to stationary phase overnight at 30°C with rotation at 250 rpm. Five ml of this culture were transferred to 100 ml of SM3 media plus 30 g/L glucose, antibiotic, the indicated sodium carbonate, 0.1% yeast extract and 1 mM IPTG in triplicate 250-ml baffled flasks and incubated at 30°C, 250 rpm. Flasks were shifted to 37°C, 250 rpm after 4 hours. To monitor cell growth and 3-HP production by these cultures, samples (2 ml) were withdrawn at 24, 48 and 60 hours for optical density measurements at 600nm (0D600, 1 cm path length) and pelleted by centrifugation at 14000 rpm for 5 min and the supernatant collected for analysis of 3-HP production as described under "Analysis of cultures for 3-HP production" in the Common Methods section. 3-HP titer and standard deviation is expressed as g/L. Dry cell weight (DCW) is calculated as 0.33 times the measured OD₆₀₀ value, based on baseline DCW per OD₆₀₀ determinations. All data are the average of triplicate cultures. For comparison purposes, product to cell ratio is calculated from the averaged data over 60 hours and is expressed as g 3-HP produced per gDCW.

3-HP titer were 0.32 (+/- 0.03), 0.87 (+/- 0.10), 2.24 (+/- 0.03), 4.15 (+/- 0.27), 6.24 (+/-0.51), 7.50 (+/- 0.55) and 8.03 (+/-0.14) g/L at 9, 11, 15, 19, 24, 48 and 60 hr, respectively. Biomass concentrations were 0.54 (+/- 0.02), 0.79 (+/- 0.03), 1.03 (+/- 0.06), 1.18 (+/- 0.04), 1.20 (+/- 0.12), 1.74 (+/- 0.30) and 1.84 (+/¬0.22) at 9, 11, 15, 19, 24, 48 and 60 hr, respectively. Maximum product to cell ratio was 4.6 g 3-HP/g DCW.

### Example 13A: General example of genetic modification to a host cell

In addition to the above specific examples, this example is meant to describe a non-limiting approach to genetic modification of a selected microorganism to introduce a nucleic acid sequence of interest. Alternatives and variations are provided within this general example. The methods of this example are conducted to achieve a combination of desired genetic modifications in a selected microorganism species, such as a combination of genetic modifications as described in sections herein, and their functional equivalents, such as in other bacterial and other microorganism species.

A gene or other nucleic acid sequence segment of interest is identified in a particular species (such as E. coli as described herein) and a nucleic acid sequence comprising that gene or segment is obtained.

Based on the nucleic acid sequences at the ends of or adjacent the ends of the segment of interest, 5' and 3' nucleic acid primers are prepared. Each primer is designed to have a sufficient overlap section that hybridizes with such ends or adjacent regions. Such primers may include enzyme recognition sites for restriction digest of transposase insertion that could be used for subsequent vector incorporation or genomic insertion. These sites are typically designed to be outward of the hybridizing overlap sections. Numerous contract services are known that prepare primer sequences to order (e.g., Integrated DNA Technologies, Coralville, IA USA).

Once primers are designed and prepared, polymerase chain reaction (PCR) is conducted to specifically amplify the desired segment of interest. This method results in multiple copies of the region of interest separated from the microorganism's genome. The microorganism's DNA, the primers, and a thermophilic polymerase are combined in a buffer solution with potassium and divalent cations (e.g., Mg or Mn) and with sufficient quantities of deoxynucleoside triphosphate molecules. This mixture is exposed to a standard regimen of temperature increases and decreases. However, temperatures, components, concentrations, and cycle times may vary according to the reaction according to length of the sequence to be copied, annealing temperature approximations and other factors known or readily learned through routine experimentation by one skilled in the art.

In an alternative embodiment the segment of interest may be synthesized, such as by a commercial vendor, and prepared via PCR, rather than obtaining from a microorganism or other natural source of DNA.

The nucleic acid sequences then are purified and separated, such as on an agarose gel via electrophoresis. Optionally, once the region is purified it can be validated by standard DNA sequencing methodology and may be introduced into a vector. Any of a number of vectors may be used, which generally comprise markers known to those skilled in the art, and standard methodologies are routinely employed for such introduction. Commonly used vector systems are pSMART (Lucigen, Middleton, WI), pET E. coli EXPRESSION SYSTEM (Stratagene, La Jolla, CA), pSC-B StrataClone Vector (Stratagene, La Jolla, CA), pRANGER-BTB vectors (Lucigen, Middleton, WI), and TOPO vector (Invitrogen Corp, Carlsbad, CA, USA). Similarly, the vector then is introduced into any of a number of host cells. Commonly used host cells are E. cloni 100 (Lucigen, Middleton, WI), E. cloni 10GF' (Lucigen, Middleton, WI), StrataClone Competent cells (Stratagene, La Jolla, CA), E. coli BL21, E. coli BW25113, and E. coli K12 MG1655. Some of these vectors possess promoters, such as inducible promoters, adjacent the region into which the sequence of interest is inserted (such as into a multiple cloning site), while other vectors, such as pSMART vectors (Lucigen, Middleton, WI), are provided without promoters and with dephosporylated blunt ends. The culturing of such plasmid-laden cells permits plasmid replication and thus replication of the segment of interest, which often corresponds to expression of the segment of interest.

Various vector systems comprise a selectable marker, such as an expressible gene encoding a protein needed for growth or survival under defined conditions. Common selectable markers contained on backbone vector sequences include genes that encode for one or more proteins required for antibiotic resistance as well as genes required to complement auxotrophic deficiencies or supply critical nutrients not present or available in a particular culture media. Vectors also comprise a replication system suitable for a host cell of interest.

The plasmids containing the segment of interest can then be isolated by routine methods and are available for introduction into other microorganism host cells of interest. Various methods of introduction are known in the art and can include vector introduction or genomic integration. In various alternative embodiments the DNA segment of interest may be separated from other plasmid DNA if the former will be introduced into a host cell of interest by means other than such plasmid.

While steps of the general example involve use of plasmids, other vectors known in the art may be used instead. These include cosmids, viruses (e.g., bacteriophage, animal viruses, plant viruses), and artificial chromosomes (e.g., yeast artificial chromosomes (YAC) and bacteria artificial chromosomes (BAC).

Host cells into which the segment of interest is introduced may be evaluated for performance as to a particular enzymatic step, and/or tolerance or bio-production of a chemical compound of interest. Selections of better performing genetically modified host cells may be made, selecting for overall performance, tolerance, or production or accumulation of the chemical of interest.

It is noted that this procedure may incorporate a nucleic acid sequence for a single gene (or other nucleic acid sequence segment of interest), or multiple genes (under control of separate promoters or a single promoter), and the procedure may be repeated to create the desired heterologous nucleic acid sequences in expression vectors, which are then supplied to a selected microorganism so as to have, for example, a desired complement of enzymatic conversion step functionality for any of the herein-disclosed metabolic pathways. However, it is noted that although many approaches rely on expression via transcription of all or part of the sequence of interest, and then translation of the transcribed mRNA to yield a polypeptide such as an enzyme, certain sequences of interest may exert an effect by means other than such expression. The specific laboratory methods used for these approaches are well-known in the art and may be found in various references known to those skilled in the art, such as Sambrook and Russell, Molecular Cloning: A Laboratory Manual, Third Edition 2001 (volumes 1-3), Cold Spring Harbor Laboratory Press, Cold Spring Harbor, N.Y. (hereinafter, Sambrook and Russell, 2001).

As an alternative to the above, other genetic modifications may also be practiced, such as a deletion of a nucleic acid sequence of the host cell's genome. One non-limiting method to achieve this is by use of Red/ET recombination, known to those of ordinary skill in the art and described in U.S. Patent Nos. 6,355,412 and 6,509,156, issued to Stewart et al. Material and kits for such method are available from Gene Bridges (Gene Bridges GmbH, Dresden, Germany, <<www.genebridges.com>>), and the method may proceed by following the manufacturer's instructions. Targeted deletion of genomic DNA may be practiced to alter a host cell's metabolism so as to reduce or eliminate production of undesired metabolic products. This may be used in combination with other genetic modifications such as described herein in this general example.

### Example 13B. Utilization of sucrose as the feedstock for production of 3-HP and other products

Common laboratory and industrial strains of E. coli, such as the strains described herein, are not capable of utilizing sucrose as the sole carbon source, although this property is found in a number of wild strains, including pathogenic E. coli strains. Sucrose, and sucrose-containing feedstocks such as molasses, are abundant and often used as feedstocks for the production by
microbial fermentation of organic acids, amino acids, vitamins, and other products. Thus further derivatives of the 3-HP-producing strains that are capable of utilizing sucrose would expand the range of feedstocks that can be utilized to produce 3-HP.

Various sucrose uptake and metabolism systems are known in the art (for example, U.S. Pat. No. 6,960,455). We describe the construction of E. coli strains that harbor the csc genes confering the ability to utilize sucrose via a non-phosphotransferase system, wherein the csc genes constitute cscA, encoding a sucrose hydrolase, cscB, encoding a sucrose permease, cscK, encoding a fructokinase, and cscR, encoding a repressor. The sequences of these genes are annotated in the NCBI database as accession No. X81461 AF473544. To allow efficient expression utilizing codons that are highly abundant in E. coli genes, an operon containing cscB, cscK, and cscA was designed and synthesized using the services of a commercial synthetic DNA provider (DNA 2.0, Menlo Park, CA). The amino acid sequences of the genes are set forth as, respectively, cscB - SEQ. ID. No. 141; cscA - SEQ. ID. No. 142; csck - SEQ. ID. No. 143. The synthetic operon consisted of 60 base pairs of the region of the E. coli genome immediately 5' (upstream) of the adhE gene, a consensus strong promoter to drive expression of the csc genes, the coding regions for cscB, cscK, and cscA with short intergenic regions containing ribosome binding sites but no promoters, and 60 by immediately 3' (downstream) of the adhE gene. The segments homologous to sequences flanking the adhE gene will be used to target insertion of the csc operon genes into the E. coli chromosome, with the concomittent deletion of adhE. The nucleotide sequence of the entire synthetic construct is shown as SEQ. ID. No. 144. The synthetic csc operon is constructed in plasmid pJ214 (DNA 2.0, Menlo Park, CA) that provides an origin of replication derived from plasmid pl5A and a gene conferring resistance to ampicillin. This plasmid is denoted pSUCR. A suitable host cell, such as E. coli strain BX_595, is transformed simultaneously with pSUCR and with plasmid pTrc_kan_mcr or other suitable plasmid, and transformed strains selected for on LB medium plates containing ampicillin and kanamycin. Transformants carrying both plasmids are grown and evaluated for 3-HP production in shake flasks as described, except that the glucose in SM3 medium is replaced with an equal concentration of sucrose.

Genes that confer functions to enable utilization of sucrose by E. coli can also be obtained from the natural isolate pUR400 (Cowan, P.J., et al. J. Bacteriol. 173:7464-7470, 1991) which carries genes for the phosphoenolpyruvate-dependent carbohydrate uptake phosphotransferase system (PTS). These genes consist of scrA, encoding the enzyme II component of the PTS transport complex, scrB, encoding sucrose-6 phosphate hydrolase, scrK, encoding fructokinase, and scrY, encoding a porin. These genes may be isolated or synthesized as described above, incorporated on a plasmid, and transformed into a suitable host cell, such as
E. coli strain BX_595, simultaneously with plasmid pTrc_kan_mcr or other suitable plasmid, and transformed strains selected for on LB medium plates containing the appropriate antibiotics. Transformants carrying both plasmids are grown and evaluated for 3-HP production in shake flasks as described, except that the glucose in SM3 medium is replaced with an equal concentration of sucrose.

### Example 13C: Construction and Evaluation of Additional Strains

Other strains are produced that comprise various combinations of the genetic elements (additions, deletions and modifications) described herein are evaluated for and used for 3-HP production, including commercial-scale production. The following table illustrates a number of these strains.

Additionally, a further deletion or other modification to reduce enzymatic activity, of multifunctional 2-keto-3-deoxygluconate 6-phosphate aldolase and 2-keto-4-hydroxyglutarate aldolase and oxaloacetate decarboxylase (eda in E. coli), may be provided to various strains. Further to the latter, in various embodiments combined with such reduction of enzymatic activity of multifunctional 2-keto-3-deoxygluconate 6-phosphate aldolase and 2-keto-4-hydroxyglutarate aldolase and oxaloacetate decarboxylase (eda in E. coli), further genetic modifications may be made to increase a glucose transporter (e.g. galP in E. coli) and/or to decrease activity of one or more of heat stable, histidyl phosphorylatable protein (of PTS) (ptsH (HPr) in E. coli), phosphoryl transfer protein (of PTS) (ptsl in E. coli), and the polypeptide chain of PTS (Crr in E. coli).

These strains are evaluated in either flasks, or fermentors, using the methods described above. Also, it is noted that after a given extent of evaluation of strains that comprise introduced plasmids, the genetic elements in the plasmids may be introduced into the microorganism genome, such as by methods described herein as well as other methods known to those skilled in the art.

**Table 35**

| **Strain** | **Host** | **Plasmids** |
|---|---|---|
| BX3P_001 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoR T5 aceEF, T5-pntAB, T5-udhA, fabB-tS | 1) ptrc-mcr |
| BX3P_002 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoR T5 aceEF, T5-pntAB, T5-udhA, fabB-tS | 1) ptrc-mcr, 2)accABCD |
| BX3P_003 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoR T5 aceEF, T5-pntAB, T5-udhA, | 1) ptrc-mcr, 2)accABCD-udhA |
| | fabB-tS | |
| BX3P-004 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, relA, spoT | 1) ptrc-mcr |
| BX3P-005 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta ack::frt, fablts (S241F)-zeoRT5 aceEF, T5-pntAB, T5-udhA, relA, spoT | 1) ptrc-mcr, 2)accABCD |
| BX3P_006 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, relA, spoT | 1) ptrc-mcr, 2)accABCD udhA |
| BX3P_007 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, del-arcA:kan | 1) ptrc-mcr |
| BX3P_008 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, del-arcA:kan | 1) ptrc-mcr, 2)accABCD |
| BX3P_009 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, del-arcA:kan | 1) ptrc-mcr, 2)accABCD-udhA |
| BX3P-010 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS | 1) ptrc-mcr |
| BX3P-011 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS | 1) ptrc-mcr, 2)accABCD |
| BX3P-012 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta ack::frt, fablts (S241F)-zeoRT5 aceEF, T5-pntAB, T5-udhA, del-aldA, del puuC, del arcA, del aldB, spoT, relA, T5-cynTS | 1) ptrc-mcr, 2)accABCD-udhA |
| BX3P_013 | F-, A(araD-araB)567, AlacZ4787(::rrnB-3), LAM-, rph-1, A(rhaDrhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS, fabBts | 1) ptrc-mcr |
| BX3P_014 | F-, A(araD-araB)567, AlacZ4787(::rrnB-3), LAM-, rph-1, A(rhaDrhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS, fabB- is | 1) ptrc-mcr, 2)accABCD |
| BX3P_015 | F-, A(araD-araB)567, AlacZ4787(::rrnB-3), LAM-, rph-1, A(rhaDrhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS, fabB- is | 1) ptrc-mcr, 2)accABCD-udhA |
| BX3P-016 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, T5-cynTS | 1) ptrc-mcr |
| BX3P-017 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, T5-cynTS | 1) ptrc-mcr, 2)accABCD |
| BX3P_018 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, T5-cynTS | 1) ptrc-mcr, 2)accABCD-udhA |
| BX3P_019 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoRT5 aceEF, T5-pntAB, T5-udhA, del puuC, del arcA, del aldB, spoT, relA, T5-cynTS | 1) ptrc-mcr |
| BX3P_020 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del puuC, del arcA, del aldB, spoT, relA, T5-cynTS | 1) ptrc-mcr, 2)accABCD |
| BX3P_021 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del puuC, del arcA, del aldB, spoT, relA, T5-cynTS | 1) ptrc-mcr, 2)accABCD-udhA |
| BX3P_022 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoRT5 aceEF, T5-pntAB, T5-udhA, del-aldA, del puuC, del aldB, spoT, relA, T5-cynTS, fabB-ts | 1) ptrc-mcr |
| BX3P_023 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del aldB, spoT, relA, T5-cynTS, fabB-ts | 1) ptrc-mcr, 2)accABCD |
| BX3P_024 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoRT5 aceEF, T5-pntAB, T5-udhA,del-aldA, del puuC, del aldB, spoT, relA, T5-cynTS, fabB-ts | 1) ptrc-mcr, 2)accABCD-udhA |
| BX3P_025 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhABSD | 1) pACYCmcr-accABCD, 2) pKK223- metE C645A |
| BX3P_026 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhABSD | 1) pACYCmcr-accABCD, 2) pKK223-ct his-thrA |
| BX3P_027 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhA-BSD | 1) pACYCmcr-accABCD, 2) pKK223-aroH*457 |
| BX3P_028 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhA-BSD | 1) pACYCmcr-accABCD, 2) psmart-hcamp-cadA |
| BX3P_029 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhA-BSD | 1) pACYCmcr-accABCD, 2) psmart-hcamp-metC |
| BX3P_030 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhA- BSD | 1) pACYCmcr-accABCD, 2) psmart- |
| | | hcampnrdAB |
| BX3P_031 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhA-BSD | 1) pACYCmcr-accABCD, 2) psmart-hcamp-prs |

### Example 14: 3-HP Production

An inoculum of a genetically modified microorganism that possesses a 3-HP production pathway and other genetic modifications as described above is provided to a culture vessel to which also is provided a liquid media comprising nutrients at concentrations sufficient for a desired bio-process culture period.

The final broth (comprising microorganism cells, largely 'spent' media and 3-HP, the latter at concentrations, in various embodiments, exceeding 1, 2, 5, 10, 30, 50, 75 or 100 grams/liter) is collected and subjected to separation and purification steps so that 3-HP is obtained in a relatively purified state. Separation and purification steps may proceed by any of a number of approaches combining various methodologies, which may include centrifugation, concentration, filtration, reduced pressure evaporation, liquid/liquid phase separation (including after forming a polyamine-3-HP complex, such as with a tertiary amine such as CAS#68814-95-9, Alamine® 336, a triC8-10 alkyl amine (Cognis, Cincinnati, OH or Henkel Corp.), membranes, distillation, and/or other methodologies recited in this patent application, incorporated herein. Principles and details of standard separation and purification steps are known in the art, for example in "Bioseparations Science and Engineering," Roger G. Harrison et al., Oxford University Press (2003), and Membrane Separations in the Recovery of Biofuels and Biochemicals - An Update Review, Stephen A. Leeper, pp. 99-194, in Separation and Purification Technology, Norman N. Li and Joseph M. Calo, Eds., Marcel Dekker (1992), incorporated herein for such teachings. The particular combination of methodologies is selected from those described herein, and in part is based on the concentration of 3-HP and other components in the final broth.

### Example 15: Genetic modification/introduction of Malonyl-CoA reductase for 3-HP production in Bacillus subtilis

For creation of a 3-HP production pathway in Bacillus Subtilis the codon optimized nucleotide sequence for the malonyl-CoA reductase gene from Chloroflexus aurantiacus that was constructed by the gene synthesis service from DNA 2.0 (Menlo Park, CA USA), a commercial DNA gene synthesis provider, was added to a Bacillus Subtilis shuttle vector. This shuttle vector, pHT08 (SEQ ID NO:17), was obtained from Boca Scientific (Boca Raton, FL USA) and carries an inducible Pgrac IPTG-inducible promoter.

This mcr gene sequence was prepared for insertion into the pHT08 shuttle vector by polymerase chain reaction amplification with primer 1
(5'GGAAGGATCCATGTCCGGTACGGGTCG-3') (SEQ ID NO:18), which contains homology to the start site of the mcr gene and a BamHI restriction site, and primer 2 (5'-Phos-GGGATTAGACGGTAATCGCACGACCG-3') (SEQ ID NO:19), which contains the stop codon of the mcr gene and a phosphorylated 5' terminus for blunt ligation cloning. The polymerase chain reaction product was purified using a PCR purification kit obtained from Qiagen Corporation (Valencia, CA USA) according to manufacturer's instructions. Next, the purified product was digested with BamHI obtained from New England BioLabs (Ipswich, MA USA) according to manufacturer's instructions. The digestion mixture was separated by agarose gel electrophoresis, and visualized under UV transillumination as described in Subsection II of the Common Methods Section. An agarose gel slice containing a DNA piece corresponding to the mcr gene was cut from the gel and the DNA recovered with a standard gel extraction protocol and components from Qiagen (Valencia, CA USA) according to manufacturer's instructions.

This pHT08 shuttle vector DNA was isolated using a standard miniprep DNA purification kit from Qiagen (Valencia, CA USA) according to manufacturer's instructions. The resulting DNA was restriction digested with BamHI and Smal obtained from New England BioLabs (Ipswich, MA USA) according to manufacturer's instructions. The digestion mixture was separated by agarose gel electrophoresis, and visualized under UV transillumination as described in Subsection II of the Common Methods Section. An agarose gel slice containing a DNA piece corresponding to digested pHT08 backbone product was cut from the gel and the DNA recovered with a standard gel extraction protocol and components from Qiagen (Valencia, CA USA) according to manufacturer's instructions.

Both the digested and purified mcr and pHT08 products were ligated together using T4 ligase obtained from New England BioLabs (Ipswich, MA USA) according to manufacturer's instructions. The ligation mixture was then transformed into chemically competent 100 E. coli cells obtained from Lucigen Corporation (Middleton WI, USA) according to the manufacturer's instructions and plated LB plates augmented with ampicillin for selection. Several of the resulting colonies were cultured and their DNA was isolated using a standard miniprep DNA purification kit from Qiagen (Valencia, CA USA) according to manufacturer's instructions. The recovered DNA was checked by restriction digest followed by agarose gel electrophoresis. DNA samples showing the correct banding pattern were further verified by DNA sequencing. The sequence verified DNA was designated as pHT08-mcr, and was then transformed into chemically competent Bacillus subtilis cells using directions obtained from Boca Scientific (Boca Raton, FL USA). Bacillus subtilis cells carrying the pHT08-mcr plasmid were selected for on LB plates augmented with chloramphenicol.

Bacillus subtilis cells carrying the pHT08-mcr, were grown overnight in 5 ml of LB media supplemented with 2Oug/mL chloramphenicol, shaking at 225 rpm and incubated at 37 degrees Celsius. These cultures were used to inoculate 1% v/v, 75 mL of M9 minimal media supplemented with 1.47 g/L glutamate, 0.021 g/L tryptophan, 20 ug/mL chloramphenicol and 1mM IPTG. These cultures were then grown for 18 hours in a 250mL baffled Erlenmeyer flask at 25 rpm, incubated at 37 degrees Celsius. After 18 hours, cells were pelleted and supernatants subjected to GCMS detection of 3-HP (described in Common Methods Section Mb)). Trace amounts of 3-HP were detected with qualifier ions.

### Example 16: Bacillus subtilis strain construction

Plasmids may be prepared and transformed into B. subtilis using a modified protocol developed from Anagnostopoulos and Spizizen (Requirements for transformation in Bacillus subtilis. J. Bacteriol. 81:741-746 (1961) as provided with the instructions for the pHT08 shuttle vector by Boca Scientific (Boca Raton, FL USA).

### Example 17: Yeast aerobic pathway for 3HP production

The following construct (SEQ ID NO:20) containing: 200 by 5' homology to ACC1,His3 gene for selection, Adhl yeast promoter, BamHI and Spel sites for cloning of MCR, eye 1 terminator, Tefl promoter from yeast and the first 200 by of homology to the yeast ACC1 open reading frame will be constructed using gene synthesis (DNA 2.0). The MCR open reading frame (SEQ ID NO:21) will be cloned into the BamHI and Spel sites, this will allow for constitutive transcription by the adhl promoter. Following the cloning of MCR into the construct the genetic element (SEQ ID NO:22) will be isolated from the plasmid by restriction digestion and transformed into relevant yeast strains. The genetic element will knock out the native promoter of yeast ACC1 and replace it with MCR expressed from the adhl promoter and the Tefl promoter will now drive yeast ACC1 expression. The integration will be selected for by growth in the absence of histidine. Positive colonies will be confirmed by PCR. Expression of MCR and increased expression of ACC1 will be confirmed by RT-PCR.

An alternative approach that could be utilized to express MCR in yeast is expression of MCR from a plasmid. The genetic element containing MCR under the control of the ADH1 promoter (SEQ ID 4) could be cloned into a yeast vector such as pRS421 (SEQ ID NO:23) using standard molecular biology techniques creating a plasmid containing MCR (SEQ ID NO:24) . A plasmid based MCR could then be transformed into different yeast strains. Based on the present disclosure, it is noted that, in addition to introducing a nucleic acid construct that comprises a sequence encoding for malonyl-CoA reductase activity in a yeast cell, in some embodiments additional genetic modifications are made to decrease enoyl-CoA reductase activity and/or other fatty acid synthase activity.

### Example 18: Yeast Strain construction

Yeast strains were constructed using standard yeast transformation and selected for by complementation of auxotrophic markers. All strains are S288C background. For general yeast transformation methods, see Gietz, R.D. and R.A. Woods. (2002) "Transformation of Yeast by the Liac/SS Carrier DNA/PEG Method." Methods in Enzymology 350: 87-96.

### Example 19: Production of Flaviolin Polyketide

This example provides data and analysis from strains to which plasmids were added in various combinations. One such plasmid comprises a gene for 1,3,6,8-tetrahydronapthalene synthase (rppA from Streptomyces coelicolor A3(2)), which was codon-optimized for E. coli (DNA2.0, Menlo Park, CA USA). Below this is referred to as THNS, which converts 5 malonyl-CoA to one molecule of 1,3,6,8-naphthalenetetrol, 5 CO2, and 5 coenzyme A. The 1,3,6,8-naphthalenetetrol product of THNS is reported to convert spontaneously to the polyketide flaviolin (CAS No. 479-05-0), which is readily detected spectrometrically at 340 nm. Another plasmid comprises acetyl-CoA carboxylase genes ABCD, which as described elsewhere herein may increase supply of malonyl-CoA from acetyl-CoA.

Two of the strains comprise mutant forms of one or more genes of the fatty acid synthase pathway. These forms are temperature-sensitive and have lower activity at 37 C. These strains are designated as BX595, comprising a temperature-sensitive mutant fabl, and BX660, comprising both temperature-sensitive fabl and fabB genes.

The results herein generally demonstrate that polyketide synthesis is increased when a genetically modified microorganism comprises both at least one heterologous nucleic acid sequence of a polyketide synthesis pathway and at least one modification to decrease activity, such as transiently, of one or more enzymatic conversion steps of the fatty acid synthase pathway. This is considered to reduce enzymatic activity in the microorganism's fatty acid synthase pathway providing for reduced conversion of malonyl-CoA to fatty acids, and in this case lead to increased polyketide synthesis.

The following strains and plasmids were obtained or made using common genetic/molecular biology methods, such as described elsewhere herein, and also in Sambrook and Russell, "Molecular Cloning: A Laboratory Manual," Third Edition 2001 (volumes 1-3), Cold Spring Harbor Laboratory Press, Cold Spring Harbor, N.Y.. Respective genotypes follow strain identifications.

BW25113 (F-, A(araD-araB)567, AlacZ4787(::rrnB-3), LAM-, rph-1, A(rhaD-rhaB)568, hsdR514)

BX595 (F-, A(araD-araB)567, AlacZ4787(::rrnB-3), LAM-, rph-1, A(rhaD-rhaB)568, hsdR514,, AldhA:frt, ApflB:frt, mgsA:frt,ApoxB:frt, Apta-ack:frt, fablts (S241F)-zeoR)

BX660 (F-, A(araD-araB)567, AlacZ4787(::rrnB-3), LAM-, rph-1, A(rhaD-rhaB)568, hsdR514,, AldhA:frt, ApflB:frt, mgsA:frt,ApoxB:frt, Apta-ack:frt, fablts (S241F)-zeoR, fabBts-BSD), pTRC-ptrc_THNS (SEQ ID NO:1) (developed from Invitrogen's ptrc-HisA plasmid, Invitrogen, Carlsbad, CA USA, with TENS under control of ptrc promoter in this plasmid pJ251-accABCD.

Using the above-listed E. coli strains and plasmids, the following were prepared by standard introduction of plasmids to E. coli strains:
1. BW25113 + pIRC-ptrc_THNS
2. BW25113 + pTRC-ptrc_THNS ; pJ251+accABCD
3. BX595 + pTRC-ptrc_THNS
4. BX595 + pTRC-ptrc_THNS ; pJ251+accABCD
5. BX660 + pTRC-ptrc_THNS
6. BX660 + pTRC-ptrc_THNS ; pJ251+accABCD

These then were evaluated as described below by following the protocols summarized for each respective evaluation.

### A: 96 Deep Well Plate Screen:

The BW25113 and BX595 strains above were run in triplicate. 1mL SM3 or LB media with amp and IPTG was added to the appropriate number of wells. The wells were inoculated with single colonies picked from plates. The 96 well plate was put at 30C for -6-8 hours then shifted to 37°C overnight. After 24 hours, a 200uL aliquot was removed and transferred to a 96 well flat bottom plate used for measuring absorbance on the spectrometer. The first read was done at OD600 to quantify cell growth and to use for normalizing the flaviolin reading. The plate was then spun at 4000rpm for 10 minutes. A 150uL aliquot was removed and read at OD340 to quantify the amount of flaviolin produced. The data is reported as both OD340/0D600 and just OD340.

### B: Shake Flask Screen #1:

25mL cultures of BW25113 and BX595 strains were grown overnight in TB medium with appropriate antibiotics. 50mL shake flask cultures were set up in SM3 with a 5% inoculation from the TB overnight cultures. The shake flasks were induced at time of inoculation. The cultures were grown for 48 hours and samples were taken for flaviolin readings throughout the experiment. Again, data is reported as both OD340/0D600 and just OD340.

### C: Shake Flask Screen #2:

The shake flask experiment above was repeated for 24 hours only and with all three background strains mentioned above. Samples were taken at the 24 hour time point only. Data is shown in the figures. ANOVA tests were run when necessary to compare data and find statistically significant results. The amounts of malonyl-CoA produced by the different strains should be evident by flaviolin levels.

### Example 20: Production of Polyketides

The following example generally describes polyketide synthesis in E. coli by expression of polyketide synthases (PKS). Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding any of numerous PKS enzymes, including type I, type II and type III PKS can be used to introduce these activities into genetically modified organisms that can then convert malonyl-CoA with or without other substrates and reductants including NADPH and NADH into numerous chemical products, for a nonlimiting list of PKS systems refer to the various PKS examples provided in Tables 1A-1H.

### Example 21: Production of Phloroglucinol

This example describes phloroglucinol production in E. coli by expression of phloroglucinol synthases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding phloroglucinol synthase such as that encoded by the phlD gene of P. fluorescens Pf-5 (or known mutants thereof) can be used to introduce this activity into genetically modified organisms that can then convert 3 molecules of malonyl-CoA into one molecule of phloroglucinol. The sequence of the expressed protein (SEQ ID NO:161) is provided below:

### Example 22: Production of Resorcinol

This example describes resorcinol production in E. coli by expression of phloroglucinol synthases and phloroglucinol reductases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding phloroglucinol synthase such as that encoded by the ph1D gene of P. fluorescens Pf-5 (or known mutants thereof) can be used to introduce this activity into genetically modified organisms that can then convert 3 molecules of malonyl-CoA into one molecule of phloroglucinol. Phloroglucinol can then serve as a substrate for phloroglucinol reductase, which acts to reduce phloroglucinol to dihydrophloroglucinol. Dihydro can then be abiotically converted to resorcinol by acidic conditions such as by acidicd extraction into solvents such as ethyl acetate as described by Armstrong & Patel, "Ablotic conversion of dihydrophloroglucinol to resorcinol" Canadian Journal of -Microbiology, 1993. 39:(9) 899-902., 10.1139/m93-1:35.

### Example 23: Production of Alkylresorcinol(s)

This example describes alkylresorcinol production in E. coli by expression of alkylresorcinol synthases (polyketide synthases). Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding alkylresorinol synthases can be used to introduce this activity into genetically modified organisms that can then convert malonyl-CoA into alkylresorcinols, generally 3 molecules of malonyl-CoA and fatty acyl-CoA of differing chain lengths are condensed to form alkylresoricnols of differing chain lengths.

### Example 24: Production of Triacetic lactone

The following example details triacetic lactone (TAL) production in E. coli by expression of TAL synthases (polyketide synthases). Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding TAL synthases can be used to introduce this activity into genetically modified organisms that can then convert malonyl-CoA and acetyl-CoA into TAL. Specifically Penicillium patulum 6-methylsalycilic acid synthase (6- MSAS) and its mutant (Y1572F) may be used as a TAL synthase for TAL production. (Biotechnol Bioeng. 2006 Mar 5;93(4):727-36.Microbial synthesis of triacetic acid lactone.Xie D, Shao Z, Achkar J, Zha W, Frost JW, Zhao H. Department of Chemistry, Michigan State University, East Lansing, Michigan 48824, USA.)

### Example 25: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. Genes encoding acetoacetyl-CoA thiolase activity such as the phaA genes from ralstonia species or rhodobacter species, may be used to produce acetoacetyl-CoA from 2 molecules of acetyl-CoA. Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P.
putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene from T brucei (Genbank accession no. AAX70671) may be expressed to convert butyryl-CoA to C10-fatty acyl-CoA (decanoyl-CoA) by the iterative condensation, dehydration and reduction of 3 molecules of malonyl-CoA, requiring electrons from either NADPH or NADH. Once decanoyl¬CoA is produced the action of a thioesterase such as encoded by the tesA gene of E. coli thioesterase I (known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 26: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl¬CoA then serves as the primer unit for the action of the elongase enzymes. The elol gene from T brucei (Genbank accession no. AAX70671) may be expressed to convert butyryl-CoA to C10-fatty acyl-CoA (decanoyl-CoA) by the iterative condensation, dehydration and reduction of 3 molecules of malonyl-CoA, requiring electrons from either NADPH or NADH. Once decanoyl-CoA is produced the action of a thioesterase such as encoded by the tesA gene of E. coli thioesterase I(known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 27: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. Genes encoding acetoacetyl-CoA thiolase activity such as the phaA genes from ralstonia species or rhodobacter species, may be used to produce acetoacetyl-CoA from 2 molecules of acetyl-CoA. Acetoacetyl-CoA can in turn be converted to (R)-3- hydroxybutyryl-CoA via the action of an NADH or NADPH dependent (R)-3-hydroxybutyryl-CoA dehydrogenase, such as that encoded by the phaB gene of C. necator. (R)-3-hydroxybutyryl-CoA can subsequently epimerized to (S) - 3 hydroxybutyryl-CoA by any number of epimerases including those encoded by the fadll genes of E. coli. (S) -3 hydroxybutyryl-CoA be dehydrated by the actions of crotonase or enoyl¬CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene (Genbank accession no. AAX70671) from T brucei may be expressed to convert butyryl-CoA to C10-fatty acyl-CoA (decanoyl-CoA) by the iterative condensation, dehydration and reduction of 3 molecules of malonyl-CoA, requiring electrons from either NADPH or NADH. Once decanoyl-CoA is produced the action of a thioesterase such as encoded by the tesA gene of E. coli thioesterase I(known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 28: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (R)-3-hydroxybutyryl-CoA via the action of an NADH or NADPH dependent (R)-3- hydroxybutyryl-CoA dehydrogenase, such as that encoded by the phaB gene of C. necator. (R)-3- hydroxybutyryl-CoA can subsequently epimerized to (S) -3 hydroxybutyryl-CoA by any number of epimerases including those encoded by the fadll genes of E. coli. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene from T brucei (Genbank accession no. AAX70671) may be expressed to convert butyr yl-CoA to C10-fatty acyl-CoA (decanoyl-CoA)
by the iterative condensation, dehydration and reduction of 3 molecules of malonyl-CoA, requiring electrons from either NADPH or NADH. Once decanoyl-CoA is produced the action of a thioesterase such as encoded by the tesA gene of E. coli thioesterase I(known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 29: C14 fatty acid (Myristic Acid) Production in E. coli with fatty acid synthase inhibition.

This example describes C14 fatty acid production in E. coli by expression of malonyl-CoA specific elongases. Any of the genetically modified mircoorganisms described above that produce C10-fatty-acyl-CoA (decanoyl-CoA) can be further genetically modified to produce C14 fatty acid or myristic acid. Vectors and tools are well known in the art for introducing further genetic modifications, as are promoter systems allowing for controlled or constitutive gene expression. In these microorganisms, decanoyl-CoA can serve as the primer unit for the action of the elongase enzymes. The elo2 gene from T brucei (Genbank accession no. AAX70672) may be expressed to convert decanoyl - CoA to C14-fatty acyl-CoA (myristyl-CoA) by the iterative condensation, dehydration and reduction of 2 molecules of malonyl-CoA, requiring electrons from either NADPH or NADH. Once myristyl-CoA is produced the action of a thioesterase such as encoded by the tesA gene of E. coli thioesterase I(known to act on C14- fatty-acyl-CoA , Bonner & Block, Journal of Biological Chemistry, Vol 247., No. 10, 1972,p3123-3133) to produce the free fatty acid, in this case C14 free fatty acid myristic acid.

### Example 30: C18 fatty acid (Stearic Acid) Production in E. coli with fatty acid synthase inhibition.

This example describes C18 fatty acid production in E. coli by expression of malonyl-CoA specific elongases. Any of the genetically modified mircoorganisms described above that produce C14-fatty-acyl-CoA (myristyl-CoA) can be further genetically modified to produce C18 fatty acid or stearic acid. Vectors and tools are well known in the art for introducing further genetic modifications, as are promoter systems allowing for controlled or constitutive gene expression. In these microorganisms, myristyl-CoA can serve as the primer unit for the action of the elongase enzymes. The elo3 gene from T brucei (Genbank accession no. AAX70673) may be expressed to convert myristyl-CoA to C18-fatty acyl-CoA (stearoyl-CoA) by the iterative condensation, dehydration and reduction of 2 molecules of malonyl-CoA, requiring electrons from either NADPH or NADH. Once stearoyl-CoA is produced the action of a thioesterase such as encoded by the tesA gene of E. coli thioesterase I(known to act on C14-fatty-acyl-CoA , Bonner & Block, Journal of Biological Chemistry, Vol 247., No. 10, 1972,p3123-3133) to produce the free fatty acid, in this case C18 free fatty acid stearic acid.

### Example 31: Fatty acid production in yeast with fatty acid synthase inhibition.

Any of the above examples can be ported to any number of other hosts for chemicals production, using tools and techniques well known in the art for genetic modification of a multitude of yeast systems. Briefly, to produce fatty acids in yeast the elongase systems, metabolic pathways to produce fatty acyl-CoAs can be introduced as described above, in addition to genetic modifications to reduce or eliminate native host fatty acid beta-oxidation and inhibit the host fatty acid synthase.

### Example 32: Fatty acid production in bacillus with fatty acid synthase inhibition.

Any of the above examples can be ported to any number of other hosts for chemicals production, using tools and techniques well known in the art for genetic modification of a multitude of gram positive systems such as bacillus subtilis. Briefly, to produce fatty acids in bacillus or other gram positive systems the elongase systems, metabolic pathways to produce fatty acyl-CoAs can be introduced as described above, in addition to genetic modifications to reduce or eliminate native host fatty acid beta-oxidation and inhibit the host fatty acid synthase.

### Example 33: Fatty acid production in C. necator with fatty acid synthase inhibition.

Any of the above examples can be ported to any number of other hosts for chemicals production, using tools and techniques well known in the art for genetic modification of a multitude of gram negative systems such as C. necator. Briefly, to produce fatty acids in C. necatot the elongase systems, metabolic pathways to produce fatty acyl-CoAs can be introduced as described above, in addition to genetic modifications to reduce or eliminate native host fatty acid beta-oxidation and inhibit the host fatty acid synthase. Additionally, in C. necator modifications to remove polyhydroxybutyrate (PHB) synthesis can be incorporated. C. necator has an additional advatnge of producing fatty acids via the feedstocks, hydrogen and carbon dioxide.

### Example 34: Diacid production with fatty acid synthase inhibition.

This example generally describes diacid synthesis in E. coli by expression of polyketide syntheses (PKS) that have been modified for diacid (dicarboxylic acid) production. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding any of numerous modified PKS enzymes, including type I, type II and type III PKS can be used to introduce these activities into genetically modified organisms that can then convert malonyl-CoA with or without other substrates and reductants including NADPH and NADH into diacids.

### Example 35: Diene production with fatty acid synthase inhibition.

This example generally describes diene synthesis in E. coli by expression of polyketide syntheses (PKS) that have been modified for diene production. Briefly, genetically modified E. coli with controlled fatty acid inhibition can be constructed as described in any of the above
examples. Any of these strains may be used as starting points for further genetic modifications. Vectors and tools are well known in the art for introducing further genetic modifications, as are promoter systems allowing for controlled or constitutive gene expression. Genes encoding any of numerous modified PKS enzymes, including type I, type II and type III PKS can be used to introduce these activities into genetically modified organisms that can then convert malonyl-CoA with or without other substrates and reductants including NADPH and NADH into dienes.

### Example 36: n-butanol production with fatty acid synthase inhibition from malonyl-CoA .

This example describes n-butanol production in E. coli by expression of malonyl-CoA dependent acetoacetyl¬CoA synthetase. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl¬CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (S)- 3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3-hydroxybutyryl-CoA via the action of an NADH or NADPH dependent (R)-3- hydroxybutyryl-CoA dehydrogenase, such as that encoded by the phaB gene of C. necator. (R)-3- hydroxybutyryl-CoA can subsequently epimerized to (S) -3 hydroxybutyryl-CoA by any number of epimerases including those encoded by the fadll genes of E. coli. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. Butyryl-CoA can then serve as the substrate for the NADH dependent butanol dehydrogenase adhE2 gene from Clostridia acetobutylicum. This enzyme carries out the two step reduction of butyryl-CoA to butanol.

### Example 37: Isobutanol production with fatty acid synthase inhibition from malonyl-CoA .

This example describes n-butanol production in E. coli by expression of malonyl-CoA dependent acetoacetyl-CoA synthetase. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (S)- 3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3-hydroxybutyryl-CoA via the action of an NADH or NADPH dependent (R)-3- hydroxybutyryl-CoA dehydrogenase, such as that encoded by the phaB gene of C. necator. (R)-3- hydroxybutyryl-CoA can subsequently epimerized to (S)-3 hydroxybutyryl-CoA by any number of epimerases including those encoded by the fadll genes of E. coli. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. Butyryl-CoA can then serve as the substrate for an isomerase capable of producing isobutyryl-CoA from butyryl-CoA, suh as that endoded by the icmA and icmB genes of Streptoriisi-e

Isobutyryl-CoA is then the substrate for the NADH dependent dehydrogenases adhE2 that can carry out the two step reduction of isobutyryl-CoA to isobutanol, such as the adhE genes from Giardia species.

### Example 38: Chemical production from butyryl-CoA as an intermediate.

Similarly to the production of butanol, described above, the following example details any chemical production stemming from butyryl-CoA as an intermediate by expression of malonyl-CoA dependent acetoacetyl-CoA synthetase. As discussed above, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools with the additional deletion of the atoB gene encoding an aceto-acetyl-CoA thiolase, which can degrade acetoacetyl-CoA into two molecules of acetyl-CoA.

Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3¬hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3-hydroxybutyryl-CoA via the action of an NADH or NADPH dependent (R)-3-hydroxybutyryl-CoA dehydrogenase, such as that encoded by the phaB gene of C. necator. (R)-3-hydroxybutyryl-CoA can subsequently epimerized to (S) -3 hydroxybutyryl-CoA by any number of epimerases including those encoded by the fadll genes of E. coli. (S)-3-hydroxybutyryl¬CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl¬CoA reductase encoded by the crr gene of S. colinus, or the NADH dependent crotonyl-CoA reductase encoded by the ter gene of T denticola. Butyryl-CoA can then serve as the substrate for numerous other chemical products, such as butyrate with activity of a butyryl-CoA hydrolase, or alternatively from the actions of a phosphotranbutyrylase and butyrate kinase such as those encoded by the ptb and bukl genes of Clostridia sp. Additionally, other chemical products may be produced via adding additional enzymes to further convert butyryl-CoA to these products from malonyl-CoA.

### Example 39: Chemical production from isobutyryl-CoA as an intermediate.

Similarly to the production of butanol, described above, the following example describes any chemical production stemming from isobutyryl-CoA as an intermediate by expression of malonyl-CoA dependent acetoacetyl-CoA synthetase. As discussed above, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools with the additional deletion of the atoB gene encoding an aceto-acetyl-CoA thiolase, which can degrade acetoacetyl-CoA into two molecules of acetyl-CoA. Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3- hydroxybutyryl-CoA via the action of an NADH or NADPH dependent (R)-3-hydroxybutyryl-CoA dehydrogenase, such as that encoded by the phaB gene of C. necator. (R)-3-hydroxybutyryl-CoA can subsequently epimerized to (S) -3 hydroxybutyryl-CoA by any number of epimerases including those encoded by the fadll genes of E. coli. (S)-3-hydroxybutyryl-CoA can be dehydrated by the actions of crotonase or enoyl¬CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. Butyryl-CoA can then serve as the substrate for an isomerase capable of producing isobutyryl-CoA from butyryl-CoA, suh as that endoded by the icmA and icmB genes of S.frt'pit)rilyt'e, Isobutyryl-CoA can then serve as the substrate for numerous other chemical products, such as isobutyrate with activity of a isobutyryl-CoA hydrolase, or alternatively from the actions of a phosphotransisobutyrylase and isobutyrate kinase. Additionally, other chemical products may be produced via adding additional enzymes to further convert isobutyryl-CoA to these products from malonyl-CoA, such as phlorisobutyrophenone via the actions of either isobutyrophenone synthase form Hypericum calycinum or phlorisobutyrophenone synthase / phlorisovalerophenone synthase encoded by the VPS gene from Humulus lupulus. Additionally, other chemical products may be produced via adding additional enzymes to further convert isobutyryl-CoA to these products from malonyl-CoA.

### Example 40: Chemical production from methacrylyl-CoA as an intermediate.

Similarly to the production of chemicals in the examples described above, the following example describes any chemical production stemming from methacrylyl-CoA as an intermediate by expression of malonyl-CoA dependent acetoacetyl-CoA synthetase. As discussed above, genetically modififed organisms can be engineered to produce butyryl-CoA and the subsequently isobutyryl-CoA as intermediates. Isobutyryl-CoA can then be further converted to methacrylyl-CoA by the actions of a methylacyl-CoA dehydrogenase such as those encoded by the acdH or Acadsb genes from Streptomyces avermitilis and Rattus norvegicus, respectively. Any of these strains may be used as starting points for further genetic modifications. Other chemical products may be produced via adding additional enzymes to further convert methacrylyl-CoA to these products from malonyl¬CoA, such as methylacrylate via the actions of a methacrylyl-CoA hydrolase. Alternatively, 3- hydroxyisobutyrate can be made from methylacrylyl-CoA via the actions of first a short chain enoyl-CoA hydratase such as those encoded by the ECHS1 or ech genes of Bos Taurus Pseudomonas fluorescens, respectively, converting methylacrylyl-CoA to 3-hydroxyisobutyryl-CoA. 3- hydroxyisobutyryl-CoA can then be converted to 3-hydroxyisobutyrate via the actions of a 3- hydroxyisobutyryl-CoA hydrolase, such as that encoded by the Hibch gene of Rattus norvegicus. Additionally, other chemical products may be produced via adding additional enzymes to further convert methylacryryl-CoA to these products from malonyl-CoA.

### Example 41: Chemical production from 3-hydroxy-3-methylglutaryl-CoA, as an intermediate.

Similarly to the production of chemicals in the examples described above, this example describes any chemical production stemming from acetoacetyl-CoA as an intermediate by expression of malonyl-CoA dependent acetoacetyl-CoA synthetase. As discussed above, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools, with the additional deletion of the atoB gene encoding an aceto-acetyl-CoA thiolase, which can degrade acetoacetyl-CoA into two molecules of acetyl-CoA. Acetoacetyl-CoA can further be converted to 3-hydroxy-3-methylglutaryl-CoA, by the actions of numerous hydroxymethylglutaryl-CoA syntheses such as that encoded by the hmgS gene of Saccharomyces species. 3-hydroxy-3-methylglutaryl-CoA can further be converted to numerous products via the mevalonate pathway, wherein mevalonate is first produced from 3-hydroxy-3-methylglutaryl-CoA (hmg-CoA) by reduction using enzymes such as the hmg-CoA reductases encoded by numerous genes including the HMG1 and HMG2 genes of Saccharomyces species. The mevalonate pathway is well known as a metabolic pathway allowing the production of numerous products, such as farnesene and other isoprenoids. Additionally, other chemical products may be produced via adding additional enzymes to further convert 3-hydroxy-3-methylglutaryl-CoA or mevalonate to these products from malonyl-CoA.

### Example 42: Chemical production in yeast

Any of the above examples can be ported to any number of other hosts for chemicals production, using tools and techniques well known in the art for genetic modification of a multitude of yeast systems. Briefly, metabolic pathways to produce products can be introduced as described above.

### Example 43: Chemical production in bacillus

Any of the above examples can be ported to any number of other hosts for chemicals production, using tools and techniques well known in the art for genetic modification of a multitude of gram positive systems, such as bacillus. Briefly, metabolic pathways to produce products can be introduced as described above.

### Example 44: Chemical production in C. necator

Any of the above examples can be ported to any number of other hosts for chemicals production, using tools and techniques well known in the art for genetic modification of a multitude of gram negative systems, such as C. necatot. Briefly, metabolic pathways to produce products can be introduced as described above. Additionally, in C. necator modifications to remove polyhydroxybutyrate (PHB) synthesis can be incorporated. C. necator has an additional advatnge of producing fatty acids via the feedstocks, hydrogen and carbon dioxide.

### Example 45: Production of Phloroglucinol

This example describes phloroglucinol production in E. coli by expression of phloroglucinol synthases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding phloroglucinol synthase such as that encoded by the ph1D gene of P. fluorescens Pf-5 (or known mutants thereof) can be used to introduce this activity into genetically modified organisms that can then convert 3 molecules of malonyl-CoA into one molecule of phloroglucinol. Additionally, as phloroglucinol is more oxidized than dextrose and other sugars additional modifications may be made to reduce acitivity and flux through the citric acid (TCA) cycle. When more reduced feedstocks are used and more oxidized products are made, more than enough electrons are generated through glycolysis for maintenance energy and production needs. Flux through the citric acid cycle can lead to wasted carbon and lower yields. Genetic modifications to reduce flux through the TCA cycle can include genetic modifications to key steps such as those aimed to reduce activity or expression of the citrate synthase enzyme ecoded by the gltA gene, alternatively enzymes that lead to oxaloacetate production can be reduced or eiliminated such as phophoenolpyruvate carboxylase (such as encoded by the ppc gene) or phosphoenolpyruvate carboxykinase such as encoded by the pck genes. Alternatively genetic modifications may be introduced to cause temperature sensitive ("ts") activity in these enzymes (such as in the genes gltA, ppc, pck) to low activity at a permissive temeperature such as 30 degrees Celsius, but no activity at a nonpermissive temperature such as 37 degrees Celsius. These "ts" mutants enable a controllable decrease in TCA flux upon temperature change. Numerous temperature sensitive alleles are known for many enzymes such as gltA, and in general methods for screening mutant libraries for such mutations are known in the art, as are more directed approaches for engineering these ts mutations denovo. (Ben-Aroya, S., Coombes, C., Kwok, T., O'Donnell, K.A., Boeke, J.D., and Hieter, P. (2008) Molecular Cell 30:248-258.)

### Example 46: Production of more oxidized products from malonyl-CoA.

This example describes chemical product production in E. coli from sugar feedstocks for chemicals more oxidized than sugar. Briefly, genetically modified E. coli with controlled inhibition of fatty acid production can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding pathway enzymes can be used to introduce activities into genetically modified organisms that can then convert molecules of malonyl-CoA into numerous chemical products. Additionally, as these products are more oxidized than dextrose and other sugars additional modifications may be made to reduce acitivty and flux through the citric acid (TCA) cycle. When more reduced feedstocks are used and more oxidized products are made, more than enough electrons are generated through glycolysis for maintenance energy and production needs. Flux through the citric acid cycle can lead to wasted carbon and lower yields. Genetic modifications to reduce flux through the TCA cycle can include genetic modifications to key steps such as those aimed to reduce activity or expression of the citrate synthase enzyme such as encoded by the gltA gene, alternatively enzymes that lead to oxaloacetate production can be reduced or eliminated such as by disruption of phophoenolpyruvate carboxylase (such as encoded by the ppc) gene or phosphoenolpyruvate carboxykinase such as encoded by the pck genes. Alternatively genetic modifications may be introduced to cause temperature sensitive activity in these enzymes (such as gltA, ppc, pck) to low activity at a permissive temeperature such as 30 degrees Celsius, but no activity at a nonpermissive temperature such as 37 degrees Celsius. These temperature sensitive ("ts") mutants enable a controllable decrease in TCA flux upon temperature change.

### Example 47: Polyhydroxybutyrate production with fatty acid synthase inhibition from malonyl-CoA.

This example describes polyhydroxybutyrate production in E. coli by expression of malonyl-CoA dependent acetoacetyl-CoA synthetase. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (R)-3-hydroxybutyryl-CoA via the action of an NADPH dependent (R)-3-hydroxybutyryl-CoA dehydrogenase, such as that encoded by the phaB gene of Rhodobacter spaeroides or Cuprivdus Necator. (R)- 3-hydroxybutyryl-CoA can be polymerized by the actions of a polyhydroxybutyrate polymerase, such as those encoded by the phaC gene from Cupriavidus necator, to form polyhydroxybutyrate.

### Example 48: General example of genetic modification to a host cell.

In addition to the above specific examples, this example is meant to describe a non-limiting approach to genetic modification of a selected microorganism to introduce a nucleic acid sequence of interest. Alternatives and variations are provided within this general example. The methods of this example are conducted to achieve a combination of desired genetic modifications in a selected microorganism species, such as a combination of genetic modifications as described in sections herein, and their functional equivalents, such as in other bacterial and other microorganism species.

A gene or other nucleic acid sequence segment of interest is identified in a particular species (such as *E. coli* as described herein) and a nucleic acid sequence comprising that gene or segment is obtained.

Based on the nucleic acid sequences at the ends of or adjacent the ends of the segment of interest, 5' and 3' nucleic acid primers are prepared. Each primer is designed to have a sufficient overlap section that hybridizes with such ends or adjacent regions. Such primers may include enzyme recognition sites for restriction digest of transposase insertion that could be used for subsequent vector incorporation or genomic insertion. These sites are typically designed to be outward of the hybridizing overlap sections. Numerous contract services are known that prepare primer sequences to order (e.g., Integrated DNA Technologies, Coralville, IA USA).

Once primers are designed and prepared, polymerase chain reaction (PCRn) is conducted to specifically amplify the desired segment of interest. This method results in multiple copies of the region of interest separated from the microorganism's genome. The microorganism's DNA, the primers, and a thermophilic polymerase are combined in a buffer solution with potassium and divalent cations (e.g., Mg or Mn) and with sufficient quantities of deoxynucleoside triphosphate molecules. This mixture is exposed to a standard regimen of temperature increases and decreases. However, temperatures, components, concentrations, and cycle times may vary according to the reaction according to length of the sequence to be copied, annealing temperature approximations and other factors known or readily learned through routine experimentation by one skilled in the art.

In an alternative embodiment the segment of interest may be synthesized, such as by a commercial vendor, and prepared via PCRn, rather than obtaining from a microorganism or other natural source of DNA.

The nucleic acid sequences then are purified and separated, such as on an agarose gel via electrophoresis. Optionally, once the region is purified it can be validated by standard DNA sequencing methodology and may be introduced into a vector. Any of a number of vectors may be used, which generally comprise markers known to those skilled in the art, and standard methodologies are routinely employed for such introduction. Commonly used vector systems are pSMART (Lucigen, Middleton, WI USA), pET *E. coli* EXPRESSION SYSTEM (Stratagene, La Jolla, CA USA), pSC-B StrataClone Vector (Stratagene, La Jolla, CA USA), pRANGER-BTB vectors (Lucigen, Middleton, WI USA), and TOPO vector (Invitrogen Corp, Carlsbad, CA, USA). Similarly, the vector then is introduced into any of a number of host cells. Commonly used host cells are *E. coli* 10G (Lucigen, Middleton, WI USA), *E. coli* 10GF' (Lucigen, Middleton, WI USA), StrataClone Competent cells (Stratagene, La Jolla, CA USA), *E. coli* BL21, *E. coli* BW25113, and *E. coli* K12 MG1655. Some of these vectors possess promoters, such as inducible promoters, adjacent the region into which the sequence of interest is inserted (such as into a multiple cloning site), while other vectors, such as pSMART vectors (Lucigen, Middleton, WI USA), are provided without promoters and with dephosporylated blunt ends. The culturing of such plasmid-laden cells permits plasmid replication and thus replication of the segment of interest, which often corresponds to expression of the segment of interest.

Various vector systems comprise a selectable marker, such as an expressible gene encoding a protein needed for growth or survival under defined conditions. Common selectable markers contained on backbone vector sequences include genes that encode for one or more proteins required for antibiotic resistance as well as genes required to complement auxotrophic deficiencies or supply critical nutrients not present or available in a particular culture media. Vectors also comprise a replication system suitable for a host cell of interest.

The plasmids containing the segment of interest can then be isolated by routine methods and are available for introduction into other microorganism host cells of interest. Various methods of introduction are known in the art and can include vector introduction or genomic integration. In various alternative embodiments the DNA segment of interest may be separated from other plasmid DNA if the former will be introduced into a host cell of interest by means other than such plasmid.

While steps of this general example involve use of plasmids, other vectors known in the art may be used instead. These include cosmids, viruses (e.g., bacteriophage, animal viruses, plant viruses), and artificial chromosomes (e.g., yeast artificial chromosomes (YAC) and bacteria artificial chromosomes (BAC)).

Host cells into which the segment of interest is introduced may be evaluated for performance as to a particular enzymatic step such as regarding biosynthesis of a chemical compound of interest. Selections of better performing genetically modified host cells may be made, selecting for overall performance, tolerance, or production or accumulation of the chemical of interest.

It is noted that this procedure may incorporate a nucleic acid sequence for a single gene (or other nucleic acid sequence segment of interest), or multiple genes (under control of separate promoters or a single promoter), and the procedure may be repeated to create the desired heterologous nucleic acid sequences in expression vectors, which are then supplied to a selected microorganism so as to have, for example, a desired complement of enzymatic conversion step functionality for any of the herein-disclosed metabolic pathways. However, it is noted that although many approaches rely on expression via transcription of all or part of the sequence of interest, and then translation of the transcribed mRNA to yield a polypeptide such as an enzyme, certain sequences of interest may exert an effect by means other than such expression. The specific laboratory methods used for these approaches are well-known in the art and may be found in various references known to those skilled in the art, such as Sambrook and Russell, Molecular Cloning: A Laboratory Manual, 3rd Ed., (2001) (Volumes 1-3), Cold Spring Harbor Laboratory Press, Cold Spring Harbor, N.Y. (hereinafter, Sambrook and Russell, 2001).

As an alternative to the above, other genetic modifications may also be practiced, such as a deletion of a nucleic acid sequence of the host cell's genome. One non-limiting method to achieve this is by use of Red/ET recombination, known to those of ordinary skill in the art and described in U.S. Patent Nos. 6,355,412 and 6,509,156, issued to Stewart et al. Material and kits for such method are available from Gene Bridges (Gene Bridges GmbH, Heidelberg, Germany), and the method may proceed by following the manufacturer's instructions. Targeted deletion of genomic DNA may be practiced to alter a host cell's metabolism so as to reduce or eliminate production of undesired metabolic products. This may be used in combination with other genetic modifications such as described herein in this general example.

### Example 49: Preparing a Genetically Modified E. coli Host Cell Comprising malonyl-CoA-reductase (Mcr) in Combination with Other Genetic Modifications to Increase 3-HP Production Relative to a Control E. coli Cell.

Genetic modifications are made to introduce a vector comprising mmsB such as from *Pseudomonas auruginos,* which further is codon-optimized for *E. coli.* Vectors comprising galP and a native or mutated ppc also may be introduced by methods known to those skilled in the art (see, e.g., Sambrook and Russell, 2001), additionally recognizing that mutations may be made by a method using the XLI-Red mutator strain, using appropriate materials following a manufacturer's instructions (Stratagene QuikChange Mutagenesis Kit, Stratagene, La Jolla, CA USA) and selected for or screened under standard protocols.

Also, genetic modifications are made to reduce or eliminate the enzymatic activities of *E. coli* genes as desired. These genetic modifications are achieved by using the RED/ET homologous recombination method with kits supplied by Gene Bridges (Gene Bridges GmbH, Heidelberg, Germany) according to manufacturer's instructions.

Also, in some embodiments genetic modifications are made to increase the NADPH cellular pool. Non-limiting examples of some targets for genetic modification are provided herein. These are pgi (in a mutated form), pntAB, overexpressed, gapA:gapN substitution/replacement, and disrupting or modifying a soluble transhydrogenase such as sthA, and genetic modifications of one or more of zwf, gnd, and edd.

The so-genetically modified microorganism of any such engineered embodiment is evaluated and found to exhibit higher productivity of 3-HP compared with a control *E. coli l*acking said genetic modifications. Productivity is measured by standard metrics, such as volumetric productivity (grams of 3-HP/hour) under similar culture conditions.

### Example 50: Polyketide production via malonyl-coA in strains with combinations of FAS mutations.

The following genetically modified *E. coli* strains (listed in Table 36) were constructed form a wild type BW25113 starting host. *E. coli* BW25113 was obtained from the Yale genetic stock center (New Haven, CT, USA). These strains were constructed by standard methods such as discussed in the Common Methods Section and also known in the art as referenced above. Briefly, chromosomal modifications were constructed via homologous recombination.

**Table 36: Strain List 1**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514 | Ptrc-THNS | NA |
| 2 | F-, Δ(araD-araB)567, ΔlacZ4787(::rrnB-3), LAM-, rph-1, Δ(rhaD-rhaB)568, hsdR514 | Ptrc-THNS | pACYC-accABCD |
| 3 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, F-, Apta-ack:frt | Ptrc-THNS | NA |
| 4 | F-, Δ(araD-araB)567, Δ1acZ4787(::rmB-3), LAM-, rph-1, Δ(rhaD-rhaB)568, hsdR514, Δ1dhA:frt, ΔpflB:frt, mgsA:frt, ΔpoxB:frt, Δpta-ack:frt | Ptrc-THNS | pACYC-accABCD |
| 5 | F-, Δ(araD-araB)567, Δ1acZ4787(::rmB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | Ptrc-THNS | NA |
| 6 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | Ptrc-THNS | pACYC-accABCD |
| 7 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, fabBts (A329V) | Ptrc-THNS | NA |
| 8 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ApoxB:frt Δpta-ack:frt, fabIts (S241F)-zeoR, fabBts (A329V) | Ptrc-THNS | pACYC-accABCD |
| 9 | F-, Δ(araD-araB)567, Δ1acZ4787(::rmB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR ,MabF | Ptrc-THNS | NA |
| 10 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR ,MabF | Ptrc-THNS | pACYC-accABCD |
| 11 | F-, Δ(araD-araB)567, ΔlacZ4787(::rmB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, ΔfabF, fabBts (A329V) | Ptrc-THNS | NA |
| 12 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, ΔfabF, fabBts (A329V) | Ptrc-THNS | pACYC-accABCD |

The above strains were evaluated in shake flasks for the production of flaviolin. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture as starting inoculum, antibiotics, and 1 mM IPTG. Flasks were grown in the 30°C in a shaking incubator. At 24 hours, samples were taken for analyses of OD at 600nm and absorbance at 340 nm, the latter an indicator of the presence of flaviolin.

### Example 51: 3-HP production via malonyl-coA in strains with additional combinations of FAS mutations.

The following genetically modified *E. coli* strains (listed in Table 37) were constructed from a wild type BW25113 starting host. *E. coli* BW25113 was obtained from the Yale genetic stock center (New Haven, CT USA). These strains were constructed by standard methods such as discussed in the Common Methods Section and also known in the art as referenced above. Briefly, chromosomal modifications were constructed via homologous recombination.

**Table 37: Strain List 2**

| **Strain** | **Chromosomal genotype** | **Plasmid 1** | **Plasmid 2** |
|---|---|---|---|
| 240 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | Ptrc-mcr | pACYC-accABCD-pntAB |
| 470 | F-, Δ (araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, ΔfabF, fabB(ts) | Ptrc-mcr | NA |
| 471 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fablts (S241F)-zeoR, ΔfabF, fabB(ts) | Ptrc-mcr | pACYC-accABCD |
| 472 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, ΔfabF, fabB(ts) | Ptrc-mcr | pACYC-accABCD-pntAB |
| 473 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, ΔfabF, fabB(ts) | Ptrc-mcr | pACYC-CAT-accADBC-udhA |
| 474 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, ΔfabF, fabB(ts) | Ptrc-mcr | pBT3-tpiA-pntAB |

The above strains were evaluated in shake flasks for the production of 3-HP. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture as the starting inoculum, antibiotics, and 1 mM IPTG. Flasks were grown at 30°C in a shaking incubator. At 4, 8, 10, 12 and 24 hours, samples were taken for analyses of OD at 600nm and 3-HP production using the 3-HP bioassay described in the Common Methods Section.

**Table 38. Specific 3-HP Production of FAS mutants**

| | **Average Specific Productivity** (g 3-HP/ gDCW-hr) | | | |
|---|---|---|---|---|
| Strain | 8 hrs | 10 hrs | 12 hrs | 24 hrs |
| 240 | 0.0622887 | 0.10102 | 0.2004337 | 0.147062 |
| 472 | 0.0264916 | 0.17765 | 0.2737726 | 0.166645 |

### Example 52: 3-HP production via malonyl-coA in strains with additional combinations of FAS mutations

The following genetically modified *E. coli* strains (listed in Table 39) were constructed form a wild type BW25113 starting host. *E. coli* BW25113 was obtained from the Yale genetic stock center. These strains were constructed by standard methods discussed in the Common Methods Section and also known in the art as referenced above. Briefly, chromosomal modifications were constructed via homologous recombination.

**Table 39: Strain List 3**

| **Strain** | **Chromosomal genotype** | **Plasmid 1** | **Plasmid 2** |
|---|---|---|---|
| 240 | F-, Δ(araD-araB)567, ΔlacZ4787(::rrnB-3), LAM-, rph-1, Δ (rhaD-rhaB)568, hsdR514, ΔldhA:frt, ΔpflB:frt, mgsA:frt, ΔpoxB:frt, Δpta-ack:frt, fabIts (S241F)-zeoR | Ptrc-mcr | pACYC-accABCD-pntAB |
| 465 | F-, Δ(araD-araB)567, ΔlacZ4787(::rrnB-3), LAM-, rph-1, Δ(rhaD- rhaB)568, hsdR514,, ΔldhA:frt, ΔpflB:frt, mgsA:frt, ΔpoxB:frt, Δpta-ack:frt, fabIts (S241F)-zeoR, ΔfabF | Ptrc-mcr | NA |
| 466 | F-, Δ (araD-araB)567, ΔlacZ4787(::rrnB-3), LAM-, rph-1, Δ (rhaD-rhaB)568, hsdR514,, ΔldhA:frt, ΔpflB:frt, mgsA:frt, ΔpoxB:frt, Δpta-ack:frt, fablts (S241F)-zeoR, ΔfabF | Ptrc-mcr | pACYC-accABCD |
| 467 | F-, Δ (araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fablts (S241F)-zeoR, ΔfabF | Ptrc-mcr | pACYC-accABCD-pntAB |
| 468 | F-, Δ (araD-araB)567, ΔlacZ4787(::rrnB-3), LAM-, rph-1, Δ(rhaD- rhaB)568, hsdR514,, ΔldhA:frt, ΔpflB:frt, mgsA:frt, ΔpoxB:frt, Δpta-ack:frt, fablts (S241F)-zeoR, ΔfabF | Ptrc-mcr | pACYC-CAT-accADBC-udhA |
| 469 | F-, Δ (araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, ΔfabF | Ptrc-mcr | pBT3-tpiA-pntAB |

The above strains were evaluated in shake flasks for the production of 3-HP. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture, antibiotics, and 1 mM IPTG. Flasks were grown in the 30°C in shaking incubator, at 4, 8, 10, 12 and 24 hours, samples were taken for analyses of OD at 600nm and 3-HP production using the 3-HP bioassay described in the general methods section.

**Table 40: Specific 3-HP Production of FAS mutants.**

| | **Average Specific Productivity** (g 3-HP/gDCW-hr) | | | |
|---|---|---|---|---|
| Strain | 8 hrs | 10 hrs | 12 hrs | 24 hrs |
| 240 | 0.0622887 | 0.10102 | 0.2004337 | 0.147062 |
| 467 | 0.0741601 | 0.17242 | 0.2229593 | 0.126147 |

### Example 53: Polyketide Production via a FAS and CoA synthesis mutant strains

The following genetically modified *E. coli* strains (listed in Table 41) were constructed form a wild type BW25113 starting host. *E. coli* BW25113 was obtained from the Yale genetic stock center (New Haven, CT USA). These strains were constructed by standard methods discussed in the Common Methods Section and also known in the art as referenced above. Briefly, chromosomal modifications were constructed via homologous recombination.

**Table 41: Strain List 4**

| **Strain** | **Chromosomal genotype** | **Plasmid 1** | **Plasmid 2** |
|---|---|---|---|
| 6 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR | Ptrc-THNS | pACYC-accABCD |
| M1 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, coaA(R106A) | Ptrc-THNS | pACYC-accABCD |
| M2 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, coaA(R106A) | Ptrc-THNS | pACYC-accABCD |

The above strains were evaluated in shake flasks for the production of flaviolin. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture, antibiotics, and 1 mM IPTG with or without 40uM pantothenic acid. Flasks were grown in the 30°C in shaking incubator; at 24 hours, samples were taken for analyses of OD at 600nm and absorbance at 340 nm.

### Example 54: 3-HP Production via FAS mutant strains.

The following genetically modified *E. coli* strains (listed in Table 42) were constructed form a wild type BW25113 starting host. *E. coli* BW25113 was obtained from the Yale genetic stock center. These strains were constructed by standard methods discussed in the Common Methods Section and also known in the art as referenced above. Briefly, chromosomal modifications were constructed via recombination. Alternatively strains including any combination of fabI, fabB, fabF and or fabD mutations may be constructed and or evaluated.

**Table 42: Strain List 5**

| **Strain** | **Chromosomal genotype** | **Plasmid 1** | **Plasmid 2** |
|---|---|---|---|
| 240 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR | Ptrc-mcr | pACYC-accABCD-pntAB |
| M5 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, fabD(ts) | Ptrc-mcr | NA |
| M6 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, fabD(ts) | Ptrc-mcr | pACYC-accABCD |
| M7 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, fabD(ts) | Ptrc-mcr | pACYC-accABCD-pntAB |

Each of the above strain are evaluated in shake flask experiments for the production of 3-HP using the methods discussed in the above examples.

### Example 55: Product production via malonyl-coA in strains with combinations of FAS mutations

In general strains are constructed from a wild type starting host. These strains are constructed by standard methods discussed in the Common Methods Section and also known in the art as referenced above. Chromosomal modifications are constructed via recombination. Strains including any combination of the mutations and modifications disclosed above may be combined, particularly including any combination of enoyl-acyl carrier protein (ACP) reductase (such as fabIi), β-ketoacyl-acyl carrier protein synthase I (such as fabB), β-ketoacyl-acyl carrier protein synthase II (such as fabF), and malonyl-CoA-acyl carrier protein transacylase (such as fabD) and or pantothenate kinase (such as coaA) mutations may be constructed and/or evaluated with or without the supplementation of pantothenate to the media.

### Example 56: Example of 3-HP Production

An inoculum of a genetically modified microorganism that possesses a 3-HP production pathway and other genetic modifications as described above is provided to a culture vessel to which also is provided a liquid media comprising nutrients at concentrations sufficient for a desired bio-process culture period.

The final broth (comprising microorganism cells, largely 'spent' media and 3-HP, the latter at concentrations, in various embodiments, exceeding 1, 2, 5, 10, 30, 50, 75 or 100 grams/liter) is collected and subjected to separation and purification steps so that 3-HP is obtained in a relatively purified state. Separation and purification steps may proceed by any of a number of approaches combining various methodologies, which may include centrifugation, concentration, filtration, reduced pressure evaporation, liquid/liquid phase separation (including after forming a polyamine-3-HP complex, such as with a tertiary amine such as CAS#68814-95-9, Alamine® 336, a triC8-10 alkyl amine (Cognis, Cincinnati, OH USA), membranes, distillation, evaporation, and/or other methodologies. Principles and details of standard separation and purification steps are known in the art, for example in *"*Bioseparations Science and Engineering," Roger G. Harrison et al., Oxford University Press (2003), and Membrane Separations in the Recovery of Biofuels and Biochemicals - An Update Review, Stephen A. Leeper, pp. 99-194, in Separation and Purification Technology, Norman N. Li and Joseph M. Calo, Eds., Marcel Dekker (1992).

The particular
combination of methodologies is selected from those described herein, and in part is based on the concentration of 3-HP and other components in the final broth.

Similar culture procedures may be applied for other chemical products disclosed herein.

### Example 57: Induction of malonyl-CoA reductase by fermentation under low-phosphate conditions

Plasmid maps are shown in the Figures. Plasmid 1 was digested with NcoI/Bst1107. A fragment size of 7059 bases was excised from a gel and purified (SEQ ID NO:168). The target promoter sequence was ordered (Integrated DNA Technologies, Coralville, Iowa USA) including with modifications to the native ribosome binding site and subsequently changed to be compatible with existing expression vectors and to accommodate expression of key downstream gene(s) within the vector(s), in this example malonyl CoA reductase (MCR, mcr). Plasmid 2, synthesized by (Integrated DNA Technologies, Coralville, IA USA) to comprise this low-phosphate promoter (see discussion above regarding SEQ ID NOs: 210 and 211), was digested with NcoI/PmlI. A fragment size of 156 bases was excised from a gel and purified, this fragment is the pYibD promoter (SEQ ID NO:210). Fragments were ligated overnight using T4 DNA ligase to create Plasmid 3, identified as pTrc-P_{yibD}-mcr (SEQ ID NO:170).

The pTrc_PyibD-mcr plasmid was evaluated by the standard shake flask protocol with variable phosphate levels.

### Example 58: Fermentation Events Using Strain 547

Strain 547 was prepared using molecular biology techniques described elsewhere herein, including use of homologous recombination to introduce and/or replace portions of the genome and construction and introduction of plasmids.

**Table 43: Strain List 6**

| **Strain** | **Chromosomal genotype** | **Plasmid 1** | **Plasmid 2** |
|---|---|---|---|
| 547 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabIts (S241F)-zeoR, fabB(ts), ΔfabF:frt, coaA*, fabD(ts), ΔaceBAK:frt | pTRC-PyibD-mcr | pACYC-Ptal-pntAB-Ptpia-accAD-PrpiA-accCD |

This strain was then evaluated under various fermentations conditions that modulated or otherwise controlled temperature, pH, oxygen concentration, glucose feed rate and concentration, and other media conditions. Among the parameters used to determine other process steps, low ambient phosphate concentration was used as a control point that lead to temperature shift from approximately 30°C to approximately 37°C. As noted elsewhere, a promoter sensitive to low ambient phosphate was utilized to control expression of the gene encoding malonyl-CoA reductase in the plasmid identified as pTrc-P_{yibD}-mcr. Also, during one or more evaluations, any one or more of dissolved oxygen, redox potential, aeration rate, agitation rate, oxygen transfer rate, and oxygen utilization rate was/were used to control the system and/or measured.

Strain BX3_547 was evaluated for 36 fermentation events that were conducted over an 8 week period using FM11 medium (described in the Common Methods Section). The duration of the fermentation events were all less than approximately 80 hours, of which a portion was after temperature increase to effectively reduce enzymatic activity of fabl(ts), fabB(ts), and fabD(ts).

Overall the results demonstrated microbial performance over a range of reduced oxygen conditions, with final 3-HP titers ranging between 50 and 62 grams of 3-HP/liter of final culture media volume. It is appreciated that the P_{yibD} promoter, or a similar low-phosphate induction promoter, could be utilized in a genetic construct to induce any one or more of the sequences described and/or taught herein, so as to enable production of any other of the chemical products disclosed herein, including in the other examples above.

### Example 59: 3HP production via malonyl-coA in strains with combinations of changes to increase pyruvate dehydrogenase activity and reduce pathway inhibition.

A strong constitutive promoter, PT5 from the bacteriophage T5 (SEQ ID NO:171), was cloned upstream of the pyruvate dehydrogenase (PDH) operon (aceEF, lpd) on a plasmid using standard molecular biology techniques described elsewhere herein. The entire operon (T5_aceEF-lpd) (SEQ ID NO: 172) was inserted into the puuC locus of *E. coli* deleting the native puuC coding sequence by using homologous recombination techniques to introduce and/or replace portions of the genome described elsewhere herein. This insertion creates a strain with two chromosomal copies of the PDH operon: one of which is transcribed by the native PDH promoter and one that is transcribed by the strong T5 promoter. In addition a mutation was made in some strains (see strain list below) in the lpdA (E354K) protein to cause the pyruvate dehydrogenase complex to be less sensitive to NADH inhibition and active during anaerobic growth (Kim et al. (J. Bacterial. 190:3851-3858, 2008). This mutation was made in either copy of lpd and in both copies creating the strains in Table 44.

**Table 44: Strain List 7**

| **Strain** | **Chromosomal genotype** | **PDH Status** |
|---|---|---|
| 775 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, | aceEF_lpdA= native promoter and protein sequence |
| 770 | F-, Δ(araD-araB)567, ΔlacZ4787(::rrnB-3),LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts)-(A329V), ΔfabF::frt, coaA(R106A), fabD(ts)-(W257Q), ΔaceBAK:frt, lpd(E354K):loxP | aceEF_lpdA (E354K) = native promoter and mutated lpd |
| 801 | F-, Δ(araD-araB)567, ΔlacZ4787(::rrnB-3),LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts)-(A329V), ΔfabF::frt, coaA(R106A), fabD(ts)-(W257Q), ΔaceBAK:frt, ΔpuuC:T5-aceEF-lpd(E354K):loxP | aceEF_lpdA = native promoter and protein sequence plus-ΔpuuC:T5-aceEF-lpd(E354K) |
| 803 | F-, Δ(araD-araB)567, ΔlacZ4787(::rrnB-3),LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts)-(A329V), ΔfabF::frt, coaA(R106A), fabD(ts)-(W257Q), ΔaceBAK:frt, lpd(E354K):loxP, ΔpuuC:T5-aceEF-lpd(E354K):loxP | aceEF_lpdA (E354K) = native promoter and mutated lpd plus-ΔpuuC:T5-aceEF-lpd(E354K) |

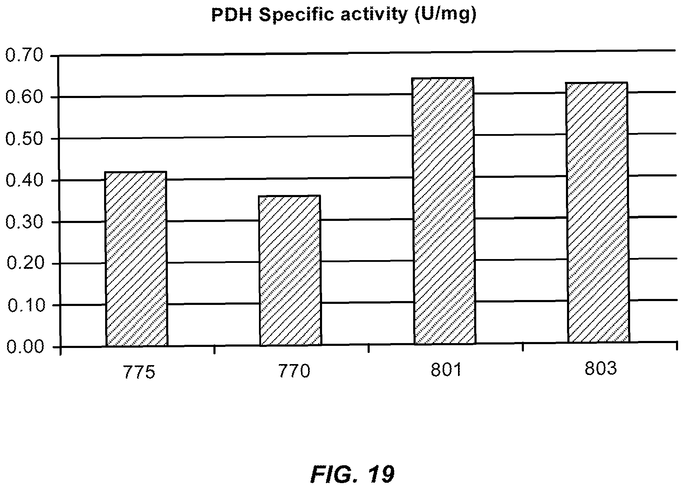

The above strains were evaluated in shake flasks for pyruvate dehydrogenase activity (assay is described in the Common Methods Section). The results in Figure 19 show an increase of PDH activity from ∼0.4 U/mg to >0.6U/mg in strains expressing the PT5-PDH operon over wild type strains.

### Example 60: Construction of a 3HP production strain via malonyl-CoA with combinations of genetic changes to reduce degradation of 3HP, reduce byproduct formation, and utilize sucrose feedstocks.

Strains were designed to reduce or eliminate enzymatic activity through the 3HP degradation pathway or competitive byproduct formation. Modifications were optionally further selected from one or more of the following gene deletions: lactate dehydrogenase (ldhA), pyruvate formate lyase (pflB), methylglyoxyl synthase (mgsA), pyruvate oxidase (poxB), phosphate acetyltransferase (pta), acetate kinase (ackA), aldehyde dehydrogenase A (aldA), acetaldehyde dehydrogenase B (aldB), alcohol dehydrogenase (adhE), γ-glutamyl-γ-aminobutyraldehyde dehydrogenase (puuC), malate synthase (aceB), isocitrate lyase (aceA), isocitrate dehydrogenase (aceK), and KASII (fabF). Additional modifications may include incorporation of additional FAS mutations described in other examples herein, incorporation of the sucrose utilization operon (cscBKA genes) into the aldA locus, incorporation of the PT5-aceEF-lpd operon into the puuC locus described in other examples herein, incorporation of a mutation in pantothenate kinase which is refractory to feedback inhibition (coaA*) (SEQ ID NO 173), and an additional insertion of the PyibD-T7PolI gene into the aldB locus.

The following genetically modified *E. coli* strains (listed in Table 45) were constructed from a wild type BW25113 starting host. *E. coli* BW25113 was obtained from the Yale genetic stock center (New Haven, CT USA). These strains were constructed by standard methods such as discussed in the Common Methods Section and also known in the art as referenced above.

**Table 45: Strain List 8**

| **Strain** | **Chromosomal genotype** | **Plasmid 1** | **Plasmid 2** |
|---|---|---|---|
| 547 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q) ΔaceBAK:frt, | pTRC-kan-PyibD-mcr, | pACYC-Ptpia-accAD-PrpiA- |
| 571 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ApuuC::T5-aceEF-lpd(E354K):loxP, ΔaceBAK:frt, lpd(E354K):loxP, ΔaldB:PyibD-T7pol:loxP, ΔadhE:frt, ΔaldA:cscBKA | pTRC-PyibD-mcr | pACYC-PyibD-accADBC |

### Example 61: Construction of a 3HP production strain via malonyl-CoA that does not require the addition of antibiotics.

Strains were designed to eliminate the need for antibiotic supplementation to the medium to maintain plasmid stability. Modifications to maintain plasmid stability without antibiotics may include auxotrophic markers (gapA) or toxin/ antitoxin systems (ccdAB). The following genetically modified *E. coli* strains (listed in Table 46) were constructed from a wild type BW25113 starting host. *E. coli* BW25113 was obtained from the Yale genetic stock center (New Haven, CT USA) using standard methods as described in other examples herein.

**Table 46: Strain List 9**

| **Strain** | **Chromosomal genotype** | **Plasmid 1** | **Plasmid 2** |
|---|---|---|---|
| 240 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR | pTRC-Ptrc-mcr | pACYC-Ptal-pntAB-Ptpia-accAD-PrpiA-accCD |
| 501 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabIts (S241F)-zeoR, ΔgapA | pTRC-Ptrc-mcr-Pkan-gapA | pACYC-Ptal-pntAB-Ptpia-accAD-PrpiA-accCD-Pkan- |

The above strains were evaluated for production in the absence of antibiotic addition in 1L fermentation systems.

**Table 47: Fermentation metrics for strains with and without antibiotic additions**

| | BX3_240 (with antibiotics) | BX3_240 (without antibiotics) | BX3_501 (without antibiotics) |
|---|---|---|---|
| Max Specific Growth (1/hr) | 0.28 | 0.29 | 0.3 |
| Avg Specific Growth (1/hr) | 0.31 | 0.22 | 0.22 |
| Max Volumetric 3-HP Rate (g/L/hr) | 3.43 | 3.1 | 7.04 |
| Max Specific 3-HP Rate (g/gDCW/hr) | 0.25 | 0.26 | 0.57 |
| Avg Volumetric 3-HP Rate (g/L/hr) | 1.73 | 1.96 | 1.94 |
| Avg Specific 3-HP Rate (g/gDCW/hr) | 0.14 | 0.17 | 0.18 |
| Max Titer (g/L) | | 47.3 | 48.2 |
| Yield During Production | 57 | 55.9 | 56.1 |

### Example 62: Utilization of sucrose as the feedstock for production of 3-HP and other products

### Cloning of csc genes

Common laboratory and industrial strains of *E. coli,* such as the strains described herein, are not capable of utilizing sucrose as the sole carbon source, although this property is found in a number of wild strains, including pathogenic *E. coli* strains. Sucrose, and sucrose-containing feedstocks such as molasses, are abundant and often used as feedstocks for the production by microbial fermentation of organic acids, amino acids, vitamins, and other products. Thus further derivatives of the strains described herein that are capable of utilizing sucrose would expand the range of feedstocks that can be utilized to produce 3-HP and other products.

Various sucrose uptake and metabolism systems are known in the art (for example, U.S. Pat. No. 6,960,455). We describe the construction of *E. coli* strains that harbor the *csc* genes conferring the ability to utilize sucrose via a non-phosphotransferase system, wherein the *csc* genes constitute *cscA*, encoding a sucrose hydrolase, *cscB*, encoding a sucrose permease, *cscK*, encoding a fructokinase, and *cscR,* encoding a repressor. The sequences of these genes are annotated in the NCBI database as accession No. X81461 AF473544. To allow efficient expression utilizing codons that are highly abundant in *E. coli* genes, an operon containing *cscB*, *cscK*, and *cscA* was designed and synthesized using the services of a commercial synthetic DNA provider (DNA 2.0, Menlo Park, CA). The sequences
of the genes are set forth as, respectively, cscB - SEQ. ID. No. 176; cscA - SEQ. ID. No. 177; csck - SEQ. ID. No. 178. The synthetic operon consisted of 60 base pairs of the region of the *E. coli* genome immediately 5' (upstream) of the *adhE* gene, a consensus strong promoter to drive expression of the *csc* genes, the coding regions for *cscB*, *cscK*, and *cscA* with short intergenic regions containing ribosome binding sites but no promoters, and 60 bp immediately 3' (downstream) of the *adhE* gene. The segments homologous to sequences flanking the *adhE* gene may be used to target insertion of the *csc* operon genes into the *E. coli* chromosome, with the concomittent deletion of *adhE.* The nucleotide sequence of the entire synthetic construct is shown as SEQ. ID. No. 179.

The synthetic *csc* operon was constructed in plasmid pJ214 (DNA 2.0, Menlo Park, CA) that provides an origin of replication derived from plasmid p15A and a gene conferring resistance to ampicillin. This plasmid is denoted pSUCR and shown as SEQ. ID No. 213. Transformation of a suitable host cell, such as *E. coli* strain 595, with pSUCR rendered it capable of growth on sucrose as the sole carbon source, where the host bearing a control plasmid without the csc gene cluster was not able to utilize this feedstock.

### Chromosomal integration of csc genes

The *csc* gene cluster was integrated into into the *aldA* locus of *E. coli* to generate strains that stably carried the sucrose utilization trait. The promoter - *csc* region of pSUCR was amplified using primers:
(SEQ ID NO: 214)HL021:
(SEQ ID NO:215)HL022:

### Improving sucrose utilization rates

To increase the utilization of sucrose, the sucrose permease encoded by the *cscB* gene, known to be the slowest step in sucrose utilization, was mutagenized and subjected to selection for increased sucrose growth rates. Mutagenic PCR was used to generate a number of libraries, each of ∼20,000 individuals, with average mutation rates ranging from 2.7 to 17.8 changes per 1000 bases, using the Diversity PCR Random Mutagenesis Kit (Clontech Laboratories, Mountain View, CA). Each library was digested with *Nco*I and *Bgl*II and ligated to pSUCR similarly digested and purified by agarose gel electrophoresis to recover the plasmid fragment from which the parental *cscB* gene was removed. The mutagenic plasmid library was then transformed into Ecloni 10G Elite electrocompetent cells (Lucigen, Madison WI). The mutagenic frequency was determined by sequencing 6 unselected clones from each transformation. Each library was recovered by scraping the colonies off transformation plates and extracting the plasmids with multiple minipreps (Qiagen). The library with the lowest number of changes per 1000 bases was transformed into 595 and the entire transformation mixture passaged repeatedly in SM8 medium containing 30g/L sucrose for 11 transfers. Twelve individual colonies were isolated from this enriched culture and sequenced. Seven (mutants designated A, B, D, E, G, H, I) of the 12 have the identical *cscB* sequence, and carry 3 coding mutations (N234D, I312V, I369V). In addition, mutant J has only the N234D change, while mutant C carries the N234D and I313V changes. In this nomenclature, the first letter denotes the amino acid present in the wildtype protein (using the one-letter code where N is asparagine), the numbers represent the position of the residue in the protein chain, and the second letter denotes the amino acid present in the mutant (where D is aspartic acid). The other clones isolated from this enrichment procedure, although carrying changes in the *cscB* gene, did not grow when re-inoculated into sucrose medium. The sequence of cscB mutant 1A is included as SEQ. ID No. 180. Strains 535 and 536 were constructed from hosts carrying mutant 1A as shown in Table 48 below.

**Table 48: Strain List 10**

| **Strain** | **Chromosomal genotype** | **Plasmid 1** | **Plasmid 2** |
|---|---|---|---|
| 535 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, ΔaldB:cscB(1A)AK | pTRC-Ptrc-mcr | pACYC-Ptal-pntAB-Ptpia-accAD-PrpiA-accCD |
| 536 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, ΔaldB:cscB(1A)AK | pTRC-Ptrc-mcr-glk | pACYC-Ptal-pntAB-Ptpia-accAD-PrpiA-accCD |

### Example 63: 3-HP production via malonyl-CoA in strains with improved acetyl-CoA carboxylase activity

Plasmid map 4 shown below (pACYC-T7-rbs accADBC) was constructed by subcloning the gene synthesized accADBC construct with optimized ribosome binding sites for each subunit (GenScript) (SEQ ID NO: 182) into pACYC-DUET (Novagen) at the EcoICRI and EcorV restriction sites to make the final pACYC-T7-rbs accADBC construct (SEQ ID NO: 183).

Plasmid pACYC-pyibD-rbsaccADBC (SEQ ID NO: 184) shown in Plasmid Map 5 was constructed by replacement of the T7 promoter with the pyibD promoter by conventional cloning methods as described elsewhere herein.

The strains described in Table 49 were evaluated in shake flasks for increased acetyl-CoA carboxylase (AccAse) activity. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 100 mL cultures of each strain in SM11 minimal medium and antibiotics to an OD 600nm = 0.2. Flasks were grown in the 30°C in a shaking incubator. When the cultures reach an OD600 nm = 0.5, the strains were induced by either phosphate depletion or 0.3 mM IPTG and further incubated at 30C. After 3 hours, the cultures were shifted to 37C. Samples were taken for analyses of AccAse enzyme activities after 24 hours as described in the Common Methods Section.

**Table 49: Strain List 11**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, DE3 | pTRC-Ptrc-mcr | pACYC (empty vector) |
| 2 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, DE3 | pTRC-Ptrc-mcr | pACYC-Ptpia-accAD-PrpiA-accBC |
| 3 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, DE3 | pTRC-Ptrc-mcr | pACYC-PT7-accADBC |
| 4 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC:T5-aceEF-lpd*, ΔaceBAK: frt, lpd*:loxP, ΔaldB:PyibD-T7pol:BSD, ΔadhE:frt | pTRC-PyibD-mcr | pACYC-Ptpia-accAD-PrpiA-accBC |
| 5 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC:T5-aceEF-lpd*, ΔaceBAK: frt, lpd*:loxP, ΔaldB:PyibD-T7pol:BSD, ΔadhE:frt | pTRC-PyibD-mcr | pACYC-PyibD-accADBC |

| | | | ACCase activity (umoles/min/mg) |
|---|---|---|---|
| | | | |
| 1 | | | 0.003 |
| 2 | | | 0.109 |
| 3 | | | 0.206 |
| 4 | | | 0.09 |
| 5 | | | 0.22 |

### Example 64: 3-HP production via malonyl-coA in strains with additional combinations of FAS mutations by 1L fermentation

Strains 472 and 479 (Table 50) were constructed by standard molecular biology techniques as described elsewhere herein. Two 1 L fed batch fermentation experiments were carried out using strains 472 and 494. Seed culture was started from 1 ml of glycerol stock for each strain inoculated into 100 mL of TB medium (Terrific Broth) in a corresponding shake flask and incubated at 30°C until the OD₆₀₀ was between 5 and 6. The shake flask culture was used to aseptically inoculate (5% volume/volume) the corresponding 1 L volume bioreactor so that the post-inoculation volume was 800 ml in each vessel. The fermentors used in this experiment were Das Gip fed-batch pro parallel fermentation systems (DASGIP AG, Julich, Germany, model SR0700ODLS). The fermentation system included real-time monitoring and control of dissolved oxygen (%DO), pH, temperature, agitation, and feeding. All fermentors contained defined FM8 medium, made as shown in the Common Methods Section. In each fermentor, the initial temperature was 30°C. Induction was effected by adding IPTG to a final concentration of 2 mM when phosphate is depleted. Glucose feed (consisting of a 500 g/L glucose solution) was initiated to maintain glucose levels between 1-10 g/L. At induction, the temperature was shifted to 37°C over 1 hour. The broth of each fermentor was maintained at a pH of approximately 7.4 by the controlled addition of a pH titrant 50% NH4(OH). At the time the temperature shift was initiated, the DO was changed to 1%. Samples were taken for optical density measurements as well as HPLC analysis for 3-HP concentration. The biomass concentration at harvest as well as the maximum 3-HP titer and specific 3HP rate during production are summarized in the Table 50 below.

**Table 50: Strain List 12**

| **Strain** | **Chromosomal genotype** | **Plasmid 1** | **Plasmid 2** |
|---|---|---|---|
| 472 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt | pTRC-Ptrc-mcr | pACYC-Ptal-pntAB-Ptpia-accAD-PrpiA-accCD |
| 479 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔarcA, lpd*,fabB(ts), | pTRC-Ptrc-mcr-glk | pACYC-Ptal-pntAB-Ptpia-accAD-PrpiA-accCD |

**Table 51**

| Strain | Airflow (vvm) | Agitation During Production (rpm) | Biomass Conc. at Harvest (g DCW/L) | 3HP Titer (g/L) at 40 hrs | Specific 3HP Rate (g/gDCW/hr) over 40 hours |
|---|---|---|---|---|---|
| BX3 472 | 1.2 | 600 | 6.6 | 39.2 | 0.15 |
| BX3 494 | 1.2 | 670 | 10.0 | 55.9 | 0.16 |

### Example 65: 3-HP production via malonyl-CoA using strain 571

Strain 571 was constructed by standard molecular biology techniques as described elsewhere herein. The genotype and corresponding plasmids used to construct 571 are listed in Table 52. 3-HP production by strain 571 is demonstrated at 100-mL scale in SM11 (minimal salts) media. Cultures were started from freezer stocks by standard practice (Sambrook and Russell, 2001) into 50 mL of SM11 media plus 35 µg/mL kanamycin and 20 µg/mL chloramphenicol and grown to stationary phase overnight at 30°C with rotation at 250 rpm. Three mL of this culture was transferred to 100 ml of SM11 media without phosphate plus 30 g/L glucose, 35 µg/ml kanamycin, and 20 µg/mL chloramphenicol in triplicate 250-ml baffled flasks and incubated at 30°C, 250 rpm. The flasks are shifted to 37°C six hours post inoculation. To monitor growth rate, samples (2 ml) are withdrawn at designated time points for optical density measurements at 600nm (OD₆₀₀, 1 cm path length). To monitor 3-HP production by these cultures, samples (10 mL) are pelleted by centrifugation at 12,000rpm for 5 min and the supernatant collected for analysis of 3-HP titer as described under "Analysis of cultures for 3-HP production" in the Common Methods section. Dry cell weight (DCW) is calculated as 0.40 times the measured OD₆₀₀ value, based on baseline DCW to OD₆₀₀ determinations. All data are the average of triplicate cultures. The average specific rate is calculated from the averaged data at the 24-h time point and expressed as g 3-HP produced per gDCW over 16 hours. Production of 3-HP by strain 571 in SM11 medium is shown in Table 52.

**Table 52: Strain List 13**

| **Strain** | **Chromosomal genotype** | **Plasmid 1** | **Plasmid 2** |
|---|---|---|---|
| 571 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ApuuC::T5-aceEF-lpd(E354K):loxP, ΔaceBAK:frt, lpd(E354K):loxP, ΔaldB:PyibD-T7pol:loxP, ΔadhE:frt, ΔaldA:cscBKA | pTRC-PyibD-mcr | pACYC-PyibD-accADBC |

**Table 53. Production of 3-HP by 571 in SM11 medium**

| Time (hr) | 3HP (g/L) | Specific Rate g 3HP/gDCW/hr |
|---|---|---|
| 6 | 0 | 0 |
| 10 | 0 | 0 |
| 24 | 1.8 | 0.37 |

### Example 66: Construction of strains carrying the glutamate dehydrogenase from the Antarctic psychrotolerant bacterium Psychrobacter sp. TAD1 and deletion of glutamate synthase function.

Deletions and replacements of genes within strains were carried out using the Red/ET Recombination system commercially available from Gene Bridges (Heidelberg, Germany) by following the manufactures instructions. For deletion of the gdhA gene within strains, a kanamycin carrying cassette for the deletion of gdhA was created by polymerase chain reaction using genomic DNA from the Keio collection (Baba et al, 2000) knockout strain for gdhA as template, using the forward primer (SEQ ID NO: 185) and reverse primer (SEQ ID NO: 186) for the reaction. For deletion of the gltB gene within strains, a kanamycin carrying cassette for the deletion of gltB was created by polymerase chain reaction using genomic DNA from the Keio collection (Baba et al, 2000) knockout strain for gltB as template, using the forward primer (SEQ ID NO: 187) and reverse primer (SEQ ID NO: 188) for the reaction. For deletion of the gltD gene within strains, a kanamycin carrying cassette for the deletion of gltD was created by polymerase chain reaction using genomic DNA from the Keio collection (Baba et al, 2000) knockout strain for gltD as template, using the forward primer (SEQ ID NO: 189) and reverse primer (SEQ ID NO: 190) for the reaction. After insertion of any of these deletion cassettes, the antibiotic marker was removed from the genome-integrated strain using FLP-mediated site specific recombination via the 708-FLPe, cm expression plasmid with chloramphenicol resistance marker from Gene Bridges (Heidelberg, Germany) using the manufactures instructions.

Replacement of the *E.coli* gdhA gene with the glutamate dehydrogenase gdh gene from the Antarctic psychrotolerant bacterium *Psychrobacter sp. TAD1* was performed using the using the Red/ET Recombination system commercially available from Gene Bridges (Heidelberg, Germany) by following the manufactures instructions. For these replacements, the *E.coli* gdhA gene was replaced with the *Psychrobacter sp. TAD1* gdh gene using a replacement cassette produced via a polymerase chain reaction. The template for the polymerase chain reaction was produced using the gene synthesis servicers of GenScript (Piscataway, NJ). Additional, the gdh gene from the Antarctic psychrotolerant bacterium *Psychrobacter sp. TAD1* was codon optimized using methods developed by GenScript. The gene synthesized cassette contained the codon optimized gdh gene, a blasticidin resistance selection marker, and homology regions targeted toward the upstream and downstream regions of *the E.coli* gdhA genomic region. The sequence of the plasmid provided by GenScript carrying this replacement cassette is provided as (SEQ ID NO: 191). This replacement cassette was amplified with that forward primer (SEQ ID NO: 185) and reverse primer (SEQ ID NO: 186) for the polymerase chain reaction and inserted into strains using the Gene Bridges method.

Using these various deletion and replacement cassettes, the several strains were created as shown in the Table 54 below using strain 822 as the parent strain.

**Table 54: Strain List 14**

| **Strain** | **Chromosomal genotype** |
|---|---|
| 822 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD- |
| 841 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-Ipd*::loxP, ΔaceBAK::frt, lpd*::loxP, ΔaldB::PyibD- |
| 842 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-Ipd*::loxP, ΔaceBAK::frt, lpd*::loxP, ΔaldB::PyibD- |
| 853 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-Ipd*::loxP, ΔaceBAK::frt, lpd*::loxP, ΔaldB::PyibD-T7pol::loxP, ΔadhE::frt, ΔaldA::CSC, gdhA(ts)::BSD |
| 844 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-Ipd*::loxP, ΔaceBAK::frt, lpd*::loxP, ΔaldB::PyibD- |

### Example 67: Evaluation of strains with controllable glutamate production using the Antarctic psychrotolerant bacterium Psychrobacter sp. TAD1

In order to evaluate the ability to control glutamate production, cultures of various strains were grown at 30 degrees Celsius for 8 hours and then shifted to 37 degrees Celsius and 40 degrees Celsius and grown for an additional 16hr. At these increased temperatures, the activity of the Antarctic psychrotolerant bacterium *Psychrobacter sp. TAD1* gdh gene should lower significantly as compared to the levels detected at 37C. Activities were measured as described in the Common Methods Section. The result of this experiment is shown in Figures 26-28. Specific activities were calculated from cultures of strains carrying the *Psychrobacter sp. TAD1* gdh gene alone (strain 853) or the gltB deletion and *Psychrobacter sp. TAD1* gdh gene in combination (strain 842). Upon the shift and growth at 37 degrees Celsius and 40 degrees Celsius, this activity decreased significantly. This result shows that the *Psychrobacter sp. TAD1* gdh gene product is inhibited at these temperatures.

### Example 68: Increased production rates, titers, and yields using strains carrying glutamate dehydrogenase from the Antarctic psychrotolerant bacterium Psychrobacter sp. TAD1 and deletion of glutamate synthase function.

The strains listed in Table 55 below were created to evaluate glutamate/glutamine production in a 3-HP production strain.

**Table 55: Strain List 15**

| **Strain** | **Chromosomal genotype** | **Plasmid 1** | **Plasmid 2** |
|---|---|---|---|
| 579 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-Ipd*::loxP, ΔaceBAK::frt, lpd*::loxP, ΔaldB::PyibD-T7pol::loxP, ΔadhE::frt, ΔaldA::CSC | pTRC-PyibD-mcr(st)-PyibD-mmsB | pACYC-PyibD-accADBC |
| 593 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-Ipd*::loxP, ΔaceBAK::frt, lpd*::loxP, ΔaldB::PyibD-T7pol::loxP, ΔadhE::frt, ΔaldA::CSC, ΔgltB::frt, gdhA(ts)::BSD | pTRC-PyibD-mcr(st)-PyibD-mmsB | pACYC-PyibD-accADBC |

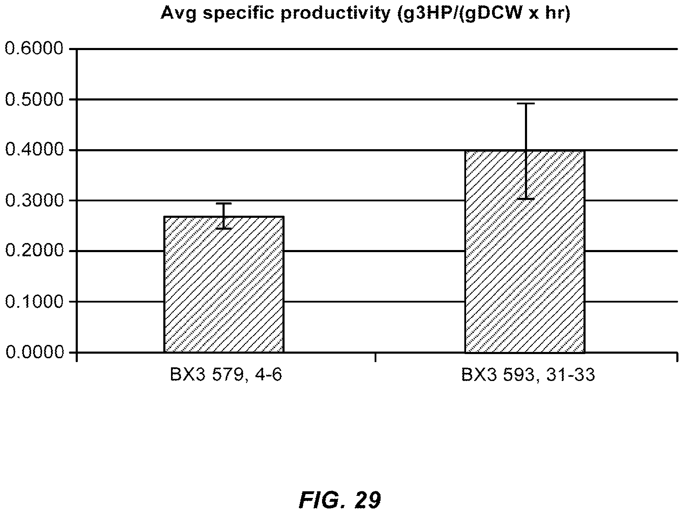

These strains were grown in shake flasks at 30 degree Celsius for 6 hours in SM11 media without phosphate to induce protein production for the production pathway. At 6 hours, the cultures were shifted to 37 degrees Celsius to shut down fatty acid production and lower the glutamate dehydrogenase activity. The results for these shake flasks are shown Figure 29. This experiment shows that strain 593 showed an average specific productivity that was greater strain 579.

### Example 69: Alternative method for limiting or controlling glutamate production.

Other routes for limiting glutamate production include 1) deleting the glutamate dehydrogenase gene or 2) deleting the glutamate dehydrogenase gene and regulating expression of glutamate synthase genes or gene products . In this embodiment, the regulation of gltB or gltD could be controlled altering expression as is common in the art or alternatively by using a temperature sensitive version of the glutamate synthase as described above. Such temperature sensitive versions of the gltB and gltD could be isolated in a manner similar to those used by Dendinger et al. (1980) to isolate temperature-sensitive versions of the Salmonella gdhA gene. Alternatively, other methods using sequence and structural-based information could be used to create variants of gltB, gltD, or gdhA genes similar to those described in Chakshusmathi et al (2004).

### Example 70: Construction of the Sulfolobus tokodaii mcr gene for expression

The MCR gene from *Sulfolobus tokodaii* was synthesized using the services of GenScript (Piscataway, NJ) using GenScripts' codon optimization methods for expression in E. coli. This gene was synthesized with a Ptrc promoter and was designated pUC57-Ptrc-StMCR (SEQ ID NO: 192).

### Example 71: Biochemical assays to measure Sulfolobus tokodaii MCR activity and combining malonyl-CoA reductase and 3-HP dehydrogenase activities

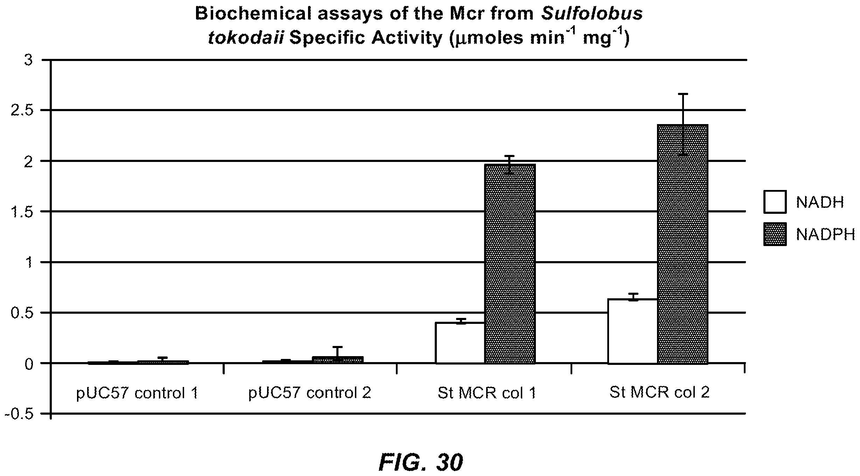

In order to evaluate the use *Sulfolobus tokodaii* MCR, lysates for assaying the specific activities were prepared from over expressed cultures. The results for the specific activity of *Sulfolobus tokodaii* MCR using NADH and NADPH cofactors, independently, are shown in Figure 30. In this experiment, culture begun from 2 different colonies for E. coli cells carrying either the pUC57 control plasmid or the pUC57-Ptrc-StMCR plasmid able to overexpress *Sulfolobus tokodaii* MCR. This experiment show no detectable malonyl CoA reductase activity in the two control colonies tested, and shows robust malonyl CoA specific activity in the culture overexpressing *Sulfolobus tokodaii* MCR. The activity with NADPH in these lysates was at least 1.9 units per mg lysate in these experiments, and agrees well for the reported specific activity of 44 units per mg for the purified proteins as evaluated by Alber et al. (2006). In addition to NADPH cofactor, NADH was also assessed as a potential cofactor even though no such activity was previous reported. The activity of *Sulfolobus tokodaii* MCR was only 4.2 less active than the specific activity with NADPH. Accordingly, this activity and this only mild preference for NADPH potentially make this enzyme more suitable than other malonyl CoA reductases depending on the fermentation process used.

The results of these experiments are shown in Figure 30. These results demonstrate that *Sulfolobus tokodaii* MCR can utilize NADH as a cofactor as well as the previously reported NADPH cofactor. None of the samples evaluating the dehydrogenases or *Sulfolobus tokodaii* MCR, independently, show significant 3HP formation. Conversely, reactions contain the combination *Sulfolobus tokodaii* MCR and any of the dehydrogenase were able to make significant amounts of 3HP. The reaction containing *Sulfolobus tokodaii* MCR and the E.coli ydfG overexpressing lysate showed less production of 3HP. Since *E.coli* ydfG has a strong preference for NADPH, this result shows how different combinations of malonyl coA reductase domains and dehydrogenase domains with various preferences for either NADPH or NADH could be exploited to yield better production characteristics depending on the fermentation conditions which are known to influence the ratios and amounts of NADPH and NADH within cells.

Production of 3-HP from malonyl-CoA can in addition be achieved with a NADH-dependent malonyl-CoA reductase activity and an activity that converts malonyl semialdehyde to 3-HP using a biological reductant other than NADPH or NADH, such as the activity encoded by the *rutE* gene of *E. coli* or by the *nemA* gene of *E. coli* which are reductases that utilize a flavin derivative as the reductant and which further require the activity of a function such as the *fre* gene product encoding FMN reductase to regenerate the reductant. See, for example, Kim et al., 2010, J. Bacteriol. 192(16): 4089-4102.

Combinations of a gene encoding malonyl-CoA reductase activity and a gene or genes encoding 3-HP dehydrogenase can be achieved by cloning the respective genes behind promoters such that the genes are operably expressed in the microorganism under conditions that induce expression. The genes may be cloned in plasmid vectors, such as plasmids based on the ColE1 replication or the p15A replication, or may be inserted into the chromosome of the microorganism, such as at a locus which encodes a dispensable function, for example the *aldA* locus of *E. coli.* Expression of these genes can be driven by regulated promoters, such as the *lac* promoter or derivatives thereof, or by the T7 bacteriophage promoter, or by the arabinose promoter, or other DNA sequences known or found to drive expression in *E. coli.* These examples of constructs and promoters are not meant to be limiting.

### Example 72: Production of 3HP and other products from Xylose

3HP production using xylose by strain 240, described elsewhere herein, is demonstrated at 100-mL scale in SM11 (minimal salts) media made without glucose. Cultures are started from freezer stocks by standard practice (Sambrook and Russell, 2001) into 50 mL of TB media plus 35 µg/mL kanamycin and 20 µg/mL chloramphenicol and grown to stationary phase overnight at 30°C with rotation at 250 rpm. 5 mL of this culture is transferred to 100 ml of SM11 media made without glucose but with 30 g/L xylose, 35 µg/ml kanamycin, 20 µg/mL chloramphenicol, and 1 mM IPTG in triplicate 250-ml baffled flasks and incubated at 30°C, 250 rpm. The flasks are shifted to 37°C four hours post inoculation. To monitor growth rate, samples (2 ml) are withdrawn at designated time points for optical density measurements at 600nm (OD₆₀₀, 1 cm path length). To monitor 3HP production by these cultures, samples are pelleted by centrifugation at 12,000rpm for 5 min and the supernatant collected for analysis of 3-HP titer as described under "Analysis of cultures for 3-HP production" in the Common Methods section. Dry cell weight (DCW) is calculated as 0.40 times the measured OD₆₀₀ value, based on baseline DCW to OD₆₀₀ determinations. All data are the average of triplicate cultures. The average specific productivity is calculated from the averaged data at the 24-h time point and expressed as g 3-HP produced per gDCW. Glucose or xylose concentrations can be determined in g/L using appropriate sensors such as those from YSI Incorporated, and the yield is calculated using the averaged data at the 24-hour time point.

### Example 73: Production of 3HP and other products via malonyl-CoA in C. necator hosts with combinations of FAS mutations from Syngas

The following homologues were identified for E. coli FAS enzymes in C. necator.

**Table 56: FAS homologues in C. necator**

| Enzyme Function | Homologue in C. necator | % Identity | e_ value |
|---|---|---|---|
| MALONYL-COA-ACP- TRANSACYL-MONOMER | gi\|113868530\|ref\|YP_727019.1\| | 59.61 | 2.00E-95 |
| FABH-MONOMER | gi\|113868531\|ref\|YP_727020.1\| | 51.7 | 6.00E-93 |
| FABB-MONOMER | gi\|113868527\|ref\|YP_727016.1\| | 38.2 | 3.00E-62 |
| FABB-MONOMER | gi\|116695606\|ref\|YP_841182.1\| | 30.96 | 1.00E-14 |
| 3-OXOACYL-ACP-SYNTHII-MONOMER | gi\|113868527\|ref\|YP_727016.1\| | 63.11 | 2.00E-151 |
| 3-OXOACYL-ACP-SYNTHII-MONOMER | gi\|116695606\|ref\|YP_841182.1\| | 38.7 | 5.00E-28 |
| ENOYL-ACP-REDUCT-NADH-MONOMER | gi\|113868381\|ref\|YP_726870.1\| | 62.06 | 6.00E-91 |
| ENOYL-ACP-REDUCT-NADH-MONOMER | gi\|38637922\|ref\|NP_942896.1\| | 57.37 | 5.00E-83 |
| ENOYL-ACP-REDUCT-NADH-MONOMER | gi\|116695568\|ref\|YP_841144.1\| | 44.71 | 2.00E-57 |
| ENOYL-ACP-REDUCT-NADH-MONOMER | gi\|113866900\|ref\|YP_725389.1\| | 28.52 | 5.00E-17 |
| ENOYL-ACP-REDUCT-NADH-MONOMER | gi\|113869529\|ref\|YP_728018.1\| | 27.95 | 4.00E-14 |
| ENOYL-ACP-REDUCT-NADH-MONOMER | gi\|116694061\|ref\|YP_728272.1\| | 29.3 | 5.00E-14 |
| ENOYL-ACP-REDUCT-NADH-MONOMER | gi\|113866064\|ref\|YP _724553.1\| | 25.87 | 1.00E-11 |
| ENOYL-ACP-REDUCT-NADH-MONOMER | gi\|116694602\|ref\|YP_728813.1\| | 24.16 | 4.00E-11 |
| ENOYL-ACP-REDUCT-NADH-MONOMER | gi\|116695241\|ref\|YP_840817.1\| | 26.36 | 6.00E-11 |
| ENOYL-ACP-REDUCT-NADH-MONOMER | gi\|116695184\|ref\|YP_840760.1\| | 24.32 | 2.00E-10 |
| ENOYL-ACP-REDUCT-NADH-MONOMER | gi\|113869434\|ref\|YP_727923.1\| | 27.41 | 6.00E-10 |
| ENOYL-ACP-REDUCT-NADH-MONOMER | gi\|116696275\|ref\|YP_841851.1\| | 24.52 | 9.00E-10 |
| ENOYL-ACP-REDUCT-NADH-MONOMER | gi\|116695085\|ref\|YP_840661.1\| | 30.7 | 1.00E-08 |
| ENOYL-ACP-REDUCT-NADH-MONOMER | gi\|116694581\|ref\|YP_728792.1\| | 22.1 | 2.00E-08 |
| ENOYL-ACP-REDUCT-NADH-MONOMER | gi\|116695770\|ref\|YP_841346.1\| | 26.62 | 5.00E-08 |
| ENOYL-ACP-REDUCT-NADH-MONOMER | gi\|113867286\|ref\|YP_725775.1\| | 26.72 | 9.00E-08 |
| ACYLCOASYN-MONOMER | gi\|113869241\|ref\|YP_727730.1\| | 56.96 | 0 |
| ACYLCOASYN-MONOMER | gi\|113868752\|ref\|YP_727241.1\| | 33.87 | 2.00E-77 |
| ACYLCOASYN-MONOMER | gi\|113867951\|ref\|YP_726440.1\| | 32.18 | 6.00E-76 |
| ACYLCOASYN-MONOMER | gi\|113869452\|ref\|YP_727941.1\| | 33.27 | 1.00E-71 |
| ACYLCOASYN-MONOMER | gi\|113867314\|ref\|YP_725803.1\| | 31.54 | 1.00E-68 |
| ACYLCOASYN-MONOMER | gi\|113868228\|ref\|YP_726717.1\| | 30.87 | 1.00E-62 |
| ACYLCOASYN-MONOMER | gi\|116694647\|ref\|YP_728858.1\| | 30.05 | 4.00E-62 |
| ACYLCOASYN-MONOMER | gi\|113868902\|ref\|YP_727391.1\| | 29.93 | 9.00E-62 |
| ACYLCOASYN-MONOMER | gi\|113866897\|ref\|YP_725386.1\| | 31.14 | 2.00E-60 |
| ACYLCOASYN-MONOMER | gi\|113868933\|ref\|YP_727422.1\| | 31.07 | 2.00E-59 |
| ACYLCOASYN-MONOMER | gi\|116695183\|ref\|YP_840759.1\| | 31.07 | 1.00E-58 |
| ACYLCOASYN-MONOMER | gi\|116694856\|ref\|YP_729067.1\| | 31.91 | 2.00E-58 |
| ACYLCOASYN-MONOMER | gi\|113866865\|ref\|YP_725354.1\| | 30.11 | 4.00E-58 |
| ACYLCOASYN-MONOMER | gi\|116694595\|ref\|YP_728806.1\| | 28.73 | 1.00E-56 |
| ACYLCOASYN-MONOMER | gi\|116694129\|ref\|YP_728340.1\| | 30.91 | 2.00E-53 |
| ACYLCOASYN-MONOMER | gi\|116695208\|ref\|YP_840784.1\| | 31.88 | 2.00E-52 |
| ACYLCOASYN-MONOMER | gi\|113868706\|ref\|YP_727195.1\| | 28.86 | 1.00E-50 |
| ACYLCOASYN-MONOMER | gi\|116695648\|ref\|YP_841224.1\| | 30.71 | 2.00E-50 |
| ACYLCOASYN-MONOMER | gi\|116694665\|ref\|YP_728876.1\| | 29.49 | 4.00E-50 |
| ACYLCOASYN-MONOMER | gi\|113867426\|ref\|YP_725915.1\| | 27.46 | 2.00E-49 |
| ACYLCOASYN-MONOMER | gi\|116695854\|ref\|YP_841430.1\| | 28.35 | 4.00E-48 |
| ACYLCOASYN-MONOMER | gi\|116694628\|ref\|YP_728839.1\| | 28.86 | 8.00E-48 |
| ACYLCOASYN-MONOMER | gi\|113868764\|ref\|YP_727253.1\| | 29.61 | 2.00E-47 |
| ACYLCOASYN-MONOMER | gi\|116695316\|ref\|YP_840892.1\| | 29.1 | 2.00E-46 |
| ACYLCOASYN-MONOMER | gi\|116695341\|ref\|YP_840917.1\| | 28.8 | 5.00E-46 |
| ACYLCOASYN-MONOMER | gi\|113866892\|ref\|YP_725381.1\| | 32.96 | 4.00E-45 |
| ACYLCOASYN-MONOMER | gi\|113868055\|ref\|YP_726544.1\| | 26.24 | 1.00E-44 |
| ACYLCOASYN-MONOMER | gi\|113867721\|ref\|YP_726210.1\| | 28.71 | 3.00E-44 |
| ACYLCOASYN-MONOMER | gi\|116696458\|ref\|YP_842034.1\| | 30.25 | 7.00E-43 |
| ACYLCOASYN-MONOMER | gi\|113867704\|ref\|YP_726193.1\| | 28.46 | 2.00E-42 |
| ACYLCOASYN-MONOMER | gi\|113868126\|ref\|YP_726615.1\| | 26.92 | 1.00E-41 |
| ACYLCOASYN-MONOMER | gi\|116695632\|ref\|YP_841208.1\| | 27.15 | 3.00E-40 |
| ACYLCOASYN-MONOMER | gi\|113868425\|ref\|YP_726914.1\| | 26.74 | 2.00E-39 |
| ACYLCOASYN-MONOMER | gi\|38638060\|ref\|NP_943034.1\| | 28.21 | 4.00E-39 |
| ACYLCOASYN-MONOMER | gi\|116694704\|ref\|YP_728915.1\| | 27.64 | 5.00E-39 |
| ACYLCOASYN-MONOMER | gi\|113869299\|ref\|YP_727788.1\| | 26.4 | 5.00E-38 |
| ACYLCOASYN-MONOMER | gi\|116695047\|ref\|YP_840623.1\| | 27.19 | 3.00E-35 |
| ACYLCOASYN-MONOMER | gi\|116695279\|ref\|YP_840855.1\| | 25.14 | 3.00E-35 |
| ACYLCOASYN-MONOMER | gi\|113867249\|ref\|YP_725738.1\| | 31.28 | 5.00E-35 |
| ACYLCOASYN-MONOMER | gi\|113867217\|ref\|YP_725706.1\| | 25.5 | 9.00E-35 |
| ACYLCOASYN-MONOMER | gi\|113867530\|ref\|YP_726019.1\| | 25.05 | 6.00E-33 |
| ACYLCOASYN-MONOMER | gi\|116695093\|ref\|YP_840669.1\| | 27.74 | 2.00E-32 |
| ACYLCOASYN-MONOMER | gi\|38638059\|ref\|NP_943033.1\| | 25.54 | 2.00E-31 |
| ACYLCOASYN-MONOMER | gi\|113867503\|ref\|YP_725992.1\| | 44.52 | 4.00E-28 |
| ACYLCOASYN-MONOMER | gi\|116696036\|ref\|YP_841612.1\| | 28.86 | 8.00E-28 |
| ACYLCOASYN-MONOMER | gi\|116694780\|ref\|YP_728991.1\| | 25.66 | 8.00E-27 |
| ACYLCOASYN-MONOMER | gi\|113868491\|ref\|YP_726980.1\| | 25.66 | 9.00E-27 |
| ACYLCOASYN-MONOMER | gi\|116695672\|ref\|YP_841248.1\| | 24.91 | 9.00E-25 |
| ACYLCOASYN-MONOMER | gi\|113867627\|ref\|YP_726116.1\| | 25.04 | 2.00E-22 |
| ACYLCOASYN-MONOMER | gi\|116695626\|ref\|YP_841202.1\| | 25.5 | 4.00E-21 |
| ACYLCOASYN-MONOMER | gi\|116695626\|ref\|YP_841202.1\| | 23.85 | 4.00E-15 |
| ACYLCOASYN-MONOMER | gi\|113868430\|ref\|YP_726919.1\| | 24.06 | 6.00E-20 |
| ACYLCOASYN-MONOMER | gi\|113866315\|ref\|YP_724804.1\| | 23.54 | 3.00E-15 |
| ACYLCOASYN-MONOMER | gi\|116695622\|ref\|YP_841198.1\| | 23.26 | 1.00E-13 |
| ACYLCOASYN-MONOMER | gi\|116695624\|ref\|YP_841200.1\| | 27.13 | 1.00E-12 |
| ACYLCOASYN-MONOMER | gi\|116695625\|ref\|YP_841201.1\| | 23.55 | 4.00E-10 |
| 3-OXOACYL-ACP-REDUCT-MONOMER | gi\|113868529\|ref\|YP_727018.1\| | 63.52 | 9.00E-86 |
| 3-OXOACYL-ACP-REDUCT-MONOMER | gi\|113867453\|ref\|YP_725942.1\| | 41.7 | 5.00E-51 |
| 3-OXOACYL-ACP-REDUCT-MONOMER | gi\|113867981\|ref\|YP_726470.1\| | 40.41 | 2.00E-50 |
| 3-OXOACYL-ACP-REDUCT-MONOMER | gi\|113868147\|ref\|YP_726636.1\| | 43.4 | 1.00E-48 |
| 3-OXOACYL-ACP-REDUCT-MONOMER | gi\|116695840\|ref\|YP_841416.1\| | 40.66 | 3.00E-47 |
| 3-OXOACYL-ACP-REDUCT-MONOMER | gi\|113869118\|ref\|YP_727607.1\| | 38.62 | 2.00E-43 |
| 3-OXOACYL-ACP-REDUCT-MONOMER | gi\|116696446\|ref\|YP_842022.1\| | 43.1 | 4.00E-42 |
| 3-OXOACYL-ACP-REDUCT-MONOMER | gi\|116694315\|ref\|YP_728526.1\| | 38.89 | 2.00E-41 |
| 3-OXOACYL-ACP-REDUCT-MONOMER | gi\|116694338\|ref\|YP_728549.1\| | 36.8 | 3.00E-39 |
| 3-OXOACYL-ACP-REDUCT-MONOMER | gi\|113867286\|ref\|YP_725775.1\| | 38.62 | 5.00E-38 |
| 3-OXOACYL-ACP-REDUCT-MONOMER | gi\|116695278\|ref\|YP_840854.1\| | 37.55 | 9.00E-36 |
| 3-OXOACYL-ACP-REDUCT-MONOMER | gi\|113867797\|ref\|YP_726286.1\| | 36.86 | 2.00E-35 |
| 3-OXOACYL-ACP-REDUCT-MONOMER | gi\|116695770\|ref\|YP_841346.1\| | 35.27 | 7.00E-35 |
| 3-OXOACYL-ACP-REDUCT-MONOMER | gi\|116694022\|ref\|YP_728233.1\| | 33.88 | 7.00E-35 |
| 3-OXOACYL-ACP-REDUCT-MONOMER | gi\|116695384\|ref\|YP_840960.1\| | 36.96 | 1.00E-34 |
| 3-OXOACYL-ACP-REDUCT-MONOMER | gi\|113867306\|ref\|YP_725795.1\| | 35.06 | 1.00E-34 |
| 3-OXOACYL-ACP-REDUCT-MONOMER | gi\|116695635\|ref\|YP_841211.1\| | 40.68 | 1.00E-32 |
| 3-OXOACYL-ACP-REDUCT-MONOMER | gi\|113867542\|ref\|YP_726031.1\| | 34.12 | 1.00E-32 |
| 3-OXOACYL-ACP-REDUCT-MONOMER | gi\|113867353\|ref\|YP_725842.1\| | 31.64 | 1.00E-31 |
| 3-OXOACYL-ACP-REDUCT-MONOMER | gi\|116695184\|ref\|YP_840760.1\| | 33.33 | 5.00E-31 |
| 3-OXOACYL-ACP-REDUCT-MONOMER | gi\|116694602\|ref\|YP_728813.1\| | 32.66 | 9.00E-31 |
| 3-OXOACYL-ACP-REDUCT-MONOMER | gi\|113868128\|ref\|YP_726617.1\| | 33.46 | 2.00E-30 |
| 3-OXOACYL-ACP-REDUCT-MONOMER | gi\|116695020\|ref\|YP_840596.1\| | 34.17 | 7.00E-30 |
| 3-OXOACYL-ACP-REDUCT-MONOMER | gi\|116695668\|ref\|YP_841244.1\| | 30.95 | 3.00E-29 |
| 3-OXOACYL-ACP-REDUCT-MONOMER | gi\|116694617\|ref\|YP_728828.1\| | 33.78 | 4.00E-29 |
| 3-OXOACYL-ACP-REDUCT-MONOMER | gi\|116694552\|ref\|YP_728763.1\| | 32.8 | 4.00E-29 |
| 3-OXOACYL-ACP-REDUCT-MONOMER | gi\|116696275\|ref\|YP_841851.1\| | 35.25 | 5.00E-29 |
| 3-OXOACYL-ACP-REDUCT-MONOMER | gi\|113868428\|ref\|YP_726917.1\| | 34.18 | 9.00E-29 |
| 3-OXOACYL-ACP-REDUCT-MONOMER | gi\|113867344\|ref\|YP_725833.1\| | 30.86 | 1.00E-28 |
| 3-OXOACYL-ACP-REDUCT-MONOMER | gi\|116695241\|ref\|YP_840817.1\| | 33.74 | 3.00E-28 |
| 3-OXOACYL-ACP-REDUCT-MONOMER | gi\|116695722\|ref\|YP_841298.1\| | 33.88 | 5.00E-28 |
| 3-OXOACYL-ACP-REDUCT-MONOMER | gi\|116694061\|ref\|YP_728272.1\| | 37.6 | 2.00E-27 |
| 3-OXOACYL-ACP-REDUCT-MONOMER | gi\|113866956\|ref\|YP_725445.1\| | 33.59 | 7.00E-27 |
| 3-OXOACYL-ACP-REDUCT-MONOMER | gi\|113868440\|ref\|YP_726929.1\| | 36.14 | 1.00E-26 |
| 3-OXOACYL-ACP-REDUCT-MONOMER | gi\|116694682\|ref\|YP_728893.1\| | 32.94 | 1.00E-26 |
| 3-OXOACYL-ACP-REDUCT-MONOMER | gi\|113866770\|ref\|YP_725259.1\| | 33.2 | 4.00E-26 |
| 3-OXOACYL-ACP-REDUCT-MONOMER | gi\|116694156\|ref\|YP_728367.1\| | 33.05 | 1.00E-25 |
| 3-OXOACYL-ACP-REDUCT-MONOMER | gi\|113867502\|ref\|YP_725991.1\| | 31.02 | 5.00E-24 |
| 3-OXOACYL-ACP-REDUCT-MONOMER | gi\|113866875\|ref\|YP_725364.1\| | 34.93 | 7.00E-24 |
| 3-OXOACYL-ACP-REDUCT-MONOMER | gi\|116694685\|ref\|YP_728896.1\| | 30.59 | 2.00E-23 |
| 3-OXOACYL-ACP-REDUCT-MONOMER | gi\|116695830\|ref\|YP_841406.1\| | 31.45 | 5.00E-23 |
| 3-OXOACYL-ACP-REDUCT-MONOMER | gi\|116694347\|ref\|YP_728558.1\| | 30.52 | 7.00E-23 |
| 3-OXOACYL-ACP-REDUCT-MONOMER | gi\|116695926\|ref\|YP_841502.1\| | 31.2 | 8.00E-23 |
| 3-OXOACYL-ACP-REDUCT-MONOMER | gi\|116694593\|ref\|YP_728804.1\| | 31.15 | 2.00E-22 |
| 3-OXOACYL-ACP-REDUCT-MONOMER | gi\|116694550\|ref\|YP_728761.1\| | 30.36 | 2.00E-22 |
| 3-OXOACYL-ACP-REDUCT-MONOMER | gi\|113869434\|ref\|YP_727923.1\| | 30.04 | 9.00E-22 |
| 3-OXOACYL-ACP-REDUCT-MONOMER | gi\|116694600\|ref\|YP_728811.1\| | 28.99 | 2.00E-21 |
| 3-OXOACYL-ACP-REDUCT-MONOMER | gi\|113866064\|ref\|YP_724553.1\| | 27.2 | 4.00E-21 |
| 3-OXOACYL-ACP-REDUCT-MONOMER | gi\|116694638\|ref\|YP_728849.1\| | 33.13 | 2.00E-20 |
| 3-OXOACYL-ACP-REDUCT-MONOMER | gi\|116694259\|ref\|YP_728470.1\| | 35.18 | 3.00E-20 |
| 3-OXOACYL-ACP-REDUCT-MONOMER | gi\|116694664\|ref\|YP_728875.1\| | 30.43 | 9.00E-20 |
| 3-OXOACYL-ACP-REDUCT-MONOMER | gi\|113867940\|ref\|YP_726429.1\| | 32.56 | 1.00E-19 |
| 3-OXOACYL-ACP-REDUCT-MONOMER | gi\|116695703\|ref\|YP_841279.1\| | 30.92 | 1.00E-19 |
| 3-OXOACYL-ACP-REDUCT-MONOMER | gi\|116695910\|ref\|YP_841486.1\| | 31.44 | 2.00E-19 |
| 3-OXOACYL-ACP-REDUCT-MONOMER | gi\|116695451\|ref\|YP_841027.1\| | 31.77 | 4.00E-19 |
| 3-OXOACYL-ACP-REDUCT-MONOMER | gi\|116695846\|ref\|YP_841422.1\| | 34.36 | 4.00E-19 |
| 3-OXOACYL-ACP-REDUCT-MONOMER | gi\|116694614\|ref\|YP_728825.1\| | 31.15 | 6.00E-19 |
| 3-OXOACYL-ACP-REDUCT-MONOMER | gi\|116696432\|ref\|YP_842008.1\| | 27.73 | 1.00E-18 |
| 3-OXOACYL-ACP-REDUCT-MONOMER | gi\|113867868\|ref\|YP_726357.1\| | 29.8 | 1.00E-18 |
| 3-OXOACYL-ACP-REDUCT-MONOMER | gi\|113866900\|ref\|YP_725389.1\| | 30 | 7.00E-18 |
| 3-OXOACYL-ACP-REDUCT-MONOMER | gi\|113869529\|ref\|YP_728018.1\| | 31.02 | 1.00E-17 |
| 3-OXOACYL-ACP-REDUCT-MONOMER | gi\|116695085\|ref\|YP_840661.1\| | 29.03 | 1.00E-16 |
| 3-OXOACYL-ACP-REDUCT-MONOMER | gi\|116695734\|ref\|YP_841310.1\| | 31.95 | 1.00E-16 |
| 3-OXOACYL-ACP-REDUCT-MONOMER | gi\|113866629\|ref\|YP_725118.1\| | 27.95 | 6.00E-16 |
| 3-OXOACYL-ACP-REDUCT-MONOMER | gi\|116695726\|ref\|YP_841302.1\| | 31.89 | 1.00E-15 |
| 3-OXOACYL-ACP-REDUCT-MONOMER | gi\|116694599\|ref\|YP_728810.1\| | 31.84 | 1.00E-15 |
| 3-OXOACYL-ACP-REDUCT-MONOMER | gi\|116694585\|ref\|YP_728796.1\| | 30.42 | 2.00E-15 |
| 3-OXOACYL-ACP-REDUCT-MONOMER | gi\|116694581\|ref\|YP_728792.1\| | 28.14 | 2.00E-15 |
| 3-OXOACYL-ACP-REDUCT-MONOMER | gi\|116695741\|ref\|YP_841317.1\| | 30.9 | 4.00E-14 |
| 3-OXOACYL-ACP-REDUCT-MONOMER | gi\|116694341\|ref\|YP_728552.1\| | 28.35 | 7.00E-14 |
| 3-OXOACYL-ACP-REDUCT-MONOMER | gi\|113866193\|ref\|YP_724682.1\| | 28.95 | 3.00E-13 |
| 3-OXOACYL-ACP-REDUCT-MONOMER | gi\|116695568\|ref\|YP_841144.1\| | 29.03 | 3.00E-13 |
| 3-OXOACYL-ACP-REDUCT-MONOMER | gi\|116695287\|ref\|YP_840863.1\| | 30.22 | 5.00E-13 |
| 3-OXOACYL-ACP-REDUCT-MONOMER | gi\|113869676\|ref\|YP_728165.1\| | 28.14 | 1.00E-12 |
| 3-OXOACYL-ACP-REDUCT-MONOMER | gi\|113867547\|ref\|YP_726036.1\| | 22.48 | 1.00E-12 |
| 3-OXOACYL-ACP-REDUCT-MONOMER | gi\|113866289\|ref\|YP_724778.1\| | 28 | 4.00E-12 |
| 3-OXOACYL-ACP-REDUCT-MONOMER | gi\|113867750\|ref\|YP_726239.1\| | 29.59 | 3.00E-11 |
| 3-OXOACYL-ACP-REDUCT-MONOMER | gi\|116696287\|ref\|YP_841863.1\| | 27.6 | 4.00E-11 |
| 3-OXOACYL-ACP-REDUCT-MONOMER | gi\|116694597\|ref\|YP_728808.1\| | 31.69 | 2.00E-10 |
| 3-OXOACYL-ACP-REDUCT-MONOMER | gi\|116694624\|ref\|YP_728835.1\| | 33.01 | 7.00E-08 |
| FABZ-MONOMER | gi\|113868023\|ref\|YP_726512.1\| | 57.25 | 3.00E-45 |

The following homologues have been annotated as homologues by <<www.metacyc.org>>.

**Table 57:**

| **E. coli gene** | **C. necator gene** |
|---|---|
| fabD | fabD |
| fabH | fabH |
| fabB | fabB |
| fabF | fabF |
| fabG | h16_B1904, h16_B0361, h16_B0385 |
| fabZ | |
| fabI | PHG261, fabI1, fabI2 |

Strains can be made with equivalent combinations of fatty acid synthesis mutations to those described elsewhere herein for improved production of malonyl-CoA derived products in *E. coli.* Deletions and temperature sensitive mutations in equivalent homologues can be made by standard recombineering techniques previously described. Identification of relevant homologues for mutation/deletion can be completed by complementation of *E. coli* strains with FAS mutations. For example, in the case of fabI, the three fabI homologues annotated for C. necator were cloned into standard E. coli expression vectors and transformed using standard techniques into *E. coli* strains with and without temperature-sensitive fabI mutations. Strains constructed for this study are listed in Table 58 below:

| **Strain** | **Chromosomal genotype** | **Plasmid 1** | **Plasmid 2** |
|---|---|---|---|
| 595 | F-, Δ (araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | N/A | |
| 1 | F-, Δ (araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | pTRC empty vector | |
| 591 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | N/A | |
| 2 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | pTRC_fabI1 | |
| 3 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | pTRC_fabI2 | |
| 4 | F-, Δ(araD-araB)567, Δ1acZ4787(::rrnB-3), LAM-, rph-1, Δ(rhaD-rhaB)568, hsdR514, Δ1dhA:frt, ΔpflB:frt, mgsA:frt, ΔpoxB:frt, Δpta-ack:frt | pTRC_PHG261 | |

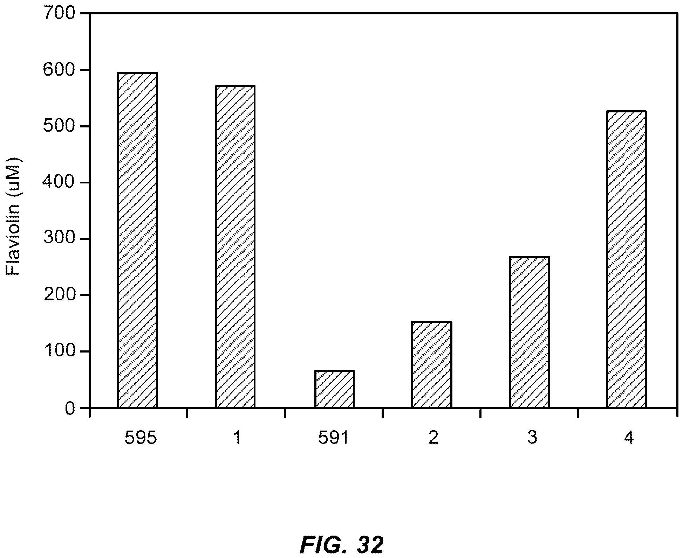

These strains were grown in shake flasks at 37 degrees Celsius in SM11 media for 24 hours. Samples were taken for OD600 and flaviolin measurements (see Common Methods section for detailed protocol). Results from flaviolin quantification are shown Figure 32. This complementation study suggests that fabI1, which shows the lowest production of flaviolin, is primarily responsible for fabI activity as seen by the diminished production and is followed by partial complementation observed for fabI2.

Strains with all combinations of fabI deletions/mutations are made by standard techniques. The genes encoding malonyl coA reductase (mcr) with combinations of other genes to enhance 3HP production described elsewhere herein are cloned into appropriate expression vectors for high level expression in *C. necator* using standard molecular biology techniques and transformed into *C. necator* hosts with FAS inhibition. Vectors were constructed on a broad host range plasmid (pBMT3) with the Ptrc induction system that drives expression of mcr and accAse. A subset of *C. necator* strains are constructed are listed in Table 59 below:

**Table 59:**

| **Strain** | **Modifications made to *C. necator H16* Chromosomal genotype** | **Plasmid 1** | **Plasmid 2** |
|---|---|---|---|
| C. necator H16 | | pBMT3-Ptrc-mcr-accABCD | N/A |
| C. necator H16-1 | fabI1(ts)-(S241F) | pBMT3-Ptrc-mcr-accABCD | N/A |
| C. necator H16-2 | fabI2(ts)-(S241F) | pBMT3-Ptrc-mcr-accABCD | N/A |
| C. necator H16-3 | fabI1 (ts)-(S241F), fabI2(ts)-(S241F) | pBMT3-Ptrc-mcr-accABCD | N/A |
| C. necator H16-4 | fabI1 (ts)-(S241F), fabI2(ts)-(S241F), ΔphaCAB | pBMT3-Ptrc-mcr-accABCD | N/A |

3HP production using syngas feedstocks is demonstrated at 0.6 L scale in SM11 (minimal salts) media. Cultures of *C. necator* strains listed in Table 59 are started from freezer stocks by standard practice (Sambrook and Russell, 2001) into 49 mL of FGN30 medium supplemented with appropriate antibiotics and incubated at 30°C for 24 hours with rotation at 250 rpm. 5 mL of this culture is transferred to 45 ml of FGN30 HN with appropriate antibiotics and is incubated at 30°C for 24 hours with rotation at 250 rpm. Cultures are used to inoculate gas-fed columns maintained at 30C as described under "Syngas Fermentation Method" in the Common Methods Section.

### Example 74: Butyrate production via malonyl-coA in strains with combinations of FAS mutations.

The following genetically modified *E. coli* strains (listed in Table 60) were constructed form a wild type BW25113 starting host. *E. coli* BW25113 was obtained from the Yale genetic stock center (New Haven, CT, USA). These strains were constructed by standard methods such as discussed in the Common Methods Section and also known in the art as referenced above. Briefly, chromosomal modifications were constructed via homologous recombination.

The following plasmids can be constructed by gene synthesis (Genscript, Piscataway, NJ). The target gene sequences were ordered (Genscript, Piscataway, NJ) including with modifications to the native ribosome binding site and subsequently changed to be compatible with existing expression vectors and to accommodate expression of key downstream gene(s) within the vector(s).

**Table 60: Strain List 16**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔfadD::frt, lambda-DE3; ΔatoDAEB:: frt | pACYC (empty) | pET28B (empty) |
| 2 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔfadD::frt, lambda-DE3; ΔatoDAEB:: frt | pACYC (empty) | pET28B (ptb-buk) |
| 3 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC(phaA-hbd-crt-ter) | pET28B (ptb-buk) |
| 4 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC(npht7-hbd-crt-ter) | pET28B (ptb-buk) |
| 5 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, fabB(ts), ΔfabF::frt, coaA*, fabD(ts), AlacI::frt, ΔpuuC::T5-aceEF-lpd*::loxP, ΔaceBAK::frt, lpd*::loxP, ΔaldB::PyibD-T7pol::loxP, ΔadhE::frt, ΔaldA::CSC, lambda-DE3, **AatoDAEB** | pACYC(npht7-hbd-crt-ter) | pET28B (ptb-buk) |

The above strains were evaluated in shake flasks for the production of butyrate. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM11 minimal medium with 5% culture as starting inoculum, and antibiotics. The cultures are grown at 30°C for approximately 4h to an OD of 0.4-0.6 and then induced with IPTG (0.5mM), after 1h cells were shifted to 37°C and monitored for 28 hours. Samples were taken at 18h and 28h and the supernatant was analyzed for the presence of Butyrate, and cells were saved to measure enzyme activity.

**Table 61. Butyrate Production.**

| **Strain:** | **Plasmid(s)** | **mg/L produced** |
|---|---|---|
| 1 | pACYC (Empty vector), pET28B (empty vector) | 0 |
| 2 | pACYC (Empty vector), pET28B (ptb-buk) | 0 |
| 3 | pACYC (pT7But4 (phaA, hbd, crt, ter), pET28B (ptb-buk) | 0 |
| 4 | pACYC (pT7But7 (nphT7, hbd, crt, ter), pET28B (ptb-buk) | 8-10mg/L in 18h 10mg/L - 14mg/L in 24h (OD=3-6) |
| 5 | pACYC (pT7But7 (nphT07, hbd, crt, ter), pET28B (ptb-buk) | 528 mg/L in 20 h (OD∼5) |

### Example 75: NADPH dependent Butyrate production via malonyl-coA in strains with combinations of FAS mutations.

The following genetically modified *E. coli* strains (listed in Table 62) were constructed form a wild type BW25113 starting host. *E. coli* BW25113 was obtained from the Yale genetic stock center (New Haven, CT, USA). These strains were constructed by standard methods such as discussed in the Common Methods Section and also known in the art as referenced above. Briefly, chromosomal modifications were constructed via homologous recombination.

The following plasmids can be constructed by gene synthesis (Genscript, Piscataway, NJ). The target gene sequences were ordered (Genscript, Piscataway, NJ) including with modifications to the native ribosome binding site and subsequently changed to be compatible with existing expression vectors and to accommodate expression of key downstream gene(s) within the vector(s).

**Table 62: Strain List 17**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔfadD::frt, lambda-DE3; ΔatoDAEB:: frt | pACYC (empty) | pET28B (empty) |
| 2 | F-, Δ(araD-araB)567, Δ1acZ4787(::rrnB-3), LAM-, rph-1, Δ(rhaD-rhaB)568, hsdR514, ΔldhA::frt, ApflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC(npht07-phaB-ech2-crr) | pET28B (ptb-buk) |

The above strains can be evaluated in shake flasks for the production of butyrate. Briefly, overnight starter cultures can be made in 50 mL of Luria Broth including the appropriate antibiotics and incubated 16-24 hours are 30°C, while shaking at 225 rpm. These cultures are used to inoculate 3 x 50 mL cultures of each strain in SM11 minimal medium with 5% culture as starting inoculum, and antibiotics. The cultures are grown at 30°C for approximately 4h to an OD of 0.4-0.6 and then induced with IPTG (0.5mM), after 1h cells are shifted to 37°C and monitored for 28 hours. Samples are taken at 18h and 28h and the supernatant is analyzed for the presence of Butyrate, and cells are saved to measure enzyme activity.

### Example 76: C10- and C14-free fatty acids production by using Trypanosoma brucei elongases in E. coli host cells that are genetically engineered for production of butyryl-CoA and malonyl-CoA.

*E. coli* host cells that are genetically engineered for production of butyryl-CoA and malonyl-CoA have been described above. The genes encoding *Trypanosoma brucei* elongases, ELO (SEQ ID NO: 199), ELO2 (SEQ ID NO: 200), and ELO3 (SEQ ID NO: 201), can be amplified from *T. brucei* genomic DNA and cloned into appropriate *E. coli* expression vectors. Alternatively, *ELO1, ELO2, and ELO3* can be synthesized chemically, codon-optimized for expression in *E. coli,* and cloned into appropriate *E. coli* expression vectors.

Genetically modified *E. coli* host cells with *T. brucei* ELO1, ELO2, and ELO3 alone will not be able to produce C10- and C14-free fatty acids, since accessory enzymes are needed. First, enzymes know as β-ketoacyl-CoA reductase, β-hydroxyacyl- CoA dehydratase, and enoyl-acyl-CoA reductase are required to work together with *T. brucei* ELO1 and/or ELO2 for biotransformation of butyryl-CoA and malonyl-CoA into C10- and C14-CoAs. *Saccharomyces cerevisiae* possess β-ketoacyl-CoA reductase, β-hydroxyacyl- CoA dehydratase, and enoyl-acyl-CoA reductase (YBR159W (SEQ ID NO: 202), PHS1 (SEQ ID NO: 203), TSC13 (SEQ ID NO: 204), respectively) that will function together with *T. brucei* ELO1 and ELO2. Genes encoding β-ketoacyl-CoA reductase (SEQ ID NO: 205), β-hydroxyacyl- CoA dehydratase (SEQ ID NO: 206), and enoyl-acyl-CoA reductase (SEQ ID NO: 207) are also present in *T. brucei* genomes. Genes encoding these 3 enzymes can be PCR-amplified from *S. cerevisiae* or *T. brucei* genomic DNA.

Alternatively, these genes can be synthesized chemically and codon-optimized for expression in *E. coli.* Genes encoding these 3 accessory enzymes can be cloned into a different *E. coli* expression vector that is compatible with the *E. coli* expression vector carrying *T. brucei* ELO1 and ELO2 genes. Alternatively, genes encoding these 3 accessory enzymes can be cloned into the same expression vector carrying *T. brucei* ELO1 and ELO2 genes. In the latter case, genes of ELO1, ELO2, β-ketoacyl-CoA reductase, β-hydroxyacyl- CoA dehydratase, and enoyl-acyl-CoA reductase will be under regulation of a single promoter and transcribed together in a polycistronic mRNA.

Second, a thioesterase is needed to convert C10- and C14-CoAs produced by ELO1, ELO2, β-ketoacyl-CoA reductase, β-hydroxyacyl- CoA dehydratase, and enoyl-acyl-CoA reductase into C10- and C14-free fatty acids. *E. coli tesA* encodes a thioesterase that is naturally expressed in the periplasm. A truncated version of *tesA* in which the secretion signal is removed (designated as *'tesA*) will allow expression of a TesA protein in *E. coli* cytoplasm (SEQ ID NO: 208). Alternatively, a second *E. coli* thioesterase gene known as *tesB* (SEQ ID NO: 209) can also be co-expressed with ELO1, ELO2, β-ketoacyl-CoA reductase, β-hydroxyacyl- CoA dehydratase, and enoyl-acyl-CoA reductase. Genes of either *tesA* or *tesB* are amplified by PCR from *E. coli* genome or chemically synthesized, and cloned into an expression vector that is compatible with the plasmids carrying ELO1, ELO2, β-ketoacyl-CoA reductase, β-hydroxyacyl-CoA dehydratase, and enoyl-acyl-CoA reductase. Alternatively, *tesA* or *tesB* can be cloned into the same plasmid carrying ELO1, ELO2, β-ketoacyl-CoA reductase, β-hydroxyacyl- CoA dehydratase, and enoyl-acyl-CoA reductase. In the latter case, all genes will be under regulation of a single promoter and transcribed together in a polycistronic mRNA.

Expression plasmids carrying *T. brucei* ELO1, β-ketoacyl-CoA reductase, β-hydroxyacyl- CoA dehydratase, enoyl-acyl-CoA reductase, and either *tesA* or *tesB* are transformed into the butyryl- and malonyl-CoA-producing *E. coli* host cells described above, giving rise to transformants that will produce C10-free fatty acid. In a separate experiment, expression plasmids carrying *T. brucei* ELO1 plus ELO2 with and without ELO3, β-ketoacyl-CoA reductase, β-hydroxyacyl- CoA dehydratase, enoyl-acyl-CoA reductase, and either *tesA* or *tesB* are transformed into the butyryl- and malonyl-CoA-producing *E. coli* host cells described above, giving rise to transformants that will produce C10-, C14-, and C-18free fatty acids.

The two different types of transformants are cultured independently in a suitable medium, such as LB broth at 37°C. Appropriate concentrations of antibiotics are included in the medium to keep the expression plasmids inside *E. coli* host cells. Expression of ELO1, ELO2, ELO3, and all accessory enzymes are induced by adding the appropriate inducers, such as IPTG, to the culture. Induced cultures are incubated at temperature between 15°C to 37°C, for 1-7 days.

Transformants expressing ELO1, β-ketoacyl-CoA reductase, β-hydroxyacyl- CoA dehydratase, enoyl-acyl-CoA reductase, and either *tesA* or *tesB* are capable of secreting C10-fatty acid into the medium. Transformants expressing ELO1, ELO2, β-ketoacyl-CoA reductase, β-hydroxyacyl- CoA dehydratase, enoyl-acyl-CoA reductase, and either *tesA* or *tesB* are capable of secreting C10- and C14-fatty acids into the medium. Transformants expressing ELO1, ELO2, ELO3, β-ketoacyl-CoA reductase, β-hydroxyacyl-CoA dehydratase, enoyl-acyl-CoA reductase, and either *tesA* or *tesB* are capable of secreting C10-, C14-, and C-18 fatty acids into the medium.

## Claims

1. A method for producing a C4-C18 fatty acid, said method comprising combining a carbon source, a microorganism, and a cell culture to produce the C4-C18 fatty acid, wherein
a) the microorganism is genetically modified for reduced enzymatic activity in at least one of beta-ketoacyl-ACP synthase (fabB), beta-ketoacyl-ACP synthase III (FabH), enoyl-ACP reductase (fabl), malonyl-CoA-ACP transacylase (fabD), β-ketoacyl-ACP reductase (fabG), and β-hydroxyacyl-ACP dehydratase (fabZ/fabA) such that enzymatic activity is reduced by at least 10, at least 20, at least 30, at least 40, at least 50, at least 60, at least 70, at least 80, or at least 90% compared with the activity of the native enzyme under standard conditions, providing for reduced conversion of malonyl-CoA to fatty acyl-ACPs; and
b) the microorganism additionally has one or more genetic modifications increasing fatty acid production, wherein the microorganism synthesizes fatty acyl-CoAs of 4-18 carbon chain length.

2. The method of claim 1, wherein the C4-C18 fatty acid is selected from the group consisting of C4, C6, C8, C10, C12, C14, C16 and C18 fatty acids.

3. The method of any of claims 1 to 2, wherein the microorganism expresses at least one heterologous elongase enzyme.

4. The method of claim 3, wherein the elongase enzyme is a peptide with a sequence corresponding to the group consisting of elo1, elo2, and elo3, or a peptide of 80% or more sequence identity with a peptide from SEQ ID NOs: 199-201.

5. The method of any one of claims 1 to 4, wherein the microorganism is E. coli.

6. The method of any one of claims 1-5, wherein the microorganism is further genetically modified to induce enzyme expression from the yibD gene promoter with a decreased environmental phosphate concentration.

7. A genetically modified microorganism for use according to any one of claims 1-6, wherein said microorganism:
a) is genetically modified for reduced enzymatic activity in at least one of beta-ketoacyl-ACP synthase (fabB), beta-ketoacyl-ACP synthase III (FabH), enoyl-ACP reductase (fabl), malonyl-CoA-ACP transacylase (fabD), β-ketoacyl-ACP reductase (fabG), and β-hydroxyacyl-ACP dehydratase (fabZ/fabA) such that enzymatic activity is reduced by at least 10, at least 20, at least 30, at least 40, at least 50, at least 60, at least 70, at least 80, or at least 90% compared with the activity of the native enzyme under standard conditions, providing for reduced conversion of malonyl-CoA to fatty acyl-ACPs; and
b) additionally has one or more genetic modifications increasing fatty acid production, wherein the microorganism synthesizes fatty acyl-CoAs of 4-18 carbon chain length.

8. The genetically modified microorganism of claim 7, wherein said microorganism is a yeast or a bacteria.

9. The genetically modified microorganism of claim 8, wherein said microorganism is E. coli.

10. The genetically modified microorganism of any one of claim 7 to claim 9, wherein said microorganism comprises at least two, three, four, five, six, seven, eight, nine, ten, or more sequences selected from group consisting of SEQ ID NO: 1-215 or sequences of 80% or more sequence identity with SEQ ID NO: 1-215.

## Patentansprüche

1. Verfahren zum Produzieren einer C4-C18 Fettsäure, wobei das genannte Verfahren das Kombinieren einer Kohlenstoffquelle, eines Mikroorganismus und einer Zellkultur zum Produzieren der C4-C18 Fettsäure beinhaltet, wobei
a) der Mikroorganismus genetisch für reduzierte enzymatische Aktivität in wenigstens einem aus Beta-ketoacyl-ACP-Synthase (fabB), Beta-ketoacyl-ACP-Synthase III (FabH), Enoyl-ACP-Reductase (fabl), Malonyl-CoA-ACP-Transacylase (fabD), β-Ketoacyl-ACP-Reductase (fabG) und β-Hydroxyacyl-ACP-Dehydratase (fabZ/fabA) modifiziert ist, so dass enzymatische Aktivität um wenigstens 10, wenigstens 20, wenigstens 30 wenigstens 40, wenigstens 50, wenigstens 60, wenigstens 70, wenigstens 80 oder wenigstens 90 % im Vergleich zur Aktivität des nativen Enzyms unter Standardbedingungen reduziert ist, um eine reduzierte Umwandlung von Malonyl-CoA in Fettsäureacyl-ACPs zu erzielen; und
b) der Mikroorganismus zusätzlich eine oder mehrere genetische Modifikationen hat, die Fettsäureproduktion erhöhen, wobei der Mikroorganismus Fettsäureacyl-CoAs mit einer Kettenlänge von 4-18 Kohlenstoffen synthetisiert.

2. Verfahren nach Anspruch 1, wobei die C4-C18 Fettsäure aus der Gruppe bestehend aus C4, C6, C8, C10, C12, C14, C16 und C18 Fettsäuren ausgewählt ist.

3. Verfahren nach einem der Ansprüche 1 bis 2, wobei der Mikroorganismus wenigstens ein heterologes Elongaseenzym exprimiert.

4. Verfahren nach Anspruch 3, wobei das Elongaseenzym ein Peptid mit einer Sequenz ist, die der Gruppe bestehend aus Elo1, Elo2 und Elo3 entspricht, oder ein Peptid von 80 % oder mehr Sequenzidentität mit einem Peptid von SEQ ID Nr. 199-201.

5. Verfahren nach einem der Ansprüche 1 bis 4, wobei der Mikroorganismus E. coli ist.

6. Verfahren nach einem der Ansprüche 1-5, wobei der Mikroorganismus ferner genetisch modifiziert ist, um Enzymexpression vom yibD-Genpromotor mit einer verringerten Umgebungsphosphatkonzentration zu induzieren.

7. Genetisch modifizierter Mikroorganismus zur Verwendung nach einem der Ansprüche 1-6, wobei der genannte Mikroorganismus:
a) genetisch für reduzierte enzymatische Aktivität in wenigstens einem aus Beta-ketoacyl-ACP-Synthase (fabB), Beta-ketoacyl-ACP-Synthase III (FabH), Enoyl-ACP-Reductase (fabl), Malonyl-CoA-ACP-Transacylase (fabD), β-Ketoacyl-ACP-Reductase (fabG) und β-Hydroxyacyl-ACP-Dehydratase (fabZ/fabA) modifiziert ist, so dass enzymatische Aktivität um wenigstens 10, wenigstens 20, wenigstens 30 wenigstens 40, wenigstens 50, wenigstens 60, wenigstens 70, wenigstens 80 oder wenigstens 90 % im Vergleich zur Aktivität des nativen Enzyms unter Standardbedingungen reduziert ist, um eine reduzierte Umwandlung von Malonyl-CoA in Fettsäureacyl-ACPs zu erzielen; und
b) zusätzlich eine oder mehrere genetische Modifikationen hat, die Fettsäureproduktion erhöhen, wobei der Mikroorganismus Fettsäureacyl-CoAs mit einer Kettenlänge von 4-18 Kohlenstoffatomen synthetisiert.

8. Genetisch modifizierter Mikroorganismus nach Anspruch 7, wobei der genannte Mikroorganismus eine Hefe oder ein Bakterium ist.

9. Genetisch modifizierter Mikroorganismus nach Anspruch 8, wobei der genannte Mikroorganismus E. coli ist.

10. Genetisch modifizierter Mikroorganismus nach einem der Ansprüche 7 bis 9, wobei der genannte Mikroorganismus wenigstens zwei, drei, vier, fünf, sechs, sieben, acht, neun, zehn oder mehr Sequenzen umfasst, ausgewählt aus der Gruppe bestehend aus SEQ ID Nr. 1-215 oder Sequenzen von 80 % oder mehr Sequenzidentität mit SEQ ID Nr. 1-215.

## Revendications

1. Procédé de production d'un acide gras en C4 à C18, ledit procédé comprenant une source de carbone, un micro-organisme et une culture cellulaire afin de produire l'acide gras en C4 à C18,
a) le micro-organisme étant génétiquement modifié pour réduire l'activité enzymatique dans au moins une enzyme parmi la bêta-cétoacyl-ACP synthase (fabB), la bêta-cétoacyl-ACP synthase III (FabH), l'énoyl-ACP réductase (fabl), la malonyl-CoA-ACP transacylase (fabD), la β-cétoacyl-ACP réductase (fabG) et la β-hydroxyacyl-ACP déshydratase (fabZ/fabA), de sorte que l'activité enzymatique soit réduite d'au moins 10, au moins 20, au moins 30, au moins 40, au moins 50, au moins 60, au moins 70, au moins 80 ou au moins 90 % par comparaison avec l'activité de l'enzyme native dans des conditions standard, prévoyant une conversion réduite de malonyl-CoA en acyl-ACP grasses ; et
b) le micro-organisme comportant en outre une ou plusieurs modifications génétiques augmentant la production d'acides gras, le micro-organisme synthétisant les acyl-CoA grasses ayant une longueur de chaîne de 4 à 18 atomes de carbone.

2. Procédé selon la revendication 1, dans lequel l'acide gras en C4 à C18 est choisi dans le groupe constitué d'acides gras en C4, C6, C8, C10, C12, C14, C16 et C18.

3. Procédé selon l'une quelconque des revendications 1 et 2, dans lequel le micro-organisme exprime au moins une enzyme élongase hétérologue.

4. Procédé selon la revendication 3, dans lequel l'enzyme élongase est un peptide avec une séquence correspondant au groupe constitué de elo1, elo2 et elo3, ou un peptide ayant une identité de séquence d'au moins 80 % avec un peptide de SEQ ID NO 199 à 201.

5. Procédé selon l'une quelconque des revendications 1 à 4, dans lequel le micro-organisme est E. coli.

6. Procédé selon l'une quelconque des revendications 1 à 5, dans lequel le micro-organisme est en outre génétiquement modifié pour induire une expression enzymatique à partir du promoteur du gène yibD avec une concentration en phosphate environnemental réduite.

7. Micro-organisme génétiquement modifié destiné à être utilisé selon l'une quelconque des revendications 1 à 6, ledit micro-organisme :
a) étant génétiquement modifié pour réduire l'activité enzymatique dans au moins une enzyme parmi la bêta-cétoacyl-ACP synthase (fabB), la bêta-cétoacyl-ACP synthase III (FabH), l'énoyl-ACP réductase (fabl), la malonyl-CoA-ACP transacylase (fabD), la β-cétoacyl-ACP réductase (fabG) et la β-hydroxyacyl-ACP déshydratase (fabZ/fabA), de sorte que l'activité enzymatique soit réduite d'au moins 10, au moins 20, au moins 30, au moins 40, au moins 50, au moins 60, au moins 70, au moins 80 ou au moins 90 % par comparaison avec l'activité de l'enzyme native dans des conditions standard, prévoyant une conversion réduite de malonyl-CoA en acyl-ACP grasses ; et
b) comportant en outre une ou plusieurs modifications génétiques augmentant la production d'acides gras, le micro-organisme synthétisant les acyl-CoA grasses ayant une longueur de chaîne de 4 à 18 atomes de carbone.

8. Micro-organisme génétiquement modifié selon la revendication 7, dans lequel ledit micro-organisme est une levure ou une bactérie.

9. Micro-organisme génétiquement modifié selon la revendication 8, dans lequel ledit micro-organisme est E. coli.

10. Micro-organisme génétiquement modifié selon l'une quelconque des revendications 7 à 9, dans lequel ledit micro-organisme comprend au moins deux, trois, quatre, cinq, six, sept, huit, neuf, dix séquences ou plus choisies dans le groupe constitué de SEQ ID NO 1 à 215 ou de séquences ayant une identité de séquence d'au moins 80 % avec SEQ ID NO 1 à 215.
